# EUROPEAN PATENT APPLICATION

(11) **EP 4 755 398 A1**
(43) Date of publication of application: **10.06.2026**
(21) Application number: 24850952.3
(22) Date of filing: 05.08.2024
(51) Int. Cl.: A61K 47/54, A61P 35/00

(54) **MULTIDRUG LINKER AND ANTIBODY-DRUG CONJUGATE**

(30) Priority: 04.08.2023 CN 202310980264; 31.01.2024 CN 202410138870; 30.07.2024 CN 202411037870
(71) Applicant: Shanghai Huao Co., Ltd., Shanghai 201401 (CN)
(72) Inventor: SUN, Wenlong, Shanghai 201401 (CN); MENG, Xun, Shanghai 201401 (CN); SHI, Yu, Shanghai 201401 (CN)
(74) Representative: Kuttenkeuler, David
(86) International application number: PCT/CN2024/109696
(87) International publication number: WO 2025/031307

(57) **Abstract**

A multidrug linker, an antibody-drug conjugate comprising the multidrug linker, a method for preparing the antibody-drug conjugate, and their use in disease treatment. A specific structure can effectively reduce the aggregation of a multi-payload antibody-drug conjugate, facilitating process scale-up. Furthermore, payload drugs can exert a significant synergistic effect, thereby achieving synergistic and enhanced anti-tumor efficacy, effectively overcoming the resistance to drugs, and improving the therapeutic window of drugs, among other benefits.

## Description

### Technical Field

The present invention relates to multidrug (e.g., dual-drug) linkers for antibody-drug conjugates (ADCs), as well as ADCs based on such multidrug linkers, their preparation methods and uses.

### Background

Traditional antibody-drug conjugates are all single-payload drug conjugates, meaning that the same toxin-linker is attached to a single antibody molecule, with differences between ADCs only in terms of drug loading number and conjugation sites. Single-payload antibody-drug conjugates achieve targeted therapy for tumor cells by linking a single drug molecule to an antibody molecule. However, due to the heterogeneity and drug resistance of tumor cells, the efficacy of single-payload antibody-drug conjugates in treatment has certain limitations. In the past decade, cancer immunotherapy and antibody-drug conjugates have achieved significant success in cancer therapy. However, high response rates are only observed in some specific tumors or tumors with high antigen expression, such as the success of HER2/3 ADCs in breast cancer, while clinical application responses in gastrointestinal solid tumors are moderate or even poor. These solid tumors exhibit significant heterogeneity of tumor cells and the surrounding tumor microenvironment (TME). In other words, a tumor is not just a cluster of transformed cells but also possesses a fibrotic microenvironment. Some solid tumor types, especially those associated with extensive fibrotic tumor stroma, respond poorly to immunotherapy, such as pancreatic ductal adenocarcinoma (PDAC), renal cell carcinoma (RCC), lung cancer, colorectal cancer, and other fibrotic solid tumors. The entire fibrotic response directly and indirectly affects the therapeutic response to chemotherapy and immunotherapy. Antibody-drug conjugates with dual-drug payloads can, on one hand, improve overall efficacy, and on the other hand, turn "cold" tumors "hot," further enhancing the therapeutic efficacy when combined with immunotherapeutic agents.

Dual toxins can reduce drug resistance because they can overcome intracellular or extracellular obstacles that a single toxin might encounter, such as downregulation of target expression, upregulation of drug pumps, activation of DNA repair mechanisms, etc. ADCs can leverage the high specificity and affinity of antibodies to precisely deliver two types of chemotherapeutic agents with synergistic effects into tumor cells, thereby achieving highly efficient killing. Therefore, researchers have begun exploring methods to combine two or more drug molecules, forming dual-payload or multi-payload antibody-drug conjugates to achieve synergistic effects of multiple drugs and improve therapeutic outcomes. Currently disclosed drug molecule combinations primarily involve different toxins, including combinations with the same or different mechanisms, for example: MMAE/MMAF combinations (Nat Commun 2021;12:3528; Angew Chem Int Ed Engl 2017;56:733-7; Bioconjugate Chem. 2023, 34, 4, 748-755); DM1/MMAE combinations (Nature Protocols 2017;12:1702-21; Pharmaceutics 2023;15:2020); PNU-159682/MMAF combinations (Antib Ther 2019;2:71-8); MMAE/α-Amanitin combinations (Int J Mol Sci 2018;19:2098); MMAE/PBD combinations (Bioorg Med Chem Lett 2018;28:3617-21). Early efficacy studies have demonstrated that dual-toxin ADC molecules can significantly enhance efficacy while also increasing the killing activity of ADC drugs against drug-resistant tumor cells. Furthermore, existing reports related to conjugation methods, processes, and dual-toxin ADCs include: using MMAF in tandem with different drugs to obtain antibody-drug conjugate molecules loaded with dual toxins (PCT/CN2021/107079); and simultaneously conjugating one or more drugs to a limited number of conjugation sites on an antibody using one or more cysteine residues or cysteine derivative residues as drug-linker carriers (PCT/CN2019/112663); parallel conjugation of traditional drugs such as VC-PAB-MMAE, GGFG-DXd, and Eribulin via bidentate linking groups (PCT/CN2022/080942); constructing multi-drug antibody-drug conjugates by "orthogonal" deprotection and drug loading in site-specific entities (PCT/US2017/066504); and using a dual Cysteine multiplexing carrier introducing two protected cysteine groups [Cys (SiPr)+Cys (Acm)], combined with different deprotection reactions to construct dual-payload ADCs (Angew Chem Int Ed Engl 2017;56:733-7). However, this process requires highly toxic catalytic reagents like Hg(OAc)₂, increasing drug safety risks and posing potential adverse effects on protein structure and stability.

The aforementioned prior art primarily achieves conjugation of different toxins to antibodies through "linear linkers" or "branched linkers". Since the water solubility of existing linker-toxin combinations is relatively poor, current dual-payload applications are limited to some high-potency toxins requiring low drug loading (low DAR values) in antibody-drug conjugates. However, the biggest problem currently limiting the clinical application of ADC drugs is the therapeutic window. As classic ADC payload toxins, MMAF, PBD, and PNU-159682 molecules have significant dose-limiting toxicities, and related ADC molecules have ended in failure in clinical development (Risk Minimization of Antibody-Drug Conjugates in Oncology: A Review. Drug Saf 2021;44:733-42). The basic strategy of the aforementioned prior art is essentially random combination of highly active drugs, further expanding the "toxicity profile" of ADC drugs, causing the same ADC drug to possess broader and greater toxicity while gaining higher activity. Furthermore, from a technical perspective, the aforementioned prior art only considers simple combination of existing linkers, obtaining dual-toxin loaded antibody-drug conjugate molecules by simple "serial" or "parallel" combination of toxin molecules and chemotherapeutic drugs with traditional linkers. However, chemotherapeutic drug-linker combinations also suffer from high hydrophobicity. Therefore, when conjugated to antibody macromolecules as dual-drug linker combinations, they significantly reduce the stability of the antibody molecule, leading to further problems such as shortened in vivo half-life, poorer PK properties, and immunogenicity of the drugs. Additionally, early research mainly focused on auristatins, a class of toxins with good hydrophilicity and high activity, making dual-drug antibody-drug conjugates relatively easy to achieve. However, in recent years, topoisomerase inhibitors, DNA damage response (DDR) inhibitors, and most tumor-targeted drugs, which are widely studied, generally have relatively low activity and very high hydrophobicity. Therefore, such compisitions as antibody-drug conjugate payload molecules first require higher DAR values and appropriate drug ratios to achieve optimal efficacy and safety, which are difficult to achieve with existing technologies.

Therefore, there is an urgent need in the art to provide more suitable novel antibody-drug conjugates loaded with multiple classes of drugs having synergistic mechanisms of action, enabling efficient, simple, and practical chemical preparation and conjugation, and/or improving pharmaceutical properties, metabolic properties, efficacy, and/or safety of existing antibody-drug conjugates, such as enhancing the stability of multi-payload drug ADC molecules, improving drug PK properties in vivo, and broadening the therapeutic window.

### Summary

One aspect of the present invention provides a compound, or a tautomer, a mesomer, a racemate, an enantiomer, or a diastereomer thereof, or a deuterated compound form thereof, or a mixture form thereof, or a pharmaceutically acceptable salt, prodrug, or solvate thereof, wherein the compound comprises a structure represented by formula I: 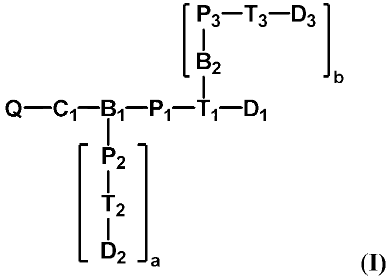 wherein,
Q is a functional group capable of conjugating to cysteine, lysine, a non-natural amino acid, or a glycosyl of an antibody molecule;
C₁ is selected from the group consisting of: a direct bond, optionally substituted alkylene, optionally substituted polyethylene glycol group, optionally substituted alkenylene, optionally substituted alkynylene, optionally substituted aliphatic cyclylene, optionally substituted aliphatic heterocyclylene, optionally substituted arylene, and optionally substituted heteroarylene; when substituted, the substituent(s) are selected from halogen, hydroxyl, amino, carboxyl, sulfonic acid group, sulfone group, phosphonic acid group, and alkoxy;
B₁ and B₂ are independently a direct bond or a branching group, provided that when C₁ is a direct bond, B₁ is not a direct bond;
P₁, P₂, and P₃ are each independently an optionally substituted polypeptide residue or a glucose fragment;
T₁, T₂, and T₃ are each independently a direct bond or an optionally substituted spacer group, provided that at most two of T₁, T₂, and T₃ are direct bonds and at least one of B₂, T₁, T₂, and T₃ is substituted with a hydrophilic group;
D₁, D₂, and D₃ are a first drug unit, a second drug unit, and a third drug unit, respectively, and are the same or different;
a and b are independently 0, 1, 2, or 3, provided that a and b are not both 0; and
when a=0, B₁ is a direct bond and T₁ is not a direct bond;
when b=0, B₁ is not a direct bond.

In some embodiments, the present invention provides a compound, or a tautomer, a mesomer, a racemate, an enantiomer, or a diastereomer thereof, or a deuterated compound form thereof, or a mixture form thereof, or a pharmaceutically acceptable salt, prodrug, or solvate thereof, wherein the compound comprises a structure represented by formula Ia: 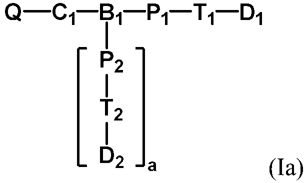 wherein:
Q is a functional group capable of conjugating to cysteine, lysine, a non-natural amino acid, or a glycosyl of an antibody molecule;
C₁ is selected from the group consisting of: a direct bond, optionally substituted alkylene, optionally substituted polyethylene glycol group, optionally substituted alkenylene, optionally substituted alkynylene, optionally substituted aliphatic cyclylene, optionally substituted aliphatic heterocyclylene, optionally substituted arylene, and optionally substituted heteroarylene; when substituted, the substituent(s) are selected from halogen, hydroxyl, amino, carboxyl, sulfonic acid group, sulfone group, phosphonic acid group, and alkoxy;
B₁ is a branching group;
P₁ and P₂ are each independently an optionally substituted polypeptide residue or a glucose fragment;
T₁ and T₂ are each independently a direct bond or an optionally substituted spacer group, provided that at most one of T₁ and T₂ is a direct bond, and at least one of T₁ and T₂ is substituted with a hydrophilic group;
D₁ and D₂ are a first drug unit and a second drug unit, respectively, and are the same or different;
a is 1, 2, or 3.

In some embodiments, the present invention provides a compound, or a tautomer, a mesomer, a racemate, an enantiomer, or a diastereomer thereof, or a deuterated compound form thereof, or a mixture form thereof, or a pharmaceutically acceptable salt, prodrug, or solvate thereof, wherein the compound comprises a structure represented by formula Ib: 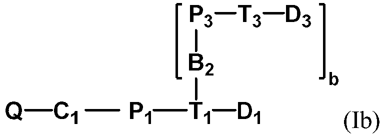 wherein:
Q is a functional group capable of conjugating to cysteine, lysine, a non-natural amino acid, or a glycosyl of an antibody molecule;
C₁ is selected from the group consisting of: optionally substituted alkylene, optionally substituted polyethylene glycol group, optionally substituted alkenylene, optionally substituted alkynylene, optionally substituted aliphatic cyclylene, optionally substituted aliphatic heterocyclylene, optionally substituted arylene, and optionally substituted heteroarylene; when substituted, the substituent(s) are selected from halogen, hydroxyl, amino, carboxyl, sulfonic acid group, sulfone group, phosphonic acid group, and alkoxy;
B₂ is a branching group;
P₁ and P₃ are each independently a direct bond or an optionally substituted polypeptide residue or a glucose fragment;
T₁ is an optionally substituted spacer group, T₃ is a direct bond or an optionally substituted spacer group, and at least one of B₂ and T₃ is substituted with a hydrophilic group;
D₁ and D₃ are a first drug unit and a third drug unit, respectively, and are the same or different;
b is 1, 2, or 3.
In some other embodiments, the present invention provides a compound, or a tautomer, a mesomer, a racemate, an enantiomer, or a diastereomer thereof, or a deuterated compound form thereof, or a mixture form thereof, or a pharmaceutically acceptable salt, prodrug, or solvate thereof, wherein the compound comprises a structure represented by formula I: 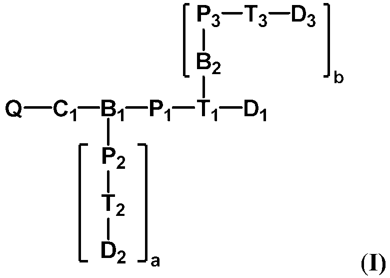
wherein,
Q is a functional group capable of conjugating to cysteine, lysine, a non-natural amino acid, or a glycosyl of an antibody molecule;
C₁ is selected from the group consisting of: a direct bond, optionally substituted alkylene, optionally substituted polyethylene glycol group, optionally substituted alkenylene, optionally substituted alkynylene, optionally substituted aliphatic cyclylene, optionally substituted aliphatic heterocyclylene, optionally substituted arylene, and optionally substituted heteroarylene; when substituted, the substituent(s) are selected from halogen, hydroxyl, amino, carboxyl, sulfonic acid group, sulfone group, phosphonic acid group, and alkoxy;
B₁ and B₂ are independently a branching group;
P₁, P₂, and P₃ are each independently an optionally substituted polypeptide residue or a glucose fragment;
T₁ is an optionally substituted spacer group, T₂ and T₃ are each independently a direct bond or an optionally substituted spacer group, and at least one of B₂, T₂, and T₃ is substituted with a hydrophilic group;
D₁, D₂, and D₃ are a first drug unit, a second drug unit, and a third drug unit, respectively, and are the same or different; and
a and b are independently 1, 2, or 3.

In preferred embodiments, the present invention provides compounds selected from formulas **A1** to **A188,** or a tautomer, a mesomer, a racemate, an enantiomer, or a diastereomer thereof, or a deuterated compound form thereof, or a mixture form thereof, or a pharmaceutically acceptable salt, prodrug, or solvate thereof.

Another aspect of the present invention provides a compound, or a tautomer, a mesomer, a racemate, an enantiomer, or a diastereomer thereof, or a deuterated compound form thereof, or a mixture form thereof, or a pharmaceutically acceptable salt, prodrug, or solvate thereof, wherein the compound comprises a structure represented by formula II: 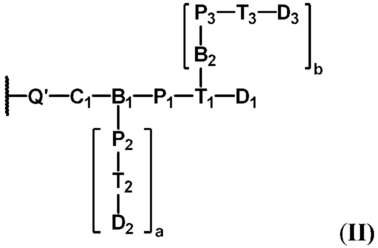 wherein,
Q' is a functional group capable of conjugating to cysteine, lysine, a non-natural amino acid, or a glycosyl of an antibody molecule;
C₁ is selected from the group consisting of: a direct bond, optionally substituted alkylene, optionally substituted polyethylene glycol group, optionally substituted alkenylene, optionally substituted alkynylene, optionally substituted aliphatic cyclylene, optionally substituted aliphatic heterocyclylene, optionally substituted arylene, and optionally substituted heteroarylene; when substituted, the substituent(s) are selected from halogen, hydroxyl, amino, carboxyl, sulfonic acid group, sulfone group, phosphonic acid group, and alkoxy;
B₁ and B₂ are independently a direct bond or a branching group, provided that when C₁ is a direct bond, B₁ is not a direct bond;
P₁, P₂, and P₃ are each independently an optionally substituted polypeptide residue or a glucose fragment;
T₁, T₂, and T₃ are each independently a direct bond or an optionally substituted spacer group, provided that at most two of T₁, T₂, and T₃ are direct bonds and at least one of B₂, T₁, T₂, and T₃ is substituted with a hydrophilic group;
D₁, D₂, and D₃ are a first drug unit, a second drug unit, and a third drug unit, respectively, and are the same or different;
a and b are independently 0, 1, 2, or 3, provided that a and b are not both 0; and
when a=0, B₁ is a direct bond and T₁ is not a direct bond;
when b=0, B₁ is not a direct bond.

In some embodiments, the present invention provides a compound, or a tautomer, a mesomer, a racemate, an enantiomer, or a diastereomer thereof, or a deuterated compound form thereof, or a mixture form thereof, or a pharmaceutically acceptable salt, prodrug, or solvate thereof, wherein the compound comprises a structure represented by formula IIa: 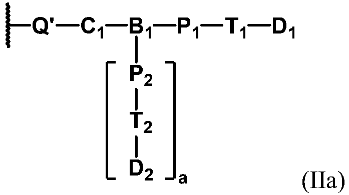 wherein:
Q' is a functional group capable of conjugating to cysteine, lysine, a non-natural amino acid, or a glycosyl of an antibody molecule;
C₁ is selected from the group consisting of: a direct bond, optionally substituted alkylene, optionally substituted polyethylene glycol group, optionally substituted alkenylene, optionally substituted alkynylene, optionally substituted aliphatic cyclylene, optionally substituted aliphatic heterocyclylene, optionally substituted arylene, and optionally substituted heteroarylene; when substituted, the substituent(s) are selected from halogen, hydroxyl, amino, carboxyl, sulfonic acid group, sulfone group, phosphonic acid group, and alkoxy;
B₁ is a branching group;
P₁ and P₂ are each independently an optionally substituted polypeptide residue or a glucose fragment;
T₁ and T₂ are each independently a direct bond or an optionally substituted spacer group, provided that at most one of T₁ and T₂ is a direct bond, and at least one of T₁ and T₂ is substituted with a hydrophilic group;
D₁ and D₂ are a first drug unit and a second drug unit, respectively, and are the same or different;
a is 1, 2, or 3.

In some embodiments, the present invention provides a compound, or a tautomer, a mesomer, a racemate, an enantiomer, or a diastereomer thereof, or a deuterated compound form thereof, or a mixture form thereof, or a pharmaceutically acceptable salt, prodrug, or solvate thereof, wherein the compound comprises a structure represented by formula IIb: 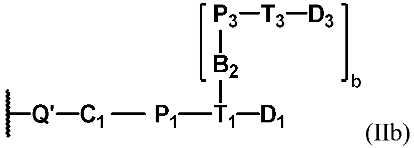
Q' is a functional group capable of conjugating to cysteine, lysine, a non-natural amino acid, or a glycosyl of an antibody molecule;
C₁ is selected from the group consisting of: optionally substituted alkylene, optionally substituted polyethylene glycol group, optionally substituted alkenylene, optionally substituted alkynylene, optionally substituted aliphatic cyclylene, optionally substituted aliphatic heterocyclylene, optionally substituted arylene, and optionally substituted heteroarylene; when substituted, the substituent(s) are selected from halogen, hydroxyl, amino, carboxyl, sulfonic acid group, sulfone group, phosphonic acid group, and alkoxy;
B₂ is a branching group;
P₁ and P₃ are each independently a direct bond or an optionally substituted polypeptide residue or a glucose fragment;
T₁ is an optionally substituted spacer group, T₃ is a direct bond or an optionally substituted spacer group, and at least one of B₂ and T₃ is substituted with a hydrophilic group;
D₁ and D₃ are a first drug unit and a third drug unit, respectively, and are the same or different;
b is 1, 2, or 3.

In some embodiments, the present invention provides a compound, or a tautomer, a mesomer, a racemate, an enantiomer, or a diastereomer thereof, or a deuterated compound form thereof, or a mixture form thereof, or a pharmaceutically acceptable salt, prodrug, or solvate thereof, wherein the compound comprises a structure represented by formula II: 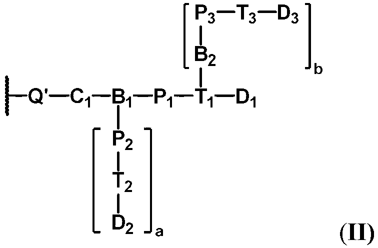 wherein,
Q' is a functional group capable of conjugating to cysteine, lysine, a non-natural amino acid, or a glycosyl of an antibody molecule;
C₁ is selected from the group consisting of: a direct bond, optionally substituted alkylene, optionally substituted polyethylene glycol group, optionally substituted alkenylene, optionally substituted alkynylene, optionally substituted aliphatic cyclylene, optionally substituted aliphatic heterocyclylene, optionally substituted arylene, and optionally substituted heteroarylene; when substituted, the substituent(s) are selected from halogen, hydroxyl, amino, carboxyl, sulfonic acid group, sulfone group, phosphonic acid group, and alkoxy;
B₁ and B₂ are independently a branching group;
P₁, P₂, and P₃ are each independently an optionally substituted polypeptide residue or a glucose fragment;
T₁ is an optionally substituted spacer group, T₂ and T₃ are each independently a direct bond or an optionally substituted spacer group, and at least one of B₂, T₂, and T₃ is substituted with a hydrophilic group;
D₁, D₂, and D₃ are a first drug unit, a second drug unit, and a third drug unit, respectively, and are the same or different; and
a and b are independently 1, 2, or 3.

In preferred embodiments, the present invention provides compounds selected from formulas **B1** to **B190,** or a tautomer, a mesomer, a racemate, an enantiomer, or a diastereomer thereof, or a deuterated compound form thereof, or a mixture form thereof, or a pharmaceutically acceptable salt, prodrug, or solvate thereof.

Another aspect of the present invention provides a linker, or a tautomer, a mesomer, a racemate, an enantiomer, or a diastereomer thereof, or a deuterated compound form thereof, or a mixture form thereof, or a pharmaceutically acceptable salt, prodrug, or solvate thereof, wherein the linker has a structure represented by formula IV: 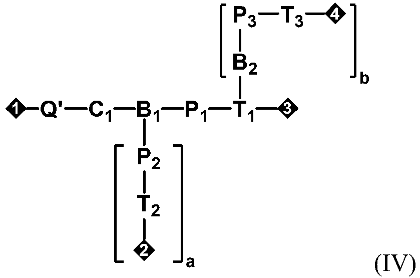 wherein,
Q' is a functional group capable of conjugating to cysteine, lysine, a non-natural amino acid, or a glycosyl of an antibody molecule, and attachment point 1 is connected to the antibody;
C₁ is selected from the group consisting of: a direct bond, optionally substituted alkylene, optionally substituted polyethylene glycol group, optionally substituted alkenylene, optionally substituted alkynylene, optionally substituted aliphatic cyclylene, optionally substituted aliphatic heterocyclylene, optionally substituted arylene, and optionally substituted heteroarylene; when substituted, the substituent(s) are selected from halogen, hydroxyl, amino, carboxyl, sulfonic acid group, sulfone group, phosphonic acid group, and alkoxy;
B₁ and B₂ are independently a direct bond or a branching group, provided that when C₁ is a direct bond, B₁ is not a direct bond;
P₁, P₂, and P₃ are each independently an optionally substituted polypeptide residue or a glucose fragment;
T₁, T₂, and T₃ are each independently a direct bond or an optionally substituted spacer group, provided that at most two of T₁, T₂, and T₃ are direct bonds and at least one of B₂, T₁, T₂, and T₃ is substituted with a hydrophilic group;
attachment points 2, 3, 4 are connected to drug units;
a and b are independently 0, 1, 2, or 3, provided that a and b are not both 0; and
when a=0, B₁ is a direct bond and T₁ is not a direct bond;
when b=0, B₁ is not a direct bond.

In some embodiments, the present invention provides a linker, or a tautomer, a mesomer, a racemate, an enantiomer, or a diastereomer thereof, or a deuterated compound form thereof, or a mixture form thereof, or a pharmaceutically acceptable salt, prodrug, or solvate thereof, wherein the compound comprises a structure represented by formula IVa: 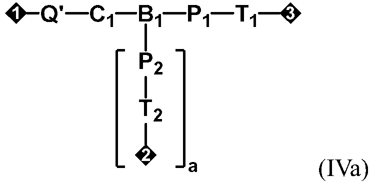 wherein:
Q' is a functional group capable of conjugating to cysteine, lysine, a non-natural amino acid, or a glycosyl of an antibody molecule, and attachment point 1 is connected to the antibody;
C₁ is selected from the group consisting of: a direct bond, optionally substituted alkylene, optionally substituted polyethylene glycol group, optionally substituted alkenylene, optionally substituted alkynylene, optionally substituted aliphatic cyclylene, optionally substituted aliphatic heterocyclylene, optionally substituted arylene, and optionally substituted heteroarylene; when substituted, the substituent(s) are selected from halogen, hydroxyl, amino, carboxyl, sulfonic acid group, sulfone group, phosphonic acid group, and alkoxy;
B₁ is a branching group;
P₁ and P₂ are each independently an optionally substituted polypeptide residue or a glucose fragment;
T₁ and T₂ are each independently a direct bond or an optionally substituted spacer group, provided that at most one of T₁ and T₂ is a direct bond, and at least one of T₁ and T₂ is substituted with a hydrophilic group;
attachment points 2 and 3 are connected to drug units;
a is 1, 2, or 3.

In some embodiments, the present invention provides a linker, or a tautomer, a mesomer, a racemate, an enantiomer, or a diastereomer thereof, or a deuterated compound form thereof, or a mixture form thereof, or a pharmaceutically acceptable salt, prodrug, or solvate thereof, wherein the compound comprises a structure represented by formula IVb: 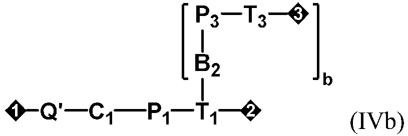
Q' is a functional group capable of conjugating to cysteine, lysine, a non-natural amino acid, or a glycosyl of an antibody molecule, and attachment point 1 is connected to the antibody;
C₁ is selected from the group consisting of: optionally substituted alkylene, optionally substituted polyethylene glycol group, optionally substituted alkenylene, optionally substituted alkynylene, optionally substituted aliphatic cyclylene, optionally substituted aliphatic heterocyclylene, optionally substituted arylene, and optionally substituted heteroarylene; when substituted, the substituent(s) are selected from halogen, hydroxyl, amino, carboxyl, sulfonic acid group, sulfone group, phosphonic acid group, and alkoxy;
B₂ is a branching group;
P₁ and P₃ are each independently a direct bond or an optionally substituted polypeptide residue or a glucose fragment;
T₁ is an optionally substituted spacer group, T₃ is a direct bond or an optionally substituted spacer group, and at least one of B₂ and T₃ is substituted with a hydrophilic group;
attachment points 2 and 3 are connected to drug units;
b is 1, 2, or 3.

In some embodiments, the present invention provides a linker, or a tautomer, a mesomer, a racemate, an enantiomer, or a diastereomer thereof, or a deuterated compound form thereof, or a mixture form thereof, or a pharmaceutically acceptable salt, prodrug, or solvate thereof, wherein the linker has a structure represented by formula IV: 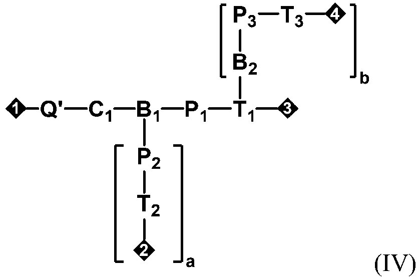 wherein,
Q' is a functional group capable of conjugating to cysteine, lysine, a non-natural amino acid, or a glycosyl of an antibody molecule, and attachment point 1 is connected to the antibody;
C₁ is selected from the group consisting of: a direct bond, optionally substituted alkylene, optionally substituted polyethylene glycol group, optionally substituted alkenylene, optionally substituted alkynylene, optionally substituted aliphatic cyclylene, optionally substituted aliphatic heterocyclylene, optionally substituted arylene, and optionally substituted heteroarylene; when substituted, the substituent(s) are selected from halogen, hydroxyl, amino, carboxyl, sulfonic acid group, sulfone group, phosphonic acid group, and alkoxy;
B₁ and B₂ are independently a branching group;
P₁, P₂, and P₃ are each independently an optionally substituted polypeptide residue or a glucose fragment;
T₁ is an optionally substituted spacer group, T₂ and T₃ are each independently a direct bond or an optionally substituted spacer group, and at least one of B₂, T₂, and T₃ is substituted with a hydrophilic group; and
attachment points 2, 3, 4 are connected to drug units;
a and b are independently 1, 2, or 3.

In some embodiments, the present invention provides linkers as shown in formulas **D1** to **D37,** or a tautomer, a mesomer, a racemate, an enantiomer, or a diastereomer thereof, or a deuterated compound form thereof, or a mixture form thereof, or a pharmaceutically acceptable salt, prodrug, or solvate thereof.

Another aspect of the present invention provides a compound, or a tautomer, a mesomer, a racemate, an enantiomer, or a diastereomer thereof, or a deuterated compound form thereof, or a mixture form thereof, or a pharmaceutically acceptable salt, prodrug, or solvate thereof, wherein the compound comprises a structure represented by formula III: 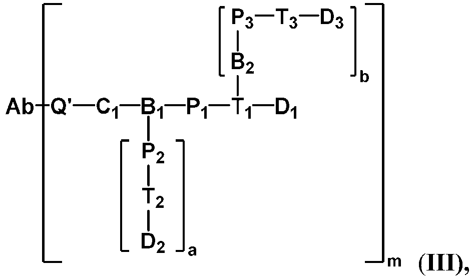
wherein, Ab is an antibody molecule, m is an integer or decimal from 1 to 8;
Q' is a functional group capable of conjugating to cysteine, lysine, a non-natural amino acid, or a glycosyl of an antibody molecule;
C₁ is selected from the group consisting of: a direct bond, optionally substituted alkylene, optionally substituted polyethylene glycol group, optionally substituted alkenylene, optionally substituted alkynylene, optionally substituted aliphatic cyclylene, optionally substituted aliphatic heterocyclylene, optionally substituted arylene, and optionally substituted heteroarylene; when substituted, the substituent(s) are selected from halogen, hydroxyl, amino, carboxyl, sulfonic acid group, sulfone group, phosphonic acid group, and alkoxy;
B₁ and B₂ are independently a direct bond or a branching group, provided that when C₁ is a direct bond, B₁ is not a direct bond;
P₁, P₂, and P₃ are each independently an optionally substituted polypeptide residue or a glucose fragment;
T₁, T₂, and T₃ are each independently a direct bond or an optionally substituted spacer group, provided that at most two of T₁, T₂, and T₃ are direct bonds and at least one of B₂, T₁, T₂, and T₃ is substituted with a hydrophilic group;
D₁, D₂, and D₃ are a first drug unit, a second drug unit, and a third drug unit, respectively, and are the same or different;
a and b are independently 0, 1, 2, or 3, provided that a and b are not both 0; and
when a=0, B₁ is a direct bond and T₁ is not a direct bond;
when b=0, B₁ is not a direct bond.

In some embodiments, the present invention provides a compound, or a tautomer, a mesomer, a racemate, an enantiomer, or a diastereomer thereof, or a deuterated compound form thereof, or a mixture form thereof, or a pharmaceutically acceptable salt, prodrug, or solvate thereof, wherein the compound comprises a structure represented by formula IIIa: 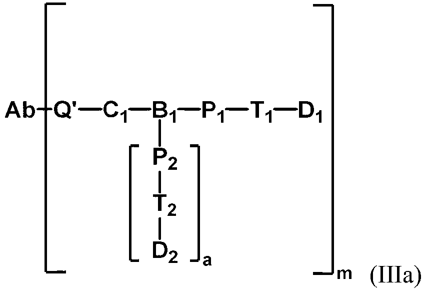 wherein:
wherein, Ab is an antibody molecule, m is an integer or decimal from 1 to 8;
Q' is a functional group capable of conjugating to cysteine, lysine, a non-natural amino acid, or a glycosyl of an antibody molecule;
C₁ is selected from the group consisting of: a direct bond, optionally substituted alkylene, optionally substituted polyethylene glycol group, optionally substituted alkenylene, optionally substituted alkynylene, optionally substituted aliphatic cyclylene, optionally substituted aliphatic heterocyclylene, optionally substituted arylene, and optionally substituted heteroarylene; when substituted, the substituent(s) are selected from halogen, hydroxyl, amino, carboxyl, sulfonic acid group, sulfone group, phosphonic acid group, and alkoxy;
B₁ is a branching group;
P₁ and P₂ are each independently an optionally substituted polypeptide residue or a glucose fragment;
T₁ and T₂ are each independently a direct bond or an optionally substituted spacer group, provided that at most one of T₁ and T₂ is a direct bond, and at least one of T₁ and T₂ is substituted with a hydrophilic group;
D₁ and D₂ are a first drug unit and a second drug unit, respectively, and are the same or different;
a is 1, 2, or 3.

In some embodiments, the present invention provides a compound, or a tautomer, a mesomer, a racemate, an enantiomer, or a diastereomer thereof, or a deuterated compound form thereof, or a mixture form thereof, or a pharmaceutically acceptable salt, prodrug, or solvate thereof, wherein the compound comprises a structure represented by formula IIIb: 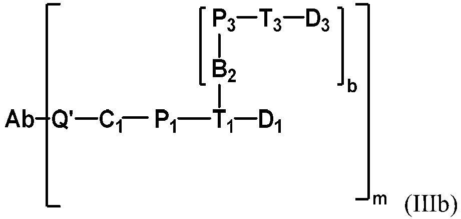
Q' is a functional group capable of conjugating to cysteine, lysine, a non-natural amino acid, or a glycosyl of an antibody molecule;
C₁ is selected from the group consisting of: optionally substituted alkylene, optionally substituted polyethylene glycol group, optionally substituted alkenylene, optionally substituted alkynylene, optionally substituted aliphatic cyclylene, optionally substituted aliphatic heterocyclylene, optionally substituted arylene, and optionally substituted heteroarylene; when substituted, the substituent(s) are selected from halogen, hydroxyl, amino, carboxyl, sulfonic acid group, sulfone group, phosphonic acid group, and alkoxy;
B₂ is a branching group;
P₁ and P₃ are each independently a direct bond or an optionally substituted polypeptide residue or a glucose fragment;
T₁ is an optionally substituted spacer group, T₃ is a direct bond or an optionally substituted spacer group, and at least one of B₂ and T₃ is substituted with a hydrophilic group;
D₁ and D₃ are a first drug unit and a third drug unit, respectively, and are the same or different;
b is 1, 2, or 3.

In some embodiments, the present invention provides a compound, or a tautomer, a mesomer, a racemate, an enantiomer, or a diastereomer thereof, or a deuterated compound form thereof, or a mixture form thereof, or a pharmaceutically acceptable salt, prodrug, or solvate thereof, wherein the compound comprises a structure represented by formula III: 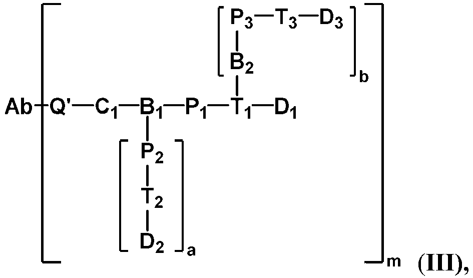
wherein, Ab is an antibody molecule, m is an integer or decimal from 1 to 8;
Q' is a functional group capable of conjugating to cysteine, lysine, a non-natural amino acid, or a glycosyl of an antibody molecule;
C₁ is selected from the group consisting of: a direct bond, optionally substituted alkylene, optionally substituted polyethylene glycol group, optionally substituted alkenylene, optionally substituted alkynylene, optionally substituted aliphatic cyclylene, optionally substituted aliphatic heterocyclylene, optionally substituted arylene, and optionally substituted heteroarylene; when substituted, the substituent(s) are selected from halogen, hydroxyl, amino, carboxyl, sulfonic acid group, sulfone group, phosphonic acid group, and alkoxy;
B₁ and B₂ are independently a branching group;
P₁, P₂, and P₃ are each independently an optionally substituted polypeptide residue or a glucose fragment;
T₁ is an optionally substituted spacer group, T₂ and T₃ are each independently a direct bond or an optionally substituted spacer group, and at least one of B₂, T₂, and T₃ is substituted with a hydrophilic group;
D₁, D₂, and D₃ are a first drug unit, a second drug unit, and a third drug unit, respectively, and are the same or different; and
a and b are independently 1, 2, or 3.

In preferred embodiments, the present invention provides compounds selected from formulas **C1** to **C190,** or a tautomer, a mesomer, a racemate, an enantiomer, or a diastereomer thereof, or a deuterated compound form thereof, or a mixture form thereof, or a pharmaceutically acceptable salt, prodrug, or solvate thereof.

Other aspects of the present invention will be readily apparent from the detailed description of the following specific embodiments.

### Brief Description of Drawings

Figure 1 shows the size exclusion chromatography (SEC-HPLC) chromatogram of the antibody conjugate T-MDP1.
Figure 2 shows the hydrophobic interaction chromatography (HIC-HPLC) chromatogram of the antibody conjugate T-MDP1.
Figure 3 shows the size exclusion chromatography (SEC-HPLC) chromatogram of the antibody conjugate T-MDP2.
Figure 4 shows the hydrophobic interaction chromatography (HIC-HPLC) chromatogram of the antibody conjugate T-MDP2.
Figure 5 shows the size exclusion chromatography (SEC-HPLC) chromatogram of the antibody conjugate T-MDP3.
Figure 6 shows the hydrophobic interaction chromatography (HIC-HPLC) chromatogram of the antibody conjugate T-MDP3.
Figure 7 shows the size exclusion chromatography (SEC-HPLC) chromatogram of the antibody conjugate T-MDP4.
Figure 8 shows the hydrophobic interaction chromatography (HIC-HPLC) chromatogram of the antibody conjugate T-MDP4.
Figure 9 shows the size exclusion chromatography (SEC-HPLC) chromatogram of the antibody conjugate T-MDP5.
Figure 10 shows the hydrophobic interaction chromatography (HIC-HPLC) chromatogram of the antibody conjugate T-MDP5.
Figure 11 shows the size exclusion chromatography (SEC-HPLC) chromatogram of the antibody conjugate T-MDP6.
Figure 12 shows the hydrophobic interaction chromatography (HIC-HPLC) chromatogram of the antibody conjugate T-MDP6.
Figure 13 shows the size exclusion chromatography (SEC-HPLC) chromatogram of the antibody conjugate T-MDP7.
Figure 14 shows the hydrophobic interaction chromatography (HIC-HPLC) chromatogram of the antibody conjugate T-MDP7.
Figure 15 shows the size exclusion chromatography (SEC-HPLC) chromatogram of the antibody conjugate T-MDP8.
Figure 16 shows the hydrophobic interaction chromatography (HIC-HPLC) chromatogram of the antibody conjugate T-MDP8.
Figure 17 shows the size exclusion chromatography (SEC-HPLC) chromatogram of the antibody conjugate T-MDP9.
Figure 18 shows the hydrophobic interaction chromatography (HIC-HPLC) chromatogram of the antibody conjugate T-MDP9.
Figure 19 shows the size exclusion chromatography (SEC-HPLC) chromatogram of the antibody conjugate T-MDP10.
Figure 20 shows the hydrophobic interaction chromatography (HIC-HPLC) chromatogram of the antibody conjugate T-MDP10.
Figure 21 shows the size exclusion chromatography (SEC-HPLC) chromatogram of the antibody conjugate T-MDP11.
Figure 22 shows the hydrophobic interaction chromatography (HIC-HPLC) chromatogram of the antibody conjugate T-MDP11.
Figure 23 shows the size exclusion chromatography (SEC-HPLC) chromatogram of the antibody conjugate T-MDP12.
Figure 24 shows the hydrophobic interaction chromatography (HIC-HPLC) chromatogram of the antibody conjugate T-MDP12.
Figure 25 shows the size exclusion chromatography (SEC-HPLC) chromatogram of the antibody conjugate T-MDP13.
Figure 26 shows the hydrophobic interaction chromatography (HIC-HPLC) chromatogram of the antibody conjugate T-MDP13.
Figure 27 shows the size exclusion chromatography (SEC-HPLC) chromatogram of the antibody conjugate T-MDP17.
Figure 28 shows the hydrophobic interaction chromatography (HIC-HPLC) chromatogram of the antibody conjugate T-MDP17.
Figure 29 shows the size exclusion chromatography (SEC-HPLC) chromatogram of the antibody conjugate T-MD1.
Figure 30 shows the hydrophobic interaction chromatography (HIC-HPLC) chromatogram of the antibody conjugate T-MD1.
Figure 31 shows the curve of human breast cancer HCC1954 tumor volume over time after administration of antibody conjugates MTX-1000, T-MDP2, T-MDP3, and MTX-1000 in combination with small molecule Rucaparib prepared in the examples of the present disclosure.
Figure 32 shows the tumor growth inhibition curve of colon cancer COLO205 after administration of antibody conjugates T-T1000e (MTX-1000), T-MDP2, T-MDP17, and T-T1000e in combination with T-MCD1 (T-Rucaparib ADC) prepared in the examples of the present disclosure.
Figure 33 shows the tumor growth inhibition curve of colon cancer COLO205 after administration of antibody conjugates T-MDP3, T-MDP7, T-MDP10, T-MDP11, T-MDP12, and huIgG-MDP2 prepared in the examples of the present disclosure.
Figure 34 shows the tumor growth inhibition curve of colon cancer COLO205 after administration of antibody conjugates RS7-MDP2, T-MDP15, T-MDP16, T-MDP27 prepared in the examples of the present disclosure.
Figure 35 shows the tumor growth inhibition curve of human colorectal adenocarcinoma SW837 after administration of antibody conjugates U3-1407, P-MDP2, P-MDP3, P-MDP12, P-MDP15, P-MDP22, P-MDP27, P-T1000e prepared in the examples of the present disclosure.
Figure 36 shows the tumor growth inhibition curve of human breast cancer MDA-MB-436 after administration of antibody conjugates DS5573a-MCD1, DS5573a-MDP2, DS5573a-MDP6 prepared in the examples of the present disclosure.
Figure 37 shows the tumor growth inhibition curve of human pancreatic cancer BXPC-3 after administration of antibody conjugate Tisotumab-TM5-2 prepared in the examples of the present disclosure.
Figure 38 shows that the DAR value of antibody conjugate T-MDP2 detected by LCMS is 8.05.
Figure 39 shows that the DAR value of antibody conjugate T-MDP12 detected by LCMS is 7.90.
Figure 40 shows a schematic diagram of antibody conjugation reaction steps according to some embodiments of the present invention.
Figure 41 shows a schematic diagram of step A for engineering a thiomab antibody according to some embodiments of the present invention.
Figure 42 shows a schematic diagram of step B for engineering a thiomab antibody according to some embodiments of the present invention.
Figure 43 shows the *in vivo* anti-tumor activity of antibody-drug conjugates RS7-A132, RS7-A46, RS7-A149 against the human pancreatic cancer cell line BXPC-3.
Figure 44 shows the *in vivo* anti-tumor activity of antibody-drug conjugates against human breast cancer T-47D.
Figure 45 shows the size exclusion chromatography (SEC-HPLC) chromatogram of the antibody conjugate T-MDP54.
Figure 46 shows the mass spectrum of the antibody conjugate T-MDP54.
Figure 47 shows the size exclusion chromatography (SEC-HPLC) chromatogram of the antibody conjugate RS7-MDP12.
Figure 48 shows the mass spectrum of the antibody conjugate RS7-MDP12.
Figure 49 shows the size exclusion chromatography (SEC-HPLC) chromatogram of the antibody conjugate RS7-MDP44.
Figure 50 shows the mass spectrum of the antibody conjugate RS7-MDP44.
Figure 51 shows the size exclusion chromatography (SEC-HPLC) chromatogram of the antibody conjugate RS7-MDP47.
Figure 52 shows the mass spectrum of the antibody conjugate RS7-MDP47.
Figure 53 shows the size exclusion chromatography (SEC-HPLC) chromatogram of the antibody conjugate thioRS7-T1000e(DAR2)-TM5-21(DAR8).
Figure 54 shows the mass spectrum of the antibody conjugate thioRS7-T1000e(DAR2)-TM5-21(DAR8).
Figure 55 shows the size exclusion chromatography (SEC-HPLC) chromatogram of the antibody conjugate thioRS7-T1000e(DAR2)-TM5-22(DAR8).
Figure 56 shows the mass spectrum of the antibody conjugate thioRS7-T1000e(DAR2)-TM5-22(DAR8).
Figure 57 shows the size exclusion chromatography (SEC-HPLC) chromatogram of the antibody conjugate thioRS7-MDP2(DAR2)-TM5-21(DAR8).
Figure 58 shows the mass spectrum of the antibody conjugate thioRS7-MDP2(DAR2)-TM5-21(DAR8).
Figure 59 shows the changes in aggregates of some antibody conjugates after 3 freeze-thaw cycles.
Figure 60 shows the changes in aggregates of some antibody conjugates after accelerated stability testing.

### Detailed Description of the Invention

### Definitions

In the present invention, the term "alkyl" generally refers to a residue derived by removing one hydrogen atom from an alkane. Alkyl can be substituted or unsubstituted, replaced or unreplaced. The term "alkyl" generally refers to a saturated straight or branched aliphatic hydrocarbon group derived by removing a hydrogen atom from the same carbon atom or two different carbon atoms of the parent alkane, which can be a straight or branched group containing 1 to 20 carbon atoms, for example, containing 1 to 12 carbon atoms, such as a chain alkyl containing 1 to 6 carbon atoms. Non-limiting examples of alkyl include, but are not limited to, methyl, ethyl, propyl, isopropyl, butyl, etc. Alkyl can be substituted or unsubstituted, replaced or unreplaced. When substituted, the substituent(s) can be present at any available point of attachment, and said substituent(s) can independently and optionally be selected from one or more substituents including alkyl, alkenyl, alkynyl, alkoxy, alkylthio, alkylamino, halogen, mercapto, hydroxyl, nitro, cyano, cycloalkyl, heterocyclyl, aryl, heteroaryl, cycloalkyloxy, heterocyclyloxy, cycloalkylthio, heterocyclylthio, and oxo. For example, it can be hydrogen, protium, deuterium, tritium, halogen, NO₂, -CN, -OH, -SH, -NH₂, -C(O)H, -CO₂H, -C(O)C(O)H, -C(O)CH₂C(O)H, -S(O)H, -S(O)₂H, -C(O)NH₂, -SO₂NH₂, -OC(O)H, - N(H)SO₂H or C₁₋₆ aliphatic group.

In the present invention, the term "alkylene" generally refers to a saturated straight or branched aliphatic hydrocarbon group derived by removing two hydrogen atoms from the same carbon atom or two different carbon atoms of the parent alkane, which can be a straight or branched group containing 1 to 20 carbon atoms. For example, the term "methylene" can refer to a residue derived by removing two hydrogen atoms from a one-carbon atom group. Alkylene can be substituted or unsubstituted, replaced or unreplaced. For example, it can be an alkylene containing 1 to 12 carbon atoms, such as an alkylene containing 1 to 6 carbon atoms. Non-limiting examples of alkylene include, but are not limited to, methylene (-CH₂-), 1,1-ethylene (-CH(CH₃)-), 1,2-ethylene (-CH₂CH₂)-, 1,1-propylene (-CH(CH₂CH₃)-), 1,2-propylene (-CH₂CH(CH₃)-), 1,3-propylene (-CH₂CH₂CH₂-), 1,4-butylene (-CH₂CH₂CH₂CH₂-), and 1,5-pentylene (-CH₂CH₂CH₂CH₂CH₂-), etc. Alkylene can be substituted or unsubstituted, replaced or unreplaced. When substituted, the substituent(s) can be present at any available point of attachment, and said substituent(s) can independently and optionally be selected from one or more substituents including alkyl, alkenyl, alkynyl, alkoxy, alkylthio, alkylamino, halogen, mercapto, hydroxyl, nitro, cyano, cycloalkyl, heterocyclyl, aryl, heteroaryl, cycloalkyloxy, heterocyclyloxy, cycloalkylthio, heterocyclylthio, and oxo. For example, it can be hydrogen, protium, deuterium, tritium, halogen, NO₂, -CN, -OH, -SH, -NH₂, -C(O)H, -CO₂H, - C(O)C(O)H, -C(O)CH₂C(O)H, -S(O)H, -S(O)₂H, -C(O)NH₂, -SO₂NH₂, -OC(O)H, -N(H)SO₂H or C₁₋₆ aliphatic group. Methylene or alkylene can be substituted or unsubstituted.

In the present invention, the term "alkenyl" generally refers to a straight or branched hydrocarbon group containing one or more double bonds. Exemplary examples of alkenyl include allyl, homoallyl, vinyl, crotyl, butenyl, pentenyl, and hexenyl, etc. Exemplary examples of C₂₋₆ alkenyl with more than one double bond include butadienyl, pentadienyl, hexadienyl, hexatrienyl, and their branched forms. The position of the unsaturated bond (double bond) can be at any position in the carbon chain. Alkenyl can be substituted or unsubstituted.

In the present invention, the term "alkenylene" generally refers to a residue derived by removing two hydrogen atoms from a carbon atom of an alkene. For example, it can be allylene, vinylene, butenylene, pentenylene, hexenylene, etc. Alkenylene can be substituted or unsubstituted.

In the present invention, the term "alkynyl" generally refers to an unsaturated straight or branched alkyne group, such as ethynyl, 1-propynyl, propargyl, butynyl, etc. Alkynyl can be substituted or unsubstituted.

In the present invention, the term "alkynylene" generally refers to a residue derived by removing two hydrogen atoms from a carbon atom of an alkyne. For example, it can be ethynylene, propynylene, propargylene, butynylene, etc. Alkynylene can be substituted or unsubstituted.

In the present invention, the term "aryl" generally refers to a residue derived by removing one hydrogen atom from an aromatic ring. The term "aromatic ring" can refer to a 6- to 14-membered all-carbon monocyclic or fused polycyclic ring (i.e., rings sharing adjacent pairs of carbon atoms) with a conjugated π-electron system, which can be 6- to 10-membered, such as benzene and naphthalene. The aromatic ring can be fused to a heteroaryl, heterocyclyl, or cycloalkyl ring, where the ring attached to the parent structure is the aryl ring. Aryl can be substituted or unsubstituted. When substituted, the substituent(s) can be one or more groups independently selected from the group consisting of: alkyl, alkenyl, alkynyl, alkoxy, alkylthio, alkylamino, halogen, mercapto, hydroxyl, nitro, cyano, cycloalkyl, heterocycloalkyl, aryl, heteroaryl, cycloalkyloxy, heterocycloalkyloxy, cycloalkylthio, and heterocycloalkylthio. Aryl can be substituted or unsubstituted.

In the present invention, the term "arylene" generally refers to a residue derived by removing two hydrogen atoms from a carbon atom of an aromatic ring. For example, it can be phenylene and naphthylene. Arylene can be substituted or unsubstituted.

In the present invention, the term "heteroaryl" generally refers to a residue derived by removing one hydrogen atom from a carbon atom of a heteroaromatic ring. The term "heteroaromatic ring" refers to a heteroaromatic system containing 1 to 4 heteroatoms and 5 to 14 ring atoms, wherein the heteroatoms can be selected from the group consisting of: oxygen, sulfur, and nitrogen. Heteroaryl can be 5- to 10-membered, and can be 5-membered or 6-membered, such as furyl, thienyl, pyridyl, pyrrolyl, N-alkylpyrrolyl, pyrimidinyl, pyrazinyl, imidazolyl, tetrazolyl, etc. The heteroaryl ring can be fused to an aryl, heterocyclyl, or cycloalkyl ring, where the ring attached to the parent structure is the heteroaryl ring. Heteroaryl can be optionally substituted or unsubstituted. When substituted, the substituent(s) can be one or more groups independently selected from the group consisting of: alkyl, alkenyl, alkynyl, alkoxy, alkylthio, alkylamino, halogen, mercapto, hydroxyl, nitro, cyano, cycloalkyl, heterocycloalkyl, aryl, heteroaryl, cycloalkyloxy, heterocycloalkyloxy, cycloalkylthio, and heterocycloalkylthio. Heteroaryl can be substituted or unsubstituted.

In the present invention, the term "heteroarylene" generally refers to a residue derived by removing two hydrogen atoms from a carbon atom of a heteroaromatic ring. For example, it can be furylene, thienylene, pyridylene, pyrrolylene, pyrimidinylene, pyrazinylene, imidazolylene, tetrazolylene, etc. Heteroarylene can be substituted or unsubstituted.

In the present invention, the term "alicyclyl" generally refers to a residue derived by removing one or more hydrogen atoms from the same or different carbon atoms of an aliphatic ring. The term "cycloalkane" generally refers to saturated or partially unsaturated monocyclic or polycyclic hydrocarbons containing 3 to 20 carbon atoms, which can contain 3 to 12 carbon atoms, 3 to 10 carbon atoms, or 3 to 8 carbon atoms. Non-limiting examples of alicyclyl include cyclopropyl, cyclobutyl, cyclopentyl, cyclopentenyl, cyclohexyl, cyclohexenyl, cyclohexadienyl, cycloheptyl, cycloheptatrienyl, cyclooctyl, etc.; polycyclic carbocycles can include spiro, fused, and bridged carbocycles. Alicyclyl can be substituted or unsubstituted. In the present invention, the term "carbocyclyl" generally refers to a residue derived by removing one hydrogen atom from a carbon atom of a carbocycle. The term "carbocycle" generally refers to saturated or partially unsaturated monocyclic or polycyclic hydrocarbons containing 3 to 20 carbon atoms, which can contain 3 to 12 carbon atoms, 3 to 10 carbon atoms, or 3 to 8 carbon atoms. Non-limiting examples of monocyclic carbocycles include cyclopropane, cyclobutane, cyclopentane, cyclopentene, cyclohexane, cyclohexene, cyclohexadiene, cycloheptane, cycloheptatriene, cyclooctane, etc.; polycyclic carbocycles can include spiro, fused, and bridged carbocycles. Carbocyclyl can be substituted or unsubstituted. In certain contexts, alicyclic and carbocyclic may be used interchangeably.

In the present invention, the term "partially unsaturated" generally refers to a cyclic structure containing at least one double or triple bond between ring molecules. The term "partially unsaturated" encompasses cyclic structures with multiple unsaturations but is not intended to include aromatic or heteroaromatic rings as defined in the present invention. The term "unsaturated" indicates that the moiety has one or more degrees of unsaturation.

In the present invention, the term "alicyclylene" generally refers to a residue derived by removing two hydrogen atoms from a carbon atom of an alicyclic ring. For example, it can be cyclopropylene, cyclobutylene, cyclopentylene, cyclopentenylene, cyclohexylene, cyclohexenylene, cyclohexadienylene, cycloheptylene, cycloheptatrienylene, cyclooctylene, etc.; polycyclic carbocycles can include spiro, fused, and bridged carbocycles. Alicyclylene can be substituted or unsubstituted.

In the present invention, the term "aliphatic heterocyclyl" generally refers to a stable, nonaromatic 3- to 7-membered monocyclic carbocyclic structure, fused 7- to 10-membered bicyclic heterocyclic structure, or bridged 6- to 10-membered bicyclic heterocyclic structure. These cyclic structures can be saturated or partially saturated and contain, in addition to carbon atoms, one or more heteroatoms selected from the group consisting of: oxygen, sulfur, and nitrogen. For example, they can contain 1-4 heteroatoms as defined above. When referring to an atom in an aliphatic heterocyclic structure, the term "nitrogen" can include substituted nitrogen. For example, aliphatic heterocyclyl can include "heterocycloalkyl", which can refer to a stable, nonaromatic 3- to 7-membered monocyclic alkyl structure, fused 7- to 10-membered bicyclic heterocyclic structure, or bridged 6- to 10-membered bicyclic heterocyclic structure containing, in addition to carbon atoms, one or more heteroatoms selected from the group consisting of: oxygen, sulfur, and nitrogen. For example, they can contain 1-4 heteroatoms as defined above. Heterocycloalkyl can be substituted or unsubstituted. Aliphatic heterocyclyl can be substituted or unsubstituted.

In the present invention, the term "aliphatic heterocyclylene" generally refers to a residue derived by removing two hydrogen atoms from a carbon atom of an aliphatic heterocyclic ring. Aliphatic heterocyclylene can be substituted or unsubstituted.

In the present invention, the terms "optional" or "optionally" generally mean that the subsequently described event or circumstance may but need not occur, and the description includes instances where the event or circumstance occurs and instances where it does not. For example, "a heterocyclic group optionally substituted with alkyl" means that the alkyl may but need not be present; this description includes both situations where the heterocyclic group is substituted with alkyl and where it is not.

In the present invention, the term "substituted" generally means that one or more hydrogen atoms in a group, for example, up to 5, such as 1 to 3 hydrogen atoms, are each independently replaced by a corresponding number of substituents. Substituents are only at their possible chemical positions, and those skilled in the art can determine (through experiment or theory) possible or impossible substitutions without undue effort. For example, amino or hydroxyl groups with free hydrogen may be unstable when attached to a carbon atom with an unsaturated (e.g., olefinic) bond.

In the present invention, as known to those skilled in the art, terms like "alkyl", "alkenyl", "cycloalkyl", etc., can be preceded by an identifier to indicate the number of atoms present in the group under specific circumstances, for example, C₁-C₄ alkyl, C₃-C₇ cycloalkyloxy, C₁-C₄ alkylcarbonylamino, etc., where the subscript number after "C" indicates the number of carbon atoms present in the group. For example, C₃ alkyl refers to an alkyl group with three carbon atoms (e.g., n-propyl, isopropyl); in C₁₋₁₀, members of the group can have any number of carbon atoms falling within the range of 1-10.

One or more hydrogen atoms in a group, for example, up to 5, such as 1 to 3 hydrogen atoms, are each independently replaced by a corresponding number of substituents. Substituents are only at their possible chemical positions, and those skilled in the art can determine (through experiment or theory) possible or impossible substitutions without undue effort. For example, amino or hydroxyl groups with free hydrogen may be unstable when attached to a carbon atom with an unsaturated (e.g., olefinic) bond.

In the present invention, the term "compound" generally refers to a substance composed of two or more different elements. For example, the compounds of the present invention can be organic compounds. For example, the compounds of the present invention can be compounds with a molecular weight below 500, can be compounds with a molecular weight below 1000, can also be compounds with a molecular weight above 1000, can also be compounds with a molecular weight above 10,000, or above 100,000. In the present invention, compounds can also refer to compounds linked by chemical bonds, for example, compounds where one or more molecules with a molecular weight below 1000 are linked to biomacromolecules via chemical bonds, said biomacromolecules being polysaccharides, proteins, nucleic acids, polypeptides, etc. For example, compounds of the present invention can include compounds where a protein is linked to one or more molecules with a molecular weight below 1000, can be compounds where a protein is linked to one or more molecules with a molecular weight below 10,000, or can be compounds where a protein is linked to one or more molecules with a molecular weight below 100,000.

In the present invention, the term "comprising" generally means including the explicitly specified features, but does not exclude other elements. The terms "above" and "below" generally include the stated number itself.

In the present invention, the term "about" generally refers to a variation of 0.5%-10% above or below the stated value, for example, within 0.5%, 1%, 1.5%, 2%, 2.5%, 3%, 3.5%, 4%, 4.5%, 5%, 5.5%, 6%, 6.5%, 7%, 7.5%, 8%, 8.5%, 9%, 9.5%, or 10% above or below the stated value.

In the present invention, the compounds of the present invention include tautomers, mesomers, racemates, enantiomers, and/or diastereomers of the compounds. In the present invention, the term "diastereomer" generally refers to stereoisomers that have two or more chiral centers and whose molecules are not mirror images of each other. Diastereomers can have different physical properties, such as melting point, boiling point, spectral properties, and reactivity. In the present invention, the terms "tautomer" and "tautomeric form" are used interchangeably and generally refer to structural isomers of differing energy that can interconvert via a low-energy barrier. For example, proton tautomers (also known as prototropic tautomers) include interconversions via proton migration, such as keto-enol and imine-enamine isomerization. Valence tautomers include interconversions via reorganization of some bonding electrons. In the present invention, the term "mesomer" generally refers to a molecule containing asymmetric atoms but having symmetry elements that result in a total optical rotation of zero within the molecule. The term "racemate" or "racemic mixture" refers to a composition consisting of equimolar amounts of two enantiomeric substances.

In the present invention, certain atoms of the compounds of the present invention may occur in more than one isotopic form. For example, hydrogen may exist as protium (¹H), deuterium (²H), and tritium (³H), and carbon naturally occurs in three different isotopes (¹²C, ¹³C, and ¹⁴C). Isotopes that can be incorporated into the compounds of the present invention include, but are not limited to, ¹⁵N, ¹⁸O, ¹⁷O, ¹⁸F, ³²P, ³³P, ¹²⁹I, ¹³¹I, ¹²³I, ¹²⁴I, ¹²⁵I, or similar isotopes. Therefore, relative to the natural abundance of these isotopes, the compounds of the present invention can be enriched in one or more of these isotopes. As known to those skilled in the art, such isotopically enriched compounds can be used for various purposes. For example, substitution with heavy isotopes such as deuterium (²H) may provide certain therapeutic advantages, possibly due to higher metabolic stability. For example, the natural abundance of deuterium (²H) is about 0.015%. Therefore, for about every 6500 hydrogen atoms in nature, there is one deuterium atom. Thus, the deuterium-containing compounds of the present invention have a deuterium abundance greater than 0.015% at one or more positions (as appropriate). Unless otherwise indicated, the structures described in the present invention also encompass compounds that differ only in the presence or absence of one or more isotopically enriched atoms. For example, compounds where hydrogen atoms are replaced by deuterium or tritium, or carbon atoms are replaced by carbon-13 or carbon-14, while the rest of the structure is consistent with the present invention, are all within the scope of the present invention.

In the present invention, the term "analog" generally refers to a compound that has the same or similar structure or function as the said inhibitor and is capable of inhibiting the activity of said enzyme by at least 50%. The analog can be an isomer, isomer, derivative, metabolite, precursor, or resolved product of said inhibitor, or a compound obtained by adding, deleting, replacing, or altering one or more atoms, chemical bonds, functional groups, substituents, or stereochemistry in the molecular structure of said inhibitor. The analog may have the same or different pharmacological properties as the inhibitor, such as solubility, stability, bioavailability, half-life, toxicity, or side effects.

In the present invention, the term "pharmaceutical composition" generally refers to a mixture containing one or more compounds of the present invention or their physiologically/pharmaceutically acceptable salts or prodrugs, along with other chemical components such as physiologically/pharmaceutically acceptable carriers and excipients. The pharmaceutical composition can facilitate administration to an organism, aid in the absorption of the active ingredient, and thus exert biological activity. Conventional preparation of pharmaceutical compositions can be found in commonly used techniques in the field.

In the present invention, the term "pharmaceutically acceptable salt" generally refers to a salt of a compound or ligand-drug conjugate of the present invention, or a salt of a compound described herein, which can be safe and/or effective when administered to a mammal and can possess the intended biological activity. The antibody-drug conjugate compounds of the present invention can form salts with acids. Non-limiting examples of pharmaceutically acceptable salts include: hydrochloride, hydrobromide, hydroiodide, sulfate, bisulfate, citrate, acetate, succinate, ascorbate, oxalate, nitrate, sorbate, phosphate, hydrogen phosphate, dihydrogen phosphate, salicylate, hydrogen citrate, tartrate, maleate, fumarate, formate, benzoate, methanesulfonate, ethanesulfonate, benzenesulfonate, or p-toluenesulfonate.

In the present invention, the term "conjugate" generally refers to a compound prepared through one or more chemical reactions of the compounds of the present invention, or compounds connected to each other via one or more linking structures such as bridges, spacers, or linkers.

In the present invention, the term "pharmaceutically acceptable carrier" generally refers to a carrier for administering a therapeutic agent, such as an antibody or polypeptide, gene, and other therapeutic agents. The term refers to any pharmaceutical carrier that itself does not induce the production of antibodies harmful to the individual receiving the composition and can be administered without causing excessive toxicity. For example, a pharmaceutically acceptable carrier can be distinguished from a nucleic acid vector used in genetic engineering to contain a target gene. Suitable carriers can be large, slowly metabolized macromolecules such as proteins, polysaccharides, polylactic acid, polyglycolic acid, polyamino acids, amino acid copolymers, lipid aggregates, and inactivated virus particles. These carriers are well known to those skilled in the art. Pharmaceutically acceptable carriers in therapeutic compositions can include liquids such as water, saline, glycerol, and ethanol. Auxiliary substances such as wetting agents or emulsifiers, pH buffering agents, etc., can also be present in these carriers.

In the present invention, the term "group capable of conjugating with a sulfhydryl group" generally means that compound A has a sulfhydryl group, compound B has a group capable of conjugating with a sulfhydryl group, and compound B reacts with the sulfhydryl group of compound A via the group capable of conjugating with a sulfhydryl group, thereby achieving the connection between compound A and compound B.

In the present invention, the term "linker" generally refers to a chemical structure, fragment, or bond that connects one group at one end and another group at the other end, or it may connect to other linkers before connecting to a drug and/or ligand. The direct or indirect connection to a ligand can mean that said group is directly connected to the ligand via a covalent bond or connected to the ligand through a linker. For example, the linker can be the structure shown as Q₁ in the present invention. For example, chemical structure fragments or bonds containing acid-labile linker structures (e.g., hydrazone), protease-sensitive (e.g., peptidase-sensitive) linker structures, photolabile linker structures, dimethyl linker structures, or disulfide-containing linker structures can be used as linkers.

In the present invention, the term "linking group" generally refers to a group with the ability to connect to another group. For example, a compound having a linking group can be connected to another group through a conjugation reaction involving this linking group, thereby linking the compound to the other group. For example, a maleimide group can serve as a linking group.

In the present invention, the term "drug unit" generally refers to a chemical moiety directly or indirectly conjugated to an antibody or antigen-binding fragment to form an immunoconjugate. For example, "drug unit" includes but is not limited to compounds with anti-tumor activity described herein. For example, drug units include topoisomerase inhibitors.

In the present invention, the term "compound with anti-tumor activity" generally refers to a compound capable of reducing the proliferation rate, viability, or metastatic activity of tumor cells. For example, anti-tumor activity can be indicated by a reduction in the growth rate of abnormal cells during treatment, stabilization or reduction in tumor size, or longer survival due to treatment compared to untreated controls. Anti-tumor activity can be evaluated using recognized in vitro or in vivo tumor models, such as xenograft models.

In some embodiments of the present invention, the bioactive molecule in the conjugate is a compound with anti-tumor activity, specifically, for example: radioisotopes such as At²¹¹, I¹³¹, I¹²⁵, Y⁹⁰, Re¹⁸⁶, Re¹⁸⁸, Sm¹⁵³, Bi²¹², P³², Pb²¹², or radioactive isotopes of Lu; metal complexes such as platinum metal complexes (e.g., oxaliplatin) or gold metal complexes; glycopeptide antibiotics such as bleomycin or pingyangmycin; topoisomerase inhibitors; drugs that interfere with DNA synthesis, such as methotrexate, 5-fluorouracil, cytarabine, gemcitabine, mercaptopurine, pentostatin, fludarabine, cladribine, or nelarabine; drugs acting on structural proteins, such as tubulin inhibitors (e.g., vinca alkaloids, vincristine, vinblastine, taxanes, maytansinoids, auristatins, Tubulysin B, or eribulin); tumor signaling pathway inhibitors, such as serine/threonine kinase inhibitors, tyrosine kinase inhibitors, aspartate kinase inhibitors, or histidine kinase inhibitors; proteasome inhibitors; epigenetics-related target inhibitors; tumor angiogenesis inhibitors; cell cycle protein inhibitors.

In the present invention, the term "topoisomerase inhibitor" or "drug targeting topoisomerase" generally refers to compounds or their derivatives that include topoisomerase I inhibitors and topoisomerase II inhibitors. Examples of topoisomerase I inhibitors include but are not limited to camptothecin and its analogs; Topoisomerase II inhibitors (such as Voreloxin (CAS 175414-77-4) or its derivatives, actinomycin D, doxorubicin, daunorubicin, duocarmycin, mitoxantrone, podophyllotoxin, or etoposide). Topoisomerase refers to enzymes that correct the linking number of DNA by breaking phosphodiester bonds in one or both strands of DNA, then rewinding and resealing.

In the present invention, the term "camptothecin analog" generally refers to compounds structurally similar to or derived from camptothecin. For example, camptothecin analogs can refer to Exatecan (CAS 171335-80-1), DXd (CAS 1599440-33-1), 7-ethyl-10-hydroxycamptothecin (SN38, CAS 86639-52-3), Belotecan (CAS 213819-48-8), (4-NH₂)-Exatecan (AZD'0132, CAS 2495742-21-5), 7-MAD-MDCPT (CAS 765871-81-6), 7-aminomethyl-10-methyl-11-fluorocamptothecin (CAS 2378616-23-8), etc.

In the present invention, the term "PARP inhibitor", short for poly ADP ribose polymerase inhibitor, refers to a new class of molecular targeted therapeutic drugs that inhibit poly ADP ribose polymerase (PARP). For example, PARP inhibitors can refer to olaparib, Niraparib, Rucaparib (Rubraca), Talazoparib (Talzenna), fluzoparib, pamiparib, A-966492, AZD5305, Venadaparib, Mefuparib (CAS 1449746-00-2), etc.

In the present invention, the term "ATR" refers to Ataxia Telangiectasia mutated gene Rad3-related kinase, a key enzyme in the DNA damage homologous recombination repair pathway, belonging to the PIKK family. For example, ATR inhibitors can be Elimusertib/BAY1895344, Berzosertib/VE-822, Ceralasertib/AZD6738, M4344, RP-3500, etc.

In the present invention, the term "CHK1" is checkpoint kinase 1, a member of the serine/threonine protein kinase family, and a core protein in the cell cycle checkpoint during DNA damage response. Studies have shown that CHK1 protein kinase promotes tumor cell proliferation, and its deficiency can make tumor cells more sensitive to radiotherapy or chemotherapy, and its inhibitors have a "synthetic lethal" effect when combined with other molecular targeted drugs. For example, CHK1 inhibitors can be Prexasertib (LY2606368), AZD7762, Rabusertib (LY2603618), MK-8776 (SCH 900776), CHIR-124, PF-477736, CCT245737 (SRA737, PNT-737), GDC-0575 (ARRY-575), SAR-020106, LY2880070, AZD7648, etc.

In the present invention, the term "ATM" is Ataxia Telangiectasia Mutation, a serine/threonine protein. For example, ATM inhibitors can be AZD-1390, M-4076, XRD-0394, etc.

In the present invention, the term "DNA-PK" refers to DNA-dependent protein kinase (DNA-PK) catalytic subunit, belonging to the phosphoinositide-3-kinase-related kinase (PIKK) family, and is a serine/threonine protein kinase. For example, DNA-PK inhibitors can be wortmannin, AZD7648, LY294002, NU7026, NU7441, VX-984, M3814, CC-115, etc.

In the present invention, the term "WEE1" is an important member of the serine/threonine protein kinase family, a cell cycle regulatory protein, a key kinase in the DNA damage repair (DDR) pathway, involved in cell cycle regulation, playing a crucial role in the G2/M checkpoint. For example, WEE1 inhibitors can be ZN-C3, Adavosertib (AZD1775), Debio 0123, etc.

In the present invention, the term "POLQ" is an error-prone translocase polymerase, also involved in DNA double-strand break repair, often upregulated in cancer states. For example, POLQ inhibitors can be ART4215, RP-6685, etc.

In the present invention, the term "PKMYT1" is a regulator of CDK1 phosphorylation, establishing a synthetic lethal relationship with CCNE1 amplification, making it a compelling therapeutic target for certain types of DNA damage response cancers. For example, PKMYT1 inhibitors can be RP-6306, etc.

In the present invention, the term "Rad51" encodes a 339-amino acid polypeptide with a relative molecular mass of 4.3×10⁴, having five homologs: XRCC2, XRCC3, RAD51B (RAD51L1), RAD51C (RAD51L2), RAD51D (RAD51L3). These, together with RAD51, participate in the HR process. Tumors overexpressing RAD51 often exhibit treatment resistance and lower overall patient survival. For example, Rad51 inhibitors can be CYT-0851, etc.

In the present invention, the term "Bcl-2 family protein" is a family of evolutionarily related proteins that can receive and transmit intracellular signals or external environmental stress signals, such as nutrient or hypoxia stress, DNA damage, oncogene overactivation, endoplasmic reticulum stress, etc. The Bcl-2 protein family plays a key role in tumorigenesis and metastasis. Bcl-2 family proteins are mainly divided into three categories: anti-apoptotic protein subfamily, containing BH1, BH2, BH3, BH4 four functional domains, including Bcl-2, Bcl-xL, Mcl-1, etc.; pro-apoptotic protein subfamily, containing BH1, BH2, BH3, BH4 four functional domains, including Bax, Bak, and Bok, etc.; another class is the BH3-only pro-apoptotic protein subfamily containing only the BH3 domain, including Bim, Bid, Puma, Noxa, and Bad, etc. For example, Bcl-2 inhibitors can be Venetoclax (CAS 1257044-40-8), Navitoclax (CAS 923564-51-6), ABT-737 (CAS 852808-04-9), A-1331852 (1430844-80-6), or analogs thereof.

In the present invention, the term "LSD1" is also known as KDM1A/AOF2, the first discovered histone-specific demethylase. LSD1 catalyzes flavin adenine dinucleotide (FAD) and belongs to the monoamine oxidase family. For example, LSD1 inhibitors can be Tranylcypromine, ORY-1001 (Iadademstat, CAS 1431303-72-8), CC-90011 (Pulrodemstat, CAS 1821307-10-1), ORY-2001, GSK-2879552, IMG-7289, INCB059872, or TAK-418, etc.

In the present invention, the term "EZH2 inhibitor" generally refers to a histone methyltransferase and the catalytic subunit of PRC2, catalyzing mono-, di-, and trimethylation of lysine 27 of histone H3 (H3K27me3). EZH2 inhibitors can promote antigen presentation ability in the tumor microenvironment, activating anti-tumor adaptive immune responses. For example, EZH2 inhibitors can be Tazemetostat (Epizyme), GSK2816126 (GlaxoSmithKline), CPI-1205 (Constellation Pharmaceuticals), PF-06821497 (Pfizer), SHR2554 (Hengrui Medicine), XNW5004 (SinoV), HH2853 (Shanghai Haihe Pharmaceutical), etc.

In the present invention, the term "PRMT5" involves arginine methylation, which is one of histone methylation, and is one of the most common post-translational modifications in mammals, primarily regulated by the PRMT gene family. PRMT5 inhibitors can be GSK3326595, AMG 193, MRTX1719, SKL27969, TNG908, SCR-6920, SH3765, SYHX2001, etc.

In the present invention, the term "TLR7/8" refers to an endosomal receptor that recognizes single-stranded RNAs (ssRNAs) with viral characteristics, such as abundant GU dinucleotide motifs. TLR7 or TLR8 agonists can be 1V209 derivatives, Resiquimod, etc.

In the present invention, the term "STING" is the stimulator of interferon genes, initiating immune responses. Many studies have shown that STING is involved in the occurrence of various diseases; activation of STING can induce effective immune responses against pathogen infections and cancer, while abnormal activation of STING can trigger autoimmune and inflammatory diseases. STING agonists can be cyclic dinucleotides like ADUS100, anthraquinones like DMXAA, aminobenzimidazoles, benzothiophenes like MSA-2, etc.

In the present invention, the term "inhibitor of the Ras-Raf-MAPK pathway" refers to compounds that specifically bind to and inhibit the activity of key protein kinases in the Ras-Raf-MAPK pathway, thereby effectively blocking signal transduction of this pathway. Such inhibitors have high selectivity and affinity, can precisely target key molecules in the Ras-Raf-MAPK pathway, reduce or eliminate their biological activity, and thereby inhibit the proliferation, migration, and invasion of tumor cells. Related targets and corresponding inhibitors include: SOS1 inhibitors BAY-293 (CAS 2244904-70-7), BI-3406 (CAS 2230836-55-0) or analogs thereof; KRAS inhibitors BI-2493 (CAS 2937344-16-4), MRTX1133 (CAS 2621928-55-8) or analogs thereof; MEK inhibitors Cobimetinib (CAS 934660-93-2), Selumetinib (CAS 606143-52-6), Mirdametinib (CAS 391210-10-9), Binimetinib (CAS 606143-89-9), TAK-733 (CAS 1035555-63-5), GDC-0623 (CAS 1168091-68-6), AZD8330 (CAS 869357-68-6), Trametinib (CAS 871700-17-3), Trametiglue (CAS 2666940-97-0) or analogs thereof; ERK inhibitors ASN007 (CAS 2055597-12-9) or analogs thereof, etc.

In the present invention, the term "cell cycle pathway inhibitor", particularly inhibitors targeting CDK kinases (Cyclin-Dependent Kinase Kinases), refers to compounds that specifically bind to and inhibit the activity of different CDK kinases, thereby effectively regulating cell cycle progression. Such inhibitors have high selectivity and specificity, can precisely target CDK kinases, reduce or eliminate their biological activity, and thereby affect various stages of the cell cycle, including DNA replication, chromosome segregation, and cell division. Examples include: CDK4/6 inhibitors Palbociclib (CAS 571190-30-2), Abemaciclib (CAS 1231929-97-7), Ribociclib (CAS 1211441-98-3), Dalpiciclib (CAS 1637781-04-4) or analogs thereof; pan-CDK inhibitors Dinaciclib (CAS 779353-01-4) or analogs thereof, etc.

In the present invention, the term "GSPT1" is a post-transcriptional modification factor that can bind to and stabilize the structure of the MYC protein, thereby enhancing its transcriptional activity. MYC is a transcription factor that regulates the expression of various genes, such as those involved in cell cycle, metabolism, and cell death. However, in many cancers, MYC is overexpressed or amplified, leading to tumorigenesis and progression. Currently, MYC inhibitors have become effective treatments for MYC-driven solid tumors, but issues remain, such as low selectivity, numerous side effects, and easy development of resistance. Therefore, degrading GSPT1 is a strategy to inhibit the MYC signaling pathway.

In the present invention, the term disease "associated with expression" of a certain target generally means that the occurrence and/or progression of the disease is related to the expression level of that target. For example, relative to the expression level in normal cells from a tissue or organ, the expression level of a target in cells from a diseased area, such as a specific tissue or organ of a patient, may be increased, i.e., overexpressed. Or, for example, relative to the expression level in normal cells from a tissue or organ, the expression level of a target in cells from a diseased area, such as a specific tissue or organ of a patient, may be decreased, i.e., underexpressed. Or, for example, cells from a diseased area, such as a specific tissue or organ of a patient, express a target, i.e., are positive. Or, for example, cells from a diseased area, such as a specific tissue or organ of a patient, do not express a target, i.e., are negative. For example, the characteristics of target expression can be determined by standard assays known in the field.

In the present invention, the term "effective amount" generally refers to an amount of a therapeutic agent that treats, alleviates, or prevents a target disease or condition, or exhibits a detectable therapeutic or preventive effect. The precise effective amount for a subject depends on the subject's size and health status, the nature and extent of the condition, and the chosen therapeutic agent and/or combination of therapeutic agents. Therefore, it is not useful to pre-specify an exact effective amount. However, an effective amount for a given condition can be determined by routine experimentation, which a clinician can judge.

Unless otherwise specified, in the present invention, all compounds mentioned are intended to include all possible optical isomers, such as single chiral compounds, or mixtures of various chiral compounds (i.e., racemates). For all compounds of the present invention, each chiral carbon atom may optionally be R configuration or S configuration, or a mixture of R and S configurations.

As used herein, the term "compound of the present invention" refers to any compound provided by the present invention. This term also includes various crystalline forms, pharmaceutically acceptable salts, hydrates, or solvates of the compounds of the present invention.

When a trade name is used herein, the trade name is intended to include the trade name product formulation, its corresponding generic drugs, and the active pharmaceutical ingredients of the trade name product.

As used herein, "antibody" is used in its broadest sense and specifically covers monoclonal antibodies, polyclonal antibodies, dimers, multimers, multispecific antibodies (e.g., bispecific antibodies), and antibody fragments, as long as they exhibit the desired biological activity. Antibodies can be murine, human, humanized, chimeric, or derived from other species. An antibody is a protein produced by the immune system that can recognize and bind to a specific antigen. A target antigen typically has numerous binding sites, also called epitopes, recognized by the CDRs of various antibodies. Antibodies that specifically bind to different epitopes have different structures. Thus, an antigen may have more than one corresponding antibody. Antibodies include full-length immunoglobulin molecules or immunologically active portions of full-length immunoglobulin molecules, i.e., molecules that specifically bind to a target of interest or part thereof, including, but not limited to, cancer cells or cells producing autoantibodies associated with autoimmune diseases. The immunoglobulins described in the present invention can have any type (e.g., IgG, IgE, IgM, IgD, and IgA), class (e.g., IgG1, IgG2, IgG3, IgG4, IgA1, and IgA2), or subclass of immunoglobulin molecules. Immunoglobulins can be derived from any species. However, in one aspect, the immunoglobulin is derived from human, murine, or rabbit. "Antibody fragment" may comprise a portion of a full-length antibody, generally its antigen-binding region or variable region. Examples of antibody fragments include: Fab, Fab', F(ab')₂, and Fv fragments; diabodies; linear antibodies; minibodies; fragments prepared from Fab expression libraries; anti-idiotypic (anti-Id) antibodies; CDRs (complementarity determining regions); and any of the above epitope-binding fragments that bind in an immunologically specific manner to cancer cell antigens, viral antigens, or microbial antigens; single-chain antibody molecules; and multispecific antibodies formed from antibody fragments. The above antibodies can also be modified using THIOMAB technology to modify natural amino acids, enabling site-specific modification of unpaired cysteines to achieve more conjugation sites and site-specific conjugation purposes. The antibodies forming the antibody-drug conjugates of the present invention can retain their original antigen-binding capability in the wild-type state. Therefore, antibodies of the present invention can, for example, specifically bind to antigens. Antigens involved include, for example, tumor-associated antigens (TAAs), cell surface receptor proteins and other cell surface molecules, cell survival regulators, cell proliferation regulators, molecules related to tissue growth and differentiation (such as those known or predicted to be functional), lymphokines, cytokines, molecules involved in cell cycle regulation, molecules involved in angiogenesis, and molecules related to angiogenesis (as known, the antigen bound by an antibody can be one or a subset of the above categories, while other subsets contain other molecules/antigens with specific properties (compared to the target antigen). Antibodies used in antibody-drug conjugates include, but are not limited to, antibodies against cell surface receptors and tumor-associated antigens. Such tumor-associated antigens are well-known in the art, and antibodies can be prepared using well-known methods and information in the art. These targets are specifically expressed on the surface of one or more cancer cell types, while expressed little or not at all on the surface of one or more non-cancerous cells. Typically, such tumor-associated polypeptides may be overexpressed on the surface of cancer cells compared to non-cancerous cell surfaces.

In the present invention, the term "polypeptide residue" generally refers to a residue formed by linking one or more amino acid residues. For example, one or more amino acids in the polypeptide residue can be optionally substituted.

In the present invention, the term "polyethylene glycol group" generally refers to a residue formed by linking one or more ethylene glycol residues. For example, a polyethylene glycol group can comprise -(CH₂CH₂O)ₚ-, where p is a number from 1 to 10. For example, the polyethylene glycol group in the present invention can be optionally substituted.

In the present invention, the term "glycol group" generally refers to a polyethylene glycol group. For example, the glycol group in the present invention can be optionally substituted. For example, a number before a glycol group can indicate the number of ethylene glycol units in the glycol group; for example, a diglycol group can refer to a residue of two ethylene glycol units polymerized.

In the present invention, the term "cyclodextrin" generally refers to cyclic oligomers composed of D(+)-pyranose glucose units: α-, β-, and γ-cyclodextrins consist of 6, 7, and 8 units, respectively.

In the present invention, the term "polysarcosine residue" generally refers to a residue formed by linking one or more sarcosine residues. For example, a polysarcosine residue can comprise -(COCH₂N(CH₃))_{q}-, where q is a number of at least 1. For example, the polysarcosine residue in the present invention can be optionally substituted. For example, a structure containing a polysarcosine residue can be 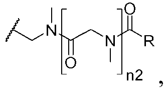 where n2 is a number from 4 to 18, R is alkyl, or alkyl substituted with polyhydroxy, sulfonate, phosphate, carboxyl, or other hydrophilic groups.

In the present invention, the term "sodium dodecyl sulfate polyacrylamide gel electrophoresis" generally refers to a material analysis and characterization technique. For example, sodium dodecyl sulfate polyacrylamide gel electrophoresis (SDS-PAGE) can detect the molecular weight of a substance.

In the present invention, the term "hydrophobic interaction chromatography" generally refers to an analytical technique based on differences in hydrophobicity of substances.

In the present invention, the term "liquid chromatography-mass spectrometry" generally refers to an analytical method for identifying substance components. For example, liquid chromatography-mass spectrometry can analyze the molecular weight of a substance to be tested through combined use of liquid chromatography-mass spectrometry.

In the present invention, the term "tumor" generally refers to any new pathological tissue proliferation. For the present invention, angiogenesis is part of the tumor's characteristics. Tumors can be benign or malignant. The term "tumor" is generally used to refer to benign or malignant tumors, while the term "cancer" is generally used to refer to malignant tumors, which can be metastatic or non-metastatic.

In the present invention, the term "multidrug" (e.g., "dual-drug") refers to an ADC where two or more drug molecules with identical or different chemical structures are covalently attached to the same site on the antibody or within the same linker. When the chemical structures are different, the two or more drug molecules can have the same mechanism of action (e.g., both are TOPO1 inhibitors) or different mechanisms of action (e.g., one is a TOPO1 inhibitor and the other is a DDR inhibitor).

### Multidrug Linkers

One aspect of the present invention provides a compound, or a tautomer, a mesomer, a racemate, an enantiomer, or a diastereomer thereof, or a deuterated compound form thereof, or a mixture form thereof, or a pharmaceutically acceptable salt, prodrug, or solvate thereof, wherein the compound comprises a structure represented by formula I: 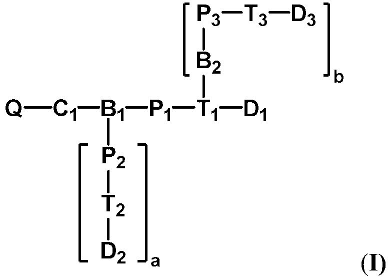 wherein,
Q is a functional group capable of conjugating to cysteine, lysine, a non-natural amino acid, or a glycosyl of an antibody molecule;
C₁ is selected from the group consisting of: a direct bond, optionally substituted alkylene, optionally substituted polyethylene glycol group, optionally substituted alkenylene, optionally substituted alkynylene, optionally substituted aliphatic cyclylene, optionally substituted aliphatic heterocyclylene, optionally substituted arylene, and optionally substituted heteroarylene; when substituted, the substituent(s) are selected from halogen, hydroxyl, amino, carboxyl, sulfonic acid group, sulfone group, phosphonic acid group, and alkoxy;
B₁ and B₂ are independently a direct bond or a branching group, provided that when C₁ is a direct bond, B₁ is not a direct bond;
P₁, P₂, and P₃ are each independently an optionally substituted polypeptide residue or a glucose fragment;
T₁, T₂, and T₃ are each independently a direct bond or an optionally substituted spacer group, provided that at most two of T₁, T₂, and T₃ are direct bonds and at least one of B₂, T₁, T₂, and T₃ is substituted with a hydrophilic group;
D₁, D₂, and D₃ are a first drug unit, a second drug unit, and a third drug unit, respectively, and are the same or different;
a and b are independently 0, 1, 2, or 3, provided that a and b are not both 0; and
when a=0, B₁ is a direct bond and T₁ is not a direct bond;
when b=0, B₁ is not a direct bond.

In some embodiments, the present invention provides a compound, or a tautomer, a mesomer, a racemate, an enantiomer, or a diastereomer thereof, or a deuterated compound form thereof, or a mixture form thereof, or a pharmaceutically acceptable salt, prodrug, or solvate thereof, wherein the compound comprises a structure represented by formula Ia: 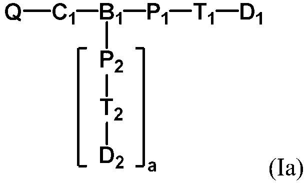 wherein:
Q is a functional group capable of conjugating to cysteine, lysine, a non-natural amino acid, or a glycosyl of an antibody molecule;
C₁ is selected from the group consisting of: a direct bond, optionally substituted alkylene, optionally substituted polyethylene glycol group, optionally substituted alkenylene, optionally substituted alkynylene, optionally substituted aliphatic cyclylene, optionally substituted aliphatic heterocyclylene, optionally substituted arylene, and optionally substituted heteroarylene; when substituted, the substituent(s) are selected from halogen, hydroxyl, amino, carboxyl, sulfonic acid group, sulfone group, phosphonic acid group, and alkoxy;
B₁ is a branching group;
P₁ and P₂ are each independently an optionally substituted polypeptide residue or a glucose fragment;
T₁ and T₂ are each independently a direct bond or an optionally substituted spacer group, provided that at most one of T₁ and T₂ is a direct bond, and at least one of T₁ and T₂ is substituted with a hydrophilic group;
D₁ and D₂ are a first drug unit and a second drug unit, respectively, and are the same or different;
a is 1, 2, or 3.

In some embodiments, the present invention provides a compound, or a tautomer, a mesomer, a racemate, an enantiomer, or a diastereomer thereof, or a deuterated compound form thereof, or a mixture form thereof, or a pharmaceutically acceptable salt, prodrug, or solvate thereof, wherein the compound comprises a structure represented by formula Ib: 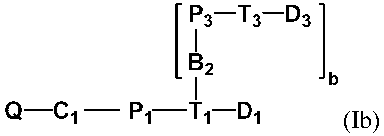 wherein:
Q is a functional group capable of conjugating to cysteine, lysine, a non-natural amino acid, or a glycosyl of an antibody molecule;
C₁ is selected from the group consisting of: optionally substituted alkylene, optionally substituted polyethylene glycol group, optionally substituted alkenylene, optionally substituted alkynylene, optionally substituted aliphatic cyclylene, optionally substituted aliphatic heterocyclylene, optionally substituted arylene, and optionally substituted heteroarylene; when substituted, the substituent(s) are selected from halogen, hydroxyl, amino, carboxyl, sulfonic acid group, sulfone group, phosphonic acid group, and alkoxy;
B₂ is a branching group;
P₁ and P₃ are each independently a direct bond or an optionally substituted polypeptide residue or a glucose fragment;
T₁ is an optionally substituted spacer group, T₃ is a direct bond or an optionally substituted spacer group, and at least one of B₂ and T₃ is substituted with a hydrophilic group;
D₁ and D₃ are a first drug unit and a third drug unit, respectively, and are the same or different;
b is 1, 2, or 3.

In some other embodiments, the present invention provides a compound, or a tautomer, a mesomer, a racemate, an enantiomer, or a diastereomer thereof, or a deuterated compound form thereof, or a mixture form thereof, or a pharmaceutically acceptable salt, prodrug, or solvate thereof, wherein the compound comprises a structure represented by formula I: 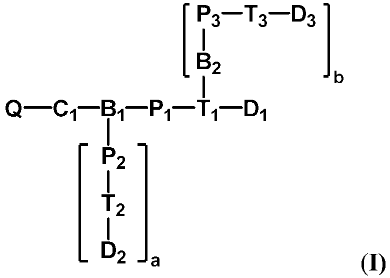 wherein,
Q is a functional group capable of conjugating to cysteine, lysine, a non-natural amino acid, or a glycosyl of an antibody molecule;
C₁ is selected from the group consisting of: a direct bond, optionally substituted alkylene, optionally substituted polyethylene glycol group, optionally substituted alkenylene, optionally substituted alkynylene, optionally substituted aliphatic cyclylene, optionally substituted aliphatic heterocyclylene, optionally substituted arylene, and optionally substituted heteroarylene; when substituted, the substituent(s) are selected from halogen, hydroxyl, amino, carboxyl, sulfonic acid group, sulfone group, phosphonic acid group, and alkoxy;
B₁ and B₂ are independently a branching group;
P₁, P₂, and P₃ are each independently an optionally substituted polypeptide residue or a glucose fragment;
T₁ is an optionally substituted spacer group, T₂ and T₃ are each independently a direct bond or an optionally substituted spacer group, and at least one of B₂, T₂, and T₃ is substituted with a hydrophilic group;
D₁, D₂, and D₃ are a first drug unit, a second drug unit, and a third drug unit, respectively, and are the same or different; and
a and b are independently 1, 2, or 3.

In some embodiments, in formulas I, Ia, and Ib, Q is selected from the group consisting of: optionally substituted 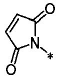 (Q1), optionally substituted 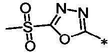 (Q2), optionally substituted 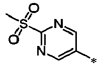 (Q3), optionally substituted 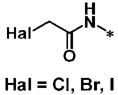 (Q4), optionally substituted 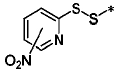 (Q5), optionally substituted 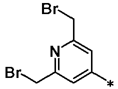 (Q6), optionally substituted 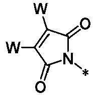 (Q7), optionally substituted 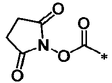 (Q8), optionally substituted 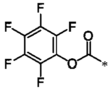 (Q9), wherein * represents a site connected to C₁, the W group in Q7 is bromo or Ar-S, wherein Ar is phenyl or substituted phenyl, and the substituent(s) in the substituted phenyl are selected from alkyl, alkoxy, halogen, ester group, nitro, and - C(O)NR1R2-, wherein R1 and R2 are independently selected from a chemical bond, H, and alkyl, or R1 and R2 together form a 5-7 membered heterocyclic ring with one or more heteroatoms selected from O, N, and S.

In some embodiments, in formulas I, Ia, and Ib, Q is selected from the group consisting of: 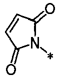 (Q1), 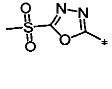 (Q2), 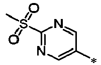 (Q3), 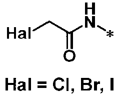 (Q4), 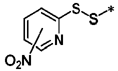 (Q5), 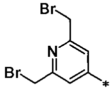 (Q6), 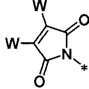 (Q7), 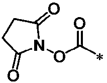 (Q8), 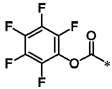 (Q9), wherein * represents a site connected to C₁, the W group in Q7 is bromo or Ar-S, wherein Ar is phenyl or substituted phenyl, and the substituent(s) in the substituted phenyl are selected from alkyl, alkoxy, halogen, ester group, nitro, and -C(O)NR1R2-, wherein R1 and R2 are independently selected from a chemical bond, H, and alkyl, or R1 and R2 together form a 5-7 membered heterocyclic ring with one or more heteroatoms selected from O, N, and S.

In preferred embodiments, in formulas I, Ia, and Ib, Q is Q1.

In preferred embodiments, in formulas I, Ia, and Ib, C₁ is selected from: a direct bond, - (CH₂)ₘ₁-, -(CH₂)ₘ₁-(O-CH₂-CH₂)ₘ₂-, -(CH₂)ₘ₁-(O-CH₂-CH₂)ₘ₂-(CH₂)ₘ₃-, -(CH₂)ₘ₁-C(O)NH-(CH₂)ₘ₂-, -(CH₂)ₘ₁-NHC(O)-(CH₂)ₘ₂-, and -(C≡C-(CH₂)ₘ₁-, wherein m1, m2, and m3 are independently integers from 1 to 6, and the indicated substituent is connected to Q on the left side and to B₁ or P₁ on the right side.

In preferred embodiments, in formulas I and Ia, B₁ is selected from the group consisting of: a direct bond, 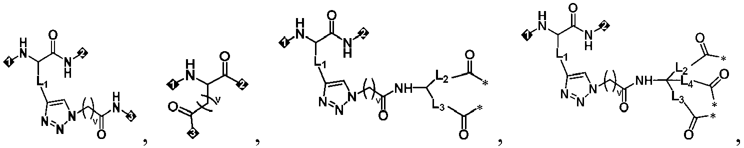 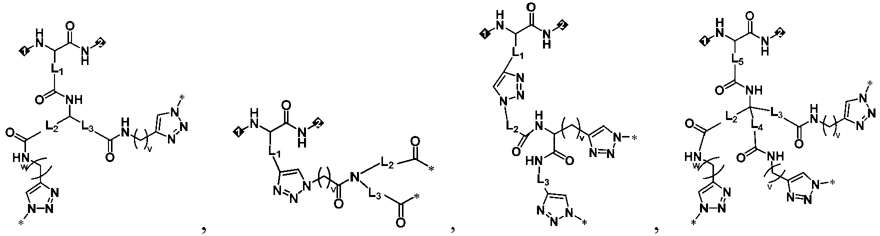 and 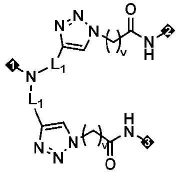 wherein attachment point 1 is connected to C₁, attachment point 2 is connected to P₁, attachment points * and 3 are connected to P₂, L₁ is selected from -(CH₂)ₘ₁-, - (CH₂)ₘ₁-C(O)NH-(CH₂)ₘ₂-, -(CH₂)ₘ₁-NHC(O)-(CH₂)ₘ₂-, L₂, L₃, and L₄ are independently - (CH₂)ₘ₁-(O-CH₂-CH₂)ₘ₂- or -(CH₂)ₘ₁-(O-CH₂-CH₂)ₘ₂-(CH₂)ₘ₃-, L₅ is -(CH₂)ₘ₁-, wherein m1, m2, m3, w, and v are each independently 1, 2, 3, 4, 5, or 6.

In preferred embodiments, in formulas I and Ia, when C₁ is a direct bond, B₁ is 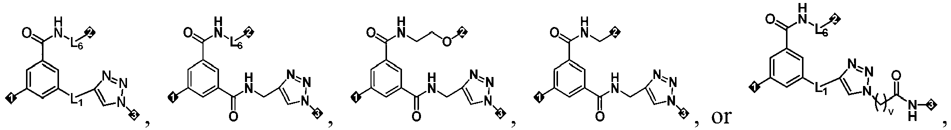 wherein attachment point 1 is connected to Q, attachment point 2 is connected to P₁, attachment point 3 is connected to P₂, L₆ is -(CH₂)ₘ₁- or -(CH₂-CH₂-O)m₂, wherein m1, m2 are each independently 1, 2, 3, 4, 5, or 6.

In preferred embodiments, in formulas I and Ia, B₁ is selected from the group consisting of: a direct bond, 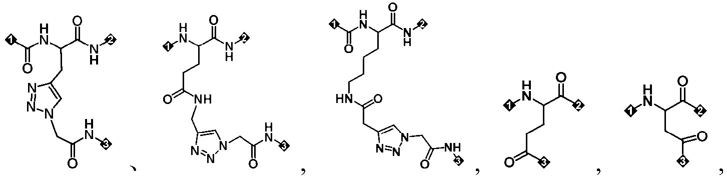 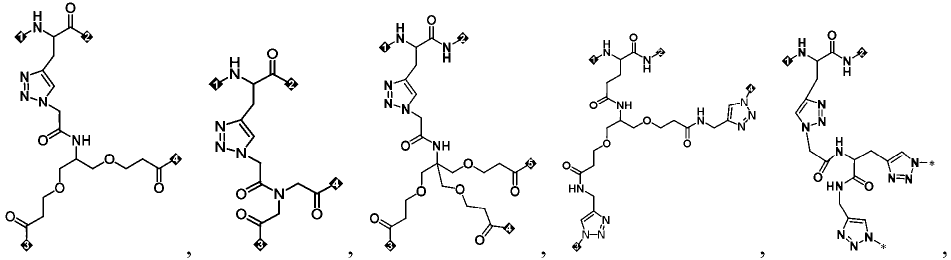 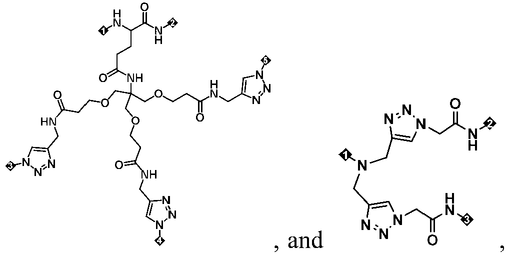 wherein attachment point 1 is connected to C₁, attachment point 2 is connected to P₁, attachment points 3, 4 and 5 are connected to P₂.

In some embodiments, in formulas I and Ib, B₂ is selected from the group consisting of: 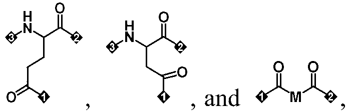 wherein attachment point 1 is connected to T₁, attachment point 2 is connected to P₃, attachment point 3 is connected to the hydrophilic group, M is selected from the group consisting of: optionally substituted C₁-C₆ alkylene, optionally substituted C₁-C₆ alkoxy, and optionally substituted C₁-C₅ alkenylene.

In some embodiments, in formula I, T₁, T₂, T₃ are independently selected from the group consisting of: a direct bond 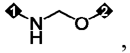 and optionally substituted 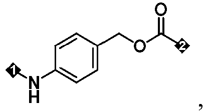 wherein attachment point 1 is connected to P₁, P₂, or P₃, and attachment point 2 is connected to D₁, D₂, or D₃.

In some embodiments, in formula Ia, T₁ and T₂ are independently selected from the group consisting of: a direct bond, 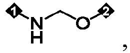 and optionally substituted 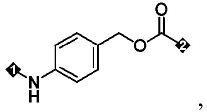 wherein attachment point 1 is connected to P₁ or P₂, and attachment point 2 is connected to D₁ or D₂.

In some embodiments, in formula Ib, T₁ is selected from the group consisting of: 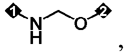 and optionally substituted 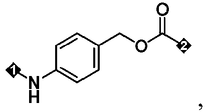 wherein attachment point 1 is connected to P₁, and attachment point 2 is connected to D₁, T₃ is selected from the group consisting of: a direct bond, 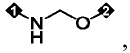 and optionally substituted 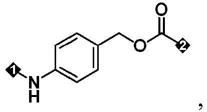 wherein attachment point 1 is connected to P₃, and attachment point 2 is connected to D₃.

In some embodiments, in formulas I, Ia, and Ib, P₁, P₂, P₃, when present, independently comprises an optionally substituted polypeptide residue composed of amino acids selected from the group consisting of: phenylalanine, isoleucine, leucine, tryptophan, valine, methionine, tyrosine, alanine, threonine, histidine, serine, glutamine, arginine, lysine, asparagine, glutamic acid, proline, citrulline, aspartic acid, and glycine.

In preferred embodiments, in formulas I, Ia, and Ib, P₁, P₂, P₃, when present, independently comprises an optionally substituted polypeptide residue composed of amino acids selected from the group consisting of: glycine, phenylalanine, valine, alanine, arginine, citrulline, aspartic acid, asparagine, and lysine.

In preferred embodiments, in formulas I, Ia, and Ib, P₁, P₂, P₃, when present, independently comprises an optionally substituted polypeptide residue selected from the group consisting of: phenylalanine-lysine (Phe-Lys), valine-alanine (Val-Ala), valine-citrulline (Val-Cit), glutamic acid-valine-alanine (Glu-Val-Ala), glutamic acid-valine-citrulline (Glu-Val-Cit), valine-lysine (Val-Lys), alanine-alanine (Ala-Ala), alanine-alanine-alanine (Ala-Ala-Ala), alanine-alanine-asparagine (Ala-Ala-Asn), alanine-leucine (Ala-Leu), leucine-leucine (Leu-Leu), phenylalanine-arginine (Phe-Arg), phenylalanine-lysine (Phe-Lys), 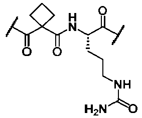 (cBu-Cit), 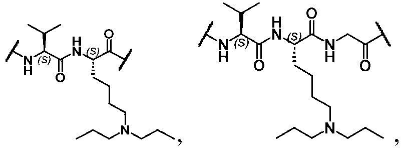 glycine-glycine-phenylalanine-glycine (Gly-Gly-Phe-Gly), glycine-proline (Gly-Pro). In preferred embodiments, in formulas I, Ia, and Ib, P₁, P₂, P₃, when present, are independently phenylalanine-lysine (Phe-Lys), valine-alanine (Val-Ala), valine-citrulline (Val-Cit), and glycine-glycine-phenylalanine-glycine (Gly-Gly-Phe-Gly).

In preferred embodiments, in formulas I, Ia, and Ib, one of the combination P₂-T₂ and the combination P₃-T₃ is 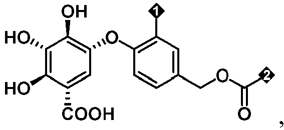 wherein attachment point 1 is connected to B₁ or B₂, and attachment point 2 is connected to D₂ or D₃.

In preferred embodiments, in formulas I, Ia, and Ib, the hydrophilic group is selected from: substituted polysarcosine residue, polyglycerol, polyol, glycosyl, cyclodextrin, substituted ethylene glycol fragment, substituted glycosylated polyethylene glycol, substituted glycosylated polyglycerol, substituted cyclodextrin polyethylene glycol, or a combination thereof.

In some preferred embodiments, in formulas I, Ia, and Ib, the substituted polysarcosine residue is 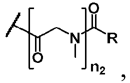 wherein n₂ is an integer between 4 and 20, for example between 4 and 16, R is selected from: C₁-C₆ alkyl, C₁-C₆ cycloalkyl, C₁-C₆ alkoxy. In some preferred embodiments, in formulas I, Ia, and Ib, the substituted glycosylated polyglycerol fragment is 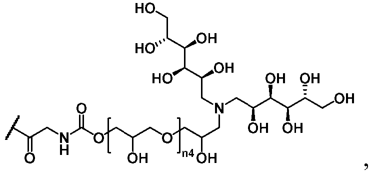 wherein n₄ is an integer between 4 and 12, for example between 4 and 10. In some preferred embodiments, in formulas I, Ia, and Ib, the substituted polyethylene glycol fragment is 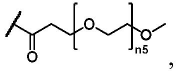 wherein n₅ is an integer between 4 and 24, for example between 8 and 18. In some other preferred embodiments, in formulas I, Ia, and Ib, the substituted glycosylated polyethylene glycol fragment is 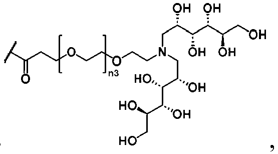 wherein n₃ is an integer between 4 and 18, for example between 4 and 12.

In some other preferred embodiments, in formulas I, at least one of T₁, T₂, T₃ is 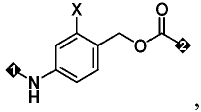 wherein X is optionally substituted 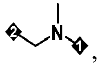 the hydrophilic group is connected to T₁, T₂, or T₃ via X, wherein in X, attachment point 1 is connected to the hydrophilic group, and attachment point 2 is connected to one of T₁, T₂, T₃.

In some other preferred embodiments, in formulas Ia, at least one of T₁ and T₂ is 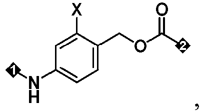 wherein X is optionally substituted 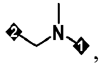 the hydrophilic group is connected to T₁ or T₂ via X, wherein in X, attachment point 1 is connected to the hydrophilic group, and attachment point 2 is connected to one of T₁ or T₂.

In some other preferred embodiments, in formulas Ib, at least one of T₁ and T₃ is 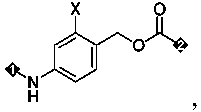 wherein X is optionally substituted 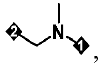 the hydrophilic group is connected to T₁ or T₃ via X, wherein in X, attachment point 1 is connected to the hydrophilic group, and attachment point 2 is connected to one of T₁ or T₃.

In preferred embodiments, in formulas I, Ia, and Ib, D₁, D₂, D₃, when present, are a first anti-cancer agent, a second anti-cancer agent, and a third anti-cancer agent, respectively; preferably, at least two of the first, second, and third anti-cancer agents have a synergistic anti-cancer effect.

In preferred embodiments, D₁, D₂, D₃, when present, are independently a cytotoxic drug or a tumor-targeted therapeutic drug.

In some embodiments, in formulas I, Ia, and Ib, D₁, D₂, and D₃, when present, are all cytotoxic drugs or are all tumor-targeted therapeutic drugs. In some embodiments, in formulas I, Ia, and Ib, D₁, D₂, and D₃, when present, are all cytotoxic drugs or are all tumor-targeted therapeutic drugs, and have identical structural formulas. In some embodiments, in formulas I, Ia, and Ib, D₁, D₂, and D₃, when present, are all cytotoxic drugs or are all tumor-targeted therapeutic drugs, but have different structural formulas.

In preferred embodiments, at least one of D₁, D₂, and D₃ is a cytotoxic drug, and at least another one is a tumor-targeted therapeutic drug. In preferred embodiments, at least two of D₁, D₂, and D₃ are tumor-targeted therapeutic drugs, for example, tumor-targeted therapeutic drugs that can produce a synergistic or additive effect. In preferred embodiments, at least two of D₁, D₂, and D₃ are cytotoxic drugs.

Preferably, the cytotoxic drug is selected from drug units targeting topoisomerase, drug units targeting tubulin, nucleoside antimetabolite anti-cancer drug units, and drug units targeting DNA.

In preferred embodiments, the tumor-targeted therapeutic drug is selected from drug units targeting DNA damage response (DDR) or "synthetic lethality" related pathways, drug units targeting epigenetics, drug units targeting apoptosis-related pathways, drug units targeting transcription factors, and drug units targeting immune activation pathway-related targets, comprising: inhibitors of EFGR pathway-related targets; Ras-Raf-MAPK pathway inhibitors; PI3K/AKT/mTOR pathway inhibitors; cell cycle pathway inhibitors; cGAS-STING signaling pathway agonists; estrogen receptor antagonists; androgen receptor antagonists; glucocorticoid receptor modulators; autophagy inhibitors; FAK inhibitors; Smo inhibitors; BTK inhibitors; PDE4 inhibitors; Lck inhibitors; PLK1 inhibitors; TLR7/8 modulators; N-myristoyltransferase (NMT) inhibitors.

In preferred embodiments, at least one of D₁, D₂, and D₃ is a drug targeting topoisomerase, and at least another one is a drug targeting DNA damage response (DDR) or "synthetic lethality" related pathways.

Accordingly, in some embodiments, at least one of D₁, D₂, and D₃ is a cytotoxic drug selected from drugs targeting topoisomerase, drugs targeting tubulin, and drugs targeting DNA, and at least another one is a tumor-targeted therapeutic drug, which is a drug targeting DNA damage response (DDR) or "synthetic lethality" related pathways. For example, at least one of D₁, D₂, and D₃ is a drug targeting topoisomerase, and at least another one is a drug targeting DNA damage response (DDR) or "synthetic lethality" related pathways. For example, at least one of D₁, D₂, and D₃ is a drug targeting tubulin, and at least another one is a drug targeting DNA damage response (DDR) or "synthetic lethality" related pathways. For example, at least one of D₁, D₂, and D₃ is a drug targeting DNA, and at least another one is a drug targeting DNA damage response (DDR) or "synthetic lethality" related pathways.

In some embodiments, at least one of D₁, D₂, and D₃ is a cytotoxic drug selected from drugs targeting topoisomerase, drugs targeting tubulin, and drugs targeting DNA, and at least another one is a tumor-targeted therapeutic drug, which is a drug targeting epigenetics. For example, at least one of D₁, D₂, and D₃ is a drug targeting topoisomerase, and at least another one is a drug targeting epigenetics. For example, at least one of D₁, D₂, and D₃ is a drug targeting tubulin, and at least another one is a drug targeting epigenetics. For example, at least one of D₁, D₂, and D₃ is a drug targeting DNA, and at least another one is a drug targeting epigenetics.

In some embodiments, at least one of D₁, D₂, and D₃ is a cytotoxic drug selected from drugs targeting topoisomerase, drugs targeting tubulin, and drugs targeting DNA, and at least another one is a tumor-targeted therapeutic drug, which is a drug targeting apoptosis-related pathways. For example, at least one of D₁, D₂, and D₃ is a drug targeting topoisomerase, and at least another one is a drug targeting apoptosis-related pathways. For example, at least one of D₁, D₂, and D₃ is a drug targeting tubulin, and at least another one is a drug targeting apoptosis-related pathways. For example, at least one of D₁, D₂, and D₃ is a drug targeting DNA, and at least another one is a drug targeting apoptosis-related pathways.

In some embodiments, at least one of D₁, D₂, and D₃ is a cytotoxic drug selected from drugs targeting topoisomerase, drugs targeting tubulin, and drugs targeting DNA, and at least another one is a tumor-targeted therapeutic drug, which is a drug targeting immune activation pathway-related targets. For example, at least one of D₁, D₂, and D₃ is a drug targeting topoisomerase, and at least another one is a drug targeting immune activation pathways. For example, at least one of D₁, D₂, and D₃ is a drug targeting tubulin, and at least another one is a drug targeting immune activation pathways. For example, at least one of D₁, D₂, and D₃ is a drug targeting DNA, and at least another one is a drug targeting immune activation pathways.

In some embodiments, at least two of D₁, D₂, and D₃ are drugs targeting DNA damage response (DDR) or "synthetic lethality" related pathways. In some embodiments, at least two of D₁, D₂, and D₃ are drugs targeting epigenetics. In some embodiments, at least two of D₁, D₂, and D₃ are drugs targeting apoptosis-related pathways. In some embodiments, at least two of D₁, D₂, and D₃ are drugs targeting immune activation pathways.

In some embodiments, at least two of D₁, D₂, and D₃ are drugs targeting topoisomerase. In some embodiments, at least two of D₁, D₂, and D₃ are nucleoside antimetabolite anti-cancer drugs. In some embodiments, at least two of D₁, D₂, and D₃ are drugs targeting tubulin. In some embodiments, at least two of D₁, D₂, and D₃ are drugs targeting DNA.

In some embodiments, at least one of D₁, D₂, and D₃ is a cytotoxic drug selected from drugs targeting topoisomerase, for example a TOPO1 inhibitor, and at least another one is a tumor-targeted therapeutic drug, which is a drug targeting DNA damage response (DDR) or "synthetic lethality" related pathways, selected from PARP inhibitors, ATR inhibitors, CHK1 inhibitors, ATM inhibitors, DNA-PK inhibitors, WEE1 inhibitors, POLQ inhibitors, CDK12 inhibitors, USP1 inhibitors, PKMYT1 inhibitors, Rad51 inhibitors.

In some embodiments, at least two of D₁, D₂, and D₃ are cytotoxic drugs and are independently selected from drugs targeting topoisomerase and drug units targeting tubulin, for example TOPO1 inhibitors, TOPO2 inhibitors, and tubulin inhibitors.

In some embodiments, at least two or all three of D₁, D₂, and D₃ are tumor-targeted therapeutic drugs, and are independently selected from drug units targeting DNA damage response (DDR) or "synthetic lethality" related pathways, drug units targeting epigenetics, drug units targeting apoptosis-related pathways, drug units targeting cell cycle pathways, drug units targeting transcription factors, and drug units targeting immune activation pathway-related targets, comprising: inhibitors targeting EFGR pathway-related targets; inhibitors targeting Ras-Raf-MAPK pathway-related targets; inhibitors targeting PI3K/AKT/mTOR pathway-related targets; inhibitors targeting cell cycle pathway-related targets; agonists targeting cGAS-STING signaling pathway-related targets; estrogen receptor antagonists, androgen receptor antagonists; glucocorticoid receptor modulators; autophagy inhibitors; FAK inhibitors; Smo inhibitors; BTK inhibitors; PDE4 inhibitors; Lck inhibitors; PLK1 inhibitors; TLR7/8 modulators; N-myristoyltransferase (NMT) inhibitors.

In some embodiments, one of D₁, D₂, and D₃ is a cytotoxic drug selected from drug units targeting topoisomerase and drug units targeting tubulin, for example a TOPO1 inhibitor or a tubulin inhibitor, and the other one or two are tumor-targeted therapeutic drugs, selected from drug units targeting DNA damage response (DDR) or "synthetic lethality" related pathways, drug units targeting epigenetics, drug units targeting apoptosis-related pathways, drug units targeting cell cycle pathways, drug units targeting transcription factors, and drug units targeting immune activation pathway-related targets, comprising: inhibitors targeting EFGR pathway-related targets; inhibitors targeting Ras-Raf-MAPK pathway-related targets; inhibitors targeting PI3K/AKT/mTOR pathway-related targets; inhibitors targeting cell cycle pathway-related targets; agonists targeting cGAS-STING signaling pathway-related targets; estrogen receptor antagonists, androgen receptor antagonists; glucocorticoid receptor modulators; autophagy inhibitors; FAK inhibitors; Smo inhibitors; BTK inhibitors; PDE4 inhibitors; Lck inhibitors; PLK1 inhibitors; TLR7/8 modulators; N-myristoyltransferase (NMT) inhibitors.

In preferred embodiments, the drug unit targeting topoisomerase is a topoisomerase I (TOPO1) inhibitor or topoisomerase II (TOPO2) inhibitor, comprising Exatecan (CAS 171335-80-1), DXd (CAS 1599440-33-1), 7-ethyl-10-hydroxycamptothecin (SN38, CAS 86639-52-3), Belotecan (CAS 213819-48-8), (4-NH₂)-Exatecan (AZD'0132, CAS 2495742-21-5), 7-MAD-MDCPT (CAS 765871-81-6), 7-aminomethyl-10-methyl-11-fluorocamptothecin (CAS 2378616-23-8), Voreloxin (CAS 175414-77-4), or derivatives and analogs thereof. In preferred embodiments, the drug unit targeting tubulin is Eribulin, Vinblastine, Paclitaxel, MMAE, MMAF, Maytansine, or derivatives thereof. In preferred embodiments, the drug unit targeting DNA is a DNA minor groove binder (PBD), a DNA alkylator (Duocarmycin), Trabectedin, Lurbinectedin, or derivatives thereof.

In preferred embodiments, the drug unit targeting DNA damage response (DDR) or "synthetic lethality" related pathways is a PARP inhibitor, ATR inhibitor, CHK1 inhibitor, ATM inhibitor, DNA-PK inhibitor, WEE1 inhibitor, POLQ inhibitor, CDK12 inhibitor, USP1 inhibitor, PKMYT1 inhibitor, or Rad51 inhibitor; the PARP inhibitor is preferably Rucaparib, Niraparib, Veliparib, A-966492, Talazoparib, AZD5305, Venadaparib, Mefuparib, or analogs thereof; the ATR inhibitor is preferably Berzosertib, Ceralasertib, or analogs thereof; and/or the CHK1 inhibitor is preferably Prexasertib (LY2606368), AZD7762, Rabusertib (LY2603618), MK-8776 (SCH 900776), CHIR-124, PF-477736, CCT245737 (SRA737, PNT-737), GDC-0575 (ARRY-575), or analogs thereof; and/or the WEE1 inhibitor is preferably ZN-C3, Adavosertib (AZD1775), or analogs thereof.

Preferably, an analog of the AZD5305 comprises the following structure: 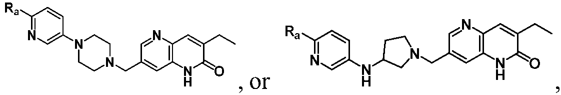 wherein Rₐ is selected from -C(O)NH-R_{b} or -NHC(O)-R_{b}, R_{b} is selected from: C₂₋₇ alkyl, C₃₋₇ monocyclic cycloalkyl, C₄₋₁₀ bicyclic cycloalkyl, C₅₋₉ spirocycloalkyl, and C₅₋₉ bridged cycloalkyl, each substituted with at least one primary or secondary amino group, or R_{b} is a 4-6 membered monocyclic heterocycloalkyl, C₄₋₁₀ bicyclic heterocycloalkyl, C₅₋₉ spiroheterocycloalkyl, C₅₋₉ bridged heterocycloalkyl containing 1-5 nitrogen, oxygen, sulfur atoms, wherein the R_{b} group comprises at least one primary or secondary amino group.

Preferably, the AZD5305 analog is selected from the following structures:

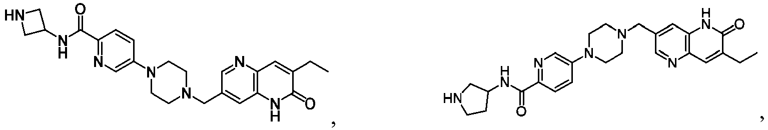

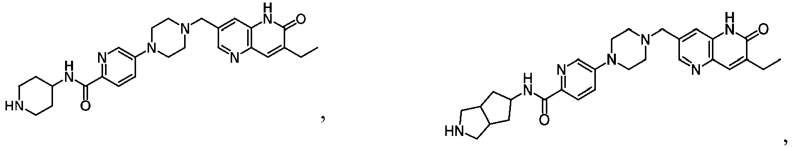

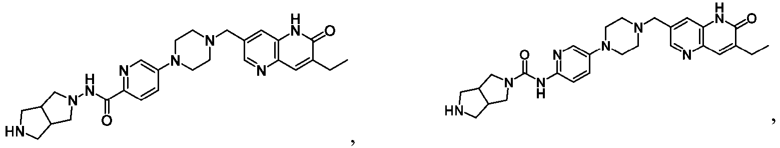

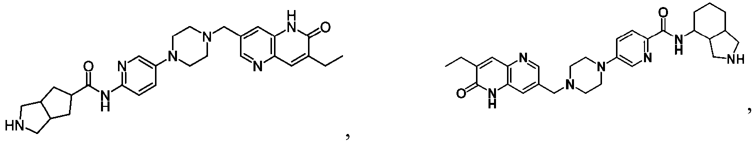

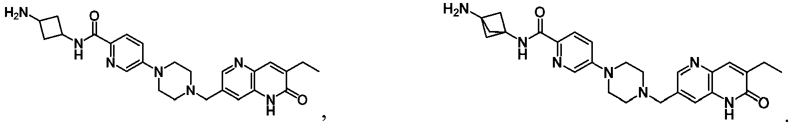

In preferred embodiments, the PKMYT1 inhibitor is preferably RP-6306 and GSK-1520489A or analogs thereof.

In preferred embodiments, analogs of the WEE1 inhibitors Adavosertib (AZD1775, CAS 955365-80-7), Debio 0123 (CAS 2243882-74-6) are selected from the following structures:

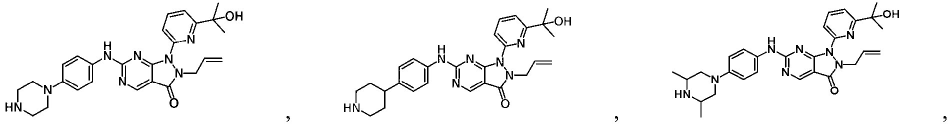

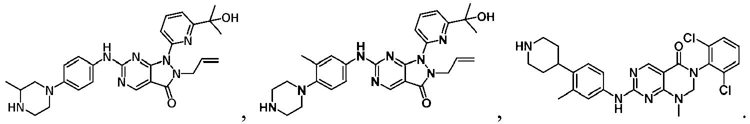

In preferred embodiments, the drug unit targeting apoptosis-related pathways is a Bcl-2 family protein inhibitor; the Bcl-2 family protein inhibitor comprises BCL-2 inhibitors, BCL-XL inhibitors, and MCL-1 inhibitors, preferably Venetoclax (CAS 1257044-40-8), Navitoclax (CAS 923564-51-6), Navitoclax analog (CAS 2143096-93-7), ABT-737 (CAS 852808-04-9), A-1331852 (CAS 1430844-80-6), S64315 (CAS 1799631-75-6), or analogs thereof.

Preferably, the A-1331852, S64315 analogs are selected from the following structures:

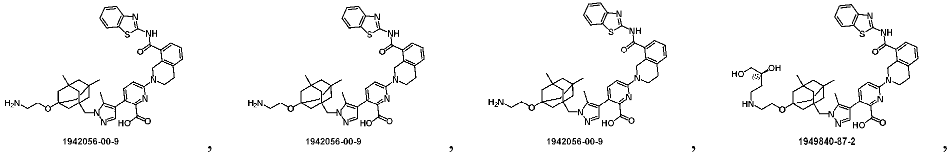

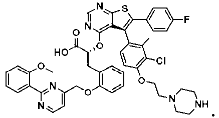

In preferred embodiments, the drug unit targeting epigenetics is an LSD1 inhibitor, EZH2 inhibitor, BRD4 inhibitor, PRMT5 inhibitor, or PRMT1 inhibitor. In preferred embodiments, the LSD1 inhibitor is preferably Tranylcypromine, ORY-1001 (Iadademstat, CAS 1431303-72-8), CC-90011 (Pulrodemstat, CAS 1821307-10-1), ORY-2001, GSK-2879552, IMG-7289, INCB059872, or TAK-418 or analogs thereof; the EZH2 inhibitor is preferably Tazemetostat (CAS 1403254-99-8), GSK2816126, CPI-1205, PF-06821497, SHR2554, XNW5004, HH2853, or analogs thereof; the BRD4 inhibitor is a BI-2536 analog, Birabresib; the PRMT5 inhibitor is preferably GSK3326595, AMG 193, MRTX1719, SKL27969, TNG908, SCR-6920, SH3765, SYHX2001, or analogs thereof; the PRMT1 inhibitor is preferably GSK3368715 (CAS 1629013-22-4), MS023 (CAS 1831110-54-3), or analogs thereof.

In preferred embodiments, the Ras-Raf-MAPK pathway inhibitors comprise: SOS1 inhibitors BAY-293 (CAS 2244904-70-7), BI-3406 (CAS 2230836-55-0), or analogs thereof; KRAS inhibitors BI-2493 (CAS 2937344-16-4), MRTX1133 (CAS 2621928-55-8), or analogs thereof; MEK inhibitors Cobimetinib (CAS 934660-93-2), Selumetinib (CAS 606143-52-6), Mirdametinib (CAS 391210-10-9), Binimetinib (CAS 606143-89-9), TAK-733 (CAS 1035555-63-5), GDC-0623 (CAS 1168091-68-6), AZD8330 (CAS 869357-68-6), Trametinib (CAS 871700-17-3), Trametiglue (CAS 2666940-97-0), or analogs thereof; ERK inhibitors ASN007 (CAS 2055597-12-9) or analogs thereof.

In preferred embodiments, the cell cycle pathway inhibitors comprise: CDK4/6 inhibitors Palbociclib (CAS 571190-30-2), Abemaciclib (CAS 1231929-97-7), Ribociclib (CAS 1211441-98-3), Dalpiciclib (CAS 1637781-04-4), or analogs thereof; pan-CDK inhibitors Dinaciclib (CAS 779353-01-4) or analogs thereof.

In preferred embodiments, the PI3K/AKT/mTOR pathway inhibitors comprise: PKI-587 (CAS 1197160-78-3), desmethyl PKI-587 (CAS 1950569-63-7), AZD8055 (CAS 1009298-09-2), Afuresertib (CAS 1047644-62-1), or analogs thereof.

In preferred embodiments, the estrogen receptor antagonists/degraders comprise Fulvestrant (CAS 129453-61-8), Elacestrant (CAS 1349723-93-8); the androgen receptor antagonists comprise abiraterone (CAS 154229-19-3), JNJ-63576253 (CAS 2110428-64-1), or analogs thereof.

In preferred embodiments, the autophagy inhibitor is Hydroxychloroquine (HCQ, CAS 118-42-3) and Chloroquine (CQ, CAS 54-05-7) or analogs thereof.

In preferred embodiments, the drug targeting transcription factors is a GSPT1 degrader (molecular glue), and the GSPT1 degrader is selected from the following structures:

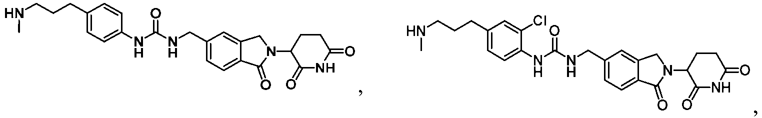

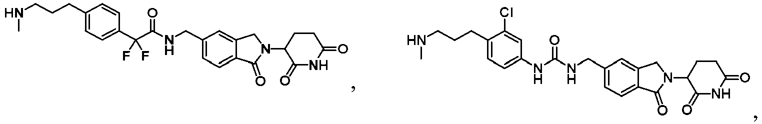

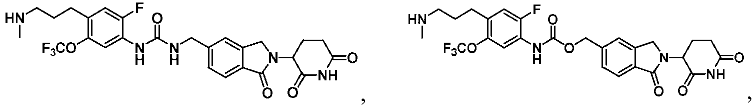

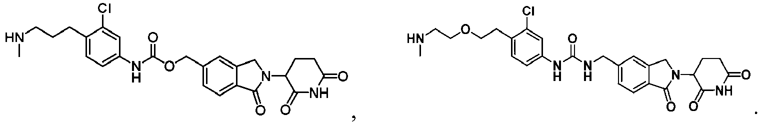

Preferably, the PLK1 inhibitor is BI2536 (CAS 755038-02-9), Volasertib (CAS 755038-65-4), GSK461364 (CAS 929095-18-1), Onvansertib (CAS 1034616-18-6), or analogs thereof.

In preferred embodiments, the N-myristoyltransferase (NMT) inhibitor is MYX1715 (CAS 2445448-66-6) or analogs thereof.

In preferred embodiments, D₁, D₂, and D₃ are selected from the combinations in the following table:

| Drug Combination | D1 | D2 | D3 |
|---|---|---|---|
| Combination 1 | TOPO1 inhibitor | PARP inhibitor | / |
| Combination 2 | TOPO1 inhibitor | ATR inhibitor | / |
| Combination 3 | TOPO1 inhibitor | CHK1 inhibitor | / |
| Combination 4 | TOPO1 inhibitor | PARP inhibitor | ATR inhibitor |
| Combination 5 | TOPO1 inhibitor | PARP inhibitor | CHK1 inhibitor |
| Combination 6 | TOPO1 inhibitor | PARP inhibitor | WEE1 inhibitor |
| Combination 7 | TOPO1 inhibitor | ATR inhibitor | WEE1 inhibitor |
| Combination 8 | TOPO1 inhibitor | CHK1 inhibitor | WEE1 inhibitor |
| Combination 9 | TOPO1 inhibitor | DNA-PK inhibitor | / |
| Combination 10 | TOPO1 inhibitor | POLQ inhibitor | / |
| Combination 11 | TOPO1 inhibitor | TOPO2 inhibitor | / |
| Combination 12 | PARP inhibitor | ATR/CHK1/WEE1 inhibitor | / |
| Combination 13 | PARP inhibitor | ATR inhibitor | CHK1 inhibitor |
| Combination 14 | TOPO1 inhibitor | Immune activator (PD-L1 inhibitor, CBL-B inhibitor, TLR7/8 agonist, PTPN2/1 inhibitor, or STING agonist) | / |
| Combination 15 | TOPO1 inhibitor | BCL-2 family inhibitor | / |
| Combination 16 | TOPO1 inhibitor | BCL-2 family inhibitor | MCL-1 inhibitor |
| Combination 17 | TOPO1 inhibitor | DNA synthesis inhibitor | / |
| Combination 18 | TOPO1 inhibitor | PI3K/mTOR inhibitor | / |
| Combination 19 | TOPO1 inhibitor | EGFR inhibitor | / |
| Combination 20 | TOPO1 inhibitor | Autophagy inhibitor | / |
| Combination 21 | TOPO1 inhibitor | FAK inhibitor | |
| Combination 22 | TOPO1 inhibitor | PARP inhibitor | FAK inhibitor |
| Combination 23 | TOPO1 inhibitor | PARP inhibitor | Androgen receptor antagonist/degrader |
| Combination 24 | TOPO1 inhibitor | GSPT1 degrader | / |
| Combination 25 | TOPO1 inhibitor | PARP inhibitor | GSPT1 degrader |
| Combination 26 | TOPO1 inhibitor | Tubulin inhibitor | / |
| Combination 27 | TOPO1 inhibitor | Epigenetic regulator | / |
| Combination 28 | Tubulin inhibitor | Immune activator (PD-L1 inhibitor, CBL-B inhibitor, TLR7/8 agonist, PTPN2/1 inhibitor, or STING agonist) | / |
| Combination 29 | Tubulin inhibitor | BCL-2/BCL-XL inhibitor | / |
| Combination 30 | Tubulin inhibitor | BCL-2/BCL-XL inhibitor | MCL-1 inhibitor |
| Combination 31 | Tubulin inhibitor | PI3K/AKT/mTOR inhibitor | / |
| Combination 32 | Tubulin inhibitor | FAK inhibitor | |
| Combination 33 | CDK4/6 inhibitor | MEK inhibitor | / |
| Combination 34 | CDK4/6 inhibitor | MEK inhibitor | EZH2 inhibitor |
| Combination 35 | CDK4/6 inhibitor | Autophagy inhibitor | / |
| Combination 36 | MEK inhibitor | Autophagy inhibitor | / |
| Combination 37 | ERK inhibitor | Autophagy inhibitor | / |
| Combination 38 | CDK4/6 inhibitor | MEK inhibitor | Autophagy inhibitor |
| Combination 39 | CDK4/6 inhibitor | ERK inhibitor | Autophagy inhibitor |
| Combination 40 | CDK4/6 inhibitor | ERK inhibitor | (MEK inhibitor) |
| Combination 41 | CDK4/6 inhibitor | MEK inhibitor | KRAS inhibitor |
| Combination 42 | CDK4/6 inhibitor | MEK inhibitor | SOS1 inhibitor |
| Combination 43 | CDK4/6 inhibitor | MEK inhibitor | FAK inhibitor |
| Combination 44 | CDK4/6 inhibitor | PRMT5 inhibitor | / |
| Combination 45 | CDK4/6 inhibitor | Estrogen receptor antagonist/degrader | / |
| Combination 46 | CDK4/6 inhibitor | Estrogen receptor antagonist/degrader | PI3K/mTOR inhibitor |
| Combination 47 | CDK4/6 inhibitor | MEK inhibitor | GSPT1 degrader |
| Combination 48 | CDK4/6 inhibitor | ERK inhibitor | GSPT1 degrader |
| Combination 49 | CDK4/6 inhibitor | Autophagy inhibitor | GSPT1 degrader |
| Combination 50 | BRD4 inhibitor | EZH2 inhibitor | / |
| Combination 51 | PRMT5 inhibitor | MAT2A inhibitor | / |
| Combination 52 | PRMT5 inhibitor | Gemcitabine | / |
| Combination 53 | PRMT5 inhibitor | CDK4/6 inhibitor | / |
| Combination 54 | PRMT5 inhibitor | PRMT1 inhibitor | / |
| Combination 55 | PRMT5 inhibitor | PRMT1 inhibitor | CDK4/6 inhibitor |
| Combination 56 | PRMT5 inhibitor | PRMT1 inhibitor | MEK inhibitor |
| Combination 57 | RAF inhibitor | MEK inhibitor | / |
| Combination 58 | EGFR inhibitor | MEK inhibitor | / |
| Combination 59 | RAF inhibitor | MEK inhibitor | SHP2 inhibitor |
| Combination 60 | CDK4/6 inhibitor | HIF-2alpha inhibitor | / |
| Combination 61 | TOPO1 inhibitor | WEE1 inhibitor | / |
| Combination 62 | WEE1 inhibitor | CHK1 inhibitor | / |
| Combination 63 | TOPO1 inhibitor | TOPO1 inhibitor | / |
| Combination 64 | CDK inhibitor | MEK inhibitor | |
| Combination 65 | FAK inhibitor | Smo inhibitor | / |
| Combination 66 | PLK1 inhibitor | CDK inhibitor | / |
| Combination 67 | PLK1 inhibitor | CDK inhibitor | PARP inhibitor/ |
| Combination 68 | TOPO1 inhibitor | PLK1 inhibitor | / |
| Combination 69 | TOPO1 inhibitor | PLK1 inhibitor | PARP inhibitor/ |
| Combination 70 | TOPO1 inhibitor | PLK1 inhibitor | ATR inhibitor/ |
| Combination 71 | TOPO1 inhibitor | NMT inhibitor | / |
| Combination 72 | Tubulin inhibitor | NMT inhibitor | / |

In preferred embodiments, at least one of D₁, D₂, and D₃ is a topoisomerase inhibitor, and at least another one is a PARP inhibitor, an ATR inhibitor, or a CHK1 inhibitor. In preferred embodiments, at least one of D₁, D₂, and D₃ is a topoisomerase inhibitor, and at least another one is a Bcl-2 family protein inhibitor. In preferred embodiments, at least one of D₁, D₂, and D₃ is a topoisomerase inhibitor, and at least another one is an LSD1 inhibitor, an EZH2 inhibitor, or a PRMT5 inhibitor. In preferred embodiments, at least one of D₁, D₂, and D₃ is a topoisomerase inhibitor, and at least another one is a PD-L1 inhibitor, a CBL-B inhibitor, a TLR7/8 agonist, or a STING agonist.

In preferred embodiments, at least one of D₁, D₂, and D₃ is selected from: Exatecan (CAS 171335-80-1), DXd (CAS 1599440-33-1), 7-ethyl-10-hydroxycamptothecin (SN38, CAS 86639-52-3), Belotecan (CAS 213819-48-8), (4-NH₂)-Exatecan (AZD'0132, CAS 2495742-21-5), 7-MAD-MDCPT (CAS 765871-81-6), 7-aminomethyl-10-methyl-11-fluorocamptothecin (CAS 2378616-23-8), Voreloxin (CAS 175414-77-4), or derivatives and analogues thereof; and at least another one is selected from PARP inhibitors, ATR inhibitors, CHK1 inhibitors, Bcl-2 family protein inhibitors, LSD1 inhibitors, EZH2 inhibitors, BRD4 inhibitors, PRMT5 inhibitors, PD-L1 inhibitors, CBL-B inhibitors, TLR7/8 agonists, and STING agonists.

In preferred embodiments, at least one of D₁, D₂, and D₃ is selected from: Exatecan (CAS 171335-80-1), DXd (CAS 1599440-33-1), 7-ethyl-10-hydroxycamptothecin (SN38, CAS 86639-52-3), Belotecan (CAS 213819-48-8), (4-NH₂)-Exatecan (AZD'0132, CAS 2495742-21-5), 7-MAD-MDCPT (CAS 765871-81-6), 7-aminomethyl-10-methyl-11-fluorocamptothecin (CAS 2378616-23-8), Voreloxin (CAS 175414-77-4), or derivatives and analogues thereof; and at least another one is selected from: Rucaparib, Niraparib, Veliparib, A-966492, Talazoparib, AZD5305, Venadaparib, Mefuparib, Prexasertib (LY2606368), AZD7762, Rabusertib (LY2603618), MK-8776 (SCH 900776), CHIR-124, PF-477736, CCT245737 (SRA737, PNT-737), GDC-0575 (ARRY-575), Berzosertib, Ceralasertib, RP-6306, GSK-1520489A, and analogues thereof.

In preferred embodiments, at least one of D₁, D₂, and D₃ is a tubulin inhibitor, and at least another one is a PARP inhibitor, an ATR inhibitor, or a CHK1 inhibitor. In preferred embodiments, at least one of D₁, D₂, and D₃ is a tubulin inhibitor, and at least another one is a Bcl-2 family protein inhibitor. In preferred embodiments, at least one of D₁, D₂, and D₃ is a tubulin inhibitor, and at least another one is an LSD1 inhibitor, an EZH2 inhibitor, or a PRMT5 inhibitor. In preferred embodiments, at least one of D₁, D₂, and D₃ is a tubulin inhibitor, and at least another one is a PD-L1 inhibitor, a CBL-B inhibitor, a TLR7/8 agonist, or a STING agonist.

In preferred embodiments, at least one of D₁, D₂, and D₃ is selected from: Eribulin, Vinblastine, Paclitaxel, MMAE, MMAF, Maytansine or derivatives thereof, and at least another one is selected from PARP inhibitors, ATR inhibitors, CHK1 inhibitors, Bcl-2 family protein inhibitors, LSD1 inhibitors, EZH2 inhibitors, BRD4 inhibitors, PRMT5 inhibitors, PD-L1 inhibitors, CBL-B inhibitors, TLR7/8 agonists, and STING agonists.

In preferred embodiments, at least one of D₁, D₂, and D₃ is selected from: Eribulin, Vinblastine, Paclitaxel, MMAE, MMAF, Maytansine or derivatives thereof; and at least another one is selected from: Rucaparib, Niraparib, Veliparib, A-966492, Talazoparib, AZD5305, Venadaparib, Mefuparib, Prexasertib (LY2606368), AZD7762, Rabusertib (LY2603618), MK-8776 (SCH 900776), CHIR-124, PF-477736, CCT245737 (SRA737, PNT-737), GDC-0575 (ARRY-575), Berzosertib, Ceralasertib, RP-6306, GSK-1520489A, and analogues thereof.

In preferred embodiments, at least one of D₁, D₂, and D₃ is selected from: a PBD or Duocarmycin, and at least another one is selected from PARP inhibitors, ATR inhibitors, CHK1 inhibitors, Bcl-2 family protein inhibitors, LSD1 inhibitors, EZH2 inhibitors, BRD4 inhibitors, PRMT5 inhibitors, PD-L1 inhibitors, CBL-B inhibitors, TLR7/8 agonists, and STING agonists.

In preferred embodiments, at least one of D₁, D₂, and D₃ is a PBD or Duocarmycin, and at least another one is selected from: Rucaparib, Niraparib, Veliparib, A-966492, Talazoparib, AZD5305, Venadaparib, Mefuparib, Prexasertib (LY2606368), AZD7762, Rabusertib (LY2603618), MK-8776 (SCH 900776), CHIR-124, PF-477736, CCT245737 (SRA737, PNT-737), GDC-0575 (ARRY-575), Berzosertib, Ceralasertib, RP-6306, GSK-1520489A, and analogues thereof.

In preferred embodiments, at least two of D₁, D₂, and D₃ are drugs targeting the DNA Damage Response (DDR) or "synthetic lethality" related pathways. In preferred embodiments, at least two of D₁, D₂, and D₃ are selected from: Rucaparib, Niraparib, Veliparib, A-966492, Talazoparib, AZD5305, Venadaparib, Mefuparib, Prexasertib (LY2606368), AZD7762, Rabusertib (LY2603618), MK-8776 (SCH 900776), CHIR-124, PF-477736, CCT245737 (SRA737, PNT-737), GDC-0575 (ARRY-575), Berzosertib, Ceralasertib, RP-6306, GSK-1520489A, and analogues thereof.

In preferred embodiments, D₁, D₂, and D₃ can be selected from the exemplary combinations shown in Table A.

**Table A. Exemplary combinations of D₁, D₂, and D₃.**

| | D1 | D2 | D3 |
|---|---|---|---|
| Combination 1-1 | 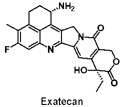 | 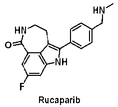 | / |
| Combination 1-2 | 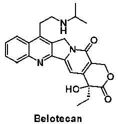 | 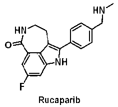 | / |
| Combination 1-3 | 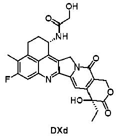 | 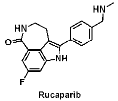 | / |
| Combination 1-4 | 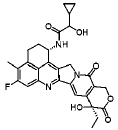 | 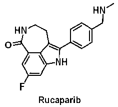 | / |
| Combination 1-5 | 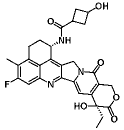 | 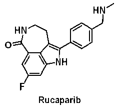 | / |
| Combination 1-6 | 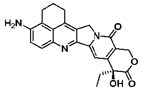 | 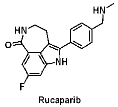 | / |
| Combination 1-7 | 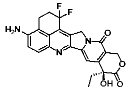 | 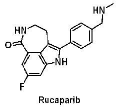 | / |
| Combination 1-8 | 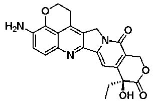 | 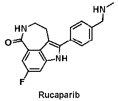 | / |
| Combination 1-9 | 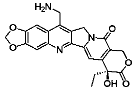 | 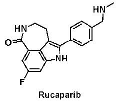 | / |
| Combination 1-10 | 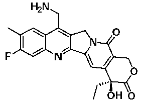 | 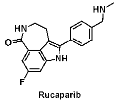 | / |
| Combination 1-11 | 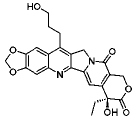 | 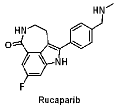 | / |
| Combination 1-12 | 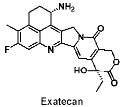 | 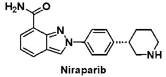 | / |
| Combination 1-13 | 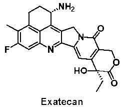 | 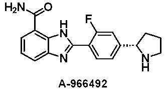 | / |
| Combination 1-14 | 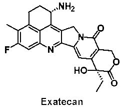 | 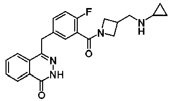 | / |
| Combination 1-15 | 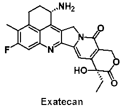 | 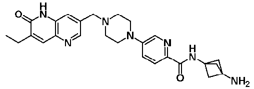 | / |
| Combination 1-16 | 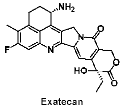 | 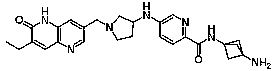 | / |
| Combination 1-17 | 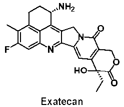 | 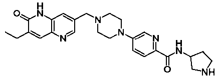 | / |
| Combination 1-18 | 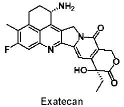 | 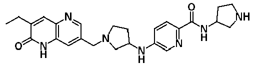 | / |
| Combination 1-19 | 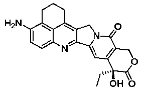 | 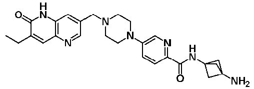 | / |
| Combination 2-1 | 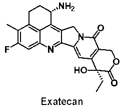 | 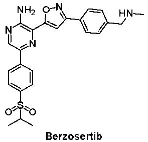 | / |
| Combination 2-2 | 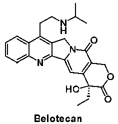 | 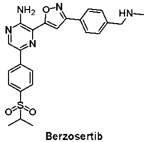 | / |
| Combination 2-3 | 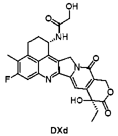 | 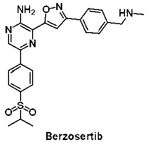 | / |
| Combination 2-4 | 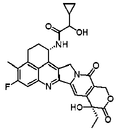 | 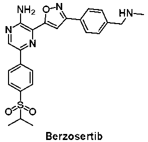 | / |
| Combination 2-5 | 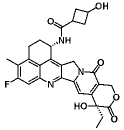 | 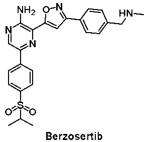 | / |
| Combination 2-6 | 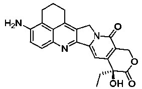 | 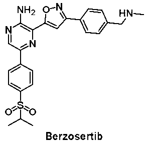 | / |
| Combination 2-7 | 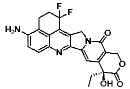 | 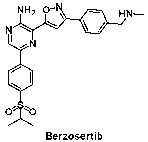 | / |
| Combination 3-1 | 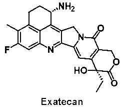 | 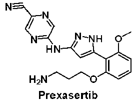 | / |
| Combination 3-2 | 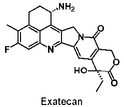 | 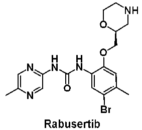 | / |
| Combination 3-3 | 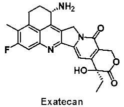 | 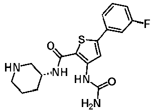 | / |
| Combination 3-4 | 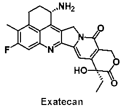 | 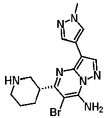 | / |
| Combination 3-5 | 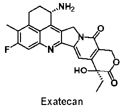 | 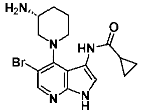 | / |
| Combination 3-6 | 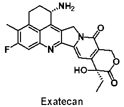 | 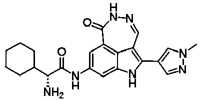 | / |
| Combination 3-7 | 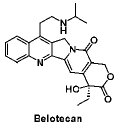 | 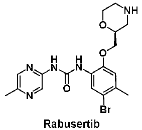 | / |
| Combination 3-8 | 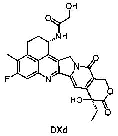 | 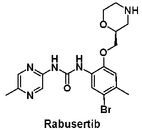 | / |
| Combination 3-9 | 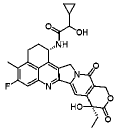 | 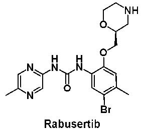 | / |
| Combination 3-10 | 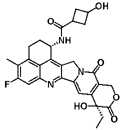 | 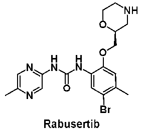 | / |
| Combination 3-11 | 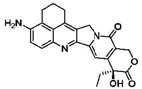 | 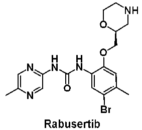 | / |
| Combination 3-12 | 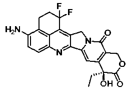 | 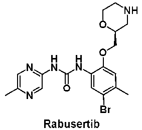 | / |
| Combination 9-1 | 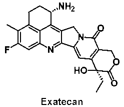 | 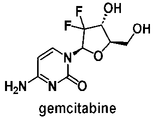 | / |
| Combination 11-1 | 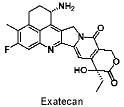 | 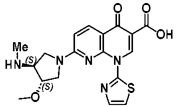 | / |
| Combination 17-1 | 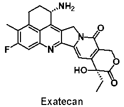 | 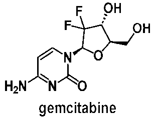 | / |
| Combination 4-1 | 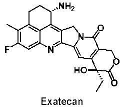 | 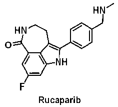 | 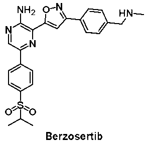 |
| Combination 5-1 | 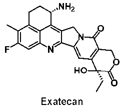 | 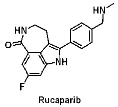 | 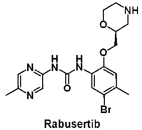 |
| Combination 5-2 | 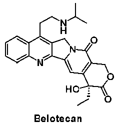 | 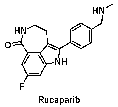 | 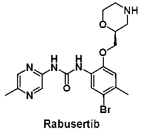 |
| Combination 13-1 | 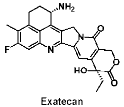 | 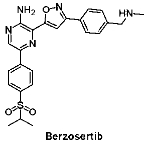 | 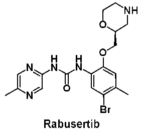 |
| Combination 4-2 | 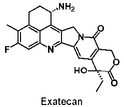 | 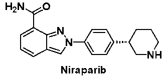 | 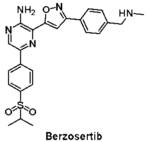 |
| Combination 13-1 | 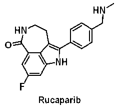 | 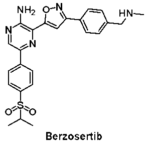 | 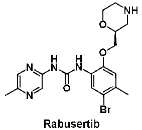 |
| Combination 13-2 | 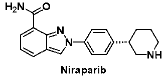 | 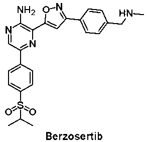 | 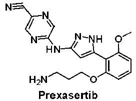 |
| Combination 14-1 | 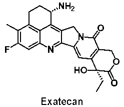 | 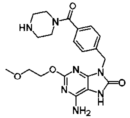 | / |
| Combination 14-2 | 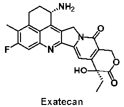 | 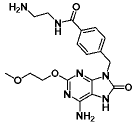 | / |
| Combination 14-3 | 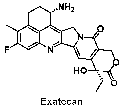 | 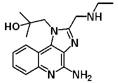 | / |
| Combination 14-4 | 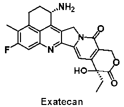 | 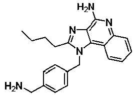 | / |
| Combination 14-5 | 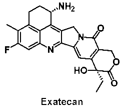 | 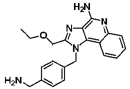 | / |
| Combination 14-6 | 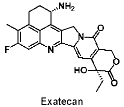 | 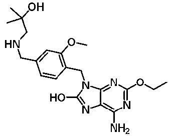 | |
| Combination 14-7 | 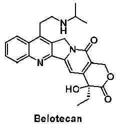 | 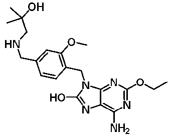 | |
| Combination 14-8 | 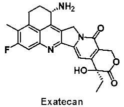 | 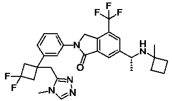 | / |
| Combination 14-9 | 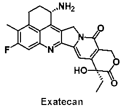 | 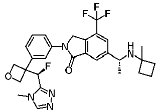 | / |
| Combination 14-10 | 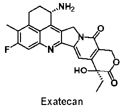 | 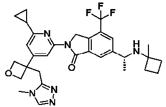 | / |
| Combination 14-11 | 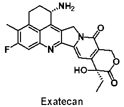 | 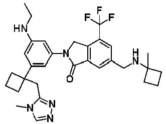 | / |
| Combination 14-12 | 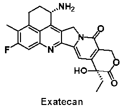 | 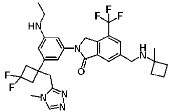 | / |
| Combination 15-1 | 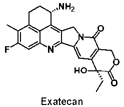 | 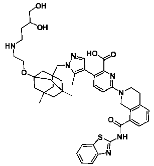 | / |
| Combination 15-2 | 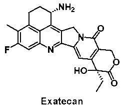 | 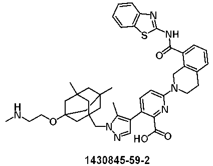 | / |
| Combination 15-3 | 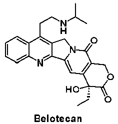 | 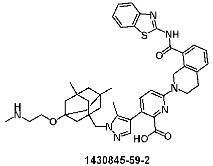 | / |
| Combination 27-1 | 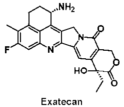 | 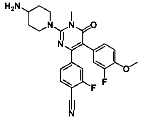 | |
| Combination 27-2 | 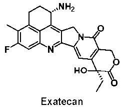 | 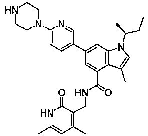 | / |
| Combination 26-1 | 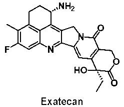 | 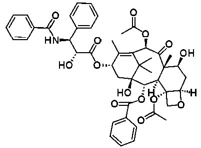 | / |
| Combination 61-1 | 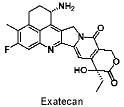 | 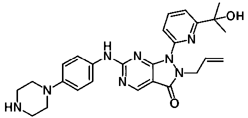 | / |
| Combination 8-1 | 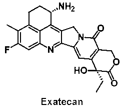 | 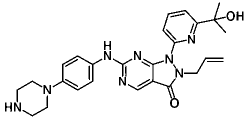 | 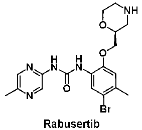 |
| Combination 8-2 | 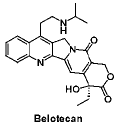 | 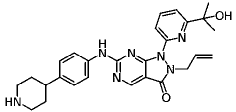 | 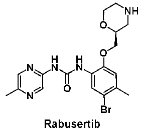 |
| Combination 62 | 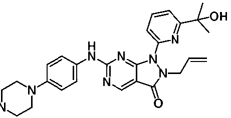 | 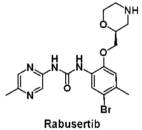 | / |
| Combination 63 | 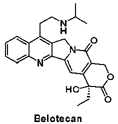 | 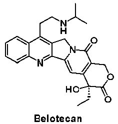 | / |
| Combination 63 | 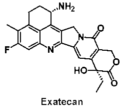 | 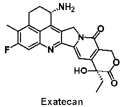 | / |
| Combination 71-1 | 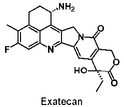 | 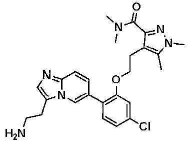 | / |
| Combination 71-2 | 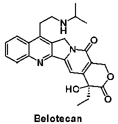 | 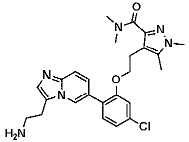 | / |
| Combination 71-3 | 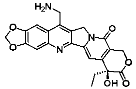 | 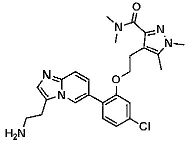 | / |
| Combination 71-4 | 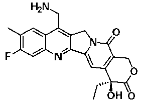 | 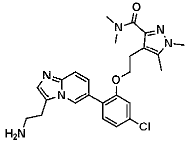 | / |
| Combination 72-1 | 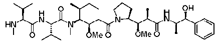 | 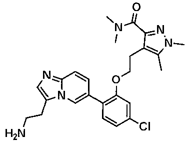 | / |
| Combination 72-2 | 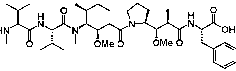 | 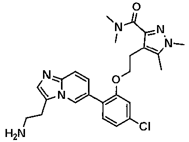 | / |
| Combination 72-3 | 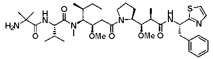 | 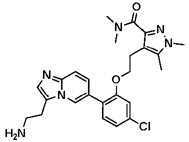 | / |
| Combination 72-4 | 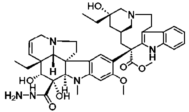 | 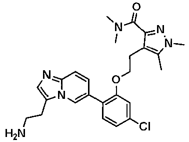 | / |

In the present invention, the embodiments or preferred embodiments of each aforementioned component (such as Q, C₁, B₁, B₂, T₁, T₂, T₃, P₁, P₂, P₃, D₁, D₂, D₃, hydrophilic group) may be individually applicable to formula I, Ia, or Ib, or applicable in various Combinations to formula I, Ia, or Ib. When the combination is applicable, the embodiments or preferred embodiments of each component may be combined in whole or in part.

In preferred embodiments, the present invention provides compounds selected from formulas **A1** to **A190,** or a tautomer, a mesomer, a racemate, an enantiomer, or a diastereomer thereof, or a deuterated compound form thereof, or a mixture form thereof, or a pharmaceutically acceptable salt, prodrug, or solvate thereof.

| Structure | Number | Drug Combination |
|---|---|---|
| 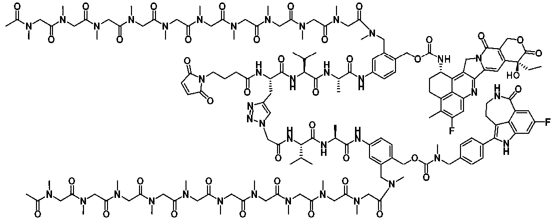 | Formula A1 | Combination 1 |
| 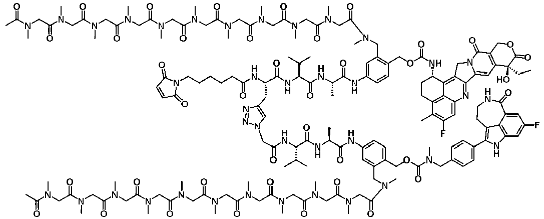 | Formula A2 | Combination 1 |
| 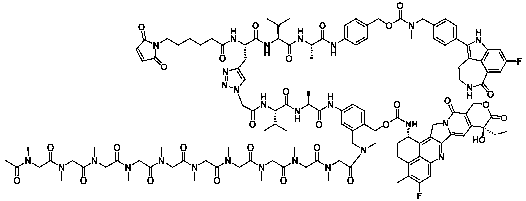 | Formula A3 | Combination 1 |
| 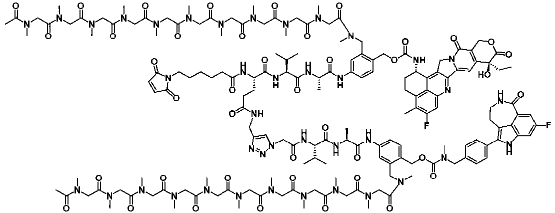 | Formula A4 | Combination 1 |
| 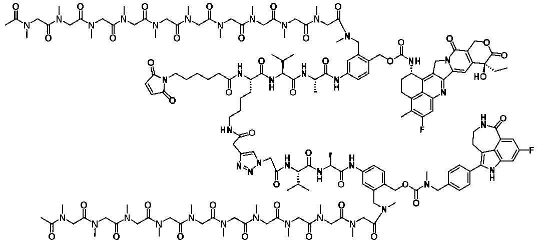 | Formula A5 | Combination 1 |
| 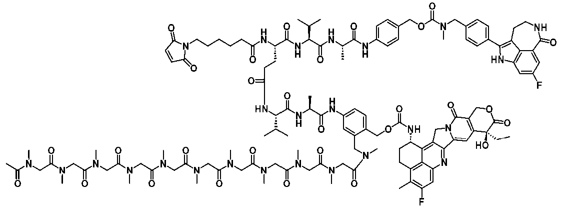 | Formula A6 | Combination 1 |
| 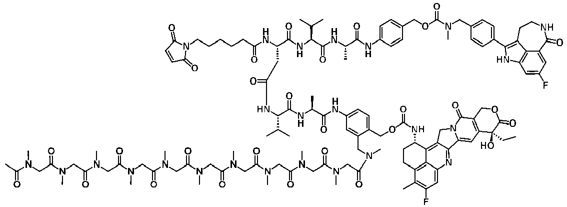 | Formula A7 | Combination 1 |
| 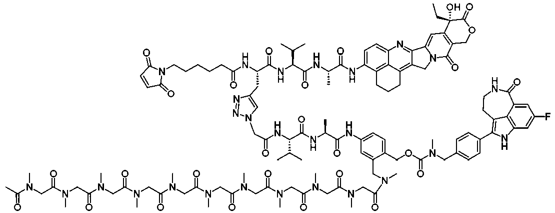 | Formula A8 | Combination 1 |
| 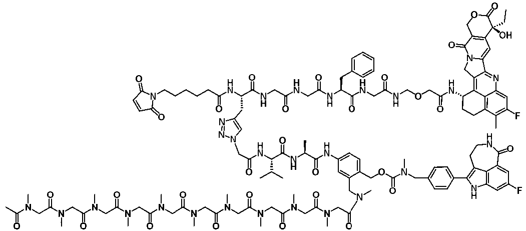 | Formula A9 | Combination 1 |
| 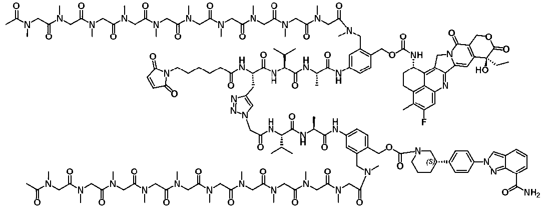 | Formula A10 | Combination 1 |
| 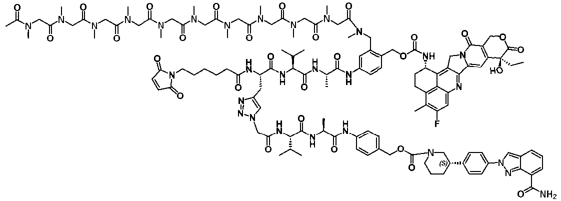 | Formula A11 | Combination 1 |
| 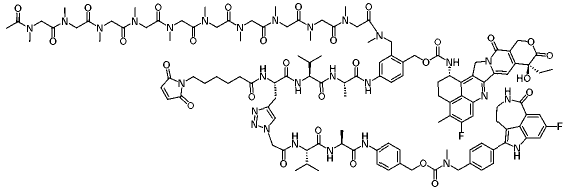 | Formula A12 | Combination 1 |
| 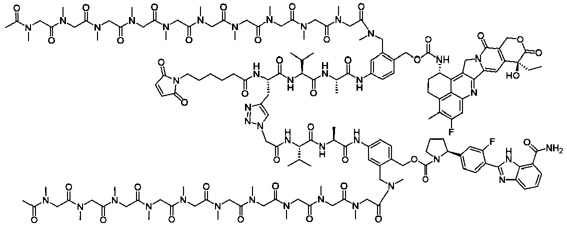 | **Formula** A13 | Combination 1 |
| 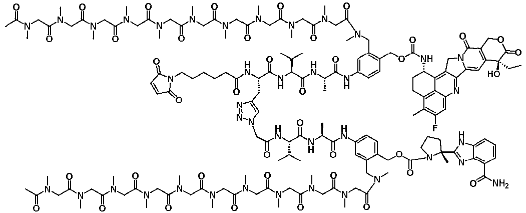 | Formula A14 | Combination 1 |
| 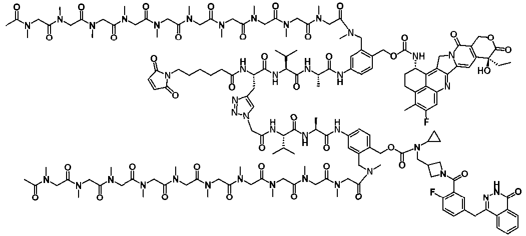 | Formula A15 | Combination 1 |
| 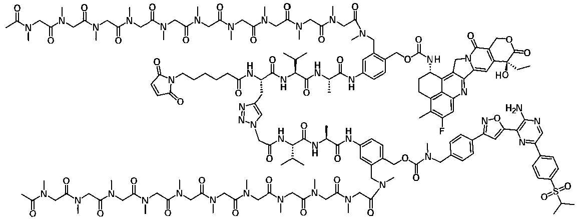 | Formula A16 | Combination 2 |
| 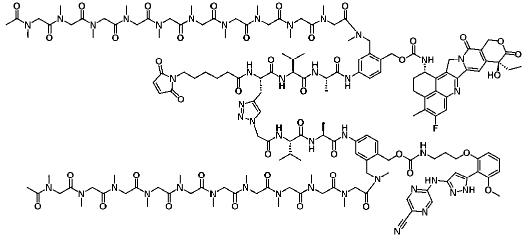 | Formula A17 | Combination 3 |
| 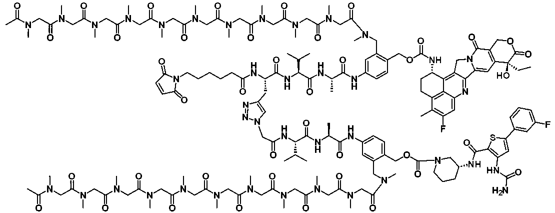 | Formula A18 | Combination 3 |
| 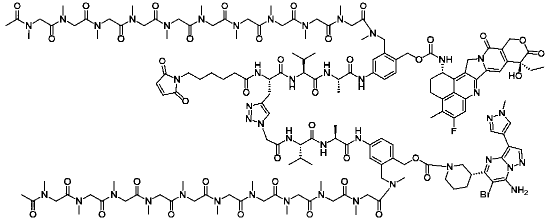 | Formula A19 | Combination 3 |
| 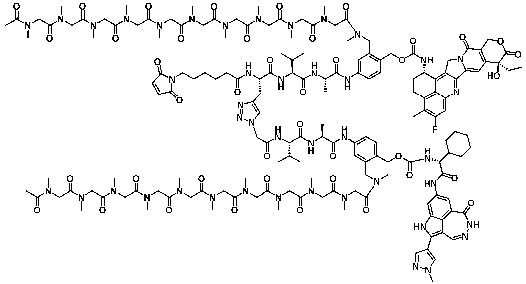 | Formula A20 | Combination 3 |
| 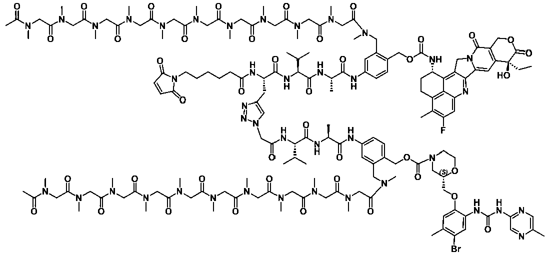 | Formula A21 | Combination 3 |
| 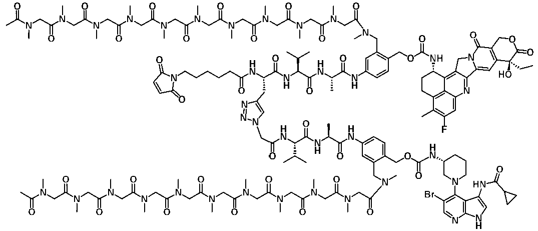 | Formula A22 | Combination 3 |
| 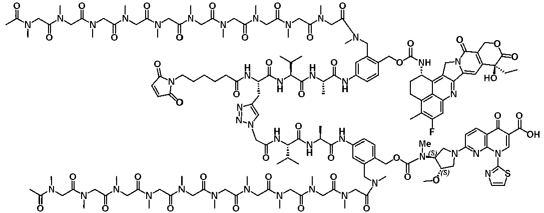 | Formula A23 | Combination 11 |
| 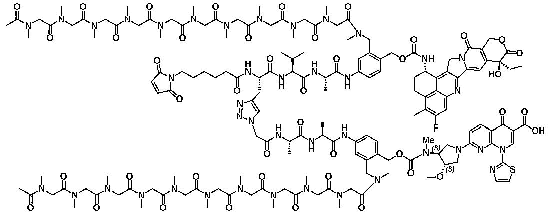 | Formula A24 | Combination 11 |
| 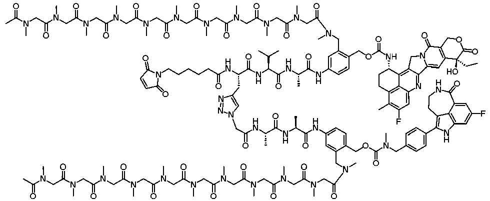 | Formula A25 | Combination 1 |
| 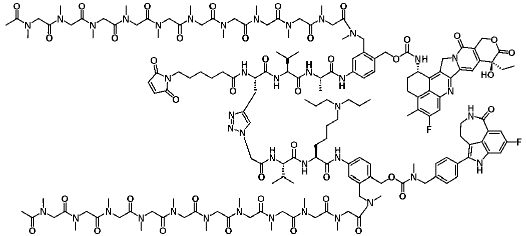 | Formula A26 | Combination 1 |
| 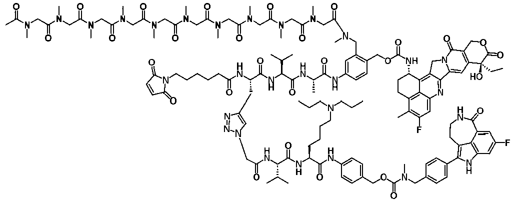 | Formula A27 | Combination 1 |
| 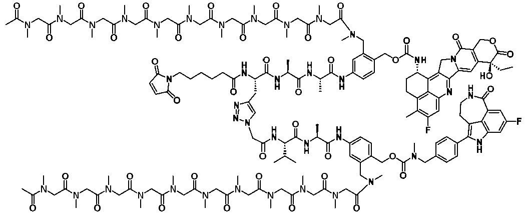 | Formula A28 | Combination 1 |
| 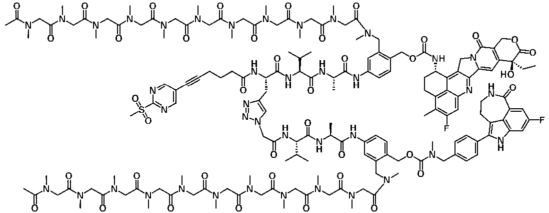 | Formula A29 | Combination 1 |
| 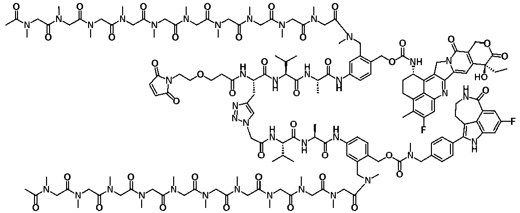 | Formula A30 | Combination 1 |
| 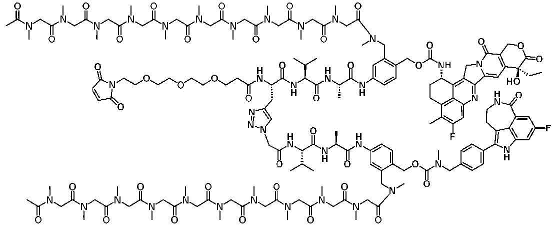 | Formula A31 | Combination 1 |
| 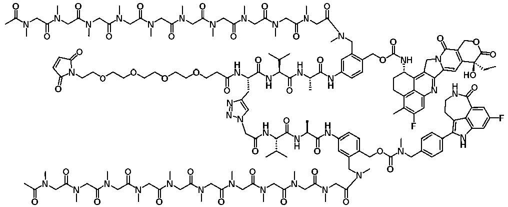 | Formula A32 | Combination 1 |
| 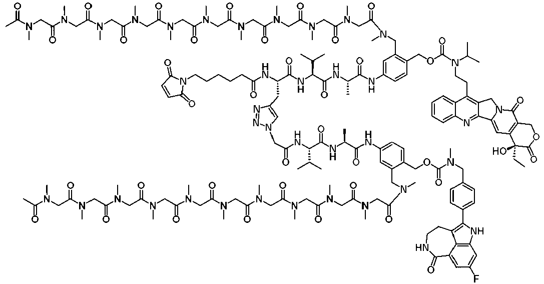 | Formula A33 | Combination 1 |
| 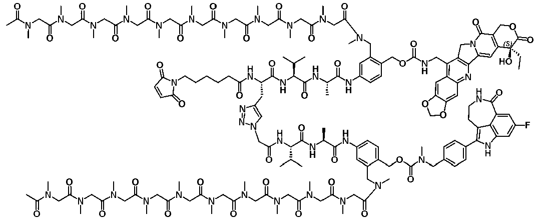 | Formula A34 | Combination 1 |
| 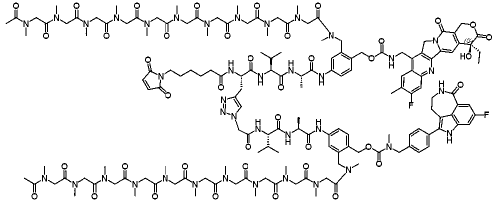 | Formula A35 | Combination 1 |
| 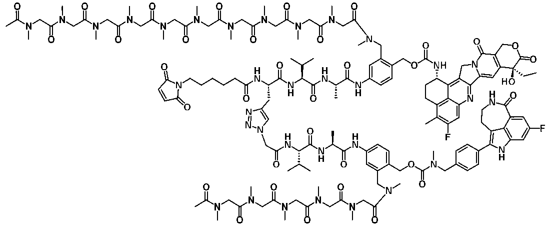 | Formula A36 | Combination 1 |
| 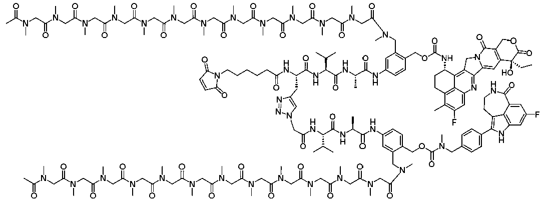 | Formula A37 | Combination 1 |
| 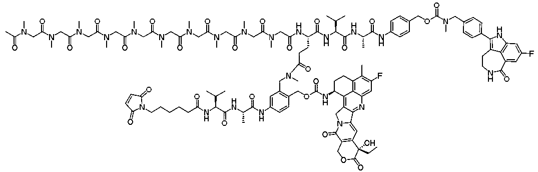 | Formula A38 | Combination 1 |
| 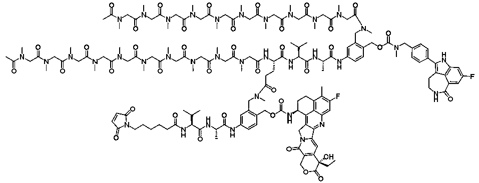 | Formula A39 | Combination 1 |
| 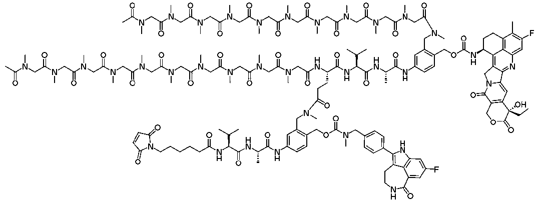 | Formula A40 | Combination 1 |
| 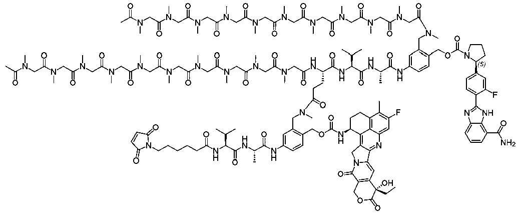 | Formula A41 | Combination 1 |
| 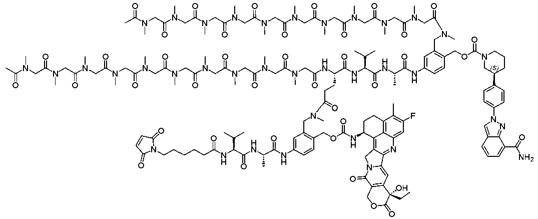 | Formula A42 | Combination 1 |
| 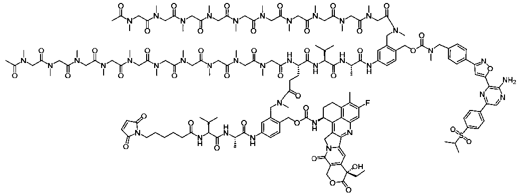 | Formula A43 | Combination 2 |
| 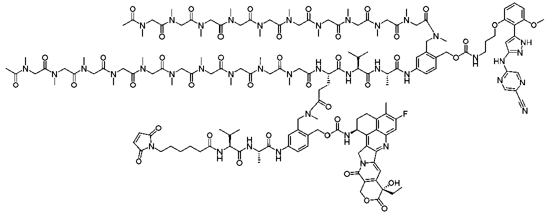 | Formula A44 | Combination 3 |
| 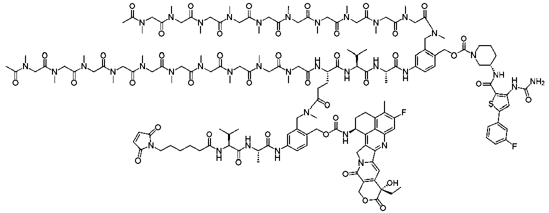 | Formula A45 | Combination 3-3 |
| 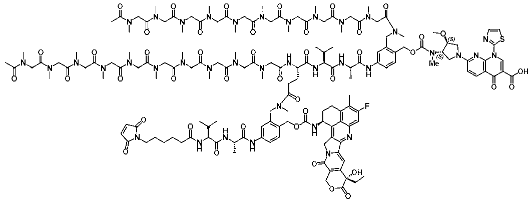 | Formula A46 | Combination 11 |
| 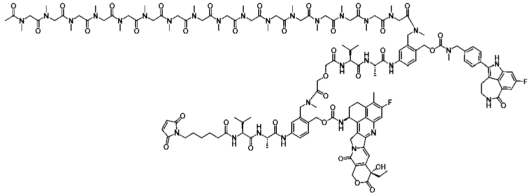 | Formula A47 | Combination 1 |
| 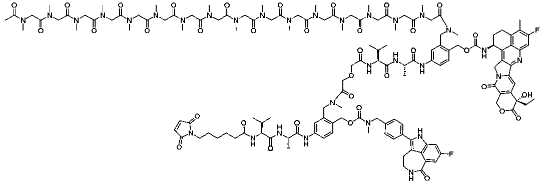 | Formula A48 | Combination 1 |
| 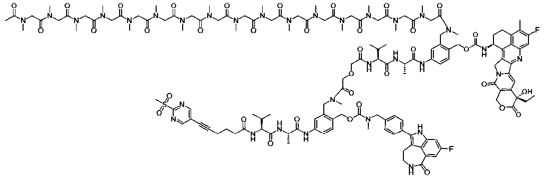 | Formula A49 | Combination 1-1 |
| 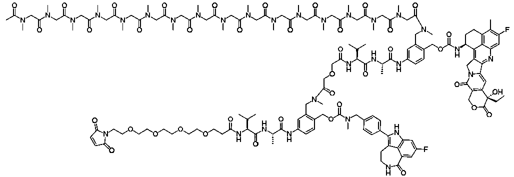 | Formula A50 | Combination 1 |
| 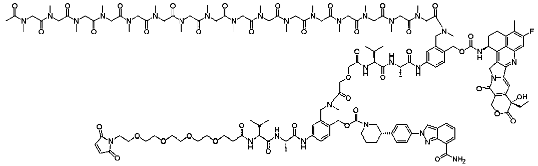 | Formula A51 | Combination 1 |
| 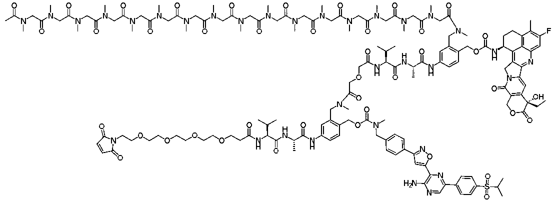 | Formula A52 | Combination 2 |
| 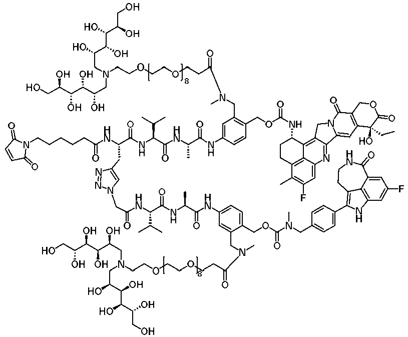 | Formula A53 | Combination 1 |
| 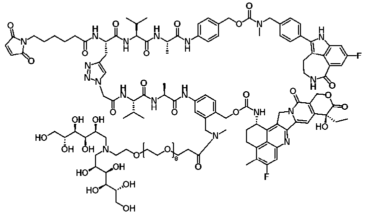 | Formula A54 | Combination 1 |
| 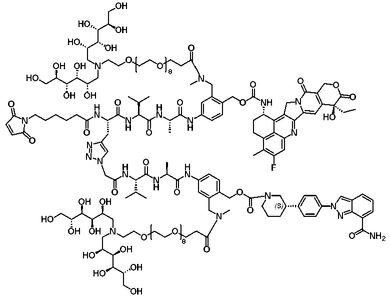 | Formula A55 | Combination 1 |
| 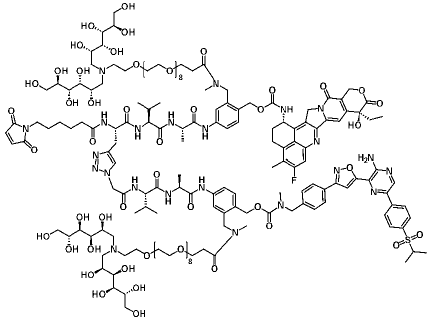 | Formula A56 | Combination 2 |
| 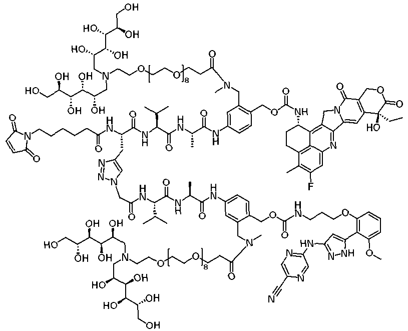 | Formula A57 | Combination 3 |
| 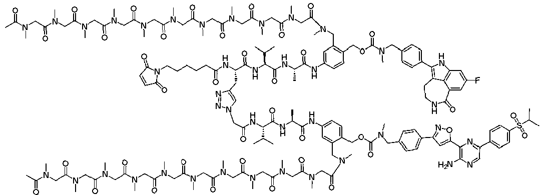 | Formula A58 | Combination 12 |
| 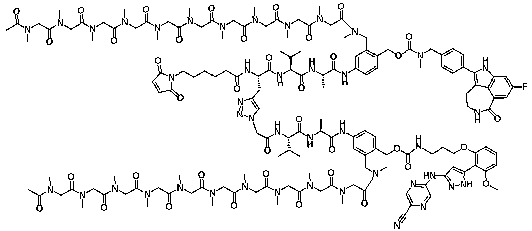 | Formula A59 | Combination 12 |
| 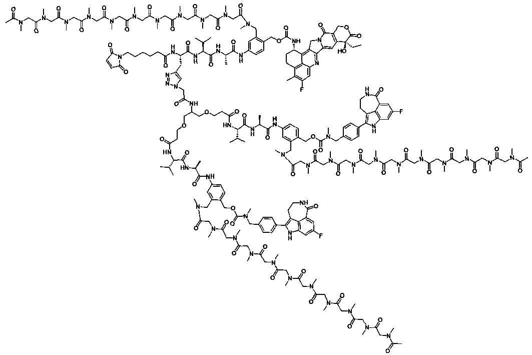 | Formula A60 | Combination 1 |
| 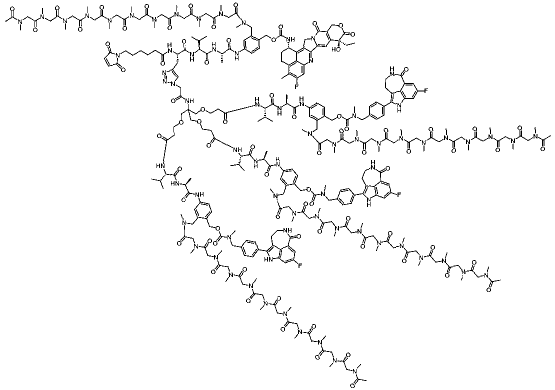 | Formula A61 | Combination 1 |
| 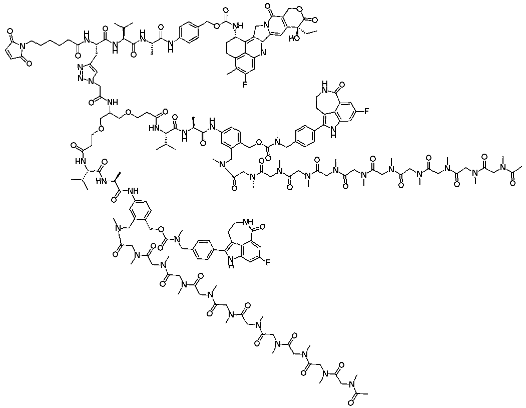 | Formula A62 | Combination 1 |
| 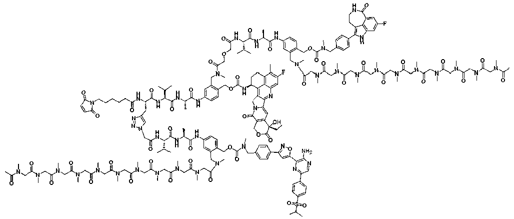 | Formula A63 | Combination 4 |
| 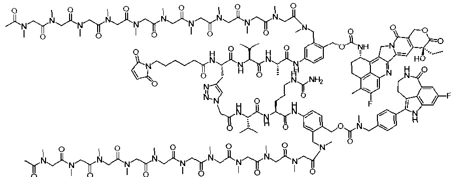 | Formula A64 | Combination 1 |
| 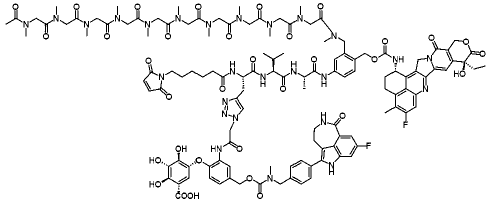 | Formula A65 | Combination 1 |
| 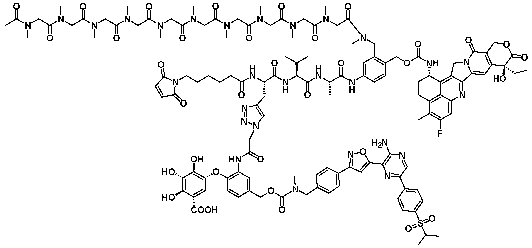 | Formula A66 | Combination 2 |
| 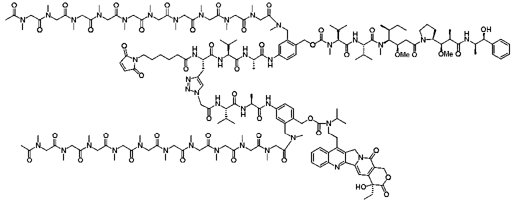 | Formula A67 | Combination 26 |
| 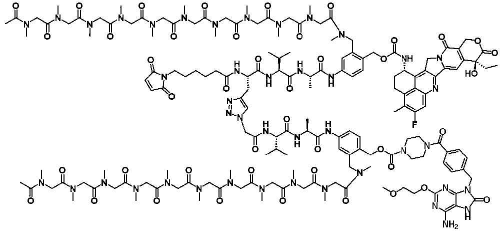 | Formula A68 | Combination 14 |
| 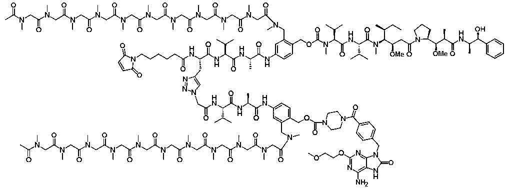 | Formula A69 | Combination 28 |
| 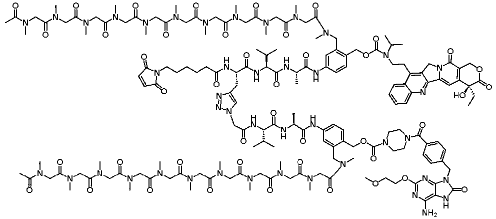 | Formula A70 | Combination 14 |
| 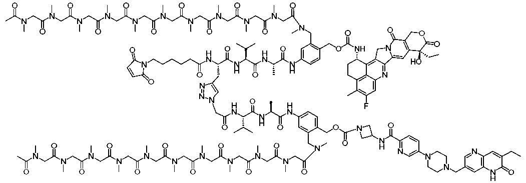 | Formula A71 | Combination 1 |
| 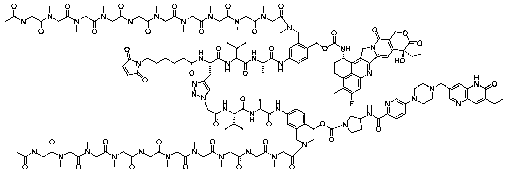 | Formula A72 | Combination 1 |
| 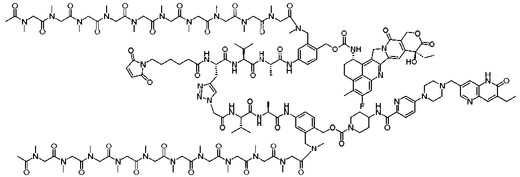 | Formula A73 | Combination 1 |
| 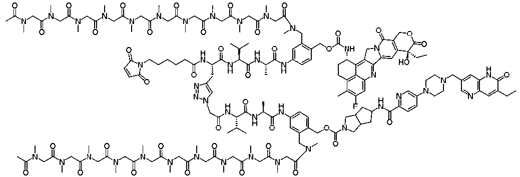 | Formula A74 | Combination 1 |
| 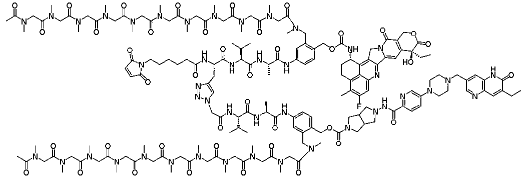 | Formula A75 | Combination 1 |
| 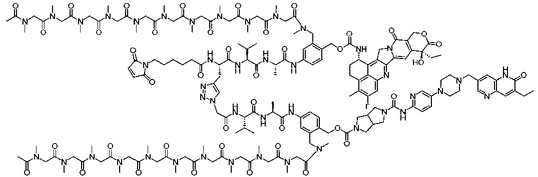 | Formula A76 | Combination 1 |
| 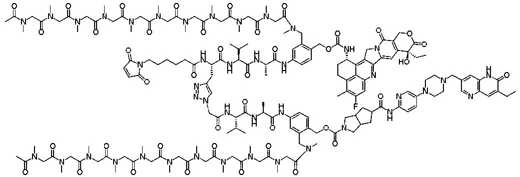 | Formula A77 | Combination 1 |
| 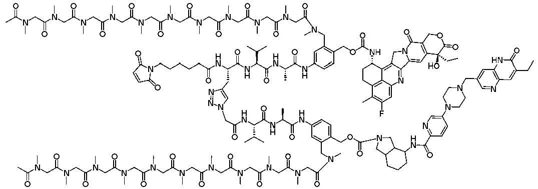 | Formula A78 | Combination 1 |
| 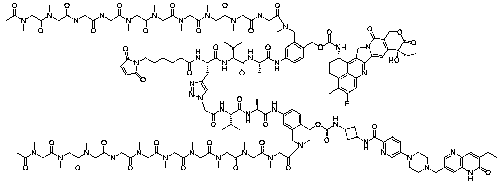 | Formula A79 | Combination 1 |
| 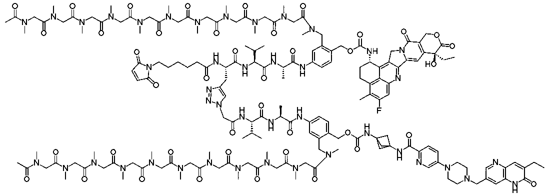 | Formula A80 | Combination 1 |
| 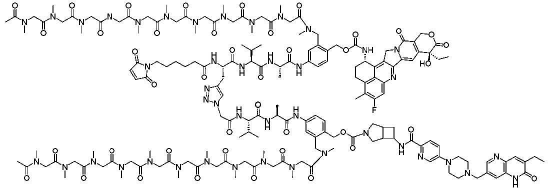 | Formula A81 | Combination 1 |
| 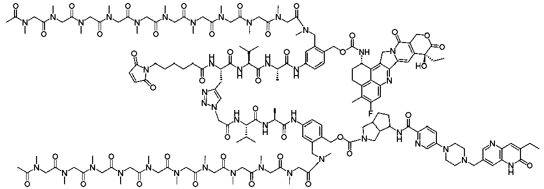 | Formula A82 | Combination 1 |
| 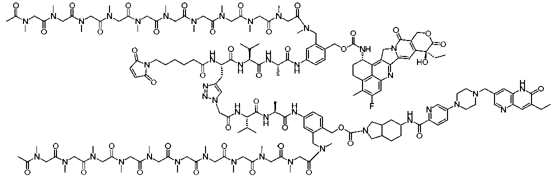 | Formula A83 | Combination 1 |
| 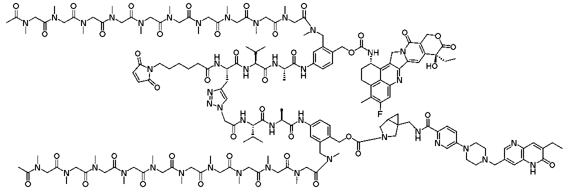 | Formula A84 | Combination 1 |
| 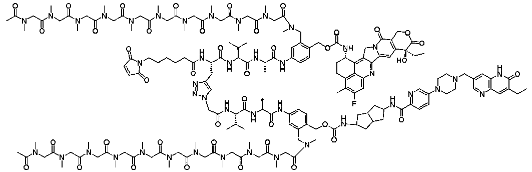 | Formula A85 | Combination 1 |
| 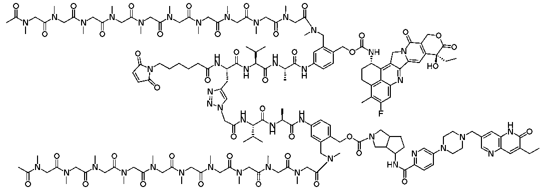 | Formula A86 | Combination 1 |
| 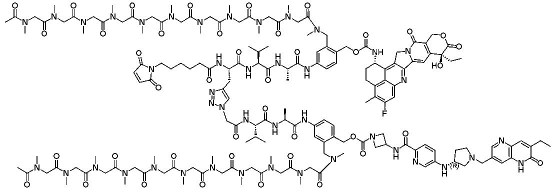 | Formula A87 | Combination 1 |
| 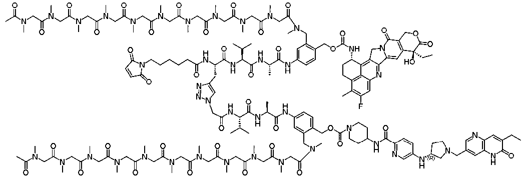 | Formula A88 | Combination 1 |
| 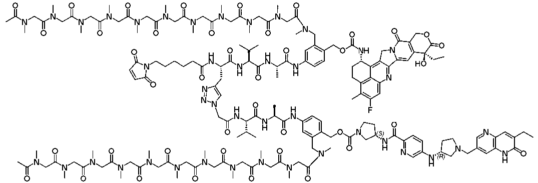 | Formula A89 | Combination 1 |
| 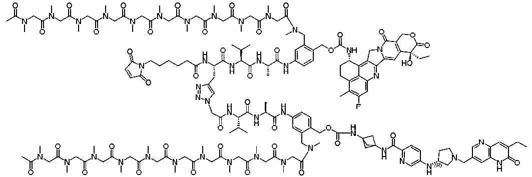 | Formula A90 | Combination 1 |
| 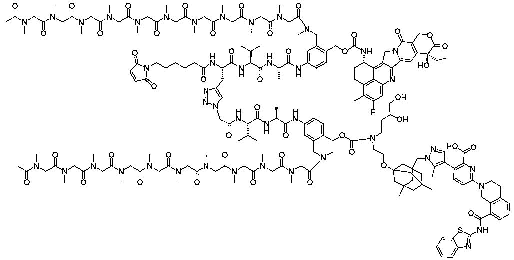 | Formula A91 | Combination 15 |
| 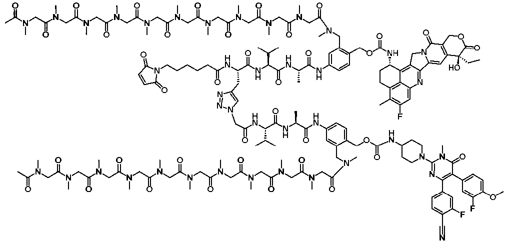 | Formula A92 | Combination 27 |
| 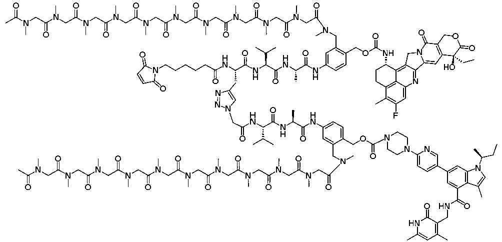 | Formula A93 | Combination 27 |
| 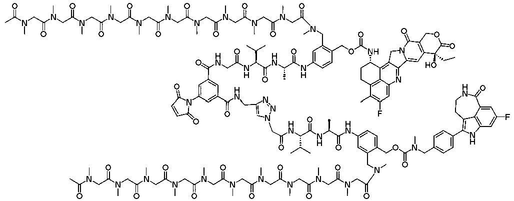 | Formula A94 | Combination 1 |
| 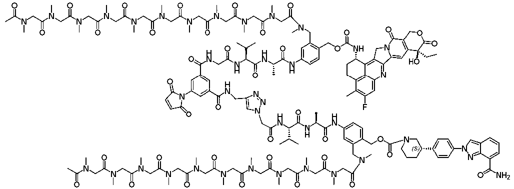 | Formula A95 | Combination 1 |
| 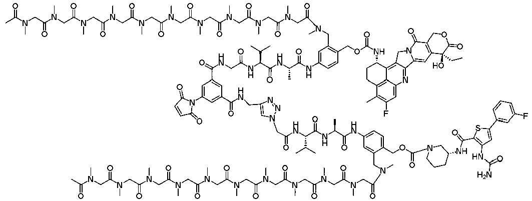 | Formula A96 | Combination 3 |
| 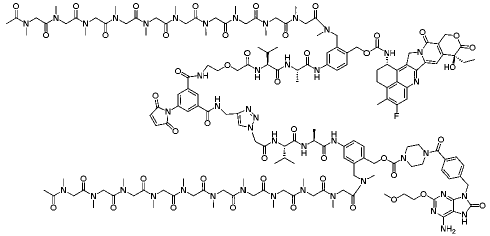 | Formula A97 | Combination 14 |
| 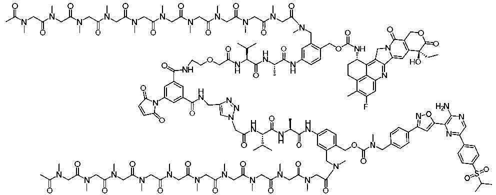 | Formula A98 | Combination 2 |
| 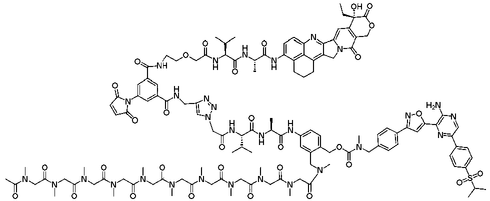 | Formula A99 | Combination 2 |
| 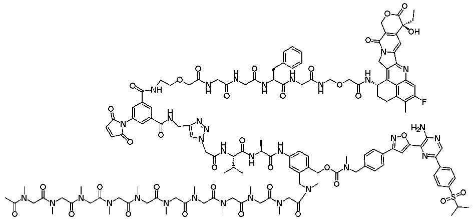 | Formula A100 | Combination 2 |
| 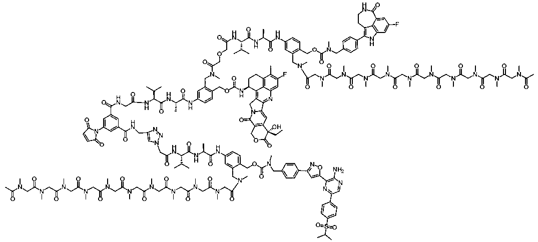 | Formula A101 | Combination 2 |
| 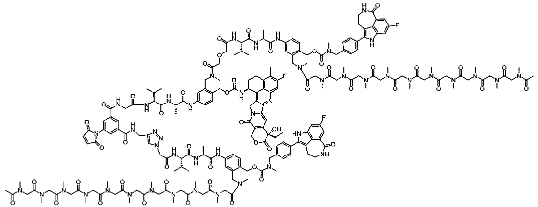 | Formula A102 | Combination 1 |
| 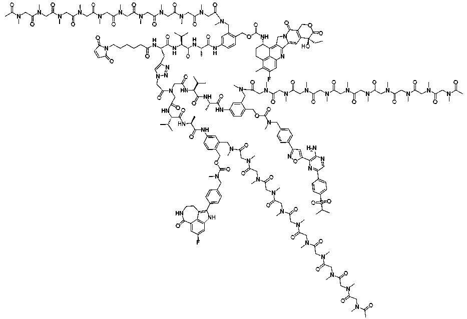 | Formula A103 | Combination 4 |
| 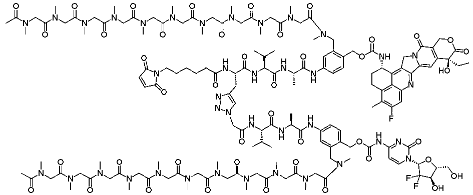 | Formula A104 | Combination 17 |
| 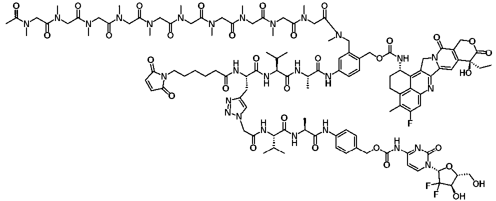 | Formula A105 | Combination 17 |
| 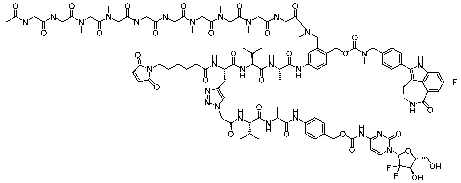 | Formula A106 | / |
| 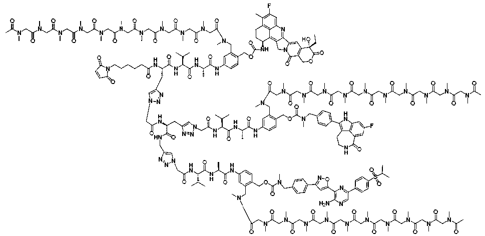 | Formula A107 | Combination 4 |
| 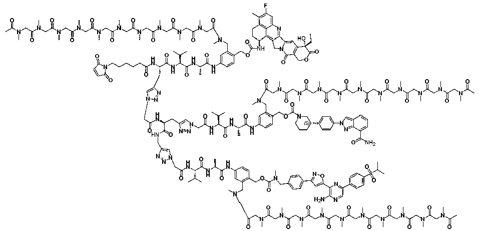 | Formula A108 | Combination 4 |
| 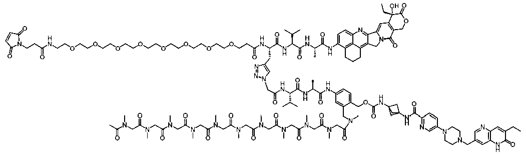 | Formula A109 | Combination 1 |
| 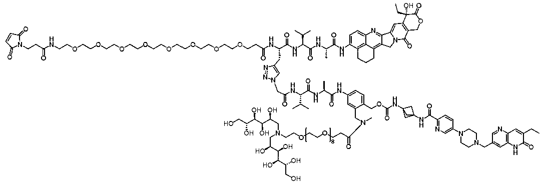 | Formula A110 | Combination 1 |
| 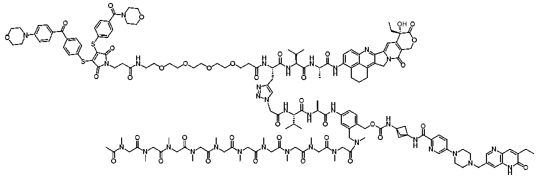 | Formula A111 | Combination 1 |
| 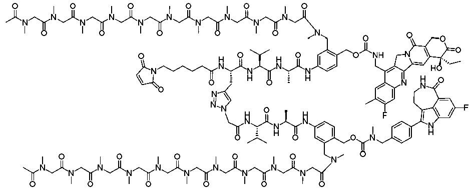 | Formula A112 | Combination 1 |
| 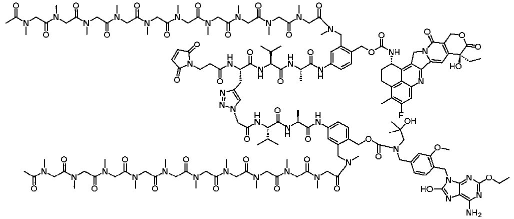 | Formula A113 | Combination 14 |
| 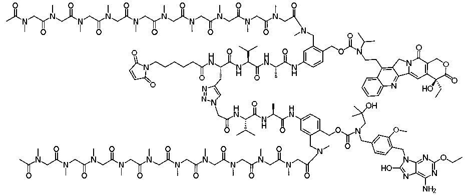 | Formula A114 | Combination 14 |
| 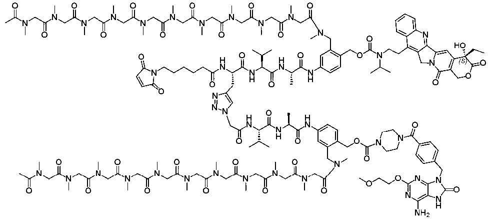 | Formula A115 | Combination 14 |
| 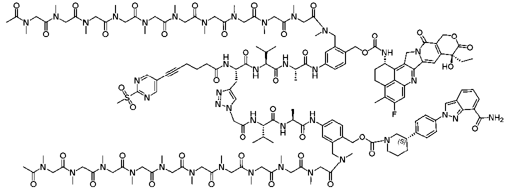 | Formula All6 | Combination 1 |
| 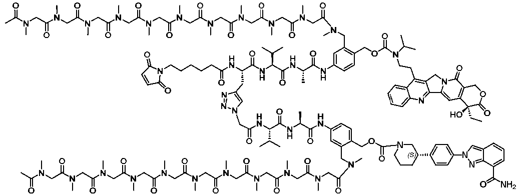 | Formula A117 | Combination 1 |
| 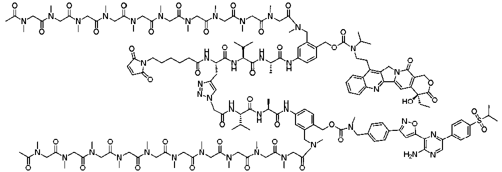 | Formula A118 | Combination 2 |
| 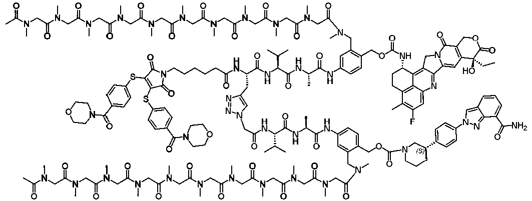 | Formula A119 | Combination 3 |
| 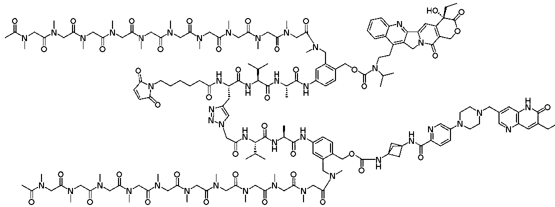 | Formula A120 | Combination 1 |
| 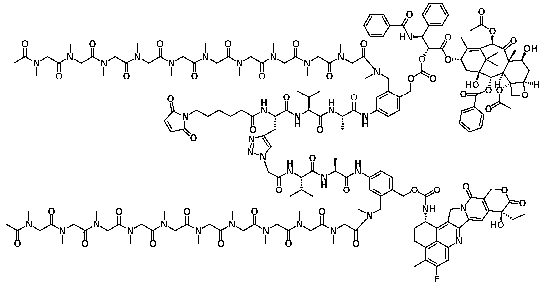 | Formula A121 | Combination 26 |
| 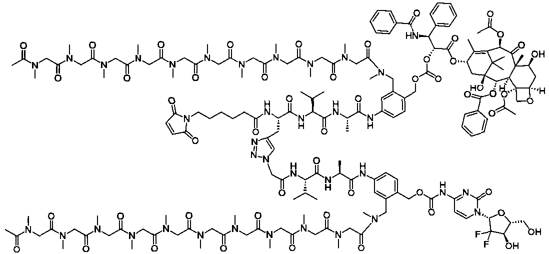 | Formula A122 | / |
| 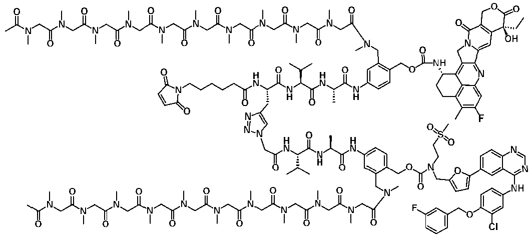 | Formula A123 | Combination 19 |
| 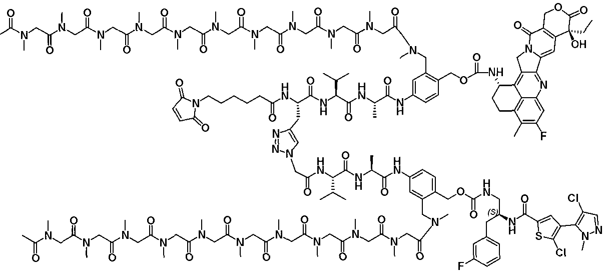 | Formula A124 | Combination 31 |
| 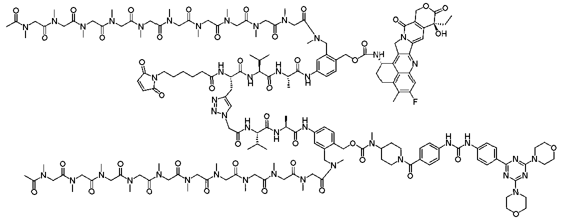 | Formula A125 | Combination 18 |
| 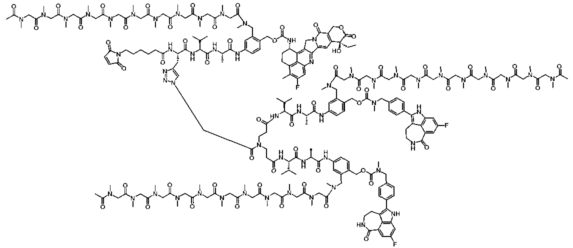 | Formula A126 | Combination 1 |
| 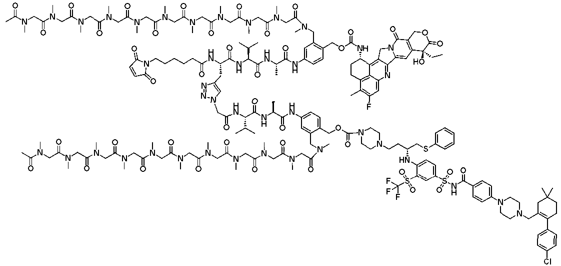 | Formula A127 | Combination 15 |
| 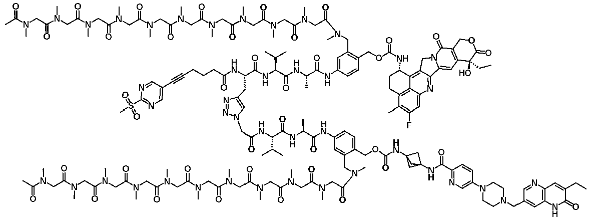 | Formula A128 | Combination 1 |
| 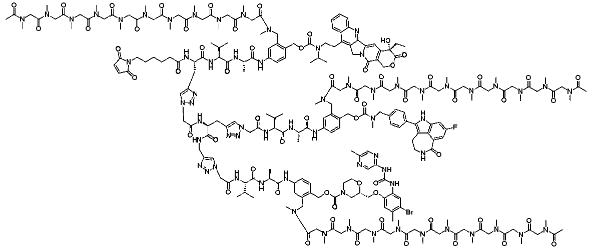 | Formula A129 | Combination 5 |
| 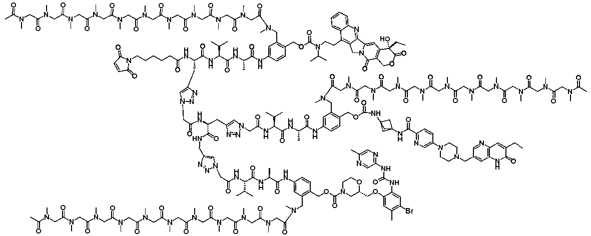 | Formula A130 | Combination 5 |
| 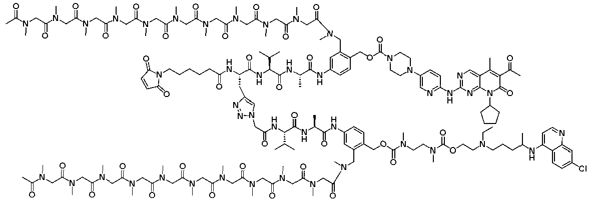 | Formula A131 | Combination 35 |
| 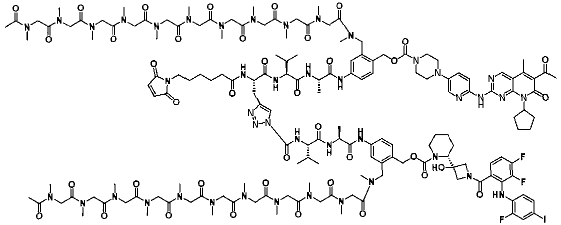 | Formula A132 | Combination 33 |
| 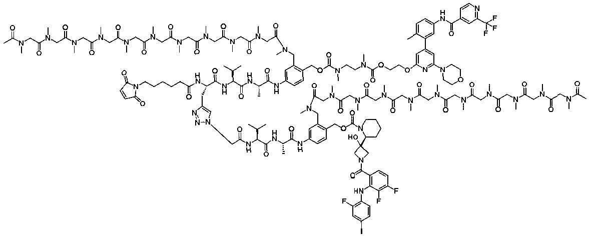 | Formula A133 | Combination 57 |
| 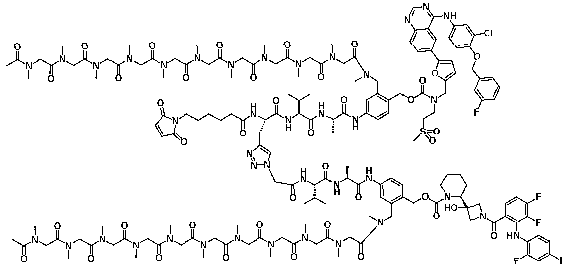 | Formula A134 | Combination 58 |
| 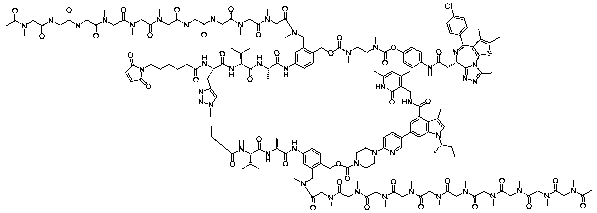 | Formula A135 | Combination 50 |
| 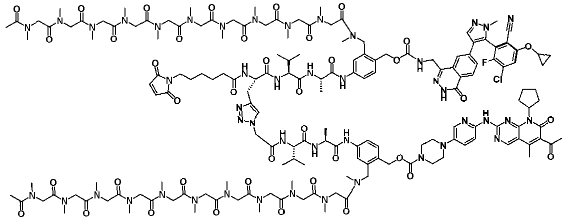 | Formula A136 | Combination 53 |
| 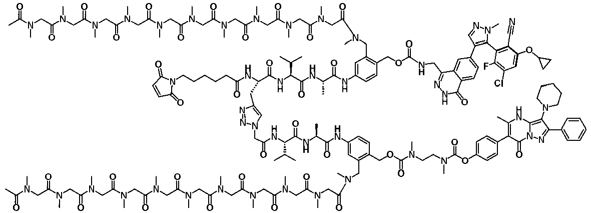 | Formula A137 | Combination 51 |
| 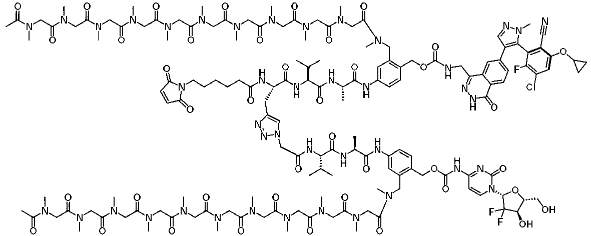 | Formula A138 | Combination 52 |
| 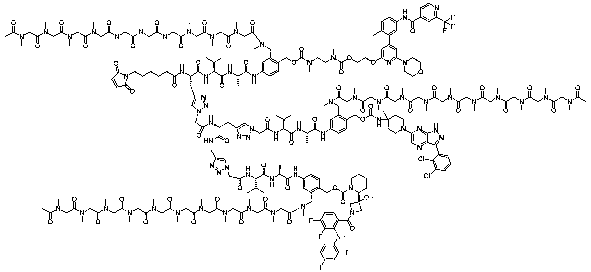 | Formula A139 | Combination 59 |
| 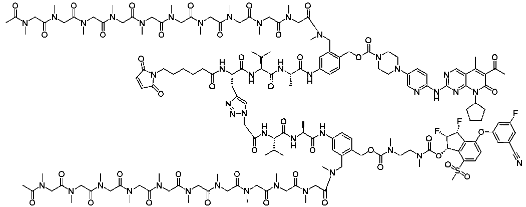 | Formula A140 | Combination 60 |
| 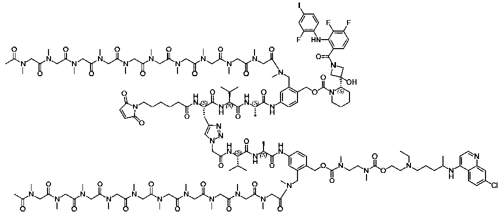 | Formula A141 | Combination 36 |
| 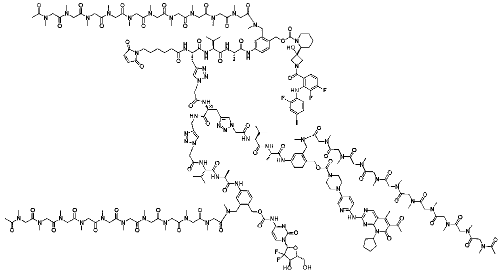 | Formula A142 | / |
| 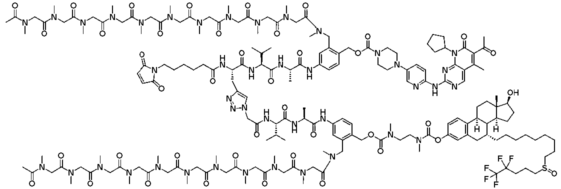 | Formula A143 | Combination 45 |
| 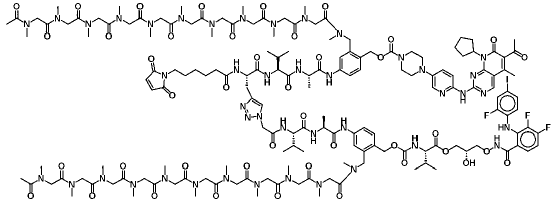 | Formula A144 | Combination 33 |
| 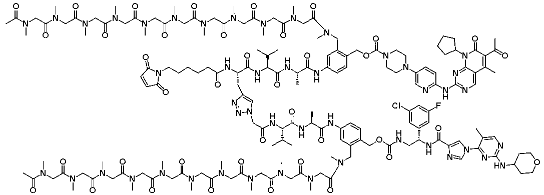 | Formula A145 | Combination 40 |
| 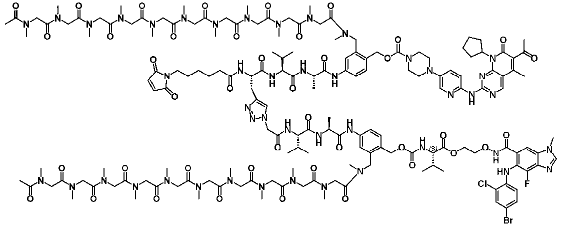 | Formula A146 | Combination 33 |
| 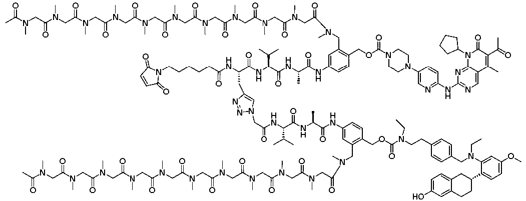 | Formula A147 | Combination 45 |
| 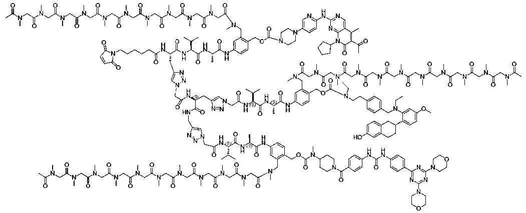 | Formula A148 | Combination 46 |
| 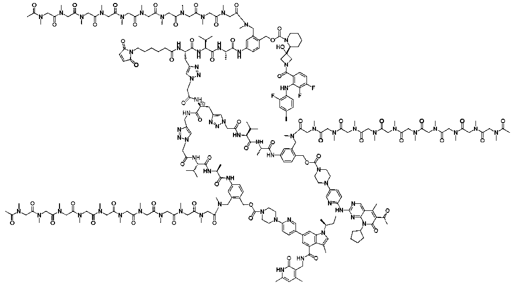 | Formula A149 | Combination 34 |
| 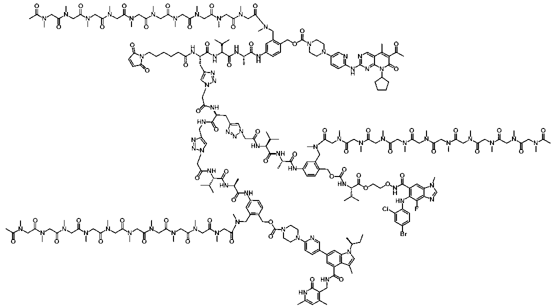 | Formula A150 | Combination 34 |
| 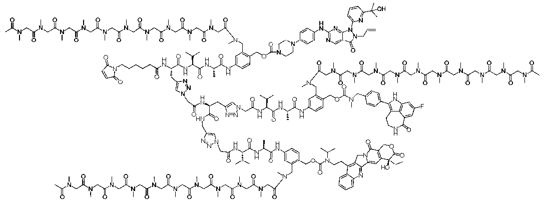 | Formula A151 | Combination 6 |
| 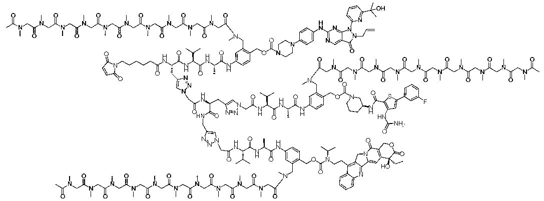 | Formula A152 | Combination 8 |
| 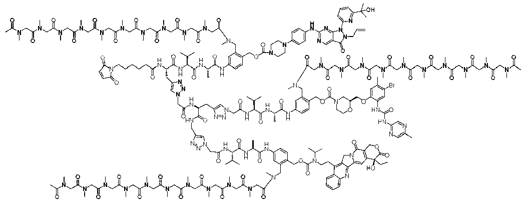 | Formula A153 | Combination 8 |
| 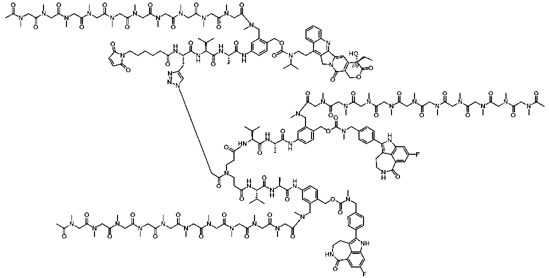 | Formula A154 | Combination 1 |
| 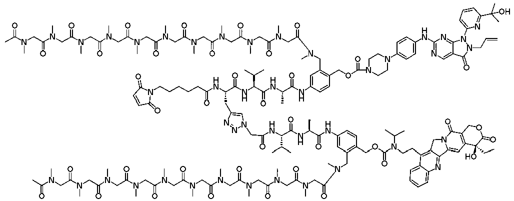 | Formula A155 | Combination 61 |
| 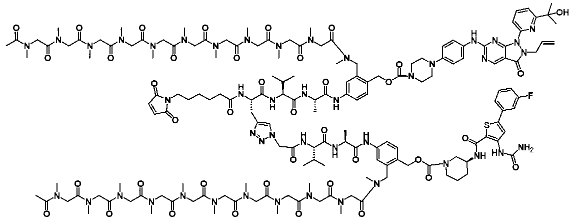 | Formula A156 | Combination 62 |
| 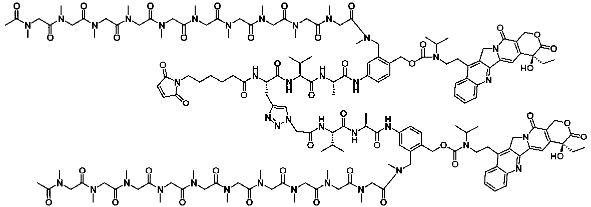 | Formula A157 | Combination 63 |
| 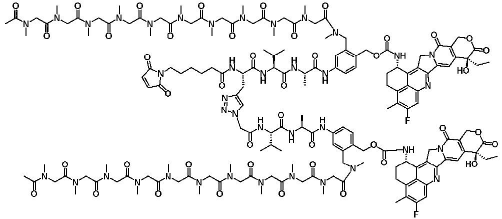 | Formula A158 | Combination 63 |
| 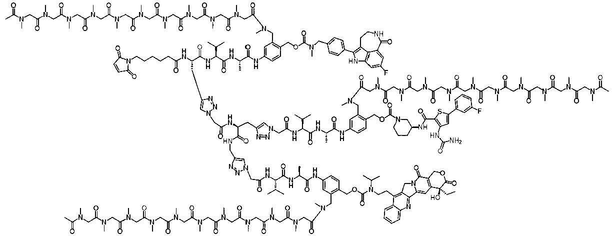 | Formula A159 | Combination 5 |
| 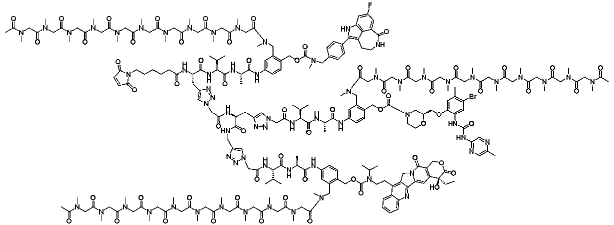 | Formula A160 | Combination 5 |
| 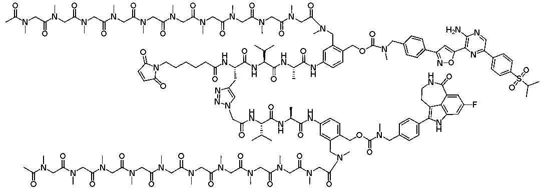 | Formula A161 | Combination 12 |
| 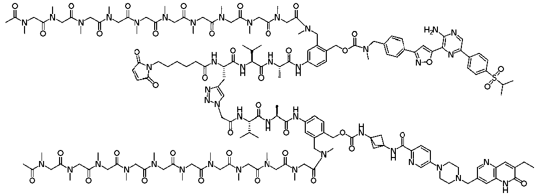 | Formula A162 | Combination 12 |
| 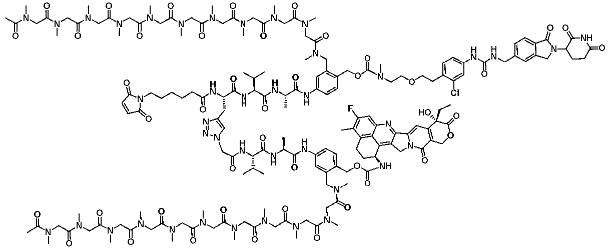 | Formula A163 | Combination 24 |
| 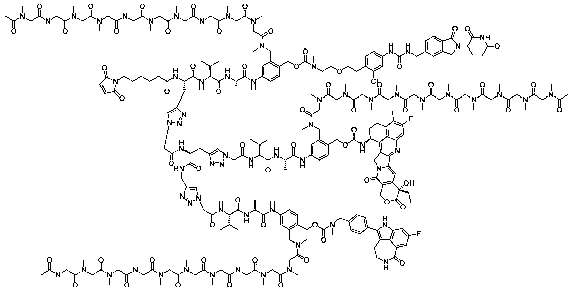 | Formula A164 | Combination 25 |
| 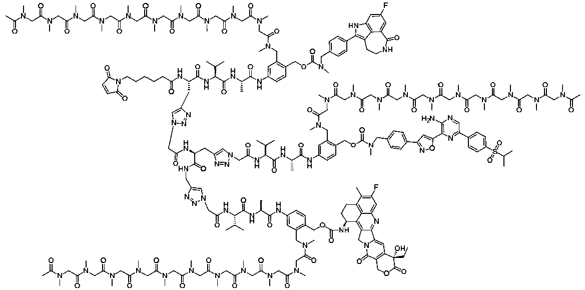 | Formula A165 | Combination 4 |
| 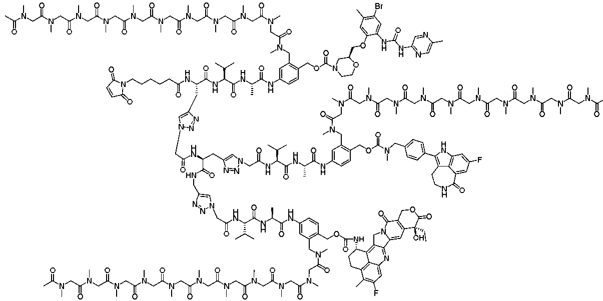 | Formula A166 | Combination 5 |
| 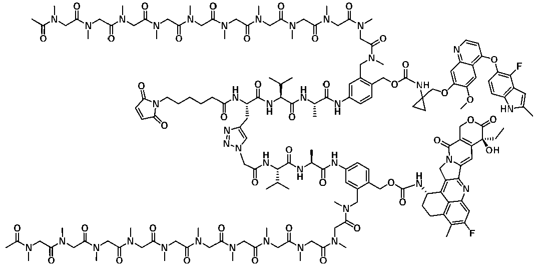 | Formula A167 | TOPO1+TK I |
| 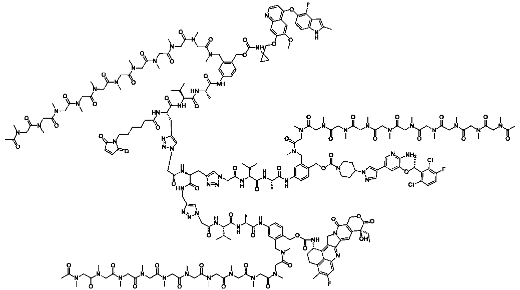 | Formula A168 | TOPO1+TKI |
| 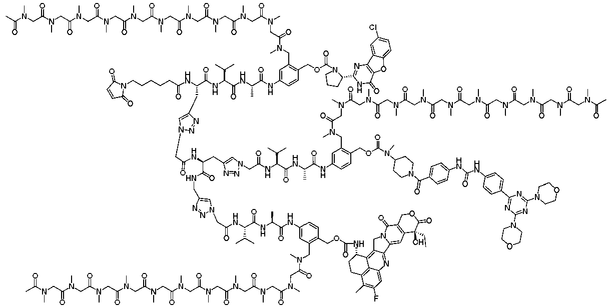 | Formula A169 | CDC7+PI3K+TO PO1 |
| 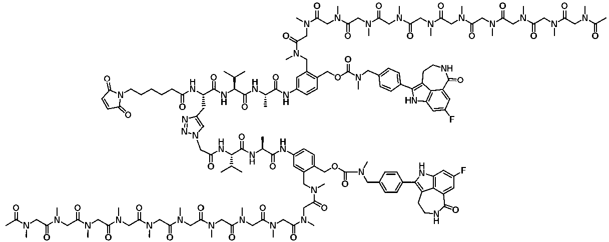 | Formula A170 | PARP+PARP |
| 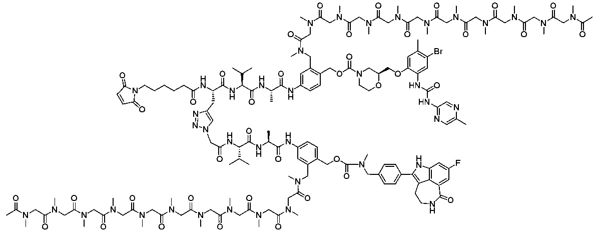 | Formula A171 | PARP+CHKI |
| 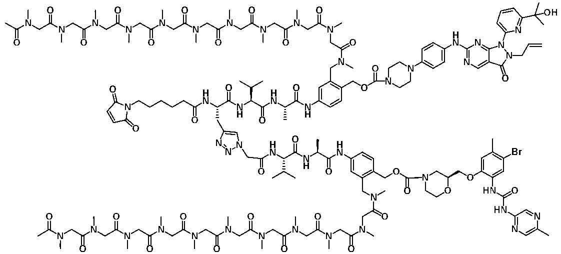 | Formula A172 | CHK1+WEE1 |
| 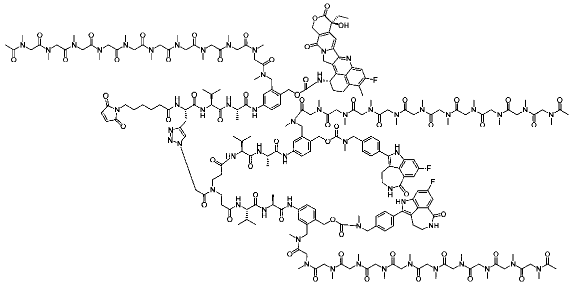 | Formula A173 | TOPO1+PARP+ PARP |
| 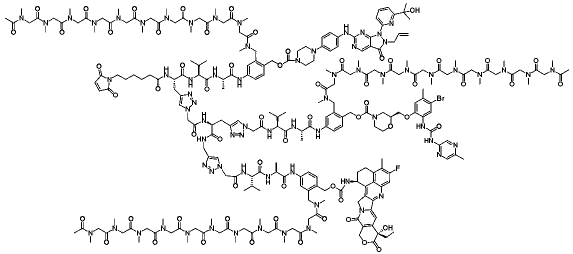 | Formula A174 | TOPO1+CHK1+ WEE1 |
| 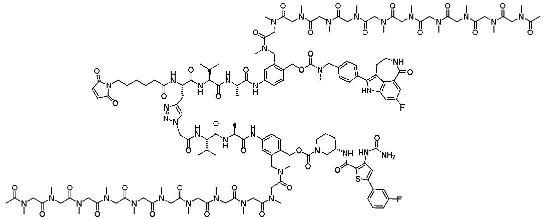 | Formula A175 | PARP+CHKI |
| 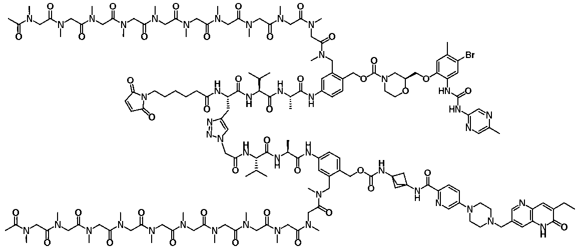 | Formula A176 | PARP+CHKI |
| 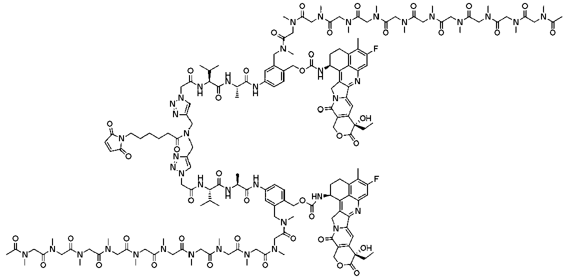 | Formula A177 | TOPO1+TOPO1 |
| 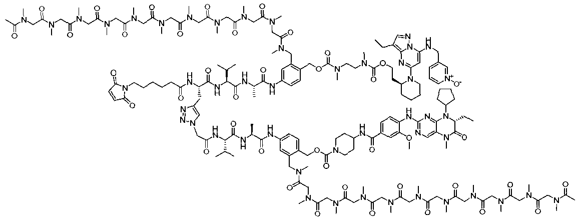 | Formula A178 | PLK1+CDK |
| 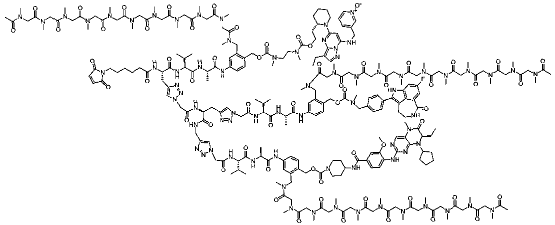 | Formula A179 | PLK1+CDK+PA RP |
| 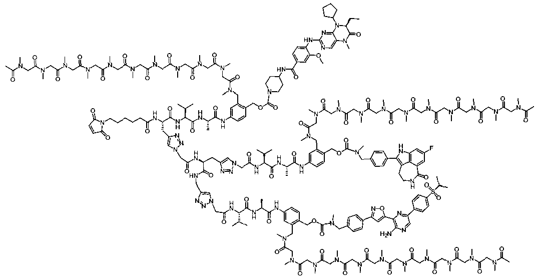 | Formula A180 | PLK1+PARP+A TR |
| 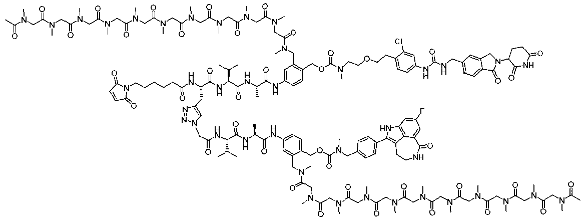 | Formula A181 | GSPT1+PARP |
| 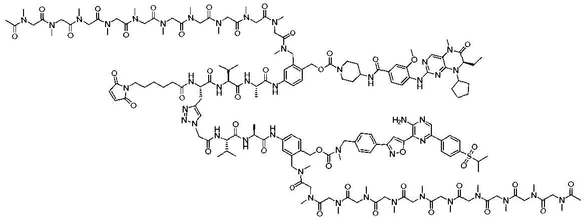 | Formula A182 | PLK1+ATR |
| 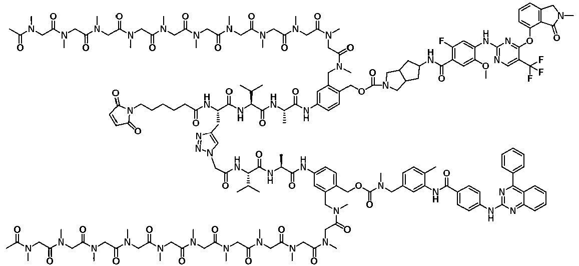 | Formula A183 | FAK+SMO |
| 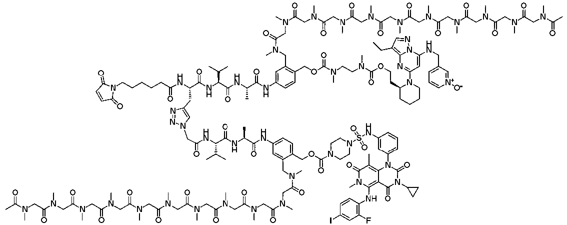 | Formula A184 | MEK+CDK |
| 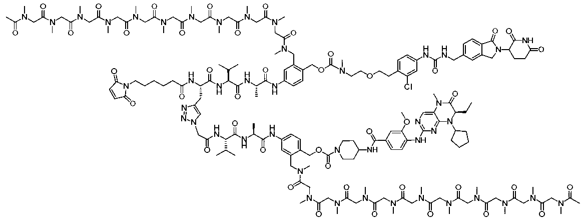 | Formula A185 | GSPT1+PLK1 |
| 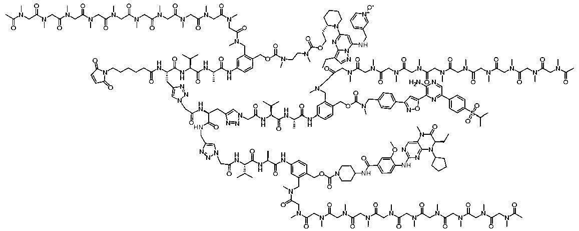 | Formula A186 | CDK+ATR+PLK 1 |
| 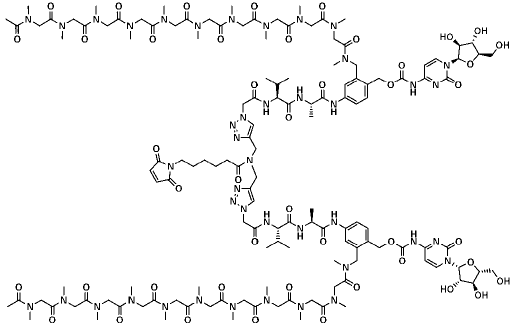 | Formula A187 | DNA+DNA |
| 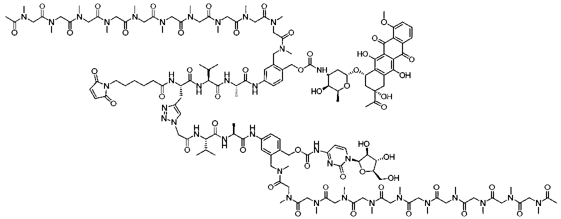 | Formula A188 | DNA+TOPO2 |
| 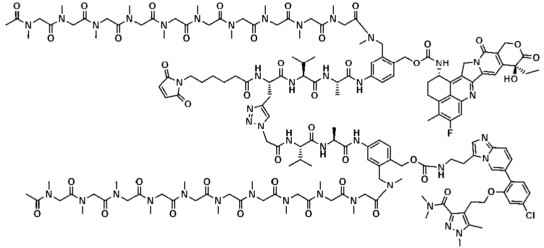 | Formula A189 | TOPO1+NMT |
| 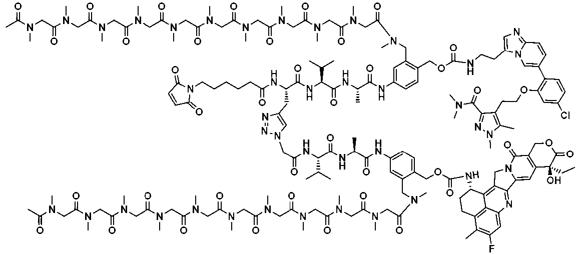 | Formula A190 | NMT+TOPO1 |

Another aspect of the present invention provides a compound, or a tautomer, a mesomer, a racemate, an enantiomer, or a diastereomer thereof, or a deuterated compound form thereof, or a mixture form thereof, or a pharmaceutically acceptable salt, prodrug, or solvate thereof, wherein the compound comprises a structure represented by formula II: 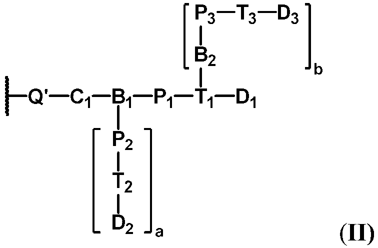 wherein,
Q' is a functional group capable of conjugating to cysteine, lysine, a non-natural amino acid, or a glycosyl of an antibody molecule;
C₁ is selected from the group consisting of: a direct bond, optionally substituted alkylene, optionally substituted polyethylene glycol group, optionally substituted alkenylene, optionally substituted alkynylene, optionally substituted aliphatic cyclylene, optionally substituted aliphatic heterocyclylene, optionally substituted arylene, and optionally substituted heteroarylene; when substituted, the substituent(s) are selected from halogen, hydroxyl, amino, carboxyl, sulfonic acid group, sulfone group, phosphonic acid group, and alkoxy;
B₁ and B₂ are independently a direct bond or a branching group, provided that when C₁ is a direct bond, B₁ is not a direct bond;
P₁, P₂, and P₃ are each independently an optionally substituted polypeptide residue or a glucose fragment;
T₁, T₂, and T₃ are each independently a direct bond or an optionally substituted spacer group, provided that at most two of T₁, T₂, and T₃ are direct bonds and at least one of B₂, T₁, T₂, and T₃ is substituted with a hydrophilic group;
D₁, D₂, and D₃ are a first drug unit, a second drug unit, and a third drug unit, respectively, and are the same or different;
a and b are independently 0, 1, 2, or 3, provided that a and b are not both 0; and
when a=0, B₁ is a direct bond and T₁ is not a direct bond;
when b=0, B₁ is not a direct bond.

In some embodiments, the present invention provides a compound, or a tautomer, a mesomer, a racemate, an enantiomer, or a diastereomer thereof, or a deuterated compound form thereof, or a mixture form thereof, or a pharmaceutically acceptable salt, prodrug, or solvate thereof, wherein the compound comprises a structure represented by formula IIa: 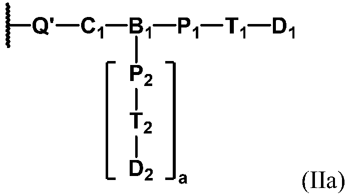 wherein:
Q' is a functional group capable of conjugating to cysteine, lysine, a non-natural amino acid, or a glycosyl of an antibody molecule;
C₁ is selected from the group consisting of: a direct bond, optionally substituted alkylene, optionally substituted polyethylene glycol group, optionally substituted alkenylene, optionally substituted alkynylene, optionally substituted aliphatic cyclylene, optionally substituted aliphatic heterocyclylene, optionally substituted arylene, and optionally substituted heteroarylene; when substituted, the substituent(s) are selected from halogen, hydroxyl, amino, carboxyl, sulfonic acid group, sulfone group, phosphonic acid group, and alkoxy;
B₁ is a branching group;
P₁ and P₂ are each independently an optionally substituted polypeptide residue or a glucose fragment;
T₁ and T₂ are each independently a direct bond or an optionally substituted spacer group, provided that at most one of T₁ and T₂ is a direct bond, and at least one of T₁ and T₂ is substituted with a hydrophilic group;
D₁ and D₂ are a first drug unit and a second drug unit, respectively, and are the same or different;
a is 1, 2, or 3.

In some embodiments, the present invention provides a compound, or a tautomer, a mesomer, a racemate, an enantiomer, or a diastereomer thereof, or a deuterated compound form thereof, or a mixture form thereof, or a pharmaceutically acceptable salt, prodrug, or solvate thereof, wherein the compound comprises a structure represented by formula IIb: 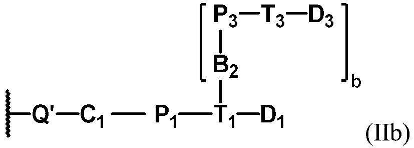
Q' is a functional group capable of conjugating to cysteine, lysine, a non-natural amino acid, or a glycosyl of an antibody molecule;
C₁ is selected from the group consisting of: optionally substituted alkylene, optionally substituted polyethylene glycol group, optionally substituted alkenylene, optionally substituted alkynylene, optionally substituted aliphatic cyclylene, optionally substituted aliphatic heterocyclylene, optionally substituted arylene, and optionally substituted heteroarylene; when substituted, the substituent(s) are selected from halogen, hydroxyl, amino, carboxyl, sulfonic acid group, sulfone group, phosphonic acid group, and alkoxy;
B₂ is a branching group;
P₁ and P₃ are each independently a direct bond or an optionally substituted polypeptide residue or a glucose fragment;
T₁ is an optionally substituted spacer group, T₃ is a direct bond or an optionally substituted spacer group, and at least one of B₂ and T₃ is substituted with a hydrophilic group;
D₁ and D₃ are a first drug unit and a third drug unit, respectively, and are the same or different;
b is 1, 2, or 3.

In some embodiments, the present invention provides a compound, or a tautomer, a mesomer, a racemate, an enantiomer, or a diastereomer thereof, or a deuterated compound form thereof, or a mixture form thereof, or a pharmaceutically acceptable salt, prodrug, or solvate thereof, wherein the compound comprises a structure represented by formula II: 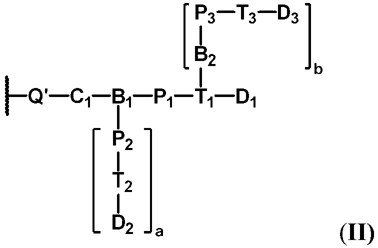 wherein,
Q' is a functional group capable of conjugating to cysteine, lysine, a non-natural amino acid, or a glycosyl of an antibody molecule;
C₁ is selected from the group consisting of: a direct bond, optionally substituted alkylene, optionally substituted polyethylene glycol group, optionally substituted alkenylene, optionally substituted alkynylene, optionally substituted aliphatic cyclylene, optionally substituted aliphatic heterocyclylene, optionally substituted arylene, and optionally substituted heteroarylene; when substituted, the substituent(s) are selected from halogen, hydroxyl, amino, carboxyl, sulfonic acid group, sulfone group, phosphonic acid group, and alkoxy;
B₁ and B₂ are independently a branching group;
P₁, P₂, and P₃ are each independently an optionally substituted polypeptide residue or a glucose fragment;
T₁ is an optionally substituted spacer group, T₂ and T₃ are each independently a direct bond or an optionally substituted spacer group, and at least one of B₂, T₂, and T₃ is substituted with a hydrophilic group;
D₁, D₂, and D₃ are a first drug unit, a second drug unit, and a third drug unit, respectively, and are the same or different; and
a and b are independently 1, 2, or 3.

In preferred embodiments, in formulas II, IIa, and IIb, 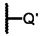 is selected from the group consisting of: optionally substituted 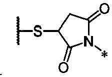 (Q1'), optionally substituted 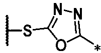 (Q2'), optionally substituted 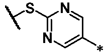 (Q3'), optionally substituted 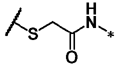 (Q4'), optionally substituted 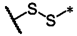 (Q5'), optionally substituted 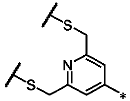 (Q6'), optionally substituted 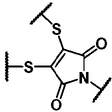 (Q7'), and optionally substituted 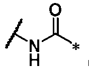 (Q8'), wherein * represents a site connected to C₁, and the wave line represents a site connected to the antibody.

In preferred embodiments, in formulas II, IIa, and IIb, 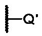 is selected from the group consisting of: 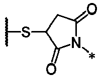 (Q1'), 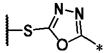 (Q2'), 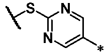 (Q3'), 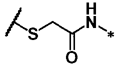 (Q4'), 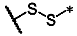 (Q5'), 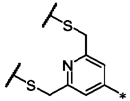 (Q6'), 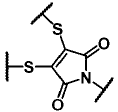 (Q7'), and 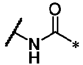 (Q8'), wherein * represents a site connected to C₁, and the wave line represents a site connected to the antibody. Preferably, in formulas II, IIa, and IIb, 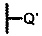 is 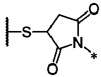 (Q1').

In preferred embodiments, in formulas II, IIa, and IIb, C₁ is selected from: a direct bond, - (CH₂)ₘ₁-, -(CH₂)ₘ₁-(O-CH₂-CH₂)ₘ₂-, -(CH₂)ₘ₁-(O-CH₂-CH₂)ₘ₂-(CH₂)ₘ₃-, -(CH₂)ₘ₁-C(O)NH-(CH₂)ₘ₂-, -(CH₂)ₘ₁-NHC(O)-(CH₂)ₘ₂-, and -(C≡C-(CH₂)ₘ₁-, wherein m1, m2, and m3 are independently integers from 1 to 6, and the indicated substituent is connected to Q' on the left side and to B₁ or P₁ on the right side.

In preferred embodiments, in formulas II and IIa, B₁ is selected from the group consisting of: a direct bond, 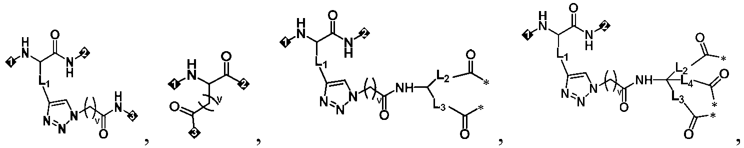 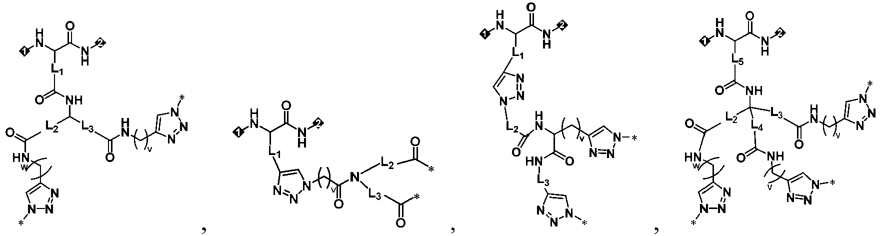 , and 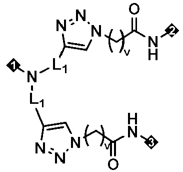 , wherein attachment point 1 is connected to C₁, attachment point 2 is connected to P₁, attachment points * and 3 are connected to P₂, L₁ is selected from -(CH₂)ₘ₁-, - (CH₂)ₘ₁-C(O)NH-(CH₂)ₘ₂-, -(CH₂)ₘ₁-NHC(O)-(CH₂)ₘ₂-, L₂, L₃, and L₄ are independently - (CH₂)ₘ₁-(O-CH₂-CH₂)ₘ₂- or -(CH₂)ₘ₁-(O-CH₂-CH₂)ₘ₂-(CH₂)ₘ₃-, L₅ is -(CH₂)ₘ₁-, wherein m1, m2, m3, w, and v are each independently 1, 2, 3, 4, 5, or 6.

In preferred embodiments, in formulas II and IIa, when C₁ is a direct bond, B₁ is 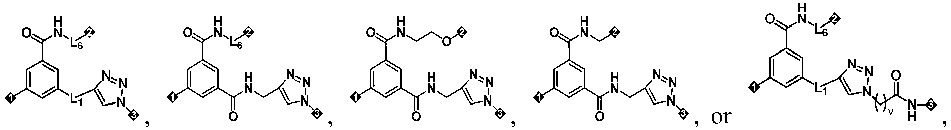 wherein attachment point 1 is connected to Q', attachment point 2 is connected to P₁, attachment point 3 is connected to P₂, L₆ is -(CH₂)ₘ₁- or -(CH₂-CH₂-O)m₂, wherein m1, m2 are each independently 1, 2, 3, 4, 5, or 6.

In preferred embodiments, in formulas II and IIa, B₁ is selected from the group consisting of: a direct bond, 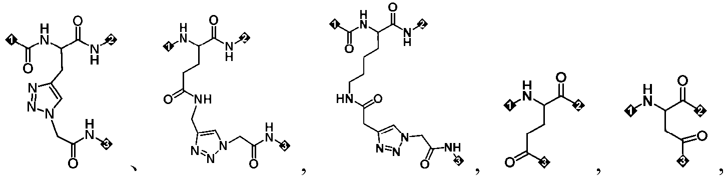 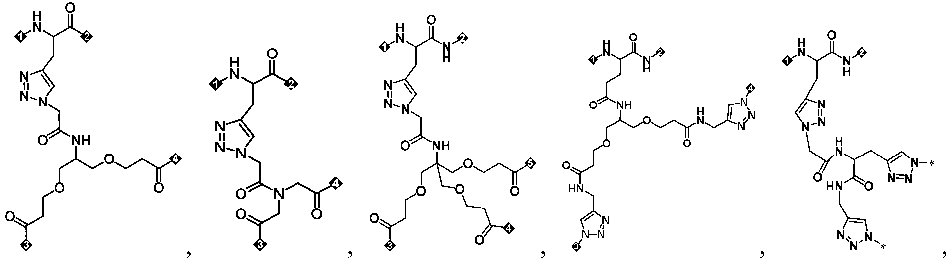 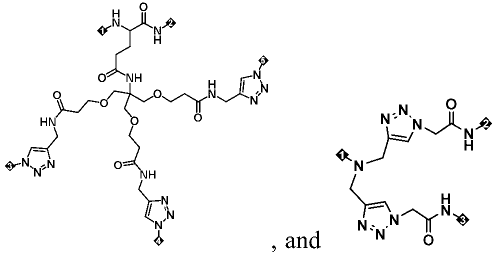 , wherein attachment point 1 is connected to C₁, attachment point 2 is connected to P₁, attachment points 3, 4 and 5 are connected to P₂.

In some embodiments, in formulas II and IIb, B₂ is selected from the group consisting of: 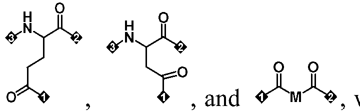 wherein attachment point 1 is connected to T₁, attachment point 2 is connected to P₃, attachment point 3 is connected to the hydrophilic group, M is selected from the group consisting of: optionally substituted C₁-C₆ alkylene, optionally substituted C₁-C₆ alkoxy, and optionally substituted C₁-C₅ alkenylene.

In some embodiments, in formula II, T₁, T₂, T₃ are independently selected from the group consisting of: a direct bond 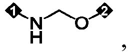 and optionally substituted 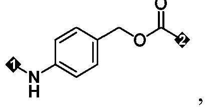 wherein attachment point 1 is connected to P₁, P₂, or P₃, and attachment point 2 is connected to D₁, D₂, or D₃.

In some embodiments, in formula IIa, T₁ and T₂ are independently selected from the group consisting of: a direct bond, 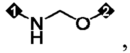 and optionally substituted 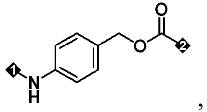 wherein attachment point 1 is connected to P₁ or P₂, and attachment point 2 is connected to D₁ or D₂.

In some embodiments, in formula IIb, T₁ is selected from the group consisting of: 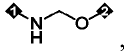 , and optionally substituted 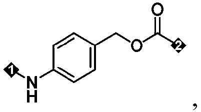 wherein attachment point 1 is connected to P₁, and attachment point 2 is connected to D₁, T₃ is selected from the group consisting of: a direct bond, 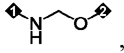 and optionally substituted 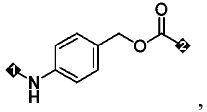 wherein attachment point 1 is connected to P₃, and attachment point 2 is connected to D₃.

In some embodiments, in formulas II, IIa, and IIb, P₁, P₂, P₃, when present, independently comprises an optionally substituted polypeptide residue composed of amino acids selected from the group consisting of: phenylalanine, isoleucine, leucine, tryptophan, valine, methionine, tyrosine, alanine, threonine, histidine, serine, glutamine, arginine, lysine, asparagine, glutamic acid, proline, citrulline, aspartic acid, and glycine.

In preferred embodiments, in formulas II, IIa, and IIb, P₁, P₂, P₃, when present, independently comprises an optionally substituted polypeptide residue composed of amino acids selected from the group consisting of: glycine, phenylalanine, valine, alanine, arginine, citrulline, aspartic acid, asparagine, and lysine.

In preferred embodiments, in formulas II, IIa, and IIb, P₁, P₂, P₃, when present, independently comprises an optionally substituted polypeptide residue selected from the group consisting of: phenylalanine-lysine (Phe-Lys), valine-alanine (Val-Ala), valine-citrulline (Val-Cit), glutamic acid-valine-alanine (Glu-Val-Ala), glutamic acid-valine-citrulline (Glu-Val-Cit), valine-lysine (Val-Lys), alanine-alanine (Ala-Ala), alanine-alanine-alanine (Ala-Ala-Ala), alanine-alanine-asparagine (Ala-Ala-Asn), alanine-leucine (Ala-Leu), leucine-leucine (Leu-Leu), phenylalanine-arginine (Phe-Arg), phenylalanine-lysine (Phe-Lys), 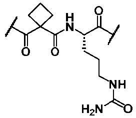 (cBu-Cit), 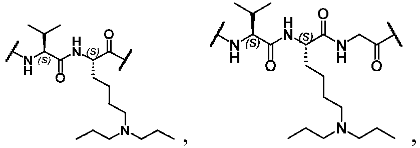 glycine-glycine-phenylalanine-glycine (Gly-Gly-Phe-Gly). In preferred embodiments, in formulas II, IIa, and IIb, P₁, P₂, P₃, when present, are independently phenylalanine-lysine (Phe-Lys), valine-alanine (Val-Ala), valine-citrulline (Val-Cit), glycine-glycine-phenylalanine-glycine (Gly-Gly-Phe-Gly), glycine-proline (Gly-Pro).

In preferred embodiments, in formulas II, IIa, and IIb, one of the combination P₂-T₂ and the combination P₃-T₃ is 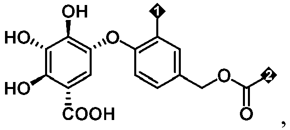 wherein attachment point 1 is connected to B₁ or B₂, and attachment point 2 is connected to D₂ or D₃.

In preferred embodiments, in formulas II, IIa, and IIb, the hydrophilic group is selected from: substituted polysarcosine residue, polyglycerol, polyol, glycosyl, cyclodextrin, substituted ethylene glycol fragment, substituted glycosylated polyethylene glycol, substituted glycosylated polyglycerol, substituted cyclodextrin polyethylene glycol, or a combination thereof.

In some preferred embodiments, in formulas II, IIa, and IIb, the substituted polysarcosine residue is 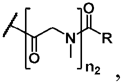 wherein n₂ is an integer between 4 and 20, for example between 4 and 16, R is selected from: C₁-C₆ alkyl, C₁-C₆ cycloalkyl, C₁-C₆ alkoxy.

In some other preferred embodiments, in formulas II, IIa, and IIb, the substituted glycosylated polyethylene glycol fragment is 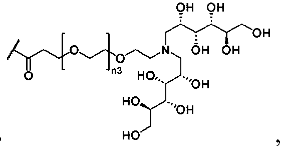 wherein n₃ is an integer between 4 and 18, for example between 4 and 12.

In preferred embodiments, the substituted glycosylated polyglycerol fragment is 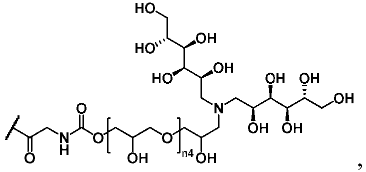 wherein n₄ is an integer between 4 and 12, for example between 4 and 10.

In preferred embodiments, the substituted polyethylene glycol fragment is 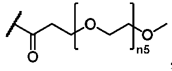 , wherein n₅ is an integer between 4 and 24, for example between 8 and 18.

In some other preferred embodiments, in formulas II, at least one of T₁, T₂, T₃ is 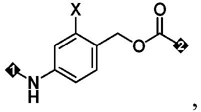 wherein X is optionally substituted 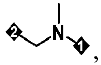 the hydrophilic group is connected to T₁, T₂, or T₃ via X, wherein in X, attachment point 1 is connected to the hydrophilic group, and attachment point 2 is connected to one of T₁, T₂, T₃.

In some other preferred embodiments, in formulas IIa, at least one of T₁ and T₂ is 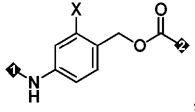 , wherein X is optionally substituted 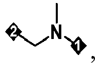 the hydrophilic group is connected to T₁ or T₂ via X, wherein in X, attachment point 1 is connected to the hydrophilic group, and attachment point 2 is connected to one of T₁ or T₂.

In some other preferred embodiments, in formulas IIb, at least one of T₁ and T₃ is 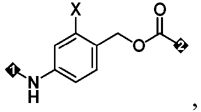 wherein X is optionally substituted 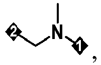 the hydrophilic group is connected to T₁ or T₃ via X, wherein in X, attachment point 1 is connected to the hydrophilic group, and attachment point 2 is connected to one of T₁ or T₃.

In preferred embodiments, in formulas II, IIa, and IIb, D₁, D₂, D₃, when present, are a first anti-cancer agent, a second anti-cancer agent, and a third anti-cancer agent, respectively; preferably, at least two of the first, second, and third anti-cancer agents have a synergistic anti-cancer effect.

In preferred embodiments, D₁, D₂, D₃, when present, are independently a cytotoxic drug or a tumor-targeted therapeutic drug.

In some embodiments, in formulas II, IIa, and IIb, D₁, D₂, and D₃, when present, are all cytotoxic drugs or are all tumor-targeted therapeutic drugs. In some embodiments, in formulas II, IIa, and IIb, D₁, D₂, and D₃, when present, are all cytotoxic drugs or are all tumor-targeted therapeutic drugs, and have identical structural formulas. In some embodiments, in formulas II, IIa, and IIb, D₁, D₂, and D₃, when present, are all cytotoxic drugs or are all tumor-targeted therapeutic drugs, but have different structural formulas.

In preferred embodiments, at least one of D₁, D₂, and D₃ is a cytotoxic drug, and at least another one is a tumor-targeted therapeutic drug. In preferred embodiments, at least two of D₁, D₂, and D₃ are tumor-targeted therapeutic drugs, for example, tumor-targeted therapeutic drugs that can produce a synergistic or additive effect. In preferred embodiments, at least two of D₁, D₂, and D₃ are cytotoxic drugs.

In preferred embodiments, the cytotoxic drug is selected from drug units targeting topoisomerase, drug units targeting tubulin, nucleoside antimetabolite anti-cancer drug units, and drug units targeting DNA.

In preferred embodiments, the tumor-targeted therapeutic drug is selected from drug units targeting DNA damage response (DDR) or "synthetic lethality" related pathways, drug units targeting epigenetics, drug units targeting apoptosis-related pathways, drug units targeting transcription factors, and drug units targeting immune activation pathway-related targets, comprising: inhibitors of EFGR pathway-related targets; Ras-Raf-MAPK pathway inhibitors; PI3K/AKT/mTOR pathway inhibitors; cell cycle pathway inhibitors; cGAS-STING signaling pathway agonists; estrogen receptor antagonists; androgen receptor antagonists; glucocorticoid receptor modulators; autophagy inhibitors; FAK inhibitors; Smo inhibitors; BTK inhibitors; PDE4 inhibitors; Lck inhibitors; PLK1 inhibitors; TLR7/8 modulators; N-myristoyltransferase (NMT) inhibitors.

In preferred embodiments, at least one of D₁, D₂, and D₃ is a drug targeting topoisomerase, and at least another one is a drug targeting DNA damage response (DDR) or "synthetic lethality" related pathways.

Accordingly, in some embodiments, at least one of D₁, D₂, and D₃ is a cytotoxic drug selected from drugs targeting topoisomerase, drugs targeting tubulin, and drugs targeting DNA, and at least another one is a tumor-targeted therapeutic drug, which is a drug targeting DNA damage response (DDR) or "synthetic lethality" related pathways. For example, at least one of D₁, D₂, and D₃ is a drug targeting topoisomerase, and at least another one is a drug targeting DNA damage response (DDR) or "synthetic lethality" related pathways. For example, at least one of D₁, D₂, and D₃ is a drug targeting tubulin, and at least another one is a drug targeting DNA damage response (DDR) or "synthetic lethality" related pathways. For example, at least one of D₁, D₂, and D₃ is a drug targeting DNA, and at least another one is a drug targeting DNA damage response (DDR) or "synthetic lethality" related pathways.

In some embodiments, at least one of D₁, D₂, and D₃ is a cytotoxic drug selected from drugs targeting topoisomerase, drugs targeting tubulin, and drugs targeting DNA, and at least another one is a tumor-targeted therapeutic drug, which is a drug targeting epigenetics. For example, at least one of D₁, D₂, and D₃ is a drug targeting topoisomerase, and at least another one is a drug targeting epigenetics. For example, at least one of D₁, D₂, and D₃ is a drug targeting tubulin, and at least another one is a drug targeting epigenetics. For example, at least one of D₁, D₂, and D₃ is a drug targeting DNA, and at least another one is a drug targeting epigenetics.

In some embodiments, at least one of D₁, D₂, and D₃ is a cytotoxic drug selected from drugs targeting topoisomerase, drugs targeting tubulin, and drugs targeting DNA, and at least another one is a tumor-targeted therapeutic drug, which is a drug targeting apoptosis-related pathways. For example, at least one of D₁, D₂, and D₃ is a drug targeting topoisomerase, and at least another one is a drug targeting apoptosis-related pathways. For example, at least one of D₁, D₂, and D₃ is a drug targeting tubulin, and at least another one is a drug targeting apoptosis-related pathways. For example, at least one of D₁, D₂, and D₃ is a drug targeting DNA, and at least another one is a drug targeting apoptosis-related pathways.

In some embodiments, at least one of D₁, D₂, and D₃ is a cytotoxic drug selected from drugs targeting topoisomerase, drugs targeting tubulin, and drugs targeting DNA, and at least another one is a tumor-targeted therapeutic drug, which is a drug targeting immune activation pathway-related targets. For example, at least one of D₁, D₂, and D₃ is a drug targeting topoisomerase, and at least another one is a drug targeting immune activation pathways. For example, at least one of D₁, D₂, and D₃ is a drug targeting tubulin, and at least another one is a drug targeting immune activation pathways. For example, at least one of D₁, D₂, and D₃ is a drug targeting DNA, and at least another one is a drug targeting immune activation pathways.

In some embodiments, at least two of D₁, D₂, and D₃ are drugs targeting DNA damage response (DDR) or "synthetic lethality" related pathways. In some embodiments, at least two of D₁, D₂, and D₃ are drugs targeting epigenetics. In some embodiments, at least two of D₁, D₂, and D₃ are drugs targeting apoptosis-related pathways. In some embodiments, at least two of D₁, D₂, and D₃ are drugs targeting immune activation pathways.

In some embodiments, at least two of D₁, D₂, and D₃ are drugs targeting topoisomerase. In some embodiments, at least two of D₁, D₂, and D₃ are nucleoside antimetabolite anti-cancer drugs. In some embodiments, at least two of D₁, D₂, and D₃ are drugs targeting tubulin. In some embodiments, at least two of D₁, D₂, and D₃ are drugs targeting DNA.

In some embodiments, at least one of D₁, D₂, and D₃ is a cytotoxic drug selected from drugs targeting topoisomerase, for example a TOPO1 inhibitor, and at least another one is a tumor-targeted therapeutic drug, which is a drug targeting DNA damage response (DDR) or "synthetic lethality" related pathways, selected from PARP inhibitors, ATR inhibitors, CHK1 inhibitors, ATM inhibitors, DNA-PK inhibitors, WEE1 inhibitors, POLQ inhibitors, CDK12 inhibitors, USP1 inhibitors, PKMYT1 inhibitors, Rad51 inhibitors.

In some embodiments, at least two of D₁, D₂, and D₃ are cytotoxic drugs and are independently selected from drugs targeting topoisomerase and drug units targeting tubulin, for example TOPO1 inhibitors, TOPO2 inhibitors, and tubulin inhibitors.

In some embodiments, at least two or all three of D₁, D₂, and D₃ are tumor-targeted therapeutic drugs, and are independently selected from drug units targeting DNA damage response (DDR) or "synthetic lethality" related pathways, drug units targeting epigenetics, drug units targeting apoptosis-related pathways, drug units targeting cell cycle pathways, drug units targeting transcription factors, and drug units targeting immune activation pathway-related targets, comprising: inhibitors targeting EFGR pathway-related targets; inhibitors targeting Ras-Raf-MAPK pathway-related targets; inhibitors targeting PI3K/AKT/mTOR pathway-related targets; inhibitors targeting cell cycle pathway-related targets; agonists targeting cGAS-STING signaling pathway-related targets; estrogen receptor antagonists, androgen receptor antagonists; glucocorticoid receptor modulators; autophagy inhibitors; FAK inhibitors; Smo inhibitors; BTK inhibitors; PDE4 inhibitors; Lck inhibitors; PLK1 inhibitors; TLR7/8 modulators; N-myristoyltransferase (NMT) inhibitors.

In some embodiments, one of D₁, D₂, and D₃ is a cytotoxic drug selected from drug units targeting topoisomerase and drug units targeting tubulin, for example a TOPO1 inhibitor or a tubulin inhibitor, and the other one or two are tumor-targeted therapeutic drugs, selected from drug units targeting DNA damage response (DDR) or "synthetic lethality" related pathways, drug units targeting epigenetics, drug units targeting apoptosis-related pathways, drug units targeting cell cycle pathways, drug units targeting transcription factors, and drug units targeting immune activation pathway-related targets, comprising: inhibitors targeting EFGR pathway-related targets; inhibitors targeting Ras-Raf-MAPK pathway-related targets; inhibitors targeting PI3K/AKT/mTOR pathway-related targets; inhibitors targeting cell cycle pathway-related targets; agonists targeting cGAS-STING signaling pathway-related targets; estrogen receptor antagonists, androgen receptor antagonists; glucocorticoid receptor modulators; autophagy inhibitors; FAK inhibitors; Smo inhibitors; BTK inhibitors; PDE4 inhibitors; Lck inhibitors; PLK1 inhibitors; TLR7/8 modulators; N-myristoyltransferase (NMT) inhibitors.

In preferred embodiments, the drug unit targeting topoisomerase is a topoisomerase I (TOPO1) inhibitor or topoisomerase II (TOPO2) inhibitor, comprising Exatecan (CAS 171335-80-1), DXd (CAS 1599440-33-1), 7-ethyl-10-hydroxycamptothecin (SN38, CAS 86639-52-3), Belotecan (CAS 213819-48-8), (4-NH₂)-Exatecan (AZD'0132, CAS 2495742-21-5), 7-MAD-MDCPT (CAS 765871-81-6), 7-aminomethyl-10-methyl-11-fluorocamptothecin (CAS 2378616-23-8), Voreloxin (CAS 175414-77-4), or derivatives and analogs thereof. In preferred embodiments, the drug unit targeting tubulin is Eribulin, Vinblastine, Paclitaxel, MMAE, MMAF, Maytansine, or derivatives thereof. In preferred embodiments, the drug unit targeting DNA is a DNA minor groove binder (PBD), a DNA alkylator (Duocarmycin), Trabectedin, Lurbinectedin, or derivatives thereof.

In preferred embodiments, the drug unit targeting DNA damage response (DDR) or "synthetic lethality" related pathways is a PARP inhibitor, ATR inhibitor, CHK1 inhibitor, ATM inhibitor, DNA-PK inhibitor, WEE1 inhibitor, POLQ inhibitor, CDK12 inhibitor, USP1 inhibitor, PKMYT1 inhibitor, or Rad51 inhibitor; the PARP inhibitor is preferably Rucaparib, Niraparib, Veliparib, A-966492, Talazoparib, AZD5305, Venadaparib, Mefuparib, or analogs thereof; the ATR inhibitor is preferably Berzosertib, Ceralasertib, or analogs thereof; and/or the CHK1 inhibitor is preferably Prexasertib (LY2606368), AZD7762, Rabusertib (LY2603618), MK-8776 (SCH 900776), CHIR-124, PF-477736, CCT245737 (SRA737, PNT-737), GDC-0575 (ARRY-575), or analogs thereof; and/or the WEE1 inhibitor is preferably ZN-C3, Adavosertib (AZD1775), or analogs thereof.

Preferably, an analog of the AZD5305 comprises the following structure: 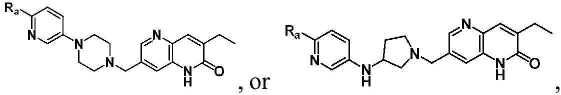 wherein Rₐ is selected from -C(O)NH-R_{b} or -NHC(O)-R_{b}, R_{b} is selected from: C₂₋₇ alkyl, C₃₋₇ monocyclic cycloalkyl, C₄₋₁₀ bicyclic cycloalkyl, C₅₋₉ spirocycloalkyl, and C₅₋₉ bridged cycloalkyl, each substituted with at least one primary or secondary amino group, or R_{b} is a 4-6 membered monocyclic heterocycloalkyl, C₄₋₁₀ bicyclic heterocycloalkyl, C₅₋₉ spiroheterocycloalkyl, C₅₋₉ bridged heterocycloalkyl containing 1-5 nitrogen, oxygen, sulfur atoms, wherein the R_{b} group comprises at least one primary or secondary amino group.

Preferably, the AZD5305 analog is selected from the following structures:

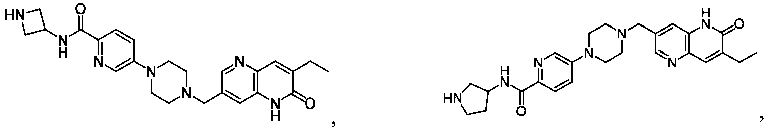

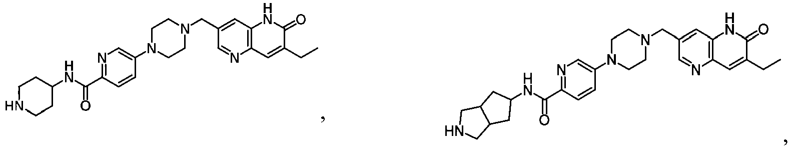

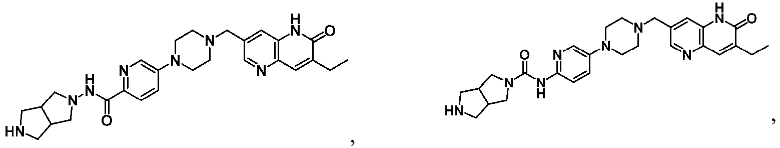

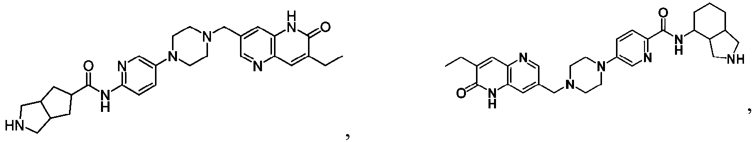

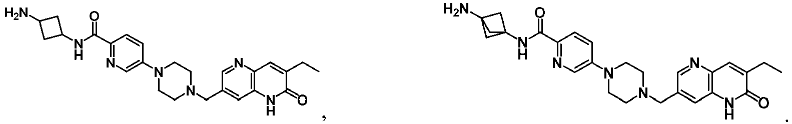

In preferred embodiments, the drug unit targeting apoptosis-related pathways is a Bcl-2 family protein inhibitor; the Bcl-2 family protein inhibitor comprises BCL-2 inhibitors, BCL-XL inhibitors, and MCL-1 inhibitors, preferably Venetoclax (CAS 1257044-40-8), Navitoclax (CAS 923564-51-6), Navitoclax analog (CAS 2143096-93-7), ABT-737 (CAS 852808-04-9), A-1331852 (CAS 1430844-80-6), S64315 (CAS 1799631-75-6), or analogs thereof.

Preferably, the A-1331852, S64315 analogs are selected from the following structures:

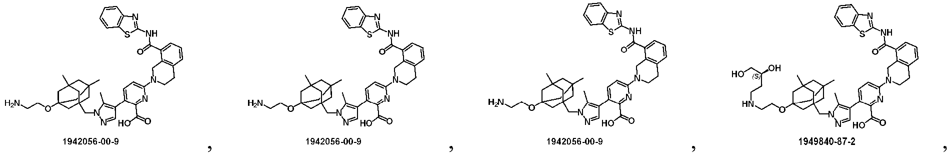

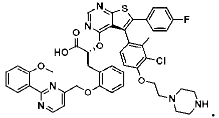

In preferred embodiments, the drug unit targeting epigenetics is an LSD1 inhibitor, EZH2 inhibitor, BRD4 inhibitor, PRMT5 inhibitor, or PRMT1 inhibitor. In preferred embodiments, the LSD1 inhibitor is preferably Tranylcypromine, ORY-1001 (Iadademstat, CAS 1431303-72-8), CC-90011 (Pulrodemstat, CAS 1821307-10-1), ORY-2001, GSK-2879552, IMG-7289, INCB059872, or TAK-418 or analogs thereof; the EZH2 inhibitor is preferably Tazemetostat (CAS 1403254-99-8), GSK2816126, CPI-1205, PF-06821497, SHR2554, XNW5004, HH2853, or analogs thereof; the BRD4 inhibitor is a BI-2536 analog, Birabresib; the PRMT5 inhibitor is preferably GSK3326595, AMG 193, MRTX1719, SKL27969, TNG908, SCR-6920, SH3765, SYHX2001, or analogs thereof. The PRMT1 inhibitor is preferably GSK3368715 (CAS 1629013-22-4), MS023 (CAS 1831110-54-3), or analogs thereof. In preferred embodiments, the drug unit targeting immune activation pathway-related targets is a PD-L1 inhibitor, CBL-B inhibitor, TLR7/8 agonist, PTPN2/1 inhibitor, or STING agonist.

In preferred embodiments, the Ras-Raf-MAPK pathway inhibitors comprise: SOS1 inhibitors BAY-293 (CAS 2244904-70-7), BI-3406 (CAS 2230836-55-0), or analogs thereof; KRAS inhibitors BI-2493 (CAS 2937344-16-4), MRTX1133 (CAS 2621928-55-8), or analogs thereof; MEK inhibitors Cobimetinib (CAS 934660-93-2), Selumetinib (CAS 606143-52-6), Mirdametinib (CAS 391210-10-9), Binimetinib (CAS 606143-89-9), TAK-733 (CAS 1035555-63-5), GDC-0623 (CAS 1168091-68-6), AZD8330 (CAS 869357-68-6), Trametinib (CAS 871700-17-3), Trametiglue (CAS 2666940-97-0), or analogs thereof; ERK inhibitors ASN007 (CAS 2055597-12-9) or analogs thereof.

In preferred embodiments, the cell cycle pathway inhibitors comprise: CDK4/6 inhibitors Palbociclib (CAS 571190-30-2), Abemaciclib (CAS 1231929-97-7), Ribociclib (CAS 1211441-98-3), Dalpiciclib (CAS 1637781-04-4), or analogs thereof; pan-CDK inhibitors Dinaciclib (CAS 779353-01-4) or analogs thereof.

In preferred embodiments, the PI3K/AKT/mTOR pathway inhibitors comprise: PKI-587 (CAS 1197160-78-3), desmethyl PKI-587 (CAS 1950569-63-7), AZD8055 (CAS 1009298-09-2), Afuresertib (CAS 1047644-62-1), or analogs thereof.

In preferred embodiments, the estrogen receptor antagonists/degraders comprise Fulvestrant (CAS 129453-61-8), Elacestrant (CAS 1349723-93-8); the androgen receptor antagonists comprise abiraterone (CAS 154229-19-3), JNJ-63576253 (CAS 2110428-64-1), or analogs thereof.

In preferred embodiments, the autophagy inhibitor is Hydroxychloroquine (HCQ, CAS 118-42-3) and Chloroquine (CQ, CAS 54-05-7) or analogs thereof.

In preferred embodiments, the drug targeting transcription factors is a GSPT1 degrader (molecular glue), and the GSPT1 degrader is selected from the following structures:

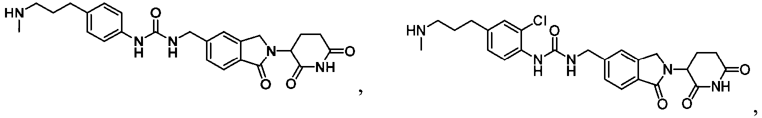

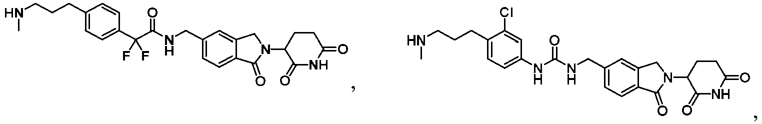

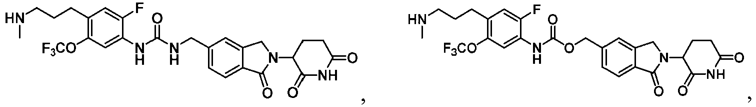

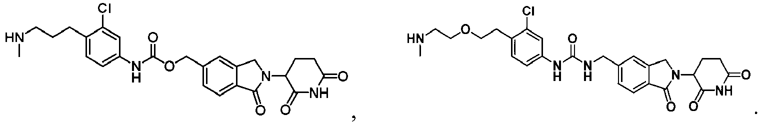

Preferably, the PLK1 inhibitor is BI2536 (CAS 755038-02-9), Volasertib (CAS 755038-65-4), GSK461364 (CAS 929095-18-1), Onvansertib (CAS 1034616-18-6), or analogs thereof;

In preferred embodiments, the N-myristoyltransferase (NMT) inhibitor is MYX1715 (CAS 2445448-66-6) or analogs thereof.

In preferred embodiments, D₁, D₂, and D₃ are selected from the combinations in the following table:

| Drug Combination | D1 | D2 | D3 |
|---|---|---|---|
| Combination 1 | TOPO1 inhibitor | PARP inhibitor | / |
| Combination 2 | TOPO1 inhibitor | ATR inhibitor | / |
| Combination 3 | TOPO1 inhibitor | CHK1 inhibitor | / |
| Combination 4 | TOPO1 inhibitor | PARP inhibitor | ATR inhibitor |
| Combination 5 | TOPO1 inhibitor | PARP inhibitor | CHK1 inhibitor |
| Combination 6 | TOPO1 inhibitor | PARP inhibitor | WEE1 inhibitor |
| Combination 7 | TOPO1 inhibitor | ATR inhibitor | WEE1 inhibitor |
| Combination 8 | TOPO1 inhibitor | CHK1 inhibitor | WEE1 inhibitor |
| Combination 9 | TOPO1 inhibitor | DNA-PK inhibitor | / |
| Combination 10 | TOPO1 inhibitor | POLQ inhibitor | / |
| Combination 11 | TOPO1 inhibitor | TOPO2 inhibitor | / |
| Combination 12 | PARP inhibitor | ATR/CHK1/WEE1 inhibitor | / |
| Combination 13 | PARP inhibitor | ATR inhibitor | CHK1 inhibitor |
| Combination 14 | TOPO1 inhibitor | Immune activator (PD-L1 inhibitor, CBL-B inhibitor, TLR7/8 agonist, PTPN2/1 inhibitor, or STING agonist) | / |
| Combination 15 | TOPO1 inhibitor | BCL-2 family inhibitor | / |
| Combination 16 | TOPO1 inhibitor | BCL-2 family inhibitor | MCL-1 inhibitor |
| Combination 17 | TOPO1 inhibitor | DNA synthesis inhibitor | / |
| Combination 18 | TOPO1 inhibitor | PI3K/mTOR inhibitor | / |
| Combination 19 | TOPO1 inhibitor | EGFR inhibitor | / |
| Combination 20 | TOPO1 inhibitor | Autophagy inhibitor | / |
| Combination 21 | TOPO1 inhibitor | FAK inhibitor | |
| Combination 22 | TOPO1 inhibitor | PARP inhibitor | FAK inhibitor |
| Combination 23 | TOPO1 inhibitor | PARP inhibitor | Androgen receptor antagonist/degrader |
| Combination 24 | TOPO1 inhibitor | GSPT1 degrader | / |
| Combination 25 | TOPO1 inhibitor | PARP inhibitor | GSPT1 degrader |
| Combination 26 | TOPO1 inhibitor | Tubulin inhibitor | / |
| Combination 27 | TOPO1 inhibitor | Epigenetic regulator | / |
| Combination 28 | Tubulin inhibitor | Immune activator (PD-L1 inhibitor, CBL-B inhibitor, TLR7/8 agonist, PTPN2/1 inhibitor, or STING agonist) | / |
| Combination 29 | Tubulin inhibitor | BCL-2/BCL-XL inhibitor | / |
| Combination 30 | Tubulin inhibitor | BCL-2/BCL-XL inhibitor | MCL-1 inhibitor |
| Combination 31 | Tubulin inhibitor | PI3K/AKT/mTOR inhibitor | / |
| Combination 32 | Tubulin inhibitor | FAK inhibitor | |
| Combination 33 | CDK4/6 inhibitor | MEK inhibitor | / |
| Combination 34 | CDK4/6 inhibitor | MEK inhibitor | EZH2 inhibitor |
| Combination 35 | CDK4/6 inhibitor | Autophagy inhibitor | / |
| Combination 36 | MEK inhibitor | Autophagy inhibitor | / |
| Combination 37 | ERK inhibitor | Autophagy inhibitor | / |
| Combination 38 | CDK4/6 inhibitor | MEK inhibitor | Autophagy inhibitor |
| Combination 39 | CDK4/6 inhibitor | ERK inhibitor | Autophagy inhibitor |
| Combination 40 | CDK4/6 inhibitor | ERK inhibitor | (MEK inhibitor) |
| Combination 41 | CDK4/6 inhibitor | MEK inhibitor | KRAS inhibitor |
| Combination 42 | CDK4/6 inhibitor | MEK inhibitor | SOS1 inhibitor |
| Combination 43 | CDK4/6 inhibitor | MEK inhibitor | FAK inhibitor |
| Combination 44 | CDK4/6 inhibitor | PRMT5 inhibitor | / |
| Combination 45 | CDK4/6 inhibitor | Estrogen receptor antagonist/degrader | / |
| Combination 46 | CDK4/6 inhibitor | Estrogen receptor antagonist/degrader | PI3K/mTOR inhibitor |
| Combination 47 | CDK4/6 inhibitor | MEK inhibitor | GSPT1 degrader |
| Combination 48 | CDK4/6 inhibitor | ERK inhibitor | GSPT1 degrader |
| Combination 49 | CDK4/6 inhibitor | Autophagy inhibitor | GSPT1 degrader |
| Combination 50 | BRD4 inhibitor | EZH2 inhibitor | / |
| Combination 51 | PRMT5 inhibitor | MAT2A inhibitor | / |
| Combination 52 | PRMT5 inhibitor | Gemcitabine | / |
| Combination 53 | PRMT5 inhibitor | CDK4/6 inhibitor | / |
| Combination 54 | PRMT5 inhibitor | PRMT1 inhibitor | / |
| Combination 55 | PRMT5 inhibitor | PRMT1 inhibitor | CDK4/6 inhibitor |
| Combination 56 | PRMT5 inhibitor | PRMT1 inhibitor | MEK inhibitor |
| Combination 57 | RAF inhibitor | MEK inhibitor | / |
| Combination 58 | EGFR inhibitor | MEK inhibitor | / |
| Combination 59 | RAF inhibitor | MEK inhibitor | SHP2 inhibitor |
| Combination 60 | CDK4/6 inhibitor | HIF-2alpha inhibitor | / |
| Combination 61 | TOPO1 inhibitor | WEE1 inhibitor | / |
| Combination 62 | WEE1 inhibitor | CHK1 inhibitor | / |
| Combination 63 | TOPO1 inhibitor | TOPO1 inhibitor | / |
| Combination 64 | CDK inhibitor | MEK inhibitor | |
| Combination 65 | FAK inhibitor | Smo inhibitor | / |
| Combination 66 | PLK1 inhibitor | CDK inhibitor | / |
| Combination 67 | PLK1 inhibitor | CDK inhibitor | PARP inhibitor/ |
| Combination 68 | TOPO1 inhibitor | PLK1 inhibitor | / |
| Combination 69 | TOPO1 inhibitor | PLK1 inhibitor | PARP inhibitor/ |
| Combination 70 | TOPO1 inhibitor | PLK1 inhibitor | ATR inhibitor/ |
| Combination 71 | TOPO1 inhibitor | NMT inhibitor | / |
| Combination 72 | Tubulin inhibitor | NMT inhibitor | / |

In preferred embodiments, at least one of D₁, D₂, and D₃ is a topoisomerase inhibitor, and at least another one is a PARP inhibitor, an ATR inhibitor, or a CHK1 inhibitor. In preferred embodiments, at least one of D₁, D₂, and D₃ is a topoisomerase inhibitor, and at least another one is a Bcl-2 family protein inhibitor. In preferred embodiments, at least one of D₁, D₂, and D₃ is a topoisomerase inhibitor, and at least another one is an LSD1 inhibitor, an EZH2 inhibitor, or a PRMT5 inhibitor. In preferred embodiments, at least one of D₁, D₂, and D₃ is a topoisomerase inhibitor, and at least another one is a PD-L1 inhibitor, a CBL-B inhibitor, a TLR7/8 agonist, or a STING agonist.

In preferred embodiments, at least one of D₁, D₂, and D₃ is selected from: Exatecan (CAS 171335-80-1), DXd (CAS 1599440-33-1), 7-ethyl-10-hydroxycamptothecin (SN38, CAS 86639-52-3), Belotecan (CAS 213819-48-8), (4-NH₂)-Exatecan (AZD'0132, CAS 2495742-21-5), 7-MAD-MDCPT (CAS 765871-81-6), 7-aminomethyl-10-methyl-11-fluorocamptothecin (CAS 2378616-23-8), Voreloxin (CAS 175414-77-4), or derivatives and analogues thereof; and at least another one is selected from PARP inhibitors, ATR inhibitors, CHK1 inhibitors, Bcl-2 family protein inhibitors, LSD1 inhibitors, EZH2 inhibitors, BRD4 inhibitors, PRMT5 inhibitors, PD-L1 inhibitors, CBL-B inhibitors, TLR7/8 agonists, and STING agonists.

In preferred embodiments, at least one of D₁, D₂, and D₃ is selected from: Exatecan (CAS 171335-80-1), DXd (CAS 1599440-33-1), 7-ethyl-10-hydroxycamptothecin (SN38, CAS 86639-52-3), Belotecan (CAS 213819-48-8), (4-NH₂)-Exatecan (AZD'0132, CAS 2495742-21-5), 7-MAD-MDCPT (CAS 765871-81-6), 7-aminomethyl-10-methyl-11-fluorocamptothecin (CAS 2378616-23-8), Voreloxin (CAS 175414-77-4), or derivatives and analogues thereof; and at least another one is selected from: Rucaparib, Niraparib, Veliparib, A-966492, Talazoparib, AZD5305, Venadaparib, Mefuparib, Prexasertib (LY2606368), AZD7762, Rabusertib (LY2603618), MK-8776 (SCH 900776), CHIR-124, PF-477736, CCT245737 (SRA737, PNT-737), GDC-0575 (ARRY-575), Berzosertib, Ceralasertib, RP-6306, GSK-1520489A, and analogues thereof.

In preferred embodiments, at least one of D₁, D₂, and D₃ is a tubulin inhibitor, and at least another one is a PARP inhibitor, an ATR inhibitor, or a CHK1 inhibitor. In preferred embodiments, at least one of D₁, D₂, and D₃ is a tubulin inhibitor, and at least another one is a Bcl-2 family protein inhibitor. In preferred embodiments, at least one of D₁, D₂, and D₃ is a tubulin inhibitor, and at least another one is an LSD1 inhibitor, an EZH2 inhibitor, or a PRMT5 inhibitor. In preferred embodiments, at least one of D₁, D₂, and D₃ is a tubulin inhibitor, and at least another one is a PD-L1 inhibitor, a CBL-B inhibitor, a TLR7/8 agonist, or a STING agonist.

In preferred embodiments, at least one of D₁, D₂, and D₃ is selected from: Eribulin, Vinblastine, Paclitaxel, MMAE, MMAF, Maytansine or derivatives thereof, and at least another one is selected from PARP inhibitors, ATR inhibitors, CHK1 inhibitors, Bcl-2 family protein inhibitors, LSD1 inhibitors, EZH2 inhibitors, BRD4 inhibitors, PRMT5 inhibitors, PD-L1 inhibitors, CBL-B inhibitors, TLR7/8 agonists, and STING agonists.

In preferred embodiments, at least one of D₁, D₂, and D₃ is selected from: Eribulin, Vinblastine, Paclitaxel, MMAE, MMAF, Maytansine or derivatives thereof; and at least another one is selected from: Rucaparib, Niraparib, Veliparib, A-966492, Talazoparib, AZD5305, Venadaparib, Mefuparib, Prexasertib (LY2606368), AZD7762, Rabusertib (LY2603618), MK-8776 (SCH 900776), CHIR-124, PF-477736, CCT245737 (SRA737, PNT-737), GDC-0575 (ARRY-575), Berzosertib, Ceralasertib, RP-6306, GSK-1520489A, and analogues thereof.

In preferred embodiments, at least one of D₁, D₂, and D₃ is selected from: a PBD or Duocarmycin, and at least another one is selected from PARP inhibitors, ATR inhibitors, CHK1 inhibitors, Bcl-2 family protein inhibitors, LSD1 inhibitors, EZH2 inhibitors, BRD4 inhibitors, PRMT5 inhibitors, PD-L1 inhibitors, CBL-B inhibitors, TLR7/8 agonists, and STING agonists.

In preferred embodiments, at least one of D₁, D₂, and D₃ is a PBD or Duocarmycin, and at least another one is selected from: Rucaparib, Niraparib, Veliparib, A-966492, Talazoparib, AZD5305, Venadaparib, Mefuparib, Prexasertib (LY2606368), AZD7762, Rabusertib (LY2603618), MK-8776 (SCH 900776), CHIR-124, PF-477736, CCT245737 (SRA737, PNT-737), GDC-0575 (ARRY-575), Berzosertib, Ceralasertib, RP-6306, GSK-1520489A, and analogues thereof.

In preferred embodiments, at least two of D₁, D₂, and D₃ are drugs targeting the DNA Damage Response (DDR) or "synthetic lethality" related pathways. In preferred embodiments, at least two of D₁, D₂, and D₃ are selected from: Rucaparib, Niraparib, Veliparib, A-966492, Talazoparib, AZD5305, Venadaparib, Mefuparib, Prexasertib (LY2606368), AZD7762, Rabusertib (LY2603618), MK-8776 (SCH 900776), CHIR-124, PF-477736, CCT245737 (SRA737, PNT-737), GDC-0575 (ARRY-575), Berzosertib, Ceralasertib, RP-6306, GSK-1520489A, and analogues thereof.

In preferred embodiments, D₁, D₂, and D₃ can be selected from the exemplary combinations shown in Table A.

**Table A. Exemplary combinations of D₁, D₂, and D₃.**

| | D1 | D2 | D3 |
|---|---|---|---|
| Combination 1-1 | 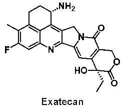 | 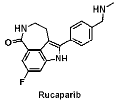 | / |
| Combination 1-2 | 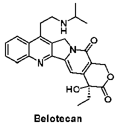 | 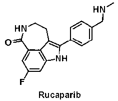 | / |
| Combination 1-3 | 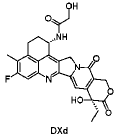 | 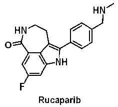 | / |
| Combination 1-4 | 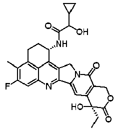 | 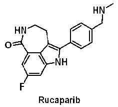 | / |
| Combination 1-5 | 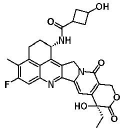 | 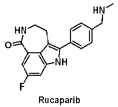 | / |
| Combination 1-6 | 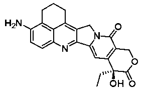 | 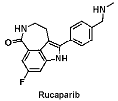 | / |
| Combination 1-7 | 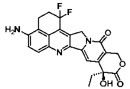 | 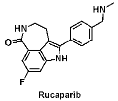 | / |
| Combination 1-8 | 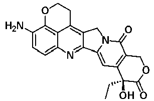 | 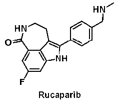 | / |
| Combination 1-9 | 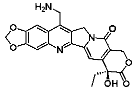 | 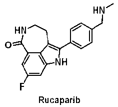 | / |
| Combination 1-10 | 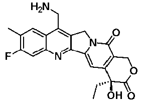 | 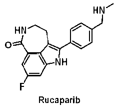 | / |
| Combination 1-11 | 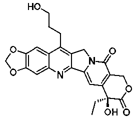 | 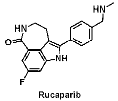 | / |
| Combination 1-12 | 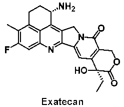 | 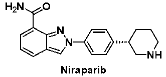 | / |
| Combination 1-13 | 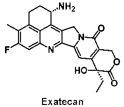 | 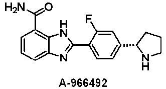 | / |
| Combination 1-14 | 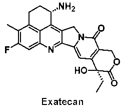 | 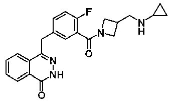 | / |
| Combination 1-15 | 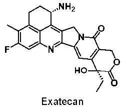 | 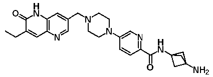 | / |
| Combination 1-16 | 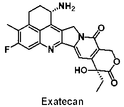 | 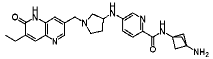 | / |
| Combination 1-17 | 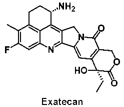 | 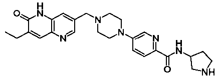 | / |
| Combination 1-18 | 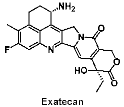 | 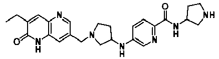 | / |
| Combination 1-19 | 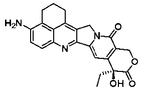 | 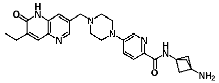 | / |
| Combination 2-1 | 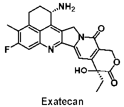 | 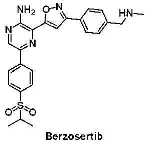 | / |
| Combination 2-2 | 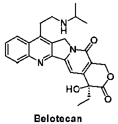 | 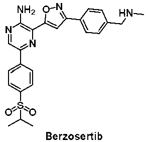 | / |
| Combination 2-3 | 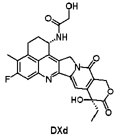 | 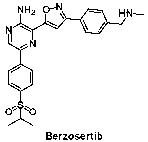 | / |
| Combination 2-4 | 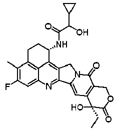 | 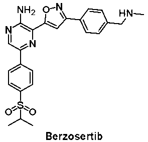 | / |
| Combination 2-5 | 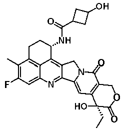 | 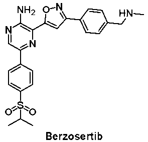 | / |
| Combination 2-6 | 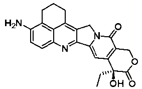 | 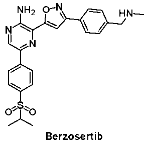 | / |
| Combination 2-7 | 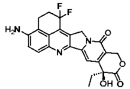 | 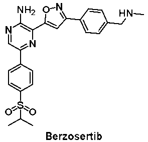 | / |
| Combination 3-1 | 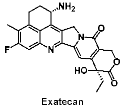 | 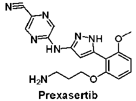 | / |
| Combination 3-2 | 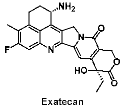 | 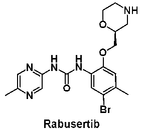 | / |
| Combination 3-3 | 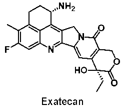 | 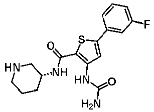 | / |
| Combination 3-4 | 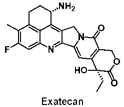 | 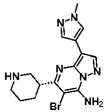 | / |
| Combination 3-5 | 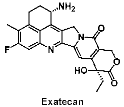 | 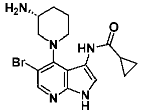 | / |
| Combination 3-6 | 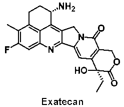 | 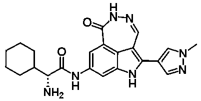 | / |
| Combination 3-7 | 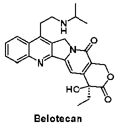 | 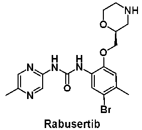 | / |
| Combination 3-8 | 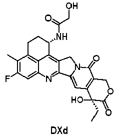 | 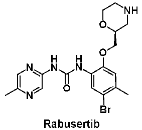 | / |
| Combination 3-9 | 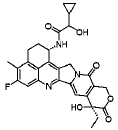 | 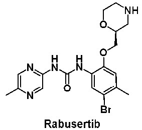 | / |
| Combination 3-10 | 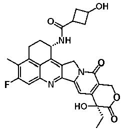 | 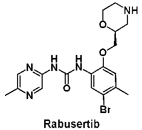 | / |
| Combination 3-11 | 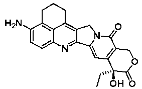 | 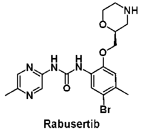 | / |
| Combination 3-12 | 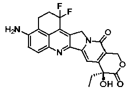 | 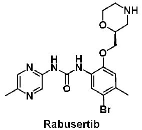 | / |
| Combination 9-1 | 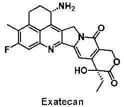 | 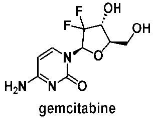 | / |
| Combination 11-1 | 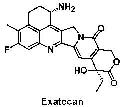 | 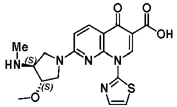 | / |
| Combination 17-1 | 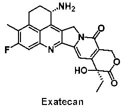 | 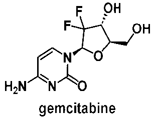 | / |
| Combination 4-1 | 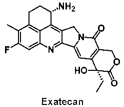 | 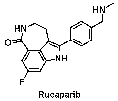 | 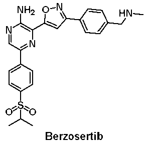 |
| Combination 5-1 | 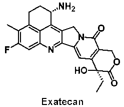 | 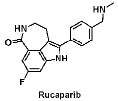 | 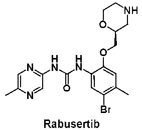 |
| Combination 5-2 | 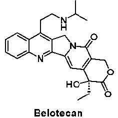 | 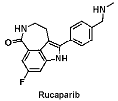 | 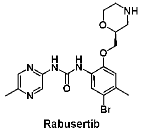 |
| Combination 13-1 | 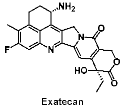 | 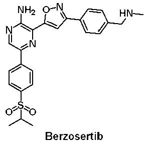 | 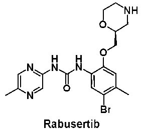 |
| Combination 4-2 | 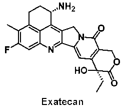 | 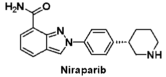 | 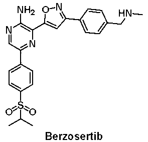 |
| Combination 13-1 | 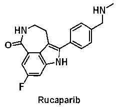 | 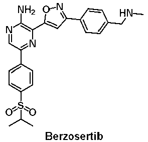 | 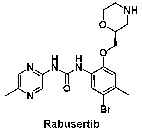 |
| Combination 13-2 | 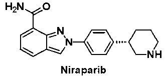 | 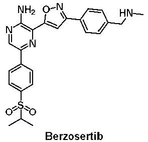 | 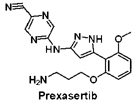 |
| Combination 14-1 | 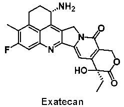 | 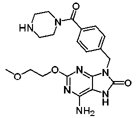 | / |
| Combination 14-2 | 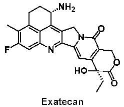 | 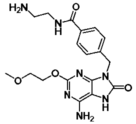 | / |
| Combination 14-3 | 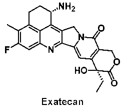 | 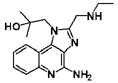 | / |
| Combination 14-4 | 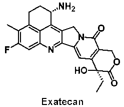 | 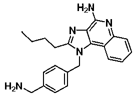 | / |
| Combination 14-5 | 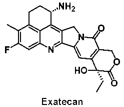 | 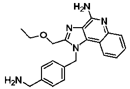 | / |
| Combination 14-6 | 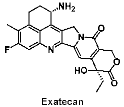 | 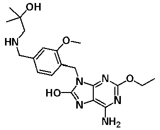 | |
| Combination 14-7 | 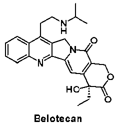 | 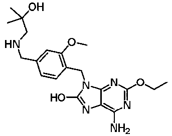 | |
| Combination 14-8 | 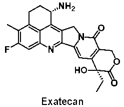 | 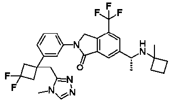 | / |
| Combination 14-9 | 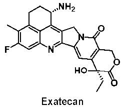 | 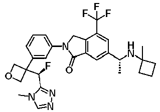 | / |
| Combination 14-10 | 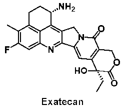 | 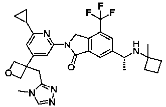 | / |
| Combination 14-11 | 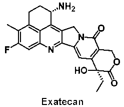 | 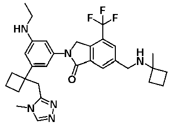 | / |
| Combination 14-12 | 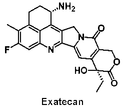 | 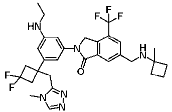 | / |
| Combination 27-1 | 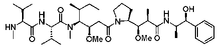 | 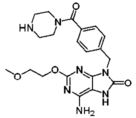 | / |
| Combination 27-2 | 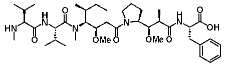 | 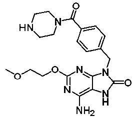 | / |
| Combination 27-3 | 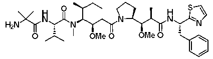 | 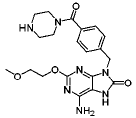 | / |
| Combination 27-4 | 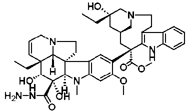 | 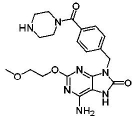 | / |
| Combination 27-5 | 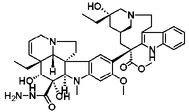 | 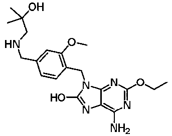 | / |
| Combination 15-1 | 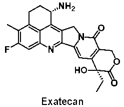 | 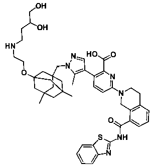 | / |
| Combination 15-2 | 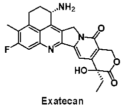 | 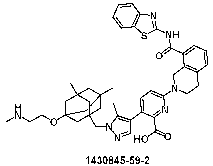 | / |
| Combination 15-3 | 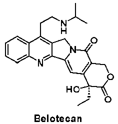 | 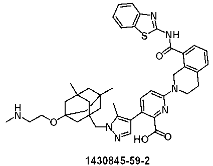 | / |
| Combination 27-1 | 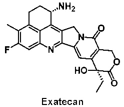 | 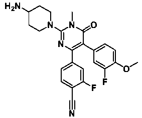 | |
| Combination 27-2 | 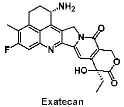 | 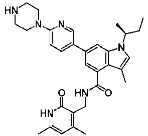 | / |
| Combination 26-1 | 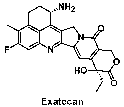 | 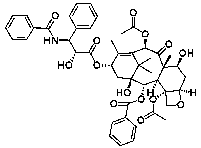 | / |
| Combination 61-1 | 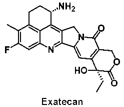 | 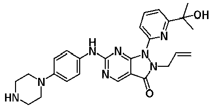 | / |
| Combination 8-1 | 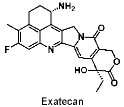 | 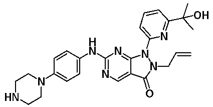 | 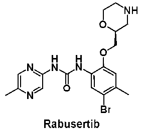 |
| Combination 8-2 | 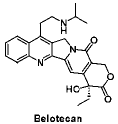 | 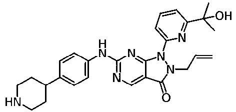 | 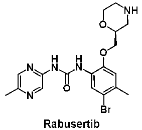 |
| Combination 62 | 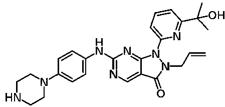 | 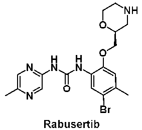 | / |
| Combination 63 | 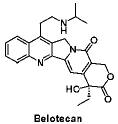 | 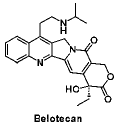 | / |
| Combination 63 | 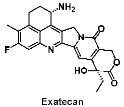 | 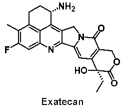 | / |
| Combination 71-1 | 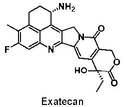 | 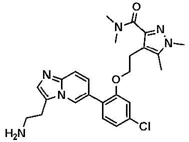 | / |
| Combination 71-2 | 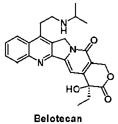 | 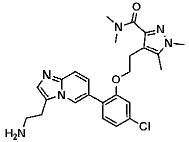 | / |
| Combination 71-3 | 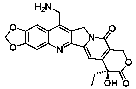 | 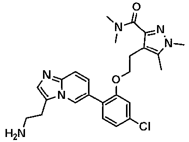 | / |
| Combination 71-4 | 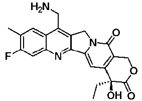 | 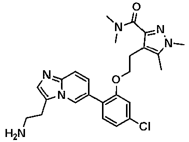 | / |
| Combination 72-1 | 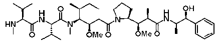 | 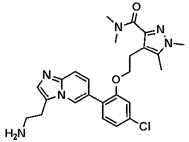 | / |
| Combination 72-2 | 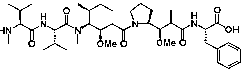 | 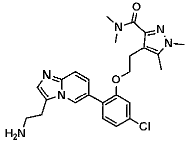 | / |
| Combination 72-3 | 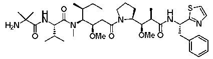 | 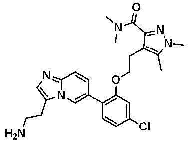 | / |
| Combination 72-4 | 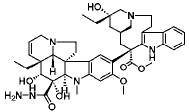 | 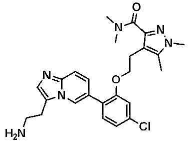 | / |

In the present invention, the embodiments or preferred embodiments of each aforementioned component (such as Q', C₁, B₁, B₂, T₁, T₂, T₃, P₁, P₂, P₃, D₁, D₂, D₃, hydrophilic group) may be individually applicable to formula II, IIa, or IIb, or applicable in various Combinations to formula II, IIa, or IIb. When the combination is applicable, the embodiments or preferred embodiments of each component may be combined in whole or in part.

In preferred embodiments, the present invention provides compounds selected from formulas **B1 to B190,** or a tautomer, a mesomer, a racemate, an enantiomer, or a diastereomer thereof, or a deuterated compound form thereof, or a mixture form thereof, or a pharmaceutically acceptable salt, prodrug, or solvate thereof.

| | |
|---|---|
| 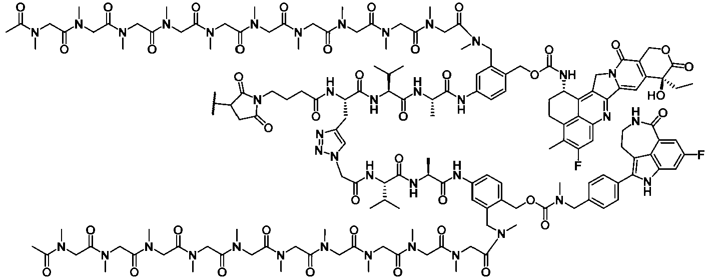 | Formula B1 |
| 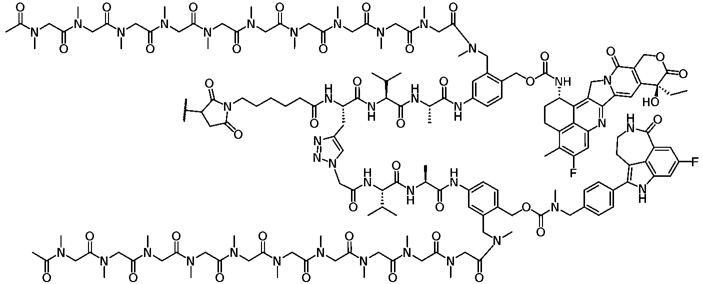 | Formula B2 |
| 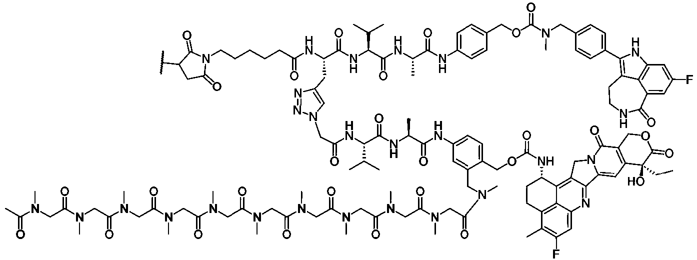 | Formula B3 |
| 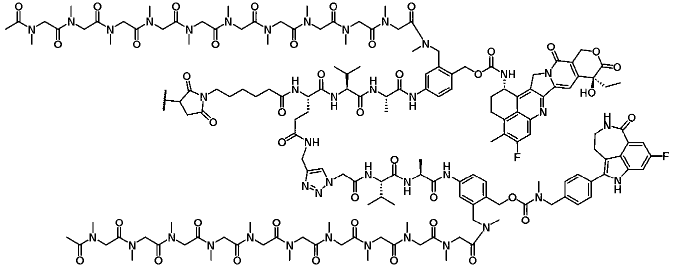 | Formula B4 |
| 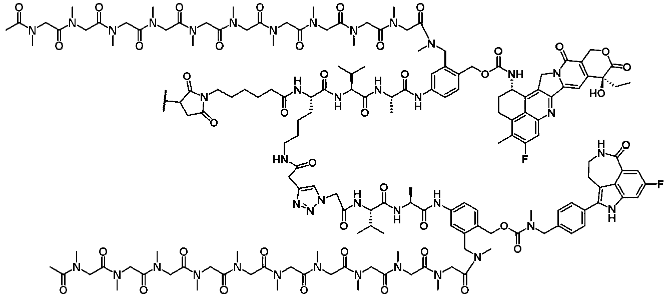 | Formula B5 |
| 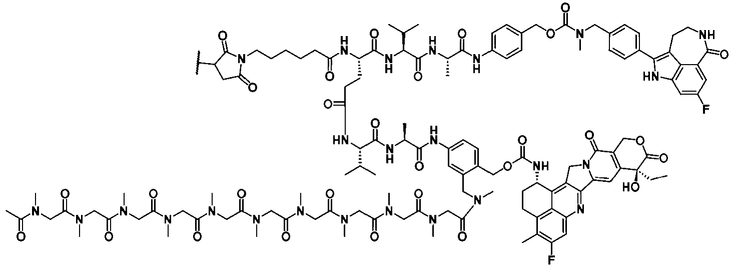 | Formula B6 |
| 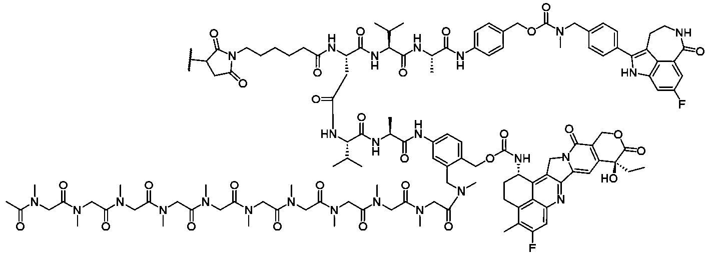 | Formula B7 |
| 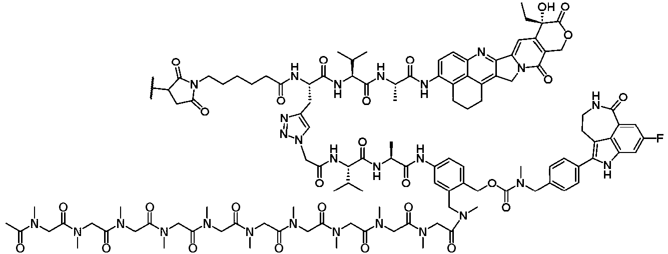 | Formula B8 |
| 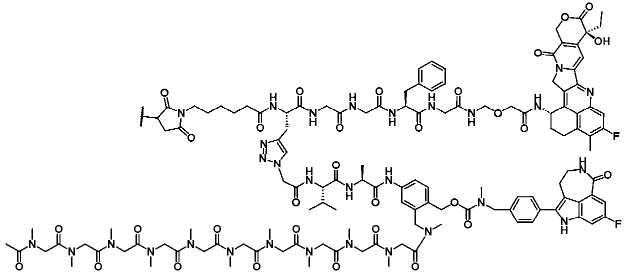 | Formula B9 |
| 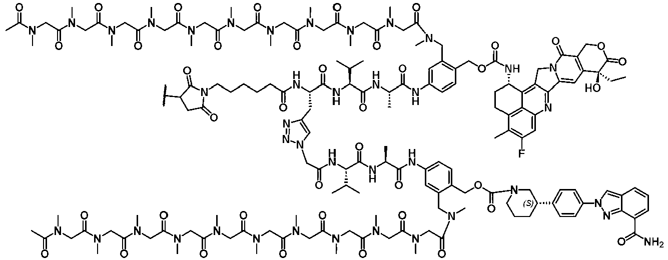 | Formula B10 |
| 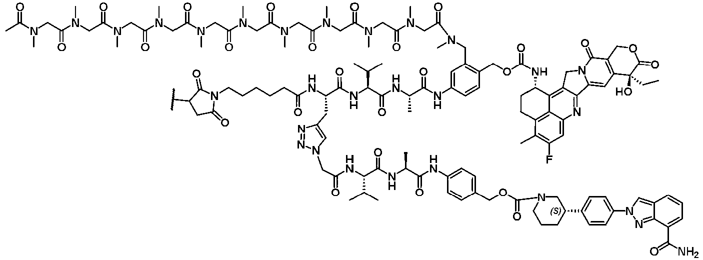 | Formula B11 |
| 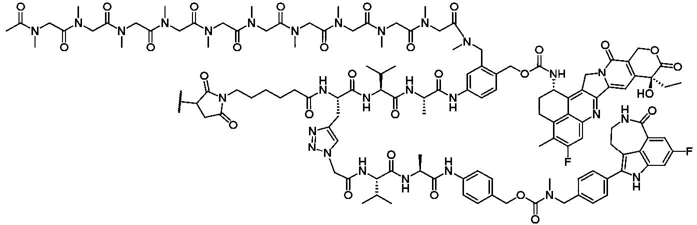 | Formula B12 |
| 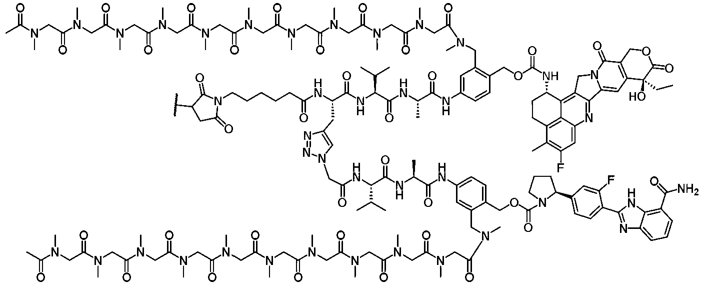 | Formula B13 |
| 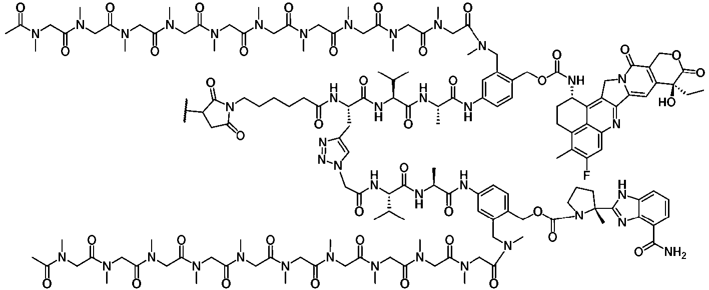 | Formula B14 |
| 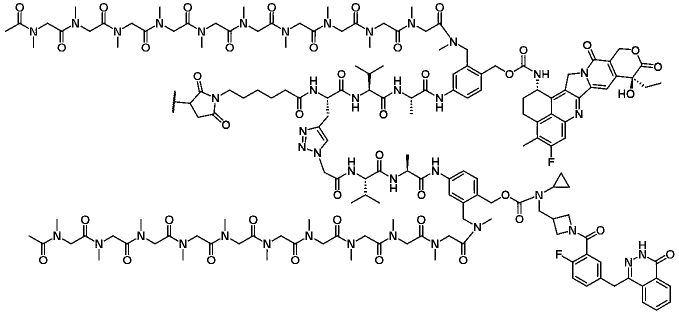 | Formula B15 |
| 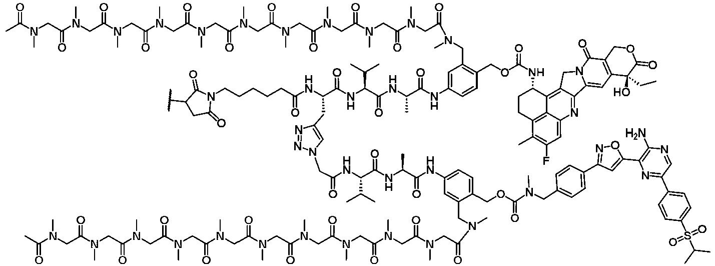 | Formula B16 |
| 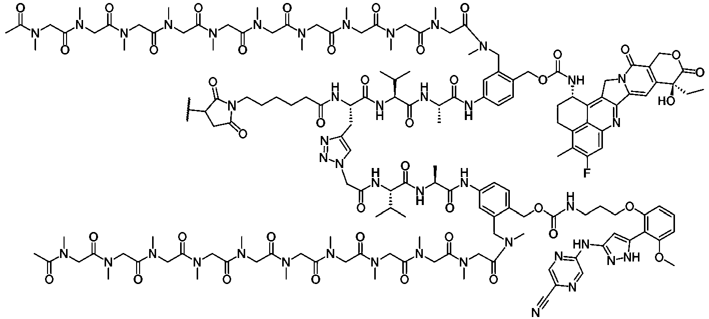 | Formula B17 |
| 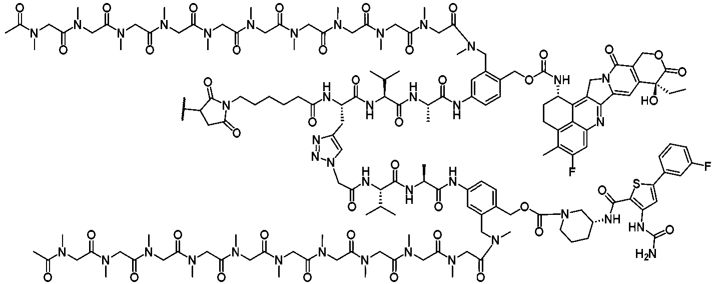 | Formula B18 |
| 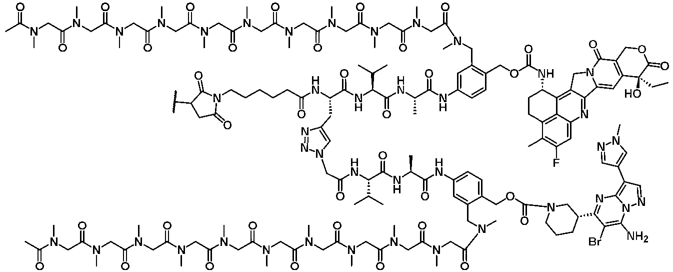 | Formula B19 |
| 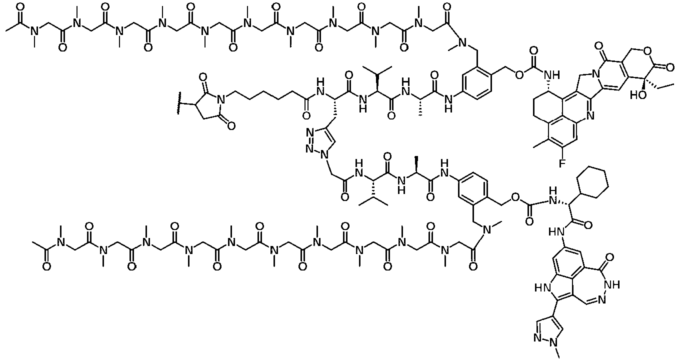 | Formula B20 |
| 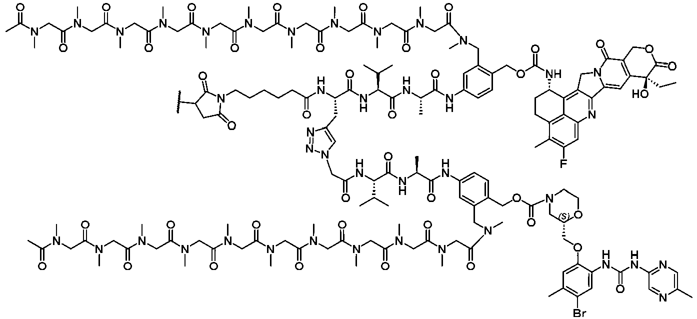 | Formula B21 |
| 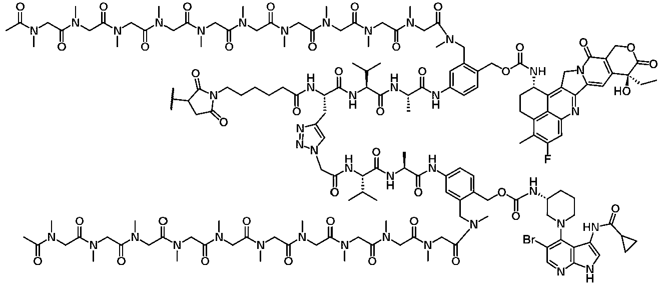 | Formula B22 |
| 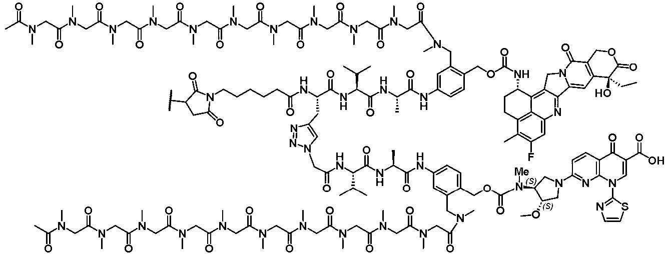 | Formula B23 |
| 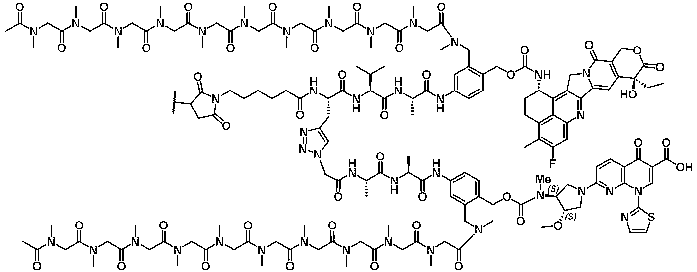 | Formula B24 |
| 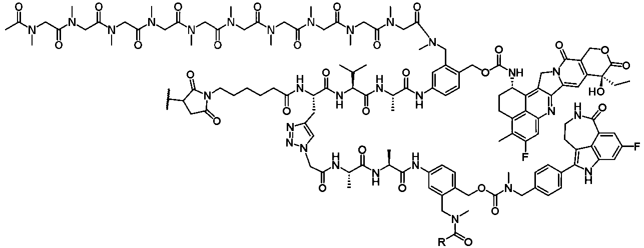 | Formula B25 |
| 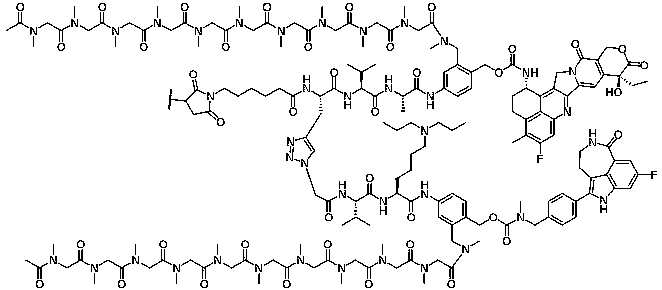 | Formula B26 |
| 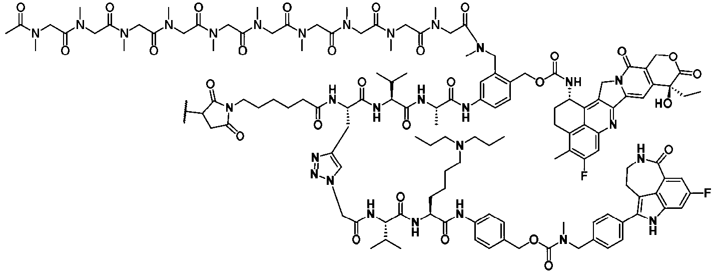 | Formula B27 |
| 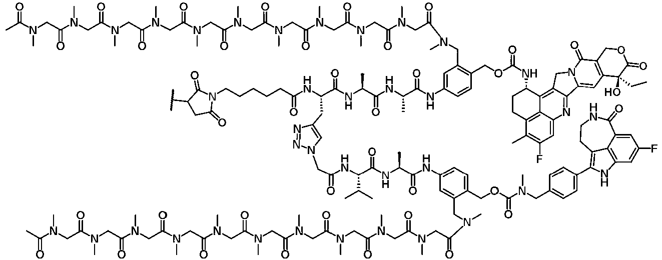 | Formula B28 |
| 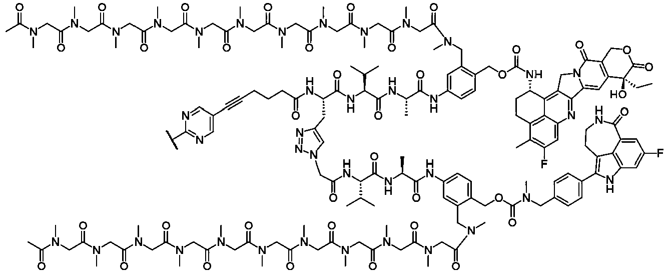 | Formula B29 |
| 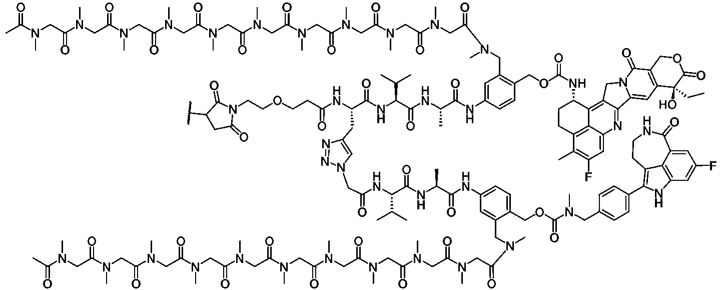 | Formula B30 |
| 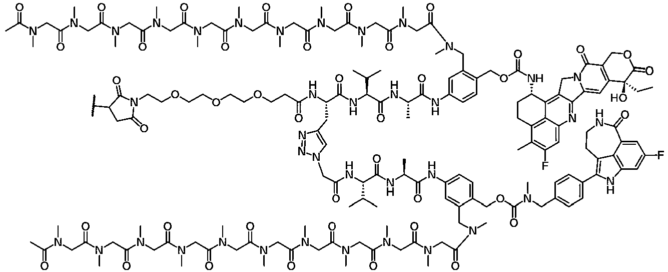 | Formula B31 |
| 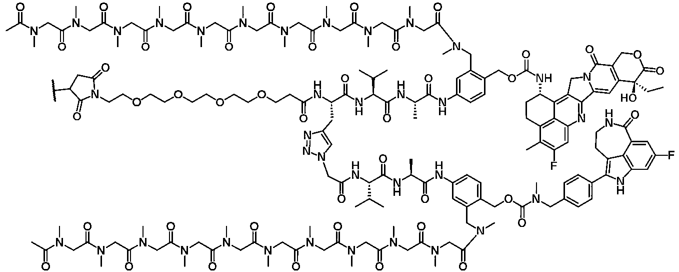 | Formula B32 |
| 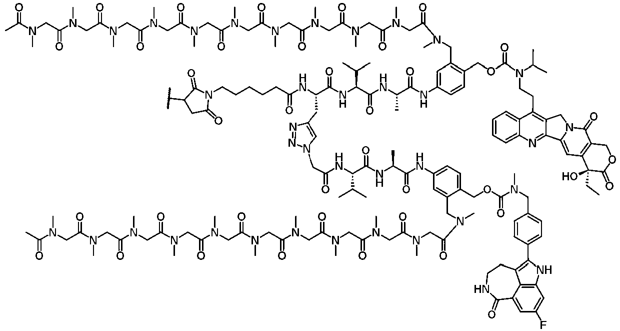 | Formula B33 |
| 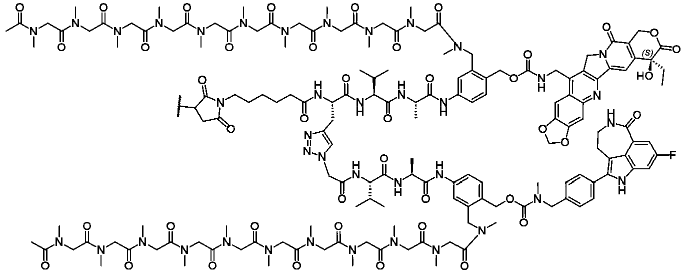 | Formula B34 |
| 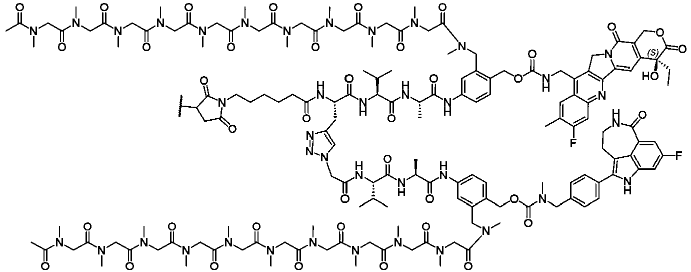 | Formula B35 |
| 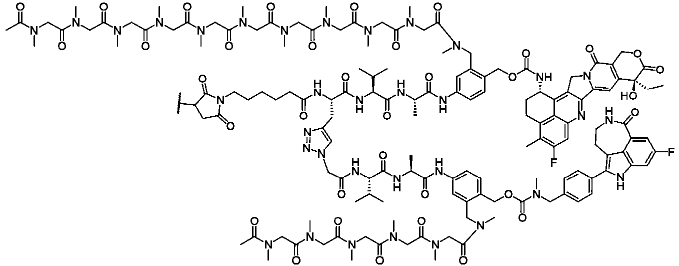 | Formula B36 |
| 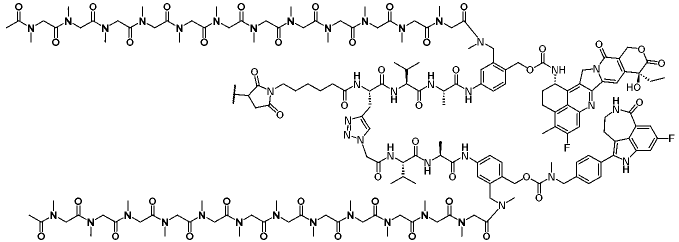 | Formula B37 |
| 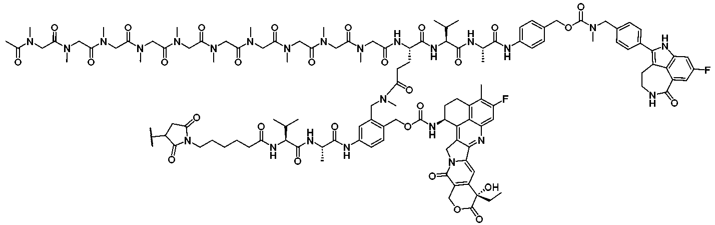 | Formula B38 |
| 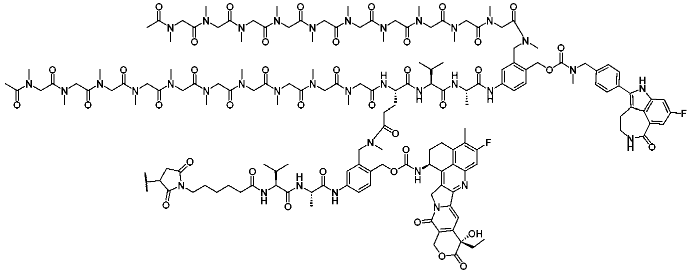 | Formula B39 |
| 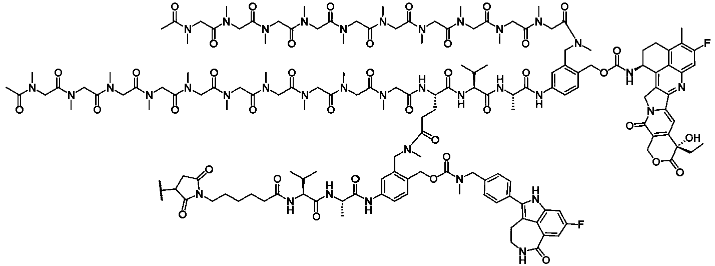 | Formula B40 |
| 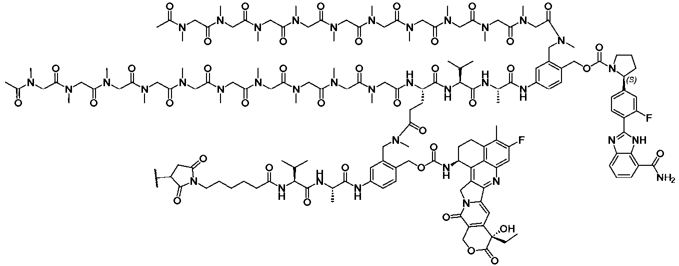 | Formula B41 |
| 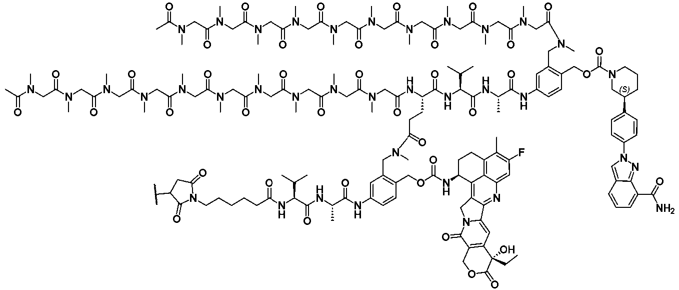 | Formula B42 |
| 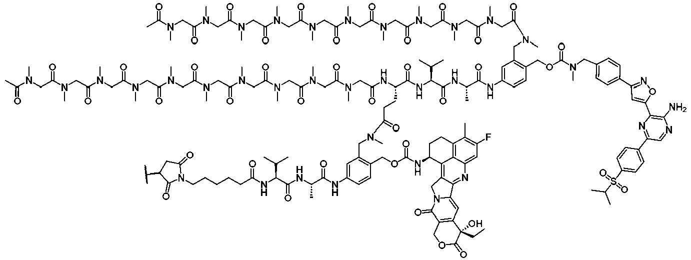 | Formula B43 |
| 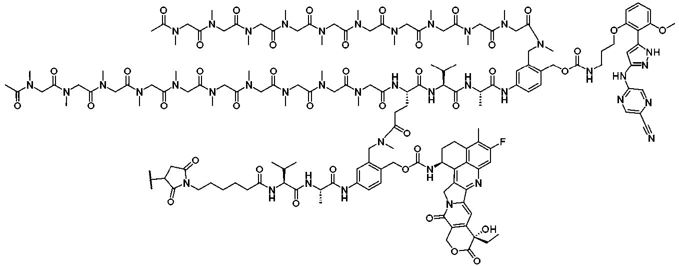 | Formula B44 |
| 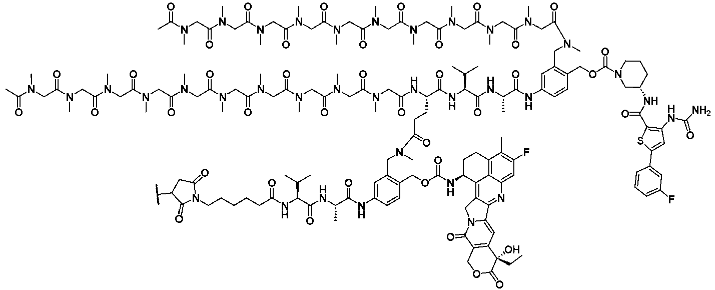 | Formula B45 |
| 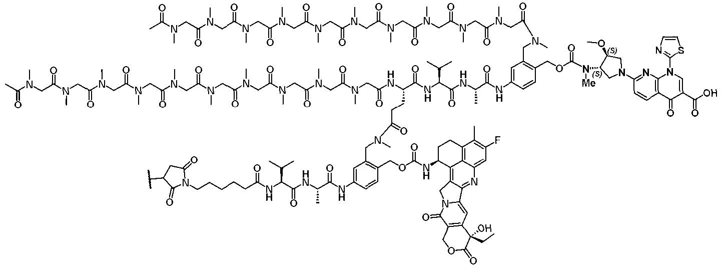 | Formula B46 |
| 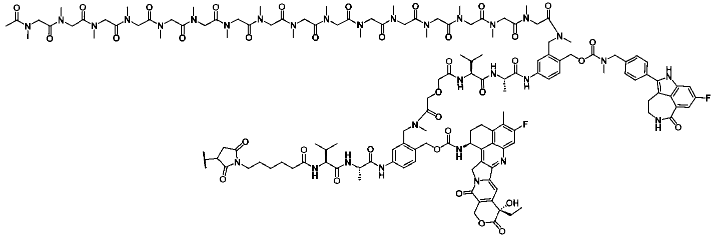 | Formula B47 |
| 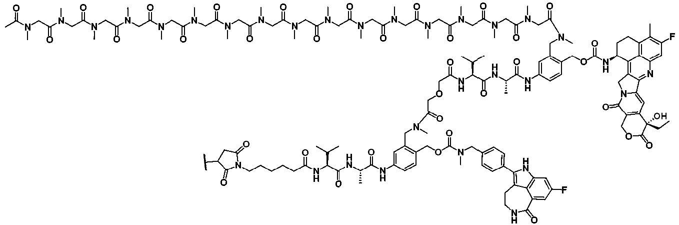 | Formula B48 |
| 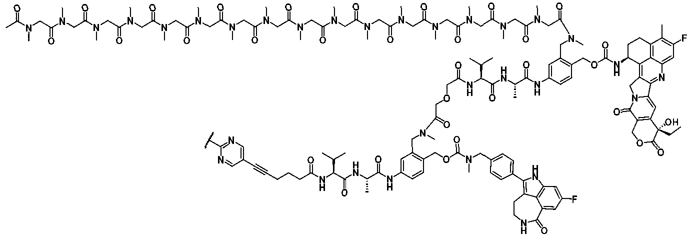 | Formula B49 |
| 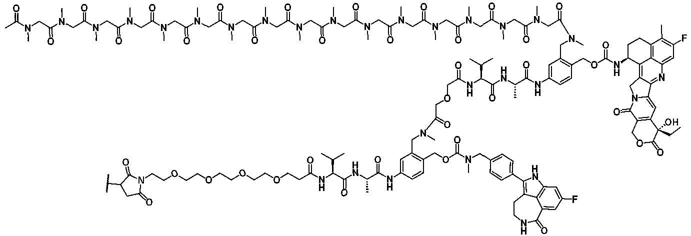 | Formula B50 |
| 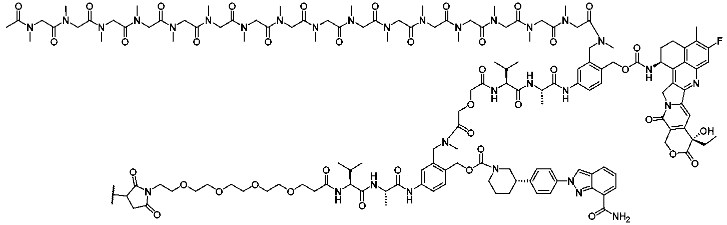 | Formula B51 |
| 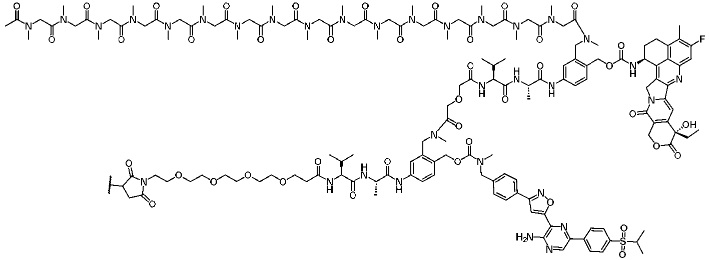 | Formula B52 |
| 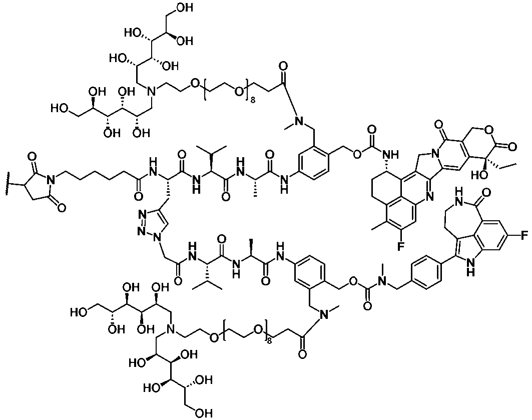 | Formula B53 |
| 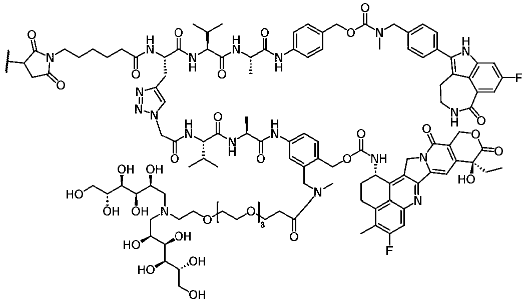 | Formula B54 |
| 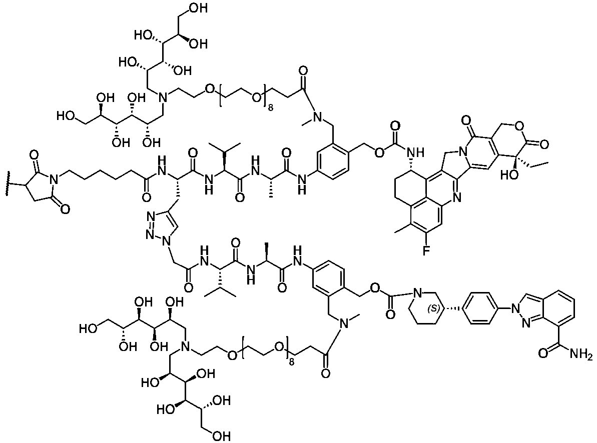 | Formula B55 |
| 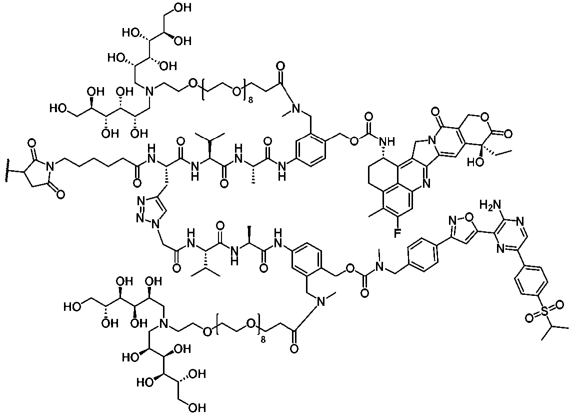 | Formula B56 |
| 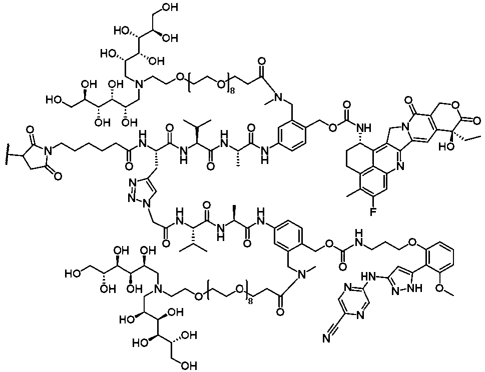 | Formula B57 |
| 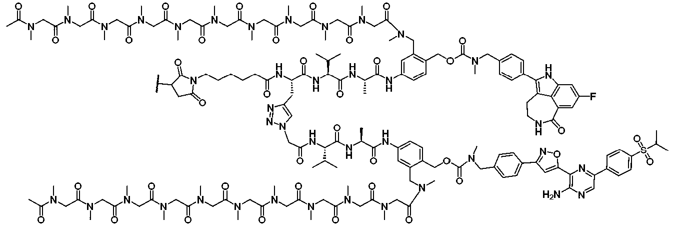 | Formula B58 |
| 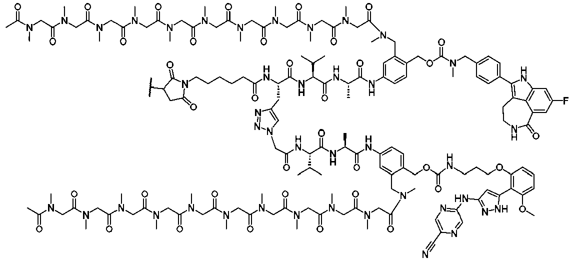 | Formula B59 |
| 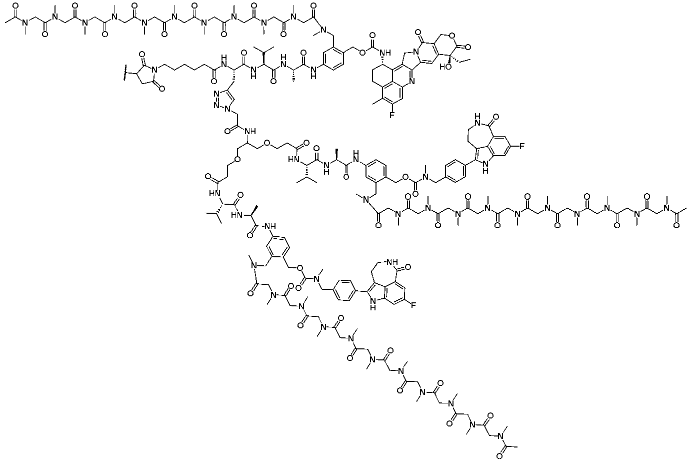 | Formula B60 |
| 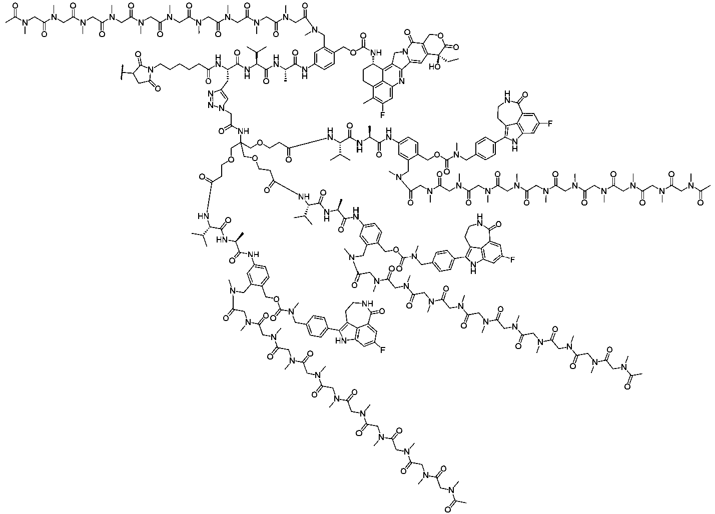 | Formula B61 |
| 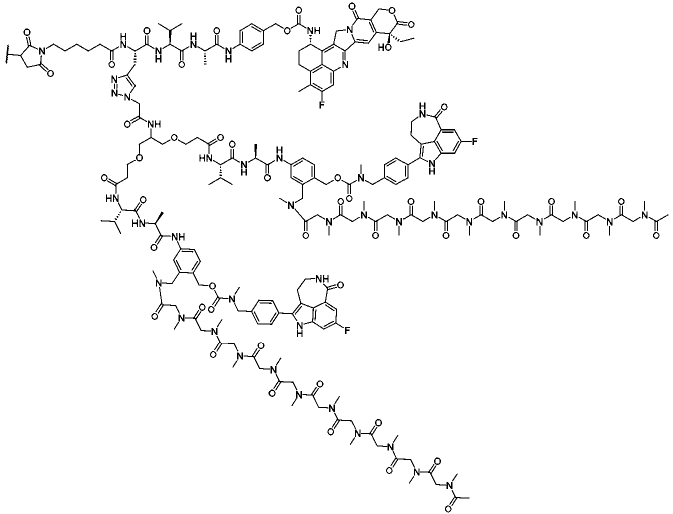 | Formula B62 |
| 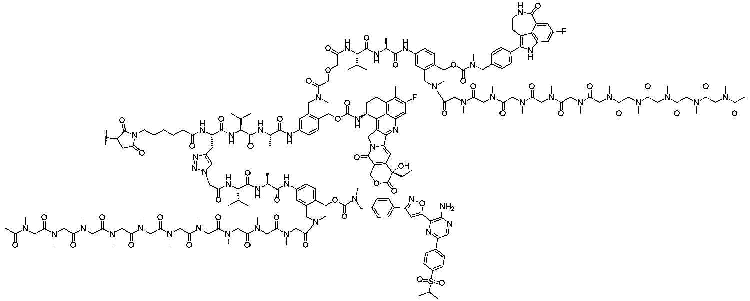 | Formula B63 |
| 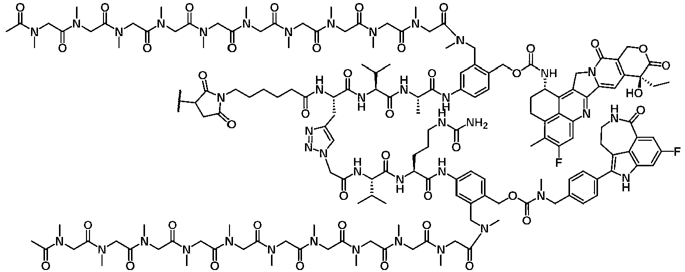 | Formula B64 |
| 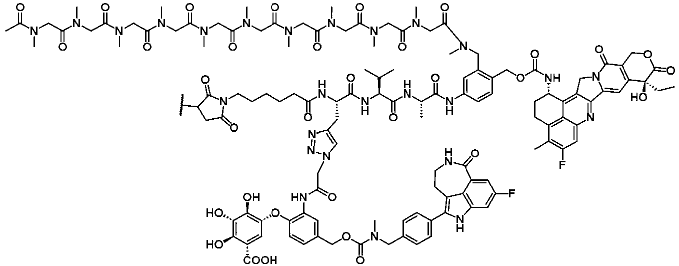 | Formula B65 |
| 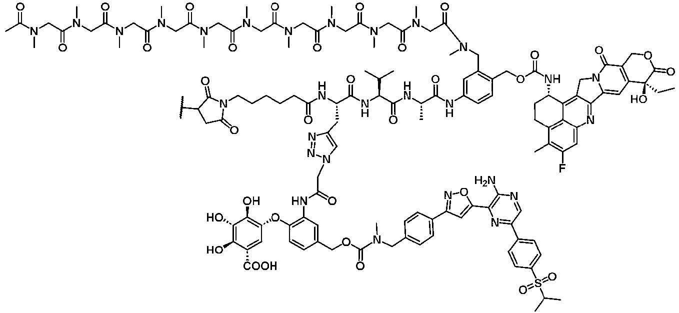 | Formula B66 |
| 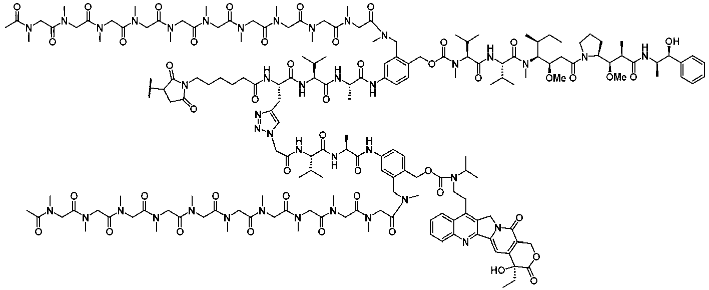 | Formula B67 |
| 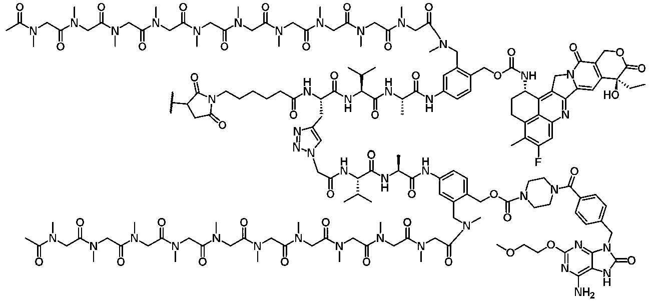 | Formula B68 |
| 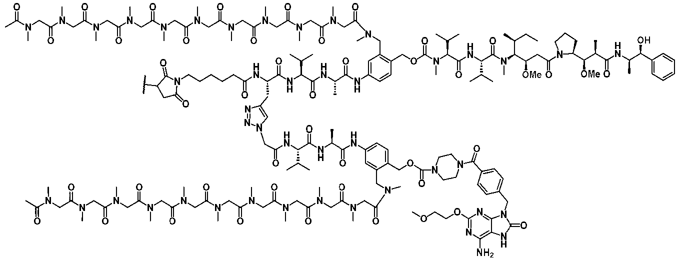 | Formula B69 |
| 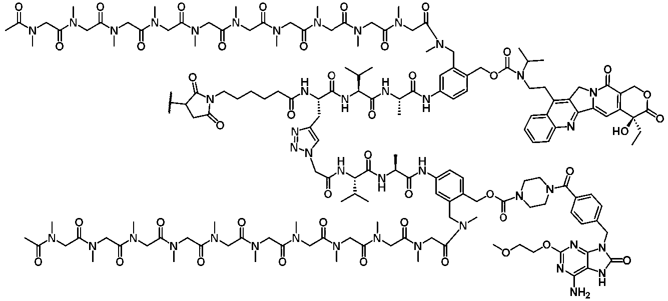 | Formula B70 |
| 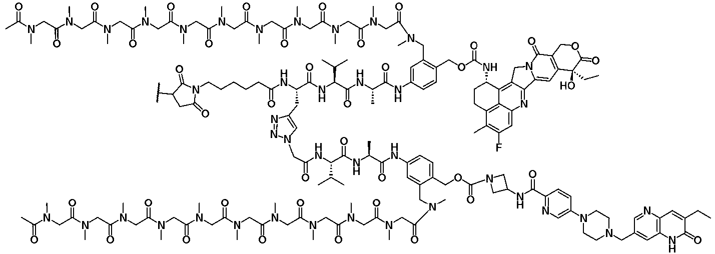 | Formula B71 |
| 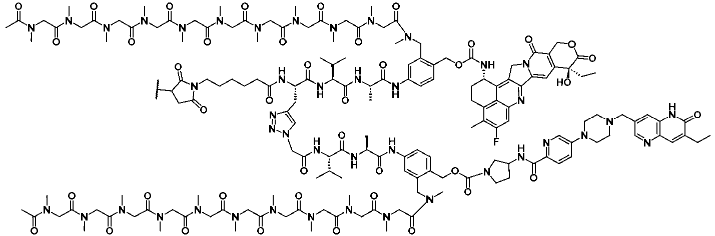 | Formula B72 |
| 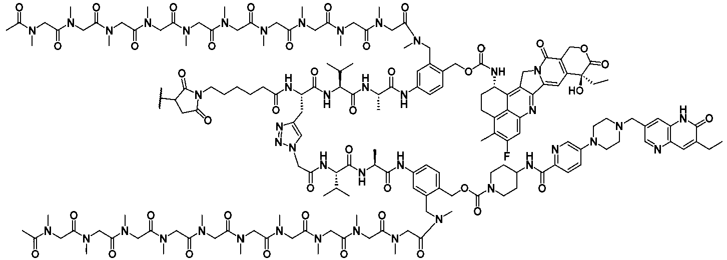 | Formula B73 |
| 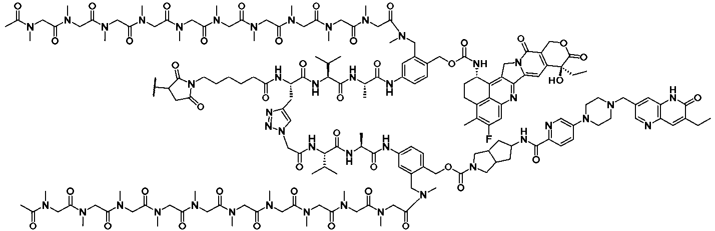 | Formula B74 |
| 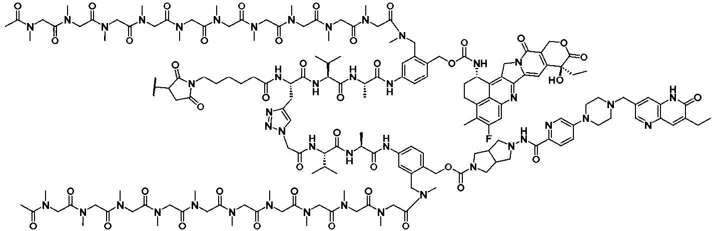 | Formula B75 |
| 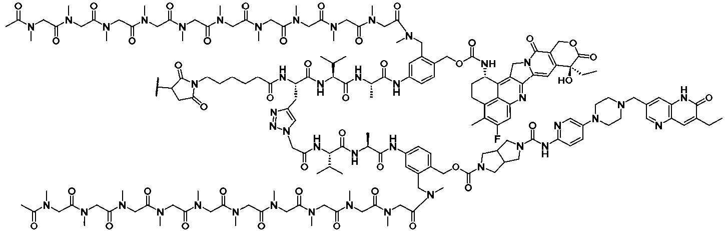 | Formula B76 |
| 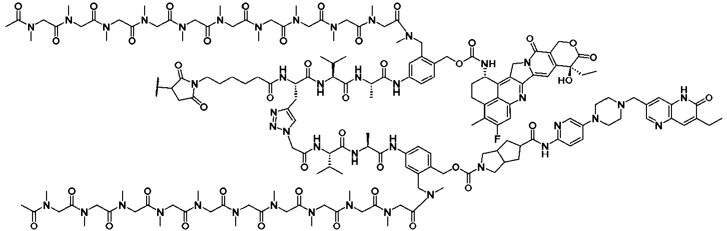 | Formula B77 |
| 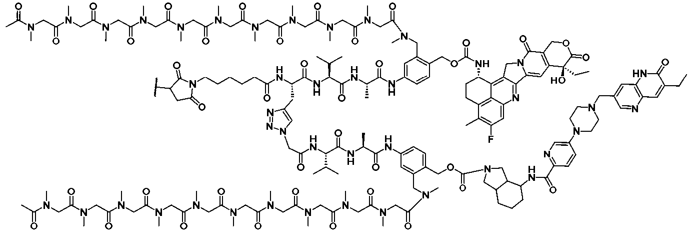 | Formula B78 |
| 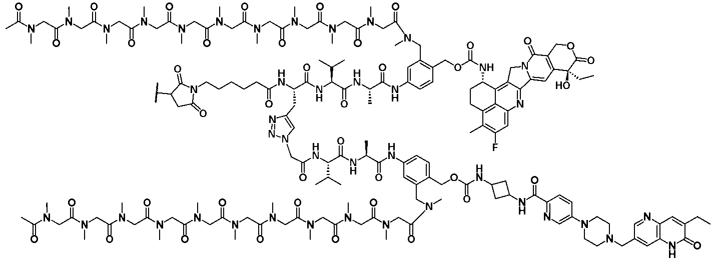 | Formula B79 |
| 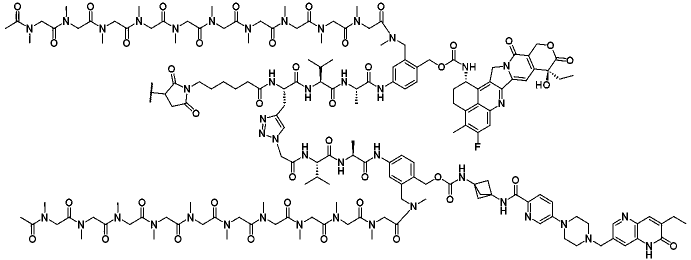 | Formula B80 |
| 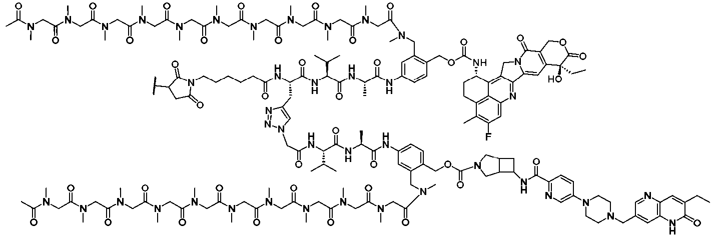 | Formula B81 |
| 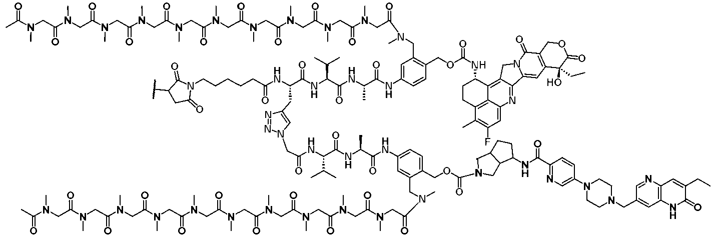 | Formula B82 |
| 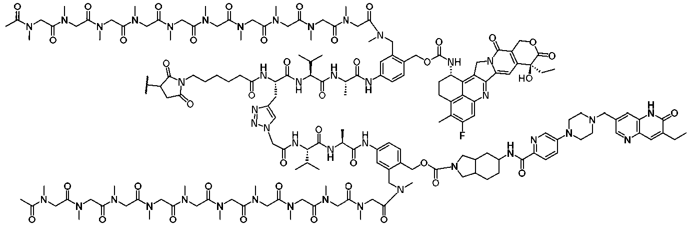 | Formula B83 |
| 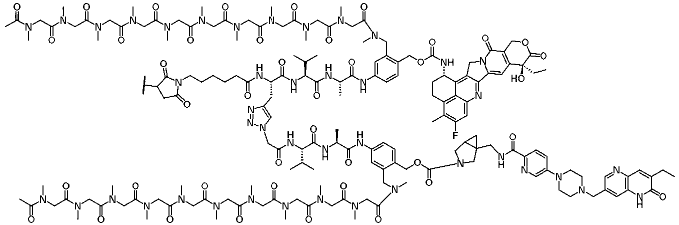 | Formula B84 |
| 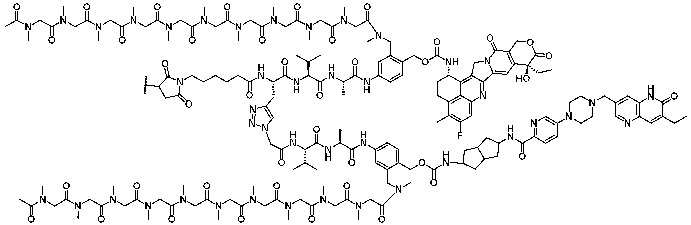 | Formula B85 |
| 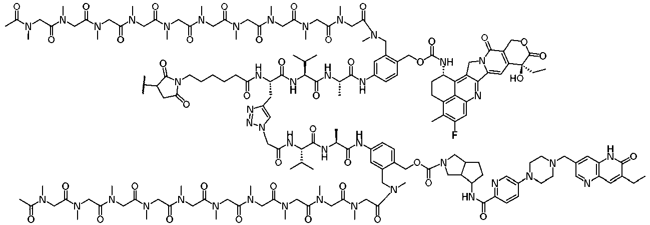 | Formula B86 |
| 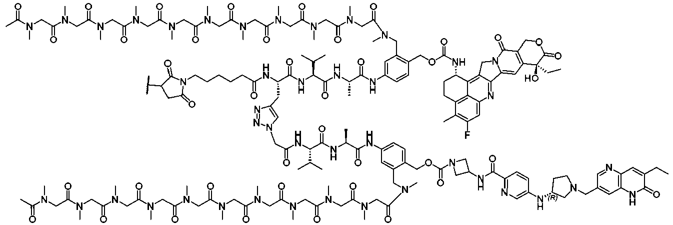 | Formula B87 |
| 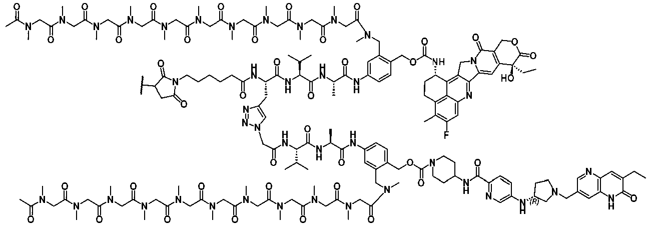 | Formula B88 |
| 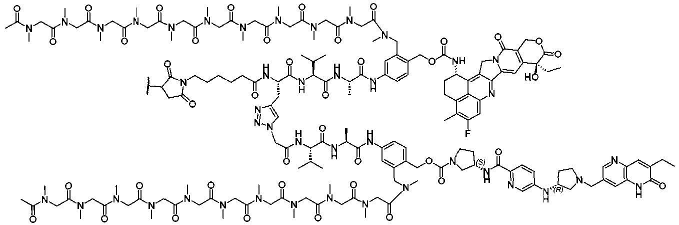 | Formula B89 |
| 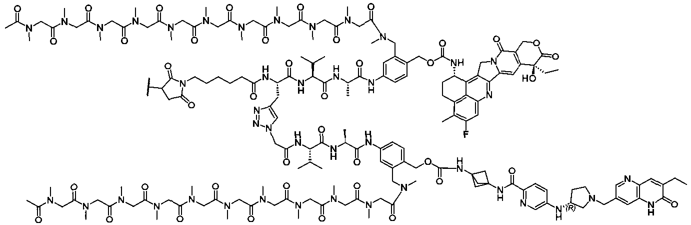 | Formula B90 |
| 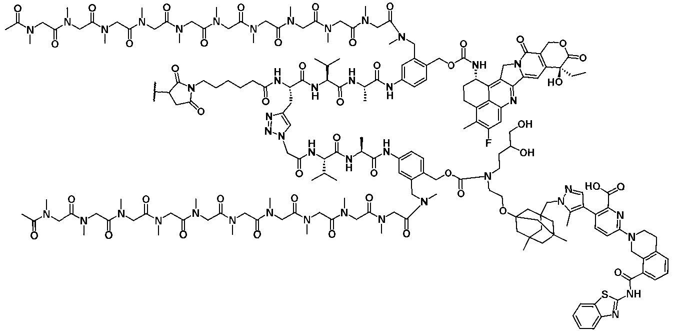 | Formula B91 |
| 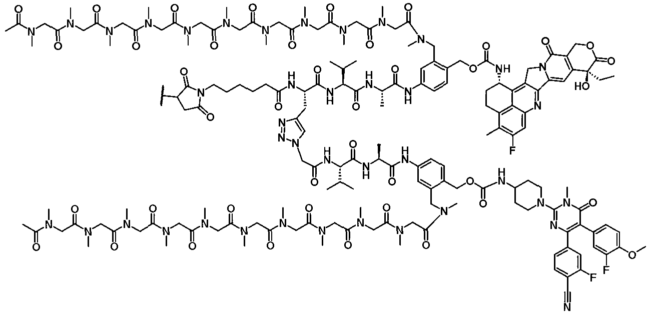 | Formula B92 |
| 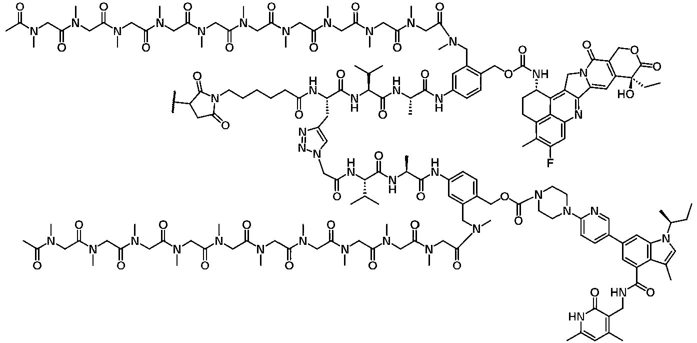 | Formula B93 |
| 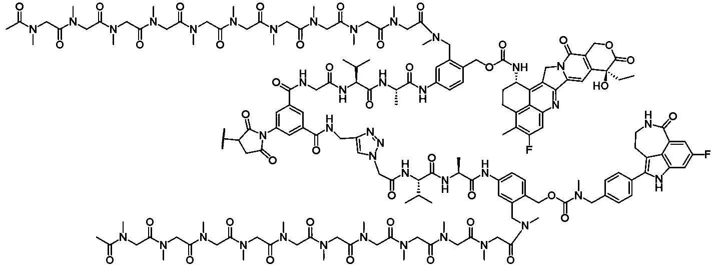 | Formula B94 |
| 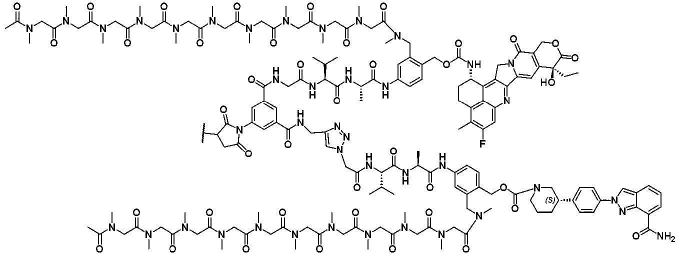 | Formula B95 |
| 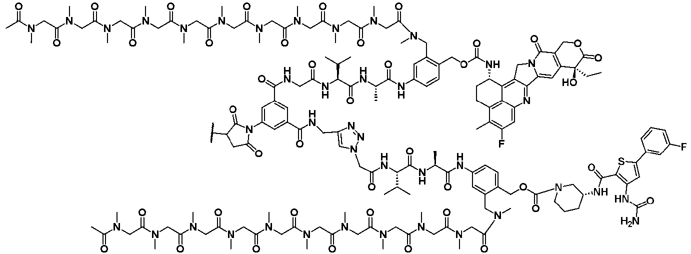 | Formula B96 |
| 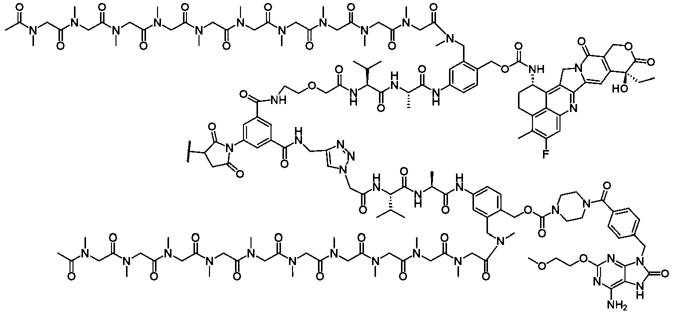 | Formula B97 |
| 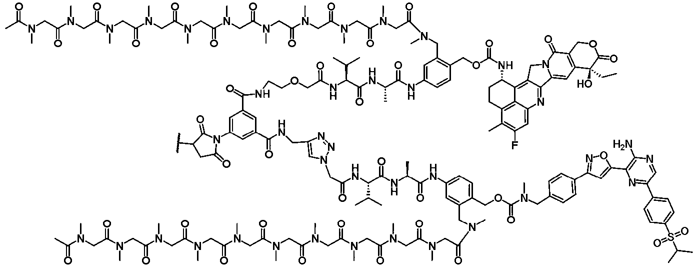 | Formula B98 |
| 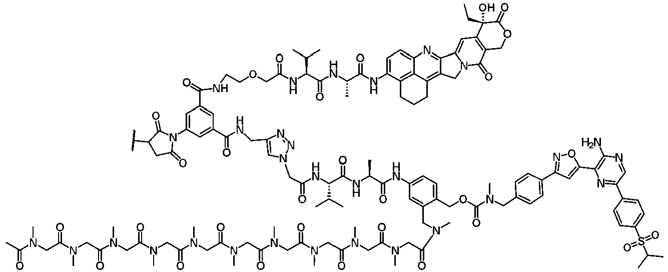 | Formula B99 |
| 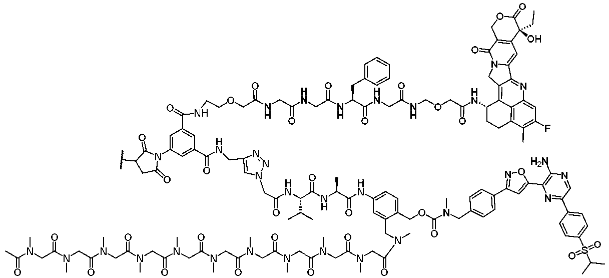 | Formula B100 |
| 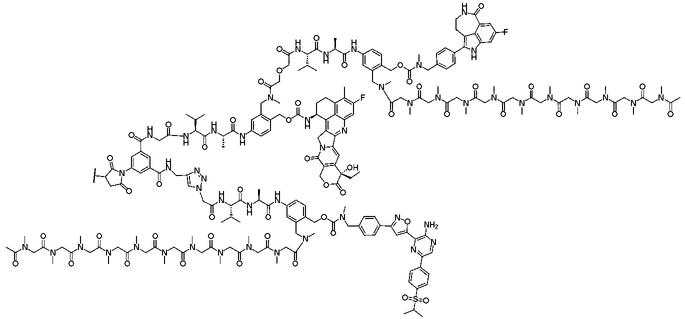 | Formula B101 |
| 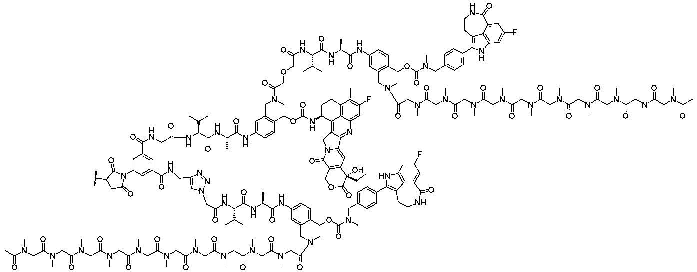 | Formula B102 |
| 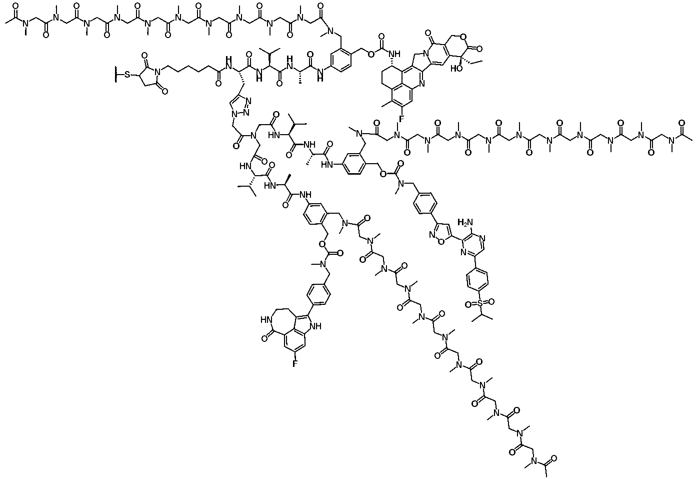 | Formula B103 |
| 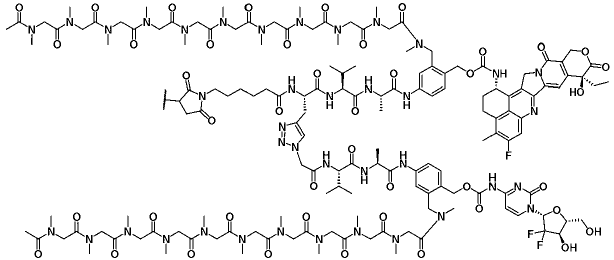 | Formula B104 |
| 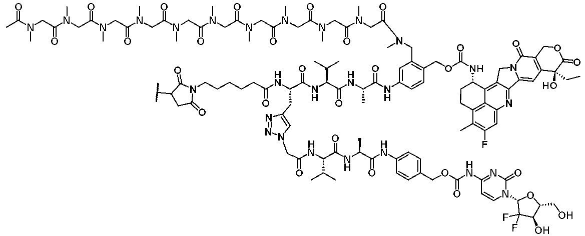 | Formula B105 |
| 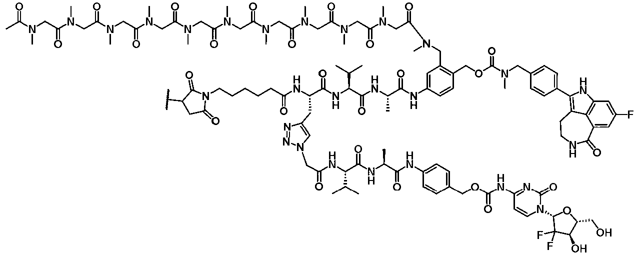 | Formula B106 |
| 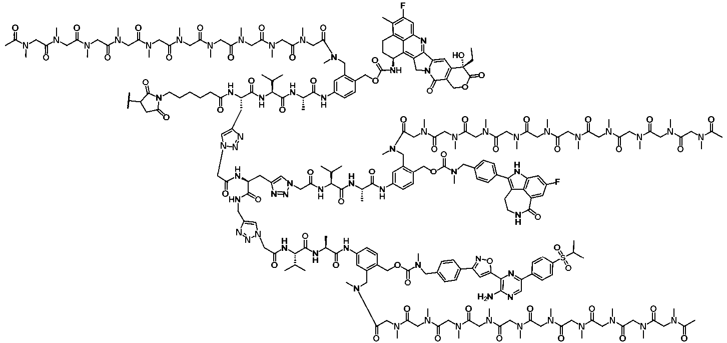 | Formula B107 |
| 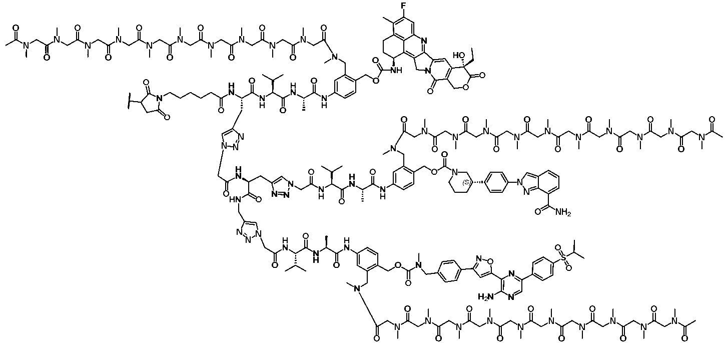 | Formula B108 |
| 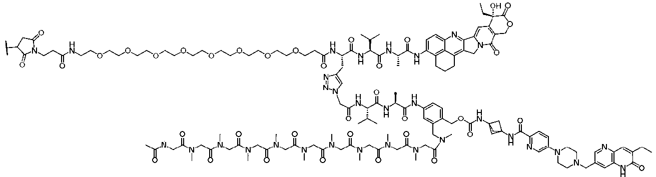 | Formula B109 |
| 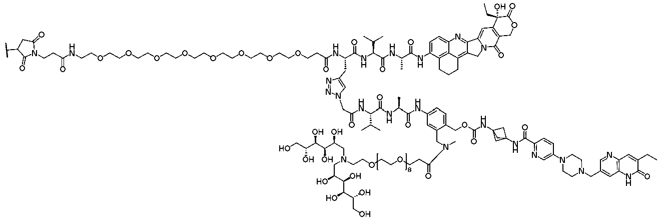 | Formula B110 |
| 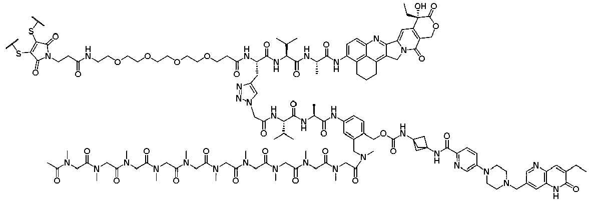 | Formula B111 |
| 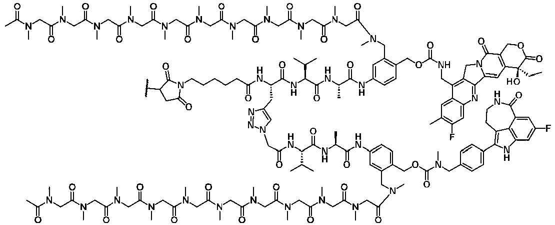 | Formula B112 |
| 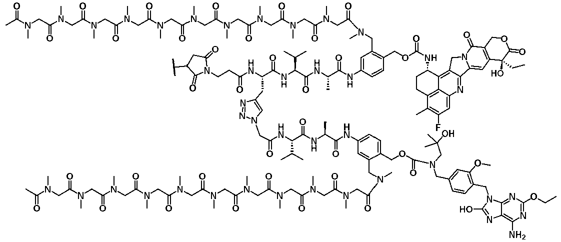 | Formula B113 |
| 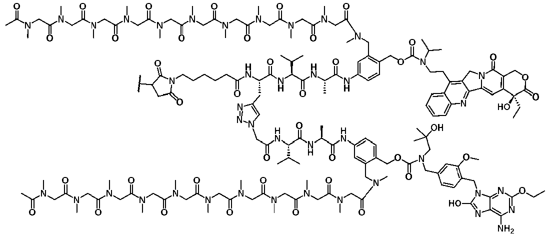 | Formula B114 |
| 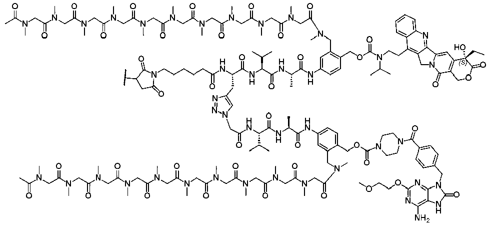 | Formula B115 |
| 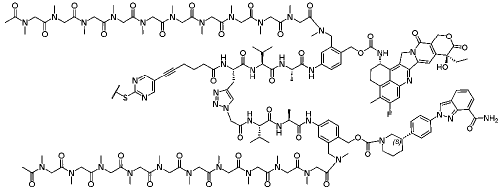 | Formula B116 |
| 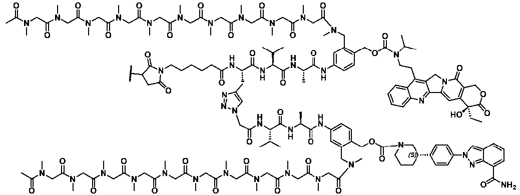 | Formula B117 |
| 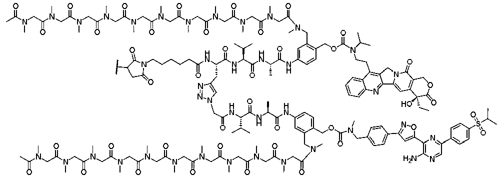 | Formula B118 |
| 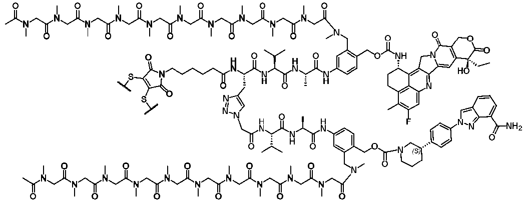 | Formula B119 |
| 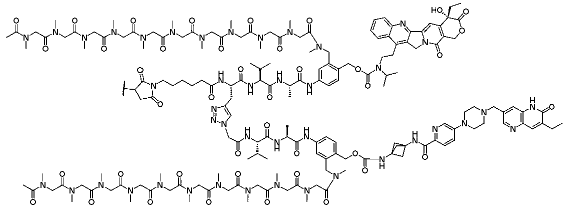 | Formula B120 |
| 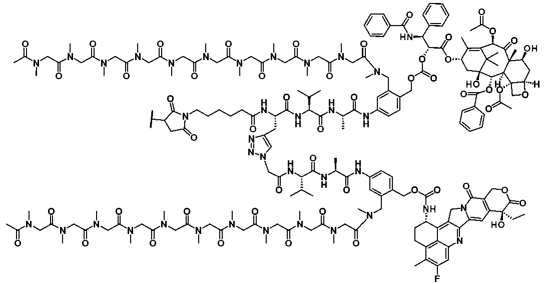 | Formula B121 |
| 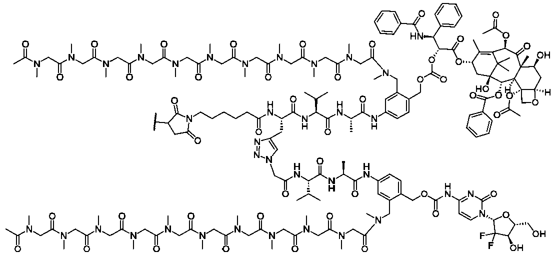 | Formula B122 |
| 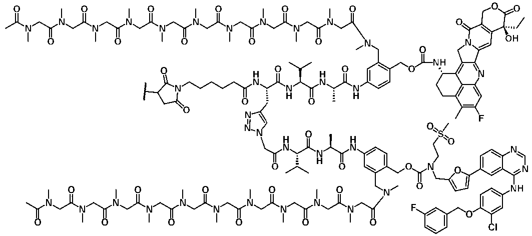 | Formula B123 |
| 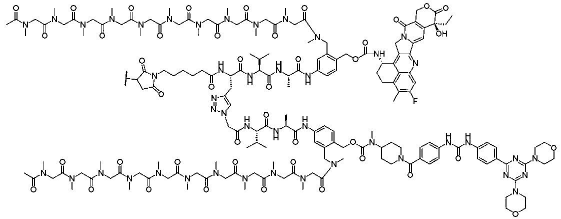 | Formula B125 |
| 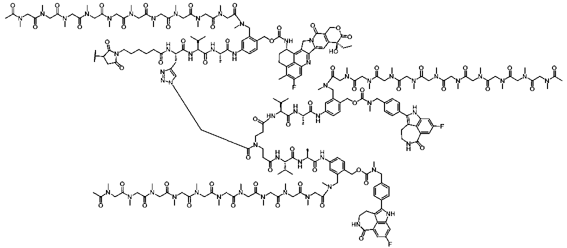 | Formula B126 |
| 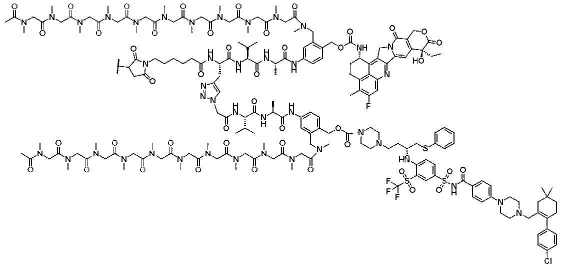 | Formula B127 |
| 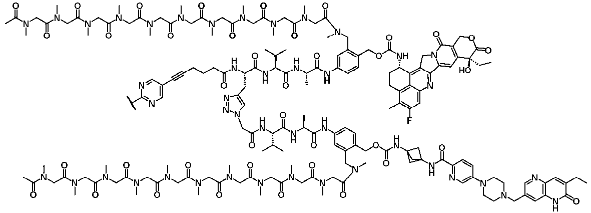 | Formula B128 |
| 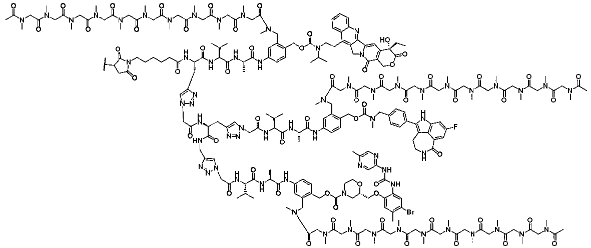 | Formula B129 |
| 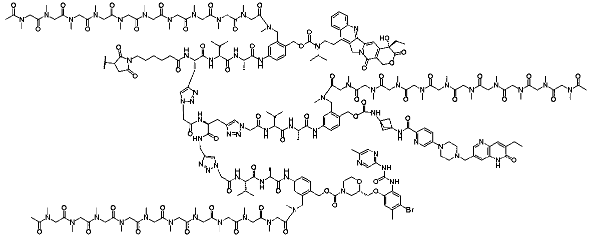 | Formula B130 |
| 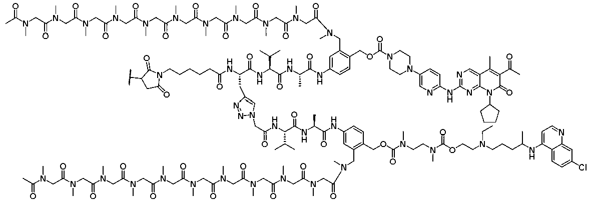 | Formula B131 |
| 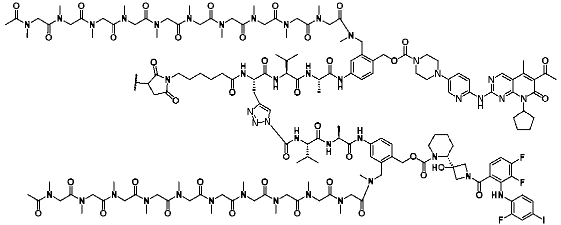 | Formula B132 |
| 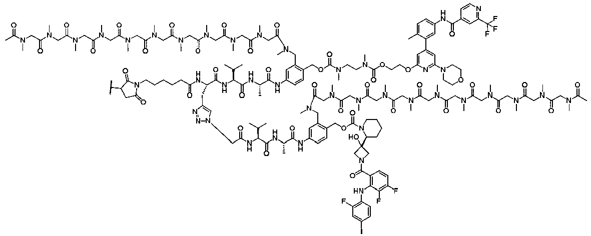 | Formula B133 |
| 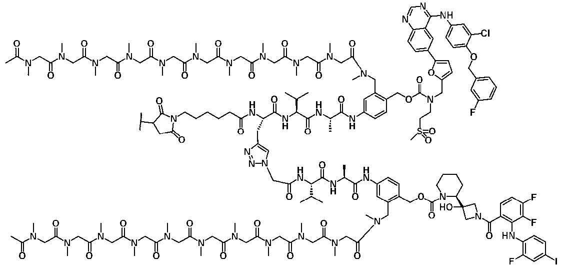 | Formula B134 |
| 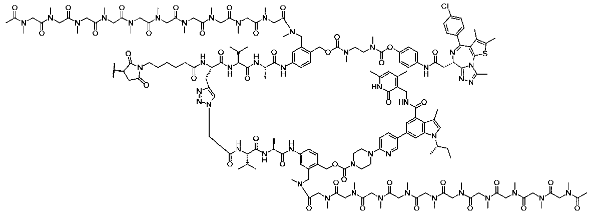 | Formula B135 |
| 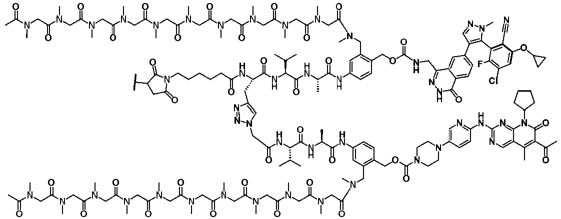 | Formula B136 |
| 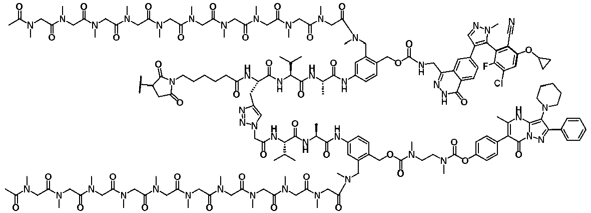 | Formula B137 |
| 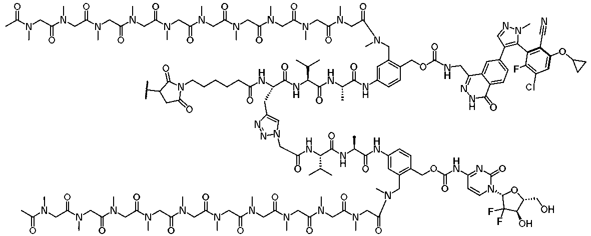 | Formula B138 |
| 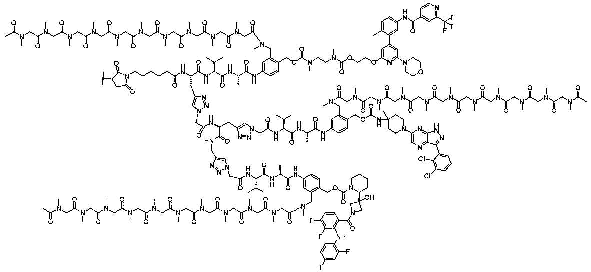 | Formula B139 |
| 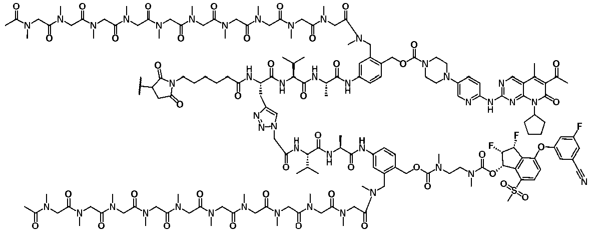 | Formula B140 |
| 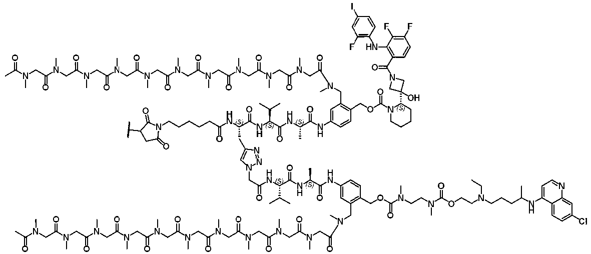 | Formula B141 |
| 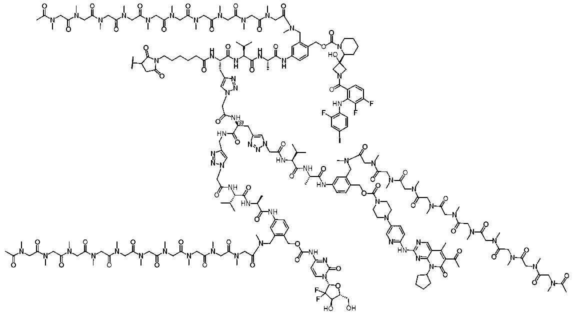 | Formula B142 |
| 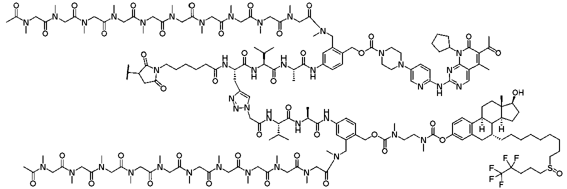 | Formula B143 |
| 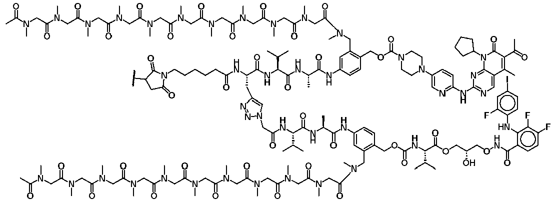 | Formula B144 |
| 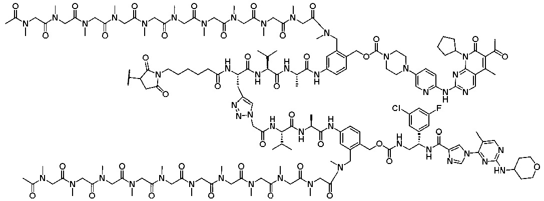 | Formula B145 |
| 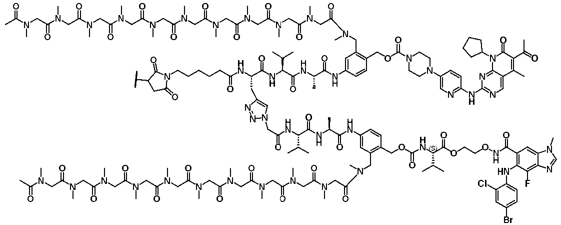 | Formula B146 |
| 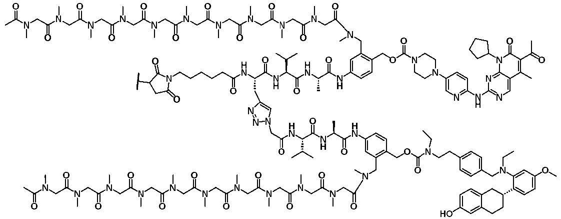 | Formula B147 |
| 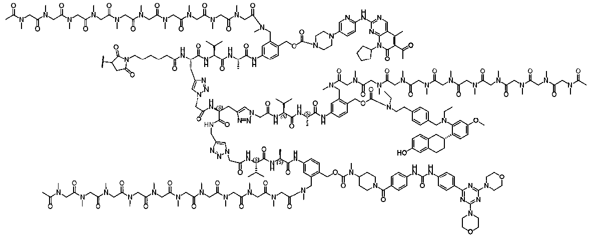 | Formula B148 |
| 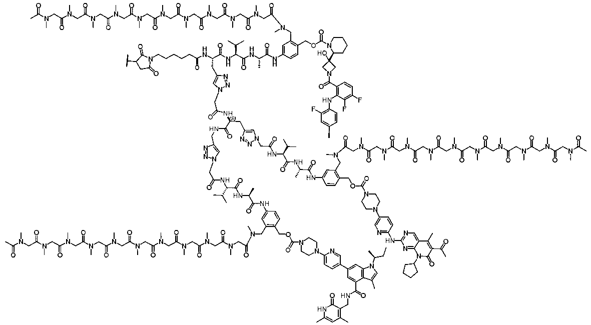 | Formula B149 |
| 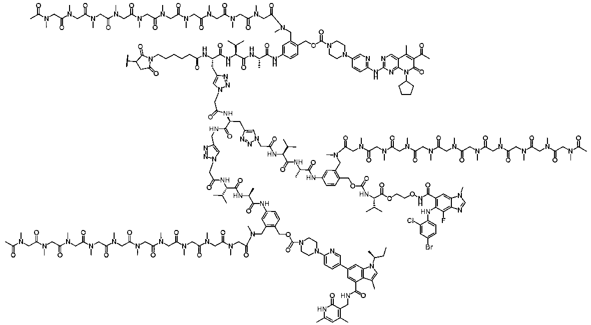 | Formula B150 |
| 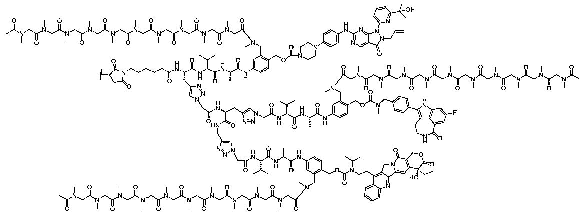 | Formula B151 |
| 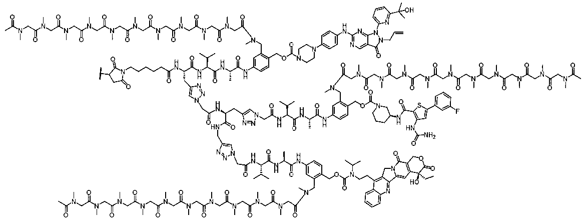 | Formula B152 |
| 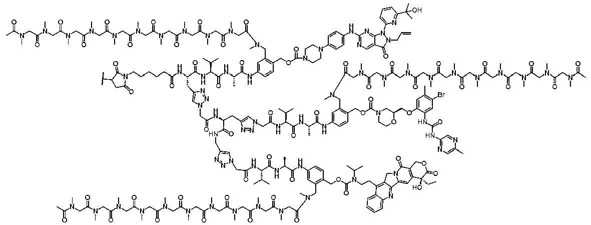 | Formula B153 |
| 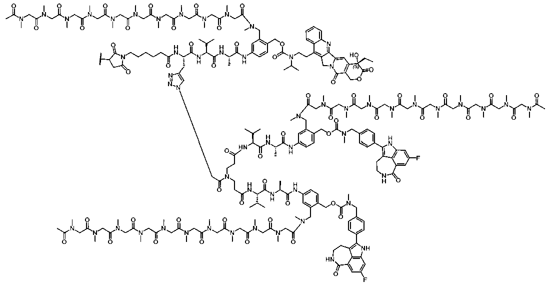 | Formula B154 |
| 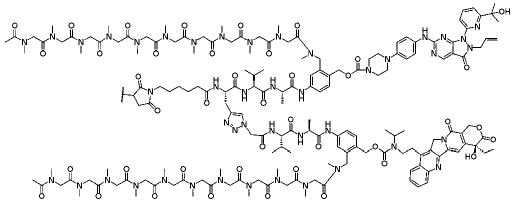 | Formula B155 |
| 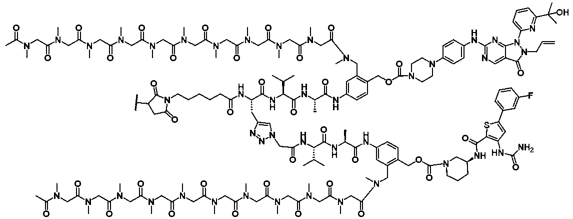 | Formula B156 |
| 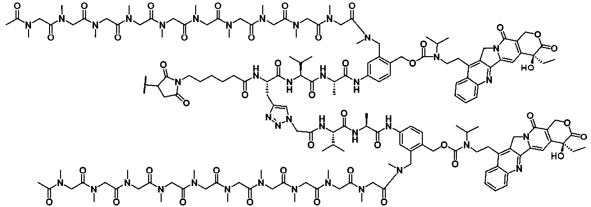 | Formula B157 |
| 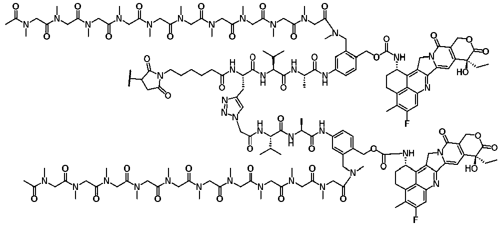 | Formula B158 |
| 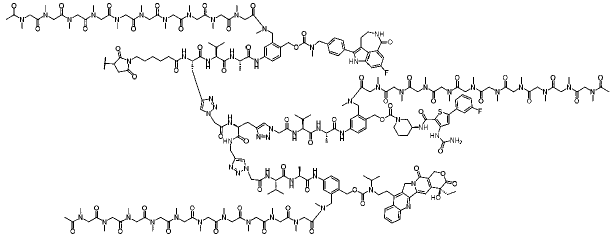 | Formula B159 |
| 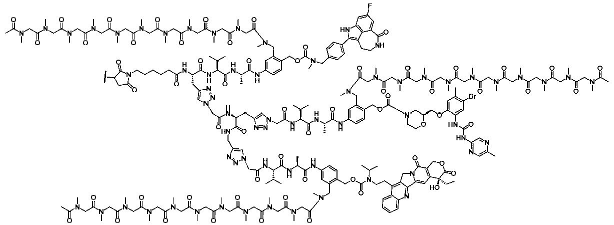 | Formula B160 |
| 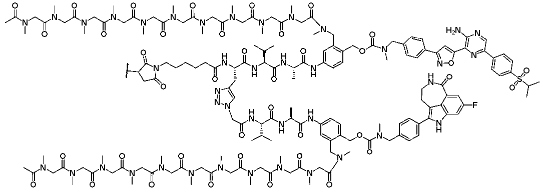 | Formula B161 |
| 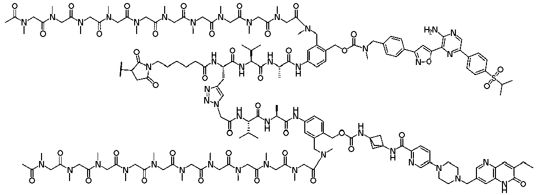 | Formula B162 |
| 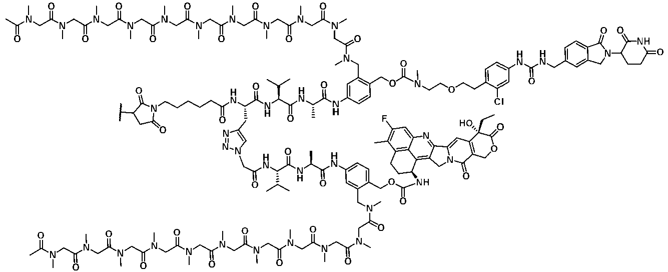 | Formula B163 |
| 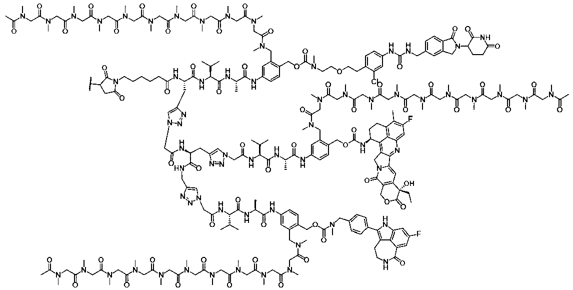 | Formula B164 |
| 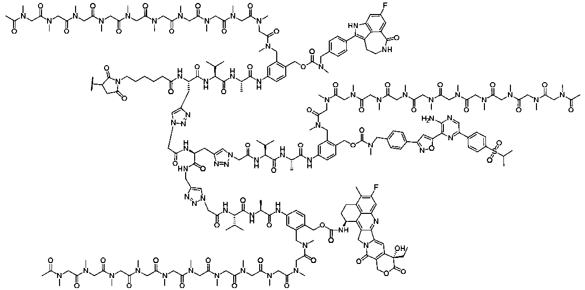 | Formula B165 |
| 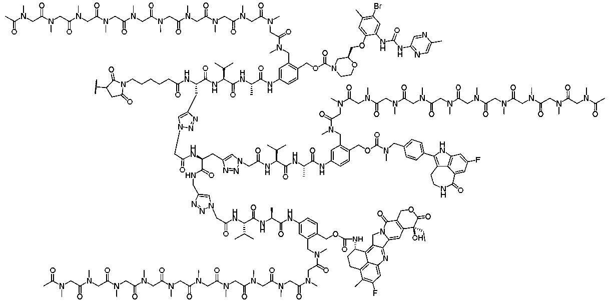 | Formula B166 |
| 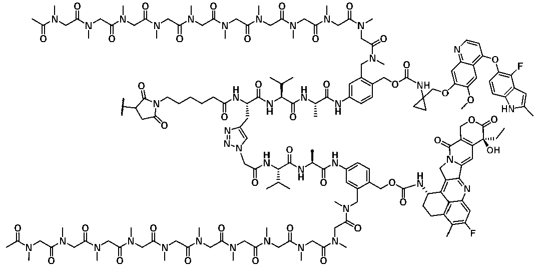 | Formula B167 |
| 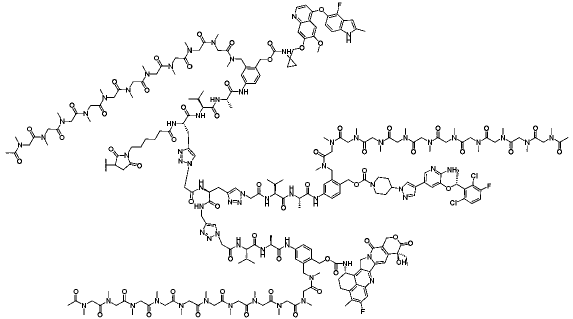 | Formula B168 |
| 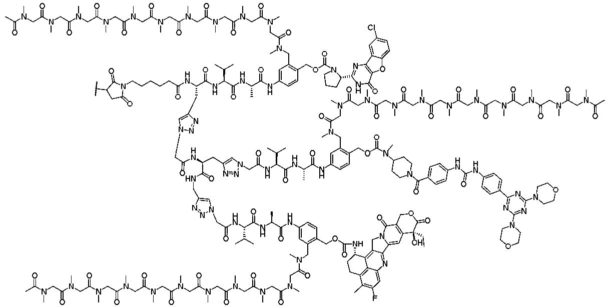 | Formula B169 |
| 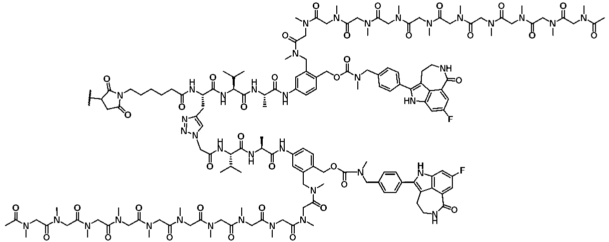 | Formula B170 |
| 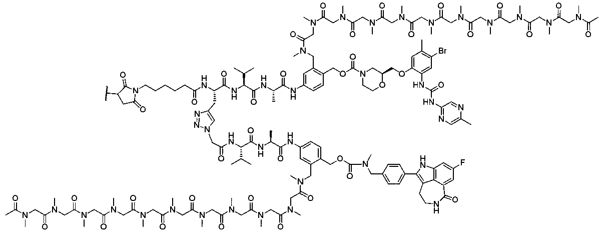 | Formula B171 |
| 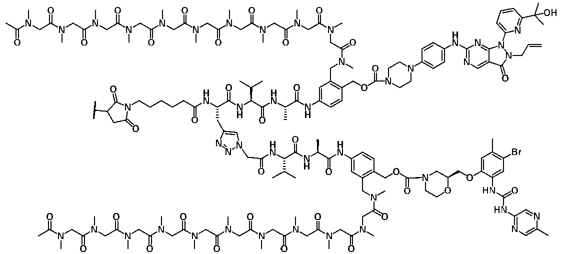 | Formula B172 |
| 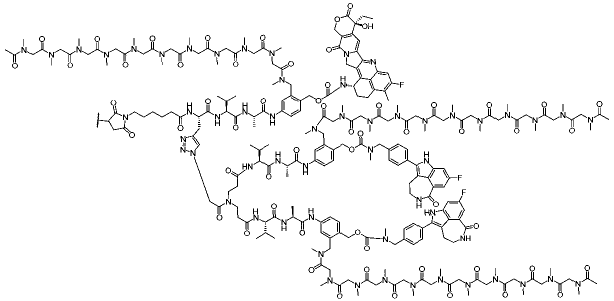 | Formula B173 |
| 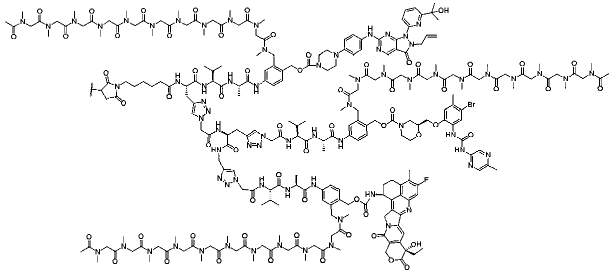 | Formula B174 |
| 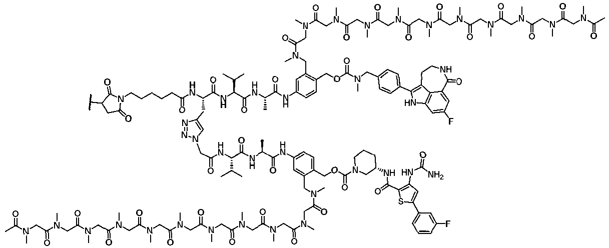 | Formula B175 |
| 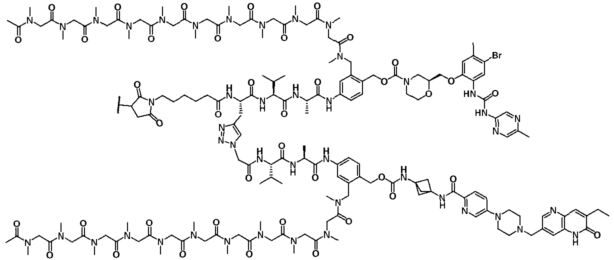 | Formula B176 |
| 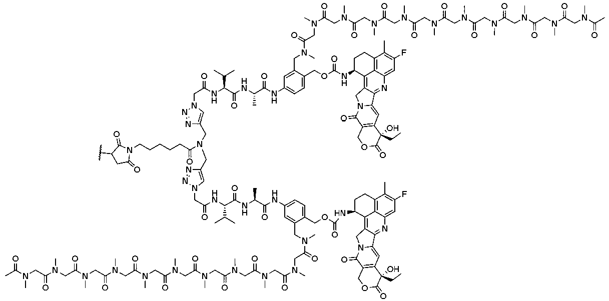 | Formula B177 |
| 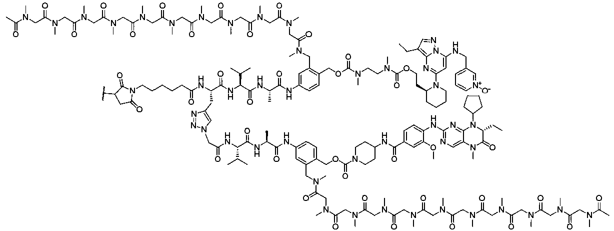 | Formula B178 |
| 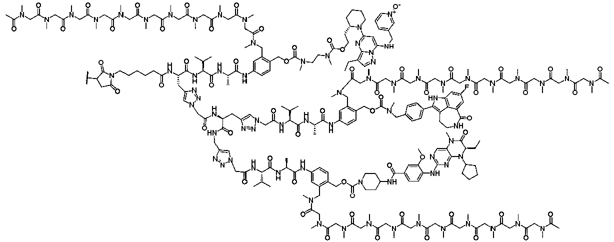 | Formula B179 |
| 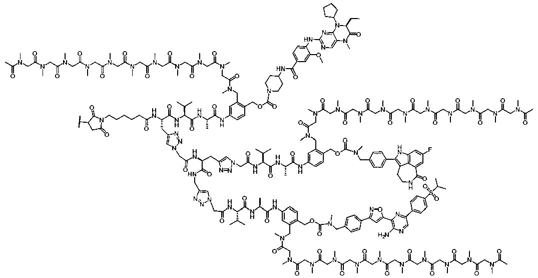 | Formula B180 |
| 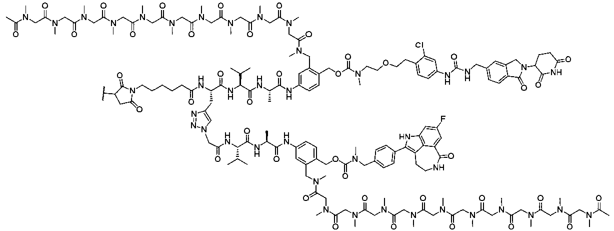 | Formula B181 |
| 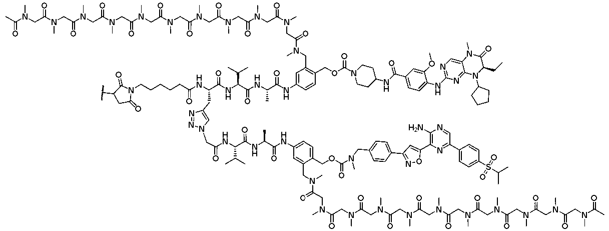 | Formula B182 |
| 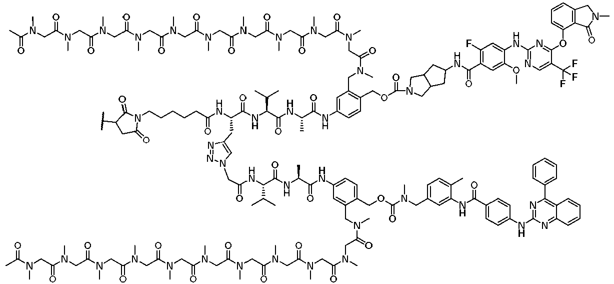 | Formula B183 |
| 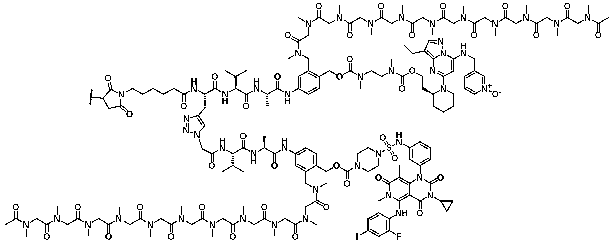 | Formula B184 |
| 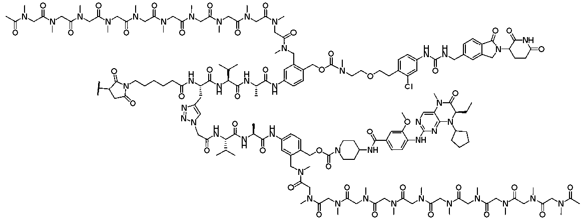 | Formula B185 |
| 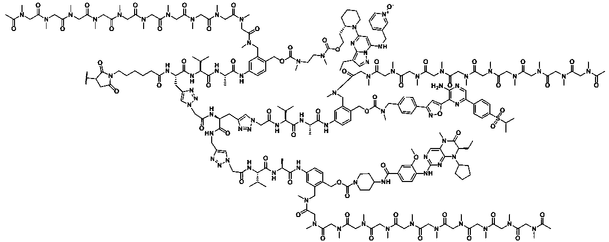 | Formula B186 |
| 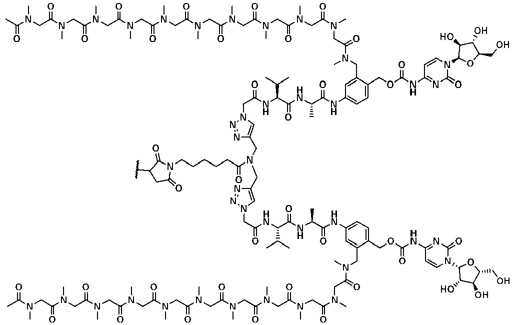 | Formula B187 |
| 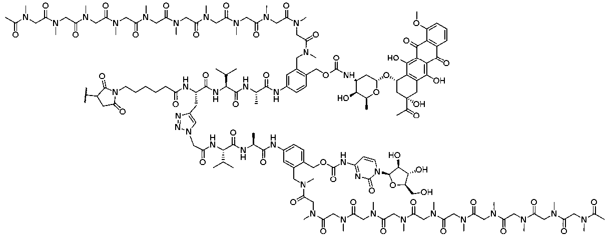 | Formula B188 |
| 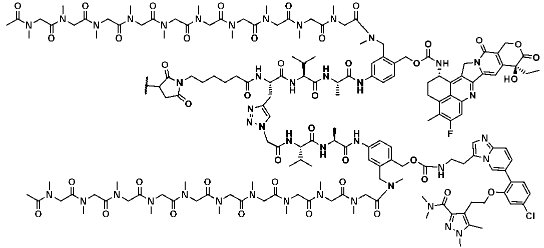 | Formula B189 |
| 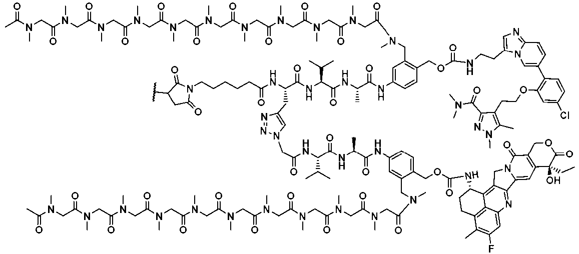 | Formula B190 |

In the present invention, the structures represented by formulas II, IIa, and IIb and their respective embodiments (including B1 to B190) are also referred to as "drug-linker fragments", "linker-drug fragments", or "LP".

Another aspect of the present invention provides a linker, or a tautomer, a mesomer, a racemate, an enantiomer, or a diastereomer thereof, or a deuterated compound form thereof, or a mixture form thereof, or a pharmaceutically acceptable salt, prodrug, or solvate thereof, wherein the linker has a structure represented by formula IV: 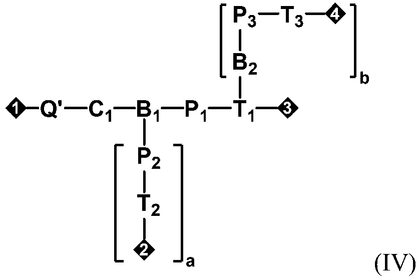 wherein,
Q' is a functional group capable of conjugating to cysteine, lysine, a non-natural amino acid, or a glycosyl of an antibody molecule, and attachment point 1 is connected to the antibody;
C₁ is selected from the group consisting of: a direct bond, optionally substituted alkylene, optionally substituted polyethylene glycol group, optionally substituted alkenylene, optionally substituted alkynylene, optionally substituted aliphatic cyclylene, optionally substituted aliphatic heterocyclylene, optionally substituted arylene, and optionally substituted heteroarylene; when substituted, the substituent(s) are selected from halogen, hydroxyl, amino, carboxyl, sulfonic acid group, sulfone group, phosphonic acid group, and alkoxy;
B₁ and B₂ are independently a direct bond or a branching group, provided that when C₁ is a direct bond, B₁ is not a direct bond;
P₁, P₂, and P₃ are each independently an optionally substituted polypeptide residue or a glucose fragment;
T₁, T₂, and T₃ are each independently a direct bond or an optionally substituted spacer group, provided that at most two of T₁, T₂, and T₃ are direct bonds and at least one of B₂, T₁, T₂, and T₃ is substituted with a hydrophilic group;
attachment points 2, 3, 4 are connected to drug units;
a and b are independently 0, 1, 2, or 3, provided that a and b are not both 0; and
when a=0, B₁ is a direct bond and T₁ is not a direct bond;
when b=0, B₁ is not a direct bond.

In some embodiments, the present invention provides a linker, or a tautomer, a mesomer, a racemate, an enantiomer, or a diastereomer thereof, or a deuterated compound form thereof, or a mixture form thereof, or a pharmaceutically acceptable salt, prodrug, or solvate thereof, wherein the compound comprises a structure represented by formula IVa: 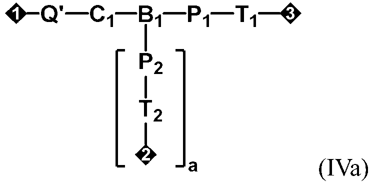 wherein:
Q' is a functional group capable of conjugating to cysteine, lysine, a non-natural amino acid, or a glycosyl of an antibody molecule, and attachment point 1 is connected to the antibody;
C₁ is selected from the group consisting of: a direct bond, optionally substituted alkylene, optionally substituted polyethylene glycol group, optionally substituted alkenylene, optionally substituted alkynylene, optionally substituted aliphatic cyclylene, optionally substituted aliphatic heterocyclylene, optionally substituted arylene, and optionally substituted heteroarylene; when substituted, the substituent(s) are selected from halogen, hydroxyl, amino, carboxyl, sulfonic acid group, sulfone group, phosphonic acid group, and alkoxy;
B₁ is a branching group;
P₁ and P₂ are each independently an optionally substituted polypeptide residue or a glucose fragment;
T₁ and T₂ are each independently a direct bond or an optionally substituted spacer group, provided that at most one of T₁ and T₂ is a direct bond, and at least one of T₁ and T₂ is substituted with a hydrophilic group;
attachment points 2 and 3 are connected to drug units;
a is 1, 2, or 3.

In some embodiments, the present invention provides a linker, or a tautomer, a mesomer, a racemate, an enantiomer, or a diastereomer thereof, or a deuterated compound form thereof, or a mixture form thereof, or a pharmaceutically acceptable salt, prodrug, or solvate thereof, wherein the compound comprises a structure represented by formula IVb: 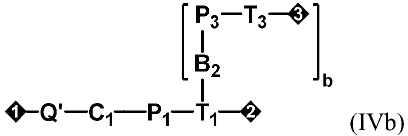
Q' is a functional group capable of conjugating to cysteine, lysine, a non-natural amino acid, or a glycosyl of an antibody molecule, and attachment point 1 is connected to the antibody;
C₁ is selected from the group consisting of: optionally substituted alkylene, optionally substituted polyethylene glycol group, optionally substituted alkenylene, optionally substituted alkynylene, optionally substituted aliphatic cyclylene, optionally substituted aliphatic heterocyclylene, optionally substituted arylene, and optionally substituted heteroarylene; when substituted, the substituent(s) are selected from halogen, hydroxyl, amino, carboxyl, sulfonic acid group, sulfone group, phosphonic acid group, and alkoxy;
B₂ is a branching group;
P₁ and P₃ are each independently a direct bond or an optionally substituted polypeptide residue or a glucose fragment;
T₁ is an optionally substituted spacer group, T₃ is a direct bond or an optionally substituted spacer group, and at least one of B₂ and T₃ is substituted with a hydrophilic group;
attachment points 2 and 3 are connected to drug units;
b is 1, 2, or 3.

In some embodiments, in formulas IV, IVa, and IVb, Q' is selected from the group consisting of: optionally substituted 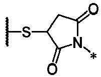 (Q1'), optionally substituted 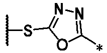 (Q2'), optionally substituted 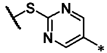 (Q3'), optionally substituted 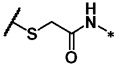 (Q4'), optionally substituted 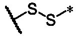 (Q5'), optionally substituted 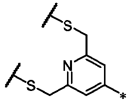 (Q6'), optionally substituted 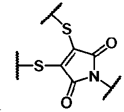 (Q7'), and optionally substituted 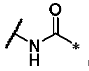 (Q8'), wherein * represents a site connected to C₁, and the wave line represents a site connected to the antibody molecule. In some embodiments, in formulas IV, IVa, and IVb, Q' is selected from the group consisting of: 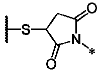 (Q1'), 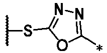 (Q2'), 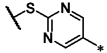 (Q3'), 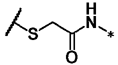 (Q4'), 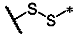 (Q5'), 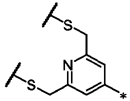 (Q6'), 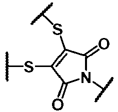 (Q7'), and 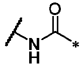 (Q8'), wherein * represents a site connected to C₁, and the wave line represents a site connected to the antibody molecule. More preferably, in formulas IV, IVa, and IVb, Q' is Q1'.

In preferred embodiments, in formulas IV, IVa, and IVb, C₁ is selected from: a direct bond, -(CH₂)ₘ₁-, -(CH₂)ₘ₁-(O-CH₂-CH₂)ₘ₂-, -(CH₂)ₘ₁-(O-CH₂-CH₂)ₘ₂-(CH₂)ₘ₃-, -(CH₂)ₘ₁-C(O)NH-(CH₂)ₘ₂-, -(CH₂)ₘ₁-NHC(O)-(CH₂)ₘ₂-, and -(C≡C-(CH₂)ₘ₁-, wherein m1, m2, and m3 are independently integers from 1 to 6, and the indicated substituent is connected to Q' on the left side and to B₁ or P₁ on the right side.

In preferred embodiments, in formulas IV and IVa, B₁ is selected from the group consisting of: a direct bond, 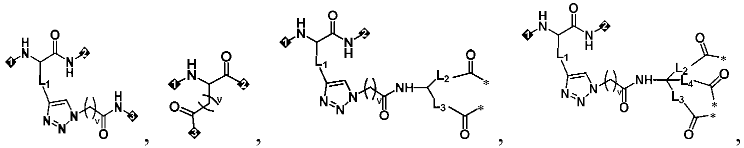 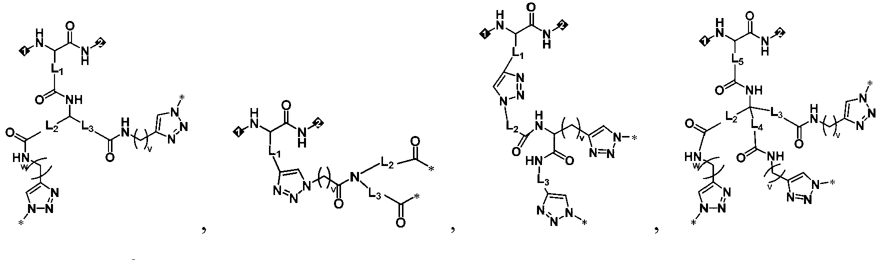 , and 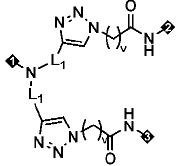 , wherein attachment point 1 is connected to C₁, attachment point 2 is connected to P₁, attachment points * and 3 are connected to P₂, L₁ is selected from -(CH₂)ₘ₁-, - (CH₂)ₘ₁-C(O)NH-(CH₂)ₘ₂-, -(CH₂)ₘ₁-NHC(O)-(CH₂)ₘ₂-, L₂, L₃, and L₄ are independently - (CH₂)ₘ₁-(O-CH₂-CH₂)ₘ₂- or -(CH₂)ₘ₁-(O-CH₂-CH₂)ₘ₂-(CH₂)ₘ₃-, L₅ is -(CH₂)ₘ₁-, wherein m1, m2, m3, w, and v are each independently 1, 2, 3, 4, 5, or 6.

In preferred embodiments, in formulas IV and IVa, when C₁ is a direct bond, B₁ is 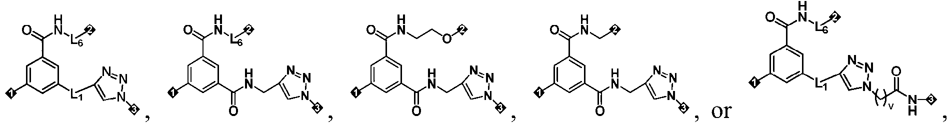 wherein attachment point 1 is connected to Q', attachment point 2 is connected to P₁, attachment point 3 is connected to P₂, L₆ is -(CH₂)ₘ₁- or -(CH₂-CH₂-O)m₂, wherein m1, m2 are each independently 1, 2, 3, 4, 5, or 6.

In preferred embodiments, in formulas IV and IVa, B₁ is selected from the group consisting of: a direct bond, 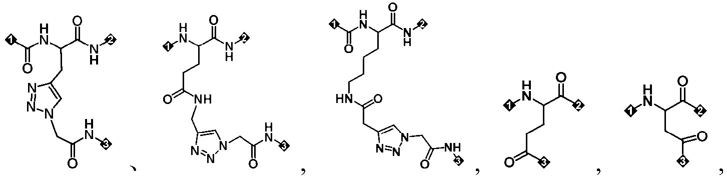 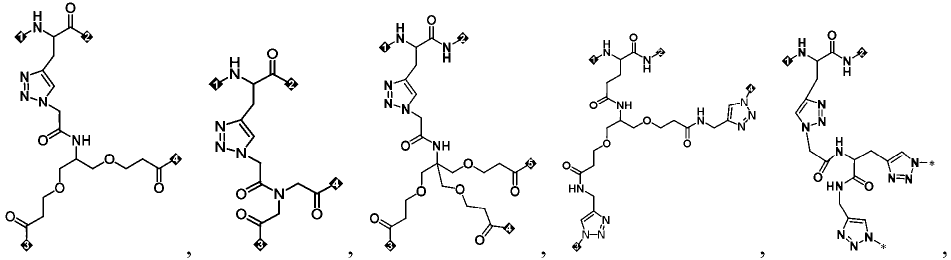 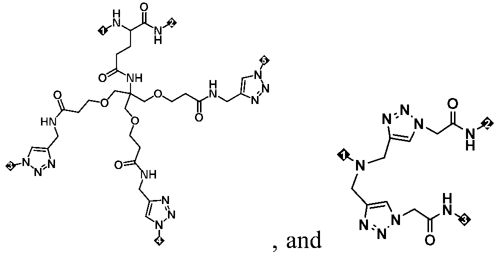 , wherein attachment point 1 is connected to C₁, attachment point 2 is connected to P₁, attachment points 3, 4 and 5 are connected to P₂.

In some embodiments, in formulas IV and IVb, B₂ is selected from the group consisting of: 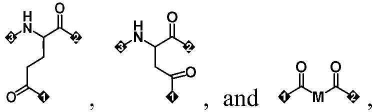 wherein attachment point 1 is connected to T₁, attachment point 2 is connected to P₃, attachment point 3 is connected to the hydrophilic group, M is selected from the group consisting of: optionally substituted C₁-C₆ alkylene, optionally substituted C₁-C₆ alkoxy, and optionally substituted C₁-C₅ alkenylene.

In some embodiments, in formula IV, T₁, T₂, T₃ are independently selected from the group consisting of: a direct bond , 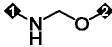 , and optionally substituted 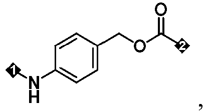 wherein attachment point 1 is connected to P₁, P₂, or P₃, and attachment point 2 is connected to D₁, D₂, or D₃.

In some embodiments, in formula IVa, T₁ and T₂ are independently selected from the group consisting of: a direct bond, 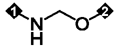 , and optionally substituted 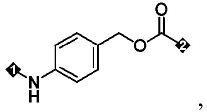 wherein attachment point 1 is connected to P₁ or P₂, and attachment point 2 is connected to D₁ or D₂.

In some embodiments, in formula IVb, T₁ is selected from the group consisting of: 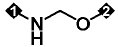 , and optionally substituted 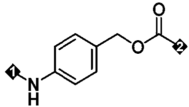 , wherein attachment point 1 is connected to P₁, and attachment point 2 is connected to D₁, T₃ is selected from the group consisting of: a direct bond, 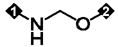 , and optionally substituted 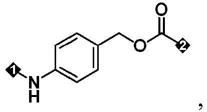 wherein attachment point 1 is connected to P₃, and attachment point 2 is connected to D₃.

In some embodiments, in formulas IV, IVa, and IVb, P₁, P₂, P₃, when present, independently comprises an optionally substituted polypeptide residue composed of amino acids selected from the group consisting of: phenylalanine, isoleucine, leucine, tryptophan, valine, methionine, tyrosine, alanine, threonine, histidine, serine, glutamine, arginine, lysine, asparagine, glutamic acid, proline, citrulline, aspartic acid, and glycine.

In preferred embodiments, in formulas IV, IVa, and IVb, P₁, P₂, P₃, when present, independently comprises an optionally substituted polypeptide residue composed of amino acids selected from the group consisting of: glycine, phenylalanine, valine, alanine, arginine, citrulline, aspartic acid, asparagine, and lysine.

In preferred embodiments, in formulas IV, IVa, and IVb, P₁, P₂, P₃, when present, independently comprises an optionally substituted polypeptide residue selected from the group consisting of: phenylalanine-lysine (Phe-Lys), valine-alanine (Val-Ala), valine-citrulline (Val-Cit), glutamic acid-valine-alanine (Glu-Val-Ala), glutamic acid-valine-citrulline (Glu-Val-Cit), valine-lysine (Val-Lys), alanine-alanine (Ala-Ala), alanine-alanine-alanine (Ala-Ala-Ala), alanine-alanine-asparagine (Ala-Ala-Asn), alanine-leucine (Ala-Leu), leucine-leucine (Leu-Leu), phenylalanine-arginine (Phe-Arg), phenylalanine-lysine (Phe-Lys), 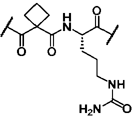 (cBu-Cit), 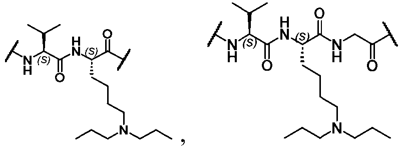 , glycine-glycine-phenylalanine-glycine (Gly-Gly-Phe-Gly), glycine-proline (Gly-Pro). In preferred embodiments, in formulas IV, IVa, and IVb, P₁, P₂, P₃, when present, are independently phenylalanine-lysine (Phe-Lys), valine-alanine (Val-Ala), valine-citrulline (Val-Cit), and glycine-glycine-phenylalanine-glycine (Gly-Gly-Phe-Gly).

In preferred embodiments, in formulas IV, IVa, and IVb, one of the combination P₂-T₂ and the combination P₃- T₃ is 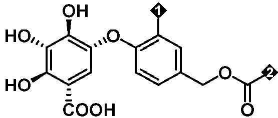 , wherein attachment point 1 is connected to B₁ or B₂, and attachment point 2 is connected to D₂ or D₃.

In preferred embodiments, in formulas IV, IVa, and IVb, the hydrophilic group is selected from: substituted polysarcosine residue, polyglycerol, polyol, glycosyl, cyclodextrin, substituted ethylene glycol fragment, substituted glycosylated polyethylene glycol, substituted glycosylated polyglycerol, substituted cyclodextrin polyethylene glycol, or a combination thereof.

In some preferred embodiments, in formulas IV, IVa, and IVb, the substituted polysarcosine residue is 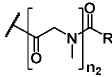 , wherein n₂ is an integer between 4 and 20, for example between 4 and 16, R is selected from: C₁-C₆ alkyl, C₁-C₆ cycloalkyl, C₁-C₆ alkoxy.

In some other preferred embodiments, in formulas IV, IVa, and IVb, the substituted glycosylated polyethylene glycol fragment is 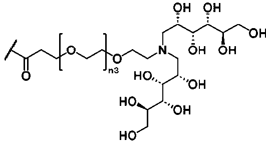 , wherein n₃ is an integer between 4 and 18, for example between 4 and 12.

In preferred embodiments, the substituted glycosylated polyglycerol fragment is 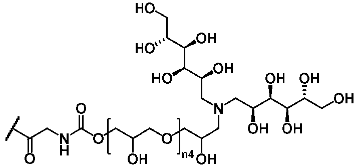 , wherein n₄ is an integer between 4 and 12, for example between 4 and 10.

In preferred embodiments, the substituted polyethylene glycol fragment is 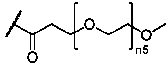 , wherein n₅ is an integer between 4 and 24, for example between 8 and 18.

In some other preferred embodiments, in formulas IV, at least one of T₁, T₂, T₃ is 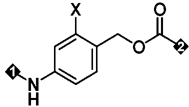 , wherein X is optionally substituted 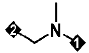 , the hydrophilic group is connected to T₁, T₂, or T₃ via X, wherein in X, attachment point 1 is connected to the hydrophilic group, and attachment point 2 is connected to one of T₁, T₂, T₃.

In some other preferred embodiments, in formulas IVa, at least one of T₁ and T₂ is 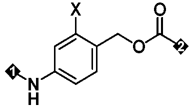 , wherein X is optionally substituted 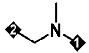 , the hydrophilic group is connected to T₁ or T₂ via X, wherein in X, attachment point 1 is connected to the hydrophilic group, and attachment point 2 is connected to one of T₁ or T₂.

In some other preferred embodiments, in formulas IVb, at least one of T₁ and T₃ is 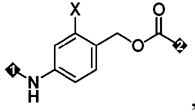 , wherein X is optionally substituted 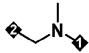 , the hydrophilic group is connected to T₁ or T₃ via X, wherein in X, attachment point 1 is connected to the hydrophilic group, and attachment point 2 is connected to one of T₁ or T₃.

In some embodiments, the present invention provides linkers as shown in formulas D1 to D38, or a tautomer, a mesomer, a racemate, an enantiomer, or a diastereomer thereof, or a deuterated compound form thereof, or a mixture form thereof, or a pharmaceutically acceptable salt, prodrug, or solvate thereof.

| | |
|---|---|
| 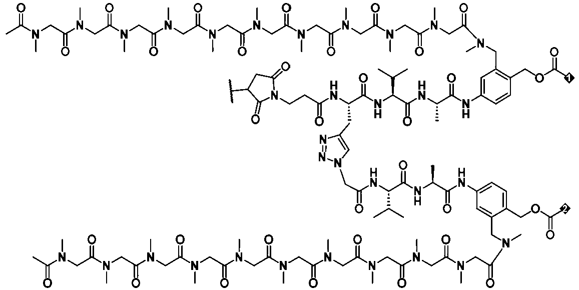 | Formula D1 |
| 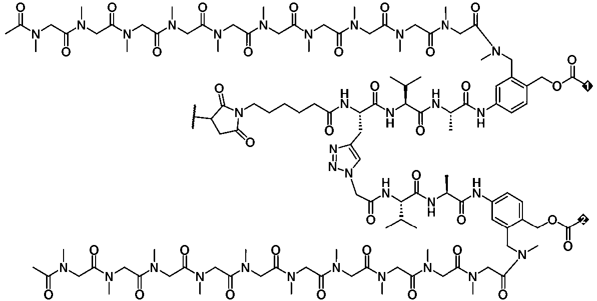 | Formula D2 |
| 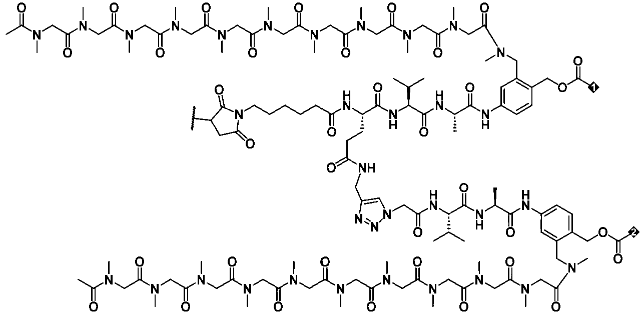 | Formula D3 |
| 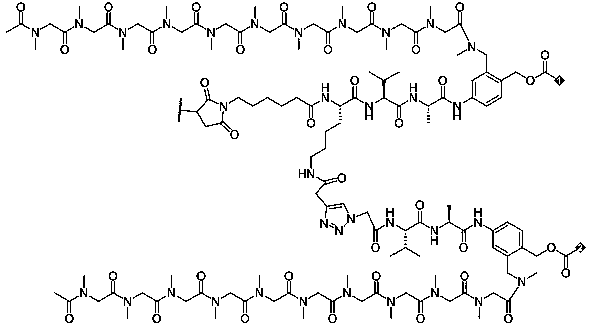 | Formula D4 |
| 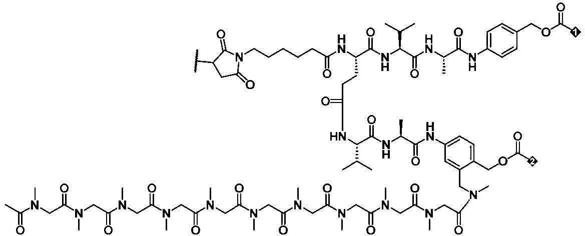 | Formula D5 |
| 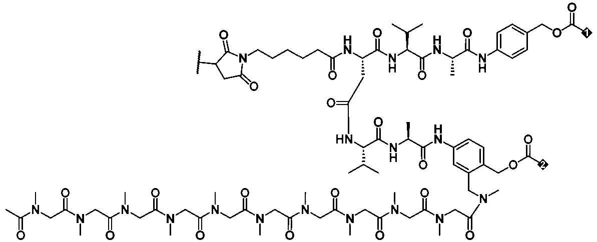 | Formula D6 |
| 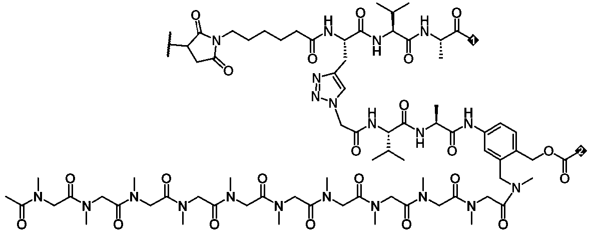 | Formula D7 |
| 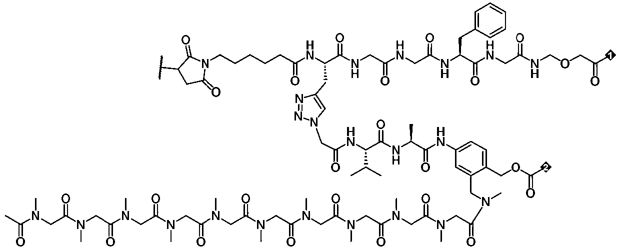 | Formula D8 |
| 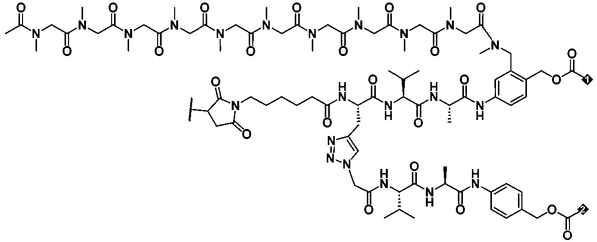 | Formula D9 |
| 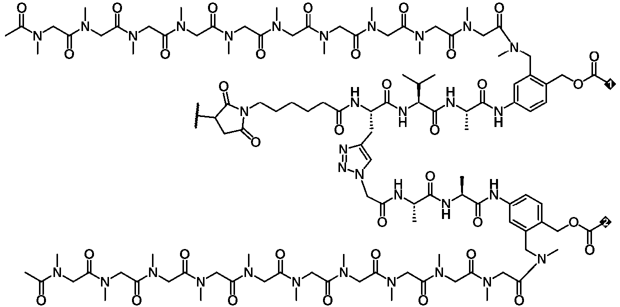 | Formula D10 |
| 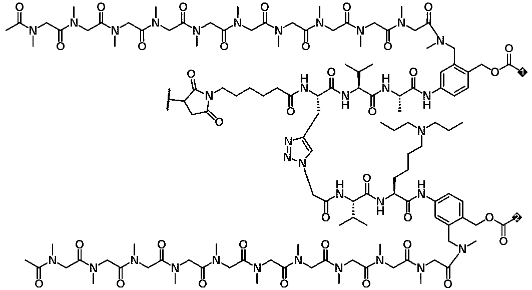 | Formula D11 |
| 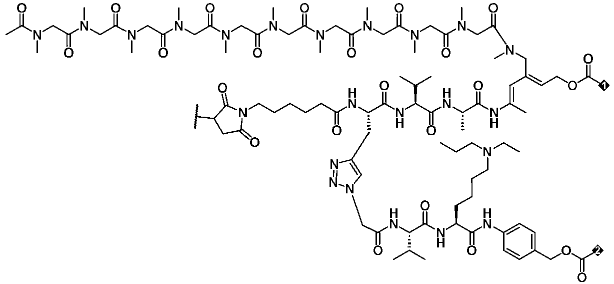 | Formula D12 |
| 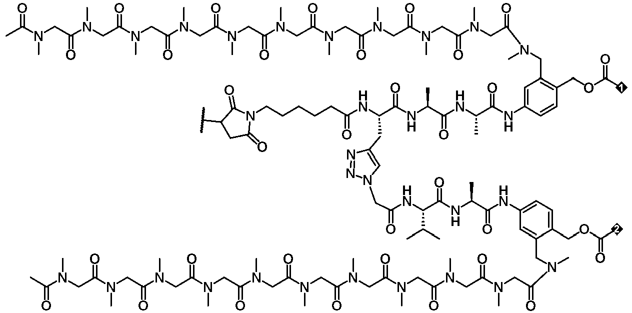 | Formula D13 |
| 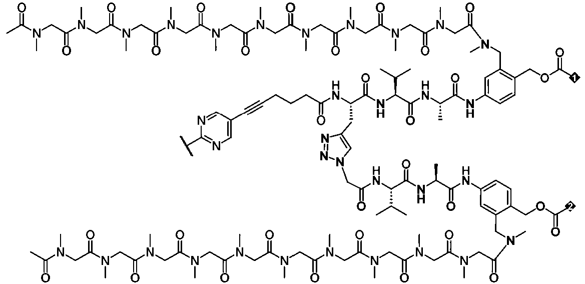 | Formula D14 |
| 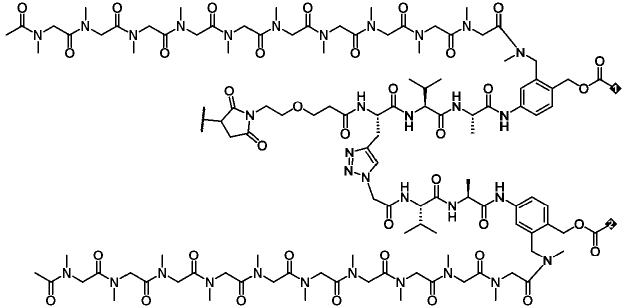 | Formula D15 |
| 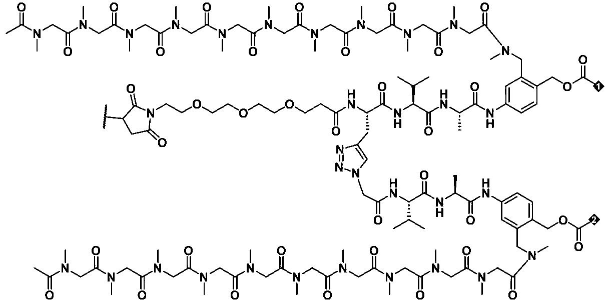 | Formula D16 |
| 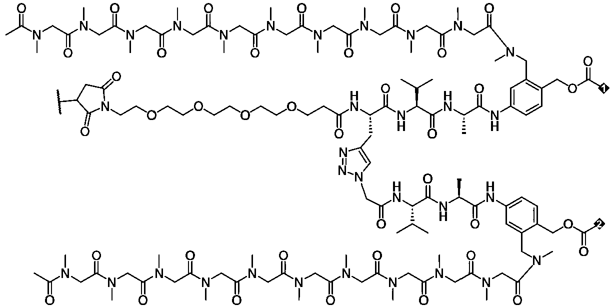 | Formula D17 |
| 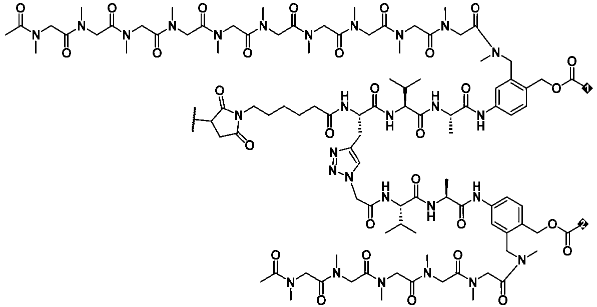 | Formula D18 |
| 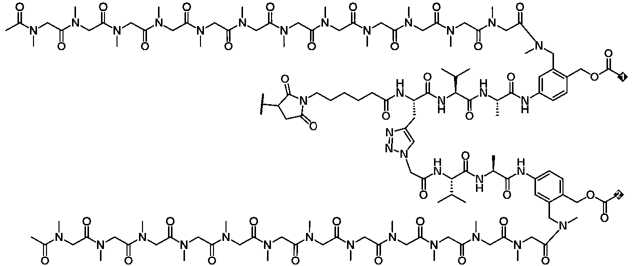 | Formula D19 |
| 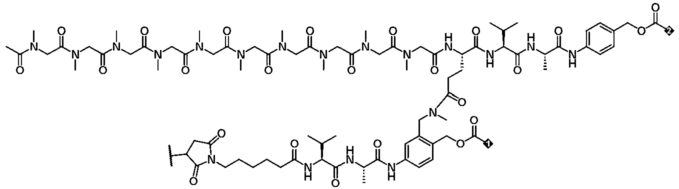 | Formula D20 |
| 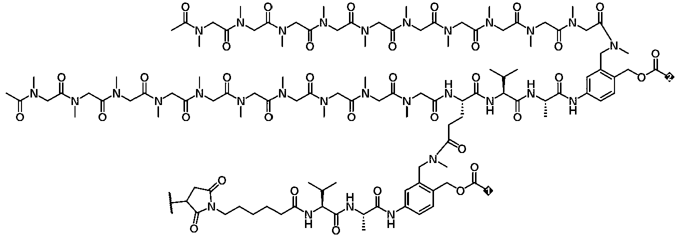 | Formula D21 |
| 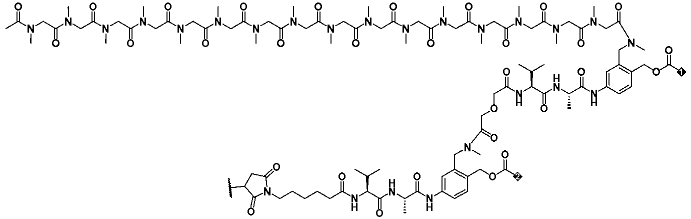 | Formula D22 |
| 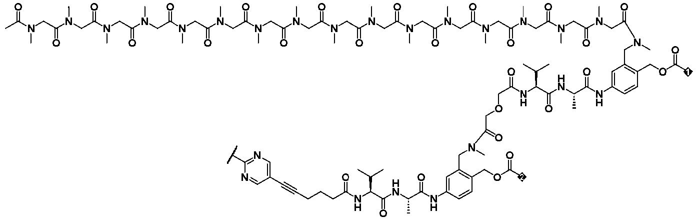 | Formula D23 |
| 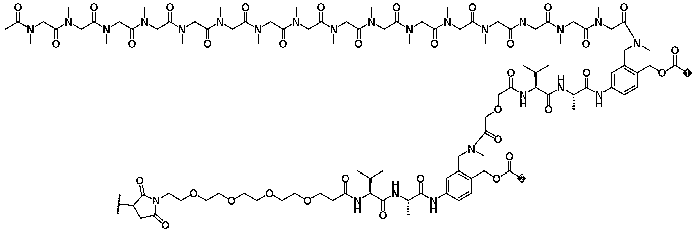 | Formula D24 |
| 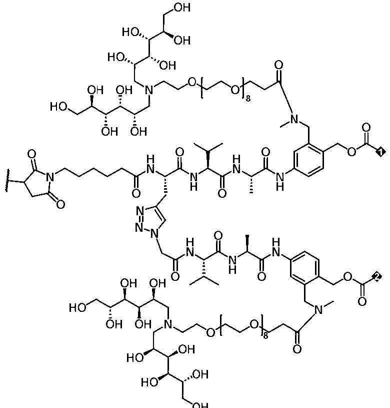 | Formula D25 |
| 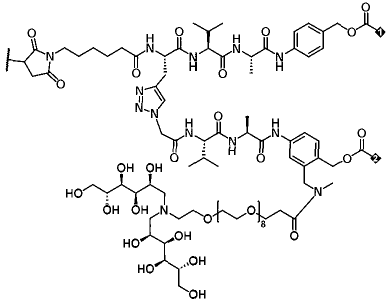 | Formula D26 |
| 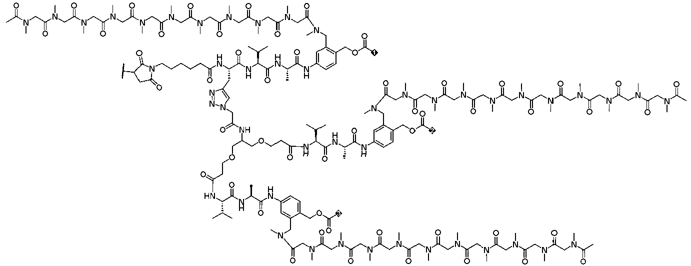 | Formula D27 |
| 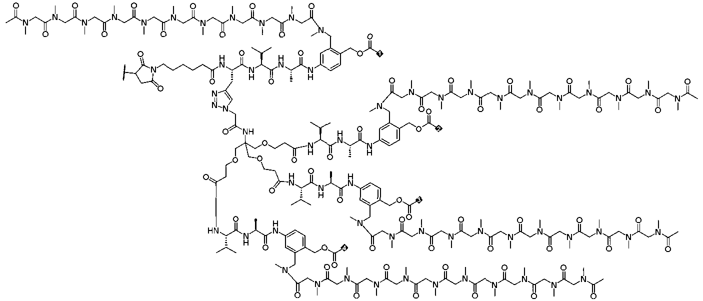 | Formula D28 |
| 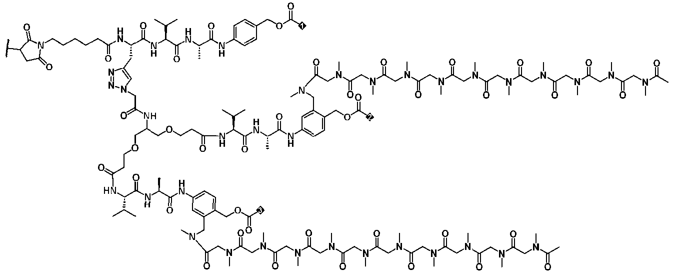 | Formula D29 |
| 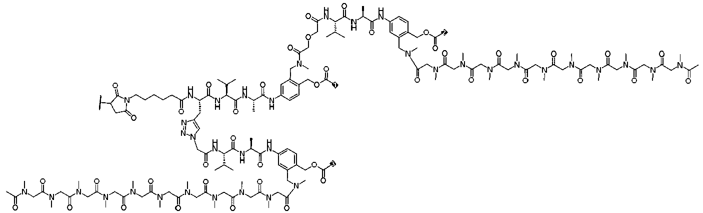 | Formula D30 |
| 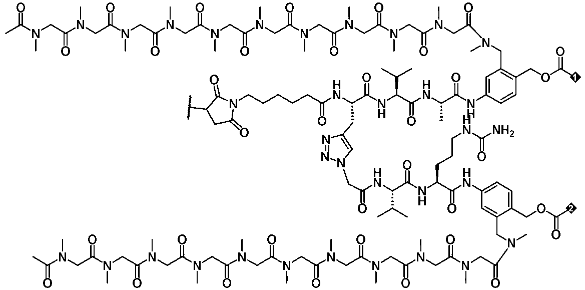 | Formula D31 |
| 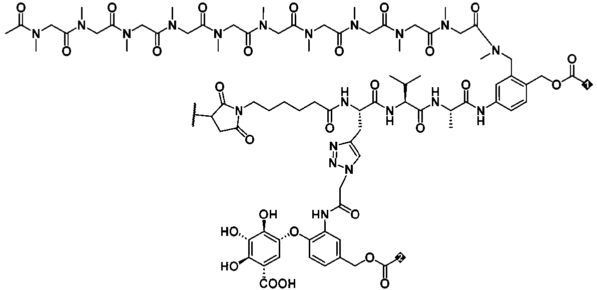 | Formula D32 |
| 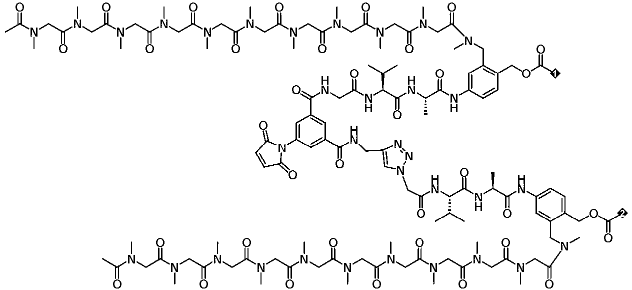 | Formula D33 |
| 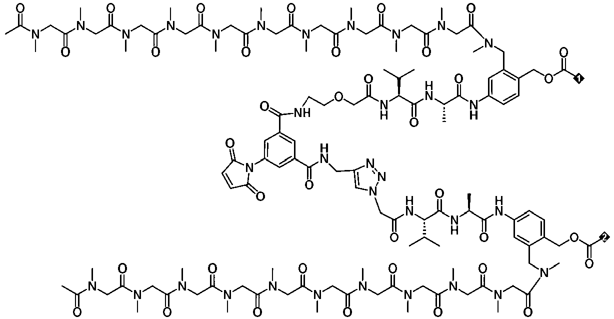 | Formula D34 |
| 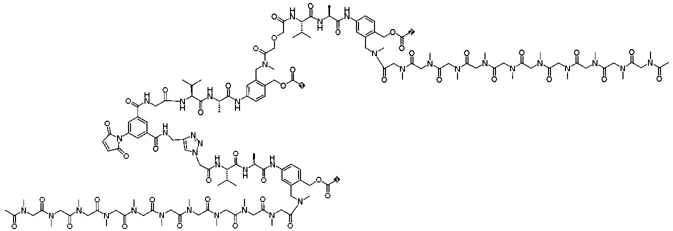 | Formula D35 |
| 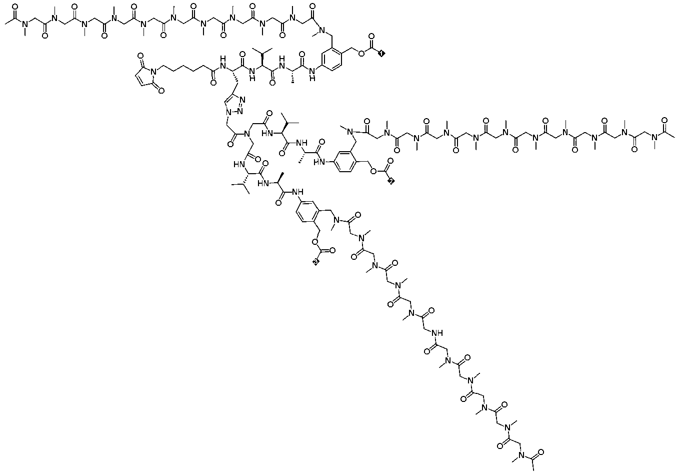 | Formula D36 |
| 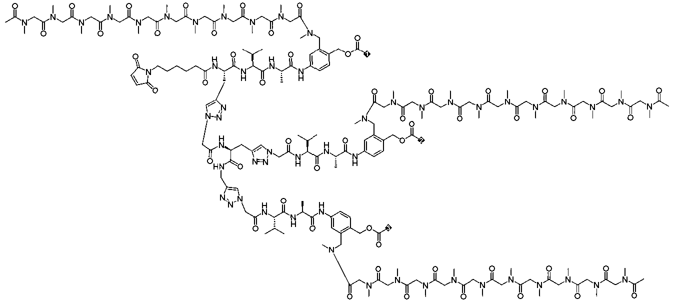 | Formula D37 |
| 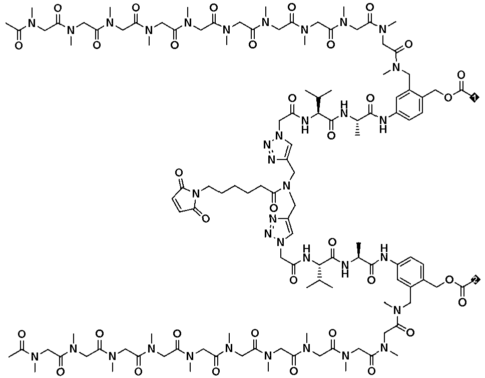 | Formula D38 |

### Antibody-Drug Conjugates (ADCs)

Another aspect of the present invention provides a compound, or a tautomer, a mesomer, a racemate, an enantiomer, or a diastereomer thereof, or a deuterated compound form thereof, or a mixture form thereof, or a pharmaceutically acceptable salt, prodrug, or solvate thereof, wherein the compound comprises a structure represented by formula III: 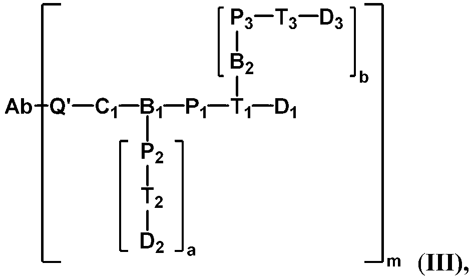
wherein, Ab is an antibody molecule, m is an integer or decimal from 1 to 8;
Q' is a functional group capable of conjugating to cysteine, lysine, a non-natural amino acid, or a glycosyl of an antibody molecule;
C₁ is selected from the group consisting of: a direct bond, optionally substituted alkylene, optionally substituted polyethylene glycol group, optionally substituted alkenylene, optionally substituted alkynylene, optionally substituted aliphatic cyclylene, optionally substituted aliphatic heterocyclylene, optionally substituted arylene, and optionally substituted heteroarylene; when substituted, the substituent(s) are selected from halogen, hydroxyl, amino, carboxyl, sulfonic acid group, sulfone group, phosphonic acid group, and alkoxy;
B₁ and B₂ are independently a direct bond or a branching group, provided that when C₁ is a direct bond, B₁ is not a direct bond;
P₁, P₂, and P₃ are each independently an optionally substituted polypeptide residue or a glucose fragment;
T₁, T₂, and T₃ are each independently a direct bond or an optionally substituted spacer group, provided that at most two of T₁, T₂, and T₃ are direct bonds and at least one of B₂, T₁, T₂, and T₃ is substituted with a hydrophilic group;
D₁, D₂, and D₃ are a first drug unit, a second drug unit, and a third drug unit, respectively, and are the same or different;
a and b are independently 0, 1, 2, or 3, provided that a and b are not both 0; and
when a=0, B₁ is a direct bond and T₁ is not a direct bond;
when b=0, B₁ is not a direct bond.

In some embodiments, the present invention provides a compound, or a tautomer, a mesomer, a racemate, an enantiomer, or a diastereomer thereof, or a deuterated compound form thereof, or a mixture form thereof, or a pharmaceutically acceptable salt, prodrug, or solvate thereof, wherein the compound comprises a structure represented by formula IIIa: 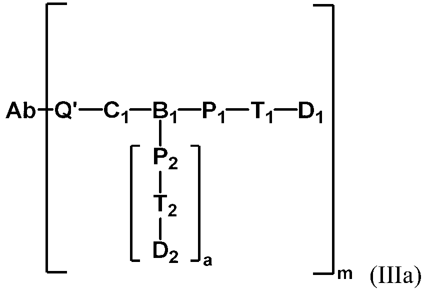 wherein:
Q' is a functional group capable of conjugating to cysteine, lysine, a non-natural amino acid, or a glycosyl of an antibody molecule;
C₁ is selected from the group consisting of: a direct bond, optionally substituted alkylene, optionally substituted polyethylene glycol group, optionally substituted alkenylene, optionally substituted alkynylene, optionally substituted aliphatic cyclylene, optionally substituted aliphatic heterocyclylene, optionally substituted arylene, and optionally substituted heteroarylene; when substituted, the substituent(s) are selected from halogen, hydroxyl, amino, carboxyl, sulfonic acid group, sulfone group, phosphonic acid group, and alkoxy;
B₁ is a branching group;
P₁ and P₂ are each independently an optionally substituted polypeptide residue or a glucose fragment;
T₁ and T₂ are each independently a direct bond or an optionally substituted spacer group, provided that at most one of T₁ and T₂ is a direct bond, and at least one of T₁ and T₂ is substituted with a hydrophilic group;
D₁ and D₂ are a first drug unit and a second drug unit, respectively, and are the same or different;
a is 1, 2, or 3.

In some embodiments, the present invention provides a compound, or a tautomer, a mesomer, a racemate, an enantiomer, or a diastereomer thereof, or a deuterated compound form thereof, or a mixture form thereof, or a pharmaceutically acceptable salt, prodrug, or solvate thereof, wherein the compound comprises a structure represented by formula IIIb: 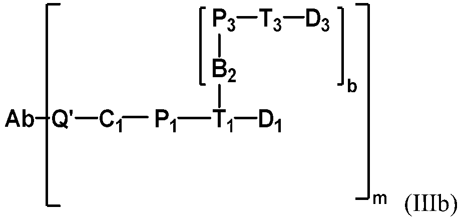
Q' is a functional group capable of conjugating to cysteine, lysine, a non-natural amino acid, or a glycosyl of an antibody molecule;
C₁ is selected from the group consisting of: optionally substituted alkylene, optionally substituted polyethylene glycol group, optionally substituted alkenylene, optionally substituted alkynylene, optionally substituted aliphatic cyclylene, optionally substituted aliphatic heterocyclylene, optionally substituted arylene, and optionally substituted heteroarylene; when substituted, the substituent(s) are selected from halogen, hydroxyl, amino, carboxyl, sulfonic acid group, sulfone group, phosphonic acid group, and alkoxy;
B₂ is a branching group;
P₁ and P₃ are each independently a direct bond or an optionally substituted polypeptide residue or a glucose fragment;
T₁ is an optionally substituted spacer group, T₃ is a direct bond or an optionally substituted spacer group, and at least one of B₂ and T₃ is substituted with a hydrophilic group;
D₁ and D₃ are a first drug unit and a third drug unit, respectively, and are the same or different;
b is 1, 2, or 3.

In some embodiments, the present invention provides a compound, or a tautomer, a mesomer, a racemate, an enantiomer, or a diastereomer thereof, or a deuterated compound form thereof, or a mixture form thereof, or a pharmaceutically acceptable salt, prodrug, or solvate thereof, wherein the compound comprises a structure represented by formula III: 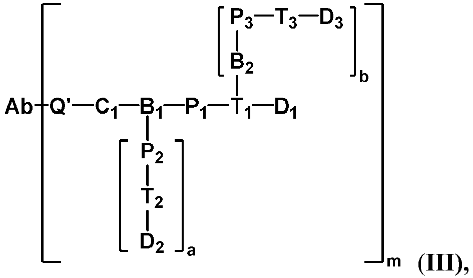
wherein, Ab is an antibody molecule, m is an integer or decimal from 1 to 8;
Q' is a functional group capable of conjugating to cysteine, lysine, a non-natural amino acid, or a glycosyl of an antibody molecule;
C₁ is selected from the group consisting of: a direct bond, optionally substituted alkylene, optionally substituted polyethylene glycol group, optionally substituted alkenylene, optionally substituted alkynylene, optionally substituted aliphatic cyclylene, optionally substituted aliphatic heterocyclylene, optionally substituted arylene, and optionally substituted heteroarylene; when substituted, the substituent(s) are selected from halogen, hydroxyl, amino, carboxyl, sulfonic acid group, sulfone group, phosphonic acid group, and alkoxy;
B₁ and B₂ are independently a branching group;
P₁, P₂, and P₃ are each independently an optionally substituted polypeptide residue or a glucose fragment;
T₁ is an optionally substituted spacer group, T₂ and T₃ are each independently a direct bond or an optionally substituted spacer group, and at least one of B₂, T₂, and T₃ is substituted with a hydrophilic group;
D₁, D₂, and D₃ are a first drug unit, a second drug unit, and a third drug unit, respectively, and are the same or different; and
a and b are independently 1, 2, or 3.

In preferred embodiments, in formulas III, IIIa, and IIIb, Q' is selected from the group consisting of: optionally substituted 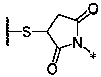 (Q1'), optionally substituted 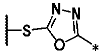 (Q2'), optionally substituted 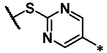 (Q3'), optionally substituted 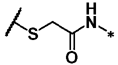 (Q4'), optionally substituted 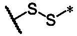 (Q5'), optionally substituted 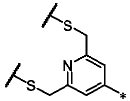 (Q6'), optionally substituted 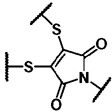 (Q7'), and optionally substituted 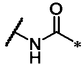 (Q8'), wherein * represents a site connected to C₁, and the wave line represents a site connected to Ab.

In preferred embodiments, in formulas III, IIIa, and IIIb, Q' is selected from the group consisting of: 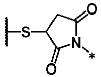 (Q1'), 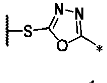 (Q2'), 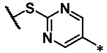 (Q3'), 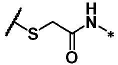 (Q4'), 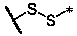 (Q5'), 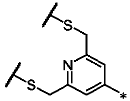 (Q6'), 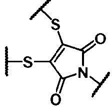 (Q7'), and 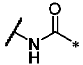 (Q8'), wherein * represents a site connected to C₁, and the wave line represents a site connected to Ab. Preferably, in formulas III, IIIa, and IIIb, Q' is 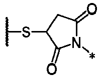 (Q1').

In preferred embodiments, in formulas III, IIIa, and IIIb, C₁ is selected from: a direct bond, -(CH₂)ₘ₁-, -(CH₂)ₘ₁-(O-CH₂-CH₂)ₘ₂-, -(CH₂)ₘ₁-(O-CH₂-CH₂)ₘ₂-(CH₂)ₘ₃-, -(CH₂)ₘ₁-C(O)NH-(CH₂)ₘ₂-, -(CH₂)ₘ₁-NHC(O)-(CH₂)ₘ₂-, and -(C≡C-(CH₂)ₘ₁-, wherein m1, m2, and m3 are independently integers from 1 to 6, and the indicated substituent is connected to Q' on the left side and to B₁ or P₁ on the right side.

In preferred embodiments, in formulas III and IIIa, B₁ is selected from the group consisting of: a direct bond, 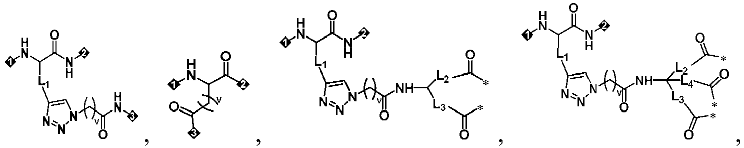 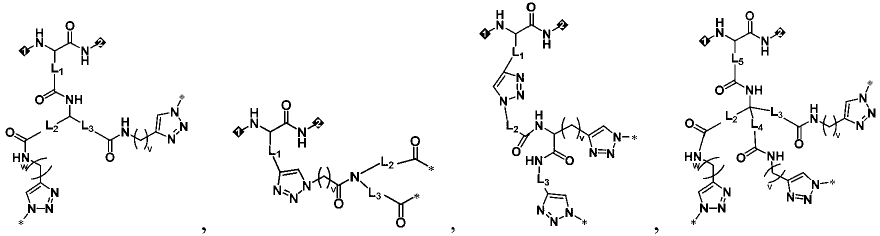 , and 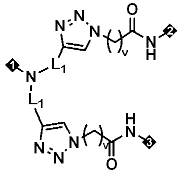 , wherein attachment point 1 is connected to C₁, attachment point 2 is connected to P₁, attachment points * and 3 are connected to P₂, L₁ is selected from -(CH₂)ₘ₁-, - (CH₂)ₘ₁-C(O)NH-(CH₂)ₘ₂-, -(CH₂)ₘ₁-NHC(O)-(CH₂)ₘ₂-, L₂, L₃, and L₄ are independently - (CH₂)ₘ₁-(O-CH₂-CH₂)ₘ₂- or -(CH₂)ₘ₁-(O-CH₂-CH₂)ₘ₂-(CH₂)ₘ₃-, L₅ is -(CH₂)ₘ₁-, wherein m1, m2, m3, w, and v are each independently 1, 2, 3, 4, 5, or 6.

In preferred embodiments, in formulas III and IIIa, when C₁ is a direct bond, B₁ is 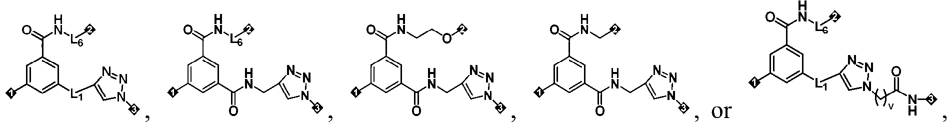 wherein attachment point 1 is connected to Q', attachment point 2 is connected to P₁, attachment point 3 is connected to P₂, L₆ is -(CH₂)ₘ₁- or -(CH₂-CH₂-O)m₂, wherein m1, m2 are each independently 1, 2, 3, 4, 5, or 6.

In preferred embodiments, in formulas III and IIIa, B₁ is selected from the group consisting of: a direct bond, 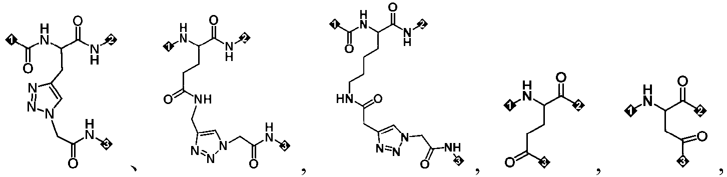 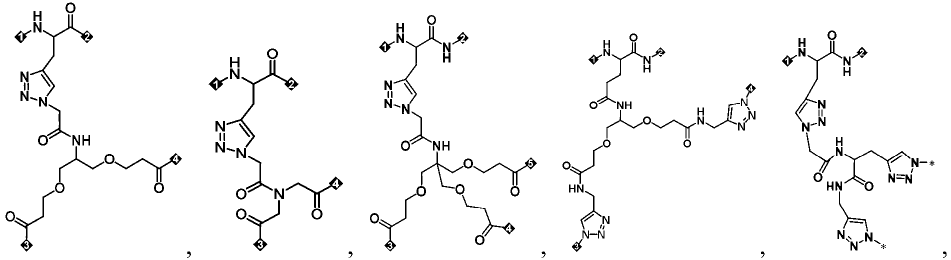 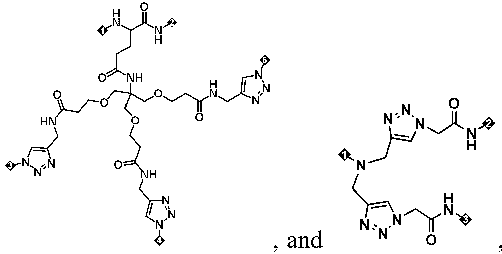 , wherein attachment point 1 is connected to C₁, attachment point 2 is connected to P₁, attachment points 3, 4 and 5 are connected to P₂.

In some embodiments, in formulas III and IIIb, B₂ is selected from the group consisting of: 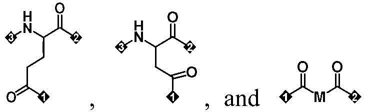 , wherein attachment point 1 is connected to T₁, attachment point 2 is connected to P₃, attachment point 3 is connected to the hydrophilic group, M is selected from the group consisting of: optionally substituted C₁-C₆ alkylene, optionally substituted C₁-C₆ alkoxy, and optionally substituted C₁-C₅ alkenylene.

In some embodiments, in formula III, T₁, T₂, T₃ are independently selected from the group consisting of: a direct bond 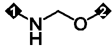 , and optionally substituted 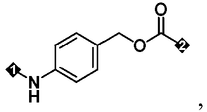 wherein attachment point 1 is connected to P₁, P₂, or P₃, and attachment point 2 is connected to D₁, D₂, or D₃.

In some embodiments, in formula IIIa, T₁ and T₂ are independently selected from the group consisting of: a direct bond, 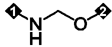 , and optionally substituted 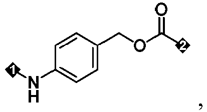 wherein attachment point 1 is connected to P₁ or P₂, and attachment point 2 is connected to D₁ or D₂.

In some embodiments, in formula IIIb, T₁ is selected from the group consisting of: 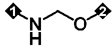 , and optionally substituted 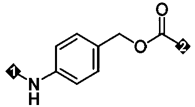 , wherein attachment point 1 is connected to P₁, and attachment point 2 is connected to D₁, T₃ is selected from the group consisting of: a direct bond, 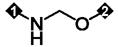 , and optionally substituted 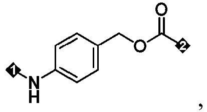 wherein attachment point 1 is connected to P₃, and attachment point 2 is connected to D₃.

In some embodiments, in formulas III, IIIa, and IIIb, P₁, P₂, P₃, when present, independently comprises an optionally substituted polypeptide residue composed of amino acids selected from the group consisting of: phenylalanine, isoleucine, leucine, tryptophan, valine, methionine, tyrosine, alanine, threonine, histidine, serine, glutamine, arginine, lysine, asparagine, glutamic acid, proline, citrulline, aspartic acid, and glycine.

In preferred embodiments, in formulas III, IIIa, and IIIb, P₁, P₂, P₃, when present, independently comprises an optionally substituted polypeptide residue composed of amino acids selected from the group consisting of: glycine, phenylalanine, valine, alanine, arginine, citrulline, aspartic acid, asparagine, and lysine.

In preferred embodiments, in formulas III, IIIa, and IIIb, P₁, P₂, P₃, when present, independently comprises an optionally substituted polypeptide residue selected from the group consisting of: phenylalanine-lysine (Phe-Lys), valine-alanine (Val-Ala), valine-citrulline (Val-Cit), glutamic acid-valine-alanine (Glu-Val-Ala), glutamic acid-valine-citrulline (Glu-Val-Cit), valine-lysine (Val-Lys), alanine-alanine (Ala-Ala), alanine-alanine-alanine (Ala-Ala-Ala), alanine-alanine-asparagine (Ala-Ala-Asn), alanine-leucine (Ala-Leu), leucine-leucine (Leu-Leu), phenylalanine-arginine (Phe-Arg), phenylalanine-lysine (Phe-Lys), 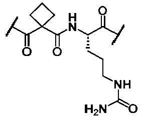 (cBu-Cit), 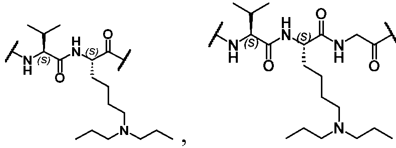 , glycine-glycine-phenylalanine-glycine (Gly-Gly-Phe-Gly), glycine-proline (Gly-Pro). In preferred embodiments, in formulas III, IIIa, and IIIb, P₁, P₂, P₃, when present, are independently phenylalanine-lysine (Phe-Lys), valine-alanine (Val-Ala), valine-citrulline (Val-Cit), and glycine-glycine-phenylalanine-glycine (Gly-Gly-Phe-Gly).

In preferred embodiments, in formulas III, IIIa, and IIIb, one of the combination P₂-T₂ and the combination P₃-T₃ is 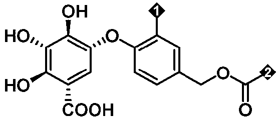 , wherein attachment point 1 is connected to B₁ or B₂, and attachment point 2 is connected to D₂ or D₃.

In preferred embodiments, in formulas III, IIIa, and IIIb, the hydrophilic group is selected from: substituted polysarcosine residue, polyglycerol, polyol, glycosyl, cyclodextrin, substituted ethylene glycol fragment, substituted glycosylated polyethylene glycol, substituted glycosylated polyglycerol, substituted cyclodextrin polyethylene glycol, or a combination thereof.

In some preferred embodiments, in formulas III, IIIa, and IIIb, the substituted polysarcosine residue is 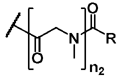 , wherein n₂ is an integer between 4 and 20, for example between 4 and 16, R is selected from: C₁-C₆ alkyl, C₁-C₆ cycloalkyl, C₁-C₆ alkoxy.

In some other preferred embodiments, in formulas III, IIIa, and IIIb, the substituted glycosylated polyethylene glycol fragment is 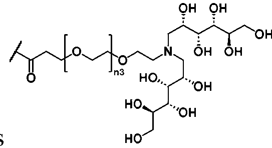 , wherein n₃ is an integer between 4 and 18, for example between 4 and 12.

In preferred embodiments, the substituted glycosylated polyglycerol fragment is 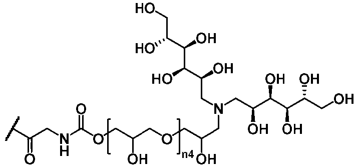 , wherein n₄ is an integer between 4 and 12, for example between 4 and 10.

In preferred embodiments, the substituted polyethylene glycol fragment is 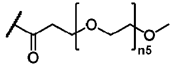 , wherein n₅ is an integer between 4 and 24, for example between 8 and 18.

In some other preferred embodiments, in formulas III, at least one of T₁, T₂, T₃ is 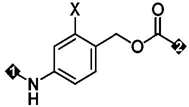 , wherein X is optionally substituted 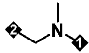 , the hydrophilic group is connected to T₁, T₂, or T₃ via X, wherein in X, attachment point 1 is connected to the hydrophilic group, and attachment point 2 is connected to one of T₁, T₂, T₃.

In some other preferred embodiments, in formulas IIIa, at least one of T₁ and T₂ is 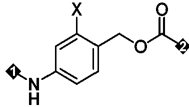 , wherein X is optionally substituted 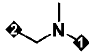 , the hydrophilic group is connected to T₁ or T₂ via X, wherein in X, attachment point 1 is connected to the hydrophilic group, and attachment point 2 is connected to one of T₁ or T₂.

In some other preferred embodiments, in formulas IIIb, at least one of T₁ and T₃ is 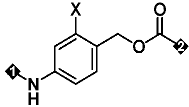 , wherein X is optionally substituted 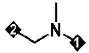 , the hydrophilic group is connected to T₁ or T₃ via X, wherein in X, attachment point 1 is connected to the hydrophilic group, and attachment point 2 is connected to one of T₁ or T₃.

In preferred embodiments, in formulas III, IIIa, and IIIb, D₁, D₂, D₃, when present, are a first anti-cancer agent, a second anti-cancer agent, and a third anti-cancer agent, respectively; preferably, at least two of the first, second, and third anti-cancer agents have a synergistic anti-cancer effect.

In preferred embodiments, D₁, D₂, D₃, when present, are independently a cytotoxic drug or a tumor-targeted therapeutic drug.

In some embodiments, in formulas III, IIIa, and IIIb, D₁, D₂, and D₃, when present, are all cytotoxic drugs or are all tumor-targeted therapeutic drugs. In some embodiments, in formulas III, IIIa, and IIIb, D₁, D₂, and D₃, when present, are all cytotoxic drugs or are all tumor-targeted therapeutic drugs, and have identical structural formulas. In some embodiments, in formulas III, IIIa, and IIIb, D₁, D₂, and D₃, when present, are all cytotoxic drugs or are all tumor-targeted therapeutic drugs, but have different structural formulas.

In preferred embodiments, at least one of D₁, D₂, and D₃ is a cytotoxic drug, and at least another one is a tumor-targeted therapeutic drug. In preferred embodiments, at least two of D₁, D₂, and D₃ are tumor-targeted therapeutic drugs, for example, tumor-targeted therapeutic drugs that can produce a synergistic or additive effect. In preferred embodiments, at least two of D₁, D₂, and D₃ are cytotoxic drugs.

In preferred embodiments, the cytotoxic drug is selected from drug units targeting topoisomerase, nucleoside antimetabolite anti-cancer drug units, drug units targeting tubulin, and drug units targeting DNA.

In preferred embodiments, the tumor-targeted therapeutic drug is selected from drug units targeting DNA damage response (DDR) or "synthetic lethality" related pathways, drug units targeting epigenetics, drug units targeting apoptosis-related pathways, drug units targeting transcription factors, and drug units targeting immune activation pathway-related targets, comprising: inhibitors of EFGR pathway-related targets; Ras-Raf-MAPK pathway inhibitors; PI3K/AKT/mTOR pathway inhibitors; cell cycle pathway inhibitors; cGAS-STING signaling pathway agonists; estrogen receptor antagonists; androgen receptor antagonists; glucocorticoid receptor modulators; autophagy inhibitors; FAK inhibitors; Smo inhibitors; BTK inhibitors; PDE4 inhibitors; Lck inhibitors; PLK1 inhibitors; TLR7/8 modulators; N-myristoyltransferase (NMT) inhibitors.

In preferred embodiments, at least one of D₁, D₂, and D₃ is a drug targeting topoisomerase, and at least another one is a drug targeting DNA damage response (DDR) or "synthetic lethality" related pathways.

Accordingly, in some embodiments, at least one of D₁, D₂, and D₃ is a cytotoxic drug selected from drugs targeting topoisomerase, drugs targeting tubulin, and drugs targeting DNA, and at least another one is a tumor-targeted therapeutic drug, which is a drug targeting DNA damage response (DDR) or "synthetic lethality" related pathways. For example, at least one of D₁, D₂, and D₃ is a drug targeting topoisomerase, and at least another one is a drug targeting DNA damage response (DDR) or "synthetic lethality" related pathways. For example, at least one of D₁, D₂, and D₃ is a drug targeting tubulin, and at least another one is a drug targeting DNA damage response (DDR) or "synthetic lethality" related pathways. For example, at least one of D₁, D₂, and D₃ is a drug targeting DNA, and at least another one is a drug targeting DNA damage response (DDR) or "synthetic lethality" related pathways.

In some embodiments, at least one of D₁, D₂, and D₃ is a cytotoxic drug selected from drugs targeting topoisomerase, drugs targeting tubulin, and drugs targeting DNA, and at least another one is a tumor-targeted therapeutic drug, which is a drug targeting epigenetics. For example, at least one of D₁, D₂, and D₃ is a drug targeting topoisomerase, and at least another one is a drug targeting epigenetics. For example, at least one of D₁, D₂, and D₃ is a drug targeting tubulin, and at least another one is a drug targeting epigenetics. For example, at least one of D₁, D₂, and D₃ is a drug targeting DNA, and at least another one is a drug targeting epigenetics.

In some embodiments, at least one of D₁, D₂, and D₃ is a cytotoxic drug selected from drugs targeting topoisomerase, drugs targeting tubulin, and drugs targeting DNA, and at least another one is a tumor-targeted therapeutic drug, which is a drug targeting apoptosis-related pathways. For example, at least one of D₁, D₂, and D₃ is a drug targeting topoisomerase, and at least another one is a drug targeting apoptosis-related pathways. For example, at least one of D₁, D₂, and D₃ is a drug targeting tubulin, and at least another one is a drug targeting apoptosis-related pathways. For example, at least one of D₁, D₂, and D₃ is a drug targeting DNA, and at least another one is a drug targeting apoptosis-related pathways.

In some embodiments, at least one of D₁, D₂, and D₃ is a cytotoxic drug selected from drugs targeting topoisomerase, drugs targeting tubulin, and drugs targeting DNA, and at least another one is a tumor-targeted therapeutic drug, which is a drug targeting immune activation pathway-related targets. For example, at least one of D₁, D₂, and D₃ is a drug targeting topoisomerase, and at least another one is a drug targeting immune activation pathways. For example, at least one of D₁, D₂, and D₃ is a drug targeting tubulin, and at least another one is a drug targeting immune activation pathways. For example, at least one of D₁, D₂, and D₃ is a drug targeting DNA, and at least another one is a drug targeting immune activation pathways.

In some embodiments, at least two of D₁, D₂, and D₃ are drugs targeting DNA damage response (DDR) or "synthetic lethality" related pathways. In some embodiments, at least two of D₁, D₂, and D₃ are drugs targeting epigenetics. In some embodiments, at least two of D₁, D₂, and D₃ are drugs targeting apoptosis-related pathways. In some embodiments, at least two of D₁, D₂, and D₃ are drugs targeting immune activation pathways.

In some embodiments, at least two of D₁, D₂, and D₃ are drugs targeting topoisomerase. In some embodiments, at least two of D₁, D₂, and D₃ are nucleoside antimetabolite anti-cancer drugs. In some embodiments, at least two of D₁, D₂, and D₃ are drugs targeting tubulin. In some embodiments, at least two of D₁, D₂, and D₃ are drugs targeting DNA.

In some embodiments, at least one of D₁, D₂, and D₃ is a cytotoxic drug selected from drugs targeting topoisomerase, for example a TOPO1 inhibitor, and at least another one is a tumor-targeted therapeutic drug, which is a drug targeting DNA damage response (DDR) or "synthetic lethality" related pathways, selected from PARP inhibitors, ATR inhibitors, CHK1 inhibitors, ATM inhibitors, DNA-PK inhibitors, WEE1 inhibitors, POLQ inhibitors, CDK12 inhibitors, USP1 inhibitors, PKMYT1 inhibitors, Rad51 inhibitors.

In some embodiments, at least two of D₁, D₂, and D₃ are cytotoxic drugs and are independently selected from drugs targeting topoisomerase and drug units targeting tubulin, for example TOPO1 inhibitors, TOPO2 inhibitors, and tubulin inhibitors.

In some embodiments, at least two or all three of D₁, D₂, and D₃ are tumor-targeted therapeutic drugs, and are independently selected from drug units targeting DNA damage response (DDR) or "synthetic lethality" related pathways, drug units targeting epigenetics, drug units targeting apoptosis-related pathways, drug units targeting cell cycle pathways, drug units targeting transcription factors, and drug units targeting immune activation pathway-related targets, comprising: inhibitors targeting EFGR pathway-related targets; inhibitors targeting Ras-Raf-MAPK pathway-related targets; inhibitors targeting PI3K/AKT/mTOR pathway-related targets; inhibitors targeting cell cycle pathway-related targets; agonists targeting cGAS-STING signaling pathway-related targets; estrogen receptor antagonists, androgen receptor antagonists; glucocorticoid receptor modulators; autophagy inhibitors; FAK inhibitors; Smo inhibitors; BTK inhibitors; PDE4 inhibitors; Lck inhibitors; PLK1 inhibitors; TLR7/8 modulators; N-myristoyltransferase (NMT) inhibitors.

In some embodiments, one of D₁, D₂, and D₃ is a cytotoxic drug selected from drug units targeting topoisomerase and drug units targeting tubulin, for example a TOPO1 inhibitor or a tubulin inhibitor, and the other one or two are tumor-targeted therapeutic drugs, selected from drug units targeting DNA damage response (DDR) or "synthetic lethality" related pathways, drug units targeting epigenetics, drug units targeting apoptosis-related pathways, drug units targeting cell cycle pathways, drug units targeting transcription factors, and drug units targeting immune activation pathway-related targets, comprising: inhibitors targeting EFGR pathway-related targets; inhibitors targeting Ras-Raf-MAPK pathway-related targets; inhibitors targeting PI3K/AKT/mTOR pathway-related targets; inhibitors targeting cell cycle pathway-related targets; agonists targeting cGAS-STING signaling pathway-related targets; estrogen receptor antagonists, androgen receptor antagonists; glucocorticoid receptor modulators; autophagy inhibitors; FAK inhibitors; Smo inhibitors; BTK inhibitors; PDE4 inhibitors; Lck inhibitors; PLK1 inhibitors; TLR7/8 modulators; N-myristoyltransferase (NMT) inhibitors.

In preferred embodiments, the drug unit targeting topoisomerase is a topoisomerase I (TOPO1) inhibitor or topoisomerase II (TOPO2) inhibitor, comprising Exatecan (CAS 171335-80-1), DXd (CAS 1599440-33-1), 7-ethyl-10-hydroxycamptothecin (SN38, CAS 86639-52-3), Belotecan (CAS 213819-48-8), (4-NH₂)-Exatecan (AZD'0132, CAS 2495742-21-5), 7-MAD-MDCPT (CAS 765871-81-6), 7-aminomethyl-10-methyl-11-fluorocamptothecin (CAS 2378616-23-8), Voreloxin (CAS 175414-77-4), or derivatives and analogs thereof. In preferred embodiments, the drug unit targeting tubulin is Eribulin, Vinblastine, Paclitaxel, MMAE, MMAF, Maytansine, or derivatives thereof. In preferred embodiments, the drug unit targeting DNA is a DNA minor groove binder (PBD), a DNA alkylator (Duocarmycin), Trabectedin, Lurbinectedin, or derivatives thereof.

In preferred embodiments, the drug unit targeting DNA damage response (DDR) or "synthetic lethality" related pathways is a PARP inhibitor, ATR inhibitor, CHK1 inhibitor, ATM inhibitor, DNA-PK inhibitor, WEE1 inhibitor, POLQ inhibitor, CDK12 inhibitor, USP1 inhibitor, PKMYT1 inhibitor, or Rad51 inhibitor; the PARP inhibitor is preferably Rucaparib, Niraparib, Veliparib, A-966492, Talazoparib, AZD5305, Venadaparib, Mefuparib, or analogs thereof; the ATR inhibitor is preferably Berzosertib, Ceralasertib, or analogs thereof; and/or the CHK1 inhibitor is preferably Prexasertib (LY2606368), AZD7762, Rabusertib (LY2603618), MK-8776 (SCH 900776), CHIR-124, PF-477736, CCT245737 (SRA737, PNT-737), GDC-0575 (ARRY-575), or analogs thereof; and/or the WEE1 inhibitor is preferably ZN-C3, Adavosertib (AZD1775), or analogs thereof.

Preferably, an analog of the AZD5305 comprises the following structure: 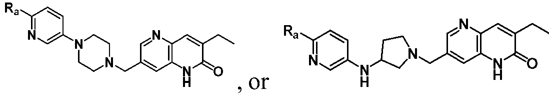 , wherein Rₐ is selected from -C(O)NH-R_{b} or -NHC(O)-R_{b}, R_{b} is selected from: C₂₋₇ alkyl, C₃₋₇ monocyclic cycloalkyl, C₄₋₁₀ bicyclic cycloalkyl, C₅₋₉ spirocycloalkyl, and C₅₋₉ bridged cycloalkyl, each substituted with at least one primary or secondary amino group, or R_{b} is a 4-6 membered monocyclic heterocycloalkyl, C₄₋₁₀ bicyclic heterocycloalkyl, C₅₋₉ spiroheterocycloalkyl, C₅₋₉ bridged heterocycloalkyl containing 1-5 nitrogen, oxygen, sulfur atoms, wherein the R_{b} group comprises at least one primary or secondary amino group.

Preferably, the AZD5305 analog is selected from the following structures:

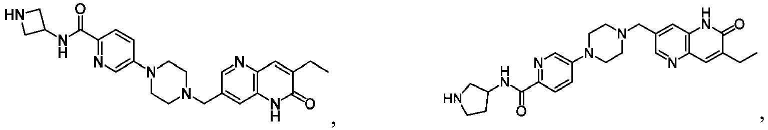

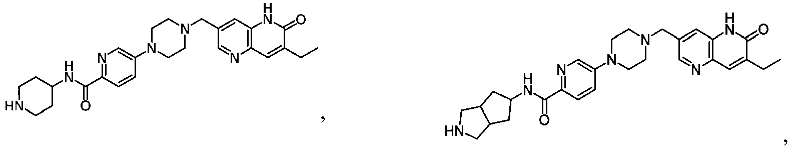

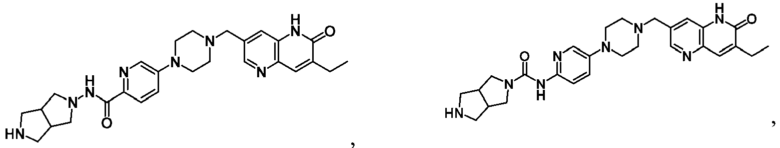

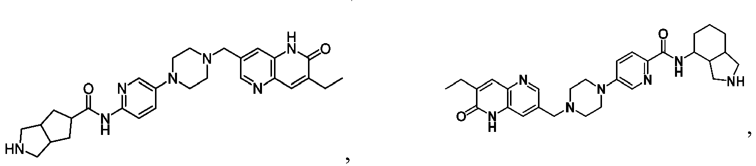

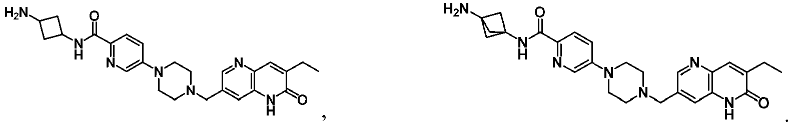

In preferred embodiments, the drug unit targeting apoptosis-related pathways is a Bcl-2 family protein inhibitor; the Bcl-2 family protein inhibitor comprises BCL-2 inhibitors, BCL-XL inhibitors, and MCL-1 inhibitors, preferably Venetoclax (CAS 1257044-40-8), Navitoclax (CAS 923564-51-6), Navitoclax analog (CAS 2143096-93-7), ABT-737 (CAS 852808-04-9), A-1331852 (CAS 1430844-80-6), S64315 (CAS 1799631-75-6), or analogs thereof.

In preferred embodiments, the drug unit targeting epigenetics is an LSD1 inhibitor, EZH2 inhibitor, BRD4 inhibitor, PRMT5 inhibitor, or PRMT1 inhibitor. In preferred embodiments, the LSD1 inhibitor is preferably Tranylcypromine, ORY-1001 (Iadademstat, CAS 1431303-72-8), CC-90011 (Pulrodemstat, CAS 1821307-10-1), ORY-2001, GSK-2879552, IMG-7289, INCB059872, or TAK-418 or analogs thereof; the EZH2 inhibitor is preferably Tazemetostat (CAS 1403254-99-8), GSK2816126, CPI-1205, PF-06821497, SHR2554, XNW5004, HH2853, or analogs thereof; the BRD4 inhibitor is a BI-2536 analog, Birabresib; the PRMT5 inhibitor is preferably GSK3326595, AMG 193, MRTX1719, SKL27969, TNG908, SCR-6920, SH3765, SYHX2001, or analogs thereof. The PRMT1 inhibitor is preferably GSK3368715 (CAS 1629013-22-4), MS023 (CAS 1831110-54-3), or analogs thereof.

In preferred embodiments, the Ras-Raf-MAPK pathway inhibitors comprise: SOS1 inhibitors BAY-293 (CAS 2244904-70-7), BI-3406 (CAS 2230836-55-0), or analogs thereof; KRAS inhibitors BI-2493 (CAS 2937344-16-4), MRTX1133 (CAS 2621928-55-8), or analogs thereof; MEK inhibitors Cobimetinib (CAS 934660-93-2), Selumetinib (CAS 606143-52-6), Mirdametinib (CAS 391210-10-9), Binimetinib (CAS 606143-89-9), TAK-733 (CAS 1035555-63-5), GDC-0623 (CAS 1168091-68-6), AZD8330 (CAS 869357-68-6), Trametinib (CAS 871700-17-3), Trametiglue (CAS 2666940-97-0), or analogs thereof; ERK inhibitors ASN007 (CAS 2055597-12-9) or analogs thereof.

In preferred embodiments, the cell cycle pathway inhibitors comprise: CDK4/6 inhibitors Palbociclib (CAS 571190-30-2), Abemaciclib (CAS 1231929-97-7), Ribociclib (CAS 1211441-98-3), Dalpiciclib (CAS 1637781-04-4), or analogs thereof; pan-CDK inhibitors Dinaciclib (CAS 779353-01-4) or analogs thereof.

In preferred embodiments, the PI3K/AKT/mTOR pathway inhibitors comprise: PKI-587 (CAS 1197160-78-3), desmethyl PKI-587 (CAS 1950569-63-7), AZD8055 (CAS 1009298-09-2), Afuresertib (CAS 1047644-62-1), or analogs thereof.

In preferred embodiments, the estrogen receptor antagonists/degraders comprise Fulvestrant (CAS 129453-61-8), Elacestrant (CAS 1349723-93-8); the androgen receptor antagonists comprise abiraterone (CAS 154229-19-3), JNJ-63576253 (CAS 2110428-64-1), or analogs thereof.

In preferred embodiments, the autophagy inhibitor is Hydroxychloroquine (HCQ, CAS 118-42-3) and Chloroquine (CQ, CAS 54-05-7) or analogs thereof.

In preferred embodiments, the drug targeting transcription factors is a GSPT1 degrader (molecular glue), and the GSPT1 degrader is selected from the following structures:

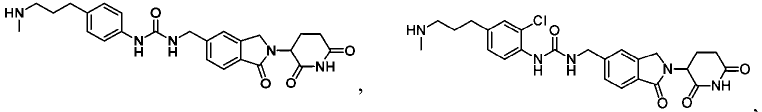

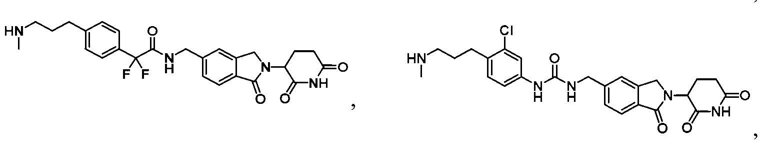

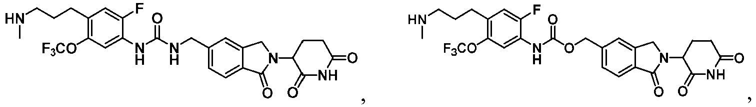

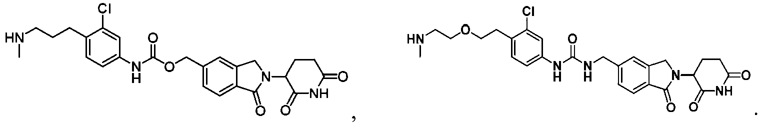

Preferably, the PLK1 inhibitor is BI2536 (CAS 755038-02-9), Volasertib (CAS 755038-65-4), GSK461364 (CAS 929095-18-1), Onvansertib (CAS 1034616-18-6), or analogs thereof;

In preferred embodiments, the N-myristoyltransferase (NMT) inhibitor is MYX1715 (CAS 2445448-66-6) or analogs thereof;

In preferred embodiments, D₁, D₂, and D₃ are selected from the combinations in the following table:

| Drug Combination | D1 | D2 | D3 |
|---|---|---|---|
| Combination 1 | TOPO1 inhibitor | PARP inhibitor | / |
| Combination 2 | TOPO1 inhibitor | ATR inhibitor | / |
| Combination 3 | TOPO1 inhibitor | CHK1 inhibitor | / |
| Combination 4 | TOPO1 inhibitor | PARP inhibitor | ATR inhibitor |
| Combination 5 | TOPO1 inhibitor | PARP inhibitor | CHK1 inhibitor |
| Combination 6 | TOPO1 inhibitor | PARP inhibitor | WEE1 inhibitor |
| Combination 7 | TOPO1 inhibitor | ATR inhibitor | WEE1 inhibitor |
| Combination 8 | TOPO1 inhibitor | CHK1 inhibitor | WEE1 inhibitor |
| Combination 9 | TOPO1 inhibitor | DNA-PK inhibitor | / |
| Combination 10 | TOPO1 inhibitor | POLQ inhibitor | / |
| Combination 11 | TOPO1 inhibitor | TOPO2 inhibitor | / |
| Combination 12 | PARP inhibitor | ATR/CHK1/WEE1 inhibitor | / |
| Combination 13 | PARP inhibitor | ATR inhibitor | CHK1 inhibitor |
| Combination 14 | TOPO1 inhibitor | Immune activator (PD-L1 inhibitor, CBL-B inhibitor, TLR7/8 agonist, PTPN2/1 inhibitor, or STING agonist) | / |
| Combination 15 | TOPO1 inhibitor | BCL-2 family inhibitor | / |
| Combination 16 | TOPO1 inhibitor | BCL-2 family inhibitor | MCL-1 inhibitor |
| Combination 17 | TOPO1 inhibitor | DNA synthesis inhibitor | / |
| Combination 18 | TOPO1 inhibitor | PI3K/mTOR inhibitor | / |
| Combination 19 | TOPO1 inhibitor | EGFR inhibitor | / |
| Combination 20 | TOPO1 inhibitor | Autophagy inhibitor | / |
| Combination 21 | TOPO1 inhibitor | FAK inhibitor | |
| Combination 22 | TOPO1 inhibitor | PARP inhibitor | FAK inhibitor |
| Combination 23 | TOPO1 inhibitor | PARP inhibitor | Androgen receptor antagonist/degrader |
| Combination 24 | TOPO1 inhibitor | GSPT1 degrader | / |
| Combination 25 | TOPO1 inhibitor | PARP inhibitor | GSPT1 degrader |
| Combination 26 | TOPO1 inhibitor | Tubulin inhibitor | / |
| Combination 27 | TOPO1 inhibitor | Epigenetic regulator | / |
| Combination 28 | Tubulin inhibitor | Immune activator (PD-L1 inhibitor, CBL-B inhibitor, TLR7/8 agonist, PTPN2/1 inhibitor, or STING agonist) | / |
| Combination 29 | Tubulin inhibitor | BCL-2/BCL-XL inhibitor | / |
| Combination 30 | Tubulin inhibitor | BCL-2/BCL-XL inhibitor | MCL-1 inhibitor |
| Combination 31 | Tubulin inhibitor | PI3K/AKT/mTOR inhibitor | / |
| Combination 32 | Tubulin inhibitor | FAK inhibitor | |
| Combination 33 | CDK4/6 inhibitor | MEK inhibitor | / |
| Combination 34 | CDK4/6 inhibitor | MEK inhibitor | EZH2 inhibitor |
| Combination 35 | CDK4/6 inhibitor | Autophagy inhibitor | / |
| Combination 36 | MEK inhibitor | Autophagy inhibitor | / |
| Combination 37 | ERK inhibitor | Autophagy inhibitor | / |
| Combination 38 | CDK4/6 inhibitor | MEK inhibitor | Autophagy inhibitor |
| Combination 39 | CDK4/6 inhibitor | ERK inhibitor | Autophagy inhibitor |
| Combination 40 | CDK4/6 inhibitor | ERK inhibitor | (MEK inhibitor) |
| Combination 41 | CDK4/6 inhibitor | MEK inhibitor | KRAS inhibitor |
| Combination 42 | CDK4/6 inhibitor | MEK inhibitor | SOS1 inhibitor |
| Combination 43 | CDK4/6 inhibitor | MEK inhibitor | FAK inhibitor |
| Combination 44 | CDK4/6 inhibitor | PRMT5 inhibitor | / |
| Combination 45 | CDK4/6 inhibitor | Estrogen receptor antagonist/degrader | / |
| Combination 46 | CDK4/6 inhibitor | Estrogen receptor antagonist/degrader | PI3K/mTOR inhibitor |
| Combination 47 | CDK4/6 inhibitor | MEK inhibitor | GSPT1 degrader |
| Combination 48 | CDK4/6 inhibitor | ERK inhibitor | GSPT1 degrader |
| Combination 49 | CDK4/6 inhibitor | Autophagy inhibitor | GSPT1 degrader |
| Combination 50 | BRD4 inhibitor | EZH2 inhibitor | / |
| Combination 51 | PRMT5 inhibitor | MAT2A inhibitor | / |
| Combination 52 | PRMT5 inhibitor | Gemcitabine | / |
| Combination 53 | PRMT5 inhibitor | CDK4/6 inhibitor | / |
| Combination 54 | PRMT5 inhibitor | PRMT1 inhibitor | / |
| Combination 55 | PRMT5 inhibitor | PRMT1 inhibitor | CDK4/6 inhibitor |
| Combination 56 | PRMT5 inhibitor | PRMT1 inhibitor | MEK inhibitor |
| Combination 57 | RAF inhibitor | MEK inhibitor | / |
| Combination 58 | EGFR inhibitor | MEK inhibitor | / |
| Combination 59 | RAF inhibitor | MEK inhibitor | SHP2 inhibitor |
| Combination 60 | CDK4/6 inhibitor | HIF-2alpha inhibitor | / |
| Combination 61 | TOPO1 inhibitor | WEE1 inhibitor | / |
| Combination 62 | WEE1 inhibitor | CHK1 inhibitor | / |
| Combination 63 | TOPO1 inhibitor | TOPO1 inhibitor | / |
| Combination 64 | CDK inhibitor | MEK inhibitor | |
| Combination 65 | FAK inhibitor | Smo inhibitor | / |
| Combination 66 | PLK1 inhibitor | CDK inhibitor | / |
| Combination 67 | PLK1 inhibitor | CDK inhibitor | PARP inhibitor/ |
| Combination 68 | TOPO1 inhibitor | PLK1 inhibitor | / |
| Combination 69 | TOPO1 inhibitor | PLK1 inhibitor | PARP inhibitor/ |
| Combination 70 | TOPO1 inhibitor | PLK1 inhibitor | ATR inhibitor/ |
| Combination 71 | TOPO1 inhibitor | NMT inhibitor | / |
| Combination 72 | Tubulin inhibitor | NMT inhibitor | / |

In preferred embodiments, at least one of D₁, D₂, and D₃ is a topoisomerase inhibitor, and at least another one is a PARP inhibitor, an ATR inhibitor, or a CHK1 inhibitor. In preferred embodiments, at least one of D₁, D₂, and D₃ is a topoisomerase inhibitor, and at least another one is a Bcl-2 family protein inhibitor. In preferred embodiments, at least one of D₁, D₂, and D₃ is a topoisomerase inhibitor, and at least another one is an LSD1 inhibitor, an EZH2 inhibitor, or a PRMT5 inhibitor. In preferred embodiments, at least one of D₁, D₂, and D₃ is a topoisomerase inhibitor, and at least another one is a PD-L1 inhibitor, a CBL-B inhibitor, a TLR7/8 agonist, or a STING agonist.

In preferred embodiments, at least one of D₁, D₂, and D₃ is selected from: Exatecan (CAS 171335-80-1), DXd (CAS 1599440-33-1), 7-ethyl-10-hydroxycamptothecin (SN38, CAS 86639-52-3), Belotecan (CAS 213819-48-8), (4-NH₂)-Exatecan (AZD'0132, CAS 2495742-21-5), 7-MAD-MDCPT (CAS 765871-81-6), 7-aminomethyl-10-methyl-11-fluorocamptothecin (CAS 2378616-23-8), Voreloxin (CAS 175414-77-4), or derivatives and analogues thereof; and at least another one is selected from PARP inhibitors, ATR inhibitors, CHK1 inhibitors, Bcl-2 family protein inhibitors, LSD1 inhibitors, EZH2 inhibitors, BRD4 inhibitors, PRMT5 inhibitors, PD-L1 inhibitors, CBL-B inhibitors, TLR7/8 agonists, and STING agonists.

In preferred embodiments, at least one of D₁, D₂, and D₃ is selected from: Exatecan (CAS 171335-80-1), DXd (CAS 1599440-33-1), 7-ethyl-10-hydroxycamptothecin (SN38, CAS 86639-52-3), Belotecan (CAS 213819-48-8), (4-NH₂)-Exatecan (AZD'0132, CAS 2495742-21-5), 7-MAD-MDCPT (CAS 765871-81-6), 7-aminomethyl-10-methyl-11-fluorocamptothecin (CAS 2378616-23-8), Voreloxin (CAS 175414-77-4), or derivatives and analogues thereof; and at least another one is selected from: Rucaparib, Niraparib, Veliparib, A-966492, Talazoparib, AZD5305, Venadaparib, Mefuparib, Prexasertib (LY2606368), AZD7762, Rabusertib (LY2603618), MK-8776 (SCH 900776), CHIR-124, PF-477736, CCT245737 (SRA737, PNT-737), GDC-0575 (ARRY-575), Berzosertib, Ceralasertib, RP-6306, GSK-1520489A, and analogues thereof.

In preferred embodiments, at least one of D₁, D₂, and D₃ is a tubulin inhibitor, and at least another one is a PARP inhibitor, an ATR inhibitor, or a CHK1 inhibitor. In preferred embodiments, at least one of D₁, D₂, and D₃ is a tubulin inhibitor, and at least another one is a Bcl-2 family protein inhibitor. In preferred embodiments, at least one of D₁, D₂, and D₃ is a tubulin inhibitor, and at least another one is an LSD1 inhibitor, an EZH2 inhibitor, or a PRMT5 inhibitor. In preferred embodiments, at least one of D₁, D₂, and D₃ is a tubulin inhibitor, and at least another one is a PD-L1 inhibitor, a CBL-B inhibitor, a TLR7/8 agonist, or a STING agonist.

In preferred embodiments, at least one of D₁, D₂, and D₃ is selected from: Eribulin, Vinblastine, Paclitaxel, MMAE, MMAF, Maytansine or derivatives thereof, and at least another one is selected from PARP inhibitors, ATR inhibitors, CHK1 inhibitors, Bcl-2 family protein inhibitors, LSD1 inhibitors, EZH2 inhibitors, BRD4 inhibitors, PRMT5 inhibitors, PD-L1 inhibitors, CBL-B inhibitors, TLR7/8 agonists, and STING agonists.

In preferred embodiments, at least one of D₁, D₂, and D₃ is selected from: Eribulin, Vinblastine, Paclitaxel, MMAE, MMAF, Maytansine or derivatives thereof; and at least another one is selected from: Rucaparib, Niraparib, Veliparib, A-966492, Talazoparib, AZD5305, Venadaparib, Mefuparib, Prexasertib (LY2606368), AZD7762, Rabusertib (LY2603618), MK-8776 (SCH 900776), CHIR-124, PF-477736, CCT245737 (SRA737, PNT-737), GDC-0575 (ARRY-575), Berzosertib, Ceralasertib, RP-6306, GSK-1520489A, and analogues thereof.

In preferred embodiments, at least one of D₁, D₂, and D₃ is selected from: a PBD or Duocarmycin, and at least another one is selected from PARP inhibitors, ATR inhibitors, CHK1 inhibitors, Bcl-2 family protein inhibitors, LSD1 inhibitors, EZH2 inhibitors, BRD4 inhibitors, PRMT5 inhibitors, PD-L1 inhibitors, CBL-B inhibitors, TLR7/8 agonists, and STING agonists.

In preferred embodiments, at least one of D₁, D₂, and D₃ is a PBD or Duocarmycin, and at least another one is selected from: Rucaparib, Niraparib, Veliparib, A-966492, Talazoparib, AZD5305, Venadaparib, Mefuparib, Prexasertib (LY2606368), AZD7762, Rabusertib (LY2603618), MK-8776 (SCH 900776), CHIR-124, PF-477736, CCT245737 (SRA737, PNT-737), GDC-0575 (ARRY-575), Berzosertib, Ceralasertib, RP-6306, GSK-1520489A, and analogues thereof.

In preferred embodiments, at least two of D₁, D₂, and D₃ are drugs targeting the DNA Damage Response (DDR) or "synthetic lethality" related pathways. In preferred embodiments, at least two of D₁, D₂, and D₃ are selected from: Rucaparib, Niraparib, Veliparib, A-966492, Talazoparib, AZD5305, Venadaparib, Mefuparib, Prexasertib (LY2606368), AZD7762, Rabusertib (LY2603618), MK-8776 (SCH 900776), CHIR-124, PF-477736, CCT245737 (SRA737, PNT-737), GDC-0575 (ARRY-575), Berzosertib, Ceralasertib, RP-6306, GSK-1520489A, and analogues thereof.

In preferred embodiments, D₁, D₂, and D₃ can be selected from the exemplary combinations shown in Table A.

**Table A. Exemplary combinations of D₁, D₂, and D₃.**

| | D1 | D2 | D3 |
|---|---|---|---|
| Combination 1-1 | 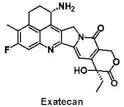 | 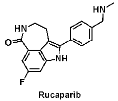 | / |
| Combination 1-2 | 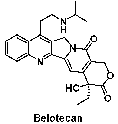 | 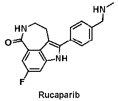 | / |
| Combination 1-3 | 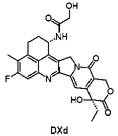 | 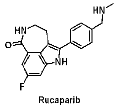 | / |
| Combination 1-4 | 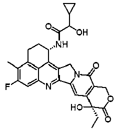 | 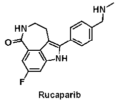 | / |
| Combination 1-5 | 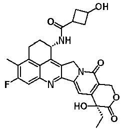 | 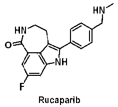 | / |
| Combination 1-6 | 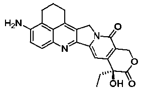 | 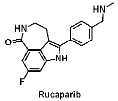 | / |
| Combination 1-7 | 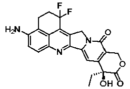 | 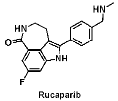 | / |
| Combination 1-8 | 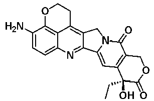 | 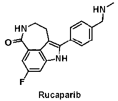 | / |
| Combination 1-9 | 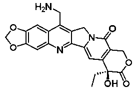 | 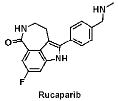 | / |
| Combination 1-10 | 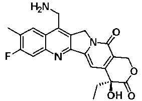 | 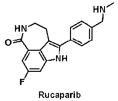 | / |
| Combination 1-11 | 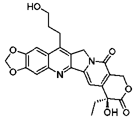 | 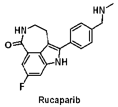 | / |
| Combination 1-12 | 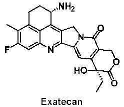 | 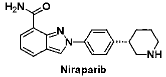 | / |
| Combination 1-13 | 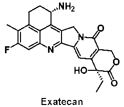 | 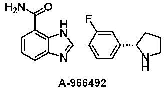 | / |
| Combination 1-14 | 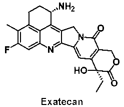 | 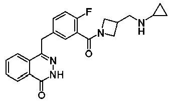 | / |
| Combination 1-15 | 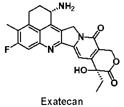 | 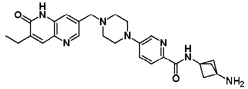 | / |
| Combination 1-16 | 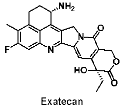 | 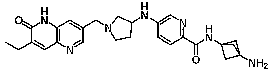 | / |
| Combination 1-17 | 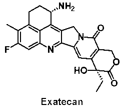 | 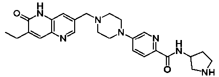 | / |
| Combination 1-18 | 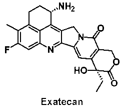 | 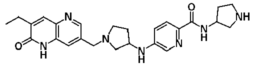 | / |
| Combination 1-19 | 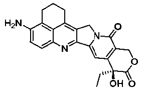 | 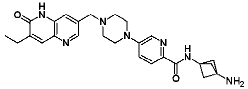 | / |
| Combination 2-1 | 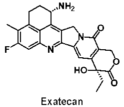 | 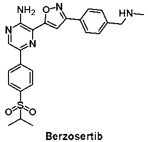 | / |
| Combination 2-2 | 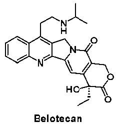 | 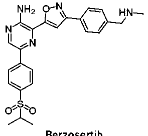 | / |
| Combination 2-3 | 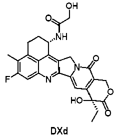 | 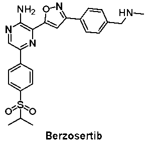 | / |
| Combination 2-4 | 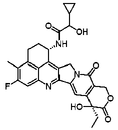 | 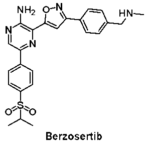 | / |
| Combination 2-5 | 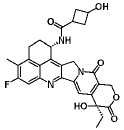 | 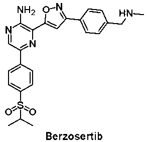 | / |
| Combination 2-6 | 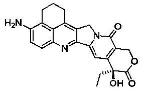 | 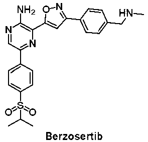 | / |
| **Combination** 2-7 | 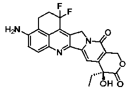 | 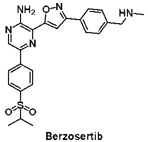 | / |
| Combination 3-1 | 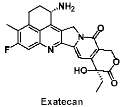 | 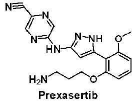 | / |
| Combination 3-2 | 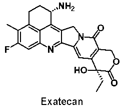 | 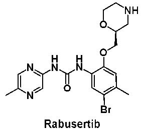 | / |
| Combination 3-3 | 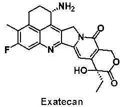 | 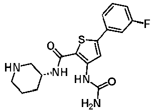 | / |
| Combination 3-4 | 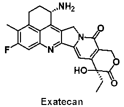 | 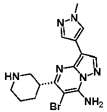 | / |
| Combination 3-5 | 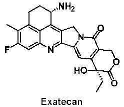 | 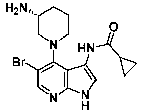 | / |
| Combination 3-6 | 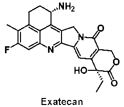 | 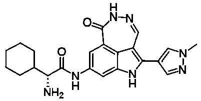 | / |
| Combination 3-7 | 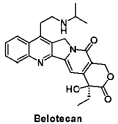 | 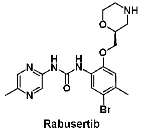 | / |
| Combination 3-8 | 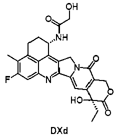 | 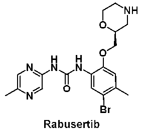 | / |
| Combination 3-9 | 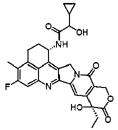 | 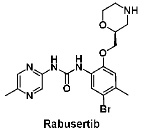 | / |
| Combination 3-10 | 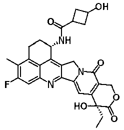 | 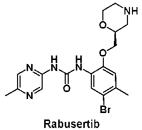 | / |
| Combination 3-11 | 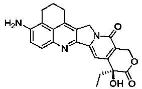 | 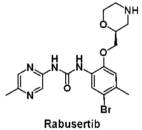 | / |
| Combination 3-12 | 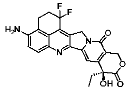 | 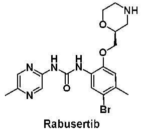 | / |
| Combination 9-1 | 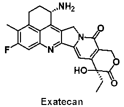 | 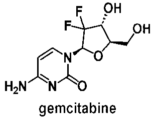 | / |
| Combination 11-1 | 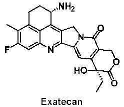 | 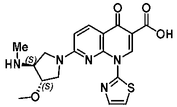 | / |
| Combination 17-1 | 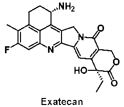 | 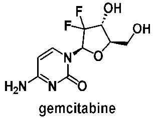 | / |
| Combination 4-1 | 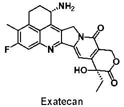 | 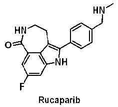 | 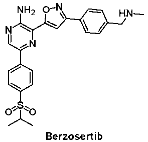 |
| Combination 5-1 | 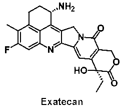 | 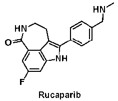 | 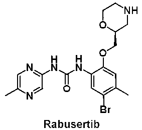 |
| Combination 5-2 | 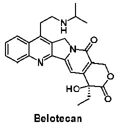 | 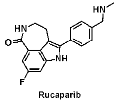 | 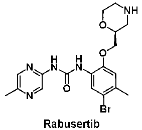 |
| Combination 13-1 | 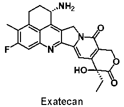 | 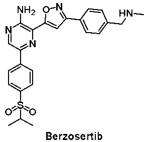 | 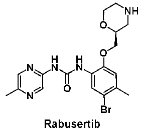 |
| Combination 4-2 | 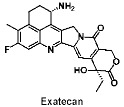 | 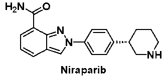 | 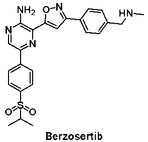 |
| Combination 13-1 | 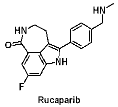 | 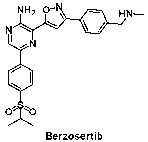 | 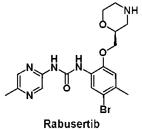 |
| Combination 13-2 | 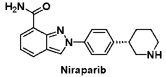 | 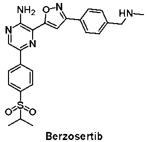 | 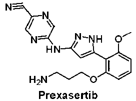 |
| Combination 14-1 | 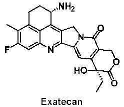 | 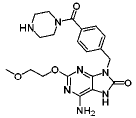 | / |
| Combination 14-2 | 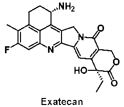 | 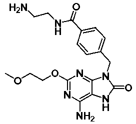 | / |
| Combination 14-3 | 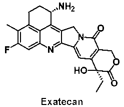 | 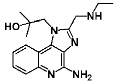 | / |
| Combination 14-4 | 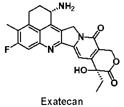 | 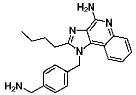 | / |
| Combination 14-5 | 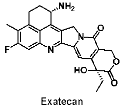 | 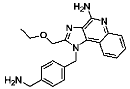 | / |
| Combination 14-6 | 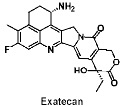 | 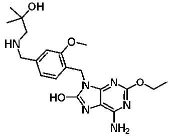 | / |
| Combination 14-7 | 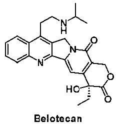 | 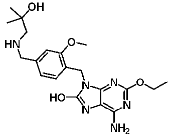 | / |
| Combination 14-8 | 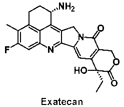 | 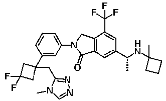 | / |
| Combination 14-9 | 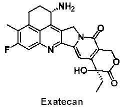 | 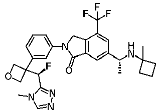 | / |
| Combination 14-10 | 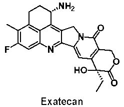 | 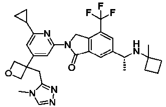 | / |
| Combination 14-11 | 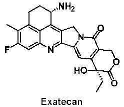 | 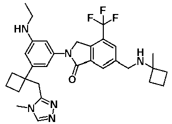 | / |
| Combination 14-12 | 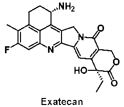 | 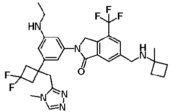 | / |
| Combination 27-1 | 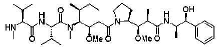 | 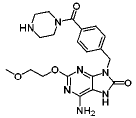 | / |
| Combination 27-2 | 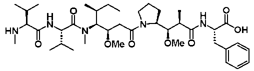 | 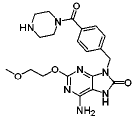 | / |
| Combination 27-3 | 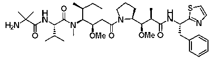 | 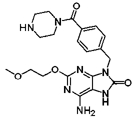 | / |
| Combination 27-4 | 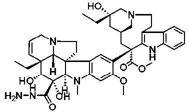 | 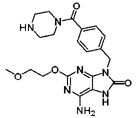 | / |
| Combination 27-5 | 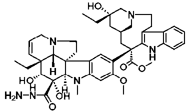 | 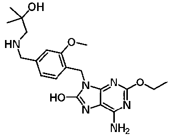 | / |
| Combination 15-1 | 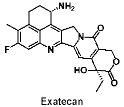 | 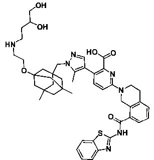 | / |
| Combination 15-2 | 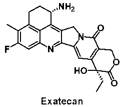 | 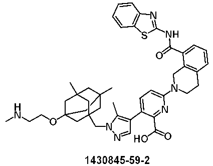 | / |
| Combination 15-3 | 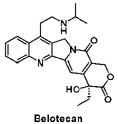 | 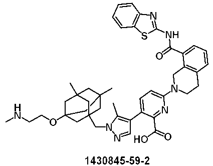 | / |
| Combination 27-1 | 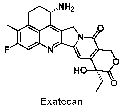 | 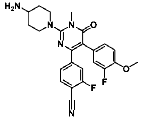 | |
| **Combination** 27-2 | 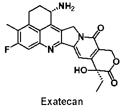 | 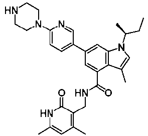 | / |
| Combination 26-1 | 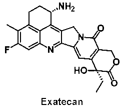 | 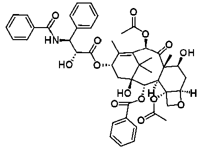 | / |
| Combination 61-1 | 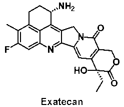 | 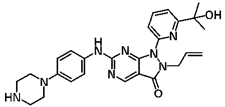 | / |
| Combination 8-1 | 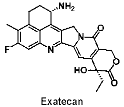 | 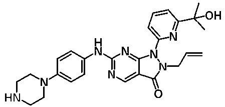 | 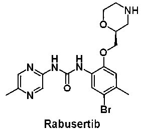 |
| Combination 8-2 | 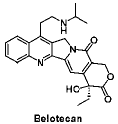 | 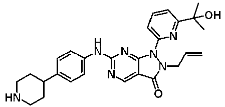 | 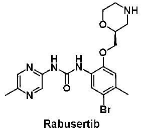 |
| Combination 62 | 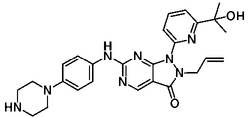 | 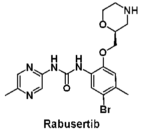 | / |
| Combination 63 | 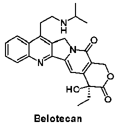 | 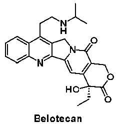 | / |
| Combination 63 | 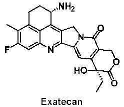 | 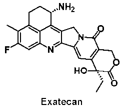 | / |
| Combination 71-1 | 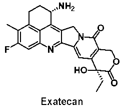 | 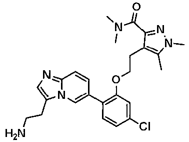 | / |
| Combination 71-2 | 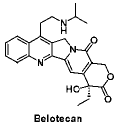 | 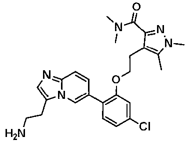 | / |
| Combination 71-3 | 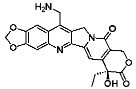 | 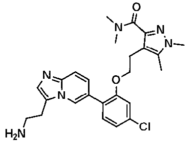 | / |
| Combination 71-4 | 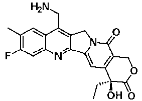 | 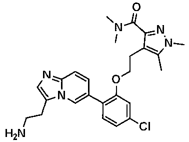 | / |
| Combination 72-1 | 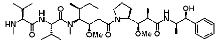 | 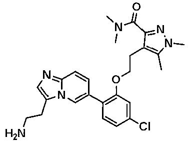 | / |
| Combination 72-2 | 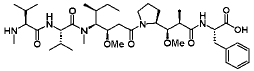 | 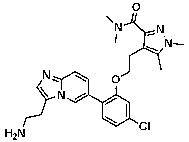 | / |
| Combination 72-3 | 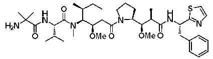 | 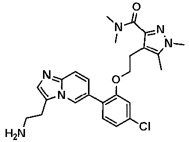 | / |
| Combination 72-4 | 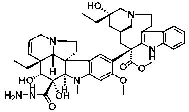 | 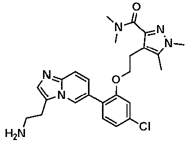 | / |

In preferred embodiments, the present invention provides compounds selected from formulas **C1** to **C190,** or a tautomer, a mesomer, a racemate, an enantiomer, or a diastereomer thereof, or a deuterated compound form thereof, or a mixture form thereof, or a pharmaceutically acceptable salt, prodrug, or solvate thereof.

| | |
|---|---|
| 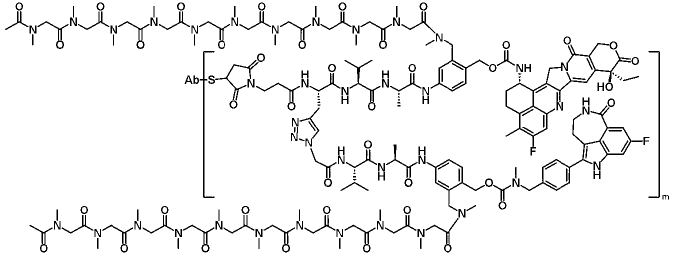 | Formula C1 |
| 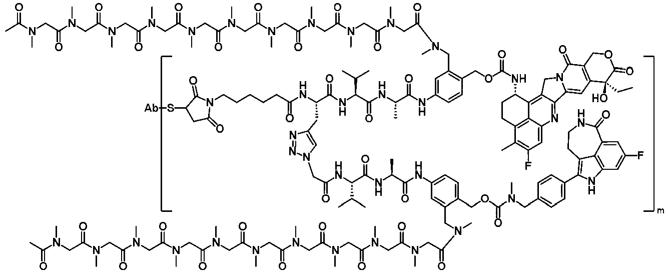 | Formula C2 |
| 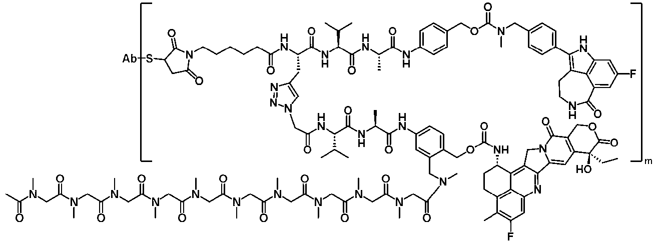 | Formula C3 |
| 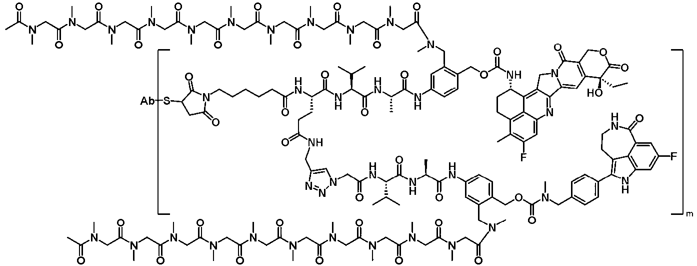 | Formula C4 |
| 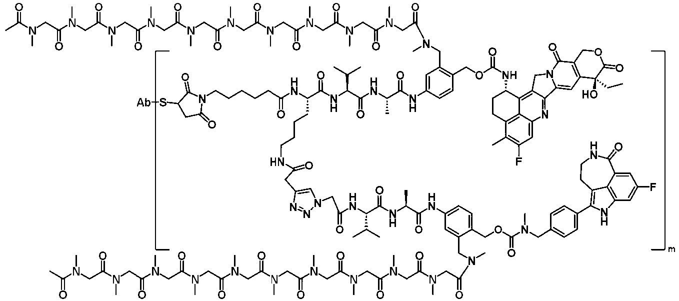 | Formula C5 |
| 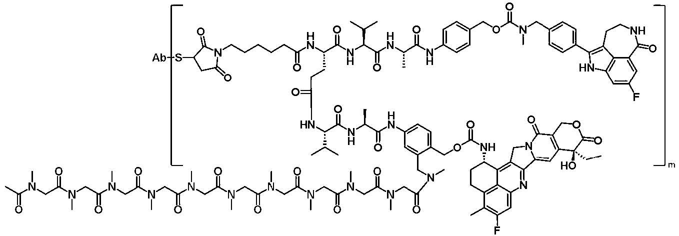 | Formula C6 |
| 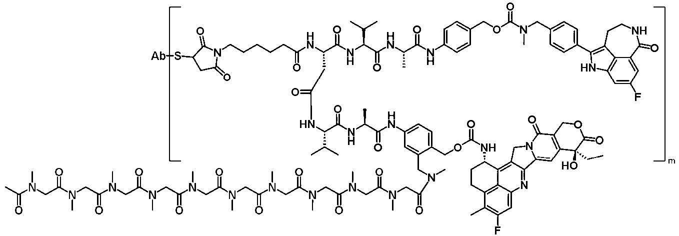 | Formula C7 |
| 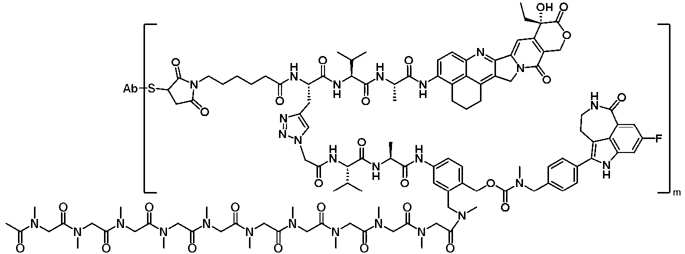 | Formula C8 |
| 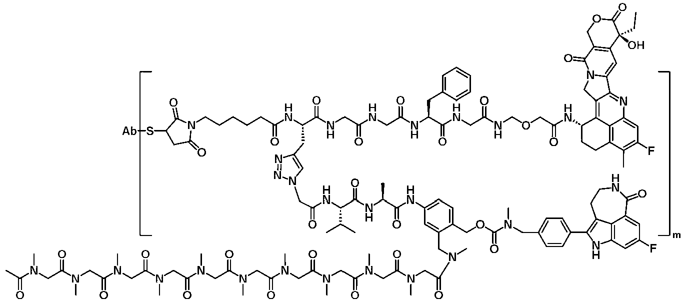 | Formula C9 |
| 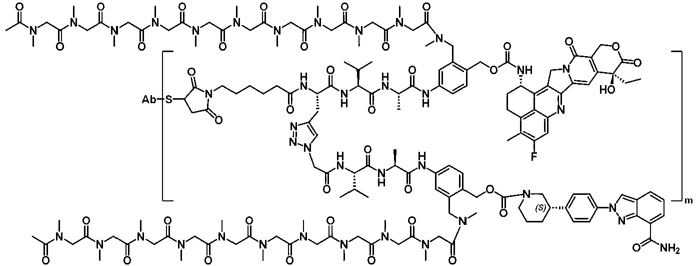 | Formula C10 |
| 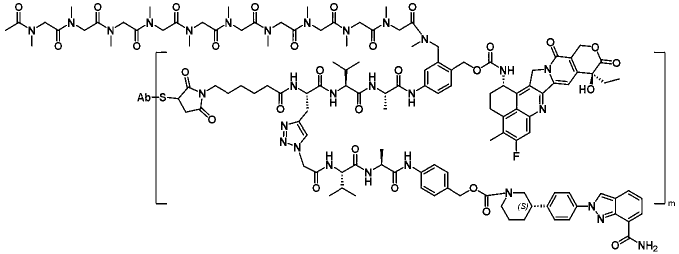 | Formula C11 |
| 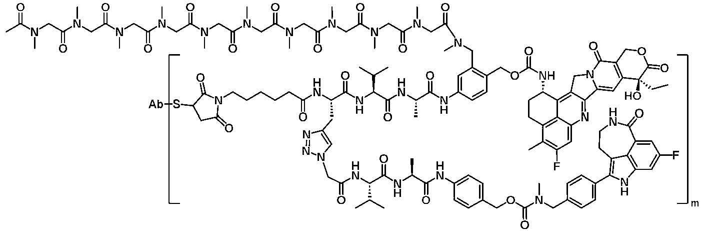 | Formula C12 |
| 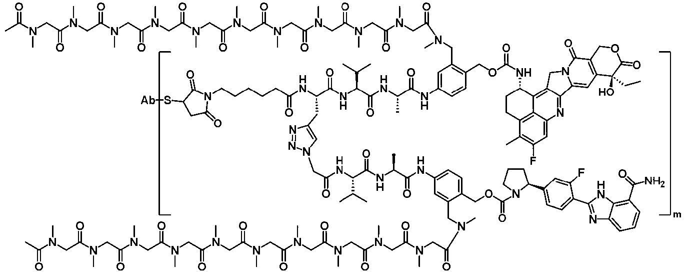 | Formula C13 |
| 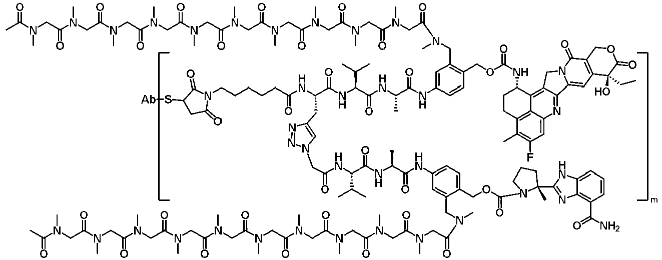 | Formula C14 |
| 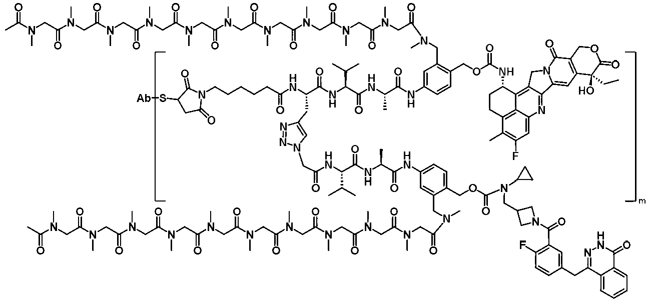 | Formula C15 |
| 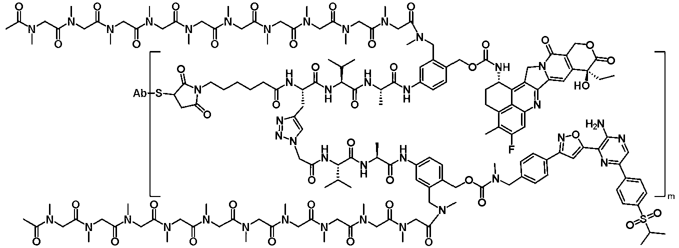 | Formula C16 |
| 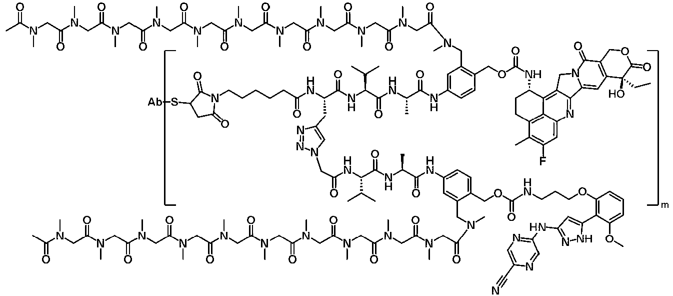 | Formula C17 |
| 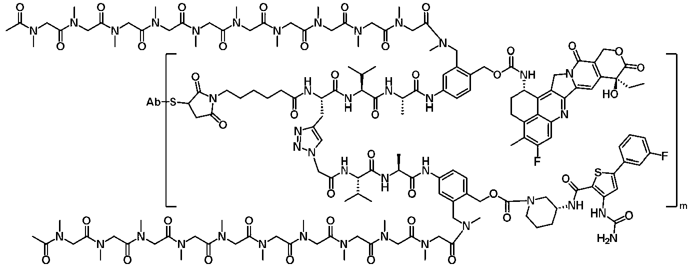 | Formula C18 |
| 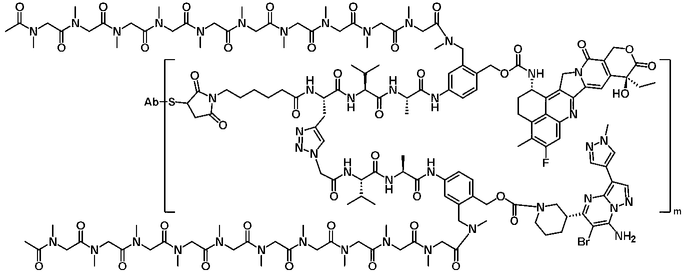 | Formula C19 |
| 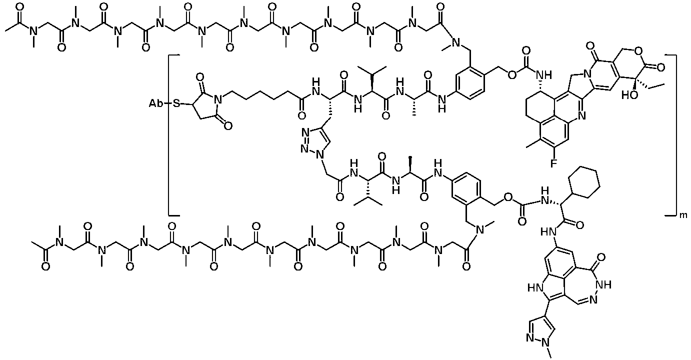 | Formula C20 |
| 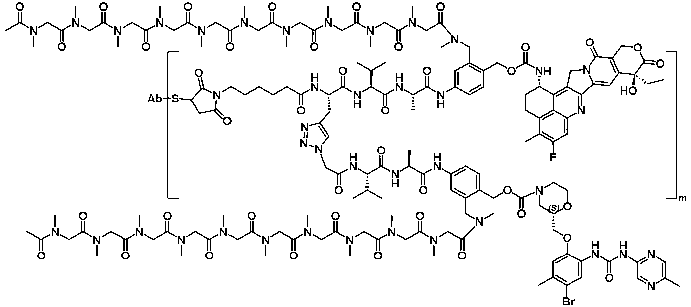 | Formula C21 |
| 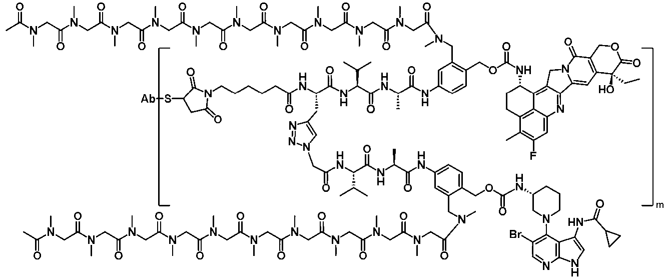 | Formula C22 |
| 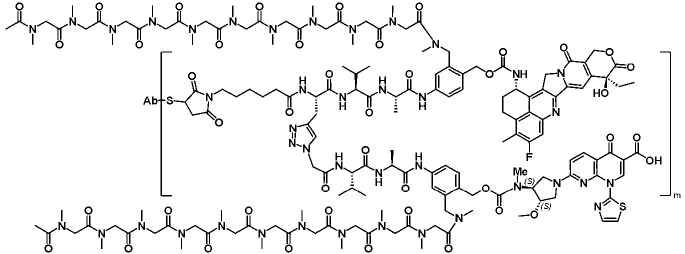 | Formula C23 |
| 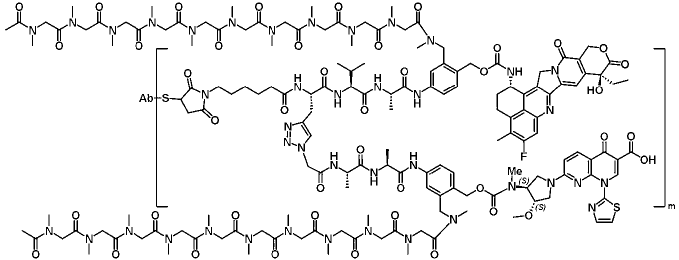 | Formula C24 |
| 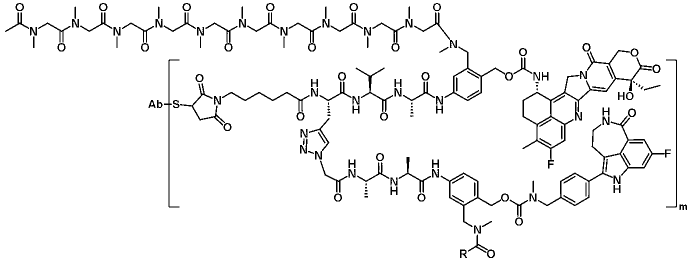 | Formula C25 |
| 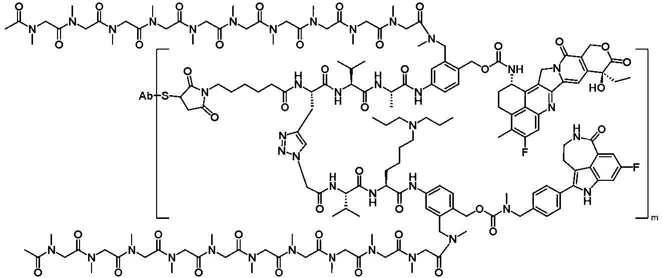 | Formula C26 |
| 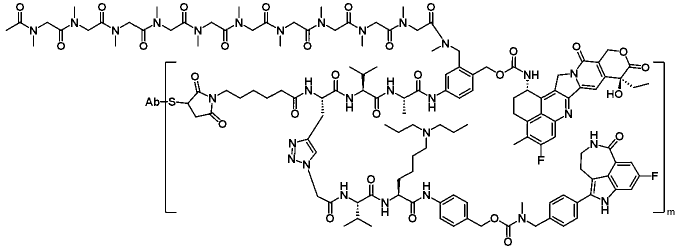 | Formula C27 |
| 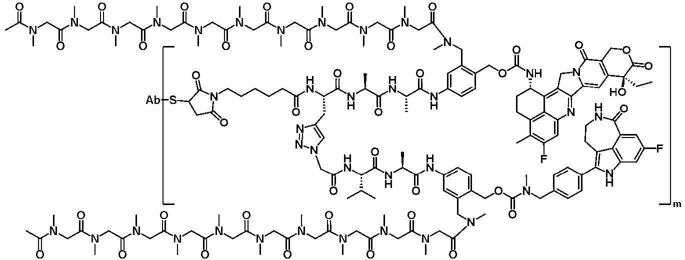 | Formula C28 |
| 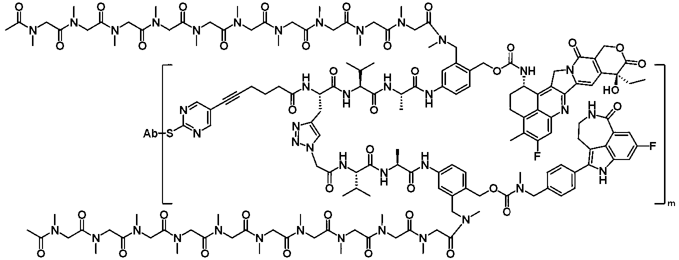 | Formula C29 |
| 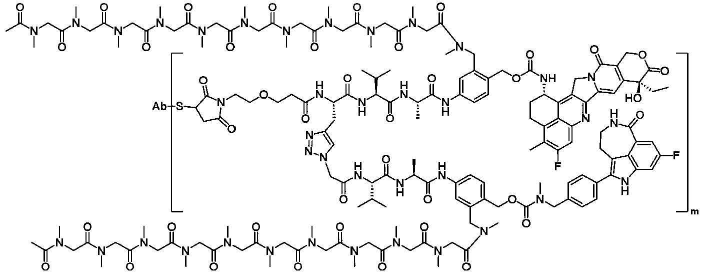 | Formula C30 |
| 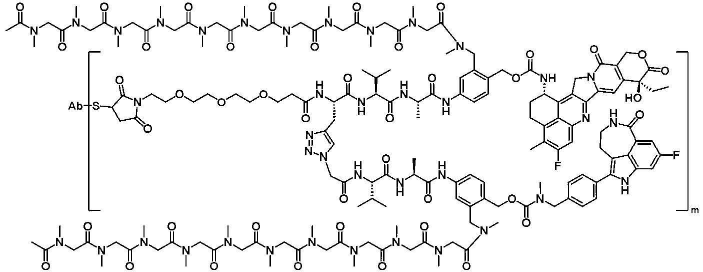 | Formula C31 |
| 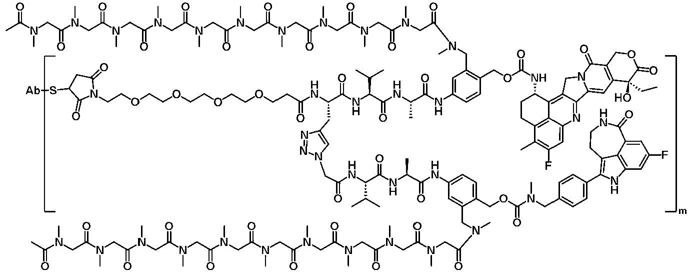 | Formula C32 |
| 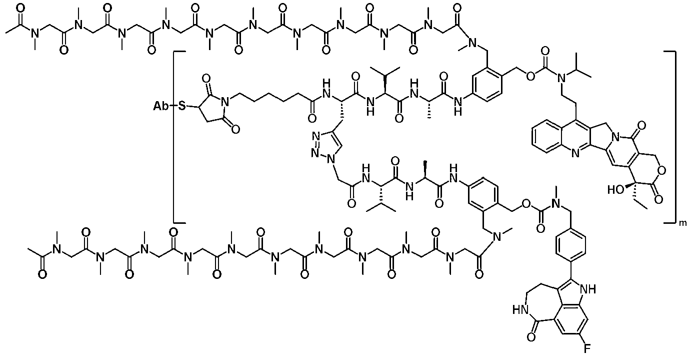 | Formula C33 |
| 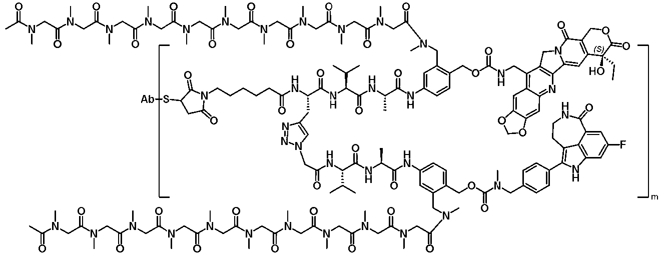 | Formula C34 |
| 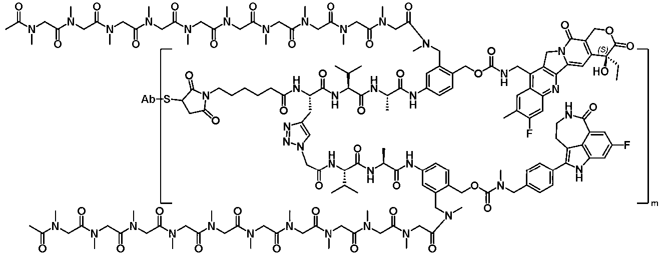 | Formula C35 |
| 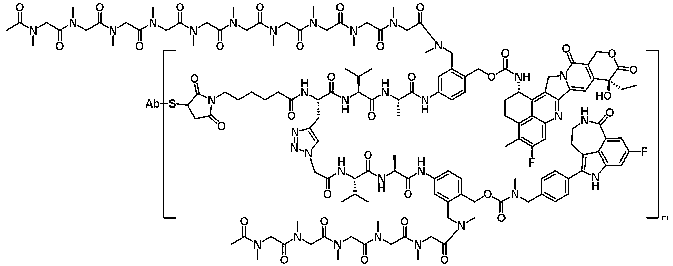 | Formula C36 |
| 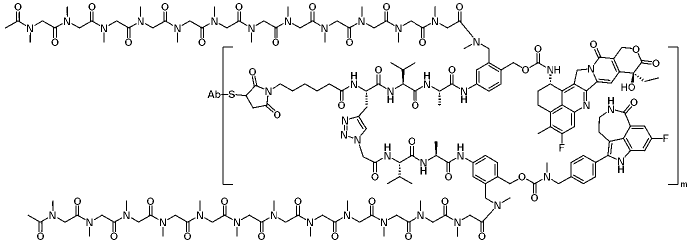 | Formula C37 |
| 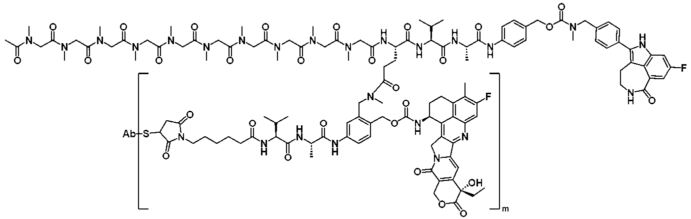 | Formula C38 |
| 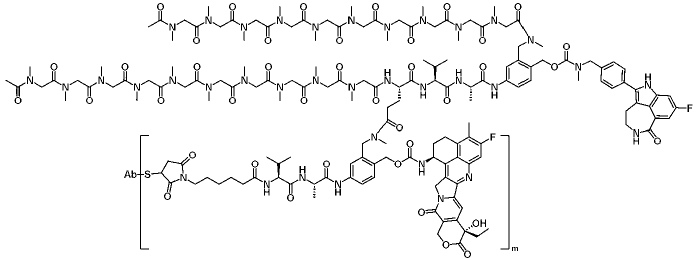 | Formula C39 |
| 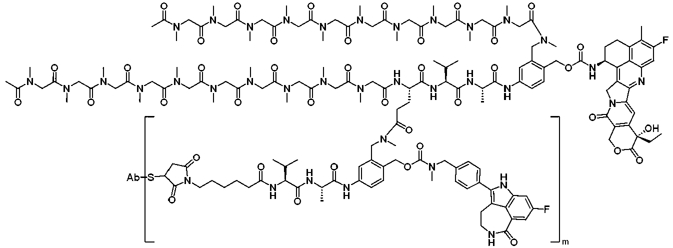 | Formula C40 |
| 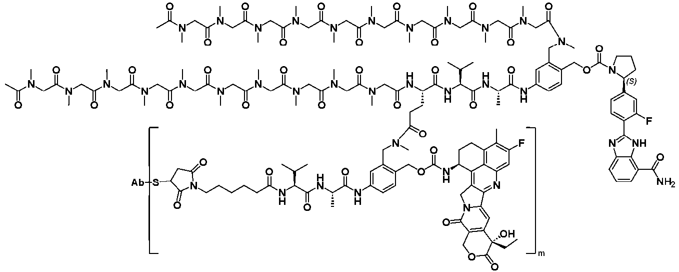 | Formula C41 |
| 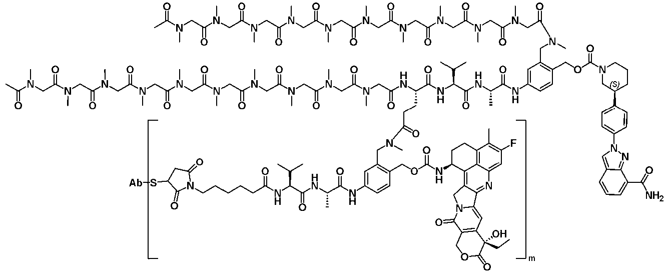 | Formula C42 |
| 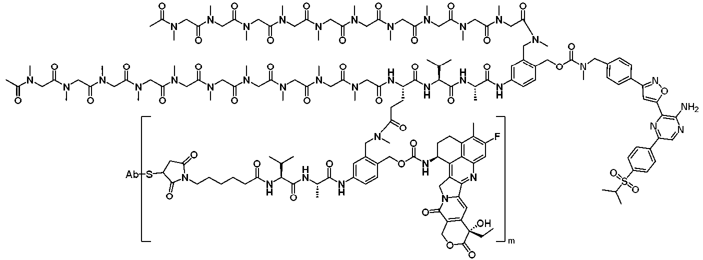 | Formula C43 |
| 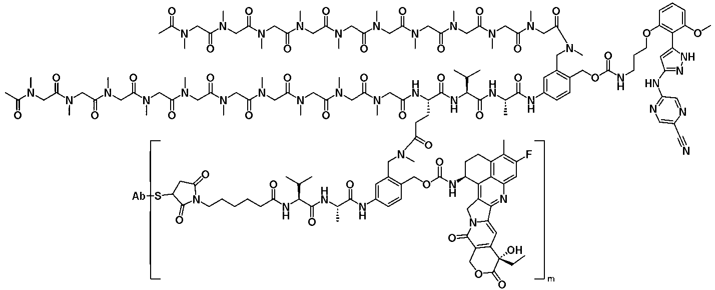 | Formula C44 |
| 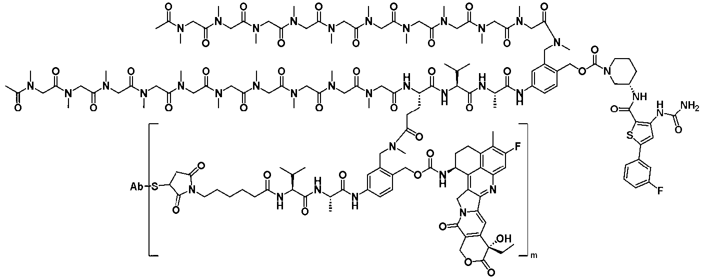 | Formula C45 |
| 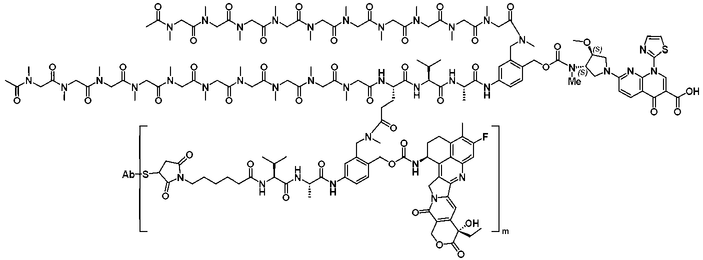 | Formula C46 |
| 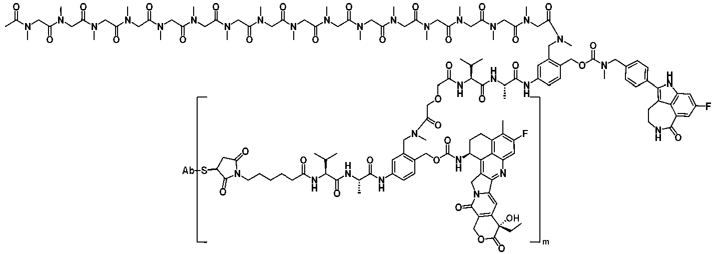 | Formula C47 |
| 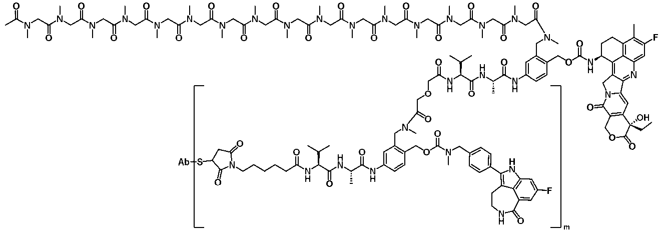 | Formula C48 |
| 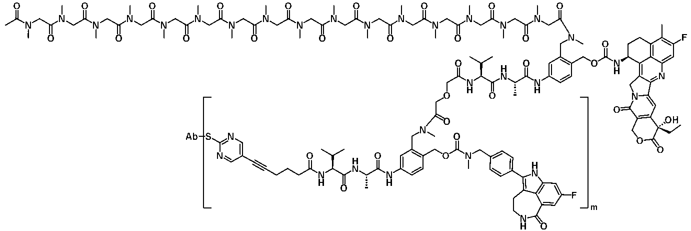 | Formula C49 |
| 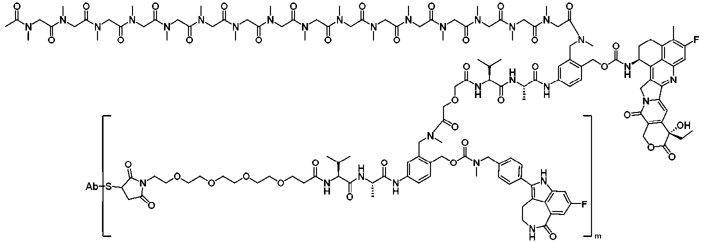 | Formula C50 |
| 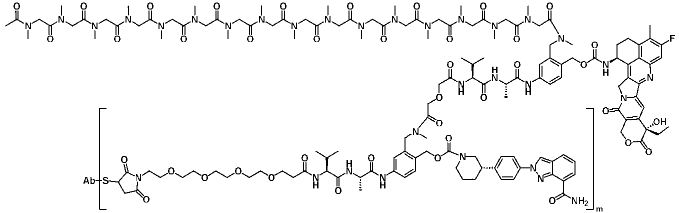 | Formula C51 |
| 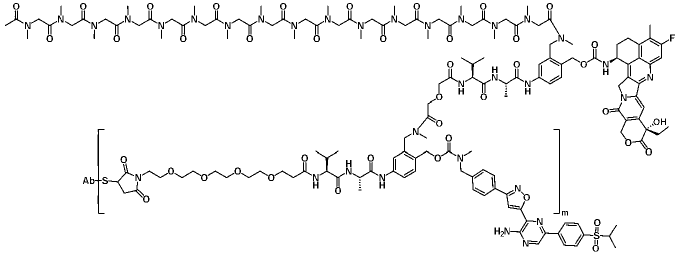 | Formula C52 |
| 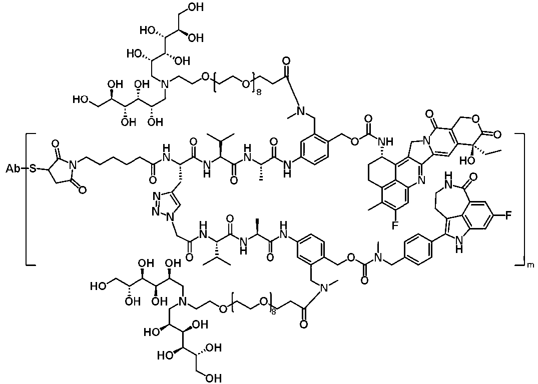 | Formula C53 |
| 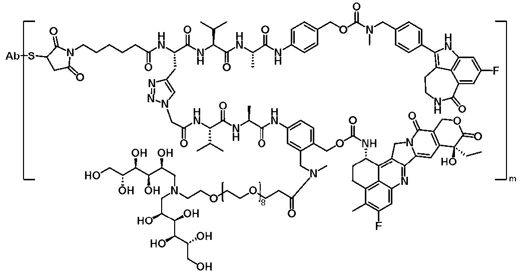 | Formula C54 |
| 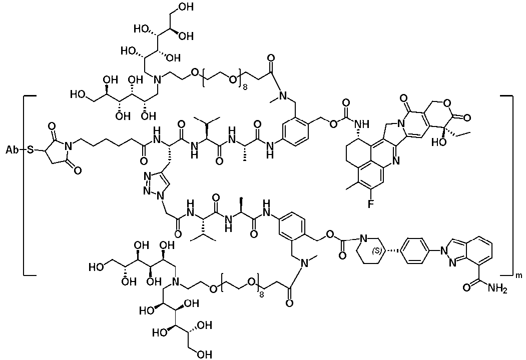 | Formula C55 |
| 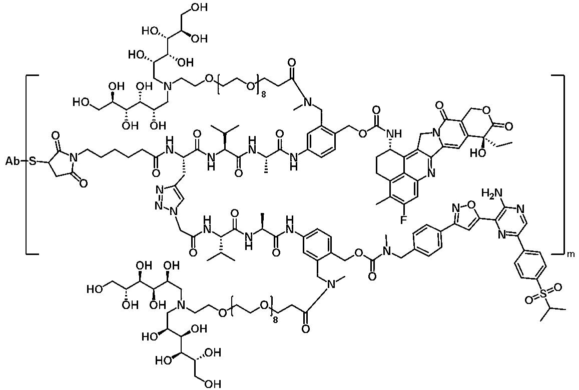 | Formula C56 |
| 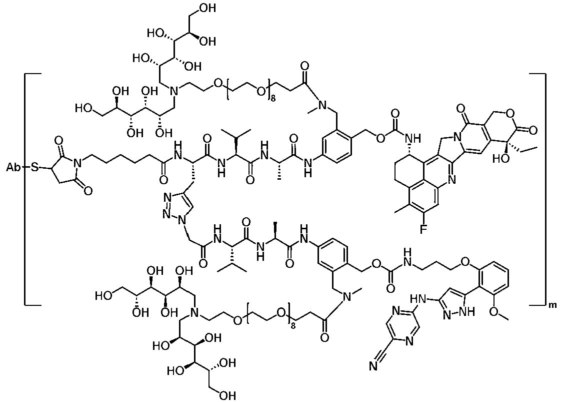 | Formula C57 |
| 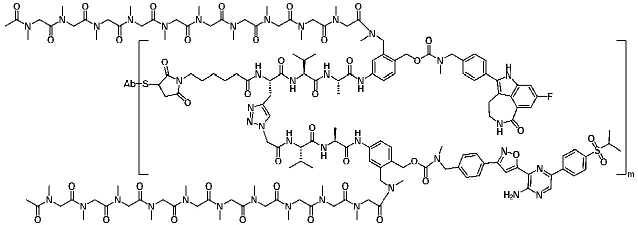 | Formula C58 |
| 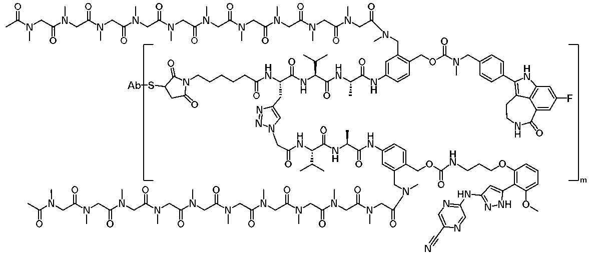 | Formula C59 |
| 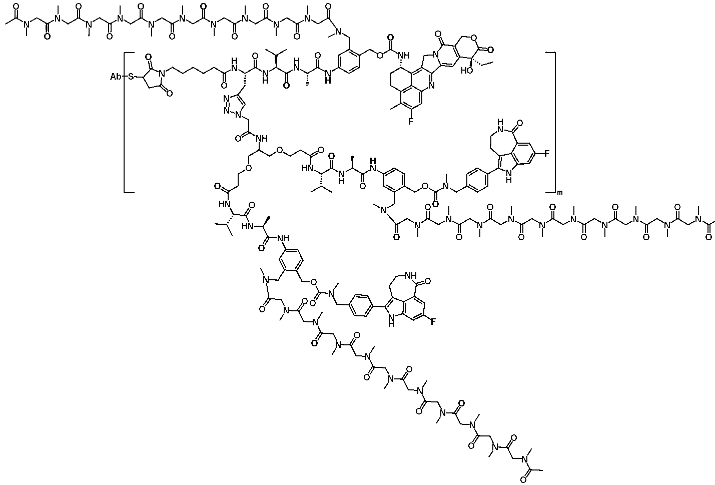 | Formula C60 |
| 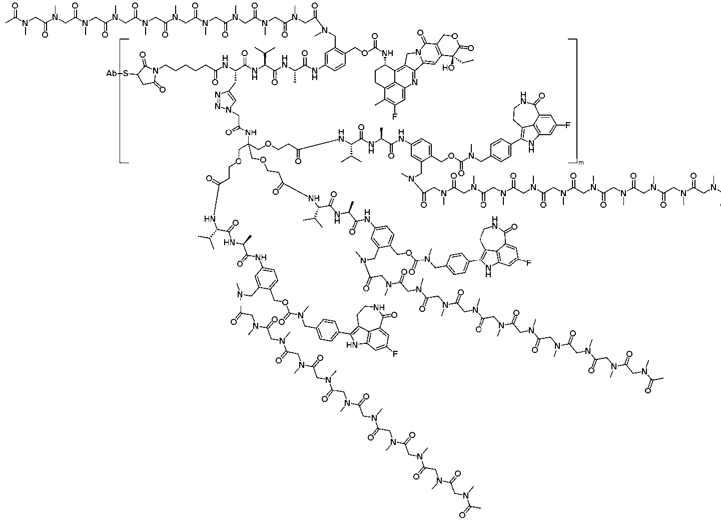 | Formula C61 |
| 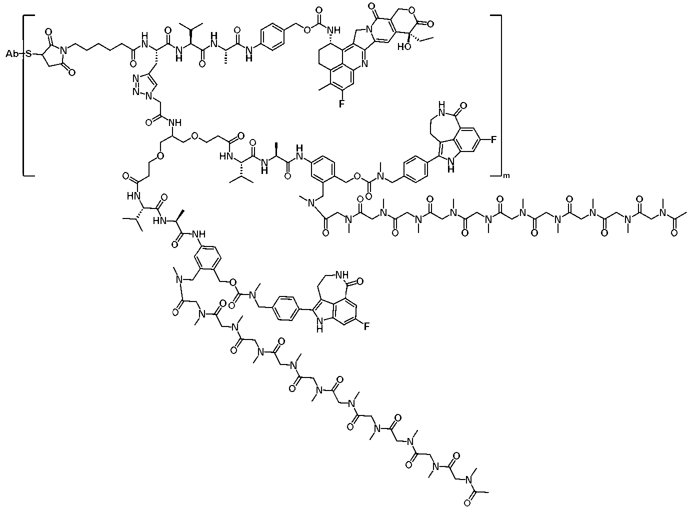 | Formula C62 |
| 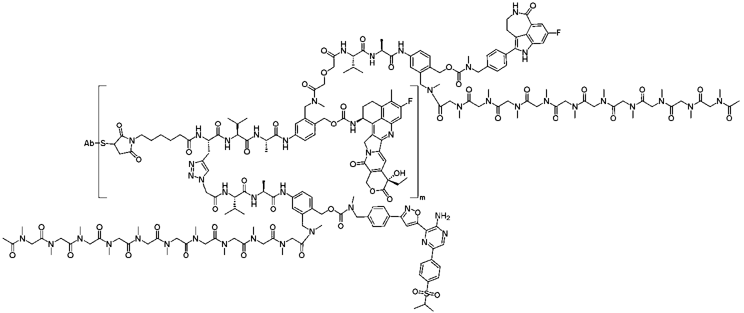 | Formula C63 |
| 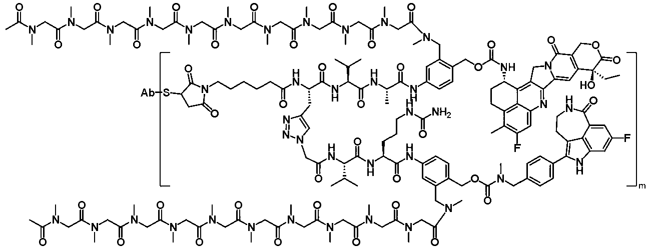 | Formula C64 |
| 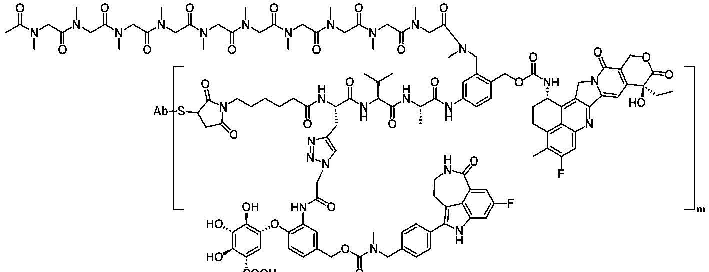 | Formula C65 |
| 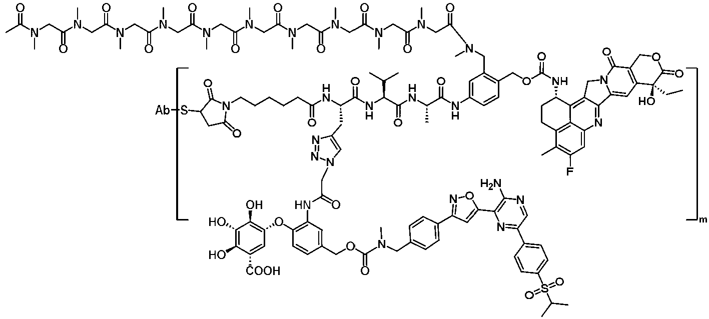 | Formula C66 |
| 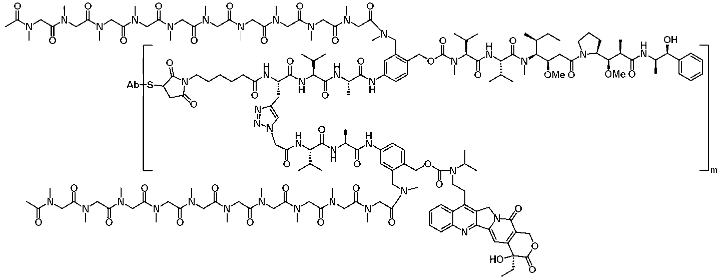 | Formula C67 |
| 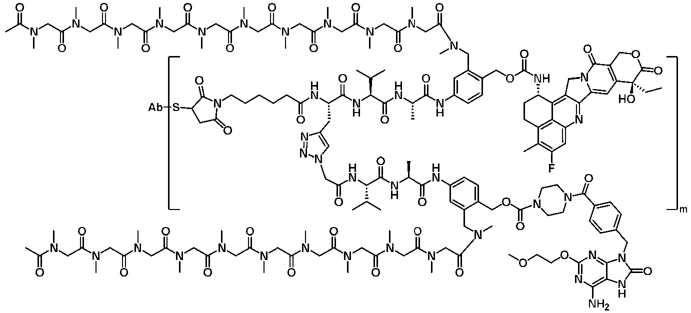 | Formula C68 |
| 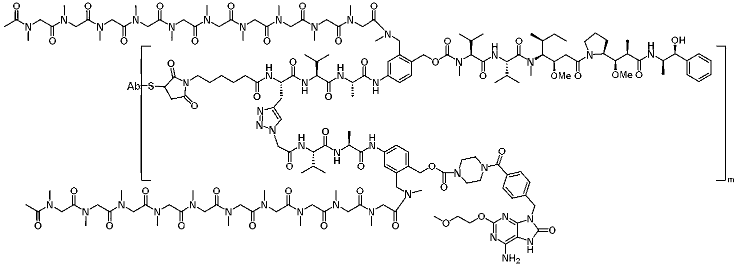 | Formula C69 |
| 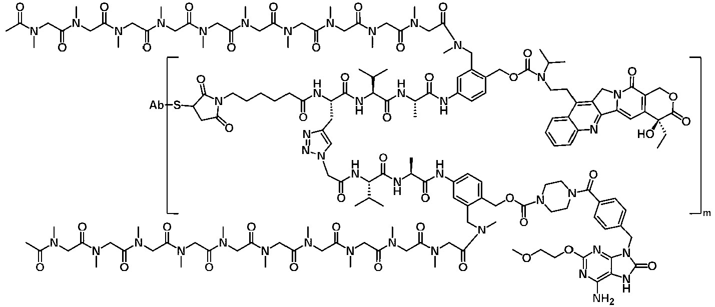 | Formula C70 |
| 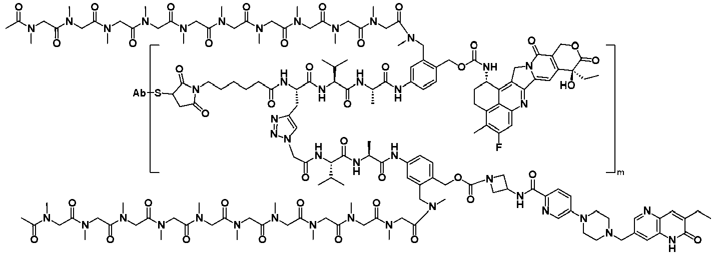 | Formula C71 |
| 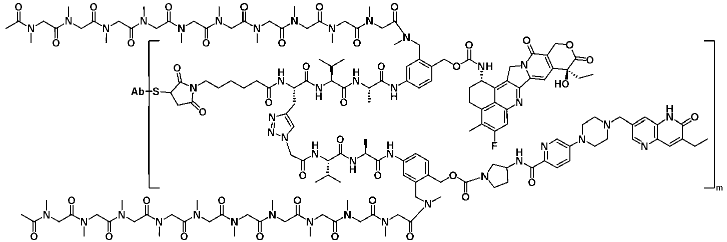 | Formula C72 |
| 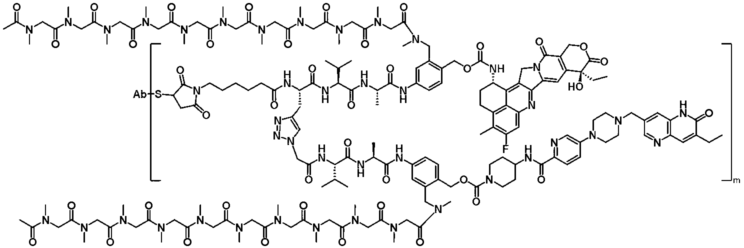 | Formula C73 |
| 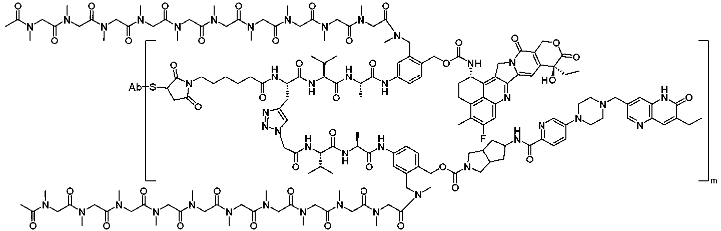 | Formula C74 |
| 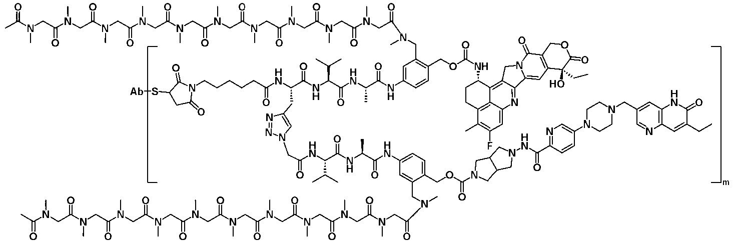 | Formula C75 |
| 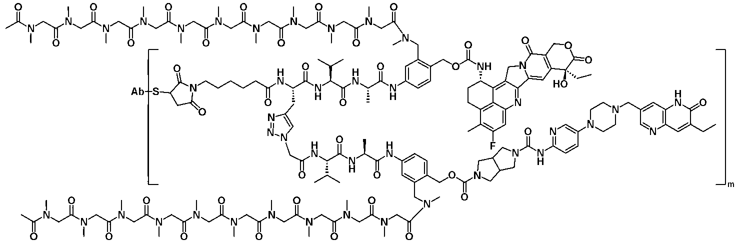 | Formula C76 |
| 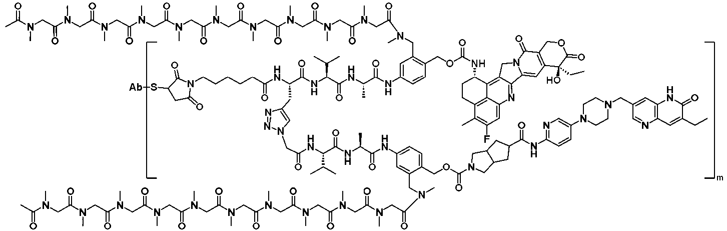 | Formula C77 |
| 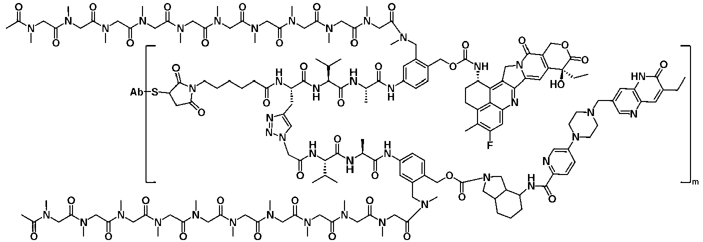 | Formula C78 |
| 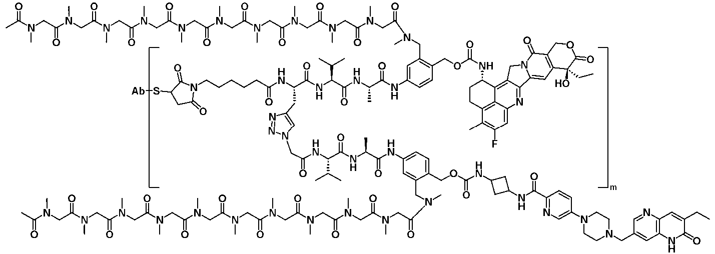 | Formula C79 |
| 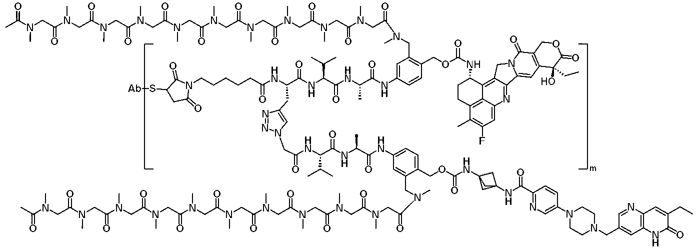 | Formula C80 |
| 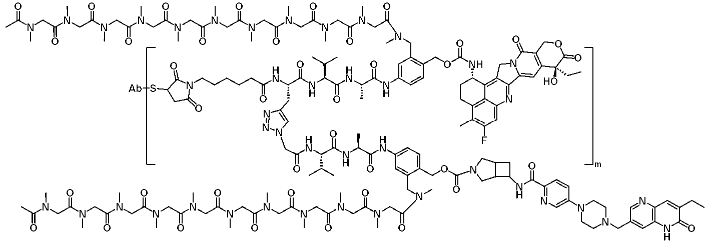 | Formula C81 |
| 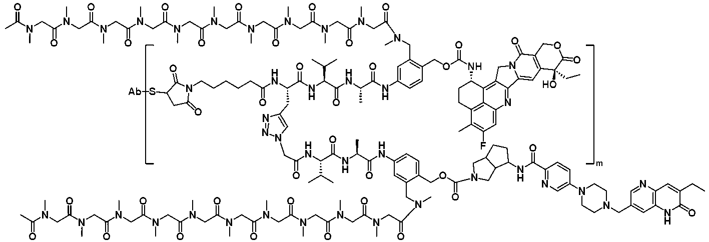 | Formula C82 |
| 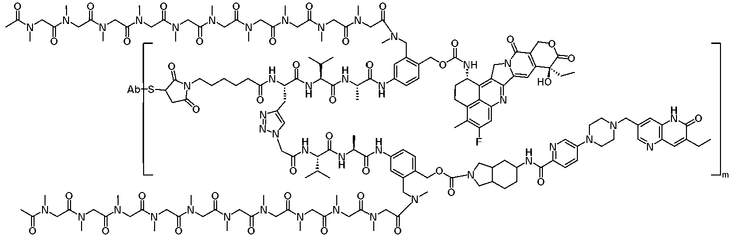 | Formula C83 |
| 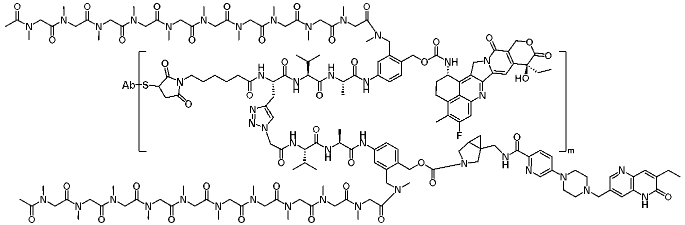 | Formula C84 |
| 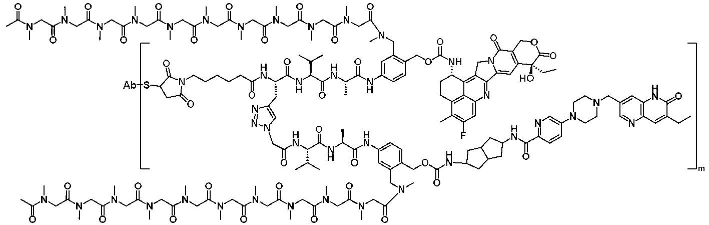 | Formula C85 |
| 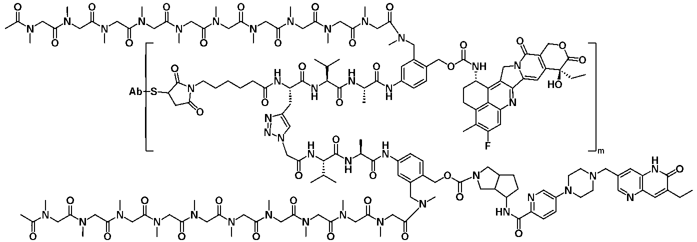 | Formula C86 |
| 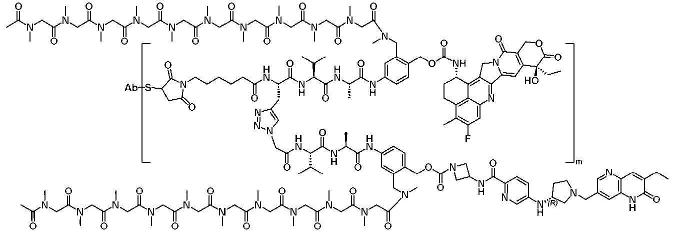 | Formula C87 |
| 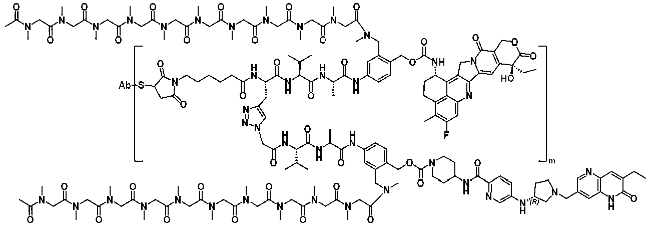 | Formula C88 |
| 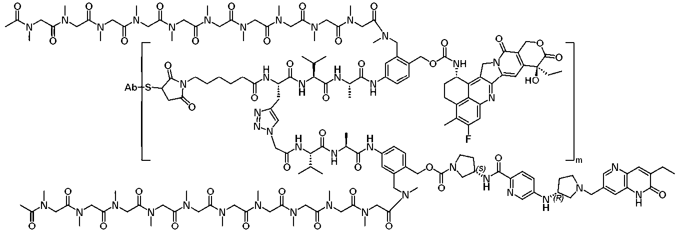 | Formula C89 |
| 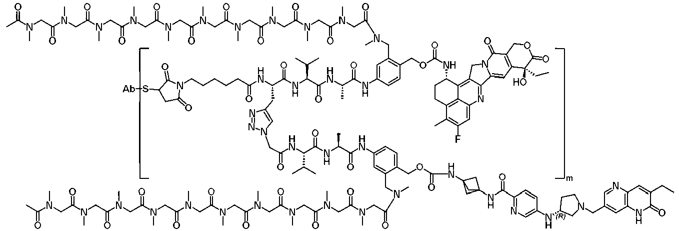 | Formula C90 |
| 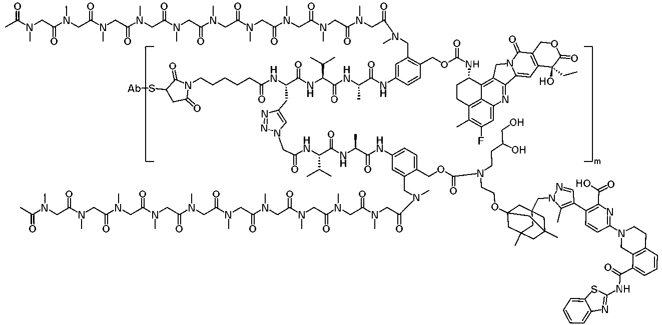 | Formula C91 |
| 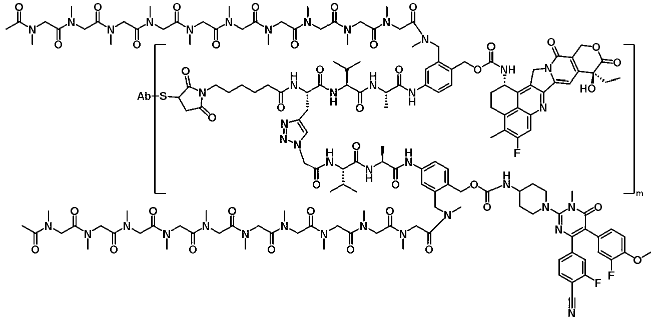 | Formula C92 |
| 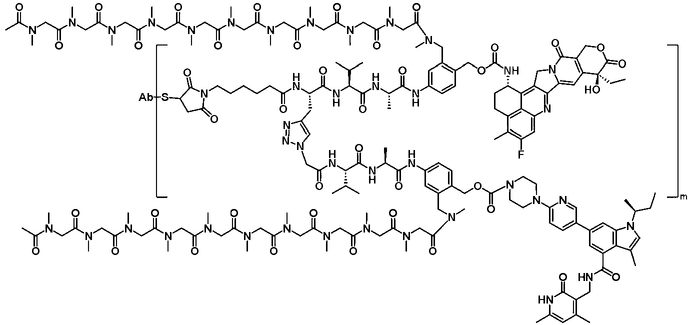 | Formula C93 |
| 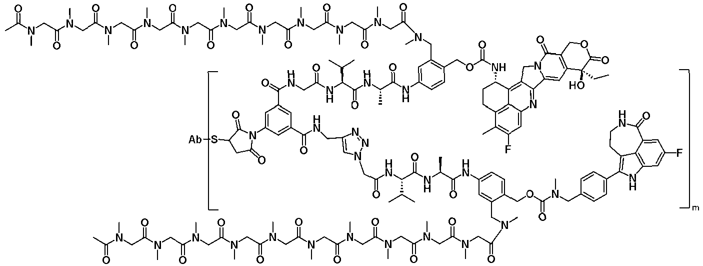 | Formula C94 |
| 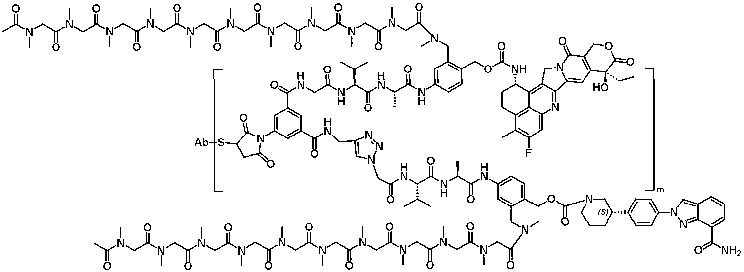 | Formula C95 |
| 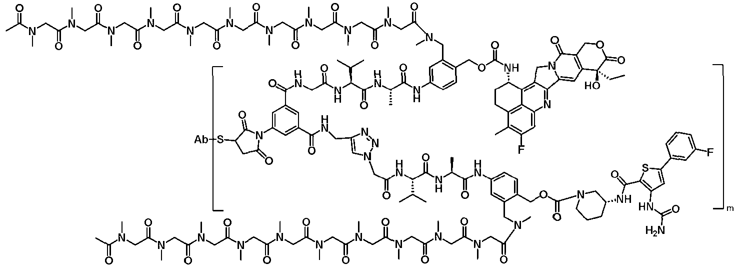 | Formula C96 |
| 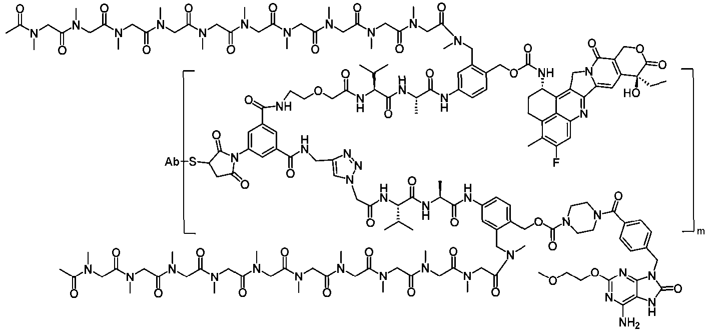 | Formula C97 |
| 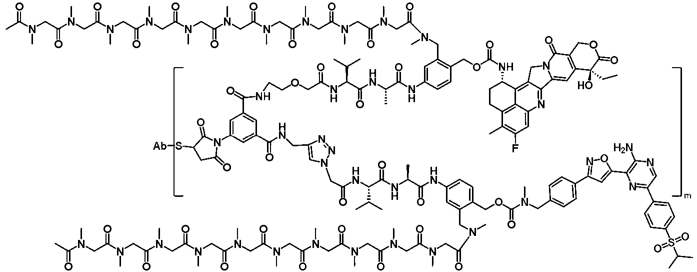 | Formula C98 |
| 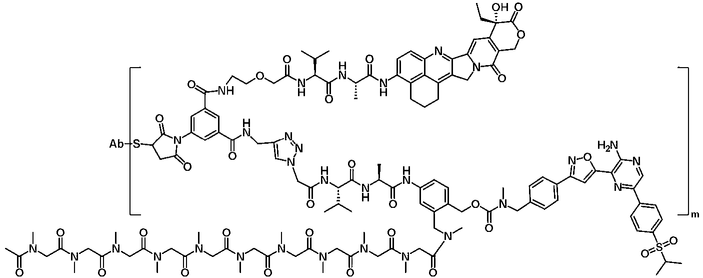 | Formula C99 |
| 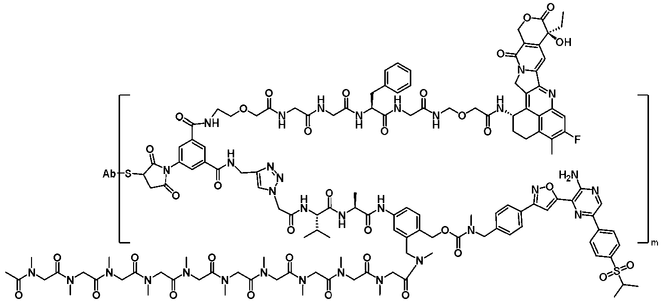 | Formula C100 |
| 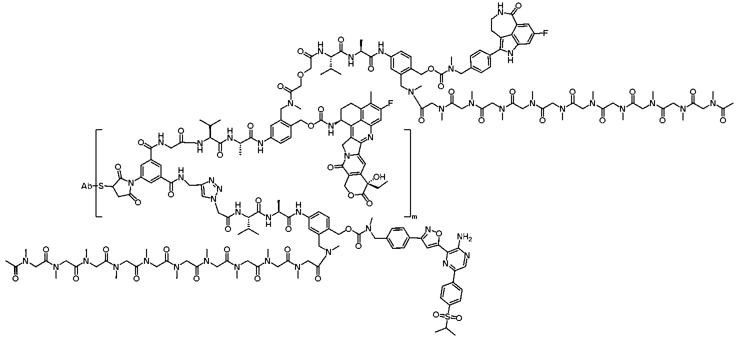 | Formula C101 |
| 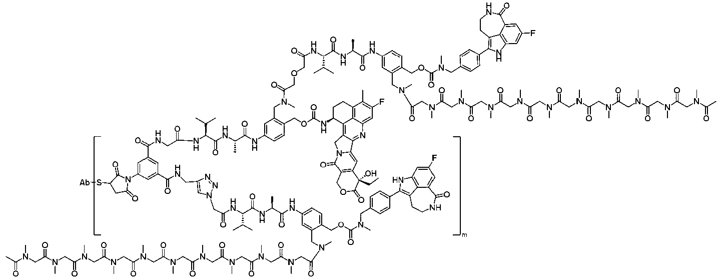 | Formula C102 |
| 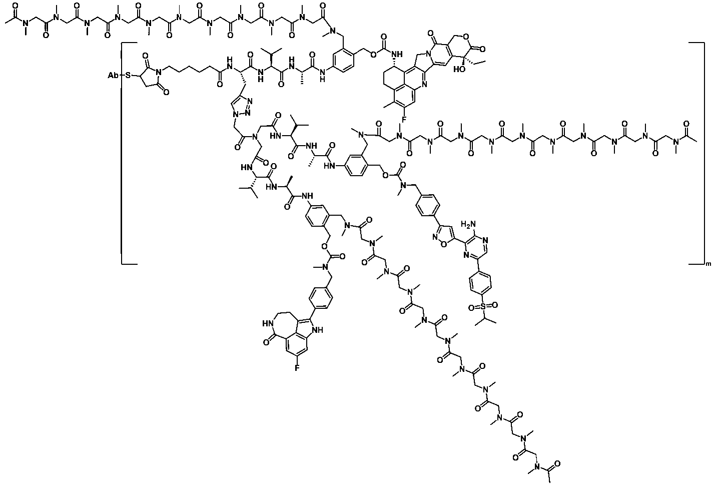 | Formula C103 |
| 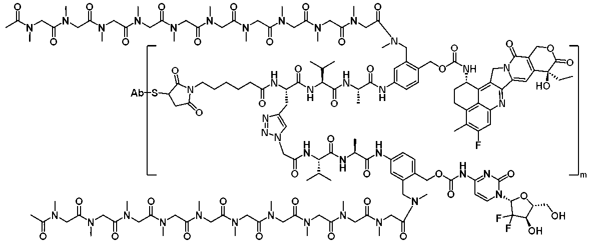 | Formula C104 |
| 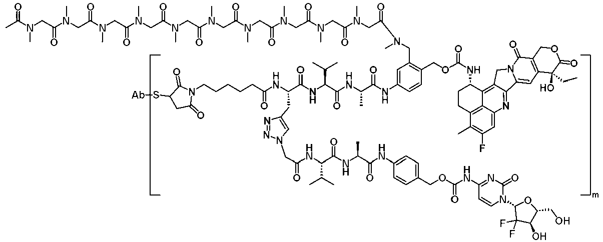 | Formula C105 |
| 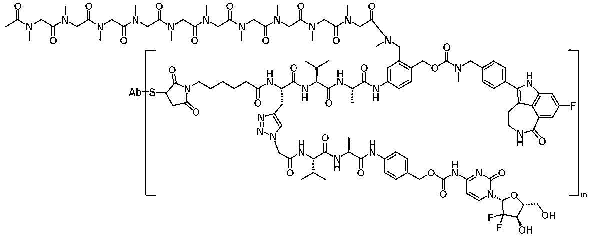 | Formula C106 |
| 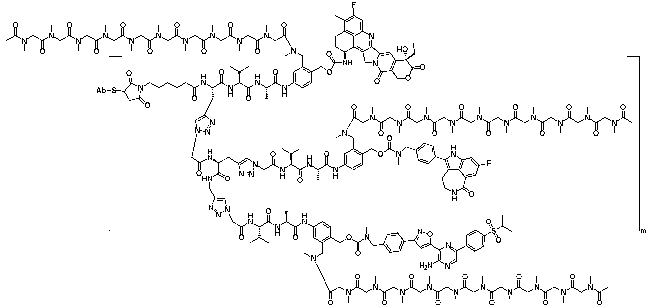 | Formula C107 |
| 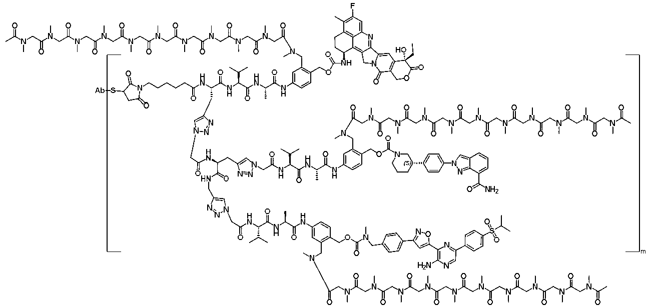 | Formula C108 |
| 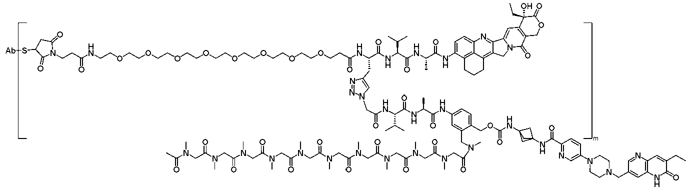 | Formula C109 |
| 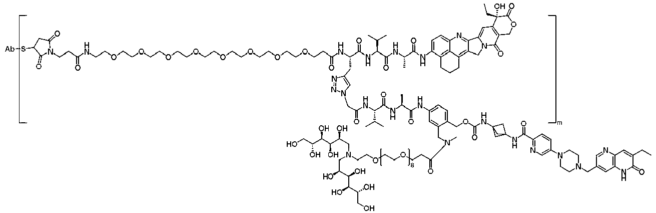 | Formula C110 |
| 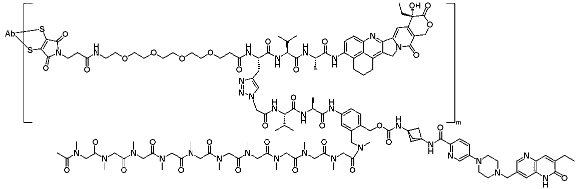 | Formula C111 |
| 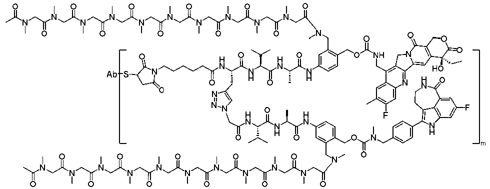 | Formula C112 |
| 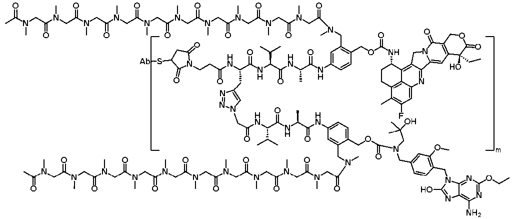 | Formula C113 |
| 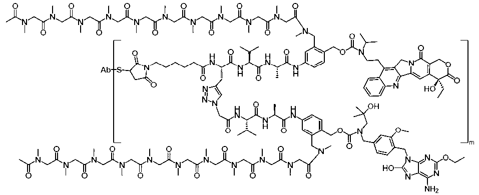 | Formula C114 |
| 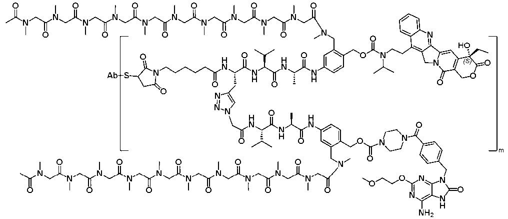 | Formula C115 |
| 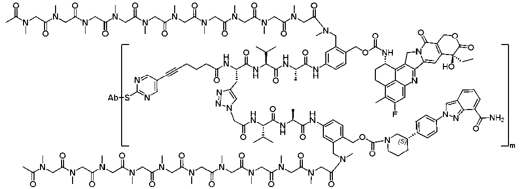 | Formula C116 |
| 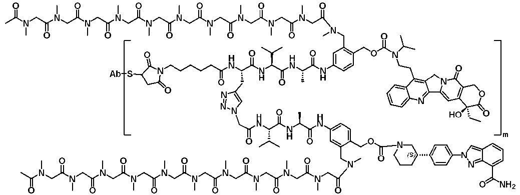 | Formula C117 |
| 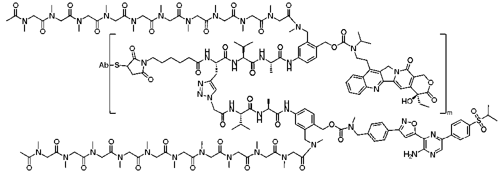 | Formula C118 |
| 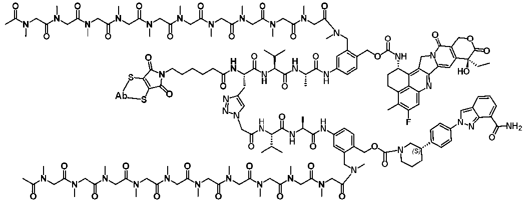 | Formula C119 |
| 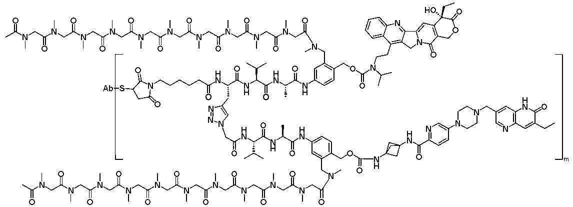 | Formula C120 |
| 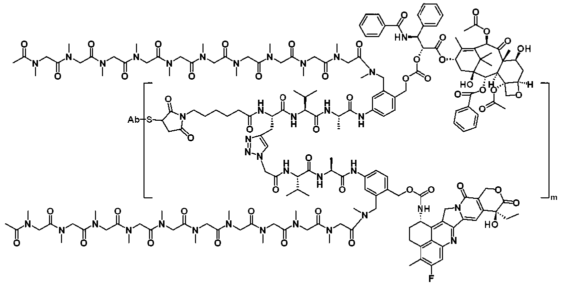 | Formula C121 |
| 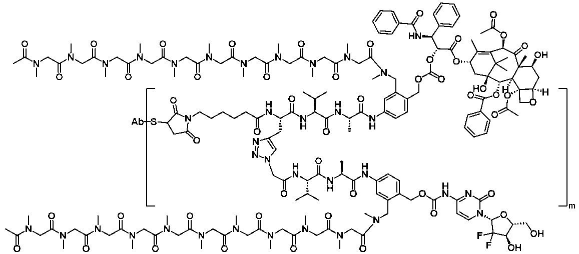 | Formula C122 |
| 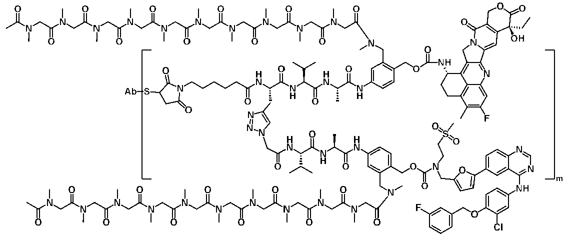 | Formula C123 |
| 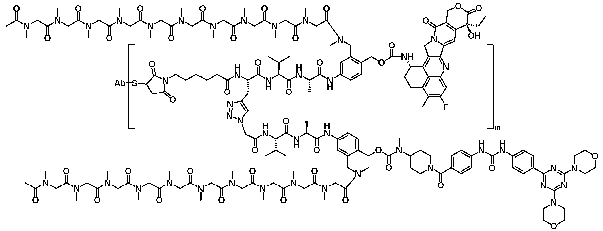 | Formula C125 |
| 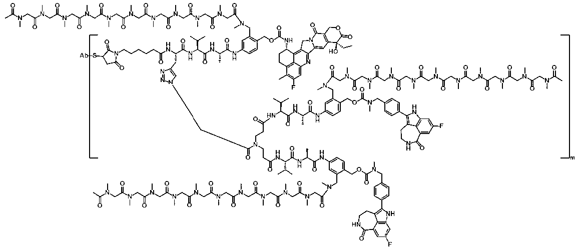 | Formula C126 |
| 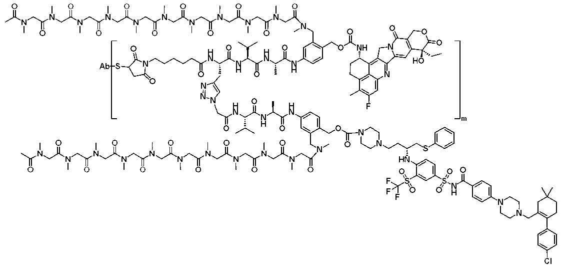 | Formula C127 |
| 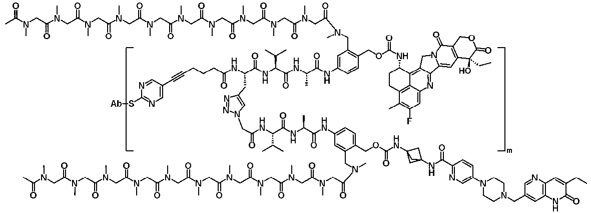 | Formula C128 |
| 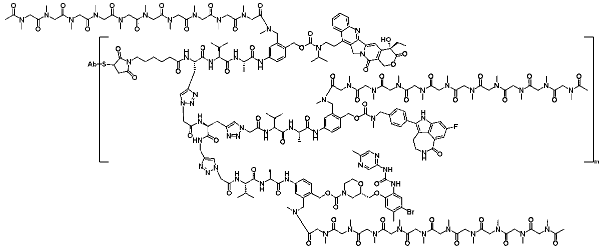 | Formula C129 |
| 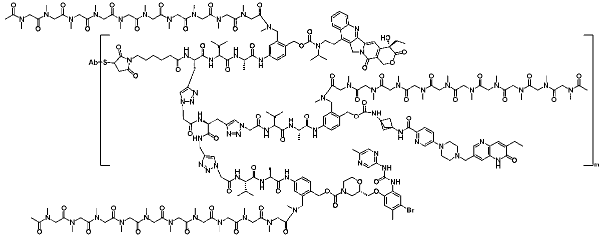 | Formula C130 |
| 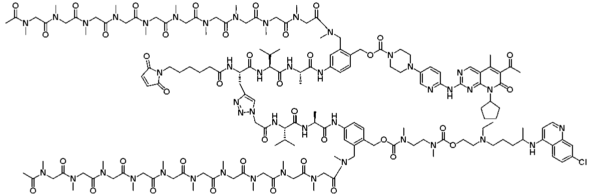 | Formula C131 |
| 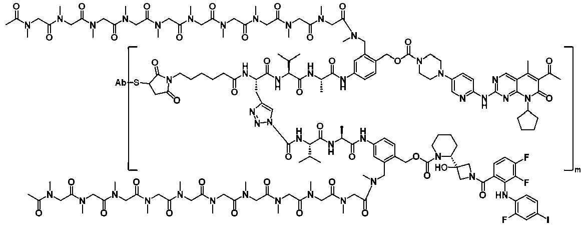 | Formula C132 |
| 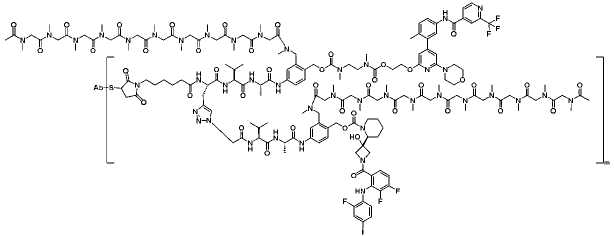 | Formula C133 |
| 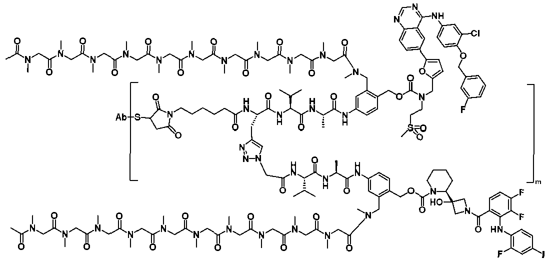 | Formula C134 |
| 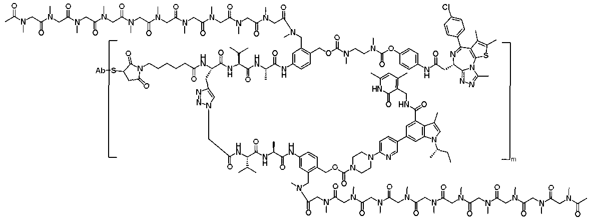 | Formula C135 |
| 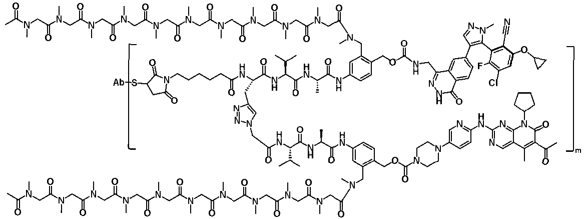 | Formula C136 |
| 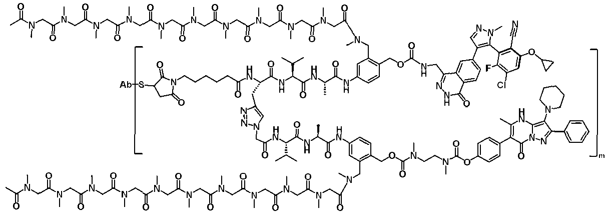 | Formula C137 |
| 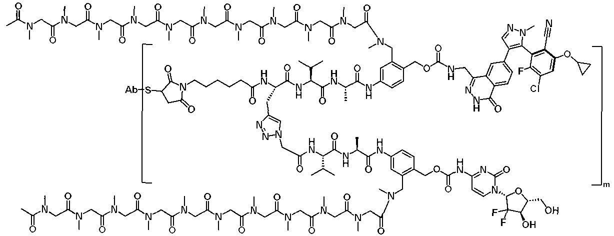 | Formula C138 |
| 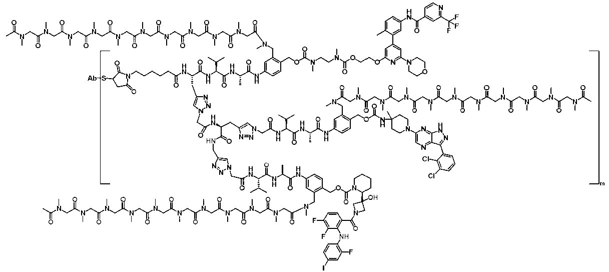 | Formula C139 |
| 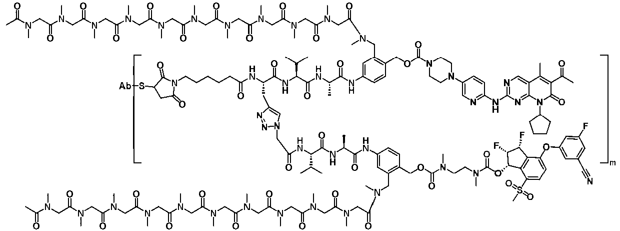 | Formula C140 |
| 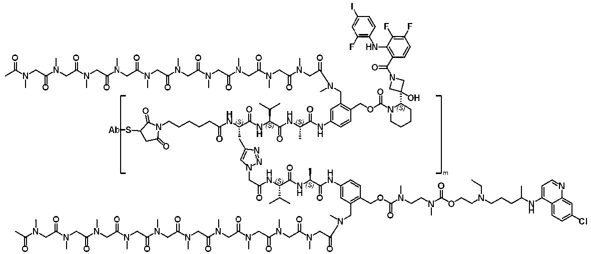 | Formula C141 |
| 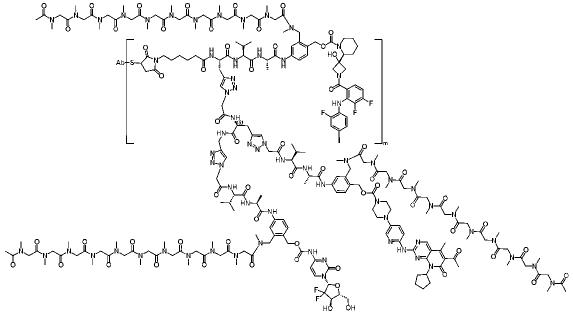 | Formula C142 |
| 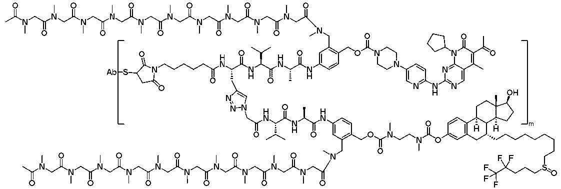 | Formula C143 |
| 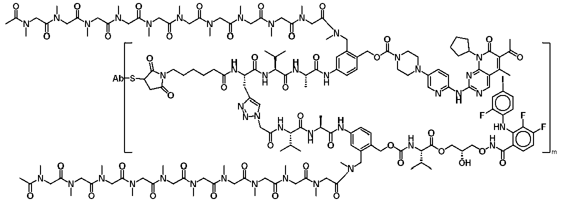 | Formula C144 |
| 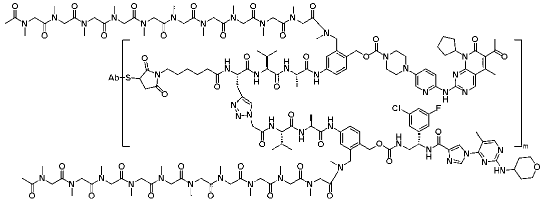 | Formula C145 |
| 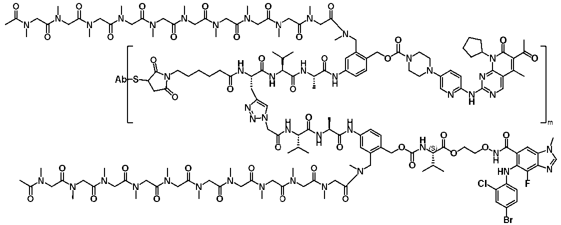 | Formula C146 |
| 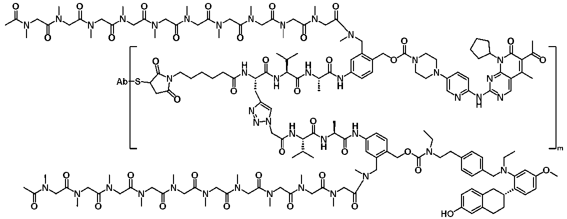 | Formula C147 |
| 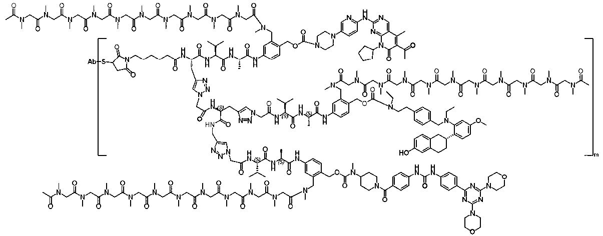 | Formula C148 |
| 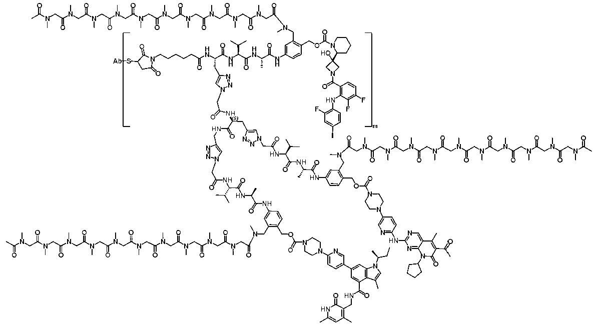 | Formula C149 |
| 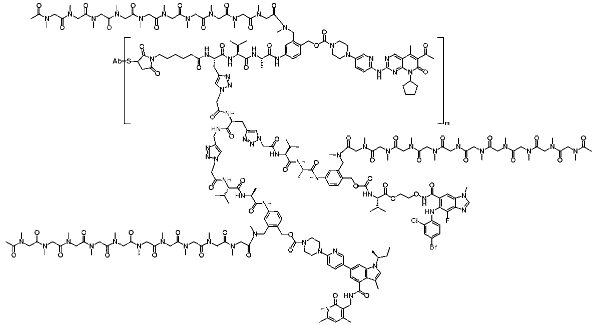 | Formula C150 |
| 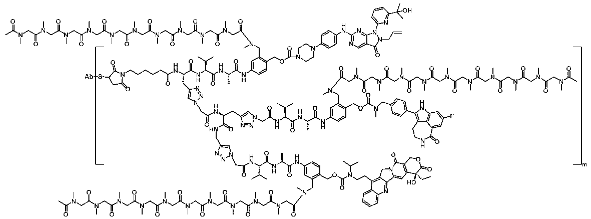 | Formula C151 |
| 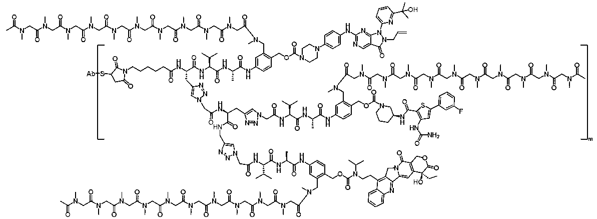 | Formula C152 |
| 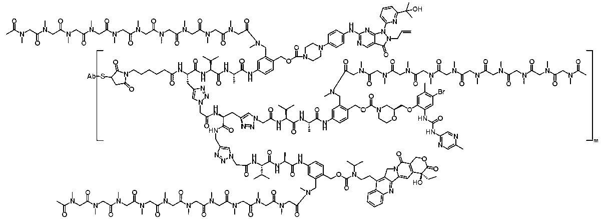 | Formula C153 |
| 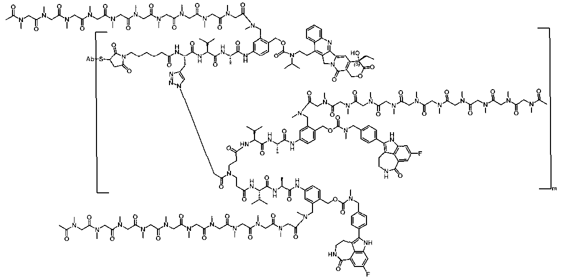 | Formula C154 |
| 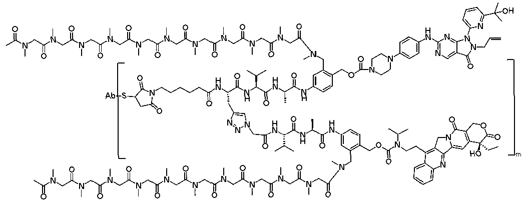 | Formula C155 |
| 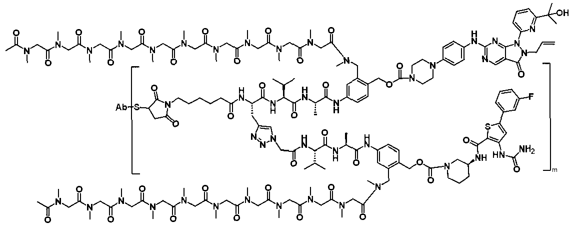 | Formula C156 |
| 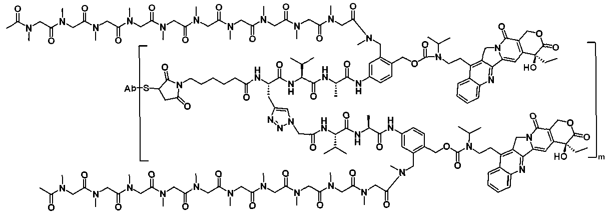 | Formula C157 |
| 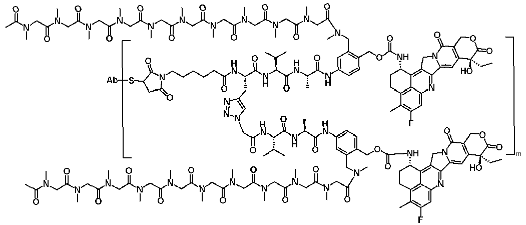 | Formula C158 |
| 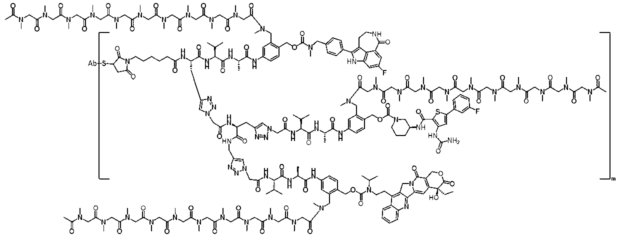 | Formula C159 |
| 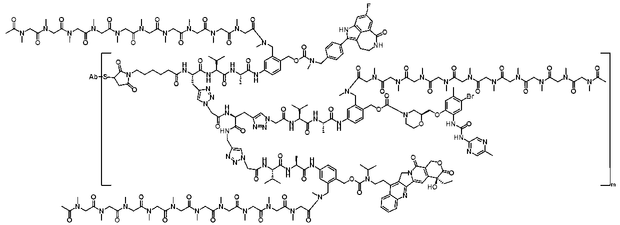 | Formula C160 |
| 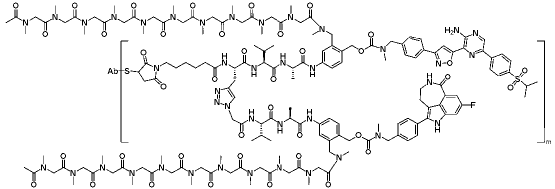 | Formula C161 |
| 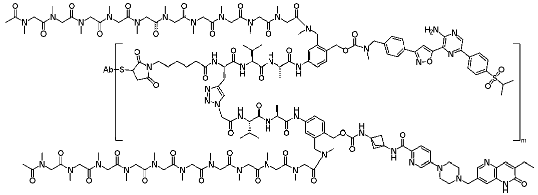 | Formula C162 |
| 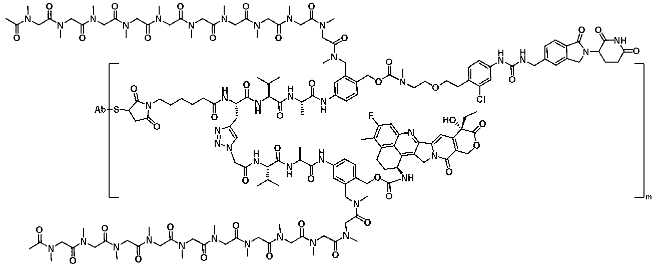 | Formula C163 |
| 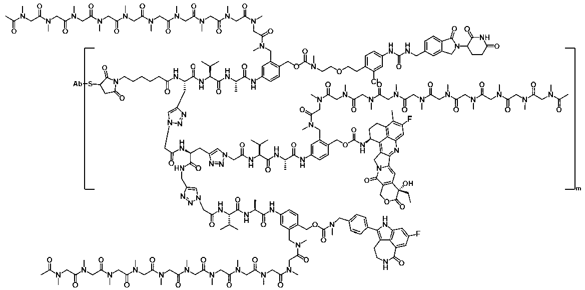 | Formula C164 |
| 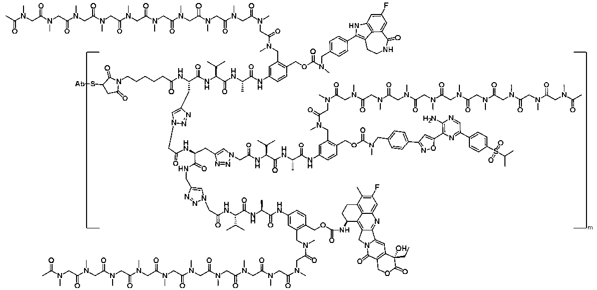 | Formula C165 |
| 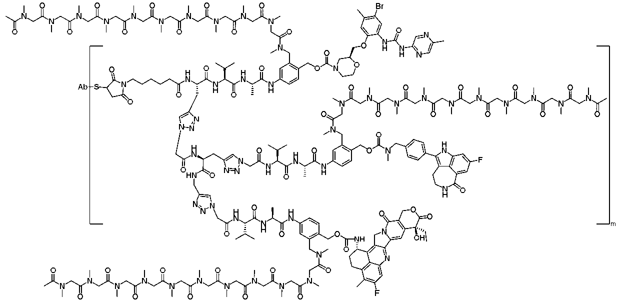 | Formula C166 |
| 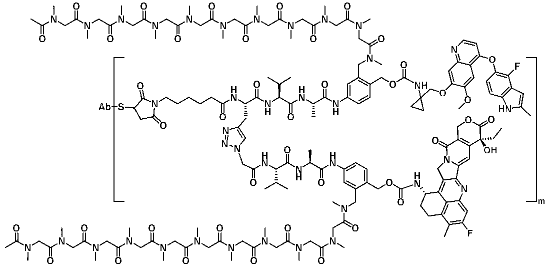 | Formula C167 |
| 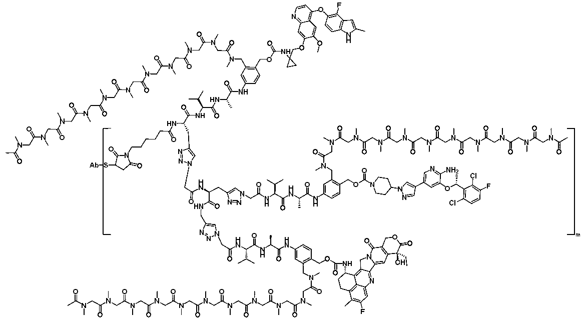 | Formula C168 |
| 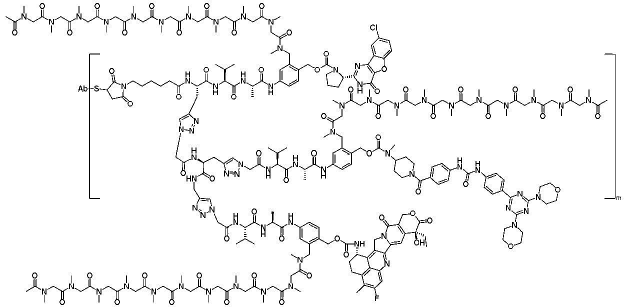 | Formula C169 |
| 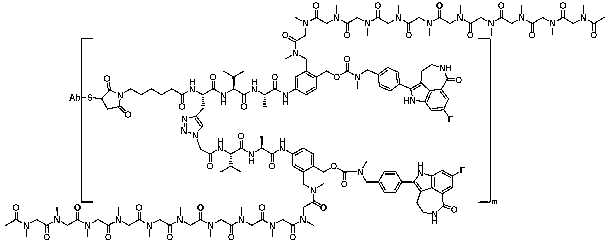 | Formula C170 |
| 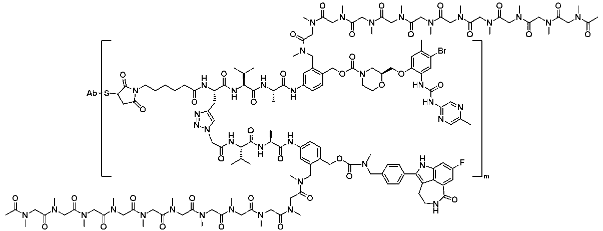 | Formula C171 |
| 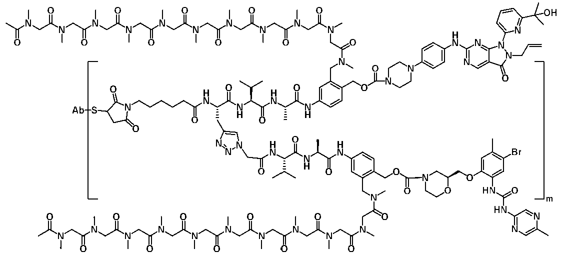 | Formula C172 |
| 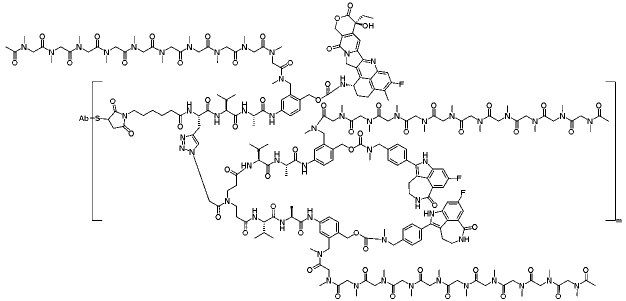 | Formula C173 |
| 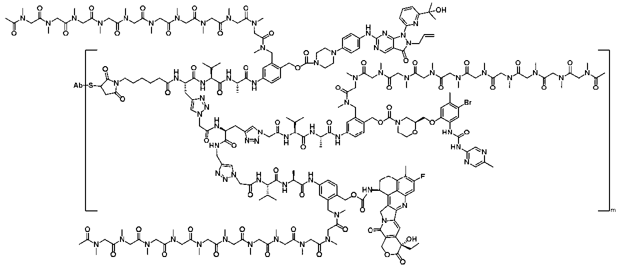 | Formula C174 |
| 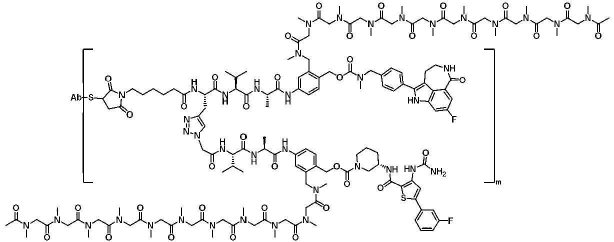 | Formula C175 |
| 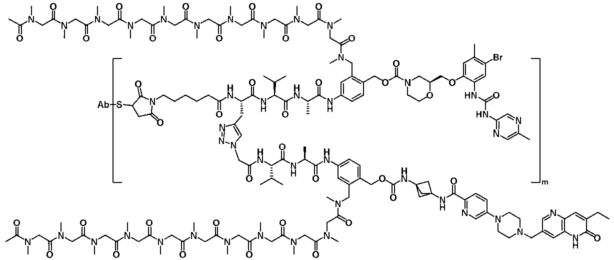 | Formula C176 |
| 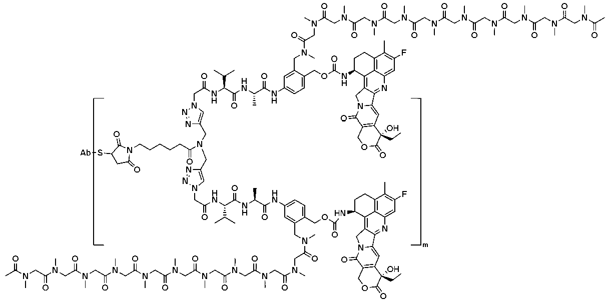 | Formula C177 |
| 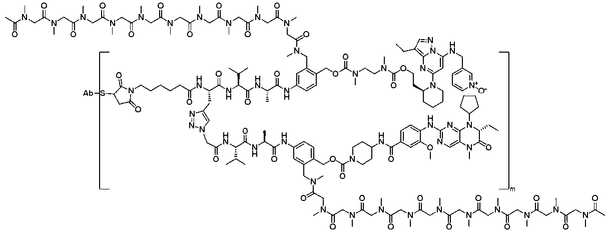 | Formula C178 |
| 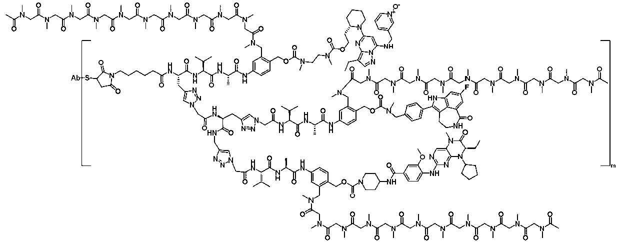 | Formula C179 |
| 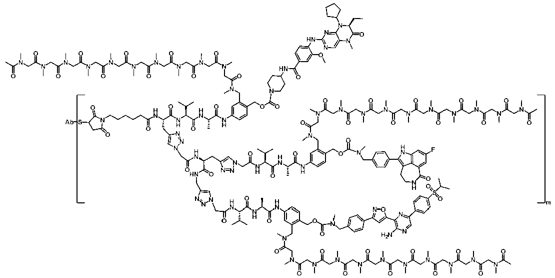 | Formula C180 |
| 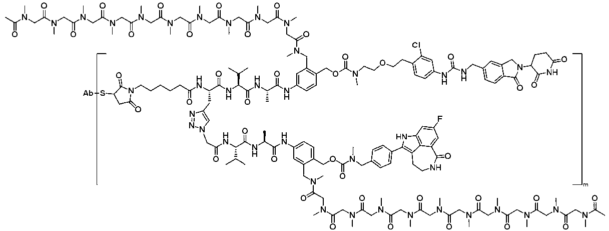 | Formula C181 |
| 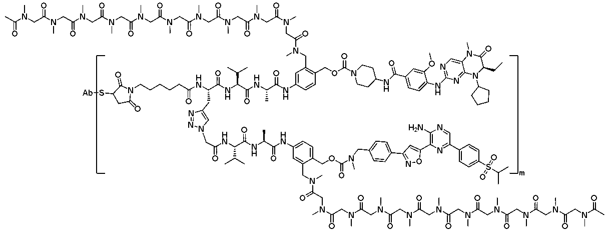 | Formula C182 |
| 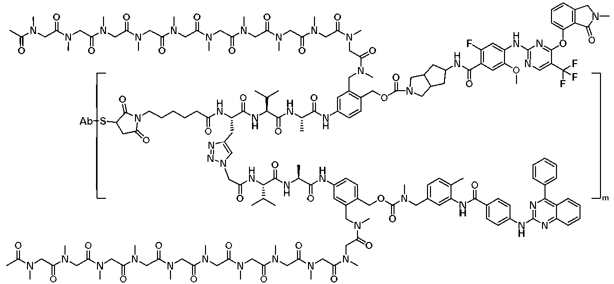 | Formula C183 |
| 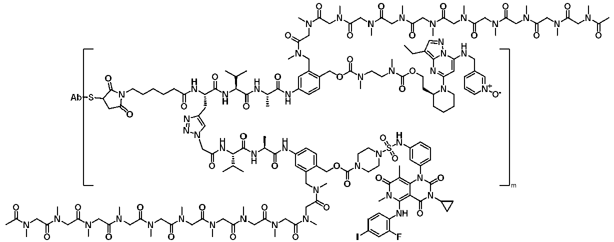 | Formula C184 |
| 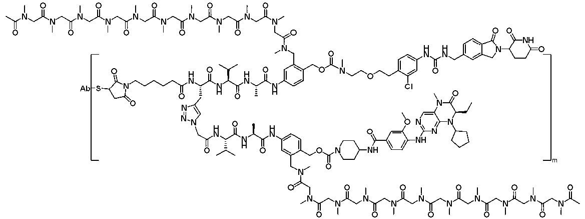 | Formula C185 |
| 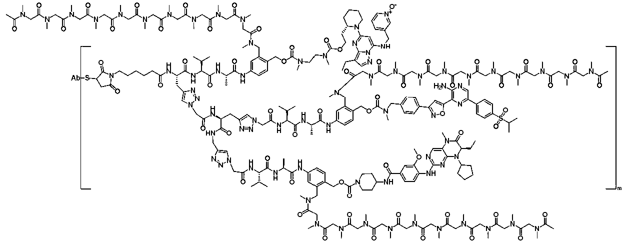 | Formula C186 |
| 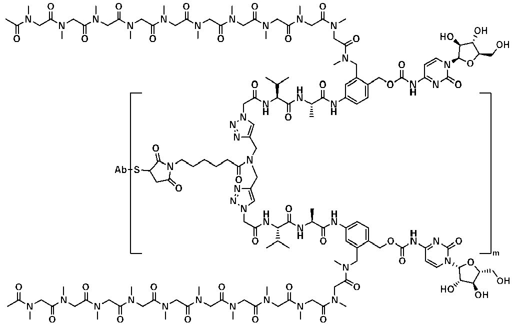 | Formula C187 |
| 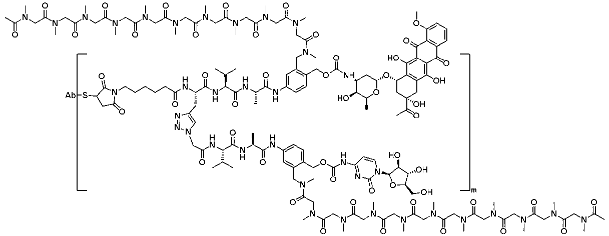 | Formula C188 |
| 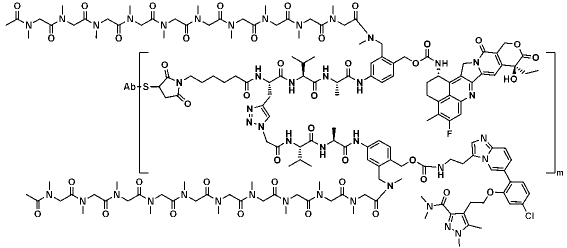 | Formula C189 |
| 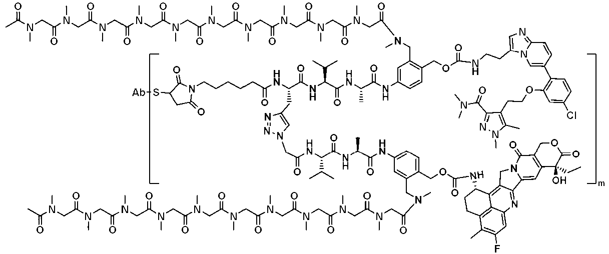 | Formula C190 |

In the present invention, in formulas III, IIIa, IIIb, C1 to C188, m may range from 1 to 7.5, 1 to 7, 1 to 6.5, 1 to 6, 1 to 5.5, 1 to 5, 1 to 4.5, 1 to 4, 1 to 3.5, 1 to 2.5, 1 to 2, 1 to 1.5, 1.5 to 9, 1.5 to 8.5, 1.5 to 8, 1.5 to 7.5, 1.5 to 5.5, 1.5 to 5, 1.5 to 4.5, 1.5 to 4, 1.5 to 3.5, 1.5 to 3, 1.5 to 2.5, 1.5 to 2, 2 to 7.5, 2 to 7, 2 to 6.5, 2 to 6, 2 to 5.5, 2 to 5, 2 to 4.5, 2 to 4, 2 to 3.5, 2 to 3, 2 to 2.5, 2.5 to 8, 2.5 to 7.5, 2.5 to 7, 2.5 to 6.5, 2.5 to 6, 2.5 to 5.5, 2.5 to 5, 2.5 to 4.5, 2.5 to 3.5, 3 to 7.5, 3 to 7, 3 to 6.5, 3 to 6, 3 to 5.5, 3 to 4.5, 3 to 4, 3 to 3.5, 3.5 to 7.5, 3.5 to 7, 3.5 to 6.5, 3.5 to 6, 3.5 to 5.5, 3.5 to 5, 3.5 to 4.5, 3.5 to 4, 4 to 8, 4 to 7.5, 4 to 7, 4 to 6.5, 4 to 6, 4 to 5.5, 4 to 5, or 4 to 4.5. For example, m may be about 1, about 1.5, about 2, about 2.5, about 3, about 3.5, about 4, about 4.5, about 5, about 5.5, about 6, about 6.5, about 7, about 7.5, or about 8.

In the present invention, in formulas III, IIIa, IIIb, C1 to C190, Ab is a monoclonal antibody or an antigen-binding fragment thereof. In preferred embodiments, the monoclonal antibody is an antibody against an antigen selected from: HER2, HER3, TROP2, B7-H3, B7-H4, GPC20, GPRC5D, CDH6, EGFR, EGFRvIII, AXL, Nectin-4, Tissue factor, TIM-1, PSMA, PTK7, EpCAM, MUC1, STEAP1, GPNMB, FGF2, FOLR1, c-MET, GFR, AGS-16, Guanylyl cyclase C, Mesothelin, SLC44A4, EphA2, AGS-5, GPC-3, c-KIT, ROR1, ROR2, PD-L1, CD27L, 5T4, MerTK, Mucin 16, NaPi2b, STEAP, SLITRK6, ETBR, BCMA, CEACAM5, ICAM1, SC-16, SLC39A6, Delta-like protein3, Claudin 18.2, Claudin 3, Claudin 6, Claudin 9, CD19, CD20, CD22, CD30, CD33, CD37, CD45, CD56, CD66e, CD70, CD73, CD74, CD79b, CD123, CD138, CD147, CD166, CD223, MUC16, MSLN, ENPP3, SLTRK6, FGFR, LIV-1, Lewis Y, av-integrin, ITGB6 (integrin beta-6), ASCT2, C4.4a, CA-IX, CD324, CD352, CD44v6, CD48a, CLL-1, Cripto, CS1, DPEP3, Ephrin-A2, Ephrin-A4, ETBR, FGFR2, FGFR3, FLT3, GD3, Globo H, GPC3, LAMP-1, LRRC15, Ly6E, TM4SF1, MFI2, NOTCH3, p-cadherin, PRLR, CLDN1, CD228, CUB domain-containing protein 1 (CDCP1), CXCR4, LGR4, FZD7, CTR, GPR56, CCR7, DDR1, and RNF43.

In preferred embodiments, the monoclonal antibody is an anti-HER2 antibody or an anti-TROP2 antibody. In some embodiments, the anti-HER2 antibody is trastuzumab, pertuzumab, or margetuximab. In some embodiments, the anti-TROP2 antibody is datopotamab or sacituzumab.

In the present invention, the embodiments or preferred embodiments of each aforementioned component (such as Q', C₁, B₁, B₂, T₁, T₂, T₃, P₁, P₂, P₃, D₁, D₂, D₃, hydrophilic group, m, and Ab) may be individually applicable to formula III, IIIa, or IIIb, or applicable in various combinations to formula III, IIIa, or IIIb. When the combination is applicable, the embodiments or preferred embodiments of each component may be combined in whole or in part.

Another aspect of the present invention provides a compound, or a tautomer, a mesomer, a racemate, an enantiomer, or a diastereomer thereof, or a deuterated compound form thereof, or a mixture form thereof, or a pharmaceutically acceptable salt, prodrug, or solvate thereof, wherein the compound is an antibody-drug conjugate, comprising an antibody molecule and at least two compounds conjugated to the antibody molecule, the at least two compounds conjugated to the antibody molecule are independently selected from the linker-drug fragments provided by the present invention, or tautomers, mesomers, racemates, enantiomers, diastereomers, or deuterated forms thereof, or mixture forms thereof, or pharmaceutically acceptable salts, prodrugs, or solvates thereof.

In preferred embodiments, the ratio of different drugs is any ratio from 1:1 to 1:24.

In preferred embodiments, the at least two compounds conjugated to the antibody molecule are conjugated to different conjugation sites on the antibody molecule; preferably, the different conjugation sites are selected from natural or modified sulfhydryl groups, amino groups, and non-natural amino acids of the antibody.

### Pharmaceutical Compositions, Uses, and Methods

One aspect of the present invention further provides a pharmaceutical composition comprising any compound of the present invention, or a tautomer, a mesomer, a racemate, an enantiomer, or a diastereomer thereof, or a mixture form thereof, or a pharmaceutically acceptable salt, prodrug, or solvate thereof, and a pharmaceutically acceptable carrier.

The pharmaceutically acceptable carrier is selected from the group consisting of fillers (diluents), binders, wetting agents, disintegrants, and excipients, among others. Depending on the route of administration, the composition may contain from 0.1 to 99% by weight of the compound of the present invention.

The pharmaceutical composition may be in a form suitable for oral administration, such as tablets, troches, lozenges, aqueous or oily suspensions, dispersible powders or granules, emulsions, hard or soft capsules, or syrups. Oral compositions may be prepared according to any known method in the art for the manufacture of pharmaceutical compositions, and such compositions may contain binders, fillers, lubricants, disintegrants, or pharmaceutically acceptable wetting agents, etc. The compositions may further contain one or more ingredients selected from the group consisting of sweeteners, flavoring agents, coloring agents, and preservatives.

The pharmaceutical composition may be in the form of a sterile injectable aqueous solution. Acceptable vehicles or solvents that may be employed include water, Ringer's solution, and isotonic sodium chloride solution. The sterile injectable preparation may also be a sterile injectable water-in-oil microemulsion in which the active ingredient is dissolved in the oil phase. For example, the active ingredient may be dissolved in a mixture of soybean oil and lecithin. The oil solution may then be processed by adding it to a mixture of water and glycerol to form a microemulsion. The injection or microemulsion may be administered by a large-volume local injection into the patient's bloodstream. Alternatively, the solution and microemulsion may be administered in a manner that maintains a constant circulating concentration of the compound of the invention. To maintain such constant concentration, a continuous intravenous delivery device may be used. For example, said device may be an intravenous infusion pump.

The pharmaceutical composition may be in the form of a sterile injectable aqueous or oily suspension for intramuscular and subcutaneous administration. This suspension may be formulated according to known techniques using suitable dispersing or wetting agents and suspending agents as described herein. The sterile injectable preparation may also be a sterile injectable solution or suspension prepared in a non-toxic parenterally acceptable diluent or solvent. Alternatively, a sterile fixed oil may conveniently be employed as a solvent or suspending medium.

The compound of the present invention may be administered in the form of a suppository for rectal administration. These compositions can be prepared by mixing the drug with a suitable non-irritating excipient that is solid at ordinary temperatures but liquid in the rectum and will therefore melt in the rectum to release the drug. Such materials include cocoa butter, glycerinated gelatin, hydrogenated vegetable oils, mixtures of polyethylene glycols of various molecular weights, and fatty acid esters of polyethylene glycol.

As is well known to those skilled in the art, the dosage of a drug depends on a variety of factors, including but not limited to: the activity of the specific compound used, the age of the patient, the patient's body weight, the patient's health status, the patient's behavior, the patient's diet, the time of administration, the route of administration, the rate of excretion, drug combinations, etc.; furthermore, the optimal treatment regimen, such as the mode of treatment, the compound of the present invention, a tautomer, a mesomer, a racemate, an enantiomer, or a diastereomer thereof, or a mixture form thereof, or a pharmaceutically acceptable salt thereof, and/or the daily dose of the compound of the present invention, a tautomer, a mesomer, a racemate, an enantiomer, or a diastereomer thereof, or a mixture form thereof, or a pharmaceutically acceptable salt thereof, or the type of pharmaceutically acceptable salt, can be validated according to conventional treatment protocols.

The pharmaceutical composition of the present invention may contain a safe and effective amount of the antibody-drug conjugate of the present invention together with a pharmaceutically acceptable carrier. Such carriers may include, but are not limited to, saline, buffers, glucose, water, glycerol, ethanol, and combinations thereof. The effective amount of the antibody-drug conjugate described herein may vary depending on the mode of administration, the severity of the disease to be treated, and other factors. The selection of an effective amount can be determined by a person of ordinary skill in the art based on various factors (e.g., through clinical trials). Such factors may include, but are not limited to, the pharmacokinetic parameters of the antibody-drug conjugate such as bioavailability, metabolism, half-life, etc.; the severity of the disease to be treated in the patient, the patient's body weight, the patient's immune status, the route of administration, etc. Generally, a satisfactory effect may be obtained when the antibody-drug conjugate of the present invention is administered daily at an appropriate dosage. For example, depending on the urgency of the treatment condition, divided doses may be administered several times a day, or the dose may be proportionally reduced.

The compounds of the present invention may be administered alone or in combination with other pharmaceutically acceptable therapeutic agents. When using the pharmaceutical composition, a safe and effective amount of the compound of the present invention may be applied to a mammal (e.g., a human) in need of treatment, wherein the dosage during administration may be a pharmaceutically recognized effective dose, and the specific dosage may also consider factors such as the route of administration and the patient's health status.

A further aspect of the present invention provides the use of a compound of the present invention, or a tautomer, a mesomer, a racemate, an enantiomer, or a diastereomer thereof, or a deuterated compound form thereof, or a mixture form thereof, or a pharmaceutically acceptable salt, prodrug, or solvate thereof, or the pharmaceutical composition of the present invention, in the manufacture of a medicament for treating and/or preventing a tumor.

A further aspect of the present invention provides a compound of the present invention, or a tautomer, a mesomer, a racemate, an enantiomer, or a diastereomer thereof, or a deuterated compound form thereof, or a mixture form thereof, or a pharmaceutically acceptable salt, prodrug, or solvate thereof, or the pharmaceutical composition of the present invention, for use in treating and/or preventing a tumor.

A further aspect of the present invention provides a method for treating and/or preventing a tumor in a subject, the method comprising administering to the subject a therapeutically effective amount of a compound of the present invention, or a tautomer, a mesomer, a racemate, an enantiomer, or a diastereomer thereof, or a deuterated compound form thereof, or a mixture form thereof, or a pharmaceutically acceptable salt, prodrug, or solvate thereof, or the pharmaceutical composition of the present invention.

In any of the above aspects, in some embodiments, the tumor is a solid tumor or a hematological malignancy. In any of the above aspects, in preferred embodiments, the tumor is a homologous recombination repair deficiency (HRD) related tumor. In any of the above aspects, in more preferred embodiments, the tumor is a tumor positive for or having high expression of a target selected from: HER2, HER3, TROP2, B7-H3, B7-H4, GPC20, GPRC5D, CDH6, EGFR, EGFRvIII, AXL, Nectin-4, Tissue factor, TIM-1, PSMA, PTK7, EpCAM, MUC1, STEAP1, GPNMB, FGF2, FOLR1, c-MET, GFR, AGS-16, Guanylyl cyclase C, Mesothelin, SLC44A4, EphA2, AGS-5, GPC-3, c-KIT, ROR1, ROR2, PD-L1, CD27L, 5T4, MerTK, Mucin 16, NaPi2b, STEAP, SLITRK6, ETBR, BCMA, CEACAM5, ICAM1, SC-16, SLC39A6, Delta-like protein3, Claudin 18.2, Claudin 3, Claudin 6, Claudin 9, CD19, CD20, CD22, CD30, CD33, CD37, CD45, CD56, CD66e, CD70, CD73, CD74, CD79b, CD123, CD138, CD147, CD166, CD223, MUC16, MSLN, ENPP3, SLTRK6, FGFR, LIV-1, Lewis Y, av-integrin, ITGB6 (integrin beta-6), ASCT2, C4.4a, CA-IX, CD324, CD352, CD44v6, CD48a, CLL-1, Cripto, CS1, DPEP3, Ephrin-A2, Ephrin-A4, ETBR, FGFR2, FGFR3, FLT3, GD3, Globo H, GPC3, LAMP-1, LRRC15, Ly6E, TM4SF1, MFI2, NOTCH3, p-cadherin, PRLR, CLDN1, CD228, CUB domain-containing protein 1 (CDCP1), CXCR4, LGR4, FZD7, CTR, GPR56, CCR7, DDR1 and RNF43.

In any of the above aspects, in some embodiments, the tumor is selected from breast cancer, ovarian cancer, non-Hodgkin's lymphoma, Hodgkin's lymphoma, acute lymphocytic leukemia, anaplastic large cell lymphoma, multiple myeloma, prostate cancer, non-small cell lung cancer, small cell lung cancer, malignant melanoma, squamous cell carcinoma, glioblastoma, renal cell carcinoma, gastrointestinal tumors, pancreatic cancer, prostate cancer, colorectal, gastric cancer, bladder cancer, gastrointestinal stromal tumor, cervical cancer, esophageal cancer, peritoneal cancer, liver cancer, colon cancer, glioma, mesothelioma, rectal cancer, colorectal cancer, uterine cancer, salivary gland cancer, kidney cancer, vulvar cancer, thyroid cancer, penile cancer, malignant lymphoma, plasmacytoma, myeloma, and sarcoma.

In some aspects, the present invention provides a method of combination administration, the combination administration comprising delivering at least two drugs selected from a first drug (D₁), a second drug (D₂), and a third drug (D₃) in a same molecule, the method comprising forming at least two of D₁, D₂, and D₃ into the ADC compound represented by formula III, IIIa, or IIIb of the present invention.

In preferred embodiments, D₁, D₂, D₃, when present, are a first anti-cancer agent, a second anti-cancer agent, and a third anti-cancer agent, respectively, the first, second, and third anticancer agents being the same or different.

In preferred embodiments, at least two of the first, second, and third anti-cancer agents have a synergistic anti-cancer effect.

In preferred embodiments, at least one of D₁, D₂, and D₃ is a cytotoxic drug, and at least another one is a tumor-targeted therapeutic drug. In preferred embodiments, at least two of D₁, D₂, and D₃ are tumor-targeted therapeutic drugs, for example, tumor-targeted therapeutic drugs that can produce a synergistic or additive effect. In preferred embodiments, at least two of D₁, D₂, and D₃ are cytotoxic drugs.

In preferred embodiments, the cytotoxic drug is selected from drug units targeting topoisomerase, nucleoside antimetabolite anti-cancer drug units, drug units targeting tubulin, and drug units targeting DNA.

In preferred embodiments, the tumor-targeted therapeutic drug is selected from drug units targeting DNA damage response (DDR) or "synthetic lethality" related pathways, drug units targeting epigenetics, drug units targeting apoptosis-related pathways, drug units targeting transcription factors, and drug units targeting immune activation pathway-related targets, comprising: inhibitors of EFGR pathway-related targets; Ras-Raf-MAPK pathway inhibitors; PI3K/AKT/mTOR pathway inhibitors; cell cycle pathway inhibitors; cGAS-STING signaling pathway agonists; estrogen receptor antagonists; androgen receptor antagonists; glucocorticoid receptor modulators; autophagy inhibitors; FAK inhibitors; Smo inhibitors; BTK inhibitors; PDE4 inhibitors; Lck inhibitors; PLK1 inhibitors; TLR7/8 modulators; N-myristoyltransferase (NMT) inhibitors.

In preferred embodiments, at least one of D₁, D₂, and D₃ is a drug targeting topoisomerase, and at least another one is a drug targeting DNA damage response (DDR) or "synthetic lethality" related pathways.

Accordingly, in some embodiments, at least one of D₁, D₂, and D₃ is a cytotoxic drug selected from drugs targeting topoisomerase, drugs targeting tubulin, and drugs targeting DNA, and at least another one is a tumor-targeted therapeutic drug, which is a drug targeting DNA damage response (DDR) or "synthetic lethality" related pathways. For example, at least one of D₁, D₂, and D₃ is a drug targeting topoisomerase, and at least another one is a drug targeting DNA damage response (DDR) or "synthetic lethality" related pathways. For example, at least one of D₁, D₂, and D₃ is a drug targeting tubulin, and at least another one is a drug targeting DNA damage response (DDR) or "synthetic lethality" related pathways. For example, at least one of D₁, D₂, and D₃ is a drug targeting DNA, and at least another one is a drug targeting DNA damage response (DDR) or "synthetic lethality" related pathways.

In some embodiments, at least one of D₁, D₂, and D₃ is a cytotoxic drug selected from drugs targeting topoisomerase, drugs targeting tubulin, and drugs targeting DNA, and at least another one is a tumor-targeted therapeutic drug, which is a drug targeting epigenetics. For example, at least one of D₁, D₂, and D₃ is a drug targeting topoisomerase, and at least another one is a drug targeting epigenetics. For example, at least one of D₁, D₂, and D₃ is a drug targeting tubulin, and at least another one is a drug targeting epigenetics. For example, at least one of D₁, D₂, and D₃ is a drug targeting DNA, and at least another one is a drug targeting epigenetics.

In some embodiments, at least one of D₁, D₂, and D₃ is a cytotoxic drug selected from drugs targeting topoisomerase, drugs targeting tubulin, and drugs targeting DNA, and at least another one is a tumor-targeted therapeutic drug, which is a drug targeting apoptosis-related pathways. For example, at least one of D₁, D₂, and D₃ is a drug targeting topoisomerase, and at least another one is a drug targeting apoptosis-related pathways. For example, at least one of D₁, D₂, and D₃ is a drug targeting tubulin, and at least another one is a drug targeting apoptosis-related pathways. For example, at least one of D₁, D₂, and D₃ is a drug targeting DNA, and at least another one is a drug targeting apoptosis-related pathways.

In some embodiments, at least one of D₁, D₂, and D₃ is a cytotoxic drug selected from drugs targeting topoisomerase, drugs targeting tubulin, and drugs targeting DNA, and at least another one is a tumor-targeted therapeutic drug, which is a drug targeting immune activation pathway-related targets. For example, at least one of D₁, D₂, and D₃ is a drug targeting topoisomerase, and at least another one is a drug targeting immune activation pathways. For example, at least one of D₁, D₂, and D₃ is a drug targeting tubulin, and at least another one is a drug targeting immune activation pathways. For example, at least one of D₁, D₂, and D₃ is a drug targeting DNA, and at least another one is a drug targeting immune activation pathways.

In some embodiments, at least two of D₁, D₂, and D₃ are drugs targeting DNA damage response (DDR) or "synthetic lethality" related pathways. In some embodiments, at least two of D₁, D₂, and D₃ are drugs targeting epigenetics. In some embodiments, at least two of D₁, D₂, and D₃ are drugs targeting apoptosis-related pathways. In some embodiments, at least two of D₁, D₂, and D₃ are drugs targeting immune activation pathways.

In some embodiments, at least two of D₁, D₂, and D₃ are drugs targeting topoisomerase. In some embodiments, at least two of D₁, D₂, and D₃ are nucleoside antimetabolite anti-cancer drugs. In some embodiments, at least two of D₁, D₂, and D₃ are drugs targeting tubulin. In some embodiments, at least two of D₁, D₂, and D₃ are drugs targeting DNA.

In some embodiments, at least one of D₁, D₂, and D₃ is a cytotoxic drug selected from drugs targeting topoisomerase, for example a TOPO1 inhibitor, and at least another one is a tumor-targeted therapeutic drug, which is a drug targeting DNA damage response (DDR) or "synthetic lethality" related pathways, selected from PARP inhibitors, ATR inhibitors, CHK1 inhibitors, ATM inhibitors, DNA-PK inhibitors, WEE1 inhibitors, POLQ inhibitors, CDK12 inhibitors, USP1 inhibitors, PKMYT1 inhibitors, Rad51 inhibitors.

In some embodiments, at least two of D₁, D₂, and D₃ are cytotoxic drugs and are independently selected from drugs targeting topoisomerase and drug units targeting tubulin, for example TOPO1 inhibitors, TOPO2 inhibitors, and tubulin inhibitors.

In some embodiments, at least two or all three of D₁, D₂, and D₃ are tumor-targeted therapeutic drugs, and are independently selected from drug units targeting DNA damage response (DDR) or "synthetic lethality" related pathways, drug units targeting epigenetics, drug units targeting apoptosis-related pathways, drug units targeting cell cycle pathways, drug units targeting transcription factors, and drug units targeting immune activation pathway-related targets, comprising: inhibitors targeting EFGR pathway-related targets; inhibitors targeting Ras-Raf-MAPK pathway-related targets; inhibitors targeting PI3K/AKT/mTOR pathway-related targets; inhibitors targeting cell cycle pathway-related targets; agonists targeting cGAS-STING signaling pathway-related targets; estrogen receptor antagonists, androgen receptor antagonists; glucocorticoid receptor modulators; autophagy inhibitors; FAK inhibitors; Smo inhibitors; BTK inhibitors; PDE4 inhibitors; Lck inhibitors; PLK1 inhibitors; TLR7/8 modulators; N-myristoyltransferase (NMT) inhibitors.

In some embodiments, one of D₁, D₂, and D₃ is a cytotoxic drug selected from drug units targeting topoisomerase and drug units targeting tubulin, for example a TOPO1 inhibitor or a tubulin inhibitor, and the other one or two are tumor-targeted therapeutic drugs, selected from drug units targeting DNA damage response (DDR) or "synthetic lethality" related pathways, drug units targeting epigenetics, drug units targeting apoptosis-related pathways, drug units targeting cell cycle pathways, drug units targeting transcription factors, and drug units targeting immune activation pathway-related targets, comprising: inhibitors targeting EFGR pathway-related targets; inhibitors targeting Ras-Raf-MAPK pathway-related targets; inhibitors targeting PI3K/AKT/mTOR pathway-related targets; inhibitors targeting cell cycle pathway-related targets; agonists targeting cGAS-STING signaling pathway-related targets; estrogen receptor antagonists, androgen receptor antagonists; glucocorticoid receptor modulators; autophagy inhibitors; FAK inhibitors; Smo inhibitors; BTK inhibitors; PDE4 inhibitors; Lck inhibitors; PLK1 inhibitors; TLR7/8 modulators; N-myristoyltransferase (NMT) inhibitors.

In preferred embodiments, the drug unit targeting topoisomerase is a topoisomerase I (TOPO1) inhibitor or topoisomerase II (TOPO2) inhibitor, comprising Exatecan (CAS 171335-80-1), DXd (CAS 1599440-33-1), 7-ethyl-10-hydroxycamptothecin (SN38, CAS 86639-52-3), Belotecan (CAS 213819-48-8), (4-NH₂)-Exatecan (AZD'0132, CAS 2495742-21-5), 7-MAD-MDCPT (CAS 765871-81-6), 7-aminomethyl-10-methyl-11-fluorocamptothecin (CAS 2378616-23-8), Voreloxin (CAS 175414-77-4), or derivatives and analogs thereof. In preferred embodiments, the drug unit targeting tubulin is Eribulin, Vinblastine, Paclitaxel, MMAE, MMAF, Maytansine, or derivatives thereof. In preferred embodiments, the drug unit targeting DNA is a DNA minor groove binder (PBD), a DNA alkylator (Duocarmycin), Trabectedin, Lurbinectedin, or derivatives thereof.

In preferred embodiments, the drug unit targeting DNA damage response (DDR) or "synthetic lethality" related pathways is a PARP inhibitor, ATR inhibitor, CHK1 inhibitor, ATM inhibitor, DNA-PK inhibitor, WEE1 inhibitor, POLQ inhibitor, CDK12 inhibitor, USP1 inhibitor, PKMYT1 inhibitor, or Rad51 inhibitor; the PARP inhibitor is preferably Rucaparib, Niraparib, Veliparib, A-966492, Talazoparib, AZD5305, Venadaparib, Mefuparib, or analogs thereof; the ATR inhibitor is preferably Berzosertib, Ceralasertib, or analogs thereof; and/or the CHK1 inhibitor is preferably Prexasertib (LY2606368), AZD7762, Rabusertib (LY2603618), MK-8776 (SCH 900776), CHIR-124, PF-477736, CCT245737 (SRA737, PNT-737), GDC-0575 (ARRY-575), or analogs thereof. In preferred embodiments, the drug unit targeting apoptosis-related pathways is a Bcl-2 family protein inhibitor. In preferred embodiments, the drug unit targeting epigenetics is an LSD1 inhibitor, EZH2 inhibitor, or PRMT5 inhibitor. In preferred embodiments, the drug unit targeting immune activation pathway-related targets is a PD-L1 inhibitor, CBL-B inhibitor, TLR7/8 agonist, or STING agonist.

In preferred embodiments, at least one of D₁, D₂, and D₃ is a topoisomerase inhibitor, and at least another one is a PARP inhibitor, an ATR inhibitor, or a CHK1 inhibitor. In preferred embodiments, at least one of D₁, D₂, and D₃ is a topoisomerase inhibitor, and at least another one is a Bcl-2 family protein inhibitor. In preferred embodiments, at least one of D₁, D₂, and D₃ is a topoisomerase inhibitor, and at least another one is an LSD1 inhibitor, an EZH2 inhibitor, or a PRMT5 inhibitor. In preferred embodiments, at least one of D₁, D₂, and D₃ is a topoisomerase inhibitor, and at least another one is a PD-L1 inhibitor, a CBL-B inhibitor, a TLR7/8 agonist, or a STING agonist.

In preferred embodiments, at least one of D₁, D₂, and D₃ is selected from: Exatecan (CAS 171335-80-1), DXd (CAS 1599440-33-1), 7-ethyl-10-hydroxycamptothecin (SN38, CAS 86639-52-3), Belotecan (CAS 213819-48-8), (4-NH₂)-Exatecan (AZD'0132, CAS 2495742-21-5), 7-MAD-MDCPT (CAS 765871-81-6), 7-aminomethyl-10-methyl-11-fluorocamptothecin (CAS 2378616-23-8), Voreloxin (CAS 175414-77-4), or derivatives and analogues thereof; and at least another one is selected from PARP inhibitors, ATR inhibitors, CHK1 inhibitors, Bcl-2 family protein inhibitors, LSD1 inhibitors, EZH2 inhibitors, BRD4 inhibitors, PRMT5 inhibitors, PD-L1 inhibitors, CBL-B inhibitors, TLR7/8 agonists, and STING agonists.

In preferred embodiments, at least one of D₁, D₂, and D₃ is selected from: Exatecan (CAS 171335-80-1), DXd (CAS 1599440-33-1), 7-ethyl-10-hydroxycamptothecin (SN38, CAS 86639-52-3), Belotecan (CAS 213819-48-8), (4-NH₂)-Exatecan (AZD'0132, CAS 2495742-21-5), 7-MAD-MDCPT (CAS 765871-81-6), 7-aminomethyl-10-methyl-11-fluorocamptothecin (CAS 2378616-23-8), Voreloxin (CAS 175414-77-4), or derivatives and analogues thereof; and at least another one is selected from: Rucaparib, Niraparib, Veliparib, A-966492, Talazoparib, AZD5305, Venadaparib, Mefuparib, Prexasertib (LY2606368), AZD7762, Rabusertib (LY2603618), MK-8776 (SCH 900776), CHIR-124, PF-477736, CCT245737 (SRA737, PNT-737), GDC-0575 (ARRY-575), Berzosertib, Ceralasertib, RP-6306, GSK-1520489A, and analogues thereof.

In preferred embodiments, at least one of D₁, D₂, and D₃ is a tubulin inhibitor, and at least another one is a PARP inhibitor, an ATR inhibitor, or a CHK1 inhibitor. In preferred embodiments, at least one of D₁, D₂, and D₃ is a tubulin inhibitor, and at least another one is a Bcl-2 family protein inhibitor. In preferred embodiments, at least one of D₁, D₂, and D₃ is a tubulin inhibitor, and at least another one is an LSD1 inhibitor, an EZH2 inhibitor, or a PRMT5 inhibitor. In preferred embodiments, at least one of D₁, D₂, and D₃ is a tubulin inhibitor, and at least another one is a PD-L1 inhibitor, a CBL-B inhibitor, a TLR7/8 agonist, or a STING agonist.

In preferred embodiments, at least one of D₁, D₂, and D₃ is selected from: Eribulin, Vinblastine, Paclitaxel, MMAE, MMAF, Maytansine or derivatives thereof, and at least another one is selected from PARP inhibitors, ATR inhibitors, CHK1 inhibitors, Bcl-2 family protein inhibitors, LSD1 inhibitors, EZH2 inhibitors, BRD4 inhibitors, PRMT5 inhibitors, PD-L1 inhibitors, CBL-B inhibitors, TLR7/8 agonists, and STING agonists.

In preferred embodiments, at least one of D₁, D₂, and D₃ is selected from: Eribulin, Vinblastine, Paclitaxel, MMAE, MMAF, Maytansine or derivatives thereof; and at least another one is selected from: Rucaparib, Niraparib, Veliparib, A-966492, Talazoparib, AZD5305, Venadaparib, Mefuparib, Prexasertib (LY2606368), AZD7762, Rabusertib (LY2603618), MK-8776 (SCH 900776), CHIR-124, PF-477736, CCT245737 (SRA737, PNT-737), GDC-0575 (ARRY-575), Berzosertib, Ceralasertib, RP-6306, GSK-1520489A, and analogues thereof.

In preferred embodiments, at least one of D₁, D₂, and D₃ is selected from: a PBD or Duocarmycin, and at least another one is selected from PARP inhibitors, ATR inhibitors, CHK1 inhibitors, Bcl-2 family protein inhibitors, LSD1 inhibitors, EZH2 inhibitors, BRD4 inhibitors, PRMT5 inhibitors, PD-L1 inhibitors, CBL-B inhibitors, TLR7/8 agonists, and STING agonists.

In preferred embodiments, at least one of D₁, D₂, and D₃ is a PBD or Duocarmycin, and at least another one is selected from: Rucaparib, Niraparib, Veliparib, A-966492, Talazoparib, AZD5305, Venadaparib, Mefuparib, Prexasertib (LY2606368), AZD7762, Rabusertib (LY2603618), MK-8776 (SCH 900776), CHIR-124, PF-477736, CCT245737 (SRA737, PNT-737), GDC-0575 (ARRY-575), Berzosertib, Ceralasertib, RP-6306, GSK-1520489A, and analogues thereof.

In preferred embodiments, at least two of D₁, D₂, and D₃ are drugs targeting the DNA Damage Response (DDR) or "synthetic lethality" related pathways. In preferred embodiments, at least two of D₁, D₂, and D₃ are selected from: Rucaparib, Niraparib, Veliparib, A-966492, Talazoparib, AZD5305, Venadaparib, Mefuparib, Prexasertib (LY2606368), AZD7762, Rabusertib (LY2603618), MK-8776 (SCH 900776), CHIR-124, PF-477736, CCT245737 (SRA737, PNT-737), GDC-0575 (ARRY-575), Berzosertib, Ceralasertib, RP-6306, GSK-1520489A, and analogues thereof.

In some aspects, the present invention provides a method for increasing the DAR value of an ADC, the method comprising forming a drug into any ADC represented by formula III, IIIa, or IIIb of the present invention. In preferred embodiments, the DAR value is an integer or decimal ranging from 16 to 56, for example, an integer or decimal from 16 to 24, from 24 to 40, or from 40 to 56. For instance, the DAR is about 16, about 24, about 32, about 40, about 48, about 56, or any integer or decimal therebetween.

In preferred embodiments, the drug is a cytotoxic drug or a tumor-targeted therapeutic drug.

In preferred embodiments, the cytotoxic drug is selected from drug units targeting topoisomerase, nucleoside antimetabolite anti-cancer drug units, drug units targeting tubulin, and drug units targeting DNA.

In preferred embodiments, the tumor-targeted therapeutic drug is selected from drug units targeting DNA damage response (DDR) or "synthetic lethality" related pathways, drug units targeting epigenetics, drug units targeting apoptosis-related pathways, and drug units targeting immune activation pathway-related targets.

In preferred embodiments, the drug unit targeting topoisomerase is Exatecan (CAS 171335-80-1), DXd (CAS 1599440-33-1), 7-ethyl-10-hydroxycamptothecin (SN38, CAS 86639-52-3), Belotecan (CAS 213819-48-8), (4-NH₂)-Exatecan (AZD'0132, CAS 2495742-21-5), 7-MAD-MDCPT (CAS 765871-81-6), 7-aminomethyl-10-methyl-11-fluorocamptothecin (CAS 2378616-23-8), Voreloxin (CAS 175414-77-4), or derivatives and analogs thereof. In preferred embodiments, the drug unit targeting tubulin is Eribulin, Vinblastine, Paclitaxel, MMAE, MMAF, Maytansine, or derivatives thereof. In preferred embodiments, the drug unit targeting DNA is a DNA minor groove binder (PBD), a DNA alkylator (Duocarmycin), Trabectedin, Lurbinectedin, or derivatives thereof.

In preferred embodiments, the nucleoside antimetabolite anti-cancer drug is Gemcitabine, Decitabine, or analogs thereof;
In preferred embodiments, the drug unit targeting DNA damage response (DDR) or "synthetic lethality" related pathways is a PARP inhibitor, ATR inhibitor, CHK1 inhibitor, ATM inhibitor, DNA-PK inhibitor, WEE1 inhibitor, POLQ inhibitor, CDK12 inhibitor, USP1 inhibitor, PKMYT1 inhibitor, or Rad51 inhibitor; the PARP inhibitor is preferably Rucaparib, Niraparib, Veliparib, A-966492, Talazoparib, AZD5305, Venadaparib, Mefuparib, or analogs thereof; the ATR inhibitor is preferably Berzosertib, Ceralasertib, or analogs thereof; and/or the CHK1 inhibitor is preferably Prexasertib (LY2606368), AZD7762, Rabusertib (LY2603618), MK-8776 (SCH 900776), CHIR-124, PF-477736, CCT245737 (SRA737, PNT-737), GDC-0575 (ARRY-575), or analogs thereof; and/or the PKMYT1 inhibitor is preferably RP-6306 and GSK-1520489A or analogs thereof. In preferred embodiments, the drug unit targeting apoptosis-related pathways is a Bcl-2 family protein inhibitor. In preferred embodiments, the drug unit targeting epigenetics is an LSD1 inhibitor, EZH2 inhibitor, BRD4 inhibitor, PRMT5 inhibitor, or PRMT1 inhibitor. In preferred embodiments, the drug unit targeting immune activation pathway-related targets is a PD-L1 inhibitor, CBL-B inhibitor, TLR7/8 agonist, or STING agonist.

In preferred embodiments, D₁, D₂, and D₃ can be selected from the exemplary combinations shown in Table A.

**Table A. Exemplary combinations of D₁, D₂, and D₃.**

| | D1 | D2 | D3 |
|---|---|---|---|
| Combination 1-1 | 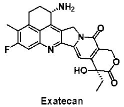 | 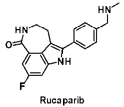 | / |
| Combination 1-2 | 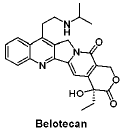 | 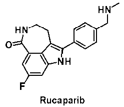 | / |
| Combination 1-3 | 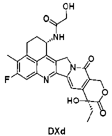 | 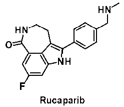 | / |
| Combination 1-4 | 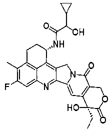 | 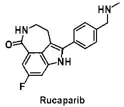 | / |
| Combination 1-5 | 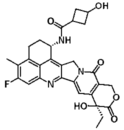 | 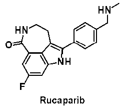 | / |
| Combination 1-6 | 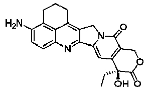 | 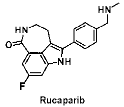 | / |
| Combination 1-7 | 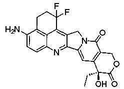 | 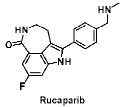 | / |
| Combination 1-8 | 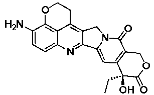 | 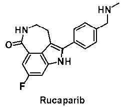 | / |
| Combination 1-9 | 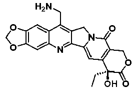 | 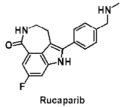 | / |
| Combination 1-10 | 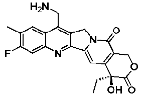 | 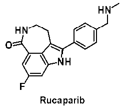 | / |
| Combination 1-11 | 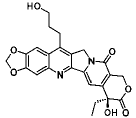 | 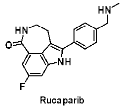 | / |
| Combination 1-12 | 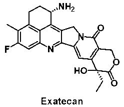 | 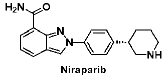 | / |
| Combination 1-13 | 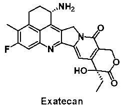 | 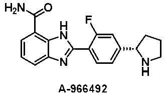 | / |
| Combination 1-14 | 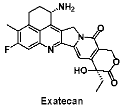 | 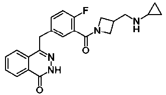 | / |
| Combination 1-15 | 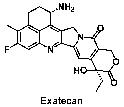 | 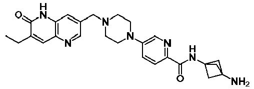 | / |
| Combination 1-16 | 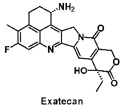 | 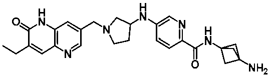 | / |
| Combination 1-17 | 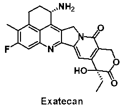 | 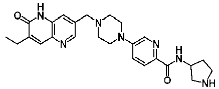 | / |
| Combination 1-18 | 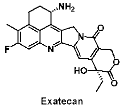 | 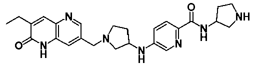 | / |
| Combination 1-19 | 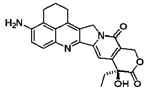 | 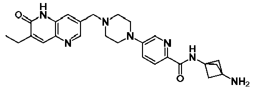 | / |
| Combination 2-1 | 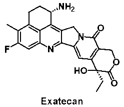 | 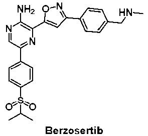 | / |
| Combination 2-2 | 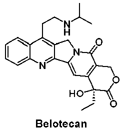 | 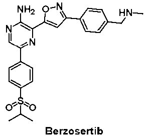 | / |
| Combination 2-3 | 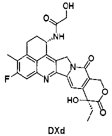 | 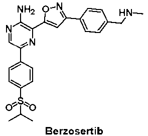 | / |
| Combination 2-4 | 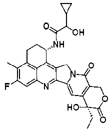 | 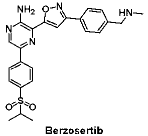 | / |
| Combination 2-5 | 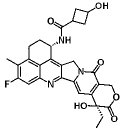 | 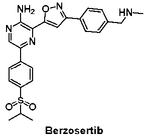 | / |
| Combination 2-6 | 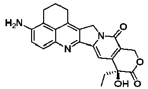 | 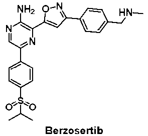 | / |
| Combination 2-7 | 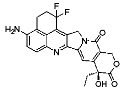 | 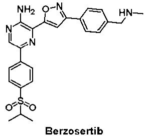 | / |
| Combination 3-1 | 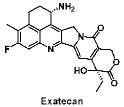 | 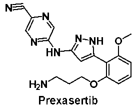 | / |
| Combination 3-2 | 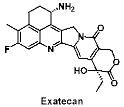 | 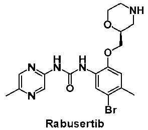 | / |
| Combination 3-3 | 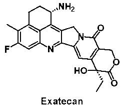 | 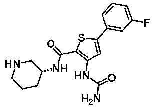 | / |
| Combination 3-4 | 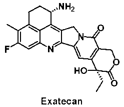 | 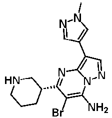 | / |
| Combination 3-5 | 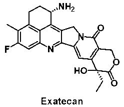 | 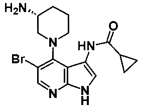 | / |
| Combination 3-6 | 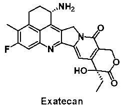 | 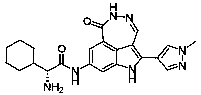 | / |
| Combination 3-7 | 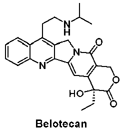 | 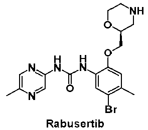 | / |
| Combination 3-8 | 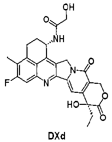 | 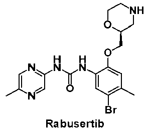 | / |
| Combination 3-9 | 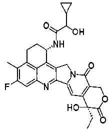 | 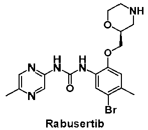 | / |
| Combination 3-10 | 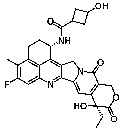 | 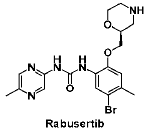 | / |
| Combination 3-11 | 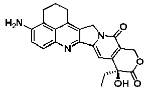 | 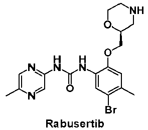 | / |
| Combination 3-12 | 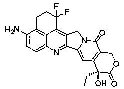 | 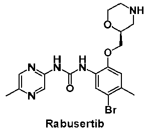 | / |
| Combination 9-1 | 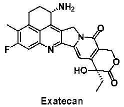 | 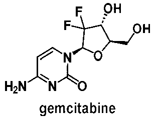 | / |
| Combination 11-1 | 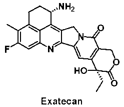 | 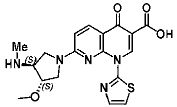 | / |
| Combination 17-1 | 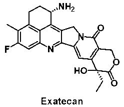 | 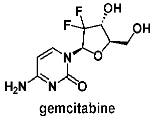 | / |
| Combination 4-1 | 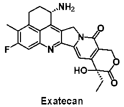 | 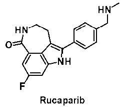 | 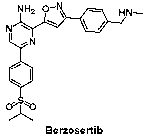 |
| Combination 5-1 | 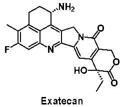 | 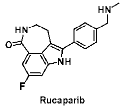 | 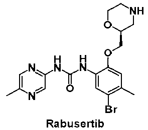 |
| Combination 5-2 | 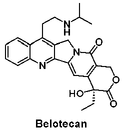 | 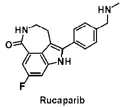 | 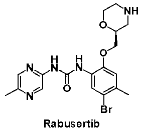 |
| Combination 13-1 | 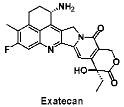 | 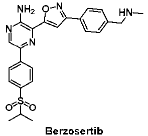 | 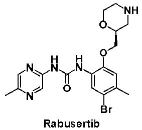 |
| Combination 4-2 | 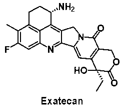 | 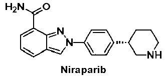 | 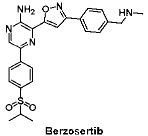 |
| Combination 13-1 | 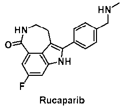 | 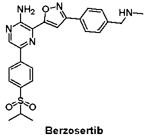 | 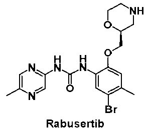 |
| Combination 13-2 | 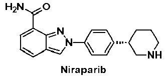 | 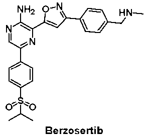 | 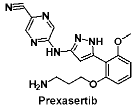 |
| Combination 14-1 | 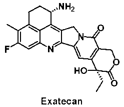 | 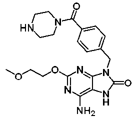 | / |
| Combination 14-2 | 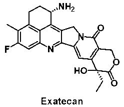 | 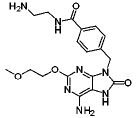 | / |
| Combination 14-3 | 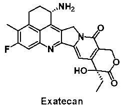 | 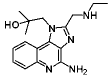 | / |
| Combination 14-4 | 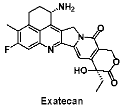 | 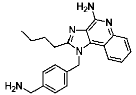 | / |
| Combination 14-5 | 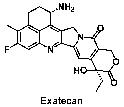 | 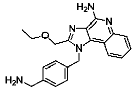 | / |
| Combination 14-6 | 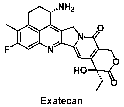 | 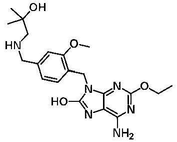 | |
| Combination 14-7 | 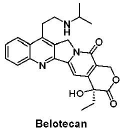 | 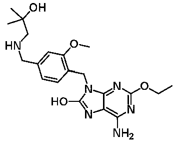 | |
| Combination 14-8 | 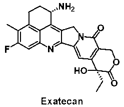 | 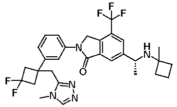 | / |
| Combination 14-9 | 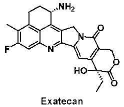 | 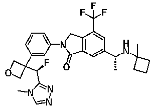 | / |
| Combination 14-10 | 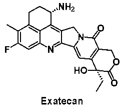 | 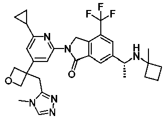 | / |
| Combination 14-11 | 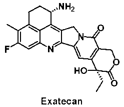 | 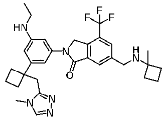 | / |
| Combination 14-12 | 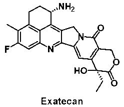 | 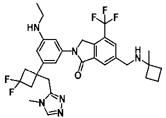 | / |
| Combination 27-1 | 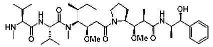 | 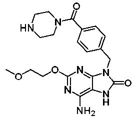 | / |
| Combination 27-2 | 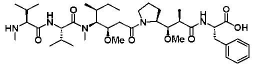 | 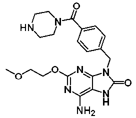 | / |
| Combination 27-3 | 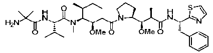 | 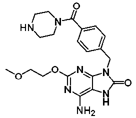 | / |
| Combination 27-4 | 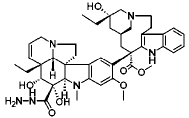 | 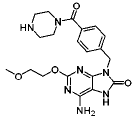 | / |
| Combination 27-5 | 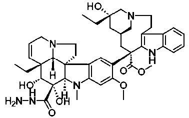 | 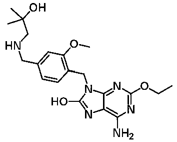 | / |
| Combination 15-1 | 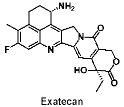 | 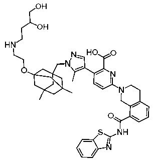 | / |
| Combination 15-2 | 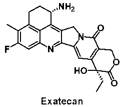 | 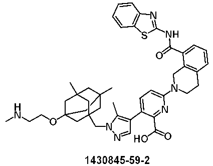 | / |
| Combination 15-3 | 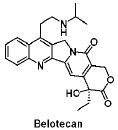 | 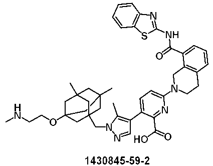 | / |
| Combination 27-1 | 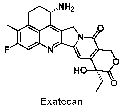 | 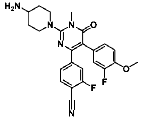 | |
| Combination 27-2 | 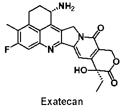 | 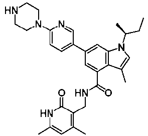 | / |
| Combination 26-1 | 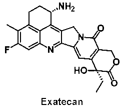 | 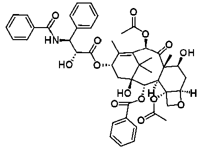 | / |
| Combination 61-1 | 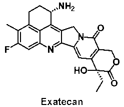 | 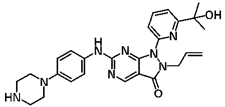 | / |
| Combination 8-1 | 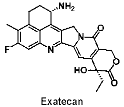 | 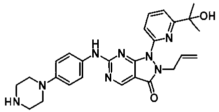 | 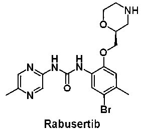 |
| Combination 8-2 | 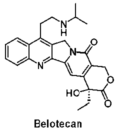 | 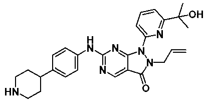 | 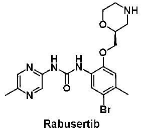 |
| Combination 62 | 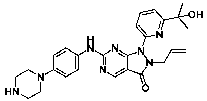 | 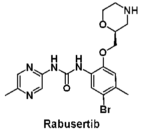 | / |
| Combination 63 | 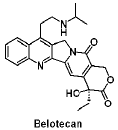 | 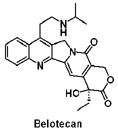 | / |
| Combination 63 | 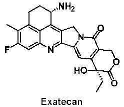 | 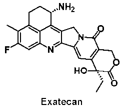 | / |
| Combination 71-1 | 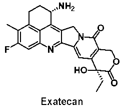 | 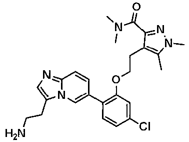 | / |
| Combination 71-2 | 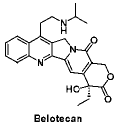 | 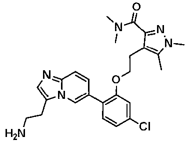 | / |
| Combination 71-3 | 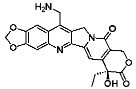 | 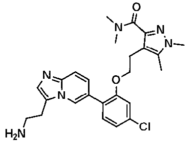 | / |
| Combination 71-4 | 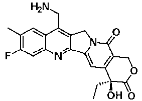 | 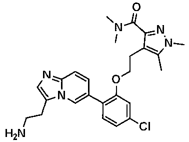 | / |
| Combination 72-1 | 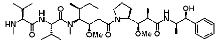 | 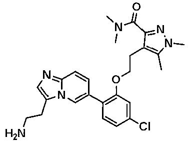 | / |
| Combination 72-2 | 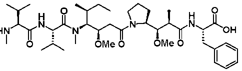 | 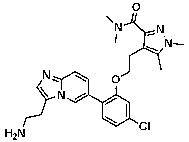 | / |
| Combination 72-3 | 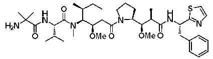 | 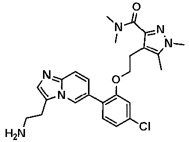 | / |
| Combination 72-4 | 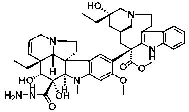 | 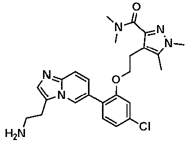 | / |

### Preparation Methods

Another aspect of the present invention also provides a method for preparing any antibody-drug conjugate of formula III, IIIa, or IIIb of the present invention. The method comprises reducing the disulfide bonds (e.g., in the hinge region) of an antibody or antibody fragment to generate a pair of cysteine residues, and subjecting the thiol groups of the cysteine residues to a substitution reaction with the Q or Q' group (e.g., a maleimido group) of a compound of formula I, Ia, Ib, II, IIa, IIb, IV, IVa, or IVb of the present invention, thereby conjugating the compound of formula I, Ia, Ib, II, IIa, IIb, IV, IVa, or IVb to the cysteine thiols of the antibody or antibody fragment to obtain the antibody-drug conjugate of formula III, IIIa, or IIIb.

In some embodiments, the drug-to-antibody ratio (DAR, m in formulas III, IIIa, or IIIb) is controlled according to the reaction conditions, commonly ranging from 2 to 8.

In the present invention, m represents the molar ratio of cytotoxic drug molecules to Ab (also known as DAR, Drug-to-Antibody Ratio). m can be an integer or decimal and can be understood as the average molar ratio of drug molecules to monoclonal antibody molecules in the antibody-drug conjugate obtained after conjugating a single monoclonal antibody molecule with cytotoxic drug(s). This can be typically determined by methods such as Hydrophobic-Interaction Chromatography (HIC), Reverse Phase High-Performance Liquid Chromatography (RP-HPLC), Sodium Dodecyl Sulfate Polyacrylamide Gel Electrophoresis (SDS-PAGE), Liquid Chromatography-Mass Spectrometry (LC-MS), or Ultraviolet/Visible Spectroscopy (UV/Vis).

In some embodiments, the present invention provides a method for preparing a compound of the present invention, comprising the following steps: synthesizing an intermediate fragment Q-C1-B1-P1-T1; activating the T1 group by reaction with bis(p-nitrophenyl) carbonate/4-nitrophenyl chloroformate, and further connecting it to drug component D1 to obtain intermediate Q-C1-B1-P1-T1-D1; the latter is deprotected and then sequentially condensed/coupled with intermediate fragment P2-T2-D2 or P3-T3-D3 to obtain the final product.

In some embodiments, T1, T2, and T3 comprise groups such as Boc/Fmoc-protected amino groups, alkynyl groups, carboxyl groups, or hydroxyl groups, which can be directly or after activation condensed/coupled with other components.

In some embodiments, T1, T2, T3 or P1, P2, P3 comprise Boc-protected amino groups which, after contact with trifluoroacetic acid, are condensed with substituted polysarcosine residues, polyols, polyglycerols, glycosyls, cyclodextrins, substituted ethylene glycol fragments, substituted glycosylated polyethylene glycols, substituted glycosylated polyglycerols, substituted cyclodextrin polyethylene glycols, or combinations thereof.

In some embodiments, the antibody is contacted with a compound of the present invention under conditions suitable for forming a bond between the antibody and the compound. In some embodiments, the antibody is contacted with the compound of the present invention in a mixture of a buffer and an organic solvent. In some embodiments, the antibody is contacted with the compound of the present invention at about 0°C to about 37°C. In one embodiment, contacting the antibody with the compound of the present invention is preceded by reacting the antibody with a reducing agent in a buffer to obtain the reduced antibody.

In some embodiments, after obtaining the reduced antibody and before contacting the antibody with the compound of the present invention, the method comprises removing the reducing agent. In the context of the antibody, removing the reducing agent comprises subjecting the reaction product to desalting column chromatography and/or ultrafiltration.

In some embodiments, the reducing agent is selected from the group consisting of: tris(2-carboxyethyl)phosphine hydrochloride (TCEP), beta-mercaptoethanol, beta-mercaptoethylamine hydrochloride, and dithiothreitol (DTT). In some embodiments, the buffer is selected from the group consisting of: potassium dihydrogen phosphate-sodium hydroxide (KH₂PO₄-NaOH)/sodium chloride (NaCl)/diethylenetriaminepentaacetic acid (DTPA) buffer, disodium hydrogen phosphate-citric acid/sodium chloride (NaCl)/diethylenetriaminepentaacetic acid (DTPA), boric acid-borax/sodium chloride (NaCl)/diethylenetriaminepentaacetic acid (DTPA), histidine-sodium hydroxide/sodium chloride (NaCl)/diethylenetriaminepentaacetic acid (DTPA), and PBS/diethylenetriaminepentaacetic acid (DTPA). In some embodiments, the organic solvent is selected from the group consisting of: acetonitrile (ACN), dimethylformamide (DMF), dimethylacetamide (DMA), and dimethyl sulfoxide (DMSO). In some embodiments, the organic solvent constitutes no more than 30% by volume of the mixture of buffer and organic solvent.

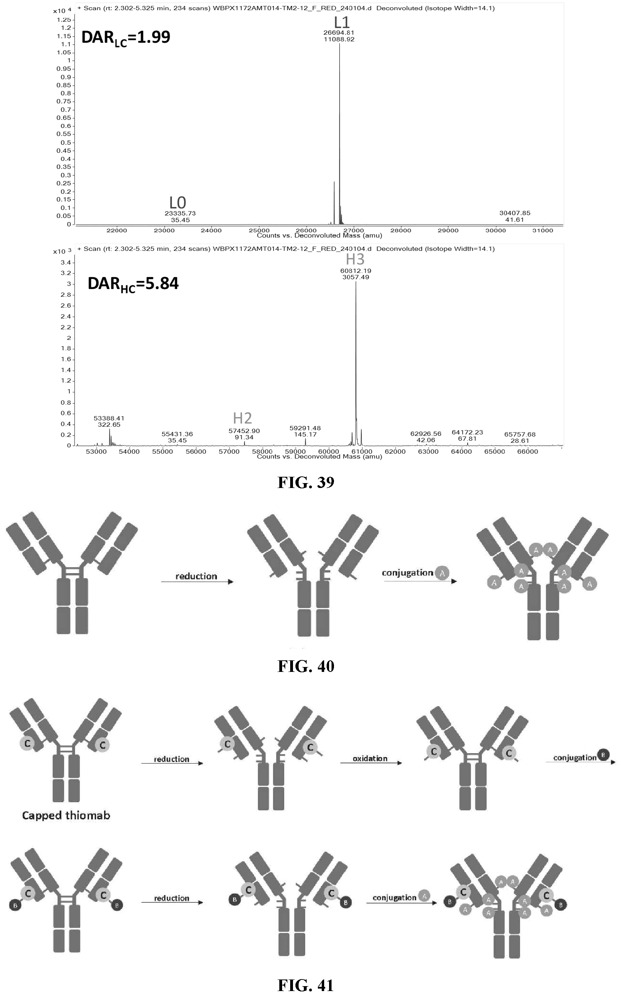

In some embodiments, the reaction steps for antibody conjugation are as shown in Figure 39.

Wherein A is the drug-linker fragment represented by formula I, Ia, or Ib, with the same definition as above, and specific embodiments are as described above.

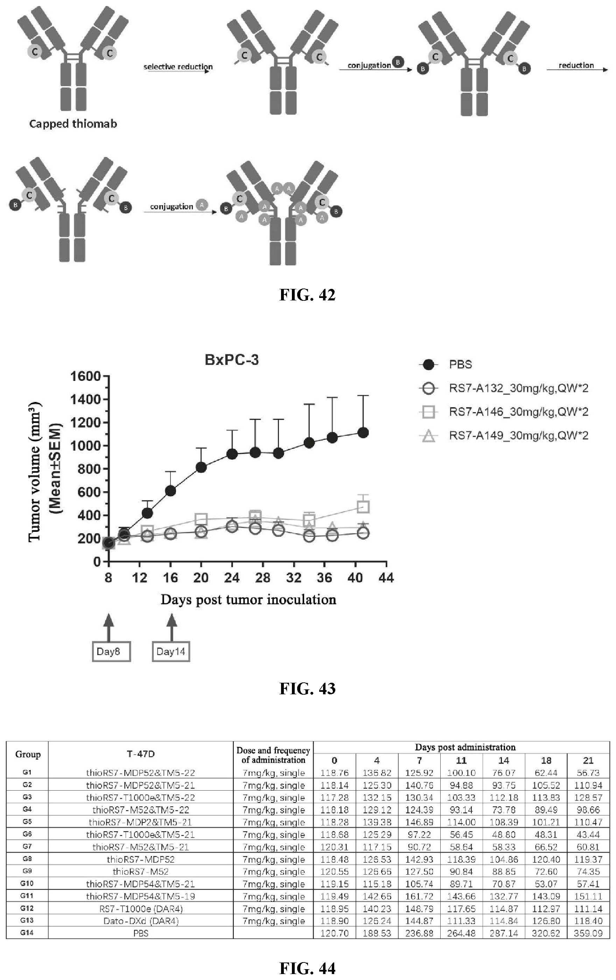

In some embodiments, the antibody is an engineered thiomab antibody. By introducing additional disulfide bonds at specific positions, and through the drug-linker fragments A or B represented by formula I, Ia, or Ib as described in the present invention, more possible antibody-drug conjugate compositions can be obtained. Engineered thiomab antibodies include but are not limited to LC-V205C, LC-214C, LC-K149C, HC-A114C, HC-174C, HC-373C/375C, Fc-S396C, etc. The antibody-drug conjugate can be obtained via Step A **(****Figure 41****):** Reduction -> Oxidation -> Conjugation with drug-linker fragment A -> Reduction -> Conjugation with drug-linker fragment B; or the antibody-drug conjugate can be obtained via Step B **(****Figure 42****):** Selective reduction -> Conjugation with drug-linker fragment B -> Reduction -> Conjugation with drug-linker fragment A.

Wherein A and B are the drug-linker fragments represented by formula I, Ia, or Ib, with the same definition as above, and specific implementation methods are as described above.

### Beneficial Effects

The design of multi-payload antibody-drug conjugates (ADCs) requires consideration of multiple factors. First, it is necessary to select two or more drugs with different mechanisms of action that are expected to produce synergistic effects, while fully considering factors such as the safety and efficacy of the payload molecules. Second, these drug molecules need to be stably conjugated to the antibody molecule to achieve precise targeted therapy. Finally, it is also necessary to ensure the stability and activity of both the drug molecules and the antibody molecule to guarantee therapeutic efficacy and safety. The inventors have adopted a novel chemical modification method that allows for the conjugation of multiple drug molecules to the antibody molecule without affecting the antibody's structure, stability, and affinity, thereby forming multi-payload antibody-drug conjugates. This design method enables precise positioning and modification of drug molecules, thereby improving drug targeting and therapeutic efficacy.

The multi-payload antibody-drug conjugates of the present invention employs two or more different drug molecules, which can achieve synergistic effects through different mechanisms of action, thereby enhancing therapeutic efficacy. One type of drug molecule is typically a chemotherapeutic agent, such as a topoisomerase I (TOPO1) inhibitor or a topoisomerase II (TOP2) inhibitor. As an important class of chemotherapeutic drugs, TOPO1 inhibitors bind to TOPO1, forming a stable TOPO1-DNA-drug complex that prevents DNA strand religation, leading to DNA single-strand breaks. DNA single-strand breaks can interfere with DNA replication, transcription, and repair, inducing apoptosis or necrosis. TOPO1 inhibitors can selectively act on rapidly proliferating tumor cells. Combining them with other DDR inhibitors can increase treatment specificity and effectiveness, thereby improving therapeutic outcomes. For example, PARP inhibitors and ATR-CHK1-WEE1 pathway inhibitors are commonly used DDR inhibitors that inhibit the cell's basic repair mechanisms, leading to accumulation of DNA damage and apoptosis. Therefore, another type of payload drugs is selected from targeted drugs that can produce synergistic effects, such as DDR inhibitors (PARP inhibitors, CHK1 inhibitors, ATR inhibitors, WEE1 inhibitors, DNA-PK inhibitors, etc.). Such combinations are expected to significantly increase efficacy against homologous recombination deficiency (HRD)-related tumors. Known genes encoding homologous recombination proteins include: BRCA1, BRCA2, ATM, ATR, BARD1, BAP1, BLM, RAD51B/C/D, DSS1, RPA1, BRIP1, FANCA, FANCC, FANCD2, FANCE, FANCF, PALB2, MRE11A, NBN, CHK1/2, CDK12, etc. HRD is prevalent across different tumors. According to research, HRD is present in 13% of tumors, with the most common mutational lineages including ovarian (14.1%), bladder (9.7%), breast (8.0%), endometrial (7.4%), prostate (7.1%), and pancreatic (6.5%) cancers.

Furthermore, by activating the immune system to enhance the body's immune surveillance and clearance of tumor cells, immunotherapy can effectively control and treat various tumors. Combining it with chemotherapeutic drugs can enhance therapeutic efficacy through multiple pathways. First, chemotherapeutic drugs can kill tumor cells, releasing antigens and nucleic acid molecules, thereby further activating the immune system's response against tumor cells. Additionally, chemotherapeutic drugs can also inhibit the immune escape mechanisms of tumor cells, enhancing the effect of immunotherapy, and can also produce targeted small molecule drugs with immunostimulatory effects, which can be selected from PD-L1 inhibitors, CBL-B inhibitors, TLR agonists, STING agonists, and epigenetic-related inhibitors like LSD1 inhibitors and EZH2 inhibitors.

Moreover, conjugating targeted molecules that inhibit tumor-associated signaling pathways to antibodies, such as inhibitors targeting EGFR pathway, inhibitors targeting Ras-Raf-MAPK pathway, inhibitors targeting PI3K/AKT/mTOR pathway, inhibitors targeting cell cycle pathways, agonists targeting cGAS-STING signaling pathway, estrogen receptor antagonists, androgen receptor antagonists, autophagy inhibitors, FAK inhibitors, drugs targeting transcription factors such as GSPT1 degraders, etc., can address mutations and drug resistance arising in the treatment of specific tumors, leading to superior or unexpected effects, for example, in pancreatic cancer, HER2+/ER- breast cancer, prostate cancer, melanoma, etc.

Experiments show that the primary advantages of the present disclosure lie in one or more of the following aspects:
1) The linker technology employed in this disclosure allows flexible adjustment of the Drug-to-Antibody Ratio (DAR) of the antibody-drug conjugates through different construction methods. It also enables fine-tuning of the ADC's physicochemical properties, efficacy, and toxicity profiles based on disease type and therapeutic goals, fully leveraging the advantages of dual-drug/multi-drug delivery by ADCs to enhance ADC activity. Furthermore, from the perspective of atom economy, it reduces the molar amount of reagents required to construct highly homogeneous multi-payload ADC drugs, thereby lowering production costs while improving efficacy.
2) Through the rational design and combination of drug molecules with different pharmacological mechanisms and characteristics as enabled by the present invention, antibody-conjugated combination drugs targeting different tumors/indications can be designed and screened, making them more "programmable" compared to traditional antibody-drug conjugates.
3) Compared to existing technologies, some embodiments of the present disclosure employ combinations of targeted inhibitors and chemotherapeutic toxin drugs for multi-warhead loading in ADCs. This is expected to achieve superior therapeutic effects against drug-resistant tumors, tumors insensitive to existing therapies, and tumors related to homologous recombination deficiency.
4) Existing technologies commonly use dual-toxin combinations, which to some extent increase the safety risks of ADCs. In contrast, the present disclosure innovatively employs medium-to-low toxicity chemotherapeutic drugs combined with tumor-targeting inhibitors. Through their synergistic effect, this not only significantly improves therapeutic efficacy but also substantially widens the safety window of the ADC. Furthermore, the present disclosure introduces a unique conjugation strategy that can efficiently and stably link dual-toxins, triple-toxins, or even multiple toxins to the antibody, thereby preparing ADCs with excellent homogeneity and stability. Additionally, combined with existing thiomab conjugation technology, we can achieve various ratio combinations of different drugs (from 1:1 to 1:10), and even obtain more diverse drug combination conjugates through thiomab technology. This innovation not only improves the efficacy of ADC drugs but also expands their therapeutic window, offering a novel combination targeted therapy strategy for the future of oncology treatment.
5) Compared to existing technologies, the present disclosure employs specific linker technology. Through the "neighboring group protection effect" of hydrophilic groups, it can effectively mitigate issues such as increased hydrophobicity and decreased stability of ADCs caused by multi-drug loading, while improving the homogeneity of the conjugated drugs. It does not require antibody engineering and is compatible with most targeting carriers such as monoclonal antibodies, bispecific antibodies, multispecific antibodies, nanobodies, and nucleic acid aptamers.
6) Provided is a linker technology that enables loading multiple drugs onto the same antibody, increasing drug concentration within tumor cells, and achieving synergistic/additive effects of different drug combinations within tumor cells and/or the tumor microenvironment. This will further enhance the drug efficacy against tumors with low antigen expression.

### Sequence Listings

| SEQ ID NO. | Description | Sequence |
|---|---|---|
| 1 | Patritumab HC (heavy chain) amino acid sequence | QVQLQQWGAGLLKPSETLSLTCAVYGGSFSGYYWSWIRQPPGKGL EWIGEINHSGSTNYNPSLKSRVTISVETSKNQFSLKLSSVTAADT AVYYCARDKWTWYFDLWGRGTLVTVSSASTKGPSVFPLAPSSKST SGGTAALGCLVKDYFPEPVTVSWNSGALTSGVHTFPAVLQSSGLY SLSSVVTVPSSSLGTQTYICNVNHKPSNTKVDKRVEPKSCDKTHT CPPCPAPELLGGPSVFLFPPKPKDTLMISRTPEVTCVVVDVSHED PEVKFNWYVDGVEVHNAKTKPREEQYNSTYRVVSVLTVLHQDWLN GKEYKCKVSNKALPAPIEKTISKAKGQPREPQVYTLPPSREEMTK NQVSLTCLVKGFYPSDIAVEWESNGQPENNYKTTPPVLDSDGSFF LYSKLTVDKSRWQQGNVFSCSVMHEALHNHYTQKSLSLSPGK |
| 2 | Patritumab LC (light chain) amino acid sequence | DIEMTQSPDSLAVSLGERATINCRSSQSVLYSSSNRNYLAWYQQN PGQPPKLLIYWASTRESGVPDRFSGSGSGTDFTLTISSLQAEDVA VYYCQQYYSTPRTFGQGTKVEIKRTVAAPSVFIFPPSDEQLKSGT ASVVCLLNNFYPREAKVQWKVDNALQSGNSQESVTEQDSKDSTYS LSSTLTLSKADYEKHKVYACEVTHQGLSSPVTKSFNRGEC |
| 3 | Trastuzumab HC amino acid sequence | EVQLVESGGGLVQPGGSLRLSCAASGFNIKDTYIHWVRQAPGKGL EWVARIYPTNGYTRYADSVKGRFTISADTSKNTAYLQMNSLRAED TAVYYCSRWGGDGFYAMDYWGQGTLVTVSSASTKGPSVFPLAPSS |
| | | KSTSGGTAALGCLVKDYFPEPVTVSWNSGALTSGVHTFPAVLQSS GLYSLSSVVTVPSSSLGTQTYICNVNHKPSNTKVDKKVEPKSCDK THTCPPCPAPELLGGPSVFLFPPKPKDTLMISRTPEVTCVVVDVS HEDPEVKFNWYVDGVEVHNAKTKPREEQYNSTYRVVSVLTVLHQD WLNGKEYKCKVSNKALPAPIEKTISKAKGQPREPQVYTLPPSREE MTKNQVSLTCLVKGFYPSDIAVEWESNGQPENNYKTTPPVLDSDG SFFLYSKLTVDKSRWQQGNVFSCSVMHEALHNHYTQKSLSLSPG |
| 4 | Trastuzumab LC amino acid sequence | DIQMTQSPSSLSASVGDRVTITCRASQDVNTAVAWYQQKPGKAPK LLIYSASFLYSGVPSRFSGSRSGTDFTLTISSLQPEDFATYYCQQ HYTTPPTFGQGTKVEIKRTVAAPSVFIFPPSDEQLKSGTASVVCL LNNFYPREAKVQWKVDNALQSGNSQESVTEQDSKDSTYSLSSTLT LSKADYEKHKVYACEVTHQGLSSPVTKSFNRGEC |
| 5 | Datopotamab HC amino acid sequence | QVQLVQSGAEVKKPGASVKVSCKASGYTFTTAGMQWVRQAPGQGL EWMGWINTHSGVPKYAEDFKGRVTISADTSTSTAYLQLSSLKSED TAVYYCARSGFGSSYWYFDVWGQGTLVTVSSASTKGPSVFPLAPS SKSTSGGTAALGCLVKDYFPEPVTVSWNSGALTSGVHTFPAVLQS SGLYSLSSVVTVPSSSLGTQTYICNVNHKPSNTKVDKRVEPKSCD KTHTCPPCPAPELLGGPSVFLFPPKPKDTLMISRTPEVTCVVVDV SHEDPEVKFNWYVDGVEVHNAKTKPREEQYNSTYRVVSVLTVLHQ DWLNGKEYKCKVSNKALPAPIEKTISKAKGQPREPQVYTLPPSRE EMTKNQVSLTCLVKGFYPSDIAVEWESNGQPENNYKTTPPVLDSD GSFFLYSKLTVDKSRWQQGNVFSCSVMHEALHNHYTQKSLSLSPG K |
| 6 | Datopotamab LC amino acid sequence | DIQMTQSPSSLSASVGDRVTITCKASQDVSTAVAWYQQKPGKAPK LLIYSASYRYTGVPSRFSGSGSGTDFTLTISSLQPEDFAVYYCQQ HYITPLTFGQGTKLEIKRTVAAPSVFIFPPSDEQLKSGTASVVCL LNNFYPREAKVQWKVDNALQSGNSQESVTEQDSKDSTYSLSSTLT LSKADYEKHKVYACEVTHQGLSSPVTKSFNRGEC |
| 7 | DS5573a (Ifinatamab) HC amino acid sequence | QVQLVQSGAEVKKPGSSVKVSCKASGYTFTNYVMHWVRQAPGQGL EWMGYINPYNDDVKYNEKFKGRVTITADESTSTAYMELSSLRSED TAVYYCARWGYYGSPLYYFDYWGQGTLVTVSSASTKGPSVFPLAP SSKSTSGGTAALGCLVKDYFPEPVTVSWNSGALTSGVHTFPAVLQ SSGLYSLSSVVTVPSSSLGTQTYICNVNHKPSNTKVDKRVEPKSC DKTHTCPPCPAPELLGGPSVFLFPPKPKDTLMISRTPEVTCVVVD VSHEDPEVKFNWYVDGVEVHNAKTKPREEQYNSTYRVVSVLTVLH QDWLNGKEYKCKVSNKALPAPIEKTISKAKGQPREPQVYTLPPSR EEMTKNQVSLTCLVKGFYPSDIAVEWESNGQPENNYKTTPPVLDS DGSFFLYSKLTVDKSRWQQGNVFSCSVMHEALHNHYTQKSLSLSP |
| | | GK |
| 8 | DS5573a (Ifinatamab) LC amino acid sequence | EIVLTQSPATLSLSPGERATLSCRASSRLIYMHWYQQKPGQAPRP LIYATSNLASGIPARFSGSGSGTDFTLTISSLEPEDFAVYYCQQW NSNPPTFGQGTKVEIKRTVAAPSVFIFPPSDEQLKSGTASVVCLL NNFYPREAKVQWKVDNALQSGNSQESVTEQDSKDSTYSLSSTLTL SKADYEKHKVYACEVTHQGLSSPVTKSFNRGEC |
| 9 | Tisotumab HC amino acid sequence | EVQLLESGGGLVQPGGSLRLSCAASGFTFSNYAMSWVRQAPGKGL EWVSSISGSGDYTYYTDSVKGRFTISRDNSKNTLYLQMNSLRAED TAVYYCARSPWGYYLDSWGQGTLVTVSSASTKGPSVFPLAPSSKS TSGGTAALGCLVKDYFPEPVTVSWNSGALTSGVHTFPAVLQSSGL YSLSSVVTVPSSSLGTQTYICNVNHKPSNTKVDKRVEPKSCDKTH TCPPCPAPELLGGPSVFLFPPKPKDTLMISRTPEVTCVVVDVSHE DPEVKFNWYVDGVEVHNAKTKPREEQYNSTYRVVSVLTVLHQDWL NGKEYKCKVSNKALPAPIEKTISKAKGQPREPQVYTLPPSREEMT KNQVSLTCLVKGFYPSDIAVEWESNGQPENNYKTTPPVLDSDGSF FLYSKLTVDKSRWQQGNVFSCSVMHEALHNHYTQKSLSLSPGK |
| 10 | Tisotumab LC amino acid sequence | DIQMTQSPPSLSASAGDRVTITCRASQGISSRLAWYQQKPEKAPK SLIYAASSLQSGVPSRFSGSGSGTDFTLTISSLQPEDFATYYCQQ YNSYPYTFGQGTKLEIKRTVAAPSVFIFPPSDEQLKSGTASVVCL LNNFYPREAKVQWKVDNALQSGNSQESVTEQDSKDSTYSLSSTLT LSKADYEKHKVYACEVTHQGLSSPVTKSFNRGEC |
| 11 | Sacituzumab HC amino acid sequence | QVQLQQSGSELKKPGASVKVSCKASGYTFTNYGMNWVKQAPGQGL KWMGWINTYTGEPTYTDDFKGRFAFSLDTSVSTAYLQISSLKADD TAVYFCARGGFGSSYWYFDVWGQGSLVTVSSASTKGPSVFPLAPS SKSTSGGTAALGCLVKDYFPEPVTVSWNSGALTSGVHTFPAVLQS SGLYSLSSVVTVPSSSLGTQTYICNVNHKPSNTKVDKRVEPKSCD KTHTCPPCPAPELLGGPSVFLFPPKPKDTLMISRTPEVTCVVVDV SHEDPEVKFNWYVDGVEVHNAKTKPREEQYNSTYRVVSVLTVLHQ DWLNGKEYKCKVSNKALPAPIEKTISKAKGQPREPQVYTLPPSRE EMTKNQVSLTCLVKGFYPSDIAVEWESNGQPENNYKTTPPVLDSD GSFFLYSKLTVDKSRWQQGNVFSCSVMHEALHNHYTQKSLSLSPG K |
| 12 | Sacituzumab LC amino acid sequence | DIQLTQSPSSLSASVGDRVSITCKASQDVSIAVAWYQQKPGKAPK LLIYSASYRYTGVPDRFSGSGSGTDFTLTISSLQPEDFAVYYCQQ HYITPLTFGAGTKVEIKRTVAAPSVFIFPPSDEQLKSGTASVVCL LNNFYPREAKVQWKVDNALQSGNSQESVTEQDSKDSTYSLSSTLT LSKADYEKHKVYACEVTHQGLSSPVTKSFNRGEC |
| 13 | thio-RS7 HC | QVQLQQSGSELKKPGASVKVSCKASGYTFTNYGMNWVKQAPGQGL |

### Examples

### Example 1 Synthesis and Preparation of Compounds

The raw materials mentioned in the present invention are commercially available products, or are prepared by methods known in the art or according to the methods described herein.

### 1) Synthesis of Compound A2

### Synthetic Route:

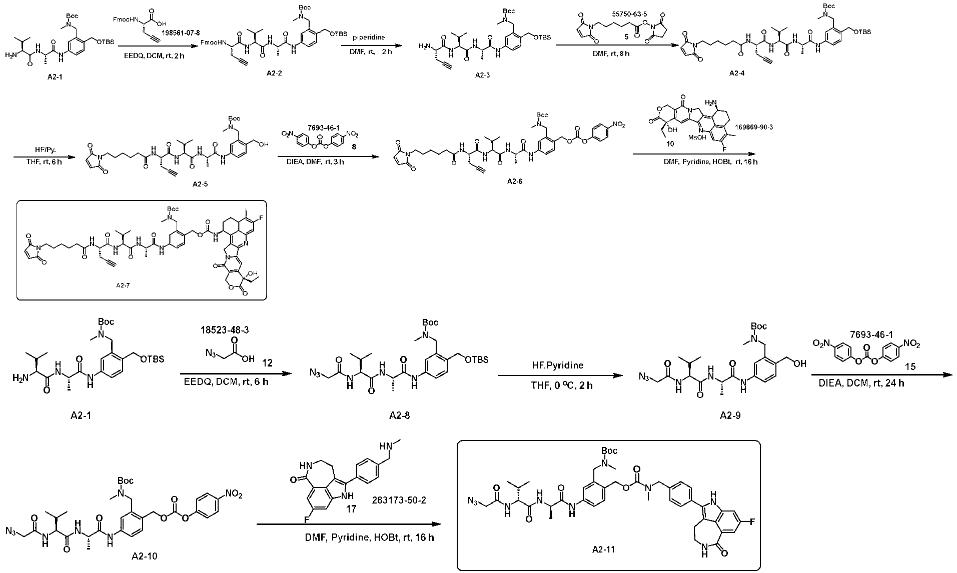

| | | |
|---|---|---|
| | amino acid sequence | KWMGWINTYTGEPTYTDDFKGRFAFSLDTSVSTAYLQISSLKADD TAVYFCARGGFGSSYWYFDVWGQGSLVTVSSASTKGPSVFPLAPS SKSTSGGTAALGCLVKDYFPEPVTVSWNSGALTSGVHTFPAVLQS SGLYSLSSVVTVPSSSLGTQTYICNVNHKPSNTKVDKRVEPKSCD KTHTCPPCPAPELLGGPSVFLFPPKPKDTLMISRTPEVTCVVVDV SHEDPEVKFNWYVDGVEVHNAKTKPREEQYNSTYRVVSVLTVLHQ DWLNGKEYKCKVSNKALPAPIEKTISKAKGQPREPQVYTLPPSRE EMTKNQVSLTCLVKGFYPSDIAVEWESNGQPENNYKTTPPVLDSD GSFFLYSKLTVDKSRWQQGNVFSCSVMHEALHNHYTQKSLSLSPG K |
| 14 | thio-RS7 LC amino acid sequence | DIQLTQSPSSLSASVGDRVSITCKASQDVSIAVAWYQQKPGKAPK LLIYSASYRYTGVPDRFSGSGSGTDFTLTISSLQPEDFAVYYCQQ HYITPLTFGAGTKVEIKRTVAAPSVFIFPPSDEQLKSGTASVVCL LNNFYPREAKVQWKVDNALQSGNSQESVTEQDSKDSTYSLSSTLT LSKADYEKHKVYACEVTHQGLSSPCTKSFNRGEC |

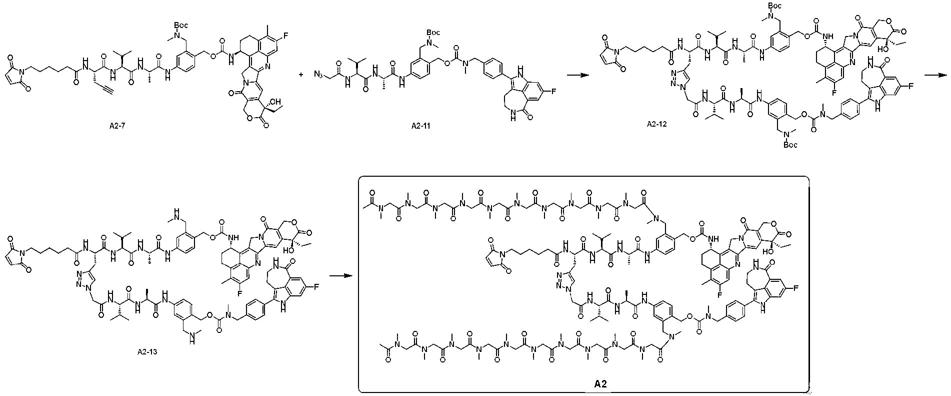

### Synthetic Steps:

### Synthesis of Intermediate A2-2

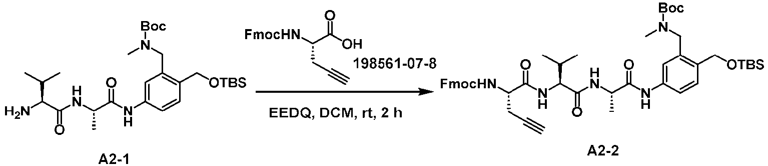

**Intermediate A2-1** (1.0 g, 1.8 mmol, 1.0 eq) was dissolved in dichloromethane (30 mL). Carboxylic acid compound (198561-07-8, 600 mg, 1.8 mmol, 1.0 eq) and 2-ethoxy-1-ethoxycarbonyl-1,2-dihydroquinoline (890 mg, 3.6 mmol, 2.0 eq) were added to the reaction mixture. The reaction mixture was stirred at room temperature for 2 hours. After the reaction was confirmed complete by LC-MS monitoring, the mixture was concentrated directly. The residue was purified by silica gel column chromatography (PE/EA = 4/6) to afford **intermediate A2-2** (1.3 g, 89%) as a pale yellow solid. LCMS: [M+Na] ⁺ = 890.3

### Synthesis of Intermediate A2-3

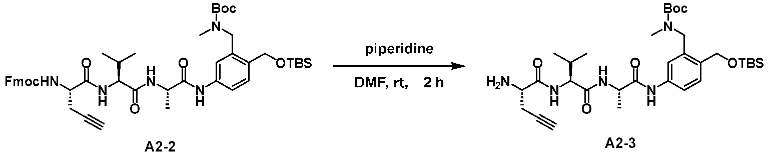

**Intermediate A2-2** (1.2 g, 3.6 mmol, 1.0 eq) was dissolved in DMF (4 mL). Piperidine (0.8 mL) was added to the reaction mixture. The reaction mixture was stirred at room temperature for 2 hours. After the reaction was confirmed complete by LC-MS monitoring, the mixture was diluted with ethyl acetate, washed with water and brine. The organic phase was dried over anhydrous sodium sulfate, filtered, and concentrated. The residue was purified by silica gel column chromatography (DCM/MeOH = 10/1) to afford **intermediate A2-3** (0.7 g, 78%) as a pale yellow solid. LCMS: [M+H] ⁺ = 646.4

### Synthesis of Intermediate A2-4

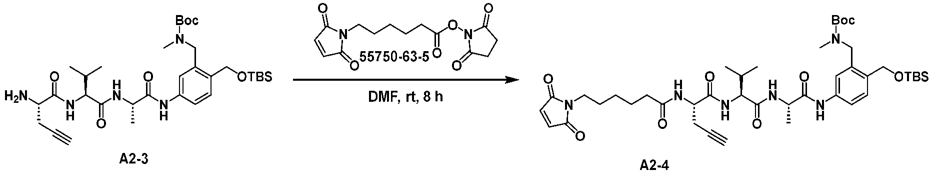

**Intermediate A2-3** (660 mg, 1.02 mmol, 1.0 eq) was dissolved in DMF (4 mL). Then, Mc-OSu (314 mg, 1.02 mmol, 1.0 eq) was added to the reaction mixture. The reaction mixture was stirred at room temperature for 8 hours. After the reaction was confirmed complete by LC-MS monitoring, the mixture was diluted with ethyl acetate, washed with water and brine. The organic phase was dried over anhydrous sodium sulfate, filtered, and concentrated. The residue was purified by silica gel column chromatography (DCM/MeOH = 93/7) to afford **intermediate A2-4** (850 mg, 99%) as a pale yellow solid. LCMS: [M+Na]⁺ = 861.3

### Synthesis of Intermediate A2-5

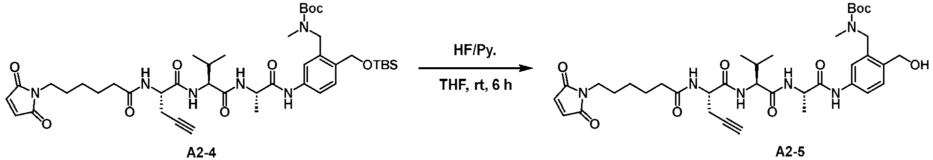

**Intermediate A2-4** (800 mg, 0.95 mmol, 1.0 eq) was dissolved in THF (10 mL). The reaction mixture was cooled to 0°C, and pyridine hydrofluoride (1160 mg, 7.62 mmol, 8.0 eq, 65% w/w) was added under a nitrogen atmosphere. The reaction mixture was stirred at room temperature under nitrogen for 6 hours. After the reaction was confirmed complete by LC-MS monitoring, water (30 mL) was added to the mixture. The mixture was extracted with ethyl acetate. The combined organic phases were washed with saturated ammonium chloride and brine, dried over anhydrous sodium sulfate, filtered, and concentrated to afford **intermediate A2-5** (650 mg, 94%) as a pale yellow solid. LCMS: [M+Na] ⁺ = 747.3

### Synthesis of Intermediate A2-6

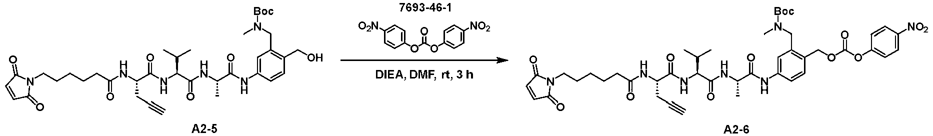

**Intermediate A2-5** (600 mg, 0.83 mmol, 1.0 eq) and bis(4-nitrophenyl) carbonate (503 mg, 1.65 mmol, 2.0 eq) were dissolved in DMF (5 mL). The reaction mixture was cooled to 0°C, and N,N-Diisopropylethylamine (214 mg, 1.65 mmol, 2.0 eq) was added under a nitrogen atmosphere. After addition, the reaction mixture was stirred at room temperature under nitrogen for 3 hours. After the reaction was confirmed complete by LC-MS monitoring, water (30 mL) was added to the mixture. The mixture was extracted with ethyl acetate. The combined organic phases were washed with water and brine, dried over anhydrous sodium sulfate, filtered, and concentrated. The residue was purified by silica gel column chromatography (DCM/MeOH = 93/7) to afford **intermediate A2-6** (650 mg, 88%) as a pale yellow solid. LCMS: [M+Na] ⁺ = 912.3

### Synthesis of Intermediate A2-7

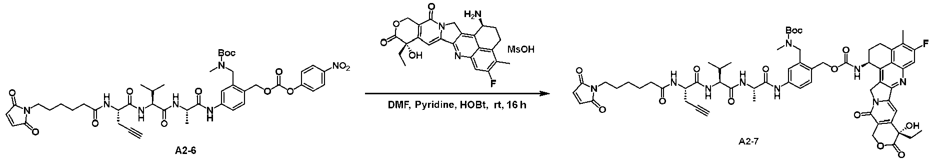

**Intermediate A2-7** (600 mg, 0.67 mmol, 1.0 eq), HOBt (91 mg, 0.67 mmol, 1.0 eq), and pyridine (533 mg, 6.70 mmol, 10.0 eq) were dissolved in DMF (8 mL). Exatecan mesylate (376 mg, 0.71 mmol, 1.05 eq) was added under a nitrogen atmosphere. After addition, the reaction mixture was stirred at room temperature under nitrogen for 18 hours. After the reaction was confirmed complete by LC-MS monitoring, water (30 mL) was added to the mixture. The mixture was extracted with ethyl acetate. The combined organic phases were washed with water and brine, dried over anhydrous sodium sulfate, filtered, and concentrated. The residue was purified by silica gel column chromatography (DCM/MeOH = 6/1) to afford **intermediate A2-**7 (300 mg, 37%) as a white solid. LCMS: [M+Na] ⁺ = 1186.4

### Synthesis of Intermediate A2-8

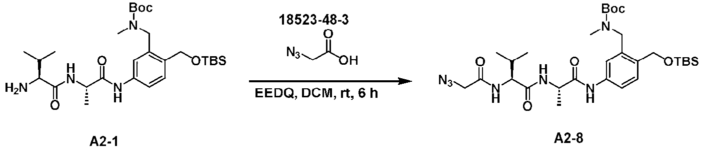

**Intermediate A2-1** (1.0 g, 1.8 mmol, 1.0 eq) was dissolved in dichloromethane (30 mL). Compound **12** (180 mg, 1.8 mmol, 1.0 eq) and 2-ethoxy-1-ethoxycarbonyl-1,2-dihydroquinoline (890 mg, 3.6 mmol, 2.0 eq) were added to the reaction mixture. The reaction mixture was stirred at room temperature for 2 hours. After the reaction was confirmed complete by LC-MS monitoring, the mixture was concentrated directly. The residue was purified by silica gel column chromatography (DCM/EA = 3/7) to afford **intermediate A2-8** as a pale yellow solid. LCMS: [M+H] ⁺ = 634.4

### Synthesis of Intermediate A2-9

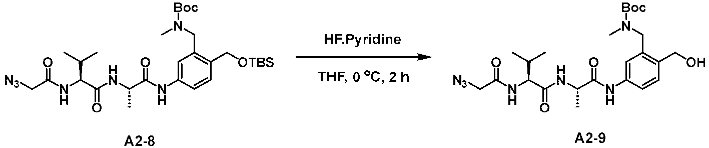

Compound **13** (1100 mg, 1.73 mmol, 1.0 eq) was dissolved in THF (20 mL). The reaction mixture was cooled to 0°C, and pyridine hydrofluoride (1320 mg, 8.66 mmol, 5.0 eq, 65%w/w) was added under a nitrogen atmosphere. The reaction mixture was stirred at room temperature under nitrogen for 6 hours. After the reaction was confirmed complete by LC-MS monitoring, water (30 mL) was added to the mixture. The mixture was extracted with ethyl acetate. The combined organic phases were washed with saturated ammonium chloride and brine, dried over anhydrous sodium sulfate, filtered, and concentrated to afford compound 7 (700 mg, 77%) as a pale yellow solid. LCMS: [M+Na] ⁺ = 542.3

### Synthesis of Intermediate A2-10

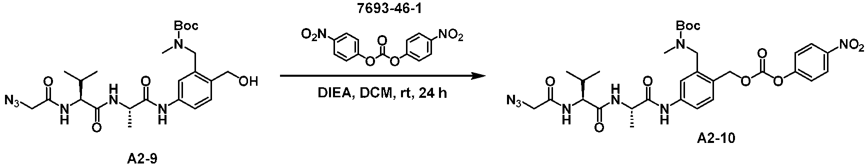

**Intermediate A2-9** (400 mg, 0.77 mmol, 1.0 eq) and bis(4-nitrophenyl) carbonate (468 mg, 1.54 mmol, 2.0 eq) were dissolved in dichloromethane (4 mL). The reaction mixture was cooled to 0°C, and N,N-Diisopropylethylamine (198 mg, 1.54 mmol, 2.0 eq) was added under a nitrogen atmosphere. After addition, the reaction mixture was stirred at room temperature under nitrogen for 24 hours. After the reaction was confirmed complete by LC-MS monitoring, the organic phase was concentrated. The residue was recrystallized from diethyl ether to afford **intermediate A2-10** (330 mg, 63%) as a white gum-like solid. LCMS: [M+Na] ⁺ = 707.3

### Synthesis of Intermediate A2-11

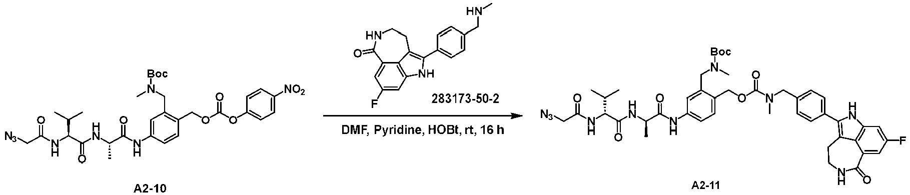

**Intermediate A2-10** (600 mg, 0.87 mmol, 1.0 eq), HOBt (118 mg, 0.87 mmol, 1.0 eq), and pyridine (692 mg, 8.75 mmol, 10.0 eq) were dissolved in DMF (8 mL). Rucaparib (376 mg, 0.71 mmol, 1.05 eq) was added under a nitrogen atmosphere. After addition, the reaction mixture was stirred at room temperature under nitrogen for 18 hours. After the reaction was confirmed complete by LC-MS monitoring, water (30 mL) was added to the mixture. The mixture was extracted with ethyl acetate. The combined organic phases were washed with water and brine, dried over anhydrous sodium sulfate, filtered, and concentrated. The residue was purified by silica gel column chromatography (DCM/MeOH = 10/1) to afford **intermediate A2-11** (500 mg, 66%) as a pale yellow solid. LCMS: [M+H] ⁺ = 869.3

### Synthesis of Intermediate A2-12

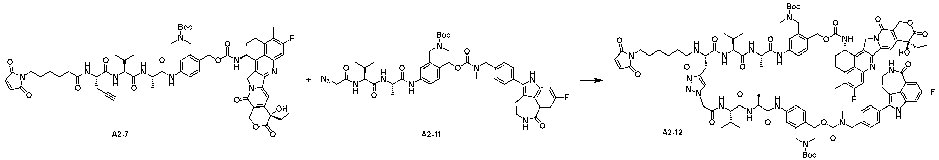

**Intermediate A2-7** (150 mg, 0.13 mmol, 1.0 eq) and **intermediate A2-11** (115 mg, 0.13 mmol, 1.0 eq) were dissolved in a mixture of dichloromethane/methanol/water (3/2/1 mL). Copper(II) sulfate pentahydrate (3.16 mg, 0.013 mmol, 0.1 eq, in 100 µL water) and sodium ascorbate (6.26 mg, 0.032 mmol, 0.25 eq, in 100 µL water) were added under a nitrogen atmosphere. After addition, the reaction mixture was stirred at room temperature under nitrogen for 4 hours. After the reaction was confirmed complete by LC-MS monitoring, water (30 mL) was added to the mixture. The mixture was extracted with dichloromethane. The combined organic phases were washed with water and brine, dried over anhydrous sodium sulfate, filtered, and concentrated to afford **intermediate A2-12** (100 mg, 38%) as a yellow solid. LCMS: [M+H] ⁺ = 1028.1 (half peak)

### Synthesis of Intermediate A2-13

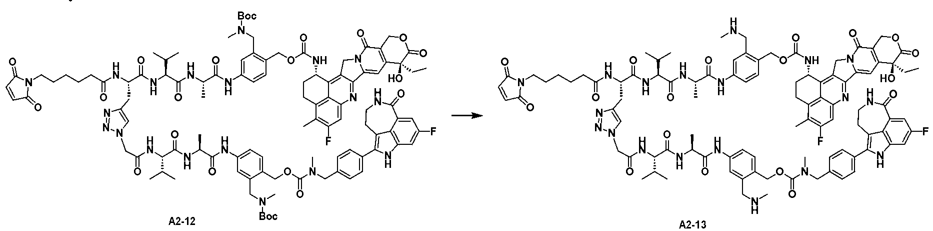

**Intermediate A2-12** (950 mg, 0.046 mmol, 1.0 eq) was dissolved in DCM (3 mL). Trifluoroacetic acid (1 mL) was added to the reaction mixture. The mixture was stirred at room temperature for 5 minutes. After the reaction was confirmed complete by LC-MS monitoring, the organic phase was concentrated to afford crude **intermediate A2-13,** which was used directly in the next step.

LCMS: [M+H] ⁺ = 928.1 (half peak)

### Synthesis of Compound A2

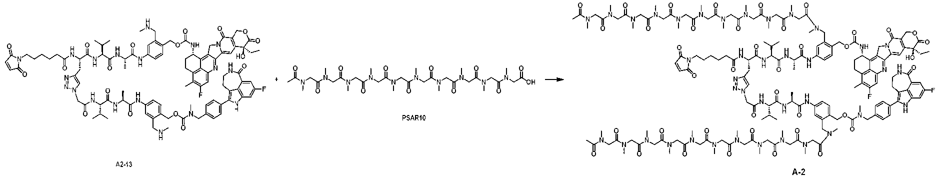

**Intermediate A2-13** was dissolved in DMF (3 mL). Ac-PSAR10-OH (71 mg, 0.092 mmol, 2.0 eq) and N,N-Diisopropylethylamine (60 mg, 0.46 mmol, 10.0 eq) were added. Finally, O-(7-Azabenzotriazol-1-yl)-N,N,N',N'-tetramethyluronium hexafluorophosphate (37 mg, 0.097 mmol, 2.1 eq) was added under a nitrogen atmosphere. After addition, the reaction mixture was stirred at room temperature under nitrogen for 3 hours. After the reaction was confirmed complete by LC-MS monitoring, the mixture was filtered. The filtrate was purified by preparative HPLC (mobile phase: 0.05% HCOOH/CH₃CN/H₂O) to afford Compound **A2** (6.5 mg, 7%) as a yellow solid.

LCMS: [M+H] ⁺ = 1681.2 (half peak)
¹H NMR (400 MHz, DMSO-*d*₆) δ 11.66 (s, 1H), 8.38 (s, 3H), 8.25 (s, 2H), 8.20-8.12 (m, 2H), 7.79-7.75 (m, 4H), 7.59-7.54 (m, 4H), 7.43 (s, 1H), 7.40 (s, 4H), 7.40-7.36 (m, 4H), 6.98 (s, 2H), 6.52 (s, 2H), 5.44 (s, 3H), 5.28 (s, 4H), 5.14-5.11 (m, 8H), 4.65-4.54 (m, 10H), 4.49 (s, 3H), 4.42-4.14 (m, 34H), 4.06-3.89 (m, 28H), 2.89-2.81 (m, 55H), 2.76-2.66 (m, 30H), 2.37 (s, 2H), 2.22-2.15 (m, 2H), 2.07 (s, 8H), 2.06-2.04 (m, 1H), 1.99-1.97 (m, 4H), 1.89-1.86 (m, 1H), 1.83 (s, 2H), 1.76-1.72 (m, 2H), 1.45-1.39 (m, 6H), 1.31-1.23 (m, 10H), 1.17-1.09 (m, 4H), 0.92-0.77 (m, 19H).

### 2) Synthesis of Compound A10

### Synthetic Route:

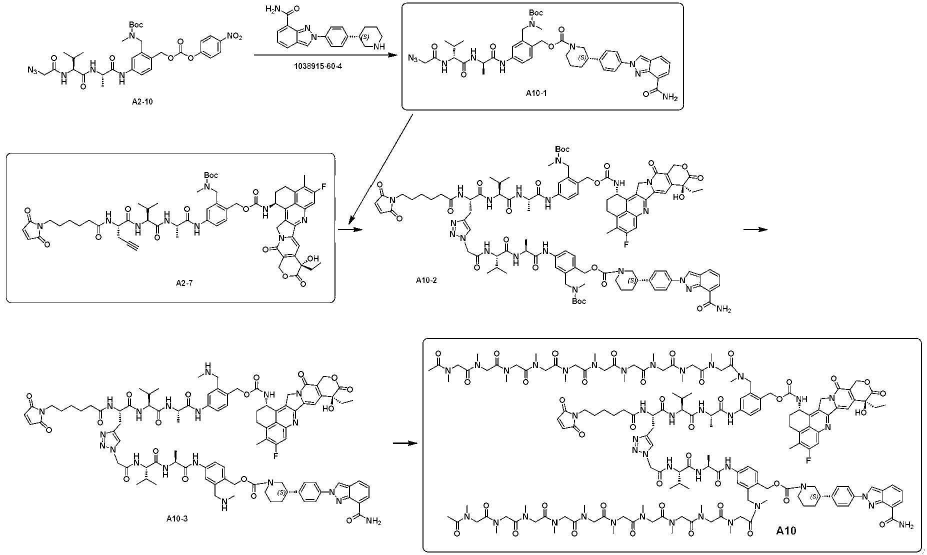

### Synthetic Steps

### Synthesis of Intermediate A10-1

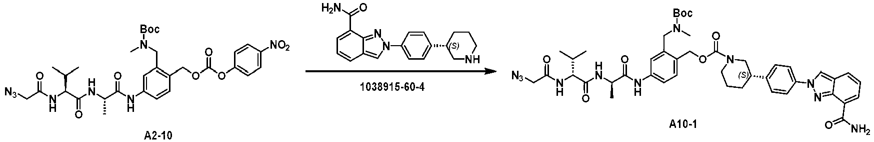

Intermediate A2-10 (300 mg, 0.44 mmol, 1.0 eq), HOBt (59 mg, 0.44 mmol, 1.0 eq), and pyridine (692 mg, 8.75 mmol, 20.0 eq) were dissolved in DMF (4 mL). Niraparib (147 mg, 0.46 mmol, 1.05 eq) was added under a nitrogen atmosphere. After addition, the reaction mixture was stirred at room temperature under nitrogen for 16 hours. After the reaction was confirmed complete by LC-MS monitoring, water (30 mL) was added to the mixture. The mixture was extracted with ethyl acetate. The combined organic phases were washed with water and brine, dried over anhydrous sodium sulfate, filtered, and concentrated. The residue was purified by silica gel column chromatography (DCM/MeOH = 10/1) to afford **intermediate A10-1** (300 mg, 79%) as a pale yellow solid.

LCMS: [M+H] ⁺ = 866.4

### Synthesis of Intermediate A10-2

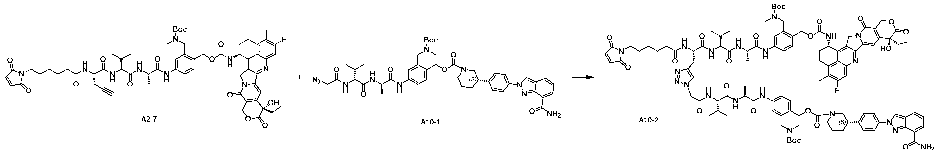

**Intermediate** compound **A2-7** (55 mg, 0.046 mmol, 1.0 eq) and **A10-1** (42 mg, 0.048 mmol, 1.05 eq) were dissolved in a mixture of dichloromethane/methanol/water (3/2/1 mL). Copper(II) sulfate pentahydrate (1.16 mg, 0.0046 mmol, 0.1 eq, in 100 µL water) and sodium ascorbate (1.83 mg, 0.0093 mmol, 0.20 eq, in 100 µL water) were added under a nitrogen atmosphere. After addition, the reaction mixture was stirred at room temperature under nitrogen for 4 hours. After the reaction was confirmed complete by LC-MS monitoring, water (30 mL) was added to the mixture. The mixture was extracted with dichloromethane. The combined organic phases were washed with water and brine, dried over anhydrous sodium sulfate, filtered, and concentrated to afford **intermediate A10-2** (90 mg, 90%) as a yellow solid.

LCMS: [M+H] ⁺ = 1027.2 (half peak)

### Synthesis of Intermediate A10-3

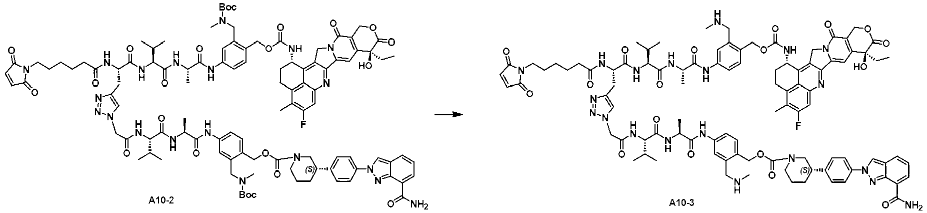

**Intermediate A10-2** (90 mg, 0.0438 mmol, 1.0 eq) was dissolved in DCM (3 mL). Trifluoroacetic acid (1 mL) was added to the reaction mixture. The mixture was stirred at room temperature for 5 minutes. After the reaction was confirmed complete by LC-MS monitoring, the organic phase was concentrated to afford crude **intermediate A10-3,** which was used directly in the next step.

LCMS: [M+H] ⁺ = 926.4 (half peak)

### Synthesis of Compound A10

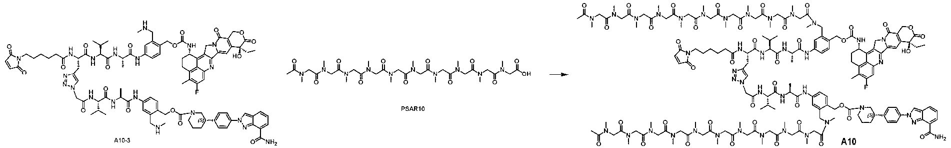

The **intermediate A10-3** from the previous step was dissolved in DMF (3 mL). Ac-PSAR10-OH (75 mg, 0.097 mmol, 2.0 eq) and N,N-Diisopropylethylamine (63 mg, 0.49 mmol, 10.0 eq) were added. Finally, (7-Azabenzotriazol-1-yl)-N,N,N',N'-tetramethyluronium hexafluorophosphate (39 mg, 0.10 mmol, 2.1 eq) was added under a nitrogen atmosphere. After addition, the reaction mixture was stirred at room temperature under nitrogen for 1 hour. After the reaction was confirmed complete by LC-MS monitoring, the mixture was filtered. The filtrate was purified by preparative HPLC (mobile phase: 0.05% HCOOH/CH₃CN/H₂O) to afford **Compound A10** (11.08 mg, 12%) as a pale yellow solid.

LCMS: [M+H] ⁺ = 1679.4 (half peak); ¹H NMR (400 MHz, DMSO-*d*₆) δ 10.07-9.99 (m, 2H), 9.28 (s, 1H), 8.63-8.33 (m, 6H), 8.24-7.97 (m, 5H), 7.82-7.58 (m, 4H), 7.52-7.50 (m, 4H), 7.45-7.20 (m, 5H), 7.10 (s, 1H), 6.98 (s, 1H), 6.60-6.42 (m, 2H), 5.44-5.07 (m, 10H), 4.65-4.53 (m, 6H), 4.49-3.80 (m, 45H), 3.12-2.62 (m, 66H), 2.40-2.33 (m, 3H), 2.08-1.74 (m, 16H), 1.61-1.40 (m, 4H), 1.35-1.23 (m, 6H), 1.13 (s, 1H), 0.93-0.72 (m, 14H).

### 3) Synthesis of Compound A11

### Synthetic Route:

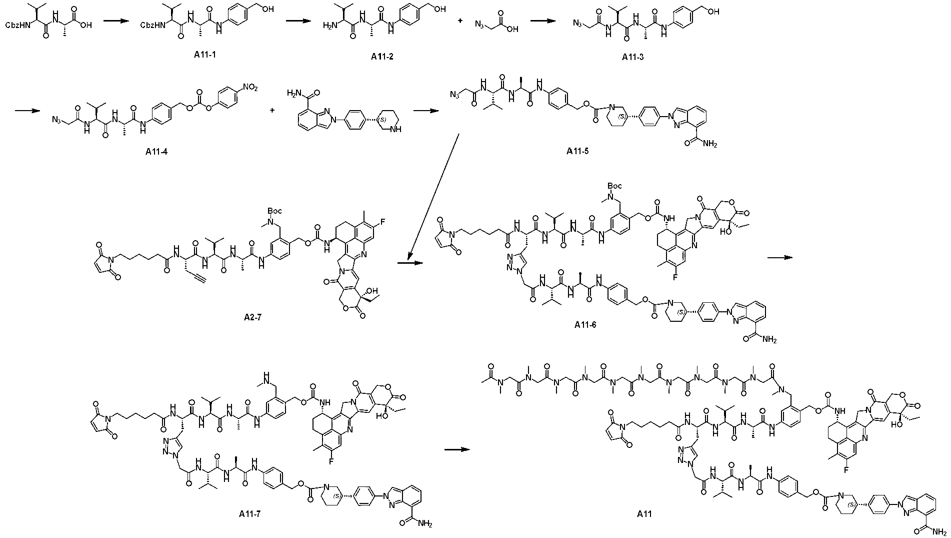

### Synthetic Steps

### Synthesis of Intermediate A11-1

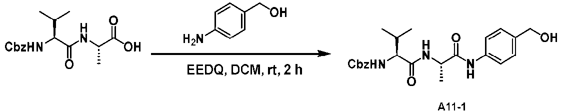

Z-Val-Ala (4.0 g, 12.4 mmol, 1.0 eq) was dissolved in a mixture of dichloromethane (40 mL) and methanol (10 mL). 4-Aminobenzyl alcohol (1.99 g, 16.12 mmol, 1.3 eq) and 2-Ethoxy-1-ethoxycarbonyl-1,2-dihydroquinoline (6.13 g, 24.8 mmol, 2.0 eq) were added to the reaction mixture. The mixture was stirred at room temperature for 2 hours. After the reaction was confirmed complete by LC-MS monitoring, the mixture was concentrated directly. The residue was purified by silica gel column chromatography (DCM/MeOH = 93/7) to afford compound **A11-1** (4.9 g, 90%) as a white solid. LCMS: [M+H] ⁺ = 428.2

### Synthesis of Intermediate A11-2

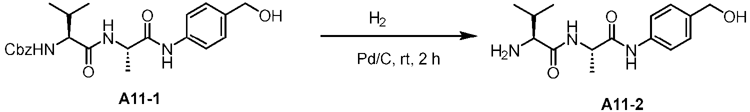

**Intermediate A11-1** (4.9 g, 11.5 mmol, 1.0 eq) was dissolved in MeOH (50 mL). Palladium on carbon (0.49 g, 10% w/w) was added. The reaction mixture was stirred at room temperature under a hydrogen atmosphere for 2 hours. After the reaction was confirmed complete by LC-MS monitoring, the mixture was filtered and concentrated to afford compound **A11-2** (3.5 g, 98%) as a colorless oily liquid. LCMS: [M+H] ⁺ = 294.4

### Synthesis of Intermediate A11-3

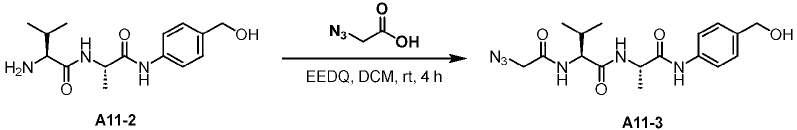

Intermediate **A11-2** (3.4 g, 11.6 mmol, 1.0 eq) was dissolved in dichloromethane (50 mL). Azidoacetic acid (1.41 g, 13.92 mmol, 1.2 eq) and 2-Ethoxy-1-ethoxycarbonyl-1,2-dihydroquinoline (5.74 g, 23.2 mmol, 2.0 eq) were added to the reaction mixture. The mixture was stirred at room temperature for 4 hours. After the reaction was confirmed complete by LC-MS monitoring, the mixture was concentrated directly. The residue was purified by silica gel column chromatography (DCM/MeOH = 93/7) to afford **A11-3** (3.4 g, 77%) as a white solid. LCMS: [M+H] ⁺ = 377.2

### Synthesis of Intermediate A11-4

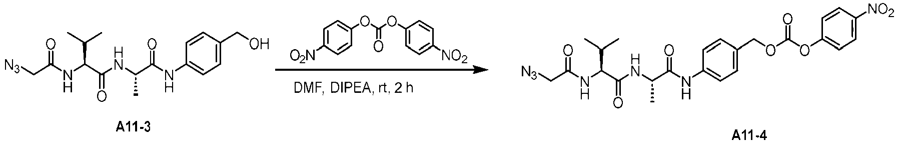

Intermediate **A11-3** (1.5 g, 4.00 mmol, 1.0 eq) and N,N-Diisopropylethylamine (1.55 g, 12.00 mmol, 3.0 eq) were dissolved in DMF (10 mL). After stirring for a while, bis(4-nitrophenyl) carbonate (2.43 g, 8.00 mmol, 2.0 eq) was added. The reaction mixture was stirred at room temperature for 2 hours. After the reaction was confirmed complete by LC-MS monitoring, the mixture was diluted with ethyl acetate, washed with water and brine. The organic phase was dried over anhydrous sodium sulfate, filtered, and concentrated. The residue was purified by silica gel column chromatography (DCM/MeOH = 93/7) to afford intermediate **A11-4** (1.7 g, 78%) as a white solid. LCMS: [M+Na] ⁺ = 564.2

### Synthesis of Intermediate A11-5

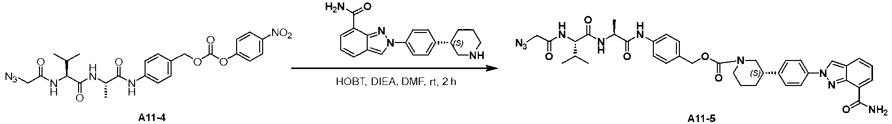

Intermediate **A11-4** (88.8 mg, 0.277 mmol, 1.0 eq) and N,N-Diisopropylethylamine (107.4 mg, 0.831 mmol, 3.0 eq) were dissolved in DMF (5 mL). After stirring until clear, Niraparib (CAS 1038915-60-4, 150 mg, 0.277 mmol, 1.0 eq) and HOBt (37.4 mg, 0.277 mmol, 1.0 eq) were added. The reaction mixture was stirred at room temperature for 2 hours. After the reaction was confirmed complete by LC-MS monitoring, water (30 mL) was added. The mixture was extracted with ethyl acetate. The combined organic phases were washed with water and brine, dried over anhydrous sodium sulfate, filtered, and concentrated. The residue was purified by silica gel column chromatography (DCM/MeOH = 93/7) to afford compound **A11-5** (170 mg, 85%) as a white solid. LCMS: [M+H] ⁺ = 723.3

### Synthesis of Intermediate All-6

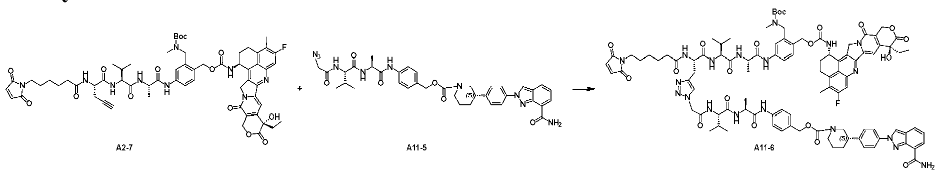

Compound A2-7 (100 mg, 0.0842 mmol, 1.0 eq) and compound **A11-5** (61 mg, 0.0842 mmol, 1.0 eq) were dissolved in a mixture of MeOH/DCM/water (10/10/2.5 mL). Then, 100 µL of an aqueous solution of copper(II) sulfate pentahydrate (2.1 mg, 0.00842 mmol, 0.1 eq) and 100 µL of an aqueous solution of sodium ascorbate (3.34 mg, 0.01684 mmol, 0.2 eq) were added. After addition, the reaction mixture was stirred at room temperature under nitrogen for 4 hours. After the reaction was confirmed complete by LC-MS monitoring, water (30 mL) was added. The mixture was extracted with dichloromethane. The combined organic phases were washed with water and brine, dried over anhydrous sodium sulfate, filtered, and concentrated to afford compound **A11-6** (160 mg, 100%) which was used directly in the next step. LCMS: [M+H] ⁺ = 954.9 (half peak)

### Synthesis of Intermediate All-7

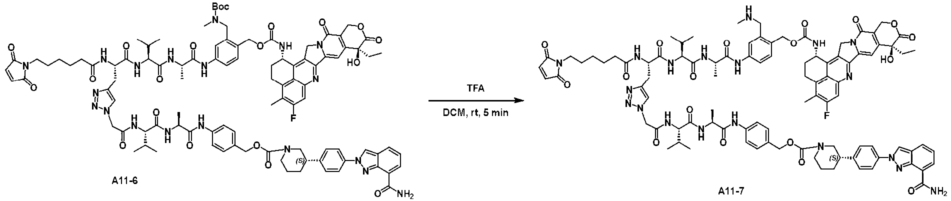

Compound **A11-6** (160 mg, 0.0842 mmol) was dissolved in DCM (6 mL). Trifluoroacetic acid (2 mL) was added to the reaction mixture. The mixture was stirred at room temperature for 5 minutes. After the reaction was confirmed complete by LC-MS monitoring, the organic phase was concentrated to afford crude compound **A11-7,** which was used directly in the next step. LCMS: [M+H] ⁺ = 905.1 (half peak)

### Synthesis of Compound A11

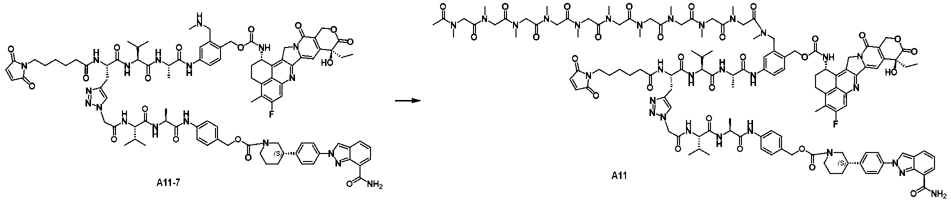

The compound **A11-7** from the previous step was dissolved in DMF (3 mL). Ac-PSAR10-OH (41 mg, 0.0842 mmol, 1.0 eq) and N,N-Diisopropylethylamine (69 mg, 0.842 mmol, 10.0 eq) were added. Finally, O-(7-Azabenzotriazol-1-yl)-N,N,N',N'-tetramethyluronium hexafluorophosphate (22 mg, 0.0926 mmol, 1.1 eq) was added. After addition, the reaction mixture was stirred at room temperature under nitrogen for 1 hour. After the reaction was confirmed complete by LC-MS monitoring, the mixture was filtered. The filtrate was purified by preparative HPLC (mobile phase: 0.05% 0.05% HCOOH/CH₃CN/H₂O) to afford Compound **A11** (24.2 mg, 11%) as a white solid. LCMS: [M+H] ⁺ = 1281.2 (half peak).

### 4) Synthesis of Compound A12

### Synthetic Route:

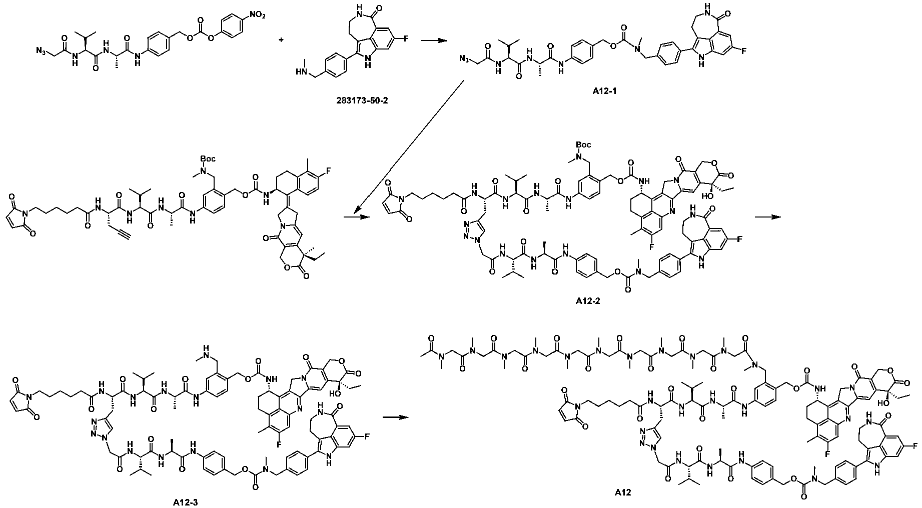

### Synthetic Steps

### Synthesis of Intermediate A12-1

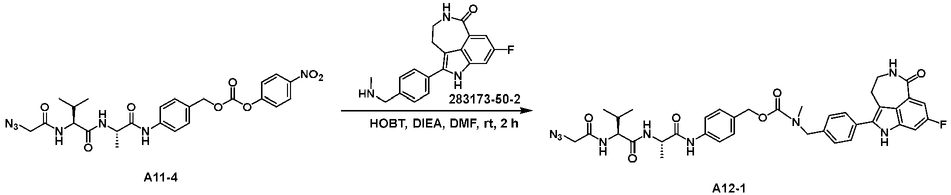

Intermediate **A11-4** (101.5 mg, 0.314 mmol, 1.0 eq) and N,N-Diisopropylethylamine (121.7 mg, 0.942 mmol, 3.0 eq) were dissolved in DMF (5 mL). After stirring until clear, **Rucaparib** (CAS 283173-50-2, 88.8 mg, 0.277 mmol, 1.0 eq) and HOBt (42.4 mg, 0.314 mmol, 1.0 eq) were added. The reaction mixture was stirred at room temperature for 2 hours. After the reaction was confirmed complete by LC-MS monitoring, water (30 mL) was added. The mixture was extracted with ethyl acetate. The combined organic phases were washed with water and brine, dried over anhydrous sodium sulfate, filtered, and concentrated. The residue was purified by silica gel column chromatography (DCM/MeOH = 93/7) to afford intermediate **A12-1** (200 mg, 88%) as a white solid. LCMS: [M+H] ⁺ = 726.3

### Synthesis of Intermediate A12-2

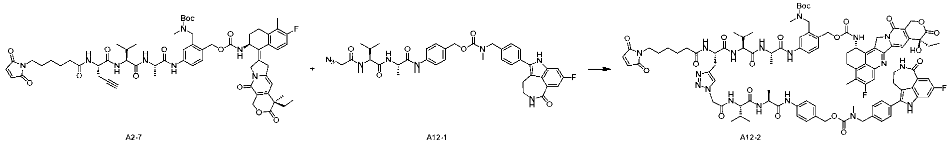

Compound **A2-7** (100 mg, 0.0842 mmol, 1.0 eq) and compound **A12-1** (61.1 mg, 0.0842 mmol, 1.0 eq) were dissolved in a mixture of methanol/dichloromethane/water (8:8:1 mL). 100 µL of an aqueous solution of copper(II) sulfate (2.08 mg, 0.0084 mmol, 0.1 eq) and 100 µL of an aqueous solution of sodium ascorbate (3.34 mg, 0.0168 mmol, 0.2 eq) were added. After addition, the reaction mixture was stirred at room temperature under nitrogen for 2 hours. After the reaction was confirmed complete by LC-MS monitoring, water (30 mL) was added. The mixture was extracted with dichloromethane. The combined organic phases were washed with brine, dried over anhydrous sodium sulfate, filtered, and concentrated to afford crude compound **A12-2** (140 mg, 78%) which was used directly in the next step. LCMS: [M+H] ⁺ = 956.0 (half peak)

### Synthesis of Intermediate A12-3

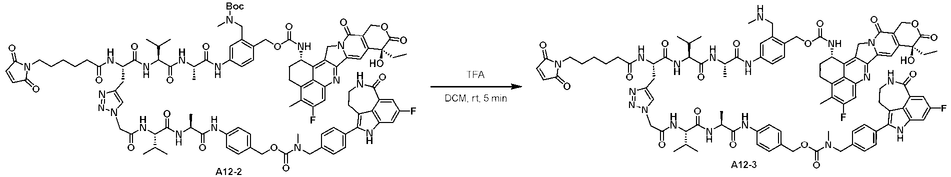

Compound **A12-2** (140 mg, 0.0732 mmol) was dissolved in dichloromethane (3 mL). Trifluoroacetic acid (1 mL) was added to the reaction mixture. The mixture was stirred at room temperature for 5 min. After the reaction was confirmed complete by LC-MS monitoring, the mixture was concentrated to afford crude compound **A12-3** (120 mg, 81.4%), which was used directly in the next step.
LCMS: [M+H]⁺ = 906.0 (half peak)

### Synthesis of Compound A12

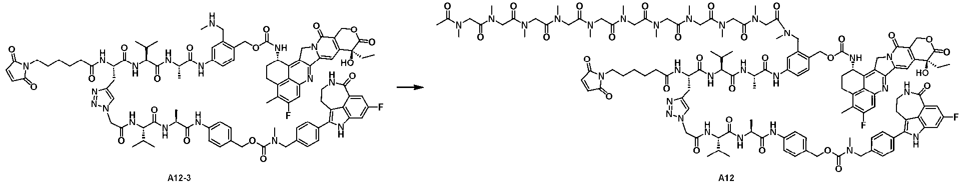

Compound **A12-3** (120 mg, 0.0663 mmol) was dissolved in DMF (3 mL). DIEA (171 mg, 1.33 mmol, 20 eq) was added and stirred for 2 minutes. Then, Ac-PSAR10-OH (43 mg, 0.0842 mmol, 1.0 eq) and HATU (23 mg, 0.0608 mmol, 1.1 eq) were added. After addition, the reaction mixture was stirred at room temperature for 0.5 hour. After the reaction was confirmed complete by LC-MS monitoring, the mixture was filtered. The filtrate was purified by preparative HPLC (mobile phase: 0.05% HCOOH/CH₃CN/H₂O) to afford Compound **A12** (30.5 mg, 24%) as a white solid. LCMS: [M+H]⁺ = 1282.6 (half peak)

### 5) Synthesis of Compound A14

### Synthetic Route:

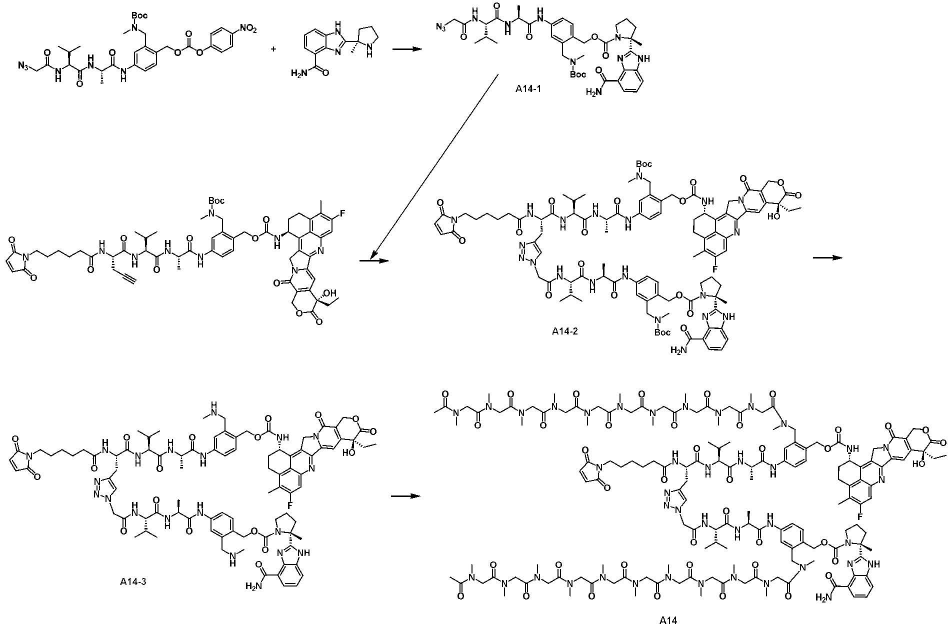

### Synthetic Steps

### Synthesis of Intermediate A14-1

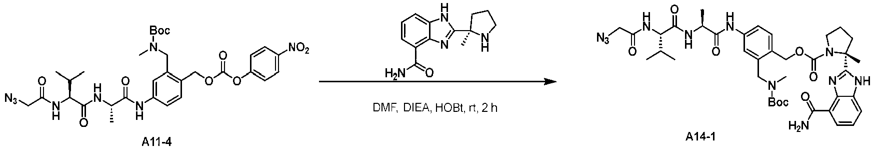

Veliparib (53.5 mg, 0.219 mmol, 1.0 eq) and N,N-Diisopropylethylamine (84.8 mg, 0.656 mmol, 3.0 eq) were dissolved in DMF (3 mL). After stirring until clear, compound A11-4 (150 mg, 0.219 mmol, 1.0 eq) and HOBt (29.6 mg, 0.277 mmol, 1.0 eq) were added. The reaction mixture was stirred at room temperature for 2 hours. After the reaction was confirmed complete by LC-MS monitoring, water (30 mL) was added. The mixture was extracted with ethyl acetate. The combined organic phases were washed with water and brine, dried over anhydrous sodium sulfate, filtered, and concentrated. The residue was purified by silica gel column chromatography (DCM/MeOH = 93/7) to afford compound A14-1 (140 mg, 81%) as a white solid. LCMS: [M+H]⁺ = 790.3

### Synthesis of Intermediate A14-2

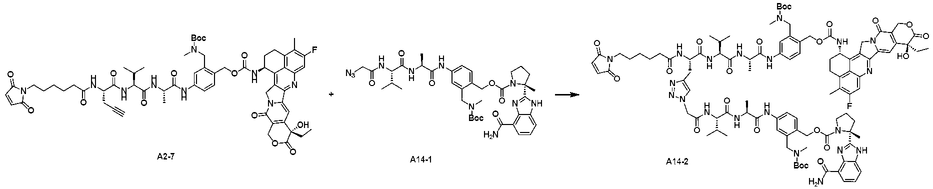

Compound **A2-7** (100 mg, 0.0842 mmol, 1.0 eq) and compound **A14-1** (66.6 mg, 0.0842 mmol, 1.0 eq) were dissolved in a mixture of MeOH/DCM/water (8/8/2 mL). Then, 100 µL of an aqueous solution of copper(II) sulfate pentahydrate (2.1 mg, 0.00842 mmol, 0.1 eq) and 100 µL of an aqueous solution of sodium ascorbate (3.34 mg, 0.01684 mmol, 0.2 eq) were added. After addition, the reaction mixture was stirred at room temperature under nitrogen for 4 hours. After the reaction was confirmed complete by LC-MS monitoring, water (30 mL) was added. The mixture was extracted with dichloromethane. The combined organic phases were washed with water and brine, dried over anhydrous sodium sulfate, filtered, and concentrated to afford compound **A14-2** (166 mg, 100%) which was used directly in the next step. LCMS: [M+H]⁺ = 988.5 (half peak)

### Synthesis of Intermediate A14-3

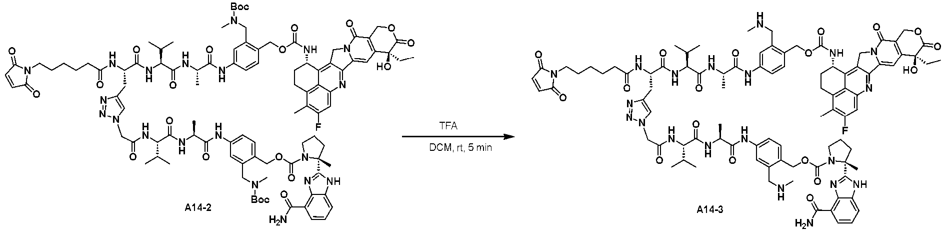

Compound **A14-2** (166 mg, 0.0842 mmol) was dissolved in DCM (6 mL). Trifluoroacetic acid (2 mL) was added to the reaction mixture. The mixture was stirred at room temperature for 5 minutes. After the reaction was confirmed complete by LC-MS monitoring, the organic phase was concentrated to afford crude compound **A14-3,** which was used directly in the next step. LCMS: [M+H]⁺ = 888.8 (half peak)

### Synthesis of Compound A14

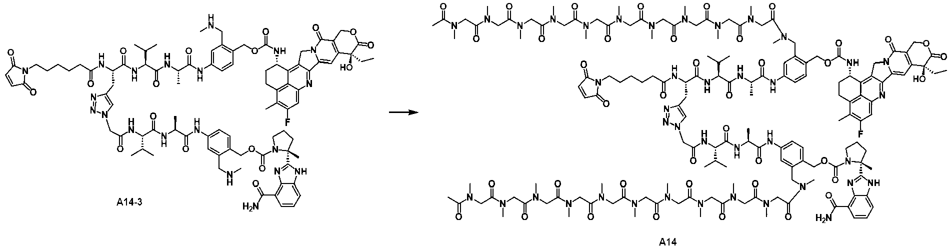

Compound **A14-3** was dissolved in DMF (3 mL). Ac-PSAR10-OH (129.8 mg, 0.1684 mmol, 2.0 eq) and N,N-Diisopropylethylamine (217.5 mg, 0.842mmol, 20.0 eq) were added. Finally, O-(7-Azabenzotriazol-1-yl)-N,N,N',N'-tetramethyluronium hexafluorophosphate (67.3 mg, 0.1768 mmol, 2.1 eq) was added. After addition, the reaction mixture was stirred at room temperature under nitrogen for 1 hour. After the reaction was confirmed complete by LC-MS monitoring, the mixture was filtered. The filtrate was purified by preparative HPLC (mobile phase: 0.05% HCOOH/CH₃CN/H₂O) to afford Compound **A14** (5.11 mg, 1.8%) as a white solid. LCMS: [M+H]⁺ = 1641.2 (half peak)

### 6) Synthesis of Compound A15

### Synthetic Route:

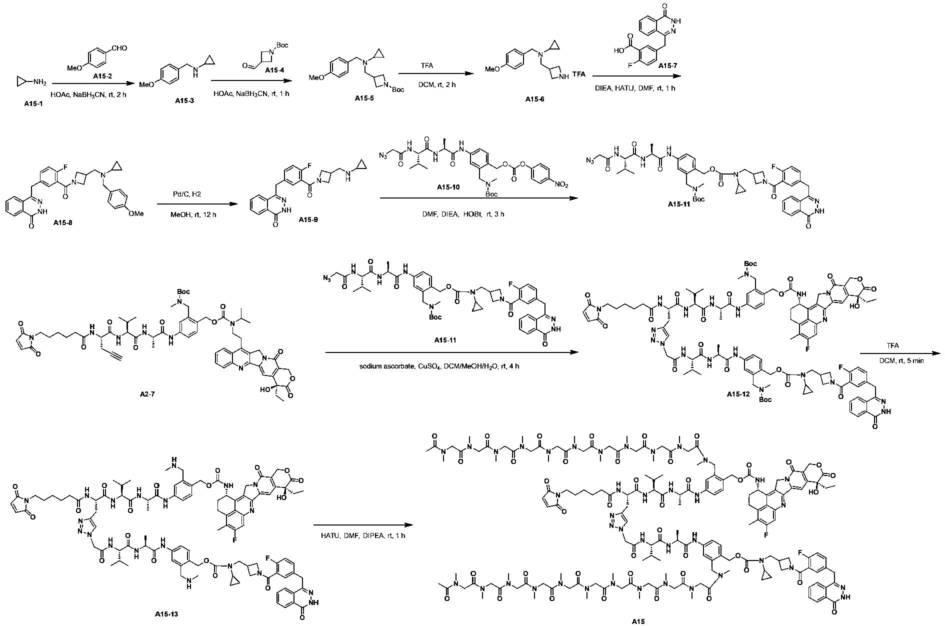

### Synthetic Steps

### Synthesis of Compound A15-3

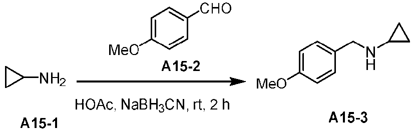

Compound A15-1 (1 g, 0.035 mol, 1.0 eq) was dissolved in MeOH (50 mL). A15-2 (4.77 g, 0.035 mol, 1 eq), HOAc (4.21 g, 0.035 mol, 1.0 eq) and NaBH₃CN (11 g, 0.175 mol, 5.0 eq) were added. After addition, the reaction mixture was stirred at room temperature for 2 hours. After the reaction was confirmed complete by LC-MS monitoring, the organic phase was concentrated. The residue was purified by silica gel column chromatography (DCM/MeOH = 92/8) to afford compound 3 (2.3 g, 33%) as a colorless oil. LCMS: [M+H]⁺ = 178.2

### Synthesis of Compound A15-5

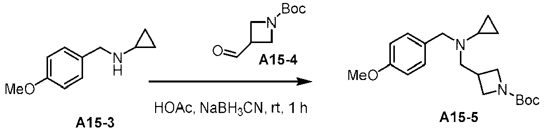

Compound A15-3 (0.96 g, 0.0054 mol, 1.0 eq), compound A15-4 (1 g, 0.0054 mol, 1.0 eq) and acetic acid (0.32 g, 0.0054 mol, 1.0 eq) were dissolved in methanol (20 mL). Sodium cyanoborohydride (1.7 g, 0.027 mol, 5.0 eq) was added. After addition, the reaction mixture was stirred at room temperature under nitrogen for 1 hour. After the reaction was confirmed complete by LC-MS monitoring, the organic phase was concentrated. The residue was purified by reverse phase column chromatography (H2O/MeCN = 80/20) to afford compound A15-5 (700 mg, 37%) as a colorless oil. LCMS: [M+H]⁺ = 347.2

### Synthesis of Compound A15-6

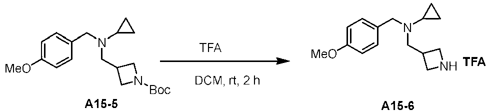

Compound A15-5 (700 mg, 1.57 mmol, 1.0 eq) was dissolved in DCM (3 mL). Trifluoroacetic acid (1 mL) was added. The mixture was stirred at room temperature for 2 hours. After the reaction was confirmed complete by LC-MS monitoring, the mixture was concentrated to afford crude compound A15-6 (500 mg, 100%), which was used directly in the next step. LCMS: [M+H]⁺ = 247.2

### Synthesis of Compound A15-8

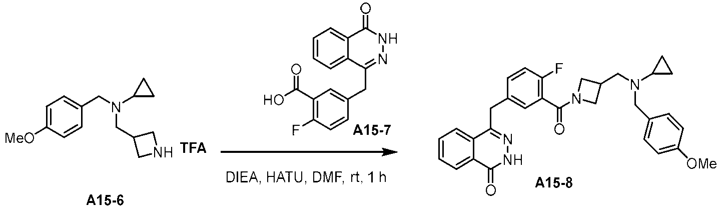

Compound A15-6 (500 mg, 2.03 mmol, 1.0 eq) and DIEA (1.311 g, 10.1 mmol, 5.0 eq) were dissolved in DMF (4 mL). After stirring for 2 minutes, compound A15-7 (605 mg, 2.03 mmol, 1.0 eq) and HATU (848 mg, 2.23 mmol, 1.1 eq) were added. The reaction mixture was stirred at room temperature for 1 hour. After the reaction was confirmed complete by LC-MS monitoring, water (50 mL) was added. The mixture was extracted with ethyl acetate. The combined organic phases were washed with water and brine, dried over anhydrous sodium sulfate, filtered, and concentrated. The residue was purified by silica gel column chromatography (DCM/MeOH = 10/1) to afford compound A15-8 (550 mg, 52%) as a yellow solid. LCMS: [M+H]⁺ = 527.2

### Synthesis of Compound A15-9

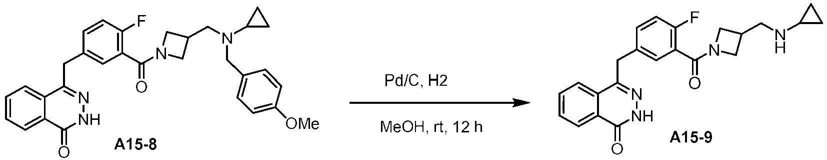

Compound A15-8 (500 mg, 0.47 mmol, 1 eq) was dissolved in methanol (10 mL). Pd/C (100 mg) and 5 drops of trifluoroacetic acid were added. The reaction vessel was purged with H₂ three times, then the mixture was stirred under a H₂ atmosphere for 12 hours. After the reaction was confirmed complete by LC-MS monitoring, the mixture was filtered through celite. The filtrate was concentrated to afford compound A15-9 (380 mg, 90%) as a white solid. 180 mg of the crude product was purified by preparative HPLC (mobile phase: 0.05% NH₃·H₂O/CH₃CN/H₂O) to afford compound A15-9 (70 mg, 39%) as a white solid. LCMS: [M+H]⁺ = 407.2

### Synthesis of Compound A15-11

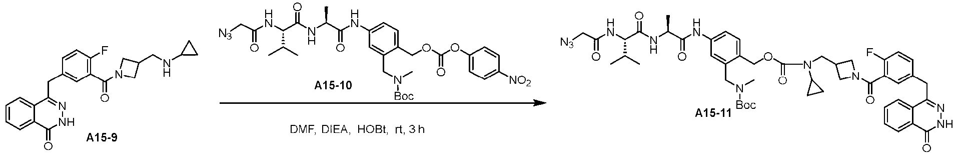

Compound A15-9 (190 mg, 0.468 mmol, 1.0 eq) and DIEA (302 mg, 2.34 mmol, 5.0 eq) were dissolved in DMF (2 mL). After stirring for 2 minutes, compound A15-10 (320 mg, 0.468 mmol, 1.0 eq) and HOBt (69 mg, 0.514 mmol, 1.1 eq) were added. The reaction mixture was stirred at room temperature for 3 hours. After the reaction was confirmed complete by LC-MS monitoring, water (50 mL) was added. The mixture was extracted with ethyl acetate. The combined organic phases were washed with water and brine, dried over anhydrous sodium sulfate, filtered, and concentrated. The residue was purified by silica gel column chromatography (DCM/MeOH = 10/1) to afford compound A15-11 (220 mg, 49%) as a white solid. LCMS: [M+H]⁺ = 974.3

### Synthesis of Compound A15-12

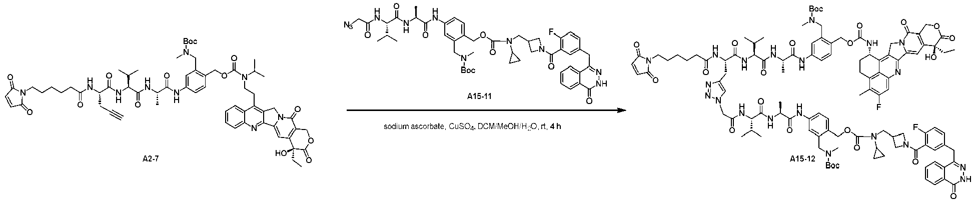

Compound A2-7 (100 mg, 0.0842 mmol, 1.0 eq) and compound A15-11 (80.44 mg,0.0842 mmol, 1.0 eq) were dissolved in a mixture of methanol/dichloromethane/water (3:3:0.5 mL). 100 µL of an aqueous solution of copper(II) sulfate (2.08 mg, 0.0084 mmol, 0.1 eq) and 100 µL of an aqueous solution of sodium ascorbate (3.34 mg, 0.0168 mmol, 0.2 eq) were added. After addition, the reaction mixture was stirred at room temperature under nitrogen for 4 hours. After the reaction was confirmed complete by LC-MS monitoring, water (30 mL) was added. The mixture was extracted with dichloromethane. The combined organic phases were washed with brine, dried over anhydrous sodium sulfate, filtered, and concentrated to afford crude compound A15-12 (170 mg, 80%) which was used directly in the next step. LCMS: [M+H]⁺ = 1069.9 (half peak)

### Synthesis of Compound A15-13

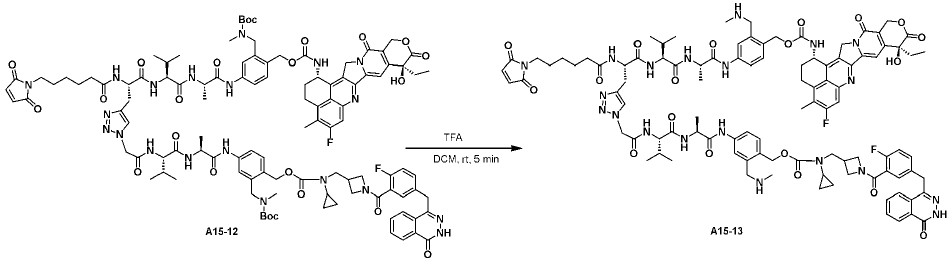

Compound A15-12 (150 mg, 0.0702 mmol) was dissolved in dichloromethane (3 mL). Trifluoroacetic acid (1 mL) was added to the reaction mixture. The mixture was stirred at room temperature for 5 min. After the reaction was confirmed complete by LC-MS monitoring, the mixture was concentrated to afford crude compound A15-13 (150 mg, 88%), which was used directly in the next step. LCMS: [M+H]⁺ = 970.0 (half peak)

### Synthesis of Compound A15

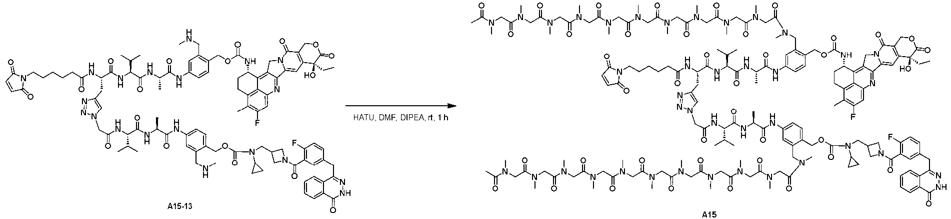

Compound A15-13 (150 mg, 0.0774 mmol) was dissolved in DMF (2 mL). N,N-Diisopropylethylamine (200 mg, 1.54 mmol, 20 eq) was added and stirred for 2 minutes. Then, Ac-PSAR10-OH (125 mg, 0.162 mmol, 2.0 eq) and HATU (64.75 mg, 0.170 mmol, 2.1 eq) were added. After addition, the reaction mixture was stirred at room temperature for 1 hour. After the reaction was confirmed complete by LC-MS monitoring, EA(20 mL) was added. The mixture was filtered, and the filter cake was dissolved in DMF. Purification by preparative HPLC (mobile phase: 0.05% HCOOH/CH₃CN/H₂O) afforded Compound **A15** (14 mg, 12%) as a white solid. LCMS: [M+H]⁺ = 1723.7 (half peak)

### 7) Synthesis of Compound A16

### Synthetic Route:

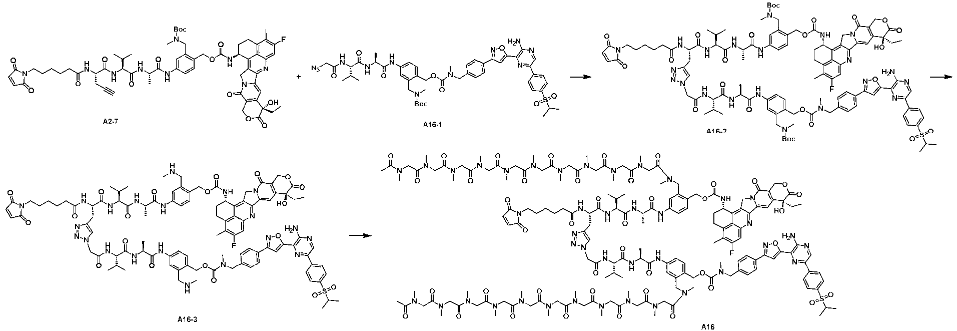

### Synthetic Steps

### Synthesis of Compound A16-2

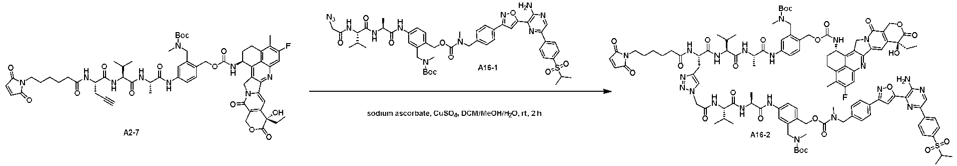

Compound **A2-7** (100 mg, 0.0842 mmol, 1.0 eq) and **A16-1** (85 mg, 0.0842 mmol, 1.0 eq) were dissolved in a mixture of methanol/dichloromethane/water (8:8:1 mL). 100 µL of an aqueous solution of copper(II) sulfate (2.08 mg, 0.0084 mmol, 0.1 eq) and 100 µL of an aqueous solution of sodium ascorbate (3.34 mg, 0.0168 mmol, 0.2 eq) were added. After addition, the reaction mixture was stirred at room temperature under nitrogen for 2 hours. After the reaction was confirmed complete by LC-MS monitoring, water (30 mL) was added. The mixture was extracted with dichloromethane. The combined organic phases were washed with brine, dried over anhydrous sodium sulfate, filtered, and concentrated to afford crude compound 3 (140 mg, 72%), which was used directly in the next step. LCMS: [M+H]⁺ = 1098.3 (half peak)

### Synthesis of Compound A16-3

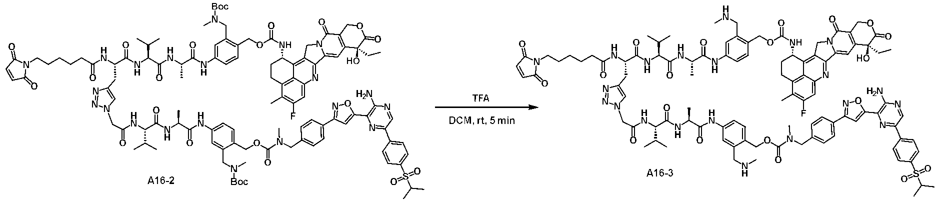

Compound **A16-2** (140 mg, 0.0637 mmol) was dissolved in dichloromethane (3 mL). Trifluoroacetic acid (1 mL) was added to the reaction mixture. The mixture was stirred at room temperature for 5 min. After the reaction was confirmed complete by LC-MS monitoring, the mixture was concentrated to afford crude compound **4** (110 mg, 72%), which was used directly in the next step.
LCMS: [M+H]⁺ = 998.1 (half peak)

### Synthesis of Compound A16

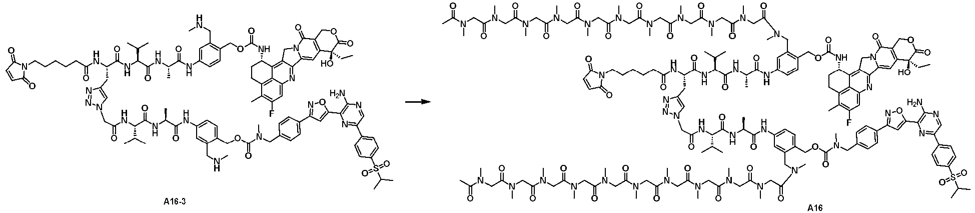

Compound **A16-3** (110 mg, 0.0551 mmol, 1.0 eq) was dissolved in DMF (3 mL). DIEA (171 mg, 1.326 mmol, 20 eq) was added and stirred for 2 minutes. Then, Ac-PSAR10-OH (85 mg, 0.110 mmol, 2.0 eq) and HATU (46 mg, 0.122 mmol, 2.1 eq) were added. After addition, the reaction mixture was stirred at room temperature for 0.5 hour. After the reaction was confirmed complete by LC-MS monitoring, the mixture was filtered. The filtrate was purified by preparative HPLC (mobile phase: 0.05% HCOOH/CH₃CN/H₂O) to afford Compound **A16** (48 mg, 43.5%) as a yellow solid. LCMS: [M+H]⁺ = 1750.7 (half peak)

### 8) Synthesis of Compound A17

### Synthetic Route:

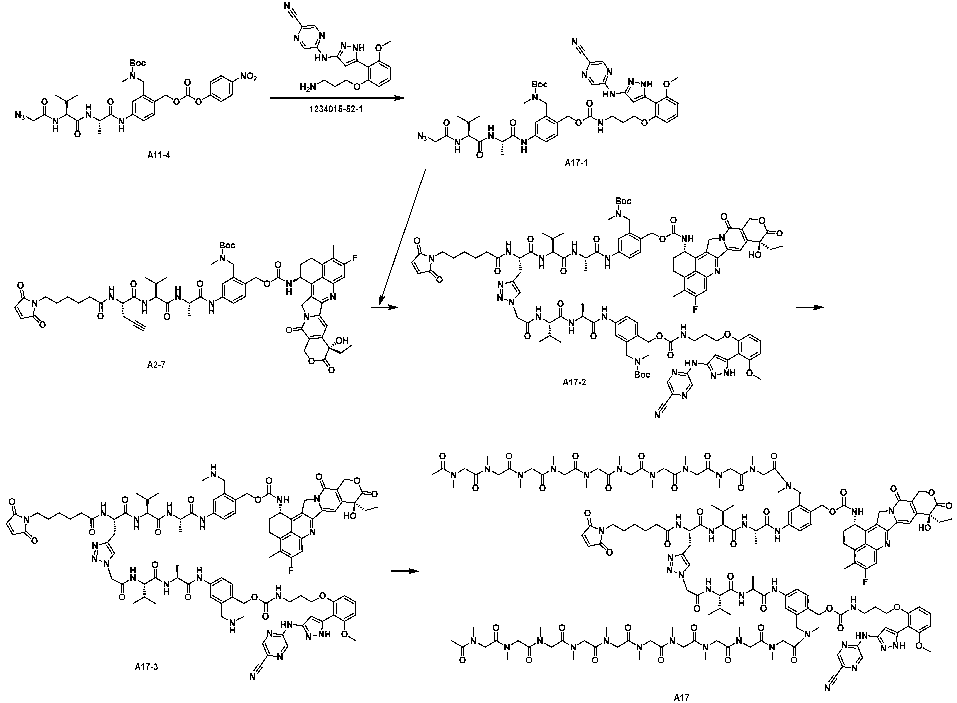

### Synthetic Steps

### Synthesis of Compound A17-1

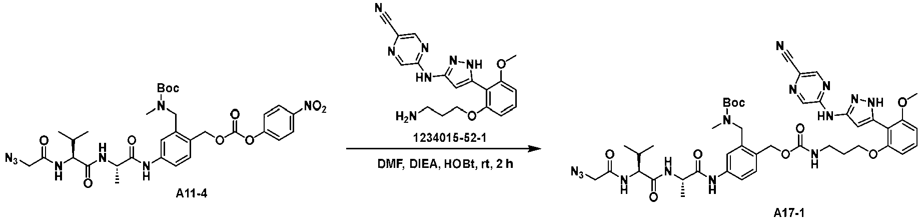

Compound **A11-4** (100 mg, 0.27 mmol, 1.0 eq) and N,N-Diisopropylethylamine (106 mg, 0.82 mmol, 3.0 eq) were dissolved in DMF (4 mL). After stirring until clear, compound **1** (188 mg, 0.27 mmol, 1.0 eq) and HOBt (37 mg, 0.28 mmol, 1.0 eq) were added. The reaction mixture was stirred at room temperature for 2 hours. After the reaction was confirmed complete by LC-MS monitoring, water (30 mL) was added. The mixture was extracted with ethyl acetate. The combined organic phases were washed with water and brine, dried over anhydrous sodium sulfate, filtered, and concentrated. The residue was purified by silica gel column chromatography (DCM/MeOH = 93/7) to afford compound **A17-1** (200 mg, 80%) as a white solid. LCMS: [M+H]⁺ = 911.4

### Synthesis of Compound A17-2

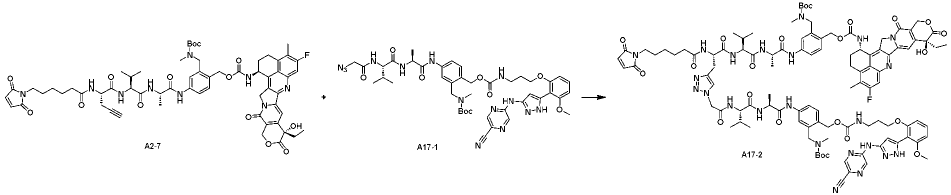

Compound **A2-7** (100 mg, 0.0842 mmol, 1.0 eq) and compound **A17-1** (77 mg, 0.0842 mmol, 1.0 eq) were dissolved in a mixture of MeOH/DCM/water (4/4/1 mL). Then, 100 µL of an aqueous solution of copper(II) sulfate pentahydrate (2.5 mg, 0.0099 mmol, 0.1 eq) and 100 µL of an aqueous solution of sodium ascorbate (3.9 mg, 0.0198 mmol, 0.2 eq) were added. After addition, the reaction mixture was stirred at 45°C under nitrogen for 8 hours. After product formation was observed by LC-MS monitoring, water (30 mL) was added. The mixture was extracted with dichloromethane. The combined organic phases were washed with water and brine, dried over anhydrous sodium sulfate, filtered, and concentrated. The residue was purified by preparative HPLC (mobile phase: 0.05% HCOOH/CH₃CN/H₂O) to afford compound **A17-2** (40 mg, 20%). LCMS: [M+H]⁺ = 1049.5 (half peak)

### Synthesis of Compound A17-3

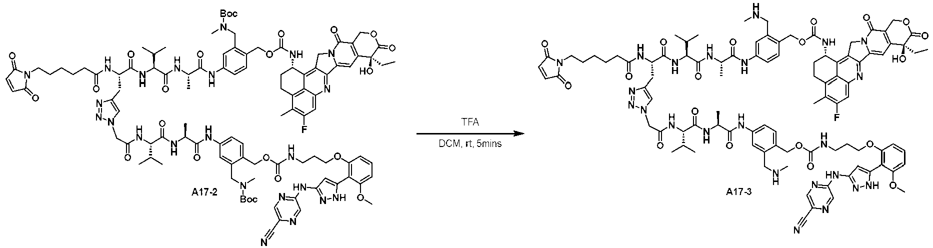

Compound **A17-2** (40 mg, 0.019 mmol, 1.0 eq) was dissolved in DCM (3 mL). Trifluoroacetic acid (1 mL) was added to the reaction mixture. The mixture was stirred at room temperature for 5 minutes. After the reaction was confirmed complete by LC-MS monitoring, the organic phase was concentrated to afford crude compound **A17-3,** which was used directly in the next step. LCMS: [M+H]⁺ = 999.6 (half peak)

### Synthesis of Compound A17

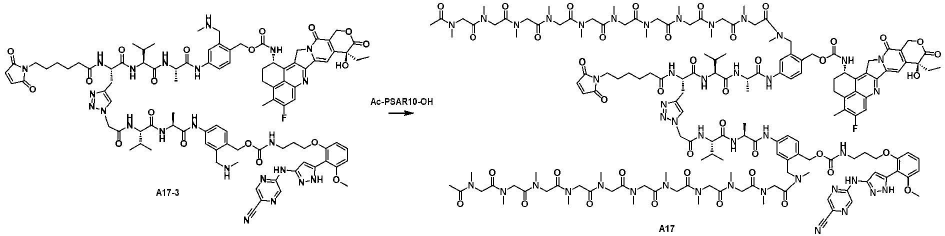

The compound **A17-3** from the previous step was dissolved in DMF (3 mL). Compound **Ac-PSAR10-OH** (29 mg, 0.038 mmol, 2.0 eq) and N,N-Diisopropylethylamine (49 mg, 0.38 mmol, 20.0 eq) were added. Finally, O-(7-Azabenzotriazol-1-yl)-N,N,N',N'-tetramethyluronium hexafluorophosphate (79 mg, 0.040 mmol, 2.1 eq) was added under a nitrogen atmosphere. After addition, the reaction mixture was stirred at room temperature under nitrogen for 1 hour. After the reaction was confirmed complete by LC-MS monitoring, the mixture was filtered. The filtrate was purified by preparative HPLC (mobile phase: 0.05% HCOOH/CH₃CN/H₂O) to afford Compound **A17** (4.5 mg, 10%) as a white solid. LCMS: [M+H]⁺ = 1701.7 (half peak)

### 9) Synthesis of Compound A21

### Synthetic Route:

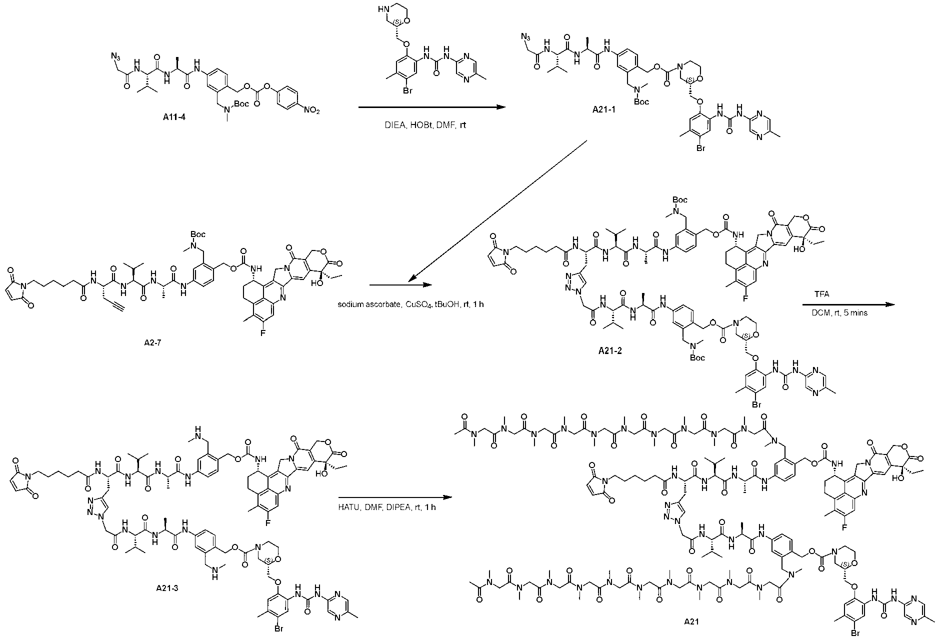

### Synthesis of Compound A21-1

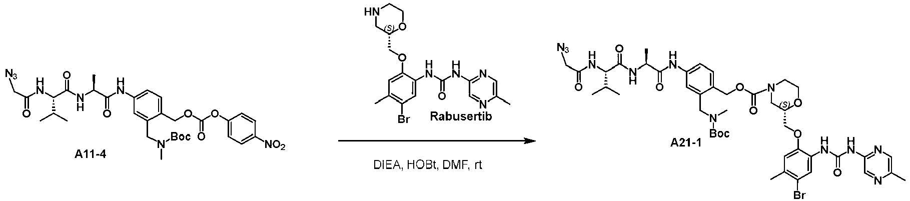

Compound **Rabusertib** (100 mg, 0.229 mmol, 1.0 eq) was dissolved in DMF (2 mL). DIEA (88.9 mg, 0.688 mmol, 3 eq) was added and stirred for 2 minutes until completely clear. Then, compound A11-4 (154 mg, 0.229 mmol, 1.0 eq) and HOBt (31.0 mg, 0.229 mmol, 1.0 eq) were added. After addition, the reaction mixture was stirred at room temperature for 1 hour. After the reaction was confirmed complete by LC-MS monitoring, water (30 mL) was added. The mixture was extracted with ethyl acetate. The combined organic phases were washed with brine, dried over anhydrous sodium sulfate, filtered, and concentrated to afford compound A21-1 (200 mg, 39%) as a yellow solid. LCMS: [M+H]⁺ = 981.2

### Synthesis of Compound 5

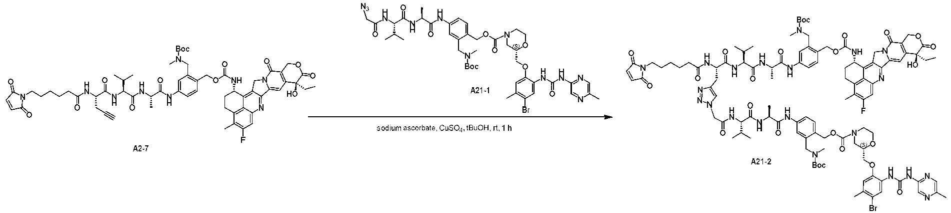

Compound **A2-7** (100 mg, 0.0842 mmol, 1.0 eq) and compound **A21-1** (82.8 mg, 0.0842 mmol, 1.0 eq) were dissolved in a mixture of methanol/dichloromethane/water (3:3:0.5 mL). 100 µL of an aqueous solution of copper(II) sulfate (2.08 mg, 0.0084 mmol, 0.1 eq) and 100 µL of an aqueous solution of sodium ascorbate (3.34 mg, 0.0168 mmol, 0.2 eq) were added. After addition, the reaction mixture was stirred at room temperature under nitrogen for 1 hour. After the reaction was confirmed complete by LC-MS monitoring, water (30 mL) was added. The mixture was extracted with dichloromethane. The combined organic phases were washed with brine, dried over anhydrous sodium sulfate, filtered, and concentrated to afford crude compound **A21-2** (150 mg, 82%) which was used directly in the next step.
LCMS: [M+H]⁺ = 1084.7 (half peak)

### Synthesis of Compound 6

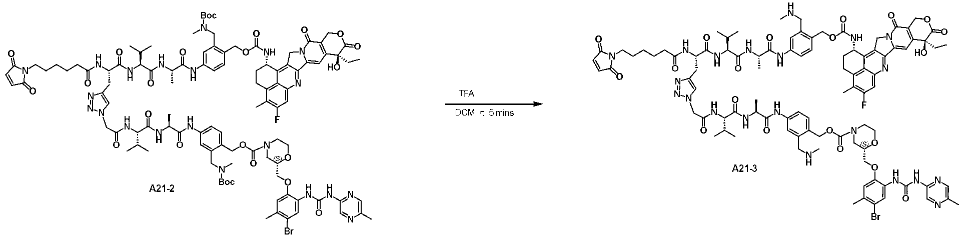

Compound **A21-2** (150 mg, 0.0691 mmol) was dissolved in dichloromethane (3 mL). Trifluoroacetic acid (1 mL) was added to the reaction mixture. The mixture was stirred at room temperature for 5 min. After the reaction was confirmed complete by LC-MS monitoring, the mixture was concentrated to afford crude compound **A21-3** (130 mg, 98%), which was used directly in the next step. LCMS: [M+H]⁺ = 984.8 (half peak)

### Synthesis of Compound A21

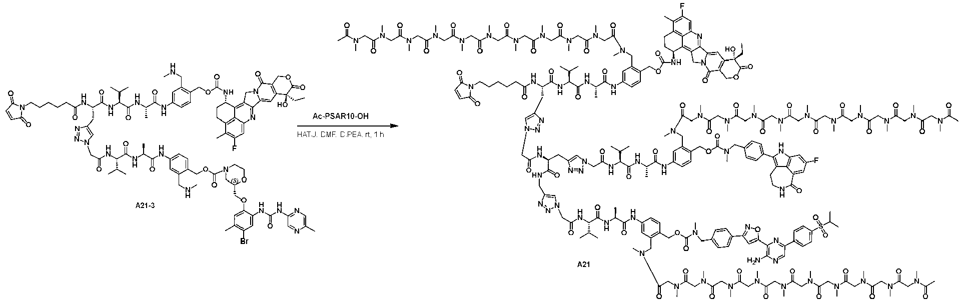

Compound **A21-3** (130 mg, 0.0661 mmol) was dissolved in DMF (2 mL). DIEA (170 mg, 1.32 mmol, 20 eq) was added and stirred for 2 minutes. Then, Ac-PSAR10-OH (102 mg, 0.132 mmol, 2.0 eq) and HATU (51.8 mg, 0.139 mmol, 2.1 eq) were added. After addition, the reaction mixture was stirred at room temperature for 1 hour. After the reaction was confirmed complete by LC-MS monitoring, ethyl acetate (20 mL) was added. The mixture was filtered, and the filter cake was dissolved in DMF. Purification by preparative HPLC (mobile phase: 0.05% HCOOH/CH₃CN/H₂O) afforded Compound **A21** (32 mg, 25%) as a white solid. LCMS: [M+Na]⁺ = 1759.7 (half peak)

### 10) Synthesis of Compound A23

### Synthetic Route:

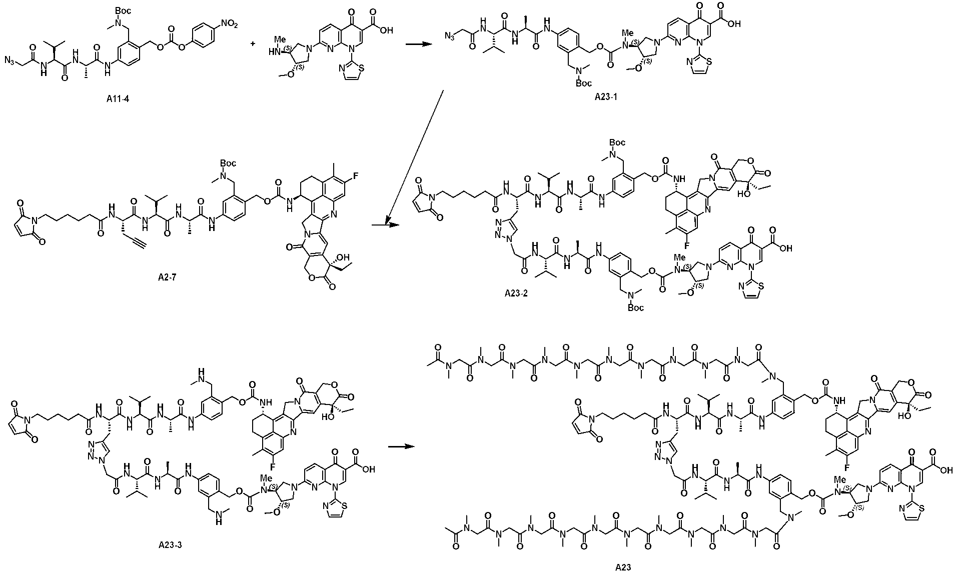

### Synthetic Steps

### Synthesis of Compound A23-1

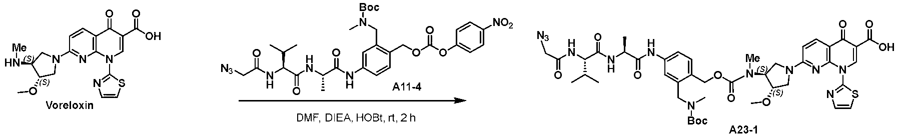

Compound Voreloxin (100 mg, 0.241 mmol, 1.0 eq) was dissolved in DMF (2 mL). DIEA (155 mg, 1.20 mmol, 5 eq) was added and stirred for 2 minutes until completely clear. Then, compound **A11-4** (165 mg, 0.241 mmol, 1.0 eq) and HOBt (32.52 mg, 0.241 mmol, 1.0 eq) were added. After addition, the reaction mixture was stirred at room temperature for 1 hour. After the reaction was confirmed complete by LC-MS monitoring, ethyl acetate was added, precipitating a solid. The mixture was filtered, and the filter cake was dried to afford compound A23-1 (90 mg, 39%) as a yellow solid.
LCMS: [M+H]⁺ = 947.3 (half peak)

### Synthesis of Compound A23-2

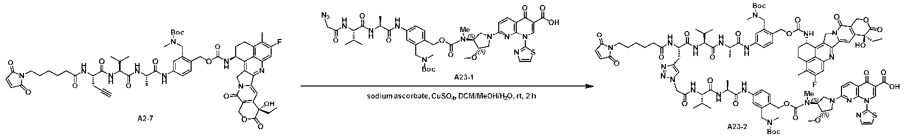

Compound **A2-7** (100 mg, 0.0842 mmol, 1.0 eq) and compound **A23-1** (79.8 mg, 0.0842 mmol, 1.0 eq) were dissolved in a mixture of methanol/dichloromethane/water (8:8:1 mL). 100 µL of an aqueous solution of copper(II) sulfate (2.08 mg, 0.0084 mmol, 0.1 eq) and 100 µL of an aqueous solution of sodium ascorbate (3.34 mg, 0.0168 mmol, 0.2 eq) were added. After addition, the reaction mixture was stirred at room temperature under nitrogen for 2 hours. After the reaction was confirmed complete by LC-MS monitoring, water (30 mL) was added. The mixture was extracted with dichloromethane. The combined organic phases were washed with brine, dried over anhydrous sodium sulfate, filtered, and concentrated to afford crude compound **A23-2** (150 mg, 80%) which was used directly in the next step. LCMS: [M+H]⁺ = 1067.1 (half peak)

### Synthesis of Compound A23-3

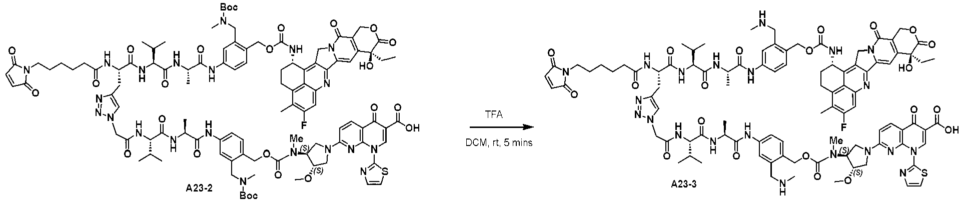

Compound **A23-2** (150 mg, 0.0702 mmol) was dissolved in dichloromethane (3 mL). Trifluoroacetic acid (1 mL) was added to the reaction mixture. The mixture was stirred at room temperature for 5 min. After the reaction was confirmed complete by LC-MS monitoring, the mixture was concentrated to afford crude compound **A23-3** (120 mg, 88%), which was used directly in the next step. LCMS: [M+H]⁺ = 966.9 (half peak)

### Synthesis of Compound A23

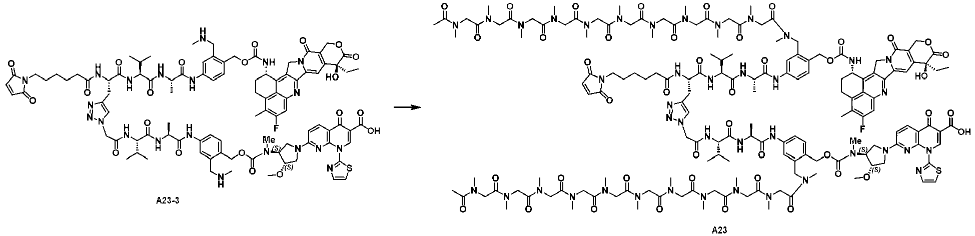

Compound **A23-3** (120 mg, 0.0621 mmol) was dissolved in DMF (2 mL). DIEA (161 mg, 1.24 mmol, 20 eq) was added and stirred for 2 minutes. Then, Ac-PSAR10-OH (100 mg, 0.131 mmol, 2.0 eq) and HATU (51.9 mg, 0.137 mmol, 2.1 eq) were added. After addition, the reaction mixture was stirred at room temperature for 1 hour. After the reaction was confirmed complete by LC-MS monitoring, the mixture was filtered. The filtrate was purified by preparative HPLC (mobile phase: 0.05% HCOOH/CH₃CN/H₂O) to afford Compound **A23** (15 mg, 12%) as a white solid. LCMS: [M+H]⁺ = 1719.9 (half peak)

### 11) Synthesis of Compound A33

### Synthetic Route:

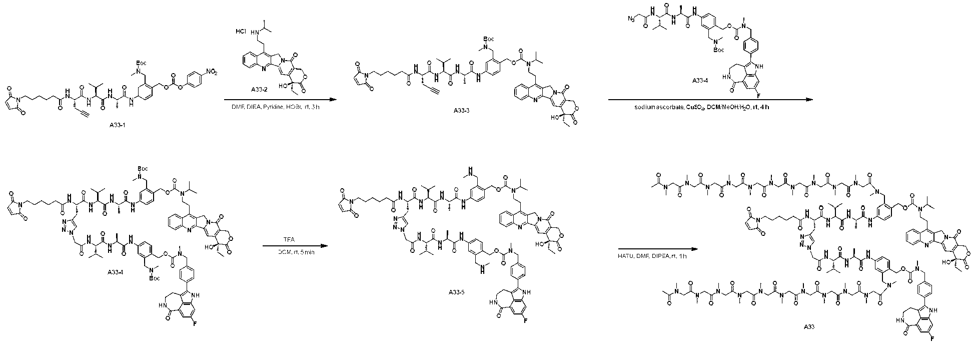

### Synthetic Steps:

### Synthesis of Compound A33-3

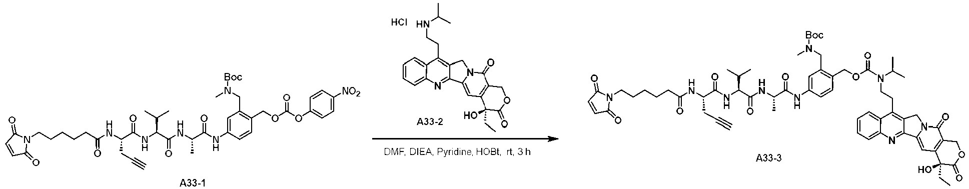

Compound A33-2 (purchased from Shanghai Haoyuan Chem, 200 mg, 0.426 mmol, 1.0 eq) and N,N-Diisopropylethylamine (165.0 mg, 1.277 mmol, 3.0 eq) were dissolved in DMF (5 mL). After stirring until clear, compound A33-1 (379.2 mg, 0.426 mmol, 1.0 eq) and HOBt (57.5 mg, 0.426 mmol, 1.0 eq) were added. After addition, the reaction mixture was stirred at room temperature for 2 hours. After the reaction was confirmed complete by LC-MS monitoring, ethyl acetate (50 mL) and water (30 mL) were added. The layers were separated, and the aqueous layer was extracted with ethyl acetate. The combined organic phases were washed with water and brine, dried over anhydrous sodium sulfate, filtered, and concentrated. The residue was purified by silica gel column chromatography (DCM/MeOH = 93/7) to afford compound **A33-3** (360 mg, 71%) as a yellow solid. LCMS: [M+H]⁺ = 1184.5

### Synthesis of Compound A33-4

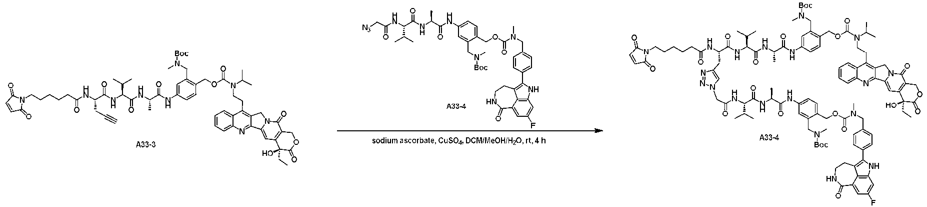

Compound A33-3 (250 mg, 0.211 mmol, 1.0 eq) and compound A33-4 (183.5 mg, 0.211 mmol, 1.0 eq) were dissolved in a mixture of MeOH/DCM/water (8/8/2 mL). Then, 100 µL of an aqueous solution of copper(II) sulfate pentahydrate (5.27 mg, 0.0211 mmol, 0.1 eq) and 100 µL of an aqueous solution of sodium ascorbate (8.36 mg, 0.0422 mmol, 0.2 eq) were added. After addition, the reaction mixture was stirred at room temperature under nitrogen for 4 hours. After the reaction was confirmed complete by LC-MS monitoring, water (30 mL) was added. The mixture was extracted multiple times with dichloromethane. The combined organic phases were dried over anhydrous sodium sulfate, filtered, and concentrated to afford compound A33-4 (420 mg, 97%) which was used directly in the next step. LCMS: [M+H]⁺ = 1027.4 (half peak)

### Synthesis of Compound A33-5

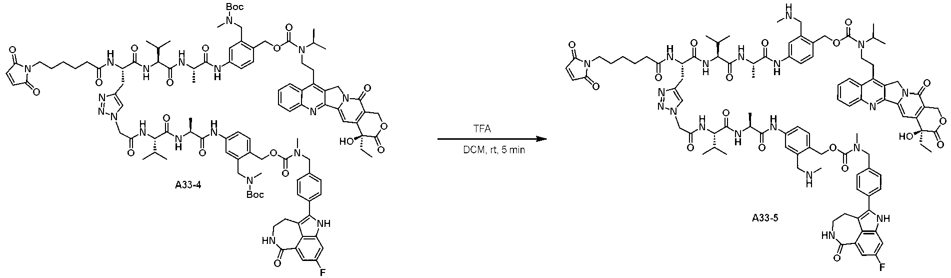

Compound A33-4 (420 mg, 0.194 mmol) was dissolved in DCM (12 mL). Trifluoroacetic acid (4 mL) was added to the reaction mixture. The mixture was stirred at room temperature for 5 minutes. After the reaction was confirmed complete by LC-MS monitoring, the organic phase was concentrated to afford crude compound A33-5, which was used directly in the next step. LCMS: [M+H]⁺ = 927.0 (half peak)

### Synthesis of Compound A33

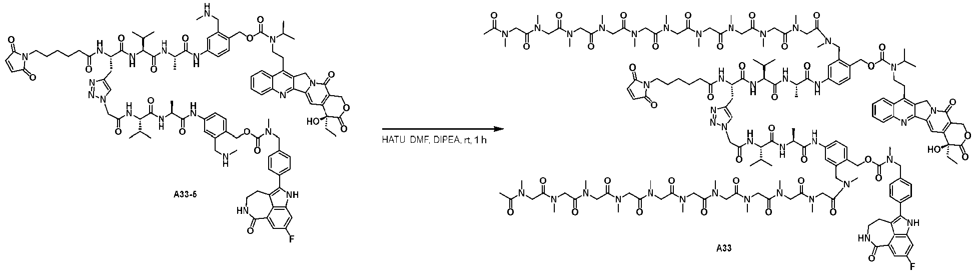

The compound **A33-5** from the previous step was dissolved in DMF (8 mL). Compound Ac-PSAR10-OH (299.2 mg, 0.388 mmol, 2.0 eq) and N,N-Diisopropylethylamine (501.3 mg, 3.88 mmol, 20.0 eq) were added. Finally, O-(7-Azabenzotriazol-1-yl)-N,N,N',N'-tetramethyluronium hexafluorophosphate (155.1 mg, 0.408 mmol, 2.1 eq) was added. After addition, the reaction mixture was stirred at room temperature under nitrogen for 1 hour. After the reaction was confirmed complete by LC-MS monitoring, ethyl acetate (80 mL) was added. The mixture was filtered, and the filter cake was dissolved in DMF. Purification by preparative HPLC (mobile phase: 0.05% HCOOH/CH₃CN/H₂O) afforded Compound **A33** (134 mg, 20.5%) as a white solid. LCMS: [M+H]⁺ = 1679.7 (half peak)

### 12) Synthesis of Compound A58

### Synthetic Route:

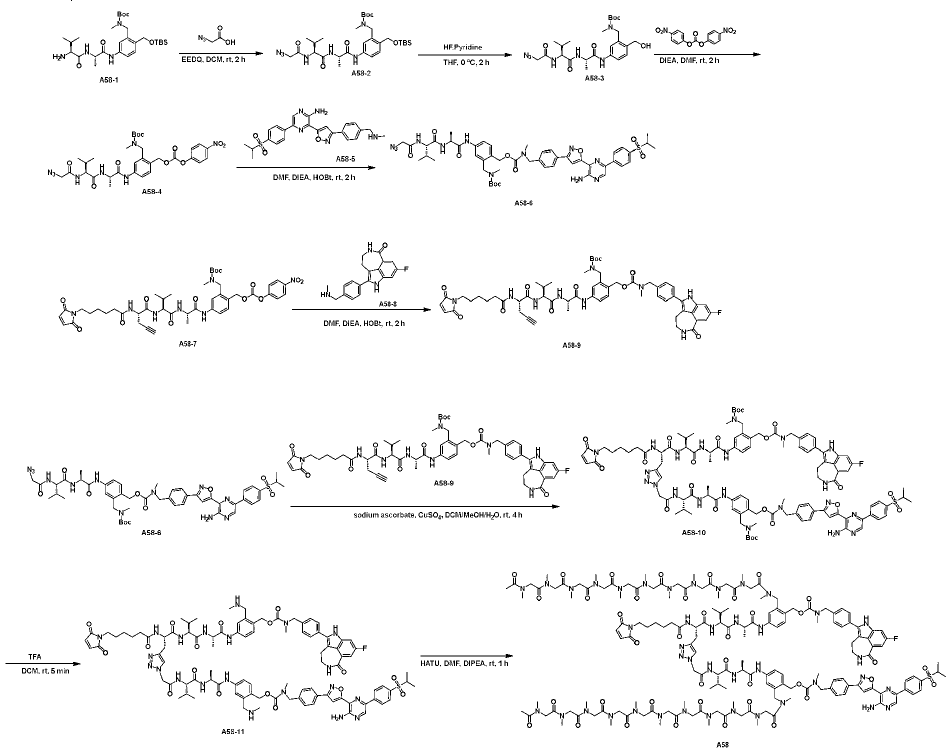

### Synthetic Steps

### Synthesis of Compound A58-2

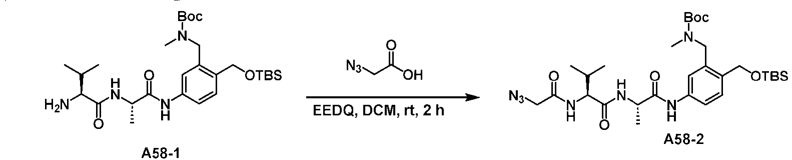

Compound A58-1 (1.0 g, 1.8 mmol, 1.0 eq) was dissolved in dichloromethane (30 mL). Azidoacetic acid (220 mg, 2.16 mmol, 1.2 eq) and 2-Ethoxy-1-ethoxycarbonyl-1,2-dihydroquinoline (890 mg, 3.6 mmol, 2.0 eq) were added to the reaction mixture. The mixture was stirred at room temperature for 2 hours. After the reaction was confirmed complete by LC-MS monitoring, the mixture was concentrated directly. The residue was purified by silica gel column chromatography (PE/EA = 1/3) to afford compound A58-2 (1.1 g, 94%) as a white solid. LCMS: [M+Na]⁺ = 656.4

### Synthesis of Compound A58-3

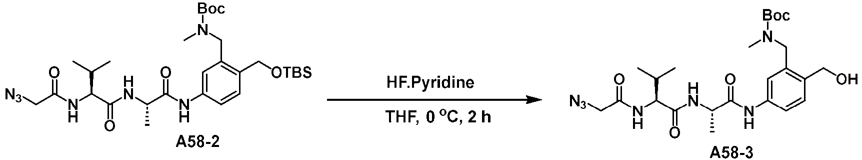

Compound A58-2 (1.1 g, 3.6 mmol, 1.0 eq) was dissolved in THF (10 mL). Under an ice bath, a solution of hydrogen fluoride/pyridine (4.4 g, 28.8 mmol, 8 eq, 65% w/w) was added. The reaction mixture was stirred at room temperature for 2 hours. After the reaction was confirmed complete by LC-MS monitoring, the mixture was diluted with ethyl acetate and washed with water, saturated sodium bicarbonate solution, and brine. The organic phase was dried over anhydrous sodium sulfate, filtered, and concentrated to afford compound A58-3 (0.8 g, 88%) as a white solid. LCMS: [M+Na]⁺ = 542.3

### Synthesis of Compound A58-4

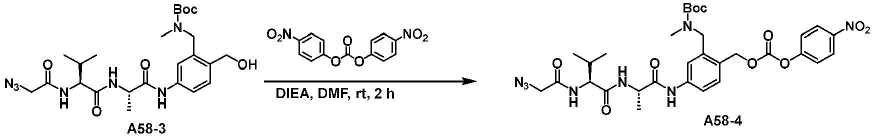

Compound A58-3 (670 mg, 1.29 mmol, 1.0 eq) and N,N-Diisopropylethylamine (499 mg, 3.86 mmol, 3.0 eq) were dissolved in DMF (5 mL). After stirring for a while, bis(4-nitrophenyl) carbonate (743 mg, 2.57 mmol, 2.0 eq) was added. The reaction mixture was stirred at room temperature for 2 hours. After the reaction was confirmed complete by LC-MS monitoring, the mixture was diluted with ethyl acetate, washed with water and brine. The organic phase was dried over anhydrous sodium sulfate, filtered, and concentrated. The residue was purified by silica gel column chromatography (DCM/MeOH = 93/7) to afford compound **A58-4** (800 mg, 91%) as a yellow solid. LCMS: [M+Na]⁺ = 707.3

### Synthesis of Compound A58-6

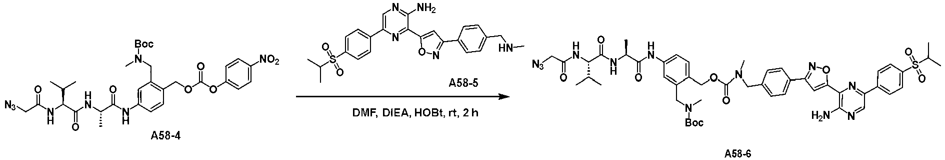

Compound A58-4 (147.9 mg, 0.22 mmol, 1.0 eq) and N,N-Diisopropylethylamine (83.6 mg, 0.647 mmol, 3.0 eq) were dissolved in DMF (4 mL). After stirring until clear, compound A58-5 (purchased from Shanghai Haoyuan Chem, 100 mg, 0.22 mmol, 1.0 eq) and HOBt (29.1 mg, 0.216 mmol, 1.0 eq) were added. The reaction mixture was stirred at room temperature for 2 hours. After the reaction was confirmed complete by LC-MS monitoring, water (30 mL) was added. The mixture was extracted with ethyl acetate. The combined organic phases were washed with water and brine, dried over anhydrous sodium sulfate, filtered, and concentrated. The residue was purified by silica gel column chromatography (DCM/MeOH = 93/7) to afford compound A58-6 (180 mg, 83%) as a white solid. LCMS: [M+Na]⁺ = 1031.3

### Synthesis of Compound A58-9

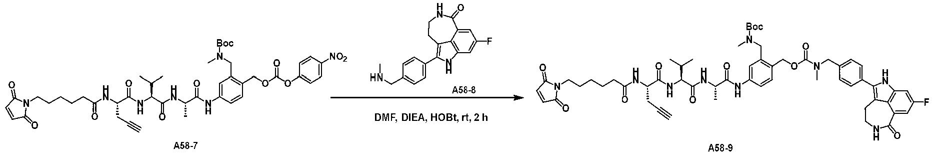

Compound A58-7 (200 mg, 0.22 mmol, 1.0 eq) and N,N-Diisopropylethylamine (87.0 mg, 0.673 mmol, 3.0 eq) were dissolved in DMF (5 mL). After stirring until clear, compound A58-8 (purchased from Shanghai Haoyuan Chem, 72.6 mg, 0.22 mmol, 1.0 eq) and HOBt (30.3 mg, 0.22 mmol, 1.0 eq) were added. The reaction mixture was stirred at room temperature for 2 hours. After the reaction was confirmed complete by LC-MS monitoring, water (30 mL) was added. The mixture was extracted with ethyl acetate. The combined organic phases were washed with water and brine, dried over anhydrous sodium sulfate, filtered, and concentrated. The residue was purified by silica gel column chromatography (DCM/MeOH = 93/7) to afford compound A58-9 (180 mg, 83%) as a white solid. LCMS: [M+H]⁺ = 1074.4

### Synthesis of Compound A58-10

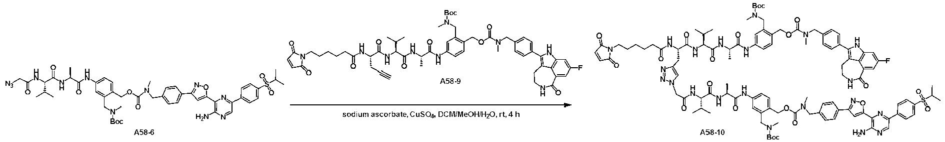

Compound A58-6 (100 mg, 0.099 mmol, 1.0 eq) and compound A58-9 (106.5 mg, 0.099 mmol, 1.0 eq) were dissolved in a mixture of MeOH/DCM/water (10/10/2.5 mL). Then, 100 µL of an aqueous solution of copper(II) sulfate pentahydrate (2.5 mg, 0.0099 mmol, 0.1 eq) and 100 µL of an aqueous solution of sodium ascorbate (3.9 mg, 0.0198 mmol, 0.2 eq) were added. After addition, the reaction mixture was stirred at room temperature under nitrogen for 4 hours. After the reaction was confirmed complete by LC-MS monitoring, water (30 mL) was added. The mixture was extracted with dichloromethane. The combined organic phases were washed with water and brine, dried over anhydrous sodium sulfate, filtered, and concentrated to afford compound A58-10 (206 mg, 100%), which was used directly in the next step. LCMS: [M+H]⁺ = 1042.6 (half peak)

### Synthesis of Compound A58-11

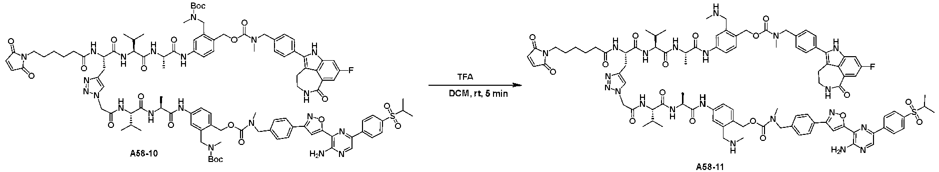

Compound A58-10 (206 mg, 0.099 mmol, 1.0 eq) was dissolved in DCM (6 mL). Trifluoroacetic acid (2 mL) was added to the reaction mixture. The mixture was stirred at room temperature for 5 minutes. After the reaction was confirmed complete by LC-MS monitoring, the organic phase was concentrated to afford crude compound A58-11, which was used directly in the next step. LCMS: [M+H]⁺ = 942.4 (half peak)

### Synthesis of Compound A58

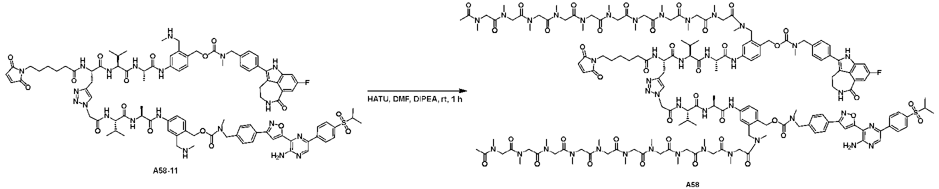

The compound A58-11 from the previous step was dissolved in DMF (3 mL). Compound Ac-PSAR10-OH (153 mg, 0.198 mmol, 2.0 eq) and N,N-Diisopropylethylamine (256 mg, 1.98 mmol, 20.0 eq) were added. Finally, O-(7-Azabenzotriazol-1-yl)-N,N,N',N'-tetramethyluronium hexafluorophosphate (79 mg, 0.208 mmol, 2.1 eq) was added under a nitrogen atmosphere. After addition, the reaction mixture was stirred at room temperature under nitrogen for 1 hour. After the reaction was confirmed complete by LC-MS monitoring, the mixture was filtered. The filtrate was purified by preparative HPLC (mobile phase: 0.05% HCOOH/CH₃CN/H₂O) to afford Compound A58 (32.8 mg, 9.8%) as a yellow solid. LCMS: [M+H]⁺ = 1695.2 (half peak)

### 13) Synthesis of Compound A68

### Synthetic Route:

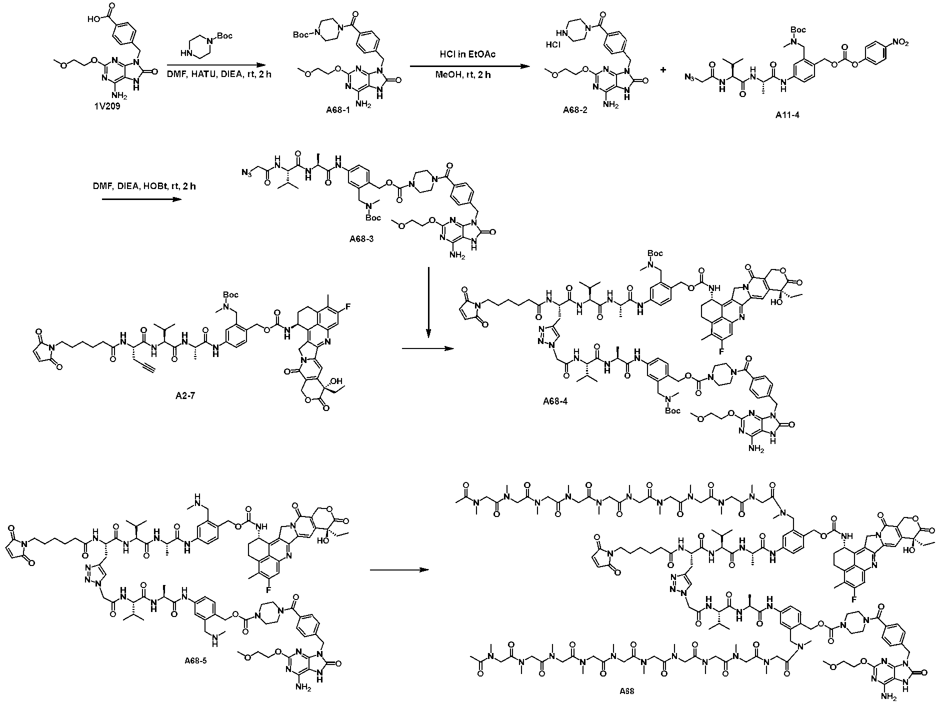

### Synthetic Steps:

### Synthesis of Compound A68-1

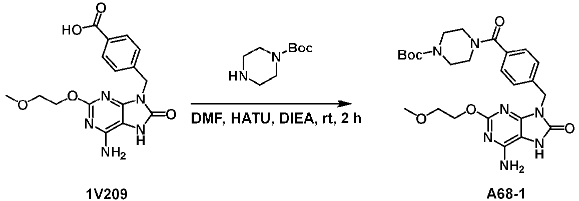

Compound **1V209** (100 mg, 0.278 mmol, 1.0 eq), Boc-piperazine (156 mg, 0.835 mmol, 3.0 eq) and N,N-Diisopropylethylamine (108 mg, 0.835 mmol, 3.0 eq) were dissolved in DMF (3 mL). Subsequently, O-(7-Azabenzotriazol-1-yl)-N,N,N',N'-tetramethyluronium hexafluorophosphate (159 mg, 0.418 mmol, 1.5 eq) was added. After addition, the reaction mixture was stirred at room temperature for 2 hours. After the reaction was confirmed complete by LC-MS monitoring, water (30 mL) was added. The mixture was extracted with ethyl acetate. The combined organic phases were washed with water and brine, dried over anhydrous sodium sulfate, filtered, and concentrated. The residue was purified by silica gel column chromatography (DCM/MeOH = 10/1) to afford compound **A68-1** (140 mg, 95%) as a white solid. LCMS: [M+H]⁺ = 528.3

### Synthesis of Compound A68-2

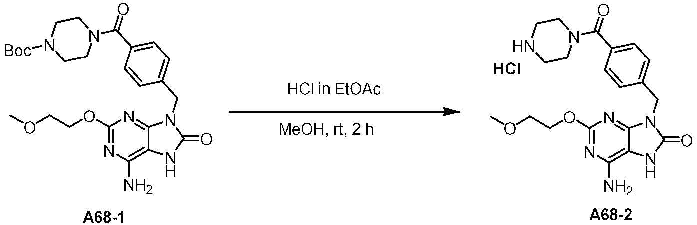

Compound **A68-1** (150 mg, 0.284 mmol, 1.0 eq) was dissolved in methanol (2 mL). A 4.0 M HCl in ethyl acetate solution (2 mL) was added to the reaction mixture. The mixture was stirred at room temperature for 2 hours. After the reaction was confirmed complete by LC-MS monitoring, the organic phase was concentrated to afford crude compound **A68-2** (120 mg, 91%).

LCMS: [M+H]⁺ = 428.1

### Synthesis of Compound A68-3

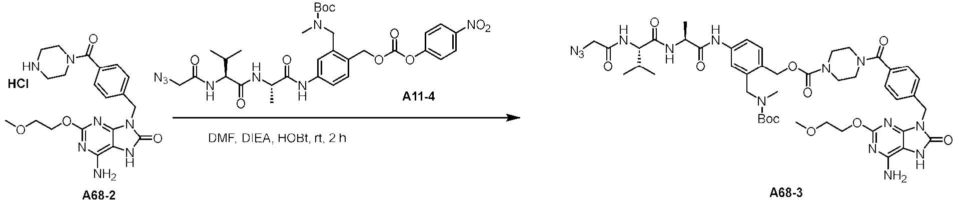

Compound **A68-2** (120 mg, 0.259 mmol, 1.0 eq) and N,N-Diisopropylethylamine (167 mg, 1.294 mmol, 5.0 eq) were dissolved in DMF (4 mL). After stirring until clear, compound **A11-4** (196 mg, 0.84 mmol, 1.1 eq) and HOBt (35 mg, 0.259 mmol, 1.0 eq) were added. The reaction mixture was stirred at room temperature for 2 hours. After the reaction was confirmed complete by LC-MS monitoring, water (30 mL) was added. The mixture was extracted with ethyl acetate. The combined organic phases were washed with water and brine, dried over anhydrous sodium sulfate, filtered, and concentrated. The residue was purified by silica gel column chromatography (DCM/MeOH = 92/8) to afford compound **A68-3** (140 mg, 56%) as a white solid. LCMS: [M+H]⁺ = 973.3

### Synthesis of Compound A68-4

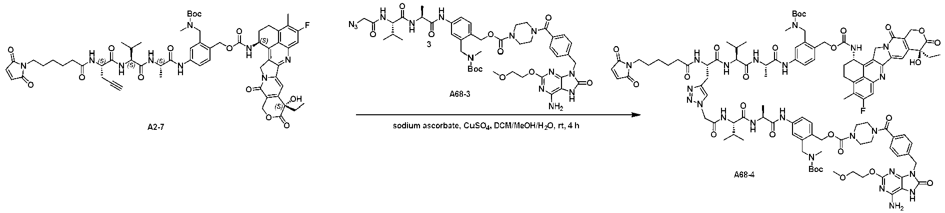

Compound **A2-7** (100 mg, 0.084 mmol, 1.0 eq) and compound **A68-3** (82 mg, 0.084 mmol, 1.0 eq) were dissolved in a mixture of MeOH/DCM/water (4/4/0.5 mL). Then, 100 µL of an aqueous solution of copper(II) sulfate pentahydrate (2.1 mg, 0.0084 mmol, 0.1 eq) and 100 µL of an aqueous solution of sodium ascorbate (3.3 mg, 0.0168 mmol, 0.2 eq) were added. After addition, the reaction mixture was stirred at room temperature under nitrogen for 4 hours. After product formation was observed by LC-MS monitoring, water (30 mL) was added. The mixture was extracted with dichloromethane. The combined organic phases were washed with water and brine, dried over anhydrous sodium sulfate, filtered, and concentrated to afford crude compound **A68-4** (180 mg, 99%). LCMS: [M+H]⁺ = 1080.5 (half peak)

### Synthesis of Compound A68-5

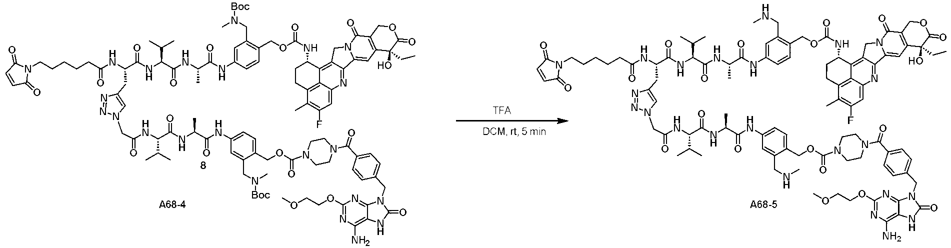

Compound **A68-4** (90 mg, 0.0417 mmol, 1.0 eq) was dissolved in DCM (3 mL). Trifluoroacetic acid (1 mL) was added to the reaction mixture. The mixture was stirred at room temperature for 5 minutes. After the reaction was confirmed complete by LC-MS monitoring, the organic phase was concentrated to afford crude compound **A68-5,** which was used directly in the next step. LCMS: [M+H]⁺ = 979.9 (half peak)

### Synthesis of Compound A68

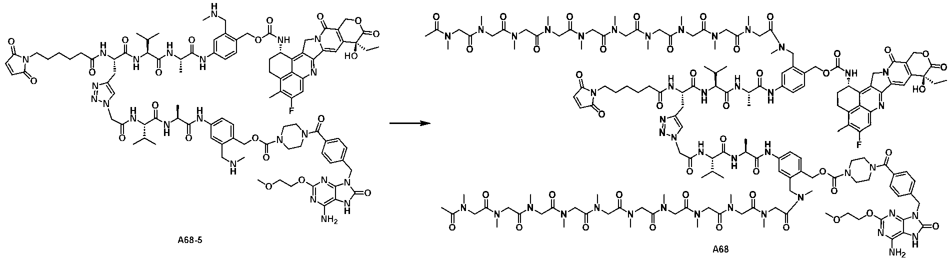

The compound **A68-5** from the previous step was dissolved in DMF (3 mL). Compound **Ac-PSAR10-OH** (63 mg, 0.082 mmol, 2.0 eq) and N,N-Diisopropylethylamine (108 mg, 0.834 mmol, 20.0 eq) were added. Finally, O-(7-Azabenzotriazol-1-yl)-N,N,N',N'-tetramethyluronium hexafluorophosphate (34 mg, 0.087 mmol, 2.1 eq) was added under a nitrogen atmosphere. After addition, the reaction mixture was stirred at room temperature under nitrogen for 1 hour. After the reaction was confirmed complete by LC-MS monitoring, the mixture was filtered. The filtrate was purified by preparative HPLC (mobile phase: 0.05% HCOOH/CH₃CN/H₂O) to afford Compound **A68** (40 mg, 43%) as a white solid. LCMS: [M+H]⁺ = 1733.4 (half peak)

### 14) Synthesis of A12 Control Linker Single-Drug Compound (MCD1)

### Synthetic Route:

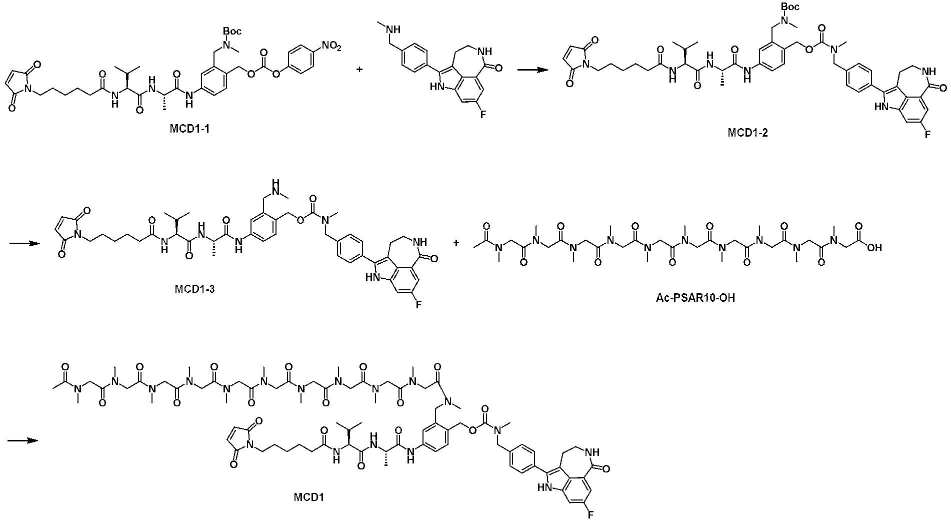

### Synthetic Steps:

### Synthesis of Compound MCD1-2

Rucaparib (purchased from Shanghai Haoyuan Chem, 244 mg, 0.755 mmol) was dissolved in anhydrous DMF. DIPEA (264 µl, 1.510 mmol) was added. MCD1-1 (600 mg, 0.755 mmol), HOBt (51.0 mg, 0.377 mmol) and pyridine (61.1 µl, 0.755 mmol) were added. The reaction mixture was stirred at room temperature overnight. The solvent was removed under reduced pressure. The residue was purified by column chromatography to afford MCD1-2 (570 mg, 77% yield). LCMS: [M+H]⁺ = 979.51.

### Synthesis of Compound MCD1-3

Compound MCD1-2 (500 mg, 0.0417 mmol, 1.0 eq) was dissolved in DCM (3 mL). Trifluoroacetic acid (1 mL) was added to the reaction mixture. The mixture was stirred at room temperature for 5 minutes. After the reaction was confirmed complete by LC-MS monitoring, the organic phase was concentrated to afford crude compound MCD1-3, which was used directly in the next step.

### Synthesis of Compound MCD1

The compound MCD1-3 from the previous step (300 mg, 0.341 mmol) was dissolved in DMF (3 mL). Compound Ac-PSAR10-OH (263 mg, 0.341 mmol) and N,N-Diisopropylethylamine (596 µl, 3.41 mmol) were added. Finally, O-(7-Azabenzotriazol-1-yl)-N,N,N',N'-tetramethyluronium hexafluorophosphate (195 mg, 0.512 mmol) was added under a nitrogen atmosphere. After addition, the reaction mixture was stirred at room temperature under nitrogen for 1 hour. After the reaction was confirmed complete by LC-MS monitoring, the mixture was filtered. The filtrate was purified by preparative HPLC (mobile phase: 0.05% HCOOH/CH₃CN/H₂O) to afford Compound MCD1 (320 mg, 57%) as a white solid.

### 15) Synthesis of Series Compounds A1, A3, A4, A5, A6, A7, A8, A13, A18, A19, A20, A22, A24, A25, A26, A27, A28, A30, A31, A32, A34, A35, A36, A37, A54, A55, A56, A57, A59, A60, A61, A62, A64, A65, A66, A67, A68, A69, A70, A71, A72, A73, A74, A75, A76, A77, A78, A79, A81, A82, A83, A84, A85, A86, A87, A88, A89, A91, A92, A93, A103, A105 and A106

Referring to the synthesis method of the aforementioned Compound A2, compounds A1, A3, A4, A5, A6, A7, A8, A13, A18, A19, A20, A22, A24, A25, A26, A27, A28, A30, A31, A32, A34, A35, A36, A37, A54, A55, A56, A57, A59, A60, A61, A62, A64, A65, A66, A67, A68, A69, A70, A71, A72, A73, A74, A75, A76, A77, A78, A79, A81, A82, A83, A84, A85, A86, A87, A88, A89, A91, A92, A93, A103, A105 and A106 can be obtained through multi-step reactions.

### 16) Synthesis of Compound A39

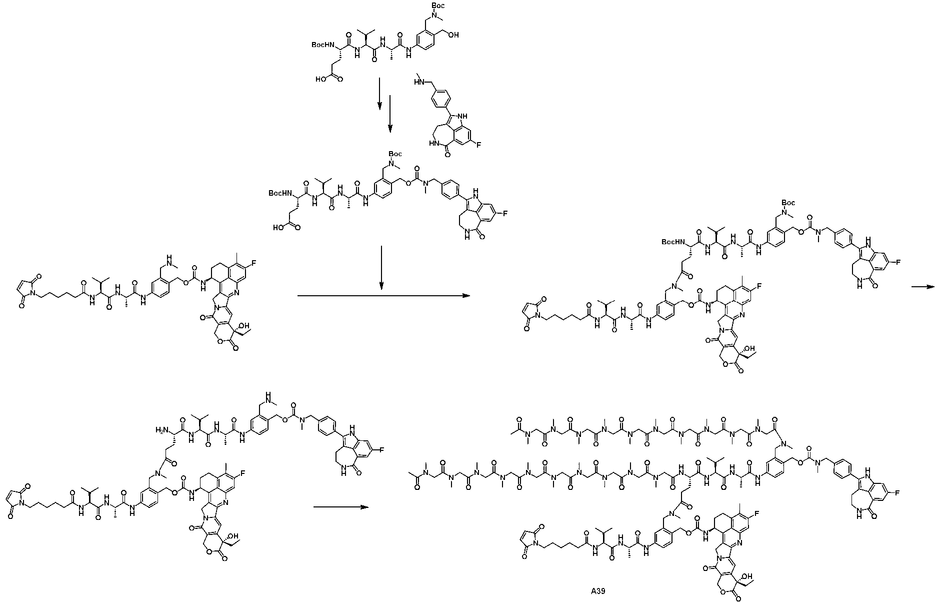

Referring to the synthesis method of the aforementioned Compound A2, Compound A39 can be obtained through multi-step reactions.

### 17) Synthesis of Series Compounds A38, A40, A41, A42, A43, A44, A45, A46, A47, A48, A49, A50, A51 and A52

Referring to the synthesis methods and routes of the aforementioned Compounds A2 and A39, compounds A38, A40, A41, A42, A43, A44, A45, A46, A47, A48, A49, A50, A51 and A52 can be obtained through multi-step reactions.

### 18) Synthesis of Compound A94

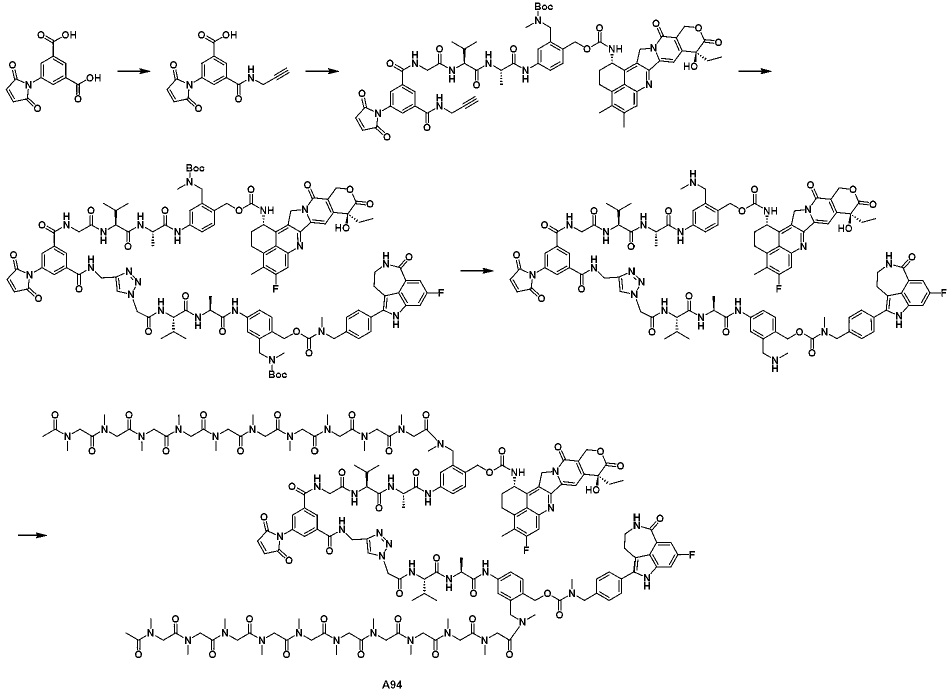

Referring to the synthesis method of the aforementioned Compound A2, Compound A94 can be obtained through multi-step reactions.

### 19) Synthesis of Series Compounds A95, A96, A97, A98, A99, A100, A101 and A102

Referring to the synthesis methods and routes of the aforementioned Compounds A2 and A94, compounds A95, A96, A97, A98, A99, A100, A101 and A102 can be obtained through multi-step reactions.

### 20) Synthesis of Compounds A107 and A108

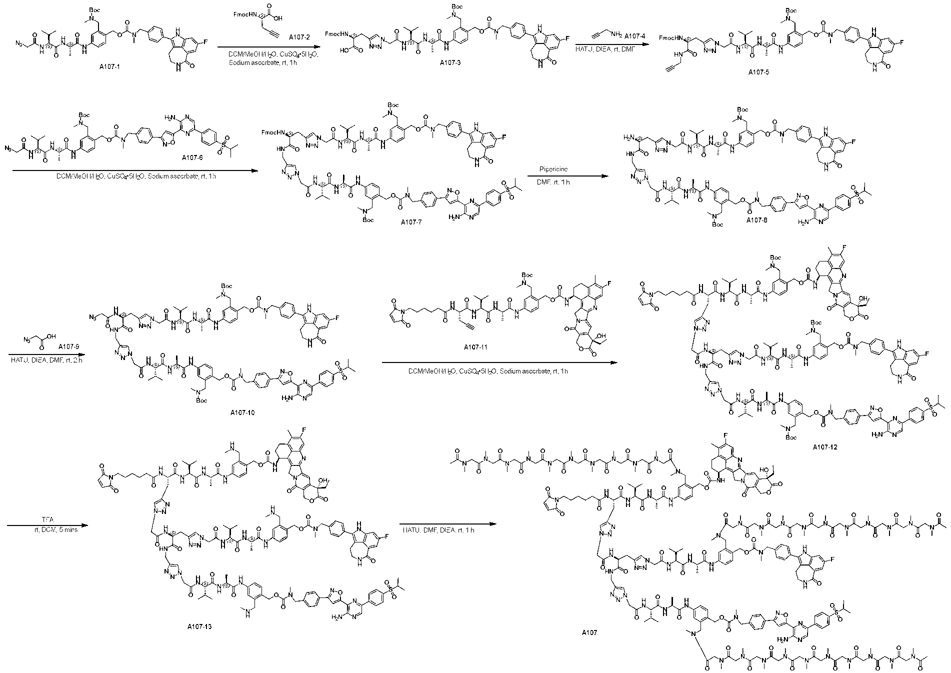

### Synthesis of Compound A107-3

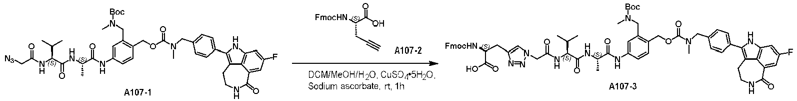

Compound A107-1 (250 mg, 0.287 mmol, 1.0 eq) and A107-2 (96 mg, 0.287 mmol, 1.0 eq) were dissolved in a mixture of dichloromethane/methanol/water (4/4/1 mL). Copper(II) sulfate pentahydrate (7.16 mg, 0.029 mmol, 0.1 eq, in 100 µL water) and sodium ascorbate (11.39 mg, 0.032 mmol, 0.25 eq, in 100 µL water) were added under a nitrogen atmosphere. After addition, the reaction mixture was stirred at room temperature under nitrogen for 1 hour. After the reaction was confirmed complete by LC-MS monitoring, water (30 mL) was added. The mixture was extracted with dichloromethane/methanol (10/1; V/V). The combined organic phases were washed with brine, dried over anhydrous sodium sulfate, filtered, and concentrated to afford compound A107-3 (340 mg, 98%) as a colored solid. LCMS: [M+H]⁺ = 1204.3

### Synthesis of Compound A107-5

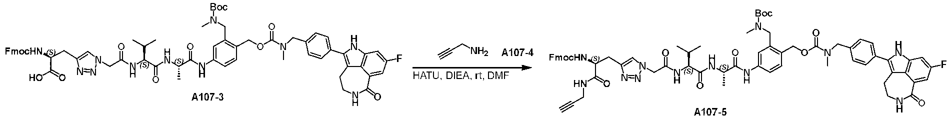

Compound A107-3 (340 mg, 0.282 mmol, 1.0 eq) was dissolved in DMF (3 mL). Compound A107-4 (47 mg, 0.846 mmol, 3.0 eq) and N,N-Diisopropylethylamine (73 mg, 0.564 mmol, 2.0 eq) were added. Finally, O-(7-Azabenzotriazol-1-yl)-N,N,N',N'-tetramethyluronium hexafluorophosphate (118 mg, 0.310 mmol, 1.1 eq) was added under a nitrogen atmosphere. After addition, the reaction mixture was stirred at room temperature under nitrogen for 3 hours. After the reaction was confirmed complete by LC-MS monitoring, ethyl acetate and water (30 mL) were added. The mixture was extracted with ethyl acetate. The combined organic phases were washed with water and brine, dried over anhydrous sodium sulfate, filtered, and concentrated. The filtrate residue was triturated with MTBE, filtered, and the filter cake was dried to afford compound A107-5 (300 mg, 86%) as a yellow solid. LCMS: [M+H]⁺ = 1242.4

### Synthesis of Compound A107-7

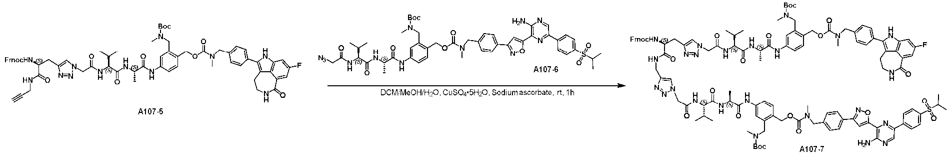

Compound A107-5 (148 mg, 0.119 mmol, 1.0 eq) and compound A107-6 (120 mg, 0.119 mmol, 1.0 eq) were dissolved in a mixture of dichloromethane/methanol/water (8/8/2 mL). Copper(II) sulfate pentahydrate (2.97 mg, 0.012 mmol, 0.1 eq, in 100 µL water) and sodium ascorbate (4.71 mg, 0.012 mmol, 0.2 eq, in 100 µL water) were added under a nitrogen atmosphere. After addition, the reaction mixture was stirred at room temperature under nitrogen for 1 hour. After the reaction was confirmed complete by LC-MS monitoring, the mixture was concentrated directly. The residue was triturated with dichloromethane, filtered, and the filter cake was dried to afford compound A107-7 (240 mg, 90%) as a gray solid. LCMS: [M+H]⁺ = 1125.2 (half peak)

### Synthesis of Compound A107-8

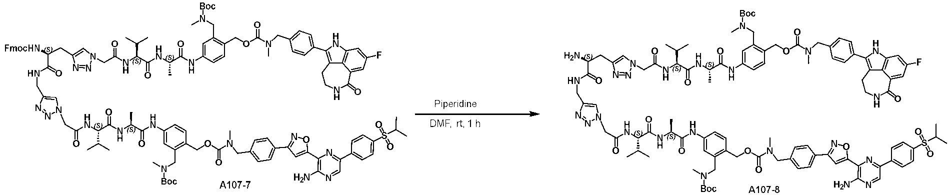

Compound **A107-7** (240 mg, 0.106 mmol, 1.0 eq) was dissolved in DMF (3 mL). The reaction mixture was cooled to 0°C, and piperidine (0.6 mL) was added under a nitrogen atmosphere. The mixture was stirred at room temperature under nitrogen for 1 hour. After the reaction was confirmed complete by LC-MS monitoring, ethyl acetate was added. The suspension was filtered directly, washed with ethyl acetate, and the filter cake was dried to afford compound **A107-8** (100 mg, 94%) as a gray solid. LCMS: [M+H]⁺ = 1014.5 (half peak)

### Synthesis of Compound A107-10

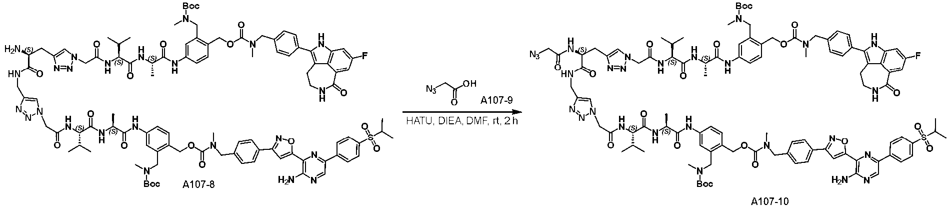

Compound A107-8 (100 mg, 0.049 mmol, 1.0 eq) was dissolved in DMF (3 mL). Compound A107-9 (7.5 mg, 0.074 mmol, 1.5 eq) and N,N-Diisopropylethylamine (19 mg, 0.147 mmol, 3.0 eq) were added. Finally, O-(7-Azabenzotriazol-1-yl)-N,N,N',N'-tetramethyluronium hexafluorophosphate (28 mg, 0.074 mmol, 1.5 eq) was added under a nitrogen atmosphere. After addition, the reaction mixture was stirred at room temperature under nitrogen for 2 hours. After the reaction was confirmed complete by LC-MS monitoring, ethyl acetate (60 mL) was added. The suspension was filtered directly, washed with ethyl acetate, and the filter cake was dried to afford compound A107-10 (100 mg, 96%) as a gray solid. LCMS: [M+H]⁺ = 1056.4 (half peak)

### Synthesis of Compound A107-12

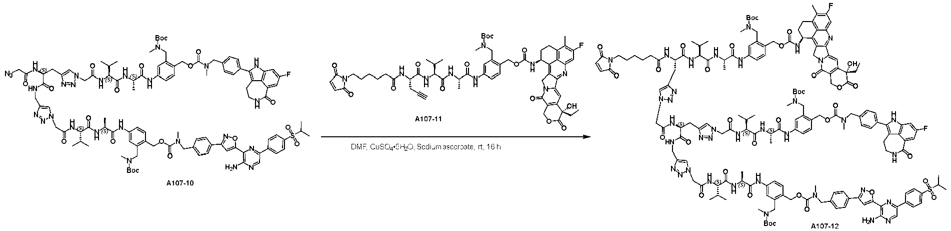

Compound A107-10 (60 mg, 0.028 mmol, 1.0 eq) and compound A107-11 (34 mg, 0.028 mmol, 1.0 eq) were placed in DMF (4 mL). Copper(II) sulfate pentahydrate (0.7 mg, 0.003 mmol, 0.1 eq, in 100 µL water) and sodium ascorbate (1.1 mg, 0.006 mmol, 0.2 eq, in 100 µL water) were added under a nitrogen atmosphere. After addition, the reaction mixture was stirred at room temperature under nitrogen for 16 hours. After the reaction was confirmed complete by LC-MS monitoring, ethyl acetate (50 mL) was added. The suspension was filtered directly, washed with ethyl acetate, and the filter cake was dried to afford compound A107-12 (90 mg, 96%) as a gray solid. LCMS: [M+H]⁺ = 1649.0 (half peak)

### Synthesis of Compound A107-13

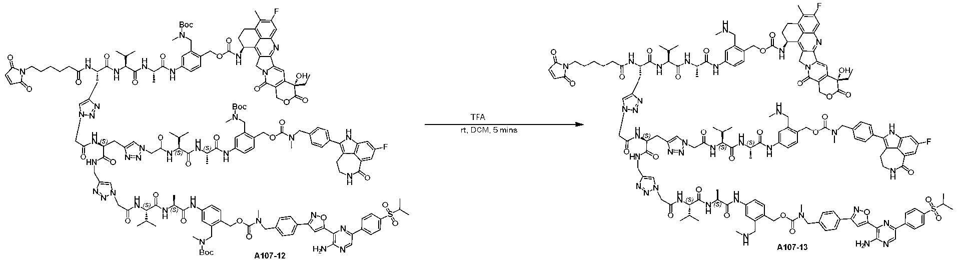

Compound A107-12 (90 mg, 0.027 mmol, 1.0 eq) was dissolved in DCM (3 mL). Trifluoroacetic acid (1 mL) was added to the reaction mixture. The mixture was stirred at room temperature for 5 minutes. After the reaction was confirmed complete by LC-MS monitoring, the organic phase was concentrated to afford crude compound A107-13, which was used directly in the next step. LCMS: [M+H]⁺ = 1499.1 (half peak)

### Synthesis of Compound A107

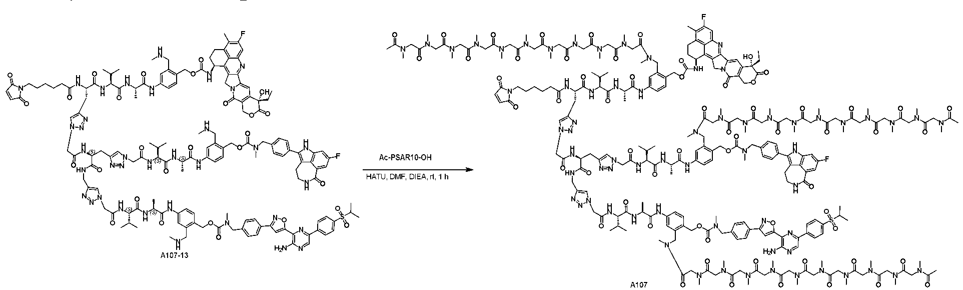

The compound A107-13 from the previous step was dissolved in DMF (3 mL). Ac-PSAR10-OH (62 mg, 0.081 mmol, 3.0 eq) and N,N-Diisopropylethylamine (104 mg, 0.81 mmol, 30.0 eq) were added. Finally, O-(7-Azabenzotriazol-1-yl)-N,N,N',N'-tetramethyluronium hexafluorophosphate (34 mg, 0.089 mmol, 3.3 eq) was added under a nitrogen atmosphere. After addition, the reaction mixture was stirred at room temperature under nitrogen for 1 hour. After the reaction was confirmed complete by LC-MS monitoring, ethyl acetate (35 mL) was added. The resulting suspension was filtered, and the filter cake was dissolved in DMF. Purification by preparative HPLC (mobile phase: 0.05% HCOOH/CH₃CN/H₂O) afforded Compound **A107** (21.7 mg, 15%) as a yellow solid. LCMS: [M+H]⁺ = 1752.3 (one-third peak).

Compound A108 can be obtained by a similar method.

### 21) Synthesis of Compound A9

### Synthetic Steps:

### Synthesis of Intermediate Compound A9-2

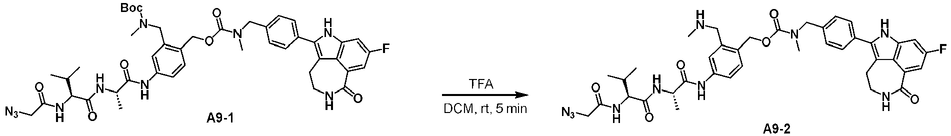

Compound **A9-1** (50 mg, 0.0575 mmol) was dissolved in DCM (3 mL). Trifluoroacetic acid (1 mL) was added to the reaction mixture. The mixture was stirred at room temperature for 5 minutes. After the reaction was confirmed complete by LC-MS monitoring, the organic phase was concentrated to afford crude compound **A9-2,** which was used directly in the next step. LCMS: [M+H]⁺ = 769.3

### Synthesis of Intermediate Compound A9-IM-1

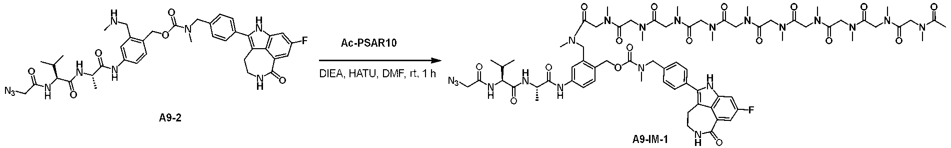

The compound **A9-2** from the previous step was dissolved in DMF (3 mL). Ac-PSAR10 (44.1 mg, 0.0575 mmol, 1.0 eq) and N,N-Diisopropylethylamine (147.9 mg, 1.150 mmol, 20.0 eq) were added. Finally, O-(7-Azabenzotriazol-1-yl)-N,N,N',N'-tetramethyluronium hexafluorophosphate (22.8 mg, 0.0633 mmol, 1.1 eq) was added. After addition, the reaction mixture was stirred at room temperature under nitrogen for 1 hour. After the reaction was confirmed complete by LC-MS monitoring, ethyl acetate (45 mL) was added. The mixture was filtered to afford compound **A9-IM-1** (80 mg, 91.4%) as a yellow solid.
LCMS: [M+H]⁺ = 761.9 (half peak)

### Synthesis of Intermediate Compound A9-5

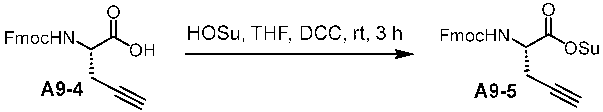

Compound **A9-4** (200 mg, 0.596 mmol, 1.0 eq), HOSu (103.0 mg, 0.895 mmol, 1.5 eq) and DCC (246.1 mg, 1.193 mmol, 2.0 eq) were dissolved in tetrahydrofuran (5 mL). The reaction mixture was stirred at room temperature for 3 hours. After the reaction was confirmed complete by LC-MS monitoring, the mixture was concentrated directly. The residue was purified by silica gel column chromatography (DCM/MeOH = 93/7) to afford compound **A9-5** (160 mg, 37%) as a white solid.
LCMS: [M+Na]⁺ = 455.1

### Synthesis of Intermediate Compound A9-8

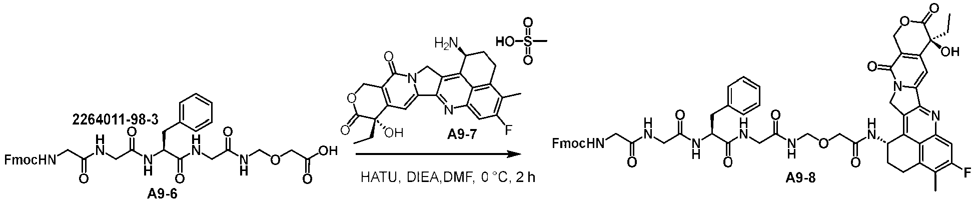

Compound 7 (247.5 mg, 0.466 mmol, 1.0 eq) and N,N-Diisopropylethylamine (180.5 mg, 1.397 mmol, 3.0 eq) were dissolved in DMF (5 mL). After stirring until clear, compound **6** (300 mg, 0.466 mmol, 1.0 eq) and O-(7-Azabenzotriazol-1-yl)-N,N,N',N'-tetramethyluronium hexafluorophosphate (283.3 mg, 0.745 mmol, 1.6 eq) were added. After addition, the reaction mixture was stirred at 0°C for 2 hours. After the reaction was confirmed complete by LC-MS monitoring, the reaction mixture was directly injected for reverse phase purification (0.1% FA water/acetonitrile = 2/3) to afford compound **8** (420 mg, 81%) as a yellow solid.
LCMS: [M+H]⁺ = 1063.3

### Synthesis of Intermediate Compound A9-9

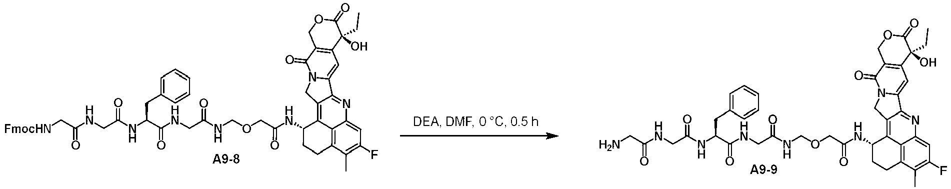

Compound **A9-8** (420 mg, 0.395 mmol, 1.0 eq) was dissolved in DMF (4 mL). After cooling to 0°C, diethylamine (0.4 mL) was added. After addition, the reaction mixture was stirred at 0°C for half an hour. After the reaction was confirmed complete by LC-MS monitoring, the organic phase was concentrated to remove diethylamine. Acetic acid (420 mg) dissolved in a small amount of DMF was added to the reaction system and shaken well. The system was directly injected for reverse phase purification (0.1% FA water/acetonitrile = 7/3) to afford compound A9-9 (290 mg, 83%) as a yellow solid.
LCMS: [M+H]⁺ = 841.3

### Synthesis of Intermediate Compound A9-10

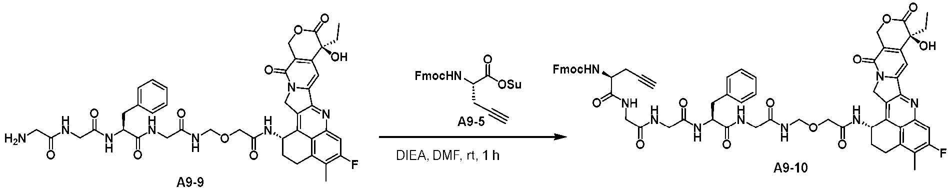

Compound **A9-9** (170 mg, 0.202 mmol, 1.0 eq), compound **A9-5** (104.9 mg, 0.243 mmol, 1.2 eq) and N,N-Diisopropylethylamine (52.3 mg, 0.404 mmol, 2.0 eq) were dissolved in DMF (5 mL). After addition, the reaction mixture was stirred at room temperature for 1 hour. After the reaction was confirmed complete by LC-MS monitoring, the reaction mixture was directly injected for reverse phase purification (0.1% HCOOH/water/acetonitrile = 1/1) to afford compound **A9-10** (190 mg, 80%) as a white solid.
LCMS: [M+H]⁺ = 1158.4

### Synthesis of Intermediate Compound A9-11

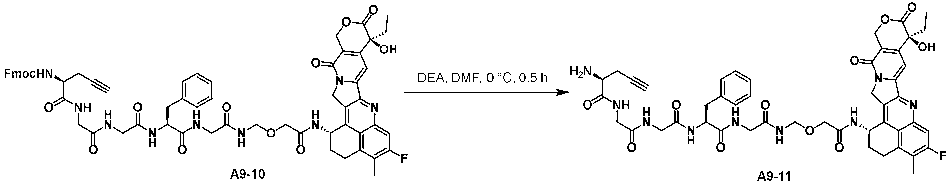

Compound **A9-10** (180 mg, 0.156 mmol, 1.0 eq) was dissolved in DMF (3 mL). After cooling to 0°C, diethylamine (0.3 mL) was added. After addition, the reaction mixture was stirred at 0°C for half an hour. After the reaction was confirmed complete by LC-MS monitoring, the organic phase was concentrated to remove diethylamine. Acetic acid (420 mg, likely a typo, should be a small amount) dissolved in a small amount of DMF was added to the reaction system and shaken well. The system was directly injected for reverse phase purification (0.1% HCOOH/water/acetonitrile = 7/3) to afford compound **A9-11** (120 mg, 81%) as a white solid.
LCMS: [M+H]⁺ = 936.2

### Synthesis of Intermediate Compound A9-13

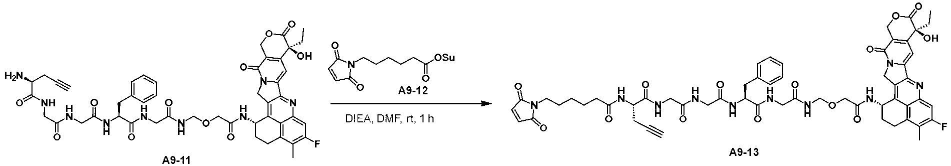

Compound **A9-11** (70 mg, 0.0748 mmol, 1.0 eq), compound **A9-12** (27.7 mg, 0.0898 mmol, 1.2 eq) and N,N-Diisopropylethylamine (19.3 mg, 0.150 mmol, 2.0 eq) were dissolved in DMF (3 mL). After addition, the reaction mixture was stirred at room temperature for 1 hour. After the reaction was confirmed complete by LC-MS monitoring, the reaction mixture was directly injected for reverse phase purification (0.1% FA water/acetonitrile = 7/3) to afford compound **A9-13** (70 mg, 81%) as a white solid.

LCMS: [M+H]⁺ = 1129.3.

### Synthesis of Target Compound A9

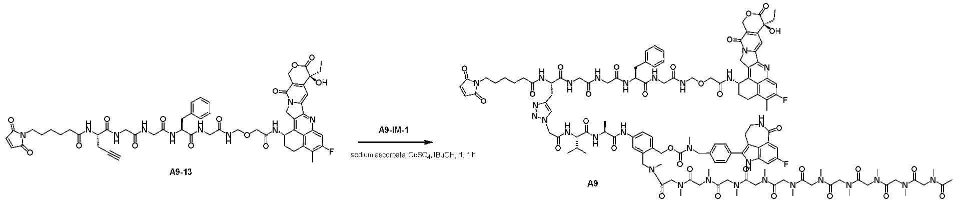

Compound **A9-13** (40 mg, 0.0354 mmol, 1.0 eq) and compound **A9-IM-1** (28.8 mg, 0.0354 mmol, 1.0 eq) were dissolved in a mixture of MeOH/DCM/water (4/4/1 mL). Then, 100 µL of an aqueous solution of copper(II) sulfate pentahydrate (0.89 mg, 0.00354 mmol, 0.1 eq) and 100 µL of an aqueous solution of sodium ascorbate (1.4 mg, 0.00708 mmol, 0.2 eq) were added. After addition, the reaction mixture was stirred at room temperature under nitrogen for 1 hour. After the reaction was confirmed complete by LC-MS monitoring, water (30 mL) was added. The mixture was extracted multiple times with dichloromethane. The combined organic phases were dried over anhydrous sodium sulfate, filtered, and concentrated. The residue was purified by preparative HPLC (mobile phase: 0.05% HCOOH/CH₃CN/H₂O) to afford Compound **A9** (7.6 mg, 12.4%) as a white solid. LCMS: [M+H]⁺ = 1326.1 (half peak)

### 22) Synthesis of Compound A131

### Synthetic Steps:

### Synthesis of Intermediate Compound A131-3

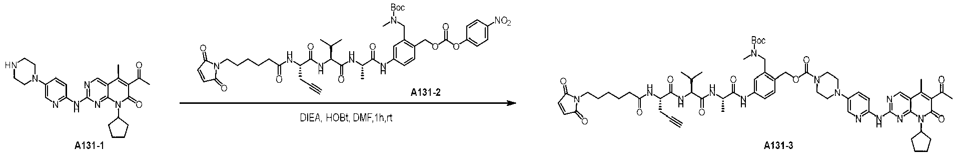

Compound **A131-1** (125 mg, 0.281 mmol, 1.0 eq) and N,N-Diisopropylethylamine (43.86 mg, 0.337 mmol, 3.0 eq) were dissolved in DMF (5 mL). After stirring until clear, compound **A131-2** (250 mg, 0.281 mmol, 1.0 eq) and HOBt (7.58 mg, 0.056 mmol, 0.2 eq) were added. After addition, the reaction mixture was stirred at room temperature for 1 hour. After the reaction was confirmed complete by LC-MS monitoring, water (30 mL) was added. The mixture was extracted with ethyl acetate. The combined organic phases were washed with water and brine, dried over anhydrous sodium sulfate, filtered, and concentrated. The residue was purified by silica gel column chromatography (DCM/MeOH = 93/7) to afford compound **A131-3** (318 mg, 85.1%) as a yellow solid.
LCMS: [M+H]⁺ = 1198.5

### Synthesis of Intermediate Compound A131-5

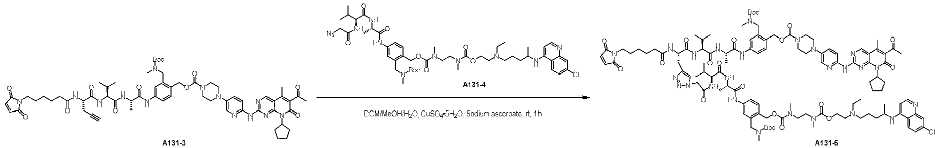

Compound **A131-3** (81 mg, 0.0675 mmol, 1.0 eq) and **A131-4** (67.27 mg, 0.0675 mmol, 1.0 eq) were dissolved in a mixture of dichloromethane/methanol/water (5/5/1 mL). Copper(II) sulfate pentahydrate (1.69 mg, 0.0067 mmol, 0.1 eq, in 100 µL water) and sodium ascorbate (2.67 mg, 0.013 mmol, 0.2 eq, in 100 µL water) were added under a nitrogen atmosphere. After addition, the reaction mixture was stirred at room temperature under nitrogen for 1 hour. After the reaction was confirmed complete by LC-MS monitoring, the reaction mixture was concentrated under reduced pressure, and tetrahydrofuran (30 mL) was added and evaporated three times to remove water, affording compound **A131-5** (143 mg, 68.1%) as a yellow solid.
LCMS: [M+H]⁺ = 1097.9 (half peak)

### Synthesis of Intermediate Compound A131-6

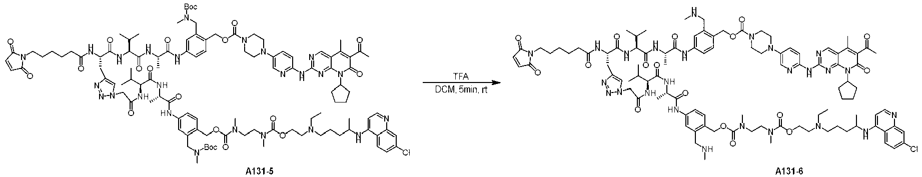

Compound **A131-5** (115 mg, 0.052 mmol) was dissolved in DCM (3 mL). Trifluoroacetic acid (1 mL) was added to the reaction mixture. The mixture was stirred at room temperature for 5 minutes. After the reaction was confirmed complete by LC-MS monitoring, the organic phase was concentrated to afford crude compound **A131-6,** which was used directly in the next step. LCMS: [M+H]⁺ = 998.7 (half peak)

### Synthesis of Target Compound A131

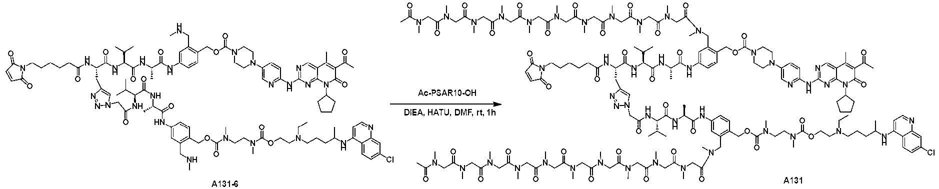

The compound **A131-6** from the previous step was dissolved in DMF (3 mL). Compound Ac-PSAR10-OH (77.27 mg, 0.100 mmol, 2.0 eq) and N,N-Diisopropylethylamine (129.67 mg, 1.003 mmol, 20.0 eq) were added. Finally, O-(7-Azabenzotriazol-1-yl)-N,N,N',N'-tetramethyluronium hexafluorophosphate (41.96 mg, 0.110 mmol, 2.1 eq) was added under a nitrogen atmosphere. After addition, the reaction mixture was stirred at room temperature under nitrogen for 1 hour. After the reaction was confirmed complete by LC-MS monitoring, ethyl acetate (35 mL) was added. The resulting suspension was filtered, and the filter cake was dissolved in DMF. Purification by preparative HPLC (mobile phase: 0.05% HCOOH/CH₃CN/H₂O) afforded Compound **A131** (12.1 mg, 6.9%) as a white solid.
LCMS: [M+H]⁺ = 1749.8 (half peak)

### 23) Synthesis of Compound A132

### Synthetic Steps:

### Synthesis of Intermediate Compound A132-3

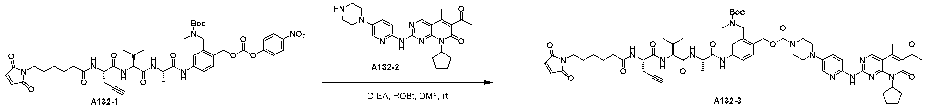

Compound A132-2 (300 mg, 0.670 mmol, 1.0 eq) was dissolved in DMF (2 mL). DIEA (259 mg, 2.01 mmol, 3 eq) was added and stirred for 2 minutes until completely clear. Then, compound A132-1 (597 mg, 0.670 mmol, 1.0 eq) and HOBt (90.5 mg, 0.670 mmol, 1.0 eq) were added. After addition, the reaction mixture was stirred at room temperature for 1 hour. After the reaction was confirmed complete by LC-MS monitoring, water (30 mL) was added. The mixture was extracted with EA. The combined organic phases were washed with brine, dried over anhydrous sodium sulfate, filtered, and concentrated. The residue was purified by silica gel column chromatography (DCM/MeOH = 10/1) to afford compound A132-3 (350 mg, 60%) as a yellow solid.
LCMS: [M+H]⁺ = 1198.5

### Synthesis of Intermediate Compound A132-6

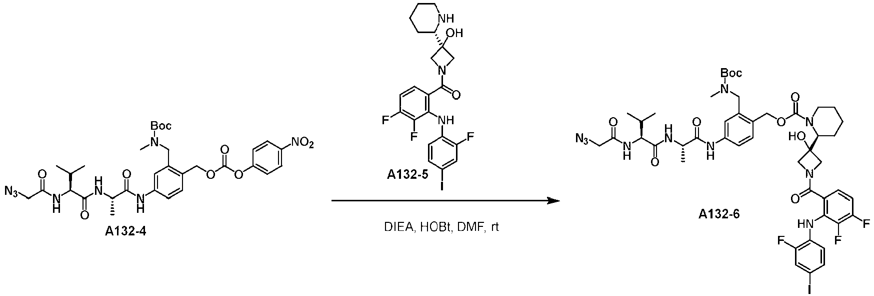

Compound A132-5 (100 mg, 0.188 mmol, 1.0 eq) was dissolved in DMF (2 mL). DIEA (72.9 mg, 0.564 mmol, 3 eq) was added and stirred for 2 minutes until completely clear. Then, compound A132-4 (129 mg, 0.188 mmol, 1.0 eq) and HOBt (25.4 mg, 0.188 mmol, 1.0 eq) were added. After addition, the reaction mixture was stirred at room temperature for 1 hour. After the reaction was confirmed complete by LC-MS monitoring, water (30 mL) was added. The mixture was extracted with EA. The combined organic phases were washed with brine, dried over anhydrous sodium sulfate, filtered, and concentrated. The residue was purified by silica gel column chromatography (DCM/MeOH = 7/1) to afford compound A132-6 (100 mg, 49%) as a white solid.
LCMS: [M+Na]⁺ = 1099.3

### Synthesis of Intermediate Compound A132-7

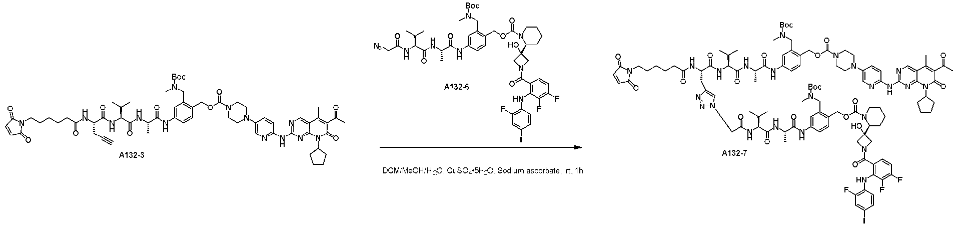

Compound A132-3 (100 mg, 0.083 mmol, 1.0 eq) and compound A132-6 (89.9 mg, 0.083 mmol, 1.0 eq) were dissolved in a mixture of methanol/dichloromethane/water (3:3:0.5 mL). 100 µL of an aqueous solution of copper(II) sulfate (2.09 mg, 0.0084 mmol, 0.1 eq) and 100 µL of an aqueous solution of sodium ascorbate (3.3 mg, 0.0167 mmol, 0.2 eq) were added. After addition, the reaction mixture was stirred at room temperature under nitrogen for 1 hour. After the reaction was confirmed complete by LC-MS monitoring, water (30 mL) was added. The mixture was extracted with dichloromethane/methanol. The combined organic phases were washed with brine, dried over anhydrous sodium sulfate, filtered, and concentrated to afford crude compound A132-7 (150 mg, 79%) which was used directly in the next step.
LCMS: [M+H]⁺ = 1137.9 (half peak)

### Synthesis of Intermediate Compound A132-8

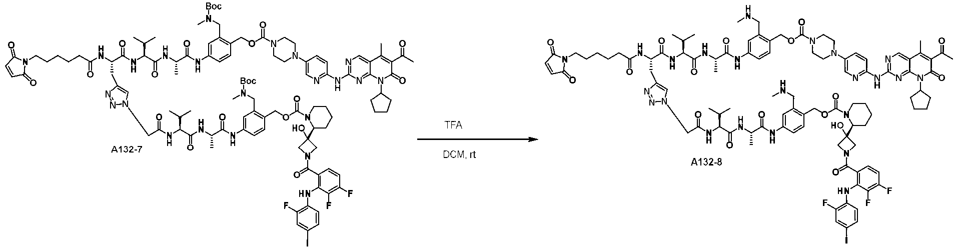

Compound A132-7 (150 mg, 0.0659 mmol) was dissolved in dichloromethane (3 mL). Trifluoroacetic acid (1 mL) was added to the reaction mixture. The mixture was stirred at room temperature for 5 min. After the reaction was confirmed complete by LC-MS monitoring, the mixture was concentrated to afford crude compound A132-8 (130 mg, 95%), which was used directly in the next step. LCMS: [M+H]⁺ = 1038.3 (half peak)

### Synthesis of Target Compound A132

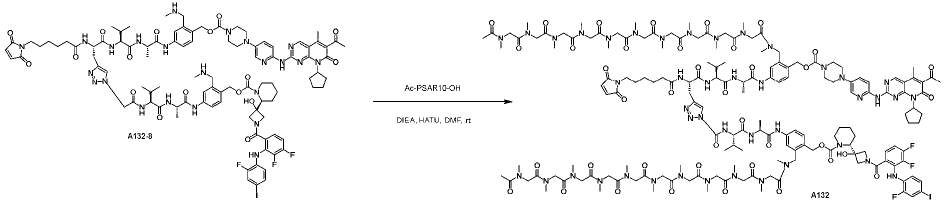

Compound A132-8 (130 mg, 0.0626 mmol, 1.0 eq) was dissolved in DMF (2 mL). DIEA (61.82 mg, 1.25 mmol, 20 eq) was added and stirred for 2 minutes. Then, compound Ac-PSAR10-OH (96.5 mg, 0.125 mmol, 2.0 eq) and HATU (47.6 mg, 0.125 mmol, 2.0 eq) were added. After addition, the reaction mixture was stirred at room temperature for 1 hour. After the reaction was confirmed complete by LC-MS monitoring, ethyl acetate (20 mL) was added. The mixture was filtered, and the filter cake was dissolved in DMF. Purification by preparative HPLC (mobile phase: 0.05% HCOOH/CH₃CN/H₂O) afforded Compound A132 (24 mg, 18%) as a white solid. LCMS: [M+H]⁺ = 1790.7 (half peak)

### 24) Synthesis of Compound A133

### Synthetic Steps:

### Synthesis of Intermediate Compound A133-3

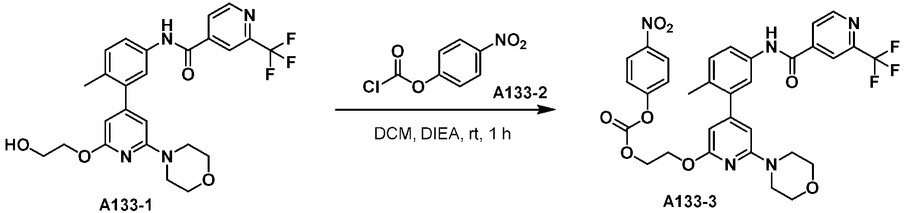

Compound **A133-1** (250 mg, 0.4975 mmol, 1.0 eq) and N,N-Diisopropylethylamine (192.9 mg, 1.4925 mmol, 3.0 eq) were dissolved in DMF (5 mL). After stirring until clear, compound **A133-2** (300.8 mg, 1.4925 mmol, 3.0 eq) was added. After addition, the reaction mixture was stirred at room temperature for 1 hour. After the reaction was confirmed complete by LC-MS monitoring, the reaction mixture was concentrated directly. The residue was purified by silica gel column chromatography (DCM/MeOH = 95/5) to afford compound **A133-3** (290 mg, 86%) as a white solid.

LCMS: [M+H]⁺ = 668.2

### Synthesis of Intermediate Compound A133-5

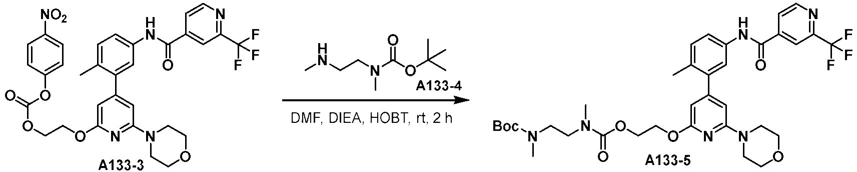

Compound **A133-3** (290 mg, 0.4344 mmol, 1.0 eq) and compound **A133-4** (89.1 mg, 0.5218 mmol, 1.2 eq) were dissolved in DMF (5 mL). Then, HOBt (58.7 mg, 0.4344 mmol, 1.0 eq) and N,N-Diisopropylethylamine (168.4 mg, 1.3032 mmol, 3.0 eq) were added. After addition, the reaction mixture was stirred at room temperature for 2 hours. After the reaction was confirmed complete by LC-MS monitoring, the mixture was diluted with ethyl acetate and washed with water and brine. The organic phase was dried over anhydrous sodium sulfate, filtered, and concentrated. The residue was purified by silica gel column chromatography (DCM/MeOH = 95/5) to afford compound **A133-5** (280 mg, 88%) as a white solid.

LCMS: [M+H]⁺ = 717.3

### Synthesis of Intermediate Compound A133-6

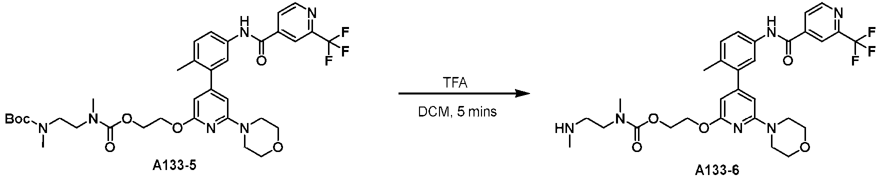

Compound **A133-5** (280 mg, 0.3901 mmol) was dissolved in DCM (6 mL). Trifluoroacetic acid (2 mL) was added to the reaction mixture. The mixture was stirred at room temperature for 5 minutes. After the reaction was confirmed complete by LC-MS monitoring, the organic phase was concentrated to afford crude compound **A133-6,** which was used directly in the next step.

LCMS: [M+H]⁺ = 617.2

### Synthesis of Intermediate Compound A133-IM-1

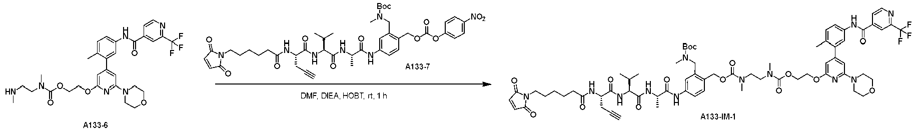

The compound 6 (A133-6) from the previous step was dissolved in DMF (5 mL). Then, compound 7 (346.8 mg, 0.3901 mmol, 1.0 eq), HOBt (52.6 mg, 0.3901 mmol, 1.0 eq) and N,N-Diisopropylethylamine (251.5 mg, 1.950 mmol, 5.0 eq) were added. After addition, the reaction mixture was stirred at room temperature for 2 hours. After the reaction was confirmed complete by LC-MS monitoring, the mixture was diluted with ethyl acetate and washed with water and brine. The organic phase was dried over anhydrous sodium sulfate, filtered, and concentrated. The residue was purified by silica gel column chromatography (DCM/MeOH = 97/3) to afford compound **A133-IM-1** (450 mg, 83%) as a white solid.

LCMS: [M+H]⁺ = 1369.4

### Synthesis of Intermediate Compound A133-8

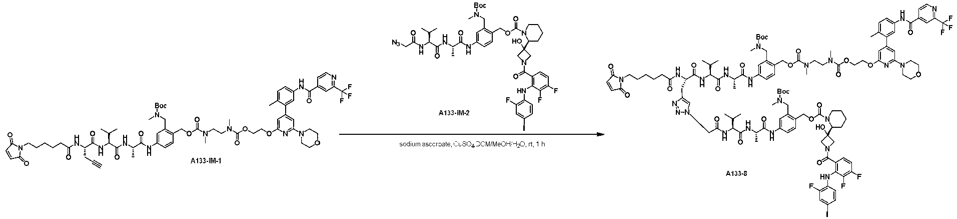

Compound **A133-IM-1** (127 mg, 0.0928 mmol, 1.0 eq) and compound **A133-IM-2** (100 mg, 0.0928 mmol, 1.0 eq) were dissolved in a mixture of MeOH/DCM/water (4/4/1 mL). Then, 100 µL of an aqueous solution of copper(II) sulfate pentahydrate (2.32 mg, 0.00928 mmol, 0.1 eq) and 100 µL of an aqueous solution of sodium ascorbate (3.68 mg, 0.01856 mmol, 0.2 eq) were added. After addition, the reaction mixture was stirred at room temperature under nitrogen for 4 hours. After the reaction was confirmed complete by LC-MS monitoring, water (30 mL) was added. The mixture was extracted with dichloromethane. The combined organic phases were washed with water and brine, dried over anhydrous sodium sulfate, filtered, and concentrated to afford compound **A133-8** (170 mg, 71%) which was used directly in the next step.

LCMS: [M+Na]⁺ = 1244.9 (half peak).

### Synthesis of Intermediate Compound A133-9

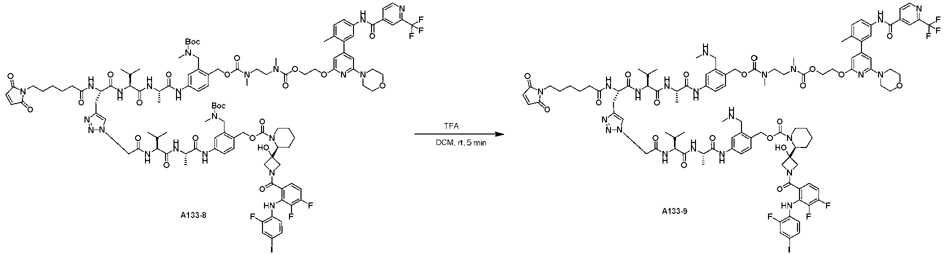

Compound **A133-8** (100 mg, 0.0409 mmol) was dissolved in DCM (3 mL). Trifluoroacetic acid (1 mL) was added to the reaction mixture. The mixture was stirred at room temperature for 5 minutes. After the reaction was confirmed complete by LC-MS monitoring, the organic phase was concentrated to afford crude compound **A133-9,** which was used directly in the next step.

LCMS: [M+H]⁺ = 1122.9 (half peak)

### Synthesis of Target Compound A133

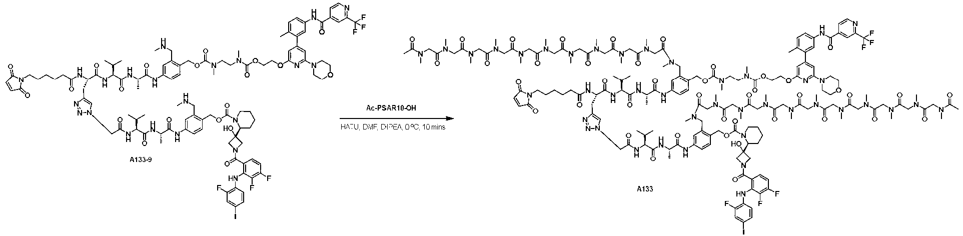

The compound **A133-9** from the previous step was dissolved in DMF (3 mL). Compound Ac-PSAR10-OH (63.1 mg, 0.0818 mmol, 2.0 eq) and N,N-Diisopropylethylamine (106.0 mg, 0.818 mmol, 20.0 eq) were added. Finally, HATU (67.3 mg, 0.0861 mmol, 2.1 eq) was added. After addition, the reaction mixture was stirred at 0°C under nitrogen for 10 minutes. After the reaction was confirmed complete by LC-MS monitoring, ethyl acetate (45 mL) was added. The mixture was filtered, and the filter cake was dissolved in DMF. Purification by preparative HPLC (mobile phase: 0.05% HCOOH/CH₃CN/H₂O) afforded target Compound **A133** (26.3 mg, 17.1%) as a white solid. LCMS: [M+H]⁺ = 1875.6 (half peak)

### 25) Synthesis of Compound A134

### Synthetic Steps:

### Synthesis of Intermediate Compound A134-3

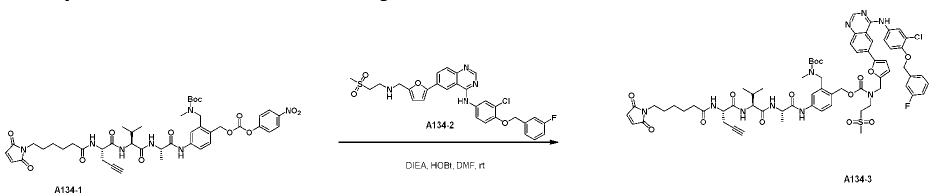

Compound A137-2 (100 mg, 0.172 mmol, 1.0 eq) was dissolved in DMF (2 mL). DIEA (66.7 mg, 0.516 mmol, 3 eq) was added and stirred for 2 minutes until completely clear. Then, compound A137-1 (153 mg, 0.172 mmol, 1.0 eq) and HOBt (23.2 mg, 0.172 mmol, 1.0 eq) were added. After addition, the reaction mixture was stirred at room temperature for 12 hours. After the reaction was confirmed complete by LC-MS monitoring, water (30 mL) was added. The mixture was extracted with ethyl acetate. The combined organic phases were washed with brine, dried over anhydrous sodium sulfate, filtered, and concentrated. The residue was purified by silica gel column chromatography (DCM/MeOH = 7/1) to afford compound A137-3 (90 mg, 39%) as a yellow solid.
LCMS: [M+H]⁺ = 1331.4

### Synthesis of Intermediate Compound A134-5

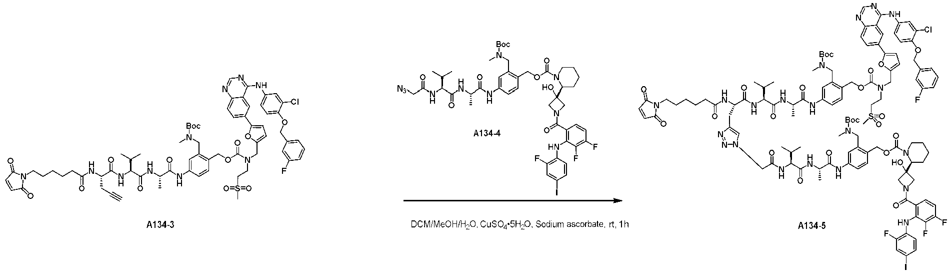

Compound A134-3 (100 mg, 0.075 mmol, 1.0 eq) and compound A134-4 (80.8 mg, 0.075 mmol, 1.0 eq) were dissolved in a mixture of methanol/dichloromethane/water (3:3:0.5 mL). 100 µL of an aqueous solution of copper(II) sulfate (1.88 mg, 0.0075 mmol, 0.1 eq) and 100 µL of an aqueous solution of sodium ascorbate (2.97 mg, 0.015 mmol, 0.2 eq) were added. After addition, the reaction mixture was stirred at room temperature under nitrogen for 1 hour. After the reaction was confirmed complete by LC-MS monitoring, water (30 mL) was added. The mixture was extracted with dichloromethane. The combined organic phases were washed with brine, dried over anhydrous sodium sulfate, filtered, and concentrated to afford crude compound A134-5 (100 mg, 55%) which was used directly in the next step.
LCMS: [M+H]⁺ = 1206.4 (half peak)

### Synthesis of Intermediate Compound A134-6

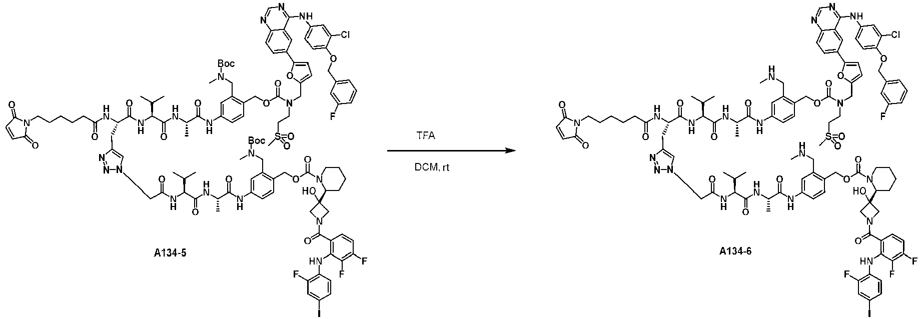

Compound A134-5 (100 mg, 0.0415 mmol) was dissolved in dichloromethane (3 mL). Trifluoroacetic acid (1 mL) was added to the reaction mixture. The mixture was stirred at room temperature for 5 min. After the reaction was confirmed complete by LC-MS monitoring, the mixture was concentrated to afford crude compound A134-6 (90 mg, 98%), which was used directly in the next step. LCMS: [M+H]⁺ = 1104.2 (half peak)

### Synthesis of Target Compound A134

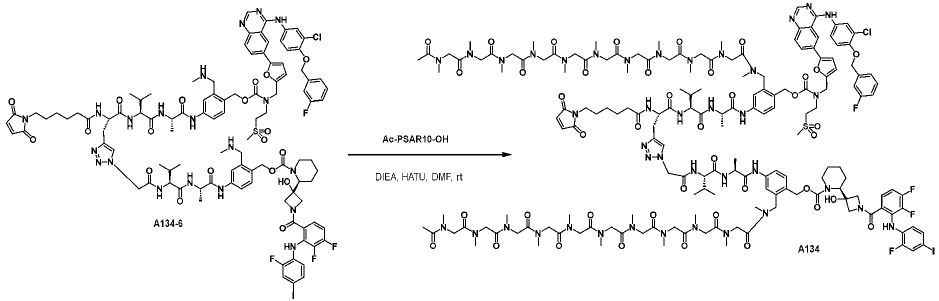

Compound A134-6 (90 mg, 0.0408 mmol) was dissolved in DMF (2 mL). DIEA (105 mg, 0.816 mmol, 20 eq) was added and stirred for 2 minutes. Then, compound Ac-PSAR10-OH (67.7 mg, 0.0816 mmol, 2.0 eq) and HATU (31.0 mg, 0.0816 mmol, 2.0 eq) were added. After addition, the reaction mixture was stirred at room temperature for 1 hour. After the reaction was confirmed complete by LC-MS monitoring, EA (30 mL) was added. The mixture was filtered, and the filter cake was dissolved in DMF. Purification by preparative HPLC (mobile phase: 0.05% HCOOH/CH₃CN/H₂O) afforded Compound A134 (15 mg, 25%) as a white solid. LCMS: [M+H]⁺ = 1857.6 (half peak)

### 26) Synthesis of Compound A135

### Synthetic Steps:

### Synthesis of Intermediate Compound A135-3

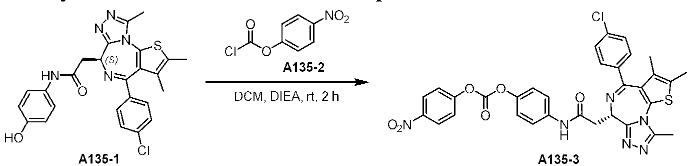

Compound **A135-1** (100 mg, 0.203 mmol, 1.0 eq) was dissolved in dichloromethane (5 mL). Compound **A135-2** (40.98 mg, 0.203 mmol, 1.0 eq) and N,N-Diisopropylethylamine (78.82 mg, 0.610 mmol, 3.0 eq) were added. After addition, the reaction mixture was stirred at room temperature for 2 hours. After the reaction was confirmed complete by LC-MS monitoring, water (30 mL) was added. The mixture was extracted with dichloromethane. The combined organic phases were washed with brine, dried over anhydrous sodium sulfate, filtered, and concentrated. The residue was purified by silica gel column chromatography (DCM/MeOH = 93/7) to afford compound **A135-3** (106 mg, 71%) as a white solid.
LCMS: [M+H]⁺ = 657.0

### Synthesis of Intermediate Compound A135-5

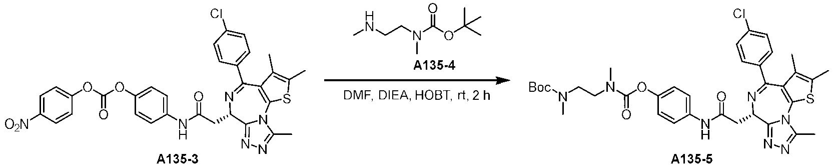

Compound **A135-3** (86 mg, 0.131 mmol, 1.0 eq) was dissolved in DMF (5 mL). Compound **A135-4** (24.64 mg, 0.131 mmol, 1.0 eq) and N,N-Diisopropylethylamine (50.76 mg, 0.397 mmol, 3.0 eq) were added. Finally, 1-hydroxybenzotriazole (8.84 mg, 0.0655 mmol, 0.5 eq) was added under a nitrogen atmosphere. After addition, the reaction mixture was stirred at room temperature under nitrogen for 2 hours. After the reaction was confirmed complete by LC-MS monitoring, ethyl acetate and water (30 mL) were added. The mixture was extracted with ethyl acetate. The combined organic phases were washed with water and brine, dried over anhydrous sodium sulfate, filtered, and concentrated. The residue was purified by silica gel column chromatography (DCM/MeOH = 93/7) to afford compound **A135-5** (86 mg, 84%) as a white solid.
LCMS: [M+H]⁺ = 706.2

### Synthesis of Intermediate Compound A135-6

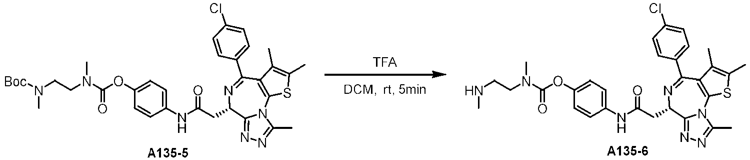

Compound **A135-5** (86 mg, 0.122 mmol) was dissolved in DCM (3 mL). Trifluoroacetic acid (1 mL) was added to the reaction mixture. The mixture was stirred at room temperature for 5 minutes. After the reaction was confirmed complete by LC-MS monitoring, the organic phase was concentrated to afford crude compound **A135-6,** which was used directly in the next step. LCMS: [M+H]⁺ = 607.8

### Synthesis of Intermediate Compound A135-8

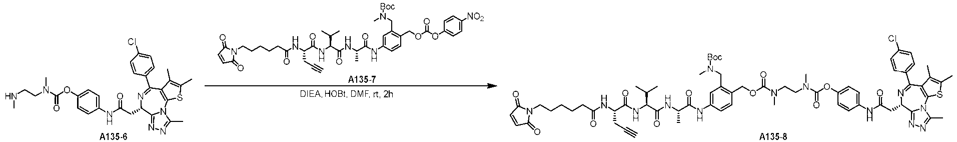

Compound **A135-6** (60.0 mg, 0.099 mmol, 1.0 eq) and N,N-Diisopropylethylamine (76.77 mg, 0.594 mmol, 6.0 eq) were dissolved in DMF (5 mL). After stirring until clear, compound **A135-7** (88.2 mg, 0.099 mmol, 1.0 eq) and HOBt (2.68 mg, 0.019 mmol, 0.2 eq) were added. After addition, the reaction mixture was stirred at room temperature for 2 hours. After the reaction was confirmed complete by LC-MS monitoring, water (30 mL) was added. The mixture was extracted with ethyl acetate. The combined organic phases were washed with water and brine, dried over anhydrous sodium sulfate, filtered, and concentrated. The residue was purified by silica gel column chromatography (DCM/MeOH = 93/7) to afford compound **A135-8** (108 mg, 72%) as a yellow solid.
LCMS: [M+H]⁺ = 1357.2

### Synthesis of Intermediate Compound A135-9

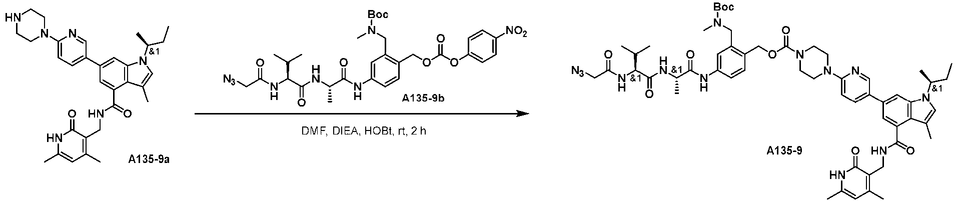

Compound **A135-9a** (100 mg, 0.190 mmol, 1.0 eq) and N,N-Diisopropylethylamine (73.5 mg, 0.567 mmol, 3.0 eq) were dissolved in DMF (3 mL). After stirring until clear, compound A135-9b (130 mg, 0.190 mmol, 1.0 eq) and HOBt (25.6 mg, 0.190 mmol, 1.0 eq) were added. After addition, the reaction mixture was stirred at room temperature for 2 hours. After the reaction was confirmed complete by LC-MS monitoring, water (40 mL) was added. The mixture was extracted with ethyl acetate. The combined organic phases were washed with water and brine, dried over anhydrous sodium sulfate, filtered, and concentrated. The residue was purified by silica gel column chromatography (DCM/MeOH = 93/7) to afford compound **A135-9** (180 mg, 87%) as a white solid.
LCMS: [M+H]⁺ = 1072.5

### Synthesis of Intermediate Compound A135-10

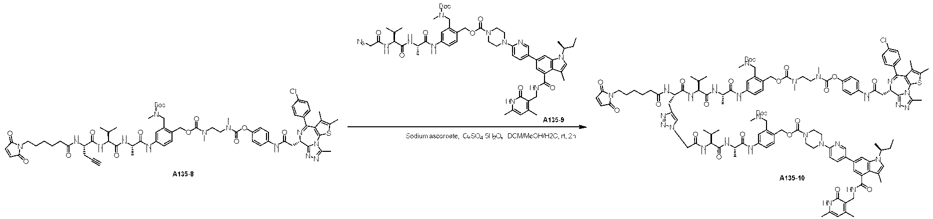

Compound **A135-8** (98 mg, 0.072 mmol, 1.0 eq) and **A135-9** (77.49 mg, 0.072 mmol, 1.0 eq) were dissolved in a mixture of dichloromethane/methanol/water (5/5/1 mL). Copper(II) sulfate pentahydrate (1.81 mg, 0.0072 mmol, 0.1 eq, in 100 µL water) and sodium ascorbate (2.86 mg, 0.014 mmol, 0.2 eq, in 100 µL water) were added under a nitrogen atmosphere. After addition, the reaction mixture was stirred at room temperature under nitrogen for 1 hour. After the reaction was confirmed complete by LC-MS monitoring, the reaction mixture was concentrated under reduced pressure, and tetrahydrofuran (30 mL) was added and evaporated three times to remove water, affording compound **A135-10** (132 mg, 75%) as a yellow solid. LCMS: [M+H]⁺ = 1215.7 (half peak)

### Synthesis of Intermediate Compound A135-11

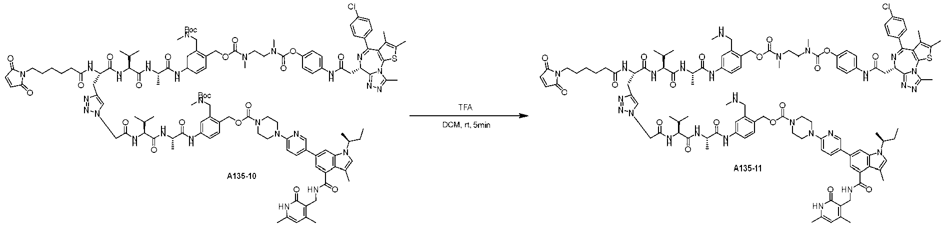

Compound **A135-10** (132 mg, 0.0543 mmol) was dissolved in DCM (3 mL). Trifluoroacetic acid (1 mL) was added to the reaction mixture. The mixture was stirred at room temperature for 5 minutes. After the reaction was confirmed complete by LC-MS monitoring, the organic phase was concentrated to afford crude compound **A135-11,** which was used directly in the next step.
LCMS: [M+H]⁺ = 1115.2 (half peak)

### Synthesis of Target Compound A135

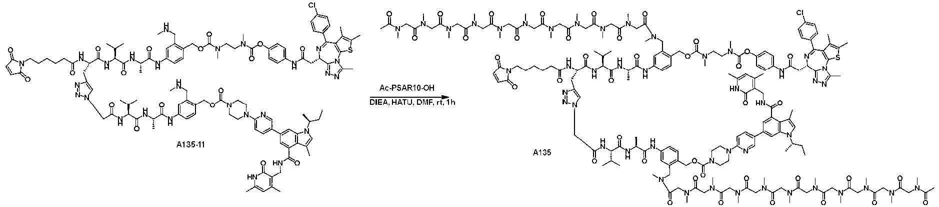

The compound **A135-11** from the previous step was dissolved in DMF (3 mL). Compound Ac-PSAR10-OH (83.78 mg, 0.109 mmol, 2.0 eq) and N,N-Diisopropylethylamine (150.69 mg, 1.166 mmol, 20.0 eq) were added. Finally, O-(7-Azabenzotriazol-1-yl)-N,N,N',N'-tetramethyluronium hexafluorophosphate (46.55 mg, 0.122 mmol, 2.1 eq) was added under a nitrogen atmosphere. After addition, the reaction mixture was stirred at room temperature under nitrogen for 1 hour. After the reaction was confirmed complete by LC-MS monitoring, ethyl acetate (35 mL) was added. The resulting suspension was filtered, and the filter cake was dissolved in DMF. Purification by preparative HPLC (mobile phase: 0.05% HCOOH/CH₃CN/H₂O) afforded Compound **A135** (49.7 mg, 36%) as a white solid.
LCMS: [M+H]⁺ = 1867.3 (half peak)

### 27) Synthesis of Compound A136

### Synthetic Steps:

### Synthesis of Intermediate Compound A136-3

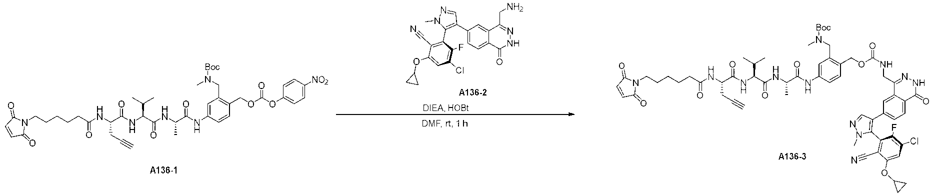

Compound A136-2 (250 mg, 0.54 mmol, 1.0 eq) and N,N-Diisopropylethylamine (209 mg, 1.61 mmol, 3.0 eq) were dissolved in DMF (8 mL). After stirring until clear, compound A136-1 (455 mg, 0.51 mmol, 0.95 eq) and HOBt (73 mg, 0.54 mmol, 1.0 eq) were added. After addition, the reaction mixture was stirred at room temperature for 1 hour. After the reaction was confirmed complete by LC-MS monitoring, ethyl acetate (50 mL) was added. The mixture was washed twice with water and three times with brine. The organic phase was dried over anhydrous sodium sulfate, filtered, and concentrated. The residue was purified by silica gel column chromatography (DCM/MeOH = 93/7) to afford compound A136-3 (380 mg, 58%) as a pale yellow solid.
LCMS: [M+Na]⁺ = 1237.2

### Synthesis of Intermediate Compound A136-6

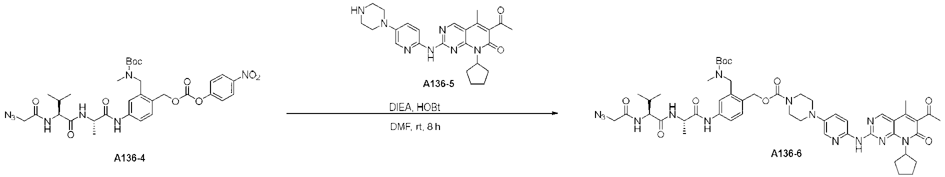

Compound A136-5 (150 mg, 0.335 mmol, 1.0 eq) and N,N-Diisopropylethylamine (130 mg, 1.006 mmol, 3.0 eq) were dissolved in DMF (6 mL). After stirring until clear, compound A136-4 (218 mg, 0.32 mmol, 0.95 eq) and HOBt (45 mg, 0.335 mmol, 1.0 eq) were added. After addition, the reaction mixture was stirred at room temperature for 8 hours. After the reaction was confirmed complete by LC-MS monitoring, ethyl acetate (40 mL) was added. The mixture was washed twice with water and three times with brine. The organic phase was dried over anhydrous sodium sulfate, filtered, and concentrated. The residue was purified by silica gel column chromatography (DCM/MeOH = 96/4) to afford compound A136-6 (260 mg, 78%) as a yellow solid.
LCMS: [M+H]⁺ = 993.4

### Synthesis of Intermediate Compound A136-7

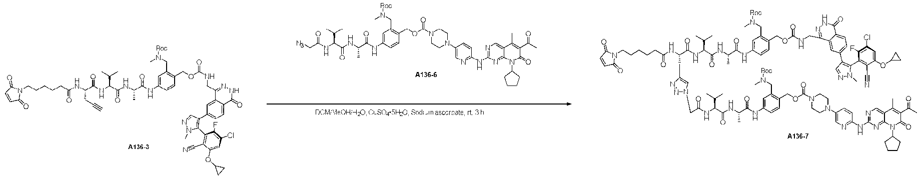

Compound A136-3 (100 mg, 0.082 mmol, 1.0 eq) and compound A136-6 (82 mg, 0.082 mmol, 1.0 eq) were dissolved in a mixture of MeOH/DCM/water (5/5/1 mL). Then, 100 µL of an aqueous solution of copper(II) sulfate pentahydrate (2.1 mg, 0.008 mmol, 0.1 eq) and 100 µL of an aqueous solution of sodium ascorbate (3.3 mg, 0.016 mmol, 0.2 eq) were added. After addition, the reaction mixture was stirred at room temperature under nitrogen for 3 hours. After product formation was observed by LC-MS monitoring, water (20 mL) was added. The mixture was extracted with dichloromethane. The combined organic phases were dried over anhydrous sodium sulfate, filtered, and concentrated to afford crude compound A136-7 (181 mg, 99%) as a yellow solid.
LCMS: [M+H]⁺ = 1104.4 (half peak)

### Synthesis of Intermediate Compound A136-8

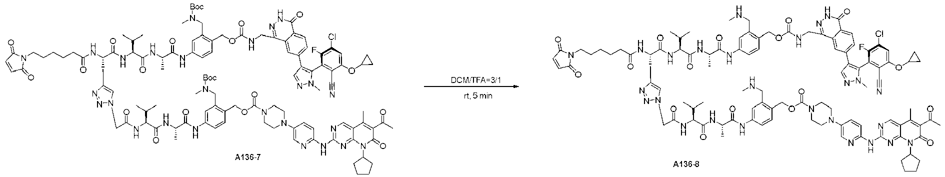

Compound A136-7 (130 mg, 0.0589 mmol, 1.0 eq) was dissolved in DCM (3 mL). Trifluoroacetic acid (1 mL) was added to the reaction mixture. The mixture was stirred at room temperature for 5 minutes. After the reaction was confirmed complete by LC-MS monitoring, the organic phase was concentrated to afford crude compound A136-8, which was used directly in the next step.
LCMS: [M+H]⁺ = 1004.5 (half peak).

### Synthesis of Target Compound A136

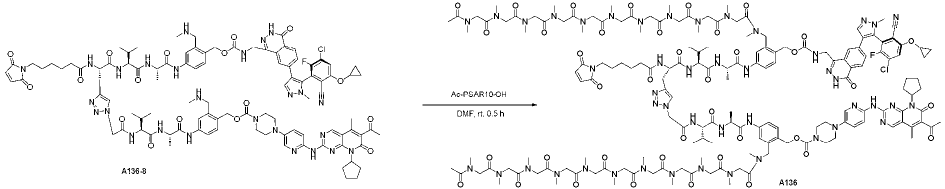

The compound A136-8 from the previous step was dissolved in DMF (4 mL). N,N-Diisopropylethylamine (152 mg, 1.176 mmol, 20.0 eq) was added. Then, Ac-PSAR10-OH (91 mg, 0.118 mmol, 2.0 eq) and O-(7-Azabenzotriazol-1-yl)-N,N,N',N'-tetramethyluronium hexafluorophosphate (47 mg, 0.123 mmol, 2.1 eq) were added to the reaction mixture. After addition, the reaction mixture was stirred at room temperature under nitrogen for 0.5 hour. After the reaction was confirmed complete by LC-MS monitoring, EA (40 mL) was added. The mixture was filtered, and the filter cake was dissolved in DMF. Purification by preparative HPLC (mobile phase: 0.05% HCOOH/CH₃CN/H₂O) afforded Compound A136 (76.7 mg, 37%) as a yellow solid.
LCMS: [M+H]⁺ = 1757.2 (half peak)

### 28) Synthesis of Compound A80

### Synthetic Route:

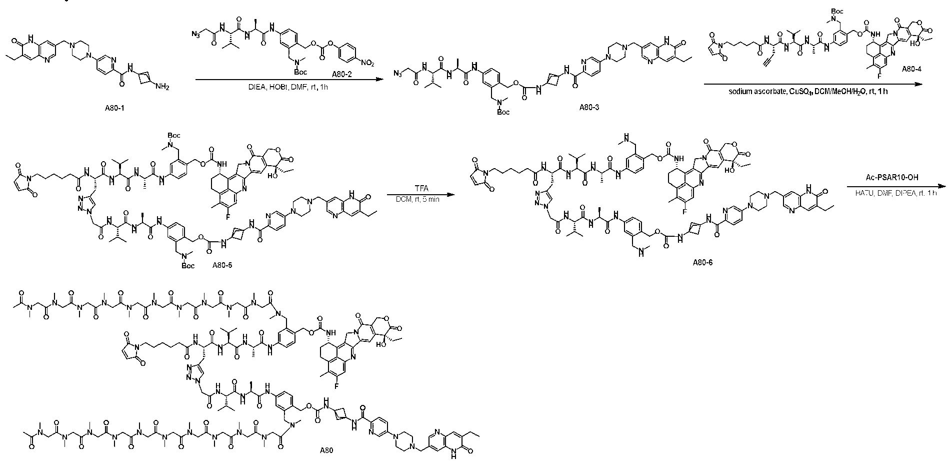

### Synthetic Steps:

### Synthesis of Intermediate Compound A80-3

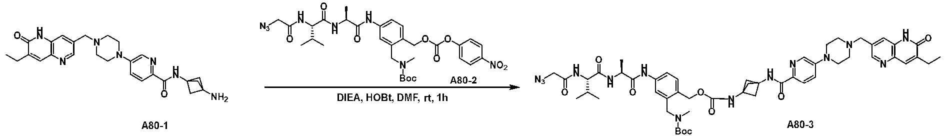

Compound **A80-1** (370 mg, 0.781 mmol, 1.0 eq) and N,N-Diisopropylethylamine (605.9 mg, 4.688 mmol, 6.0 eq) were dissolved in DMF (5 mL). After stirring until clear, compound **A80-2** (535.7 mg, 0.7813 mmol, 1.0 eq) and HOBt (52.8 mg, 0.391 mmol, 0.5 eq) were added. After addition, the reaction mixture was stirred at room temperature for 1 hour. After the reaction was confirmed complete by LC-MS monitoring, ethyl acetate (50 mL) and water (30 mL) were added. The layers were separated, and the aqueous layer was extracted with ethyl acetate. The combined organic phases were washed with water and brine, dried over anhydrous sodium sulfate, filtered, and concentrated. The residue was purified by silica gel column chromatography (DCM/MeOH = 93/7) to afford compound **A80-3** (351 mg, 40%) as a yellow solid.

LCMS: [M+H]⁺ = 1019.5.

### Synthesis of Intermediate Compound A80-5

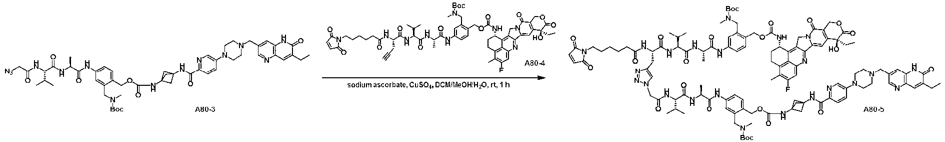

Compound **A80-3** (200 mg, 0.196 mmol, 1.0 eq) and **A80-4** (232.7 mg, 0.196 mmol, 1.0 eq) were dissolved in a mixture of dichloromethane/methanol/water (5/5/1 mL). Copper(II) sulfate pentahydrate (3.1 mg, 0.0196 mmol, 0.1 eq, in 100 µL water) and sodium ascorbate (7.8 mg, 0.0392 mmol, 0.2 eq, in 100 µL water) were added under a nitrogen atmosphere. After addition, the reaction mixture was stirred at room temperature under nitrogen for 1 hour. After the reaction was confirmed complete by LC-MS monitoring, the reaction mixture was concentrated under reduced pressure, and tetrahydrofuran (30 mL) was added and evaporated three times to remove water, affording compound **A80-5** (430 mg, 99%) as a yellow solid.

LCMS: [M+H]⁺ = 1103.6 (half peak).

### Synthesis of Intermediate Compound A80-6

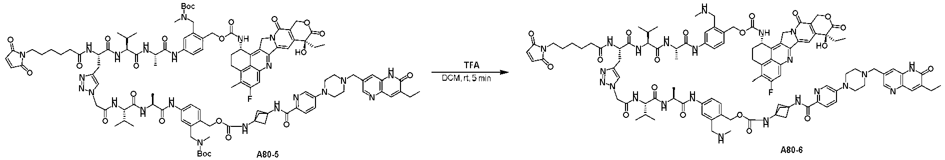

Compound **A80-5** (430 mg, 0.195 mmol) was dissolved in DCM (3 mL). Trifluoroacetic acid (1 mL) was added to the reaction mixture. The mixture was stirred at room temperature for 5 minutes. After the reaction was confirmed complete by LC-MS monitoring, the organic phase was concentrated to afford crude compound **A80-6,** which was used directly in the next step.

LCMS: [M+H]⁺ = 1003.4 (half peak).

### Synthesis of Target Compound A80

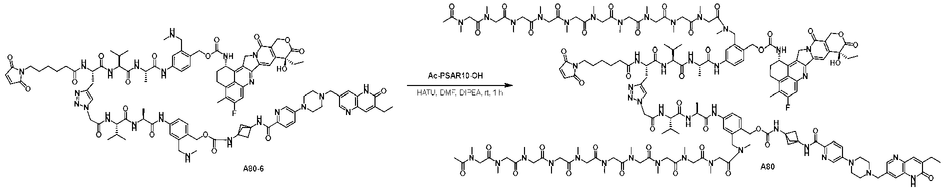

Compound **A80-6** (200 mg, 0.099 mmol, 1.0 eq) and N,N-Diisopropylethylamine (257.8 mg, 1.99 mmol, 20 eq) were dissolved in DMF (5 mL). After stirring until clear, compound **Ac-PSAR10-OH** (153.6 mg, 0.199 mmol, 2.0 eq) and HATU (79.6 mg, 0.209 mmol, 2.1 eq) were added. After addition, the reaction mixture was stirred at room temperature for 2 hours. After the reaction was confirmed complete by LC-MS monitoring, ethyl acetate (80 mL) was added. The mixture was filtered, and the filter cake was dissolved in DMF. Purification by preparative HPLC (mobile phase: 0.05% HCOOH/CH₃CN/H₂O) afforded Compound **A80** (60.2 mg, 27%) as a white solid.

LCMS: [M+H]⁺ = 1756.6 (half peak).

### 29) Synthesis of Compound A90

### Synthetic Route:

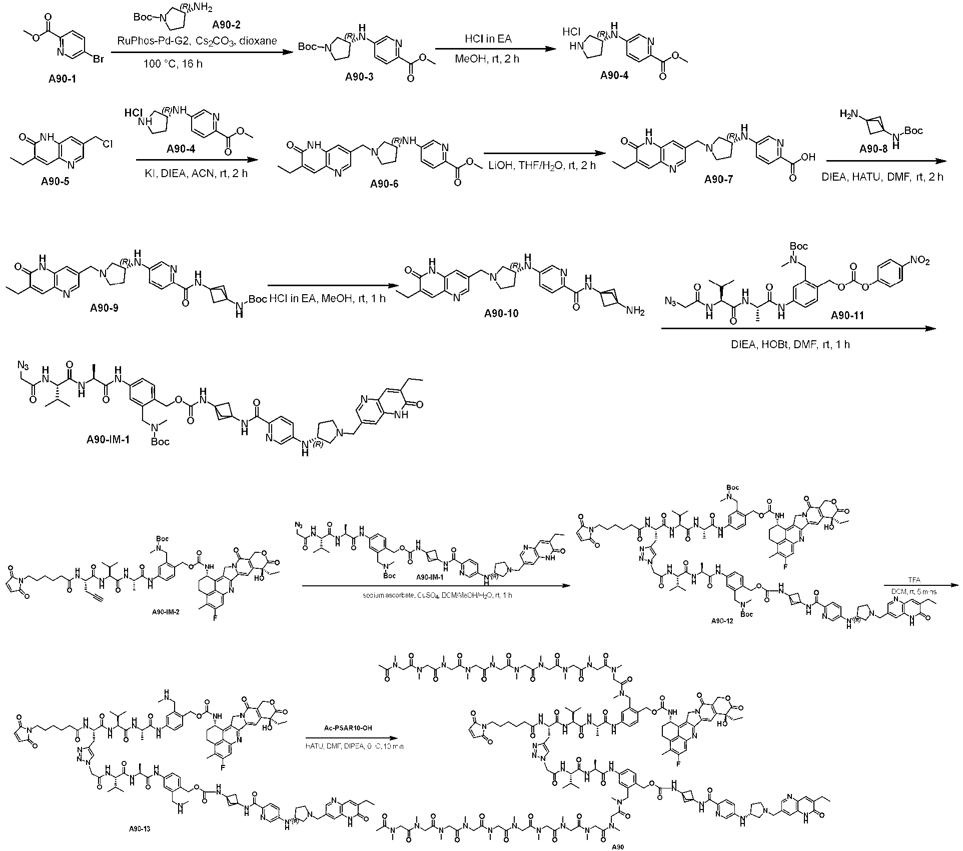

### Synthetic Steps:

### Synthesis of Intermediate Compound A90-3

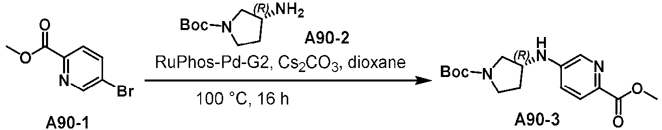

Compound **A90-1** (2.0 g, 9.30 mmol, 1.0 eq), compound **A90-2** (3.5 g, 18.60 mmol, 2.0 eq) and Cs₂CO₃ (9.1 g, 27.90 mmol, 3.0 eq) were dissolved in 1,4-dioxane (50 mL). Then, RuPhos-Pd-G2 (721 mg, 0.93 mmol, 0.1 eq) was added to the reaction mixture. After addition, nitrogen was purged, and the reaction mixture was stirred at 100°C under nitrogen for 16 hours. After the reaction was confirmed complete by LC-MS monitoring, the mixture was filtered, and the solid was washed with ethyl acetate. The organic phase was washed with saturated ammonium chloride, then water and brine. The organic phase was dried over anhydrous sodium sulfate, filtered, and concentrated. The residue was purified by silica gel column chromatography (PE/EA = 1/1) to afford compound **A90-3** (2.3 g, 77%) as a white solid.

LCMS: [M+H]⁺ = 322.2.

### Synthesis of Intermediate Compound A90-4

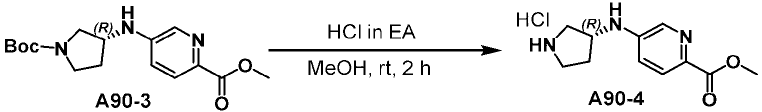

Compound **A90-3** (1.2 g, 3.73 mmol) was dissolved in MeOH (5 mL). Then, a 4.0 M HCl in EA (10 mL) was added to the reaction mixture. The mixture was stirred at room temperature for 2 hours. After the reaction was confirmed complete by LC-MS monitoring, the mixture was concentrated directly. The residue was triturated with ethyl acetate, filtered, and the filter cake was collected to afford compound **A90-4** (900 mg, 92%) as a white solid.

LCMS: [M+H]⁺ = 222.1.

### Synthesis of Intermediate Compound A90-6

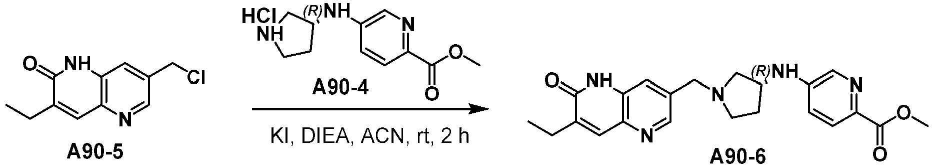

Compound **A90-5** (500 mg, 2.246 mmol, 1.0 eq), compound **A90-4** (578.7 mg, 2.246 mmol, 1.0 eq), KI (744.9 mg, 4.491 mmol, 2.0 eq) and N,N-Diisopropylethylamine (1.451 g, 11.228 mmol, 5.0 eq) were dissolved in a mixture of acetonitrile (15 mL) and water (15 mL). After addition, the reaction mixture was stirred at room temperature for 2 hours. After the reaction was confirmed complete by LC-MS monitoring, the mixture was diluted with ethyl acetate and washed with water and brine. The organic phase was dried over anhydrous sodium sulfate, filtered, and concentrated. The residue was purified by silica gel column chromatography (PE/EA = 7/3) to afford compound **A90-6** (900 mg, 97%) as a white solid.

LCMS: [M+H]⁺ = 408.2.

### Synthesis of Intermediate Compound A90-7

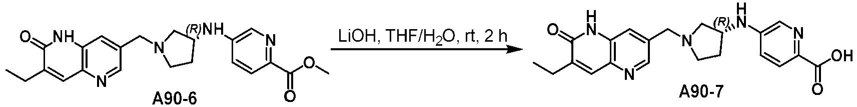

Compound **A90-6** (900 mg, 2.209 mmol, 1.0 eq) was dissolved in tetrahydrofuran (10 mL). Then, a 10 mL aqueous solution of LiOH (158.7 mg, 6.626 mmol, 3.0 eq) was added. The mixture was stirred at room temperature for 2 hours. After the reaction was confirmed complete by LC-MS monitoring, the mixture was filtered, and the filter cake was washed with water to afford compound **A90-7** (1000 mg, 98%) as a white solid.

LCMS: [M+H]⁺ = 394.2.

### Synthesis of Intermediate Compound A90-9

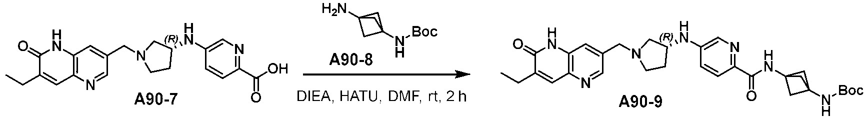

Compound **A90-7** (400 mg, 1.017 mmol, 1.0 eq), compound **A90-8** (202.6 mg, 1.017 mmol, 1.0 eq) and N,N-Diisopropylethylamine (394.2 mg, 3.050 mmol, 3.0 eq) were dissolved in DMF (5 mL). Then, HATU (386.6 mg, 1.017 mmol, 1.0 eq) was added. After addition, the reaction mixture was stirred at room temperature for 2 hours. After the reaction was confirmed complete by LC-MS monitoring, the mixture was diluted with ethyl acetate and washed with water and brine. The organic phase was dried over anhydrous sodium sulfate, filtered, and concentrated. The residue was purified by silica gel column chromatography (PE/EA = 7/3) to afford compound **A90-9** (220 mg, 37%) as a white solid.

LCMS: [M+H]⁺ = 574.3.

### Synthesis of Intermediate Compound A90-10

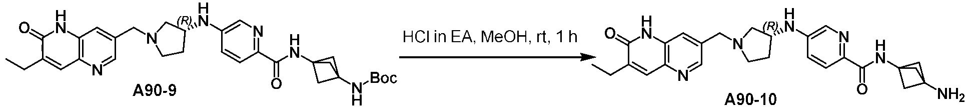

Compound **A90-9** (200 mg, 0.348 mmol) was dissolved in MeOH (2 mL). Then, a HCl in EA (4 mL) was added to the reaction mixture. The mixture was stirred at room temperature for 1 hour. After the reaction was confirmed complete by LC-MS monitoring, the organic phase was concentrated to afford crude compound **A90-10,** which was used directly in the next step.

LCMS: [M+H]⁺ = 474.3.

### Synthesis of Intermediate Compound A90-IM-1

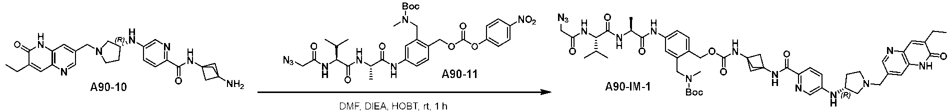

The compound **A90-10** from the previous step was dissolved in DMF (3 mL). Then, compound **A90-11** (238.9 mg, 0.348 mmol, 1.0 eq), HOBt (47.0 mg, 0.348 mmol, 1.0 eq) and N,N-Diisopropylethylamine (135.0 mg, 1.044 mmol, 3.0 eq) were added. After addition, the reaction mixture was stirred at room temperature for 1 hour. After the reaction was confirmed complete by LC-MS monitoring, the mixture was diluted with ethyl acetate and washed with water and brine. The organic phase was dried over anhydrous sodium sulfate, filtered, and concentrated. The residue was purified by silica gel column chromatography (DCM/MeOH = 92/8) to afford compound **A90-IM-1** (290 mg, 80%) as a white solid.

LCMS: [M+H]⁺ = 1020.0.

### Synthesis of Intermediate Compound A90-12

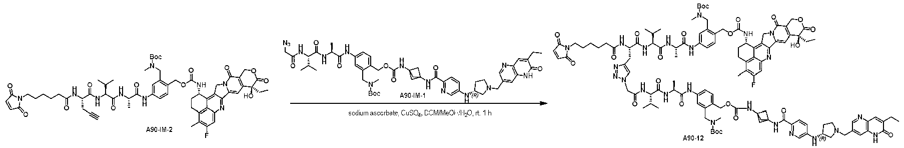

Compound **A90-IM-2** (120 mg, 0.1011 mmol, 1.0 eq) and compound **A90-IM-1** (103.1 mg, 0.1011 mmol, 1.0 eq) were dissolved in a mixture of MeOH/DCM/water (5/5/1 mL). Then, 100 µL of an aqueous solution of copper(II) sulfate pentahydrate (2.53 mg, 0.01011 mmol, 0.1 eq) and 100 µL of an aqueous solution of sodium ascorbate (4.01 mg, 0.02022 mmol, 0.2 eq) were added. After addition, the reaction mixture was stirred at room temperature under nitrogen for 1 hour. After the reaction was confirmed complete by LC-MS monitoring, water (30 mL) was added. The mixture was extracted with dichloromethane. The combined organic phases were washed with water and brine, dried over anhydrous sodium sulfate, filtered, and concentrated to afford compound **A90-12** (220 mg, 98%) as a yellow solid.

LCMS: [M+Na]⁺ = 1103.3 (half peak).

### Synthesis of Intermediate Compound A90-12

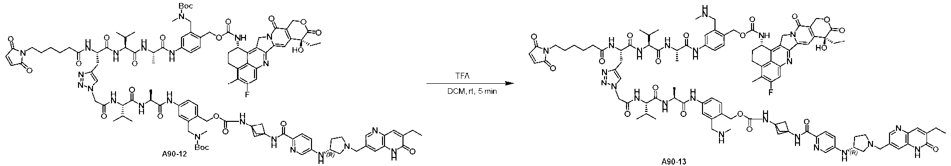

Compound **A90-12** (100 mg, 0.0453 mmol) was dissolved in DCM (3 mL). Trifluoroacetic acid (1 mL) was added to the reaction mixture. The mixture was stirred at room temperature for 5 minutes. After the reaction was confirmed complete by LC-MS monitoring, the organic phase was concentrated to afford crude compound **A90-13,** which was used directly in the next step.

LCMS: [M+H]⁺ = 1003.0 (half peak).

### Synthesis of Target Compound A90

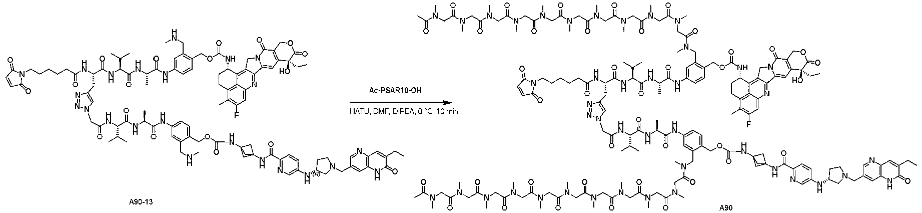

The compound **A90-13** from the previous step was dissolved in DMF (3 mL). Compound **Ac-PSAR10-OH** (69.8 mg, 0.0906 mmol, 2.0 eq) and N,N-Diisopropylethylamine (116.0 mg, 0.906 mmol, 20.0 eq) were added. Finally, HATU (36.2 mg, 0.0951 mmol, 2.1 eq) was added. After addition, the reaction mixture was stirred at 0°C for 10 minutes. After the reaction was confirmed complete by LC-MS monitoring, ethyl acetate (45 mL) was added. The mixture was filtered, and the filter cake was dissolved in DMF. Purification by preparative HPLC (mobile phase: 0.05% HCOOH/CH₃CN/H₂O) afforded Compound **A90** (38.1 mg, 23.9%) as a white solid.

LCMS: [M+H]⁺ = 1755.7 (half peak).

### 30) Synthesis of Compound A104

### Synthetic Route:

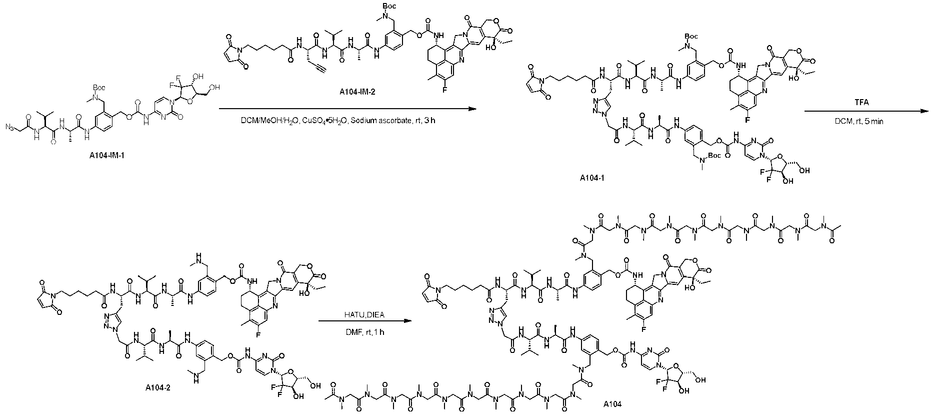

### Synthetic Steps:

### Synthesis of Intermediate Compound A104-1

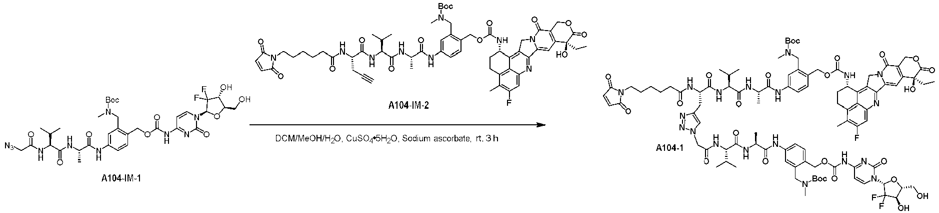

Compound **A104-IM-1** (90 mg, 0.111 mmol, 1.0 eq) and **A104-IM-2** (132 mg, 0.111 mmol, 1.0 eq) were dissolved in a mixture of dichloromethane/methanol/water (3/2/1 mL). Copper(II) sulfate pentahydrate (2.77 mg, 0.011 mmol, 0.1 eq, in 100 µL water) and sodium ascorbate (4.4 mg, 0.022 mmol, 0.20 eq, in 100 µL water) were added under a nitrogen atmosphere. After addition, the reaction mixture was stirred at room temperature under nitrogen for 3 hours. After the reaction was confirmed complete by LC-MS monitoring, the mixture was concentrated under reduced pressure and triturated with ethyl acetate to afford compound **A104-1** (217 mg, 88%) as a yellow solid.
LCMS: [M+H]⁺ = 1995.8

### Synthesis of Intermediate Compound A104-2

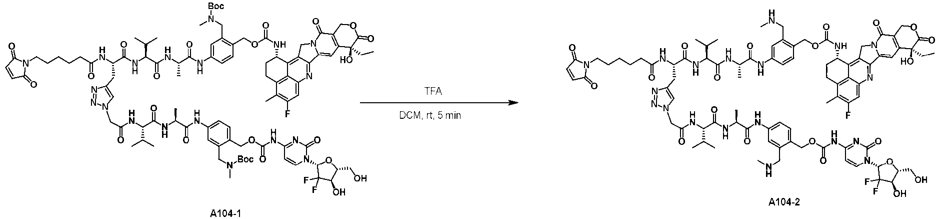

Compound **A104-3** (150 mg, 0.075 mmol, 1.0 eq) was dissolved in DCM (2 mL). Trifluoroacetic acid (0.6 mL) was added to the reaction mixture. The mixture was stirred at room temperature for 5 minutes. After the reaction was confirmed complete by LC-MS monitoring, the organic phase was concentrated to afford crude compound **A104-2,** which was used directly in the next step.
LCMS: [M+H]⁺ = 1794.5

### Synthesis of Target Compound A104

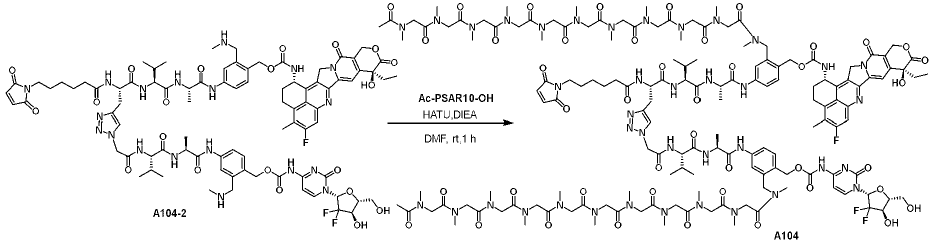

The compound **A104-2** from the previous step was dissolved in DMF (3 mL). **Ac-PSAR10-OH** (115 mg, 0.15 mmol, 2.0 eq) and N,N-Diisopropylethylamine (96 mg, 0.75 mmol, 10.0 eq) were added. Finally, O-(7-Azabenzotriazol-1-yl)-N,N,N',N'-tetramethyluronium hexafluorophosphate (57 mg, 0.15 mmol, 2.0 eq) was added under a nitrogen atmosphere. After addition, the reaction mixture was stirred at room temperature under nitrogen for 1 hour. After the reaction was confirmed complete by LC-MS monitoring, the mixture was filtered. The filtrate was purified by preparative HPLC (mobile phase: 0.05% HCOOH/CH₃CN/H₂O) to afford Compound **A104** (30.2 mg, 12%) as a pale yellow solid.
LCMS: [M+H]⁺ = 1650.7 (half peak)

### 31) Synthesis of Compound A116

### Synthetic Route:

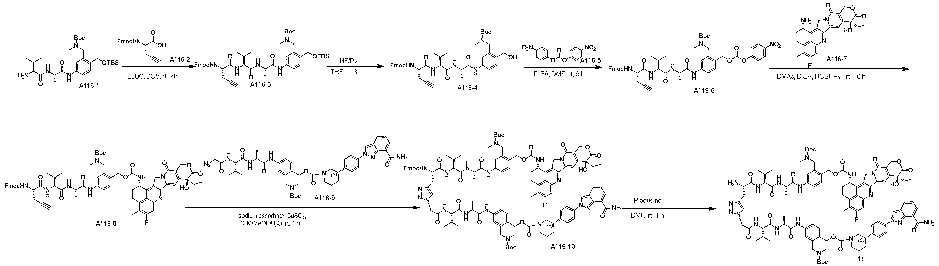

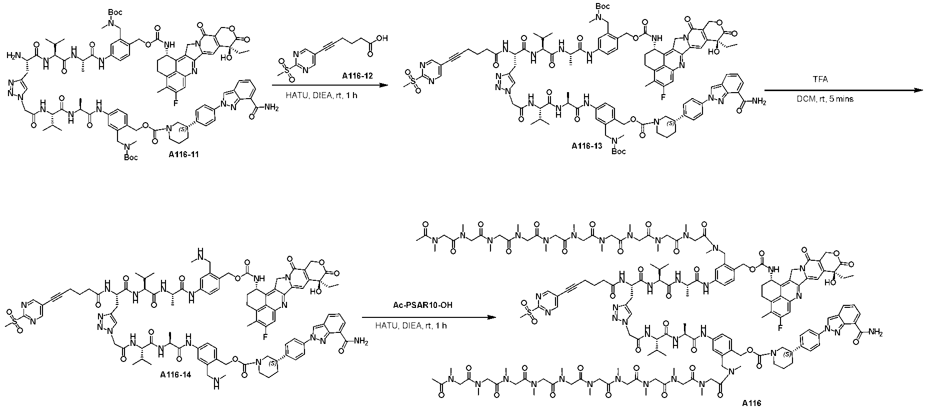

### Synthetic Steps

### Synthesis of Intermediate Compound A116-3

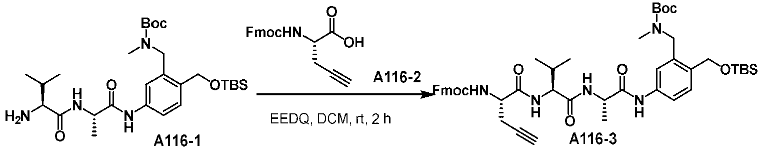

Compound **A116-1** (4.0 g, 7.2 mmol, 1.0 eq) was dissolved in dichloromethane (60 mL). Compound **A116-2** (2.4 g, 7.2 mmol, 1.0 eq) and 2-Ethoxy-1-ethoxycarbonyl-1,2-dihydroquinoline (3.6 g, 14.4 mmol, 2.0 eq) were added to the reaction mixture. The mixture was stirred at room temperature for 2 hours. After the reaction was confirmed complete by LC-MS monitoring, the mixture was concentrated directly. The residue was purified by silica gel column chromatography (PE/EA = 4/6) to afford compound **A116-3** (5.2 g, 89%) as a pale yellow solid.
LCMS: [M+Na]⁺ = 890.3

### Synthesis of Intermediate Compound A116-4

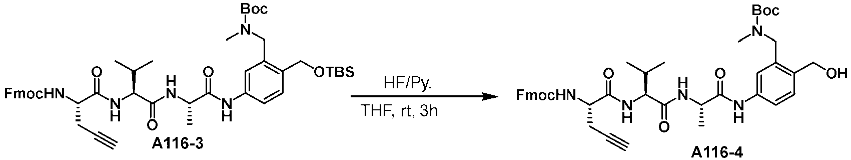

Compound **A116-3** (5.0 g, 5.77 mmol, 1.0 eq) was dissolved in THF (80 mL). The reaction mixture was cooled to 0°C, and pyridine hydrofluoride (65% w/w, 7.0 g, 46.16 mmol, 8.0 eq) was added under a nitrogen atmosphere. The mixture was stirred at room temperature under nitrogen for 3 hours. After the reaction was confirmed complete by LC-MS monitoring, water (100 mL) was added. The mixture was extracted with ethyl acetate. The combined organic phases were washed with water, then with saturated sodium bicarbonate solution, and finally with brine. The organic phase was dried over anhydrous sodium sulfate, filtered, and concentrated to afford compound **A116-4** (4.1 g, 94%) as a pale yellow solid.

LCMS: [M+Na]⁺ = 776.3.

### Synthesis of Intermediate Compound A116-6

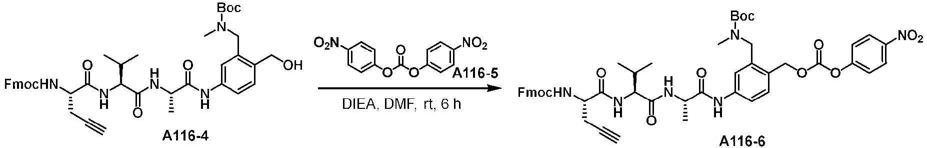

Compound **A116-4** (4.0 g, 5.31 mmol, 1.0 eq) and compound **A116-5** (3.2 g, 10.62 mmol, 2.0 eq) were dissolved in DMF (40 mL). The reaction mixture was cooled to 0°C, and N,N-Diisopropylethylamine (1.4 g, 10.62 mmol, 2.0 eq) was added under a nitrogen atmosphere. After addition, the reaction mixture was stirred at room temperature under nitrogen for 3 hours. After the reaction was confirmed complete by LC-MS monitoring, water (100 mL) was added. The mixture was extracted with ethyl acetate. The combined organic phases were washed with water and brine, dried over anhydrous sodium sulfate, filtered, and concentrated. The residue was triturated with methyl tert-butyl ether to afford compound **A116-6** (4.1 g, 85%) as a pale yellow solid.
LCMS: [M+Na]⁺ = 941.3

### Synthesis of Intermediate Compound A116-8

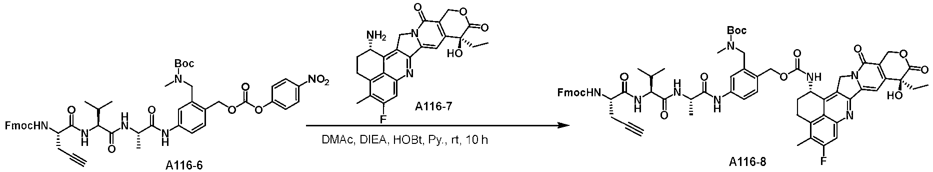

Compound **A116-7** (800 mg, 1.50 mmol, 1.0 eq) and N,N-Diisopropylethylamine (231 mg, 1.79 mmol, 1.2 eq) were dissolved in DMF (8 mL). After stirring until clear in an ice bath, compound **A116-6** (1.38 g, 1.50 mmol, 1.0 eq), HOBt (101 mg, 0.75 mmol, 0.5 eq) and pyridine (237 mg, 3.00 mmol, 2.0 eq) were added. After addition, the reaction mixture was stirred at room temperature under nitrogen for 10 hours. After the reaction was confirmed complete by LC-MS monitoring, water (30 mL) was added. The mixture was extracted with 2-methyltetrahydrofuran. The combined organic phases were washed with water and brine, dried over anhydrous sodium sulfate, filtered, and concentrated. The residue was purified by silica gel column chromatography (DCM/MeOH = 91/9) to afford compound **A116-8** (1.1 g, 60%) as a white solid.
LCMS: [M+H]⁺ = 1215.4

### Synthesis of Intermediate Compound A116-10

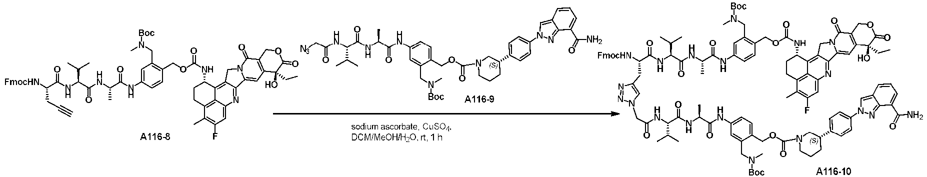

Compound **A116-8** (300 mg, 0.247 mmol, 1.0 eq) and **A116-9** (213 mg, 0.247 mmol, 1.0 eq) were dissolved in a mixture of dichloromethane/methanol/water (5/5/1 mL). Copper(II) sulfate pentahydrate (6.25 mg, 0.025 mmol, 0.1 eq, in 100 µL water) and sodium ascorbate (12.23 mg, 0.062 mmol, 0.25 eq, in 100 µL water) were added under a nitrogen atmosphere. After addition, the reaction mixture was stirred at room temperature under nitrogen for 1 hour. After the reaction was confirmed complete by LC-MS monitoring, the mixture was concentrated directly. The residue was dissolved in tetrahydrofuran and concentrated to dryness under reduced pressure to afford compound **A116-10** (513 mg, 100%) as a yellow solid.
LCMS: [M+H]⁺ = 1041.5 (half peak)

### Synthesis of Intermediate Compound A116-11

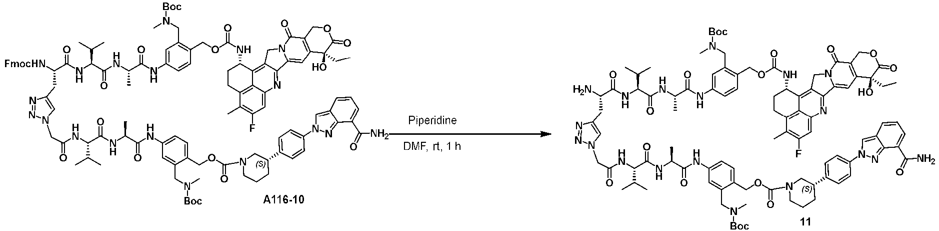

Compound **A116-10** (500 mg, 0.240 mmol, 1.0 eq) was dissolved in DMF (8 mL). Piperidine (1.6 mL) was added. After addition, the reaction mixture was stirred at room temperature under nitrogen for 11 hours. After the reaction was confirmed complete by LC-MS monitoring, the reaction mixture was poured into methyl tert-butyl ether (150 mL). The resulting suspension was filtered and washed with methyl tert-butyl ether. The filter cake was dried to afford compound **A116-11** (312 mg, 60%) as a brownish-yellow solid.
LCMS: [M+H]⁺ = 930.0 (half peak)

### Synthesis of Intermediate Compound A116-13

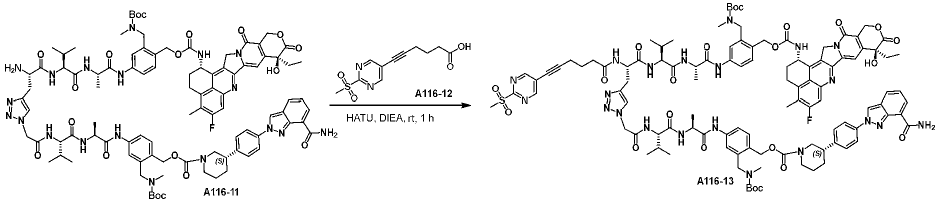

Compound **A116-12** (19 mg, 0.071 mmol, 1.1 eq) was dissolved in DMF (5 mL). O-(7-Azabenzotriazol-1-yl)-N,N,N',N'-tetramethyluronium hexafluorophosphate (29 mg, 0.077 mmol, 1.2 eq) and N,N-Diisopropylethylamine (25 mg, 0.194 mmol, 3.0 eq) were added, and the mixture was stirred for 5 minutes. Then, compound **A116-11** (120 mg, 0.065 mmol, 1.0 eq) was added. After addition, the reaction mixture was stirred at room temperature under nitrogen for 1 hour. After the reaction was confirmed complete by LC-MS monitoring, methyl tert-butyl ether (75 mL) was added. The resulting suspension was filtered and washed with methyl tert-butyl ether. The filter cake was dried to afford compound **A116-13** (120 mg, 88%) as a yellow solid.
LCMS: [M+H]⁺ = 1055.2 (half peak).

### Synthesis of Intermediate Compound A116-14

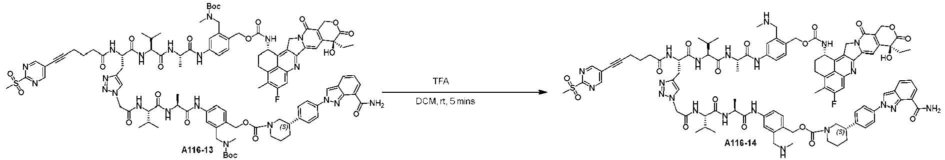

Compound **A116-13** (100 mg, 0.047 mmol, 1.0 eq) was dissolved in DCM (3 mL). Trifluoroacetic acid (1 mL) was added to the reaction mixture. The mixture was stirred at room temperature for 5 minutes. After the reaction was confirmed complete by LC-MS monitoring, the organic phase was concentrated to afford crude compound **A116-14,** which was used directly in the next step.

LCMS: [M+H]⁺ = 955.0 (half peak).

### Synthesis of Target Compound A116

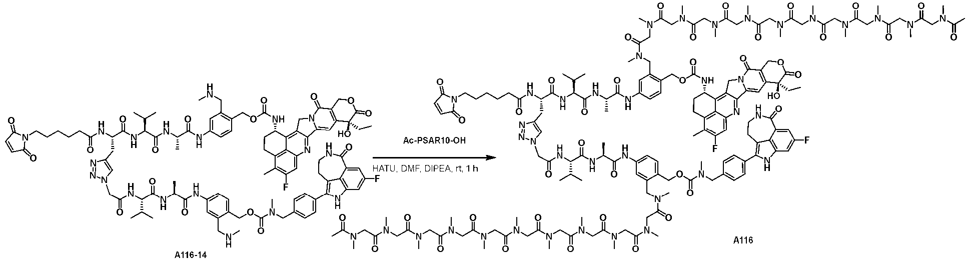

The compound **A116-14** from the previous step was dissolved in DMF (3 mL). Compound **Ac-PSAR10-OH** (72 mg, 0.094 mmol, 2.0 eq) and N,N-Diisopropylethylamine (121 mg, 0.94 mmol, 20.0 eq) were added. Finally, O-(7-Azabenzotriazol-1-yl)-N,N,N',N'-tetramethyluronium hexafluorophosphate (37 mg, 0.099 mmol, 2.1 eq) was added under a nitrogen atmosphere. After addition, the reaction mixture was stirred at room temperature under nitrogen for 1 hour. After the reaction was confirmed complete by LC-MS monitoring, ethyl acetate (45 mL) was added. The resulting suspension was filtered. The filter cake was dissolved in DMF and purified by preparative HPLC (mobile phase: 0.05% HCOOH/CH₃CN/H₂O) to afford target Compound **A116** (6.5 mg, 7%) as a yellow solid.
LCMS: [M+H]⁺ = 1707.8 (half peak)

### 32) Synthesis of Compound A117

### Synthetic Route:

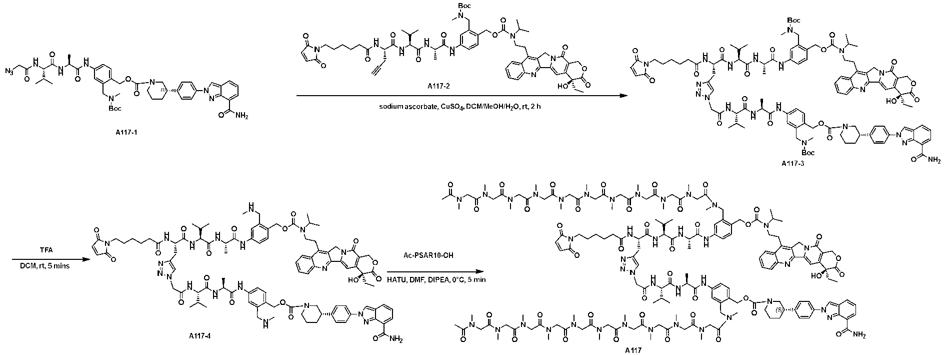

### Synthetic Steps:

### Synthesis of Intermediate Compound A117-3

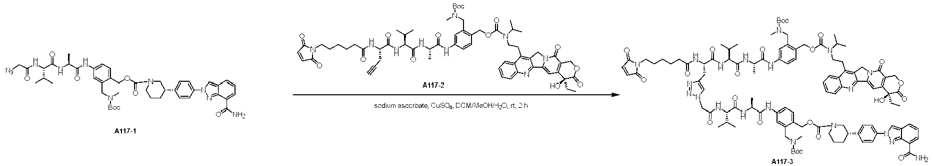

Compound **A117-1** (110 mg, 0.127 mmol, 1.0 eq) and compound **A117-2** (150 mg, 0.127 mmol, 1.0 eq) were dissolved in a mixture of MeOH/DCM/water (5/5/1 mL). Then, 100 µL of an aqueous solution of copper(II) sulfate pentahydrate (3.18 mg, 0.0127 mmol, 0.1 eq) and 100 µL of an aqueous solution of sodium ascorbate (5.03 mg, 0.0254 mmol, 0.2 eq) were added. After addition, the reaction mixture was stirred at room temperature under nitrogen for 2 hours. After the reaction was confirmed complete by LC-MS monitoring, the mixture was concentrated directly. The residue was dissolved in tetrahydrofuran and concentrated to dryness to afford compound **A117-3** (240 mg, 92%) as a yellow solid.
LCMS: [M+Na]⁺ = 1047.9 (half peak)

### Synthesis of Intermediate Compound A117-4

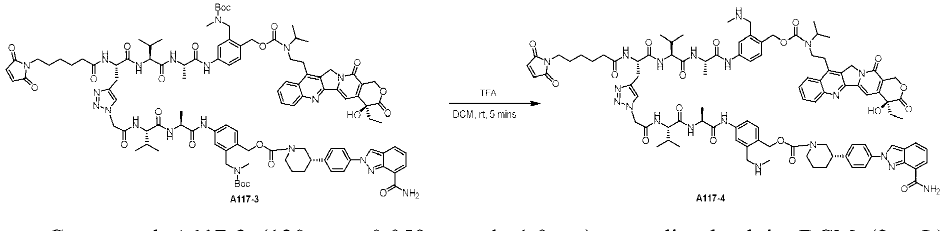

Compound **A117-3** (120 mg, 0.059 mmol, 1.0 eq) was dissolved in DCM (3 mL). Trifluoroacetic acid (1 mL) was added to the reaction mixture. The mixture was stirred at room temperature for 5 minutes. After the reaction was confirmed complete by LC-MS monitoring, the organic phase was concentrated to afford crude compound **A117-4,** which was used directly in the next step.
LCMS: [M+H]⁺ = 925.9 (half peak)

### Synthesis of Target Compound A117

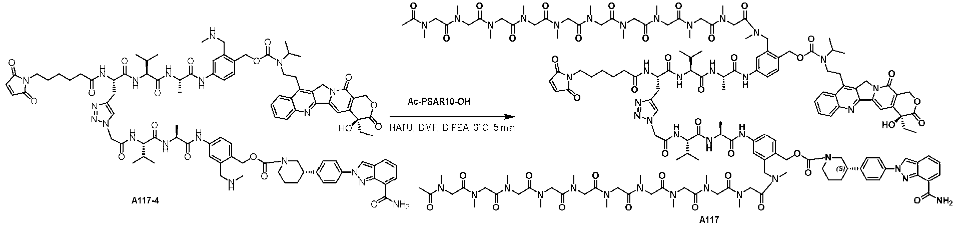

The compound **A117-4** from the previous step was dissolved in DMF (3 mL). Compound **Ac-PSAR10-OH** (90.1 mg, 0.117 mmol, 2.0 eq) and N,N-Diisopropylethylamine (151 mg, 1.17 mmol, 20.0 eq) were added. Finally, O-(7-Azabenzotriazol-1-yl)-N,N,N',N'-tetramethyluronium hexafluorophosphate (47 mg, 0.123 mmol, 2.1 eq) was added under a nitrogen atmosphere. The reaction mixture was stirred in an ice bath for 5 minutes. After the reaction was confirmed complete by LC-MS monitoring, ethyl acetate (60 mL) was added. The resulting suspension was filtered. The filter cake was dissolved in DMF and purified by preparative HPLC (mobile phase: 0.05% HCOOH/CH₃CN/H₂O) to afford Compound **A117** (55.4 mg, 28%) as a white solid.
LCMS: [M+H]⁺ = 1678.2 (half peak)

### 33) Synthesis of Compound A118

### Synthetic Route:

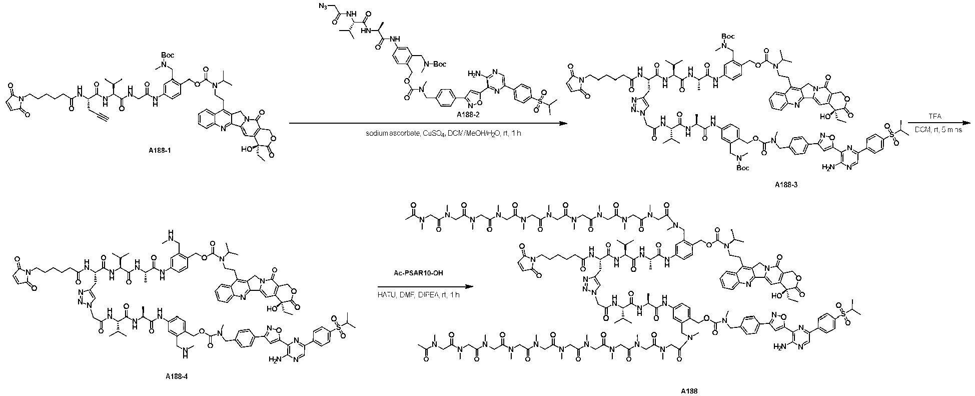

### Synthetic Steps:

### Synthesis of Intermediate Compound A118-3

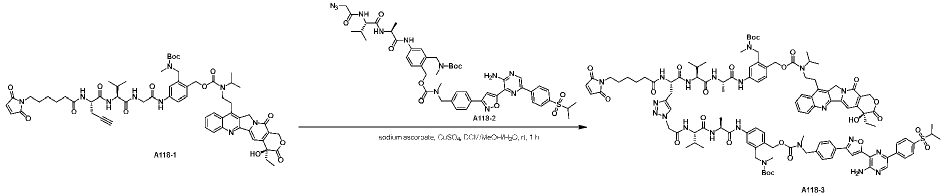

Compound **A118-1** (110 mg, 0.0928 mmol, 1.0 eq) and compound **A118-2** (93.7 mg, 0.0928 mmol, 1.0 eq) were dissolved in a mixture of MeOH/DCM/water (5/5/1 mL). Then, 100 µL of an aqueous solution of copper(II) sulfate pentahydrate (2.32 mg, 0.00928 mmol, 0.1 eq) and 100 µL of an aqueous solution of sodium ascorbate (3.368 mg, 0.01856 mmol, 0.2 eq) were added. After addition, the reaction mixture was stirred at room temperature under nitrogen for 1 hour. After the reaction was confirmed complete by LC-MS monitoring, water (30 mL) was added. The mixture was extracted with dichloromethane. The combined organic phases were washed with water and brine, dried over anhydrous sodium sulfate, filtered, and concentrated to afford compound **A118-3** (200 mg, 98%) as a yellow solid.
LCMS: [M+Na]⁺ = 1119.2 (half peak)

### Synthesis of Intermediate Compound A118-4

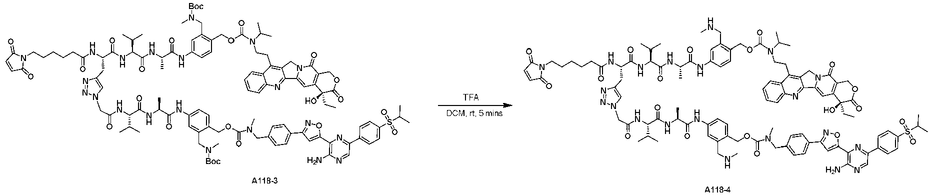

Compound **A118-3** (100 mg, 0.0455 mmol, 1.0 eq) was dissolved in DCM (3 mL). Trifluoroacetic acid (1 mL) was added to the reaction mixture. The mixture was stirred at room temperature for 5 minutes. After the reaction was confirmed complete by LC-MS monitoring, the organic phase was concentrated to afford crude compound **A118-4,** which was used directly in the next step.
LCMS: [M+H]⁺ = 997.0 (half peak)

### Synthesis of Target Compound A118

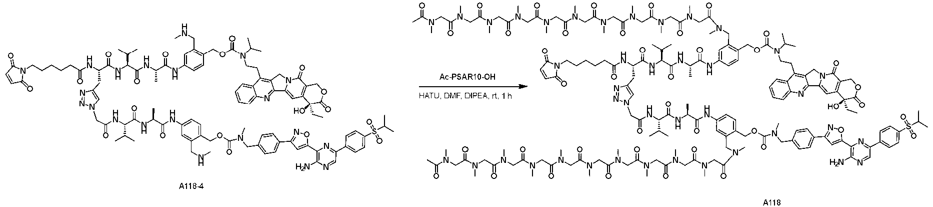

The compound **A118-4** from the previous step was dissolved in DMF (3 mL). Compound **Ac-PSAR10-OH** (70.1 mg, 0.091 mmol, 2.0 eq) and N,N-Diisopropylethylamine (117.4 mg, 0.910 mmol, 20.0 eq) were added. Finally, O-(7-Azabenzotriazol-1-yl)-N,N,N',N'-tetramethyluronium hexafluorophosphate (36.3 mg, 0.096 mmol, 2.1 eq) was added under a nitrogen atmosphere. After addition, the reaction mixture was stirred at room temperature under nitrogen for 1 hour. After the reaction was confirmed complete by LC-MS monitoring, ethyl acetate (45 mL) was added. The mixture was filtered. The filter cake was dissolved in DMF and purified by preparative HPLC (mobile phase: 0.05% HCOOH/CH₃CN/H₂O) to afford Compound **A118** (39.9 mg, 22.7%) as a yellow solid.
LCMS: [M+H]⁺ = 1749.7 (half peak)

### 34) Synthesis of Compound A119

### Synthetic Route:

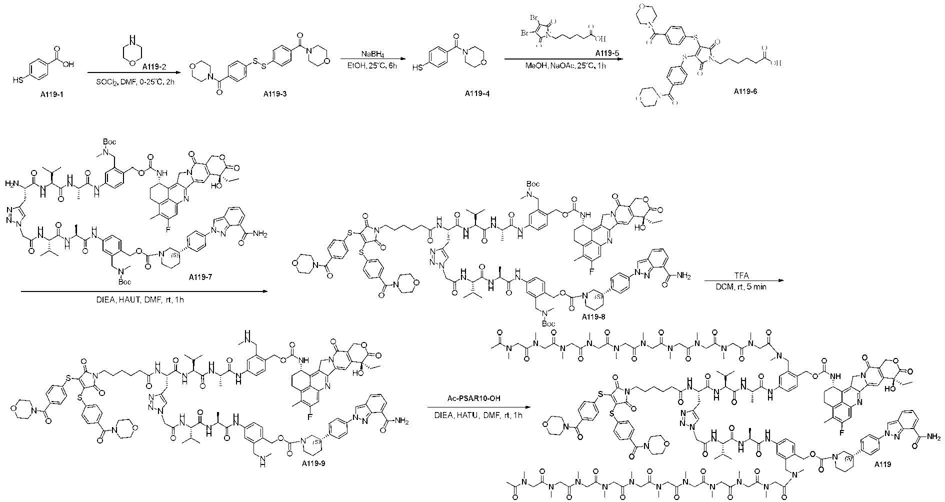

### Synthetic Steps:

### Synthesis of Intermediate Compound A119-3

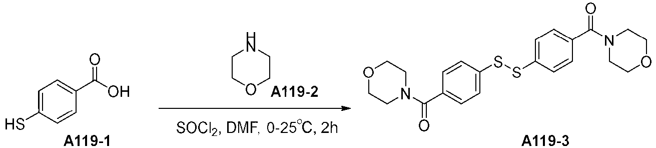

Compound **A119-1** (1.00 g, 6.5 mmol, 1.0 eq) was dissolved in thionyl chloride (20 mL). Under an ice bath, DMF (1 mL) and morpholine (28.31 g, 325 mmol, 50 eq) were added. After addition, the reaction mixture was stirred at room temperature for 2 hours. After the reaction was confirmed complete by LC-MS monitoring, dichloromethane (50 mL) and water (30 mL) were added. The layers were separated, and the aqueous layer was extracted with dichloromethane. The combined organic phases were washed with water and brine, dried over anhydrous sodium sulfate, filtered, and concentrated. The residue was purified by silica gel column chromatography (PE/EA = 1/1) to afford compound **A119-3** (1.4 g, 86%) as a yellow solid.
LCMS: [M+H]⁺ = 445.1

### Synthesis of Intermediate Compound A119-4

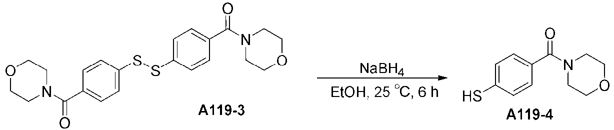

Compound **A119-3** (1.0 g, 2.25 mmol, 1.0 eq) and sodium borohydride (212.7 mg, 5.62 mmol, 2.5 eq) were dissolved in ethanol (5 mL). After addition, the reaction mixture was stirred at room temperature under nitrogen for 6 hours. After the reaction was confirmed complete by LC-MS monitoring, saturated ammonium chloride was added to quench the reaction. After evaporating the ethanol, the aqueous phase was extracted with ethyl acetate (10 mL × 3). The combined organic phases were washed with brine, dried over anhydrous sodium sulfate, and concentrated to afford compound **A119-4** (500 mg, 89%) as a yellow solid.

LCMS: [M+H]⁺ = 224.1.

### Synthesis of Intermediate Compound A119-6

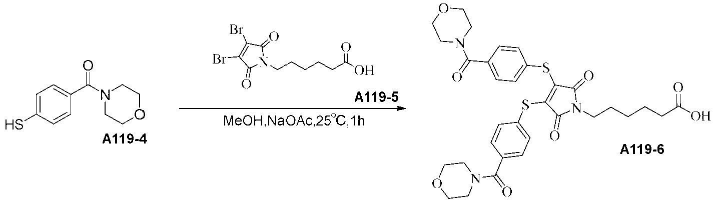

Compound **A119-5** (570 mg, 1.54 mmol, 1.0 eq) was dissolved in methanol (20 mL), and sodium acetate (266.2 mg, 3.24 mmol, 2.1 eq) was added. A methanol solution (10 mL) of compound **A119-4** (759 mg, 3.39 mmol, 2.2 eq) was added dropwise. After addition, the reaction mixture was stirred at room temperature for one hour. After the reaction was confirmed complete by LC-MS monitoring, 1 M dilute hydrochloric acid (10 mL) was added to quench the reaction. The mixture was extracted with ethyl acetate (20 mL). The organic phase was washed with brine, dried over anhydrous sodium sulfate, and concentrated. The residue was purified by silica gel column chromatography (PE/EA = 1/1) to afford compound **A119-6** (1.1 g, 98%) as a white solid.
LCMS: [M+H]⁺ = 654.2

### Synthesis of Intermediate Compound A119-8

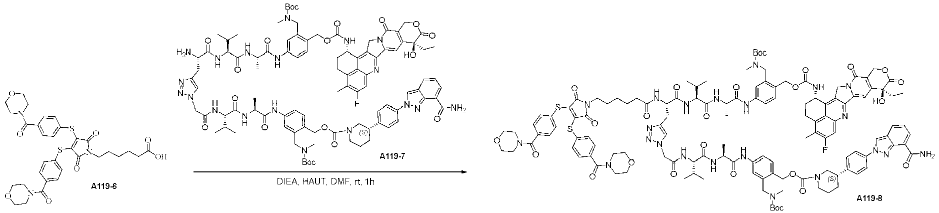

Compound **A119-6** (57 mg, 0.08 mmol, 1.0 eq) and DIPEA (33.8 mg, 0.26 mmol, 3 eq) were dissolved in DMF (2 mL). After stirring until clear, compound **A119-7** (154.2 mg, 0.08 mmol, 0.95 eq) and HATU (43.1 mg, 0.11 mmol, 1.3 eq) were added. After addition, the reaction mixture was stirred at room temperature for 2 hours. After the reaction was confirmed complete by LC-MS monitoring, methyl tert-butyl ether (20 mL) was added. The mixture was filtered, and the filter cake was washed with methyl tert-butyl ether and dried to afford compound **A119-8** (164 mg, 68%) as a white solid, which was used directly in the next step.

LCMS: [M+H]⁺ = 1247.9 (half peak)

### Synthesis of Intermediate Compound A119-9

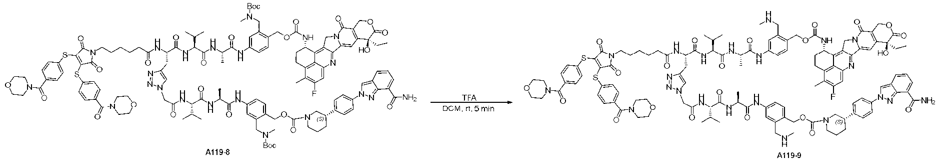

Compound **A119-8** (60 mg, 0.024 mmol) was dissolved in DCM (3 mL). Trifluoroacetic acid (1 mL) was added to the reaction mixture. The mixture was stirred at room temperature for 5 minutes. After the reaction was confirmed complete by LC-MS monitoring, the organic phase was concentrated to afford crude compound **A119-9,** which was used directly in the next step.

LCMS: [M+H]⁺ = 1148.1 (half peak)

### Synthesis of Target Compound A119

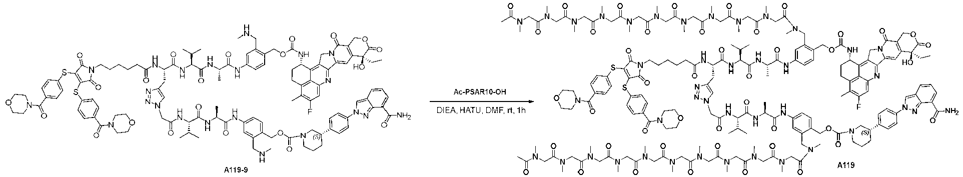

Compound **A119-9** (55 mg, 0.024 mmol, 1.0 eq) and N,N-Diisopropylethylamine (19.1 mg, 0.051 mmol, 20 eq) were dissolved in DMF (2 mL). After stirring until clear, Ac-PSAR10-OH (37 mg, 0.048 mmol, 2.0 eq) and HATU (19.16 mg, 0.051 mmol, 2.1 eq) were added. After addition, the reaction mixture was stirred at room temperature for 2 hours. After the reaction was confirmed complete by LC-MS monitoring, ethyl acetate (40 mL) was added. The mixture was filtered. The filter cake was dissolved in DMF and purified by preparative HPLC (mobile phase: 0.05% HCOOH/CH₃CN/H₂O) to afford Compound **A119** (11.2 mg, 19%) as a white solid. LCMS: [M+H]⁺ = 1900.2 (half peak)

### 40) Synthesis of Compound A120

### Synthetic Route:

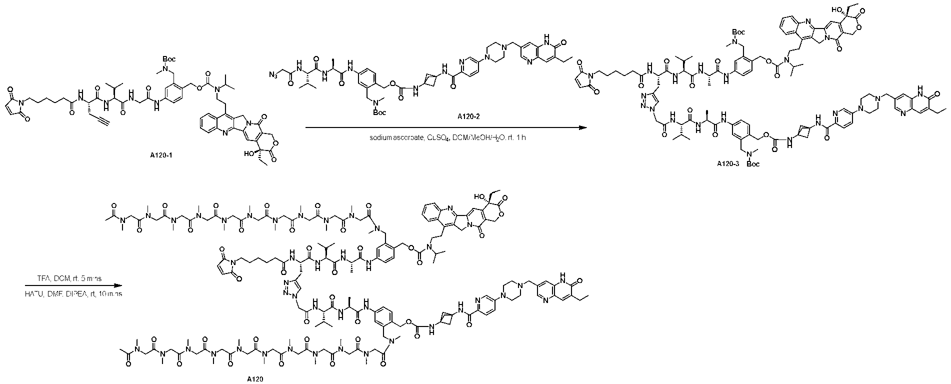

### Synthetic Steps:

### Synthesis of Intermediate Compound A120-3

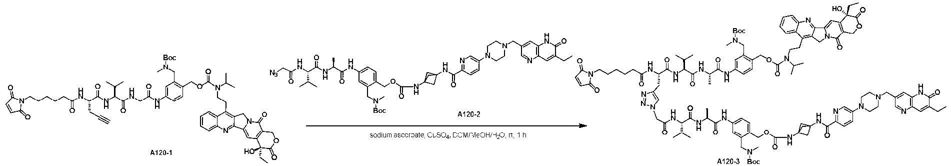

Compound **A120-1** (80 mg, 0.0675 mmol, 1.0 eq) and compound **A120-2** (68.9 mg, 0.0675 mmol, 1.0 eq) were dissolved in a mixture of MeOH/DCM/water (5/5/4 mL). Then, 100 µL of an aqueous solution of copper(II) sulfate pentahydrate (1.69 mg, 0.00675 mmol, 0.1 eq) and 100 µL of an aqueous solution of sodium ascorbate (2.67 mg, 0.0135 mmol, 0.2 eq) were added. After addition, the reaction mixture was stirred at room temperature under nitrogen for 1 hour. After the reaction was confirmed complete by LC-MS monitoring, water (30 mL) was added. The mixture was extracted with dichloromethane. The combined organic phases were washed with water and brine, dried over anhydrous sodium sulfate, filtered, and concentrated to afford compound **A120-3** (140 mg, 92%) as a yellow solid.
LCMS: [M+H]⁺ = 1102.4 (half peak)

### Synthesis of Target Compound A120

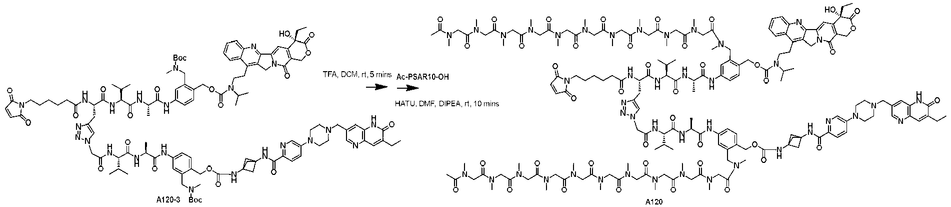

Compound **A120-3** (100 mg, 0.0453 mmol, 1.0 eq) was dissolved in DCM (3 mL). Trifluoroacetic acid (1 mL) was added to the reaction mixture. The mixture was stirred at room temperature for 5 minutes, then concentrated. The residue was dried under an oil pump for 1 hour, then redissolved in DMF (3 mL). Compound **Ac-PSAR10-OH** (69.8 mg, 0.0906 mmol, 2.0 eq) and N,N-Diisopropylethylamine (36.2 mg, 0.906 mmol, 20.0 eq) were added. Finally, O-(7-Azabenzotriazol-1-yl)-N,N,N',N'-tetramethyluronium hexafluorophosphate (36.2 mg, 0.0951 mmol, 2.1 eq) was added. After addition, the reaction mixture was stirred at room temperature for 10 minutes. After the reaction was confirmed complete by LC-MS monitoring, ethyl acetate (45 mL) was added. The mixture was filtered. The filter cake was dissolved in DMF and purified by preparative HPLC (mobile phase: 0.05% HCOOH/CH₃CN/H₂O) to afford Compound **A120** (13.7 mg, 8.6%) as a white solid.

LCMS: [M+H]⁺ = 1755.2 (half peak)

### 41) Synthesis of Compound A29

### Synthetic Route:

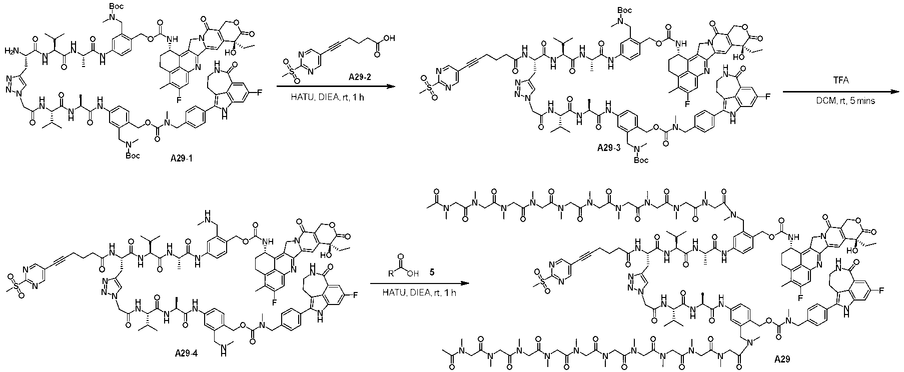

### Synthetic Steps:

### Synthesis of Intermediate Compound A29-3

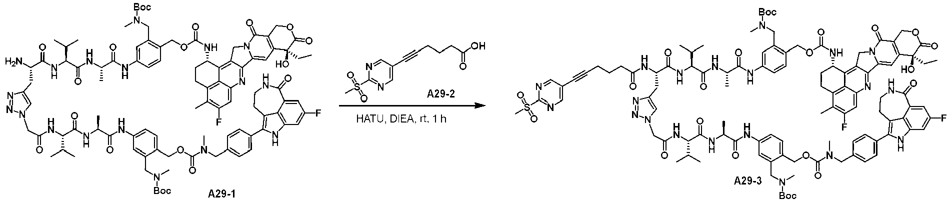

Compound A29-1 (200 mg, 0.10 mmol, 1.0 eq) and N,N-Diisopropylethylamine (41.6 mg, 0.32 mmol, 3.0 eq) were dissolved in DMF (5 mL). After stirring until clear, compound A29-2 (31.7 mg, 0.12 mmol, 1.1 eq) and O-(7-Azabenzotriazol-1-yl)-N,N,N',N'-tetramethyluronium hexafluorophosphate (53.0 mg, 0.14 mmol, 1.3 eq) were added. After addition, the reaction mixture was stirred at room temperature for 1 hour. After the reaction was confirmed complete by LC-MS monitoring, methyl tert-butyl ether (100 mL) was added. The suspension was filtered directly, washed with methyl tert-butyl ether, and the filter cake was dried to afford compound A29-3 (181 mg, 75%) as a yellow solid.
LCMS: [M+H]⁺ = 1056.7 (half peak)

### Synthesis of Intermediate Compound A29-4

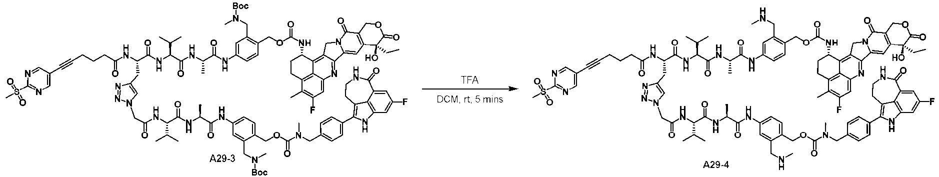

Compound A29-3 (181 mg, 0.086 mmol) was dissolved in DCM (3 mL). Trifluoroacetic acid (1 mL) was added to the reaction mixture. The mixture was stirred at room temperature for 5 minutes. After the reaction was confirmed complete by LC-MS monitoring, the organic phase was concentrated to afford crude compound A29-4, which was used directly in the next step.
LCMS: [M+H]⁺ = 956.9 (half peak)

### Synthesis of Target Compound A29

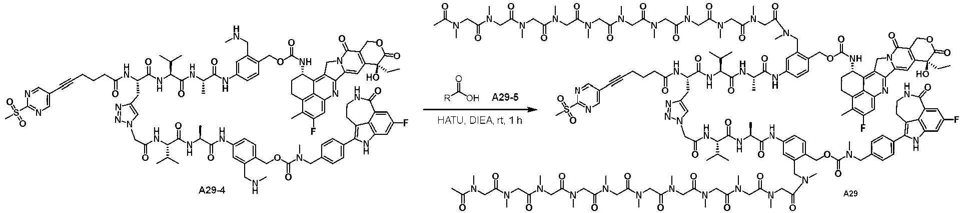

The compound A29-4 from the previous step was dissolved in DMF (3 mL). Ac-PSAR10-OH (65.6 mg, 0.085 mmol, 2.0 eq) and N,N-Diisopropylethylamine (121.8 mg, 0.942 mmol, 20.0 eq) were added. Finally, O-(7-Azabenzotriazol-1-yl)-N,N,N',N'-tetramethyluronium hexafluorophosphate (37.6 mg, 0.099 mmol, 2.1 eq) was added under a nitrogen atmosphere. After addition, the reaction mixture was stirred at room temperature under nitrogen for 1 hour. After the reaction was confirmed complete by LC-MS monitoring, ethyl acetate (60 mL) was added. The resulting suspension was filtered. The filter cake was dissolved in DMF and purified by preparative HPLC (mobile phase: 0.05% HCOOH/CH₃CN/H₂O) to afford Compound A29 (16.1 mg, 17%) as a white solid.
LCMS: [M+H]⁺ = 1709.6 (half peak)

### 42) Synthesis of Compound A123

### Synthetic Route:

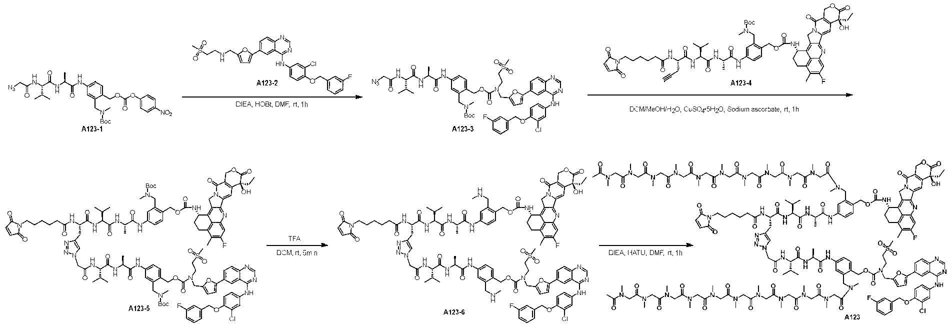

### Synthetic Steps:

### Synthesis of Intermediate Compound A123-3

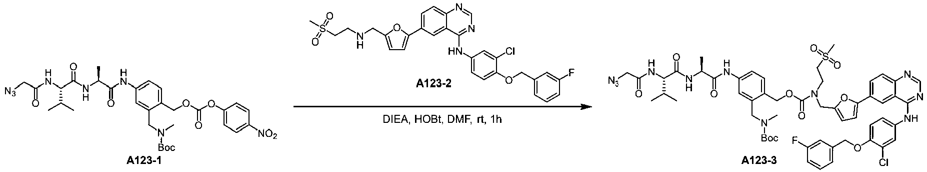

Compound A123-1 (118.01 mg, 0.17 mmol, 1.0 eq) and N,N-Diisopropylethylamine (66.73 mg, 0.52 mmol, 3.0 eq) were dissolved in DMF (5 mL). After stirring until clear, compound A123-2 (100 mg, 0.17 mmol, 1.0 eq) and HOBt (11.63 mg, 0.08 mmol, 0.5 eq) were added. After addition, the reaction mixture was stirred at room temperature for 1 hour. After the reaction was confirmed complete by LC-MS monitoring, ethyl acetate and water (30 mL) were added. The mixture was extracted with ethyl acetate. The combined organic phases were washed with water and brine, dried over anhydrous sodium sulfate, filtered, and concentrated. The residue was purified by silica gel column chromatography (DCM/MeOH = 93/7) to afford compound A123-3 (126 mg, 58.5%) as a white solid.
LCMS: [M+H]⁺ = 1126.3

### Synthesis of Intermediate Compound A123-5

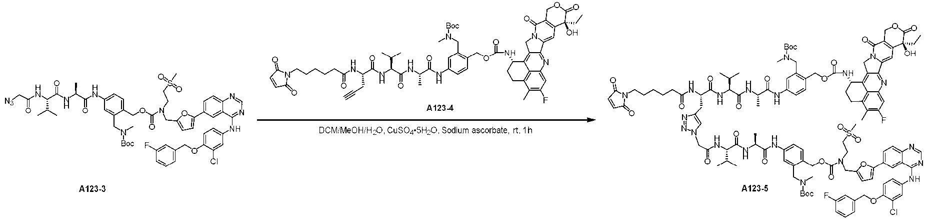

Compound A123-3 (148 mg, 0.124 mmol, 1.0 eq) and compound A123-4 (140 mg, 0.124 mmol, 1.0 eq) were dissolved in a mixture of dichloromethane/methanol/water (5/5/1 mL). Copper(II) sulfate pentahydrate (3.12 mg, 0.012 mmol, 0.1 eq, in 100 µL water) and sodium ascorbate (4.94 mg, 0.025 mmol, 0.2 eq, in 100 µL water) were added under a nitrogen atmosphere. After addition, the reaction mixture was stirred at room temperature under nitrogen for 1 hour. After the reaction was confirmed complete by LC-MS monitoring, the mixture was concentrated directly, and tetrahydrofuran (30 mL) was added and evaporated three times to remove water, affording compound A123-5 (238 mg, 74.2%) as a yellow solid.
LCMS: [M+H]⁺ = 1156.9 (half peak)

### Synthesis of Intermediate Compound A123-6

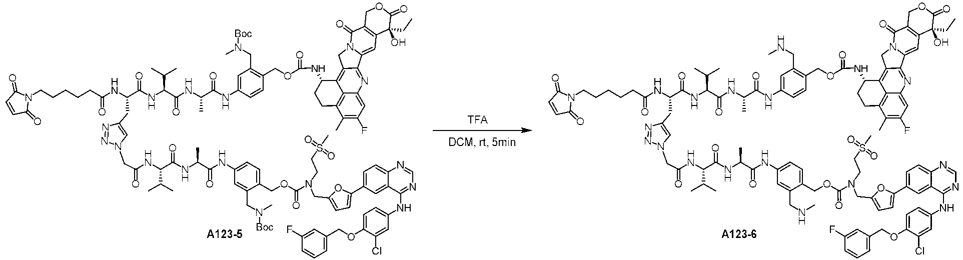

Compound A123-5 (120 mg, 0.052 mmol) was dissolved in DCM (3 mL). Trifluoroacetic acid (1 mL) was added to the reaction mixture. The mixture was stirred at room temperature for 5 minutes. After the reaction was confirmed complete by LC-MS monitoring, the organic phase was concentrated to afford crude compound A123-6, which was used directly in the next step. LCMS: [M+H]⁺ = 1056.5 (half peak)

### Synthesis of Target Compound A123

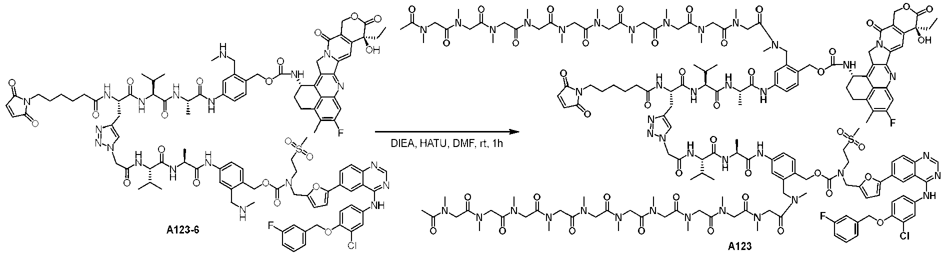

The compound A123-6 from the previous step was dissolved in DMF (3 mL). Compound Ac-PSAR10-OH (91.91 mg, 0.12 mmol, 2.0 eq) and N,N-Diisopropylethylamine (154.9 mg, 1.19 mmol, 20.0 eq) were added. Finally, O-(7-Azabenzotriazol-1-yl)-N,N,N',N'-tetramethyluronium hexafluorophosphate (47.5 mg, 0.12 mmol, 2.1 eq) was added under a nitrogen atmosphere. After addition, the reaction mixture was stirred at room temperature under nitrogen for 1 hour. After the reaction was confirmed complete by LC-MS monitoring, ethyl acetate (60 mL) was added. The resulting suspension was filtered. The filter cake was dissolved in DMF and purified by preparative HPLC (mobile phase: 0.05% HCOOH/CH₃CN/H₂O) to afford Compound A123 (22.1 mg, 20.5%) as a white solid.
LCMS: [M+H]⁺ = 1809.7 (half peak)

### 43) Synthesis of Compound A124

### Synthetic Route:

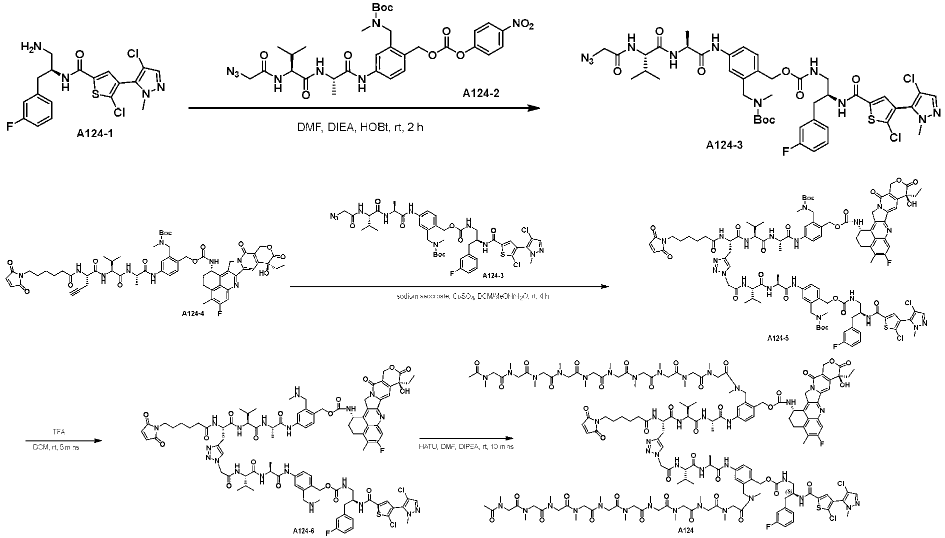

### Synthetic Steps:

### Synthesis of Intermediate Compound A124-3

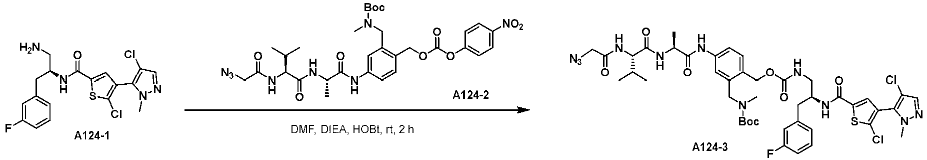

Compound **A124-1** (120 mg, 0.2808 mmol, 1.0 eq) was dissolved in DMF (3 mL). Then, compound **A124-2** (192.6 mg, 0.2808 mmol, 1.0 eq), HOBt (37.9 mg, 0.2808 mmol, 1.0 eq) and N,N-Diisopropylethylamine (108.9 mg, 0.8424 mmol, 3.0 eq) were added. After addition, the reaction mixture was stirred at room temperature for 2 hours. After the reaction was confirmed complete by LC-MS monitoring, the mixture was diluted with ethyl acetate and washed with water and brine. The organic phase was dried over anhydrous sodium sulfate, filtered, and concentrated. The residue was purified by silica gel column chromatography (DCM/MeOH = 93/7) to afford compound **A124-3** (200 mg, 72%) as a white solid.
LCMS: [M+Na]⁺ = 996.3

### Synthesis of Intermediate Compound A124-5

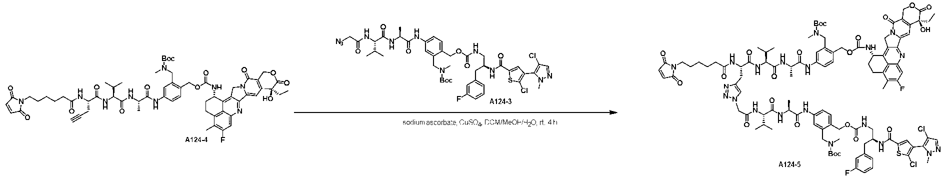

Compound **A124-4** (100 mg, 0.0842 mmol, 1.0 eq) and compound **A124-3** (82.0 mg, 0.0842 mmol, 1.0 eq) were dissolved in a mixture of MeOH/DCM/water (5/5/1 mL). Then, 100 µL of an aqueous solution of copper(II) sulfate pentahydrate (2.10 mg, 0.00842 mmol, 0.1 eq) and 100 µL of an aqueous solution of sodium ascorbate (3.34 mg, 0.01684 mmol, 0.2 eq) were added. After addition, the reaction mixture was stirred at room temperature under nitrogen for 4 hours. After the reaction was confirmed complete by LC-MS monitoring, water (30 mL) was added. The mixture was extracted with dichloromethane. The combined organic phases were washed with water and brine, dried over anhydrous sodium sulfate, filtered, and concentrated to afford compound **A124-5** (180 mg, 98%) as a yellow solid.
LCMS: [M+H]⁺ = 1079.7 (half peak)

### Synthesis of Intermediate Compound A124-6

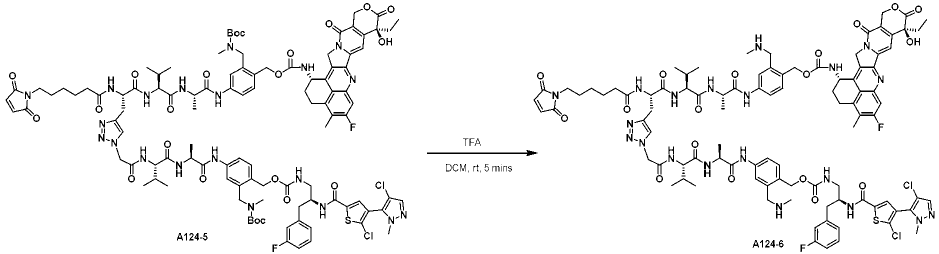

Compound **A124-5** (170 mg, 0.0787 mmol) was dissolved in DCM (3 mL). Trifluoroacetic acid (1 mL) was added to the reaction mixture. The mixture was stirred at room temperature for 5 minutes. After the reaction was confirmed complete by LC-MS monitoring, the organic phase was concentrated to afford crude compound **A124-6,** which was used directly in the next step. LCMS: [M+H]⁺ = 980.3 (half peak)

### Synthesis of Target Compound A124

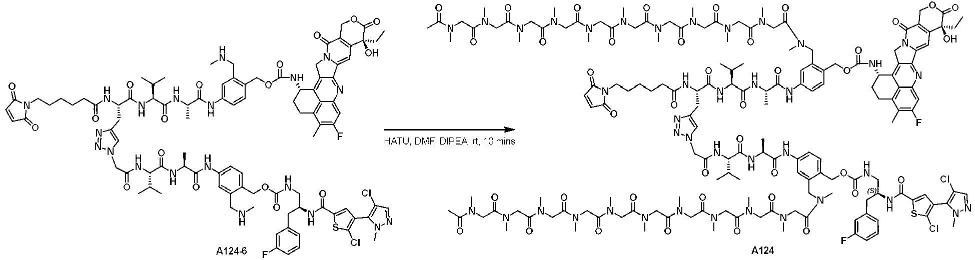

The compound **A124-6** from the previous step was dissolved in DMF (3 mL). Ac-PSAR10-OH (121.0 mg, 0.1574 mmol, 2.0 eq) and N,N-Diisopropylethylamine (203.2 mg, 1.574 mmol, 20.0 eq) were added. Finally, HATU (62.8 mg, 0.1653 mmol, 2.1 eq) was added. After addition, the reaction mixture was stirred at room temperature for 10 minutes. After the reaction was confirmed complete by LC-MS monitoring, ethyl acetate (45 mL) was added. The mixture was filtered. The filter cake was dissolved in DMF and purified by preparative HPLC (mobile phase: 0.05% HCOOH/CH₃CN/H₂O) to afford Compound **A124** (62.2 mg, 22.8%) as a pink solid. LCMS: [M+H]⁺ = 1732.6 (half peak)

### (44) Synthesis of Compound A125

### Synthetic Route:

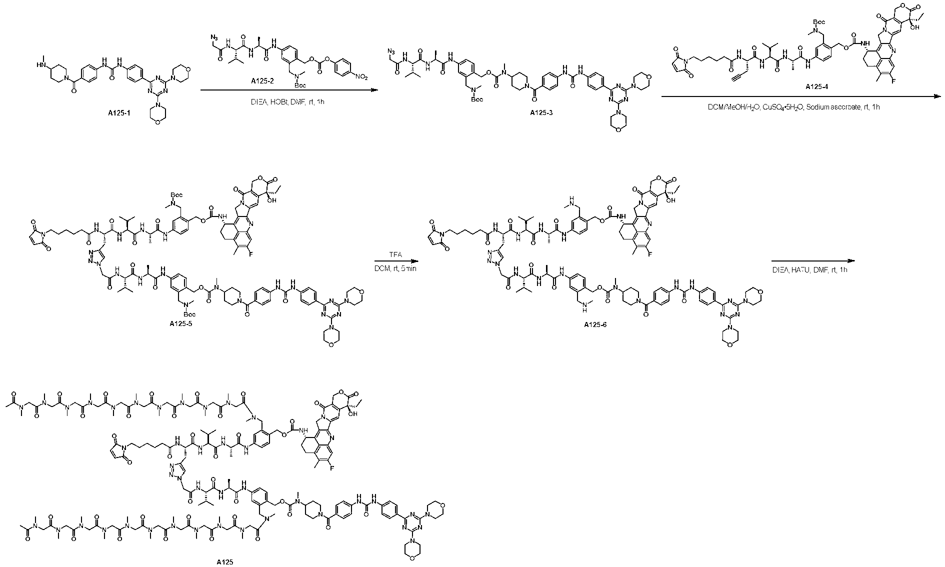

### Synthetic Steps:

### Synthesis of Intermediate Compound A125-3

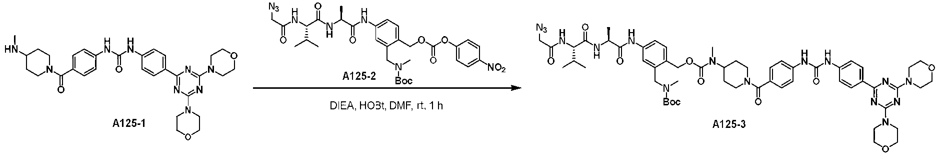

Compound A125-1 (200 mg, 0.332 mmol, 1.0 eq) and N,N-Diisopropylethylamine (257.8 mg, 1.99 mmol, 6.0 eq) were dissolved in DMF (3 mL). After stirring until clear, compound A125-2 (227.9 mg, 0.332 mmol, 1.0 eq) and HOBt (22.46 mg, 0.166 mmol, 0.5 eq) were added. After the addition was complete, the reaction mixture was stirred at room temperature for 1 hour. After LC-MS monitoring indicated completion, ethyl acetate (50 mL) and water (30 mL) were added to the reaction mixture. The layers were separated, and the aqueous phase was extracted with ethyl acetate. The combined organic phases were washed with water and then with saturated brine, dried over anhydrous sodium sulfate, filtered, and concentrated. The residue was purified by silica gel column chromatography (DCM/MeOH=93/7) to afford compound A125-3 (292 mg, 76%) as a white solid.
LCMS: [M+H]⁺ = 1147.5

### Synthesis of Intermediate Compound A125-5

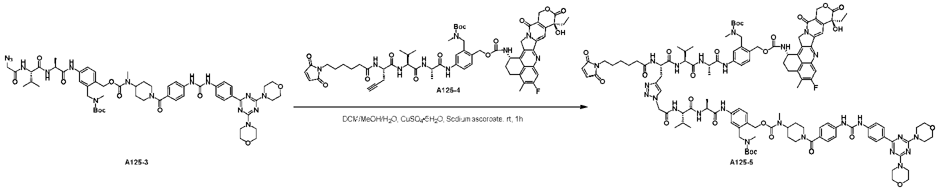

Compound A125-3 (96.7 mg, 0.084 mmol, 1.0 eq) and A125-4 (100 mg, 0.084 mmol, 1.0 eq) were dissolved in dichloromethane/methanol/water (5/5/1 mL). Under a nitrogen atmosphere, copper(II) sulfate pentahydrate (2.1 mg, 0.008 mmol, 0.1 eq, in 100 uL water) and sodium ascorbate (3.3 mg, 0.016 mmol, 0.2 eq, in 100 uL water) were added. After the addition was complete, the reaction mixture was stirred at room temperature under a nitrogen atmosphere for 1 hour. After LC-MS monitoring indicated completion, the reaction mixture was concentrated under reduced pressure, and tetrahydrofuran (30 mL) was added and evaporated three times to remove water, affording compound A125-5 (158 mg, 72%) as a yellow solid. LCMS: [M+H]⁺ = 1167.6 (half peak)

### Synthesis of Intermediate Compound A125-6

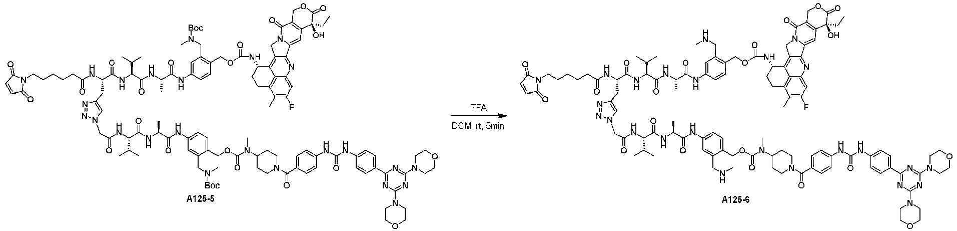

Compound A125-5 (158 mg, 0.067 mmol) was dissolved in DCM (3 mL). Trifluoroacetic acid (1 mL) was added to the reaction mixture, which was then stirred at room temperature for 5 minutes. After LC-MS monitoring indicated completion, the organic phase was concentrated to give the crude product compound A125-6, which was used directly in the next step.
LCMS: [M+H]⁺ = 1067.4 (half peak)

### Synthesis of Target Compound A125

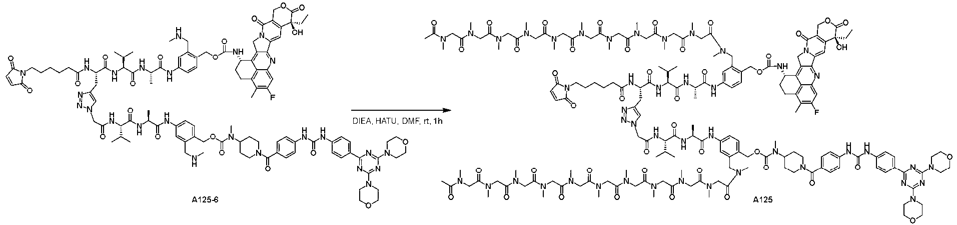

Compound A125-6 (128 mg, 0.06 mmol, 1.0 eq) and N,N-Diisopropylethylamine (155.1 mg, 1.2 mmol, 20 eq) were dissolved in DMF (5 mL). After stirring until clear, Ac-PSAR10-OH (97.2 mg, 0.125 mmol, 2.0 eq) and HATU (50.2 mg, 0.132 mmol, 2.2 eq) were added. After the addition was complete, the reaction mixture was stirred at room temperature for 1 hour. After LC-MS monitoring indicated completion, ethyl acetate (80 mL) was added to the reaction mixture. The mixture was filtered, and the filter cake was dissolved in DMF and purified by prep-HPLC (mobile phase: 0.05% HCOOH/CH₃CN/H₂O) to afford compound A125 (20.2 mg, 15%) as a white solid.
LCMS: [M+H]⁺ = 1213.6 (one-third peak)

### (45) Synthesis of Compound A127

### Synthetic Route:

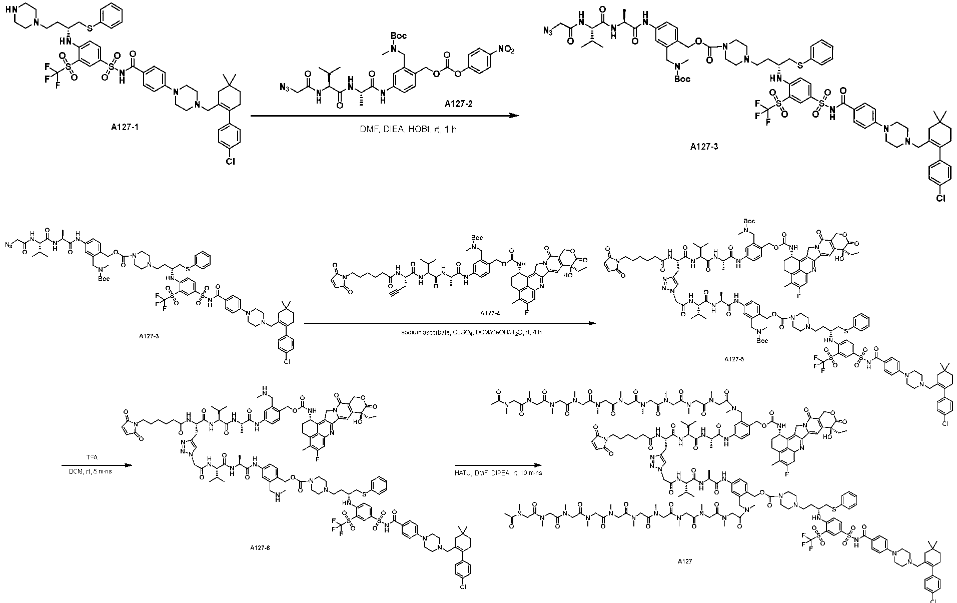

### Synthetic Steps:

### Synthesis of Intermediate Compound A127-3

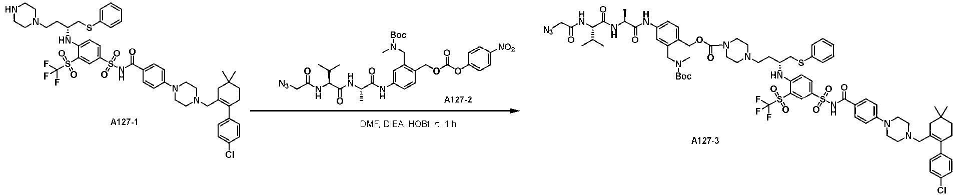

Compound A127-1 (120 mg, 0.1233 mmol, 1.0 eq) was dissolved in DMF (3 mL). Then compound A127-2 (84.6 mg, 0.1233 mmol, 1.0 eq), HOBt (16.7 mg, 0.1233 mmol, 1.0 eq), and N,N-Diisopropylethylamine (47.8 mg, 0.3699 mmol, 3.0 eq) were added. After the addition was complete, the reaction mixture was stirred at room temperature for 1 hour. After LC-MS monitoring indicated completion, the reaction mixture was diluted with ethyl acetate, washed with water and saturated brine. The organic phase was dried over anhydrous sodium sulfate, filtered, and concentrated. The residue was purified by silica gel column chromatography (DCM/MeOH=93/7) to afford compound A127-3 (100 mg, 52%) as a white solid.
LCMS: [M+H]⁺ = 759.9 (half peak)

### Synthesis of Intermediate Compound A127-5

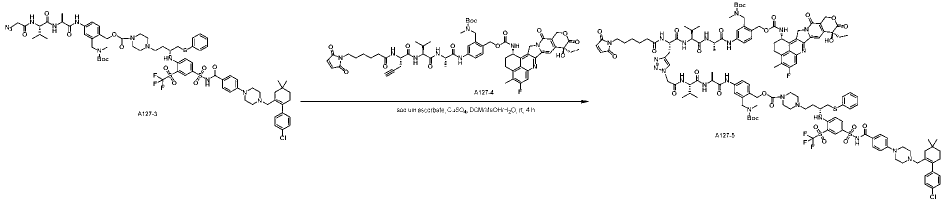

Compound A127-3 (50 mg, 0.0329 mmol, 1.0 eq) and compound A127-4 (39.1 mg, 0.0329 mmol, 1.0 eq) were dissolved in MeOH/DCM/water (5/5/1 mL). Then 100 uL of an aqueous solution of copper(II) sulfate pentahydrate (8.22 mg, 0.0329 mmol, 1.0 eq) and 100 uL of an aqueous solution of sodium ascorbate (13.04 mg, 0.0658 mmol, 0.2 eq) were added. After the addition was complete, the reaction mixture was stirred at room temperature under a nitrogen atmosphere for 4 hours. After LC-MS monitoring indicated completion, water (30 mL) was added to the reaction mixture. The mixture was extracted with dichloromethane. The organic phase was washed with water and then with saturated brine, dried over anhydrous sodium sulfate, filtered, and concentrated to afford compound A127-5 (90 mg, 99%) as a yellow solid. LCMS: [M+H]⁺ = 1352.4 (half peak)

### Synthesis of Intermediate Compound A127-6

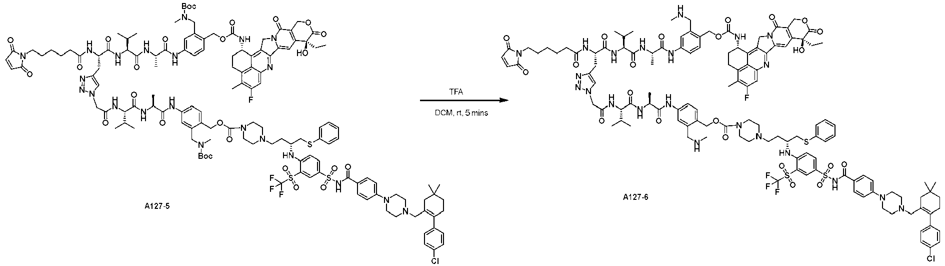

Compound A127-5 (80 mg, 0.0295 mmol) was dissolved in DCM (3 mL). Trifluoroacetic acid (1 mL) was added to the reaction mixture, which was then stirred at room temperature for 5 minutes. After LC-MS monitoring indicated completion, the organic phase was concentrated to give the crude product compound A127-6, which was used directly in the next step.
LCMS: [M+Na]⁺ = 1252.5 (half peak)

### Synthesis of Target Compound A127

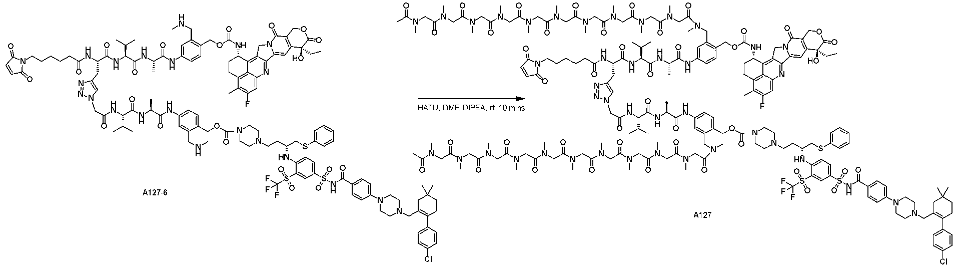

The compound A127-6 from the previous step was dissolved in DMF (3 mL). Ac-PSAR10-OH (45.4 mg, 0.059 mmol, 2.0 eq) and N,N-Diisopropylethylamine (76.3 mg, 0.590 mmol, 20.0 eq) were added to the reaction mixture, followed by HATU (23.6 mg, 0.062 mmol, 2.1 eq). After the addition was complete, the reaction mixture was stirred at room temperature for 10 minutes. After LC-MS monitoring indicated completion, ethyl acetate (45 mL) was added to the reaction mixture. The mixture was filtered, and the filter cake was dissolved in DMF and purified by prep-HPLC (mobile phase: 0.05% HCOOH/CH₃CN/H₂O) to afford compound A127 (18.9 mg, 15.9%) as a white solid.
LCMS: [M+H]⁺ = 1337.8 (one-third peak)

### (46) Synthesis of Compound A129

### Synthetic Route:

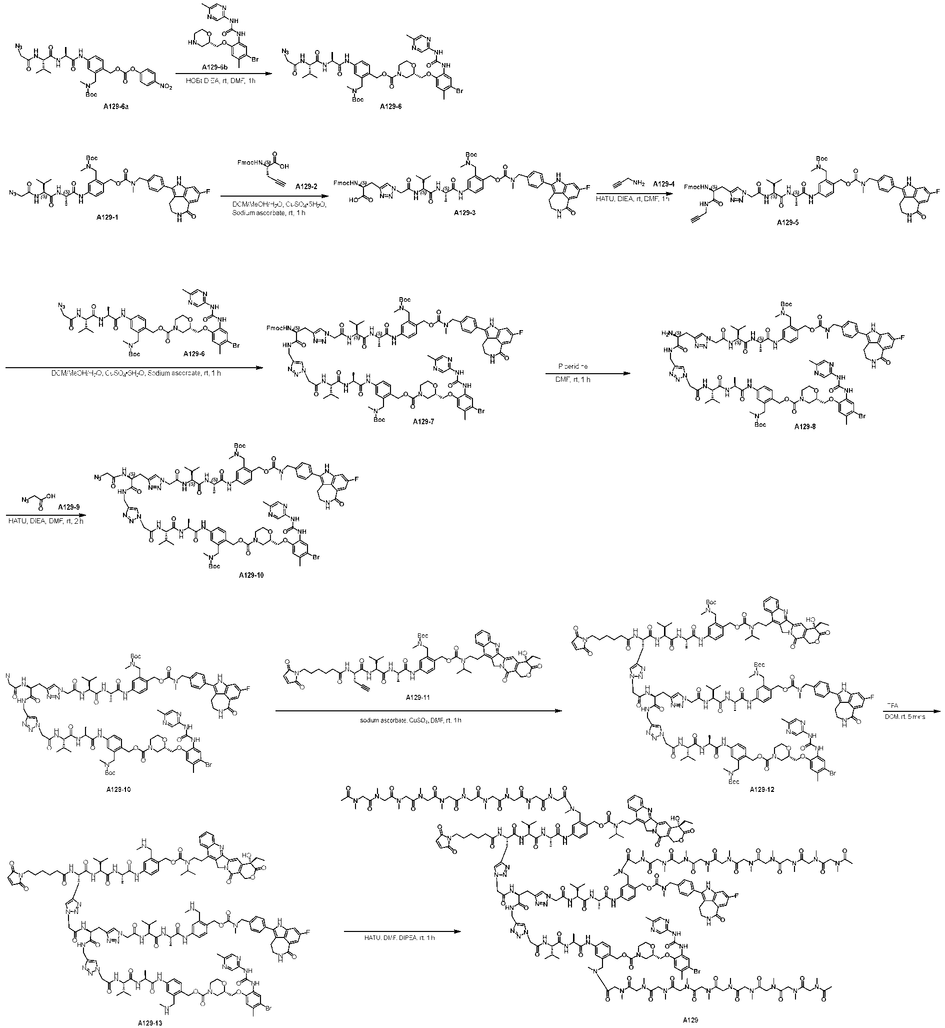

### Synthetic Steps:

### Synthesis of Intermediate Compound A129-6

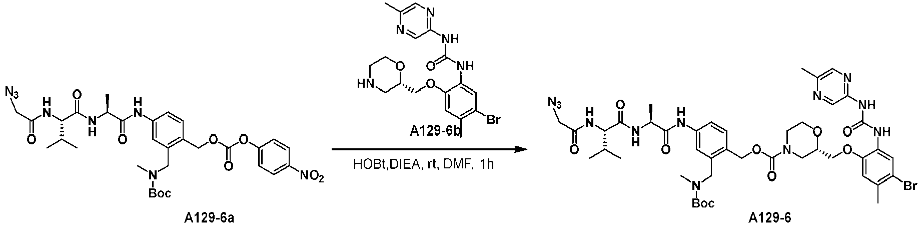

Compound **A129-6b** (700 mg, 1.604 mmol, 1.0 eq) and N,N-Diisopropylethylamine (622.1 mg, 4.81 mmol, 3.0 eq) were dissolved in DMF (10 mL). After stirring until clear, compound **A129-6** (1100.1 mg, 1.604 mmol, 1.0 eq) and HOBt (108.4 mg, 0.802 mmol, 0.5 eq) were added. After the addition was complete, the reaction mixture was stirred at room temperature for 1 hour. After LC-MS monitoring indicated completion, ethyl acetate (50 mL) and water (30 mL) were added to the reaction mixture. The layers were separated, and the aqueous phase was extracted with ethyl acetate. The combined organic phases were washed with water and then with saturated brine, dried over anhydrous sodium sulfate, filtered, and concentrated. The residue was purified by silica gel column chromatography (DCM/MeOH=93/7) to afford compound **A129-6** (1400 mg, 80%) as a yellow solid.
LCMS: [M+H]⁺ = 981.3

### Synthesis of Intermediate Compound A129-3

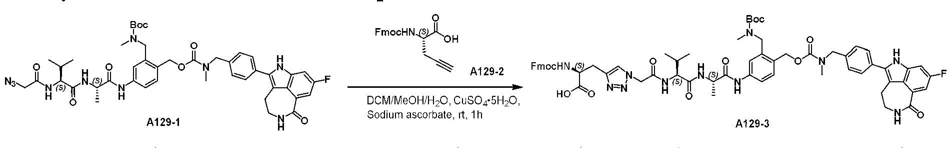

Compound **A129-1** (300 mg, 0.345 mmol, 1.0 eq) and **A129-2** (115.6 mg, 0.345 mmol, 1.0 eq) were dissolved in dichloromethane/methanol/water (5/5/1 mL). Under a nitrogen atmosphere, copper(II) sulfate pentahydrate (8.6 mg, 0.034 mmol, 0.1 eq, in 100 uL water) and sodium ascorbate (13.7 mg, 0.069 mmol, 0.2 eq, in 100 uL water) were added. After the addition was complete, the reaction mixture was stirred at room temperature under a nitrogen atmosphere for 1 hour. After LC-MS monitoring indicated completion, the reaction mixture was concentrated directly, and tetrahydrofuran was added multiple times and evaporated to remove water, affording compound **A129-3** (420 mg, 91%) as a yellow solid.
LCMS: [M+H]⁺ = 1205.4

### Synthesis of Intermediate Compound A129-5

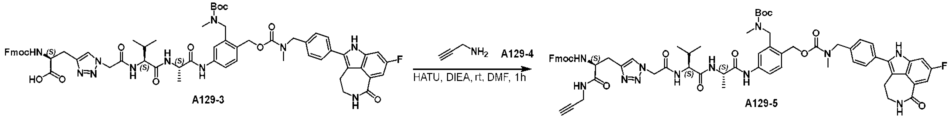

Compound **A129-3** (420 mg, 0.348 mmol, 1.0 eq) was dissolved in DMF (5 mL). Compound **A129-4** (57.6 mg, 1.045 mmol, 3.0 eq) and N,N-Diisopropylethylamine (90 mg, 0.697 mmol, 2.0 eq) were added to the reaction mixture. Finally, under a nitrogen atmosphere, O-(7-Azabenzotriazol-1-yl)-N,N,N',N'-tetramethyluronium hexafluorophosphate (145.8 mg, 0.383 mmol, 1.1 eq) was added. After the addition was complete, the reaction mixture was stirred at room temperature under a nitrogen atmosphere for 1 hour. After LC-MS monitoring indicated completion, ethyl acetate and water (30 mL) were added to the reaction mixture. The mixture was extracted with ethyl acetate. The organic phase was washed with water and then with saturated brine, dried over anhydrous sodium sulfate, filtered, and concentrated. The residue was slurried with MTBE, filtered, and the filter cake was collected and dried to afford compound **A129-5** (368 mg, 77%) as a yellow solid.
LCMS: [M+Na]⁺ = 1264.5

### Synthesis of Intermediate Compound A129-7

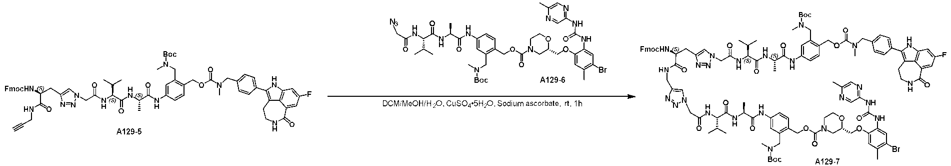

Compound **A129-5** (270 mg, 0.217 mmol, 1.0 eq) and compound **A129-6** (213.6 mg, 0.217 mmol, 1.0 eq) were dissolved in dichloromethane/methanol/water (5/5/1 mL). Under a nitrogen atmosphere, copper(II) sulfate pentahydrate (10.9 mg, 0.043 mmol, 0.1 eq, in 100 uL water) and sodium ascorbate (17.2 mg, 0.087 mmol, 0.2 eq, in 100 uL water) were added. After the addition was complete, the reaction mixture was stirred at room temperature under a nitrogen atmosphere for 1 hour. After LC-MS monitoring indicated completion, the reaction mixture was concentrated directly, and tetrahydrofuran was added multiple times and evaporated to remove water, affording compound **A129-7** (406 mg, 76%) as a yellow solid.
LCMS: [M+H]⁺ = 1112.9 (half peak)

### Synthesis of Intermediate Compound A129-8

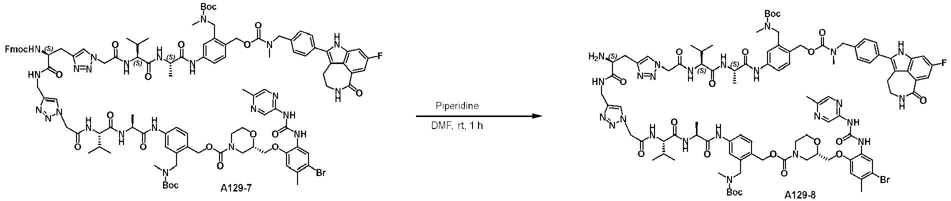

Compound **A129-7** (396 mg, 0.178 mmol, 1.0 eq) was dissolved in DMF (4 mL). Piperidine (1 mL) was added, and the reaction mixture was stirred at room temperature for 1 hour. After LC-MS monitoring indicated completion, MTBE was added to the reaction mixture. The suspension was filtered directly, washed with MTBE, and the filter cake was dried directly to afford compound **A129-8** (308 mg, 78%) as a yellow solid.
LCMS: [M+H]⁺ = 1000.3 (half peak)

### Synthesis of Intermediate Compound A129-10

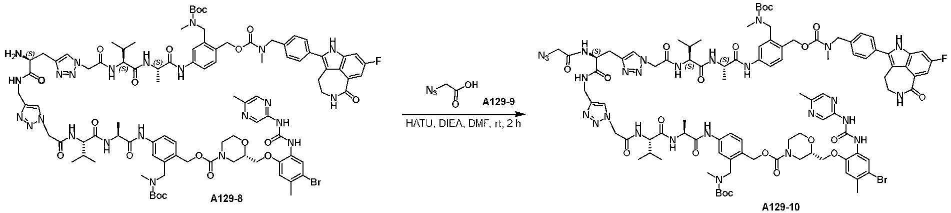

Compound **A129-8** (298 mg, 0.148 mmol, 1.0 eq) was dissolved in DMF (3 mL). Compound **A129-9** (22.6 mg, 0.223 mmol, 1.5 eq) and N,N-Diisopropylethylamine (57.7 mg, 0.446 mmol, 3.0 eq) were added to the reaction mixture. Finally, under a nitrogen atmosphere, O-(7-Azabenzotriazol-1-yl)-N,N,N',N'-tetramethyluronium hexafluorophosphate (84.87 mg, 0.223 mmol, 1.5 eq) was added. After the addition was complete, the reaction mixture was stirred at room temperature under a nitrogen atmosphere for 2 hours. After LC-MS monitoring indicated completion, ethyl acetate (60 mL) was added to the reaction mixture. The suspension was filtered directly, washed with ethyl acetate, and the filter cake was dried directly to afford compound **A129-10** (252 mg, 73%) as a yellow solid.
LCMS: [M+H]⁺ = 1042.8 (half peak)

### Synthesis of Intermediate Compound A129-12

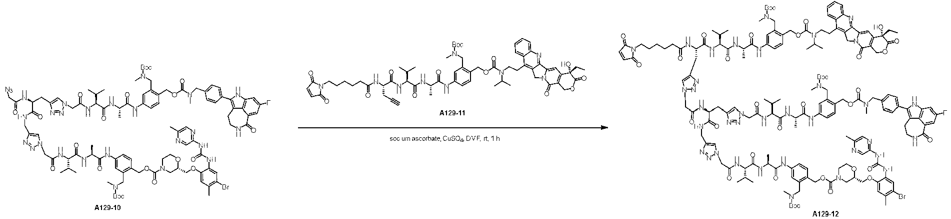

Compound **A129-10** (232 mg, 0.111 mmol, 1.0 eq) and compound **A129-11** (131.8 mg, 0.111 mmol, 1.0 eq) were placed in DMF (5 mL). Under a nitrogen atmosphere, copper(II) sulfate pentahydrate (5.6 mg, 0.022 mmol, 0.2 eq, in 100 uL water) and sodium ascorbate (8.8 mg, 0.044 mmol, 0.4 eq, in 100 uL water) were added. After the addition was complete, the reaction mixture was stirred at room temperature under a nitrogen atmosphere for 1 hour. After LC-MS monitoring indicated completion, ethyl acetate (50 mL) was added to the reaction mixture. The suspension was filtered directly, washed with ethyl acetate, and the filter cake was dried directly to afford compound **A129-12** (306 mg, 76%) as a yellow solid.
LCMS: [M+H]⁺ = 1635.4 (half peak)

### Synthesis of Intermediate Compound A129-13

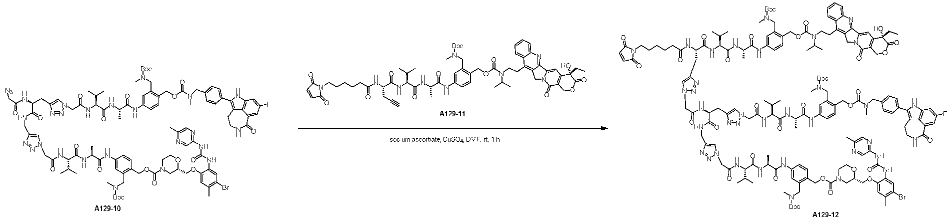

Compound **A129-12** (150 mg, 0.046 mmol, 1.0 eq) was dissolved in DCM (3 mL). Trifluoroacetic acid (1 mL) was added to the reaction mixture, which was then stirred at room temperature for 5 minutes. After LC-MS monitoring indicated completion, the organic phase was concentrated to give the crude product compound **A129-13,** which was used directly in the next step.
LCMS: [M+H]⁺ = 990.3 (one-third peak)

### Synthesis of Target Compound A129

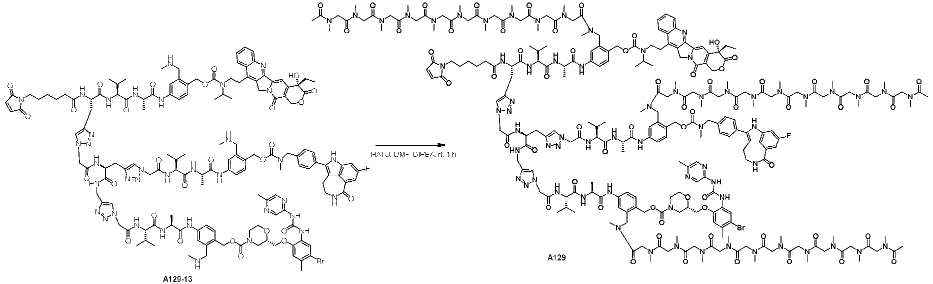

The compound **A129-13** from the previous step was dissolved in DMF (3 mL). Ac-PSAR10-OH (101.1 mg, 0.034 mmol, 3.0 eq) and N,N-Diisopropylethylamine (113.2 mg, 0.87 mmol, 20.0 eq) were added to the reaction mixture. Finally, under a nitrogen atmosphere, O-(7-Azabenzotriazol-1-yl)-N,N,N',N'-tetramethyluronium hexafluorophosphate (55 mg, 0.144 mmol, 3.3 eq) was added. After the addition was complete, the reaction mixture was stirred at room temperature under a nitrogen atmosphere for 1 hour. After LC-MS monitoring indicated completion, ethyl acetate (35 mL) was added to the reaction mixture. The resulting suspension was filtered, and the filter cake was dissolved in DMF and purified by prep-HPLC (mobile phase: 0.05% HCOOH/CH₃CN/H₂O) to afford compound **A129** (34.9 mg, 23%) as a white solid. LCMS: [M+H]⁺ = 1742.8 (one-third peak)

### (47) Synthesis of Compound A115

### Synthetic Route:

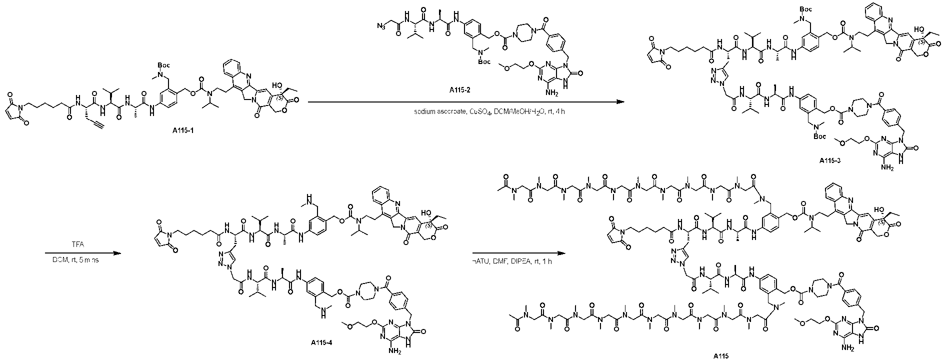

### Synthetic Steps:

### Synthesis of Intermediate Compound A115-3

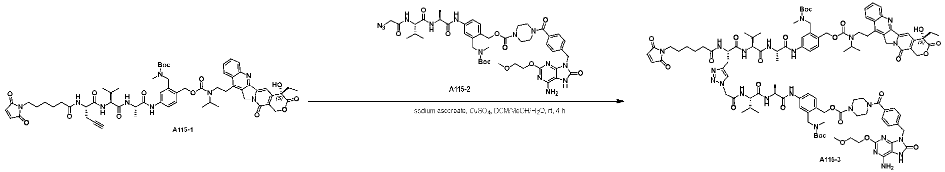

Compound **A115-1** (180 mg, 0.152 mmol, 1.0 eq) and compound **A115-2** (148.0 mg, 0.152 mmol, 1.0 eq) were dissolved in MeOH/DCM/water (5/5/1 mL). Then 100 uL of an aqueous solution of copper(II) sulfate pentahydrate (3.80 mg, 0.0152 mmol, 0.1 eq) and 100 uL of an aqueous solution of sodium ascorbate (6.02 mg, 0.0304 mmol, 0.2 eq) were added. After the addition was complete, the reaction mixture was stirred at room temperature under a nitrogen atmosphere for 4 hours. After LC-MS monitoring indicated completion, water (30 mL) was added to the reaction mixture. The mixture was extracted with dichloromethane. The organic phase was washed with water and then with saturated brine, dried over anhydrous sodium sulfate, filtered, and concentrated to afford compound **A115-3** (320 mg, 97%) as a yellow solid. LCMS: [M+H]⁺ = 1079.2 (half peak)

### Synthesis of Intermediate Compound A115-4

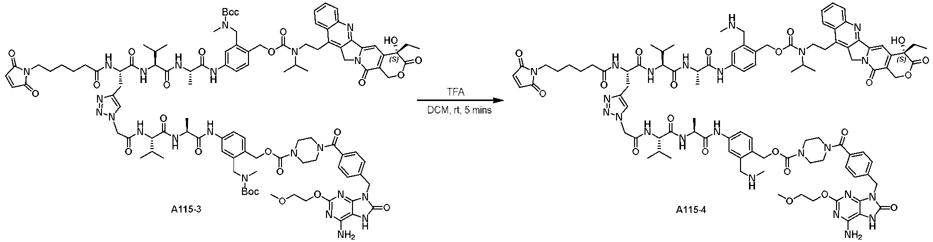

Compound **A115-3** (300 mg, 0.139 mmol, 1.0 eq) was dissolved in DCM (6 mL). Trifluoroacetic acid (2 mL) was added to the reaction mixture, which was then stirred at room temperature for 5 minutes. After LC-MS monitoring indicated completion, the organic phase was concentrated to give the crude product compound **A115-4,** which was used directly in the next step.
LCMS: [M+H]⁺ = 979.3 (half peak)

### Synthesis of Target Compound A115

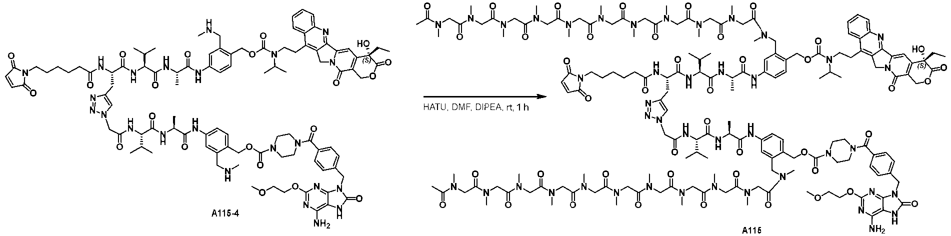

The compound **A115-4** from the previous step was dissolved in DMF (5 mL). Ac-PSAR10-OH (214.1 mg, 0.278 mmol, 2.0 eq) and N,N-Diisopropylethylamine (359.3 mg, 2.78 mmol, 20.0 eq) were added to the reaction mixture. Finally, under a nitrogen atmosphere, O-(7-Azabenzotriazol-1-yl)-N,N,N',N'-tetramethyluronium hexafluorophosphate (111.0 mg, 0.292 mmol, 2.1 eq) was added. After the addition was complete, the reaction mixture was stirred at room temperature under a nitrogen atmosphere for 1 hour. After LC-MS monitoring indicated completion, ethyl acetate (75 mL) was added to the reaction mixture. The mixture was filtered, and the filter cake was dissolved in DMF and purified by prep-HPLC (mobile phase: 0.05% HCOOH/CH₃CN/H₂O) to afford compound **A115** (102.9 mg, 21.4%) as a yellow solid.
LCMS: [M+H]⁺ = 1732.2 (half peak)

### (48) Synthesis of Compound A130

### Synthetic Route:

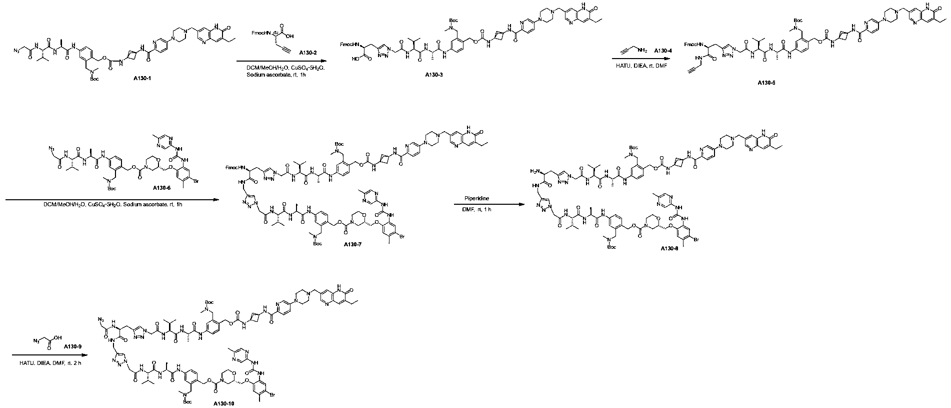

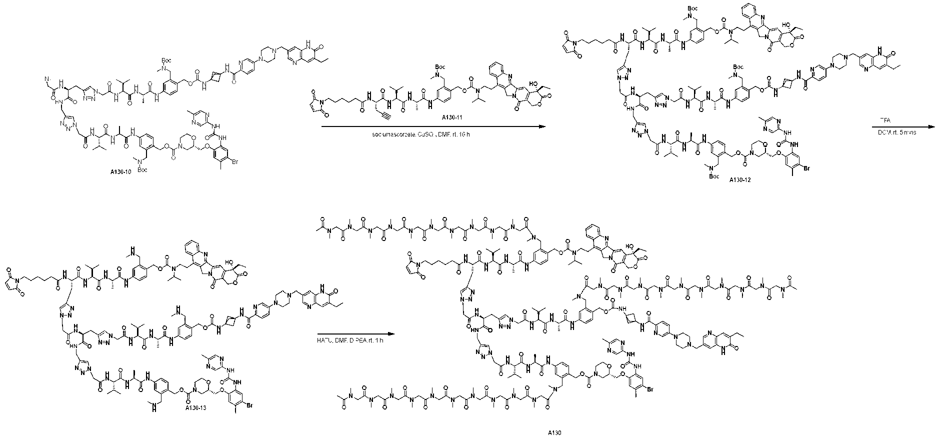

### Synthetic Steps:

### Synthesis of Intermediate Compound A130-3

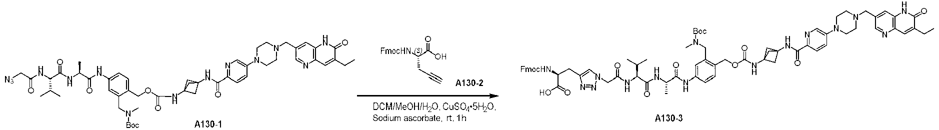

Compound A130-1 (500 mg, 0.490 mmol, 1.0 eq) and A130-2 (164 mg, 0.490 mmol, 1.0 eq) were dissolved in dichloromethane/methanol/water (6/6/0.5 mL). Under a nitrogen atmosphere, copper(II) sulfate pentahydrate (13.3 mg, 0.049 mmol, 0.1 eq, in 100 uL water) and sodium ascorbate (19.5 mg, 0.098 mmol, 0.2 eq, in 100 uL water) were added. After the addition was complete, the reaction mixture was stirred at room temperature under a nitrogen atmosphere for 1 hour. After LC-MS monitoring indicated completion, the reaction mixture was concentrated directly. Tetrahydrofuran was added, and the mixture was concentrated again to dryness, affording compound A130-3 (663 mg, 100%) as a grey solid.
LCMS: [M+H]⁺ = 1355.7

### Synthesis of Intermediate Compound A130-5

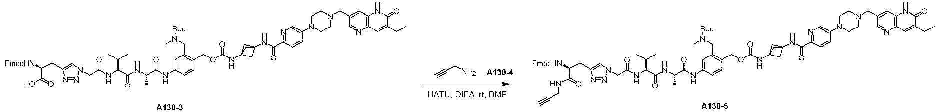

Compound A130-3 (663 mg, 0.490 mmol, 1.0 eq) was dissolved in DMF (6 mL). Compound A130-4 (81 mg, 1.470 mmol, 3.0 eq) and N,N-Diisopropylethylamine (126 mg, 0.980 mmol, 2.0 eq) were added to the reaction mixture. Finally, under a nitrogen atmosphere, O-(7-Azabenzotriazol-1-yl)-N,N,N',N'-tetramethyluronium hexafluorophosphate (242 mg, 0.637 mmol, 1.3 eq) was added. After the addition was complete, the reaction mixture was stirred at room temperature under a nitrogen atmosphere for 1 hour. After LC-MS monitoring indicated completion, methyl tert-butyl ether (80 mL) was added to the reaction mixture. The suspension was filtered directly, and the filter cake was collected and dried to afford compound A130-5 (545 mg, 80%) as a grey solid.
LCMS: [M+H]⁺ = 1391.7

### Synthesis of Intermediate Compound A130-7

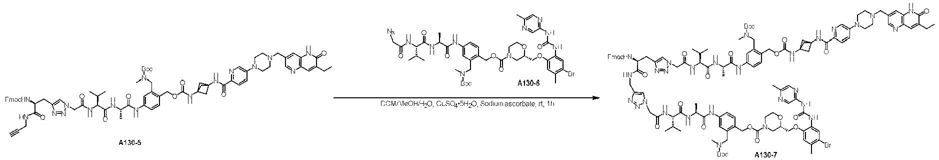

Compound A130-5 (545 mg, 0.392 mmol, 1.0 eq) and compound A130-6 (384 mg, 0.392 mmol, 1.0 eq) were dissolved in dichloromethane/methanol/water (6/6/0.5 mL). Under a nitrogen atmosphere, copper(II) sulfate pentahydrate (9.8 mg, 0.0392 mmol, 0.1 eq, in 100 uL water) and sodium ascorbate (15.5 mg, 0.0784 mmol, 0.25 eq, in 100 uL water) were added. After the addition was complete, the reaction mixture was stirred at room temperature under a nitrogen atmosphere for 1 hour. After LC-MS monitoring indicated completion, the reaction mixture was concentrated directly. The residue was dissolved in tetrahydrofuran and concentrated again to dryness, affording compound A130-7 (929 mg, 100%) as a brownish-yellow solid.
LCMS: [M+H]⁺ = 1188.0 (half peak)

### Synthesis of Intermediate Compound A130-8

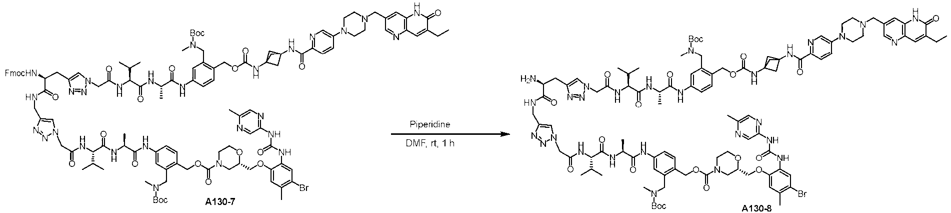

Compound A130-7 (900 mg, 0.380 mmol, 1.0 eq) was dissolved in DMF (5 mL). The reaction mixture was cooled to 0 °C, and under a nitrogen atmosphere, piperidine (1 mL) was added. The reaction mixture was stirred at room temperature under a nitrogen atmosphere for half an hour. After LC-MS monitoring indicated completion, methyl tert-butyl ether (50 mL) was added to the reaction mixture. The suspension was filtered directly, washed with methyl tert-butyl ether, and the filter cake was dried directly to afford compound A130-8 (595 mg, 73%) as a yellow solid.
LCMS: [M+H]⁺ = 1075.8 (half peak)

### Synthesis of Intermediate Compound A130-10

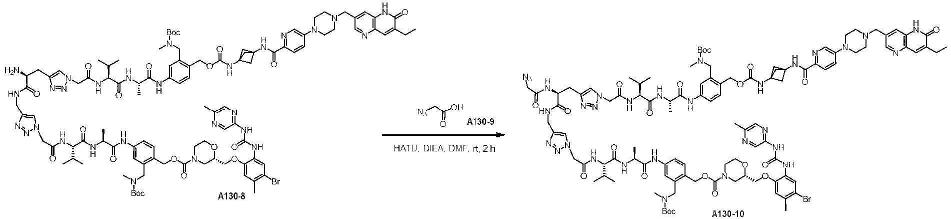

Compound A130-8 (595 mg, 0.277 mmol, 1.0 eq) was dissolved in DMF (6 mL). N,N-Diisopropylethylamine (107 mg, 0.831 mmol, 3.0 eq) and A130-9 (56 mg, 0.554 mmol, 2.0 eq) were added to the reaction mixture. Finally, under a nitrogen atmosphere, O-(7-Azabenzotriazol-1-yl)-N,N,N',N'-tetramethyluronium hexafluorophosphate (238 mg, 0.609 mmol, 2.2 eq) was added. After the addition was complete, the reaction mixture was stirred at room temperature under a nitrogen atmosphere for 1 hour. After LC-MS monitoring indicated completion, methyl tert-butyl ether (100 mL) was added to the reaction mixture. The suspension was filtered directly, washed with methyl tert-butyl ether, and the filter cake was dried directly to afford compound A130-10 (562 mg, 91%) as a yellow solid.
LCMS: [M+H]⁺ = 1117.5 (half peak)

### Synthesis of Intermediate Compound A130-12

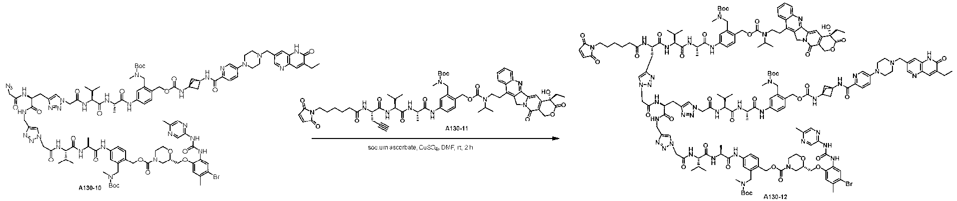

Compound A130-10 (250 mg, 0.112 mmol, 1.0 eq) and compound A130-11 (132 mg, 0.112 mmol, 1.0 eq) were dissolved in DMF (4 mL). Under a nitrogen atmosphere, copper(II) sulfate pentahydrate (2.8 mg, 0.0112 mmol, 0.1 eq, in 100 uL water) and sodium ascorbate (4.5 mg, 0.0224 mmol, 0.25 eq, in 100 uL water) were added. After the addition was complete, the reaction mixture was stirred at room temperature under a nitrogen atmosphere for 2 hours. After LC-MS monitoring indicated completion, methyl tert-butyl ether (60 mL) was added to the reaction mixture. The suspension was filtered directly, washed with methyl tert-butyl ether, and the filter cake was dried directly to afford compound A130-12 (306 mg, 80%) as a yellow solid.
LCMS: [M+H]⁺ = 1709.6 (half peak)

### Synthesis of Intermediate Compound A130-13

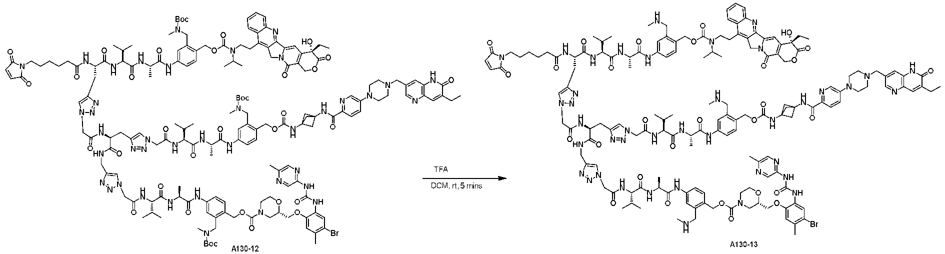

Compound A130-12 (306 mg, 0.090 mmol, 1.0 eq) was dissolved in DCM (3 mL). Trifluoroacetic acid (1 mL) was added to the reaction mixture, which was then stirred at room temperature for 5 minutes. After LC-MS monitoring indicated completion, the organic phase was concentrated to give the crude product compound A130-13, which was used directly in the next step.
LCMS: [M+H]⁺ = 1559.9 (half peak)

### Synthesis of Target Compound A130

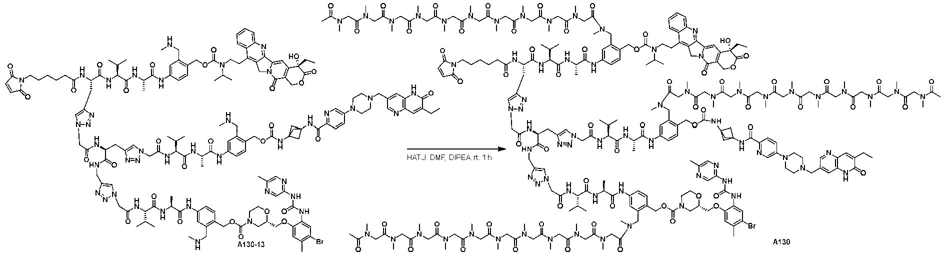

The compound A130-13 from the previous step was dissolved in DMF (6 mL). Ac-PSAR10-OH (208 mg, 0.270 mmol, 3.0 eq) and N,N-Diisopropylethylamine (232 mg, 1.80 mmol, 20.0 eq) were added to the reaction mixture. Finally, under a nitrogen atmosphere, O-(7-Azabenzotriazol-1-yl)-N,N,N',N'-tetramethyluronium hexafluorophosphate (113 mg, 0.297 mmol, 3.3 eq) was added. After the addition was complete, the reaction mixture was stirred at room temperature under a nitrogen atmosphere for 1 hour. After LC-MS monitoring indicated completion, ethyl acetate (60 mL) was added to the reaction mixture. The resulting suspension was filtered, and the filter cake was dissolved in DMF and purified by prep-HPLC (mobile phase: 0.05% HCOOH/CH₃CN/H₂O) to afford compound A130 (82 mg, 32%) as a yellow solid. LCMS: [M+H]⁺ = 1792.8 (one-third peak)

### (49) Synthesis of Compound A151

### Synthetic Route:

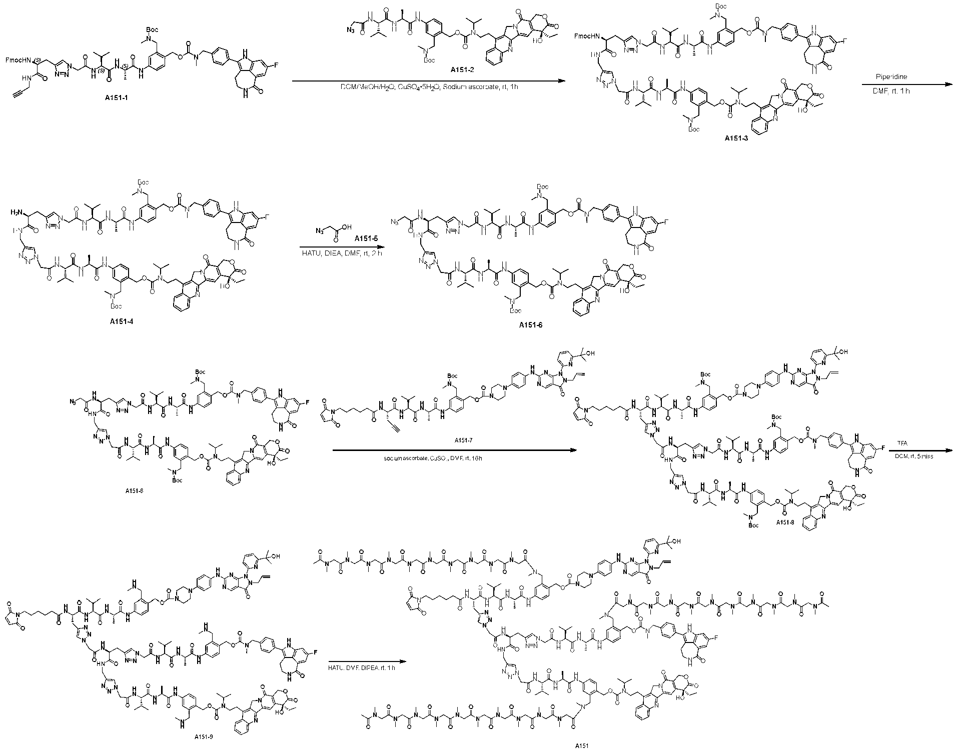

### Synthetic Steps:

### Synthesis of Intermediate Compound A151-3

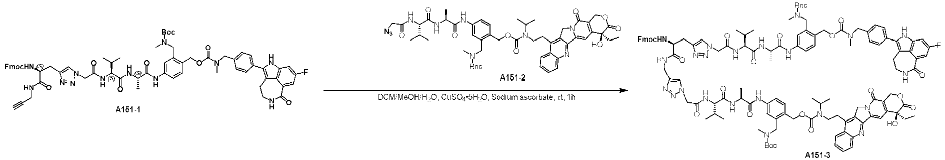

Compound A151-1 (250 mg, 0.201 mmol, 1.0 eq) and compound A151-2 (197 mg, 0.201 mmol, 1.0 eq) were dissolved in dichloromethane/methanol/water (6/6/0.5 mL). Under a nitrogen atmosphere, copper(II) sulfate pentahydrate (10.1 mg, 0.040 mmol, 0.2 eq, in 100 uL water) and sodium ascorbate (16.0 mg, 0.080 mmol, 0.4 eq, in 100 uL water) were added. After the addition was complete, the reaction mixture was stirred at room temperature under a nitrogen atmosphere for 1 hour. After LC-MS monitoring indicated completion, the reaction mixture was concentrated directly. The residue was dissolved in tetrahydrofuran and concentrated again to dryness, affording compound A151-3 (446 mg, 100%) as a brownish-yellow solid.
LCMS: [M+H]⁺ = 1110.8 (half peak)

### Synthesis of Intermediate Compound A151-4

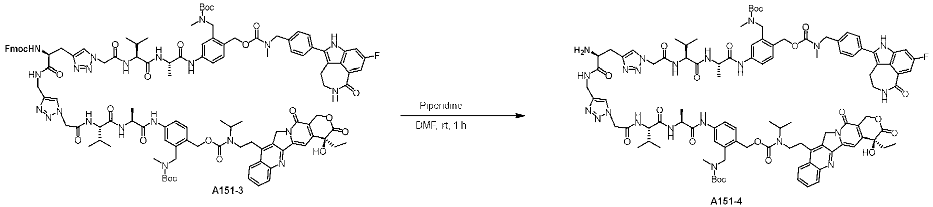

Compound A151-3 (446 mg, 0.201 mmol, 1.0 eq) was dissolved in DMF (5 mL). Under a nitrogen atmosphere, piperidine (1 mL) was added. The reaction mixture was stirred at room temperature under a nitrogen atmosphere for half an hour. After LC-MS monitoring indicated completion, methyl tert-butyl ether (50 mL) was added to the reaction mixture. The suspension was filtered directly, washed with methyl tert-butyl ether, and the filter cake was dried directly to afford compound A151-4 (380 mg, 95%) as a yellow solid.
LCMS: [M+H]⁺ = 999.8 (half peak)

### Synthesis of Intermediate Compound A151-6

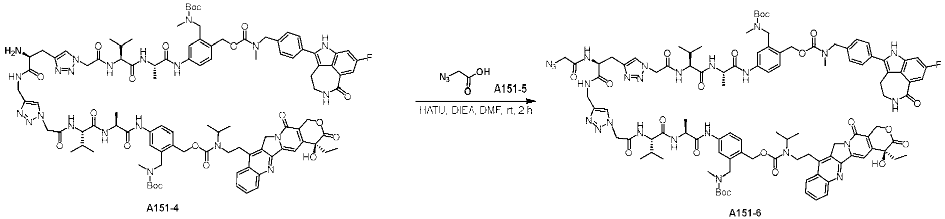

Compound A151-4 (390 mg, 0.195 mmol, 1.0 eq) was dissolved in DMF (6 mL). N,N-Diisopropylethylamine (126 mg, 0.975 mmol, 5.0 eq) and A151-5 (39 mg, 0.390 mmol, 2.0 eq) were added to the reaction mixture. Finally, under a nitrogen atmosphere, O-(7-Azabenzotriazol-1-yl)-N,N,N',N'-tetramethyluronium hexafluorophosphate (185 mg, 0.488 mmol, 2.5 eq) was added. After the addition was complete, the reaction mixture was stirred at room temperature under a nitrogen atmosphere for 1 hour. After LC-MS monitoring indicated completion, methyl tert-butyl ether (100 mL) was added to the reaction mixture. The suspension was filtered directly, washed with methyl tert-butyl ether, and the filter cake was dried directly to afford compound A151-6 (310 mg, 76%) as a brown solid.
LCMS: [M+H]⁺ = 1041.3 (half peak)

### Synthesis of Intermediate Compound A151-8

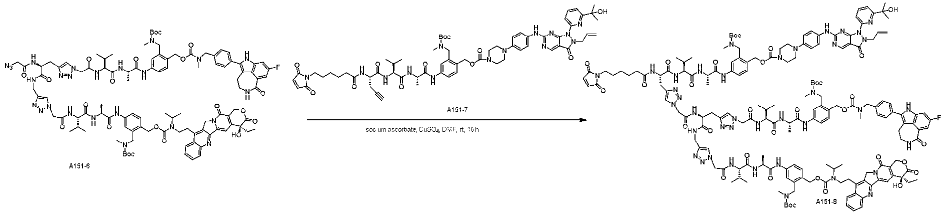

Compound A151-6 (240 mg, 0.115 mmol, 1.0 eq) and compound A151-7 (143 mg, 0.115 mmol, 1.0 eq) were dissolved in DMF (4 mL). Under a nitrogen atmosphere, copper(II) sulfate pentahydrate (5.8 mg, 0.023 mmol, 0.2 eq, in 100 uL water) and sodium ascorbate (9.1 mg, 0.046 mmol, 0.4 eq, in 100 uL water) were added. After the addition was complete, the reaction mixture was stirred at room temperature under a nitrogen atmosphere for 2 hours. After LC-MS monitoring indicated completion, methyl tert-butyl ether (60 mL) was added to the reaction mixture. The suspension was filtered directly, washed with methyl tert-butyl ether, and the filter cake was dried directly to afford compound A151-8 (280 mg, 73%) as a yellow solid.
LCMS: [M+Na]⁺ = 1681.5 (half peak)

### Synthesis of Intermediate Compound A151-9

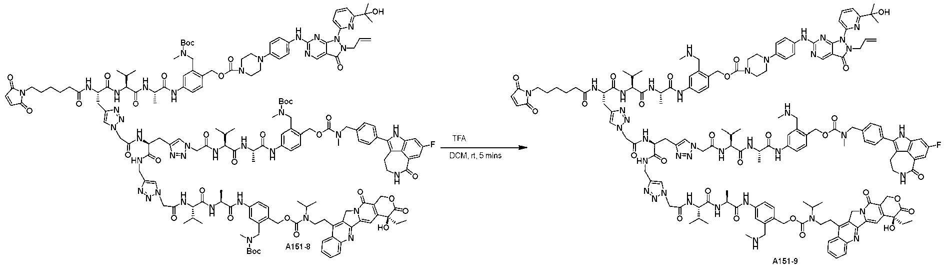

Compound A151-8 (260 mg, 0.078 mmol, 1.0 eq) was dissolved in DCM (3 mL). Trifluoroacetic acid (1 mL) was added to the reaction mixture, which was then stirred at room temperature for 5 minutes. After LC-MS monitoring indicated completion, the organic phase was concentrated to give the crude product compound A151-9, which was used directly in the next step.
LCMS: [M+H]⁺ = 1059.3 (half peak)

### Synthesis of Target Compound A151

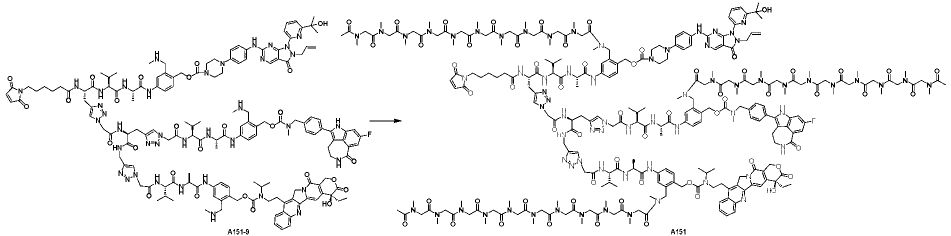

The compound A151-9 from the previous step was dissolved in DMF (5 mL). Ac-PSAR10-OH (180 mg, 0.234 mmol, 3.0 eq) and N,N-Diisopropylethylamine (201 mg, 1.56 mmol, 20.0 eq) were added to the reaction mixture. Finally, under a nitrogen atmosphere, O-(7-Azabenzotriazol-1-yl)-N,N,N',N'-tetramethyluronium hexafluorophosphate (98 mg, 0.257 mmol, 3.3 eq) was added. After the addition was complete, the reaction mixture was stirred at room temperature under a nitrogen atmosphere for 1 hour. After LC-MS monitoring indicated completion, ethyl acetate (60 mL) was added to the reaction mixture. The resulting suspension was filtered, and the filter cake was dissolved in DMF and purified by prep-HPLC (mobile phase: 0.05% HCOOH/CH₃CN/H₂O) to afford compound A151 (50 mg, 20%) as a yellow solid. LCMS: [M+H]⁺ = 1759.4 (one-third peak)

### (50) Synthesis of Compound A152

### Synthetic Route:

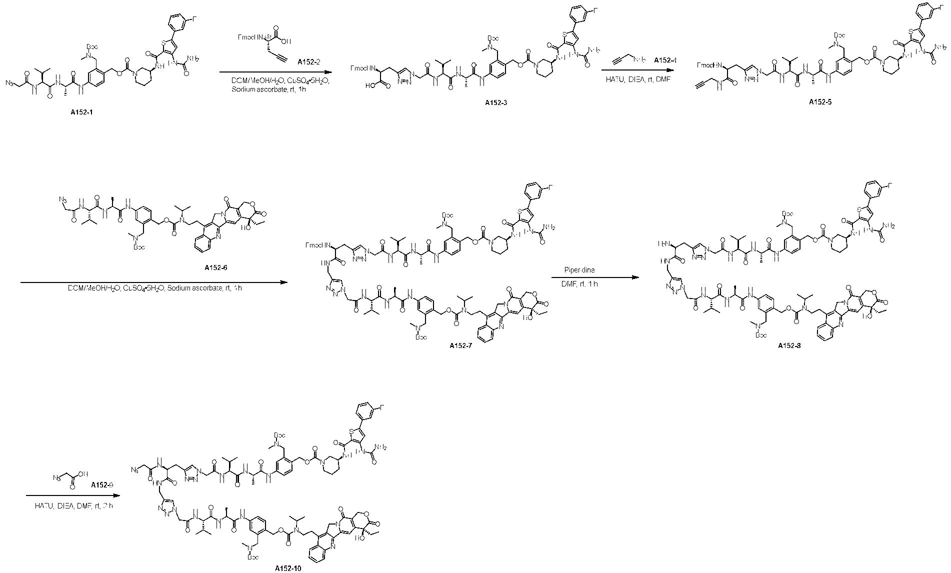

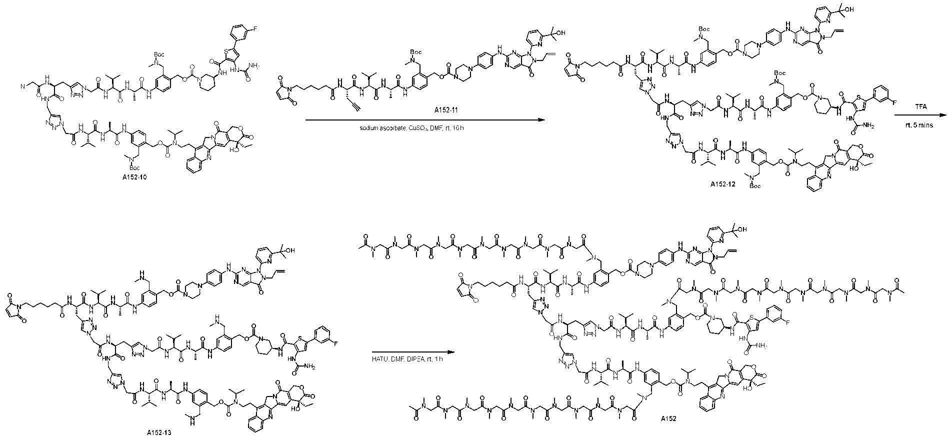

### Synthetic Steps:

### Synthesis of Intermediate Compound A152-3

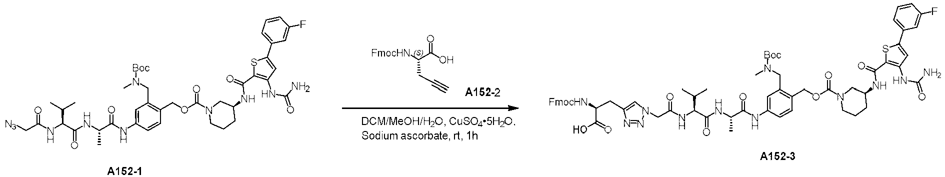

Compound A152-1 (500 mg, 0.551 mmol, 1.0 eq) and A152-2 (185 mg, 0.551 mmol, 1.0 eq) were dissolved in dichloromethane/methanol/water (10/10/1 mL). Under a nitrogen atmosphere, copper(II) sulfate pentahydrate (27.5 mg, 0.110 mmol, 0.1 eq, in 500 uL water) and sodium ascorbate (43.6 mg, 0.220 mmol, 0.2 eq, in 500 uL water) were added. After the addition was complete, the reaction mixture was stirred at room temperature under a nitrogen atmosphere for 1 hour. After LC-MS monitoring indicated completion, the reaction mixture was concentrated directly. Tetrahydrofuran was added, and the mixture was concentrated again to dryness, affording compound A152-3 (684 mg, 100%) as a grey solid.
LCMS: [M+Na]⁺ = 1266.3

### Synthesis of Intermediate Compound A152-5

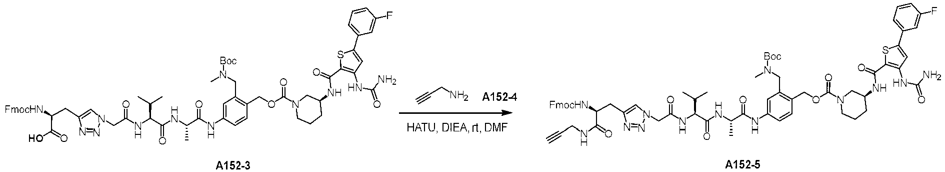

Compound A152-3 (660 mg, 0.531 mmol, 1.0 eq) was dissolved in DMF (8 mL). Compound A152-4 (44 mg, 0.797 mmol, 1.5 eq) and N,N-Diisopropylethylamine (205 mg, 1.593 mmol, 3.0 eq) were added to the reaction mixture. Finally, under a nitrogen atmosphere, O-(7-Azabenzotriazol-1-yl)-N,N,N',N'-tetramethyluronium hexafluorophosphate (262 mg, 0.690 mmol, 1.3 eq) was added. After the addition was complete, the reaction mixture was stirred at room temperature under a nitrogen atmosphere for 1 hour. After LC-MS monitoring indicated completion, methyl tert-butyl ether (80 mL) was added to the reaction mixture. The suspension was filtered directly, and the filter cake was collected and dried to afford compound A152-5 (557 mg, 82%) as a grey solid.
LCMS: [M+Na]⁺ = 1304.3

### Synthesis of Intermediate Compound A152-7

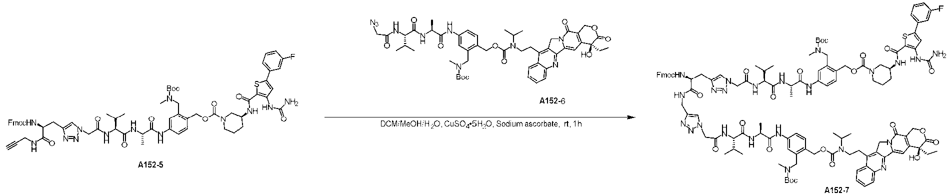

Compound A152-5 (530 mg, 0.414 mmol, 1.0 eq) and compound A152-6 (405 mg, 0.414 mmol, 1.0 eq) were dissolved in dichloromethane/methanol/water (6/6/0.5 mL). Under a nitrogen atmosphere, copper(II) sulfate pentahydrate (20.7 mg, 0.0828 mmol, 0.2 eq, in 100 uL water) and sodium ascorbate (32.7 mg, 0.166 mmol, 0.4 eq, in 100 uL water) were added. After the addition was complete, the reaction mixture was stirred at room temperature under a nitrogen atmosphere for 1 hour. After LC-MS monitoring indicated completion, the reaction mixture was concentrated directly. The residue was dissolved in tetrahydrofuran and concentrated again to dryness, affording compound A152-7 (935 mg, 100%) as a brownish-yellow solid.
LCMS: [M+H]⁺ = 1130.1 (half peak)

### Synthesis of Intermediate Compound A152-8

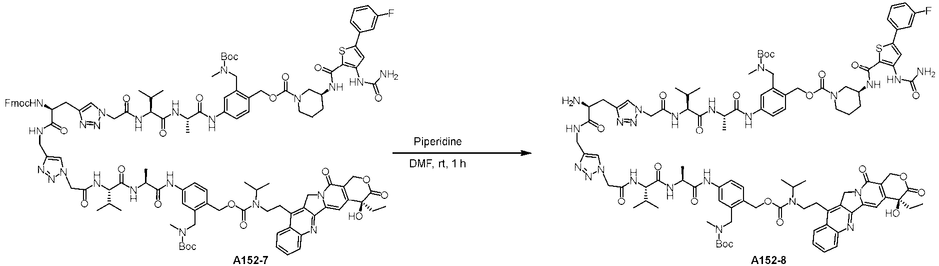

Compound A152-7 (935 mg, 0.414 mmol, 1.0 eq) was dissolved in DMF (10 mL). Under a nitrogen atmosphere, piperidine (2 mL) was added. The reaction mixture was stirred at room temperature under a nitrogen atmosphere for half an hour. After LC-MS monitoring indicated completion, methyl tert-butyl ether (150 mL) was added to the reaction mixture. The suspension was filtered directly, washed with methyl tert-butyl ether, and the filter cake was dried directly to afford compound A 152-8 (632 mg, 75%) as a yellow solid.
LCMS: [M+H]⁺ = 1019.3 (half peak)

### Synthesis of Intermediate Compound A152-10

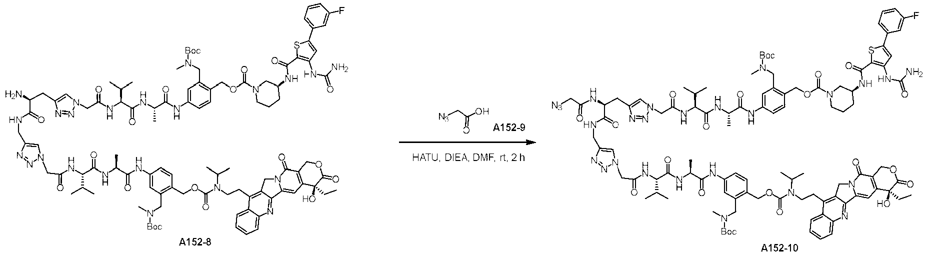

Compound A152-8 (610 mg, 0.300 mmol, 1.0 eq) was dissolved in DMF (10 mL). N,N-Diisopropylethylamine (117 mg, 0.900 mmol, 3.0 eq) and A152-9 (61 mg, 0.600 mmol, 2.0 eq) were added to the reaction mixture. Finally, under a nitrogen atmosphere, O-(7-Azabenzotriazol-1-yl)-N,N,N',N'-tetramethyluronium hexafluorophosphate (251 mg, 0.66 mmol, 2.2 eq) was added. After the addition was complete, the reaction mixture was stirred at room temperature under a nitrogen atmosphere for 1 hour. After LC-MS monitoring indicated completion, methyl tert-butyl ether (100 mL) was added to the reaction mixture. The suspension was filtered directly, washed with methyl tert-butyl ether, and the filter cake was dried directly to afford compound A152-10 (566 mg, 89%) as a yellow solid.
LCMS: [M+H]⁺ = 1060.8 (half peak)

### Synthesis of Intermediate Compound A152-12

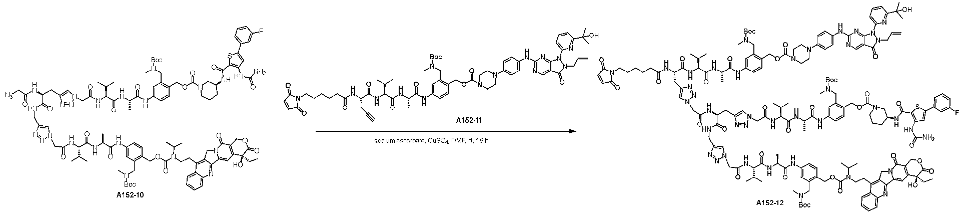

Compound A152-10 (160 mg, 0.075 mmol, 1.0 eq) and compound A152-11 (93 mg, 0.075 mmol, 1.0 eq) were dissolved in DMF (4 mL). Under a nitrogen atmosphere, copper(II) sulfate pentahydrate (4 mg, 0.015 mmol, 0.2 eq, in 100 uL water) and sodium ascorbate (6.0 mg, 0.030 mmol, 0.4 eq, in 100 uL water) were added. After the addition was complete, the reaction mixture was stirred at room temperature under a nitrogen atmosphere for 2 hours. After LC-MS monitoring indicated completion, methyl tert-butyl ether (80 mL) was added to the reaction mixture. The suspension was filtered directly, washed with methyl tert-butyl ether, and the filter cake was dried directly to afford compound A152-12 (220 mg, 87%) as a yellow solid.
LCMS: [M+H]⁺ = 1679.0 (half peak)

### Synthesis of Intermediate Compound A152-13

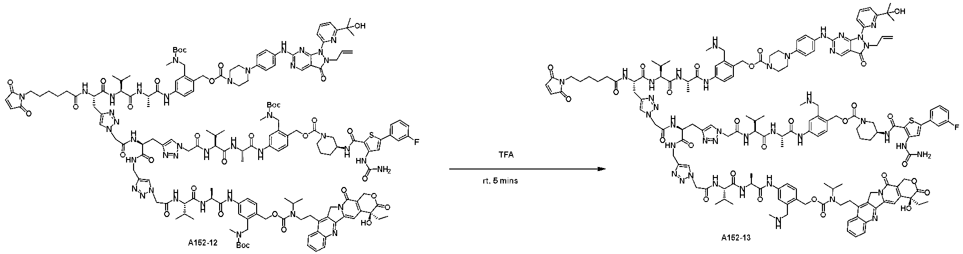

Compound A152-12 (220 mg, 0.066 mmol, 1.0 eq) was dissolved in DCM (3 mL). Trifluoroacetic acid (1 mL) was added to the reaction mixture, which was then stirred at room temperature for 5 minutes. After LC-MS monitoring indicated completion, the organic phase was concentrated to give the crude product compound A152-13, which was used directly in the next step.
LCMS: [M+H]⁺ = 1529.4 (half peak)

### Synthesis of Target Compound A152

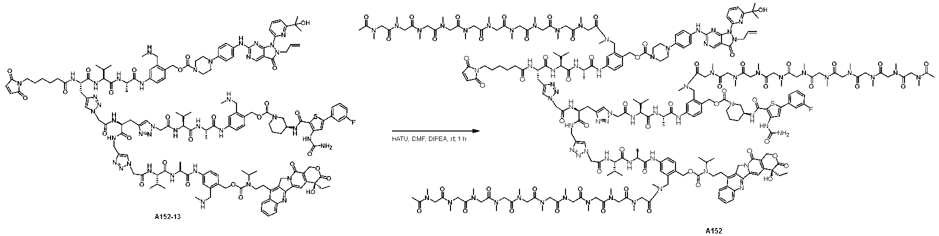

The compound A152-13 from the previous step was dissolved in DMF (4 mL). Ac-PSAR10-OH (153 mg, 0.198 mmol, 3.0 eq) and N,N-Diisopropylethylamine (170 mg, 1.32 mmol, 20.0 eq) were added to the reaction mixture. Finally, under a nitrogen atmosphere, O-(7-Azabenzotriazol-1-yl)-N,N,N',N'-tetramethyluronium hexafluorophosphate (83 mg, 0.218 mmol, 3.3 eq) was added. After the addition was complete, the reaction mixture was stirred at room temperature under a nitrogen atmosphere for 1 hour. After LC-MS monitoring indicated completion, ethyl acetate (60 mL) was added to the reaction mixture. The resulting suspension was filtered, and the filter cake was dissolved in DMF and purified by prep-HPLC (mobile phase: 0.05% HCOOH/CH₃CN/H₂O) to afford compound A152 (50 mg, 14%) as a yellow solid. LCMS: [M+H]⁺ = 1772.5 (one-third peak)

### (51) Synthesis of Compound A153

### Synthetic Route:

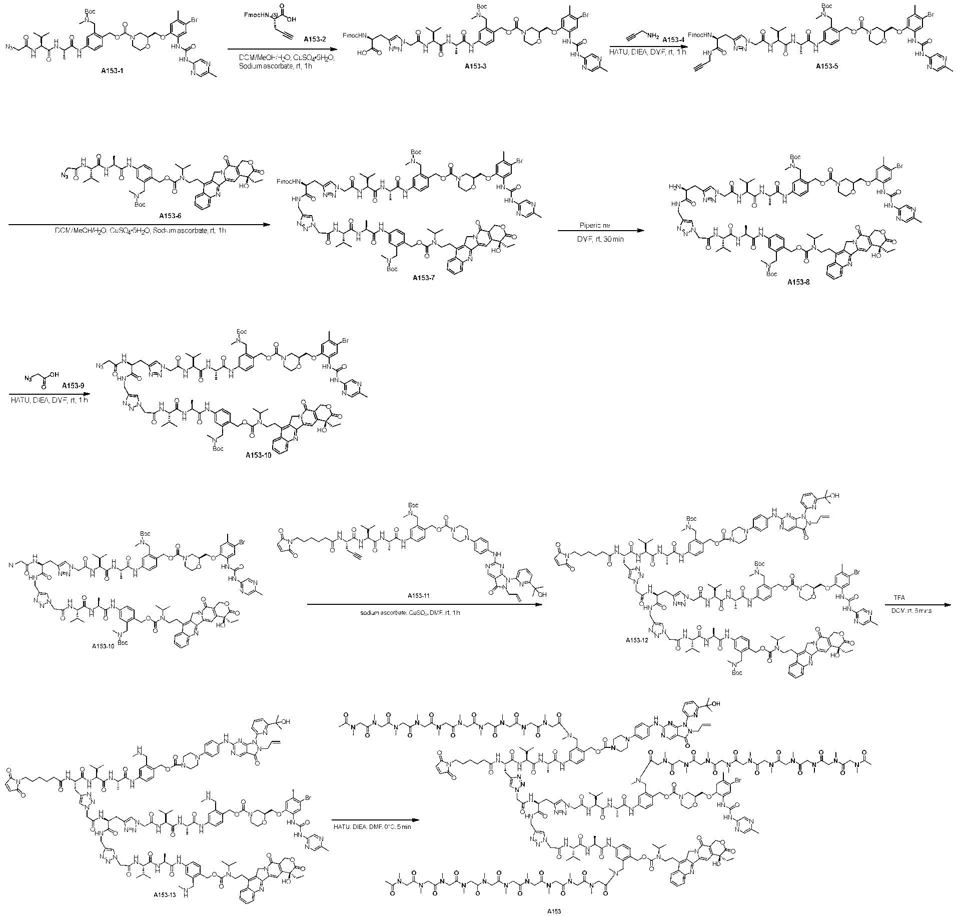

### Synthetic Steps:

### Synthesis of Intermediate Compound A153-3

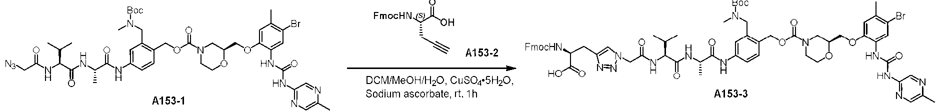

Compound A153-1 (510 mg, 0.519 mmol, 1.0 eq) and A153-2 (174 mg, 0.519 mmol, 1.0 eq) were dissolved in dichloromethane/methanol/water (10/10/1 mL). Under a nitrogen atmosphere, copper(II) sulfate pentahydrate (26.0 mg, 0.104 mmol, 0.2 eq, in 500 uL water) and sodium ascorbate (41.1 mg, 0.208 mmol, 0.4 eq, in 500 uL water) were added. After the addition was complete, the reaction mixture was stirred at room temperature under a nitrogen atmosphere for 1 hour. After LC-MS monitoring indicated completion, the reaction mixture was concentrated directly. Tetrahydrofuran was added, and the mixture was concentrated again to dryness, affording compound A153-3 (680 mg, 100%) as a yellow solid.
LCMS: [M+H]⁺ = 1316.4

### Synthesis of Intermediate Compound A153-5

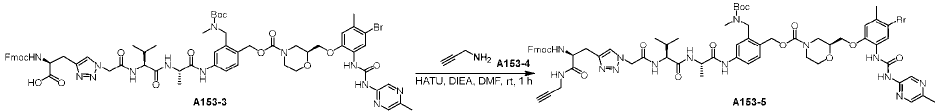

Compound A153-3 (670 mg, 0.508 mmol, 1.0 eq) was dissolved in DMF (8 mL). Compound A153-4 (35 mg, 0.610 mmol, 1.5 eq) and N,N-Diisopropylethylamine (197 mg, 1.525 mmol, 3.0 eq) were added to the reaction mixture. Finally, under a nitrogen atmosphere, O-(7-Azabenzotriazol-1-yl)-N,N,N',N'-tetramethyluronium hexafluorophosphate (251 mg, 0.661 mmol, 1.3 eq) was added. After the addition was complete, the reaction mixture was stirred at room temperature under a nitrogen atmosphere for 1 hour. After LC-MS monitoring indicated completion, methyl tert-butyl ether (80 mL) was added to the reaction mixture. The suspension was filtered directly, and the filter cake was collected and dried to afford compound A153-5 (480 mg, 70%) as a yellow solid.
LCMS: [M+H]⁺ = 1354.3

### Synthesis of Intermediate Compound A153-7

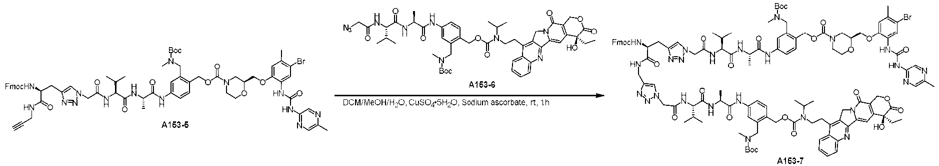

Compound A153-5 (450 mg, 0.332 mmol, 1.0 eq) and compound A153-6 (325 mg, 0.332 mmol, 1.0 eq) were dissolved in dichloromethane/methanol/water (5/5/0.5 mL). Under a nitrogen atmosphere, copper(II) sulfate pentahydrate (16.6 mg, 0.0664 mmol, 0.2 eq, in 100 uL water) and sodium ascorbate (26.3 mg, 0.133 mmol, 0.4 eq, in 100 uL water) were added. After the addition was complete, the reaction mixture was stirred at room temperature under a nitrogen atmosphere for 1 hour. After LC-MS monitoring indicated completion, the reaction mixture was concentrated directly. The residue was dissolved in tetrahydrofuran and concentrated again to dryness, affording compound A153-7 (770 mg, 100%) as a brownish-yellow solid.
LCMS: [M+H]⁺ = 1167.4 (half peak)

### Synthesis of Intermediate Compound A153-8

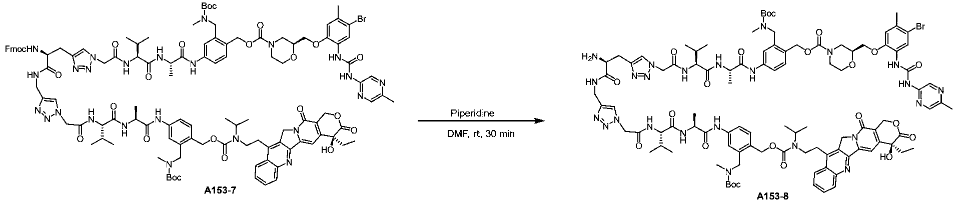

Compound A153-7 (770 mg, 0.414 mmol, 1.0 eq) was dissolved in DMF (10 mL). Under a nitrogen atmosphere, piperidine (2 mL) was added. The reaction mixture was stirred at room temperature under a nitrogen atmosphere for half an hour. After LC-MS monitoring indicated completion, methyl tert-butyl ether (150 mL) was added to the reaction mixture. The suspension was filtered directly, washed with methyl tert-butyl ether, and the filter cake was dried directly to afford compound A153-8 (670 mg, 96%) as a yellow solid.
LCMS: [M+H]⁺ = 1056.3 (half peak)

### Synthesis of Intermediate Compound A153-10

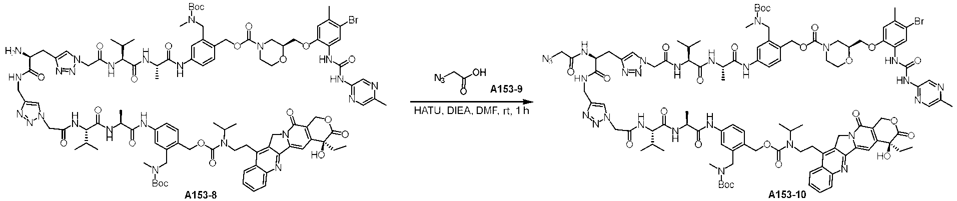

Compound A153-8 (470 mg, 0.222 mmol, 1.0 eq) was dissolved in DMF (8 mL). N,N-Diisopropylethylamine (86 mg, 0.667 mmol, 3.0 eq) and A153-9 (27 mg, 0.267 mmol, 1.2 eq) were added to the reaction mixture. Finally, under a nitrogen atmosphere, O-(7-Azabenzotriazol-1-yl)-N,N,N',N'-tetramethyluronium hexafluorophosphate (128 mg, 0.336 mmol, 1.5 eq) was added. After the addition was complete, the reaction mixture was stirred at room temperature under a nitrogen atmosphere for 1 hour. After LC-MS monitoring indicated completion, methyl tert-butyl ether (80 mL) was added to the reaction mixture. The suspension was filtered directly, washed with methyl tert-butyl ether, and the filter cake was dissolved in DMF and purified by prep-HPLC (mobile phase: 0.05% HCOOH/CH₃CN/H₂O) to afford compound A153-10 (130 mg, 27%) as a yellow solid.
LCMS: [M+H]⁺ = 1097.9 (half peak)

### Synthesis of Intermediate Compound A153-12

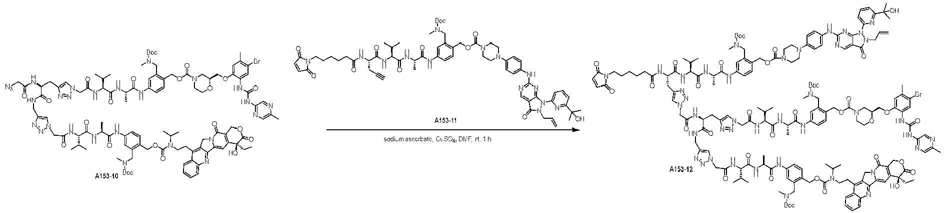

Compound A153-10 (100 mg, 0.0455 mmol, 1.0 eq) and compound A153-11 (56 mg, 0.0455 mmol, 1.0 eq) were dissolved in DMF (4 mL). Under a nitrogen atmosphere, copper(II) sulfate pentahydrate (2.3 mg, 0.0091 mmol, 0.2 eq, in 100 uL water) and sodium ascorbate (3.6 mg, 0.0182 mmol, 0.4 eq, in 100 uL water) were added. After the addition was complete, the reaction mixture was stirred at room temperature under a nitrogen atmosphere for 1 hour. After LC-MS monitoring indicated completion, methyl tert-butyl ether (80 mL) was added to the reaction mixture. The suspension was filtered directly, washed with methyl tert-butyl ether, and the filter cake was dried directly to afford compound A153-12 (150 mg, 96%) as a yellow solid. LCMS: [M+Na]⁺ = 1738.6 (half peak)

### Synthesis of Intermediate Compound A153-13

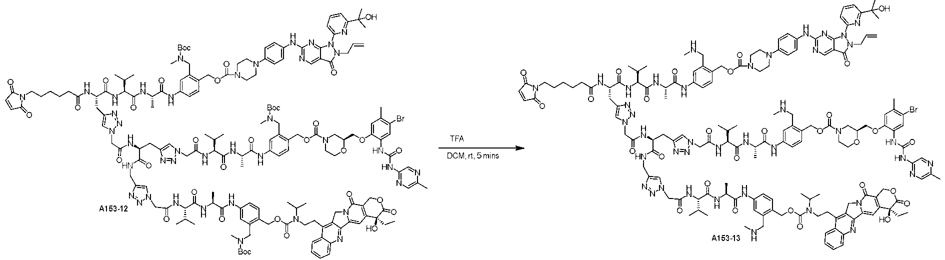

Compound A153-12 (140 mg, 0.0408 mmol, 1.0 eq) was dissolved in DCM (3 mL). Trifluoroacetic acid (1 mL) was added to the reaction mixture, which was then stirred at room temperature for 5 minutes. After LC-MS monitoring indicated completion, the organic phase was concentrated to give the crude product compound A153-13, which was used directly in the next step.
LCMS: [M+H]⁺ = 1565.6 (half peak)

### Synthesis of Target Compound A153

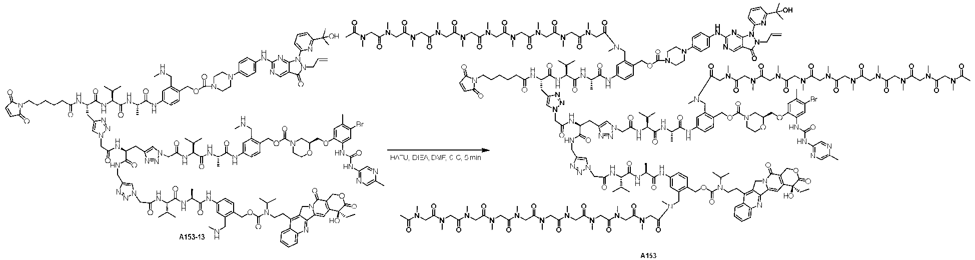

The compound A153-13 from the previous step was dissolved in DMF (3 mL). Compound A153-14 (94 mg, 0.1224 mmol, 3.0 eq) and N,N-Diisopropylethylamine (106 mg, 0.816 mmol, 20.0 eq) were added to the reaction mixture. Finally, under a nitrogen atmosphere, O-(7-Azabenzotriazol-1-yl)-N,N,N',N'-tetramethyluronium hexafluorophosphate (52 mg, 0.1346 mmol, 3.3 eq) was added. After the addition was complete, the reaction mixture was stirred at room temperature under a nitrogen atmosphere for 1 hour. After LC-MS monitoring indicated completion, ethyl acetate (60 mL) was added to the reaction mixture. The resulting suspension was filtered, and the filter cake was dissolved in DMF and purified by prep-HPLC (mobile phase: 0.05% HCOOH/CH₃CN/H₂O) to afford compound A153 (44.3 mg, 20%) as a yellow solid.
LCMS: [M+H]⁺ = 1797.9 (one-third peak)

### (52) Synthesis of Compound A154

### Synthetic Route:

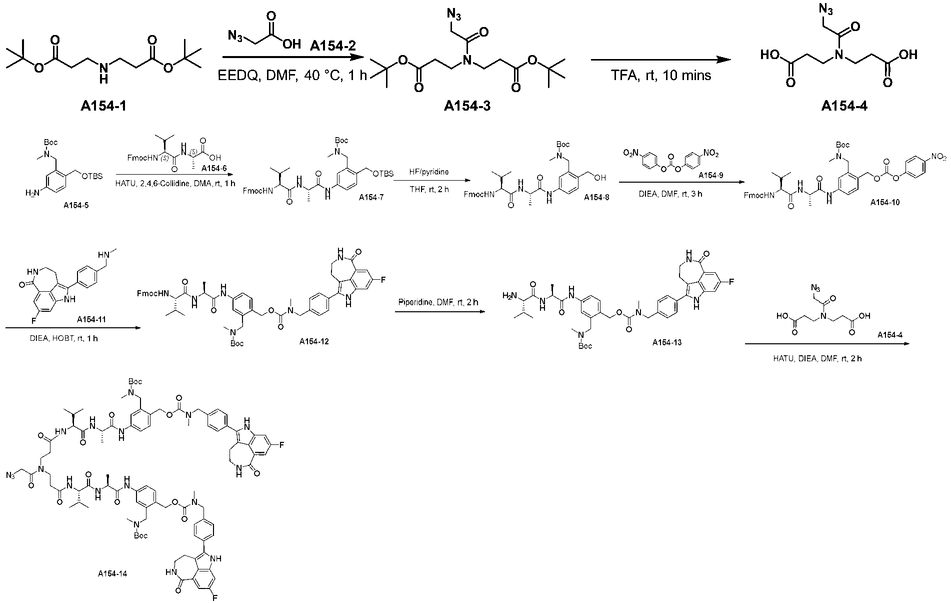

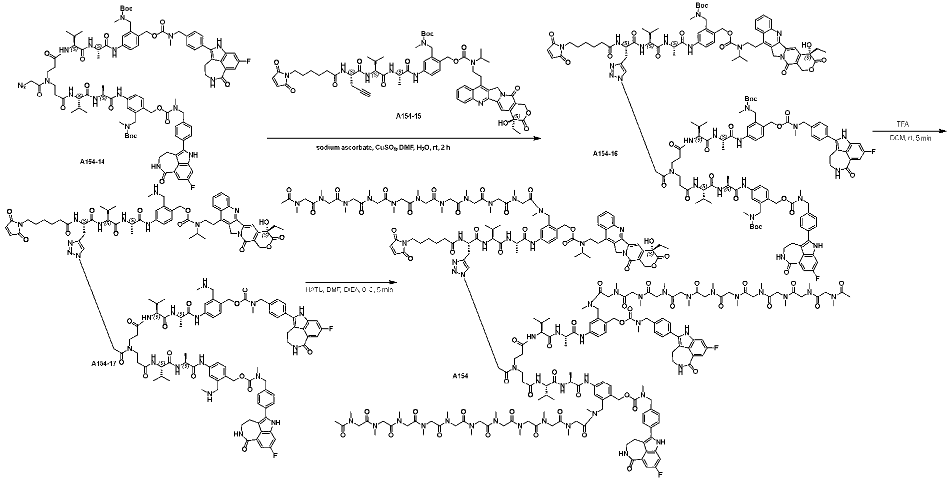

### Synthetic Steps:

### Synthesis of Intermediate Compound A154-3

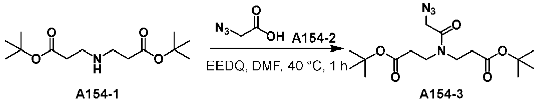

Compound A154-1 (500 mg, 1.829 mmol, 1.0 eq), compound A154-2 (222 mg, 2.195 mmol, 1.2 eq) and EEDQ (588 mg, 2.378 mmol, 1.3 eq) were dissolved in DMF (5 mL). After the addition was complete, the reaction mixture was stirred at 40 °C for 1 hour. After LC-MS monitoring indicated completion, the reaction mixture was directly loaded onto a reverse phase purification system (0.1% formic acid in water/acetonitrile = 1/1) and purified to afford compound A154-3 (500 mg, 75%) as a white solid.
LCMS: [M+Na]⁺ = 379.1

### Synthesis of Intermediate Compound A154-4

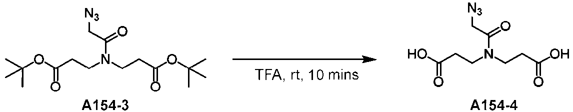

Compound A154-3 (250 mg, 0.701 mmol) was dissolved in TFA (3 mL). The reaction mixture was stirred at room temperature for 10 minutes. After LC-MS monitoring indicated completion, the organic phase was concentrated to give the crude product compound A154-4, which was used directly in the next step.
LCMS: [M+H]⁺ = 267.0

### Synthesis of Intermediate Compound A154-7

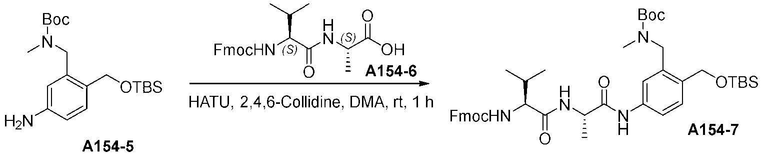

Compound A154-5 (820 mg, 2.149 mmol, 1.0 eq) was dissolved in DMA (10 mL). Compound A154-6 (882 mg, 2.149 mmol, 1.0 eq), 2,4,6-trimethylpyridine (781 mg, 6.447 mmol, 3.0 eq) and HATU (981 mg, 2.579 mmol, 1.3 eq) were added to the reaction mixture. The reaction mixture was stirred at room temperature for 1 hour. After LC-MS monitoring indicated completion, the reaction mixture was diluted with ethyl acetate, washed with water and saturated brine. The organic phase was dried over anhydrous sodium sulfate, filtered, and concentrated. The residue was purified by silica gel column chromatography (PE/EA=6/4) to afford compound A154-7 (1.4 g, 84%) as a yellow solid.
LCMS: [M+Na]⁺ = 795.3

### Synthesis of Intermediate Compound A154-8

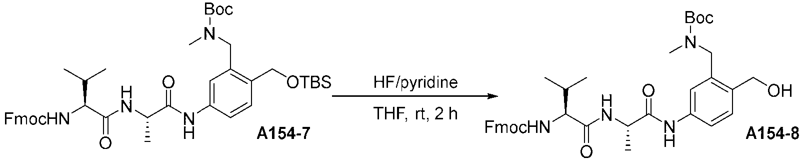

Compound A154-7 (1.3 g, 1.68 mmol, 1.0 eq) was dissolved in tetrahydrofuran (10 mL). The reaction mixture was cooled to 0 °C, and under a nitrogen atmosphere, HF/pyridine (2.05 g, 13.44 mmol, 8.0 eq, 65% w/w) was added. The reaction mixture was stirred at room temperature under a nitrogen atmosphere for 2 hours. After LC-MS monitoring indicated completion, the reaction mixture was diluted with ethyl acetate, washed with water, saturated sodium bicarbonate aqueous solution and saturated brine. The organic phase was dried over anhydrous sodium sulfate, filtered, and concentrated to afford compound A154-8 (1.1 g, 99%) as a white solid.
LCMS: [M+Na]⁺ = 681.3

### Synthesis of Intermediate Compound A154-10

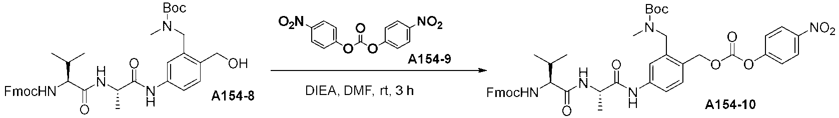

Compound A154-8 (600 mg, 0.909 mmol, 1.0 eq) and compound A154-9 (553 mg, 1.819 mmol, 2.0 eq) were dissolved in DMF (8 mL). N,N-Diisopropylethylamine (353 mg, 2.728 mmol, 3.0 eq) was then added. After the addition was complete, the reaction mixture was stirred at room temperature for 3 hours. After LC-MS monitoring indicated completion, the reaction mixture was diluted with ethyl acetate, washed with water and saturated brine. The organic phase was dried over anhydrous sodium sulfate, filtered, and concentrated. The residue was purified by silica gel column chromatography (DCM/MeOH=95/5) to afford compound A154-10 (700 mg, 93%) as a white solid.
LCMS: [M+Na]⁺ = 847.3

### Synthesis of Intermediate Compound A154-12

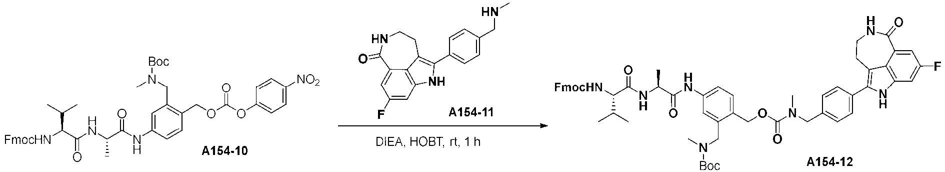

Compound A154-11 (196 mg, 0.606 mmol, 1.0 eq) was dissolved in DMF (5 mL). Then compound A154-10 (500 mg, 0.606 mmol, 1.0 eq), HOBt (80 mg, 0.606 mmol, 1.0 eq) and N,N-Diisopropylethylamine (235 mg, 1.818 mmol, 3.0 eq) were added. After the addition was complete, the reaction mixture was stirred at room temperature for 1 hour. After LC-MS monitoring indicated completion, the reaction mixture was diluted with ethyl acetate, washed with water and saturated brine. The organic phase was dried over anhydrous sodium sulfate, filtered, and concentrated. The residue was purified by silica gel column chromatography (DCM/MeOH=93/7) to afford compound A154-12 (560 mg, 92%) as a white solid.
LCMS: [M+H]⁺ = 1010.3

### Synthesis of Intermediate Compound A154-13

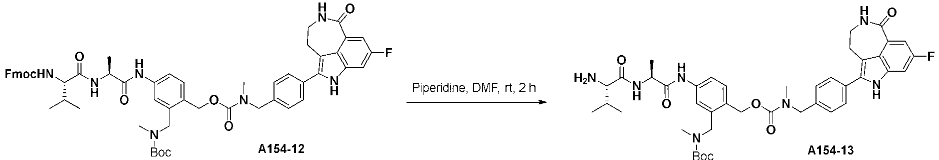

Compound A154-12 (550 mg, 0.545 mmol, 1.0 eq) was dissolved in DMF (5 mL). Piperidine (1 mL) was then added to the reaction mixture, which was stirred at room temperature for 2 hours. After LC-MS monitoring indicated completion, the reaction mixture was diluted with ethyl acetate, washed with water and saturated brine. The organic phase was dried over anhydrous sodium sulfate, filtered, and concentrated. The residue was purified by silica gel column chromatography (DCM/MeOH=93/7) to afford compound A154-13 (400 mg, 93%) as a white solid.
LCMS: [M+H]⁺ = 786.3

### Synthesis of Intermediate Compound A154-14

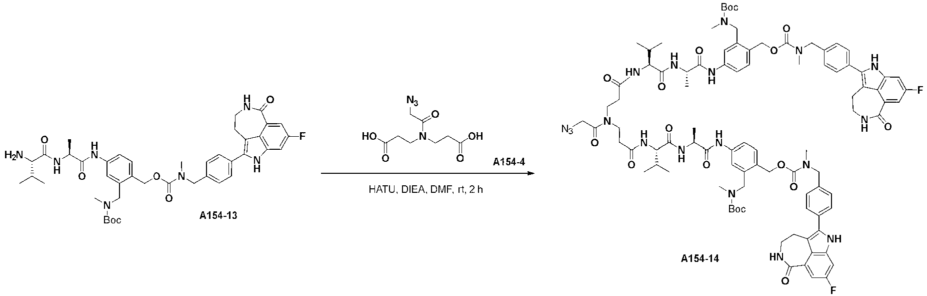

Compound A154-4 (26 mg, 0.1065 mmol, 1.0 eq) was dissolved in DMF (3 mL). N,N-Diisopropylethylamine (69 mg, 0.5325 mmol, 5.0 eq) was added to the reaction mixture, and after stirring for 10 minutes, compound A154-13 (168 mg, 0.213 mmol, 2.0 eq) and HATU (85 mg, 0.2237 mmol, 2.1 eq) were added. After the addition was complete, the reaction mixture was stirred at room temperature for 2 hours. After LC-MS monitoring indicated completion, the reaction mixture was diluted with ethyl acetate, washed with water and saturated brine. The organic phase was dried over anhydrous sodium sulfate, filtered, and concentrated. The residue was purified by silica gel column chromatography (DCM/MeOH=92/8) to afford compound A154-14 (180 mg, 95%) as a yellow solid.
LCMS: [M+H]⁺ = 890.3 (half peak)

### Synthesis of Intermediate Compound A154-16

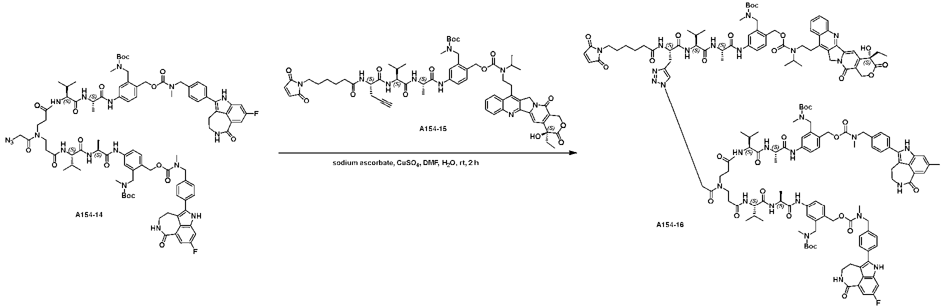

Compound A154-14 (160 mg, 0.0898 mmol, 1.0 eq) and compound A154-15 (106 mg, 0.0898 mmol, 1.0 eq) were dissolved in DMF (3 mL). Under a nitrogen atmosphere, copper(II) sulfate pentahydrate (4.5 mg, 0.0180 mmol, 0.2 eq, in 100 uL water) and sodium ascorbate (7.1 mg, 0.0359 mmol, 0.4 eq, in 100 uL water) were added. After the addition was complete, the reaction mixture was stirred at room temperature under a nitrogen atmosphere for 2 hours. After LC-MS monitoring indicated completion, methyl tert-butyl ether (60 mL) was added to the reaction mixture. The suspension was filtered directly, washed with methyl tert-butyl ether, and the filter cake was dried directly to afford compound A154-16 (230 mg, 86%) as a yellow solid. LCMS: [M+H]⁺ = 1482.5 (half peak)

### Synthesis of Intermediate Compound A154-17

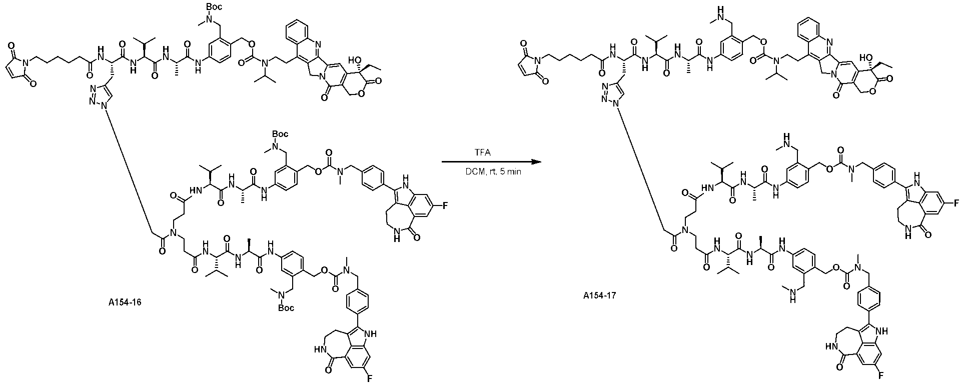

Compound A154-16 (220 mg, 0.0741 mmol, 1.0 eq) was dissolved in DCM (3 mL). Trifluoroacetic acid (1 mL) was added to the reaction mixture, which was then stirred at room temperature for 5 minutes. After LC-MS monitoring indicated completion, the organic phase was concentrated to give the crude product compound A154-17, which was used directly in the next step.
LCMS: [M+H]⁺ = 1332.2 (half peak)

### Synthesis of Target Compound A154

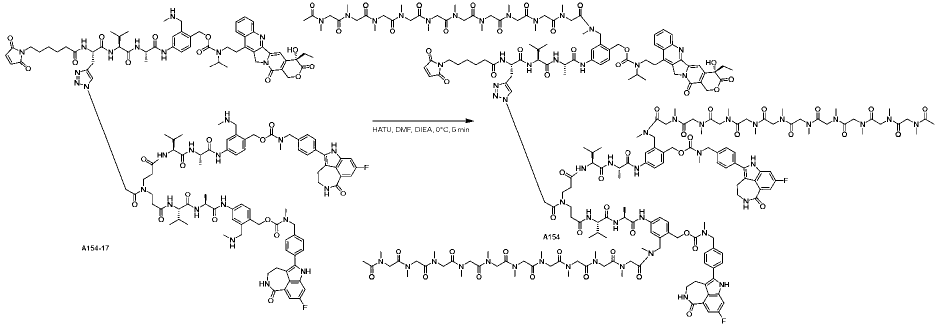

The compound A154-17 from the previous step was dissolved in DMF (3 mL). Compound A154-18 (172 mg, 0.2223 mmol, 3.0 eq) was added to the reaction mixture. Finally, under a nitrogen atmosphere, N,N-Diisopropylethylamine (287 mg, 2.223 mmol, 30.0 eq) and O-(7-Azabenzotriazol-1-yl)-N,N,N',N'-tetramethyluronium hexafluorophosphate (93 mg, 0.2445 mmol, 3.3 eq) were added. After the addition was complete, the reaction mixture was stirred at 0 °C under a nitrogen atmosphere for 5 minutes. After LC-MS monitoring indicated completion, ethyl acetate (60 mL) was added to the reaction mixture. The resulting suspension was filtered, and the filter cake was dissolved in DMF and purified by prep-HPLC (mobile phase: 0.05% HCOOH/CH₃CN/H₂O) to afford compound A154 (51.3 mg, 14%) as a white solid.
LCMS: [M+H]⁺ = 1641.3 (one-third peak)

### (53) Synthesis of Compound A155

### Synthetic Route:

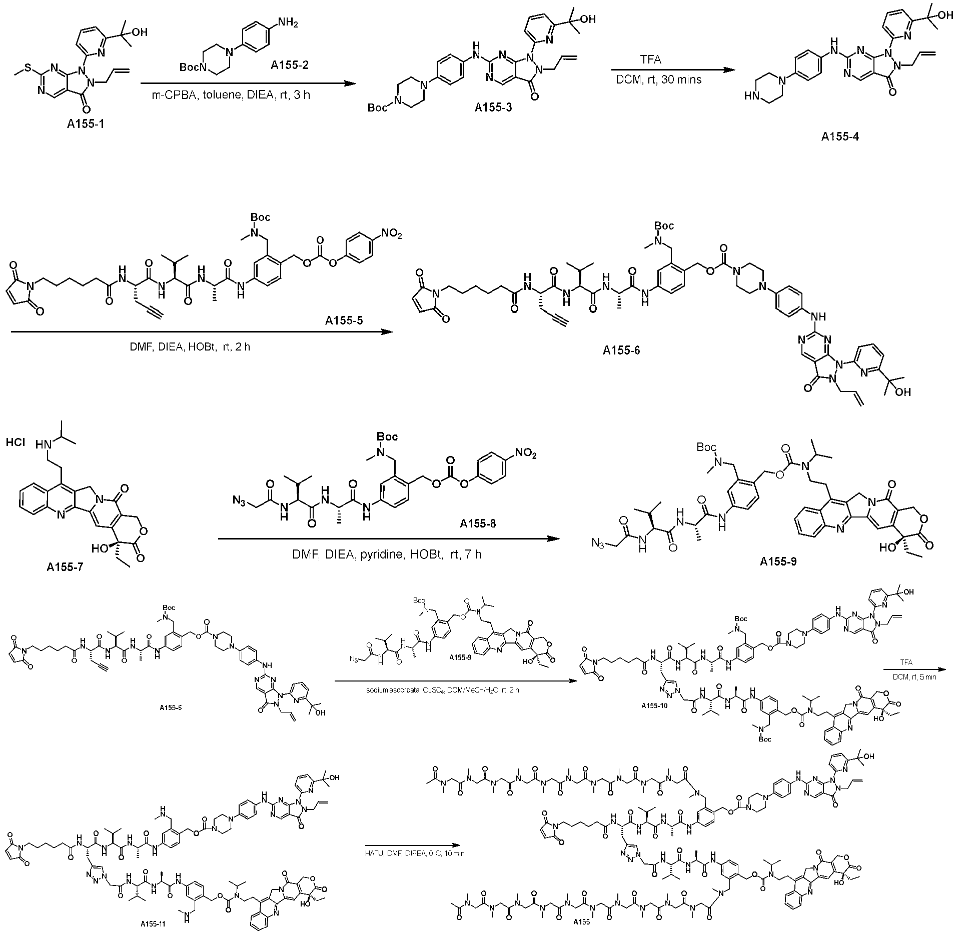

### Synthetic Steps:

### Synthesis of Intermediate Compound A155-3

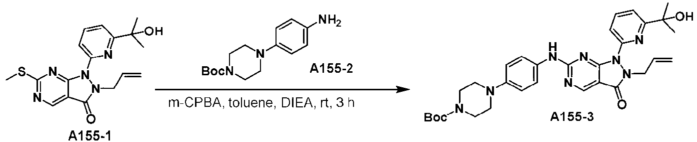

Compound **A155-1** (2 g, 5.596 mmol, 1.0 eq) was dissolved in toluene (30 mL). Then m-CPBA (1.36 g, 6.715 mmol, 1.2 eq, 85%) was added, and the reaction mixture was stirred at room temperature for 1 hour. Then N,N-Diisopropylethylamine (3.25 g, 25.180 mmol, 4.5 eq) and compound **A155-2** (1.55 g, 6.715 mmol, 1.2 eq) were added. After the addition was complete, the reaction mixture was stirred at room temperature for 2 hours. After LC-MS monitoring indicated completion, the reaction mixture was diluted with ethyl acetate, washed with water and saturated brine. The organic phase was dried over anhydrous sodium sulfate, filtered, and concentrated. The residue was purified by silica gel column chromatography (DCM/EA=3/7) to afford compound **A155-3** (2.2 g, 67%) as a dark green solid.

LCMS: [M+H]⁺ = 587.3

### Synthesis of Intermediate Compound A155-4

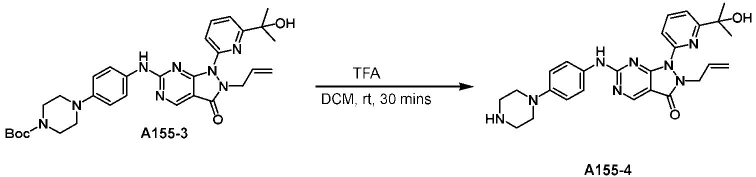

Compound **A155-3** (2.1 g, 3.573 mmol, 1.0 eq) was dissolved in DCM (15 mL). Trifluoroacetic acid (5 mL) was added to the reaction mixture, which was then stirred at room temperature for 30 minutes. After LC-MS monitoring indicated completion, the organic phase was concentrated to give the crude product compound **A155-4,** which was used directly in the next step.

LCMS: [M+H]⁺ = 487.2

### Synthesis of Compound A155-6

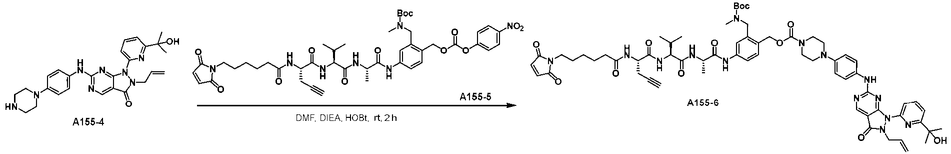

The compound **A155-4** from the previous step was dissolved in DMF (15 mL). N,N-Diisopropylethylamine (4.62 g, 35.733 mmol, 10.0 eq) was added, and after stirring for 10 minutes, compound **A155-5** (3.18 g, 3.573 mmol, 1.0 eq) and HOBt (242 mg, 1.787 mmol, 0.5 eq) were added. After the addition was complete, the reaction mixture was stirred at room temperature for 2 hours. After LC-MS monitoring indicated completion, the reaction mixture was diluted with ethyl acetate, washed with water and saturated brine. The organic phase was dried over anhydrous sodium sulfate, filtered, and concentrated. The residue was purified by silica gel column chromatography (DCM/MeOH=93/7) to afford compound **A155-6** (2.1 g, 47%) as a yellow solid.

LCMS: [M+H]⁺ = 1238.5

### Synthesis of Compound A155-9

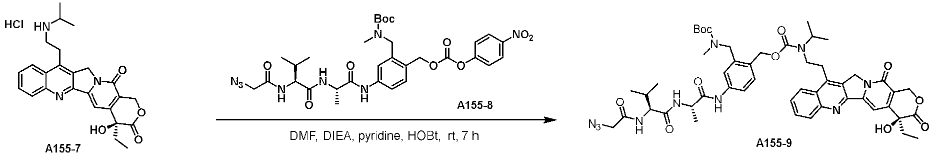

Compound **A155-7** (500 mg, 1.064 mmol, 1.0 eq) was dissolved in DMF (10 mL). N,N-Diisopropylethylamine (138 mg, 1.064 mmol, 1.0 eq) was added and stirred until clear, then pyridine (168 mg, 2.128 mmol, 2.0 eq), compound **A155-8** (730 mg, 1.064 mmol, 1.0 eq) and HOBt (15 mg, 0.106 mmol, 0.1 eq) were added. After the addition was complete, the reaction mixture was stirred at room temperature for 7 hours. After LC-MS monitoring indicated completion, the reaction mixture was diluted with 2-methyltetrahydrofuran, washed with water and saturated brine. The organic phase was dried over anhydrous sodium sulfate, filtered, and concentrated. The residue was purified by silica gel column chromatography (DCM/MeOH=93/7) to afford compound **A155-9** (850 mg, 82%) as a white solid.

LCMS: [M+H]⁺ = 979.4

### Synthesis of Compound A155-10

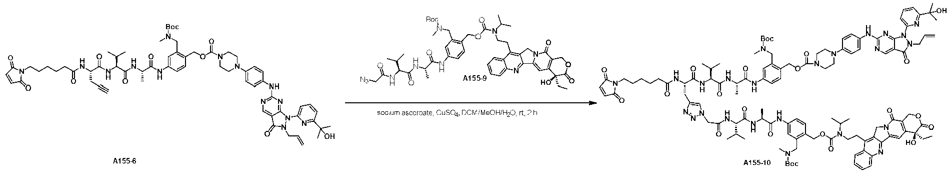

Compound **A155-6** (150 mg, 0.121 mmol, 1.0 eq) and compound **A155-9** (119 mg, 0.121 mmol, 1.0 eq) were dissolved in MeOH/DCM/water (5/5/1 mL). Then 100 uL of an aqueous solution of copper(II) sulfate pentahydrate (6.06 mg, 0.0242 mmol, 0.2 eq) and 100 uL of an aqueous solution of sodium ascorbate (9.6 mg, 0.0484 mmol, 0.4 eq) were added. After the addition was complete, the reaction mixture was stirred at room temperature under a nitrogen atmosphere for 2 hours. After LC-MS monitoring indicated completion, the reaction mixture was concentrated directly. Tetrahydrofuran was added, and the mixture was concentrated again to dryness, affording compound **A155-10** (250 mg, 93%) as a yellow solid.

LCMS: [M+H]⁺ = 1108.5 (half peak)

### Synthesis of Compound A155-11

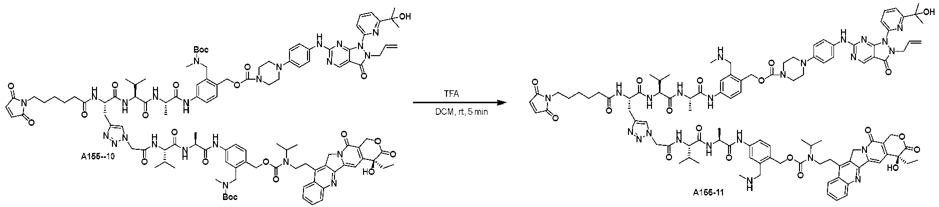

Compound **A155-10** (240 mg, 0.108 mmol) was dissolved in DCM (3 mL). Trifluoroacetic acid (1 mL) was added to the reaction mixture, which was then stirred at room temperature for 5 minutes. After LC-MS monitoring indicated completion, the organic phase was concentrated to give the crude product compound **A155-11,** which was used directly in the next step.

LCMS: [M+H]⁺ = 1009.0 (half peak)

### Synthesis of Compound A155

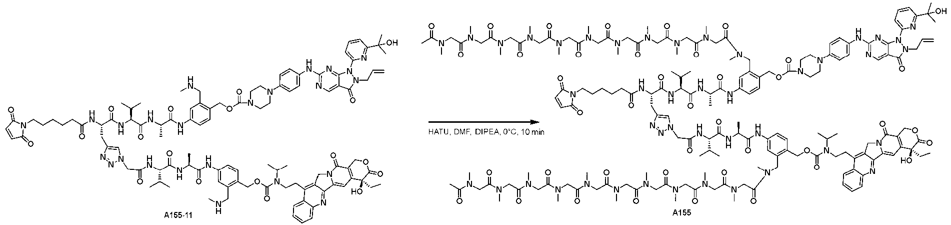

The compound **A155-11** from the previous step was dissolved in DMF (3 mL). Ac-PSAR10-OH (167 mg, 0.216 mmol, 2.0 eq) was added to the reaction mixture. Finally, under a nitrogen atmosphere, N,N-Diisopropylethylamine (280 mg, 2.164 mmol, 20.0 eq) and O-(7-Azabenzotriazol-1-yl)-N,N,N',N'-tetramethyluronium hexafluorophosphate (86 mg, 0.227 mmol, 2.1 eq) were added. After the addition was complete, the reaction mixture was stirred at 0 °C under a nitrogen atmosphere for 10 minutes. After LC-MS monitoring indicated completion, ethyl acetate (45 mL) was added to the reaction mixture. The mixture was filtered, and the filter cake was dissolved in DMF and purified by prep-HPLC (mobile phase: 0.05% HCOOH/CH₃CN/H₂O) to afford compound **A155** (111.4 mg, 29%) as a yellow solid.
LCMS: [M+H]⁺ = 1761.7 (half peak)

### (54) Synthesis of Compound A159

### Synthetic Route:

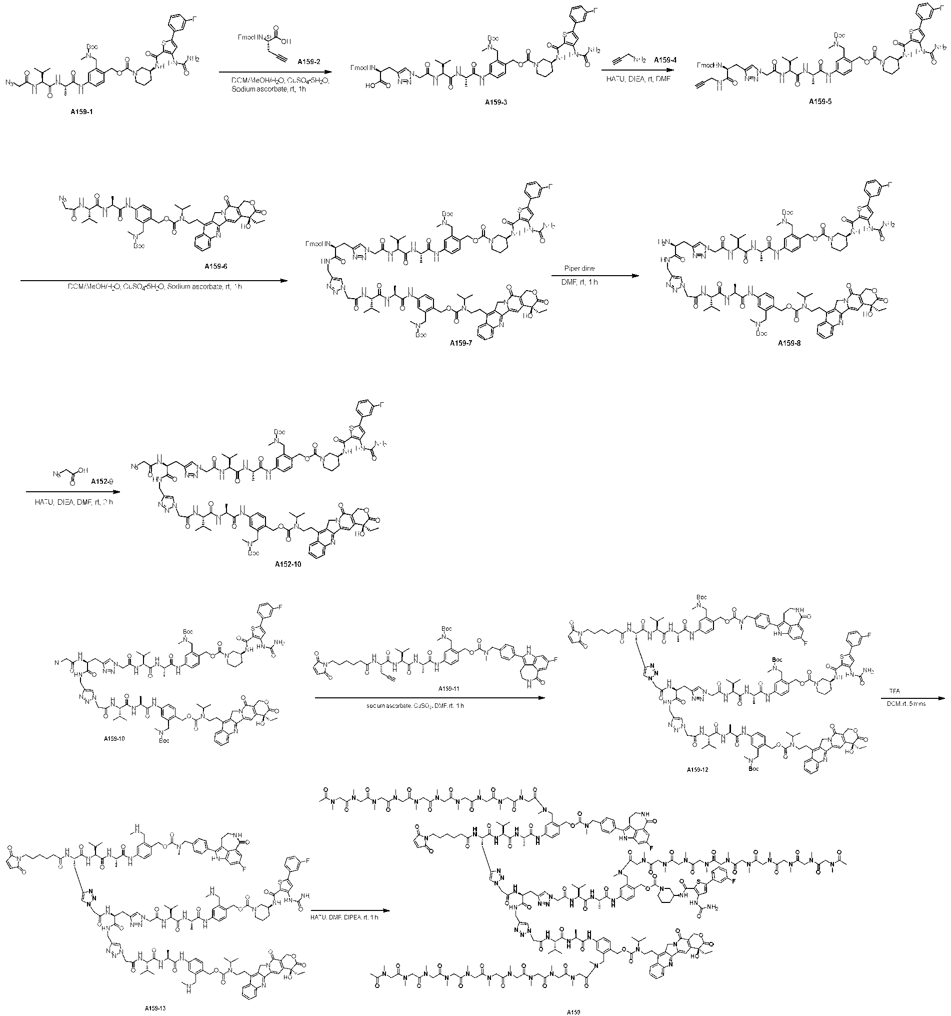

### Synthetic Steps:

### Synthesis of Intermediate Compound A159-3

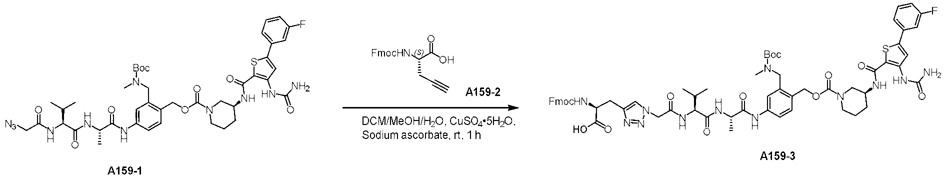

Compound **A159-1** (500 mg, 0.551 mmol, 1.0 eq) and **A159-2** (185 mg, 0.551 mmol, 1.0 eq) were dissolved in dichloromethane/methanol/water (10/10/1 mL). Under a nitrogen atmosphere, copper(II) sulfate pentahydrate (27.5 mg, 0.110 mmol, 0.1 eq, in 500 uL water) and sodium ascorbate (43.6 mg, 0.220 mmol, 0.2 eq, in 500 uL water) were added. After the addition was complete, the reaction mixture was stirred at room temperature under a nitrogen atmosphere for 1 hour. After LC-MS monitoring indicated completion, the reaction mixture was concentrated directly. Tetrahydrofuran was added, and the mixture was concentrated again to dryness, affording compound **A159-3** (684 mg, 100%) as a grey solid.
LCMS: [M+Na]⁺ = 1266.3

### Synthesis of Intermediate Compound A159-5

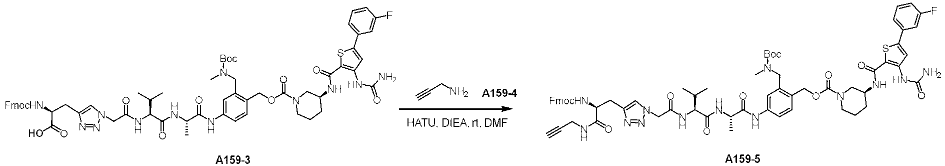

Compound **A159-3** (660 mg, 0.531 mmol, 1.0 eq) was dissolved in DMF (8 mL). Compound **A159-4** (44 mg, 0.797 mmol, 1.5 eq) and N,N-Diisopropylethylamine (205 mg, 1.593 mmol, 3.0 eq) were added to the reaction mixture. Finally, under a nitrogen atmosphere, O-(7-Azabenzotriazol-1-yl)-N,N,N',N'-tetramethyluronium hexafluorophosphate (262 mg, 0.690 mmol, 1.3 eq) was added. After the addition was complete, the reaction mixture was stirred at room temperature under a nitrogen atmosphere for 1 hour. After LC-MS monitoring indicated completion, methyl tert-butyl ether (80 mL) was added to the reaction mixture. The suspension was filtered directly, and the filter cake was collected and dried to afford compound **A159-5** (557 mg, 82%) as a grey solid.
LCMS: [M+Na]⁺ = 1304.3

### Synthesis of Intermediate Compound A159-7

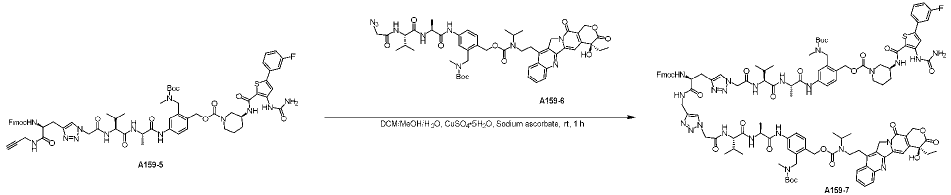

Compound **A159-5** (530 mg, 0.414 mmol, 1.0 eq) and compound **A159-6** (405 mg, 0.414 mmol, 1.0 eq) were dissolved in dichloromethane/methanol/water (6/6/0.5 mL). Under a nitrogen atmosphere, copper(II) sulfate pentahydrate (20.7 mg, 0.0828 mmol, 0.2 eq, in 100 uL water) and sodium ascorbate (32.7 mg, 0.166 mmol, 0.4 eq, in 100 uL water) were added. After the addition was complete, the reaction mixture was stirred at room temperature under a nitrogen atmosphere for 1 hour. After LC-MS monitoring indicated completion, the reaction mixture was concentrated directly. The residue was dissolved in tetrahydrofuran and concentrated again to dryness, affording compound **A159-7** (935 mg, 100%) as a brownish-yellow solid.
LCMS: [M+H]⁺ = 1130.1 (half peak)

### Synthesis of Intermediate Compound A159-8

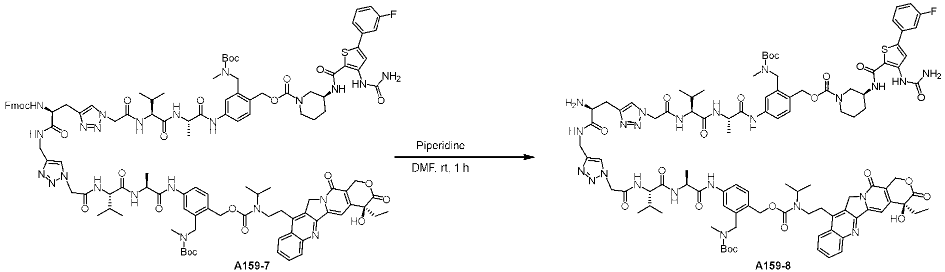

Compound **A159-7** (935 mg, 0.414 mmol, 1.0 eq) was dissolved in DMF (10 mL). Under a nitrogen atmosphere, piperidine (2 mL) was added. The reaction mixture was stirred at room temperature under a nitrogen atmosphere for 1 hour. After LC-MS monitoring indicated completion, methyl tert-butyl ether (150 mL) was added to the reaction mixture. The suspension was filtered directly, washed with methyl tert-butyl ether, and the filter cake was dried directly to afford compound **A159-8** (632 mg, 75%) as a yellow solid.
LCMS: [M+H]⁺ = 1019.3 (half peak)

### Synthesis of Intermediate Compound A159-10

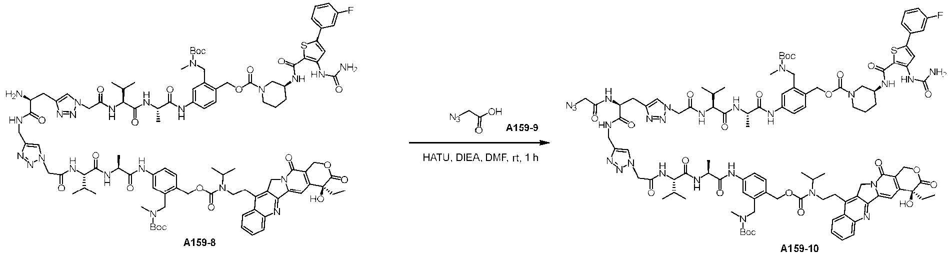

Compound **A159-8** (610 mg, 0.300 mmol, 1.0 eq) was dissolved in DMF (10 mL). N,N-Diisopropylethylamine (117 mg, 0.900 mmol, 3.0 eq) and **A159-9** (61 mg, 0.600 mmol, 2.0 eq) were added to the reaction mixture. Finally, under a nitrogen atmosphere, O-(7-Azabenzotriazol-1-yl)-N,N,N',N'-tetramethyluronium hexafluorophosphate (251 mg, 0.66 mmol, 2.2 eq) was added. After the addition was complete, the reaction mixture was stirred at room temperature under a nitrogen atmosphere for 1 hour. After LC-MS monitoring indicated completion, methyl tert-butyl ether (100 mL) was added to the reaction mixture. The suspension was filtered directly, washed with methyl tert-butyl ether, and the filter cake was dried directly to afford compound **A159-10** (566 mg, 89%) as a yellow solid.
LCMS: [M+H]⁺ = 1060.8 (half peak)

### Synthesis of Intermediate Compound A159-12

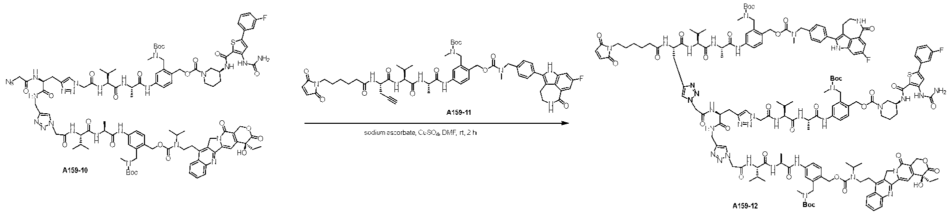

Compound **A159-10** (296 mg, 0.139 mmol, 1.0 eq) and compound **A159-11** (150 mg, 0.139 mmol, 1.0 eq) were dissolved in DMF (4 mL). Under a nitrogen atmosphere, copper(II) sulfate pentahydrate (7 mg, 0.028 mmol, 0.2 eq, in 100 uL water) and sodium ascorbate (11 mg, 0.0558 mmol, 0.4 eq, in 100 uL water) were added. After the addition was complete, the reaction mixture was stirred at room temperature under a nitrogen atmosphere for 2 hours. After LC-MS monitoring indicated completion, methyl tert-butyl ether (80 mL) was added to the reaction mixture. The suspension was filtered directly, washed with methyl tert-butyl ether, and the filter cake was dried directly to afford compound **A159-12** (370 mg, 83%) as a yellow solid.
LCMS: [M+H]⁺ = 1597.5 (half peak)

### Synthesis of Intermediate Compound A159-13

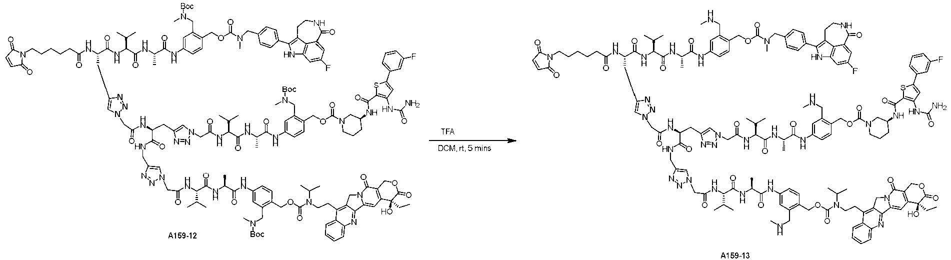

Compound A159-12 (180 mg, 0.0546 mmol, 1.0 eq) was dissolved in DCM (3 mL). Trifluoroacetic acid (1 mL) was added to the reaction mixture, which was then stirred at room temperature for 5 minutes. After LC-MS monitoring indicated completion, the organic phase was concentrated to give the crude product compound A159-13, which was used directly in the next step.
LCMS: [M+H]⁺ = 1447.4 (half peak)

### Synthesis of Target Compound A159

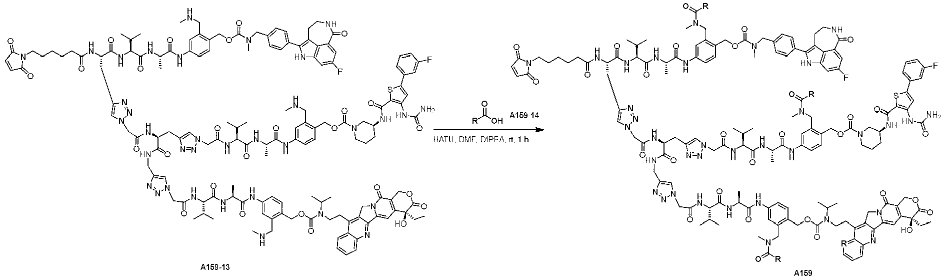

The compound A159-13 from the previous step was dissolved in DMF (3 mL). Compound A159-14 (126 mg, 0.164 mmol, 3.0 eq) and N,N-Diisopropylethylamine (141 mg, 1.09 mmol, 20.0 eq) were added to the reaction mixture. Finally, under a nitrogen atmosphere, O-(7-Azabenzotriazol-1-yl)-N,N,N',N'-tetramethyluronium hexafluorophosphate (69 mg, 0.180 mmol, 3.3 eq) was added. After the addition was complete, the reaction mixture was stirred at room temperature under a nitrogen atmosphere for 1 hour. After LC-MS monitoring indicated completion, ethyl acetate (60 mL) was added to the reaction mixture. The resulting suspension was filtered, and the filter cake was dissolved in DMF and purified by prep-HPLC (mobile phase: 0.05% HCOOH/CH₃CN/H₂O) to afford compound A159 (12 mg, 4%) as a white solid. LCMS: [M+H]⁺ = 1718.0 (one-third peak)

### (55) Synthesis of Compound A160

### Synthetic Route:

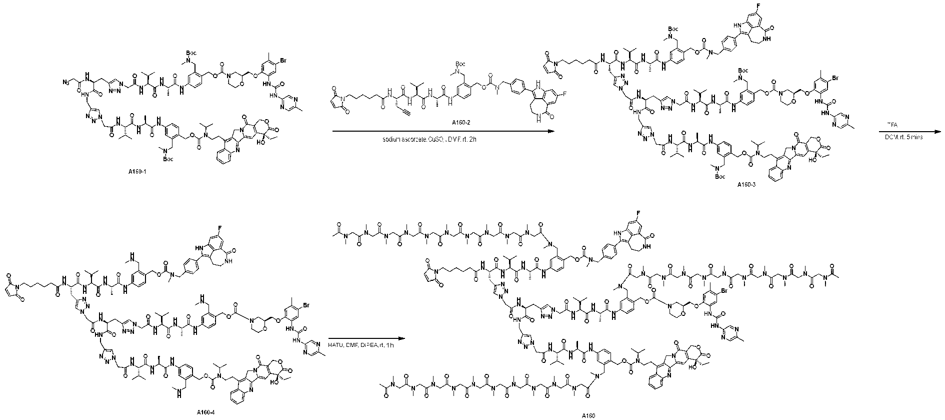

### Synthetic Steps:

### Synthesis of Intermediate Compound A160-3

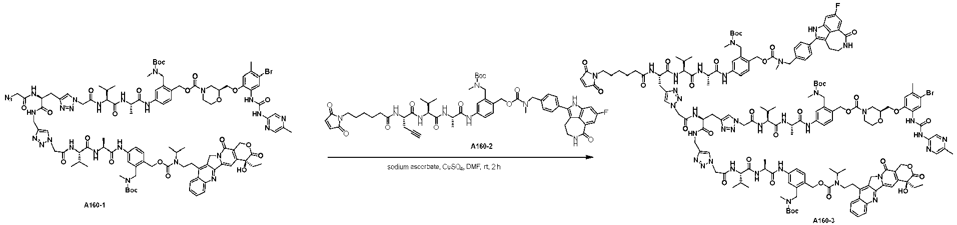

Compound **A160-1** (1500 mg, 0.70 mmol, 1.0 eq) and compound **A160-2** (750 mg, 0.70 mmol, 1.0 eq) were dissolved in DMF (24 mL). Under a nitrogen atmosphere, copper(II) sulfate pentahydrate (20 mg, 0.07 mmol, 0.1 eq, in 500 uL water) and sodium ascorbate (60 mg, 0.28 mmol, 0.4 eq, in 500 uL water) were added. After the addition was complete, the reaction mixture was stirred at room temperature under a nitrogen atmosphere for 2 hours. After LC-MS monitoring indicated completion, methyl tert-butyl ether (2400 mL) was added to the reaction mixture. The suspension was filtered directly, washed with methyl tert-butyl ether, and the filter cake was dried directly to afford compound **A160-3** (1500 mg, 59%) as a yellow solid.
LCMS: [M+Na]⁺ = 1656.0 (half peak)

### Synthesis of Intermediate Compound A160-4

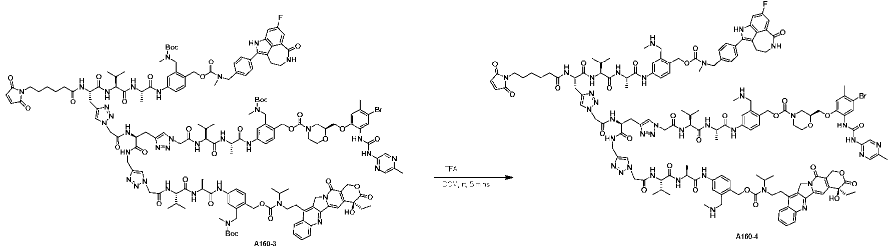

Compound **A160-3** (1500 mg, 0.50 mmol, 1.0 eq) was dissolved in DCM (27 mL). Trifluoroacetic acid (9 mL) was added to the reaction mixture, which was then stirred at room temperature for 5 minutes. After LC-MS monitoring indicated completion, the organic phase was concentrated to give the crude product compound **A160-4,** which was used directly in the next step.

LCMS: [M+Na]⁺ = 1529.4 (half peak)

### Synthesis of Target Compound A160

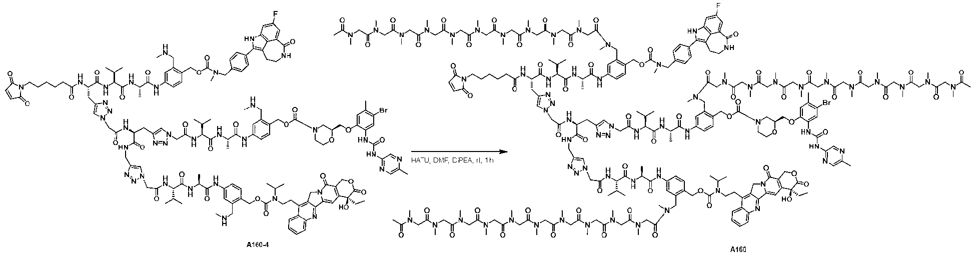

The compound **A160-4** from the previous step was dissolved in DMF (30 mL). Ac-PSAR10-OH (1157 mg, 1.50 mmol, 3.0 eq) and N,N-Diisopropylethylamine (1935 mg, 15.0 mmol, 30.0 eq) were added to the reaction mixture. Finally, under a nitrogen atmosphere, O-(7-Azabenzotriazol-1-yl)-N,N,N',N'-tetramethyluronium hexafluorophosphate (627 mg, 1.65 mmol, 3.3 eq) was added. After the addition was complete, the reaction mixture was stirred at room temperature under a nitrogen atmosphere for 1 hour. After LC-MS monitoring indicated completion, ethyl acetate (300 mL) was added to the reaction mixture. The resulting suspension was filtered, and the filter cake was dissolved in DMF and purified by prep-HPLC (mobile phase: 0.05% HCOOH/CH₃CN/H₂O) to afford compound **A160** (500 mg, 41%) as a yellow solid.

LCMS: [M+H]⁺ = 1742.5 (one-third peak)

### (56) Synthesis of Compound A156

### Synthetic Route:

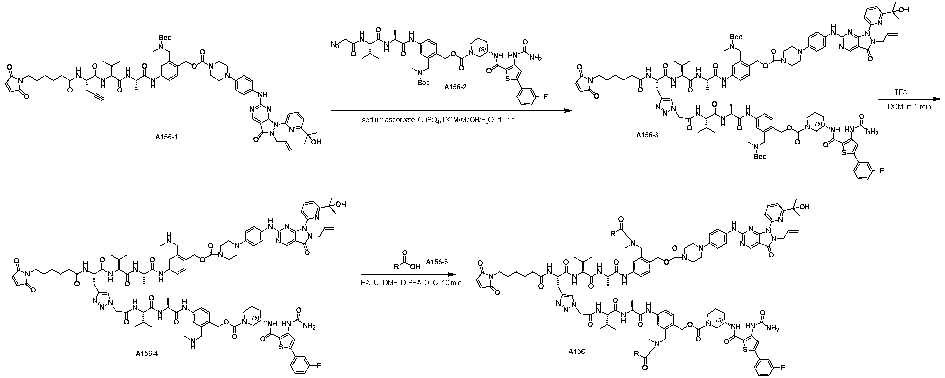

### Synthetic Steps:

### Synthesis of Intermediate Compound A156-3

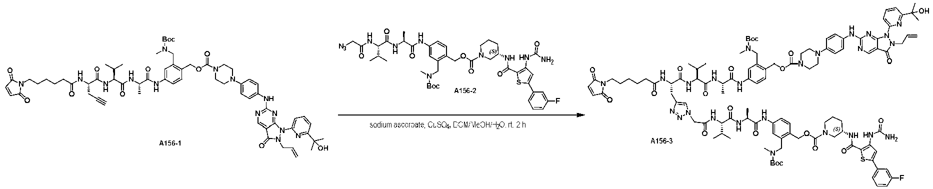

Compound **A156-1** (200 mg, 0.162 mmol, 1.0 eq) and compound **A156-2** (147 mg, 0.162 mmol, 1.0 eq) were dissolved in MeOH/DCM/water (5/5/1 mL). Then 100 uL of an aqueous solution of copper(II) sulfate pentahydrate (8.08 mg, 0.0323 mmol, 0.2 eq) and 100 uL of an aqueous solution of sodium ascorbate (12.8 mg, 0.0646 mmol, 0.4 eq) were added. After the addition was complete, the reaction mixture was stirred at room temperature under a nitrogen atmosphere for 2 hours. After LC-MS monitoring indicated completion, the reaction mixture was concentrated directly. Tetrahydrofuran was added, and the mixture was concentrated again to dryness, affording compound **A156-3** (340 mg, 98%) as a yellow solid.

LCMS: [M+H]⁺ = 1073.3 (half peak)

### Synthesis of Intermediate Compound A156-4

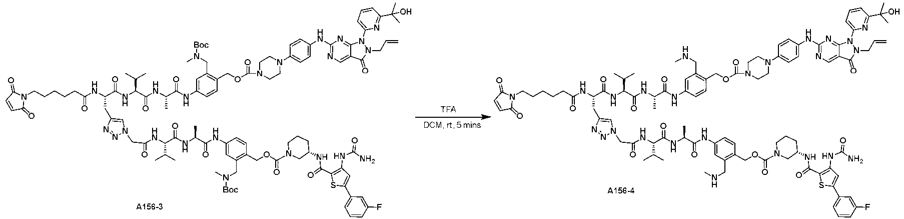

Compound **A156-3** (330 mg, 0.154 mmol, 1.0 eq) was dissolved in DCM (6 mL). Trifluoroacetic acid (2 mL) was added to the reaction mixture, which was then stirred at room temperature for 5 minutes. After LC-MS monitoring indicated completion, the organic phase was concentrated to give the crude product compound **A156-4,** which was used directly in the next step.

LCMS: [M+H]⁺ = 973.3 (half peak)

### Synthesis of Target Compound A156

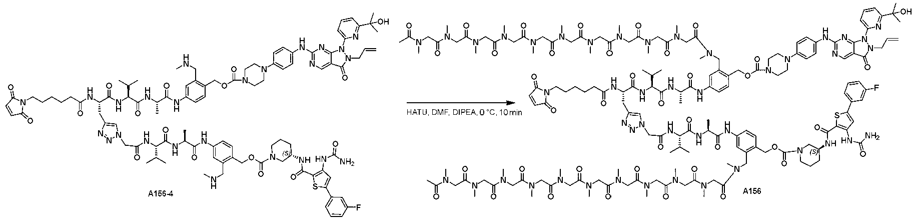

The compound **A156-4** from the previous step was dissolved in DMF (5 mL). **Ac-PSAR10-OH** (237 mg, 0.307 mmol, 2.0 eq) was added to the reaction mixture. Finally, under a nitrogen atmosphere, N,N-Diisopropylethylamine (397 mg, 3.074 mmol, 20.0 eq) and O-(7-Azabenzotriazol-1-yl)-N,N,N',N'-tetramethyluronium hexafluorophosphate (123 mg, 0.323 mmol, 2.1 eq) were added. After the addition was complete, the reaction mixture was stirred at 0 °C under a nitrogen atmosphere for 10 minutes. After LC-MS monitoring indicated completion, ethyl acetate (45 mL) was added to the reaction mixture. The mixture was filtered, and the filter cake was dissolved in DMF and purified by prep-HPLC (mobile phase: 0.05% HCOOH/CH₃CN/H₂O) to afford compound **A156** (212.7 mg, 40%) as a yellow solid.

LCMS: [M+H]⁺ = 1726.2 (half peak)

### (57) Synthesis of Compound A161

### Synthetic Route:

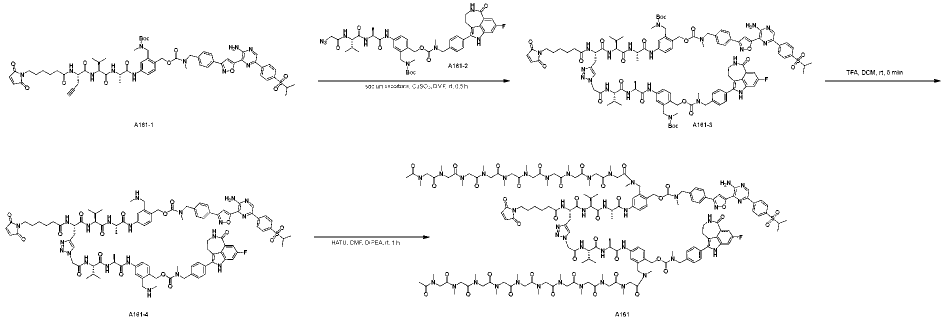

### Synthetic Steps:

### Synthesis of Intermediate Compound A161-3

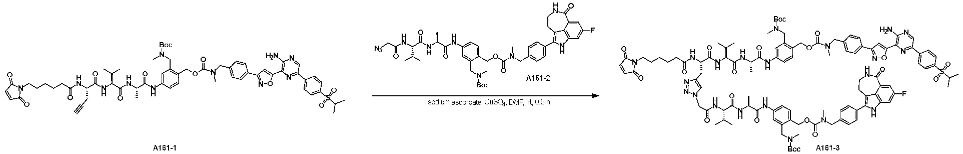

Compound A161-1 (200 mg, 0.16 mmol, 1.0 eq) and compound A161-2 (150 mg, 0.17 mmol, 1.05 eq) were dissolved in N,N-dimethylformamide (5 mL). At room temperature, 0.1 mL each of aqueous solutions of copper(II) sulfate pentahydrate (8.23 mg, 0.03 mmol, 0.2 eq) and sodium ascorbate (13 mg, 0.06 mmol, 0.4 eq) were added, and the mixture was stirred at room temperature for 0.5 h. After the reaction was complete, the reaction mixture was concentrated to dryness to afford compound A161-3 (360 mg, 99%) as a yellow solid.
LCMS: [M/2+Na]⁺ = 1064.0

### Synthesis of Intermediate Compound A161-4

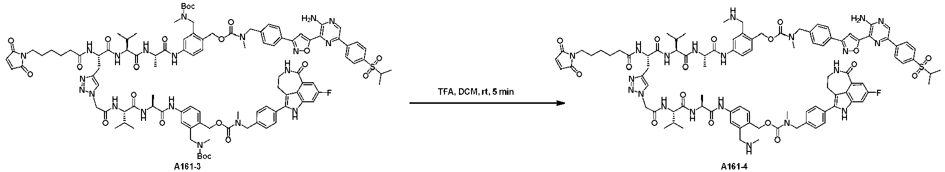

Compound A161-3 (360 mg, 0.17 mmol, 1.0 eq) was dissolved in dichloromethane/trifluoroacetic acid = 3/1 (8 mL). The reaction mixture was stirred at room temperature for 5 minutes. After the reaction was complete, the mixture was concentrated to dryness to give the crude product compound A161-4 as a yellow solid.
LCMS: [M/2+H]⁺ = 941.9

### Synthesis of Target Compound A161

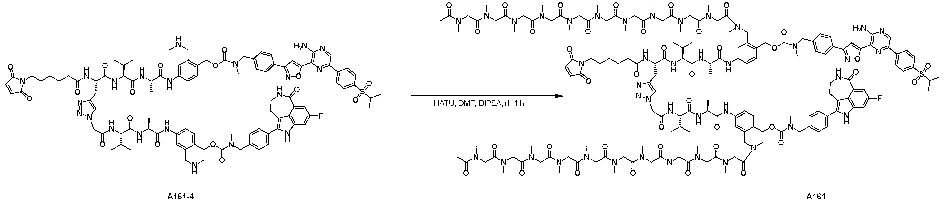

The crude compound A161-4 obtained from the previous step, **Ac-PSAR10-OH** (127 mg, 0.16 mmol, 1.0 eq), and N,N-N,N-Diisopropylethylamine (439 mg, 3.39 mmol, 20 eq) were dissolved in N,N-dimethylformamide (5 mL). At room temperature, O-(7-Azabenzotriazol-1-yl)-N,N,N',N'-tetramethyluronium hexafluorophosphate (142 mg, 0.37 mmol, 2.2 eq) was added, and the reaction mixture was stirred at room temperature for 1 hour. After the reaction was complete, methyl tert-butyl ether (100 mL) was added to the reaction mixture, causing solid precipitation. After filtration and drying, the solid was concentrated. Purification by prep-HPLC (mobile phase: 0.05% HCOOH/CH₃CN/H₂O) afforded compound A161 (100.9 mg, 22%) as a yellow solid.
LCMS: [M/2+H]⁺ = 1694.6

### (58) Synthesis of Compound A162

### Synthetic Route:

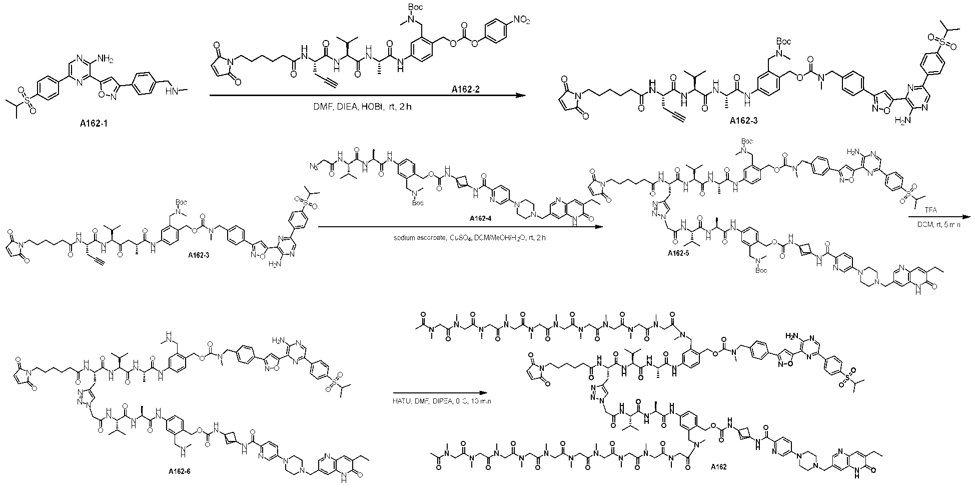

### Synthetic Steps:

### Synthesis of Intermediate Compound A162-3

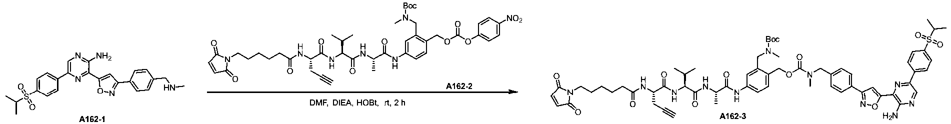

Compound A162-1 (400 mg, 0.863 mmol, 1.0 eq) was dissolved in DMF (5 mL). N,N-Diisopropylethylamine (335 mg, 2.589 mmol, 3.0 eq) was added and stirred until clear. Then compound A162-2 (769 mg, 0.863 mmol, 1.0 eq) and HOBt (117 mg, 0.863 mmol, 1.0 eq) were added. After the addition was complete, the reaction mixture was stirred at room temperature for 2 hours. After LC-MS monitoring indicated completion, the reaction mixture was diluted with ethyl acetate, washed with water and saturated brine. The organic phase was dried over anhydrous sodium sulfate, filtered, and concentrated. The residue was purified by silica gel column chromatography (DCM/MeOH=93/7) to afford compound A162-3 (400 mg, 38%) as a yellow solid.
LCMS: [M+Na]⁺ = 1236.4

### Synthesis of Intermediate Compound A162-5

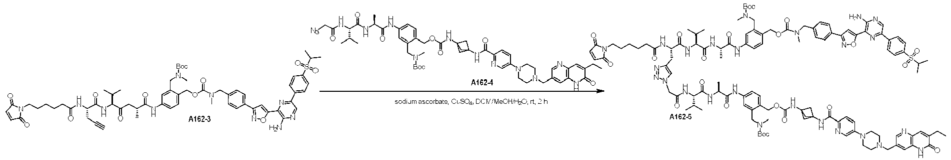

Compound A162-3 (200 mg, 0.165 mmol, 1.0 eq) and compound A162-4 (168 mg, 0.165 mmol, 1.0 eq) were dissolved in MeOH/DCM/water (5/5/1 mL). Then copper(II) sulfate pentahydrate (8.3 mg, 0.0329 mmol, 0.2 eq, in 100 uL water) and sodium ascorbate (13.1 mg, 0.0658 mmol, 0.4 eq, in 100 uL water) were added. After the addition was complete, the reaction mixture was stirred at room temperature under a nitrogen atmosphere for 2 hours. After LC-MS monitoring indicated completion, the reaction mixture was concentrated directly. Tetrahydrofuran was added, and the mixture was concentrated again to dryness, affording compound A162-5 (350 mg, 95%) as a yellow solid.
LCMS: [M+H]⁺ = 1117.4 (half peak)

### Synthesis of Intermediate Compound A162-6

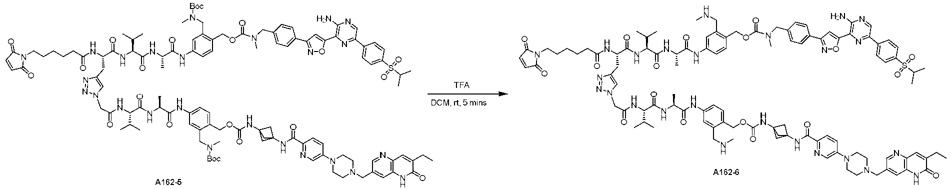

Compound A162-5 (340 mg, 0.152 mmol, 1.0 eq) was dissolved in DCM (3 mL). Trifluoroacetic acid (1 mL) was added to the reaction mixture, which was then stirred at room temperature for 5 minutes. After LC-MS monitoring indicated completion, the organic phase was concentrated to give the crude product compound A162-6, which was used directly in the next step.
LCMS: [M+H]⁺ = 1017.2 (half peak)

### Synthesis of Target Compound A162

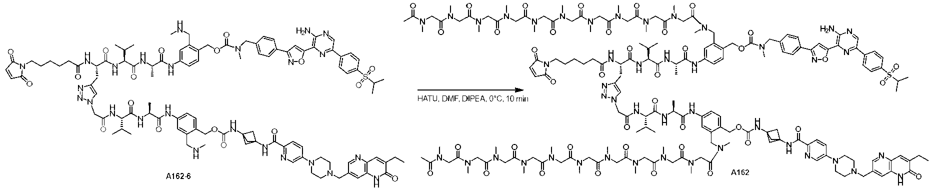

The compound A162-6 from the previous step was dissolved in DMF (5 mL). **Ac-PSAR10-OH** (235 mg, 0.304 mmol, 2.0 eq) was added to the reaction mixture. Finally, under a nitrogen atmosphere, N,N-Diisopropylethylamine (395 mg, 3.042 mmol, 20.0 eq) and O-(7-Azabenzotriazol-1-yl)-N,N,N',N'-tetramethyluronium hexafluorophosphate (121 mg, 0.319 mmol, 2.1 eq) were added. The reaction mixture was stirred in an ice bath under a nitrogen atmosphere for 10 minutes. After LC-MS monitoring indicated completion, ethyl acetate (60 mL) was added to the reaction mixture. The resulting suspension was filtered, and the filter cake was dissolved in DMF and purified by prep-HPLC (mobile phase: 0.05% HCOOH/CH₃CN/H₂O) to afford compound A162 (152.5 mg, 28%) as a yellow solid.
LCMS: [M+H]⁺ = 1769.7 (half peak)

### (59) Synthesis of Compound A163

### Synthetic Route:

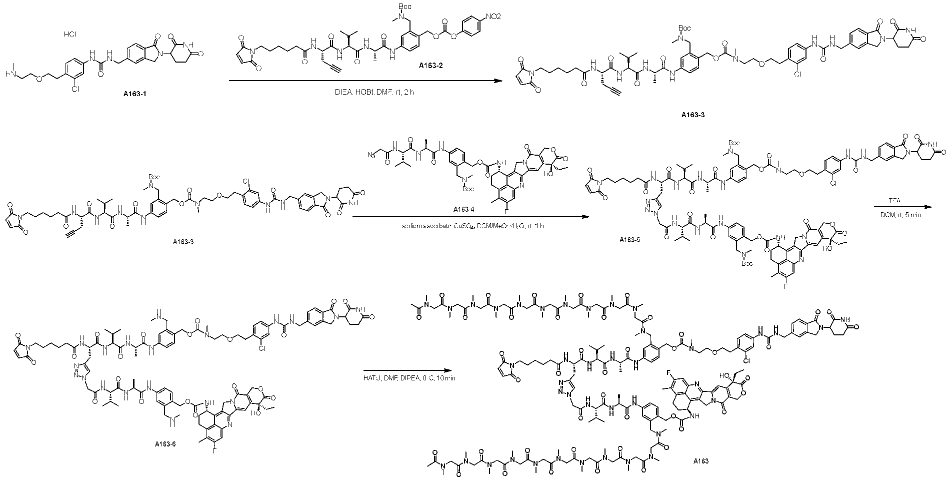

### Synthetic Steps:

### Synthesis of Intermediate Compound A163-3

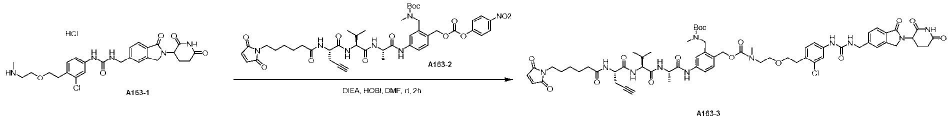

Compound A163-1 (360 mg, 0.638 mmol, 1.0 eq) was dissolved in DMF (5 mL). N,N-Diisopropylethylamine (248 mg, 1.913 mmol, 3.0 eq) was added and stirred until clear. Then compound A163-2 (568 mg, 0.638 mmol, 1.0 eq) and HOBt (43 mg, 0.319 mmol, 0.5 eq) were added. After the addition was complete, the reaction mixture was stirred at room temperature for 2 hours. After LC-MS monitoring indicated completion, the reaction mixture was diluted with ethyl acetate, washed with water and saturated brine. The organic phase was dried over anhydrous sodium sulfate, filtered, and concentrated. The residue was purified by silica gel column chromatography (DCM/MeOH=93/7) to afford compound A163-3 (470 mg, 58%) as a yellow solid.
LCMS: [M+Na]⁺ = 1300.3

### Synthesis of Intermediate Compound A163-5

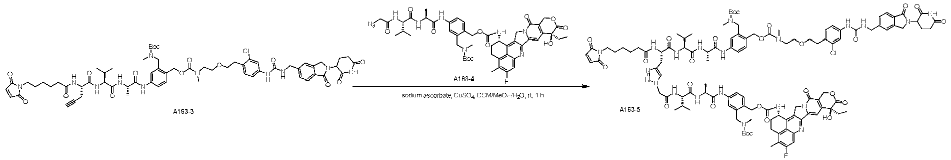

Compound A163-3 (140 mg, 0.109 mmol, 1.0 eq) and compound A163-4 (107 mg, 0.109 mmol, 1.0 eq) were dissolved in MeOH/DCM (5/5 mL). Then copper(II) sulfate pentahydrate (5.5 mg, 0.0219 mmol, 0.2 eq, in 100 uL water) and sodium ascorbate (8.7 mg, 0.0434 mmol, 0.4 eq, in 100 uL water) were added. After the addition was complete, the reaction mixture was stirred at room temperature under a nitrogen atmosphere for 2 hours. After LC-MS monitoring indicated completion, the reaction mixture was concentrated directly. Tetrahydrofuran was added, and the mixture was concentrated again to dryness, affording compound A163-5 (240 mg, 97%) as a yellow solid.
LCMS: [M+H]⁺ = 1130.6 (half peak)

### Synthesis of Intermediate Compound A163-6

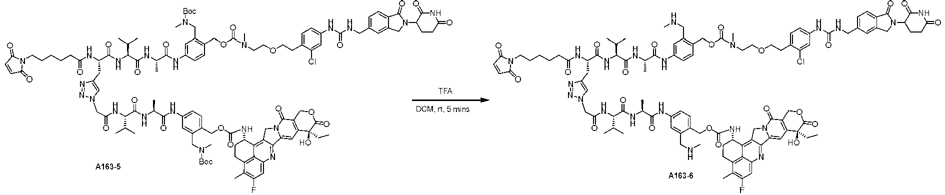

Compound A163-5 (230 mg, 0.102 mmol, 1.0 eq) was dissolved in DCM (3 mL). Trifluoroacetic acid (1 mL) was added to the reaction mixture, which was then stirred at room temperature for 5 minutes. After LC-MS monitoring indicated completion, the organic phase was concentrated to give the crude product compound A163-6, which was used directly in the next step.
LCMS: [M+H]⁺ = 1030.5 (half peak)

### Synthesis of Target Compound A163

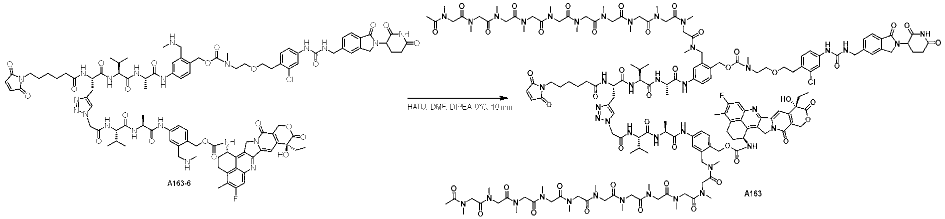

The compound A163-6 from the previous step was dissolved in DMF (3 mL). Compound A163-7 (157 mg, 0.203 mmol, 2.0 eq) was added to the reaction mixture. Finally, under a nitrogen atmosphere, N,N-Diisopropylethylamine (263 mg, 2.034 mmol, 20.0 eq) and O-(7-Azabenzotriazol-1-yl)-N,N,N',N'-tetramethyluronium hexafluorophosphate (82 mg, 0.214 mmol, 2.1 eq) were added. The reaction mixture was stirred in an ice bath under a nitrogen atmosphere for 10 minutes. After LC-MS monitoring indicated completion, ethyl acetate (60 mL) was added to the reaction mixture. The resulting suspension was filtered, and the filter cake was dissolved in DMF and purified by prep-HPLC (mobile phase: 0.05% HCOOH/CH₃CN/H₂O) to afford compound A163 (42.1 mg, 12%) as a white solid.
LCMS: [M+H]⁺ = 1783.7 (half peak)

### (60) Synthesis of Compound A138

### Synthetic Route:

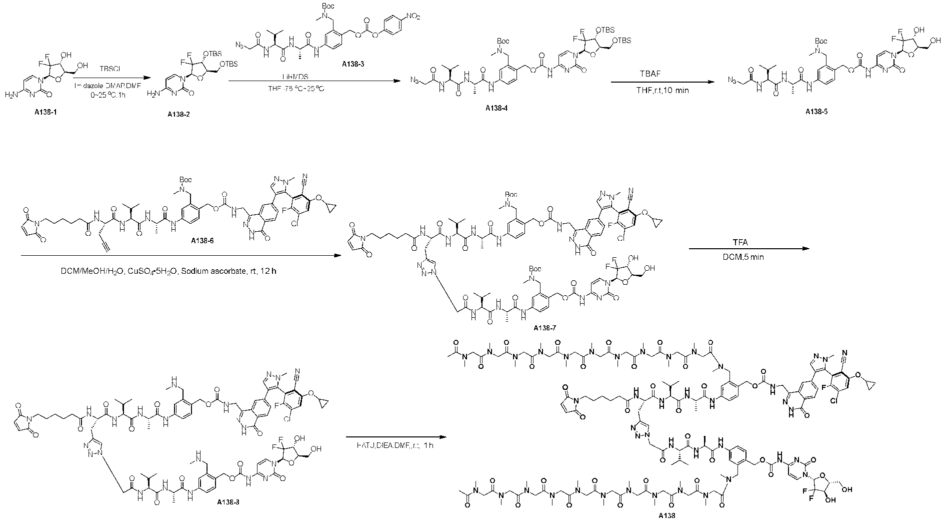

### Synthetic Steps:

### Synthesis of Intermediate Compound A138-2

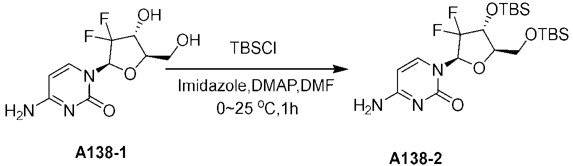

CompoundA138-1 (500 mg, 1.90 mmol, 1 eq) was dissolved in DMF (8.0 mL). Imidazole (776 mg, 11.40 mmol, 6 eq) and 4-dimethylaminopyridine (46 mg, 0.38 mmol, 0.2 eq) were added. Under an ice bath, tert-butyldimethylchlorosilane (1431 mg, 11.40 mmol, 6 eq) was added to the stirred solution. Subsequently, the mixture was stirred at room temperature for 1 hour. After LC-MS monitoring indicated completion, water (50 mL) was added to the reaction mixture. The mixture was extracted with ethyl acetate. The organic phase was washed with water and then with saturated brine, dried over anhydrous sodium sulfate, filtered, and concentrated. The residue was purified by silica gel column chromatography (EA/PE = 0-100%) to afford compound A138-2 (700 mg, 75%) as a white solid.
LCMS: [M+H]⁺ = 492.2

### Synthesis of Intermediate Compound A138-4

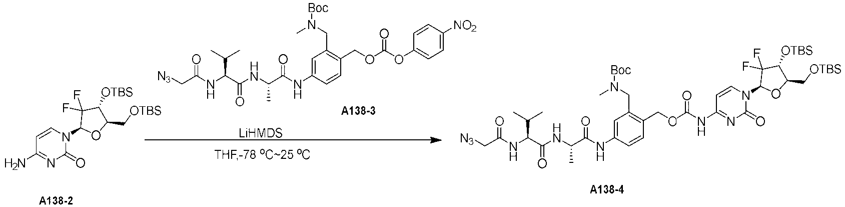

Compound A138-2 (550 mg, 0.80 mmol, 1.5 eq) was dissolved in tetrahydrofuran (40 mL). At -78 °C, LiHMDS (1.2 mL, 1.20 mmol, 1M in THF solution, 1.5 eq) was slowly added dropwise, and the mixture was gradually warmed to room temperature. At this point, a solution of compound A138-3 (550 mg, 0.80 mmol, 1.0 eq) in THF (30 mL) was slowly added dropwise to the reaction solution. The mixture was stirred at room temperature for 0.5 h. After LC-MS monitoring indicated completion, the reaction was quenched with saturated ammonium chloride aqueous solution in an ice bath. Water (70 mL) was added to the reaction mixture. The mixture was extracted with ethyl acetate. The organic phase was washed with water and then with saturated brine, dried over anhydrous sodium sulfate, filtered, and concentrated. The residue was purified by silica gel column chromatography (DCM/MeOH = 0-3%) to afford compound A138-4 (500 mg, 60%) as a yellow solid.
LCMS: [M+Na]⁺ = 1059.3

### Synthesis of Intermediate Compound A138-5

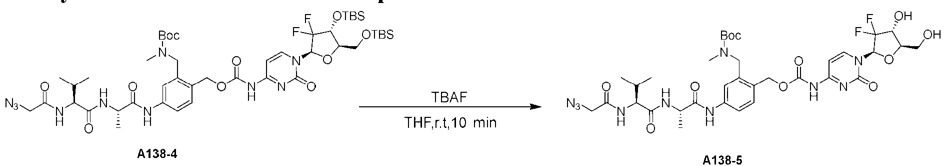

CompoundA138-4 (500 mg, 0.48 mmol, 1.0 eq) was dissolved in tetrahydrofuran (25 mL). Tetrabutylammonium fluoride solution (0.96 mL, 0.96 mmol, 2 eq, 1N in THF) was added dropwise, and the mixture was stirred at room temperature for 10 minutes. The reaction mixture was concentrated under reduced pressure to give a yellow residue. Purification by reverse-phase column chromatography (mobile phase: 0.05% HCOOH/CH₃CN/H₂O = 0-40%) followed by lyophilization afforded compound A138-5 (360 mg, 92%) as a white solid.
LCMS: [M+H]⁺ = 831.2

### Synthesis of Intermediate Compound A138-7

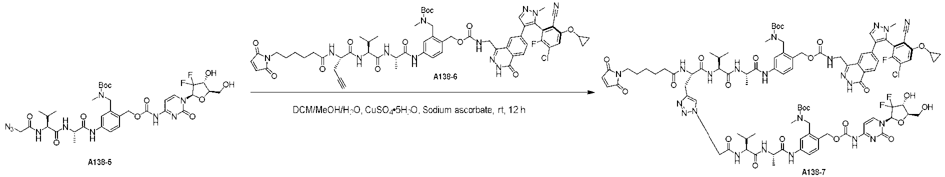

Compound A138-5 (70 mg, 0.0864 mmol, 1 eq) and compound A138-6 (105 mg, 0.0864 mmol, 1 eq) were dissolved in dichloromethane/methanol/water (3/2/1 mL). Under a nitrogen atmosphere, copper(II) sulfate pentahydrate (2.16 mg, 0.0086 mmol, 0.1 eq, in 100 uL water) and sodium ascorbate (3.42 mg, 0.0172 mmol, 0.25 eq, in 100 uL water) were added. After the addition was complete, the reaction mixture was stirred at room temperature under a nitrogen atmosphere for 12 hours. After LC-MS monitoring indicated completion, concentration under reduced pressure afforded the crude product compound A138-7 (170 mg, 38%) as a yellow solid.
LCMS: [1/2M+Na]⁺ = 1034.8 (half peak)

### Synthesis of Intermediate Compound A138-8

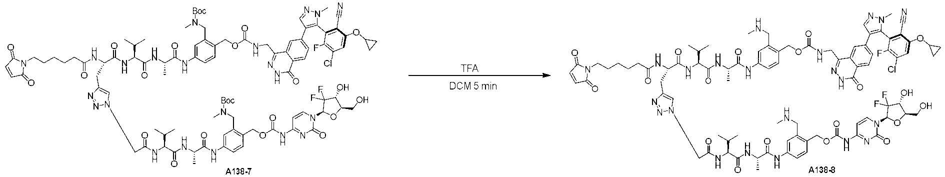

Compound A138-7 (170 mg, 0.0839 mmol, 1 eq) was dissolved in dichloromethane (2.0 mL). Trifluoroacetic acid (0.6 mL) was slowly added dropwise, and the reaction mixture was stirred at room temperature under a nitrogen atmosphere for 5 minutes. After LC-MS monitoring indicated completion, concentration under reduced pressure afforded the crude product compound A138-8 (150 mg) as a brown solid.
LCMS: [M+H]⁺ = 912.8 (half peak)

### Synthesis of Target Compound A138

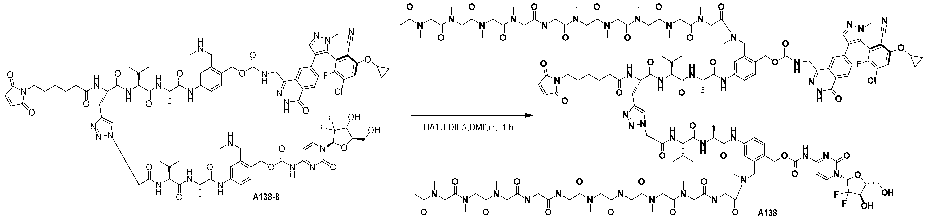

Compound A138-8 (150 mg, 0.08 mmol, 1.0 eq) was dissolved in DMF (2 mL). **Ac-PSAR10-OH** (126 mg, 0.16 mmol, 2.0 eq) and N,N-Diisopropylethylamine (212 mg, 1.66 mmol, 20.0 eq) were added to the reaction mixture. Finally, under a nitrogen atmosphere, O-(7-Azabenzotriazol-1-yl)-N,N,N',N'-tetramethyluronium hexafluorophosphate (66 mg, 0.17 mmol, 2.1 eq) was added. After the addition was complete, the reaction mixture was stirred at room temperature under a nitrogen atmosphere for 1 hour. After LC-MS monitoring indicated completion, the reaction mixture was filtered. The filtrate was purified by prep-HPLC (mobile phase: 0.05% HCOOH/CH₃CN/H₂O) to afford compound A138 (34.3 mg, 22%) as a white solid. LCMS: [M+H]⁺ = 1665.4 (half peak)

### (61) Synthesis of Compound A139

### Synthetic Route:

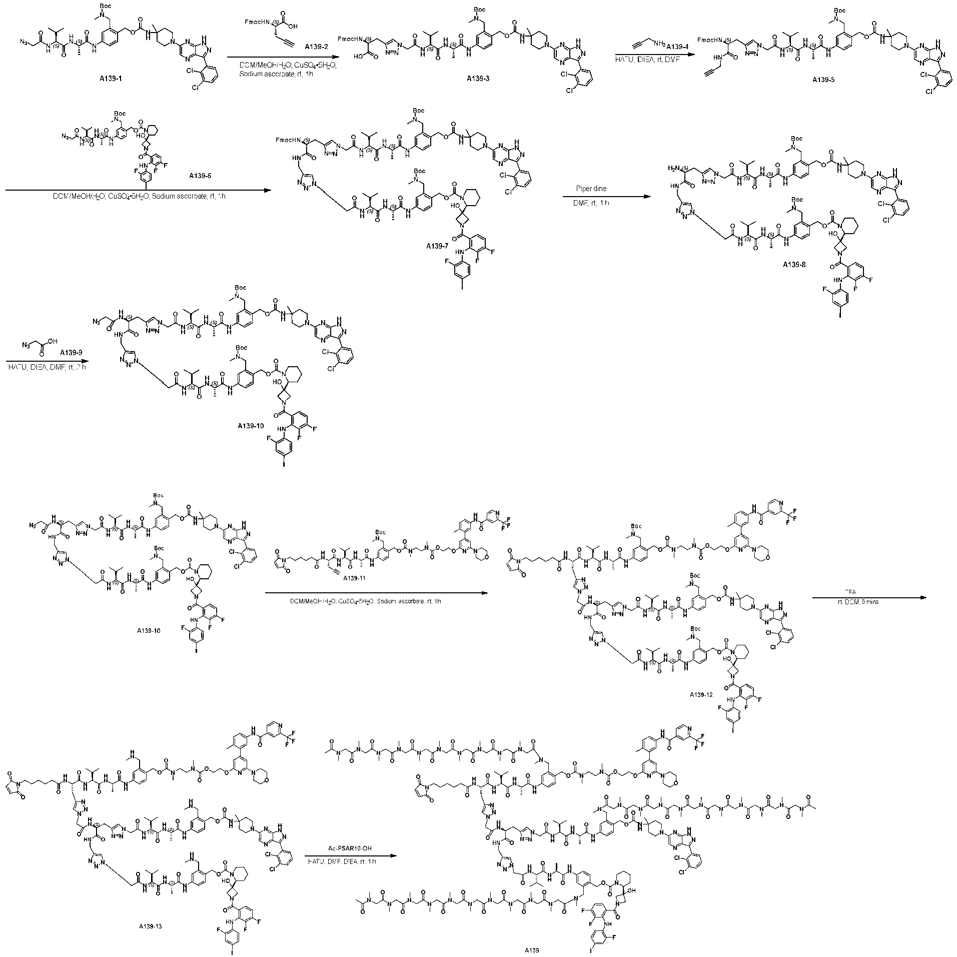

### Synthetic Steps:

### Synthesis of Intermediate Compound A139-3

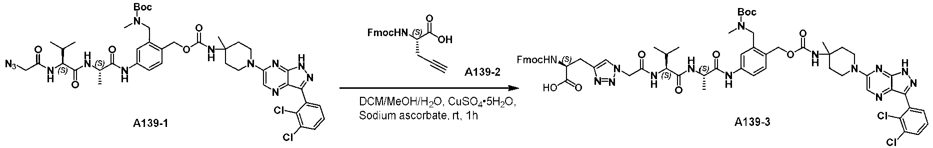

Compound A139-1 (180 mg, 0.195 mmol, 1.0 eq) and A139-2 (65 mg, 0.195 mmol, 1.0 eq) were dissolved in dichloromethane/methanol/water (4/4/1 mL). Under a nitrogen atmosphere, copper(II) sulfate pentahydrate (4.8 mg, 0.019 mmol, 0.1 eq, in 100 uL water) and sodium ascorbate (7.7 mg, 0.039 mmol, 0.2 eq, in 100 uL water) were added. After the addition was complete, the reaction mixture was stirred at room temperature under a nitrogen atmosphere for 1 hour. After LC-MS monitoring indicated completion, water (30 mL) was added to the reaction mixture. The mixture was extracted with dichloromethane/methanol (10/1; V/V). The organic phase was washed with saturated brine, dried over anhydrous sodium sulfate, filtered, and concentrated to afford compound A139-3 (240 mg, 98%) as a grey solid.
LCMS: [M+H]⁺ = 1257.2

### Synthesis of Intermediate Compound A139-5

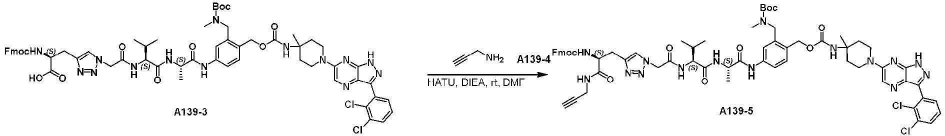

Compound A139-3 (200 mg, 0.159 mmol, 1.0 eq) was dissolved in DMF (3 mL). Compound A139-4 (26 mg, 0.476 mmol, 3.0 eq) and N,N-Diisopropylethylamine (61.5 mg, 0.476 mmol, 3.0 eq) were added to the reaction mixture. Finally, under a nitrogen atmosphere, O-(7-Azabenzotriazol-1-yl)-N,N,N',N'-tetramethyluronium hexafluorophosphate (90.6 mg, 0.238 mmol, 1.5 eq) was added. After the addition was complete, the reaction mixture was stirred at room temperature under a nitrogen atmosphere for 1 hour. After LC-MS monitoring indicated completion, ethyl acetate and water (30 mL) were added to the reaction mixture. The mixture was extracted with ethyl acetate. The organic phase was washed with water and then with saturated brine, dried over anhydrous sodium sulfate, filtered, and concentrated. The residue was slurried with MTBE, filtered, and the filter cake was collected and dried to afford compound A139-5 (160 mg, 72%) as a grey solid.
LCMS: [M+H]⁺ = 1294.2

### Synthesis of Intermediate Compound A139-7

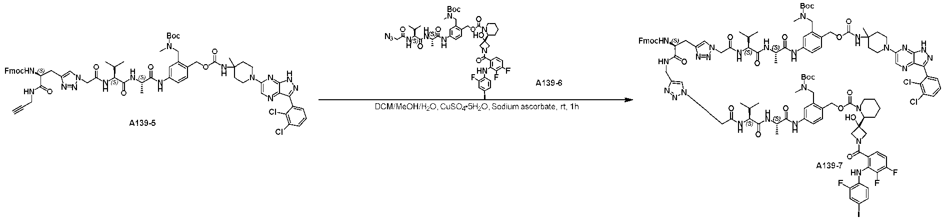

Compound A139-5 (132 mg, 0.102 mmol, 1.0 eq) and compound A139-6 (110 mg, 0.102 mmol, 1.0 eq) were dissolved in dichloromethane/methanol/water (8/8/2 mL). Under a nitrogen atmosphere, copper(II) sulfate pentahydrate (2.6 mg, 0.01 mmol, 0.1 eq, in 100 uL water) and sodium ascorbate (4.71 mg, 0.02 mmol, 0.2 eq, in 100 uL water) were added. After the addition was complete, the reaction mixture was stirred at room temperature under a nitrogen atmosphere for 1 hour. After LC-MS monitoring indicated completion, the reaction mixture was concentrated directly. The residue was slurried with dichloromethane/methyl tert-butyl ether (1:1, 10 mL), filtered, and the filter cake was collected and dried to afford compound A139-7 (170 mg, 70%) as a grey solid.
LCMS: [M+H]⁺ = 1186.1 (half peak)

### Synthesis of Intermediate Compound A139-8

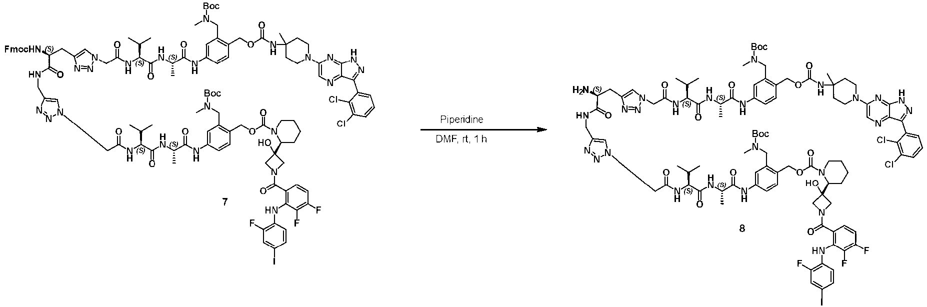

Compound A139-7 (170 mg, 0.072 mmol, 1.0 eq) was dissolved in DMF (3 mL). The reaction mixture was cooled to 0 °C, and under a nitrogen atmosphere, piperidine (0.6 mL) was added. The reaction mixture was stirred at room temperature under a nitrogen atmosphere for half an hour. After LC-MS monitoring indicated completion, methyl tert-butyl ether (50 mL) was added to the reaction mixture. The suspension was filtered directly, washed with methyl tert-butyl ether, and the filter cake was dried directly to afford compound A139-8 (110 mg, 71%) as a yellow solid.
LCMS: [M+H]⁺ = 1075.3 (half peak)

### Synthesis of Intermediate Compound A139-10

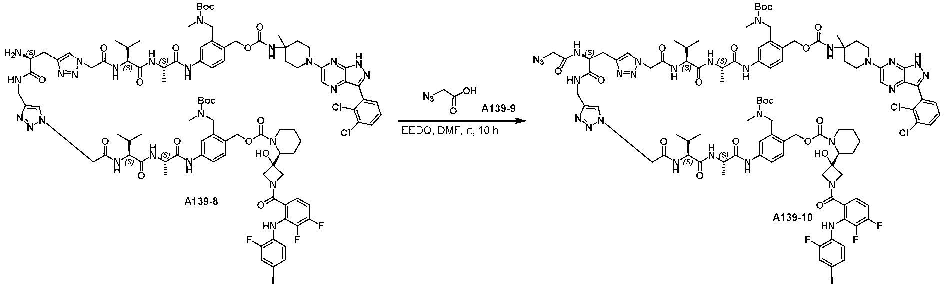

Compound A139-8 (100 mg, 0.047 mmol, 1.0 eq) was dissolved in DMF (3 mL). Compound A139-9 (7.1 mg, 0.070 mmol, 1.5 eq) was added to the reaction mixture. Finally, under a nitrogen atmosphere, EEDQ (34.5 mg, 0.139 mmol, 3.0 eq) was added. After the addition was complete, the reaction mixture was stirred at room temperature under a nitrogen atmosphere for 10 hours. After LC-MS monitoring indicated completion, methyl tert-butyl ether (50 mL) was added to the reaction mixture. The suspension was filtered directly, washed with methyl tert-butyl ether, and the filter cake was dried directly to afford compound A139-10 (80 mg, 77%) as a yellow solid.
LCMS: [M+H]⁺ = 1116.1 (half peak)

### Synthesis of Intermediate Compound A139-12

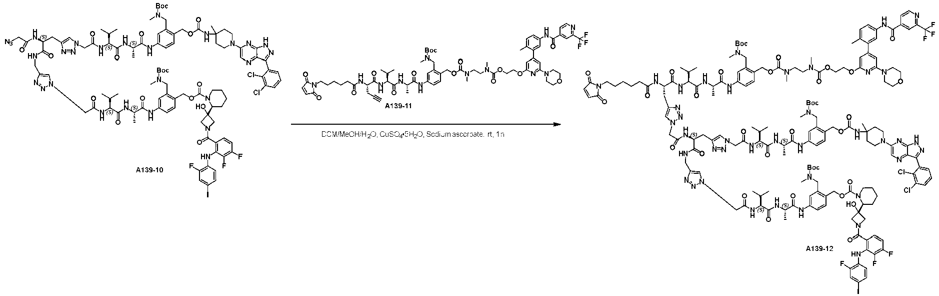

Compound A139-10 (60 mg, 0.027 mmol, 1.0 eq) and compound A139-11 (37 mg, 0.027 mmol, 1.0 eq) were dissolved in DMF (4 mL). Under a nitrogen atmosphere, copper(II) sulfate pentahydrate (0.7 mg, 0.0027 mmol, 0.1 eq, in 100 uL water) and sodium ascorbate (1.1 mg, 0.005 mmol, 0.2 eq, in 100 uL water) were added. After the addition was complete, the reaction mixture was stirred at room temperature under a nitrogen atmosphere for 16 hours. After LC-MS monitoring indicated completion, ethyl acetate (60 mL) was added to the reaction mixture. The suspension was filtered directly, washed with ethyl acetate, and the filter cake was dried directly to afford compound A139-12 (41 mg, 43%) as a yellow solid.
LCMS: [M+H]⁺ = 1200.7 (one-third peak)

### Synthesis of Intermediate Compound A139-13

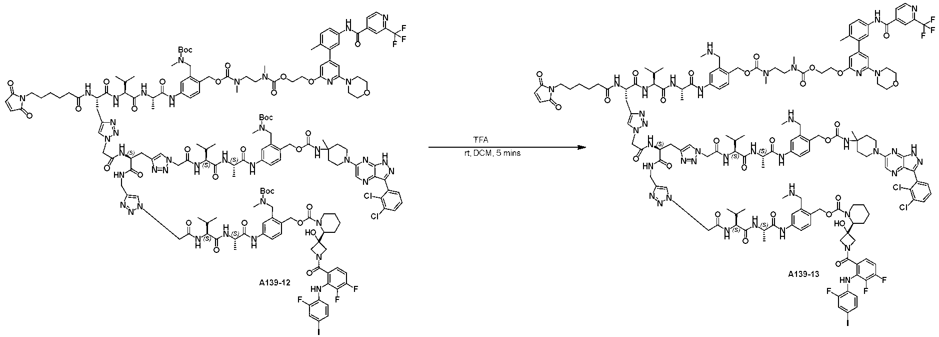

Compound A139-12 (40 mg, 0.011 mmol, 1.0 eq) was dissolved in DCM (3 mL). Trifluoroacetic acid (1 mL) was added to the reaction mixture, which was then stirred at room temperature for 5 minutes. After LC-MS monitoring indicated completion, the organic phase was concentrated to give the crude product compound A139-13, which was used directly in the next step.
LCMS: [M+H]⁺ = 1650.0 (half peak)

### Synthesis of Compound A139

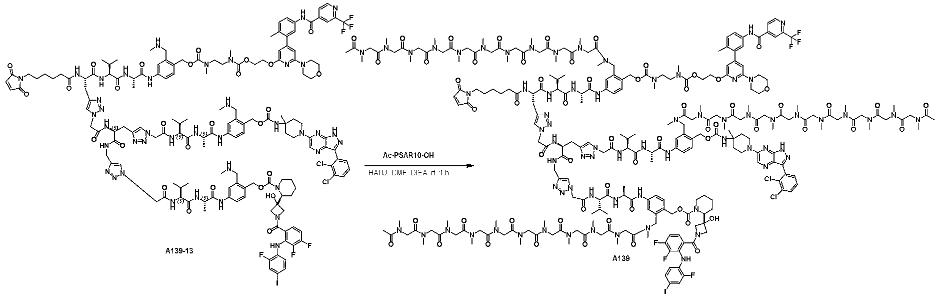

The compound A139-13 from the previous step was dissolved in DMF (3 mL). **Ac-PSAR10-OH** (28 mg, 0.037 mmol, 3.3 eq) and N,N-Diisopropylethylamine (29 mg, 0.22 mmol, 20.0 eq) were added to the reaction mixture. Finally, under a nitrogen atmosphere, O-(7-Azabenzotriazol-1-yl)-N,N,N',N'-tetramethyluronium hexafluorophosphate (14 mg, 0.037 mmol, 3.3 eq) was added. After the addition was complete, the reaction mixture was stirred at room temperature under a nitrogen atmosphere for 1 hour. After LC-MS monitoring indicated completion, ethyl acetate (50 mL) was added to the reaction mixture. The resulting suspension was filtered, and the filter cake was dissolved in DMF and purified by prep-HPLC (mobile phase: 0.05% HCOOH/CH₃CN/H₂O) to afford compound A139 (4.1 mg, 6%) as a white solid. LCMS: [M+H]⁺ = 1853.4 (one-third peak)

### (62) Synthesis of Compound A140

### Synthetic Route:

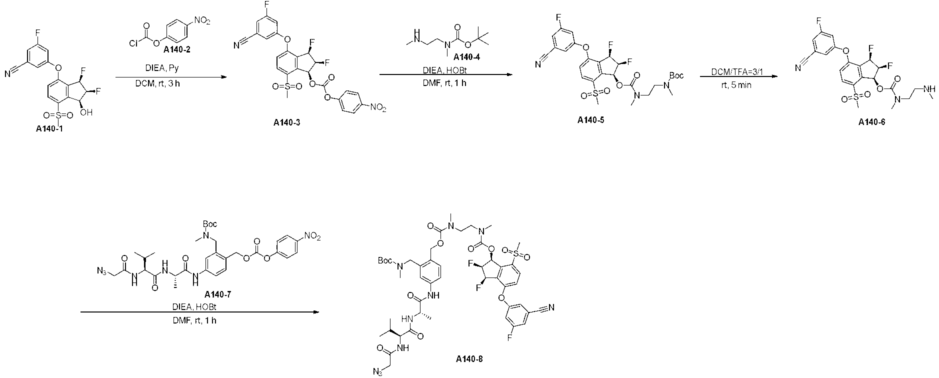

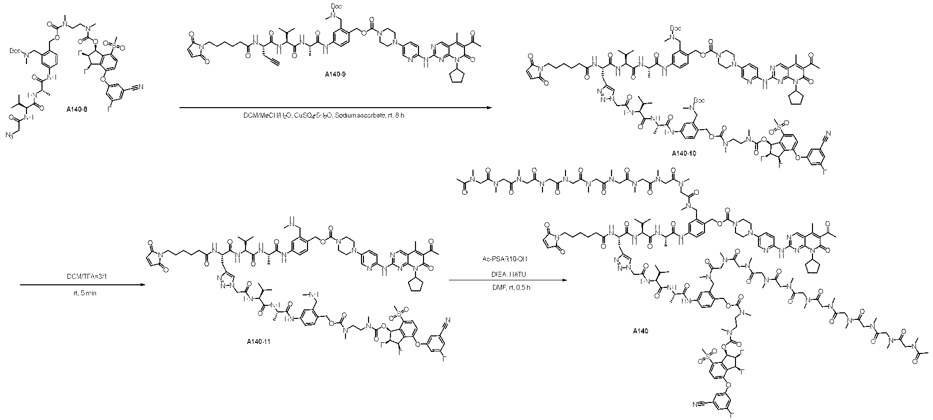

### Synthetic Steps:

### Synthesis of Intermediate Compound A140-3

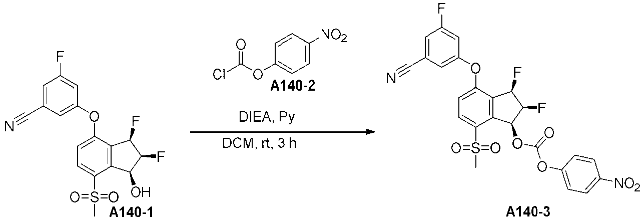

Compound A140-1 (300 mg, 0.783 mmol, 1.0 eq) was dissolved in DCM (15 mL). DIEA (303 mg, 2.348 mmol, 3.0 eq) was added and stirred for 2 minutes. Then compound A140-2 (631 mg, 3.130 mmol, 4.0 eq) and 20 drops of pyridine were added. After the addition was complete, the reaction mixture was stirred at room temperature for 3 hours. After LC-MS monitoring indicated completion, the reaction mixture was diluted with ethyl acetate. Water (15 mL) was added to the reaction mixture. The organic phase was washed with 10% aqueous citric acid and then with saturated brine. The obtained organic phase was dried over anhydrous sodium sulfate, filtered, and concentrated. The residue was purified by silica gel column chromatography (PE/DCM=100/0-0/100) to afford compound A140-3 (270 mg, 63%) as a yellow solid.
LCMS: [M+H]⁺ = 571.0

### Synthesis of Intermediate Compound A140-5

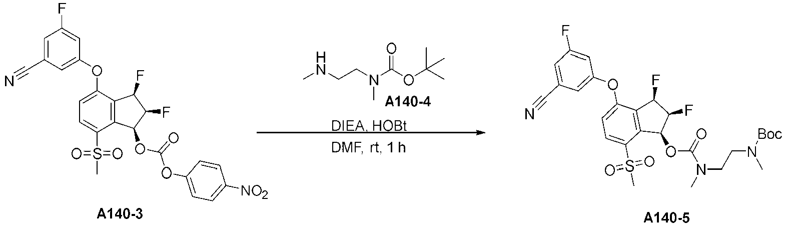

Compound A140-3 (270 mg, 0.492 mmol, 1.0 eq) and N,N-Diisopropylethylamine (191 mg, 1.477 mmol, 3.0 eq) were dissolved in DMF (6 mL). After stirring until clear, compound A140-4 (120 mg, 0.640 mmol, 1.3 eq) and HOBt (33 mg, 0.246 mmol, 0.5 eq) were added. After the addition was complete, the reaction mixture was stirred at room temperature for 1 hour. After LC-MS monitoring indicated completion, ethyl acetate (50 mL) was added to the reaction mixture. The mixture was washed twice with water and then twice with saturated brine. The obtained organic phase was dried over anhydrous sodium sulfate, filtered, and concentrated. The residue was purified by silica gel column chromatography (DCM/MeOH=95/5) to afford compound A140-5 (260 mg, 88%) as a pale yellow solid.
LCMS: [M+Na]⁺ = 620.1

### Synthesis of Intermediate Compound A140-6

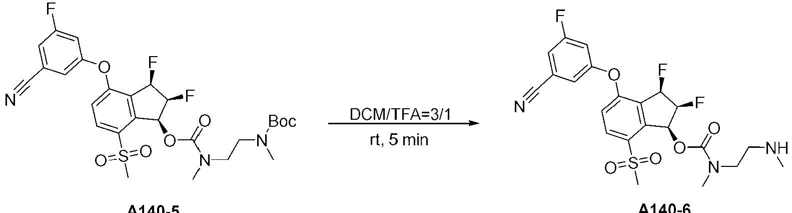

Compound A140-5 (150 mg, 0.25 mmol, 1.0 eq) was dissolved in dichloromethane (3 mL). Trifluoroacetic acid (1 mL) was added to the reaction mixture, which was then stirred at room temperature for 5 min. After LC-MS monitoring indicated completion, the reaction mixture was concentrated to give the crude product compound A140-6, which was used directly in the next step.
LCMS: [M+H]⁺ = 498.1

### Synthesis of Intermediate Compound A140-8

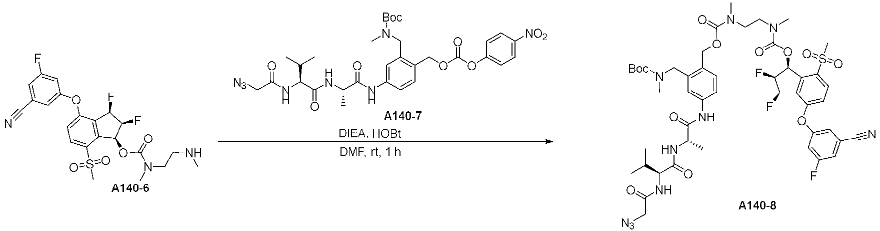

The crude product compound A140-6 from the previous step was dissolved in DMF (2 mL). N,N-Diisopropylethylamine (324 mg, 2.506 mmol, 10.0 eq) was added and stirred for 2 minutes. Then compound A140-7 (155 mg, 0.225 mmol, 0.9 eq) and HOBt (17 mg, 0.125 mmol, 0.5 eq) were added. After the addition was complete, the reaction mixture was stirred at room temperature for 1 hour. After LC-MS monitoring indicated completion, ethyl acetate (40 mL) was added to the reaction mixture. The mixture was washed twice with water and then twice with saturated brine. The obtained organic phase was dried over anhydrous sodium sulfate, filtered, and concentrated. The residue was purified by silica gel column chromatography (DCM/MeOH=94/6) to afford compound A140-8 (230 mg, 88%) as a white solid.
LCMS: [M+Na]⁺ = 1065.3

### Synthesis of Intermediate Compound A140-10

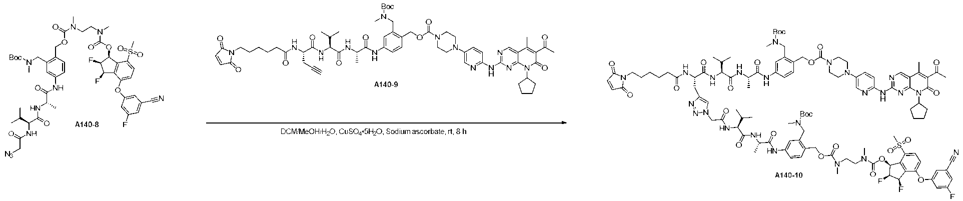

CompoundA140-8 (100 mg, 0.0958 mmol, 1 eq) and compound A140-9 (114.9 mg, 0.0958 mmol, 1 eq) were dissolved in MeOH/DCM/water (5/5/1 mL). Then 100 uL of an aqueous solution of copper(II) sulfate pentahydrate (2.4 mg, 0.0096 mmol, 0.1 eq) and 100 uL of an aqueous solution of sodium ascorbate (7.6 mg, 0.0383 mmol, 0.4 eq) were added. After the addition was complete, the reaction mixture was stirred at room temperature under a nitrogen atmosphere for 8 hours. After LC-MS monitoring indicated completion, the reaction mixture was concentrated directly to afford the crude product compound A140-10 (200 mg), which was used directly in the next step.
LCMS: [M+H]⁺ = 1121.3 (half peak)

### Synthesis of Intermediate Compound A140-11

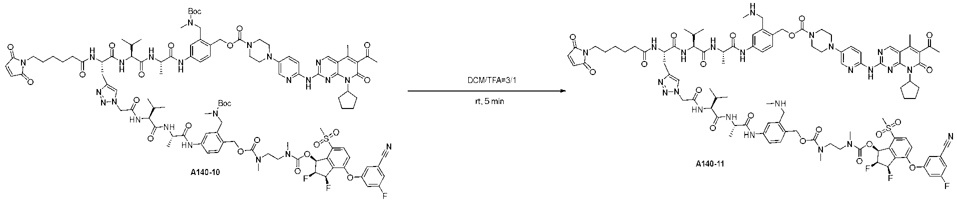

Compound A140-10 (100 mg, 0.045 mmol) was dissolved in DCM (3 mL). Trifluoroacetic acid (1 mL) was added to the reaction mixture, which was then stirred at room temperature for 5 minutes. After LC-MS monitoring indicated completion, the organic phase was concentrated to give the crude product compoundA140-11, which was used directly in the next step.
LCMS: [M+H]⁺ = 1021.1 (half peak)

### Synthesis of Target Compound A140

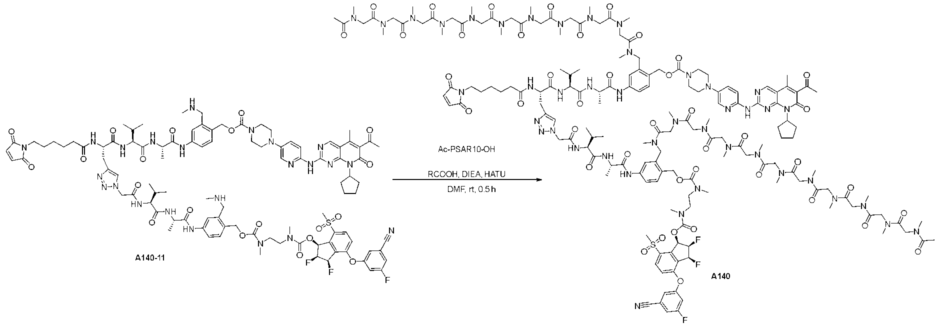

The crude product compound A140-11 from the previous step was dissolved in DMF (2 mL). N,N-Diisopropylethylamine (115 mg, 0.895 mmol, 20.0 eq) was added. Then A140-12 (69 mg, 0.089 mmol, 2.0 eq) and O-(7-Azabenzotriazol-1-yl)-N,N,N',N'-tetramethyluronium hexafluorophosphate (36 mg, 0.094 mmol, 2.1 eq) were added to the reaction mixture. After the addition was complete, the reaction mixture was stirred at room temperature under a nitrogen atmosphere for 0.5 hour. After LC-MS monitoring indicated completion, EA (20 mL) was added to the reaction mixture. The reaction mixture was filtered, and the filter cake was dissolved in DMF and purified by prep-HPLC (mobile phase: 0.05% HCOOH/CH₃CN/H₂O) to afford compound A140 (35.8 mg, 23%) as a yellow solid.
LCMS: [M+H]⁺ = 1773.7 (half peak)

### (63) Synthesis of Compound A141

### Synthetic Route:

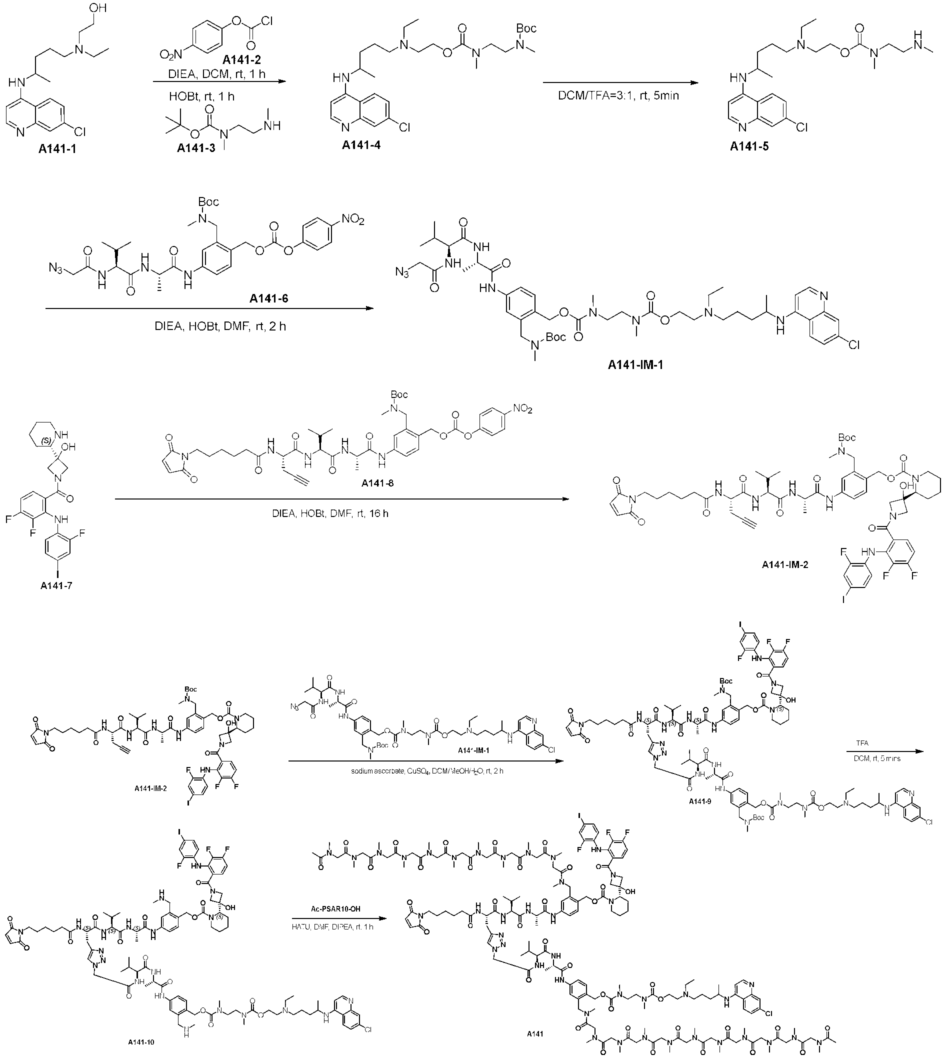

### Synthetic Steps:

### Synthesis of Compound A141-4

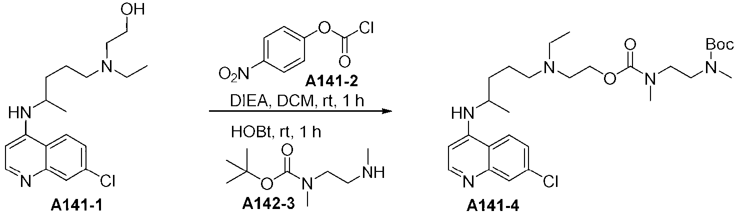

Compound A141-1 (200 mg, 0.596 mmol, 1.0 eq), compound A141-2 (132 mg, 0.655 mmol, 1.1 eq) and N,N-Diisopropylethylamine (230.9 mg, 1.787 mmol, 3.0 eq) were dissolved in DCM (5 mL). The mixture was stirred at room temperature for 1 hour. Then compound A141-3 (168.2 mg, 0.893 mmol, 1.5 eq) and HOBt (80.5 mg, 0.596 mmol, 1.0 eq) were added. After the addition was complete, the reaction mixture was stirred at room temperature for 1 hour. After LC-MS monitoring indicated completion, the reaction mixture was concentrated directly. The residue was purified by silica gel column chromatography (PE/EA=1/1) to afford compound A141-4 (140 mg, 42%) as a colorless transparent liquid.
LCMS: [M+H]⁺ = 550.3

### Synthesis of Compound A141-5

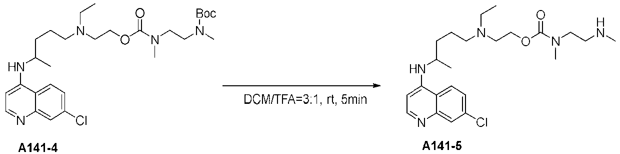

Compound A141-4 (140 mg, 0.255 mmol) was dissolved in DCM (3 mL). Trifluoroacetic acid (1 mL) was added to the reaction mixture, which was then stirred at room temperature for 5 minutes. After LC-MS monitoring indicated completion, the organic phase was concentrated to give the crude product compound A141-5, which was used directly in the next step.
LCMS: [M+H]⁺ = 450.2

### Synthesis of Compound A141-IM-1

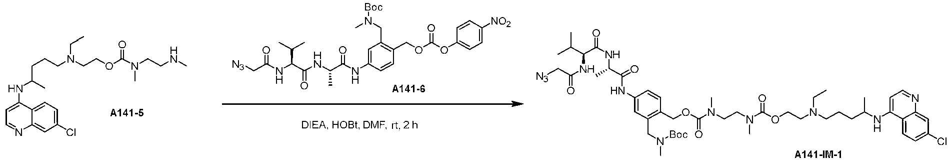

The compound A141-5 from the previous step was dissolved in DMF (3 mL). Then compound A141-6 (175.2 mg, 0.255 mmol, 1.0 eq), HOBt (34.5 mg, 0.255 mmol, 1.0 eq) and N,N-Diisopropylethylamine (99.0 mg, 0.765 mmol, 3.0 eq) were added. After the addition was complete, the reaction mixture was stirred at room temperature for 2 hours. After LC-MS monitoring indicated completion, the reaction mixture was diluted with ethyl acetate, washed with water and saturated brine. The organic phase was dried over anhydrous sodium sulfate, filtered, and concentrated. The residue was purified by silica gel column chromatography (DCM/MeOH=93/7) to afford compound A141-IM-1 (140 mg, 54%) as a white solid.
LCMS: [M+H]⁺ = 997.5

### Synthesis of Compound A141-IM-2

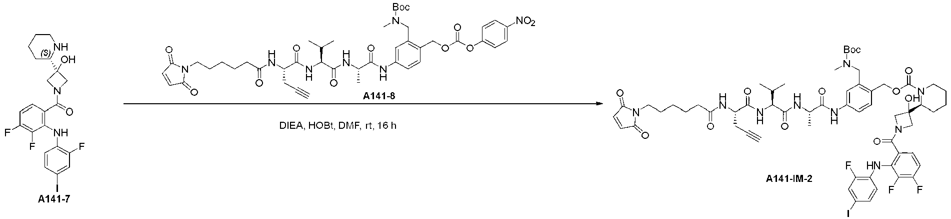

Compound A141-7 (200 mg, 0.376 mmol, 1.0 eq) was dissolved in DMF (3 mL). Then compound A141-8 (335.0 mg, 0.376 mmol, 1.0 eq), HOBt (50.8 mg, 0.376 mmol, 1.0 eq) and N,N-Diisopropylethylamine (145.6 mg, 1.128 mmol, 3.0 eq) were added. After the addition was complete, the reaction mixture was stirred at room temperature for 16 hours. After LC-MS monitoring indicated completion, the reaction mixture was diluted with ethyl acetate, washed with water and saturated brine. The organic phase was dried over anhydrous sodium sulfate, filtered, and concentrated. The residue was purified by silica gel column chromatography (DCM/MeOH=93/7) to afford compound A141-IM-2 (230 mg, 48%) as a white solid.
LCMS: [M+H]⁺ = 1283.4

### Synthesis of Compound A141-9

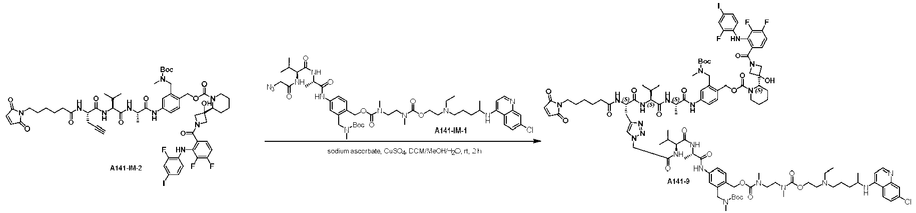

Compound A141-IM-2 (80 mg, 0.0623 mmol, 1.0 eq) and compound A141-IM-1 (62.1 mg, 0.0623 mmol, 1.0 eq) were dissolved in MeOH/DCM/water (5/5/1 mL). Then 100 uL of an aqueous solution of copper(II) sulfate pentahydrate (1.56 mg, 0.00623 mmol, 0.1 eq) and 100 uL of an aqueous solution of sodium ascorbate (2.47 mg, 0.01246 mmol, 0.2 eq) were added. After the addition was complete, the reaction mixture was stirred at room temperature under a nitrogen atmosphere for 2 hours. After LC-MS monitoring indicated completion, water (30 mL) was added to the reaction mixture. The mixture was extracted with dichloromethane. The organic phase was washed with water and then with saturated brine, dried over anhydrous sodium sulfate, filtered, and concentrated to afford compound A141-9 (140 mg, 97%), which was used directly in the next step.
LCMS: [M+H]⁺ = 1139.0 (half peak)

### Synthesis of Compound A141-10

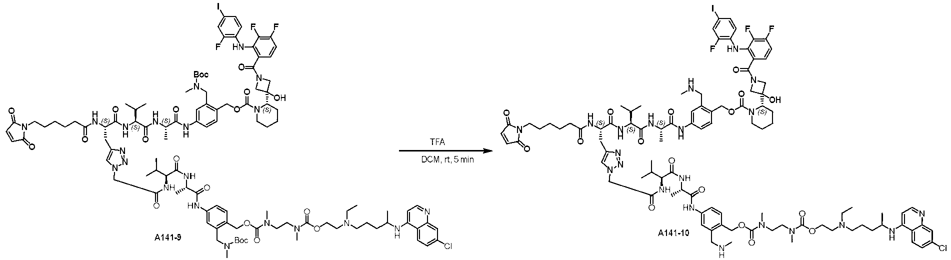

Compound A141-9 (130 mg, 0.057 mmol) was dissolved in DCM (3 mL). Trifluoroacetic acid (1 mL) was added to the reaction mixture, which was then stirred at room temperature for 5 minutes. After LC-MS monitoring indicated completion, the organic phase was concentrated to give the crude product compound A141-10, which was used directly in the next step.
LCMS: [M+H]⁺ = 1040.0 (half peak)

### Synthesis of Compound A141

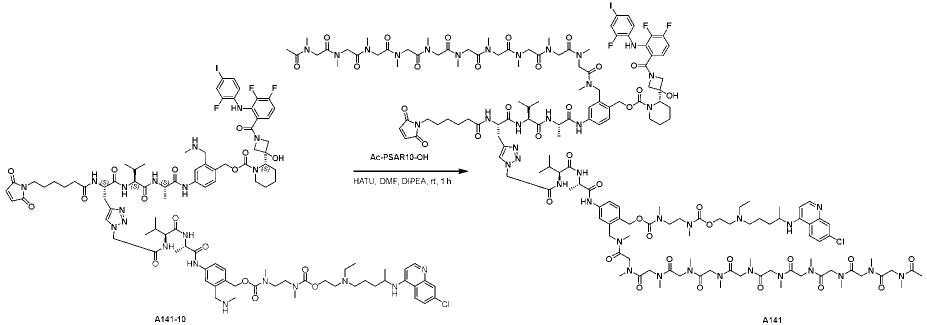

The compound A141-10 from the previous step was dissolved in DMF (3 mL). **Ac-PSAR10-OH** (87.8 mg, 0.114 mmol, 2.0 eq) and N,N-Diisopropylethylamine (147.1 mg, 1.14 mmol, 20.0 eq) were added to the reaction mixture. Finally, HATU (45.5 mg, 0.1197 mmol, 2.1 eq) was added. After the addition was complete, the reaction mixture was stirred at 0 °C under a nitrogen atmosphere for 10 minutes. After LC-MS monitoring indicated completion, ethyl acetate (45 mL) was added to the reaction mixture. The mixture was filtered, and the filter cake was dissolved in DMF and purified by prep-HPLC (mobile phase: 0.05% HCOOH/CH₃CN/H₂O) to afford compound A141 (10.7 mg, 5.2%) as a white solid.
LCMS: [M+H]⁺ = 1792.2 (half peak)

### (64) Synthesis of Compound A142

### Synthetic Route:

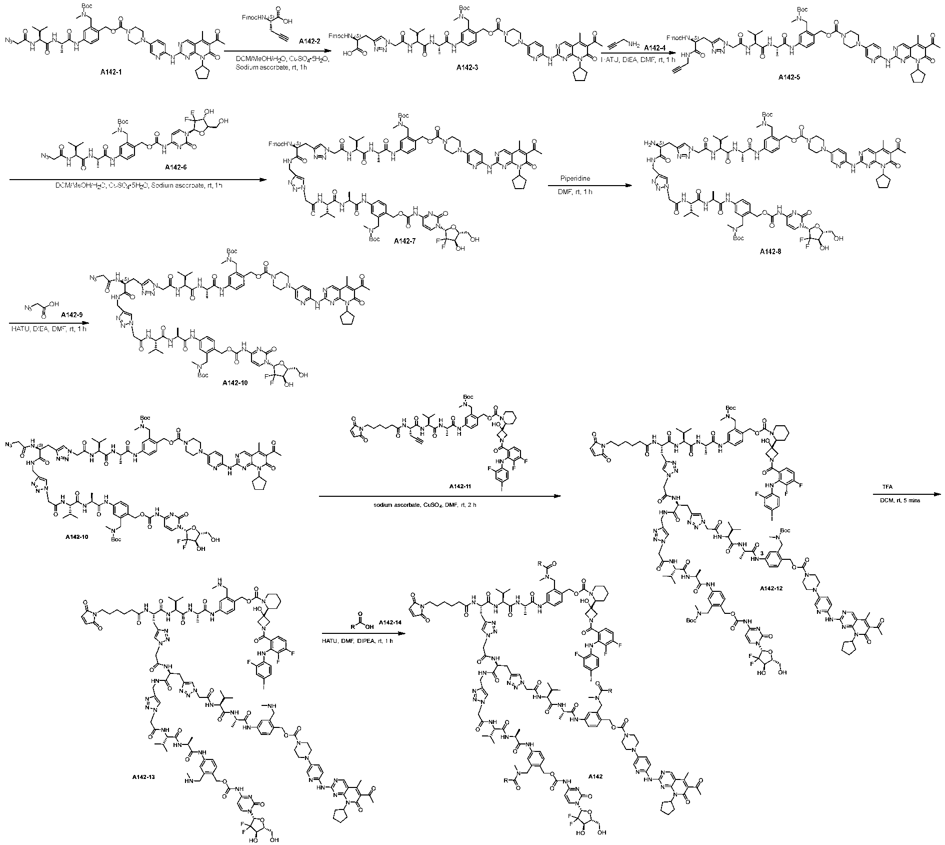

### Synthetic Steps:

### Synthesis of Intermediate Compound A142-3

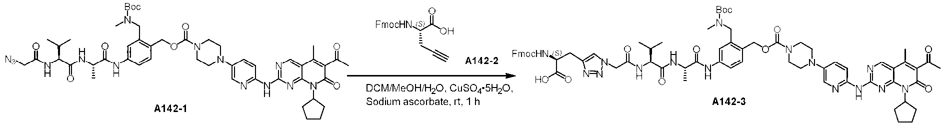

Compound A142-1 (130 mg, 0.131 mmol, 1.0 eq) and A142-2 (44 mg, 0.131 mmol, 1.0 eq) were dissolved in dichloromethane/methanol (4/4 mL). Under a nitrogen atmosphere, copper(II) sulfate pentahydrate (6.6 mg, 0.0262 mmol, 0.2 eq, in 100 uL water) and sodium ascorbate (10.4 mg, 0.0523 mmol, 0.4 eq, in 100 uL water) were added. After the addition was complete, the reaction mixture was stirred at room temperature under a nitrogen atmosphere for 1 hour. After LC-MS monitoring indicated completion, the reaction mixture was concentrated directly. Tetrahydrofuran was added, and the mixture was concentrated again to dryness. The crude product was slurried with ethyl acetate, then filtered and dried to afford compound A142-3 (170 mg, 98%) as a yellow solid.
LCMS: [M+H]⁺ = 1328.4

### Synthesis of Intermediate Compound A142-5

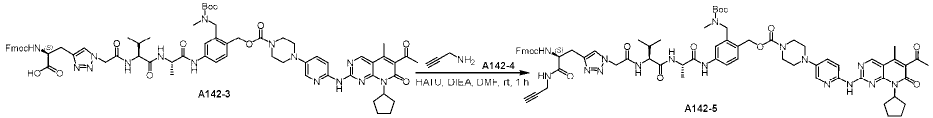

Compound A142-3 (160 mg, 0.120 mmol, 1.0 eq) was dissolved in DMF (5 mL). Compound A142-4 (10 mg, 0.180 mmol, 1.5 eq) and N,N-Diisopropylethylamine (47 mg, 0.361 mmol, 3.0 eq) were added to the reaction mixture. Finally, under a nitrogen atmosphere, O-(7-Azabenzotriazol-1-yl)-N,N,N',N'-tetramethyluronium hexafluorophosphate (60 mg, 0.156 mmol, 1.3 eq) was added. After the addition was complete, the reaction mixture was stirred at room temperature under a nitrogen atmosphere for 1 hour. After LC-MS monitoring indicated completion, the reaction mixture was diluted with 2-methyltetrahydrofuran and washed twice with water. The organic phase was dried over anhydrous sodium sulfate, filtered, and concentrated. The crude product was slurried with ethyl acetate, then filtered directly, and the filter cake was collected and dried to afford compound A142-5 (160 mg, 97%) as a yellow solid.
LCMS: [M+H]⁺ = 1366.6

### Synthesis of Intermediate Compound A142-7

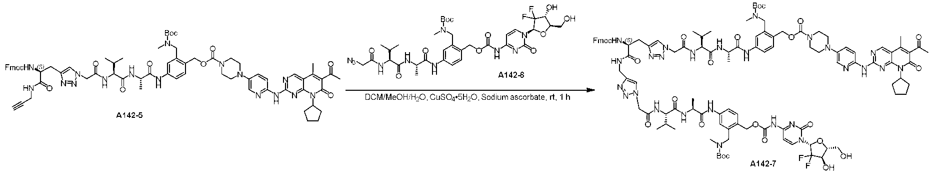

Compound A142-5 (160 mg, 0.117 mmol, 1.0 eq) and compound A142-6 (95 mg, 0.117 mmol, 1.0 eq) were dissolved in dichloromethane/methanol (5/5 mL). Under a nitrogen atmosphere, copper(II) sulfate pentahydrate (5.9 mg, 0.0234 mmol, 0.2 eq, in 100 uL water) and sodium ascorbate (9.3 mg, 0.0468 mmol, 0.4 eq, in 100 uL water) were added. After the addition was complete, the reaction mixture was stirred at room temperature under a nitrogen atmosphere for 1 hour. After LC-MS monitoring indicated completion, the reaction mixture was concentrated directly. The residue was dissolved in tetrahydrofuran and concentrated again to dryness. The crude product was slurried with ethyl acetate, filtered, and dried to afford compound A142-7 (220 mg, 86%) as a yellow solid.
LCMS: [M+H]⁺ = 1087.4 (half peak)

### Synthesis of Intermediate Compound A142-8

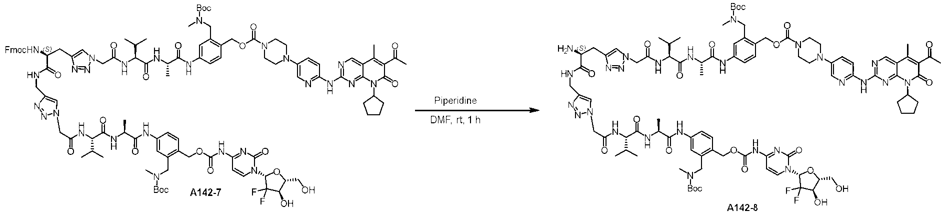

Compound A142-7 (100 mg, 0.0459 mmol, 1.0 eq) was dissolved in DMF (5 mL). Under a nitrogen atmosphere, piperidine (1 mL) was added. The reaction mixture was stirred at room temperature under a nitrogen atmosphere for 1 hour. After LC-MS monitoring indicated completion, methyl tert-butyl ether (100 mL) was added to the reaction mixture. The suspension was filtered directly, washed with methyl tert-butyl ether, and the filter cake was dried directly to afford compound A142-8 (70 mg, 78%) as a yellow solid.
LCMS: [M+H]⁺ = 977.0 (half peak)

### Synthesis of Intermediate Compound A142-10

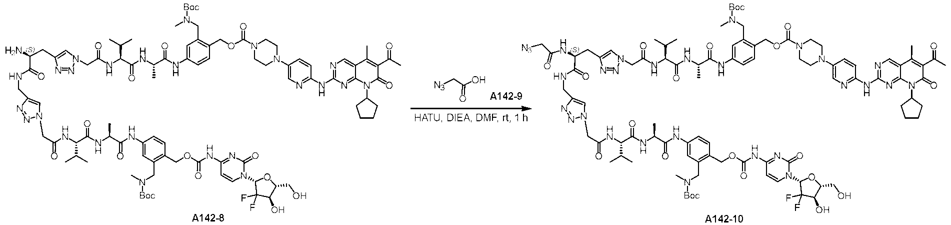

Compound A142-8 (70 mg, 0.0358 mmol, 1.0 eq) was dissolved in DMF (3 mL). N,N-Diisopropylethylamine (14 mg, 0.1074 mmol, 3.0 eq) and A142-9 (3.2 mg, 0.0573 mmol, 1.6 eq) were added to the reaction mixture. Finally, under a nitrogen atmosphere, O-(7-Azabenzotriazol-1-yl)-N,N,N',N'-tetramethyluronium hexafluorophosphate (24 mg, 0.0609 mmol, 1.7 eq) was added. After the addition was complete, the reaction mixture was stirred at room temperature under a nitrogen atmosphere for 1 hour. After LC-MS monitoring indicated completion, methyl tert-butyl ether (60 mL) was added to the reaction mixture. The suspension was filtered directly, washed with methyl tert-butyl ether, and the filter cake was dried. It was then slurried again with methanol, filtered, and dried to afford compound A142-10 (56 mg, 77%) as a yellow solid.
LCMS: [M+H]⁺ = 1018.1 (half peak)

### Synthesis of Intermediate Compound A142-12

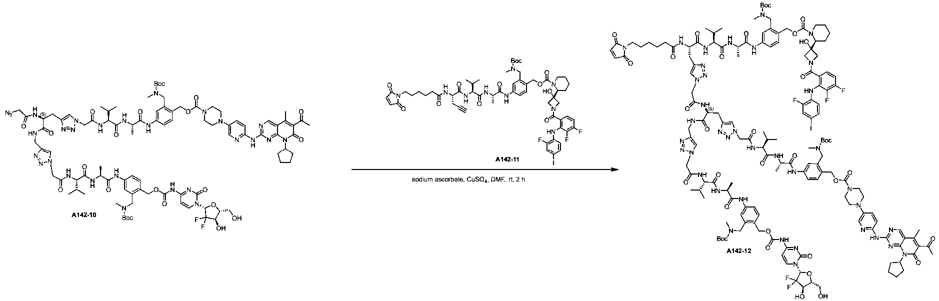

Compound A142-10 (56 mg, 0.0275 mmol, 1.0 eq) and compound A142-11 (35 mg, 0.0275 mmol, 1.0 eq) were dissolved in DMF (3 mL). Under a nitrogen atmosphere, copper(II) sulfate pentahydrate (1.4 mg, 0.0055 mmol, 0.2 eq, in 100 uL water) and sodium ascorbate (2.2 mg, 0.0110 mmol, 0.4 eq, in 100 uL water) were added. After the addition was complete, the reaction mixture was stirred at room temperature under a nitrogen atmosphere for 2 hours. After LC-MS monitoring indicated completion, the reaction mixture was diluted with ethyl acetate. The resulting suspension was filtered directly, washed with ethyl acetate, and the filter cake was dried directly to afford compound A142-12 (53 mg, 58%) as a yellow solid.
LCMS: [M+H]⁺ = 1658.9 (half peak)

### Synthesis of Intermediate Compound A142-13

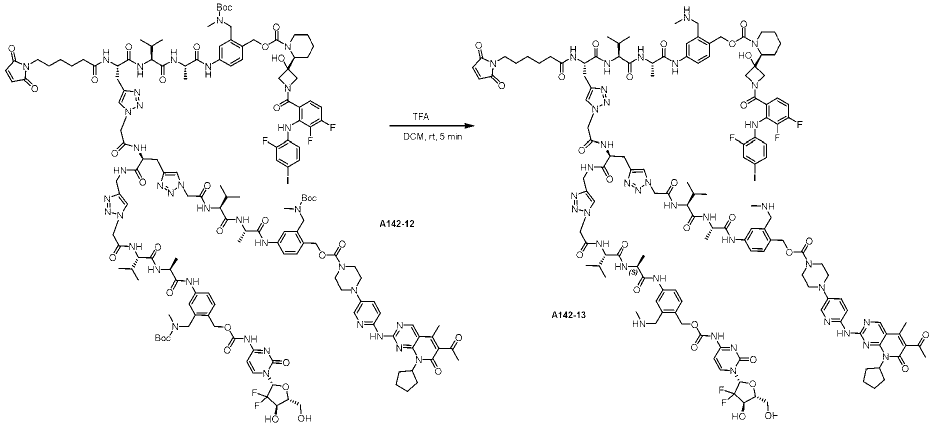

Compound A142-12 (53 mg, 0.016 mmol, 1.0 eq) was dissolved in DCM (3 mL). Trifluoroacetic acid (1 mL) was added to the reaction mixture, which was then stirred at room temperature for 5 minutes. After LC-MS monitoring indicated completion, the organic phase was concentrated to give the crude product compound A142-13, which was used directly in the next step.
LCMS: [M+H]⁺ = 1508.7 (half peak)

### Synthesis of Target Compound A142

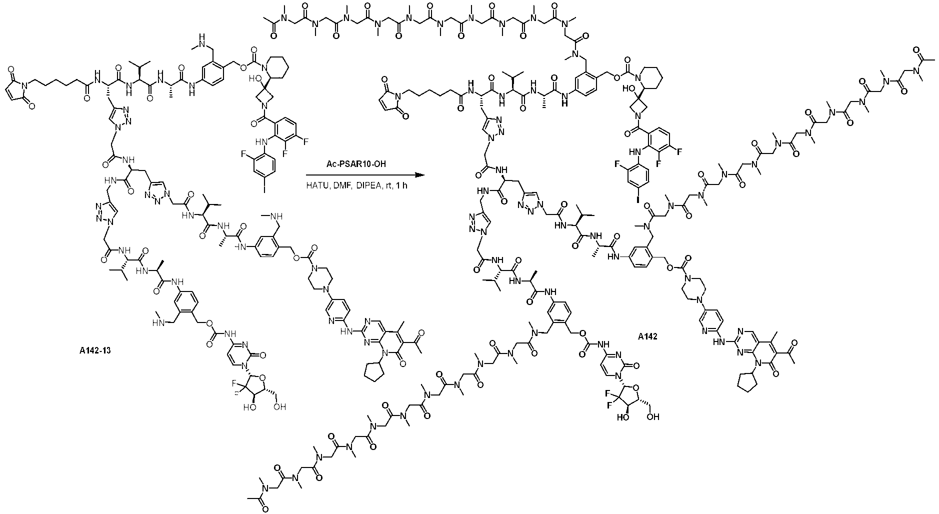

The compound A142-13 from the previous step was dissolved in DMF (4 mL). **Ac-PSAR10-OH** (71 mg, 0.048 mmol, 3.0 eq) was added to the reaction mixture. Finally, under a nitrogen atmosphere, N,N-Diisopropylethylamine (41 mg, 0.32 mmol, 20.0 eq) and HATU (20 mg, 0.0528 mmol, 3.3 eq) were added. After the addition was complete, the reaction mixture was stirred at 0 °C under a nitrogen atmosphere for 10 minutes. After LC-MS monitoring indicated completion, ethyl acetate (60 mL) was added to the reaction mixture. The resulting suspension was filtered, and the filter cake was dissolved in DMF and purified by prep-HPLC (mobile phase: 0.05% HCOOH/CH₃CN/H₂O) to afford compound A142 (3.2 mg, 7%) as a white solid.
LCMS: [M+H]⁺ = 1758.9 (one-third peak)

### (65) Synthesis of Compound A143

### Synthetic Route:

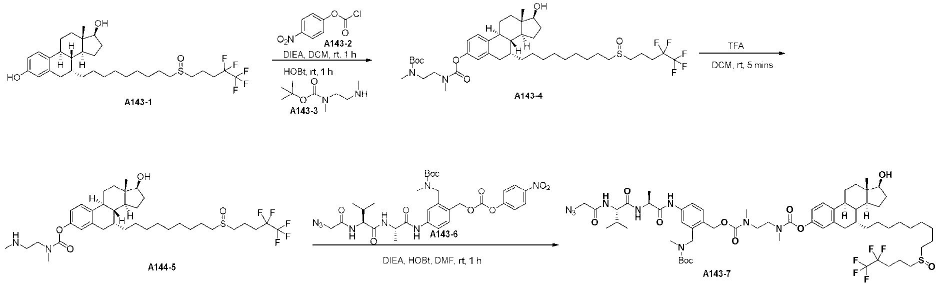

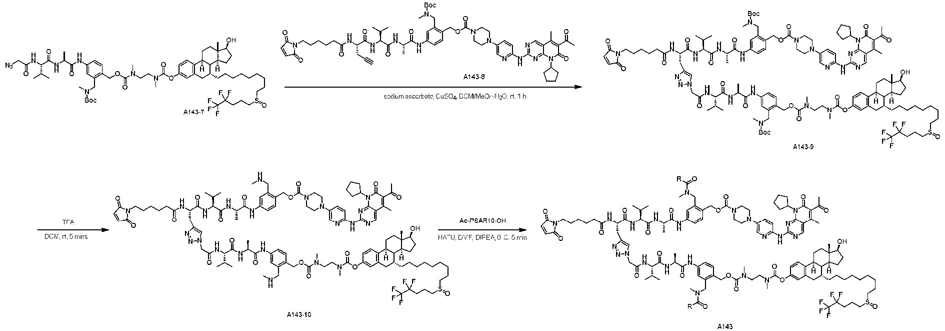

### Synthetic Steps:

### Synthesis of Compound A143-4

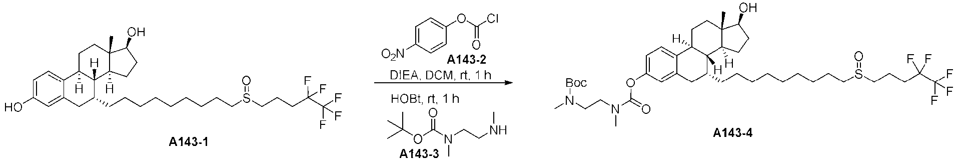

Compound A143-1 (120 mg, 0.198 mmol, 1.0 eq), compound A143-2 (43.9 mg, 0.218 mmol, 1.1 eq) and N,N-Diisopropylethylamine (76.7 mg, 0.593 mmol, 3.0 eq) were dissolved in DCM (5 mL). The reaction mixture was stirred at room temperature for 1 hour. Then compound A143-3 (55.9 mg, 0.297 mmol, 1.5 eq) and HOBt (26.7 mg, 0.198 mmol, 1.0 eq) were added. After the addition was complete, the reaction mixture was stirred at room temperature for 1 hour. After LC-MS monitoring indicated completion, the reaction mixture was concentrated directly. The residue was purified by silica gel column chromatography (PE/EA=1/1) to afford compound A143-4 (150 mg, 92%) as a colorless transparent liquid.
LCMS: [M+Na]⁺ = 843.4

### Synthesis of Compound A143-5

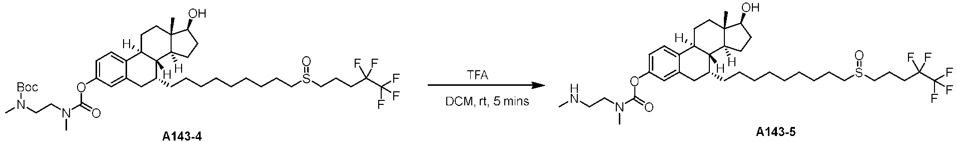

Compound A143-4 (150 mg, 0.183 mmol) was dissolved in DCM (3 mL). Trifluoroacetic acid (1 mL) was added to the reaction mixture, which was then stirred at room temperature for 5 minutes. After LC-MS monitoring indicated completion, the organic phase was concentrated to give the crude product compound A143-5, which was used directly in the next step.
LCMS: [M+H]⁺ = 721.4

### Synthesis of Compound A143-7

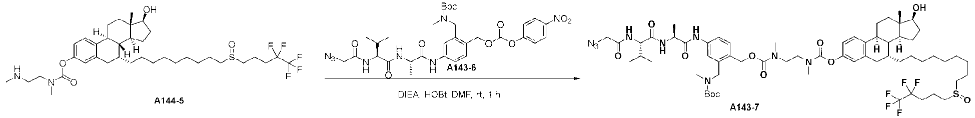

The compound A143-5 from the previous step was dissolved in DMF (3 mL). N,N-Diisopropylethylamine (118.1 mg, 0.915 mmol, 5.0 eq) was added and stirred for 10 minutes. Then compound A143-6 (125.5 mg, 0.183 mmol, 1.0 eq) and HOBt (24.7 mg, 0.183 mmol, 1.0 eq) were added. After the addition was complete, the reaction mixture was stirred at room temperature for 2 hours. After LC-MS monitoring indicated completion, the reaction mixture was diluted with ethyl acetate, washed with water and saturated brine. The organic phase was dried over anhydrous sodium sulfate, filtered, and concentrated. The residue was purified by silica gel column chromatography (DCM/MeOH=93/7) to afford compound A143-7 (140 mg, 61%) as a white solid.
LCMS: [M+Na]⁺ = 1288.5

### Synthesis of Compound A143-9

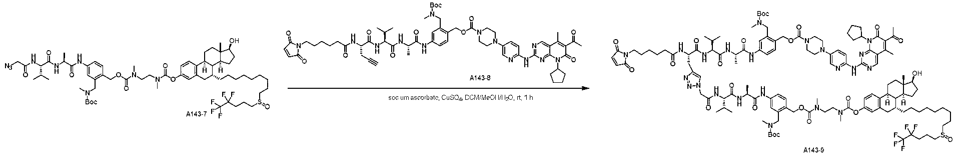

Compound A143-8 (104 mg, 0.0868 mmol, 1.0 eq) and compound A143-7 (110 mg, 0.0868 mmol, 1.0 eq) were dissolved in MeOH/DCM/water (4/4/1 mL). Then 100 uL of an aqueous solution of copper(II) sulfate pentahydrate (2.2 mg, 0.00868 mmol, 0.1 eq) and 100 uL of an aqueous solution of sodium ascorbate (3.5 mg, 0.01736 mmol, 0.2 eq) were added. After the addition was complete, the reaction mixture was stirred at room temperature under a nitrogen atmosphere for 1 hour. After LC-MS monitoring indicated completion, the reaction mixture was concentrated directly. Tetrahydrofuran was added, and the mixture was concentrated again to dryness, affording compound A143-9 (210 mg, 100%) as a yellow solid.
LCMS: [M+H]⁺ = 1232.6 (half peak)

### Synthesis of Compound A143-10

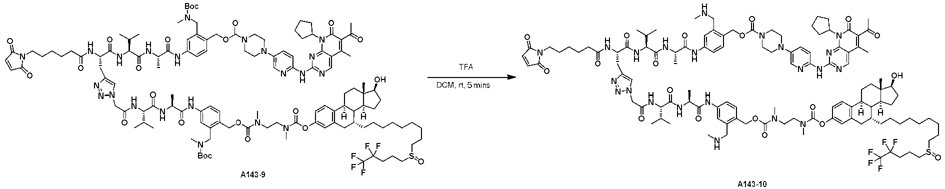

Compound A143-9 (100 mg, 0.0405 mmol) was dissolved in DCM (3 mL). Trifluoroacetic acid (1 mL) was added to the reaction mixture, which was then stirred at room temperature for 5 minutes. After LC-MS monitoring indicated completion, the reaction mixture was concentrated directly to give the crude product compound A143-10, which was used directly in the next step.
LCMS: [M+H]⁺ = 1132.4 (half peak)

### Synthesis of Compound A143

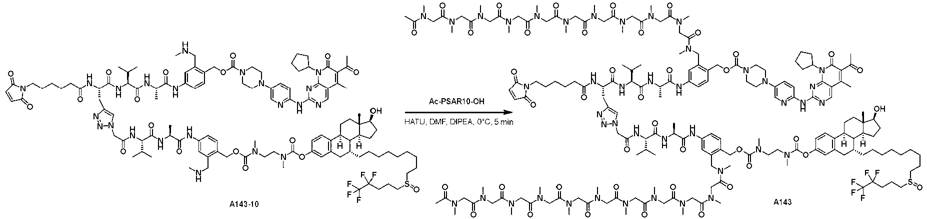

The compound A143-10 from the previous step was dissolved in DMF (3 mL). **Ac-PSAR10-OH** (63 mg, 0.081 mmol, 2.0 eq) and N,N-Diisopropylethylamine (105 mg, 0.81 mmol, 20.0 eq) were added to the reaction mixture. Finally, under a nitrogen atmosphere, O-(7-Azabenzotriazol-1-yl)-N,N,N',N'-tetramethyluronium hexafluorophosphate (32 mg, 0.0851 mmol, 2.1 eq) was added. After the addition was complete, the reaction mixture was stirred in an ice bath under a nitrogen atmosphere for 5 minutes. After LC-MS monitoring indicated completion, ethyl acetate (60 mL) was added to the reaction mixture. The resulting suspension was filtered, and the filter cake was dissolved in DMF and purified by prep-HPLC (mobile phase: 0.05% HCOOH/CH₃CN/H₂O) to afford compound A143 (22.8 mg, 15%) as a white solid. LCMS: [M+H]⁺ = 1885.3 (half peak)

### (66) Synthesis of Compound A146

### Synthetic Route:

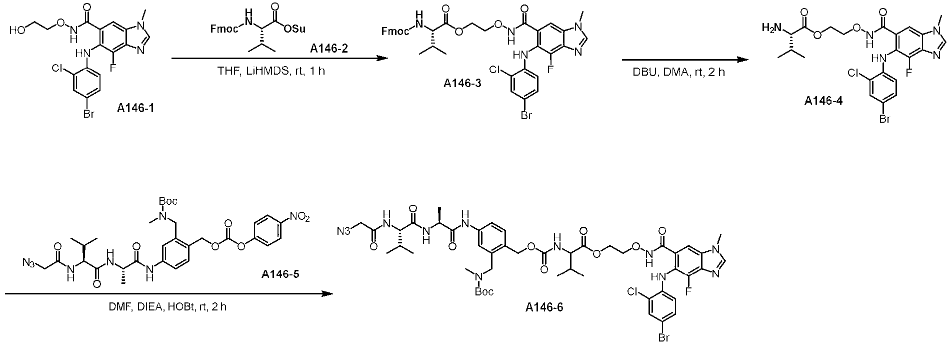

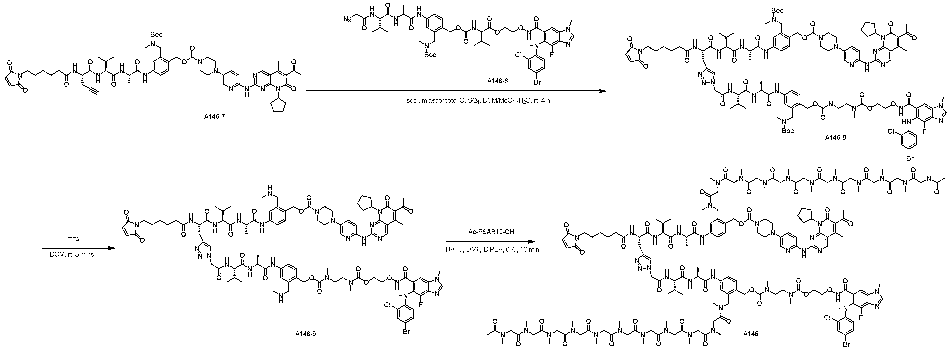

### Synthetic Steps:

### Synthesis of Intermediate Compound A146-3

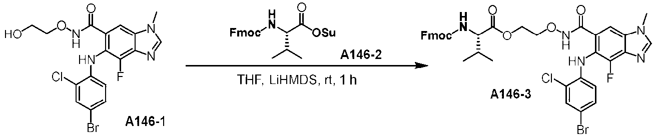

Compound A146-1 (250 mg, 0.546 mmol, 1.0 eq) was dissolved in anhydrous tetrahydrofuran (5 mL). Then 1 mol/L LiHMDS (1.2 mL, 1.202 mmol, 2.2 eq) was added, and the reaction mixture was stirred at room temperature for 15 minutes. Compound A146-2 (263 mg, 0.600 mmol, 1.1 eq, in 2 mL THF) was then added. After the addition was complete, the reaction mixture was stirred at room temperature for 1 hour. After LC-MS monitoring indicated completion, the reaction mixture was concentrated directly. The residue was purified by silica gel column chromatography (DCM/MeOH=93/7) to afford compound A146-3 (280 mg, 67%) as a white solid.
LCMS: [M+H]⁺ = 778.1

### Synthesis of Intermediate Compound A146-4

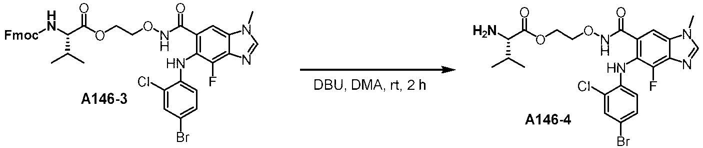

Compound A146-3 (280 mg, 0.359 mmol, 1.0 eq) was dissolved in DMA (3 mL). Then DBU (22 mg, 0.144 mmol, 0.7 eq) was added. After the addition was complete, the reaction mixture was stirred at room temperature for 2 hours. After LC-MS monitoring indicated completion, the reaction mixture was diluted with ethyl acetate, washed with water and saturated brine. The organic phase was dried over anhydrous sodium sulfate, filtered, and concentrated. The residue was purified by silica gel column chromatography (DCM/MeOH=93/7) to afford compound A146-4 (140 mg, 70%) as a white solid.
LCMS: [M+H]⁺ = 558.0

### Synthesis of Compound A146-6

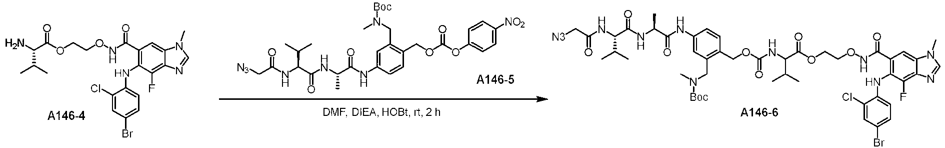

Compound A146-4 (140 mg, 0.251 mmol, 1.0 eq) was dissolved in DMF (5 mL). Then compound A146-5 (172 mg, 0.251 mmol, 1.0 eq), HOBt (34 mg, 0.251 mmol, 1.0 eq) and N,N-Diisopropylethylamine (97 mg, 0.754 mmol, 3.0 eq) were added. After the addition was complete, the reaction mixture was stirred at room temperature for 2 hours. After LC-MS monitoring indicated completion, the reaction mixture was diluted with ethyl acetate, washed with water and saturated brine. The organic phase was dried over anhydrous sodium sulfate, filtered, and concentrated. The residue was purified by silica gel column chromatography (DCM/MeOH=97/3) to afford compound A146-6 (150 mg, 54%) as a white solid.
LCMS: [M+H]⁺ = 1101.2

### Synthesis of Compound A146-8

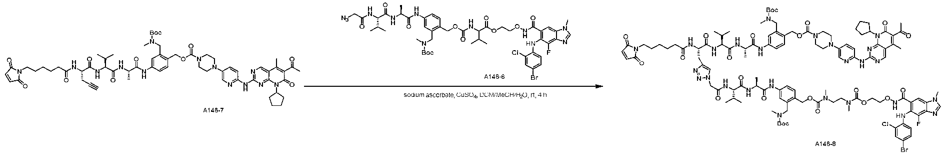

Compound A146-7 (100 mg, 0.0834 mmol, 1.0 eq) and compound A146-6 (92 mg, 0.0834 mmol, 1.0 eq) were dissolved in MeOH/DCM/water (5/5/1 mL). Then 100 uL of an aqueous solution of copper(II) sulfate pentahydrate (2.08 mg, 0.00834 mmol, 0.1 eq) and 100 uL of an aqueous solution of sodium ascorbate (3.3 mg, 0.01668 mmol, 0.2 eq) were added. After the addition was complete, the reaction mixture was stirred at room temperature under a nitrogen atmosphere for 4 hours. After LC-MS monitoring indicated completion, the reaction mixture was concentrated directly. Tetrahydrofuran was added, and the mixture was concentrated again to dryness, affording compound A146-8 (190 mg, 99%) as a yellow solid.
LCMS: [M+H]⁺ = 1151.0 (half peak)

### Synthesis of Compound A146-9

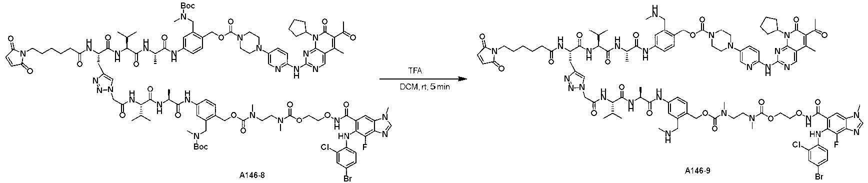

Compound A146-8 (100 mg, 0.0434 mmol) was dissolved in DCM (3 mL). Trifluoroacetic acid (1 mL) was added to the reaction mixture, which was then stirred at room temperature for 5 minutes. After LC-MS monitoring indicated completion, the organic phase was concentrated to give the crude product compound A146-9, which was used directly in the next step.
LCMS: [M+H]⁺ = 1050.1 (half peak)

### Synthesis of Compound A146

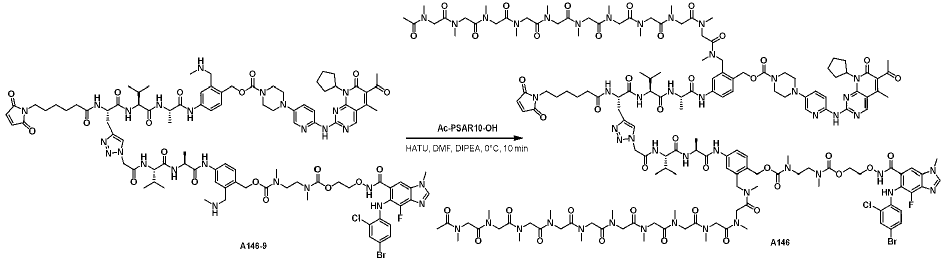

The compound A 146-9 from the previous step was dissolved in DMF (3 mL). **Ac-PSAR10-OH** (67 mg, 0.0869 mmol, 2.0 eq) was added to the reaction mixture. Finally, under a nitrogen atmosphere, N,N-Diisopropylethylamine (112 mg, 0.869 mmol, 20.0 eq) and O-(7-Azabenzotriazol-1-yl)-N,N,N',N'-tetramethyluronium hexafluorophosphate (35 mg, 0.0912 mmol, 2.1 eq) were added. After the addition was complete, the reaction mixture was stirred at 0 °C under a nitrogen atmosphere for 10 minutes. After LC-MS monitoring indicated completion, ethyl acetate (45 mL) was added to the reaction mixture. The mixture was filtered, and the filter cake was dissolved in DMF and purified by prep-HPLC (mobile phase: 0.05% HCOOH/CH₃CN/H₂O) to afford compound A146 (42.9 mg, 28.1%) as a white solid.
LCMS: [M+H]⁺ = 1804.1 (half peak)

### (67) Synthesis of Compound A147

### Synthetic Route:

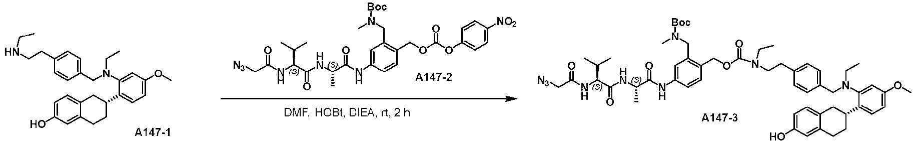

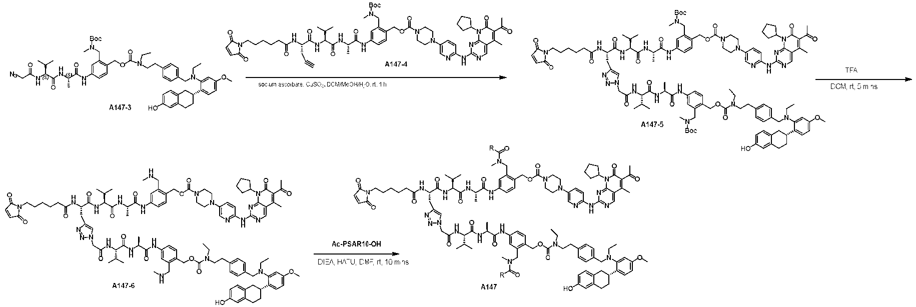

### Synthetic Steps:

### Synthesis of Intermediate Compound A147-3

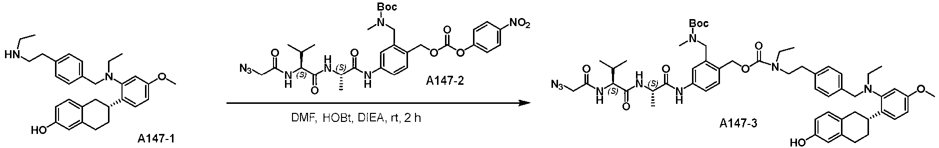

Compound A147-1 (400 mg, 0.87 mmol, 1.0 eq) was dissolved in DMF (4 mL). N,N-Diisopropylethylamine (338 mg, 2.62 mmol, 3.0 eq) was added and stirred for 2 minutes. Then compound A147-2 (598 mg, 0.87 mmol, 1.0 eq) was added. After the addition was complete, the reaction mixture was stirred at room temperature for 2 hours. After LC-MS monitoring indicated completion, ethyl acetate (40 mL) was added to the reaction mixture. The mixture was washed with water and then twice with saturated brine. The obtained organic phase was dried over anhydrous sodium sulfate, filtered, and concentrated. The residue was purified by silica gel column chromatography (DCM/MeOH=93/7) to afford compound A147-3 (56 mg, 81%) as a white solid.
LCMS: [M+H]⁺ = 1004.4

### Synthesis of Intermediate Compound A147-5

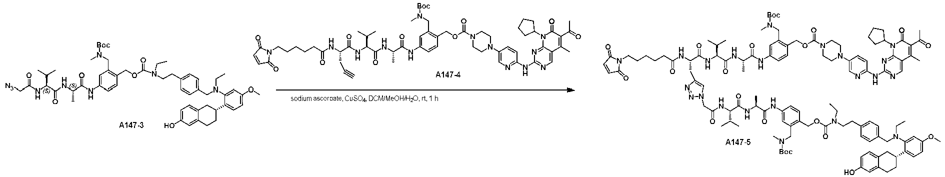

Compound A147-3 (100 mg, 0.099 mmol, 1 eq) and compound A147-4 (119 mg, 0.099 mmol, 1 eq) were dissolved in MeOH/DCM/water (4/4/0.8 mL). Then 100 uL of an aqueous solution of copper(II) sulfate pentahydrate (2.5 mg, 0.0099 mmol, 0.1 eq) and 100 uL of an aqueous solution of sodium ascorbate (4.9 mg, 0.025 mmol, 0.25 eq) were added. After the addition was complete, the reaction mixture was stirred at room temperature under a nitrogen atmosphere for 1 hour. After LC-MS monitoring indicated the reaction was basically complete, the reaction mixture was concentrated directly to afford the crude product compound A147-5 (219 mg, 100%).
LCMS: [M+H]⁺ = 1102.1 (half peak)

### Synthesis of Intermediate Compound A147-6

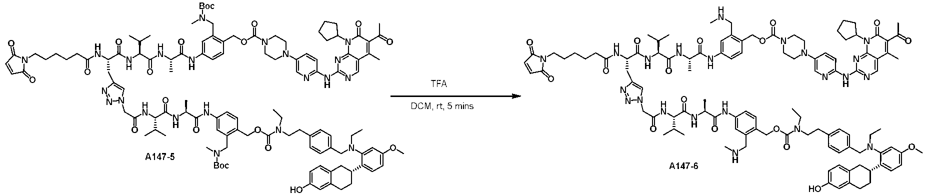

Compound A147-5 (140 mg, 0.064 mmol, 1.0 eq) was dissolved in dichloromethane (3 mL). Trifluoroacetic acid (1 mL) was added to the reaction mixture, which was then stirred at room temperature for 5 min. After LC-MS monitoring indicated completion, the reaction mixture was concentrated to give the crude product compound A147-6, which was used directly in the next step.
LCMS: [M+H]⁺ = 1001.7 (half peak)

### Synthesis of Target Compound A147

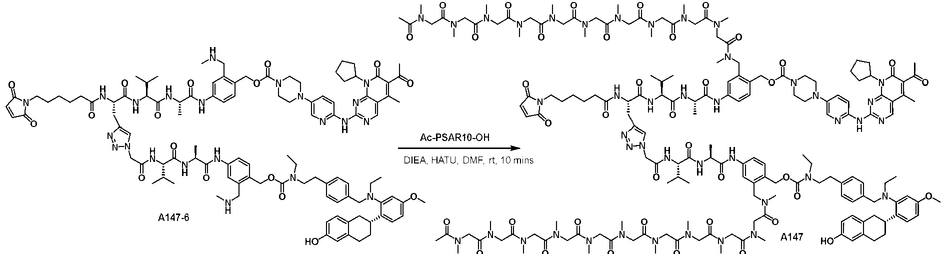

The crude product compound A147-6 from the previous step was dissolved in DMF (3 mL). N,N-Diisopropylethylamine (165 mg, 1.28 mmol, 20.0 eq) was added. Then **Ac-PSAR10-OH** (98.7 mg, 0.128 mmol, 2.0 eq) and O-(7-Azabenzotriazol-1-yl)-N,N,N',N'-tetramethyluronium hexafluorophosphate (54 mg, 0.141 mmol, 2.2 eq) were added to the reaction mixture. After the addition was complete, the reaction mixture was stirred at room temperature for 10 minutes. After LC-MS monitoring indicated completion, EA (45 mL) was added to the reaction mixture. The resulting suspension was filtered, and the filter cake was dissolved in DMF and purified by prep-HPLC (mobile phase: 0.05% HCOOH/CH₃CN/H₂O) to afford compound A147 (45 mg, 34%) as a white solid.
LCMS: [M+H]⁺ = 1754.8 (half peak)

### (68) Synthesis of Compound A148

### Synthetic Route:

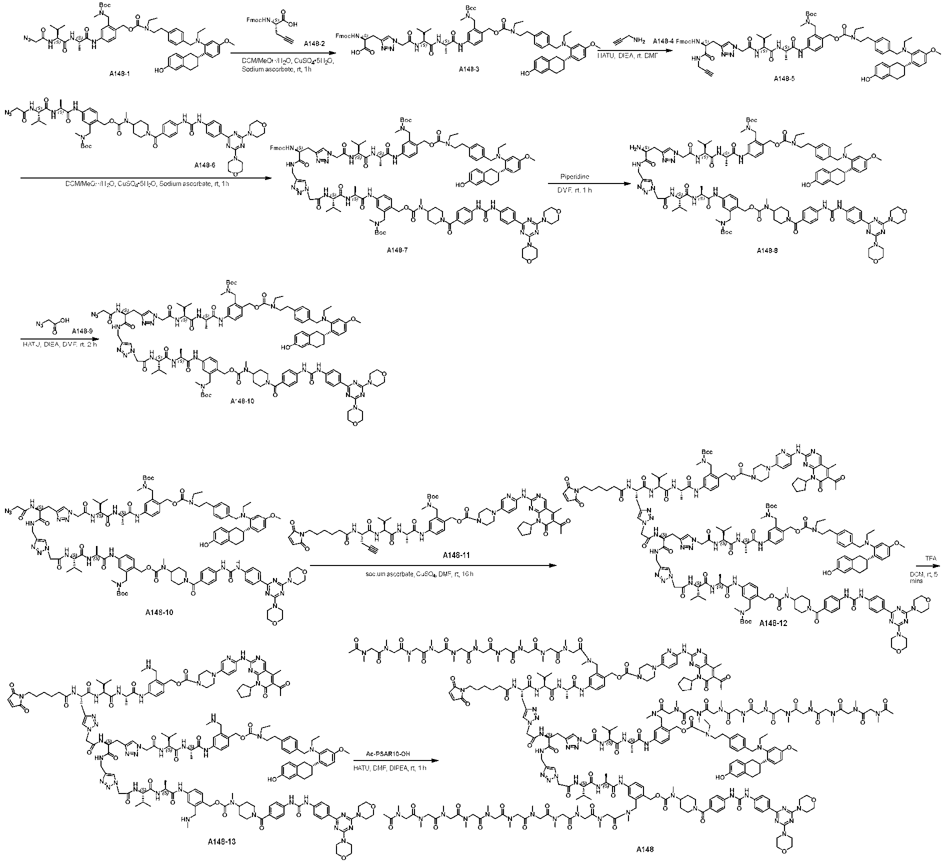

### Synthetic Steps:

### Synthesis of Intermediate Compound A148-3

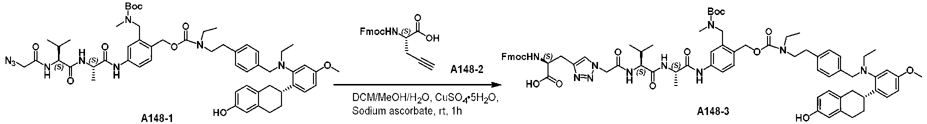

Compound **A148-1** (350 mg, 0.348 mmol, 1.0 eq) and **A148-2** (116 mg, 0.348 mmol, 1.0 eq) were dissolved in dichloromethane/methanol/water (4/4/1 mL). Under a nitrogen atmosphere, copper(II) sulfate pentahydrate (8.7 mg, 0.0348 mmol, 0.1 eq, in 100 uL water) and sodium ascorbate (13.8 mg, 0.0696 mmol, 0.2 eq, in 100 uL water) were added. After the addition was complete, the reaction mixture was stirred at room temperature under a nitrogen atmosphere for 1 hour. After LC-MS monitoring indicated completion, the reaction mixture was concentrated directly. Tetrahydrofuran was added, and the mixture was concentrated again to dryness, affording compound **A148-3** (460 mg, 98%) as a grey solid.
LCMS: [M+H]⁺ = 1339.6

### Synthesis of Intermediate Compound A148-5

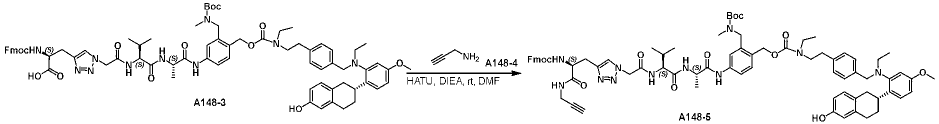

Compound **A148-3** (440 mg, 0.328 mmol, 1.0 eq) was dissolved in DMF (3 mL). Compound **A148-4** (54 mg, 0.985 mmol, 3.0 eq) and N,N-Diisopropylethylamine (84.8 mg, 0.656 mmol, 2.0 eq) were added to the reaction mixture. Finally, under a nitrogen atmosphere, O-(7-Azabenzotriazol-1-yl)-N,N,N',N'-tetramethyluronium hexafluorophosphate (162 mg, 0.427 mmol, 1.3 eq) was added. After the addition was complete, the reaction mixture was stirred at room temperature under a nitrogen atmosphere for 1 hour. After LC-MS monitoring indicated completion, methyl tert-butyl ether (80 mL) was added to the reaction mixture. The suspension was filtered directly, and the filter cake was collected and dried to afford compound **A148-5** (450 mg, 99%) as a grey solid.
LCMS: [M+H]⁺ = 1377.6

### Synthesis of Intermediate Compound A148-7

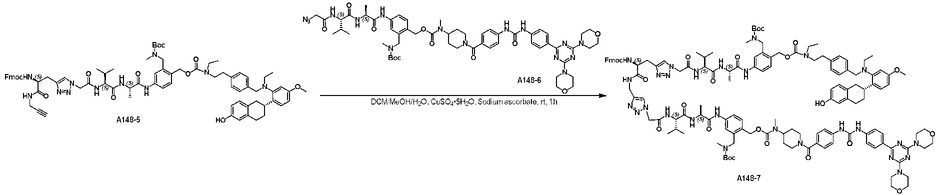

Compound **A148-5** (200 mg, 0.145 mmol, 1.0 eq) and compound **A148-6** (167 mg, 0.145 mmol, 1.0 eq) were dissolved in dichloromethane/methanol/water (4/4/0.5 mL). Under a nitrogen atmosphere, copper(II) sulfate pentahydrate (3.6 mg, 0.0145 mmol, 0.1 eq, in 100 uL water) and sodium ascorbate (7.2 mg, 0.0363 mmol, 0.25 eq, in 100 uL water) were added. After the addition was complete, the reaction mixture was stirred at room temperature under a nitrogen atmosphere for 1 hour. After LC-MS monitoring indicated completion, the reaction mixture was concentrated directly. The residue was dissolved in tetrahydrofuran and concentrated again to dryness, affording compound **A148-7** (366 mg, 100%) as a brownish-yellow solid.
LCMS: [M+H]⁺ = 1262.6 (half peak)

### Synthesis of Intermediate Compound A148-8

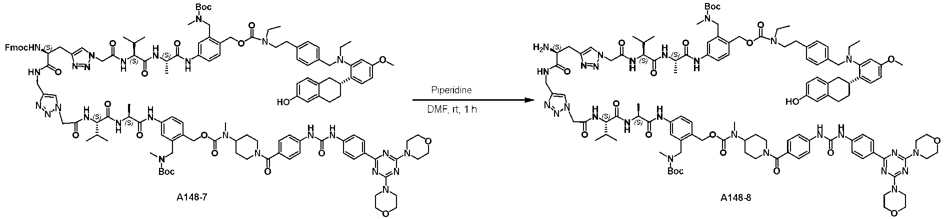

Compound **A148-7** (366 mg, 0.145 mmol, 1.0 eq) was dissolved in DMF (5 mL). The reaction mixture was cooled to 0 °C, and under a nitrogen atmosphere, piperidine (1 mL) was added. The reaction mixture was stirred at room temperature under a nitrogen atmosphere for half an hour. After LC-MS monitoring indicated completion, methyl tert-butyl ether (50 mL) was added to the reaction mixture. The suspension was filtered directly, washed with methyl tert-butyl ether, and the filter cake was dried directly to afford compound **A148-8** (334 mg, 75%) as a yellow solid.
LCMS: [M+H]⁺ = 1500.9 (half peak)

### Synthesis of Intermediate Compound A148-10

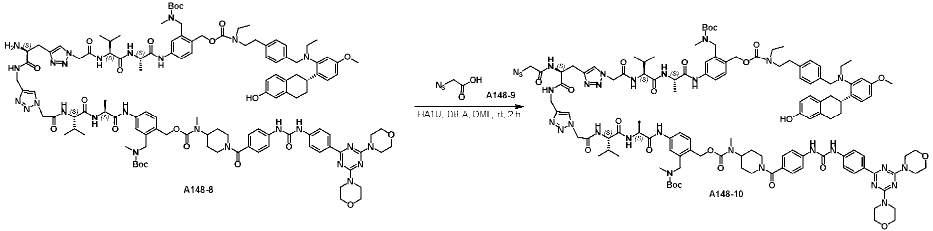

Compound **A148-8** (240 mg, 0.104 mmol, 1.0 eq) was dissolved in DMF (3 mL). N,N-Diisopropylethylamine (67 mg, 0.520 mmol, 5.0 eq) and **A148-9** (21 mg, 0.208 mmol, 2.0 eq) were added to the reaction mixture. Finally, under a nitrogen atmosphere, O-(7-Azabenzotriazol-1-yl)-N,N,N',N'-tetramethyluronium hexafluorophosphate (99 mg, 0.260 mmol, 2.5 eq) was added. After the addition was complete, the reaction mixture was stirred at room temperature under a nitrogen atmosphere for 2 hours. After LC-MS monitoring indicated completion, methyl tert-butyl ether (100 mL) was added to the reaction mixture. The suspension was filtered directly, washed with methyl tert-butyl ether, and the filter cake was dried directly to afford compound **A148-10** (140 mg, 56%) as a yellow solid.
LCMS: [M+H]⁺ = 1193.1 (half peak)

### Synthesis of Intermediate Compound A148-12

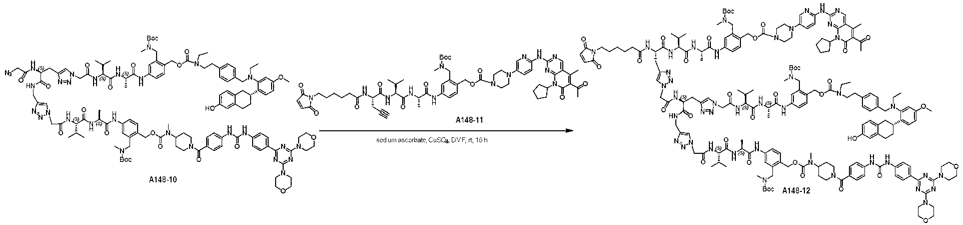

Compound **A148-10** (130 mg, 0.055 mmol, 1.0 eq) and compound **A148-11** (66 mg, 0.055 mmol, 1.0 eq) were dissolved in DMF (4 mL). Under a nitrogen atmosphere, copper(II) sulfate pentahydrate (1.4 mg, 0.0055 mmol, 0.1 eq, in 100 uL water) and sodium ascorbate (2.7 mg, 0.013 mmol, 0.25 eq, in 100 uL water) were added. After the addition was complete, the reaction mixture was stirred at room temperature under a nitrogen atmosphere for 16 hours. After LC-MS monitoring indicated completion, methyl tert-butyl ether (60 mL) was added to the reaction mixture. The suspension was filtered directly, washed with methyl tert-butyl ether, and the filter cake was dried directly to afford compound **A148-12** (170 mg, 86%) as a yellow solid.
LCMS: [M+H]⁺ = 1195.1 (one-third peak)

### Synthesis of Intermediate Compound A148-13

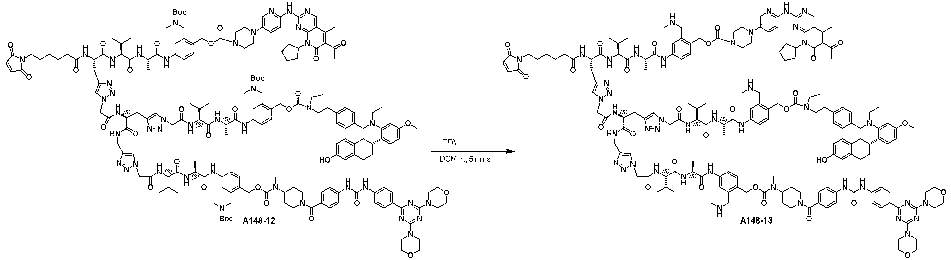

Compound **A148-12** (170 mg, 0.047 mmol, 1.0 eq) was dissolved in DCM (3 mL). Trifluoroacetic acid (1 mL) was added to the reaction mixture, which was then stirred at room temperature for 5 minutes. After LC-MS monitoring indicated completion, the organic phase was concentrated to give the crude product compound **A148-13,** which was used directly in the next step.
LCMS: [M+H]⁺ = 1641.3 (half peak)

### Synthesis of Compound A148

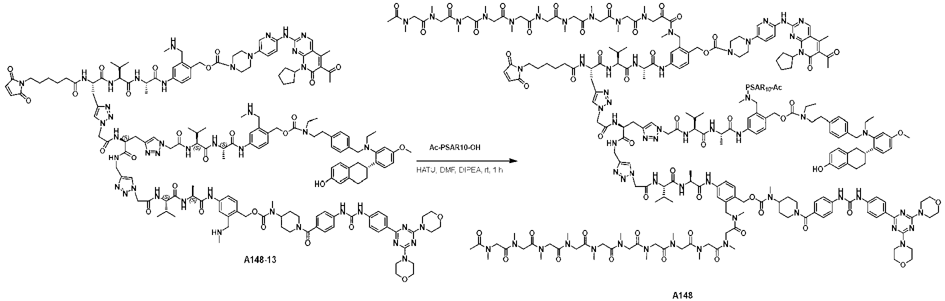

The compound **A148-13** from the previous step was dissolved in DMF (3 mL). **Ac-PSAR10-OH** (108.7 mg, 0.141 mmol, 3.0 eq) and N,N-Diisopropylethylamine (121 mg, 0.94 mmol, 20.0 eq) were added to the reaction mixture. Finally, under a nitrogen atmosphere, O-(7-Azabenzotriazol-1-yl)-N,N,N',N'-tetramethyluronium hexafluorophosphate (59 mg, 0.155 mmol, 3.3 eq) was added. After the addition was complete, the reaction mixture was stirred at room temperature under a nitrogen atmosphere for 1 hour. After LC-MS monitoring indicated completion, ethyl acetate (50 mL) was added to the reaction mixture. The resulting suspension was filtered, and the filter cake was dissolved in DMF and purified by prep-HPLC (mobile phase: 0.05% HCOOH/CH₃CN/H₂O) to afford compound **A148** (51 mg, 32%) as a yellow solid. LCMS: [M+H]⁺ = 1846.8 (one-third peak)

### (68) Synthesis of Compound A164

### Synthetic Route:

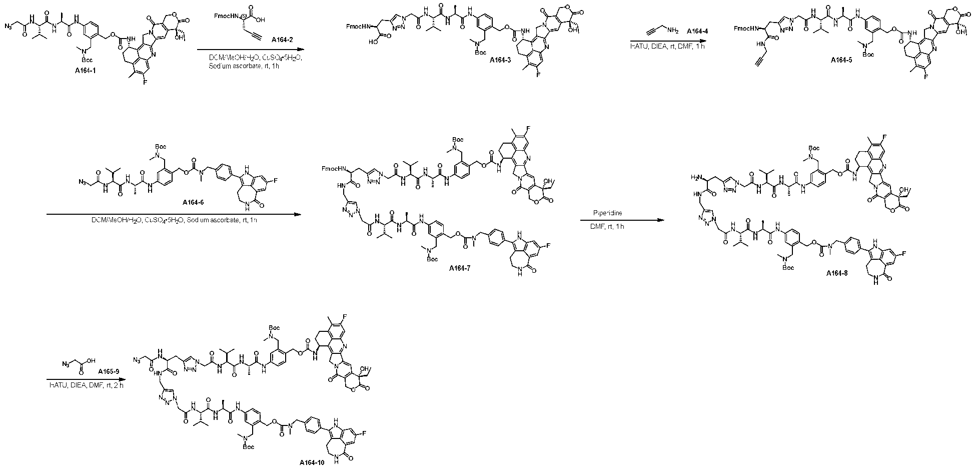

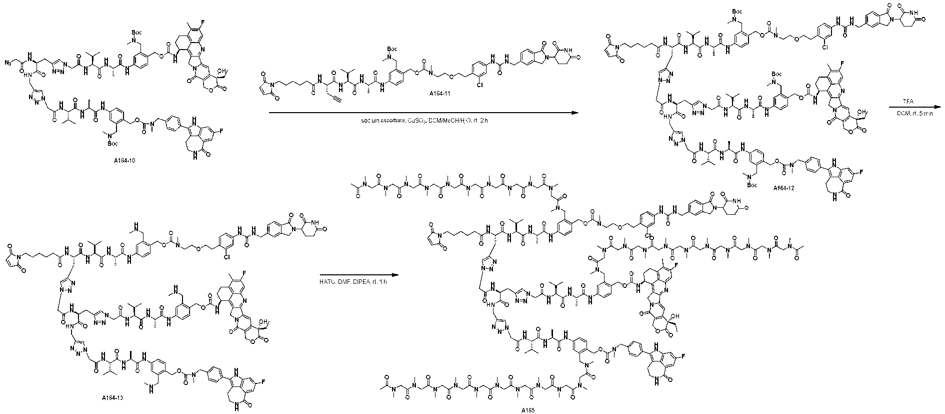

### Synthetic Steps:

### Synthesis of Intermediate Compound A164-3

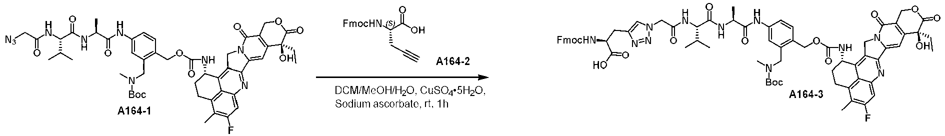

Compound A164-1 (800 mg, 0.815 mmol, 1.0 eq) and A164-2 (273 mg, 0.815 mmol, 1.0 eq) were dissolved in dichloromethane/methanol (15/15 mL). Under a nitrogen atmosphere, copper(II) sulfate pentahydrate (40.7 mg, 0.163 mmol, 0.2 eq, in 500 uL water) and sodium ascorbate (64.5 mg, 0.326 mmol, 0.4 eq, in 500 uL water) were added. After the addition was complete, the reaction mixture was stirred at room temperature under a nitrogen atmosphere for 1 hour. After LC-MS monitoring indicated completion, the reaction mixture was concentrated directly. Tetrahydrofuran was added, and the mixture was concentrated again to dryness. The crude product was slurried with ethyl acetate, then filtered and dried to afford compound A164-3 (800 mg, 75%) as a yellow solid.
LCMS: [M+Na]⁺ = 1316.3

### Synthesis of Intermediate Compound A164-5

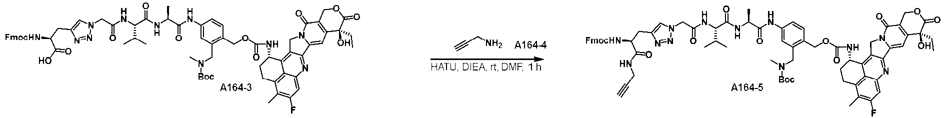

Compound A164-3 (800 mg, 0.607 mmol, 1.0 eq) was dissolved in DMF (10 mL). Compound A164-4 (50 mg, 0.911 mmol, 1.5 eq) and N,N-Diisopropylethylamine (235 mg, 1.821 mmol, 3.0 eq) were added to the reaction mixture. Finally, under a nitrogen atmosphere, O-(7-Azabenzotriazol-1-yl)-N,N,N',N'-tetramethyluronium hexafluorophosphate (300 mg, 0.789 mmol, 1.3 eq) was added. After the addition was complete, the reaction mixture was stirred at room temperature under a nitrogen atmosphere for 1 hour. After LC-MS monitoring indicated completion, the reaction mixture was added to methyl tert-butyl ether/ethyl acetate (160 mL, 7:1). The resulting suspension was filtered directly, and the filter cake was collected and dried to afford compound A164-5 (790 mg, 96%) as a yellow solid.
LCMS: [M+Na]⁺ = 1354.4

### Synthesis of Intermediate Compound A164-7

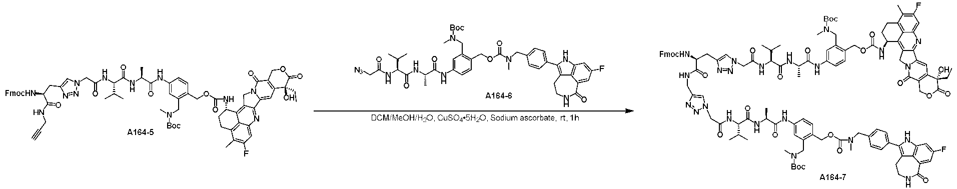

Compound A164-5 (800 mg, 0.590 mmol, 1.0 eq) and compound A164-6 (514 mg, 0.590 mmol, 1.0 eq) were dissolved in dichloromethane/methanol (6/6 mL). Under a nitrogen atmosphere, copper(II) sulfate pentahydrate (29.5 mg, 0.118 mmol, 0.2 eq, in 300 uL water) and sodium ascorbate (46.8 mg, 0.237 mmol, 0.4 eq, in 300 uL water) were added. After the addition was complete, the reaction mixture was stirred at room temperature under a nitrogen atmosphere for 1 hour. After LC-MS monitoring indicated completion, the reaction mixture was concentrated directly. The residue was dissolved in tetrahydrofuran and concentrated again to dryness. The crude product was slurried with ethyl acetate, filtered, and dried to afford compound A164-7 (1100 mg, 84%) as a brownish-yellow solid.
LCMS: [M+H]⁺ = 1111.9 (half peak)

### Synthesis of Intermediate Compound A164-8

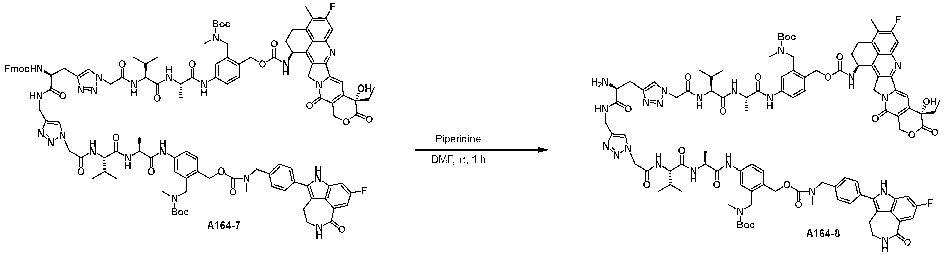

Compound A164-7 (1000 mg, 0.450 mmol, 1.0 eq) was dissolved in DMF (20 mL). Under a nitrogen atmosphere, piperidine (4 mL) was added. The reaction mixture was stirred at room temperature under a nitrogen atmosphere for 1 hour. After LC-MS monitoring indicated completion, methyl tert-butyl ether (300 mL) was added to the reaction mixture. The suspension was filtered directly, washed with methyl tert-butyl ether, and the filter cake was dried directly to afford compound A164-8 (760 mg, 84%) as a yellow solid.
LCMS: [M+H]⁺ = 1000.5 (half peak)

### Synthesis of Intermediate Compound A164-10

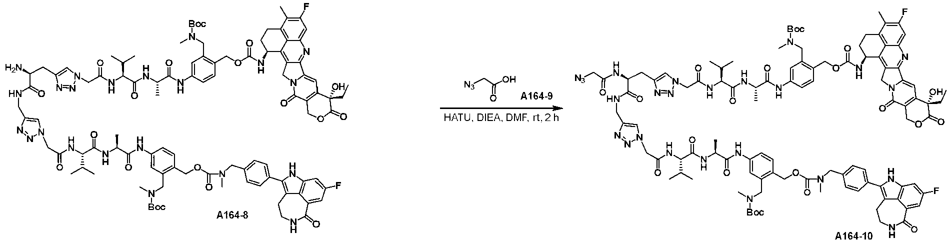

Compound A164-8 (760 mg, 0.380 mmol, 1.0 eq) was dissolved in DMF (10 mL). N,N-Diisopropylethylamine (147 mg, 1.140 mmol, 3.0 eq) and A164-9 (77 mg, 0.760 mmol, 2.0 eq) were added to the reaction mixture. Finally, under a nitrogen atmosphere, O-(7-Azabenzotriazol-1-yl)-N,N,N',N'-tetramethyluronium hexafluorophosphate (317 mg, 0.836 mmol, 2.2 eq) was added. After the addition was complete, the reaction mixture was stirred at room temperature under a nitrogen atmosphere for 1 hour. After LC-MS monitoring indicated completion, methyl tert-butyl ether (100 mL) was added to the reaction mixture. The suspension was filtered directly, washed with methyl tert-butyl ether, and the filter cake was dried. It was then slurried with ethyl acetate, filtered, and dried to afford compound A164-10 (649 mg, 82%) as a yellow solid.
LCMS: [M+H]⁺ = 1042.3 (half peak)

### Synthesis of Intermediate Compound A164-12

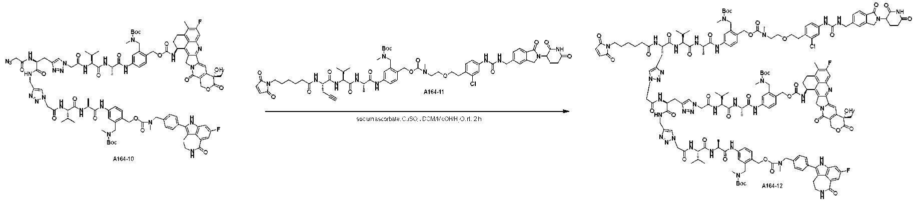

Compound A164-10 (160 mg, 0.077 mmol, 1.0 eq) and compound A164-11 (98 mg, 0.077 mmol, 1.0 eq) were dissolved in DMF (4 mL). Under a nitrogen atmosphere, copper(II) sulfate pentahydrate (4 mg, 0.015 mmol, 0.2 eq, in 100 uL water) and sodium ascorbate (3.0 mg, 0.015 mmol, 0.2 eq, in 100 uL water) were added. After the addition was complete, the reaction mixture was stirred at room temperature under a nitrogen atmosphere for 2 hours. After LC-MS monitoring indicated completion, methyl tert-butyl ether/ethyl acetate (200 mL, 9:1) was added to the reaction mixture. The resulting suspension was filtered directly, washed with methyl tert-butyl ether, and the filter cake was dried directly to afford compound A164-12 (230 mg, 89%) as a yellow solid.
LCMS: [M+H]⁺ = 1683.0 (half peak)

### Synthesis of Intermediate Compound A164-13

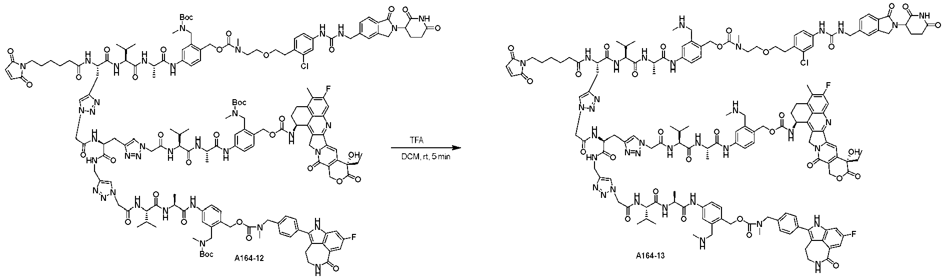

Compound A164-12 (230 mg, 0.068 mmol, 1.0 eq) was dissolved in DCM (3 mL). Trifluoroacetic acid (1 mL) was added to the reaction mixture, which was then stirred at room temperature for 5 minutes. After LC-MS monitoring indicated completion, the organic phase was concentrated to give the crude product compound A164-13, which was used directly in the next step.
LCMS: [M+H]⁺ = 1531.1 (half peak)

### Synthesis of Target Compound A164

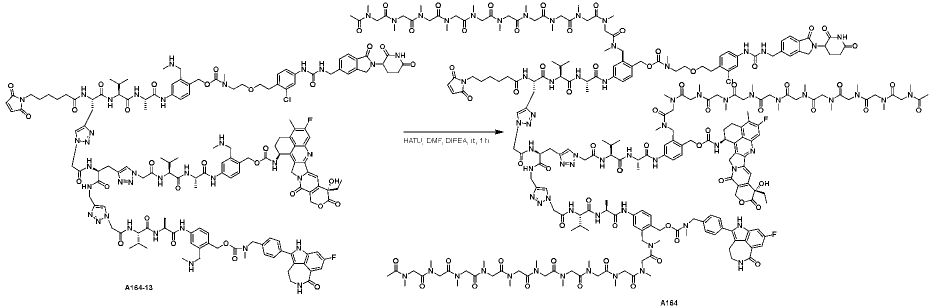

The compound A164-13 from the previous step was dissolved in DMF (4 mL). Ac-PSAR10-OH (157 mg, 0.204 mmol, 3.0 eq) and N,N-Diisopropylethylamine (265 mg, 2.049 mmol, 30.0 eq) were added to the reaction mixture. Finally, under a nitrogen atmosphere, O-(7-Azabenzotriazol-1-yl)-N,N,N',N'-tetramethyluronium hexafluorophosphate (86 mg, 0.225 mmol, 3.3 eq) was added. After the addition was complete, the reaction mixture was stirred at room temperature under a nitrogen atmosphere for 1 hour. After LC-MS monitoring indicated completion, ethyl acetate (60 mL) was added to the reaction mixture. The resulting suspension was filtered, and the filter cake was dissolved in DMF and purified by prep-HPLC (mobile phase: 0.05% HCOOH/CH₃CN/H₂O) to afford compound A164 (80 mg, 22%) as a yellow solid. LCMS: [M+H]⁺ = 1773.8 (one-third peak)

### (69) Synthesis of Compound A165

### Synthetic Route:

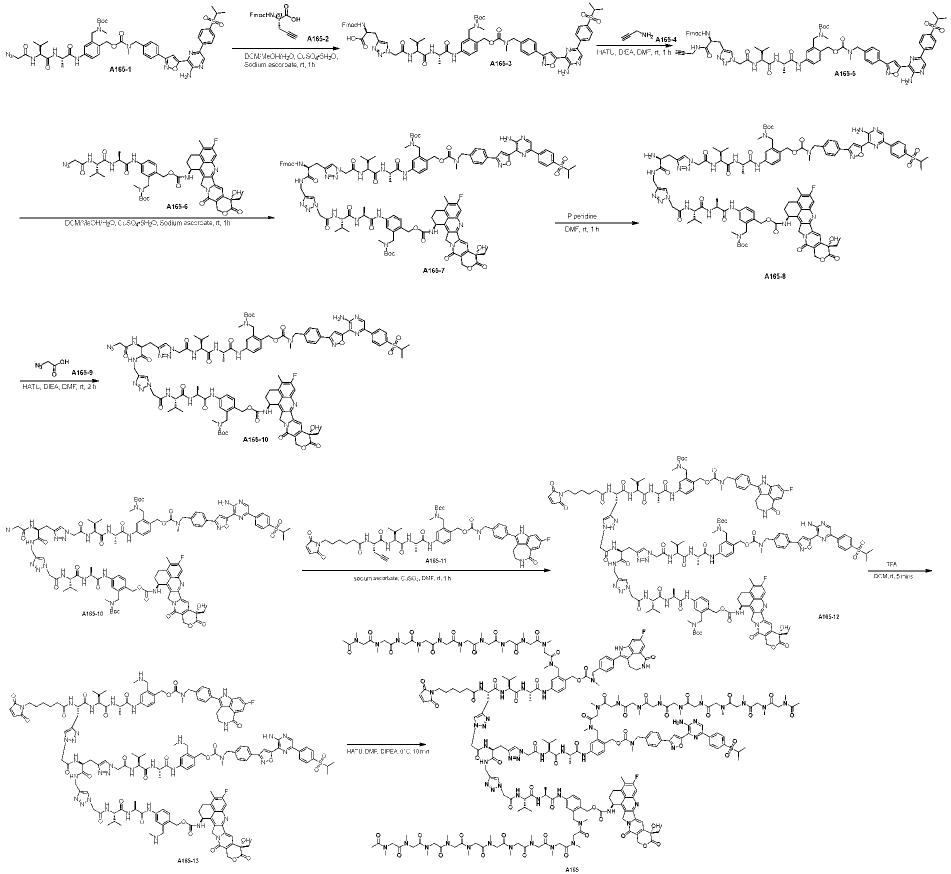

### Synthetic Steps:

### Synthesis of Intermediate Compound A165-3 (ENBW230543-051)

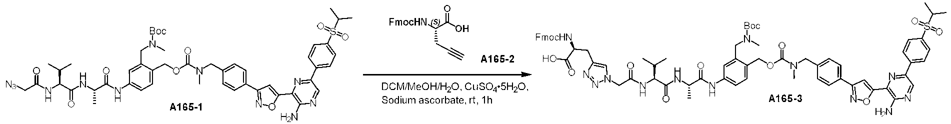

Compound **A165-1** (3 g, 2.970 mmol, 1.0 eq) and **A165-2** (1.01 g, 2.970 mmol, 1.0 eq) were dissolved in dichloromethane/methanol (30/30 mL). Under a nitrogen atmosphere, copper(II) sulfate pentahydrate (149 mg, 0.594 mmol, 0.2 eq, in 2 mL water) and sodium ascorbate (235 mg, 1.188 mmol, 0.4 eq, in 2 mL water) were added. After the addition was complete, the reaction mixture was stirred at room temperature under a nitrogen atmosphere for 1 hour. After LC-MS monitoring indicated completion, the reaction mixture was concentrated directly. Tetrahydrofuran was added, and the mixture was concentrated again to dryness. The crude product was slurried with ethyl acetate, then filtered and dried to afford compound **A165-3** (3.4 g, 85%) as a yellow solid.
LCMS: [M+Na]⁺ = 1366.4

### Synthesis of Intermediate Compound A165-5

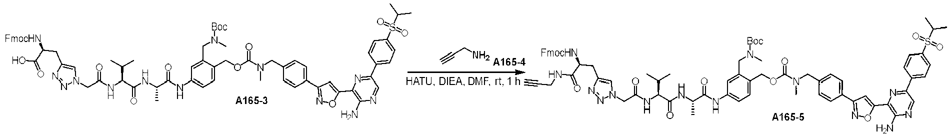

Compound **A165-3** (450 mg, 0.334 mmol, 1.0 eq) was dissolved in DMF (5 mL). Compound **A165-4** (28 mg, 0.502 mmol, 1.5 eq) and N,N-Diisopropylethylamine (131 mg, 1.003 mmol, 3.0 eq) were added to the reaction mixture. Finally, under a nitrogen atmosphere, O-(7-Azabenzotriazol-1-yl)-N,N,N',N'-tetramethyluronium hexafluorophosphate (166 mg, 0.435 mmol, 1.3 eq) was added. After the addition was complete, the reaction mixture was stirred at room temperature under a nitrogen atmosphere for 1 hour. After LC-MS monitoring indicated completion, the reaction mixture was diluted with 2-methyltetrahydrofuran and washed twice with water. The organic phase was dried over anhydrous sodium sulfate, filtered, and concentrated. The crude product was slurried with ethyl acetate, then filtered directly, and the filter cake was collected and dried to afford compound **A165-5** (420 mg, 91%) as a yellow solid.
LCMS: [M+Na]⁺ = 1403.4

### Synthesis of Intermediate Compound A165-7

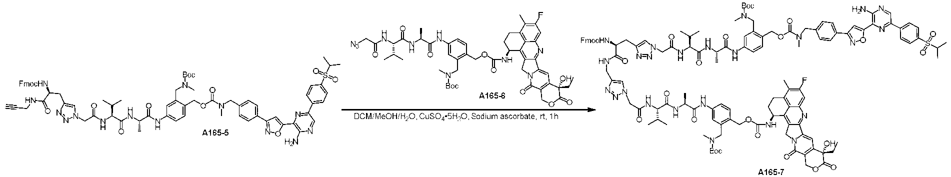

Compound **A165-5** (400 mg, 0.289 mmol, 1.0 eq) and compound **A165-6** (285 mg, 0.289 mmol, 1.0 eq) were dissolved in dichloromethane/methanol (5/5 mL). Under a nitrogen atmosphere, copper(II) sulfate pentahydrate (14.5 mg, 0.0579 mmol, 0.2 eq, in 200 uL water) and sodium ascorbate (22.9 mg, 0.116 mmol, 0.4 eq, in 200 uL water) were added. After the addition was complete, the reaction mixture was stirred at room temperature under a nitrogen atmosphere for 1 hour. After LC-MS monitoring indicated completion, the reaction mixture was concentrated directly. The residue was dissolved in tetrahydrofuran and concentrated again to dryness. The crude product was slurried with ethyl acetate, filtered, and dried to afford compound **A165-7** (500 mg, 73%) as a yellow solid.
LCMS: [M+Na]⁺ = 1203.8 (half peak)

### Synthesis of Intermediate Compound A165-8

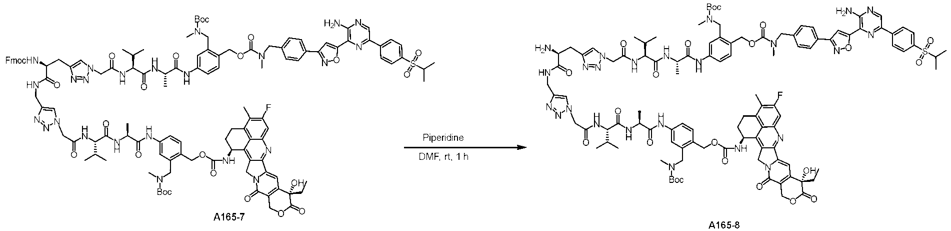

Compound **A165-7** (500 mg, 0.212 mmol, 1.0 eq) was dissolved in DMF (5 mL). Under a nitrogen atmosphere, piperidine (1 mL) was added. The reaction mixture was stirred at room temperature under a nitrogen atmosphere for 1 hour. After LC-MS monitoring indicated completion, methyl tert-butyl ether (100 mL) was added to the reaction mixture. The suspension was filtered directly, washed with methyl tert-butyl ether, and the filter cake was dried directly to afford compound **A165-8** (430 mg, 95%) as a yellow solid.
LCMS: [M+H]⁺ = 1070.9 (half peak)

### Synthesis of Intermediate Compound A165-10

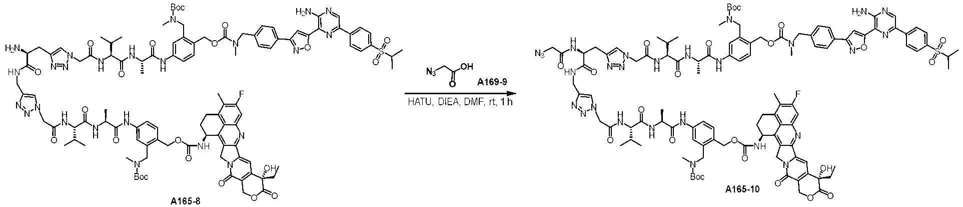

Compound **A165-8** (420 mg, 0.196 mmol, 1.0 eq) was dissolved in DMF (5 mL). N,N-Diisopropylethylamine (76 mg, 0.588 mmol, 3.0 eq) and **A165-9** (32 mg, 0.314 mmol, 1.6 eq) were added to the reaction mixture. Finally, under a nitrogen atmosphere, O-(7-Azabenzotriazol-1-yl)-N,N,N',N'-tetramethyluronium hexafluorophosphate (127 mg, 0.333 mmol, 1.7 eq) was added. After the addition was complete, the reaction mixture was stirred at room temperature under a nitrogen atmosphere for 1 hour. After LC-MS monitoring indicated completion, methyl tert-butyl ether (100 mL) was added to the reaction mixture. The suspension was filtered directly, washed with methyl tert-butyl ether, and the filter cake was dried. It was then slurried again with methanol, filtered, and dried to afford compound **A165-10** (420 mg, 96%) as a yellow solid.
LCMS: [M+H]⁺ = 1112.3 (half peak)

### Synthesis of Intermediate Compound A165-12

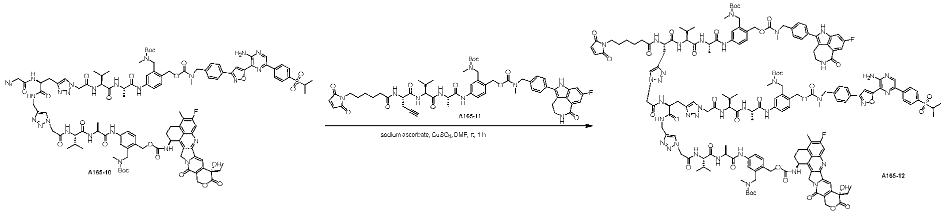

Compound **A165-10** (207 mg, 0.093 mmol, 1.0 eq) and compound **A165-11** (100 mg, 0.093 mmol, 1.0 eq) were dissolved in DMF (4 mL). Under a nitrogen atmosphere, copper(II) sulfate pentahydrate (2.3 mg, 0.0093 mmol, 0.1 eq, in 100 uL water) and sodium ascorbate (7.4 mg, 0.0372 mmol, 0.4 eq, in 100 uL water) were added. After the addition was complete, the reaction mixture was stirred at room temperature under a nitrogen atmosphere for 1 hour. After LC-MS monitoring indicated completion, the reaction mixture was diluted with ethyl acetate. The resulting suspension was filtered directly, washed with ethyl acetate, and the filter cake was dried directly to afford compound **A165-12** (280 mg, 91%) as a yellow solid.
LCMS: [M+H]⁺ = 1649.4 (half peak)

### Synthesis of Intermediate Compound A165-13

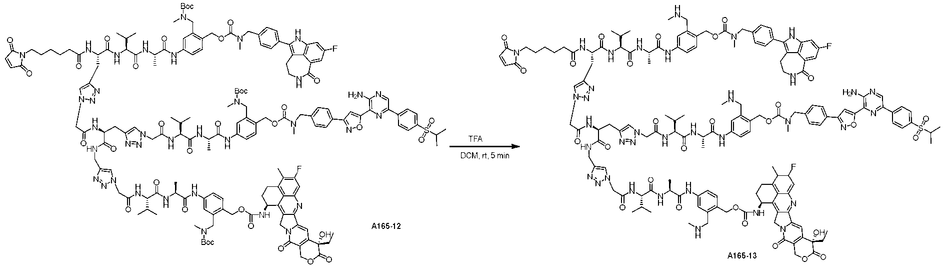

Compound **A165-12** (260 mg, 0.0788 mmol, 1.0 eq) was dissolved in DCM (3 mL). Trifluoroacetic acid (1 mL) was added to the reaction mixture, which was then stirred at room temperature for 5 minutes. After LC-MS monitoring indicated completion, the organic phase was concentrated to give the crude product compound **A165-13,** which was used directly in the next step.
LCMS: [M+H]⁺ = 1499.1 (half peak)

### Synthesis of Target Compound A165

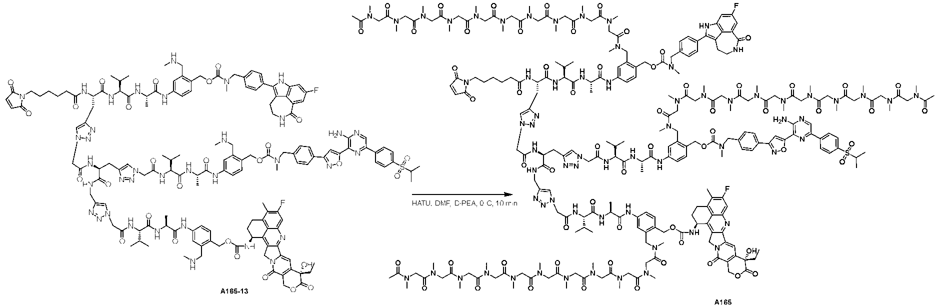

The compound **A165-13** from the previous step was dissolved in DMF (4 mL). **Ac-PSAR10-OH** (182 mg, 0.236 mmol, 3.0 eq) was added to the reaction mixture. Finally, under a nitrogen atmosphere, N,N-Diisopropylethylamine (305 mg, 2.364 mmol, 30.0 eq) and O-(7-Azabenzotriazol-1-yl)-N,N,N',N'-tetramethyluronium hexafluorophosphate (99 mg, 0.260 mmol, 3.3 eq) were added. After the addition was complete, the reaction mixture was stirred at 0 °C under a nitrogen atmosphere for 10 minutes. After LC-MS monitoring indicated completion, ethyl acetate (60 mL) was added to the reaction mixture. The resulting suspension was filtered, and the filter cake was dissolved in DMF and purified by prep-HPLC (mobile phase: 0.05% HCOOH/CH₃CN/H₂O) to afford compound **A165** (42.5 mg, 10%) as a yellow solid.
LCMS: [M+H]⁺ = 1752.3 (one-third peak)

### (70) Synthesis of Compound A166

### Synthetic Route:

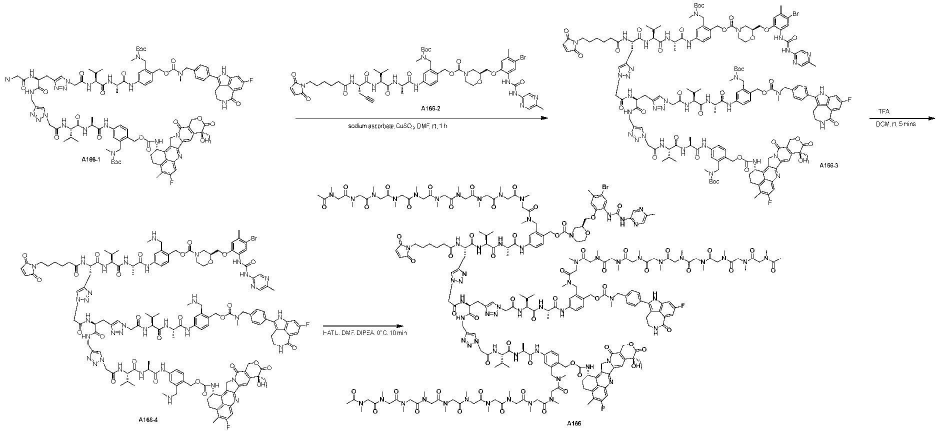

### Synthetic Steps:

### Synthesis of Intermediate Compound A166-3

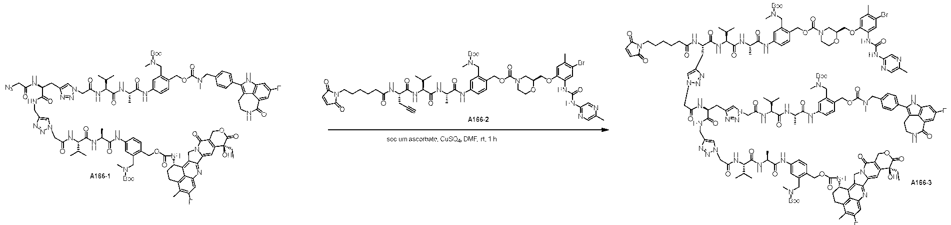

Compound **A166-1** (474 mg, 0.227 mmol, 1.0 eq) and compound **A166-2** (270 mg, 0.227 mmol, 1.0 eq) were dissolved in DMF (4 mL). Under a nitrogen atmosphere, copper(II) sulfate pentahydrate (5.7 mg, 0.0227 mmol, 0.1 eq, in 100 uL water) and sodium ascorbate (18 mg, 0.0909 mmol, 0.4 eq, in 100 uL water) were added. After the addition was complete, the reaction mixture was stirred at room temperature under a nitrogen atmosphere for 1 hour. After LC-MS monitoring indicated completion, the reaction mixture was diluted with ethyl acetate. The resulting suspension was filtered directly, washed with ethyl acetate, and the filter cake was dried directly to afford compound **A166-3** (700 mg, 94%) as a yellow solid.
LCMS: [M+H]⁺ = 1634.9 (half peak)

### Synthesis of Intermediate Compound A166-4

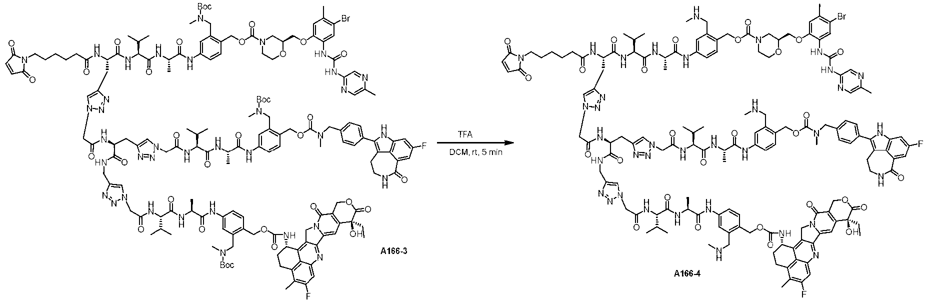

Compound **A166-3** (600 mg, 0.183 mmol, 1.0 eq) was dissolved in DCM (6 mL). Trifluoroacetic acid (2 mL) was added to the reaction mixture, which was then stirred at room temperature for 5 minutes. After LC-MS monitoring indicated completion, the organic phase was concentrated to give the crude product compound **A166-4,** which was used directly in the next step.
LCMS: [M+H]⁺ = 1485.4 (half peak)

### Synthesis of Target Compound A166

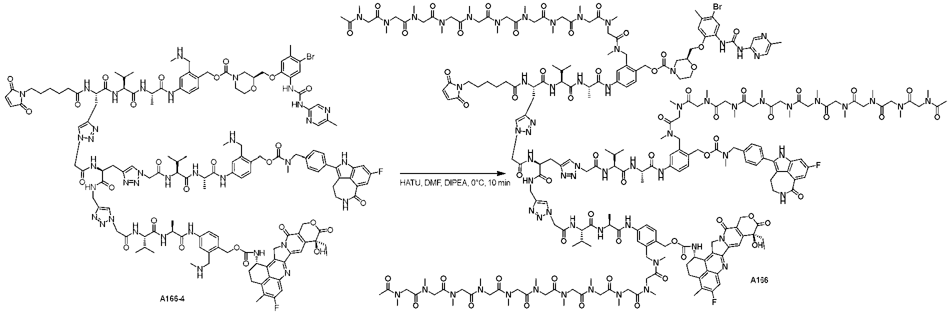

The compound **A166-4** from the previous step was dissolved in DMF (6 mL). **Ac-PSAR10-OH** (424 mg, 0.550 mmol, 3.0 eq) was added to the reaction mixture. Finally, under a nitrogen atmosphere, N,N-Diisopropylethylamine (710 mg, 5.499 mmol, 30.0 eq) and O-(7-Azabenzotriazol-1-yl)-N,N,N',N'-tetramethyluronium hexafluorophosphate (230 mg, 0.605 mmol, 3.3 eq) were added. After the addition was complete, the reaction mixture was stirred at 0 °C under a nitrogen atmosphere for 10 minutes. After LC-MS monitoring indicated completion, ethyl acetate (90 mL) was added to the reaction mixture. The resulting suspension was filtered, and the filter cake was dissolved in DMF and purified by prep-HPLC (mobile phase: 0.05% HCOOH/CH₃CN/H₂O) to afford compound **A166** (180 mg, 19%) as a white solid. LCMS: [M+H]⁺ = 1743.1 (one-third peak)

### (71) Synthesis of Compound A167

### Synthetic Route:

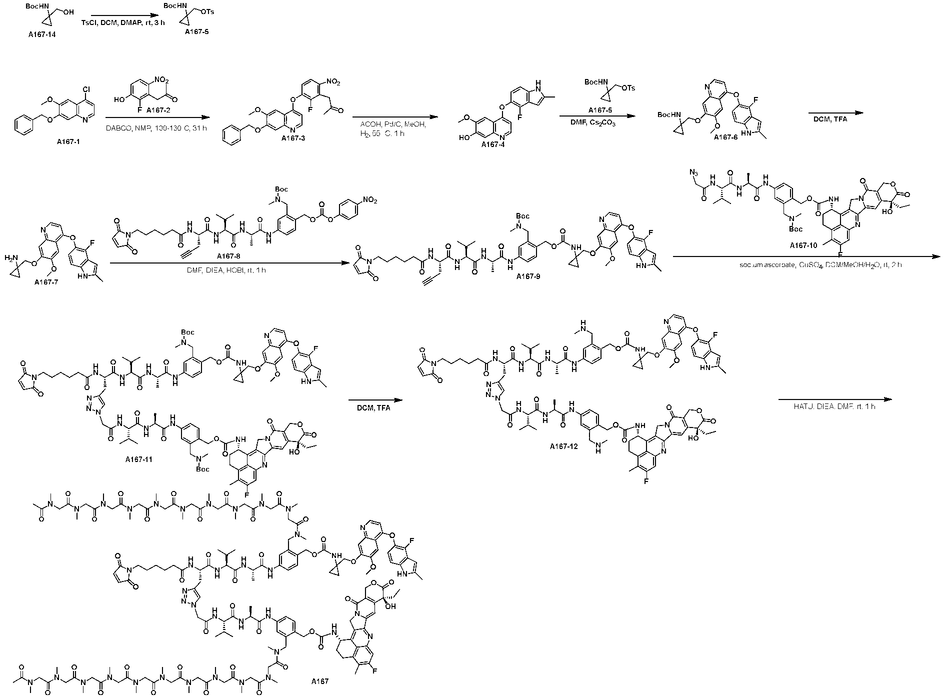

### Synthetic Steps:

### Synthesis of Intermediate Compound A167-5

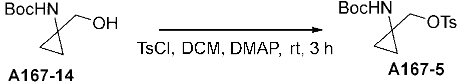

Compound **A167-14** (10 g, 0.053 mol, 1 eq) was dissolved in dichloromethane (200 mL). p-Toluenesulfonyl chloride (11.2 g, 0.059 mol, 1.1 eq) and 4-dimethylaminopyridine (7.8 g, 0.064 mol, 1.2 eq) were added, and the reaction was stirred at room temperature for 3 hours. After the reaction was complete, water was added for washing. The organic phase was concentrated under reduced pressure. The product obtained was **A167-5** (10 g, 52%) as a white solid. The product was used directly in the next step without purification.

LCMS: [M-100+H]⁺ = 242.2

### Synthesis of Intermediate Compound A167-3

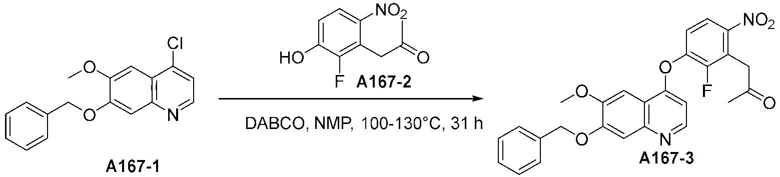

Compound **A167-1** (2 g, 6.672 mmol, 1.0 eq) and **A167-2** (1.42 g, 6.672 mmol, 1.0 eq) were dissolved in N-methylpyrrolidone (20 mL). DABCO (749 mg, 6.672 mmol, 1.0 eq) was added. After the addition was complete, the reaction mixture was stirred at 100 °C for 24 hours, then heated to 130 °C and stirred for 7 hours. After LC-MS monitoring indicated completion, the reaction mixture was concentrated directly. The residue was purified by silica gel column chromatography (PE/EA=1/1) to afford compound **A167-3** (1.6 g, 50%) as a brown solid.

LCMS: [M+H]⁺ = 477.1

### Synthesis of Intermediate Compound A167-4

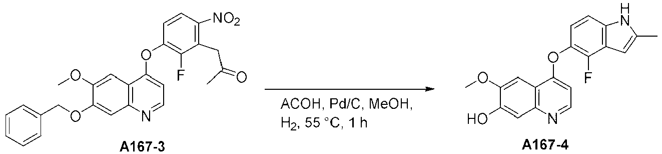

Compound **A167-3** (2.1 g, 0.004 mol, 1 eq) was dissolved in methanol (30 mL). Palladium on carbon (230 mg, 10%) and acetic acid (2.1 mL) were added. The reaction was stirred under a hydrogen atmosphere at 55 °C for 1 hour. After the reaction was complete, the mixture was cooled to room temperature. The reaction mixture was filtered, and the filtrate was concentrated under reduced pressure. Petroleum ether was added, and the product precipitated. Filtration afforded **A167-4** (0.6 g, 39%) as a red solid.

LCMS: [M+H]⁺ = 339.1

### Synthesis of Intermediate Compound A167-6

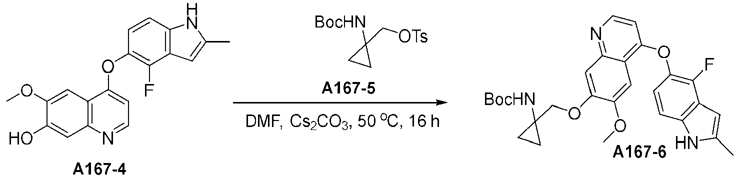

Compound **A167-4** (600 mg, 1.773 mmol, 1 eq) was dissolved in N,N-dimethylformamide (10 mL). **A167-5** (908 mg, 2.66 mmol, 1.5 eq) and cesium carbonate (1733 mg, 5.32 mmol, 3 eq) were added, and the reaction was stirred at 50 °C for 16 hours. After the reaction was complete, the mixture was cooled to room temperature. The reaction mixture was filtered, and the filtrate was concentrated under reduced pressure. The residue was purified by silica gel column chromatography (petroleum ether/ethyl acetate = 3/1) to afford **A167-6** (265 mg, 29%) as a yellow solid.

LCMS: [M+H]⁺ = 508.2

### Synthesis of Intermediate Compound A167-7

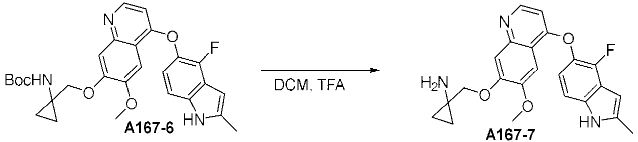

Compound **A167-6** (265 mg, 0.522 mmol, 1 eq) was dissolved in dichloromethane (6 mL). Trifluoroacetic acid (2 mL) was added, and the reaction was stirred at room temperature for 1 hour. After the reaction was complete, the mixture was concentrated under reduced pressure and dried under an oil pump to afford **A167-7** (160 mg, 75%) as a yellow solid.

LCMS: [M+H]⁺ = 408.1

### Synthesis of Intermediate Compound A167-9

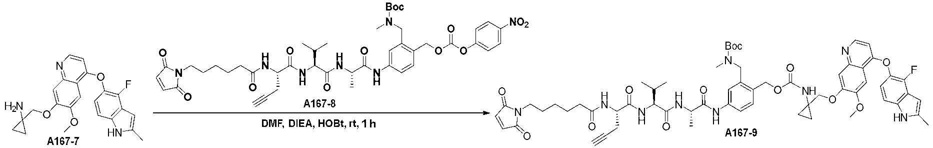

Compound **A167-7** (160 mg, 0.315 mmol, 1 eq) was dissolved in N,N-dimethylformamide (4 mL). **A167-8** (252 mg, 0.284 mmol, 0.9 eq), N,N-Diisopropylethylamine (122 mg, 0.946 mmol, 3 eq), and 1-hydroxybenzotriazole (21 mg, 0.158 mmol, 0.5 eq) were added. The reaction was stirred at room temperature for 1 hour. After the reaction was complete, ethyl acetate (40 mL) was added. The mixture was washed twice with water and twice with saturated brine. The organic phase was collected, dried, filtered, and concentrated. The residue was purified by silica gel column chromatography (dichloromethane/methanol = 93/7) to afford **A167-9** (235 mg, 61%) as a yellow solid.

LCMS: [M+H]⁺ = 1159.4

### Synthesis of Intermediate Compound A167-11

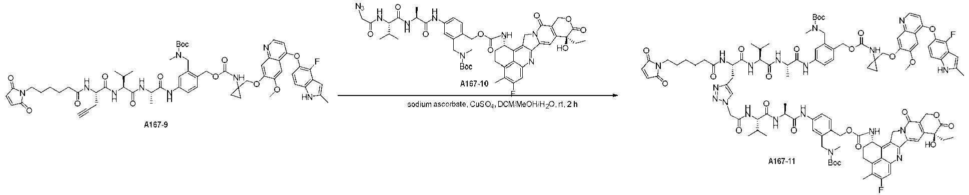

Compound **A167-9** (235 mg, 0.203 mmol, 1 eq) was dissolved in dichloromethane/methanol (4 mL, 1:1). **A167-10** (199 mg, 0.203 mmol, 1 eq), 100 uL of an aqueous solution of copper(II) sulfate pentahydrate (10 mg, 0.04 mmol, 0.2 eq), and 100 uL of an aqueous solution of sodium ascorbate (16 mg, 0.08 mmol, 0.4 eq) were added. The reaction was stirred under a nitrogen atmosphere at room temperature for 2 hours. After the reaction was complete, concentration under reduced pressure afforded the crude product compound **A167-11** (400 mg, 100 %) as a yellow solid.

LCMS: [M+H]⁺ = 1070.5 (half peak)

### Synthesis of Intermediate Compound A167-12

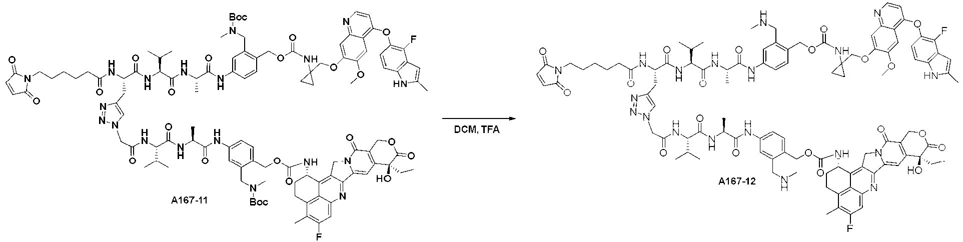

Compound **A167-11** (400 mg, 0.187 mmol, 1 eq) was dissolved in dichloromethane (3 mL). Trifluoroacetic acid (1 mL) was added, and the reaction was stirred at room temperature for 1 hour. After the reaction was complete, concentration under reduced pressure afforded compound **A167-12** (crude) as a yellow solid.

LCMS: [M+H]⁺ = 970.0 (half peak)

### Synthesis of Target Compound A167

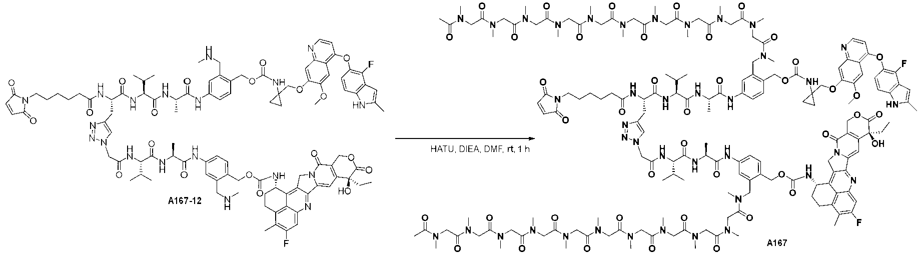

The crude compound **A167-12** was dissolved in N,N-dimethylformamide (2 mL). **Ac-PSAR10-OH** (302 mg, 0.392 mmol, 2.1 eq) was added, and the reaction mixture was cooled to 0 °C. N,N-Diisopropylethylamine (483 mg, 3.736 mmol, 20 eq) and HATU (156 mg, 0.411 mmol, 2.2 eq) were added. The reaction was stirred at room temperature for 1 hour. After the reaction was complete, ethyl acetate (40 mL) was added, and the product precipitated. Filtration afforded the crude target compound. The crude product was dissolved in DMF and purified by prep-HPLC (mobile phase: 0.05% HCOOH/CH₃CN/H₂O) to afford compound **A167** (21 mg, 6%) as a white solid.

LCMS: [M+H]⁺ = 1722.7 (half peak)

### (72) Synthesis of Compound A169

### Synthetic Route:

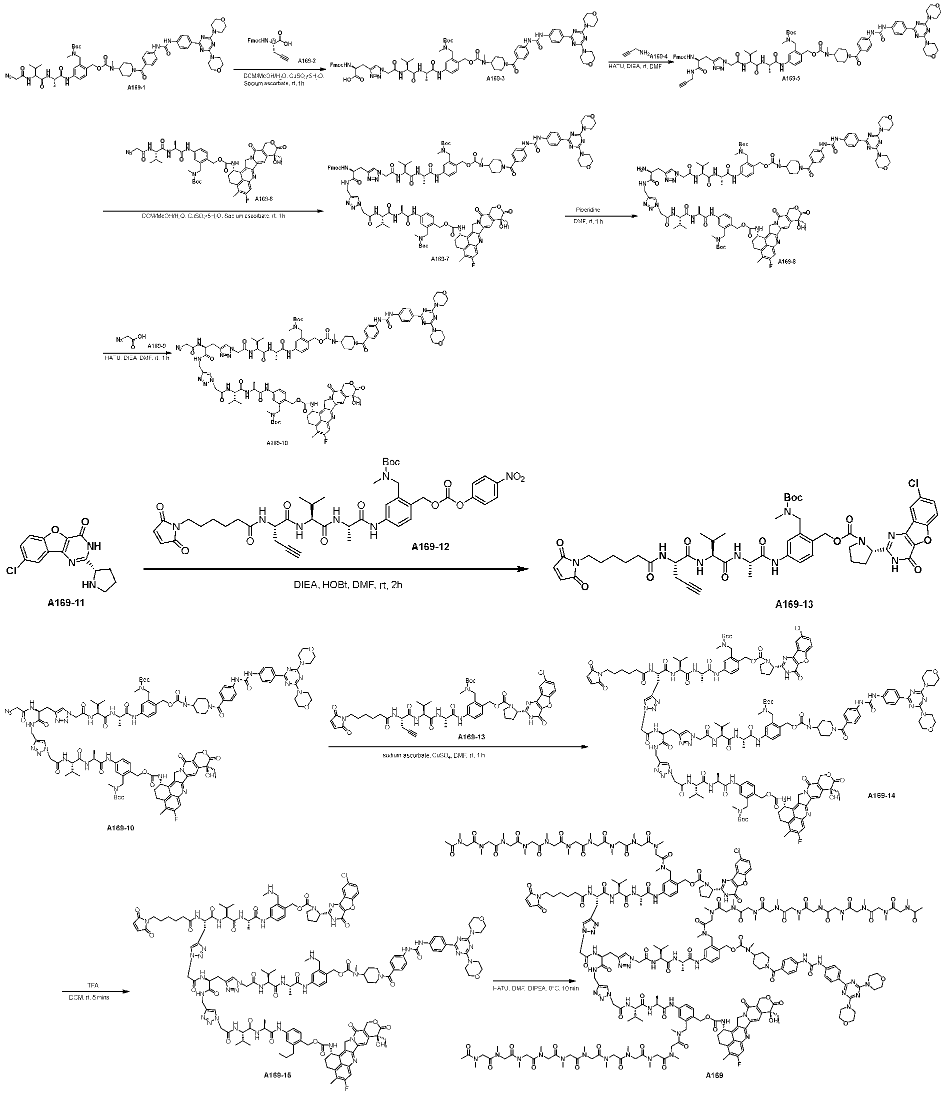

### Synthetic Steps:

### Synthesis of Intermediate Compound A169-3

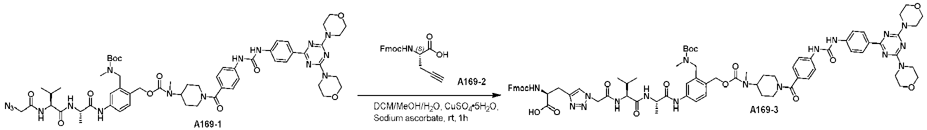

Compound **A169-1** (400 mg, 0.348 mmol, 1.0 eq) and **A169-2** (117 mg, 0.348 mmol, 1.0 eq) were dissolved in dichloromethane/methanol (5/5 mL). Under a nitrogen atmosphere, copper(II) sulfate pentahydrate (17.4 mg, 0.163 mmol, 0.2 eq, in 300 uL water) and sodium ascorbate (27.6 mg, 0.139 mmol, 0.4 eq, in 300 uL water) were added. After the addition was complete, the reaction mixture was stirred at room temperature under a nitrogen atmosphere for 1 hour. After LC-MS monitoring indicated completion, the reaction mixture was concentrated directly. Tetrahydrofuran was added, and the mixture was concentrated again to dryness. The crude product was slurried with ethyl acetate, then filtered and dried to afford compound **A169-3** (500 mg, 97%) as a white solid.
LCMS: [M+Na]⁺ = 741.8 (half peak)

### Synthesis of Intermediate Compound A169-5

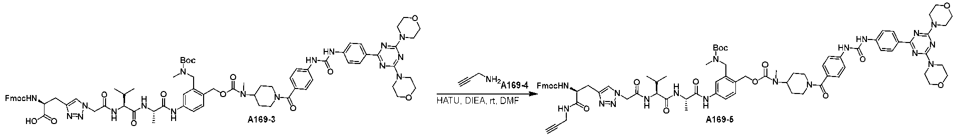

Compound **A169-3** (500 mg, 0.337 mmol, 1.0 eq) was dissolved in DMF (6 mL). Compound **A169-4** (37 mg, 0.674 mmol, 2.0 eq) and N,N-Diisopropylethylamine (131 mg, 1.011 mmol, 3.0 eq) were added to the reaction mixture. Finally, under a nitrogen atmosphere, O-(7-Azabenzotriazol-1-yl)-N,N,N',N'-tetramethyluronium hexafluorophosphate (167 mg, 0.438 mmol, 1.3 eq) was added. After the addition was complete, the reaction mixture was stirred at room temperature under a nitrogen atmosphere for 1 hour. After LC-MS monitoring indicated completion, the reaction mixture was diluted with 2-methyltetrahydrofuran and washed twice with water. The organic phase was dried over anhydrous sodium sulfate, filtered, and concentrated. The crude product was slurried with ethyl acetate, then filtered directly, and the filter cake was collected and dried to afford compound **A169-5** (400 mg, 78%) as a grey solid.
LCMS: [M+Na]⁺ = 1520.5

### Synthesis of Intermediate Compound A169-7

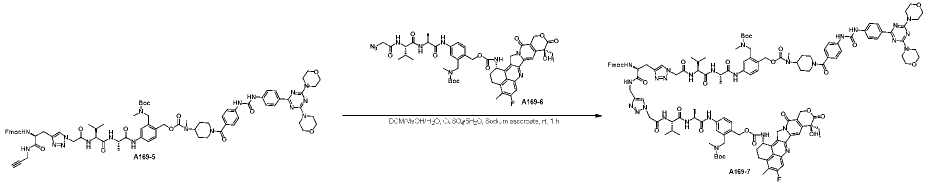

Compound **A169-5** (400 mg, 0.263 mmol, 1.0 eq) and compound **A169-6** (258 mg, 0.263 mmol, 1.0 eq) were dissolved in dichloromethane/methanol (4/4 mL). Under a nitrogen atmosphere, copper(II) sulfate pentahydrate (13.2 mg, 0.0526 mmol, 0.2 eq, in 200 uL water) and sodium ascorbate (20.8 mg, 0.105 mmol, 0.4 eq, in 200 uL water) were added. After the addition was complete, the reaction mixture was stirred at room temperature under a nitrogen atmosphere for 1 hour. After LC-MS monitoring indicated completion, the reaction mixture was concentrated directly. The residue was dissolved in tetrahydrofuran and concentrated again to dryness. The crude product was slurried with ethyl acetate, filtered, and dried to afford compound **A169-7** (500 mg, 76%) as a grey solid.
LCMS: [M+H]⁺ = 1250.9 (half peak)

### Synthesis of Intermediate Compound A169-8

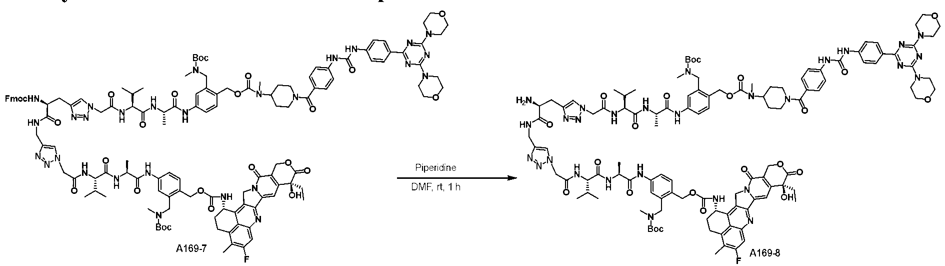

Compound **A169-7** (500 mg, 0.200 mmol, 1.0 eq) was dissolved in DMF (8 mL). Under a nitrogen atmosphere, piperidine (1.6 mL) was added. The reaction mixture was stirred at room temperature under a nitrogen atmosphere for 1 hour. After LC-MS monitoring indicated completion, methyl tert-butyl ether (100 mL) was added to the reaction mixture. The suspension was filtered directly, washed with methyl tert-butyl ether, and the filter cake was dried directly to afford compound **A169-8** (420 mg, 92%) as a yellow solid.
LCMS: [M+H]⁺ = 1139.9 (half peak)

### Synthesis of Intermediate Compound A169-10

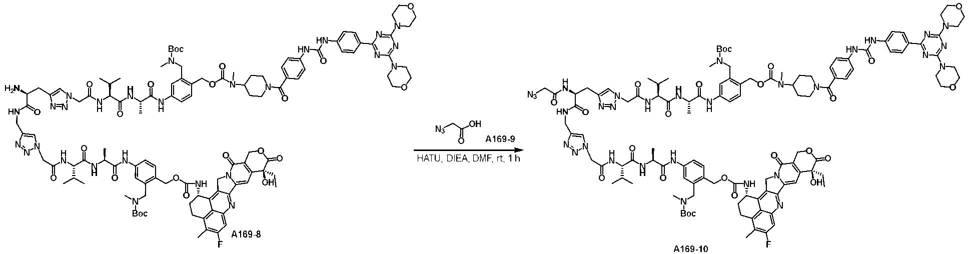

Compound **A169-8** (400 mg, 0.175 mmol, 1.0 eq) was dissolved in DMF (5 mL). N,N-Diisopropylethylamine (68 mg, 0.526 mmol, 3.0 eq) and A169-9 (28 mg, 0.281 mmol, 1.6 eq) were added to the reaction mixture. Finally, under a nitrogen atmosphere, O-(7-Azabenzotriazol-1-yl)-N,N,N',N'-tetramethyluronium hexafluorophosphate (113 mg, 0.298 mmol, 1.7 eq) was added. After the addition was complete, the reaction mixture was stirred at room temperature under a nitrogen atmosphere for 1 hour. After LC-MS monitoring indicated completion, methyl tert-butyl ether (100 mL) was added to the reaction mixture. The suspension was filtered directly, washed with methyl tert-butyl ether, and the filter cake was dried. It was then slurried with ethyl acetate, filtered, and dried to afford compound **A169-10** (649 mg, 82%) as a yellow solid.
LCMS: [M+H]⁺ = 1181.4 (half peak)

### Synthesis of Compound A169-13

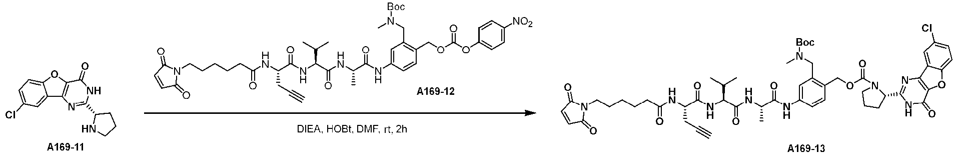

Compound **A169-11** (230 mg, 0.794 mmol, 1.0 eq) was dissolved in DMF (5 mL). N,N-Diisopropylethylamine (308 mg, 2.382 mmol, 3.0 eq) was added and stirred until clear. Then compound **A169-12** (707 mg, 0.794 mmol, 1.0 eq) and HOBt (54 mg, 0.397 mmol, 0.5 eq) were added. After the addition was complete, the reaction mixture was stirred at room temperature for 2 hours. After LC-MS monitoring indicated completion, the reaction mixture was diluted with ethyl acetate, washed with water and saturated brine. The organic phase was dried over anhydrous sodium sulfate, filtered, and concentrated. The residue was purified by silica gel column chromatography (DCM/MeOH=93/7) to afford compound **A169-13** (320 mg, 39%) as a yellow solid.
LCMS: [M+H]⁺ = 1040.3

### Synthesis of Intermediate Compound A169-14

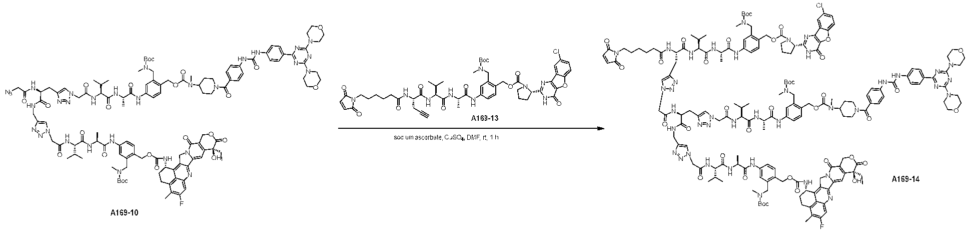

Compound **A169-10** (227 mg, 0.096 mmol, 1.0 eq) and compound **A169-13** (100 mg, 0.096 mmol, 1.0 eq) were dissolved in DMF (4 mL). Under a nitrogen atmosphere, copper(II) sulfate pentahydrate (4.8 mg, 0.0192 mmol, 0.2 eq, in 100 uL water) and sodium ascorbate (3.8 mg, 0.0192 mmol, 0.2 eq, in 100 uL water) were added. After the addition was complete, the reaction mixture was stirred at room temperature under a nitrogen atmosphere for 1 hour. After LC-MS monitoring indicated completion, the reaction mixture was diluted with ethyl acetate. The resulting suspension was filtered directly, washed with ethyl acetate, and the filter cake was dried directly to afford compound **A169-14** (310 mg, 95%) as a yellow solid.
LCMS: [M+H]⁺ = 1702.2 (half peak)

### Synthesis of Intermediate Compound A169-15

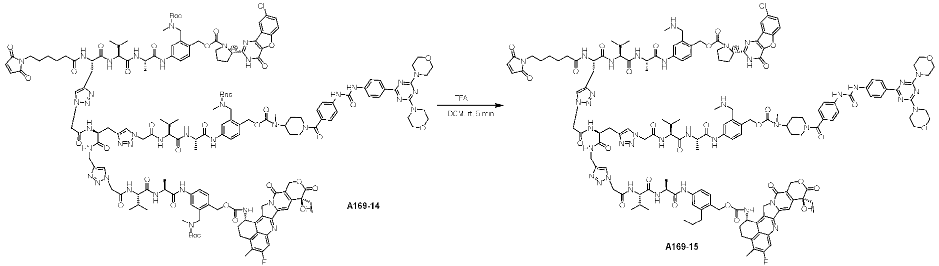

Compound **A169-14** (300 mg, 0.088 mmol, 1.0 eq) was dissolved in DCM (3 mL). Trifluoroacetic acid (1 mL) was added to the reaction mixture, which was then stirred at room temperature for 5 minutes. After LC-MS monitoring indicated completion, the organic phase was concentrated to give the crude product compound **A169-15,** which was used directly in the next step.
LCMS: [M+H]⁺ = 1551.9 (half peak)

### Synthesis of Target Compound A169

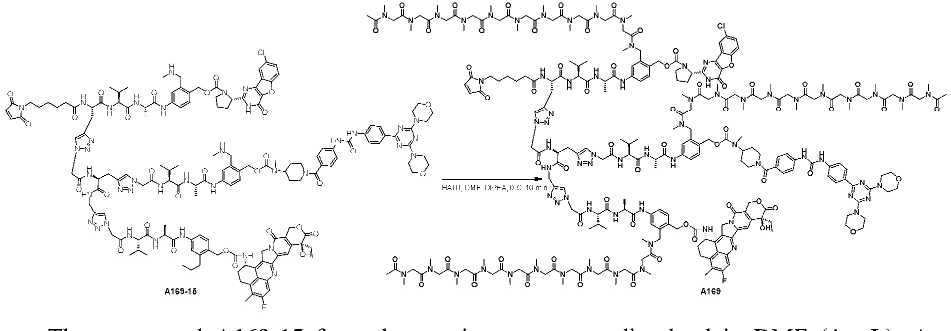

the compound **A169-15** trom the previous step was dissolved in DMF (4 mL). **Ac-PSAR10-OH** (204 mg, 0.264 mmol, 3.0 eq) was added to the reaction mixture. Finally, under a nitrogen atmosphere, N,N-Diisopropylethylamine (342 mg, 2.64 mmol, 30.0 eq) and O-(7-Azabenzotriazol-1-yl)-N,N,N',N'-tetramethyluronium hexafluorophosphate (111 mg, 0.290 mmol, 3.3 eq) were added. After the addition was complete, the reaction mixture was stirred at 0 °C under a nitrogen atmosphere for 10 minutes. After LC-MS monitoring indicated completion, ethyl acetate (60 mL) was added to the reaction mixture. The resulting suspension was filtered, and the filter cake was dissolved in DMF and purified by prep-HPLC (mobile phase: 0.05% HCOOH/CH₃CN/H₂O) to afford compound **A169** (21.8 mg, 4.6%) as a white solid.
LCMS: [M+H]⁺ = 1787.2 (one-third peak)

### (73) Synthesis of Compound A158

### Synthetic Route:

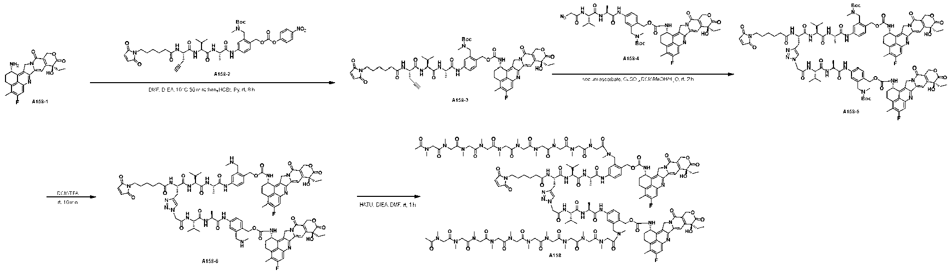

### Synthetic Steps:

### Synthesis of Intermediate Compound A158-3

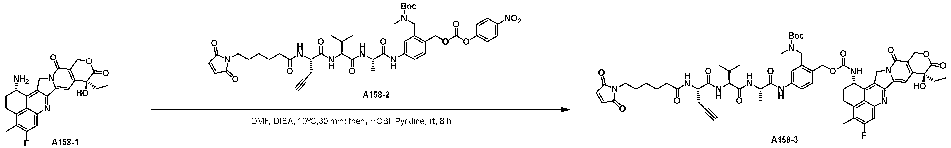

Compound A158-1 (650 mg, 1.49 mmol, 1.0 eq) was dissolved in DMF (12 mL). At 10 °C, N,N-Diisopropylethylamine (193 mg, 1.49 mmol, 1.0 eq) was added and stirred for 30 minutes. Then compound A158-2 (1.2 g, 1.34 mmol, 0.9 eq), HOBt (20 mg, 0.15 mmol, 0.1 eq) and pyridine (236 mg, 2.99 mol, 2.0 eq) were added. After the addition was complete, the reaction mixture was stirred at room temperature for 8 hours. After LC-MS monitoring indicated completion, the reaction mixture was diluted with 2-methyltetrahydrofuran (100 mL) and washed with water (50 mL). The organic phase was then washed sequentially with 0.5 M hydrochloric acid aqueous solution (50 mL x 2) and water (50 mL x 2), dried over anhydrous sodium sulfate, filtered, and concentrated. The residue was purified by silica gel column chromatography (DCM/MeOH=94/6) to afford compound A158-3 (750 mg, 42%) as a yellow solid.
LCMS: [M+H]⁺ = 1186.4

### Synthesis of Intermediate Compound A158-5

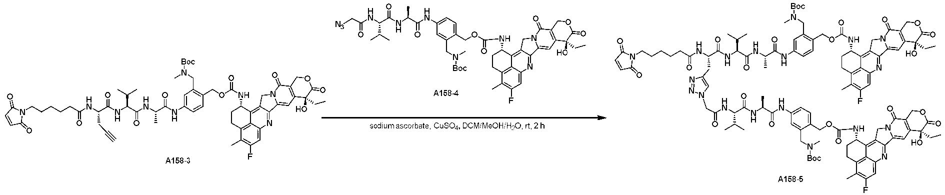

Compound A158-3 (140 mg, 0.118 mmol, 1.0 eq) and compound A158-4 (127 mg, 0.13 mmol, 1.0 eq) were dissolved in MeOH/DCM (2/2 mL). Then 100 uL of an aqueous solution of copper(II) sulfate pentahydrate (5.9 mg, 0.024 mmol, 0.2 eq) and 100 uL of an aqueous solution of sodium ascorbate (9.3 mg, 0.047 mmol, 0.4 eq) were added. After the addition was complete, the reaction mixture was stirred at room temperature under a nitrogen atmosphere for 2 hours. After LC-MS monitoring indicated completion, concentration afforded the crude product compound A158-5 (270 mg) as a brownish-yellow solid.
LCMS: [M+H]⁺ = 1084.3 (half peak)

### Synthesis of Intermediate Compound A158-6

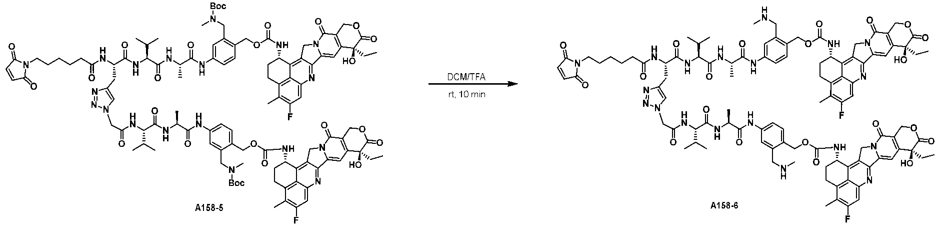

Compound A158-5 (260 mg, 0.12 mmol, 1.0 eq) was dissolved in DCM (4.5 mL). Trifluoroacetic acid (1.5 mL) was added to the reaction mixture, which was then stirred at room temperature for 10 minutes. After LC-MS monitoring indicated completion, the organic phase was concentrated to give the crude product compound A158-6, which was used directly in the next step.
LCMS: [M+H]⁺ = 984.0 (half peak)

### Synthesis of Target Compound A158

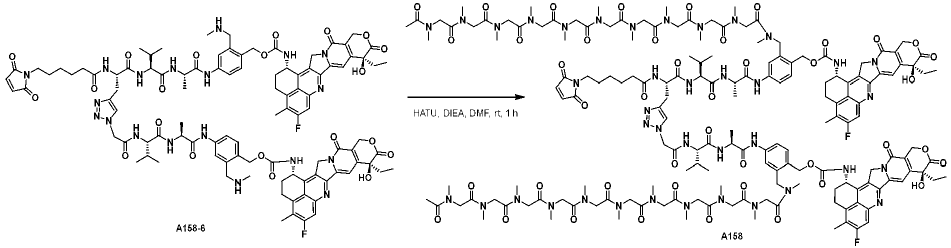

The compound A158-6 from the previous step was dissolved in DMF (3 mL). Compound Ac-PSAR10-OH (185 mg, 0.24 mmol, 2.0 eq) was added. Then at 0 °C, N,N-Diisopropylethylamine (465 mg, 3.60 mmol, 30.0 eq) and O-(7-Azabenzotriazol-1-yl)-N,N,N',N'-tetramethyluronium hexafluorophosphate (96 mg, 0.25 mmol, 2.1 eq) were added to the reaction mixture. After the addition was complete, the reaction mixture was stirred at 0 °C for 1 hour. After LC-MS monitoring indicated completion, EA (45 mL) was added to the reaction mixture. The reaction mixture was filtered, and the filter cake was dissolved in DMF and purified by prep-HPLC (mobile phase: 0.1% HCOOH/CH₃CN/H₂O) to afford compound A158 (38 mg, 9%) as a white solid.
LCMS: [M+H]⁺ = 1736.7 (half peak)

### (74) Synthesis of Compound A170

### Synthetic Route:

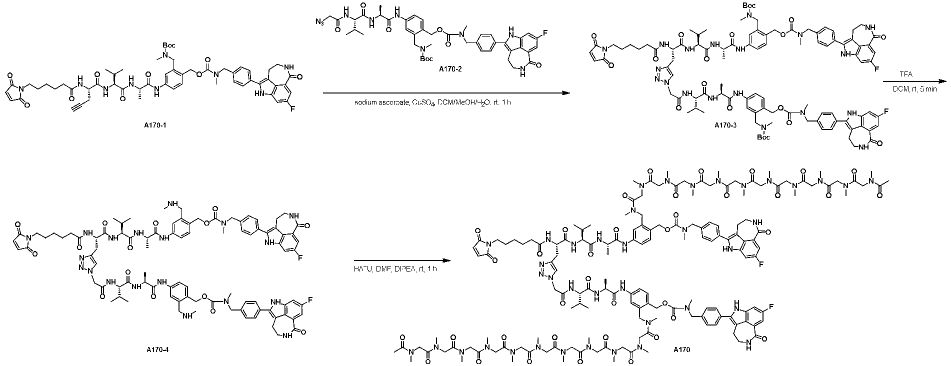

### Synthetic Steps:

### Synthesis of Intermediate Compound A170-3

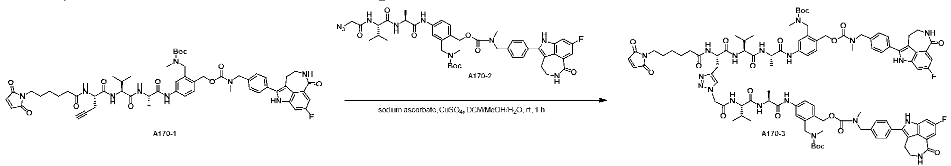

Compound **A170-1** (200 mg, 0.186 mmol, 1.0 eq) and compound **A170-2** (162 mg, 0.186 mmol, 1.0 eq) were dissolved in MeOH/DCM (4/4 mL). Then 100 uL of an aqueous solution of copper(II) sulfate pentahydrate (9.3 mg, 0.037 mmol, 0.2 eq) and 100 uL of an aqueous solution of sodium ascorbate (14.7 mg, 0.074 mmol, 0.4 eq) were added. After the addition was complete, the reaction mixture was stirred at room temperature under a nitrogen atmosphere for 1 hour. After LC-MS monitoring indicated completion, the organic phase was concentrated, and tetrahydrofuran was added and evaporated to remove water, affording the crude product compound **A170-3** (350 mg, 97%) as a yellow solid.
LCMS: [M+H]⁺ = 972.6 (half peak)

### Synthesis of Intermediate Compound A170-4

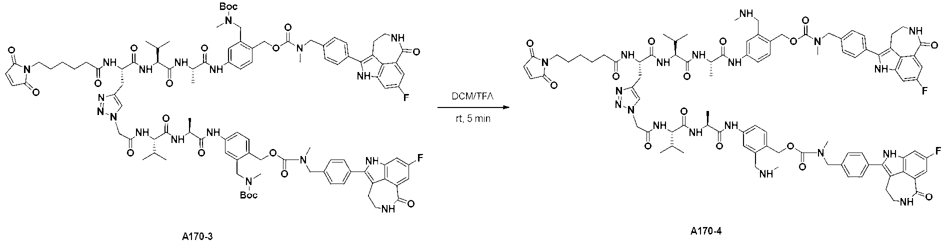

Compound **A170-3** (350 mg, 0.180 mmol, 1.0 eq) was dissolved in DCM (3 mL). Trifluoroacetic acid (1 mL) was added to the reaction mixture, which was then stirred at room temperature for 5 minutes. After LC-MS monitoring indicated completion, the organic phase was concentrated to give the crude product compound **A170-4,** which was used directly in the next step. LCMS: [M+H]⁺ = 872.0 (half peak)

### Synthesis of Target Compound A170

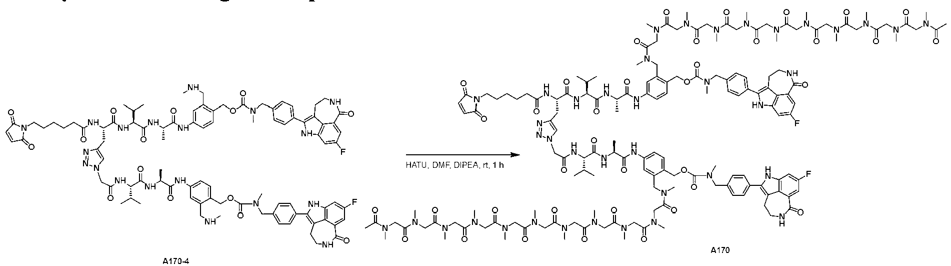

The compound **A170-4** from the previous step was dissolved in DMF (4 mL). **Ac-PSAR10-OH** (278 mg, 0.360 mmol, 2.0 eq) was added. Under an ice-water bath, N,N-Diisopropylethylamine (466 mg, 3.60 mmol, 20.0 eq) and O-(7-Azabenzotriazol-1-yl)-N,N,N',N'-tetramethyluronium hexafluorophosphate (144 mg, 0.378 mmol, 2.1 eq) were added to the reaction mixture. After the addition was complete, the reaction mixture was stirred at room temperature under a nitrogen atmosphere for 1 hour. After LC-MS monitoring indicated completion, EA (60 mL) was added to the reaction mixture. The reaction mixture was filtered, and the filter cake was dissolved in DMF and purified by prep-HPLC (mobile phase: 0.05% HCOOH/CH₃CN/H₂O) to afford compound A170 (28.9 mg, 5%) as a white solid. LCMS: [M+H]⁺ = 1624.8 (half peak)

### (75) Synthesis of Compound A171

### Synthetic Route:

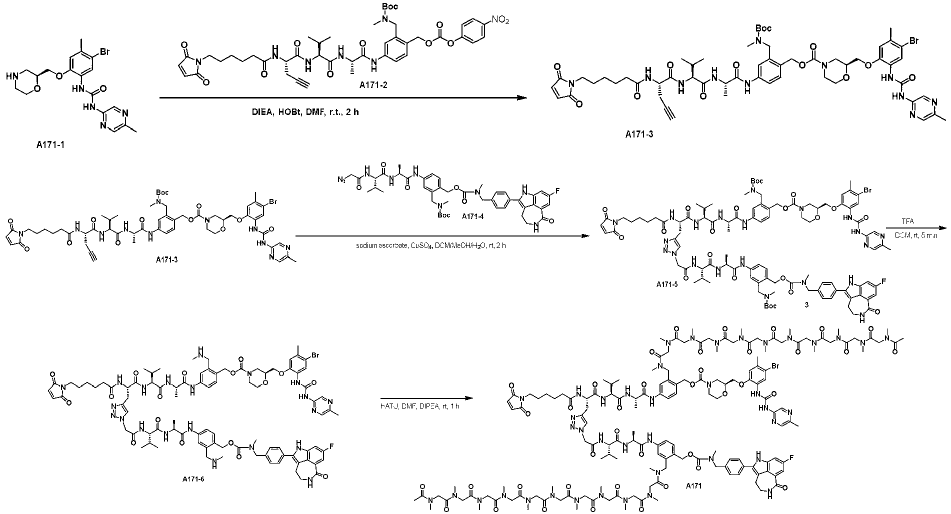

### Synthetic Steps:

### Synthesis of Intermediate Compound A171-3 (ENBW240201-08)

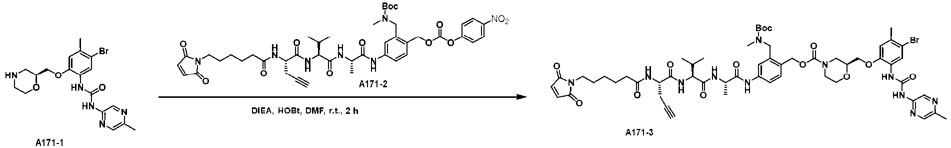

Compound **A171-1** (1 g, 2.23 mmol, 1.0 eq) and N,N-Diisopropylethylamine (865 mg, 6.69 mmol, 3.0 eq) were dissolved in DMF (10 mL). After stirring until clear, compound **A171-2** (1.84 g, 2.01 mmol, 0.90 eq) and HOBt (151 mg, 1.12 mmol, 0.5 eq) were added. After the addition was complete, the reaction mixture was stirred at room temperature for 2 hours. After LC-MS monitoring indicated completion, ethyl acetate (100 mL) was added to the reaction mixture. The mixture was washed twice with water and then with saturated brine. The obtained organic phase was dried over anhydrous sodium sulfate, filtered, and concentrated. The residue was purified by silica gel column chromatography (DCM/MeOH=92/8) to afford compound **A171-3** (1 g, 37%) as a white solid.
LCMS: [M+H]⁺ = 1186.3

### Synthesis of Intermediate Compound A171-5

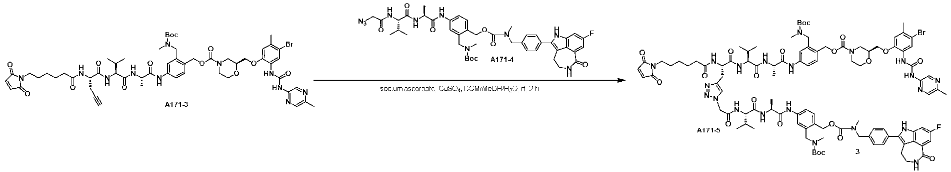

Compound **A171-3** (200 mg, 0.169 mmol, 1.0 eq) and compound **A171-4** (147 mg, 0.169 mmol, 1.0 eq) were dissolved in MeOH/DCM/water (5/5/1 mL). Then 100 uL of an aqueous solution of copper(II) sulfate pentahydrate (5.4 mg, 0.0338 mmol, 0.2 eq) and 100 uL of an aqueous solution of sodium ascorbate (13.4 mg, 0.0676 mmol, 0.4 eq) were added. After the addition was complete, the reaction mixture was stirred at room temperature under a nitrogen atmosphere for 2 hours. After LC-MS monitoring indicated completion, concentration of the organic phase afforded the crude product compound **A171-5** (347 mg, 99%) as a yellow solid. LCMS: [M+H]⁺ = 1028.5 (half peak)

### Synthesis of Intermediate Compound A171-6

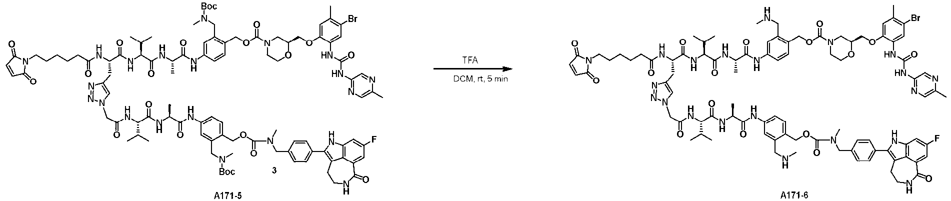

Compound **A171-5** (347 mg, 0.169 mmol, 1.0 eq) was dissolved in DCM (3 mL). Trifluoroacetic acid (1 mL) was added to the reaction mixture, which was then stirred at room temperature for 5 minutes. After LC-MS monitoring indicated completion, the organic phase was concentrated to give the crude product compound **A171-6,** which was used directly in the next step.
LCMS: [M+H]⁺ = 928.7 (half peak)

### Synthesis of Target Compound A171

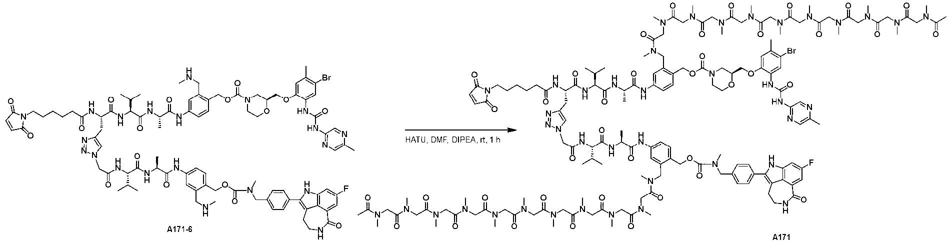

The compound **A171-6** from the previous step was dissolved in DMF (4 mL). N,N-Diisopropylethylamine (437 mg, 3.38 mmol, 20.0 eq) was added. Then **Ac-PSAR10-OH** (261 mg, 0.338 mmol, 2.0 eq) and O-(7-Azabenzotriazol-1-yl)-N,N,N',N'-tetramethyluronium hexafluorophosphate (135 mg, 0.355 mmol, 2.1 eq) were added to the reaction mixture. After the addition was complete, the reaction mixture was stirred at room temperature under a nitrogen atmosphere for 1 hour. After LC-MS monitoring indicated completion, EA (40 mL) was added to the reaction mixture. The reaction mixture was filtered, and the filter cake was dissolved in DMF and purified by prep-HPLC (mobile phase: 0.05% HCOOH/CH₃CN/H₂O) to afford compound **A171** (120 mg, 21%) as a yellow solid.
LCMS: [M+H]⁺ = 1681.6 (half peak)

### (76) Synthesis of Compound A172

### Synthetic Route:

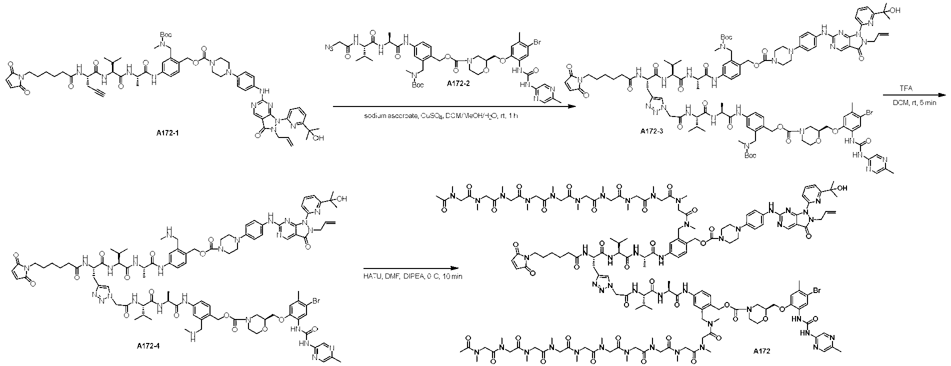

### Synthetic Steps:

### Synthesis of Intermediate Compound A172-3

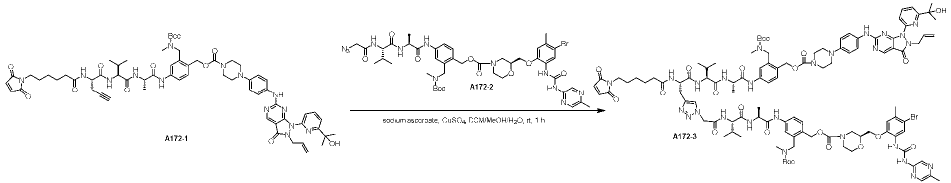

Compound **A172-1** (200 mg, 0.162 mmol, 1.0 eq) and compound **A172-2** (159 mg, 0.162 mmol, 1.0 eq) were dissolved in MeOH/DCM/water (5/5/1 mL). Then copper(II) sulfate pentahydrate (8.1 mg, 0.0323 mmol, 0.2 eq, in 100 uL water) and sodium ascorbate (12.8 mg, 0.0646 mmol, 0.4 eq, in 100 uL water) were added. After the addition was complete, the reaction mixture was stirred at room temperature under a nitrogen atmosphere for 1 hour. After LC-MS monitoring indicated completion, the reaction mixture was concentrated directly. Tetrahydrofuran was added, and the mixture was concentrated again to dryness, affording compound **A172-3** (350 mg, 97%) as a yellow solid.
LCMS: [M+H]⁺ = 1110.4 (half peak)

### Synthesis of Intermediate Compound A172-4

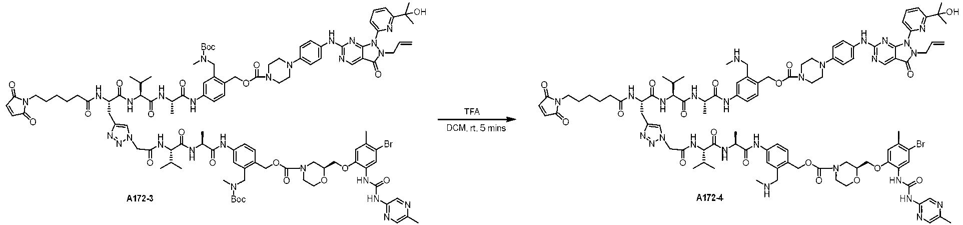

Compound **A172-3** (170 mg, 0.0765 mmol, 1.0 eq) was dissolved in DCM (3 mL). Trifluoroacetic acid (1 mL) was added to the reaction mixture, which was then stirred at room temperature for 5 minutes. After LC-MS monitoring indicated completion, the organic phase was concentrated to give the crude product compound **A172-4,** which was used directly in the next step.
LCMS: [M+H]⁺ = 1009.5 (half peak)

### Synthesis of Target Compound A172

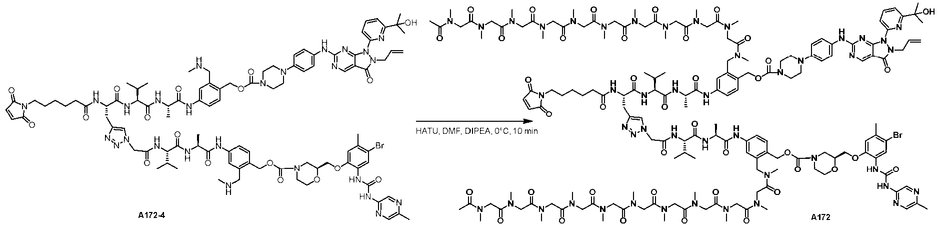

The compound **A172-4** from the previous step was dissolved in DMF (3 mL). **Ac-PSAR10-OH** (118 mg, 0.153 mmol, 2.0 eq) was added to the reaction mixture. Finally, under a nitrogen atmosphere, N,N-Diisopropylethylamine (198 mg, 1.53 mmol, 20.0 eq) and O-(7-Azabenzotriazol-1-yl)-N,N,N',N'-tetramethyluronium hexafluorophosphate (61 mg, 0.161 mmol, 2.1 eq) were added. After the addition was complete, the reaction mixture was stirred at 0 °C under a nitrogen atmosphere for 10 minutes. After LC-MS monitoring indicated completion, ethyl acetate (45 mL) was added to the reaction mixture. The mixture was filtered, and the filter cake was dissolved in DMF and purified by prep-HPLC (mobile phase: 0.05% HCOOH/CH₃CN/H₂O) to afford compound **A172** (83.6 mg, 31%) as a white solid.
LCMS: [M+H]⁺ = 1763.1 (half peak)

### (77) Synthesis of Compound A149

### Synthetic Route:

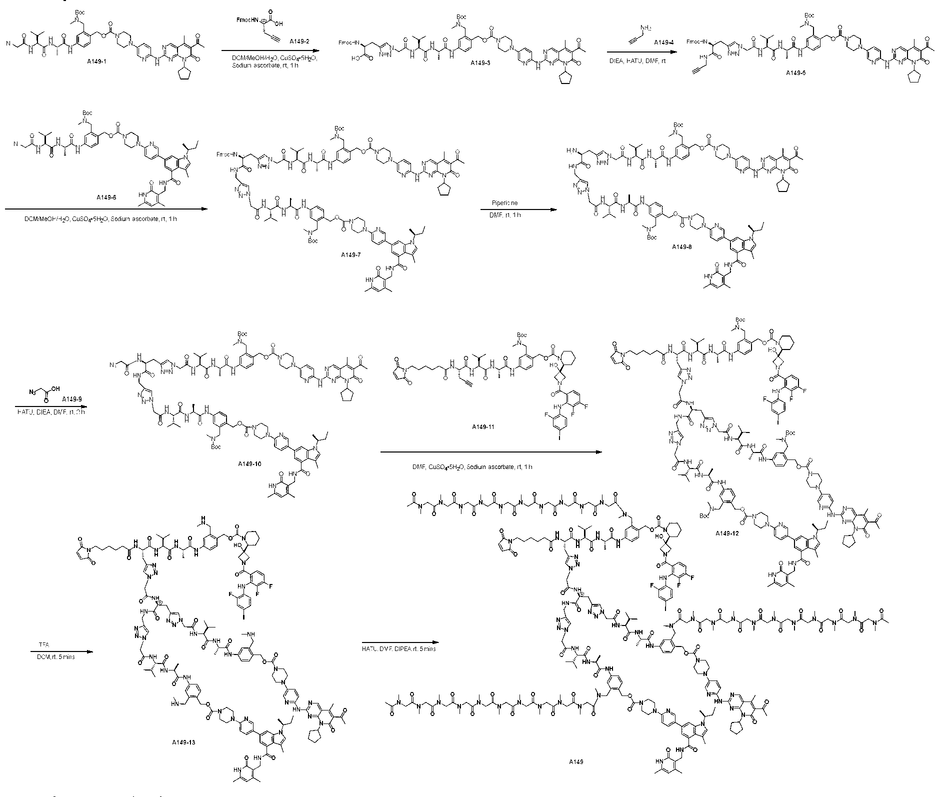

### Synthetic Steps:

### Synthesis of Intermediate Compound A149-3

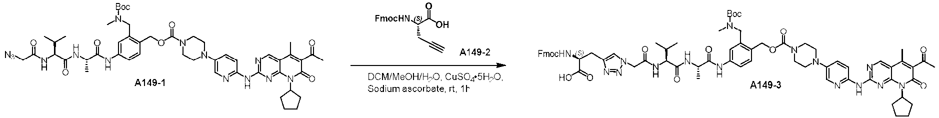

Compound **A149-1** (270 mg, 0.272 mmol, 1.0 eq) and **A149-2** (91.08 mg, 0.272 mmol, 1.0 eq) were dissolved in dichloromethane/methanol/water (5/5/1 mL). Under a nitrogen atmosphere, copper(II) sulfate pentahydrate (6.79 mg, 0.027 mmol, 0.1 eq, in 100 uL water) and sodium ascorbate (10.76 mg, 0.054 mmol, 0.2 eq, in 100 uL water) were added. After the addition was complete, the reaction mixture was stirred at room temperature under a nitrogen atmosphere for 1 hour. After LC-MS monitoring indicated completion, the reaction mixture was concentrated directly, and tetrahydrofuran was added multiple times and evaporated to remove water, affording compound **A149-3** (320 mg, 80%) as a yellow solid.
LCMS: [M+H]⁺ = 1329.5

### Synthesis of Intermediate Compound A149-5

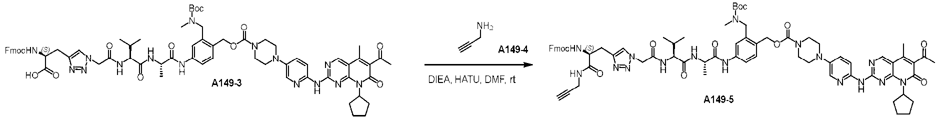

Compound **A149-3** (280 mg, 0.21 mmol, 1.0 eq) was dissolved in DMF (3 mL). Compound **A149-4** (34.8 mg, 0.63 mmol, 3.0 eq) and N,N-Diisopropylethylamine (54.44 mg, 0.42 mmol, 2.0 eq) were added to the reaction mixture. Finally, under a nitrogen atmosphere, O-(7-Azabenzotriazol-1-yl)-N,N,N',N'-tetramethyluronium hexafluorophosphate (88.08 mg, 0.23 mmol, 1.1 eq) was added. After the addition was complete, the reaction mixture was stirred at room temperature under a nitrogen atmosphere for 1 hour. After LC-MS monitoring indicated completion, ethyl acetate and water (30 mL) were added to the reaction mixture. The mixture was extracted with ethyl acetate. The organic phase was washed with water and then with saturated brine, dried over anhydrous sodium sulfate, filtered, and concentrated. The residue was slurried with MTBE, filtered, and the filter cake was collected and dried to afford compound **A149-5** (145 mg, 45%) as a yellow solid.
LCMS: [M+Na]⁺ = 1387.4

### Synthesis of Intermediate Compound A149-7

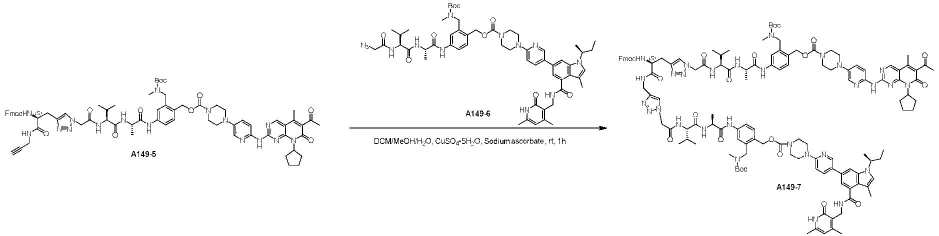

Compound **A149-5** (135 mg, 0.098 mmol, 1.0 eq) and compound **A149-6** (106.04 mg, 0.098 mmol, 1.0 eq) were dissolved in dichloromethane/methanol/water (5/5/1 mL). Under a nitrogen atmosphere, copper(II) sulfate pentahydrate (2.5 mg, 0.00988 mmol, 0.1 eq, in 100 uL water) and sodium ascorbate (3.9 mg, 0.01976 mmol, 0.2 eq, in 100 uL water) were added. After the addition was complete, the reaction mixture was stirred at room temperature under a nitrogen atmosphere for 1 hour. After LC-MS monitoring indicated completion, the reaction mixture was concentrated directly, and tetrahydrofuran was added multiple times and evaporated to remove water, affording compound **A149-7** (207 mg, 86%) as a yellow solid.
LCMS: [M+H]⁺ = 1219.6 (half peak)

### Synthesis of Intermediate Compound A149-8

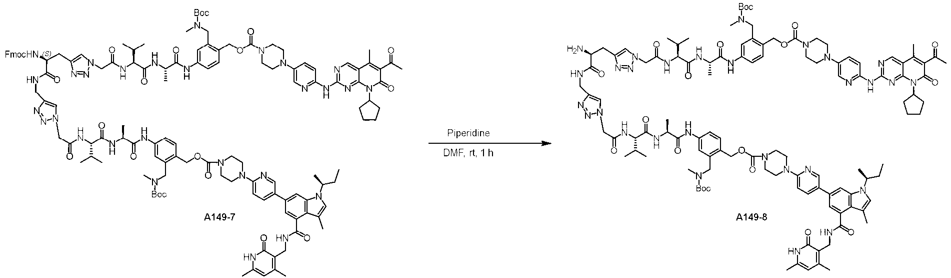

Compound **A149-7** (197 mg, 0.081 mmol, 1.0 eq) was dissolved in DMF (4 mL). Piperidine (1 mL) was added, and the reaction mixture was stirred at room temperature for 1 hour. After LC-MS monitoring indicated completion, MTBE was added to the reaction mixture. The suspension was filtered directly, washed with MTBE, and the filter cake was dried directly to afford compound **A149-8** (164 mg, 82.5%) as a yellow solid.
LCMS: [M+H]⁺ = 1108.5 (half peak)

### Synthesis of Intermediate Compound A149-10

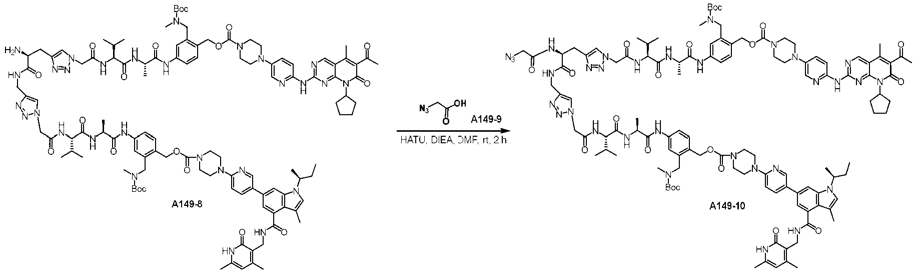

Compound **A149-8** (154 mg, 0.069 mmol, 1.0 eq) was dissolved in DMF (3 mL). Compound **A149-9** (10.52 mg, 0.104 mmol, 1.5 eq) and N,N-Diisopropylethylamine (26.91 mg, 0.208 mmol, 3.0 eq) were added to the reaction mixture. Finally, under a nitrogen atmosphere, O-(7-Azabenzotriazol-1-yl)-N,N,N',N'-tetramethyluronium hexafluorophosphate (39.58 mg, 0.104 mmol, 1.5 eq) was added. After the addition was complete, the reaction mixture was stirred at room temperature under a nitrogen atmosphere for 2 hours. After LC-MS monitoring indicated completion, ethyl acetate (50 mL) was added to the reaction mixture. The suspension was filtered directly, washed with ethyl acetate, and the filter cake was dried directly to afford compound **A149-10** (147 mg, 83%) as a yellow solid.
LCMS: [M+H]⁺ = 1150.1 (half peak)

### Synthesis of Intermediate Compound A149-12

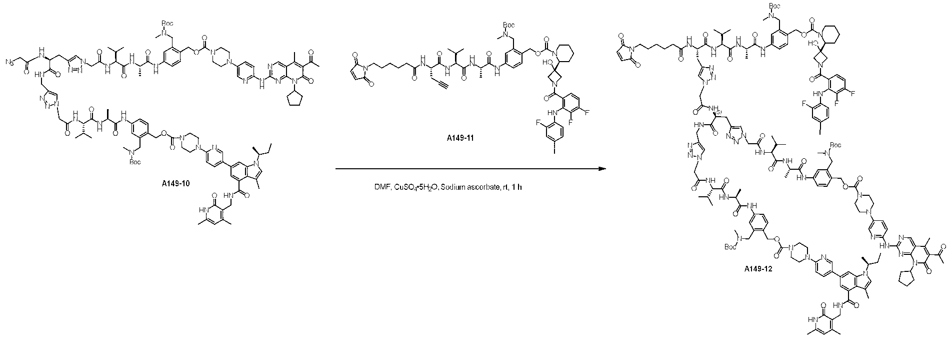

Compound **A149-10** (127 mg, 0.055 mmol, 1.0 eq) and compound **A149-11** (70.8 mg, 0.055 mmol, 1.0 eq) were dissolved in DMF (3 mL). Under a nitrogen atmosphere, copper(II) sulfate pentahydrate (1.38 mg, 0.005 mmol, 0.1 eq, in 100 uL water) and sodium ascorbate (2.19 mg, 0.011 mmol, 0.2 eq, in 100 uL water) were added. After the addition was complete, the reaction mixture was stirred at room temperature under a nitrogen atmosphere for 1 hour. After LC-MS monitoring indicated completion, ethyl acetate (50 mL) was added to the reaction mixture. The suspension was filtered directly, washed with ethyl acetate, and the filter cake was dried directly to afford compound **A149-12** (162 mg, 74%) as a yellow solid.
LCMS: [M+H]⁺ = 1194.4 (one-third peak)

### Synthesis of Intermediate Compound A149-13

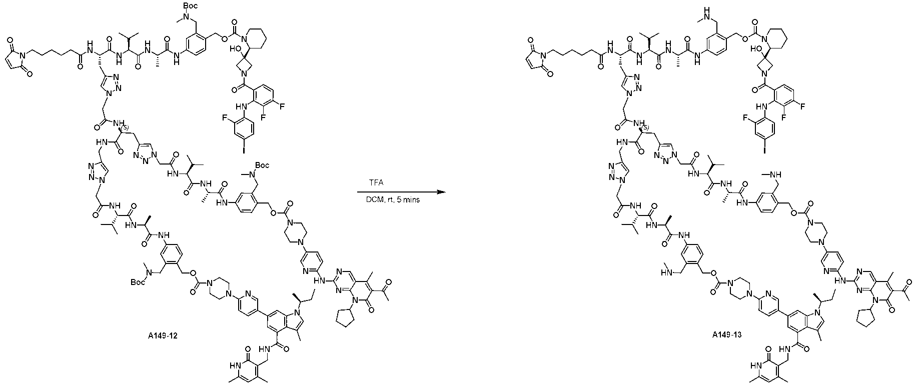

Compound **A149-12** (160 mg, 0.045 mmol, 1.0 eq) was dissolved in DCM (3 mL). Trifluoroacetic acid (1 mL) was added to the reaction mixture, which was then stirred at room temperature for 5 minutes. After LC-MS monitoring indicated completion, the organic phase was concentrated to give the crude product compound **A149-13,** which was used directly in the next step.
LCMS: [M+H]⁺ = 1641.1 (half peak)

### Synthesis of Target Compound A149

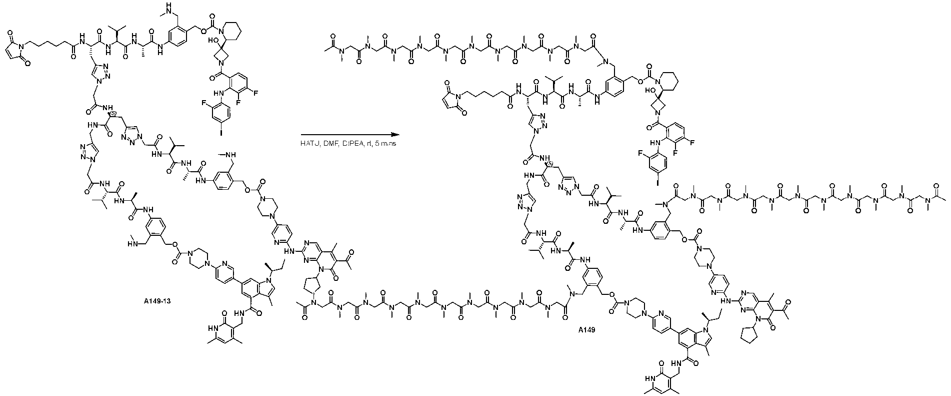

The compound **A149-13** from the previous step was dissolved in DMF (3 mL). **Ac-PSAR10-OH** (108.7 mg, 0.141 mmol, 3.1 eq) and N,N-Diisopropylethylamine (121 mg, 0.94 mmol, 21.0 eq) were added to the reaction mixture. Finally, under a nitrogen atmosphere, O-(7-Azabenzotriazol-1-yl)-N,N,N',N'-tetramethyluronium hexafluorophosphate (59 mg, 0.155 mmol, 3.5 eq) was added. After the addition was complete, the reaction mixture was stirred at room temperature under a nitrogen atmosphere for 5 minutes. After LC-MS monitoring indicated completion, ethyl acetate (50 mL) was added to the reaction mixture. The resulting suspension was filtered, and the filter cake was dissolved in DMF and purified by prep-HPLC (mobile phase: 0.05% HCOOH/CH₃CN/H₂O) to afford compound **A149** (34 mg, 14%) as a yellow solid.
LCMS: [M+H]⁺ = 1846.8 (one-third peak)

### (78) Synthesis of Compound A178

### Synthetic Route:

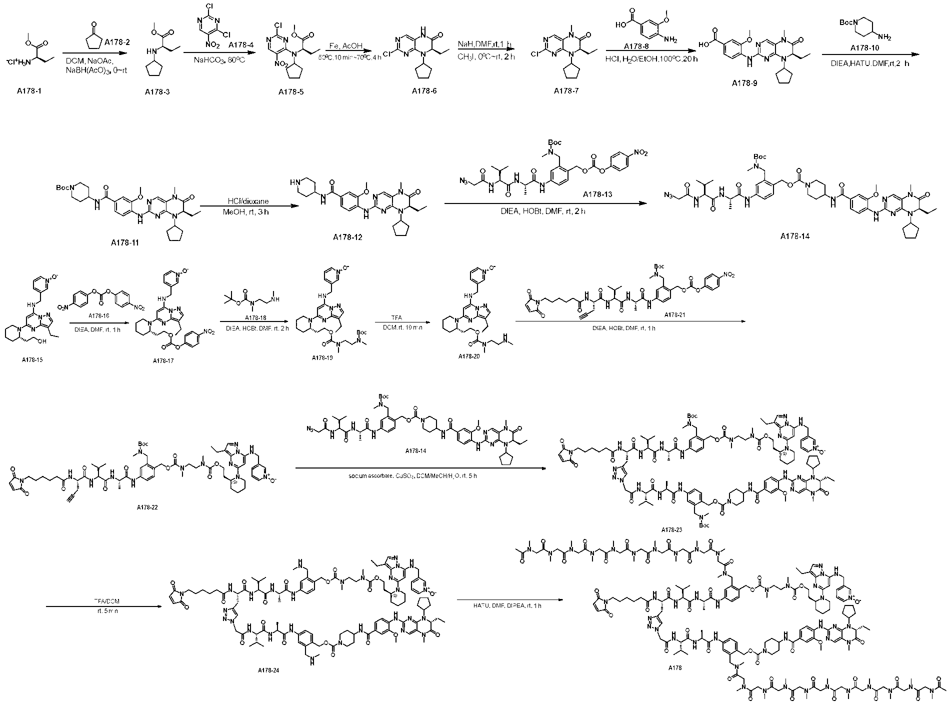

### Synthetic Steps:

### Synthesis of Intermediate Compound A178-3

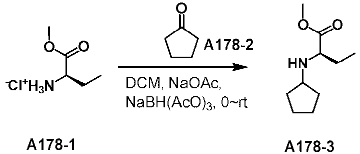

At room temperature, compound **A178-1** (34.5 g, 0.22 mol, 1.0 eq) and compound **A178-2** (18.89 g, 0.22 mol, 1.0 eq) were dissolved in dichloromethane (350 mL). At 0 °C, sodium acetate (18.42 g, 0.22 mol, 1.0 eq) was added, and the reaction mixture was moved to room temperature and stirred for 1.5 hours. Then at 0 °C, sodium triacetoxyborohydride (57.12 g, 0.27 mol, 1.2 eq) was slowly added. After the addition, the reaction mixture was stirred at room temperature for 2 hours. After the reaction was complete, the reaction mixture was adjusted to neutral with sodium bicarbonate solution. The aqueous phase was extracted with dichloromethane. The organic phase was dried over anhydrous sodium sulfate and concentrated to afford compound **A178-3** (36.6 g, 83%) as a yellow liquid.
LCMS: [M+H]⁺ = 186.2

### Synthesis of Intermediate Compound A178-5

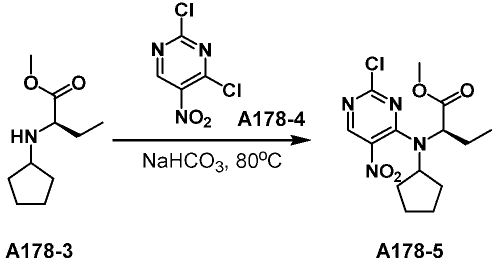

Compound **A178-3** (12.5 g, 0.068 mol, 1.0 eq) and sodium bicarbonate (5.84 g, 0.07 mol, 1.03 eq) were added to cyclohexane (80 mL). At 0 °C, compound **A178-4** (12.13 g, 0.063 mol, 0.93 eq) was added. After the addition, the reaction mixture was heated to 80 °C and stirred for 12 hours. After the reaction was complete, the mixture was filtered, and the filtrate was concentrated. Methanol (30 mL) was added to the filtrate, causing solid precipitation. The solid was collected by filtration and dried to afford compound **A178-5** (9.6 g, 41%) as a yellow solid. LCMS: [M+H]⁺ = 343.1

### Synthesis of Intermediate Compound A178-6

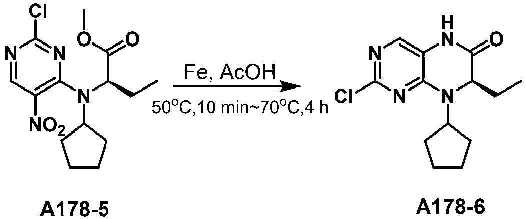

Iron powder (9.84 g, 0.176 mol, 6.5 eq) was added to glacial acetic acid (90 mL). Under a nitrogen atmosphere, the mixture was heated to 50 °C and stirred for 10 minutes for activation. Then compound **A178-5** (9.3 g, 0.027 mol, 1.0 eq) was added. After the addition, the reaction mixture was heated to 70 °C and reacted for 4 hours. After LC-MS monitoring indicated completion, the reaction mixture was filtered through a pad of Celite, and the filter cake was washed with dichloromethane/methanol=1/1 (160 mL) mixed solvent until colorless. The filtrate was collected and concentrated. Water was added, and the mixture was stirred, causing solid precipitation. The mixture was filtered, and the filter cake was washed with water, collected, and dried to afford compound **A178-6** (6.62 g, 87%) as a white solid.
LCMS: [M+H]⁺ = 281.1

### Synthesis of Intermediate Compound A178-7

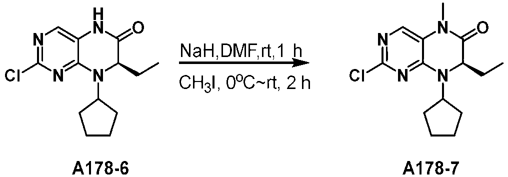

Compound **A178-6** (6.5 g, 23.2 mmol, 1.0 eq) was dissolved in DMF (50 mL). Sodium hydride (0.61 g, 25.52 mmol, 1.1 eq) was added, and the mixture was stirred under a nitrogen atmosphere at room temperature for 1 hour. Then in an ice bath (0 °C), iodomethane (3.95 g, 27.84 mmol, 1.2 eq) was added. After the addition, the reaction mixture was warmed to room temperature and reacted for 2 hours. After LC-MS monitoring indicated completion, ice water (200 mL) was added and stirred. Solid precipitated. After filtration, the filter cake was washed with water, collected, and dried to afford compound **A178-7** (6.2 g, 91%) as a brownish-yellow solid.
LCMS: [M+H]⁺ = 295.1

### Synthesis of Intermediate Compound A178-9

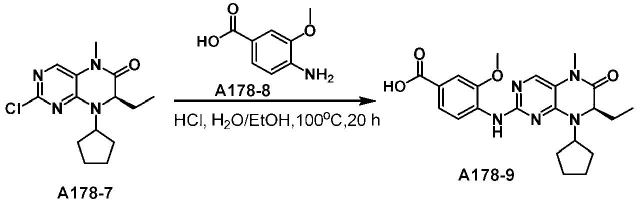

Compound **A178-7** (6.2 g, 21 mmol, 1.0 eq) and compound **A178-8** (5.27 g, 31.5 mmol, 1.5 eq) were dissolved in a mixed solution of H₂O (56 mL) and EtOH (14 mL). Concentrated hydrochloric acid (8.4 mL) was then added. The reaction was stirred at 100 °C for 20 hours. After LC-MS monitoring indicated completion, water (280 mL) was added and stirred. Solid precipitated. After filtration, the filter cake was washed with water, collected, and dried to afford compound **A178-9** (7 g, 78%) as a dark grey solid.
LCMS: [M+H]⁺ = 426.2

### Synthesis of Intermediate Compound A178-11

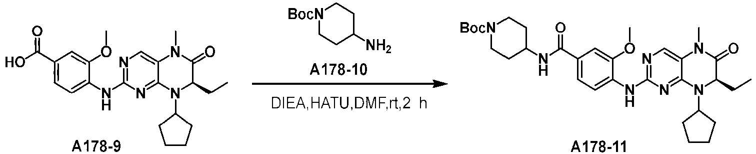

Compound **A178-9** (7 g, 16.4 mmol, 1.0 eq) was dissolved in DMF (55 mL). N,N-Diisopropylethylamine (5.43 g, 42 mmol, 2.0 eq) was added and stirred for 1 minute. Then compound **A178-10** (6.31 g, 31.5 mmol, 1.5 eq) and O-(7-Azabenzotriazol-1-yl)-N,N,N',N'-tetramethyluronium hexafluorophosphate (9.58 g, 25.2 mmol, 1.2 eq) were added. After the addition was complete, the reaction mixture was stirred at room temperature for 2 hours. After LC-MS monitoring indicated completion, water (240 mL) was added and stirred. Solid precipitated. After filtration, the filter cake was washed with water, collected, and dried to afford compound **A178-11** (10.5 g) as a dark grey solid.
LCMS: [M+H]⁺ = 608.3

### Synthesis of Intermediate Compound A178-12

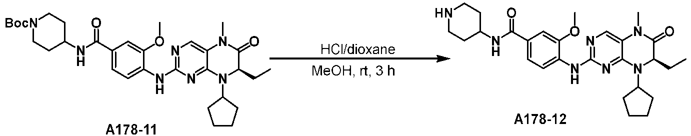

To compound **A178-11** (5.6 g, 9.2 mmol, 1.0 eq), MeOH (8.5 mL) and a solution of HCl/dioxane (48 mL) were added. The system was stirred at room temperature for 3 hours. After LC-MS monitoring indicated completion, ethyl acetate (180 mL) was added and stirred. Solid precipitated. After filtration, the filter cake was washed with water, collected, and dried to afford compound **A178-12** (4.7 g, 100%) as a grey solid.
LCMS: [M+H]⁺ = 508.3

### Synthesis of Intermediate Compound A178-14

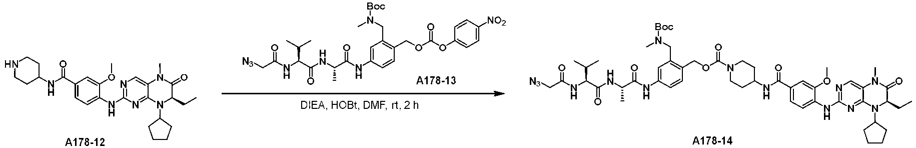

Compound **A178-12** (2.8 g, 5.5 mmol, 1.0 eq) was dissolved in DMF (35 mL). N,N-Diisopropylethylamine (5.69 g, 44 mmol, 8.0 eq) was added and stirred for 2 minutes. Then compound **A178-13** (3.39 g, 4.95 mmol, 0.9 eq) and HOBt (0.37 g, 2.75 mmol, 0.5 eq) were added. After the addition was complete, the reaction mixture was stirred at room temperature for 2 hours. After LC-MS monitoring indicated completion, the reaction mixture was diluted with ethyl acetate (80 mL) and washed with water (40 mL). The organic phase was then washed sequentially with 10% citric acid aqueous solution (30 mL), water (30 mL), and saturated brine (30 mL), dried over anhydrous sodium sulfate, filtered, and concentrated. The residue was purified by silica gel column chromatography (DCM/MeOH=93/7) to afford compound **A178-14** (3.6 g, 62%) as a yellow solid.
LCMS: [M+H]⁺ = 1053.9

### Synthesis of Intermediate Compound A178-17

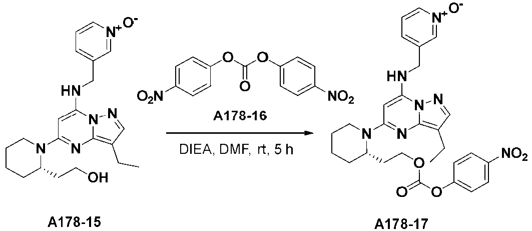

Compound **A178-15** (2.11 g, 5.3 mmol, 1.0 eq) was dissolved in DMF (50 mL). N,N-Diisopropylethylamine (2.05 g, 15.9 mmol, 3.0 eq) and compound **A178-16** (3.22 g, 10.6 mmol, 2.0 eq) were added. After the addition was complete, the reaction mixture was stirred at room temperature for 5 hours. After LC-MS monitoring indicated completion, the reaction mixture was diluted with ethyl acetate (100 mL) and washed with water (40 mL x 2). The organic phase was then washed with saturated brine (30 mL x 3), dried over anhydrous sodium sulfate, filtered, and concentrated. The residue was purified by silica gel column chromatography (DCM/MeOH=94/6) to afford compound **A178-17** (2.63 g, 88%) as a yellow solid.
LCMS: [M+H]⁺ = 562.2

### Synthesis of Intermediate Compound A178-19

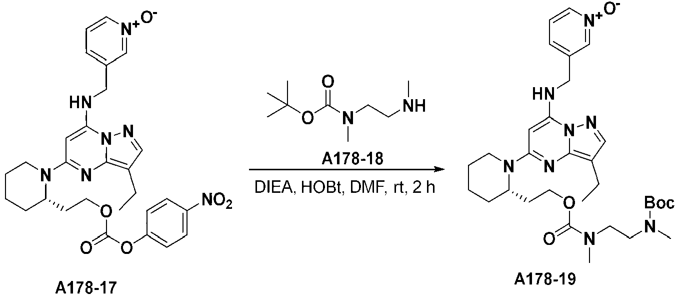

Compound **A178-17** (2.83 g, 5 mmol, 1.0 eq) was dissolved in DMF (35 mL). N,N-Diisopropylethylamine (1.94 g, 15 mmol, 3.0 eq) was added and stirred for 1 minute. Then compound **A178-18** (1.88 g, 10 mmol, 2.0 eq) and HOBt (0.34 g, 2.5 mmol, 0.5 eq) were added. After the addition was complete, the reaction mixture was stirred at room temperature for 2 hours. After LC-MS monitoring indicated completion, the reaction mixture was diluted with ethyl acetate (60 mL) and washed with water (20 mL x 2). The organic phase was then washed with saturated brine (20 mL x 3), dried over anhydrous sodium sulfate, filtered, and concentrated. The residue was purified by silica gel column chromatography (DCM/MeOH=94/6-93/7) to afford compound **A178-19** (2.7 g, 88%) as a yellow solid.
LCMS: [M+H]⁺ = 611.8

### Synthesis of Intermediate Compound A178-20

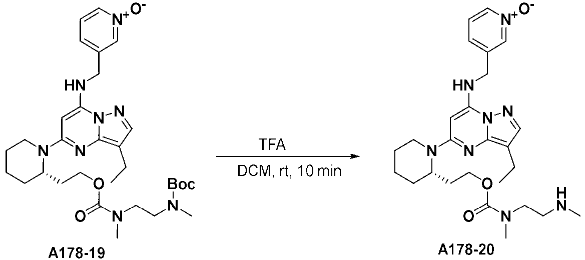

Compound **A178-19** (1.9 g, 3.1 mmol, 1.0 eq) was dissolved in DCM (21 mL). Trifluoroacetic acid (7 mL) was added to the reaction mixture, which was then stirred at room temperature for 10 minutes. After LC-MS monitoring indicated completion, the organic phase was concentrated to give the crude product compound **A178-20,** which was used directly in the next step.
LCMS: [M+H]⁺ = 511.3

### Synthesis of Intermediate Compound A178-22

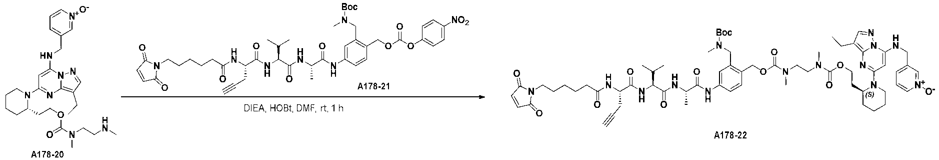

The compound **A178-20** from the previous step was dissolved in DMF (15 mL). N,N-Diisopropylethylamine (4.01 g, 31 mmol, 10.0 eq) was added and stirred for 1 minute. Then compound **A178-21** (2.62 g, 2.95 mmol, 0.95 eq) and HOBt (0.21 g, 1.55 mmol, 0.5 eq) were added. After the addition was complete, the reaction mixture was stirred at room temperature for 1 hour. After LC-MS monitoring indicated completion, the reaction mixture was diluted with ethyl acetate (50 mL) and washed with water (20 mL x 2). The organic phase was then washed with saturated brine (20 mL x 2), dried over anhydrous sodium sulfate, filtered, and concentrated. The residue was purified by silica gel column chromatography (DCM/MeOH=94/6-93/7) to afford compound **A178-22** (2.6 g, 66%) as a yellow solid.
LCMS: [M+H]⁺ = 631.6 (half peak)

### Synthesis of Intermediate Compound A178-23

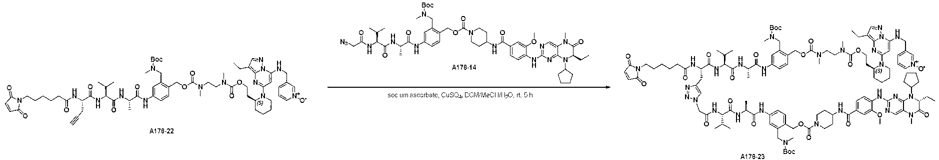

Compound **A178-22** (130 mg, 0.103 mmol, 1.0 eq) and compound **A178-14** (98 mg, 0.093 mmol, 0.9 eq) were dissolved in MeOH/DCM (2/2 mL). Then 100 uL of an aqueous solution of copper(II) sulfate pentahydrate (5 mg, 0.02 mmol, 0.2 eq) and 100 uL of an aqueous solution of sodium ascorbate (8 mg, 0.04 mmol, 0.4 eq) were added. After the addition was complete, the reaction mixture was stirred at room temperature under a nitrogen atmosphere for 5 hours. After LC-MS monitoring indicated completion, concentration afforded the crude product compound **A178-23** (250 mg) as a yellow solid.
LCMS: [M+H]⁺ = 1158.1 (half peak)

### Synthesis of Intermediate Compound A178-24

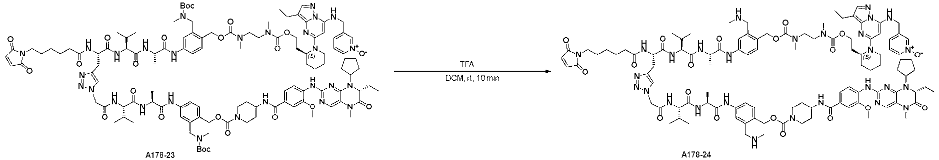

Compound **A178-23** (250 mg, 0.108 mmol, 1.0 eq) was dissolved in DCM (4.5 mL). Trifluoroacetic acid (1.5 mL) was added to the reaction mixture, which was then stirred at room temperature for 10 minutes. After LC-MS monitoring indicated completion, the organic phase was concentrated to give the crude product compound **A178-24,** which was used directly in the next step.
LCMS: [M+H]⁺ = 1057.7 (half peak)

### Synthesis of Target Compound A178

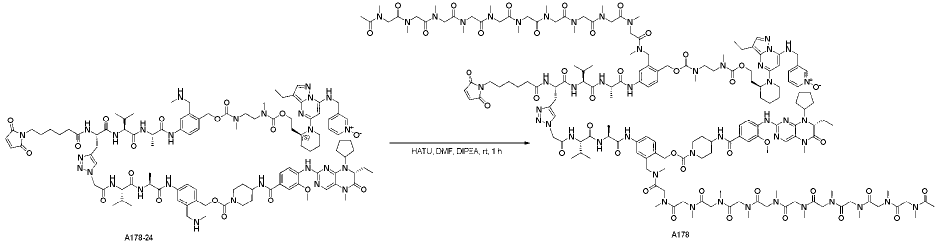

The compound **A178-24** from the previous step was dissolved in DMF (3 mL). Compound **A178-25** (166 mg, 0.216 mmol, 2.0 eq) was added. Then at 0 °C, N,N-Diisopropylethylamine (279 mg, 2.16 mmol, 20.0 eq) and O-(7-Azabenzotriazol-1-yl)-N,N,N',N'-tetramethyluronium hexafluorophosphate (86 mg, 0.23 mmol, 2.1 eq) were added to the reaction mixture. After the addition was complete, the reaction mixture was stirred at 0 °C for 1 hour. After LC-MS monitoring indicated completion, ethyl acetate (45 mL) was added to the reaction mixture. The reaction mixture was filtered, and the filter cake was dissolved in DMF and purified by prep-HPLC (mobile phase: 0.1% HCOOH/CH₃CN/H₂O) to afford compound **A178** (63.5 mg, 27%) as a white solid.
LCMS: [M+H]⁺ = 1810.3 (half peak)

### (79) Synthesis of Compound A179

### Synthetic Route:

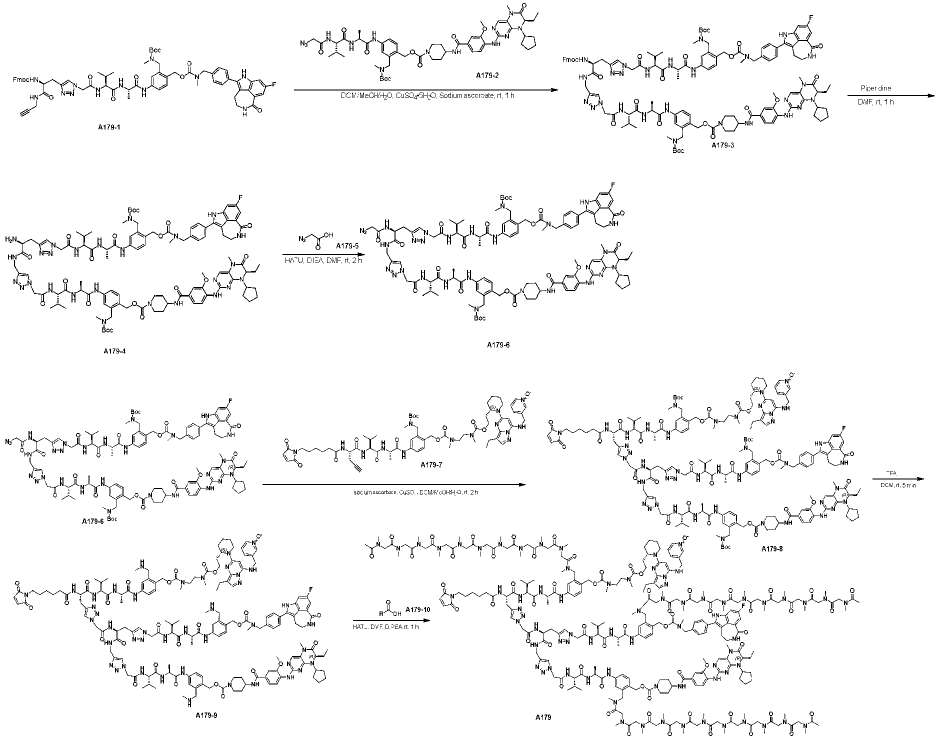

### Synthetic Steps:

### Synthesis of Intermediate Compound A179-3

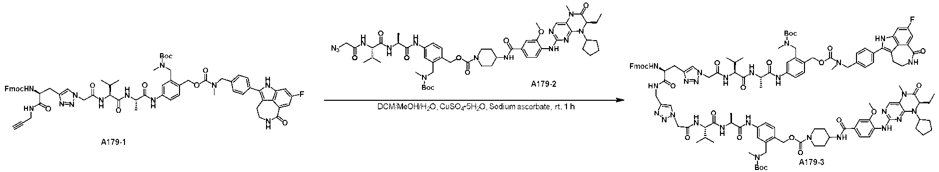

Compound **A179-1** (1700 mg, 1.368 mmol, 1.0 eq) and compound **A179-2** (1442 mg, 1.368 mmol, 1.0 eq) were dissolved in dichloromethane/methanol (60/60 mL). Under a nitrogen atmosphere, copper(II) sulfate pentahydrate (68.4 mg, 0.274 mmol, 0.2 eq, in 1 mL water) and sodium ascorbate (108.4 mg, 0.547 mmol, 0.4 eq, in 1 mL water) were added. After the addition was complete, the reaction mixture was stirred at room temperature under a nitrogen atmosphere for 1 hour. After LC-MS monitoring indicated completion, the reaction mixture was concentrated directly. The residue was dissolved in tetrahydrofuran and concentrated again to dryness. The crude product was slurried with ethyl acetate, filtered, and dried to afford compound **A179-3** (2200 mg, 70%) as a yellow solid.
LCMS: [M+H]⁺ = 1147.9 (half peak)

### Synthesis of Intermediate Compound A179-4

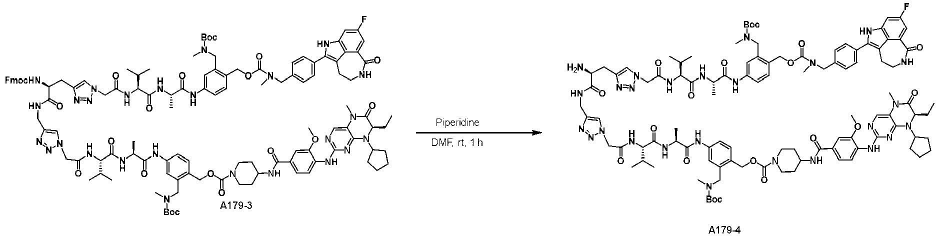

Compound **A179-3** (2200 mg, 0.958 mmol, 1.0 eq) was dissolved in DMF (40 mL). Under a nitrogen atmosphere, piperidine (8 mL) was added. The reaction mixture was stirred at room temperature under a nitrogen atmosphere for 1 hour. After LC-MS monitoring indicated completion, methyl tert-butyl ether (400 mL) was added to the reaction mixture. The suspension was filtered directly, washed with methyl tert-butyl ether, and the filter cake was dried directly to afford compound **A179-4** (1700 mg, 85%) as a yellow solid.
LCMS: [M+H]⁺ = 1036.7 (half peak)

### Synthesis of Intermediate Compound A179-6

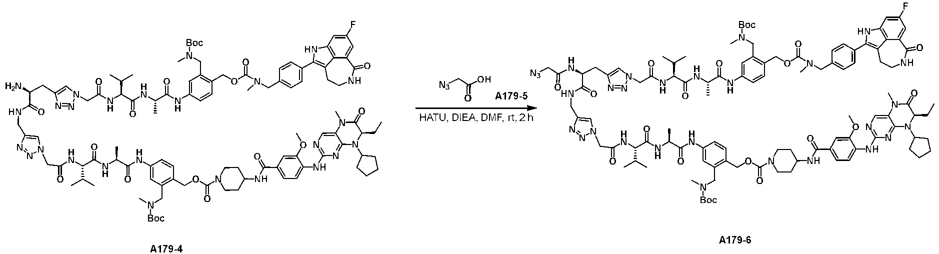

Compound **A179-4** (1600 mg, 0.771 mmol, 1.0 eq) was dissolved in DMF (10 mL). N,N-Diisopropylethylamine (299 mg, 2.314 mmol, 3.0 eq) and **A179-5** (125 mg, 1.234 mmol, 1.6 eq) were added to the reaction mixture. Finally, under a nitrogen atmosphere, O-(7-Azabenzotriazol-1-yl)-N,N,N',N'-tetramethyluronium hexafluorophosphate (499 mg, 1.311 mmol, 1.7 eq) was added. After the addition was complete, the reaction mixture was stirred at room temperature under a nitrogen atmosphere for 2 hours. After LC-MS monitoring indicated completion, methyl tert-butyl ether (400 mL) was added to the reaction mixture. The suspension was filtered directly, washed with methyl tert-butyl ether, and the filter cake was dried. It was then slurried with ethyl acetate, filtered, and dried to afford compound **A179-6** (1500 mg, 90%) as a grey solid.
LCMS: [M+H]⁺ = 1078.2 (half peak)

### Synthesis of Intermediate Compound A179-8

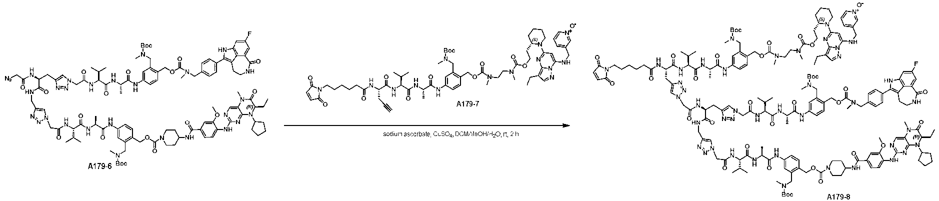

Compound **A179-6** (1800 mg, 0.834 mmol, 1.0 eq) and compound **A179-7** (1053 mg, 0.834 mmol, 1.0 eq) were dissolved in DMF (30 mL). Under a nitrogen atmosphere, copper(II) sulfate pentahydrate (21 mg, 0.083 mmol, 0.1 eq, in 500 uL water) and sodium ascorbate (66 mg, 0.033 mmol, 0.4 eq, in 500 uL water) were added. After the addition was complete, the reaction mixture was stirred at room temperature under a nitrogen atmosphere for 2 hours. After LC-MS monitoring indicated completion, methyl tert-butyl ether (500 mL) was added to the reaction mixture. The resulting suspension was filtered directly, washed with methyl tert-butyl ether, and the filter cake was dried directly to afford compound **A179-8** (2600 mg, 91%) as a grey solid.
LCMS: [M+H]⁺ = 1709.0 (half peak)

### Synthesis of Intermediate Compound A179-9

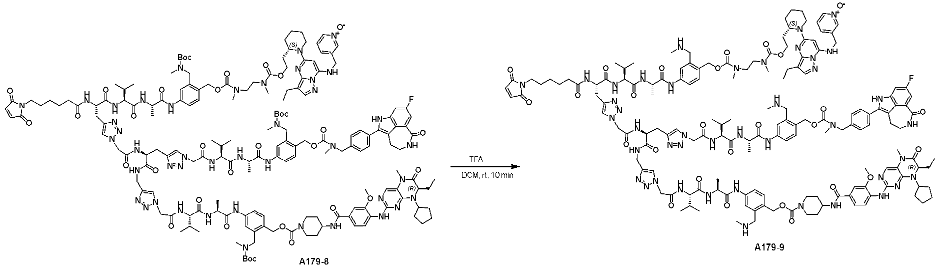

Compound **A179-8** (1500 mg, 0.437 mmol, 1.0 eq) was dissolved in DCM (21 mL). Trifluoroacetic acid (7 mL) was added to the reaction mixture, which was then stirred at room temperature for 10 minutes. After LC-MS monitoring indicated completion, the organic phase was concentrated to give the crude product compound **A179-9,** which was used directly in the next step.
LCMS: [M+H]⁺ = 1558.6 (half peak)

### Synthesis of Target Compound A179

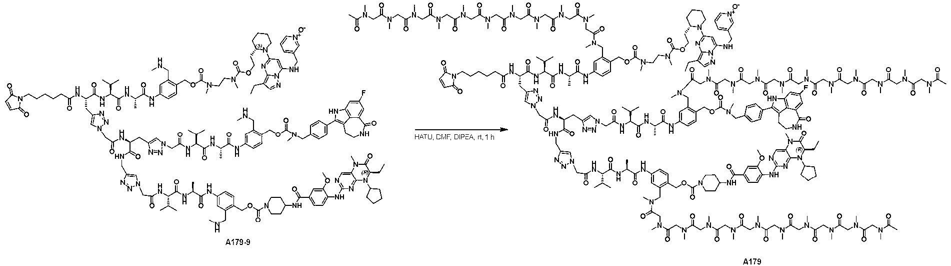

The compound **A179-9** from the previous step was dissolved in DMF (20 mL). Compound **A179-10** (1044 mg, 1.355 mmol, 3.1 eq) and N,N-Diisopropylethylamine (1692 mg, 13.110 mmol, 30.0 eq) were added to the reaction mixture. Finally, under a nitrogen atmosphere, O-(7-Azabenzotriazol-1-yl)-N,N,N',N'-tetramethyluronium hexafluorophosphate (548 mg, 1.442 mmol, 3.3 eq) was added. After the addition was complete, the reaction mixture was stirred at room temperature under a nitrogen atmosphere for 1 hour. After LC-MS monitoring indicated completion, ethyl acetate (260 mL) was added to the reaction mixture. The resulting suspension was filtered, and the filter cake was dissolved in DMF and purified by prep-HPLC (mobile phase: 0.05% HCOOH/CH₃CN/H₂O) to afford compound **A179** (500 mg, 35%) as a white solid. LCMS: [M+H]⁺ = 1792.1 (one-third peak)

### (80) Synthesis of Compound A173

### Synthetic Route:

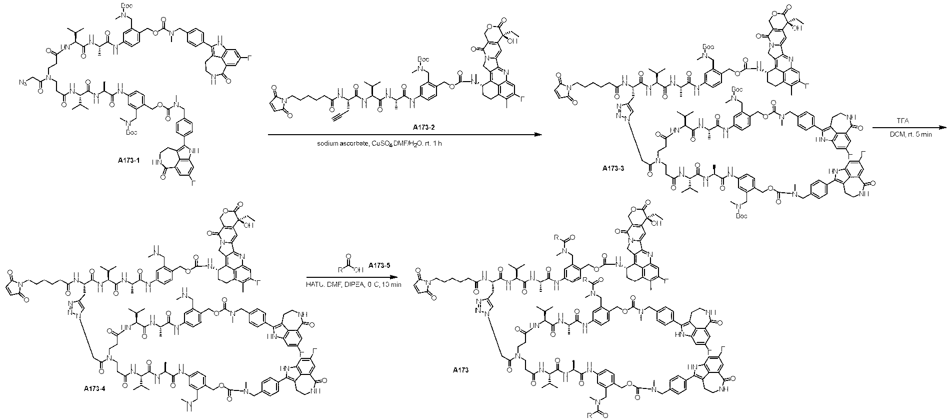

### Synthetic Steps:

### Synthesis of Intermediate Compound A173-3

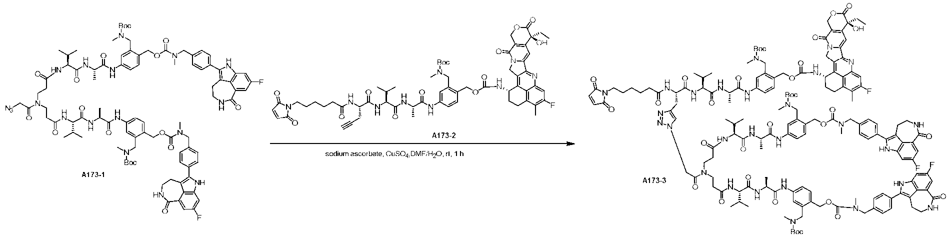

Compound **A173-1** (160 mg, 0.0898 mmol, 1.0 eq) and compound **A173-2** (107 mg, 0.0898 mmol, 1.0 eq) were dissolved in DMF (5 mL). Under a nitrogen atmosphere, copper(II) sulfate pentahydrate (4.5 mg, 0.0180 mmol, 0.2 eq, in 100 uL water) and sodium ascorbate (7.1 mg, 0.0359 mmol, 0.4 eq, in 100 uL water) were added. After the addition was complete, the reaction mixture was stirred at room temperature under a nitrogen atmosphere for 1 hour. After LC-MS monitoring indicated completion, the reaction mixture was diluted with ethyl acetate. The resulting suspension was filtered directly, washed with ethyl acetate, and the filter cake was dried directly to afford compound **A173-3** (260 mg, 97%) as a yellow solid.
LCMS: [M+H]⁺ = 1483.4 (half peak)

### Synthesis of Intermediate Compound A173-4

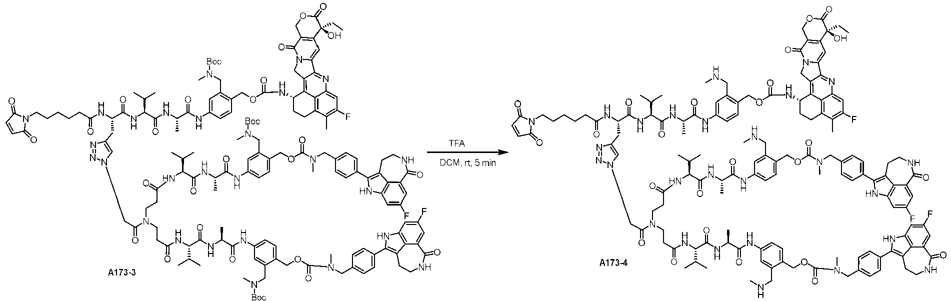

Compound **A173-3** (250 mg, 0.0842 mmol, 1.0 eq) was dissolved in DCM (3 mL). Trifluoroacetic acid (1 mL) was added to the reaction mixture, which was then stirred at room temperature for 5 minutes. After LC-MS monitoring indicated completion, the organic phase was concentrated to give the crude product compound **A173-4,** which was used directly in the next step.
LCMS: [M+H]⁺ = 1334.1 (half peak)

### Synthesis of Target Compound A173

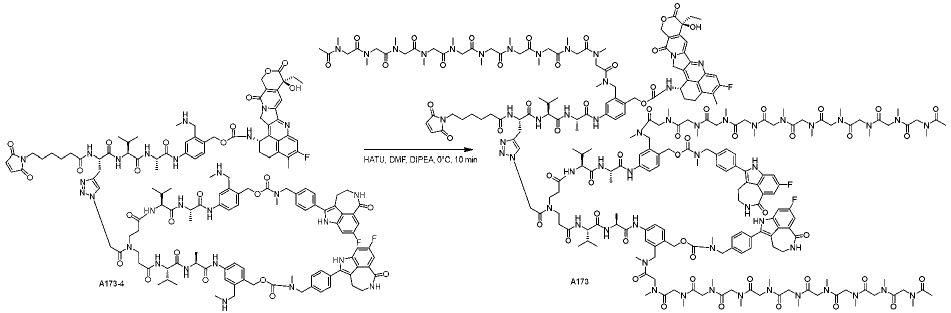

The compound **A173-4** from the previous step was dissolved in DMF (3 mL). **Ac-PSAR10-OH** (195 mg, 0.253 mmol, 3.0 eq) was added to the reaction mixture. Finally, under a nitrogen atmosphere, N,N-Diisopropylethylamine (326 mg, 2.526 mmol, 30.0 eq) and O-(7-Azabenzotriazol-1-yl)-N,N,N',N'-tetramethyluronium hexafluorophosphate (106 mg, 0.278 mmol, 3.3 eq) were added. After the addition was complete, the reaction mixture was stirred at 0 °C under a nitrogen atmosphere for 10 minutes. After LC-MS monitoring indicated completion, ethyl acetate (45 mL) was added to the reaction mixture. The resulting suspension was filtered, and the filter cake was dissolved in DMF and purified by prep-HPLC (mobile phase: 0.05% HCOOH/CH₃CN/H₂O) to afford compound **A173** (56.6 mg, 14%) as a white solid. LCMS: [M+H]⁺ = 1642.2 (one-third peak)

### (81) Synthesis of Compound A174

### Synthetic Route:

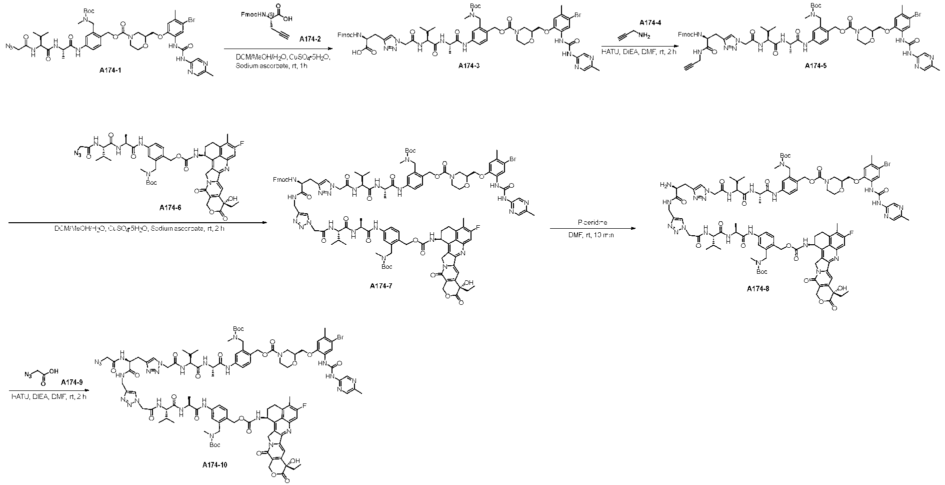

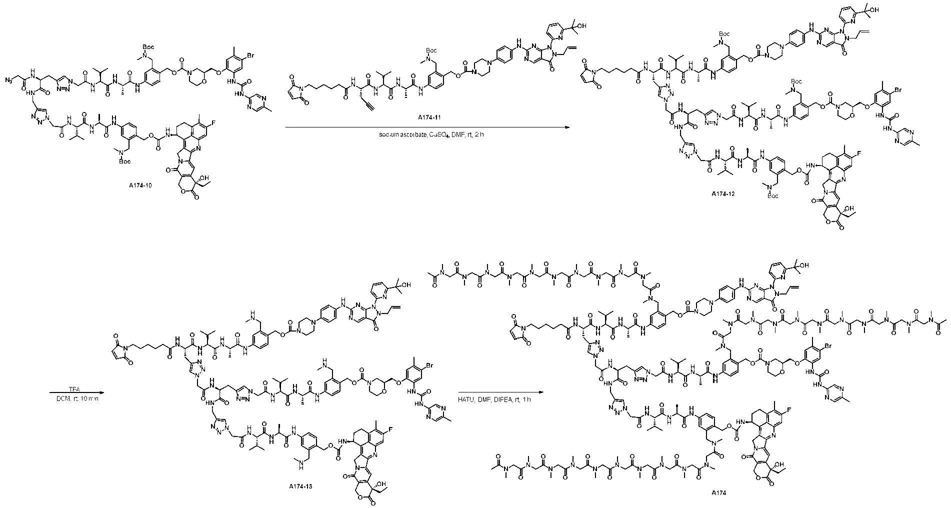

### Synthetic Steps:

### Synthesis of Intermediate Compound A174-3

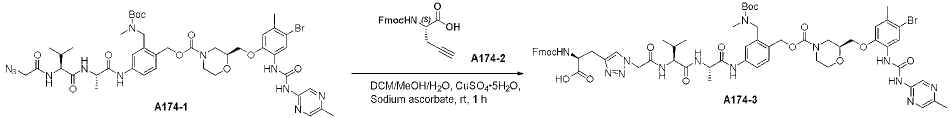

Compound **A174-1** (350 mg, 0.356 mmol, 1.0 eq) and compound **A174-2** (119 mg, 0.356 mmol, 1.0 eq) were dissolved in dichloromethane/methanol (6/6 mL). Then, an aqueous solution (300 µL) of copper(II) sulfate pentahydrate (18 mg, 0.07 mmol, 0.2 eq) and an aqueous solution (300 µL) of sodium ascorbate (28 mg, 0.14 mmol, 0.4 eq) were added. After the addition was complete, the reaction mixture was stirred at room temperature under a nitrogen atmosphere for 1 hour. After LC-MS monitoring indicated completion, the reaction mixture was concentrated directly. Tetrahydrofuran was added multiple times to azeotrope and dry the residue. The obtained crude product was triturated with ethyl acetate, filtered, and dried to afford compound **A174-3** (240 mg, 51%) as a yellow solid.
LCMS: [M+H]⁺ = 1318.5

### Synthesis of Intermediate Compound A174-5

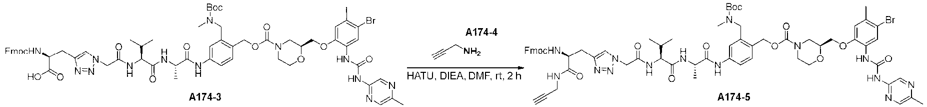

Compound **A174-3** (240 mg, 0.18 mmol, 1.0 eq) was dissolved in DMF (3 mL). O-(7-Azabenzotriazol-1-yl)-N,N,N',N'-tetramethyluronium hexafluorophosphate (90 mg, 0.24 mmol, 1.3 eq) and N,N-Diisopropylethylamine (71 mg, 0.55 mmol, 3.0 eq) were added to the reaction mixture. Finally, compound **A174-4** (15 mg, 0.27 mmol, 1.5 eq) was added. After the addition was complete, the reaction mixture was stirred at room temperature under a nitrogen atmosphere for 2 hours. After LC-MS monitoring indicated completion, 2-methyltetrahydrofuran was added to the reaction mixture for dilution. The mixture was washed with water, then with 10% aqueous citric acid solution, and then with saturated brine. The organic phase was dried over anhydrous sodium sulfate, filtered, and the filtrate was concentrated. The residue was purified by silica gel column chromatography (DCM/MeOH=93/7). After concentration, the product was triturated with methanol, filtered, and the filter cake was collected and dried to afford compound **A174-5** (210 mg, 86%) as a brown solid.
LCMS: [M+H]⁺ = 677.4 (half peak)

### Synthesis of Intermediate Compound A174-7

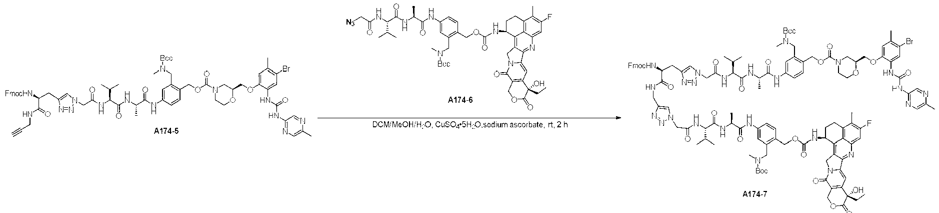

Compound **A174-5** (210 mg, 0.15 mmol, 1.0 eq) and compound **A174-6** (176 mg, 0.18 mmol, 1.16 eq) were dissolved in dichloromethane/methanol (2/2 mL). Then, an aqueous solution (100 µL) of copper(II) sulfate pentahydrate (8 mg, 0.031 mmol, 0.2 eq) and an aqueous solution (100 µL) of sodium ascorbate (12 mg, 0.062 mmol, 0.4 eq) were added. After the addition was complete, the reaction mixture was stirred at room temperature under a nitrogen atmosphere for 2 hours. After LC-MS monitoring indicated completion, the reaction mixture was concentrated directly. Tetrahydrofuran was added multiple times to azeotrope and dry the residue. The residue was then triturated with methanol, filtered, and the filter cake was collected and dried to afford compound **A174-7** (280 mg, 80%) as a brown solid.
LCMS: [M+H]⁺ = 1168.6 (half peak)

### Synthesis of Intermediate Compound A174-8

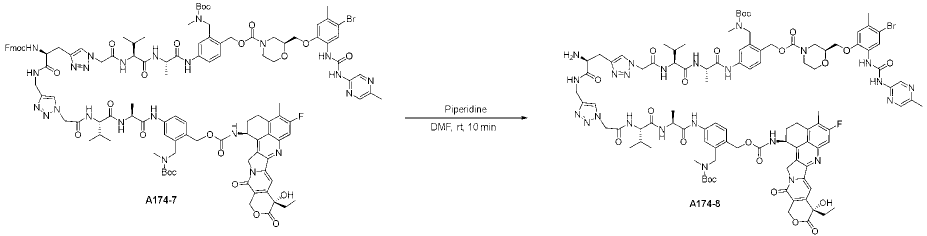

Compound **A174-7** (280 mg, 0.12 mmol, 1.0 eq) was dissolved in DMF (2 mL). Piperidine (0.4 mL) was added, and the reaction mixture was stirred at room temperature for 10 minutes. After LC-MS monitoring indicated completion, methyl tert-butyl ether was added to the reaction mixture. The resulting suspension was filtered directly. The filter cake was washed with methyl tert-butyl ether, then triturated with methanol, filtered again, and the filter cake was collected and dried to afford compound **A174-8** (180 mg, 71%) as a dark gray solid.
LCMS: [M+H]⁺ = 1057.3 (half peak)

### Synthesis of Intermediate Compound A174-10

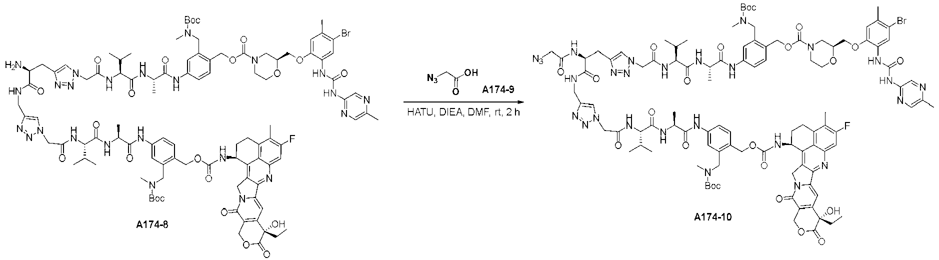

Compound **A174-8** (180 mg, 0.085 mmol, 1.0 eq) was dissolved in DMF (3 mL). N,N-Diisopropylethylamine (33 mg, 0.26 mmol, 3.0 eq) was added, and the mixture was stirred for 2 minutes. Then, compound **A174-9** (13 mg, 0.13 mmol, 1.5 eq) and O-(7-Azabenzotriazol-1-yl)-N,N,N',N'-tetramethyluronium hexafluorophosphate (36 mg, 0.09 mmol, 1.1 eq) were added to the reaction mixture. After the addition was complete, the reaction mixture was stirred at room temperature for 2 hours. After LC-MS monitoring indicated completion, methyl tert-butyl ether was added to the reaction mixture. The resulting suspension was filtered directly. The filter cake was washed with methyl tert-butyl ether, then triturated with methanol, filtered again, and the filter cake was collected and dried to afford compound **A174-10** (170 mg, 91%) as a grayish-green solid.
LCMS: [M+H]⁺ = 1098.6 (half peak)

### Synthesis of Intermediate Compound A174-12

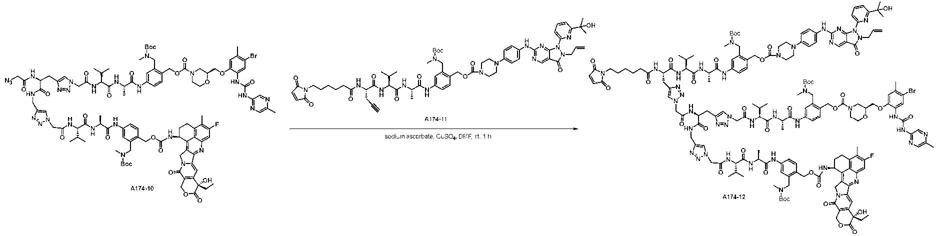

Compound **A174-10** (170 mg, 0.077 mmol, 1.0 eq) and compound **A174-11** (96 mg, 0.077 mmol, 1.0 eq) were dissolved in DMF (6 mL). Then, an aqueous solution (100 µL) of copper(II) sulfate pentahydrate (2 mg, 0.0077 mmol, 0.1 eq) and an aqueous solution (100 µL) of sodium ascorbate (6 mg, 0.0309 mmol, 0.4 eq) were added. After the addition was complete, the reaction mixture was stirred at room temperature under a nitrogen atmosphere for 1 hour. After LC-MS monitoring indicated completion, methanol was added to the reaction mixture, causing solid precipitation. The suspension was filtered directly, and the filter cake was collected and dried to afford compound **A174-12** (220 mg, 83%) as a green solid.
LCMS: [M+H]⁺ = 1145.3 (one-third peak)

### Synthesis of Intermediate Compound A174-13

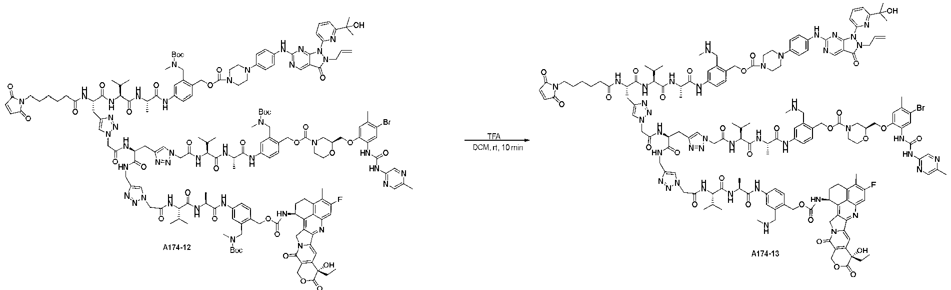

Compound **A174-12** (220 mg, 0.064 mmol, 1.0 eq) was dissolved in DCM (6 mL). Trifluoroacetic acid (2 mL) was added to the reaction mixture. The reaction mixture was stirred at room temperature for 10 minutes. After LC-MS monitoring indicated completion, the organic phase was concentrated to afford the crude product **A174-13,** which was used directly in the next step.
LCMS: [M+H]⁺ = 1045.1 (one-third peak)

### Synthesis of Target Compound A174

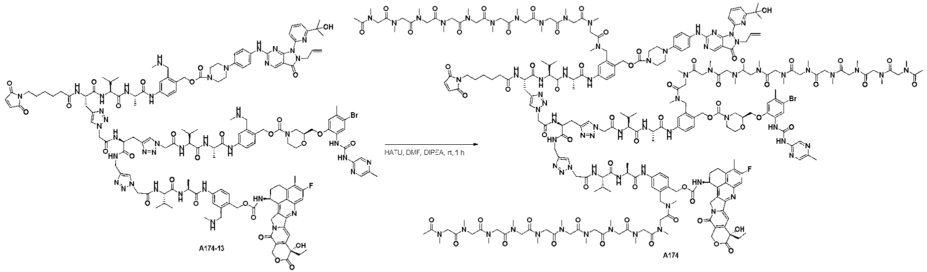

The crude product **A174-13** from the previous step was dissolved in DMF (5 mL). **Ac-PSAR10-OH** (148 mg, 0.19 mmol, 3.0 eq) was added. Then, under an ice bath, N,N-Diisopropylethylamine (249 mg, 1.92 mmol, 30.0 eq) and O-(7-Azabenzotriazol-1-yl)-N,N,N',N'-tetramethyluronium hexafluorophosphate (76 mg, 0.199 mmol, 3.1 eq) were added to the reaction mixture. After the addition was complete, the reaction mixture was stirred under the ice bath for 1 hour. After LC-MS monitoring indicated completion, EA (75 mL) was added to the reaction mixture. The mixture was filtered. The filter cake was dissolved in DMF and purified by prep-HPLC (mobile phase: 0.1% HCOOH/CH₃CN/H₂O) to afford compound **A174** (74.2 mg, 22%) as a white solid.
LCMS: [M+H]⁺ = 1798.1 (one-third peak)

### (82) Synthesis of Compound A180

### Synthetic Route:

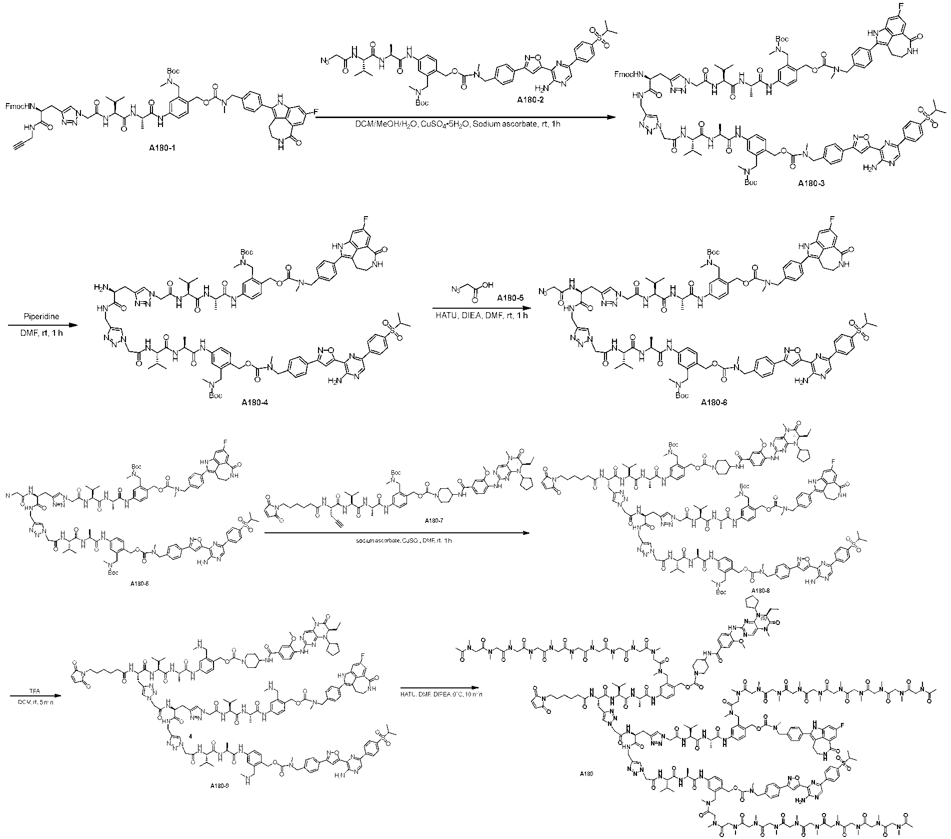

### Synthetic Steps:

### Synthesis of Intermediate Compound A180-3

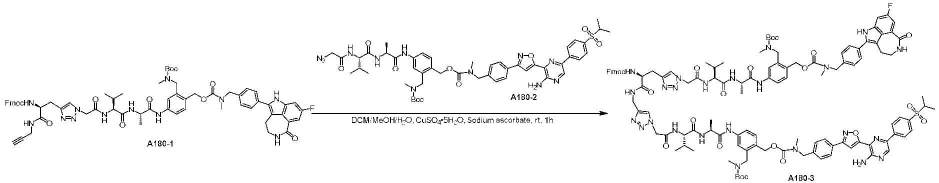

Compound **A180-1** (246 mg, 0.198 mmol, 1.0 eq) and compound **A180-2** (200 mg, 0.198 mmol, 1.0 eq) were dissolved in dichloromethane/methanol (12/12 mL). Under a nitrogen atmosphere, an aqueous solution of copper(II) sulfate pentahydrate (9.9 mg, 0.0396 mmol, 0.2 eq, in 1 mL water) and an aqueous solution of sodium ascorbate (15.7 mg, 0.0723 mmol, 0.4 eq, in 1 mL water) were added. After the addition was complete, the reaction mixture was stirred at room temperature under a nitrogen atmosphere for 1 hour. After LC-MS monitoring indicated completion, the reaction mixture was concentrated directly. The residue was dissolved in tetrahydrofuran and concentrated again to dryness. The obtained crude product was triturated with ethyl acetate, filtered, and dried to afford compound **A180-3** (380 mg, 85%) as a yellow solid.
LCMS: [M+Na]⁺ = 1147.4 (half peak)

### Synthesis of Intermediate Compound A180-4

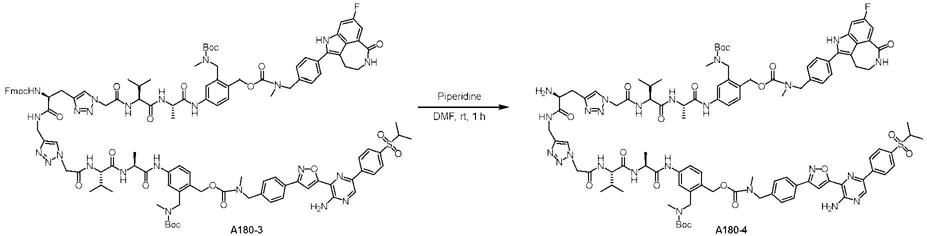

Compound **A180-3** (380 mg, 0.168 mmol, 1.0 eq) was dissolved in DMF (5 mL). Under a nitrogen atmosphere, piperidine (1 mL) was added. The reaction mixture was stirred at room temperature under a nitrogen atmosphere for 1 hour. After LC-MS monitoring indicated completion, methyl tert-butyl ether (50 mL) was added to the reaction mixture. The resulting suspension was filtered directly. The filter cake was washed with methyl tert-butyl ether and dried directly to afford compound **A180-4** (300 mg, 88%) as a yellow solid.
LCMS: [M+H]⁺ = 1014.5 (half peak)

### Synthesis of Intermediate Compound A180-6

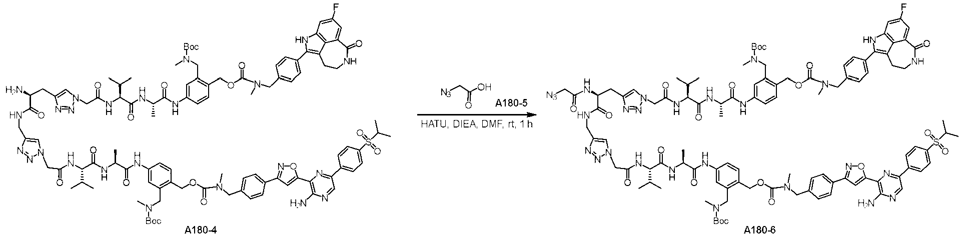

Compound **A180-4** (350 mg, 0.172 mmol, 1.0 eq) was dissolved in DMF (6 mL). N,N-Diisopropylethylamine (67 mg, 0.517 mmol, 3.0 eq) and **A180-5** (28 mg, 0.276 mmol, 1.6 eq) were added to the reaction mixture. Finally, under a nitrogen atmosphere, O-(7-Azabenzotriazol-1-yl)-N,N,N',N'-tetramethyluronium hexafluorophosphate (111 mg, 0.293 mmol, 1.7 eq) was added. After the addition was complete, the reaction mixture was stirred at room temperature under a nitrogen atmosphere for 1 hour. After LC-MS monitoring indicated completion, methyl tert-butyl ether (80 mL) was added to the reaction mixture. The resulting suspension was filtered directly. The filter cake was washed with methyl tert-butyl ether, dried directly, triturated with ethyl acetate, filtered, and dried to afford compound **A180-6** (360 mg, 98%) as a yellow solid.
LCMS: [M+Na]⁺ = 1078.4 (half peak)

### Synthesis of Intermediate Compound A180-8

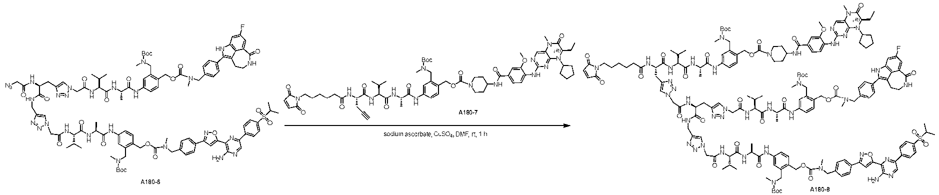

Compound **A180-6** (180 mg, 0.085 mmol, 1.0 eq) and compound **A180-7** (107 mg, 0.085 mmol, 1.0 eq) were dissolved in DMF (4 mL). Under a nitrogen atmosphere, an aqueous solution of copper(II) sulfate pentahydrate (4.3 mg, 0.017 mmol, 0.2 eq, in 100 µL water) and an aqueous solution of sodium ascorbate (6.8 mg, 0.034 mmol, 0.4 eq, in 100 µL water) were added. After the addition was complete, the reaction mixture was stirred at room temperature under a nitrogen atmosphere for 1 hour. After LC-MS monitoring indicated completion, the reaction mixture was diluted with ethyl acetate. The resulting suspension was filtered directly. The filter cake was washed with ethyl acetate and dried directly to afford compound **A180-8** (200 mg, 69%) as a yellow solid.
LCMS: [M+H]⁺ = 1685.6 (half peak)

### Synthesis of Intermediate Compound A180-9

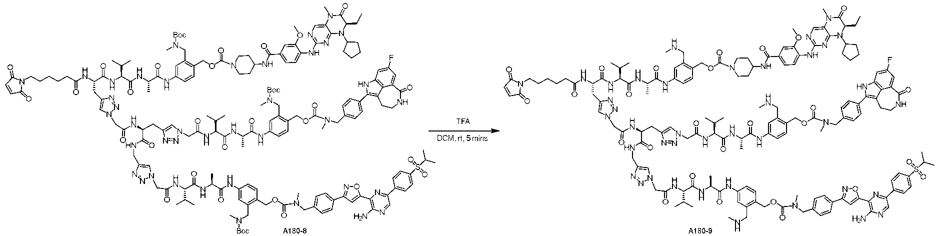

Compound **A180-8** (200 mg, 0.0593 mmol, 1.0 eq) was dissolved in DCM (3 mL). Trifluoroacetic acid (1 mL) was added to the reaction mixture. The reaction mixture was stirred at room temperature for 5 minutes. After LC-MS monitoring indicated completion, the organic phase was concentrated to afford the crude product **A180-9,** which was used directly in the next step.
LCMS: [M+H]⁺ = 1535.1 (half peak)

### Synthesis of Target Compound A180

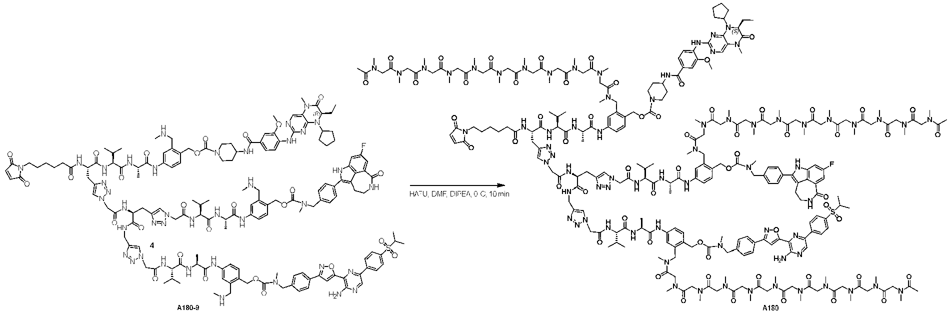

The compound **A180-9** from the previous step was dissolved in DMF (5 mL). **Ac-PSAR10-OH** (137 mg, 0.178 mmol, 3.0 eq) was added to the reaction mixture. Finally, under a nitrogen atmosphere, N,N-Diisopropylethylamine (229 mg, 1.779 mmol, 30.0 eq) and O-(7-Azabenzotriazol-1-yl)-N,N,N',N'-tetramethyluronium hexafluorophosphate (74 mg, 0.196 mmol, 3.3 eq) were added. After the addition was complete, the reaction mixture was stirred at 0 °C under a nitrogen atmosphere for 10 minutes. After LC-MS monitoring indicated completion, ethyl acetate (45 mL) was added to the reaction mixture. The resulting suspension was filtered. The filter cake was dissolved in DMF and purified by prep-HPLC (mobile phase: 0.05% HCOOH/CH₃CN/H₂O) to afford compound **A180** (28 mg, 9%) as a yellow solid.
LCMS: [M+H]⁺ = 1776.5 (one-third peak)

### (83) Synthesis of Compound A181

### Synthetic Route:

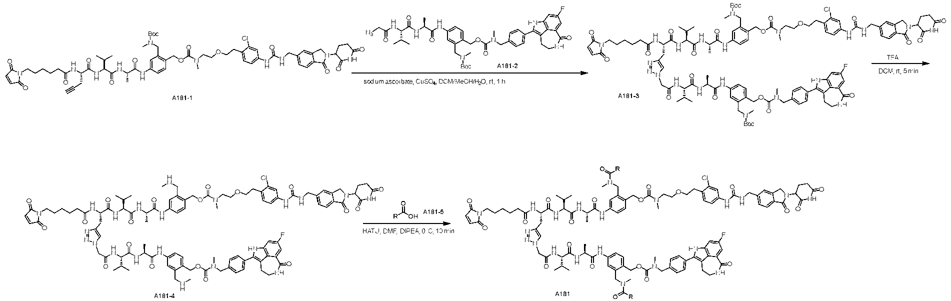

### Synthetic Steps:

### Synthesis of Intermediate Compound A181-3

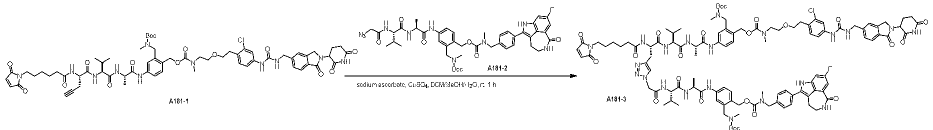

Compound A181-1 (140 mg, 0.109 mmol, 1.0 eq) and compound A181-2 (95 mg, 0.109 mmol, 1.0 eq) were dissolved in MeOH/DCM/water (5/5/1 mL). Then, an aqueous solution of copper(II) sulfate pentahydrate (5.5 mg, 0.0219 mmol, 0.2 eq, in 100 µL water) and an aqueous solution of sodium ascorbate (8.7 mg, 0.0438 mmol, 0.4 eq, in 100 µL water) were added. After the addition was complete, the reaction mixture was stirred at room temperature under a nitrogen atmosphere for 1 hour. After LC-MS monitoring indicated completion, the reaction mixture was concentrated directly. The residue was dissolved in tetrahydrofuran and concentrated again to dryness to afford compound A181-3 (230 mg, 98%) as a yellow solid.
LCMS: [M+H]⁺ = 1074.3 (half peak)

### Synthesis of Intermediate Compound A181-4

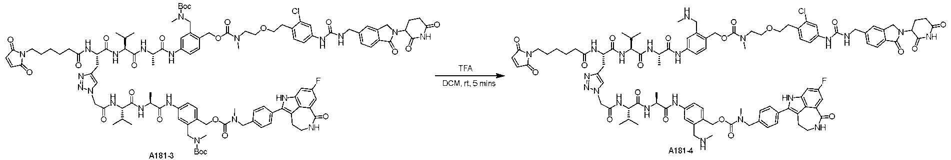

Compound A181-3 (220 mg, 0.102 mmol, 1.0 eq) was dissolved in DCM (3 mL). Trifluoroacetic acid (1 mL) was added to the reaction mixture. The reaction mixture was stirred at room temperature for 5 minutes. After LC-MS monitoring indicated completion, the organic phase was concentrated to afford the crude product A181-4, which was used directly in the next step.
LCMS: [M+H]⁺ = 974.3 (half peak)

### Synthesis of Target Compound A181

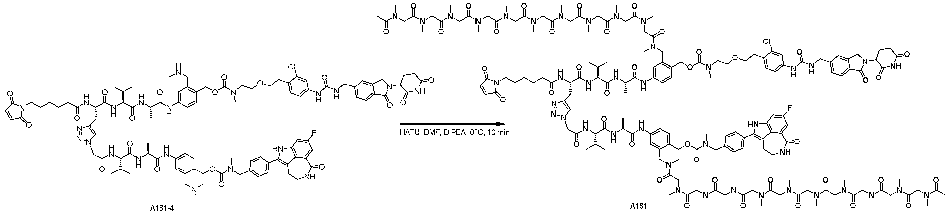

The compound A181-4 from the previous step was dissolved in DMF (3 mL). Compound A181-5 (158 mg, 0.205 mmol, 2.0 eq) was added to the reaction mixture. Finally, under a nitrogen atmosphere, N,N-Diisopropylethylamine (364 mg, 2.046 mmol, 20.0 eq) and O-(7-Azabenzotriazol-1-yl)-N,N,N',N'-tetramethyluronium hexafluorophosphate (82 mg, 0.215 mmol, 2.1 eq) were added. After the addition was complete, the reaction mixture was stirred at 0 °C under a nitrogen atmosphere for 10 minutes. After LC-MS monitoring indicated completion, ethyl acetate (45 mL) was added to the reaction mixture. The mixture was filtered. The filter cake was dissolved in DMF and purified by prep-HPLC (mobile phase: 0.05% HCOOH/CH₃CN/H₂O) to afford compound A181 (77.8 mg, 22%) as a white solid.
LCMS: [M+H]⁺ = 1726.7 (half peak)

### (84) Synthesis of Compound A182

### Synthetic Route:

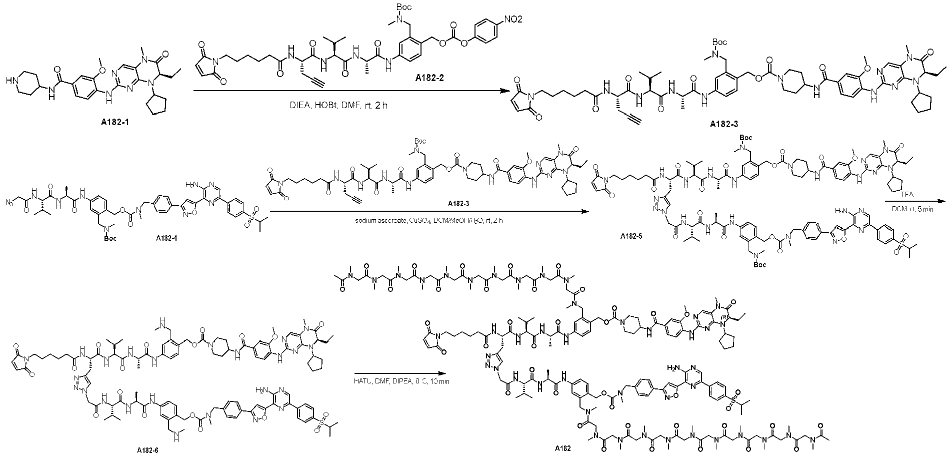

### Synthetic Steps:

### Synthesis of Intermediate Compound A182-3

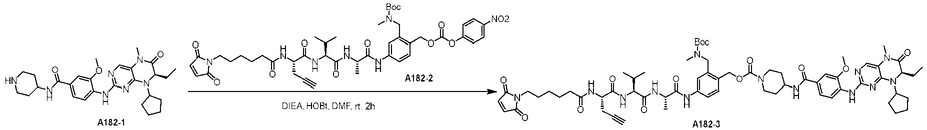

Compound **A182-1** (400 mg, 0.788 mmol, 1.0 eq) was dissolved in DMF (5 mL). N,N-Diisopropylethylamine (510 mg, 3.94 mmol, 5.0 eq) was added and stirred until clear. Then, compound **A182-2** (632 mg, 0.702 mmol, 0.9 eq) and HOBt (52 mg, 0.394 mmol, 0.5 eq) were added. After the addition was complete, the reaction mixture was stirred at room temperature for 2 hours. After LC-MS monitoring indicated completion, the reaction mixture was diluted with ethyl acetate and washed with water and saturated brine. The organic phase was dried over anhydrous sodium sulfate, filtered, and concentrated. The residue was purified by silica gel column chromatography (DCM/MeOH=93/7) to afford compound **A182-3** (600 mg, 60%) as a white solid.
LCMS: [M+H]⁺ = 1259.5

### Synthesis of Intermediate Compound A182-5

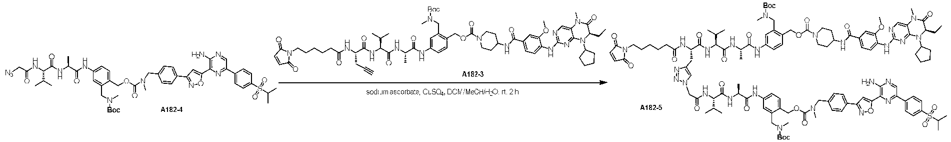

Compound **A182-3** (150 mg, 0.149 mmol, 1.0 eq) and compound **A182-4** (187 mg, 0.149 mmol, 1.0 eq) were dissolved in MeOH/DCM/water (5/5/1 mL). Then, an aqueous solution of copper(II) sulfate pentahydrate (7.4 mg, 0.0297 mmol, 0.2 eq, in 100 µL water) and an aqueous solution of sodium ascorbate (11.8 mg, 0.0594 mmol, 0.4 eq, in 100 µL water) were added. After the addition was complete, the reaction mixture was stirred at room temperature for 2 hours. After LC-MS monitoring indicated completion, the reaction mixture was concentrated directly. The residue was dissolved in tetrahydrofuran and concentrated again to dryness to afford compound **A182-5** (330 mg, 98%) as a yellow solid.
LCMS: [M+H]⁺ = **1134.1** (half peak)

### Synthesis of Intermediate Compound A182-6

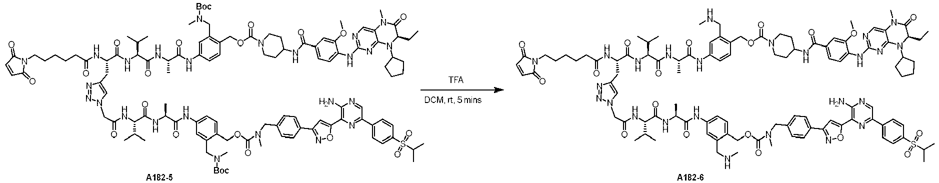

Compound **A182-5** (160 mg, 0.0705 mmol, 1.0 eq) was dissolved in DCM (3 mL). Trifluoroacetic acid (1 mL) was added to the reaction mixture. The reaction mixture was stirred at room temperature for 5 minutes. After LC-MS monitoring indicated completion, the organic phase was concentrated to afford the crude product **A182-6,** which was used directly in the next step.
LCMS: [M+H]⁺ = 1034.1 (half peak)

### Synthesis of Target Compound A182

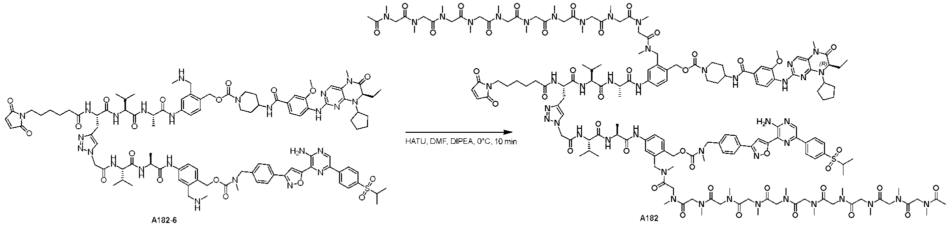

The compound **A182-6** from the previous step was dissolved in DMF (3 mL). **Ac-PSAR10-OH** (109 mg, 0.141 mmol, 2.0 eq) was added to the reaction mixture. Finally, under a nitrogen atmosphere, N,N-Diisopropylethylamine (182 mg, 1.41 mmol, 20.0 eq) and O-(7-Azabenzotriazol-1-yl)-N,N,N',N'-tetramethyluronium hexafluorophosphate (57 mg, 0.148 mmol, 2.1 eq) were added. The reaction mixture was stirred under an ice bath in a nitrogen atmosphere for 10 minutes. After LC-MS monitoring indicated completion, ethyl acetate (60 mL) was added to the reaction mixture. The resulting suspension was filtered. The filter cake was dissolved in DMF and purified by prep-HPLC (mobile phase: 0.05% HCOOH/CH₃CN/H₂O) to afford compound **A182** (45.9 mg, 18%) as a yellow solid.
LCMS: [M+H]⁺ = 1786.8 (half peak)

### (84) Synthesis of Compound A183

### Synthetic Route:

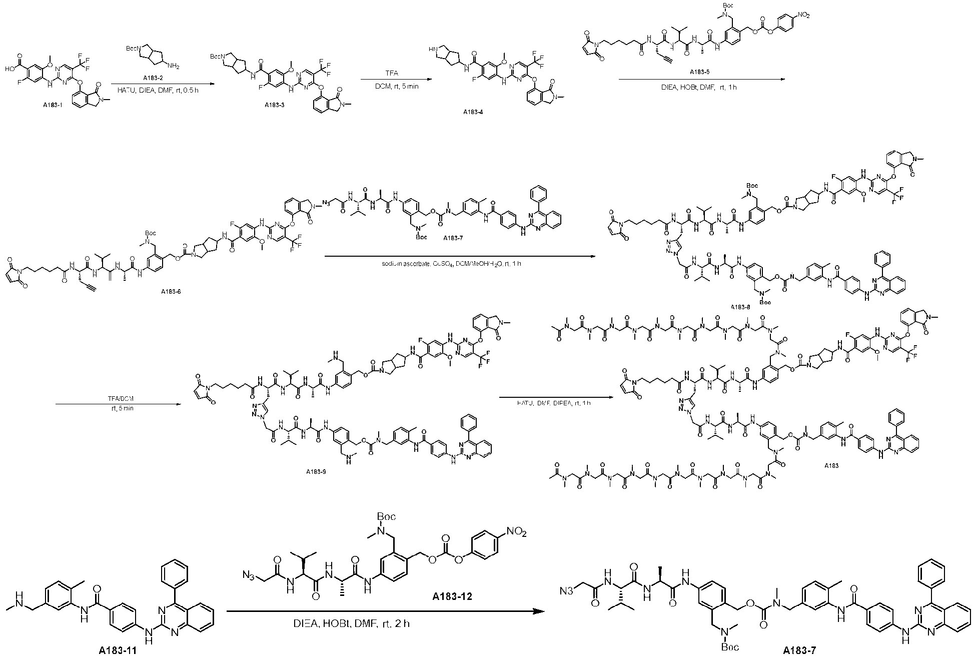

### Synthetic Steps:

### Synthesis of Intermediate Compound A182-7

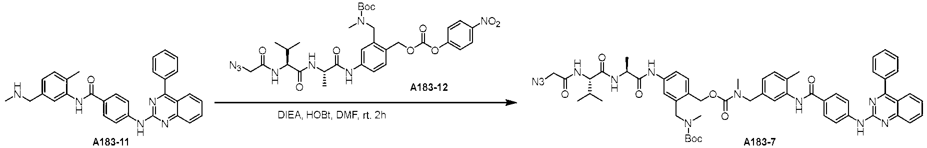

Compound A182-11 (240 mg, 0.507 mmol, 1.0 eq) was dissolved in DMF (5 mL). N,N-Diisopropylethylamine (197 mg, 1.520 mmol, 3.0 eq) was added and stirred until clear. Then, compound A182-12 (348 mg, 0.507 mmol, 1.0 eq) and HOBt (34 mg, 0.253 mmol, 0.5 eq) were added. After the addition was complete, the reaction mixture was stirred at room temperature for 2 hours. After LC-MS monitoring indicated completion, the reaction mixture was diluted with ethyl acetate and washed with water and saturated brine. The organic phase was dried over anhydrous sodium sulfate, filtered, and concentrated. The residue was purified by silica gel column chromatography (DCM/MeOH=93/7) to afford compound A182-7 (410 mg, 79%) as a white solid.
LCMS: [M+H]⁺ = 1019.3

### Synthesis of Intermediate Compound A183-3

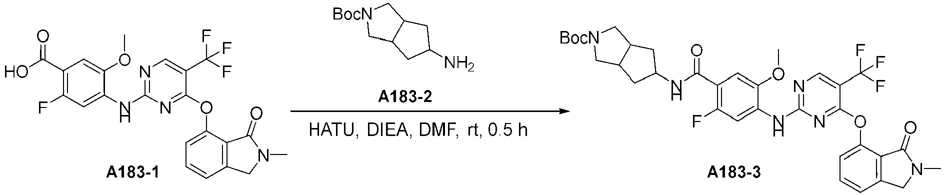

Compound A183-1 (472 mg, 0.959 mmol, 1.0 eq) was dissolved in DMF (10 mL). A183-2 (217 mg, 0.959 mmol, 1.0 eq) was added. At room temperature, N,N-Diisopropylethylamine (372 mg, 1.054 mmol, 3.0 eq) and O-(7-Azabenzotriazol-1-yl)-N,N,N',N'-tetramethyluronium hexafluorophosphate (401 mg, 2.876 mmol, 1.1 eq) were added to the reaction mixture. After the addition was complete, the reaction mixture was stirred at room temperature for 0.5 hour. After LC-MS monitoring indicated completion, water (100 mL) was added to the reaction mixture. The mixture was extracted with ethyl acetate. The organic phase was washed with water and saturated brine, dried over anhydrous sodium sulfate, filtered, and concentrated. The residue was purified by silica gel column chromatography (DCM/MeOH = 0~10%) to afford compound A183-3 (633 mg, 94%) as a white solid.

LCMS: [M+H]⁺ = 701.4

### Synthesis of Intermediate Compound A183-4

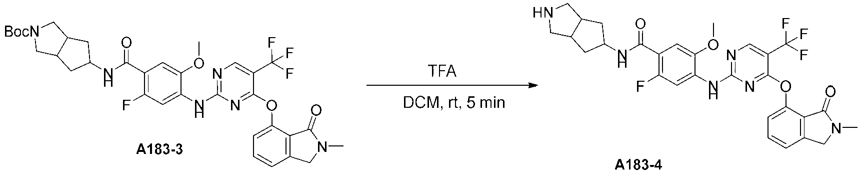

Compound A183-3 (300 mg, 0.428 mmol, 1.0 eq) was dissolved in DCM (3 mL). Trifluoroacetic acid (1 mL) was added to the reaction mixture. The reaction mixture was stirred at room temperature for 5 minutes. After LC-MS monitoring indicated completion, the organic phase was concentrated to afford the crude product A183-4, which was used directly in the next step.

LCMS: [M+H]⁺ = 601.3

### Synthesis of Intermediate Compound A183-6

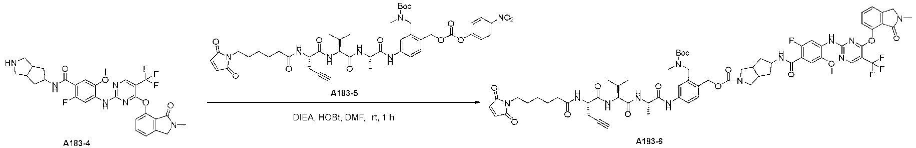

The compound A183-4 was dissolved in DMF (10 mL). At room temperature, N,N-Diisopropylethylamine (129 mg, 0.999 mmol, 2.0 eq) was added to the reaction mixture. Then, A183-5 (400 mg, 0.449 mmol, 0.9 eq) and HOBt (34 mg, 0.249 mmol, 0.5 eq) were added. After the addition was complete, the reaction mixture was stirred at room temperature for 1 hour. After LC-MS monitoring indicated completion, water (100 mL) was added to the reaction mixture. The mixture was extracted with ethyl acetate. The organic phase was washed with water and saturated brine, dried over anhydrous sodium sulfate, filtered, and concentrated. The residue was purified by silica gel column chromatography (DCM/MeOH = 0~10%) to afford compound A183-6 (633 mg, 94%) as a white solid.

LCMS: [M+H]⁺ = 1351.4

### Synthesis of Intermediate Compound A183-8

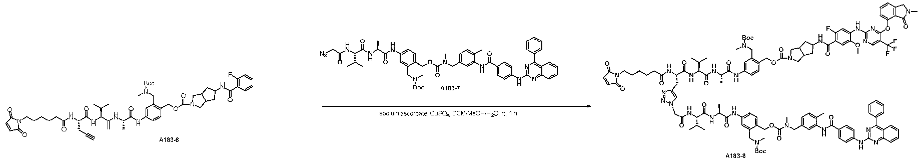

Compound A183-6 (200 mg, 0.148 mmol, 1.05 eq) and compound A183-7 (159 mg, 0.140 mmol, 1.0 eq) were dissolved in MeOH/DCM (4/4 mL). Then, an aqueous solution (100 µL) of copper(II) sulfate pentahydrate (7.4 mg, 0.028 mmol, 0.2 eq) and an aqueous solution (100 µL) of sodium ascorbate (11.7 mg, 0.056 mmol, 0.4 eq) were added. After the addition was complete, the reaction mixture was stirred at room temperature under a nitrogen atmosphere for 1 hour. After LC-MS monitoring detected the product, the organic phase was concentrated. Tetrahydrofuran was used to azeotrope and dry the residue to afford the crude product A183-8 (320 mg, 96%) as a yellow solid.

LCMS: [M+H]⁺ = 1185.9 (half peak)

### Synthesis of Intermediate Compound A183-9

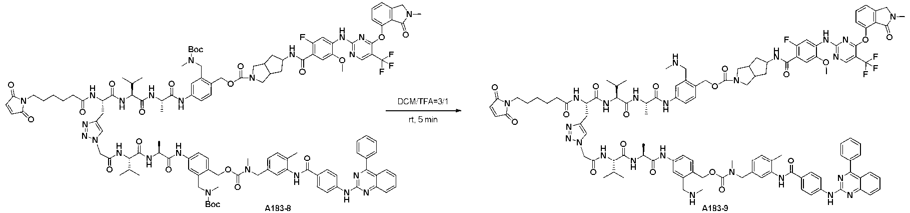

Compound A183-8 (320 mg, 0.135 mmol, 1.0 eq) was dissolved in DCM (3 mL). Trifluoroacetic acid (1 mL) was added to the reaction mixture. The reaction mixture was stirred at room temperature for 5 minutes. After LC-MS monitoring indicated completion, the organic phase was concentrated to afford the crude product A183-9, which was used directly in the next step.

LCMS: [M+H]⁺ = 1085.6 (half peak)

### Synthesis of Target Compound A183

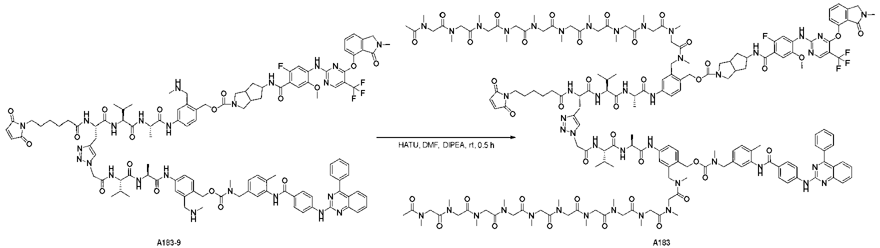

The compound A183-9 from the previous step was dissolved in DMF (4 mL). A183-10 (220 mg, 0.285 mmol, 2.0 eq) was added. Under an ice-water bath, N,N-Diisopropylethylamine (370 mg, 2.853 mmol, 20.0 eq) and O-(7-Azabenzotriazol-1-yl)-N,N,N',N'-tetramethyluronium hexafluorophosphate (110 mg, 0.299 mmol, 2.1 eq) were added to the reaction mixture. After the addition was complete, the reaction mixture was stirred at room temperature under a nitrogen atmosphere for 0.5 hour. After LC-MS monitoring indicated completion, ethyl acetate (60 mL) was added to the reaction mixture. The mixture was filtered. The filter cake was dissolved in DMF and purified by prep-HPLC (mobile phase: 0.05% HCOOH/CH₃CN/H₂O) to afford compound A183-P1 (93.6 mg, 18%) and A183-P2 (53.3 mg, 10%) as white solids.

LCMS: [M+H]⁺ = 1838.2 (half peak)

### (85) Synthesis of Compound A184

### Synthetic Route:

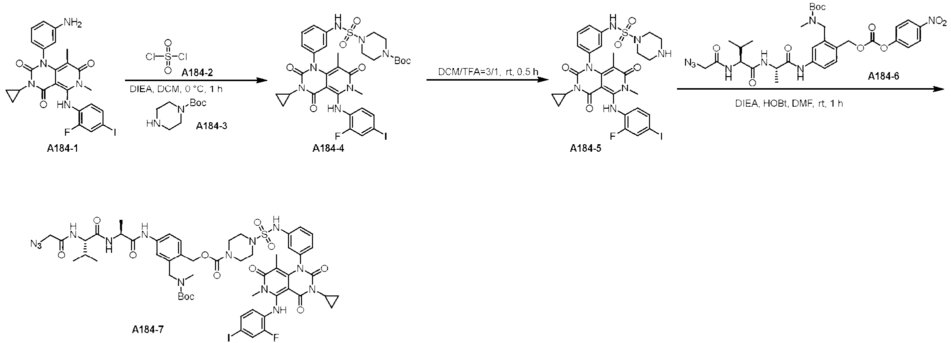

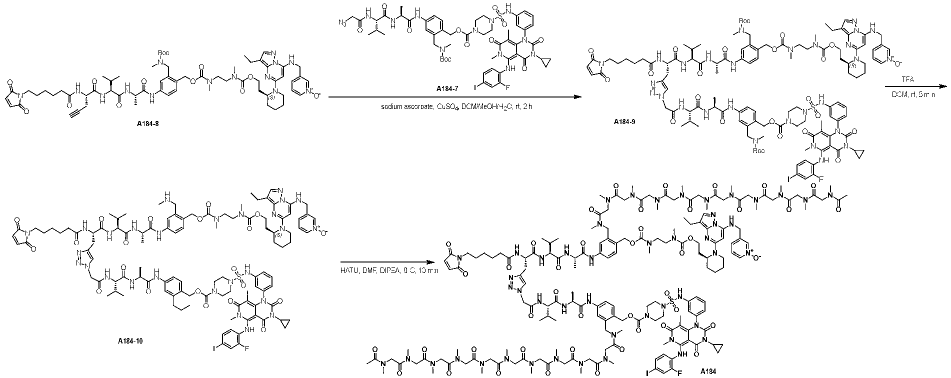

### Synthetic Steps:

### Synthesis of Intermediate Compound A184-4

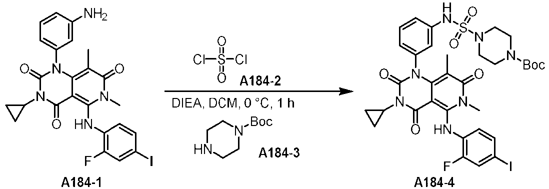

Compound **A184-2** (257 mg, 1.919 mmol, 10.0 eq) and **A184-3** (357 mg, 1.919 mmol, 10.0 eq) were dissolved in super-dry dichloromethane (5 mL). The reaction mixture was stirred in an ice bath for 30 minutes. Compound **A184-1** (110 mg, 0.192 mmol, 1.0 eq) and N,N-Diisopropylethylamine (248 mg, 1.919 mmol, 10.0 eq) were dissolved in super-dry dichloromethane (2 mL). Then, the previous mixture was added to it. After the addition was complete, the reaction mixture was stirred in an ice bath for 30 minutes. After LC-MS monitoring indicated completion, the reaction mixture was concentrated directly. The residue was dissolved in methanol and purified by reversed-phase purification (0.1% FA in water/acetonitrile=40/60) to afford compound **A184-4** (130 mg, 82%) as a white solid.
LCMS: [M+H]⁺ = 822.2

### Synthesis of Intermediate Compound A184-5

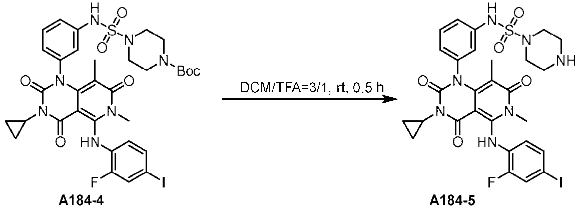

Compound **A184-4** (120 mg, 0.146 mmol, 1.0 eq) was dissolved in DMA (3 mL). Trifluoroacetic acid (1 mL) was added to the reaction mixture. The reaction mixture was stirred at room temperature for 0.5 hour. After LC-MS monitoring indicated completion, the organic phase was concentrated to afford the crude product **A184-5,** which was used directly in the next step.
LCMS: [M+H]⁺ = 722.0

### Synthesis of Compound A184-7

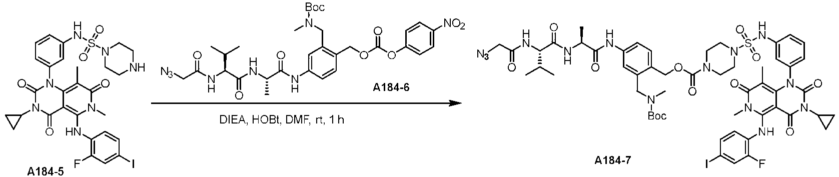

The compound **A184-5** from the previous step was dissolved in DMF (3 mL). N,N-Diisopropylethylamine (95 mg, 0.730 mmol, 5.0 eq) was added and stirred until clear. Then, compound **A184-6** (130 mg, 0.146 mmol, 1.0 eq) and HOBt (10 mg, 0.0730 mmol, 0.5 eq) were added. After the addition was complete, the reaction mixture was stirred at room temperature for 1 hour. After LC-MS monitoring indicated completion, the reaction mixture was diluted with ethyl acetate and washed with water and saturated brine. The organic phase was dried over anhydrous sodium sulfate, filtered, and concentrated. The residue was purified by silica gel column chromatography (DCM/MeOH=93/7) to afford compound A184-7 (140 mg, 76%) as a white solid.
LCMS: [M+H]⁺ = 1267.2

### Synthesis of Compound A184-9

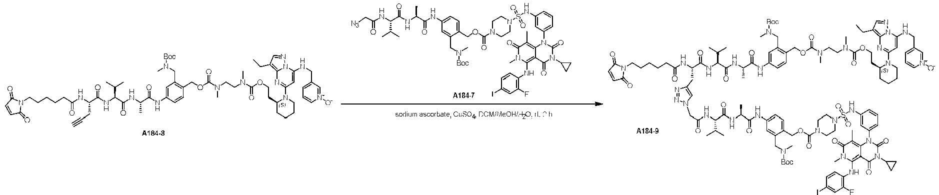

Compound **A184-8** (120 mg, 0.0951 mmol, 1.0 eq) and compound **A184-7** (121 mg, 0.0951 mmol, 1.0 eq) were dissolved in MeOH/DCM/water (5/5/1 mL). Then, an aqueous solution of copper(II) sulfate pentahydrate (4.8 mg, 0.0190 mmol, 0.2 eq, in 100 µL water) and an aqueous solution of sodium ascorbate (7.6 mg, 0.0380 mmol, 0.4 eq, in 100 µL water) were added. After the addition was complete, the reaction mixture was stirred at room temperature under a nitrogen atmosphere for 2 hours. After LC-MS monitoring indicated completion, the reaction mixture was concentrated directly. The residue was dissolved in tetrahydrofuran and concentrated again to dryness to afford compound **A184-9** (240 mg, 100%) as a yellow solid.
LCMS: [M+H]⁺ = 1265.0 (half peak)

### Synthesis of Compound A184-10

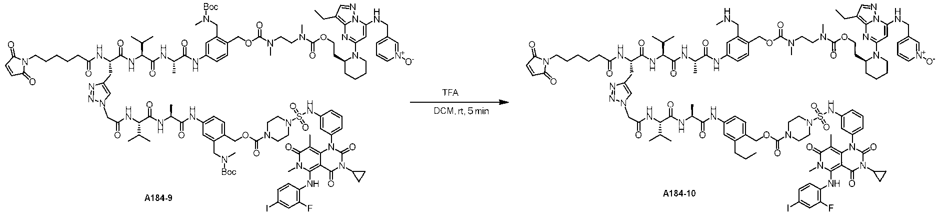

Compound **A184-9** (230 mg, 0.0909 mmol) was dissolved in DCM (3 mL). Trifluoroacetic acid (1 mL) was added to the reaction mixture. The reaction mixture was stirred at room temperature for 5 minutes. After LC-MS monitoring indicated completion, the organic phase was concentrated to afford the crude product **A184-10,** which was used directly in the next step.
LCMS: [M+H]⁺ = 1164.8 (half peak)

### Synthesis of Compound A184

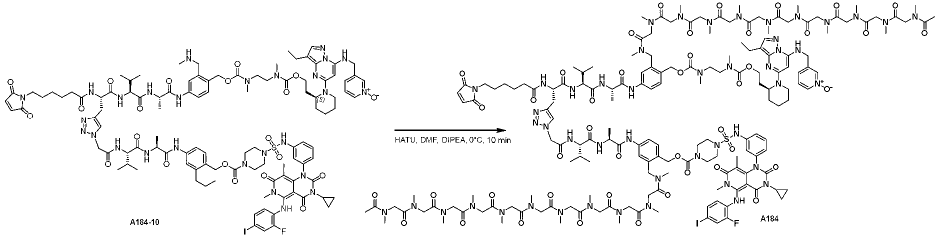

The compound **A184-10** from the previous step was dissolved in DMF (3 mL). Ac-PSAR10-OH (140 mg, 0.182 mmol, 2.0 eq) was added to the reaction mixture. Finally, under a nitrogen atmosphere, N,N-Diisopropylethylamine (235 mg, 1.818 mmol, 20.0 eq) and O-(7-Azabenzotriazol-1-yl)-N,N,N',N'-tetramethyluronium hexafluorophosphate (73 mg, 0.191 mmol, 2.1 eq) were added. After the addition was complete, the reaction mixture was stirred at 0 °C under a nitrogen atmosphere for 10 minutes. After LC-MS monitoring indicated completion, ethyl acetate (45 mL) was added to the reaction mixture. The mixture was filtered. The filter cake was dissolved in DMF and purified by prep-HPLC (mobile phase: 0.05% HCOOH/CH₃CN/H₂O) to afford compound **A184** (63.3 mg, 18%) as a white solid.
LCMS: [M+H]⁺ = 1918.1 (half peak)

### (86) Synthesis of Compound A185

### Synthetic Route:

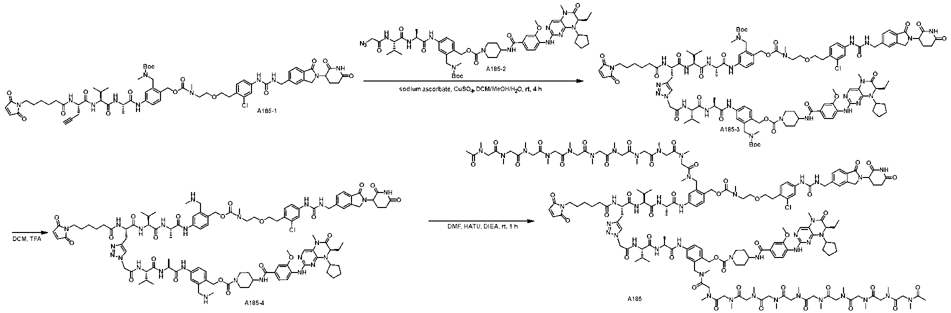

### Synthetic Steps:

### Synthesis of Intermediate Compound A185-3

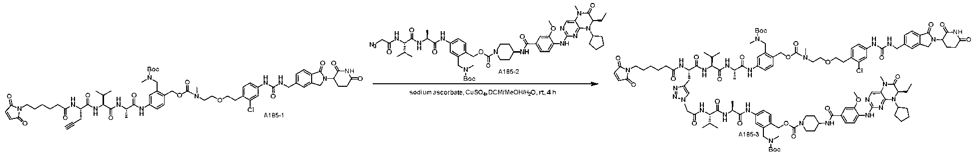

Compound **A185-1** (164 mg, 0.128 mmol, 0.9 eq) was dissolved in dichloromethane/methanol (4 mL, 1:1). **A185-2** (150 mg, 0.142 mmol, 1 eq), an aqueous solution (100 µL) of copper(II) sulfate pentahydrate (7 mg, 0.028 mmol, 0.2 eq), and an aqueous solution (100 µL) of sodium ascorbate (11 mg, 0.057 mmol, 0.4 eq) were added. The mixture was reacted at room temperature under a nitrogen atmosphere for 4 hours. After the reaction was complete, the mixture was concentrated under reduced pressure to afford the crude product **A185-3** (200 mg, 85 %) as a yellow solid.

LCMS: [M+Na]⁺ = 1189.1 (half peak)

### Synthesis of Intermediate Compound A185-4

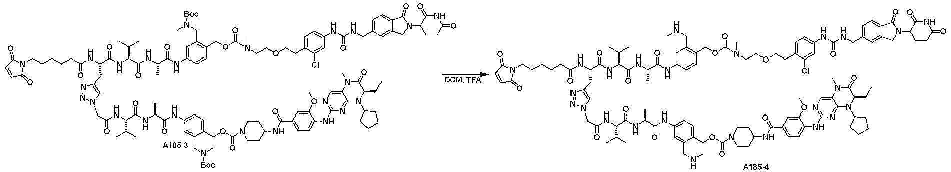

Compound **A185-3** (150 mg, 0.064 mmol, 1 eq) was dissolved in dichloromethane (3 mL). Trifluoroacetic acid (1 mL) was added, and the mixture was reacted at room temperature for 1 hour. After the reaction was complete, the mixture was concentrated under reduced pressure to afford compound **A185-4** (crude) as a yellow solid.

LCMS: [M+H]⁺ = 1066.0 (half peak)

### Synthesis of Intermediate Compound A185

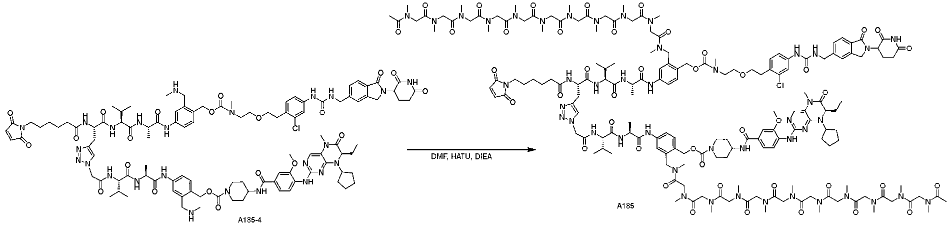

The compound **A185-4** (crude) was dissolved in N,N-dimethylformamide (2 mL). **Ac-PSAR10-OH** (104 mg, 0.135 mmol, 2.1 eq) was added, and the reaction mixture was cooled to 0 °C. N,N-Diisopropylethylamine (166 mg, 1.286 mmol, 20 eq) and HATU (54 mg, 0.141 mmol, 2.2 eq) were added, and the mixture was reacted at room temperature for 1 hour. After the reaction was complete, ethyl acetate (40 mL) was added to the reaction mixture. The product precipitated. The solid was filtered to obtain the crude target compound. The crude product was dissolved in DMF and purified by prep-HPLC (mobile phase: 0.05% HCOOH/CH₃CN/H₂O) to afford compound **A185** (59 mg, 41%) as a white solid.

LCMS: [M+H]⁺ = 1818.8 (half peak)

### (87) Synthesis of Compound A168

### Synthetic Route:

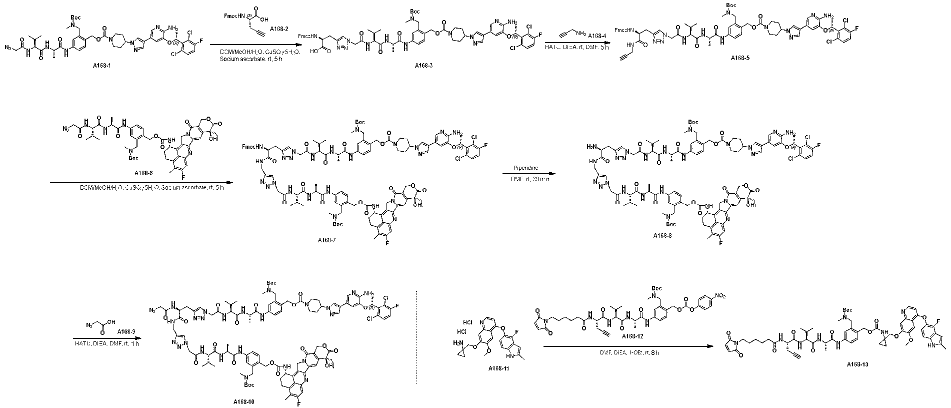

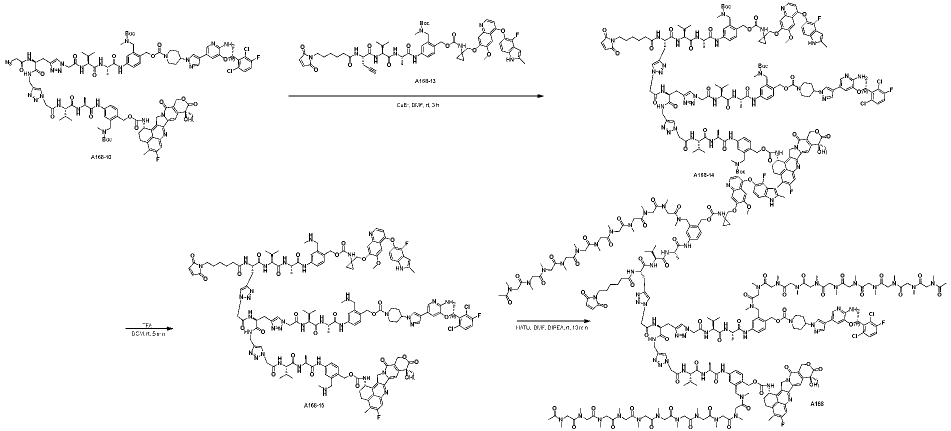

### Synthetic Steps:

### Synthesis of Intermediate Compound A168-3

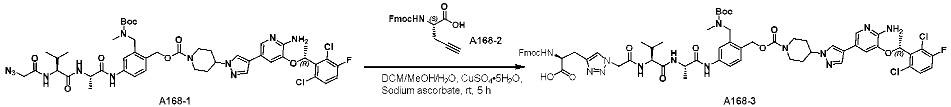

Compound **A168-1** (500 mg, 0.50 mmol, 1.0 eq) and compound **A168-2** (185 mg, 0.55 mmol, 1.1 eq) were dissolved in dichloromethane/methanol (5/5 mL). Then, an aqueous solution (250 µL) of copper(II) sulfate pentahydrate (25 mg, 0.10 mmol, 0.2 eq) and an aqueous solution (250 µL) of sodium ascorbate (39.7 mg, 0.20 mmol, 0.4 eq) were added. After the addition was complete, the reaction mixture was stirred at room temperature under a nitrogen atmosphere for 5 hours. After LC-MS monitoring indicated completion, the reaction mixture was concentrated directly. Tetrahydrofuran was added to azeotrope and dry the residue. The residue was then triturated with ethyl acetate, filtered, and the filter cake was collected and dried to afford compound **A168-3** (710 mg, 100%) as a purple solid.
LCMS: [M+H]⁺ = 1331.3

### Synthesis of Intermediate Compound A168-5

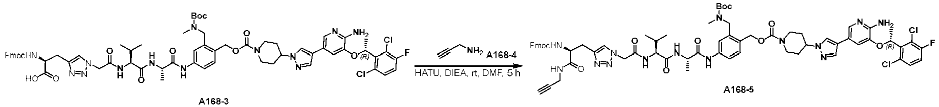

Compound **A168-3** (710 mg, 0.53 mmol, 1.0 eq) was dissolved in DMF (6 mL). N,N-Diisopropylethylamine (207 mg, 1.60 mmol, 3.0 eq) and O-(7-Azabenzotriazol-1-yl)-N,N,N',N'-tetramethyluronium hexafluorophosphate (405 mg, 1.07 mmol, 2.0 eq) were added to the reaction mixture. Finally, compound **A168-4** (88 mg, 1.60 mmol, 3.0 eq) was added. After the addition was complete, the reaction mixture was stirred at room temperature under a nitrogen atmosphere for 5 hours. After LC-MS monitoring indicated completion, 2-methyltetrahydrofuran was added to the reaction mixture for dilution. The mixture was washed with water (60 mL x 2) and then with saturated brine (50 mL x 2). The organic phase was concentrated and then triturated with ethyl acetate, filtered, and the filter cake was collected. The filter cake was then triturated with methanol, filtered again, and the filter cake was collected and dried to afford compound **A168-5** (470 mg, 65%) as a brown solid.
LCMS: [M+H]⁺ = 1367.3

### Synthesis of Intermediate Compound A168-7

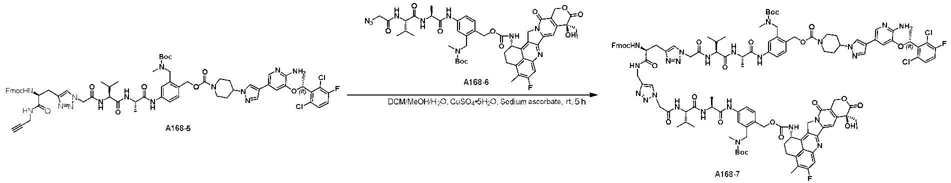

Compound **A168-5** (470 mg, 0.34 mmol, 1.0 eq) and compound **A168-6** (472 mg, 0.48 mmol, 1.4 eq) were dissolved in dichloromethane/methanol (10/8 mL). Then, an aqueous solution (250 µL) of copper(II) sulfate pentahydrate (17 mg, 0.07 mmol, 0.2 eq) and an aqueous solution (250 µL) of sodium ascorbate (27 mg, 0.14 mmol, 0.4 eq) were added. After the addition was complete, the reaction mixture was stirred at room temperature under a nitrogen atmosphere for 5 hours. After LC-MS monitoring indicated completion, the reaction mixture was concentrated directly. Tetrahydrofuran was added multiple times to azeotrope and dry the residue. The residue was then triturated with methanol, filtered, and the filter cake was collected and dried to afford compound **A168-7** (790 mg, 99%) as a brown solid.
LCMS: [M+H]⁺ = 1174.9 (half peak)

### Synthesis of Intermediate Compound A168-8

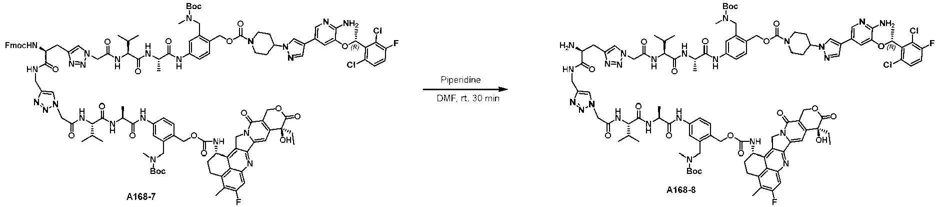

Compound A168-7 (790 mg, 0.34 mmol, 1.0 eq) was dissolved in DMF (8 mL). Piperidine (1.6 mL) was added. The reaction mixture was stirred at room temperature for 30 minutes. After LC-MS monitoring indicated completion, methyl tert-butyl ether was added to the reaction mixture. The resulting suspension was filtered directly. The filter cake was washed with methyl tert-butyl ether, then triturated with ethyl acetate, filtered again, and the filter cake was collected and dried to afford compound **A168-8** (730 mg, 100%) as a brown solid.
LCMS: [M+H]⁺ = 1063.4 (half peak)

### Synthesis of Intermediate Compound A168-10

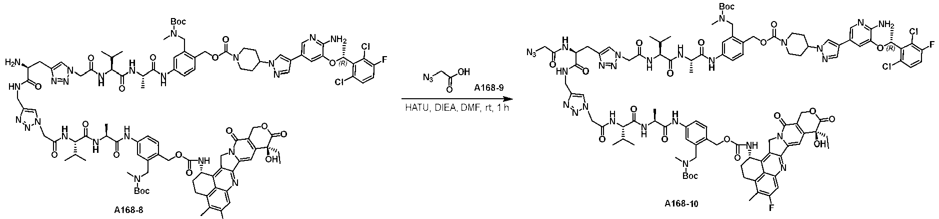

Compound **A168-8** (730 mg, 0.34 mmol, 1.0 eq) was dissolved in DMF (8 mL). N,N-Diisopropylethylamine (133 mg, 1.03 mmol, 3.0 eq) was added, and the mixture was stirred for 1 minute. Then, compound **A168-9** (69 mg, 0.69 mmol, 2.0 eq) and O-(7-Azabenzotriazol-1-yl)-N,N,N',N'-tetramethyluronium hexafluorophosphate (274 mg, 0.72 mmol, 2.1 eq) were added to the reaction mixture. After the addition was complete, the reaction mixture was stirred at room temperature for 1 hour. After LC-MS monitoring indicated completion, methyl tert-butyl ether was added to the reaction mixture. The resulting suspension was filtered directly. The filter cake was washed with methyl tert-butyl ether, then triturated with methanol, filtered again, and the filter cake was collected and dried to afford compound **A168-10** (650 mg, 87%) as a brown solid.
LCMS: [M+H]⁺ = 1105.8 (half peak)

### Synthesis of Intermediate Compound A168-13

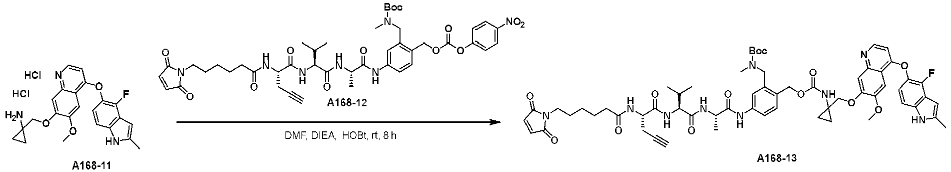

Compound **A168-11** (110 mg, 0.23 mmol, 1.0 eq) was dissolved in DMF (3 mL). N,N-Diisopropylethylamine (177 mg, 1.37 mmol, 6.0 eq) was added, and the mixture was stirred for 2 minutes. Then, compound **A168-12** (173 mg, 0.22 mmol, 0.95 eq) and HOBt (15 mg, 0.11 mmol, 0.5 eq) were added. After the addition was complete, the reaction mixture was stirred at room temperature for 8 hours. After LC-MS monitoring indicated completion, ethyl acetate (80 mL) was added to the reaction mixture for dilution. The mixture was washed with water (40 mL x 2). Solid precipitated when the organic phase was washed with 0.5 M aqueous hydrochloric acid solution (30 mL). The solid was filtered off and collected. The organic phase in the filtrate was separated and dried. The collected filter cake and the organic phase were combined and concentrated. The residue was purified by silica gel column chromatography (DCM/MeOH=94/6) to afford compound **A168-13** (150 mg, 56%) as a yellow solid.
LCMS: [M+H]⁺ = 1158.4

### Synthesis of Intermediate Compound A168-14

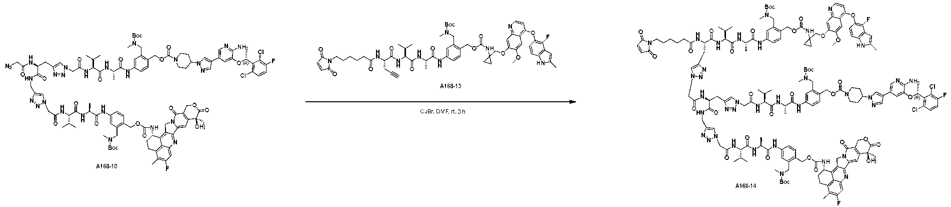

Compound **A168-10** (140 mg, 0.063 mmol, 1.0 eq) and compound **A168-13** (117 mg, 0.101 mmol, 1.6 eq) were dissolved in DMF (5 mL). Then, copper(I) bromide (182 mg, 1.266 mmol, 20.0 eq) was added. After the addition was complete, the reaction mixture was stirred at room temperature under a nitrogen atmosphere for 3 hours. After LC-MS monitoring indicated completion, ethyl acetate was added, causing solid precipitation. The solid was filtered. The filter cake was then triturated with methanol and filtered again. The filter cake was collected and dried to afford the crude product. The crude product was dissolved in DMF and purified by prep-HPLC (mobile phase: 0.1% HCOOH/CH₃CN/H₂O) to afford compound **A168-14** (37 mg, 17%) as a white solid.
LCMS: [M+H]⁺ = 1684.6 (half peak)

### Synthesis of Intermediate Compound A168-15

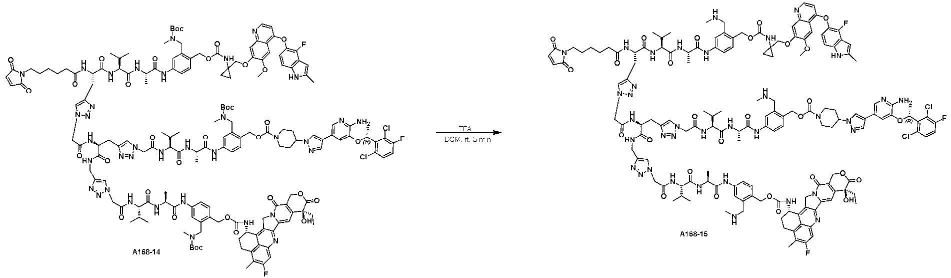

Compound **A168-14** (37 mg, 0.011 mmol, 1.0 eq) was dissolved in DCM (3 mL). Trifluoroacetic acid (1 mL) was added to the reaction mixture. The reaction mixture was stirred at room temperature for 5 minutes. After LC-MS monitoring indicated completion, the organic phase was concentrated to afford the crude product **A168-15,** which was used directly in the next step.
LCMS: [M+H]⁺ = 1534.6 (half peak)

### Synthesis of Target Compound A168

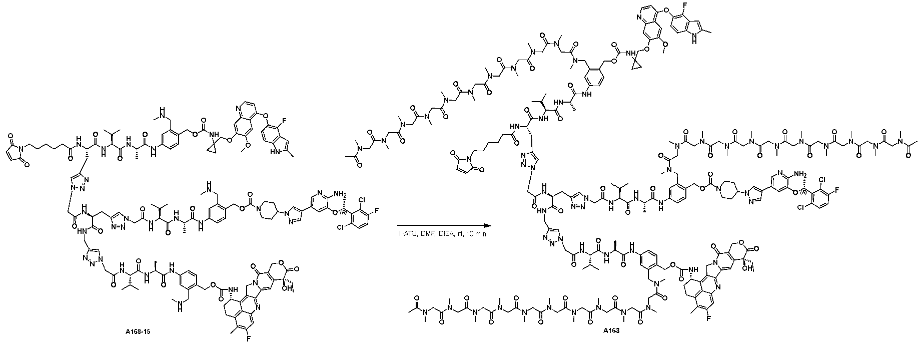

The crude product **A168-15** from the previous step was dissolved in DMF (2 mL). Ac-**PSAR10-OH** (25 mg, 0.033 mmol, 3.0 eq) was added. Then, under an ice bath, N,N-Diisopropylethylamine (43 mg, 0.33 mmol, 30.0 eq) and O-(7-Azabenzotriazol-1-yl)-N,N,N',N'-tetramethyluronium hexafluorophosphate (13 mg, 0.034 mmol, 3.1 eq) were added to the reaction mixture. After the addition was complete, the reaction mixture was stirred under the ice bath for 10 minutes. After LC-MS monitoring indicated completion, EA (30 mL) was added to the reaction mixture. The mixture was filtered. The filter cake was dissolved in DMF and purified by prep-HPLC (mobile phase: 0.1% HCOOH/CH₃CN/H₂O) to afford compound **A168** (13 mg, 22%) as a white solid.
LCMS: [M+H]⁺ = 1775.8 (one-third peak)

### (88) Synthesis of Compound A175

### Synthetic Route:

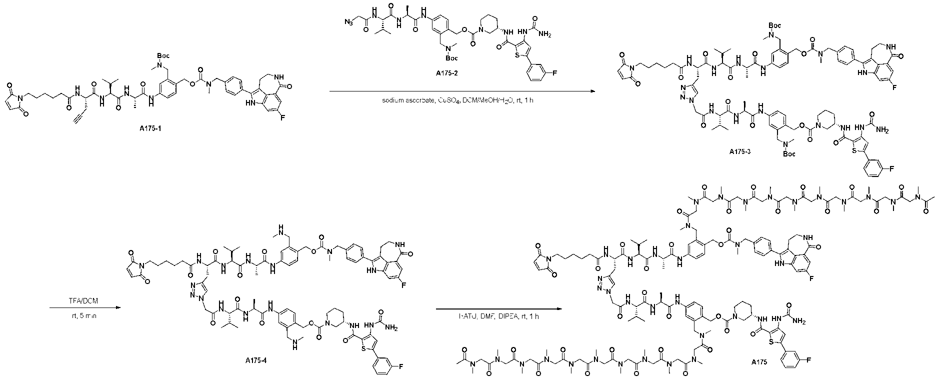

### Synthetic Steps:

### Synthesis of Intermediate Compound A175-3

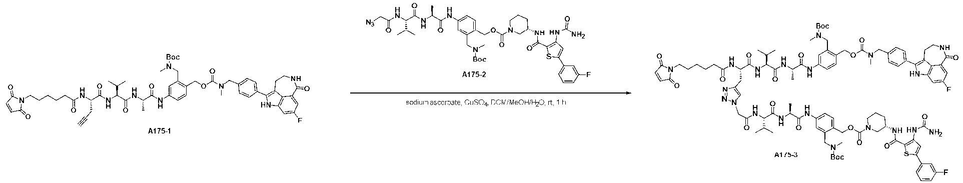

Compound **A175-1** (200 mg, 0.186 mmol, 1.0 eq) and compound **A175-2** (169 mg, 0.186 mmol, 1.0 eq) were dissolved in MeOH/DCM (4/4 mL). Then, an aqueous solution (100 µL) of copper(II) sulfate pentahydrate (9 mg, 0.037 mmol, 0.2 eq) and an aqueous solution (100 µL) of sodium ascorbate (15 mg, 0.074 mmol, 0.4 eq) were added. After the addition was complete, the reaction mixture was stirred at room temperature under a nitrogen atmosphere for 1 hour. After LC-MS monitoring indicated completion, the mixture was concentrated to afford the crude product **A175-3** (330 mg) as a yellow solid.
LCMS: [M+H]⁺ = 991.1 (half peak)

### Synthesis of Intermediate Compound A175-4

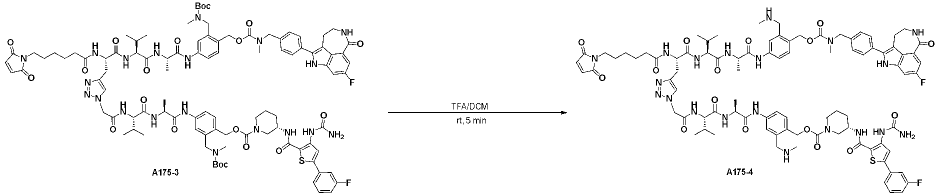

Compound **A175-3** (330 mg, 0.166 mmol, 1.0 eq) was dissolved in DCM (4.5 mL). Trifluoroacetic acid (1.5 mL) was added to the reaction mixture. The reaction mixture was stirred at room temperature for 5 minutes. After LC-MS monitoring indicated completion, the organic phase was concentrated to afford the crude product **A175-4,** which was used directly in the next step.
LCMS: [M+H]⁺ = 891.5 (half peak)

### Synthesis of Target Compound A175

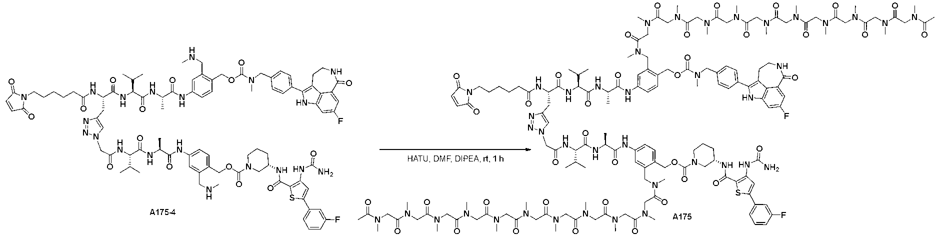

The compound **A175-4** from the previous step was dissolved in DMF (6 mL). **Ac-PSAR10-OH** (269 mg, 0.35 mmol, 2.1 eq) was added. Then, at 0 °C, N,N-Diisopropylethylamine (430 mg, 3.33 mmol, 20.0 eq) and O-(7-Azabenzotriazol-1-yl)-N,N,N',N'-tetramethyluronium hexafluorophosphate (139 mg, 0.37 mmol, 2.2 eq) were added to the reaction mixture. After the addition was complete, the reaction mixture was stirred at 0 °C for 1 hour. After LC-MS monitoring indicated completion, EA (90 mL) was added to the reaction mixture. The mixture was filtered. The filter cake was dissolved in DMF and purified by prep-HPLC (mobile phase: 0.05% HCOOH/CH₃CN/H₂O) to afford compound A175 (209.6 mg, 38%) as a white solid.
LCMS: [M+H]⁺ = 1642.1 (half peak)

### (89) Synthesis of Compound A176

### Synthetic Route:

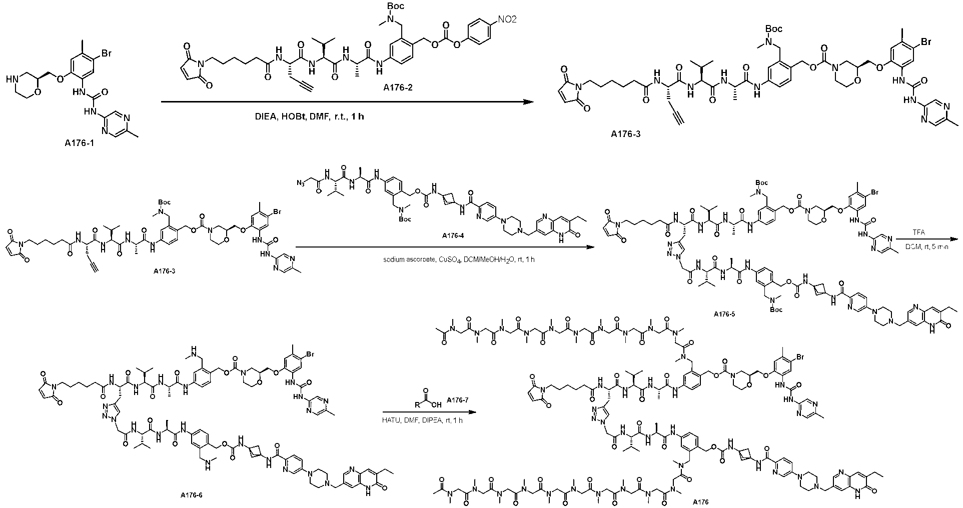

### Synthetic Steps:

### Synthesis of Intermediate Compound A176-3

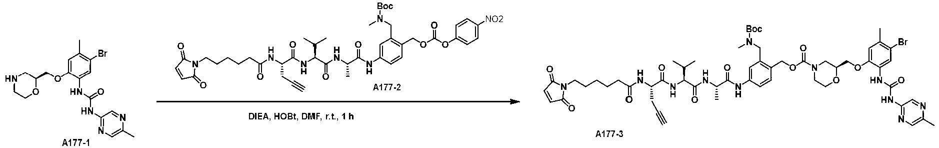

Compound **A176-1** (200 mg, 0.46 mmol, 1.0 eq) and N,N-Diisopropylethylamine (178 mg, 1.38 mmol, 3.0 eq) were dissolved in DMF (5 mL) and stirred until clear. Then, compound **A176-2** (368 mg, 0.41 mmol, 0.9 eq) and HOBt (31 mg, 0.23 mmol, 0.5 eq) were added. After the addition was complete, the reaction mixture was stirred at room temperature for 1 hour. After LC-MS monitoring indicated completion, ethyl acetate (50 mL) was added to the reaction mixture. The mixture was washed twice with water and three times with saturated brine. The organic phase was dried over anhydrous sodium sulfate, filtered, and concentrated. The residue was purified by silica gel column chromatography (DCM/MeOH=94/6) to afford compound **A176-3** (380 mg, 70%) as a pale yellow solid.
LCMS: [M+H]⁺ = 1186.3

### Synthesis of Intermediate Compound A176-5

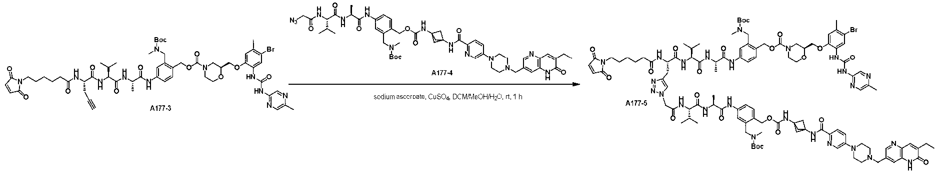

Compound **A176-3** (150 mg, 0.126 mmol, 1.05 eq) and compound **A176-4** (123 mg, 0.120 mmol, 1.0 eq) were dissolved in MeOH/DCM (2/4 mL). Then, an aqueous solution (100 µL) of copper(II) sulfate pentahydrate (3.8 mg, 0.024 mmol, 0.2 eq) and an aqueous solution (100 µL) of sodium ascorbate (9.5 mg, 0.048 mmol, 0.4 eq) were added. After the addition was complete, the reaction mixture was stirred at room temperature under a nitrogen atmosphere for 1 hour. After LC-MS monitoring detected the product, water (20 mL) was added to the reaction mixture. The mixture was extracted with dichloromethane. The organic phase was dried over anhydrous sodium sulfate, filtered, and concentrated to afford the crude product A176-5 (300 mg, 99%) as a yellow solid.
LCMS: [M+H]⁺ = 1104.1 (half peak)

### Synthesis of Intermediate Compound A176-6

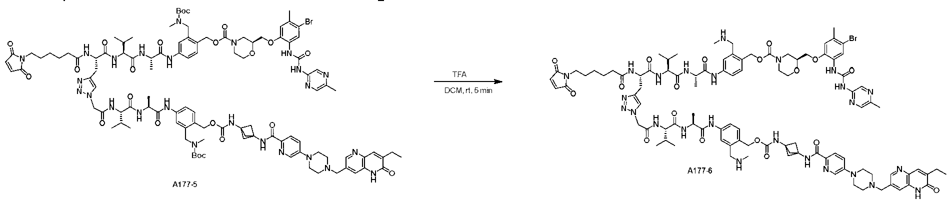

Compound **A176-5** (300 mg, 0.136 mmol, 1.0 eq) was dissolved in DCM (3 mL). Trifluoroacetic acid (1 mL) was added to the reaction mixture. The reaction mixture was stirred at room temperature for 5 minutes. After LC-MS monitoring indicated completion, the organic phase was concentrated to afford the crude product **A176-6,** which was used directly in the next step.
LCMS: [M+H]⁺ = 1003.3 (half peak)

### Synthesis of Target Compound A176

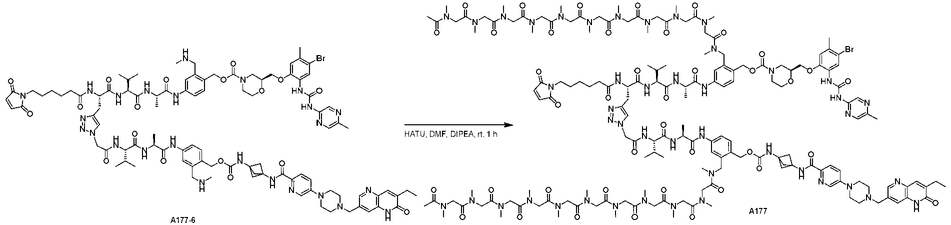

The compound **A176-6** from the previous step was dissolved in DMF (4 mL). N,N-Diisopropylethylamine (109 mg, 0.843 mmol, 20.0 eq) was added. Then, **Ac-PSAR10-OH** (65 mg, 0.084 mmol, 2.0 eq) and O-(7-Azabenzotriazol-1-yl)-N,N,N',N'-tetramethyluronium hexafluorophosphate (34 mg, 0.089 mmol, 2.1 eq) were added to the reaction mixture. After the addition was complete, the reaction mixture was stirred at room temperature under a nitrogen atmosphere for 10 minutes. After LC-MS monitoring indicated completion, EA (60 mL) was added to the reaction mixture. The mixture was filtered. The filter cake was dissolved in DMF and purified by prep-HPLC (mobile phase: 0.1% HCOOH/CH₃CN/H₂O) to afford compound **A176** (84 mg, 37%) as a yellow solid.
LCMS: [M+H]⁺ = 1756.5 (half peak)

### (90) Synthesis of Compound A177

### Synthetic Route:

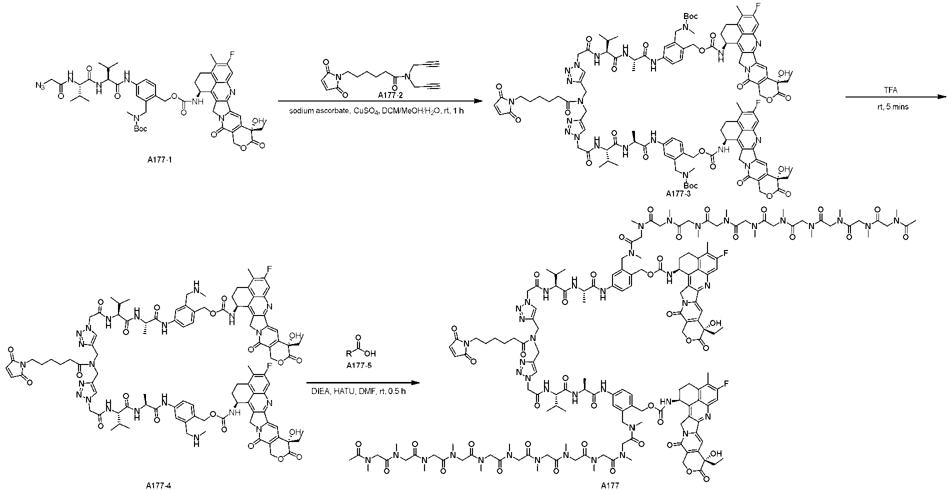

### Synthetic Steps:

### Synthesis of Intermediate Compound A177-3

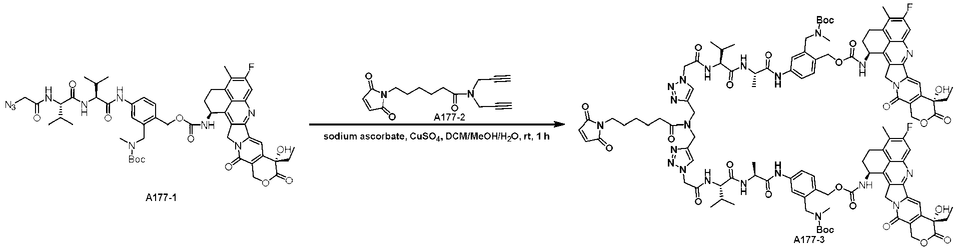

Compound **A177-1** (200 mg, 0.204 mmol, 1.0 eq) and compound **A177-2** (29 mg, 0.102 mmol, 0.5 eq) were dissolved in dichloromethane (4 mL) and methanol (4 mL). Under a nitrogen atmosphere, an aqueous solution of copper(II) sulfate pentahydrate (10 mg, 0.041 mmol, 0.2 eq, in 200 µL water) and an aqueous solution of L-sodium ascorbate (16 mg, 0.082 mmol, 0.4 eq, in 200 µL water) were added. After the addition was complete, the reaction mixture was stirred at room temperature under a nitrogen atmosphere for 1 hour. After LC-MS monitoring indicated completion, the reaction mixture was evaporated to dryness to afford compound **A177-3** (228 mg, 100%) as a yellow solid.
LCMS: [M+H]⁺ = 1146.5 (half peak)

### Synthesis of Intermediate Compound A177-4

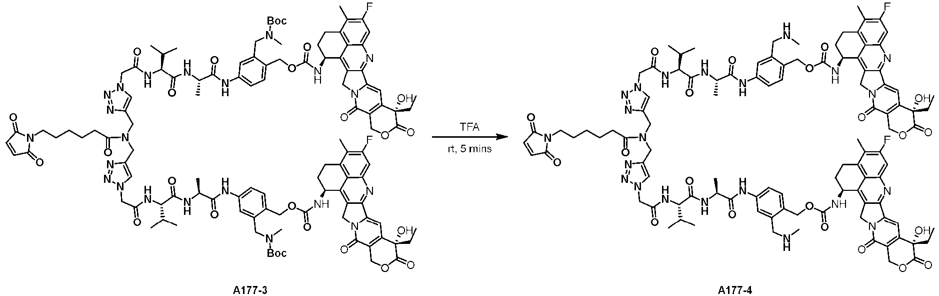

Compound **A177-3** (228 mg, 0.102 mmol, 1.0 eq) was dissolved in DCM (3 mL). Trifluoroacetic acid (1 mL) was added to the reaction mixture. The reaction mixture was stirred at room temperature for 5 minutes. After LC-MS monitoring indicated completion, the organic phase was concentrated to afford the crude product **A177-4,** which was used directly in the next step.
LCMS: [M+H]⁺ = 1024.5 (half peak)

### Synthesis of Compound A177

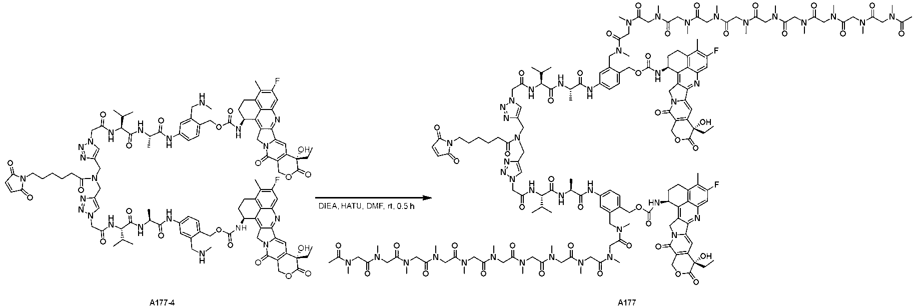

The compound **A177-4** from the previous step was dissolved in DMF (4 mL). **Ac-PSAR10-OH** (157 mg, 0.204 mmol, 2.0 eq) and N,N-Diisopropylethylamine (1053 mg, 0.94 mmol, 40.0 eq) were added to the reaction mixture. Finally, under a nitrogen atmosphere, O-(7-Azabenzotriazol-1-yl)-N,N,N',N'-tetramethyluronium hexafluorophosphate (93 mg, 0.245 mmol, 1.2 eq) was added. After the addition was complete, the reaction mixture was stirred at room temperature under a nitrogen atmosphere for 0.5 hour. After LC-MS monitoring indicated completion, ethyl acetate (60 mL) was added to the reaction mixture. The resulting suspension was filtered. The filter cake was dissolved in DMF **and** purified by prep-HPLC (mobile phase: 0.05% HCOOH/CH₃CN/H₂O) to afford compound **A177** (40.9 mg, 6%) as a white solid.
LCMS: [M+H]⁺ = 1777.7 (half peak)

### (91) Synthesis of Compound A186

### Synthetic Route:

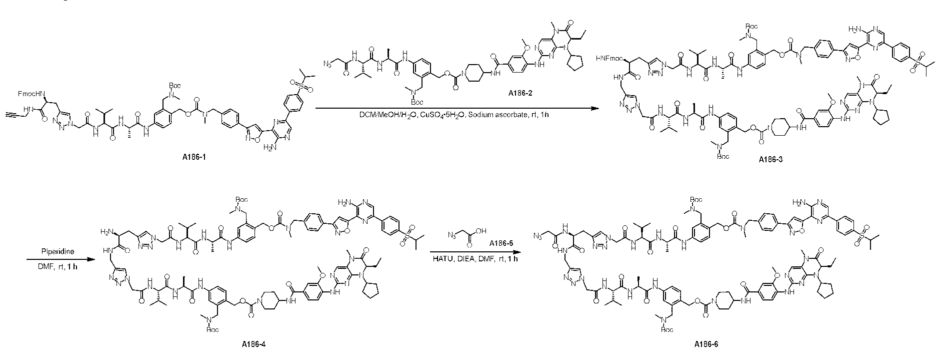

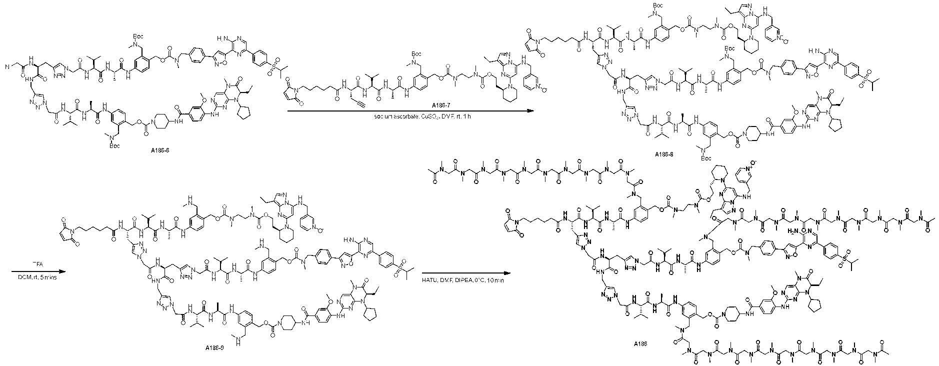

### Synthetic Steps:

### Synthesis of Intermediate Compound A186-3

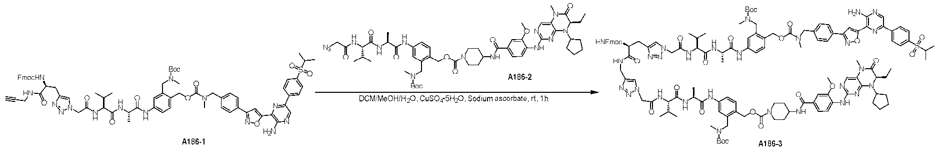

Compound **A186-1** (250 mg, 0.181 mmol, 1.0 eq) and compound **A186-2** (191 mg, 0.181 mmol, 1.0 eq) were dissolved in dichloromethane/methanol (4/4 mL). Under a nitrogen atmosphere, an aqueous solution of copper(II) sulfate pentahydrate (9.1 mg, 0.0362 mmol, 0.2 eq, in 200 µL water) and an aqueous solution of sodium ascorbate (14.4 mg, 0.0723 mmol, 0.4 eq, in 200 µL water) were added. After the addition was complete, the reaction mixture was stirred at room temperature under a nitrogen atmosphere for 1 hour. After LC-MS monitoring indicated completion, the reaction mixture was concentrated directly. The residue was dissolved in tetrahydrofuran and concentrated again to dryness. The obtained crude product was triturated with ethyl acetate, filtered, and dried to afford compound **A186-3** (400 mg, 91%) as a yellow solid.
LCMS: [M+H]⁺ = 1218.0 (half peak)

### Synthesis of Intermediate Compound A186-4

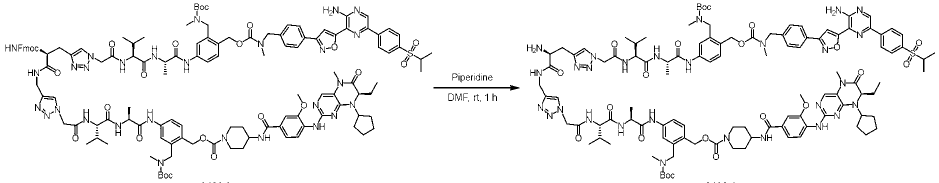

Compound **A186-3** (400 mg, 0.164 mmol, 1.0 eq) was dissolved in DMF (5 mL). Under a nitrogen atmosphere, piperidine (1 mL) was added. The reaction mixture was stirred at room temperature under a nitrogen atmosphere for 1 hour. After LC-MS monitoring indicated completion, methyl tert-butyl ether (100 mL) was added to the reaction mixture. The resulting suspension was filtered directly. The filter cake was washed with methyl tert-butyl ether and dried directly to afford compound **A186-4** (340 mg, 94%) as a yellow solid.
LCMS: [M+H]⁺ = 1106.9 (half peak)

### Synthesis of Intermediate Compound A186-6

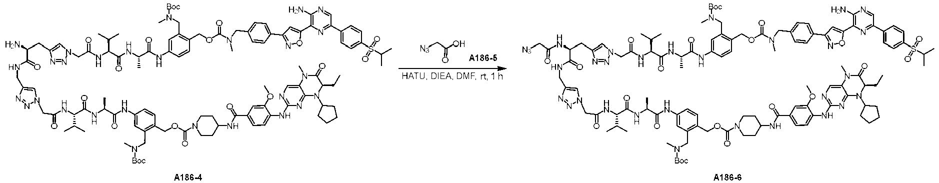

Compound **A186-4** (320 mg, 0.145 mmol, 1.0 eq) was dissolved in DMF (5 mL). N,N-Diisopropylethylamine (56 mg, 0.434 mmol, 3.0 eq) and **A186-5** (24 mg, 0.231 mmol, 1.6 eq) were added to the reaction mixture. Finally, under a nitrogen atmosphere, O-(7-Azabenzotriazol-1-yl)-N,N,N',N'-tetramethyluronium hexafluorophosphate (94 mg, 0.246 mmol, 1.7 eq) was added. After the addition was complete, the reaction mixture was stirred at room temperature under a nitrogen atmosphere for 1 hour. After LC-MS monitoring indicated completion, methyl tert-butyl ether (100 mL) was added to the reaction mixture. The resulting suspension was filtered directly. The filter cake was washed with methyl tert-butyl ether, dried directly, triturated with ethyl acetate, filtered, and dried to afford compound **A186-6** (310 mg, 93%) as a yellow solid.
LCMS: [M+H]⁺ = 1148.5 (half peak)

### Synthesis of Intermediate Compound A186-8

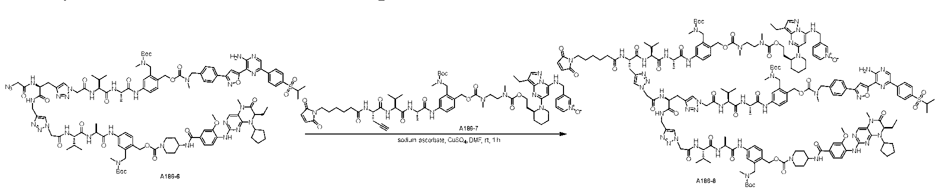

Compound **A186-6** (150 mg, 0.0653 mmol, 1.0 eq) and compound **A186-7** (82.5 mg, 0.0653 mmol, 1.0 eq) were dissolved in DMF (4 mL). Under a nitrogen atmosphere, an aqueous solution of copper(II) sulfate pentahydrate (3.3 mg, 0.0131 mmol, 0.2 eq, in 100 µL water) and an aqueous solution of sodium ascorbate (3.9 mg, 0.0196 mmol, 0.3 eq, in 100 µL water) were added. After the addition was complete, the reaction mixture was stirred at room temperature under a nitrogen atmosphere for 1 hour. After LC-MS monitoring indicated completion, the reaction mixture was diluted with ethyl acetate. The resulting suspension was filtered directly. The filter cake was washed with ethyl acetate and dried directly to afford compound **A186-8** (200 mg, 86%) as a yellow solid.
LCMS: [M+H]⁺ = 1778.8 (half peak)

### Synthesis of Intermediate Compound A186-9

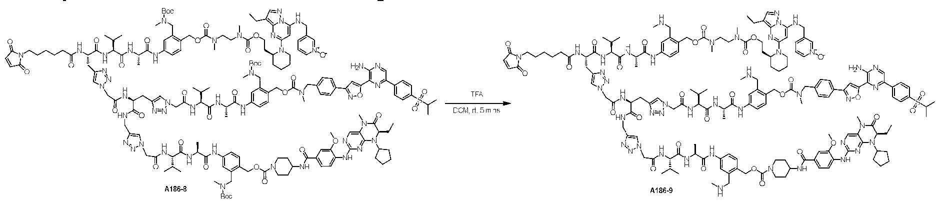

Compound **A186-8** (200 mg, 0.0562 mmol, 1.0 eq) was dissolved in DCM (3 mL). Trifluoroacetic acid (1 mL) was added to the reaction mixture. The reaction mixture was stirred at room temperature for 5 minutes. After LC-MS monitoring indicated completion, the organic phase was concentrated to afford the crude product **A186-9,** which was used directly in the next step.
LCMS: [M+H]⁺ = 1629.1 (half peak)

### Synthesis of Target Compound A186

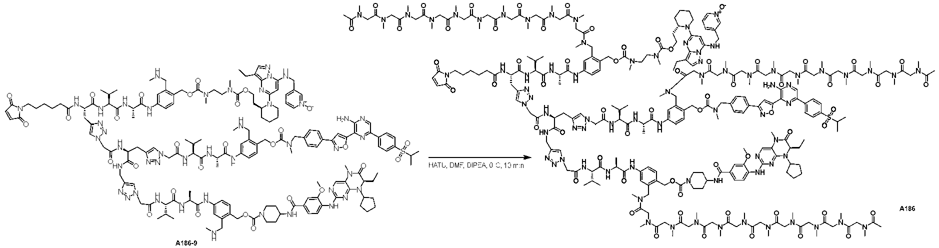

The compound **A186-9** from the previous step was dissolved in DMF (3 mL). **Ac-PSAR10-OH** (130 mg, 0.169 mmol, 3.0 eq) was added to the reaction mixture. Finally, under a nitrogen atmosphere, N,N-Diisopropylethylamine (218 mg, 1.686 mmol, 30.0 eq) and O-(7-Azabenzotriazol-1-yl)-N,N,N',N'-tetramethyluronium hexafluorophosphate (71 mg, 0.185 mmol, 3.3 eq) were added. After the addition was complete, the reaction mixture was stirred at 0 °C under a nitrogen atmosphere for 10 minutes. After LC-MS monitoring indicated completion, ethyl acetate (45 mL) was added to the reaction mixture. The resulting suspension was filtered. The filter cake was dissolved in DMF and purified by prep-HPLC (mobile phase: 0.05% HCOOH/CH₃CN/H₂O) to afford compound **A186** (96.6 mg, 31%) as a white solid.
LCMS: [M+H]⁺ = 1839.1 (one-third peak)

### (92) Synthesis of Compound A187

### Synthetic Route:

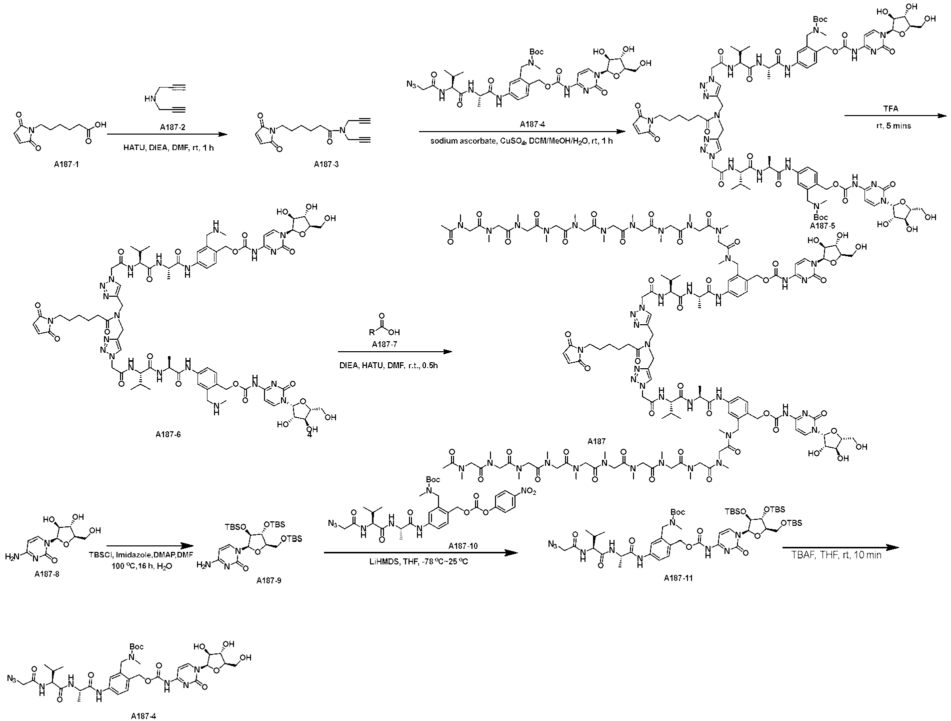

### Synthetic Steps:

### Synthesis of Intermediate Compound A187-3

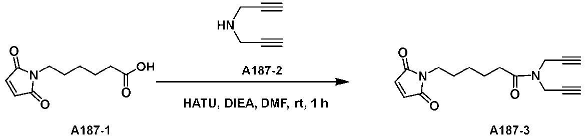

Compound **A187-1** (1000 mg, 4.738 mmol, 1.0 eq) was dissolved in DMF (10 mL). N,N-Diisopropylethylamine (1836 mg, 14.206 mmol, 3.0 eq) was added to the reaction mixture, followed by O-(7-Azabenzotriazol-1-yl)-N,N,N',N'-tetramethyluronium hexafluorophosphate (2160 mg, 5.684 mmol, 1.2 eq). Finally, compound **A187-2** (441 mg, 4.738 mmol, 1.0 eq) was added to the reaction mixture. After the addition was complete, the reaction mixture was stirred at room temperature for 1 hour. After LC-MS monitoring indicated completion, water (200 mL) was added to the reaction mixture. The mixture was extracted with ethyl acetate. The organic phase was washed with water and saturated brine, dried over anhydrous sodium sulfate, filtered, and concentrated. The residue was purified by silica gel column chromatography (EA/PE = 0~100%) to afford compound **A187-3** (900 mg, 67%) as a pale yellow oil.
LCMS: [M+H]⁺ = 287.1

### Synthesis of Intermediate Compound A187-9

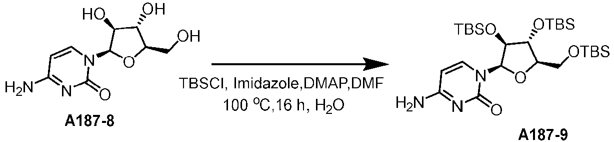

Compound **A187-8** (3 g, 0.012 mol, 1 eq) was dissolved in DMF (30 mL). Imidazole (5.02 g, 0.074 mol, 6 eq) and 4-dimethylaminopyridine (1.2 g, 0.01 mol, 0.8 eq) were added. Under an ice bath, tert-butyldimethylchlorosilane (9.3 g, 0.062 mmol, 5 eq) was added to the stirring solution. Subsequently, the mixture was stirred at 100 °C for 16 hours. Then, water (1.3 g, 0.072 mol, 6 eq) was added to the reaction system, and the mixture was stirred at 100 °C for 10 minutes. After LC-MS monitoring indicated completion, water (50 mL) was added to the reaction mixture. The mixture was extracted with ethyl acetate. The organic phase was washed with water and saturated brine, dried over anhydrous sodium sulfate, filtered, and concentrated. The residue was purified by silica gel column chromatography (EA/PE = 40/60) to afford compound **A187-9** (1.74 g, 24%) as a white solid.
LCMS: [M+H]⁺= 586.2

### Synthesis of Compound A187-11

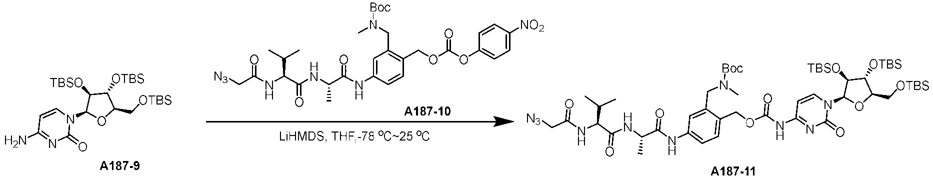

To a solution of compound **A187-9** (1.1 g, 0.002 mol, 2 eq) in tetrahydrofuran (40 mL) at -78 °C, a solution of lithium bis(trimethylsilyl)amide (2.8 mL, mmol, 1N in THF, 1.5 eq) was slowly added dropwise, and the mixture was gradually warmed to room temperature. At this point, a solution of compound **A187-10** (1.2 g, 0.0017 mol, 0.9 eq) in tetrahydrofuran THF (30 mL) was slowly added dropwise to the reaction mixture. The mixture was stirred at room temperature for 0.5 h. After LC-MS monitoring indicated completion, the reaction was quenched with saturated aqueous ammonium chloride solution under an ice bath. Water (70 mL) was added to the reaction mixture. The mixture was extracted with ethyl acetate. The organic phase was washed with water and saturated brine, dried over anhydrous sodium sulfate, filtered, and concentrated. The residue was purified by silica gel column chromatography (DCM/MeOH = 97/3) to afford compound **A187-11** (1.28 g, 58%) as a yellow solid.
LCMS: [M+Na]⁺ = 1154.3

### Synthesis of Compound A187-4

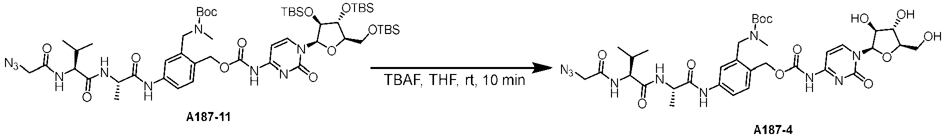

A solution of compound **A187-11** (640 mg, 0.565 mmol, 1.0 eq) in tetrahydrofuran (25 mL) was treated with a solution of tetrabutylammonium fluoride (2.3 mL, 2.3 mmol, 4 eq, 1N in THF) dropwise. The mixture was stirred at room temperature for 10 minutes. The reaction mixture was concentrated under reduced pressure to afford a yellow residue, which was purified by reversed-phase column (mobile phase: H₂O/MeCN = 65/35). The fractions were lyophilized to afford compound **A187-4** (270 mg, 58%) as a white solid.
LCMS: [M+Na]⁺ = 811.2

### Synthesis of Intermediate Compound A187-5

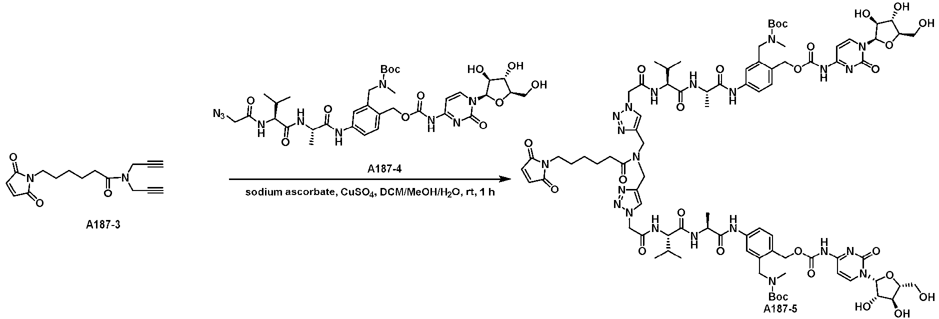

Compound **A187-4** (175 mg, 0.222 mmol, 1.0 eq) and compound **A187-3** (29 mg, 0.0999 mmol, 0.45 eq) were dissolved in dichloromethane/methanol/water (4/4/0.4 mL). Under a nitrogen atmosphere, an aqueous solution of copper(II) sulfate pentahydrate (11.1 mg, 0.0444 mmol, 0.2 eq, in 200 µL water) and an aqueous solution of sodium ascorbate (17.6 mg, 0.0888 mmol, 0.4 eq, in 200 µL water) were added. After the addition was complete, the reaction mixture was stirred at room temperature under a nitrogen atmosphere for 1 hour. After LC-MS monitoring indicated completion, the reaction mixture was concentrated directly. The residue was dissolved in tetrahydrofuran and concentrated again to dryness to afford compound **A187-5** (204 mg, 100%) as a brownish-yellow solid.
LCMS: [M+H]⁺ = 1863.6

### Synthesis of Intermediate Compound A187-6

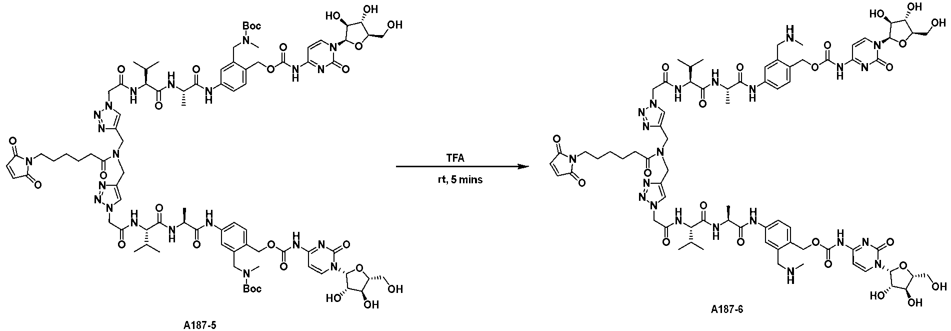

Compound **A187-5** (207 mg, 0.111 mmol, 1.0 eq) was dissolved in DCM (3 mL). Trifluoroacetic acid (1 mL) was added to the reaction mixture. The reaction mixture was stirred at room temperature for 5 minutes. After LC-MS monitoring indicated completion, the organic phase was concentrated to afford the crude product **A187-6,** which was used directly in the next step.
LCMS: [M+H]⁺ = 1663.5

### Synthesis of Compound A187

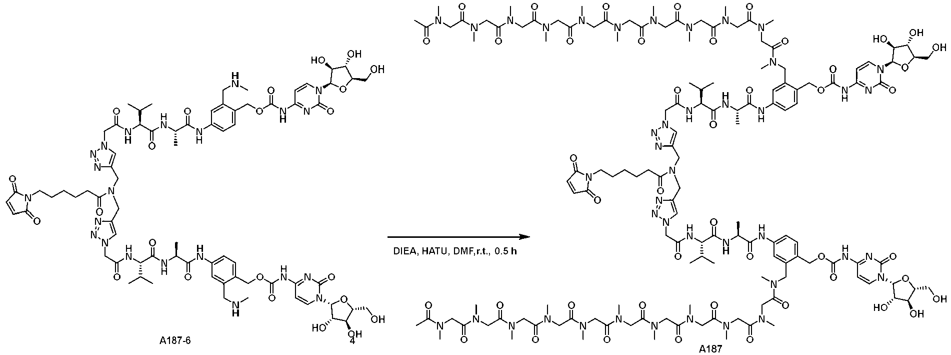

The compound **A187-6** from the previous step was dissolved in DMF (4 mL). **Ac-PSAR10-OH** (171 mg, 0.222 mmol, 2.0 eq) and N,N-Diisopropylethylamine (574 mg, 4.444 mmol, 40.0 eq) were added to the reaction mixture. Finally, under a nitrogen atmosphere, O-(7-Azabenzotriazol-1-yl)-N,N,N',N'-tetramethyluronium hexafluorophosphate (93 mg, 0.244 mmol, 2.2 eq) was added. After the addition was complete, the reaction mixture was stirred at room temperature under a nitrogen atmosphere for 0.5 hour. After LC-MS monitoring indicated completion, ethyl acetate (60 mL) was added to the reaction mixture. The resulting suspension was filtered. The filter cake was dissolved in DMF and purified by prep-HPLC (mobile phase: 0.05% HCOOH/CH₃CN/H₂O) to afford compound **A187** (68.8 mg, 20%) as a white solid.
LCMS: [M+H]⁺ = 1585.1 (half peak)

### Example 2 Preparation of ADCs

### General Method

The antibody or antigen-binding fragment, for example, Trastuzumab targeting HER2, was diluted to 5 mg/mL with a reaction buffer of 50 mM disodium hydrogen phosphate-sodium dihydrogen phosphate (NaH₂PO₄-Na₂HPO₄), 50 mM sodium chloride (NaCl), 2 mM ethylenediaminetetraacetic acid (EDTA), pH 7. Tris(2-carboxyethyl)phosphine hydrochloride (TCEP) at 2-10 molar equivalents excess was added, and the reaction mixture was stirred at room temperature for 2 hours. The reaction mixture was cooled in an ice bath, and an appropriate amount of dimethylacetamide (DMA) was added without purification. Then, the drug-linker conjugate (10 mM pre-dissolved in DMA) at 6-25 molar equivalents excess was added separately, ensuring that the volume percentage of DMA in the final reaction system did not exceed 20%. The mixture was stirred at 37°C for 1-4 hours for conjugation. The reaction mixture was filtered and purified using a desalting column pre-equilibrated with histidine-acetate/sodium chloride buffer at pH 5.5. The filtrate was collected, sterilized by filtration through a 0.22 µm filter, and stored at -80°C.

### 1) Preparation of Antibody-Drug Conjugate T-MDP1

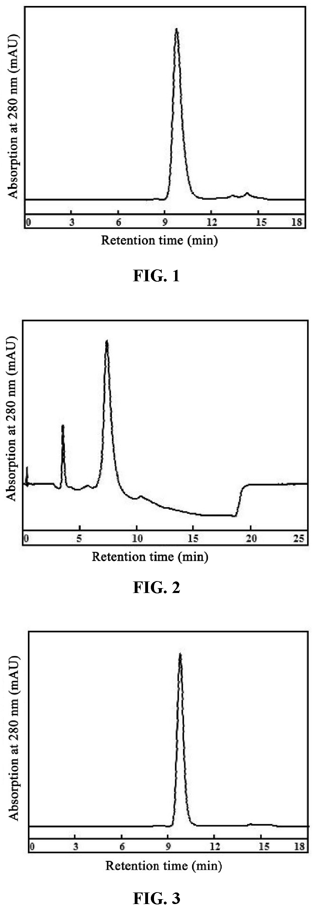

The stock solution of the Her2-targeting monoclonal antibody Trastuzumab was buffer-exchanged into a reaction buffer of 50 mM disodium hydrogen phosphate-sodium dihydrogen phosphate (NaH₂PO₄-Na₂HPO₄), 50 mM sodium chloride (NaCl), 2 mM ethylenediaminetetraacetic acid (EDTA), pH 7. The antibody concentration in the solution was measured and diluted to 5 mg/mL with the above buffer. Tris(2-carboxyethyl)phosphine hydrochloride (TCEP) at 9 molar equivalents excess was added, and the reaction mixture was stirred at room temperature for 2 hours. The reaction mixture was cooled in an ice bath, and an appropriate amount of dimethylacetamide (DMA) was added. Then, the drug-linker conjugate **A1** (10 mM, pre-dissolved in DMA) at 14 molar equivalents excess was added, where the volume percentage of DMA in the reaction system was 20%. The mixture was stirred at 37°C for 1 hour for conjugation. The reaction mixture was filtered and purified using a desalting column pre-equilibrated with histidine-acetate/sodium chloride buffer at pH 5.5. The filtrate was collected. One-tenth volume of an activated charcoal-histidine-acetate-sodium chloride solution (300 mg/mL) was added to the sample and stirred at room temperature for 2 hours to fully absorb free small-molecule drugs. The mixture was then sterilized by filtration through a 0.22 µm filter and stored at -80°C. The aggregate content was analyzed by size-exclusion chromatography (SEC-HPLC). The analysis chromatogram is shown in Figure 1. The monomer purity of the antibody-drug conjugate T-MDP1 was 99.57%. The conjugated drug concentration in the antibody-drug conjugate can be measured by BCA or UV method. The proportion of conjugate to unconjugated naked antibody in the product can be detected by hydrophobic interaction chromatography. The analysis chromatogram is shown in Figure 2. The antibody-drug conjugate T-MDP1 mainly consists of the DAR8 component.

### 2) Preparation of Antibody-Drug Conjugate T-MDP2

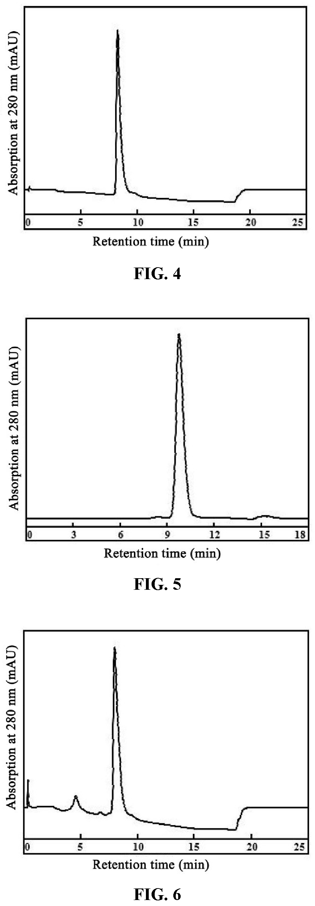

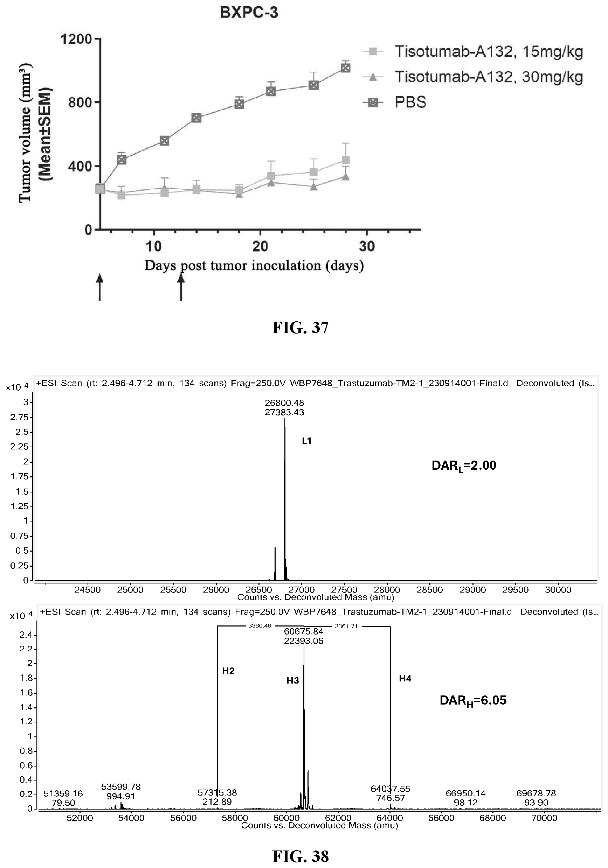

Referring to the preparation and post-treatment method of the antibody-drug conjugate T-MDP1, the Her2-targeting monoclonal antibody Trastuzumab was conjugated with the drug-linker conjugate **A2** to obtain the antibody-drug conjugate T-MDP2. The aggregate content was analyzed by size-exclusion chromatography (SEC-HPLC). The analysis chromatogram is shown in Figure 3. The monomer purity of the antibody-drug conjugate T-MDP2 was 99.50%. The conjugated drug concentration in the antibody-drug conjugate can be measured by BCA or UV method. The proportion of conjugate to unconjugated naked antibody in the product can be detected by hydrophobic interaction chromatography. The analysis chromatogram is shown in Figure 4. The antibody-drug conjugate T-MDP2 mainly consists of the DAR8 component. Further LCMS analysis determined the DAR value of this antibody-drug conjugate to be 8.05 (Figure 38).

### 3) Preparation of Antibody-Drug Conjugate T-MDP3

Referring to the preparation and post-treatment method of the antibody-drug conjugate T-MDP1, the Her2-targeting monoclonal antibody Trastuzumab was conjugated with compound **A10** to obtain the antibody-drug conjugate T-MDP3. The aggregate content was analyzed by size-exclusion chromatography (SEC-HPLC). The analysis chromatogram is shown in Figure 5. The monomer purity of the antibody-drug conjugate T-MDP3 was 99.36%. The conjugated drug concentration in the antibody-drug conjugate can be measured by BCA method. The proportion of conjugate to unconjugated naked antibody in the product can be detected by hydrophobic interaction chromatography. The analysis chromatogram is shown in Figure 6. The antibody-drug conjugate T-MDP3 mainly consists of the DAR8 component.

### 4) Preparation of Antibody-Drug Conjugate T-MDP4

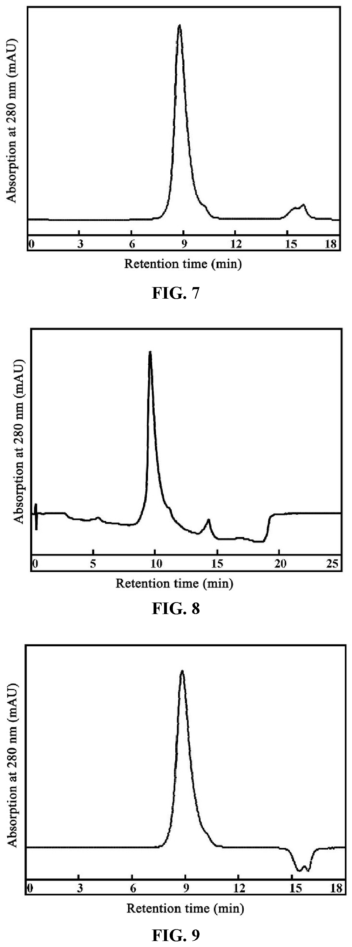

Referring to the preparation and post-treatment method of the antibody-drug conjugate T-MDP1, the Her2-targeting monoclonal antibody Trastuzumab was conjugated with compound **A12** to obtain the antibody-drug conjugate T-MDP4. The aggregate content was analyzed by size-exclusion chromatography (SEC-HPLC). The analysis chromatogram is shown in Figure 7. The monomer content of the antibody-drug conjugate T-MDP4 was very low, with most of the conjugate detected as aggregates, accounting for about 95.8%. The conjugated drug concentration in the antibody-drug conjugate can be measured by BCA method. The proportion of conjugate to unconjugated naked antibody in the product can be detected by hydrophobic interaction chromatography. The analysis chromatogram is shown in Figure 8. The antibody-drug conjugate T-MDP4 mainly consists of the DAR8 component.

### 5) Preparation of Antibody-Drug Conjugate T-MDP5

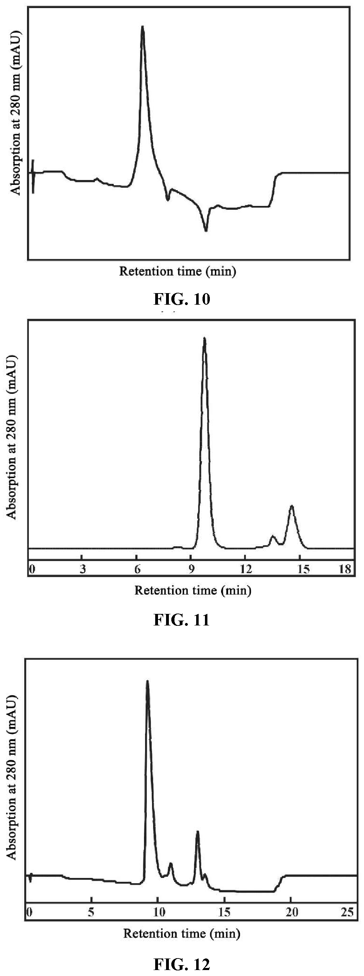

Referring to the preparation and post-treatment method of the antibody-drug conjugate T-MDP1, the Her2-targeting monoclonal antibody Trastuzumab was conjugated with compound **A11** to obtain the antibody-drug conjugate T-MDP5. The aggregate content was analyzed by size-exclusion chromatography (SEC-HPLC). The analysis chromatogram is shown in Figure 9. The monomer content was very low, with most of the conjugate detected as aggregates, accounting for about 96.2%. The conjugated drug concentration in the antibody-drug conjugate can be measured by BCA method. The proportion of conjugate to unconjugated naked antibody in the product can be detected by hydrophobic interaction chromatography. The analysis chromatogram is shown in Figure 10. The antibody-drug conjugate T-MDP5 mainly consists of the DAR8 component.

### 6) Preparation of Antibody-Drug Conjugate T-MDP6

Referring to the preparation and post-treatment method of the antibody-drug conjugate T-MDP1, the Her2-targeting monoclonal antibody Trastuzumab was conjugated with compound **A58** to obtain the antibody-drug conjugate T-MDP6. The aggregate content was analyzed by size-exclusion chromatography (SEC-HPLC). The analysis chromatogram is shown in Figure 11. The monomer purity of the antibody-drug conjugate T-MDP3 was 99.44%. The conjugated drug concentration in the antibody-drug conjugate can be measured by BCA method. The proportion of conjugate to unconjugated naked antibody in the product can be detected by hydrophobic interaction chromatography. The analysis chromatogram is shown in Figure 12. The antibody-drug conjugate T-MDP6 mainly consists of the DAR8 component.

### 7) Preparation of Antibody-Drug Conjugate T-MDP7

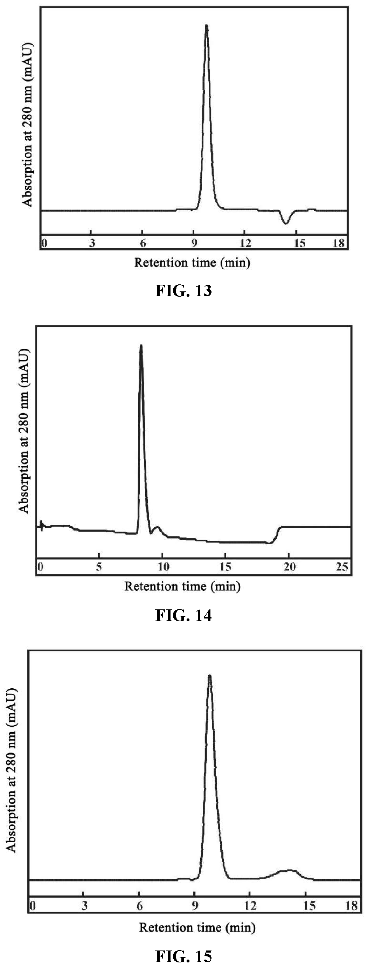

Referring to the preparation and post-treatment method of the antibody-drug conjugate T-MDP1, the Her2-targeting monoclonal antibody Trastuzumab was conjugated with compound **A16** to obtain the antibody-drug conjugate T-MDP7. The aggregate content was analyzed by size-exclusion chromatography (SEC-HPLC). The analysis chromatogram is shown in Figure 13. The monomer purity of the antibody-drug conjugate T-MDP7 was 99.57%. The conjugated drug concentration in the antibody-drug conjugate can be measured by BCA method. The proportion of conjugate to unconjugated naked antibody in the product can be detected by hydrophobic interaction chromatography. The analysis chromatogram is shown in Figure 14. The antibody-drug conjugate T-MDP7 mainly consists of the DAR8 component.

### 8) Preparation of Antibody-Drug Conjugate T-MDP8

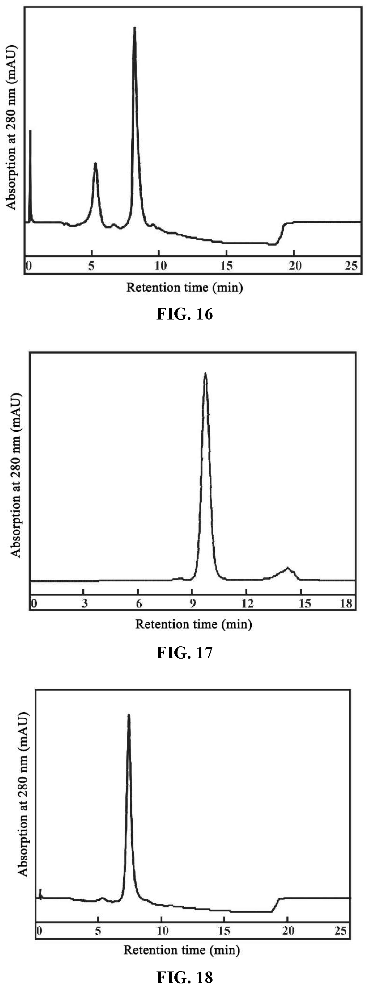

Referring to the preparation and post-treatment method of the antibody-drug conjugate T-MDP1, the Her2-targeting monoclonal antibody Trastuzumab was conjugated with compound **A17** to obtain the antibody-drug conjugate T-MDP8. The aggregate content was analyzed by size-exclusion chromatography (SEC-HPLC). The analysis chromatogram is shown in Figure 15. The monomer purity of the antibody-drug conjugate T-MDP3 was 99.46%. The conjugated drug concentration in the antibody-drug conjugate can be measured by BCA method. The proportion of conjugate to unconjugated naked antibody in the product can be detected by hydrophobic interaction chromatography. The analysis chromatogram is shown in Figure 16. The antibody-drug conjugate T-MDP8 mainly consists of the DAR8 component.

### 9) Preparation of Antibody-Drug Conjugate T-MDP9

Referring to the preparation and post-treatment method of the antibody-drug conjugate T-MDP1, the Her2-targeting monoclonal antibody Trastuzumab was conjugated with compound **A23** to obtain the antibody-drug conjugate T-MDP9. The aggregate content was analyzed by size-exclusion chromatography (SEC-HPLC). The analysis chromatogram is shown in Figure 17. The monomer purity of the antibody-drug conjugate T-MDP3 was 99.27%. The conjugated drug concentration in the antibody-drug conjugate can be measured by BCA method. The proportion of conjugate to unconjugated naked antibody in the product can be detected by hydrophobic interaction chromatography. The analysis chromatogram is shown in Figure 18. The antibody-drug conjugate T-MDP9 mainly consists of the DAR8 component.

### 10) Preparation of Antibody-Drug Conjugate T-MDP10

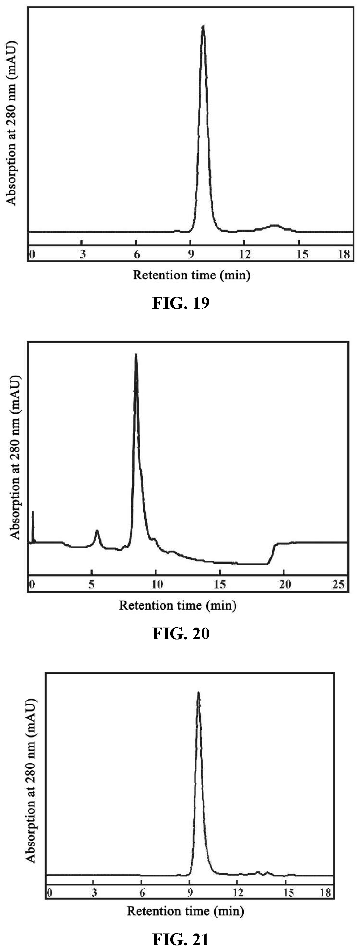

Referring to the preparation and post-treatment method of the antibody-drug conjugate T-MDP1, the Her2-targeting monoclonal antibody Trastuzumab was conjugated with compound **A14** to obtain the antibody-drug conjugate T-MDP10. The aggregate content was analyzed by size-exclusion chromatography (SEC-HPLC). The analysis chromatogram is shown in Figure 19. The monomer purity of the antibody-drug conjugate T-MDP10 was 99.59%. The conjugated drug concentration in the antibody-drug conjugate can be measured by BCA method. The proportion of conjugate to unconjugated naked antibody in the product can be detected by hydrophobic interaction chromatography. The analysis chromatogram is shown in Figure 20. The antibody-drug conjugate T-MDP10 mainly consists of the DAR8 component.

### 11) Preparation of Antibody-Drug Conjugate T-MDP11

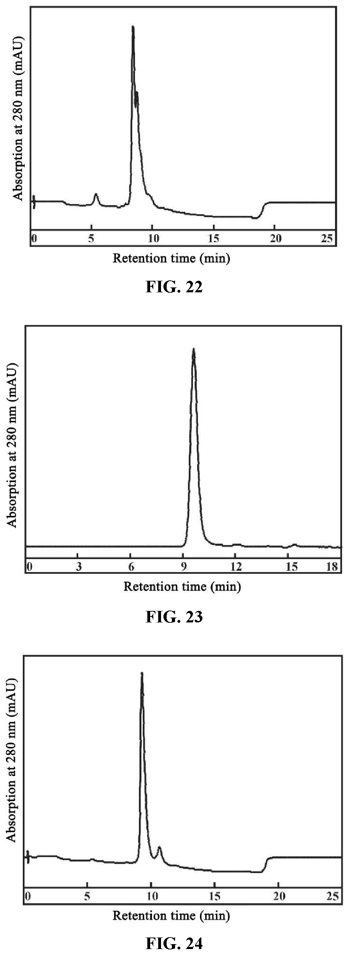

Referring to the preparation and post-treatment method of the antibody-drug conjugate T-MDP1, the Her2-targeting monoclonal antibody Trastuzumab was conjugated with compound **A68** to obtain the antibody-drug conjugate T-MDP11. The aggregate content was analyzed by size-exclusion chromatography (SEC-HPLC). The analysis chromatogram is shown in Figure 21. The monomer purity of the antibody-drug conjugate T-MDP11 was 99.78%. The conjugated drug concentration in the antibody-drug conjugate can be measured by BCA method. The proportion of conjugate to unconjugated naked antibody in the product can be detected by hydrophobic interaction chromatography. The analysis chromatogram is shown in Figure 22. The antibody-drug conjugate T-MDP11 mainly consists of the DAR8 component.

### 12) Preparation of Antibody-Drug Conjugate T-MDP12

Referring to the preparation and post-treatment method of the antibody-drug conjugate T-MDP1, the Her2-targeting monoclonal antibody Trastuzumab was conjugated with compound **A33** to obtain the antibody-drug conjugate T-MDP12. The aggregate content was analyzed by size-exclusion chromatography (SEC-HPLC). The analysis chromatogram is shown in Figure 23. The monomer purity of the antibody-drug conjugate T-MDP12 was 99.84%. The conjugated drug concentration in the antibody-drug conjugate can be measured by BCA method. The proportion of conjugate to unconjugated naked antibody in the product can be detected by hydrophobic interaction chromatography. The analysis chromatogram is shown in Figure 24. The antibody-drug conjugate T-MDP12 mainly consists of the DAR8 component. Further LCMS analysis determined the DAR value of this antibody-drug conjugate to be 7.90 (Figure 39).

### 13) Preparation of Antibody-Drug Conjugate T-MDP13

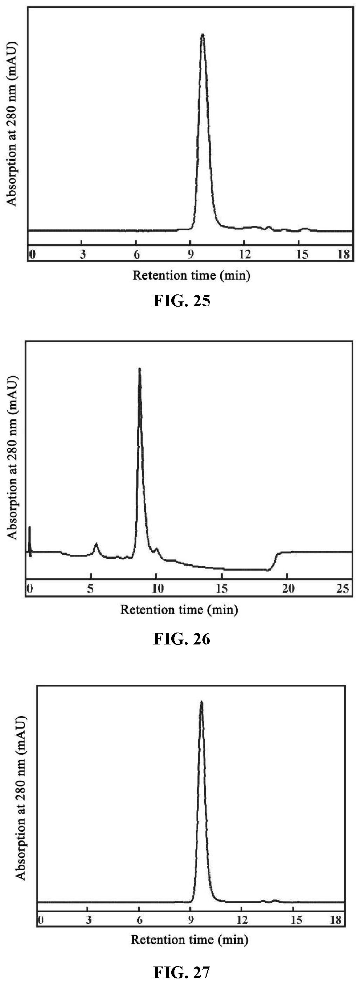

Referring to the preparation and post-treatment method of the antibody-drug conjugate T-MDP1, the Her2-targeting monoclonal antibody Trastuzumab was conjugated with compound **A15** to obtain the antibody-drug conjugate T-MDP13. The aggregate content was analyzed by size-exclusion chromatography (SEC-HPLC). The analysis chromatogram is shown in Figure 25. The monomer purity of the antibody-drug conjugate T-MDP13 was 99.56%. The conjugated drug concentration in the antibody-drug conjugate can be measured by BCA method. The proportion of conjugate to unconjugated naked antibody in the product can be detected by hydrophobic interaction chromatography. The analysis chromatogram is shown in Figure 26. The antibody-drug conjugate T-MDP13 mainly consists of the DAR8 component.

### 14) Preparation of Antibody-Drug Conjugate T-MDP17

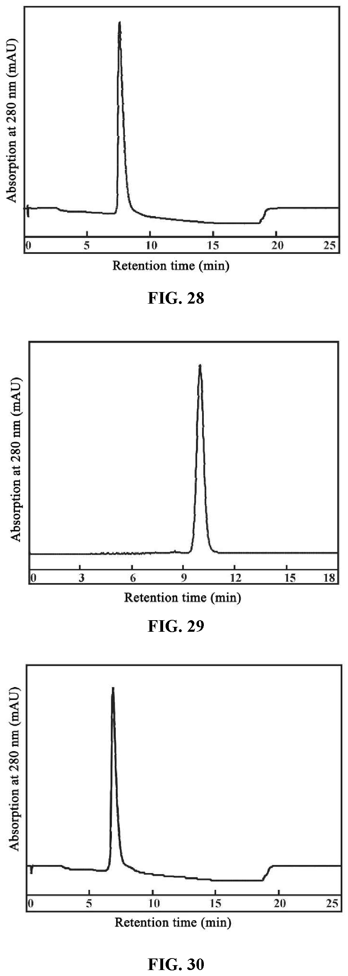

Referring to the preparation and post-treatment method of the antibody-drug conjugate T-MDP1, the Her2-targeting monoclonal antibody Trastuzumab was conjugated with compound A21 to obtain the antibody-drug conjugate T-MDP17. The aggregate content was analyzed by size-exclusion chromatography (SEC-HPLC). The analysis chromatogram is shown in Figure 27. The monomer purity of the antibody-drug conjugate T-MDP17 was 99.70%. The conjugated drug concentration in the antibody-drug conjugate can be measured by BCA method. The proportion of conjugate to unconjugated naked antibody in the product can be detected by hydrophobic interaction chromatography. The analysis chromatogram is shown in Figure 28. The antibody-drug conjugate T-MDP13 mainly consists of the DAR8 component.

### 15) Preparation of Control Antibody Single-Drug Conjugates T-T1000e (MTX-1000), RS7-T1000e and T-MCD1

Referring to the preparation and post-treatment method of the antibody-drug conjugate T-MDP1, the Her2-targeting monoclonal antibody Trastuzumab and the TROP2-targeting monoclonal antibody Sacituzumab were respectively conjugated with compound T1000e to obtain the antibody-drug conjugates T-T1000e (MTX-1000) and RS7-T1000e (compound T1000e is T1000-exatecan, synthesized and conjugated according to the method described in Cancer Discov 2023;13:950-73). Trastuzumab was conjugated with MCD1 to obtain the antibody-drug conjugate T-MCD1. The aggregate content was analyzed by size-exclusion chromatography (SEC-HPLC). The monomer purity of the antibody-drug conjugate T-MCD1 (Figure 29) was >98%. The conjugated drug concentration in the antibody-drug conjugate can be measured by BCA method. Hydrophobic interaction chromatography can detect the proportion of conjugate to unconjugated naked antibody in T-MCD1, which mainly consists of the DAR8 component (Figure 30).

### 16) Preparation of Antibody-Drug Conjugate T-MDP54

The stock solution of the Her2-targeting monoclonal antibody Trastuzumab was buffer-exchanged into a reaction buffer of 50 mM disodium hydrogen phosphate-sodium dihydrogen phosphate (NaH₂PO₄-Na₂HPO₄), 50 mM sodium chloride (NaCl), 2 mM ethylenediaminetetraacetic acid (EDTA), pH 7. The antibody concentration in the solution was measured. Tris(2-carboxyethyl)phosphine hydrochloride (TCEP) at 8 molar equivalents excess was added, and the reaction mixture was stirred at 37°C for 2 hours. The reaction mixture was cooled in an ice bath, and an appropriate amount of dimethylacetamide (DMA) was added. Then, the drug-linker conjugate A165 (10 mM, pre-dissolved in a 1:1 mixture of DMA and water) at 12 molar equivalents excess was added, where the volume percentage of DMA in the reaction system was 10%. The mixture was stirred at 22 °C for 1 hour for conjugation. The reaction mixture was purified using a protein purification system, employing a strong cation exchange (SP) column. The collected fractions were sampled for mini-pool analysis. The collected pool was then subjected to ultrafiltration and diafiltration, and finally exchanged into the final formulation buffer (20 mM Histidine-acetate, 8% sucrose, pH 5.5). The solution was sterilized by filtration through a 0.22 µm filter and stored at -80°C.

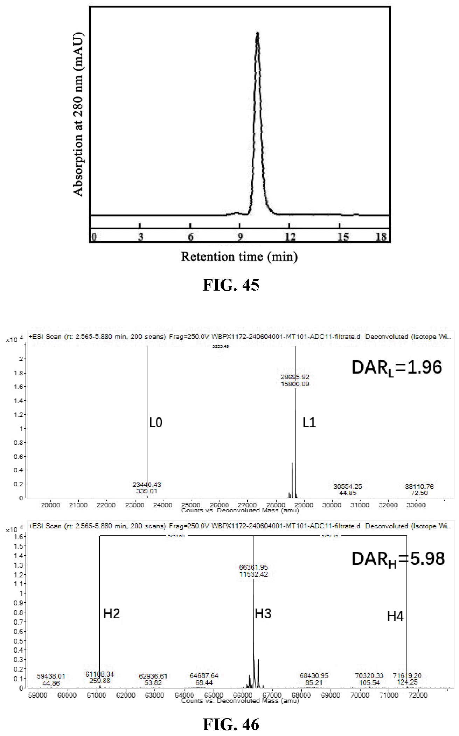

The concentration of the antibody-drug conjugate T-MDP54 was quantified by BCA method to be 10.37 mg/mL. The aggregate content was analyzed by size-exclusion chromatography (SEC-HPLC). The SEC chromatogram is shown in **Figure 45****.** The monomer purity of the antibody-drug conjugate T-MDP54 was 98.38%, and the aggregate content was 1.62%. The drug-to-antibody ratio (DAR) was analyzed by LC-MS method. The MS spectrum is shown in **Figure 46****.** The DAR value of the antibody-drug conjugate T-MDP54 was 7.94. The level of unconjugated small molecule drug-related substance MDP54 relative to that in the antibody-drug conjugate T-MDP54 was detected by precipitation method and found to be 0.95%. The endotoxin level in the conjugate stock solution was quantified by dynamic turbidimetric method, and the result was <0.096 EU/mg.

### 17) Preparation of Antibody-Drug Conjugate RS7-MDP12

The stock solution of the Trop2-targeting monoclonal antibody RS7 was buffer-exchanged into a reaction buffer of 50 mM disodium hydrogen phosphate-sodium dihydrogen phosphate (NaH₂PO₄-Na₂HPO₄), 50 mM sodium chloride (NaCl), 2 mM ethylenediaminetetraacetic acid (EDTA), pH 7. The antibody concentration in the solution was measured. Tris(2-carboxyethyl)phosphine hydrochloride (TCEP) at 8 molar equivalents excess was added, and the reaction mixture was stirred at 37°C for 2 hours. The reaction mixture was cooled in an ice bath, and an appropriate amount of dimethylacetamide (DMA) was added. Then, the drug-linker conjugate A33 (10 mM, pre-dissolved in a 1:1 mixture of DMA and water) at 12 molar equivalents excess was added, where the volume percentage of DMA in the reaction system was 10%. The mixture was stirred at 22 °C for 1 hour for conjugation. The reaction mixture was purified using a protein purification system, employing a strong cation exchange (SP) column. The collected fractions were sampled for mini-pool analysis. The collected pool was then subjected to ultrafiltration and diafiltration, and finally exchanged into the final formulation buffer (20 mM Histidine-acetate, 8% sucrose, pH 5.5). The solution was sterilized by filtration through a 0.22 µm filter and stored at -80°C.

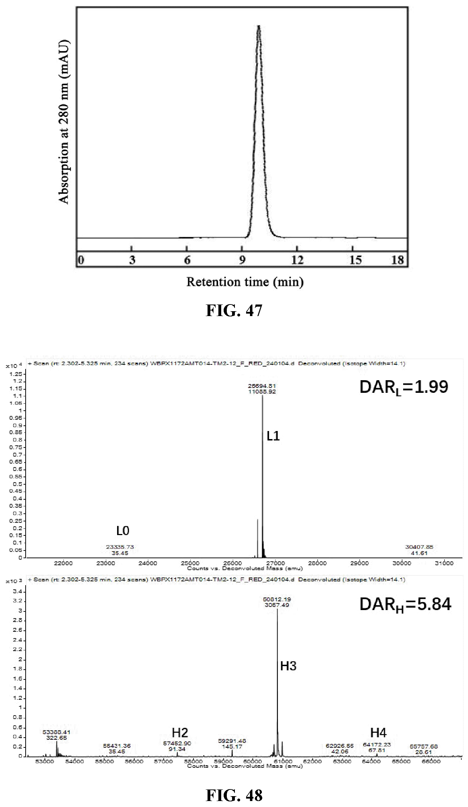

The concentration of the antibody-drug conjugate RS7-MDP12 was quantified by BCA method to be 15.01 mg/mL. The aggregate content was analyzed by size-exclusion chromatography (SEC-HPLC). The SEC chromatogram is shown in **Figure 47****.** The monomer purity of the antibody-drug conjugate RS7-MDP12 was 99.70%, and the aggregate content was 0.30%. The drug-to-antibody ratio (DAR) was analyzed by LC-MS method. The MS spectrum is shown in **Figure 48****.** The DAR value of the antibody-drug conjugate RS7-MDP12 was 7.90. The level of unconjugated small molecule drug-related substance A33 relative to that in the antibody-drug conjugate RS7-MDP12 was detected by precipitation method and found to be 2.20%. The endotoxin level in the conjugate stock solution was quantified by dynamic turbidimetric method, and the result was <0.133 EU/mg.

### 18) Preparation of Antibody-Drug Conjugate RS7-MDP44

The stock solution of the Trop2-targeting monoclonal antibody RS7 was buffer-exchanged into a reaction buffer of 50 mM disodium hydrogen phosphate-sodium dihydrogen phosphate (NaH₂PO₄-Na₂HPO₄), 50 mM sodium chloride (NaCl), 2 mM ethylenediaminetetraacetic acid (EDTA), pH 7. The antibody concentration in the solution was measured. Tris(2-carboxyethyl)phosphine hydrochloride (TCEP) at 8 molar equivalents excess was added, and the reaction mixture was stirred at 37°C for 2 hours. The reaction mixture was cooled in an ice bath, and an appropriate amount of dimethylacetamide (DMA) was added. Then, the drug-linker conjugate A153 (10 mM, pre-dissolved in a 1:1 mixture of DMA and water) at 12 molar equivalents excess was added, where the volume percentage of DMA in the reaction system was 10%. The mixture was stirred at 22 °C for 1 hour for conjugation. The reaction mixture was purified using a protein purification system, employing a strong cation exchange (SP) column. The collected fractions were sampled for mini-pool analysis. The collected pool was then subjected to ultrafiltration and diafiltration, and finally exchanged into the final formulation buffer (20 mM Histidine-acetate, 8% sucrose, pH 5.5). The solution was sterilized by filtration through a 0.22 µm filter and stored at -80°C.

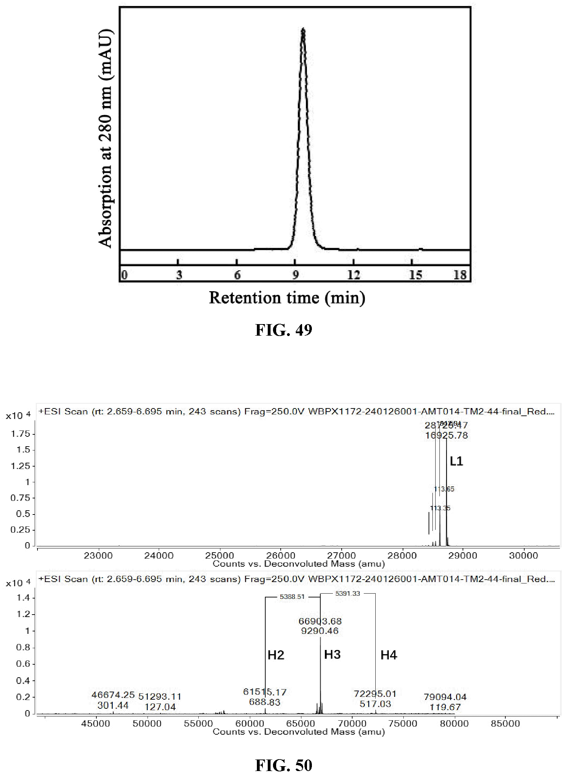

The concentration of the antibody-drug conjugate RS7-MDP44 was quantified by BCA method to be 13.45 mg/mL. The aggregate content was analyzed by size-exclusion chromatography (SEC-HPLC). The SEC chromatogram is shown in **Figure 49****.** The monomer purity of the antibody-drug conjugate RS7-MDP44 was 99.35%, and the aggregate content was 0.65%. The drug-to-antibody ratio (DAR) was analyzed by LC-MS method. The MS spectrum is shown in **Figure 50****.** The DAR value of the antibody-drug conjugate RS7-MDP44 was 7.97. The level of unconjugated small molecule drug-related substance A153 relative to that in the antibody-drug conjugate RS7-MDP44 was detected by precipitation method and found to be 0.29%. The endotoxin level in the conjugate stock solution was quantified by Charles River's endotoxin detection method (Endosafe^{®}), and the result was <0.112 EU/mg.

### 19) Preparation of Antibody-Drug Conjugate RS7-MDP47

The stock solution of the Trop2-targeting monoclonal antibody RS7 was buffer-exchanged into a reaction buffer of 50 mM disodium hydrogen phosphate-sodium dihydrogen phosphate (NaH₂PO₄-Na₂HPO₄), 50 mM sodium chloride (NaCl), 2 mM ethylenediaminetetraacetic acid (EDTA), pH 7. The antibody concentration in the solution was measured. Tris(2-carboxyethyl)phosphine hydrochloride (TCEP) at 8 molar equivalents excess was added, and the reaction mixture was stirred at 37°C for 2 hours. The reaction mixture was cooled in an ice bath, and an appropriate amount of dimethylacetamide (DMA) was added. Then, the drug-linker conjugate A160 (10 mM, pre-dissolved in a 1:1 mixture of DMA and water) at 12 molar equivalents excess was added, where the volume percentage of DMA in the reaction system was 10%. The mixture was stirred at 22 °C for 1 hour for conjugation. The reaction mixture was purified using a protein purification system, employing a strong cation exchange (SP) column. The collected fractions were sampled for mini-pool analysis. The collected pool was then subjected to ultrafiltration and diafiltration, and finally exchanged into the final formulation buffer (20 mM Histidine-acetate, 8% sucrose, pH 5.5). The solution was sterilized by filtration through a 0.22 µm filter and stored at -80°C.

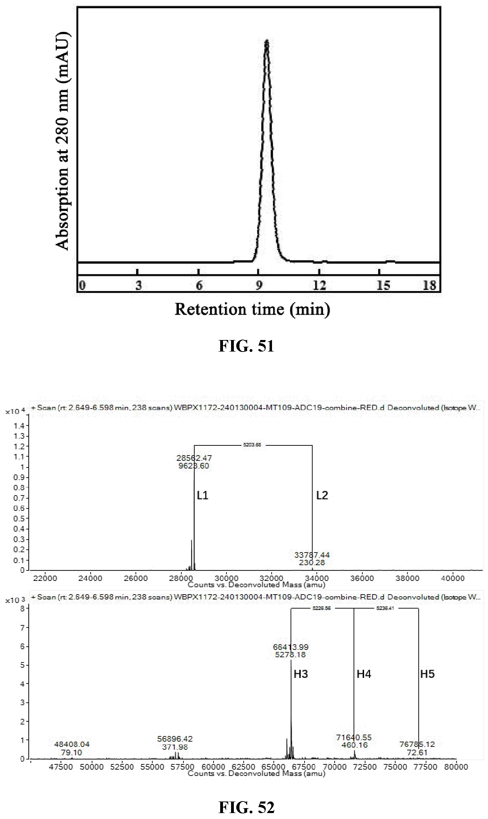

The concentration of the antibody-drug conjugate RS7-MDP47 was quantified by BCA method to be 10.42 mg/mL. The aggregate content was analyzed by size-exclusion chromatography (SEC-HPLC). The SEC chromatogram is shown in **Figure 51****.** The monomer purity of the antibody-drug conjugate RS7-MDP47 was 99.84%, and the aggregate content was 0.16%. The drug-to-antibody ratio (DAR) was analyzed by LC-MS method. The MS spectrum is shown in **Figure 52****.** The DAR value of the antibody-drug conjugate RS7-MDP47 was 8.22. The level of unconjugated small molecule drug-related substance A160 relative to that in the antibody-drug conjugate RS7-MDP47 was detected by precipitation method and found to be 0.11%. The endotoxin level in the conjugate stock solution was quantified by Charles River's endotoxin detection method (Endosafe^{®}), and the result was <0.096 EU/mg.

### 20) Preparation of a Series of Antibody-Drug Conjugates

Referring to the above preparation, purification, and analysis conditions for antibody-drug conjugates, the antibody-drug conjugates listed in the table below can be obtained. The purity of the obtained antibody-drug conjugates is >90%, and they all mainly consist of the DAR8 component.

| Antibody-Drug Conjugate | Antibody | Drug-Linker |
|---|---|---|
| T-MDP15 | Trastuzumab | A80 |
| T-MDP16 | Trastuzumab | A90 |
| T-MDP27 | Trastuzumab | A120 |
| U3-1402 | Patritumab | GGFG-DXd |
| Dato-DXd | Datopotamab | GGFG-DXd |
| P-T1000e | Patritumab | T1000e (T1000-exatecan) |
| P-MDP2 | Patritumab | A2 |
| P-MDP3 | Patritumab | A10 |
| P-MDP12 | Patritumab | A33 |
| P-MDP15 | Patritumab | A80 |
| P-MDP22 | Patritumab | A117 |
| P-MDP27 | Patritumab | A120 |
| DS5573a-MCD1 | DS5573a | MCD1 |
| DS5573a-MDP2 | DS5573a | A2 |
| DS5573a-MDP6 | DS5573a | A58 |
| Tisotumab-A132 | Tisotumab | A132 |
| RS7-A132 | hRS7(Sacituzumab) | A132 |
| RS7-A146 | hRS7(Sacituzumab) | A146 |
| RS7-A149 | hRS7(Sacituzumab) | A149 |
| The amino acid sequences of the HC (heavy chain) and LC (light chain) of Trastuzumab are shown in SEQ ID NO. 3 and 4, respectively; | | |
| The amino acid sequences of the HC (heavy chain) and LC (light chain) of Patritumab are shown in SEQ ID NO. 1 and 2, respectively; | | |
| The amino acid sequences of the HC (heavy chain) and LC (light chain) of Datopotamab are shown in SEQ ID NO. 5 and 6, respectively; | | |
| The amino acid sequences of the HC (heavy chain) and LC (light chain) of DS5573a (Ifinatamab) are shown in SEQ ID NO. 7 and 8, respectively; | | |
| The amino acid sequences of the HC (heavy chain) and LC (light chain) of Tisotumab are shown in SEQ ID NO. 9 and 10, respectively; | | |
| The amino acid sequences of the HC (heavy chain) and LC (light chain) of hRS7 (Sacituzumab) are shown in SEQ ID NO. 11 and 12, respectively. | | |

### 17) Preparation of a Series of Thiomab-based Antibody-Drug Conjugates

General Method, as shown in Figure 41:

### 1) THIOMAB^{™} Deblocking

**Reduction:** The concentration of **THIOMAB^{™}** was determined by UV280 nm. The pH of the solution was adjusted to 7.5 using 1 M Tris (hydroxymethyl) aminomethane hydrochloride (Tris-HCl) buffer. A 0.2 M EDTA solution was added dropwise to a final concentration of 2 mM. Then, dithiothreitol (DTT) equivalent to 50-150 molar equivalents of the antibody (prepared as a 100 mM aqueous solution) was added. The reaction mixture was thoroughly mixed and stirred at 37°C for 2 hours to ensure complete reaction-fully removing glutathione and cysteine protection from the cysteine mutation sites of THIOMAB^{™}. The deblocking efficiency of the blocker was analyzed by MS detection. Small-scale samples can be concentrated and buffer-exchanged (exchanging the reduced antibody into a phosphate buffer at pH 7-7.5) using ultrafiltration devices. The buffer exchange volume should be greater than 200-fold to ensure sufficient removal of reducing agents and free glutathione and cysteine blockers from the reaction system. For large-scale samples, ultrafiltration/diafiltration (UF/DF) equipment can be used for thorough buffer exchange.

**Re-oxidation:** The concentration of the above reduced and buffer-exchanged **THIOMAB^{™}** was determined by UV280 nm. The pH of the solution was adjusted to 7.5 using 1 M Tris (hydroxymethyl) aminomethane hydrochloride (Tris-HCl) buffer. Dehydroascorbic acid powder was weighed and dissolved in N,N-dimethylacetamide to prepare a 100 mM solution (DHAA solution). Then, DHAA solution equivalent to 15 molar equivalents of the antibody was slowly added dropwise to the THIOMAB^{™}. The reaction mixture was thoroughly mixed and stirred at room temperature for 1-3 hours to ensure complete reaction-the interchain disulfide bonds opened during reduction were re-oxidized. The oxidation efficiency was analyzed by MS detection. Similarly, small-scale samples can be concentrated and buffer-exchanged using ultrafiltration devices to exchange the oxidized antibody into a phosphate buffer at pH 7.2. The buffer exchange volume should also be greater than 200-fold to ensure sufficient removal of oxidants from the reaction system. For large-scale samples, ultrafiltration/diafiltration (UF/DF) equipment can be used for thorough buffer exchange. The concentration of the final deblocked **THIOMAB^{™}** was determined again by UV280 nm. The purity of the **THIOMAB^{™}** solution was analyzed by size-exclusion chromatography (SEC-HPLC). The sample was sterilized by filtration through a 0.22 µm filter, aliquoted, and stored at -80°C.

### 2) THIOMAB^{™} Conjugation

**DAR2 Part Conjugation:** An appropriate amount of dimethylacetamide (DMA) was added to the **THIOMAB^{™}** antibody, followed by the drug-linker conjugate LP1/○,A at 3 molar equivalents of the antibody (the small molecule was pre-dissolved in pure DMA or a 1:1 H₂O:DMA solution at a concentration of 10 mM). The reaction mixture was stirred at room temperature for 1-4 hours for conjugation. It was ensured the reaction concentration for this step was 5-10 mg/mL. After the reaction was complete, L-cysteine equivalent to 1.5 molar equivalents of the antibody was added to quench the reaction for 15 minutes.

**Reduction:** Tris(2-carboxyethyl)phosphine hydrochloride (TCEP) at 2-10 molar equivalents excess was added to the above reaction system. The reaction mixture was stirred at 37°C for 2 hours to ensure complete reduction of antibody interchain disulfide bonds. The reaction can also be carried out under mild conditions at 4-10°C (the reduction time generally needs to be greater than 8 hours to ensure complete reaction).

**DAR8 Part Conjugation:** The above reduced reaction mixture was cooled in an ice bath, and then the drug-linker conjugate LP2/○,B at 8-25 molar equivalents excess was added (the small molecule was pre-dissolved in pure DMA or a 1:1 H₂O:DMA solution at a concentration of 10 mM). This step must ensure that the total volume percentage of DMA in the reaction system so far does not exceed 20%. The reaction mixture was stirred at room temperature for 1-4 hours for further conjugation.

**Purification and Storage of Antibody-Drug Conjugates:** For small-scale samples, desalting columns can be used for purification to quickly remove excess drug-linker conjugates from the reaction system and exchange the buffer. For large-scale samples, protein purification systems (e.g., ÄKTA) and relevant preparative columns can be used for separation and purification to obtain relatively pure ADC products. After column purification of large-scale samples, ultrafiltration/diafiltration (UF/DF) equipment can be used for buffer exchange. The final ADC product can be stored in a histidine-acetate/sodium chloride buffer system (20 mM Histidine-acetate, 150 mM NaCl, pH 5.5) or a histidine-acetate/sucrose buffer system (20 mM Histidine-acetate, 8% sucrose, pH 5.5). Finally, the product is sterilized by filtration through a 0.22 µm filter and stored at -80°C.

This conjugation can also be achieved through selective reduction, followed by conjugation with LP1/○ ,A, then reduction and further conjugation with the drug-linker conjugate LP2/, as shown in Figure 42.

### Preparation of Antibody-Drug Conjugate thioRS7-T1000e(DAR2)-TM5-21(DAR8):

An appropriate amount of dimethylacetamide (DMA) was added to the stock solution of the Trop2-targeting antibody thioRS7 monoclonal antibody (its HC and LC amino acid sequences are shown in SEQ ID NO. 13 and 14, respectively). The drug-linker conjugate T1000e (T1000-exatecan, small molecule pre-dissolved in DMA at a concentration of 10 mM) at 3 molar equivalents of the antibody was added. The reaction mixture was stirred at room temperature for 1 hour for conjugation. After the reaction was complete, L-cysteine equivalent to 1.5 molar equivalents of the antibody was added to quench the reaction for 15 minutes. Then, tris(2-carboxyethyl)phosphine hydrochloride (TCEP) at 8 molar equivalents excess was added to the system. The reaction mixture was stirred at 37°C for another 2 hours to completely open the interchain disulfide bonds of the antibody. The reduced reaction mixture was then placed in an ice bath for cooling. The drug-linker conjugate A178 (TM5-21, small molecule pre-dissolved in a 1:1 H₂O:DMA solution at a concentration of 10 mM) at 12 molar equivalents excess was added. The reaction mixture was stirred at room temperature for 2 hours for further conjugation. The reaction mixture was purified using a protein purification system, employing a strong cation exchange (SP) column. The collected fractions were sampled for mini-pool analysis. The collected pool was then subjected to ultrafiltration and diafiltration, and finally exchanged into the final formulation buffer (20 mM Histidine-acetate, 8% sucrose, pH 5.5). The solution was sterilized by filtration through a 0.22 µm filter and stored at -80°C.

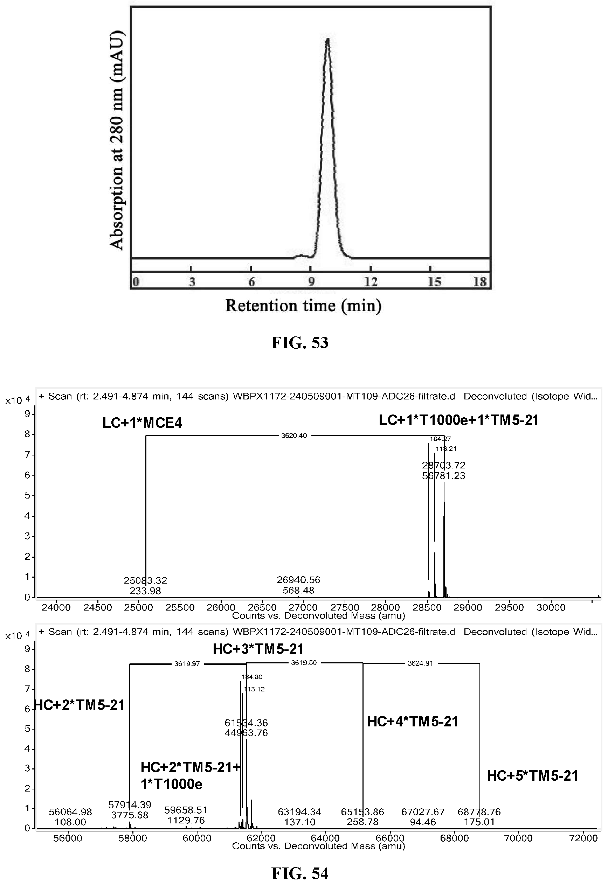

The concentration of the antibody-drug conjugate thioRS7-T1000e(DAR2)-TM5-21(DAR8) was quantified by BCA method to be 16.26 mg/mL. The aggregate content was analyzed by size-exclusion chromatography (SEC-HPLC). The SEC chromatogram is shown in **Figure 53****.** The monomer purity of the antibody-drug conjugate thioRS7-T1000e(DAR2)-TM5-21(DAR8) was 98.82%, and the aggregate content was 1.18%. The drug-to-antibody ratio (DAR) was analyzed by LC-MS method. The MS spectrum is shown in **Figure 54****.** In the antibody-drug conjugate thioRS7-T1000e(DAR2)-TMS-21(DAR8), the DAR value for the T1000e part was 2.04, and for the TM5-21 part was 7.83, resulting in a final total DAR value of 9.87. The level of unconjugated small molecule drug-related substance T1000e relative to the T1000e part in the antibody-drug conjugate was detected by precipitation method to be <0.26%. Similarly, the level of unconjugated small molecule drug-related substance A178 relative to the A178 part in the antibody-drug conjugate was detected by precipitation method to be <1.17%. The endotoxin level in the conjugate stock solution was quantified by dynamic turbidimetric method, and the result was <0.123 EU/mg.

### Preparation of Antibody-Drug Conjugate thioRS7-T1000e(DAR2)-TM5-22(DAR8):

An appropriate amount of dimethylacetamide (DMA) was added to the stock solution of the Trop2-targeting antibody thioRS7 monoclonal antibody. The drug-linker conjugate T1000e (T1000-exatecan, small molecule pre-dissolved in DMA at a concentration of 10 mM) at 3 molar equivalents of the antibody was added. The reaction mixture was stirred at room temperature for 1 hour for conjugation. After the reaction was complete, L-cysteine equivalent to 1.5 molar equivalents of the antibody was added to quench the reaction for 15 minutes. Then, tris(2-carboxyethyl)phosphine hydrochloride (TCEP) at 8 molar equivalents excess was added to the system. The reaction mixture was stirred at 37°C for another 2 hours to completely open the interchain disulfide bonds of the antibody. The reduced reaction mixture was then placed in an ice bath for cooling. The drug-linker conjugate A179 (TM5-22) (small molecule pre-dissolved in a 1:1 H₂O:DMA solution at a concentration of 10 mM) at 12 molar equivalents excess was added. The reaction mixture was stirred at room temperature for 2 hours for further conjugation. The reaction mixture was purified using a protein purification system, employing a strong cation exchange (SP) column. The collected fractions were sampled for mini-pool analysis. The collected pool was then subjected to ultrafiltration and diafiltration, and finally exchanged into the final formulation buffer (20 mM Histidine-acetate, 8% sucrose, pH 5.5). The solution was sterilized by filtration through a 0.22 µm filter and stored at -80°C.

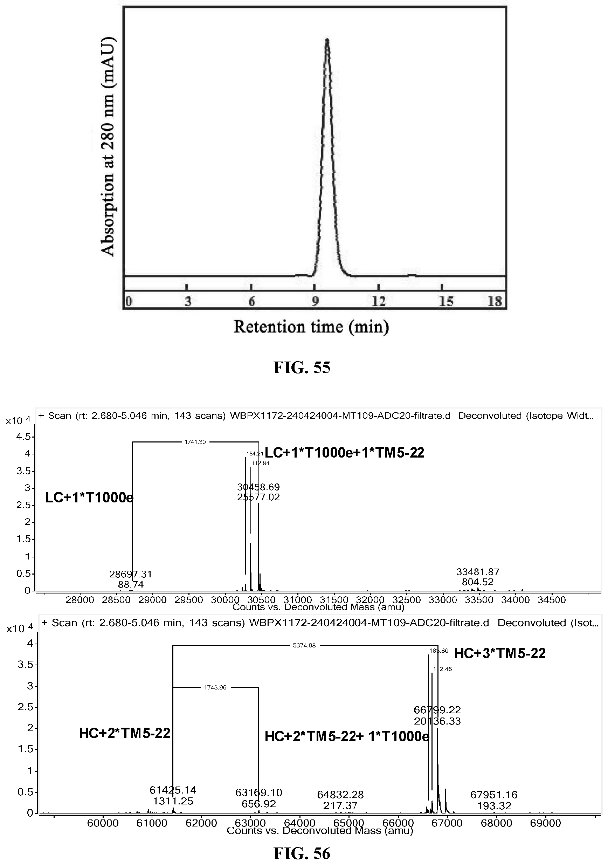

The concentration of the antibody-drug conjugate thioRS7-T1000e(DAR2)-TMS-22(DAR8) was quantified by BCA method to be 11.46 mg/mL. The aggregate content was analyzed by size-exclusion chromatography (SEC-HPLC). The SEC chromatogram is shown in **Figure 55****.** The monomer purity of the antibody-drug conjugate thioRS7-T1000e(DAR2)-TM5-22(DAR8) was 99.17%, and the aggregate content was 0.83%. The drug-to-antibody ratio (DAR) was analyzed by LC-MS method. The MS spectrum is shown in **Figure 56****.** In the antibody-drug conjugate thioRS7-T1000e(DAR2)-TM5-22(DAR8), the DAR value for the T1000e part was 2.05, and for the TM5-22 part was 7.85, resulting in a final total DAR value of 9.89. The level of unconjugated small molecule drug-related substance T1000e relative to the T1000e part in the antibody-drug conjugate was detected by precipitation method to be <0.36%. Similarly, the level of unconjugated small molecule drug-related substance A179 relative to the TM5-22 part in the antibody-drug conjugate was detected by precipitation method to be <1.66%. The endotoxin level in the conjugate stock solution was quantified by dynamic turbidimetric method, and the result was <0.087 EU/mg.

### Preparation of Antibody-Drug Conjugate thioRS7-MDP2(DAR2)-TM5-21(DAR8):

An appropriate amount of dimethylacetamide (DMA) was added to the stock solution of the Trop2-targeting antibody thioRS7 monoclonal antibody. The drug-linker conjugate A2 (MDP2) (small molecule pre-dissolved in a 1:1 H₂O:DMA solution at a concentration of 10 mM) at 3 molar equivalents of the antibody was added. The reaction mixture was stirred at room temperature for 1 hour for conjugation. After the reaction was complete, L-cysteine equivalent to 1.5 molar equivalents of the antibody was added to quench the reaction for 15 minutes. Then, tris(2-carboxyethyl)phosphine hydrochloride (TCEP) at 8 molar equivalents excess was added to the system. The reaction mixture was stirred at 37°C for another 2 hours to completely open the interchain disulfide bonds of the antibody. The reduced reaction mixture was then placed in an ice bath for cooling. The drug-linker conjugate A178 (TM5-21) (small molecule pre-dissolved in a 1:1 H₂O:DMA solution at a concentration of 10 mM) at 12 molar equivalents excess was added. The reaction mixture was stirred at room temperature for 2 hours for further conjugation. The reaction mixture was purified using a protein purification system, employing a strong cation exchange (SP) column. The collected fractions were sampled for mini-pool analysis. The collected pool was then subjected to ultrafiltration and diafiltration, and finally exchanged into the final formulation buffer (20 mM Histidine-acetate, 8% sucrose, pH 5.5). The solution was sterilized by filtration through a 0.22 µm filter and stored at -80°C.

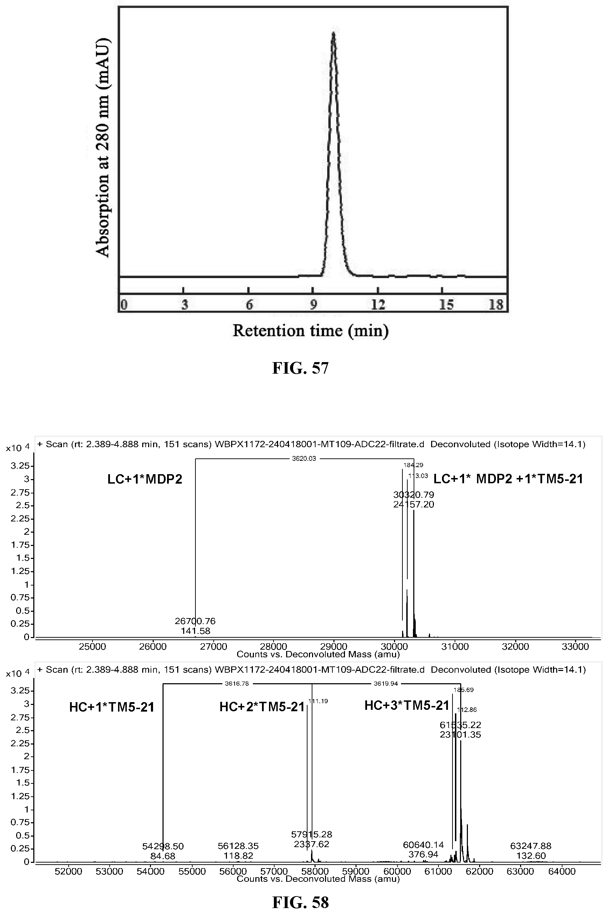

The concentration of the antibody-drug conjugate thioRS7- MDP2 (DAR2)-TM5-21(DAR8) was quantified by BCA method to be 12.01 mg/mL. The aggregate content was analyzed by size-exclusion chromatography (SEC-HPLC). The SEC chromatogram is shown in **Figure 57****.** The monomer purity of the antibody-drug conjugate thioRS7- MDP2 (DAR2)-TM5-21(DAR8) was 99.45%, and the aggregate content was 0.55%. The drug-to-antibody ratio (DAR) was analyzed by LC-MS method. The MS spectrum is shown in **Figure 58****.** In the antibody-drug conjugate thioRS7- MDP2 (DAR2)-TMS-21(DAR8), the DAR value for the A10 (MDP2) part was 2.00, and for the A178 (TM5-21) part was 7.80, resulting in a final total DAR value of 9.80. The level of unconjugated small molecule drug-related substance MDP2 relative to the MDP2 part in the antibody-drug conjugate was detected by precipitation method to be 0.68%. Similarly, the level of unconjugated small molecule drug-related substance A178 (TM5-21) relative to the A178 (TM5-21) part in the antibody-drug conjugate was detected by precipitation method to be 0.73%. The endotoxin level in the conjugate stock solution was quantified by dynamic turbidimetric method, and the result was <0.083 EU/mg.

### Example 3 Stability Testing of Antibody-Drug Conjugates

Due to the strong hydrophobicity of antibody-drug conjugates with high drug loading, the stability of such ADC molecules is inevitably affected. The ADCs prepared in the present invention incorporate hydrophilic groups, such as polysarcosine peptide chains, at specific positions, which have a better "shielding effect" and may exhibit improved stability. We designed accelerated stability tests to verify this hypothesis.

The compared molecules include:
1. Trastuzumab-MDP2 (T-MDP2)
2. Trastuzumab-MDP54 (T-MDP54)
3. RS7-MDP12
4. RS7-MDP44
5. thioRS7-T1000e(DAR2)-TM5-21(DAR8)
6. thioRS7-MDP2(DAR2)-TM5-21(DAR8)

We diluted the test molecule samples with formulation buffer (20 mM Histidine-acetic acid, 150 mM NaCl, pH 5.5) to a concentration of 5 mg/mL, and took 50 µL directly for freeze-thaw testing (Freeze&Thaw) 1, 3, and 5 times. Simultaneously, we placed 50 µL of the samples in a 37°C water bath for stability testing at 0 hours, 24 hours, 48 hours, 72 hours, 168 hours, and 336 hours. The content of normal structured ADC molecules (main peak) and aggregated molecules in the samples was quantitatively analyzed using a TSK-GEL SWXL3000 size-exclusion chromatography column on an Agilent 1260 Infinity II bio-inert HPLC system.

The HPLC mobile phase was 200 mM phosphate buffer (pH 7.0) + 150 mM KCl + 15% IPA. The column temperature was 25 °C, the injection volume was 7 µL, the flow rate was 0.75 mL/min, and the UV detection wavelengths were 280 nm and 370 nm.

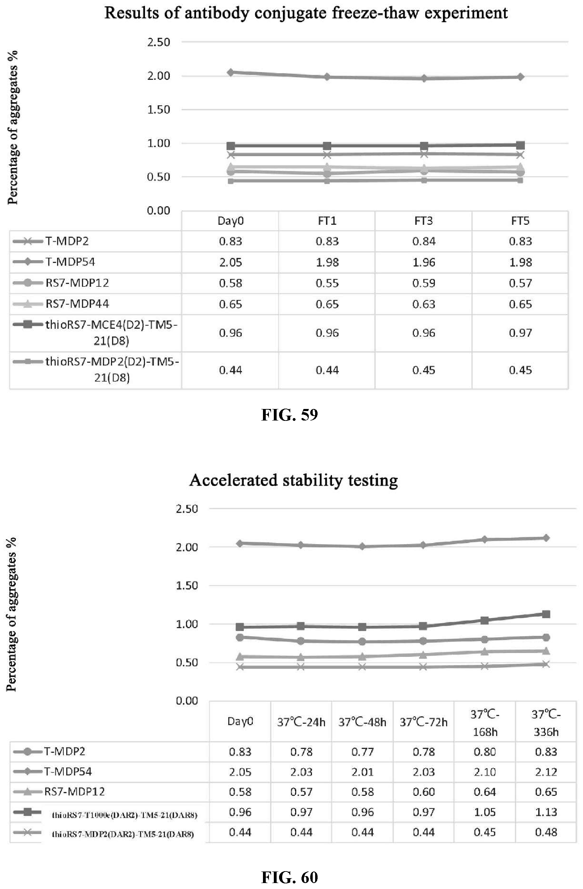

The statistical results of sample concentration changes are shown in **Figures 59 and 60****.** The analysis of the increase in aggregate molecule proportion in the freeze-thaw test and accelerated stability test samples by size-exclusion chromatography showed that the above multi-payload antibody-drug conjugates all have good stability. In contrast, multi-payload antibody-drug conjugates typically are difficult to obtain as monomers (e.g., drug-linker A3 and its corresponding antibody conjugate C3 mostly appear as aggregates), or the stability of such multi-payload conjugates is extremely poor. The above results verify that the hydrophilic polysarcosine peptide chain introduced at specific design positions in the present invention has a "shielding effect," providing an "unexpected" improvement in stability for multi-payload antibody-drug conjugate molecules.

### Example 4 In Vitro Cytotoxic Activity Testing

The human breast cancer cell lines SK-BR-3 and BT-474 with low Her2 expression, and the human gastric cancer cell line NCI-N87, were selected as the cell lines for *in vitro* activity testing in this experiment, to observe the dose-response of different antibody-drug conjugates on cell killing. The initial seeding density for each cell line was set at: 2 × 10³ cells/well. After 16-24 hours, the cytotoxic activity was measured. The final concentration of the antibody-drug conjugates prepared in Example 2 for testing was set starting at 5000 nM, with a series of 10 concentrations designed from 5000 to 0.006 nM (4-10-fold serial dilution). The killing (or inhibition) changes were observed over 120 hours. Luminescent Cell Viability Assay was used, and IC₅₀ values were calculated after reading the luminescence data.

| | HCC1954 | HCT-15 | HCT-116 |
|---|---|---|---|
| | (HER2 high) | (HER2 low) | (HER2 low) |
| | IC₅₀ (nM) | IC₅₀ (nM) | IC₅₀ (nM) |
| Trastuzumab | > 1000 | > 1000 | > 1000 |
| IgG-T1000e | 1178 | / | / |
| MTX-1000 (control sample) | 0.456 | 595.2 | 150.9 |
| T-MCD1 | / | > 1000 | > 1000 |
| T-MDP2 | 0.206 | 28.2 | 8.1 |
| T-MDP3 | 0.185 | 72.6 | 38.8 |
| T -MDP7 | / | 24.6 | 10.9 |
| T-MDP8 | / | 778.7 | 72.3 |
| T-MDP9 | / | 79.1 | 46.5 |
| T-MDP10 | / | 151.3 | 33.5 |

From the activity test results, the dual-drug ADCs prepared from the compounds of the present invention exhibit good anti-proliferative activity on low-expression tumor cells, showing significantly stronger anti-tumor activity compared to control single-drug ADCs.

### Example 5 In Vivo Anti-Tumor Efficacy Determination

The efficacy of the combinations of the present invention can be measured *in vivo,* i.e., in allografts or xenografts of cancer cells implanted in rodents, and the tumors are treated with said combinations. Test mice are treated with the drug or control and monitored for weeks or longer to measure the time to tumor doubling, log cell kill, and tumor suppression.

### 5.1 In Vivo Anti-Tumor Activity of Antibody-Drug Conjugates Against Human Breast Cancer Cell Line HCC1954

HCC1954 human breast cancer cells (ATCC), which express medium to high levels of HER2, were suspended in PBS, and 5×10⁶ cells were subcutaneously transplanted into the right flank of female nude mice. Random grouping was performed on day 12. On the grouping day, the antibody-drug conjugates T-MDP2, T-MDP3, T-T1000e, and the combination group of T-T1000e and Rucaparib were administered intravenously via the tail vein at a dose of 3 mg/kg each. A negative control group was set up, receiving an equivalent dose of PBS via tail vein injection. Administration was performed once a week, for a total of 1 administration.

After administration, tumor volume was measured twice a week using calipers, and body weight was recorded. Tumor volume was calculated according to the following formula: V= 1/2×a×b², where a and b represent length and width, respectively.

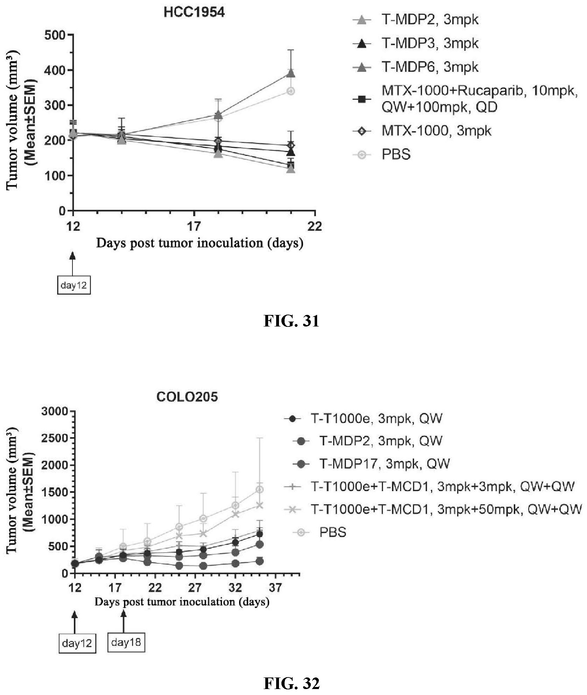

Figure 31 shows the *in vivo* tumor volume-time curve for the HCC1954 mouse model. The results show that the antibody-drug conjugates MTX-1000, T-MDP2, T-MDP3, and the combination of MTX-1000 with the small molecule Rucaparib all exhibited good anti-tumor activity. At 3 mg/kg, the antibody-drug conjugates T-MDP2 and T-MDP3 showed stronger efficacy than the control single-drug antibody conjugate MTX-1000, as well as the corresponding single-drug antibody conjugate T-T1000e combined with the small molecule Rucaparib.

### 5.2 In Vivo Anti-Tumor Activity of Antibody-Drug Conjugates Against Human Colon Cancer Cell Line COLO205

COLO205 human colon cancer cells (ATCC), which express low to medium levels of HER2, were suspended in PBS, and 5×10⁶ cells were subcutaneously transplanted into the right flank of female nude mice. Random grouping was performed on days 12, 14, and 11 in three separate experiments, respectively. On the grouping day, the antibody-drug conjugates T-MDP2, T-MDP17, T-T1000e, and the combination group of T-T1000e and T-MCD1 were administered intravenously via the tail vein at a dose of 3 mg/kg each. A negative control group was set up, receiving an equivalent dose of PBS via tail vein injection. Administration was performed once a week, for a total of 2 administrations.

After administration, tumor volume was measured twice a week using calipers, and body weight was recorded. Tumor volume was calculated according to the following formula: V= 1/2×a×b², where a and b represent length and width, respectively.

Figure 32 shows the *in vivo* tumor volume-time curve for the COLO205 mouse model. The results show that the antibody-drug conjugates MTX-1000, T-MDP2, T-MDP17, and the combination of MTX-1000 with T-MCD1 all exhibited good anti-tumor activity. At 3 mg/kg, the antibody-drug conjugates T-MDP2 and T-MDP17 showed stronger efficacy than the control single-drug antibody conjugate T-T1000e, and were significantly superior to the combination of the single-drug antibody conjugates T-T1000e and T-MCD1. This indicates that the multidrug conjugate is superior to the combination of single-drug conjugates.

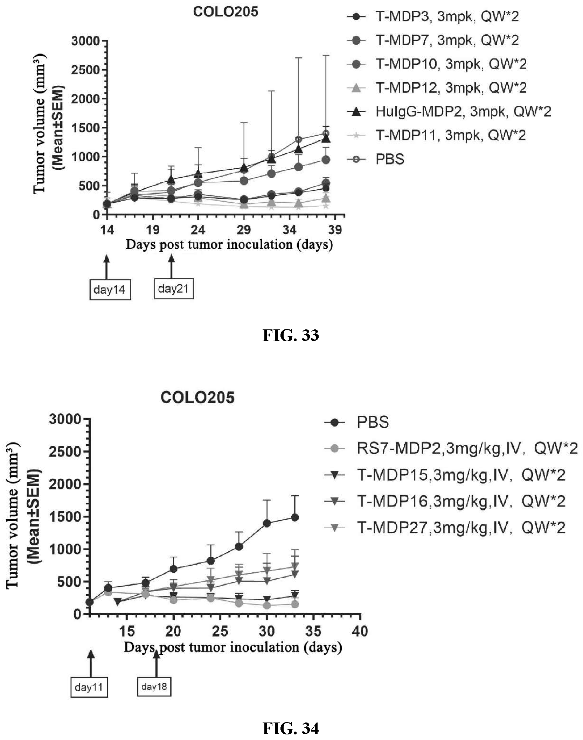

Furthermore, Figures 33 and 34 respectively show the *in vivo* tumor volume-time curves for the COLO205 mouse model after administration of the HER2-targeting trastuzumab conjugates T-MDP3, T-MDP7, T-MDP10, T-MDP11, T-MDP12, T-MDP15, T-MDP16, T-MDP27 and the TROP2-targeting RS7 conjugate RS7-MDP2. The results show that the above antibody-drug conjugates have a significant inhibitory effect on tumor growth.

### 5.3 In Vivo Anti-Tumor Activity of Antibody-Drug Conjugates Against Human Colorectal Cancer Cell Line SW837

SW837 human colorectal cancer cells (ATCC), which express low to medium levels of HER3, were suspended in PBS, and 5×10⁶ cells were subcutaneously transplanted into the right flank of female nude mice. Random grouping was performed on day 14. On the grouping day, the antibody-drug conjugates P-MDP2, P-MDP3, P-MDP12, P-MDP15, P-MDP22, P-MDP27, P-T1000e, and U3-1402 were administered intravenously via the tail vein at a dose of 5 mg/kg each. A negative control group was set up, receiving an equivalent dose of PBS via tail vein injection. Administration was performed once a week, for a total of 1 administration.

After administration, tumor volume was measured twice a week using calipers, and body weight was recorded. Tumor volume was calculated according to the following formula: V= 1/2×a×b², where a and b represent length and width, respectively.

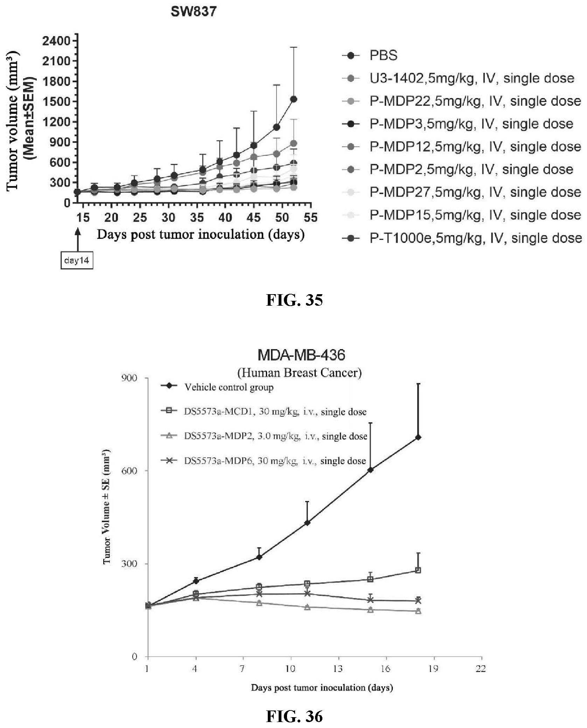

Figure 35 shows the *in vivo* tumor volume-time curve for the SW837 mouse model. The results show that the antibody-drug conjugates P-MDP2, P-MDP3, P-MDP12, P-MDP15, P-MDP22, P-MDP27, and P-T1000e all exhibited good anti-tumor activity. At 5 mg/kg, the antibody-drug conjugates P-MDP2, P-MDP3, P-MDP12, P-MDP15, P-MDP22, and P-MDP27 showed stronger efficacy than the control single-drug antibody conjugate P-T1000e and were significantly stronger than the control drug U3-1402.

### 5.4 In Vivo Anti-Tumor Activity of Antibody-Drug Conjugates Against Human Triple-Negative Breast Cancer Cell Line MDA-MB-436

MDA-MB-436 human breast cancer cells (ATCC), which express medium level of B7H3, were suspended in PBS, and 1.1×10⁷ cells were subcutaneously transplanted into the right flank of female nude mice. Random grouping was performed on day 21. Taking the grouping day as day 1, the antibody-drug conjugates DS5573a-MDP2, as well as DS5573a-MCD1 and DS5573a-MDP6 at 30 mg/kg, were administered intravenously via the tail vein. A negative control group was set up, receiving an equivalent dose of PBS via tail vein injection. Administration was performed once a week, for a total of 1 administration.

After administration, tumor volume was measured twice a week using calipers, and body weight was recorded. Tumor volume was calculated according to the following formula: V= 1/2×a×b², where a and b represent length and width, respectively.

Figure 36 shows the *in vivo* tumor volume-time curve for the MDA-MB-436 mouse model. The results show that the above antibody-drug conjugates all exhibited good anti-tumor activity.

### 5.5 In Vivo Anti-Tumor Activity of Antibody-Drug Conjugates Against Human Pancreatic Cancer Cell Line BXPC-3

BXPC-3 human pancreatic cancer cells (ATCC), which highly express Tissue Factor, were suspended in PBS, and 5×10⁶ cells were subcutaneously transplanted into the right flank of female nude mice. Random grouping was performed on day 5. On the grouping day, the antibody-drug conjugate Tisotumab-A132 was administered intravenously via the tail vein at doses of 15 mg/kg and 30 mg/kg. A negative control group was set up, receiving an equivalent dose of PBS via tail vein injection. Administration was performed once a week, for a total of 2 administrations.

After administration, tumor volume was measured twice a week using calipers, and body weight was recorded. Tumor volume was calculated according to the following formula: V= 1/2×a×b², where a and b represent length and width, respectively.

Figure 37 shows the *in vivo* tumor volume-time curve for the BXPC-3 mouse model. The results show that the antibody-drug conjugate Tisotumab-A132 exhibited dose-dependent good anti-tumor activity.

### 5.6 In Vivo Anti-Tumor Activity of Antibody-Drug Conjugates RS7-A132, RS7-A46, RS7-A149 Against Human Pancreatic Cancer Cell Line BXPC-3

BXPC-3 human pancreatic cancer cells (ATCC), which express medium to high levels of TROP2, were suspended in PBS, and 5×10⁶ cells were subcutaneously transplanted into the right flank of female nude mice. Random grouping was performed on day 5. On the grouping day, the antibody-drug conjugates RS7-A132, RS7-A146, and RS7-A149 were administered intravenously via the tail vein at a dose of 30 mg/kg each. A negative control group was set up, receiving an equivalent dose of PBS via tail vein injection. Administration was performed once a week, for a total of 2 administrations.

After administration, tumor volume was measured twice a week using calipers, and body weight was recorded. Tumor volume was calculated according to the following formula: V= 1/2×a×b², where a and b represent length and width, respectively.

**Figure 43** shows the *in vivo* tumor volume-time curve for the BXPC-3 mouse model. The results show that the antibody-drug conjugates RS7-A132, RS7-A146, and RS7-A149 exhibited good anti-tumor activity.

### 5.7 In Vivo Anti-Tumor Activity of Antibody-Drug Conjugates Against Human Breast Cancer T-47D

T-47D human breast cancer cells (ATCC), which express medium level of TROP2, were suspended in PBS, and 5×10⁶ cells were subcutaneously transplanted into the right flank of female nude mice. Random grouping was performed on day 5. On the grouping day, the antibody-drug conjugates listed in **Figure 44** were administered intravenously via the tail vein at a dose of 7 mg/kg each. Positive and negative control groups were set up, receiving an equivalent dose of PBS via tail vein injection. Administration was performed once a week, for a total of 2 administrations.

After administration, tumor volume was measured twice a week using calipers, and body weight was recorded. Tumor volume was calculated according to the following formula: V= 1/2×a×b², where a and b represent length and width, respectively.

**Figure 44** shows the *in vivo* tumor volume-time curve for the T-47D mouse model. The results show that most antibody-drug conjugates exhibited better anti-tumor activity compared to the positive control. At the same time, since Exatecan as a cytotoxin has a lower DAR value and superior anti-tumor activity, this new class of antibody-drug conjugates is expected to have a better therapeutic index and safety window in humans.

From the activity test results, the multi-payload ADCs prepared from the compounds of the present invention all exhibited certain *in vivo* anti-tumor activity and showed significantly stronger anti-tumor activity compared to the control samples. Tumor-bearing mice tolerated all the above drugs well, with no symptoms such as weight loss occurring.

The present invention will be applied to, but not limited to, including tumors with low expression of tumor targets, tumors with innate and/or acquired resistance to single-drug antibody conjugates (e.g., camptothecin-based ADC drugs), and further improving the response to combined tumor immunotherapy (e.g., PD-1, PD-L1, CTLA-4, etc.).

The present disclosure describes several embodiments, but this description is exemplary and not restrictive. For those of ordinary skill in the art, it is apparent that there may be more embodiments and implementations within the scope of the embodiments described in this disclosure. Although many possible feature combinations are shown herein and discussed in the specific description, many other combinations of the disclosed features are also possible. Unless specifically limited, any feature or element of any embodiment may be combined with or substituted for any other feature or element in any other embodiment.

## Claims

1. A compound, or a tautomer, a mesomer, a racemate, an enantiomer, or a diastereomer thereof, or a deuterated compound form thereof, or a mixture form thereof, or a pharmaceutically acceptable salt, prodrug, or solvate thereof, wherein the compound comprises a structure represented by formula I: 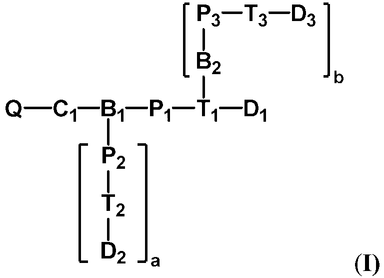 wherein,
Q is a functional group capable of conjugating to cysteine, lysine, a non-natural amino acid, or a glycosyl of an antibody molecule;
C₁ is selected from the group consisting of: a direct bond, optionally substituted alkylene, optionally substituted polyethylene glycol group, optionally substituted alkenylene, optionally substituted alkynylene, optionally substituted alicyclylene, optionally substituted aliphatic heterocyclylene, optionally substituted arylene, and optionally substituted heteroarylene; when substituted, the substituent(s) are selected from halogen, hydroxyl, amino, carboxyl, sulfonic acid group, sulfone group, phosphonic acid group, and alkoxy;
B₁ and B₂ are independently a direct bond or a branching group, provided that when C₁ is a direct bond, B₁ is not a direct bond;
P₁, P₂, and P₃ are each independently an optionally substituted polypeptide residue or a glucose fragment;
T₁, T₂, and T₃ are each independently a direct bond or an optionally substituted spacer group, provided that at most two of T₁, T₂, and T₃ are direct bonds and at least one of B₂, T₁, T₂, and T₃ is substituted with a hydrophilic group;
D₁, D₂, and D₃ are a first drug unit, a second drug unit, and a third drug unit, respectively, and are the same or different;
a and b are independently 0, 1, 2, or 3, provided that a and b are not both 0; and
when a=0, B₁ is a direct bond and T₁ is not a direct bond;
when b=0, B₁ is not a direct bond.

2. The compound, or a tautomer, a mesomer, a racemate, an enantiomer, or a diastereomer thereof, or a deuterated compound form thereof, or a mixture form thereof, or a pharmaceutically acceptable salt, prodrug, or solvate thereof, according to claim 1, wherein the compound comprises a structure represented by formula Ia: 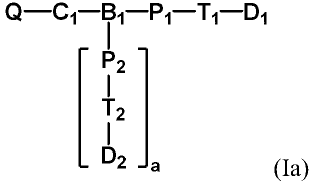 wherein:
Q is a functional group capable of conjugating to cysteine, lysine, a non-natural amino acid, or a glycosyl of an antibody molecule;
C₁ is selected from the group consisting of: a direct bond, optionally substituted alkylene, optionally substituted polyethylene glycol group, optionally substituted alkenylene, optionally substituted alkynylene, optionally substituted alicyclylene, optionally substituted aliphatic heterocyclylene, optionally substituted arylene, and optionally substituted heteroarylene; when substituted, the substituent(s) are selected from halogen, hydroxyl, amino, carboxyl, sulfonic acid group, sulfone group, phosphonic acid group, and alkoxy;
B₁ is a branching group;
P₁ and P₂ are each independently an optionally substituted polypeptide residue or a glucose fragment;
T₁ and T₂ are each independently a direct bond or an optionally substituted spacer group, provided that at most one of T₁ and T₂ is a direct bond, and at least one of T₁ and T₂ is substituted with a hydrophilic group;
D₁ and D₂ are a first drug unit and a second drug unit, respectively, and are the same or different;
a is 1, 2, or 3.

3. The compound, or a tautomer, a mesomer, a racemate, an enantiomer, or a diastereomer thereof, or a deuterated compound form thereof, or a mixture form thereof, or a pharmaceutically acceptable salt, prodrug, or solvate thereof, according to claim 1, wherein the compound comprises a structure represented by formula Ib: 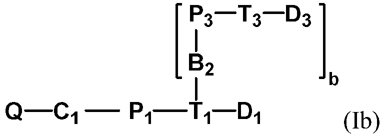 wherein:
Q is a functional group capable of conjugating to cysteine, lysine, a non-natural amino acid, or a glycosyl of an antibody molecule;
C₁ is selected from the group consisting of: optionally substituted alkylene, optionally substituted polyethylene glycol group, optionally substituted alkenylene, optionally substituted alkynylene, optionally substituted alicyclylene, optionally substituted aliphatic heterocyclylene, optionally substituted arylene, and optionally substituted heteroarylene; when substituted, the substituent(s) are selected from halogen, hydroxyl, amino, carboxyl, sulfonic acid group, sulfone group, phosphonic acid group, and alkoxy;
B₂ is a branching group;
P₁ and P₃ are each independently a direct bond or an optionally substituted polypeptide residue or a glucose fragment;
T₁ is an optionally substituted spacer group, T₃ is a direct bond or an optionally substituted spacer group, and at least one of B₂ and T₃ is substituted with a hydrophilic group;
D₁ and D₃ are a first drug unit and a third drug unit, respectively, and are the same or different;
b is 1, 2, or 3.

4. The compound, or a tautomer, a mesomer, a racemate, an enantiomer, or a diastereomer thereof, or a deuterated compound form thereof, or a mixture form thereof, or a pharmaceutically acceptable salt, prodrug, or solvate thereof, according to claim 1, wherein:
B₁ and B₂ are independently a branching group;
P₁, P₂, and P₃ are each independently an optionally substituted polypeptide residue or a glucose fragment;
T₁ is an optionally substituted spacer group, T₂ and T₃ are each independently a direct bond or an optionally substituted spacer group, and at least one of B₂, T₂, and T₃ is substituted with a hydrophilic group;
D₁, D₂, and D₃ are a first drug unit, a second drug unit, and a third drug unit, respectively, and are the same or different; and
a and b are independently 1, 2, or 3.

5. The compound, or a tautomer, a mesomer, a racemate, an enantiomer, or a diastereomer thereof, or a deuterated compound form thereof, or a mixture form thereof, or a pharmaceutically acceptable salt, prodrug, or solvate thereof, according to any one of claims 1 to 4, wherein:
Q is selected from the group consisting of: optionally substituted 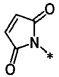 (Q1), optionally substituted 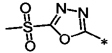 (Q2), optionally substituted 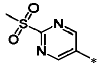 (Q3), optionally substituted 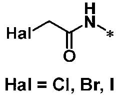 (Q4), optionally substituted 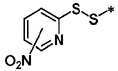 (Q5), optionally substituted 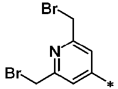 (Q6), optionally substituted 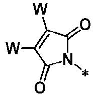 (Q7), optionally substituted 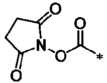 (Q8), optionally substituted 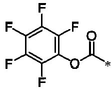 (Q9), wherein * represents a site connected to C₁, the W group in Q7 is bromo or Ar-S, wherein Ar is phenyl or substituted phenyl, and the substituent(s) in the substituted phenyl are selected from alkyl, alkoxy, halogen, ester group, nitro, and -C(O)NR1R2-, wherein R1 and R2 are independently selected from a chemical bond, H, and alkyl, or R1 and R2 together form a 5-7 membered heterocyclic ring with one or more heteroatoms selected from O, N, and S.

6. The compound, or a tautomer, a mesomer, a racemate, an enantiomer, or a diastereomer thereof, or a deuterated compound form thereof, or a mixture form thereof, or a pharmaceutically acceptable salt, prodrug, or solvate thereof, according to any one of claims 1 to 5, wherein:
C₁ is selected from: a direct bond, -(CH₂)ₘ₁-, -(CH₂)ₘ₁-(O-CH₂-CH₂)ₘ₂-, -(CH₂)ₘ₁-(O-CH₂-CH₂)ₘ₂-(CH₂)ₘ₃-, -(CH₂)ₘ₁-C(O)NH-(CH₂)ₘ₂-, -(CH₂)ₘ₁-NHC(O)-(CH₂)ₘ₂-, and -(C≡ C-(CH₂)ₘ₁-, wherein m1, m2, and m3 are independently integers from 1 to 6, and the indicated substituent is connected to Q on the left side and to B₁ or P₁ on the right side.

7. The compound, or a tautomer, a mesomer, a racemate, an enantiomer, or a diastereomer thereof, or a deuterated compound form thereof, or a mixture form thereof, or a pharmaceutically acceptable salt, prodrug, or solvate thereof, according to any one of claims 1 to 6, wherein:
B₁ is selected from the group consisting of: a direct bond, 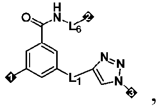 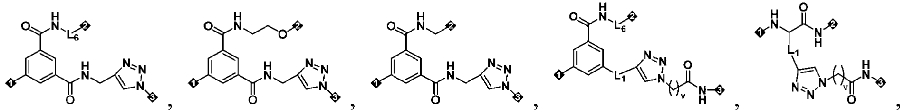 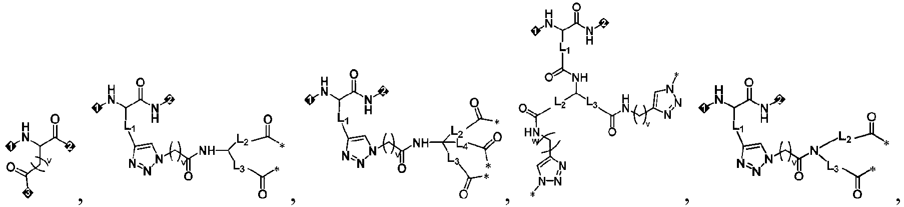 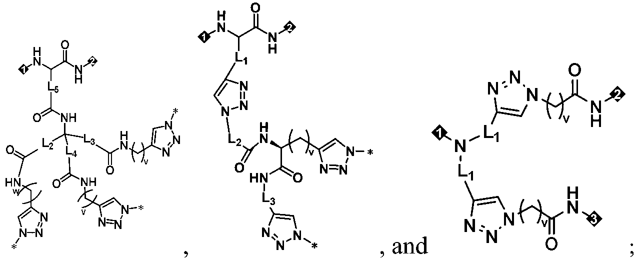
preferably, 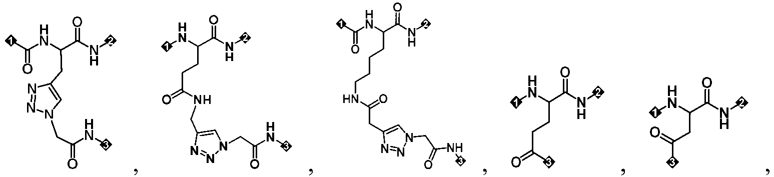 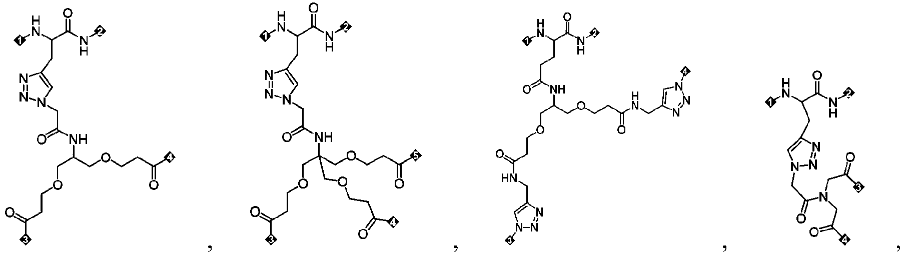 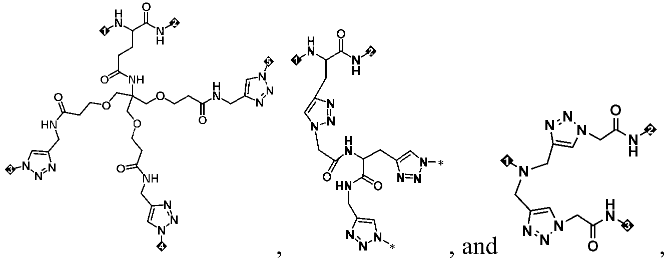 wherein attachment point 1 is connected to C₁ or Q (when C₁ is a direct bond), attachment point 2 is connected to P₁, attachment points *, 3, 4, and 5 are connected to P₂, L₁ is selected from -(CH₂)ₘ₁-, -(CH₂)ₘ₁-C(O)NH-(CH₂)ₘ₂-, -(CH₂)ₘ₁-NHC(O)-(CH₂)ₘ₂-, L₂, L₃, and L₄ are independently -(CH₂)ₘ₁-(O-CH₂-CH₂)ₘ₂- or -(CH₂)ₘ₁-(O-CH₂-CH₂)ₘ₂-(CH₂)ₘ₃-, L₅ is -(CH₂)ₘ₁-, L₆ is -(CH₂)ₘ₁- or - (CH₂-CH₂-O)m₂, wherein m1, m2, m3, w, and v are each independently 1, 2, 3, 4, 5, or 6;
preferably, when C₁ is a direct bond, B₁ is

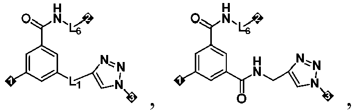

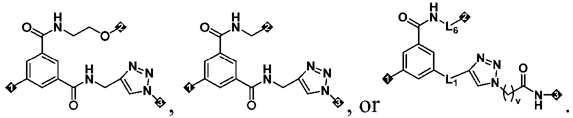

8. The compound, or a tautomer, a mesomer, a racemate, an enantiomer, or a diastereomer thereof, or a deuterated compound form thereof, or a mixture form thereof, or a pharmaceutically acceptable salt, prodrug, or solvate thereof, according to any one of claims 1 to 7, wherein:
B₂ is selected from the group consisting of: 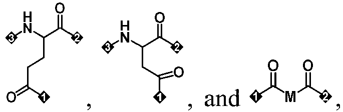 wherein attachment point 1 is connected to T₁, attachment point 2 is connected to P₃, attachment point 3 is connected to the hydrophilic group, M is selected from the group consisting of: optionally substituted C₁-C₆ alkylene, optionally substituted C₁-C₆ alkoxy, and optionally substituted C₁-C₅ alkenylene.

9. The compound, or a tautomer, a mesomer, a racemate, an enantiomer, or a diastereomer thereof, or a deuterated compound form thereof, or a mixture form thereof, or a pharmaceutically acceptable salt, prodrug, or solvate thereof, according to any one of claims 1 to 8, wherein:
T₁, T₂, T₃ are independently selected from the group consisting of: a direct bond, 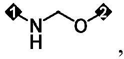 and optionally substituted 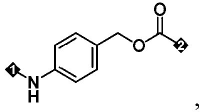 wherein attachment point 1 is connected to P₁, P₂, or P₃, and attachment point 2 is connected to D₁, D₂, or D₃.

10. The compound, or a tautomer, a mesomer, a racemate, an enantiomer, or a diastereomer thereof, or a deuterated compound form thereof, or a mixture form thereof, or a pharmaceutically acceptable salt, prodrug, or solvate thereof, according to any one of claims 1 to 9, wherein:
P₁, P₂, P₃ comprises an optionally substituted polypeptide residue composed of amino acids selected from the group consisting of: phenylalanine, isoleucine, leucine, tryptophan, valine, methionine, tyrosine, alanine, threonine, histidine, serine, glutamine, arginine, lysine, asparagine, glutamic acid, proline, citrulline, aspartic acid, and glycine; preferably, P₁, P₂, P₃ comprises an optionally substituted polypeptide residue composed of amino acids selected from the group consisting of: glycine, phenylalanine, valine, alanine, arginine, citrulline, aspartic acid, asparagine, and lysine;
more preferably, P₁, P₂, P₃ comprises an optionally substituted polypeptide residue selected from the group consisting of: phenylalanine-lysine (Phe-Lys), valine-alanine (Val-Ala), valine-citrulline (Val-Cit), glutamic acid-valine-alanine (Glu-Val-Ala), glutamic acid-valine-citrulline (Glu-Val-Cit), valine-lysine (Val-Lys), alanine-alanine (Ala-Ala), alanine-alanine-alanine (Ala-Ala-Ala), alanine-alanine-asparagine (Ala-Ala-Asn), alanine-leucine (Ala-Leu), leucine-leucine (Leu-Leu), phenylalanine-arginine (Phe-Arg), phenylalanine-lysine (Phe-Lys), 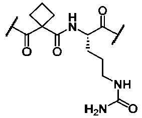 (cBu-Cit), 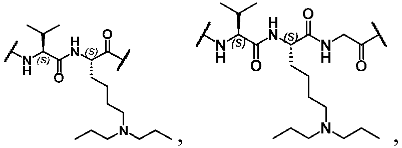 glycine-glycine-phenylalanine-glycine (Gly-Gly-Phe-Gly), glycine-proline (Gly-Pro);
or, wherein one of the combination P₂-T₂ and the combination P₃-T₃ is 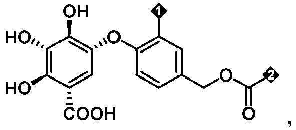 wherein attachment point 1 is connected to B₁ or B₂, and attachment point 2 is connected to D₂ or D₃.

11. The compound, or a tautomer, a mesomer, a racemate, an enantiomer, or a diastereomer thereof, or a deuterated compound form thereof, or a mixture form thereof, or a pharmaceutically acceptable salt, prodrug, or solvate thereof, according to any one of claims 1 to 10, wherein:
the hydrophilic group is selected from: substituted polysarcosine residue, polyglycerol, polyol, glycosyl, cyclodextrin, substituted ethylene glycol fragment, substituted glycosylated polyethylene glycol, substituted glycosylated polyglycerol, substituted cyclodextrin polyethylene glycol, or a combination thereof;
preferably, the substituted polysarcosine residue is 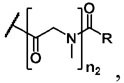 wherein n₂ is an integer between 4 and 20, for example between 4 and 16, R is selected from: C₁-C₆ alkyl, C₁-C₆ cycloalkyl, and C₁-C₆ alkoxy;
preferably, the substituted glycosylated polyethylene glycol fragment is 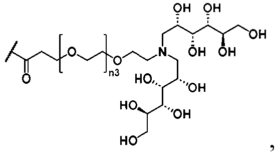 wherein n₃ is an integer between 4 and 18, for example between 4 and 12;
preferably, the substituted glycosylated polyglycerol fragment is 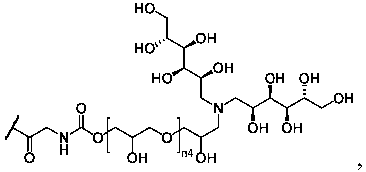 wherein n₄ is an integer between 4 and 12, for example between 4 and 10;
preferably, the substituted polyethylene glycol fragment is 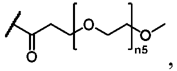 wherein n₅ is an integer between 4 and 24, for example between 8 and 18;
preferably, at least one of T₁, T₂, T₃ is 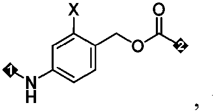 wherein X is optionally substituted 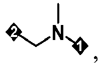 the hydrophilic group is connected to T₁, T₂, or T₃ via X, wherein attachment point 1 is connected to the hydrophilic group, and attachment point 2 is connected to one of T₁, T₂, T₃.

12. The compound, or a tautomer, a mesomer, a racemate, an enantiomer, or a diastereomer thereof, or a deuterated compound form thereof, or a mixture form thereof, or a pharmaceutically acceptable salt, prodrug, or solvate thereof, according to any one of claims 1 to 11, wherein:
D₁, D₂, D₃ are a first anti-cancer agent, a second anti-cancer agent, and a third anti-cancer agent; preferably, at least two of the first, second, and third anti-cancer agents have a synergistic anti-cancer effect;
preferably, D₁, D₂, D₃ are independently a cytotoxic drug or a tumor-targeted therapeutic drug; preferably, at least one of D₁, D₂, and D₃ is a cytotoxic drug, and at least another one is a tumor-targeted therapeutic drug; also preferably, at least two of D₁, D₂, and D₃ are tumor-targeted therapeutic drugs, for example, tumor-targeted therapeutic drugs that can produce a synergistic or additive effect;
preferably, the cytotoxic drug is selected from drug units targeting topoisomerase, drug units targeting tubulin, nucleoside antimetabolite anti-cancer drug units, and drug units targeting DNA;
preferably, the tumor-targeted therapeutic drug is selected from drug units targeting DNA damage response (DDR) or "synthetic lethality" related pathways, drug units targeting epigenetics, drug units targeting apoptosis-related pathways, drug units targeting transcription factors, and drug units targeting immune activation pathway-related targets, comprising: inhibitors of EFGR pathway-related targets; Ras-Raf-MAPK pathway inhibitors; PI3K/AKT/mTOR pathway inhibitors; cell cycle pathway inhibitors; cGAS-STING signaling pathway agonists; estrogen receptor antagonists; androgen receptor antagonists; glucocorticoid receptor modulators; autophagy inhibitors; FAK inhibitors; Smo inhibitors; BTK inhibitors; PDE4 inhibitors; Lck inhibitors; PLK1 inhibitors; TLR7/8 modulators; N-myristoyltransferase (NMT) inhibitors;
preferably, the drug unit targeting topoisomerase is a topoisomerase I (TOPO1) inhibitor or topoisomerase II (TOPO2) inhibitor, comprising Exatecan (CAS 171335-80-1), DXd (CAS 1599440-33-1), 7-ethyl-10-hydroxycamptothecin (SN38, CAS 86639-52-3), Belotecan (CAS 213819-48-8), (4-NH₂)-Exatecan (AZD'0132, CAS 2495742-21-5), 7-MAD-MDCPT (CAS 765871-81-6), 7-aminomethyl-10-methyl-11-fluorocamptothecin (CAS 2378616-23-8), Voreloxin (CAS 175414-77-4), or derivatives and analogs thereof;
preferably, the drug unit targeting tubulin is Eribulin, Vinblastine, Paclitaxel, MMAE, MMAF, Maytansine, or derivatives thereof;
preferably, the nucleoside antimetabolite anti-cancer drug is Gemcitabine, Decitabine;
preferably, the drug unit targeting DNA is a DNA minor groove binder (PBD), a DNA alkylator (Duocarmycin), Trabectedin, Lurbinectedin, or derivatives thereof;
preferably, the drug unit targeting DNA damage response (DDR) or "synthetic lethality" related pathways is a PARP inhibitor, ATR inhibitor, CHK1 inhibitor, ATM inhibitor, DNA-PK inhibitor, WEE1 inhibitor, POLQ inhibitor, CDK12 inhibitor, USP1 inhibitor, PKMYT1 inhibitor, or Rad51 inhibitor;
the PARP inhibitor is preferably Rucaparib, Niraparib, Veliparib, A-966492, Talazoparib, AZD5305, Venadaparib, Mefuparib, or analogs thereof; the ATR inhibitor is preferably Berzosertib, Ceralasertib, or analogs thereof; the CHK1 inhibitor is preferably Prexasertib (LY2606368), AZD7762, Rabusertib (LY2603618), MK-8776 (SCH 900776), CHIR-124, PF-477736, CCT245737 (SRA737, PNT-737), GDC-0575 (ARRY-575), or analogs thereof; and/or the WEE1 inhibitor is preferably ZN-C3 (CAS 2376146-48-2), Adavosertib (AZD1775, CAS 955365-80-7), Debio 0123 (CAS 2243882-74-6), or analogs thereof;
preferably, an analog of the AZD5305 comprises the following structure: 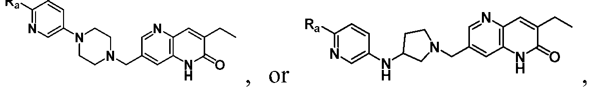 wherein Rₐ is selected from - C(O)NH-R_{b} or -NHC(O)-R_{b}, R_{b} is selected from: C₂₋₇ alkyl, C₃₋₇ monocyclic cycloalkyl, C₄₋₁₀ bicyclic cycloalkyl, C₅₋₉ spirocycloalkyl, and C₅₋₉ bridged cycloalkyl, each substituted with at least one primary or secondary amino group, or R_{b} is a 4-6 membered monocyclic heterocycloalkyl, C₄₋₁₀ bicyclic heterocycloalkyl, C₅₋₉ spiroheterocycloalkyl, C₅₋₉ bridged heterocycloalkyl containing 1-5 nitrogen, oxygen, sulfur atoms, wherein the R_{b} group comprises at least one primary or secondary amino group;
preferably, the AZD5305 analog is selected from the following structures: 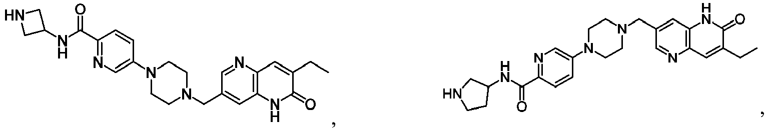 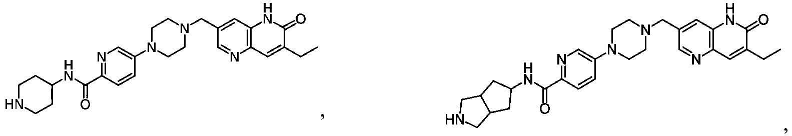 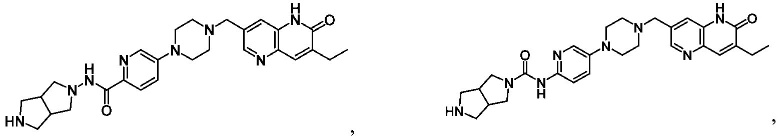 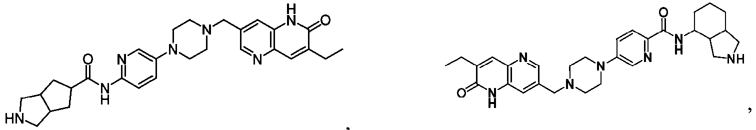 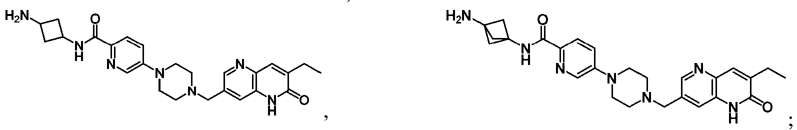
preferably, the PKMYT1 inhibitor is preferably RP-6306 and GSK-1520489A or analogs thereof;
preferably, analogs of the WEE1 inhibitors Adavosertib (AZD1775, CAS 955365-80-7), Debio 0123 (CAS 2243882-74-6) are selected from the following structures: 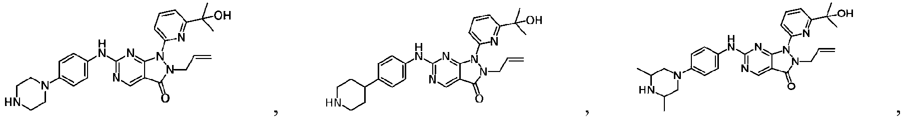 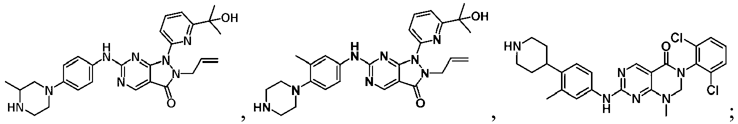
preferably, the drug unit targeting apoptosis-related pathways is a Bcl-2 family protein inhibitor; the Bcl-2 family protein inhibitor comprises BCL-2 inhibitors, BCL-XL inhibitors, and MCL-1 inhibitors, preferably Venetoclax (CAS 1257044-40-8), Navitoclax (CAS 923564-51-6), Navitoclax analog (CAS 2143096-93-7), ABT-737 (CAS 852808-04-9), A-1331852 (CAS 1430844-80-6), S64315 (CAS 1799631-75-6), or analogs thereof;
preferably, the A-1331852, S64315 analogs are selected from the following structures: 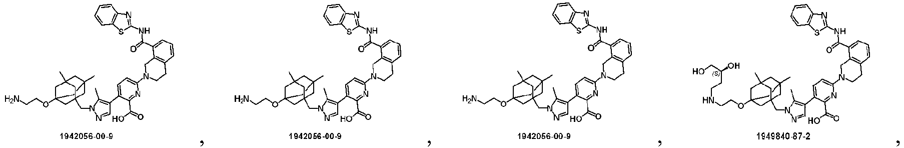 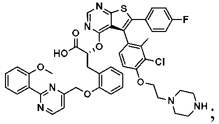
preferably, the drug unit targeting epigenetics is an LSD1 inhibitor, EZH2 inhibitor, BRD4 inhibitor, PRMT5 inhibitor, or PRMT1 inhibitor;
the LSD1 inhibitor is preferably Tranylcypromine, ORY-1001 (Iadademstat, CAS 1431303-72-8), CC-90011 (Pulrodemstat, CAS 1821307-10-1), ORY-2001, GSK-2879552, IMG-7289, INCB059872, or TAK-418 or analogs thereof; the EZH2 inhibitor is preferably Tazemetostat (CAS 1403254-99-8), GSK2816126, CPI-1205, PF-06821497, SHR2554, XNW5004, HH2853, or analogs thereof; the BRD4 inhibitor is a BI-2536 analog, Birabresib; the PRMT5 inhibitor is preferably GSK3326595, AMG 193, MRTX1719, SKL27969, TNG908, SCR-6920, SH3765, SYHX2001, or analogs thereof; the PRMT1 inhibitor is preferably GSK3368715 (CAS 1629013-22-4), MS023 (CAS 1831110-54-3), or analogs thereof;
preferably, the drug unit targeting immune activation pathway-related targets is a PD-L1 inhibitor, CBL-B inhibitor, TLR7/8 agonist, PTPN2/1 inhibitor, or STING agonist;
preferably, the Ras-Raf-MAPK pathway inhibitors comprise: SOS1 inhibitors BAY-293 (CAS 2244904-70-7), BI-3406 (CAS 2230836-55-0), or analogs thereof; KRAS inhibitors BI-2493 (CAS 2937344-16-4), MRTX1133 (CAS 2621928-55-8), or analogs thereof; MEK inhibitors Cobimetinib (CAS 934660-93-2), Selumetinib (CAS 606143-52-6), Mirdametinib (CAS 391210-10-9), Binimetinib (CAS 606143-89-9), TAK-733 (CAS 1035555-63-5), GDC-0623 (CAS 1168091-68-6), AZD8330 (CAS 869357-68-6), Trametinib (CAS 871700-17-3), Trametiglue (CAS 2666940-97-0), or analogs thereof; ERK inhibitors ASN007 (CAS 2055597-12-9) or analogs thereof;
preferably, the cell cycle pathway inhibitors comprise: CDK4/6 inhibitors Palbociclib (CAS 571190-30-2), Abemaciclib (CAS 1231929-97-7), Ribociclib (CAS 1211441-98-3), Dalpiciclib (CAS 1637781-04-4), or analogs thereof; pan-CDK inhibitors Dinaciclib (CAS 779353-01-4) or analogs thereof;
preferably, the PI3K/AKT/mTOR pathway inhibitors comprise: PKI-587 (CAS 1197160-78-3), desmethyl PKI-587 (CAS 1950569-63-7), AZD8055 (CAS 1009298-09-2), Afuresertib (CAS 1047644-62-1), or analogs thereof;
preferably, the estrogen receptor antagonists/degraders comprise Fulvestrant (CAS 129453-61-8), Elacestrant (CAS 1349723-93-8); the androgen receptor antagonists comprise abiraterone (CAS 154229-19-3), JNJ-63576253 (CAS 2110428-64-1), or analogs thereof;
preferably, the autophagy inhibitor is Hydroxychloroquine (HCQ, CAS 118-42-3) and Chloroquine (CQ, CAS 54-05-7) or analogs thereof;
preferably, the drug targeting transcription factors is a GSPT1 degrader (molecular glue), and the GSPT1 degrader is selected from the following structures: 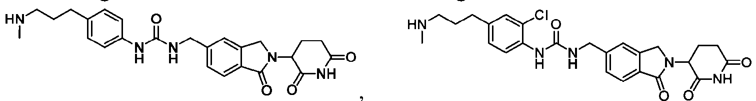 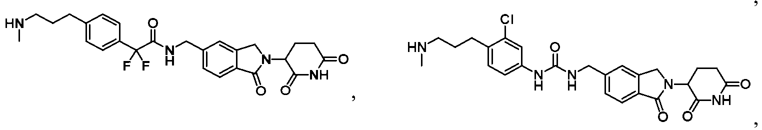 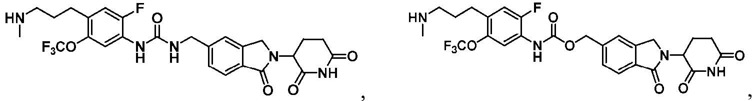 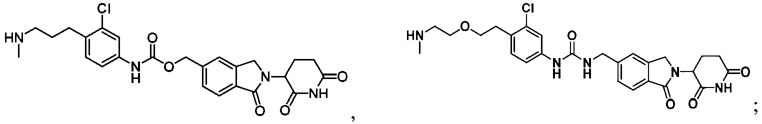
preferably, the FAK inhibitor is Ifebemtinib (CAS 1227948-82-4), Defactinib (CAS 1073154-85-4), PF-562271 (CAS 717907-75-0), or analogs thereof, selected from the following structures: 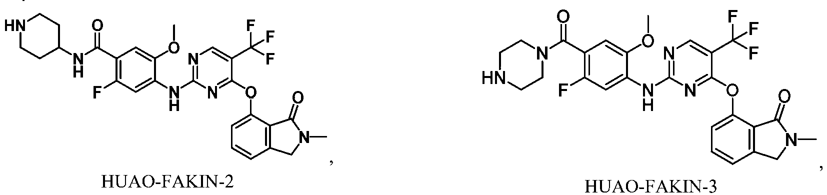 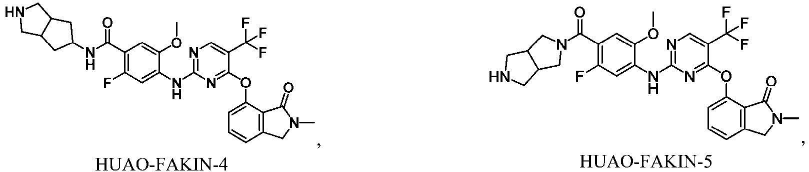 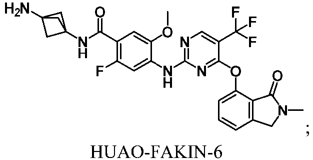
preferably, the PLK1 inhibitor is BI2536 (CAS 755038-02-9), Volasertib (CAS 755038-65-4), GSK461364 (CAS 929095-18-1), Onvansertib (CAS 1034616-18-6), or analogs thereof;
preferably, the N-myristoyltransferase (NMT) inhibitor is MYX1715 (CAS 2445448-66-6) or analogs thereof;
more oreferably, D₁, D₂, and D₃ are selected from the combinations in the following table:
| Drug Combination | D1 | D2 | D3 |
|---|---|---|---|
| Combination 1 | TOPO1 inhibitor | PARP inhibitor | / |
| Combination 2 | TOPO1 inhibitor | ATR inhibitor | / |
| Combination 3 | TOPO1 inhibitor | CHK1 inhibitor | / |
| Combination 4 | TOPO1 inhibitor | PARP inhibitor | ATR inhibitor |
| Combination 5 | TOPO1 inhibitor | PARP inhibitor | CHK1 inhibitor |
| Combination 6 | TOPO1 inhibitor | PARP inhibitor | WEE1 inhibitor |
| Combination 7 | TOPO1 inhibitor | ATR inhibitor | WEE1 inhibitor |
| Combination 8 | TOPO1 inhibitor | CHK1 inhibitor | WEE1 inhibitor |
| Combination 9 | TOPO1 inhibitor | DNA-PK inhibitor | / |
| Combination 10 | TOPO1 inhibitor | POLQ inhibitor | / |
| Combination 11 | TOPO1 inhibitor | TOPO2 inhibitor | / |
| Combination 12 | PARP inhibitor | ATR/CHK1/WEE1 inhibitor | / |
| Combination 13 | PARP inhibitor | ATR inhibitor | CHK1 inhibitor |
| Combination 14 | TOPO1 inhibitor | Immune activator (PD-L1 inhibitor, CBL-B inhibitor, TLR7/8 agonist, PTPN2/1 inhibitor, or STING agonist) | / |
| Combination 15 | TOPO1 inhibitor | BCL-2 family inhibitor | / |
| Combination 16 | TOPO1 inhibitor | BCL-2 family inhibitor | MCL-1 inhibitor |
| Combination 17 | TOPO1 inhibitor | DNA synthesis inhibitor | / |
| Combination 18 | TOPO1 inhibitor | PI3K/mTOR inhibitor | / |
| Combination 19 | TOPO1 inhibitor | EGFR inhibitor | / |
| Combination 20 | TOPO1 inhibitor | Autophagy inhibitor | / |
| Combination 21 | TOPO1 inhibitor | FAK inhibitor | |
| Combination 22 | TOPO1 inhibitor | PARP inhibitor | FAK inhibitor |
| Combination 23 | TOPO1 inhibitor | PARP inhibitor | Androgen receptor antagonist/degrader |
| Combination 24 | TOPO1 inhibitor | GSPT1 degrader | / |
| Combination 25 | TOPO1 inhibitor | PARP inhibitor | GSPT1 degrader |
| Combination 26 | TOPO1 inhibitor | Tubulin inhibitor | / |
| Combination 27 | TOPO1 inhibitor | Epigenetic regulator | / |
| Combination 28 | Tubulin inhibitor | Immune activator (PD-L1 inhibitor, CBL-B inhibitor, TLR7/8 agonist, PTPN2/1 inhibitor, or STING agonist) | / |
| Combination 29 | Tubulin inhibitor | BCL-2/BCL-XL inhibitor | / |
| Combination 30 | Tubulin inhibitor | BCL-2/BCL-XL inhibitor | MCL-1 inhibitor |
| Combination 31 | Tubulin inhibitor | PI3K/AKT/mTOR inhibitor | / |
| Combination 32 | Tubulin inhibitor | FAK inhibitor | |
| Combination 33 | CDK4/6 inhibitor | MEK inhibitor | / |
| Combination 34 | CDK4/6 inhibitor | MEK inhibitor | EZH2 inhibitor |
| Combination 35 | CDK4/6 inhibitor | Autophagy inhibitor | / |
| Combination 36 | MEK inhibitor | Autophagy inhibitor | / |
| Combination 37 | ERK inhibitor | Autophagy inhibitor | / |
| Combination 38 | CDK4/6 inhibitor | MEK inhibitor | Autophagy inhibitor |
| Combination 39 | CDK4/6 inhibitor | ERK inhibitor | Autophagy inhibitor |
| Combination 40 | CDK4/6 inhibitor | ERK inhibitor | (MEK inhibitor) |
| Combination 41 | CDK4/6 inhibitor | MEK inhibitor | KRAS inhibitor |
| Combination 42 | CDK4/6 inhibitor | MEK inhibitor | SOS1 inhibitor |
| Combination 43 | CDK4/6 inhibitor | MEK inhibitor | FAK inhibitor |
| Combination 44 | CDK4/6 inhibitor | PRMT5 inhibitor | / |
| Combination 45 | CDK4/6 inhibitor | Estrogen receptor antagonist/degrader | / |
| Combination 46 | CDK4/6 inhibitor | Estrogen receptor antagonist/degrader | PI3K/mTOR inhibitor |
| Combination 47 | CDK4/6 inhibitor | MEK inhibitor | GSPT1 degrader |
| Combination 48 | CDK4/6 inhibitor | ERK inhibitor | GSPT1 degrader |
| Combination 49 | CDK4/6 inhibitor | Autophagy inhibitor | GSPT1 degrader |
| Combination 50 | BRD4 inhibitor | EZH2 inhibitor | / |
| Combination 51 | PRMT5 inhibitor | MAT2A inhibitor | / |
| Combination 52 | PRMT5 inhibitor | Gemcitabine | / |
| Combination 53 | PRMT5 inhibitor | CDK4/6 inhibitor | / |
| Combination 54 | PRMT5 inhibitor | PRMT1 inhibitor | / |
| Combination 55 | PRMT5 inhibitor | PRMT1 inhibitor | CDK4/6 inhibitor |
| Combination 56 | PRMT5 inhibitor | PRMT1 inhibitor | MEK inhibitor |
| Combination 57 | RAF inhibitor | MEK inhibitor | / |
| Combination 58 | EGFR inhibitor | MEK inhibitor | / |
| Combination 59 | RAF inhibitor | MEK inhibitor | SHP2 inhibitor |
| Combination 60 | CDK4/6 inhibitor | HIF-2alpha inhibitor | / |
| Combination 61 | TOPO1 inhibitor | WEE1 inhibitor | / |
| Combination 62 | WEE1 inhibitor | CHK1 inhibitor | / |
| Combination 63 | TOPO1 inhibitor | TOPO1 inhibitor | / |
| Combination 64 | CDK inhibitor | MEK inhibitor | |
| Combination 65 | FAK inhibitor | Smo inhibitor | / |
| Combination 66 | PLK1 inhibitor | CDK inhibitor | / |
| Combination 67 | PLK1 inhibitor | CDK inhibitor | PARP inhibitor/ |
| Combination 68 | TOPO1 inhibitor | PLK1 inhibitor | / |
| Combination 69 | TOPO1 inhibitor | PLK1 inhibitor | PARP inhibitor/ |
| Combination 70 | TOPO1 inhibitor | PLK1 inhibitor | ATR inhibitor/ |
| Combination 71 | TOPO1 inhibitor | NMT inhibitor | / |
| Combination 72 | Tubulin inhibitor | NMT inhibitor | / |
more preferably,
(i) at least one of D₁, D₂, and D₃ is a cytotoxic drug, the cytotoxic drug is selected from drugs targeting topoisomerase, for example a TOPO1 inhibitor, at least another one is a tumor-targeted therapeutic drug, the tumor-targeted therapeutic drug is a drug targeting DNA damage response (DDR) or "synthetic lethality" related pathways, selected from PARP inhibitors, ATR inhibitors, CHK1 inhibitors, ATM inhibitors, DNA-PK inhibitors, WEE1 inhibitors, POLQ inhibitors, CDK12 inhibitors, USP1 inhibitors, PKMYT1 inhibitors, Rad51 inhibitors;
(ii) at least two of D₁, D₂, and D₃ are cytotoxic drugs and are independently selected from drug units targeting topoisomerase and drug units targeting tubulin, for example TOPO1 inhibitors, TOPO2 inhibitors, and tubulin inhibitors;
(iii) at least two or all three of D₁, D₂, and D₃ are tumor-targeted therapeutic drugs, and are independently selected from drug units targeting DNA damage response (DDR) or "synthetic lethality" related pathways, drug units targeting epigenetics, drug units targeting apoptosis-related pathways, drug units targeting cell cycle pathways, drug units targeting transcription factors, and drug units targeting immune activation pathway-related targets, comprising: inhibitors of EFGR pathway-related targets; Ras-Raf-MAPK pathway inhibitors; PI3K/AKT/mTOR pathway inhibitors; cell cycle pathway inhibitors; cGAS-STING signaling pathway agonists; estrogen receptor antagonists; androgen receptor antagonists; glucocorticoid receptor modulators; autophagy inhibitors; FAK inhibitors; Smo inhibitors; BTK inhibitors; PDE4 inhibitors; Lck inhibitors; PLK1 inhibitors; TLR7/8 modulators; N-myristoyltransferase (NMT) inhibitors;
(iv) one of D₁, D₂, and D₃ is a cytotoxic drug, the cytotoxic drug is selected from drug units targeting topoisomerase and drug units targeting tubulin, for example a TOPO1 inhibitor or a tubulin inhibitor, and the other one or two are tumor-targeted therapeutic drugs, the tumor-targeted therapeutic drugs are selected from drug units targeting DNA damage response (DDR) or "synthetic lethality" related pathways, drug units targeting epigenetics, drug units targeting apoptosis-related pathways, drug units targeting cell cycle pathways, drug units targeting transcription factors, and drug units targeting immune activation pathway-related targets, comprising: inhibitors of EFGR pathway-related targets; Ras-Raf-MAPK pathway inhibitors; PI3K/AKT/mTOR pathway inhibitors; cell cycle pathway inhibitors; cGAS-STING signaling pathway agonists; estrogen receptor antagonists; androgen receptor antagonists; glucocorticoid receptor modulators; autophagy inhibitors; FAK inhibitors; Smo inhibitors; BTK inhibitors; PDE4 inhibitors; Lck inhibitors; PLK1 inhibitors; TLR7/8 modulators; N-myristoyltransferase (NMT) inhibitors;
more preferably, D₁, D₂, and D₃ are selected from the combinations shown in Table A.

13. The compound, or a tautomer, a mesomer, a racemate, an enantiomer, or a diastereomer thereof, or a deuterated compound form thereof, or a mixture form thereof, or a pharmaceutically acceptable salt, prodrug, or solvate thereof, according to any one of claims 1 to 12, wherein the compound is selected from formulas **A1 to A190:**
| Structure | Number | Drug Combination |
|---|---|---|
| 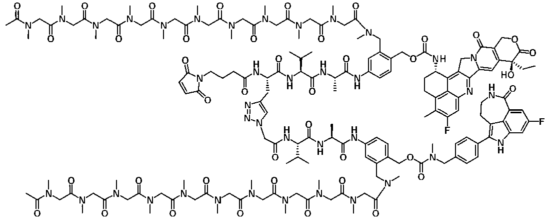 | Formula A1 | Combination 1 |
| 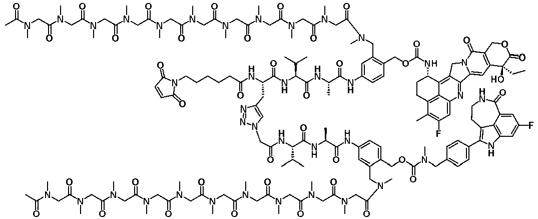 | Formula A2 | Combination 1 |
| 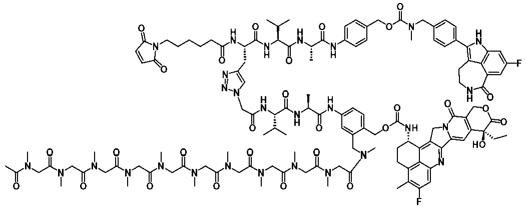 | Formula A3 | Combination 1 |
| 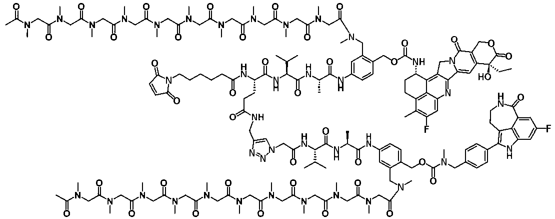 | Formula A4 | Combination 1 |
| 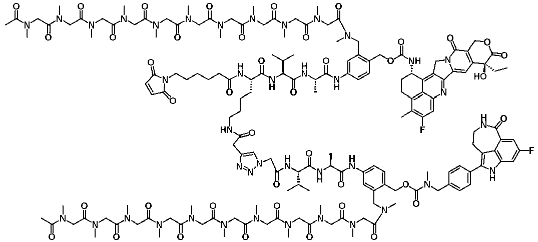 | Formula A5 | Combination 1 |
| 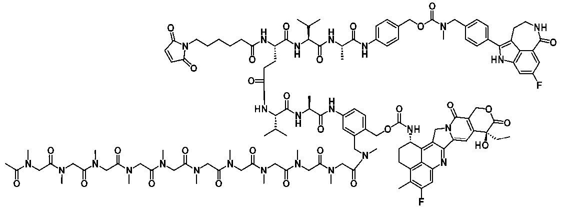 | Formula A6 | Combination 1 |
| 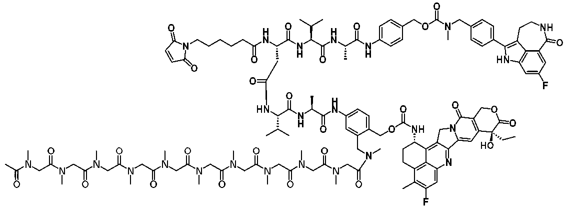 | Formula A7 | Combination 1 |
| 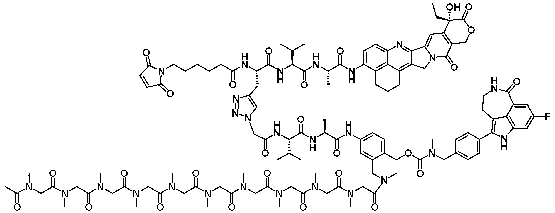 | Formula A8 | Combination 1 |
| 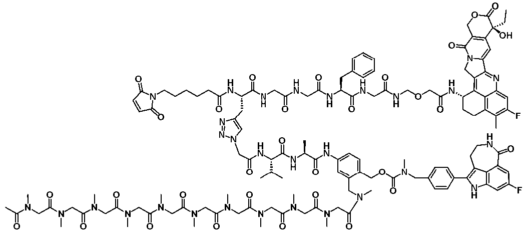 | Formula A9 | Combination 1 |
| 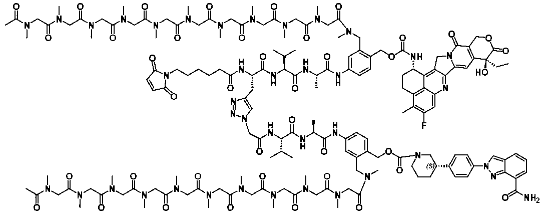 | Formula A10 | Combination 1 |
| 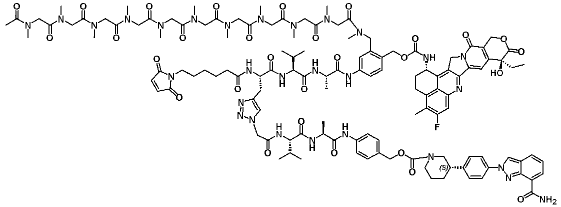 | Formula A11 | Combination 1 |
| 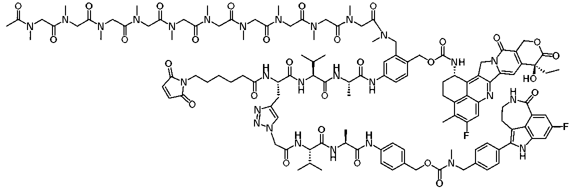 | Formula A12 | Combination 1 |
| 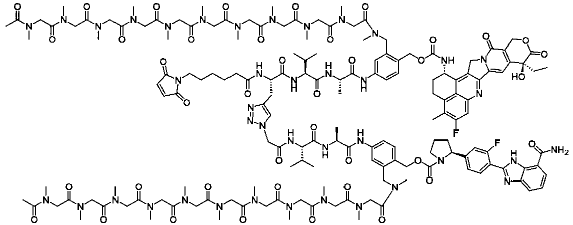 | Formula A13 | Combination 1 |
| 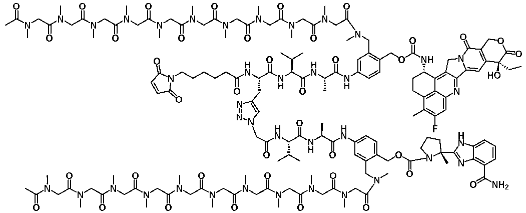 | Formula A14 | Combination 1 |
| 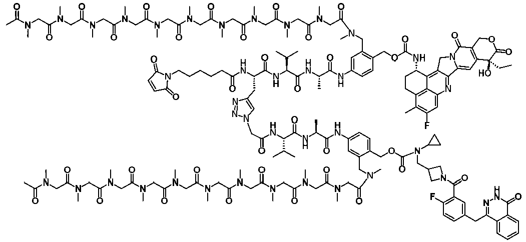 | Formula A15 | Combination 1 |
| 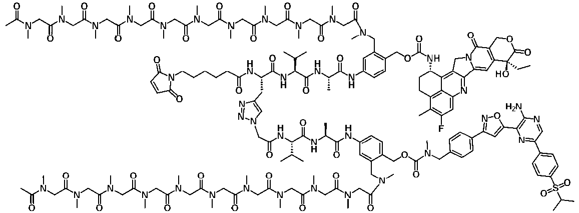 | Formula A16 | Combination 2 |
| 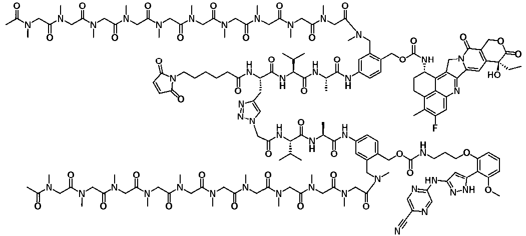 | Formula A17 | Combination 3 |
| 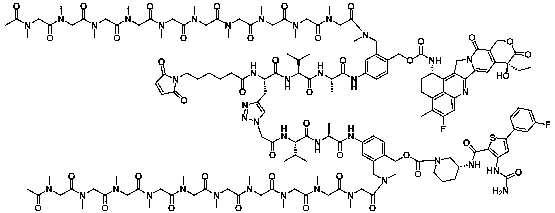 | Formula A18 | Combination 3 |
| 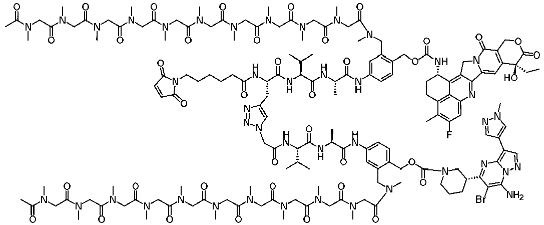 | Formula A19 | Combination 3 |
| 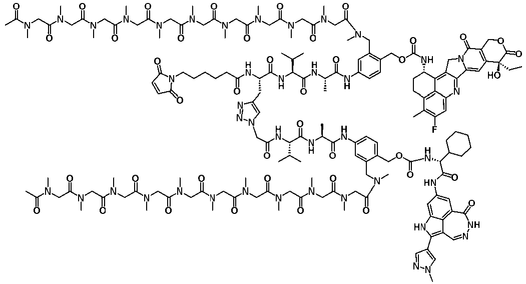 | Formula A20 | Combination 3 |
| 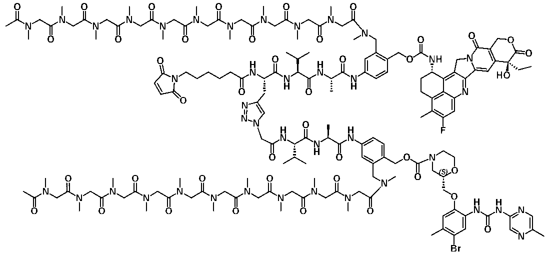 | Formula A21 | Combination 3 |
| 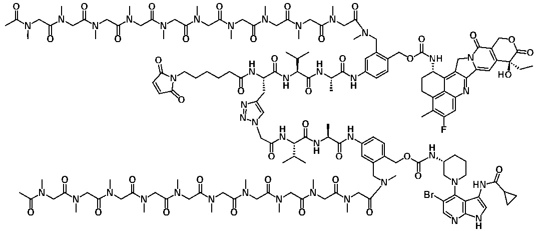 | Formula A22 | Combination 3 |
| 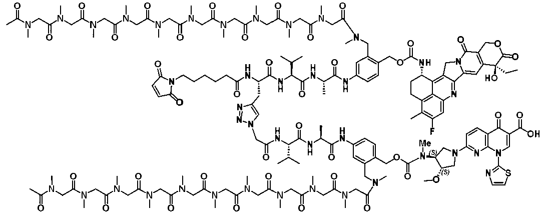 | Formula A23 | Combination 11 |
| 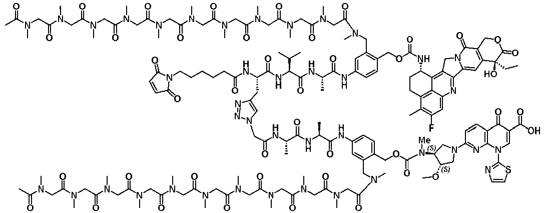 | Formula A24 | Combination 11 |
| 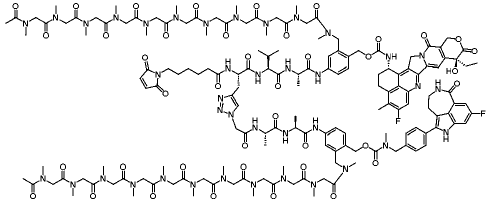 | Formula A25 | Combination 1 |
| 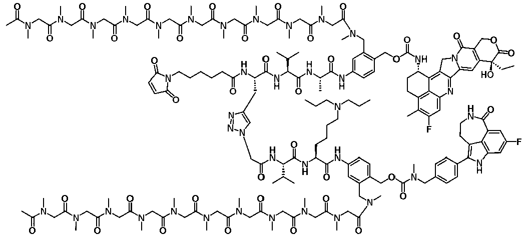 | Formula A26 | Combination 1 |
| 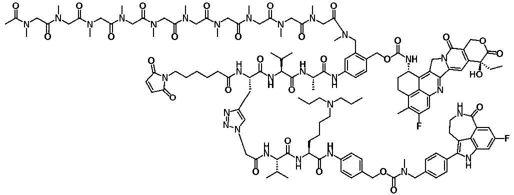 | Formula A27 | Combination 1 |
| 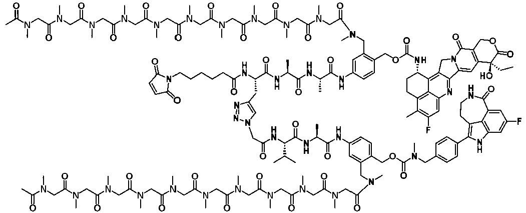 | Formula A28 | Combination 1 |
| 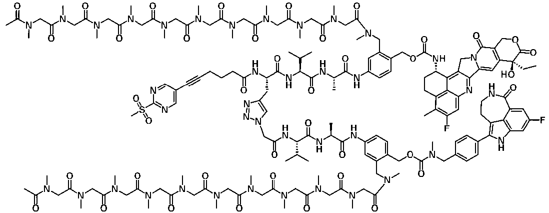 | Formula A29 | Combination 1 |
| 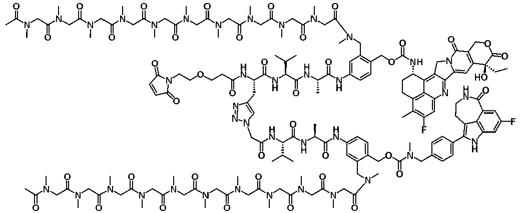 | Formula A30 | Combination 1 |
| 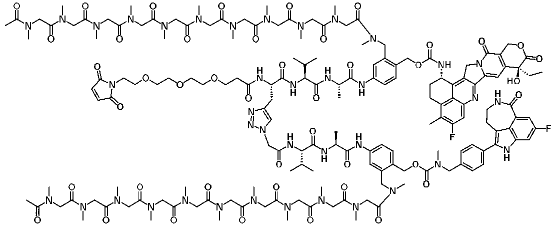 | Formula A31 | Combination 1 |
| 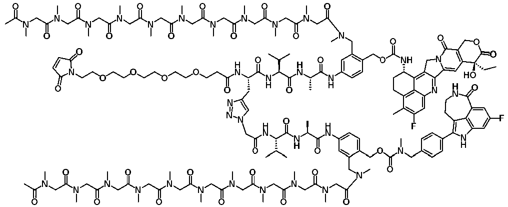 | Formula A32 | Combination 1 |
| 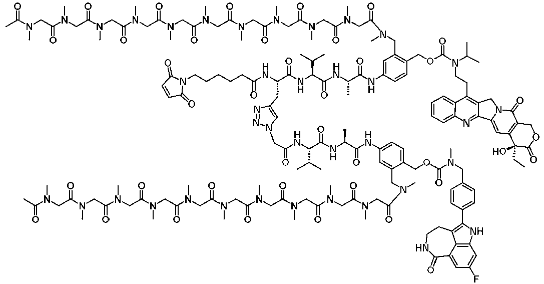 | Formula A33 | Combination 1 |
| 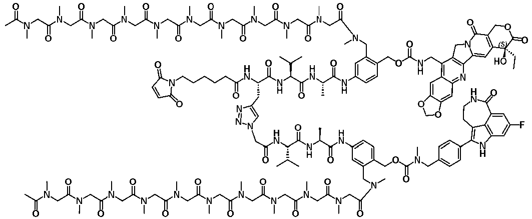 | Formula A34 | Combination 1 |
| 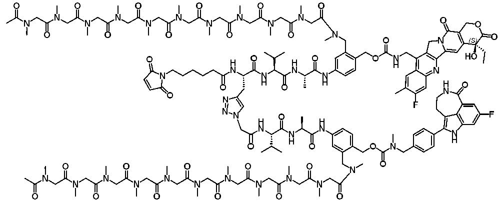 | Formula A35 | Combination 1 |
| 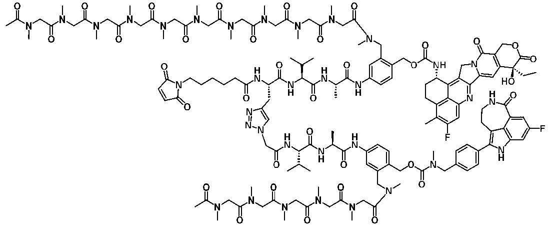 | Formula A36 | Combination 1 |
| 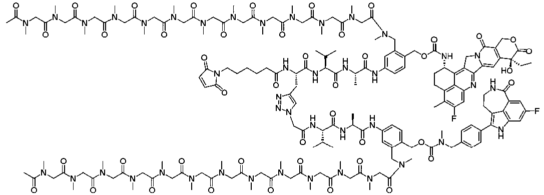 | Formula A37 | Combination 1 |
| 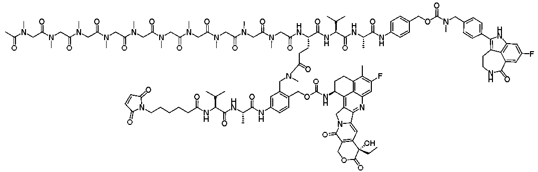 | Formula A38 | Combination 1 |
| 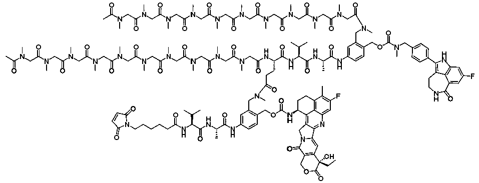 | Formula A39 | Combination 1 |
| 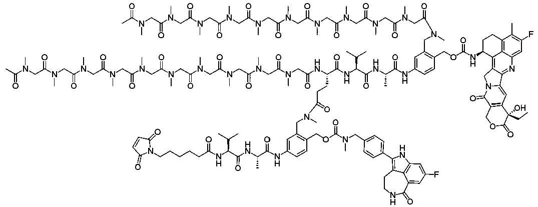 | Formula A40 | Combination 1 |
| 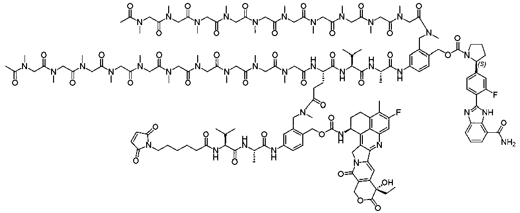 | Formula A41 | Combination 1 |
| 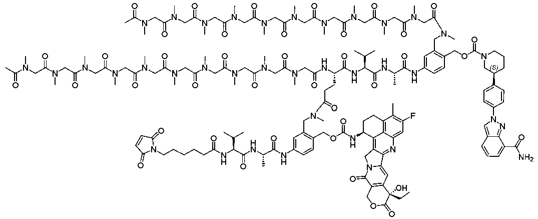 | Formula A42 | Combination 1 |
| 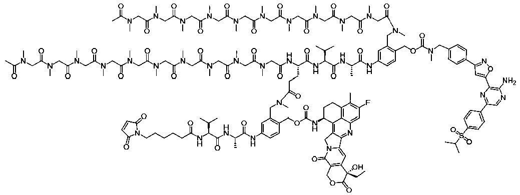 | Formula A43 | Combination 2 |
| 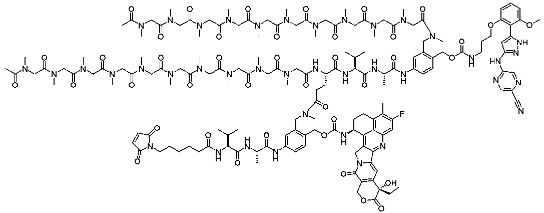 | Formula A44 | Combination 3 |
| 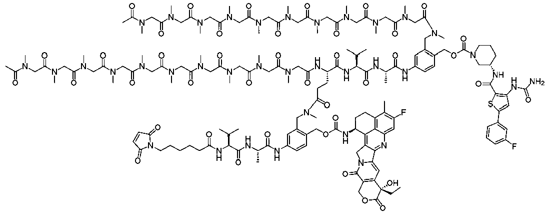 | Formula A45 | Combination 3-3 |
| 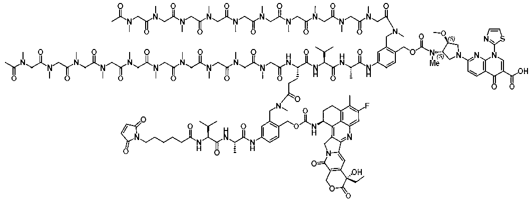 | Formula A46 | Combination 11 |
| 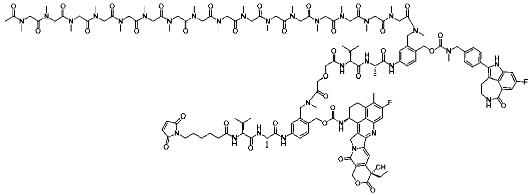 | Formula A47 | Combination 1 |
| 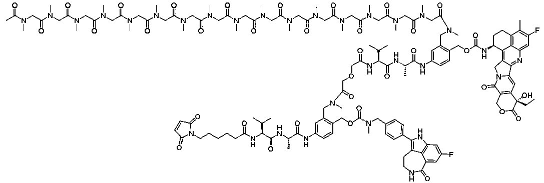 | Formula A48 | Combination 1 |
| 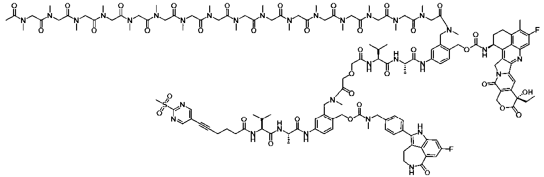 | Formula A49 | Combination 1-1 |
| 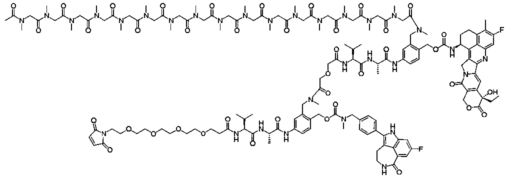 | Formula A50 | Combination 1 |
| 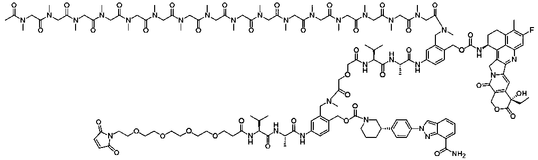 | Formula A51 | Combination 1 |
| 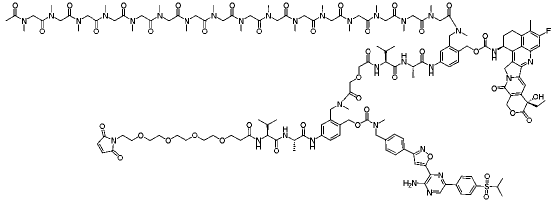 | Formula A52 | Combination 2 |
| 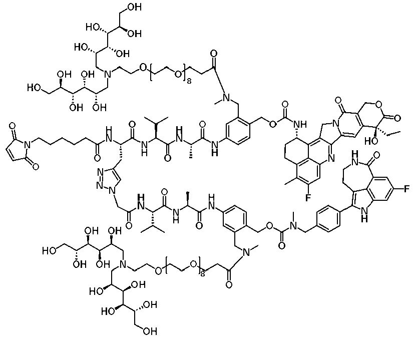 | Formula A53 | Combination 1 |
| 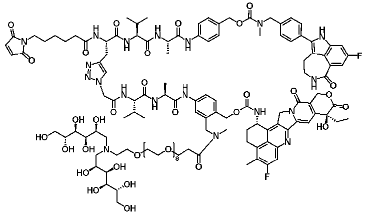 | Formula A54 | Combination 1 |
| 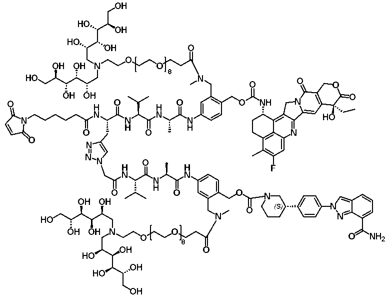 | Formula A55 | Combination 1 |
| 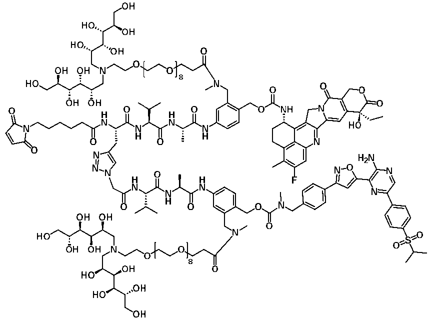 | Formula A56 | Combination 2 |
| 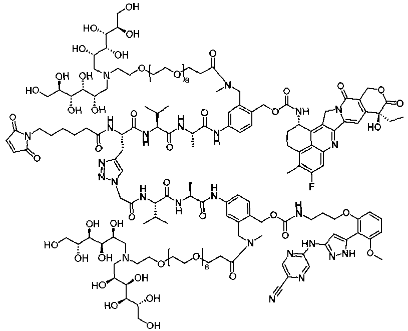 | Formula A57 | Combination 3 |
| 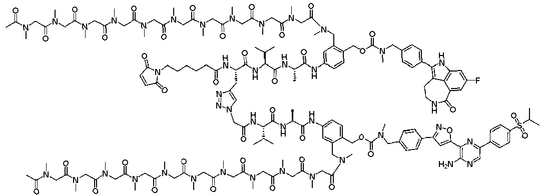 | Formula A58 | Combination 12 |
| 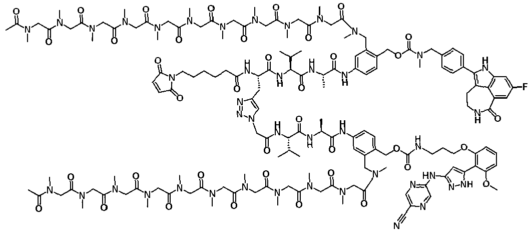 | Formula A59 | Combination 12 |
| 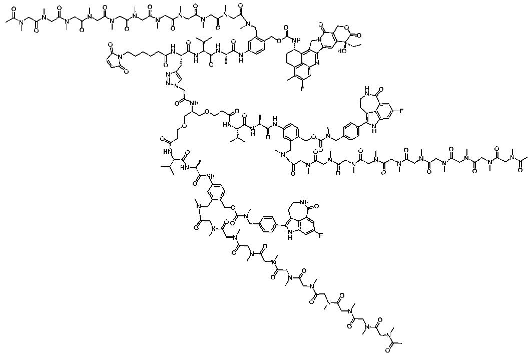 | Formula A60 | Combination 1 |
| 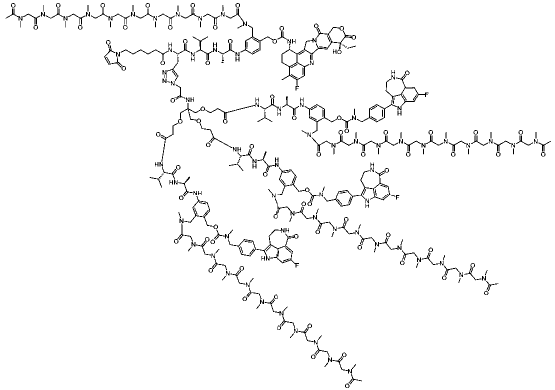 | Formula A61 | Combination 1 |
| 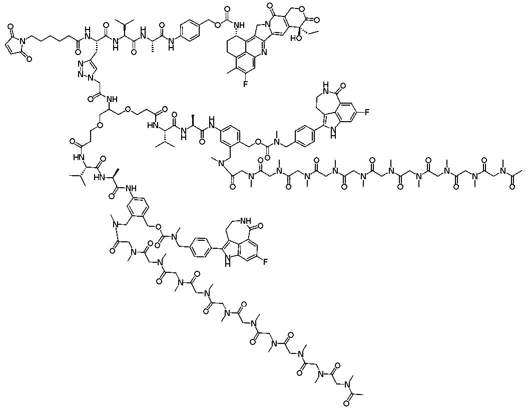 | Formula A62 | Combination 1 |
| 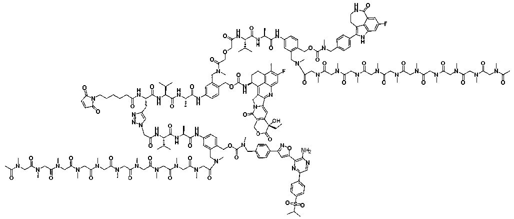 | Formula A63 | Combination 4 |
| 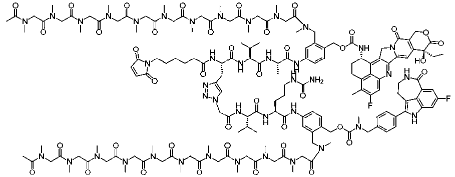 | Formula A64 | Combination 1 |
| 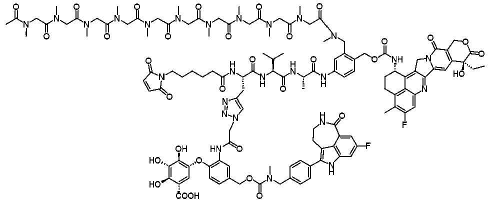 | Formula A65 | Combination 1 |
| 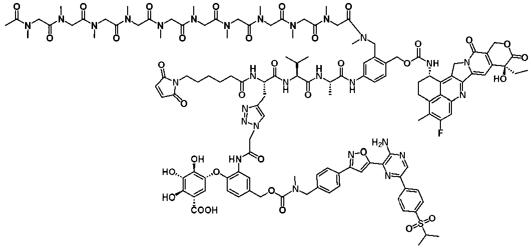 | Formula A66 | Combination 2 |
| 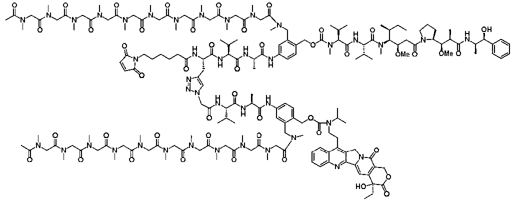 | Formula A67 | Combination 26 |
| 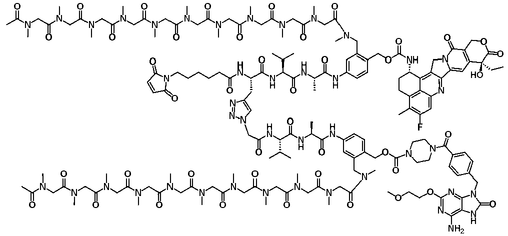 | Formula A68 | Combination 14 |
| 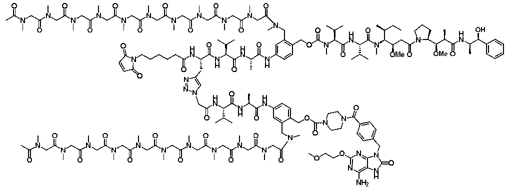 | Formula A69 | Combination 28 |
| 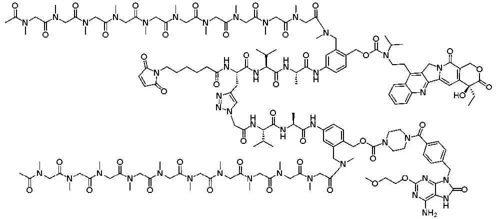 | Formula A70 | Combination 14 |
| 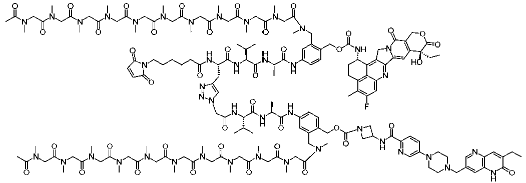 | Formula A71 | Combination 1 |
| 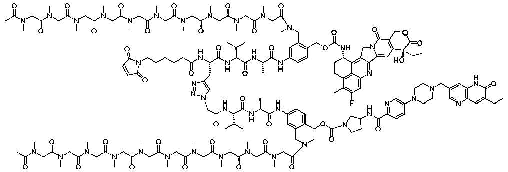 | Formula A72 | Combination 1 |
| 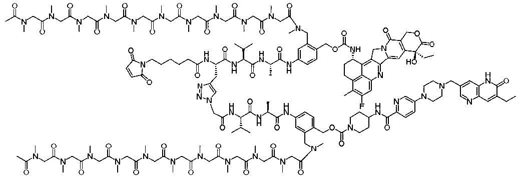 | Formula A73 | Combination 1 |
| 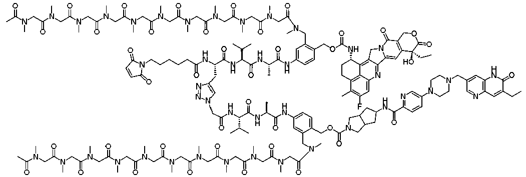 | Formula A74 | Combination 1 |
| 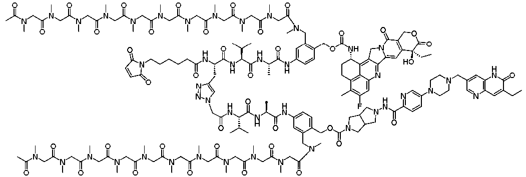 | Formula A75 | Combination 1 |
| 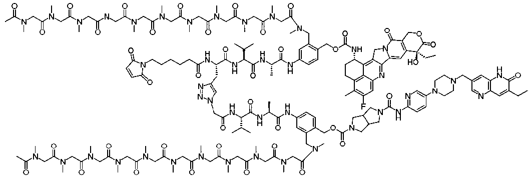 | Formula A76 | Combination 1 |
| 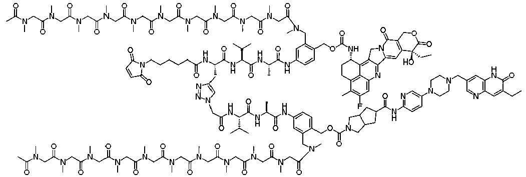 | Formula A77 | Combination 1 |
| 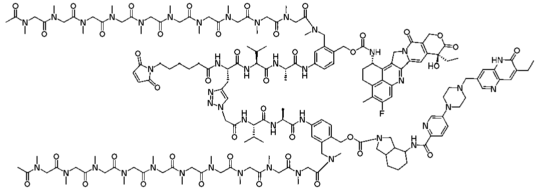 | Formula A78 | Combination 1 |
| 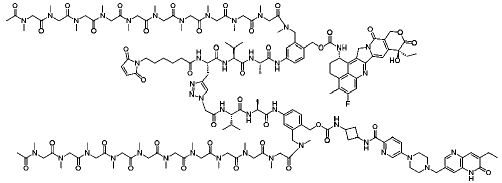 | Formula A79 | Combination 1 |
| 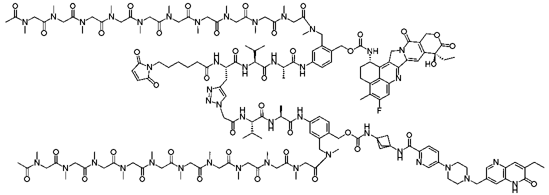 | Formula A80 | Combination 1 |
| 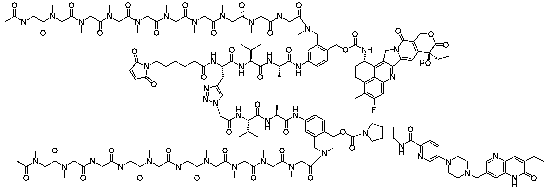 | Formula A81 | Combination 1 |
| 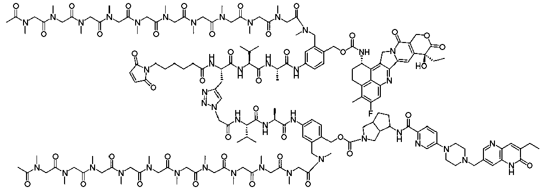 | Formula A82 | Combination 1 |
| 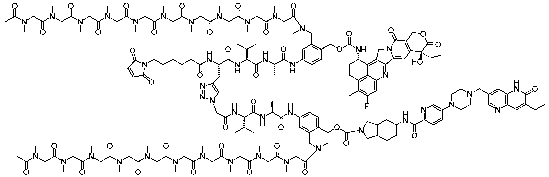 | Formula A83 | Combination 1 |
| 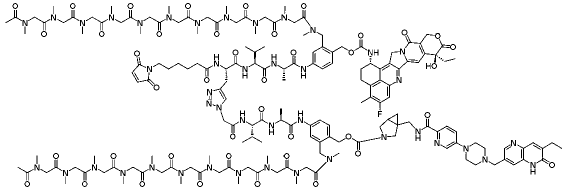 | Formula A84 | Combination 1 |
| 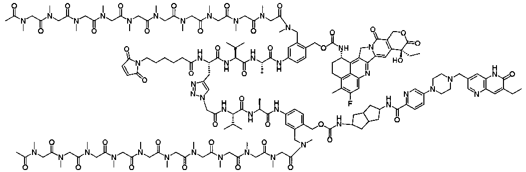 | Formula A85 | Combination 1 |
| 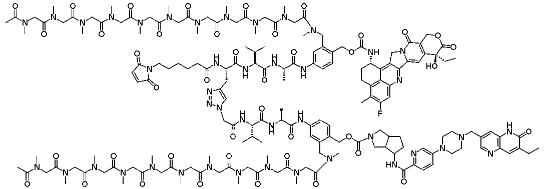 | Formula A86 | Combination 1 |
| 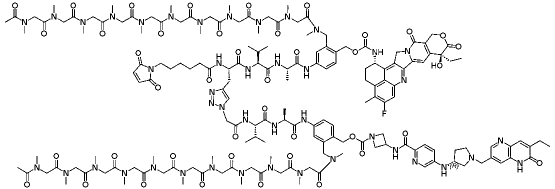 | Formula A87 | Combination 1 |
| 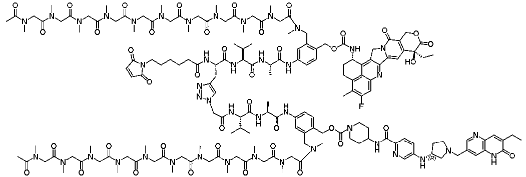 | Formula A88 | Combination 1 |
| 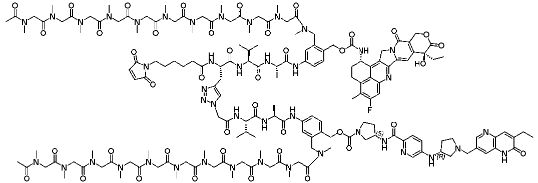 | Formula A89 | Combination 1 |
| 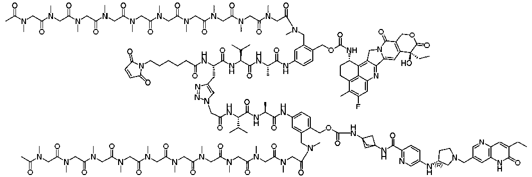 | Formula A90 | Combination 1 |
| 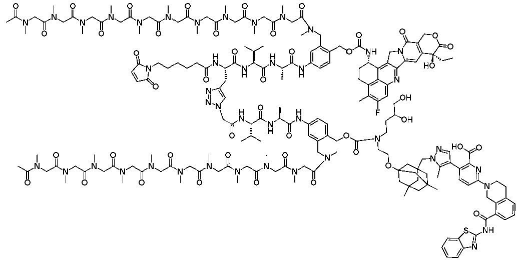 | Formula A91 | Combination 15 |
| 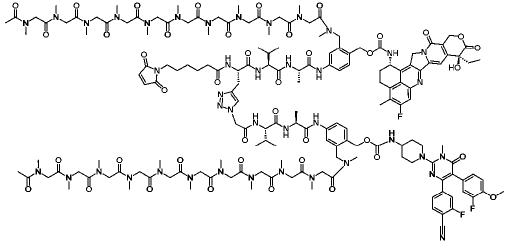 | Formula A92 | Combination 27 |
| 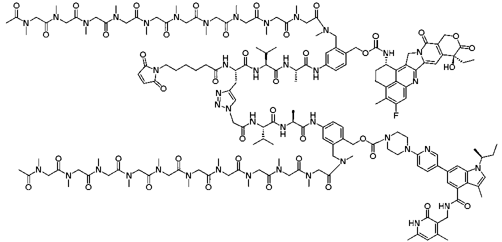 | Formula A93 | Combination 27 |
| 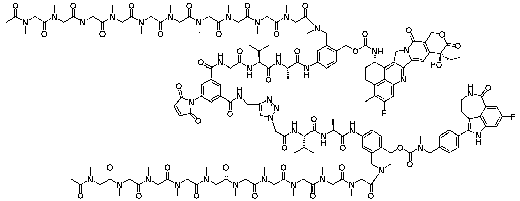 | Formula A94 | Combination 1 |
| 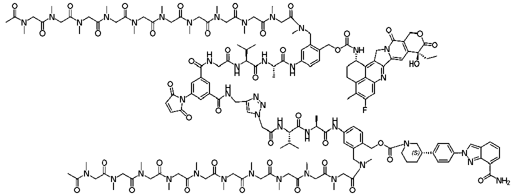 | Formula A95 | Combination 1 |
| 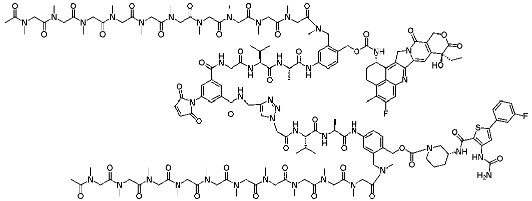 | Formula A96 | Combination 3 |
| 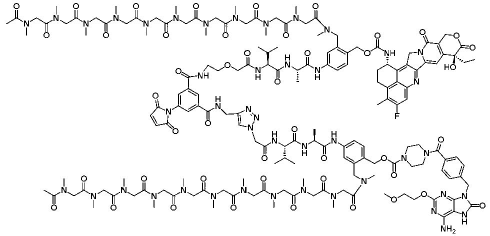 | Formula A97 | Combination 14 |
| 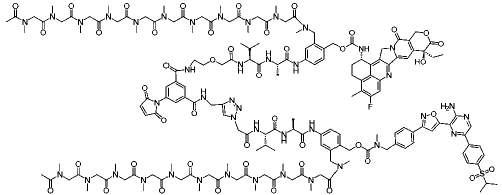 | Formula A98 | Combination 2 |
| 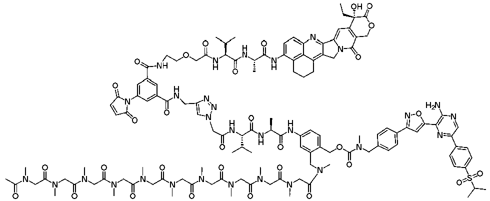 | Formula A99 | Combination 2 |
| 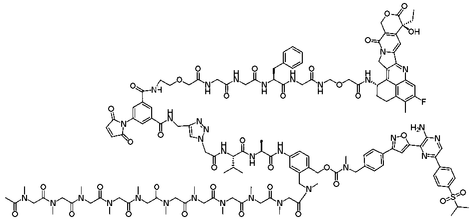 | Formula A100 | Combination 2 |
| 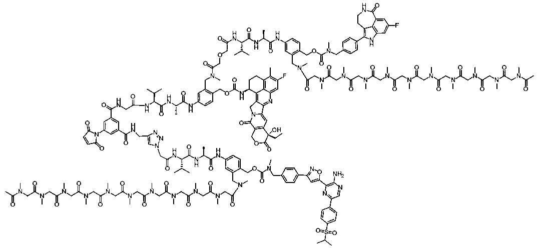 | Formula A101 | Combination 2 |
| 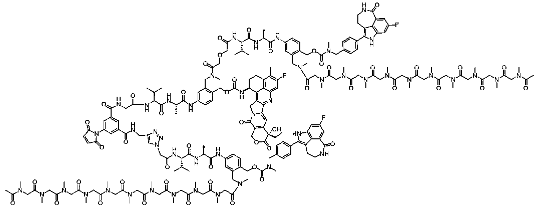 | Formula A102 | Combination 1 |
| 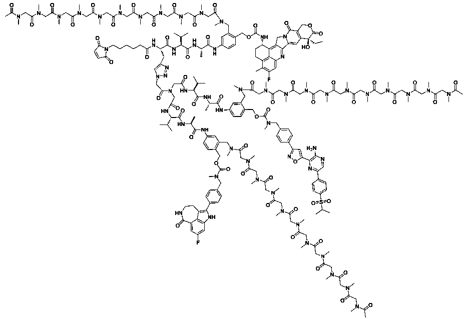 | Formula A103 | Combination 4 |
| 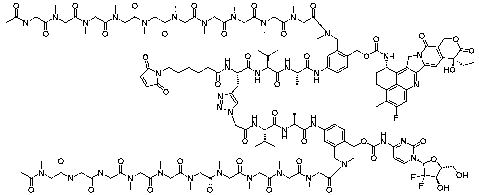 | Formula A104 | Combination 17 |
| 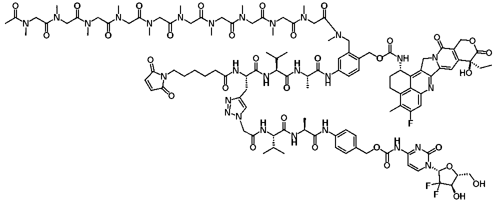 | Formula A105 | Combination 17 |
| 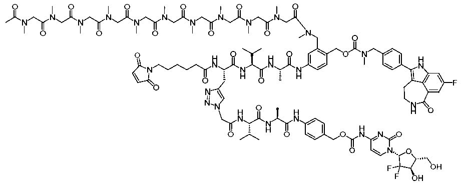 | Formula A106 | / |
| 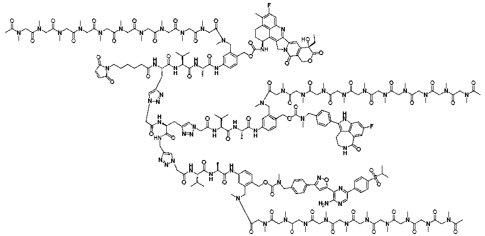 | Formula A107 | Combination 4 |
| 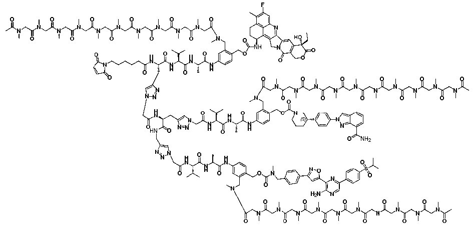 | Formula A108 | Combination 4 |
| 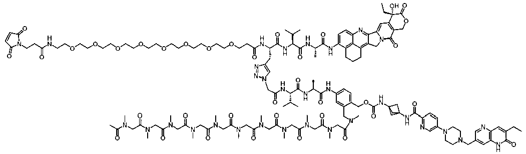 | Formula A109 | Combination 1 |
| 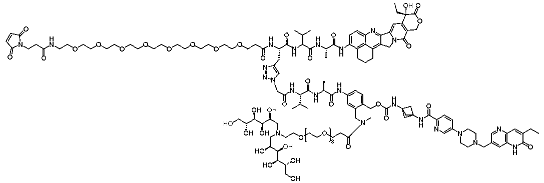 | Formula A110 | Combination 1 |
| 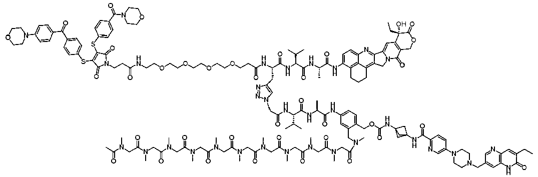 | Formula A111 | Combination 1 |
| 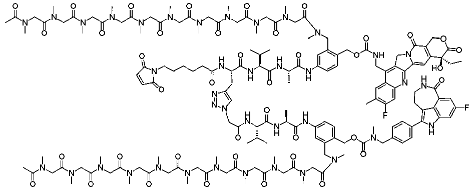 | Formula A112 | Combination 1 |
| 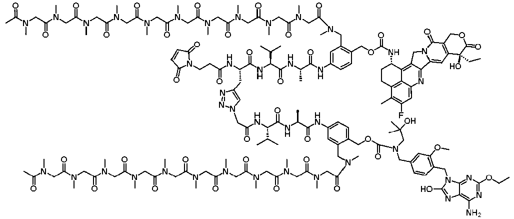 | Formula A113 | Combination 14 |
| 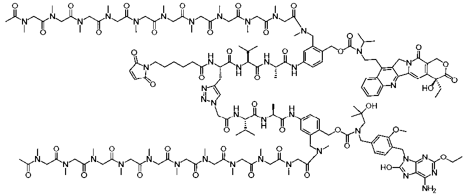 | Formula A114 | Combination 14 |
| 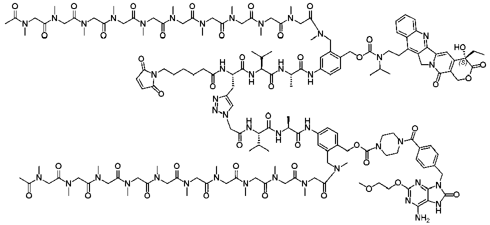 | Formula A115 | Combination 14 |
| 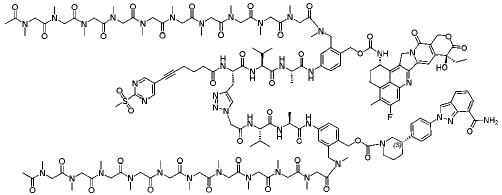 | Formula A116 | Combination 1 |
| 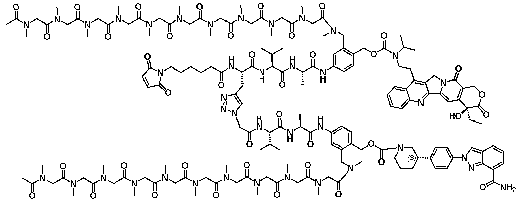 | Formula A117 | Combination 1 |
| 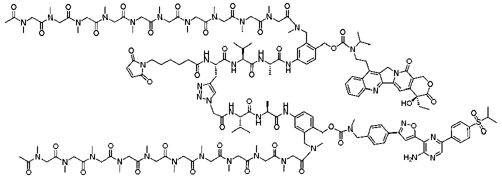 | Formula A118 | Combination 2 |
| 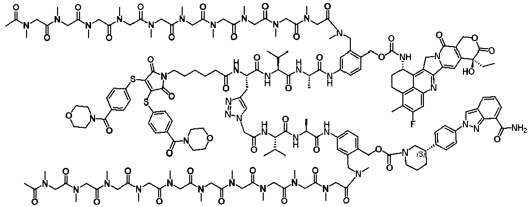 | Formula A119 | Combination 3 |
| 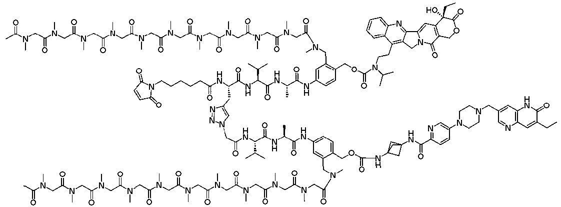 | Formula A120 | Combination 1 |
| 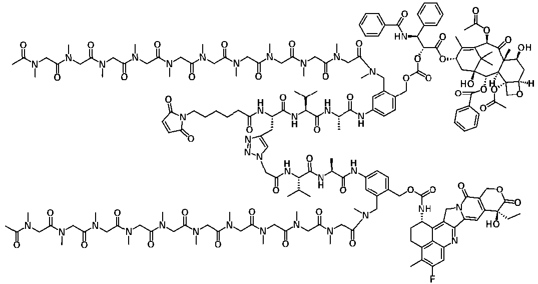 | Formula A121 | Combination 26 |
| 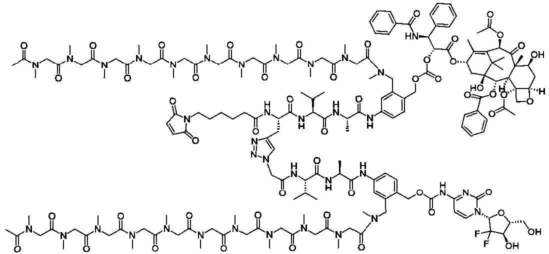 | Formula A122 | Tubulin inhibitor + DNA synthesis inhibitor |
| 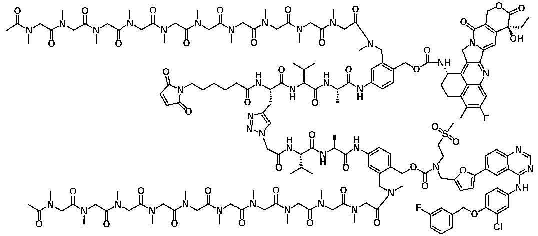 | Formula A123 | Combination 19 |
| 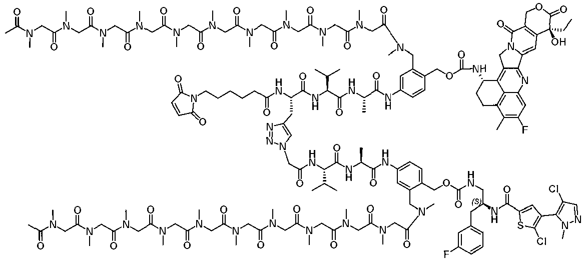 | Formula A124 | Combination 31 |
| 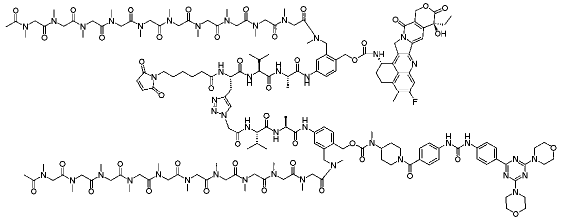 | Formula A125 | Combination 18 |
| 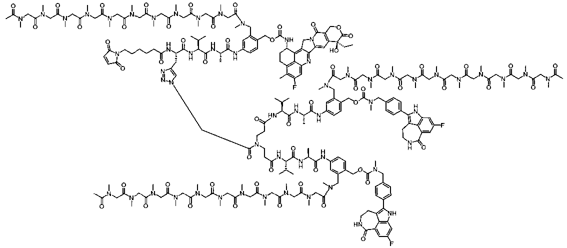 | Formula A126 | Combination 1 |
| 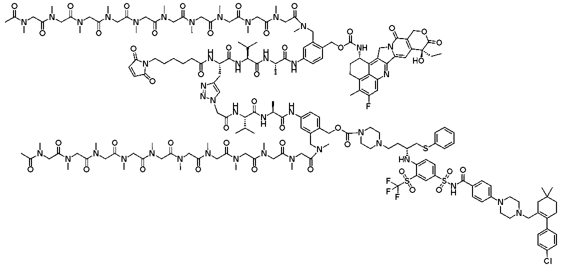 | Formula A127 | Combination 15 |
| 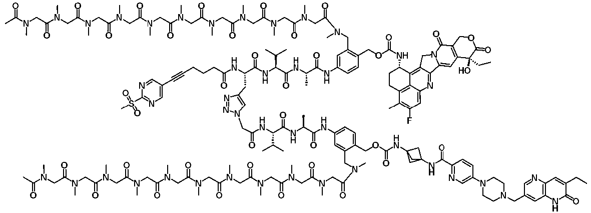 | Formula A128 | Combination 1 |
| 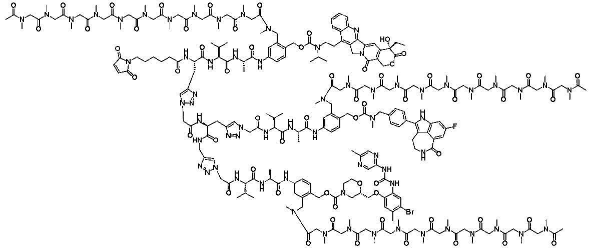 | Formula A129 | Combination 5 |
| 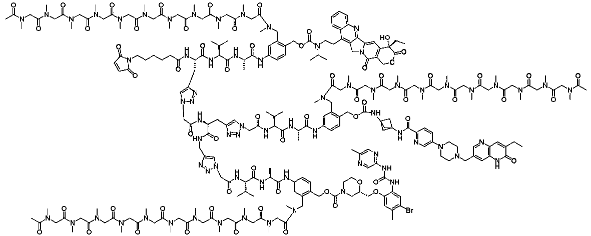 | Formula A130 | Combination 5 |
| 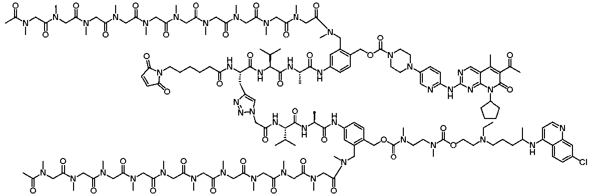 | Formula A131 | Combination 35 |
| 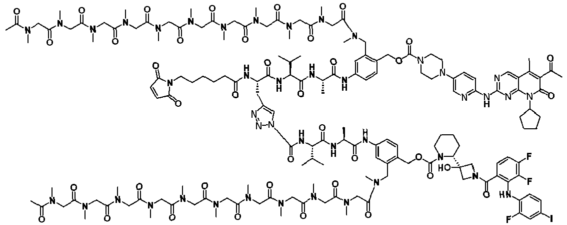 | Formula A132 | Combination 33 |
| 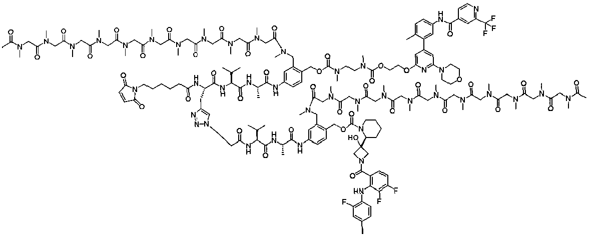 | Formula A133 | Combination 57 |
| 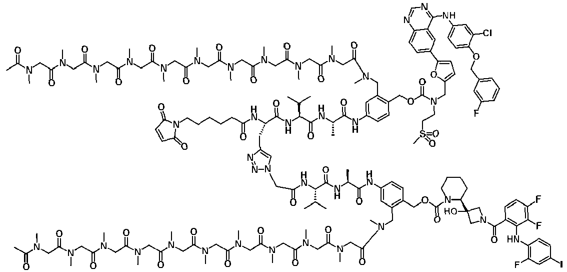 | Formula A134 | Combination 58 |
| 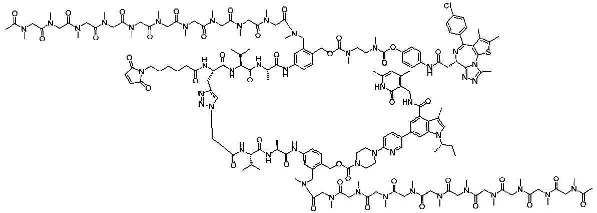 | Formula A135 | Combination 50 |
| 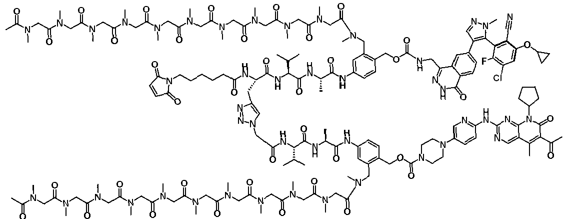 | Formula A136 | Combination 53 |
| 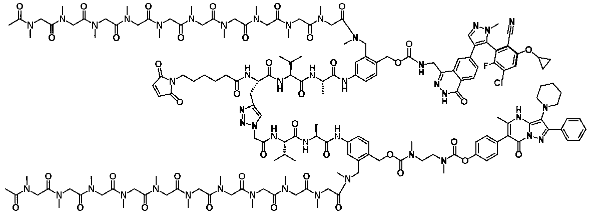 | Formula A137 | Combination 51 |
| 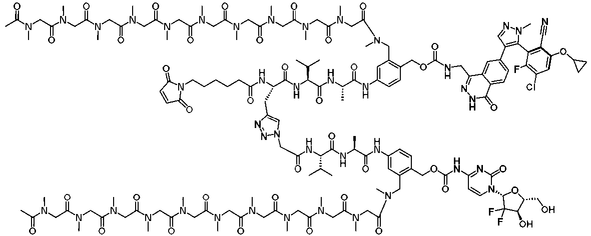 | Formula A138 | Combination 52 |
| 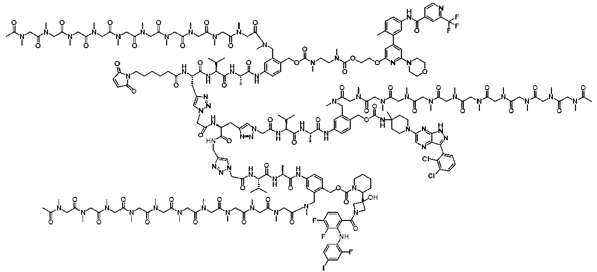 | Formula A139 | Combination 59 |
| 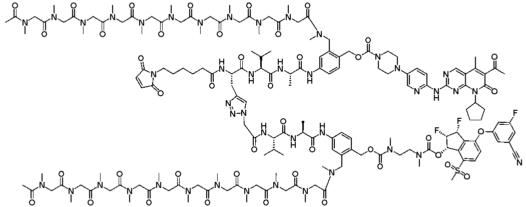 | Formula A140 | Combination 60 |
| 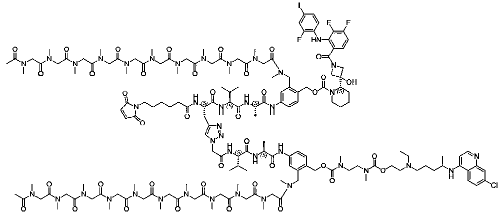 | Formula A141 | Combination 36 |
| 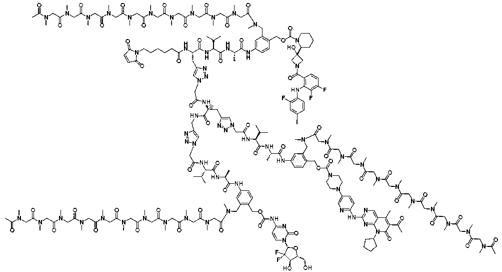 | Formula A142 | MEK+CDK4 /6+DNA synthesis |
| 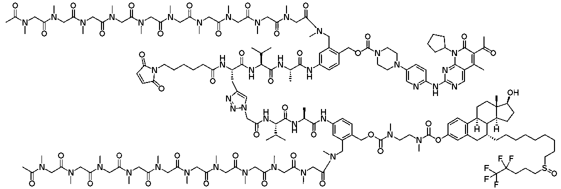 | Formula A143 | Combination 45 |
| 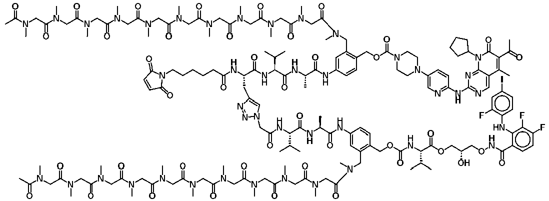 | Formula A144 | Combination 33 |
| 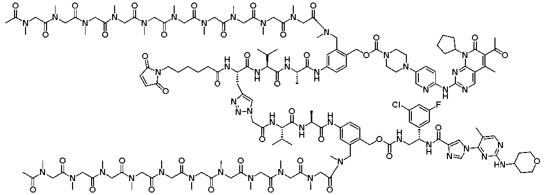 | Formula A145 | Combination 40 |
| 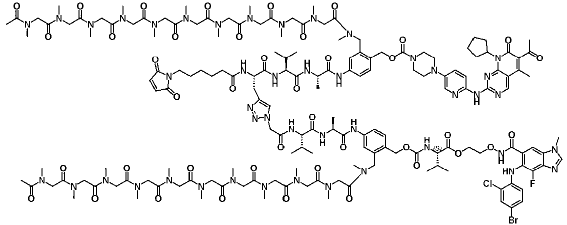 | Formula A146 | Combination 33 |
| 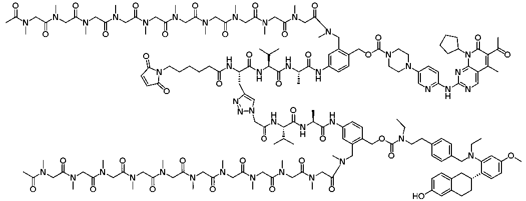 | Formula A147 | Combination 45 |
| 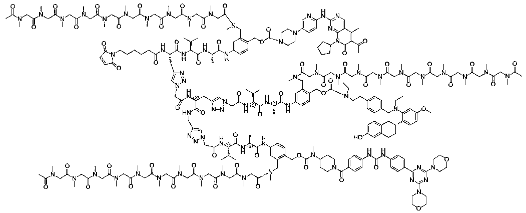 | Formula A148 | Combination 46 |
| 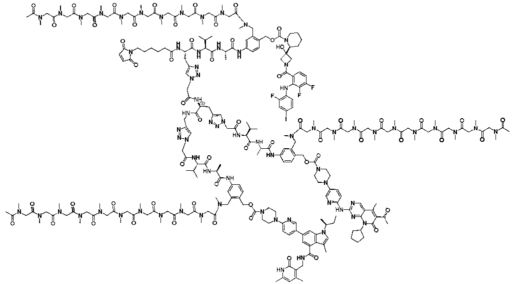 | Formula A149 | Combination 34 |
| 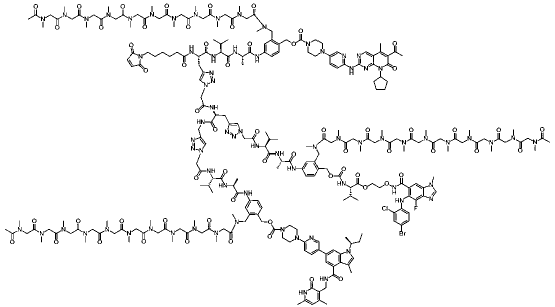 | Formula A150 | Combination 34 |
| 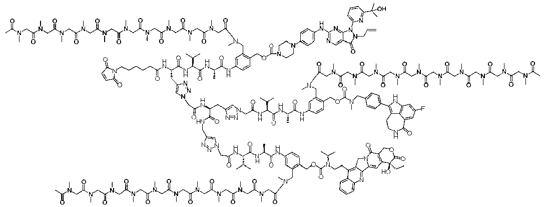 | Formula A151 | Combination 6 |
| 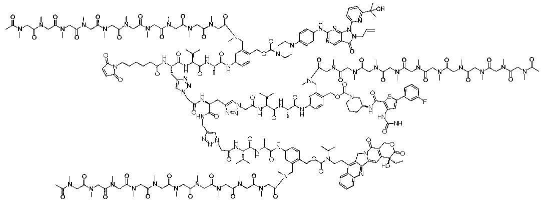 | Formula A152 | Combination 8 |
| 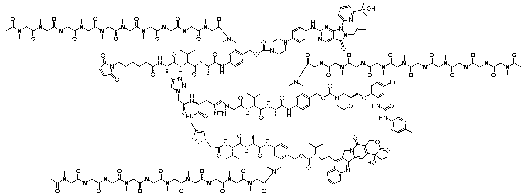 | Formula A153 | Combination 8 |
| 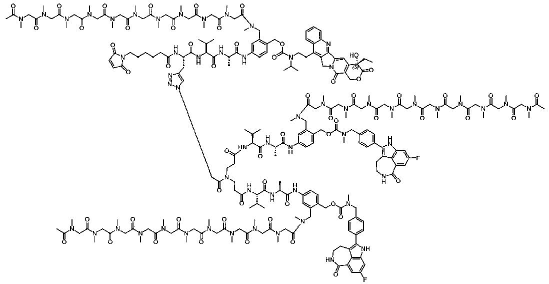 | Formula A154 | Combination 1 |
| 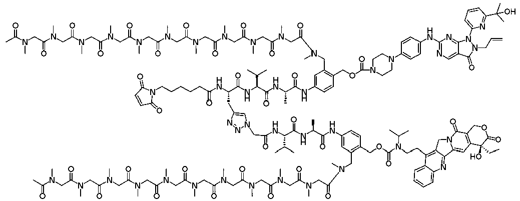 | Formula A155 | Combination 61 |
| 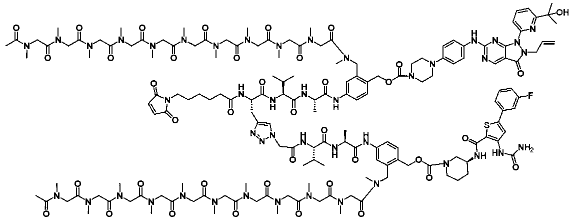 | Formula A156 | Combination 62 |
| 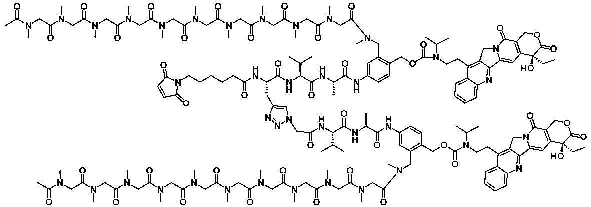 | Formula A157 | Combination 63 |
| 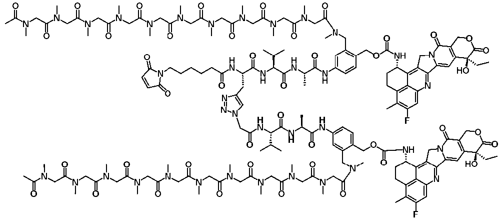 | Formula A158 | Combination 63 |
| 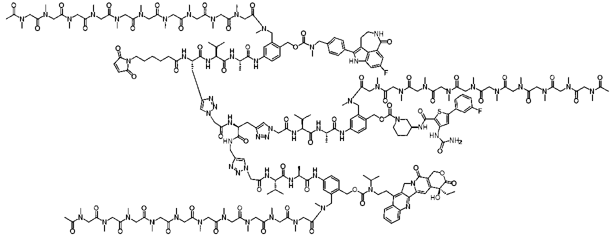 | Formula A159 | Combination 5 |
| 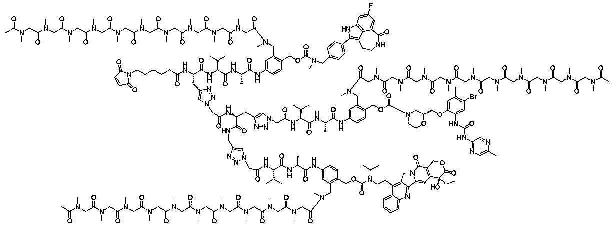 | Formula A160 | Combination 5 |
| 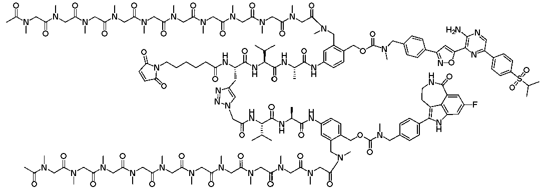 | Formula A161 | Combination 12 |
| 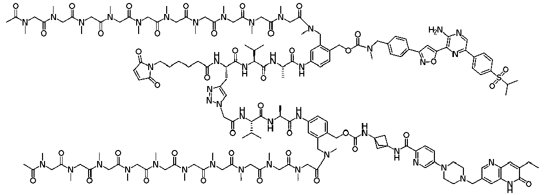 | Formula A162 | Combination 12 |
| 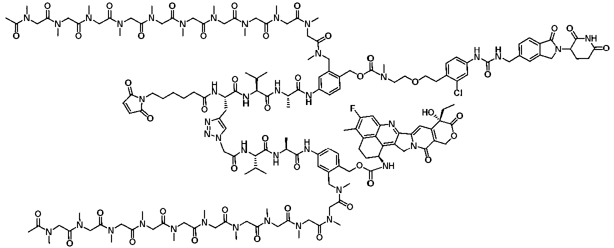 | Formula A163 | Combination 24 |
| 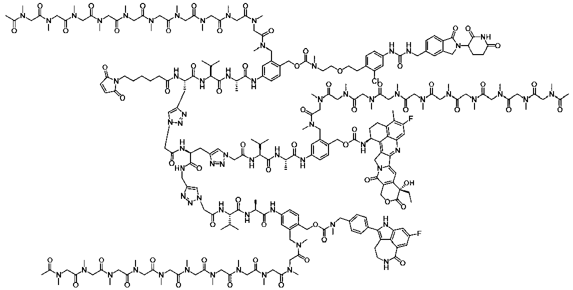 | Formula A164 | Combination 25 |
| 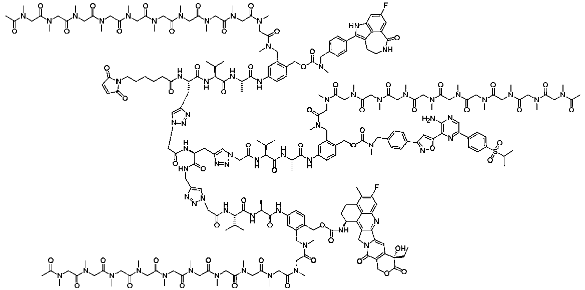 | Formula A165 | Combination 4 |
| 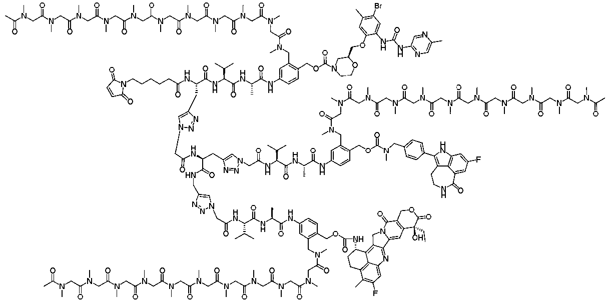 | Formula A166 | Combination 5 |
| 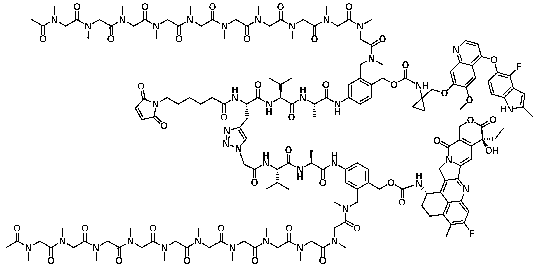 | Formula A167 | TOPO1+TKI |
| 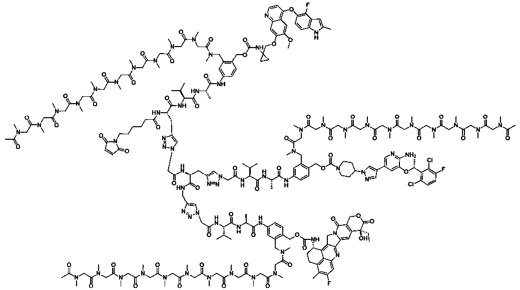 | Formula A168 | TOPO1+TKI |
| 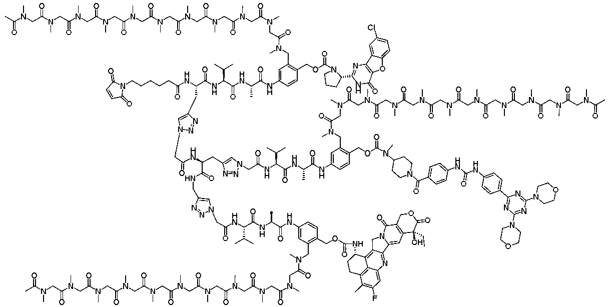 | Formula A169 | CDC7+PI3K+ TOPO1 |
| 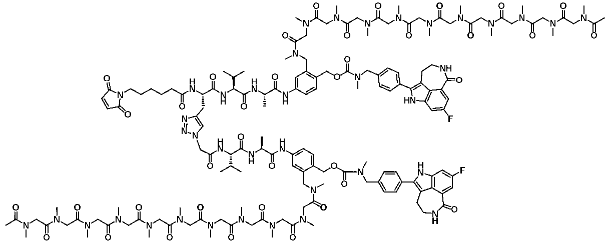 | Formula A170 | PARP+PARP |
| 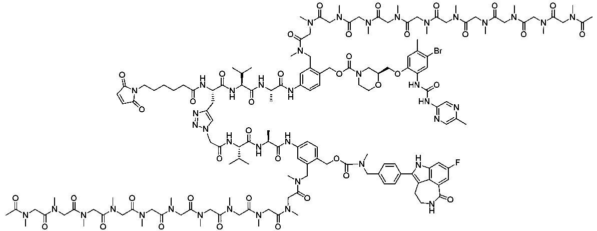 | Formula A171 | PARP+CHK1 |
| 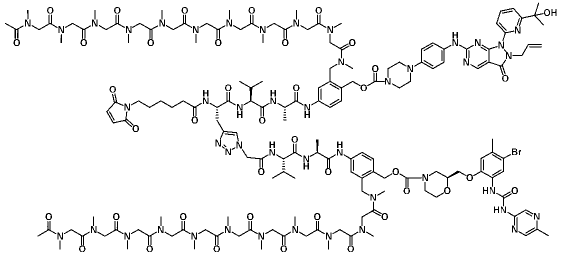 | Formula A172 | CHK1+WEE1 |
| 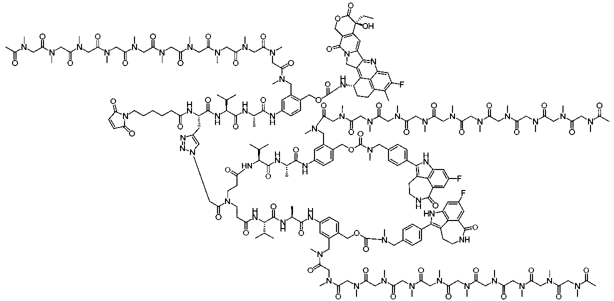 | Formula A173 | TOPO1+PARP+PARP |
| 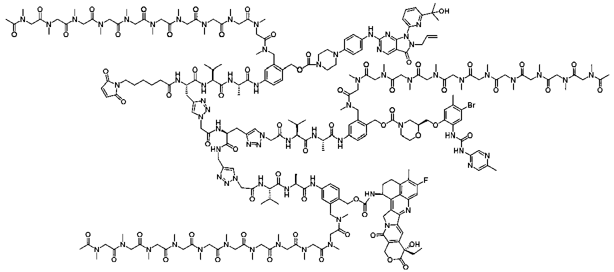 | Formula A174 | TOPO1+CHK1+WEE1 |
| 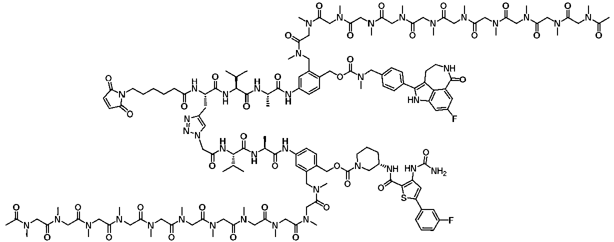 | Formula A175 | PARP+CHK1 |
| 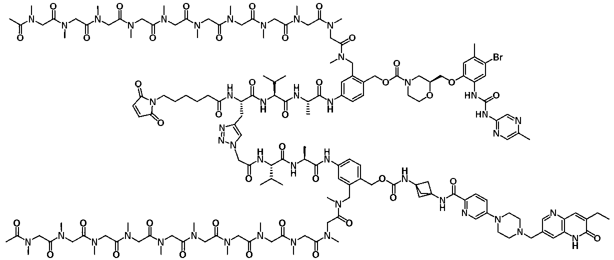 | Formula A176 | PARP+CHK1 |
| 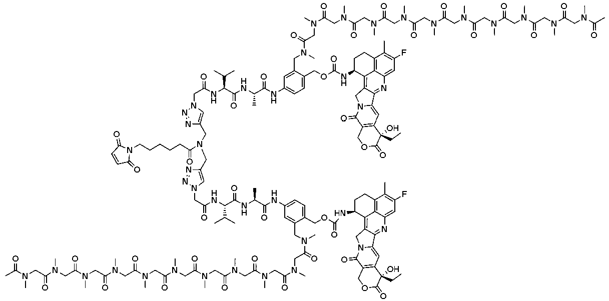 | Formula A177 | TOPO1+ TOPO1 |
| 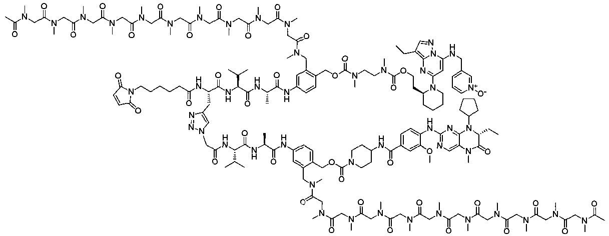 | Formula A178 | PLK1+CDK |
| 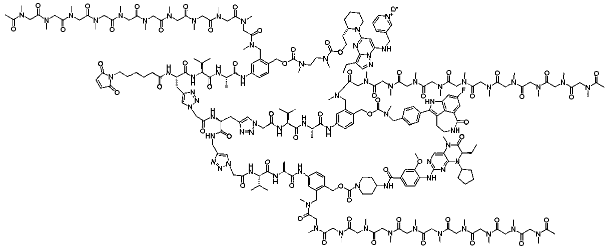 | Formula A179 | PLK1+CDK+ PARP |
| 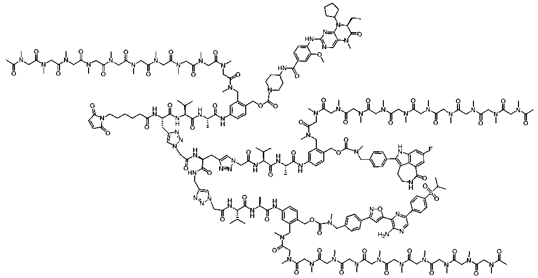 | Formula A180 | PLK1+PARP+ ATR |
| 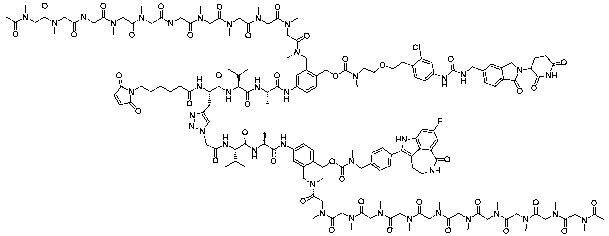 | Formula A181 | GSPT1+PARP |
| 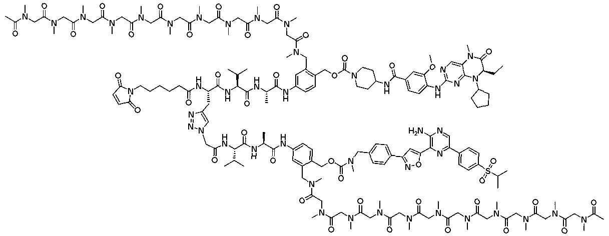 | Formula A182 | PLK1+ATR |
| 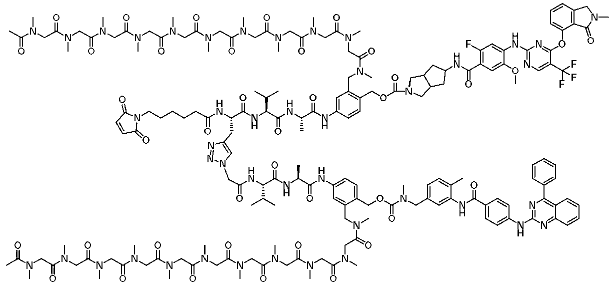 | Formula A183 | FAK+SMO |
| 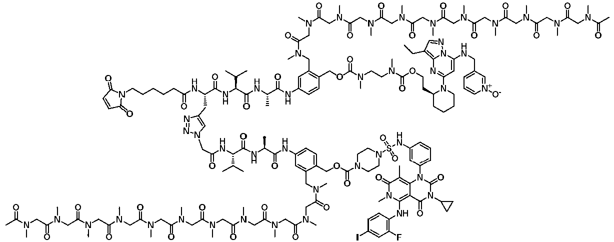 | Formula A184 | MEK+CDK |
| 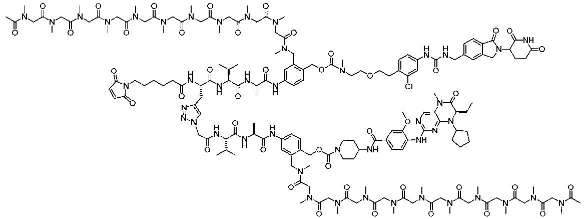 | Formula A185 | GSPT1+PLK1 |
| 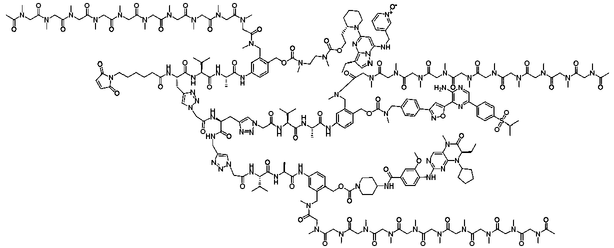 | Formula A186 | CDK+ATR+P LK1 |
| 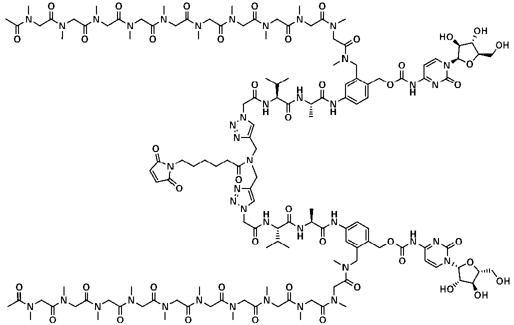 | Formula A187 | DNA+DNA synthesis |
| 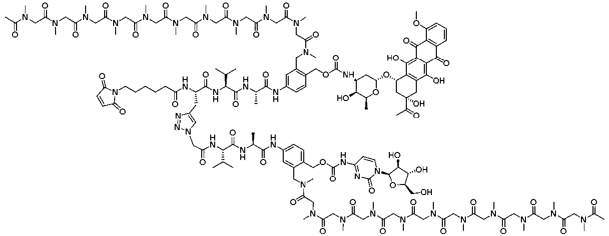 | Formula A188 | DNA+TOPO2 |
| 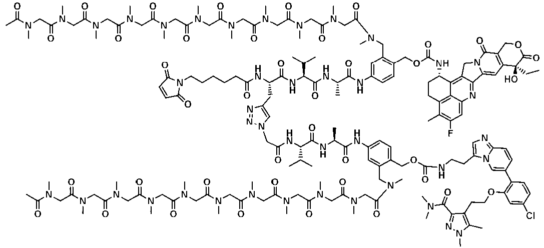 | Formula A189 | TOPO1+NMT |
| 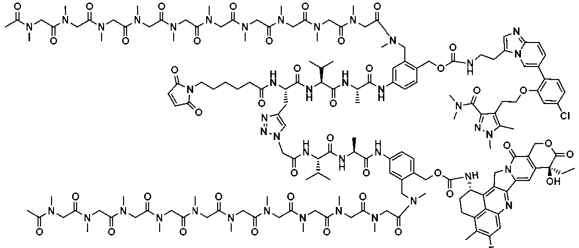 | Formula A190 | NMT+TOPO1 |

14. A compound, or a tautomer, a mesomer, a racemate, an enantiomer, or a diastereomer thereof, or a deuterated compound form thereof, or a mixture form thereof, or a pharmaceutically acceptable salt, prodrug, or solvate thereof, wherein the compound comprises a structure represented by formula II: 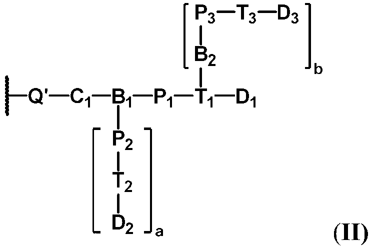 wherein,
Q' is a functional group capable of conjugating to cysteine, lysine, a non-natural amino acid, or a glycosyl of an antibody molecule;
C₁ is selected from the group consisting of: a direct bond, optionally substituted alkylene, optionally substituted polyethylene glycol group, optionally substituted alkenylene, optionally substituted alkynylene, optionally substituted alicyclylene, optionally substituted aliphatic heterocyclylene, optionally substituted arylene, and optionally substituted heteroarylene; when substituted, the substituent(s) are selected from halogen, hydroxyl, amino, carboxyl, sulfonic acid group, sulfone group, phosphonic acid group, and alkoxy;
B₁ and B₂ are independently a direct bond or a branching group, provided that when C₁ is a direct bond, B₁ is not a direct bond;
P₁, P₂, and P₃ are each independently an optionally substituted polypeptide residue or a glucose fragment;
T₁, T₂, and T₃ are each independently a direct bond or an optionally substituted spacer group, provided that at most two of T₁, T₂, and T₃ are direct bonds and at least one of B₂, T₁, T₂, and T₃ is substituted with a hydrophilic group;
D₁, D₂, and D₃ are a first drug unit, a second drug unit, and a third drug unit, respectively, and are the same or different;
a and b are independently 0, 1, 2, or 3, provided that a and b are not both 0; and
when a=0, B₁ is a direct bond and T₁ is not a direct bond;
when b=0, B₁ is not a direct bond.

15. The compound, or a tautomer, a mesomer, a racemate, an enantiomer, or a diastereomer thereof, or a deuterated compound form thereof, or a mixture form thereof, or a pharmaceutically acceptable salt, prodrug, or solvate thereof, according to claim 14, wherein the compound comprises a structure represented by formula IIa: 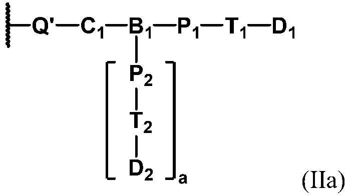 wherein:
Q' is a functional group capable of conjugating to cysteine, lysine, a non-natural amino acid, or a glycosyl of an antibody molecule;
C₁ is selected from the group consisting of: a direct bond, optionally substituted alkylene, optionally substituted polyethylene glycol group, optionally substituted alkenylene, optionally substituted alkynylene, optionally substituted alicyclylene, optionally substituted aliphatic heterocyclylene, optionally substituted arylene, and optionally substituted heteroarylene; when substituted, the substituent(s) are selected from halogen, hydroxyl, amino, carboxyl, sulfonic acid group, sulfone group, phosphonic acid group, and alkoxy;
B₁ is a branching group;
P₁ and P₂ are each independently an optionally substituted polypeptide residue or a glucose fragment;
T₁ and T₂ are each independently a direct bond or an optionally substituted spacer group, provided that at most one of T₁ and T₂ is a direct bond, and at least one of T₁ and T₂ is substituted with a hydrophilic group;
D₁ and D₂ are a first drug unit and a second drug unit, respectively, and are the same or different;
a is 1, 2, or 3.

16. The compound, or a tautomer, a mesomer, a racemate, an enantiomer, or a diastereomer thereof, or a deuterated compound form thereof, or a mixture form thereof, or a pharmaceutically acceptable salt, prodrug, or solvate thereof, according to claim 14, wherein the compound comprises a structure represented by formula IIb: 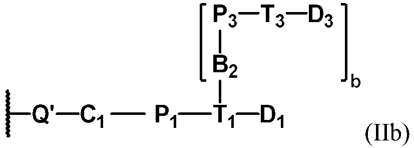
Q' is a functional group capable of conjugating to cysteine, lysine, a non-natural amino acid, or a glycosyl of an antibody molecule;
C₁ is selected from the group consisting of: optionally substituted alkylene, optionally substituted polyethylene glycol group, optionally substituted alkenylene, optionally substituted alkynylene, optionally substituted alicyclylene, optionally substituted aliphatic heterocyclylene, optionally substituted arylene, and optionally substituted heteroarylene; when substituted, the substituent(s) are selected from halogen, hydroxyl, amino, carboxyl, sulfonic acid group, sulfone group, phosphonic acid group, and alkoxy;
B₂ is a branching group;
P₁ and P₃ are each independently a direct bond or an optionally substituted polypeptide residue or a glucose fragment;
T₁ is an optionally substituted spacer group, T₃ is a direct bond or an optionally substituted spacer group, and at least one of B₂ and T₃ is substituted with a hydrophilic group;
D₁ and D₃ are a first drug unit and a third drug unit, respectively, and are the same or different;
b is 1, 2, or 3.

17. The compound, or a tautomer, a mesomer, a racemate, an enantiomer, or a diastereomer thereof, or a deuterated compound form thereof, or a mixture form thereof, or a pharmaceutically acceptable salt, prodrug, or solvate thereof, according to claim 14, wherein:
B₁ and B₂ are independently a branching group;
P₁, P₂, and P₃ are each independently an optionally substituted polypeptide residue or a glucose fragment;
T₁ is an optionally substituted spacer group, T₂ and T₃ are each independently a direct bond or an optionally substituted spacer group, and at least one of B₂, T₂, and T₃ is substituted with a hydrophilic group;
D₁, D₂, and D₃ are a first drug unit, a second drug unit, and a third drug unit, respectively, and are the same or different; and
a and b are independently 1, 2, or 3.

18. The compound, or a tautomer, a mesomer, a racemate, an enantiomer, or a diastereomer thereof, or a deuterated compound form thereof, or a mixture form thereof, or a pharmaceutically acceptable salt, prodrug, or solvate thereof, according to any one of claims 14 to 17, wherein: 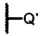 is selected from the group consisting of: optionally substituted 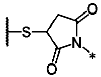 (Q1'), optionally substituted 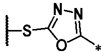 (Q2'), optionally substituted 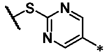 (Q3'), optionally substituted 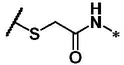 (Q4'), optionally substituted 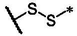 (Q5'), optionally substituted 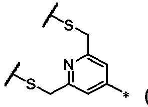 (Q6'), optionally substituted 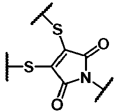 (Q7'), and optionally substituted 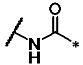 (Q8'), wherein * represents a site connected to C₁, and the wave line represents a site connected to the antibody.

19. The compound, or a tautomer, a mesomer, a racemate, an enantiomer, or a diastereomer thereof, or a deuterated compound form thereof, or a mixture form thereof, or a pharmaceutically acceptable salt, prodrug, or solvate thereof, according to any one of claims 14 to 18, wherein:
C₁ is selected from: a direct bond, -(CH₂)ₘ₁-, -(CH₂)ₘ₁-(O-CH₂-CH₂)ₘ₂-, -(CH₂)ₘ₁-(O-CH₂-CH₂)ₘ₂-(CH₂)ₘ₃-, -(CH₂)ₘ₁-C(O)NH-(CH₂)ₘ₂-, -(CH₂)ₘ₁-NHC(O)-(CH₂)ₘ₂-, and -(C≡ C-(CH₂)ₘ₁-, wherein m1, m2, and m3 are independently integers from 1 to 6, and the indicated substituent is connected to Q' on the left side and to B₁ or P₁ on the right side.

20. The compound, or a tautomer, a mesomer, a racemate, an enantiomer, or a diastereomer thereof, or a deuterated compound form thereof, or a mixture form thereof, or a pharmaceutically acceptable salt, prodrug, or solvate thereof, according to any one of claims 14 to 19, wherein:
B₁ is selected from the group consisting of: a direct bond, 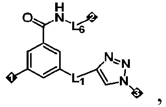 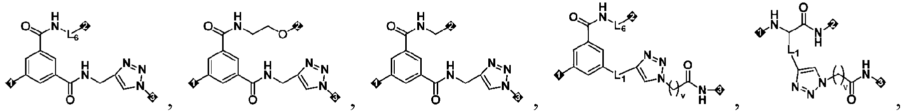 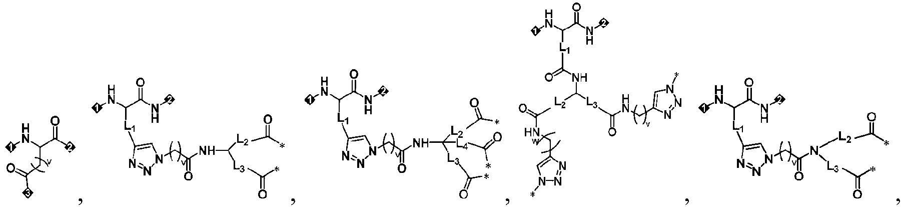 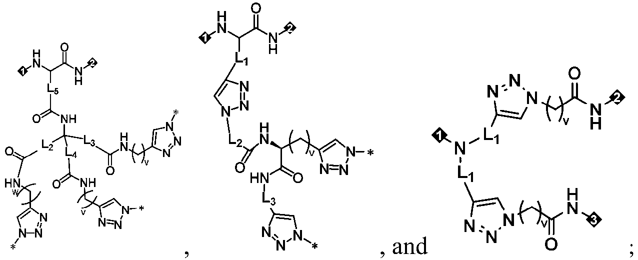
preferably, 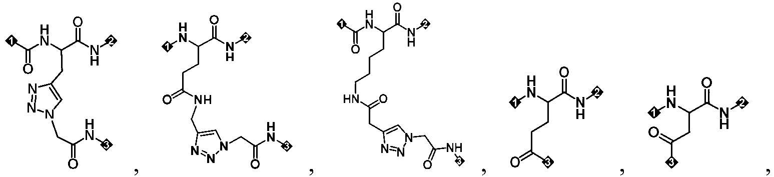 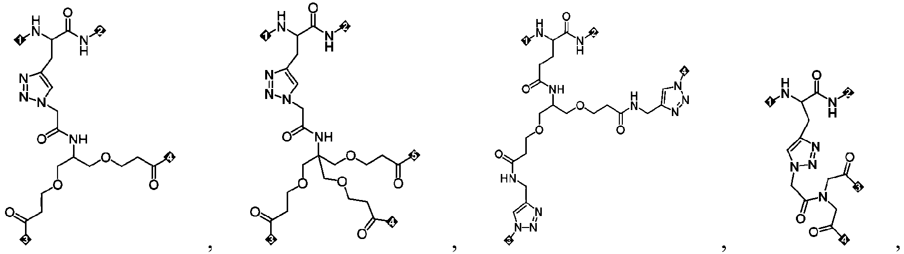 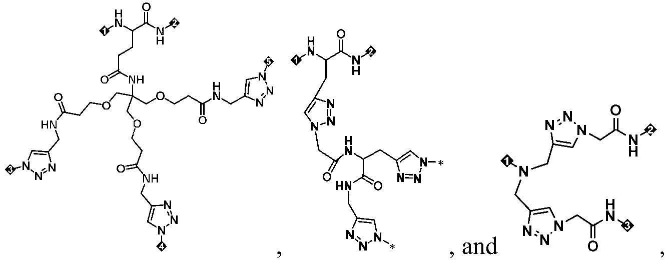
wherein attachment point 1 is connected to C₁ or Q' (when C₁ is a direct bond), attachment point 2 is connected to P₁, attachment points *, 3, 4, and 5 are connected to P₂, L₁ is selected from -(CH₂)ₘ₁-, -(CH₂)ₘ₁-C(O)NH-(CH₂)ₘ₂-, -(CH₂)ₘ₁-NHC(O)-(CH₂)ₘ₂-, L₂, L₃, and L₄ are independently -(CH₂)ₘ₁-(O-CH₂-CH₂)ₘ₂- or -(CH₂)ₘ₁-(O-CH₂-CH₂)ₘ₂-(CH₂)ₘ₃-, L₅ is -(CH₂)ₘ₁-, L₆ is -(CH₂)ₘ₁- or - (CH₂-CH₂-O)m₂, wherein m1, m2, m3, w, and v are each independently 1, 2, 3, 4, 5, or 6;
preferably, when C₁ is a direct bond, B₁ is

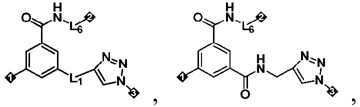

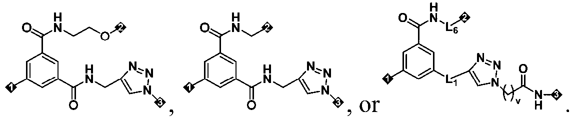

21. The compound, or a tautomer, a mesomer, a racemate, an enantiomer, or a diastereomer thereof, or a deuterated compound form thereof, or a mixture form thereof, or a pharmaceutically acceptable salt, prodrug, or solvate thereof, according to any one of claims 14 to 20, wherein:
B₂ is selected from the group consisting of: 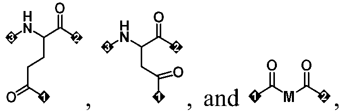 wherein attachment point 1 is connected to T₁, attachment point 2 is connected to P₃, attachment point 3 is connected to the hydrophilic group, M is selected from the group consisting of: optionally substituted C₁-C₆ alkylene, optionally substituted C₁-C₆ alkoxy, and optionally substituted C₁-C₅ alkenylene.

22. The compound, or a tautomer, a mesomer, a racemate, an enantiomer, or a diastereomer thereof, or a deuterated compound form thereof, or a mixture form thereof, or a pharmaceutically acceptable salt, prodrug, or solvate thereof, according to any one of claims 14 to 21, wherein:
T₁, T₂, T₃ are independently selected from the group consisting of: a direct bond, 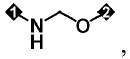 and optionally substituted 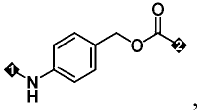 wherein attachment point 1 is connected to P₁, P₂, or P₃, and attachment point 2 is connected to D₁, D₂, or D₃.

23. The compound, or a tautomer, a mesomer, a racemate, an enantiomer, or a diastereomer thereof, or a deuterated compound form thereof, or a mixture form thereof, or a pharmaceutically acceptable salt, prodrug, or solvate thereof, according to any one of claims 14 to 22, wherein:
P₁, P₂, P₃ comprises an optionally substituted polypeptide residue composed of amino acids selected from the group consisting of: phenylalanine, isoleucine, leucine, tryptophan, valine, methionine, tyrosine, alanine, threonine, histidine, serine, glutamine, arginine, lysine, asparagine, glutamic acid, proline, citrulline, aspartic acid, and glycine; preferably, P₁, P₂, P₃ comprises an optionally substituted polypeptide residue composed of amino acids selected from the group consisting of: glycine, phenylalanine, valine, alanine, arginine, citrulline, aspartic acid, asparagine, and lysine;
more preferably, P₁, P₂, P₃ comprises an optionally substituted polypeptide residue selected from the group consisting of: phenylalanine-lysine (Phe-Lys), valine-alanine (Val-Ala), valine-citrulline (Val-Cit), glutamic acid-valine-alanine (Glu-Val-Ala), glutamic acid-valine-citrulline (Glu-Val-Cit), valine-lysine (Val-Lys), alanine-alanine (Ala-Ala), alanine-alanine-alanine (Ala-Ala-Ala), alanine-alanine-asparagine (Ala-Ala-Asn), alanine-leucine (Ala-Leu), leucine-leucine (Leu-Leu), phenylalanine-arginine (Phe-Arg), phenylalanine-lysine (Phe-Lys), 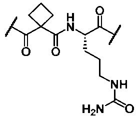 (cBu-Cit), 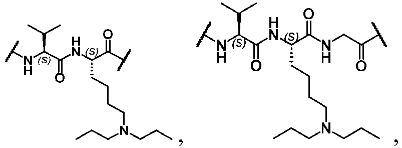 glycine-glycine-phenylalanine-glycine (Gly-Gly-Phe-Gly), glycine-proline (Gly-Pro);
or, wherein one of the combination P₂-T₂ and the combination P₃-T₃ is 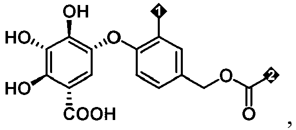 wherein attachment point 1 is connected to B₁ or B₂, and attachment point 2 is connected to D₂ or D₃.

24. The compound, or a tautomer, mesomer, racemate, enantiomer, or diastereomer thereof, or a deuterated compound form thereof, or a mixture form thereof, or a pharmaceutically acceptable salt, prodrug, or solvate thereof, according to any one of claims 14 to 23, wherein:
the hydrophilic group is selected from: substituted polysarcosine residue, polyglycerol, polyol, glycosyl, cyclodextrin, substituted ethylene glycol fragment, substituted glycosylated polyethylene glycol, substituted glycosylated polyglycerol, substituted cyclodextrin polyethylene glycol, or a combination thereof;
preferably, the substituted polysarcosine residue is 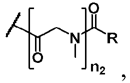 wherein n₂ is an integer between 4 and 20, for example between 4 and 16, R is selected from: C₁-C₆ alkyl, C₁-C₆ cycloalkyl, and C₁-C₆ alkoxy;
preferably, the substituted glycosylated polyethylene glycol fragment is 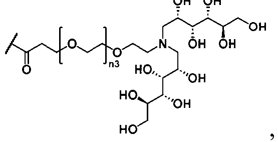 wherein n₃ is an integer between 4 and 18, for example between 4 and 12;
preferably, the substituted glycosylated polyglycerol fragment is 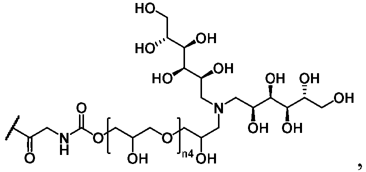 wherein n₄ is an integer between 4 and 12, for example between 4 and 10;
preferably, the substituted polyethylene glycol fragment is 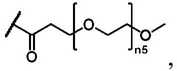 wherein n₅ is an integer between 4 and 24, for example between 8 and 18;
preferably, at least one of T₁, T₂, T₃ is 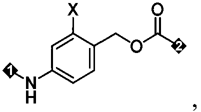 wherein X is optionally substituted 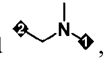 the hydrophilic group is connected to T₁, T₂, or T₃ via X, wherein attachment point 1 is connected to the hydrophilic group, and attachment point 2 is connected to one of T₁, T₂, T₃.

25. The compound, or a tautomer, a mesomer, a racemate, an enantiomer, or a diastereomer thereof, or a deuterated compound form thereof, or a mixture form thereof, or a pharmaceutically acceptable salt, prodrug, or solvate thereof, according to any one of claims 14 to 24, wherein:
D₁, D₂, D₃ are a first anti-cancer agent, a second anti-cancer agent, and a third anti-cancer agent; preferably, at least two of the first, second, and third anti-cancer agents have a synergistic anti-cancer effect;
preferably, D₁, D₂, D₃ are independently a cytotoxic drug or a tumor-targeted therapeutic drug; preferably, at least one of D₁, D₂, and D₃ is a cytotoxic drug, and at least another one is a tumor-targeted therapeutic drug; also preferably, at least two of D₁, D₂, and D₃ are tumor-targeted therapeutic drugs, for example, tumor-targeted therapeutic drugs that can produce a synergistic or additive effect;
preferably, the cytotoxic drug is selected from drug units targeting topoisomerase, drug units targeting tubulin, nucleoside antimetabolite anti-cancer drug units, and drug units targeting DNA;
preferably, the tumor-targeted therapeutic drug is selected from drug units targeting DNA damage response (DDR) or "synthetic lethality" related pathways, drug units targeting epigenetics, drug units targeting apoptosis-related pathways, drug units targeting transcription factors, and drug units targeting immune activation pathway-related targets, comprising: inhibitors of EFGR pathway-related targets; Ras-Raf-MAPK pathway inhibitors; PI3K/AKT/mTOR pathway inhibitors; cell cycle pathway inhibitors; cGAS-STING signaling pathway agonists; estrogen receptor antagonists; androgen receptor antagonists; glucocorticoid receptor modulators; autophagy inhibitors; FAK inhibitors; Smo inhibitors; BTK inhibitors; PDE4 inhibitors; Lck inhibitors; PLK1 inhibitors; TLR7/8 modulators; N-myristoyltransferase (NMT) inhibitors;
preferably, the drug unit targeting topoisomerase is a topoisomerase I (TOPO1) inhibitor or topoisomerase II (TOPO2) inhibitor, comprising Exatecan (CAS 171335-80-1), DXd (CAS 1599440-33-1), 7-ethyl-10-hydroxycamptothecin (SN38, CAS 86639-52-3), Belotecan (CAS 213819-48-8), (4-NH₂)-Exatecan (AZD'0132, CAS 2495742-21-5), 7-MAD-MDCPT (CAS 765871-81-6), 7-aminomethyl-10-methyl-11-fluorocamptothecin (CAS 2378616-23-8), Voreloxin (CAS 175414-77-4), or derivatives and analogs thereof;
preferably, the drug unit targeting tubulin is Eribulin, Vinblastine, Paclitaxel, MMAE, MMAF, Maytansine, or derivatives thereof;
preferably, the nucleoside antimetabolite anti-cancer drug is Gemcitabine, Decitabine;
preferably, the drug unit targeting DNA is a DNA minor groove binder (PBD), a DNA alkylator (Duocarmycin), Trabectedin, Lurbinectedin, or derivatives thereof;
preferably, the drug unit targeting DNA damage response (DDR) or "synthetic lethality" related pathways is a PARP inhibitor, ATR inhibitor, CHK1 inhibitor, ATM inhibitor, DNA-PK inhibitor, WEE1 inhibitor, POLQ inhibitor, CDK12 inhibitor, USP1 inhibitor, PKMYT1 inhibitor, or Rad51 inhibitor;
the PARP inhibitor is preferably Rucaparib, Niraparib, Veliparib, A-966492, Talazoparib, AZD5305, Venadaparib, Mefuparib, or analogs thereof; the ATR inhibitor is preferably Berzosertib, Ceralasertib, or analogs thereof; the CHK1 inhibitor is preferably Prexasertib (LY2606368), AZD7762, Rabusertib (LY2603618), MK-8776 (SCH 900776), CHIR-124, PF-477736, CCT245737 (SRA737, PNT-737), GDC-0575 (ARRY-575), or analogs thereof; and/or the WEE1 inhibitor is preferably ZN-C3 (CAS 2376146-48-2), Adavosertib (AZD1775, CAS 955365-80-7), Debio 0123 (CAS 2243882-74-6), or analogs thereof;
preferably, an analog of the AZD5305 comprises the following structure: 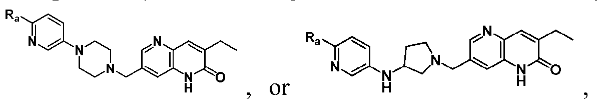 wherein Rₐ is selected from - C(O)NH-R_{b} or -NHC(O)-R_{b}, R_{b} is selected from: C₂₋₇ alkyl, C₃₋₇ monocyclic cycloalkyl, C₄₋₁₀ bicyclic cycloalkyl, C₅₋₉ spirocycloalkyl, and C₅₋₉ bridged cycloalkyl, each substituted with at least one primary or secondary amino group, or R_{b} is a 4-6 membered monocyclic heterocycloalkyl, C₄₋₁₀ bicyclic heterocycloalkyl, C₅₋₉ spiroheterocycloalkyl, C₅₋₉ bridged heterocycloalkyl containing 1-5 nitrogen, oxygen, sulfur atoms, wherein the R_{b} group comprises at least one primary or secondary amino group;
preferably, the AZD5305 analog is selected from the following structures: 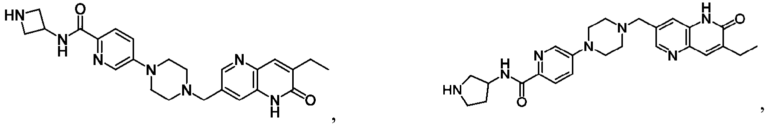 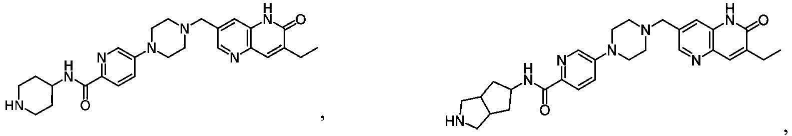 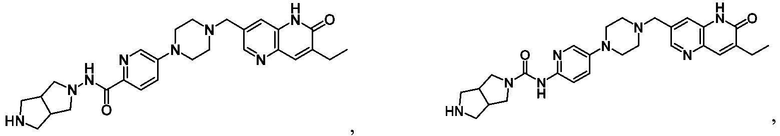 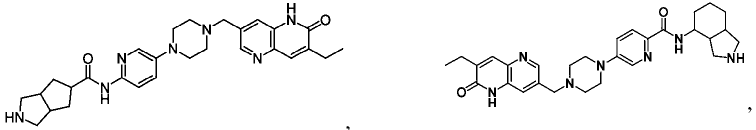 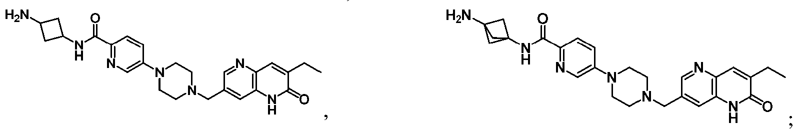
preferably, the PKMYT1 inhibitor is preferably RP-6306 and GSK-1520489A or analogs thereof;
preferably, analogs of the WEE1 inhibitors Adavosertib (AZD1775, CAS 955365-80-7), Debio 0123 (CAS 2243882-74-6) are selected from the following structures: 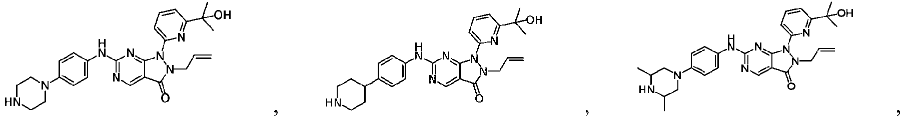 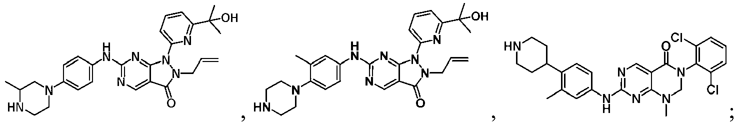
preferably, the drug unit targeting apoptosis-related pathways is a Bcl-2 family protein inhibitor; the Bcl-2 family protein inhibitor comprises BCL-2 inhibitors, BCL-XL inhibitors, and MCL-1 inhibitors, preferably Venetoclax (CAS 1257044-40-8), Navitoclax (CAS 923564-51-6), ABT-737 (CAS 852808-04-9), A-1331852 (CAS 1430844-80-6), S64315 (CAS 1799631-75-6), or analogs thereof;
preferably, the A-1331852, S64315 analogs are selected from the following structures: 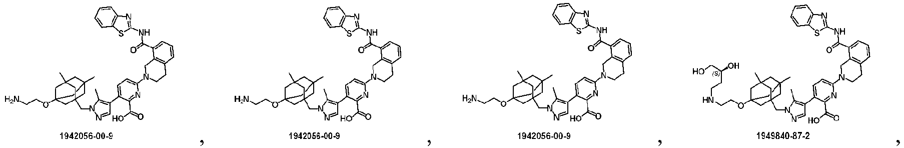 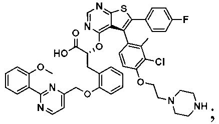
preferably, the drug unit targeting epigenetics is an LSD1 inhibitor, EZH2 inhibitor, BRD4 inhibitor, PRMT5 inhibitor, or PRMT1 inhibitor;
the LSD1 inhibitor is preferably Tranylcypromine, ORY-1001 (Iadademstat, CAS 1431303-72-8), CC-90011 (Pulrodemstat, CAS 1821307-10-1), ORY-2001, GSK-2879552, IMG-7289, INCB059872, or TAK-418 or analogs thereof; the EZH2 inhibitor is preferably Tazemetostat (CAS 1403254-99-8), GSK2816126, CPI-1205, PF-06821497, SHR2554, XNW5004, HH2853, or analogs thereof; the BRD4 inhibitor is a BI-2536 analog, Birabresib; the PRMT5 inhibitor is preferably GSK3326595, AMG 193, MRTX1719, SKL27969, TNG908, SCR-6920, SH3765, SYHX2001, or analogs thereof; the PRMT1 inhibitor is preferably GSK3368715 (CAS 1629013-22-4), MS023 (CAS 1831110-54-3), or analogs thereof;
preferably, the drug unit targeting immune activation pathway-related targets is a PD-L1 inhibitor, CBL-B inhibitor, TLR7/8 agonist, PTPN2/1 inhibitor, or STING agonist;
preferably, the Ras-Raf-MAPK pathway inhibitors comprise: SOS1 inhibitors BAY-293 (CAS 2244904-70-7), BI-3406 (CAS 2230836-55-0), or analogs thereof; KRAS inhibitors BI-2493 (CAS 2937344-16-4), MRTX1133 (CAS 2621928-55-8), or analogs thereof; MEK inhibitors Cobimetinib (CAS 934660-93-2), Selumetinib (CAS 606143-52-6), Mirdametinib (CAS 391210-10-9), Binimetinib (CAS 606143-89-9), TAK-733 (CAS 1035555-63-5), GDC-0623 (CAS 1168091-68-6), AZD8330 (CAS 869357-68-6), Trametinib (CAS 871700-17-3), Trametiglue (CAS 2666940-97-0), or analogs thereof; ERK inhibitors ASN007 (CAS 2055597-12-9) or analogs thereof;
preferably, the cell cycle pathway inhibitors comprise: CDK4/6 inhibitors Palbociclib (CAS 571190-30-2), Abemaciclib (CAS 1231929-97-7), Ribociclib (CAS 1211441-98-3), Dalpiciclib (CAS 1637781-04-4), or analogs thereof; pan-CDK inhibitors Dinaciclib (CAS 779353-01-4) or analogs thereof;
preferably, the PI3K/AKT/mTOR pathway inhibitors comprise: PKI-587 (CAS 1197160-78-3), desmethyl PKI-587 (CAS 1950569-63-7), AZD8055 (CAS 1009298-09-2), Afuresertib (CAS 1047644-62-1), or analogs thereof;
preferably, the estrogen receptor antagonists/degraders comprise Fulvestrant (CAS 129453-61-8), Elacestrant (CAS 1349723-93-8); the androgen receptor antagonists comprise abiraterone (CAS 154229-19-3), JNJ-63576253 (CAS 2110428-64-1), or analogs thereof;
preferably, the autophagy inhibitor is Hydroxychloroquine (HCQ, CAS 118-42-3) and Chloroquine (CQ, CAS 54-05-7) or analogs thereof;
preferably, the drug targeting transcription factors is a GSPT1 degrader (molecular glue), and the GSPT1 degrader is selected from the following structures: 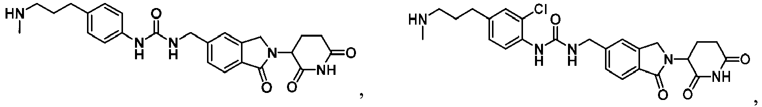 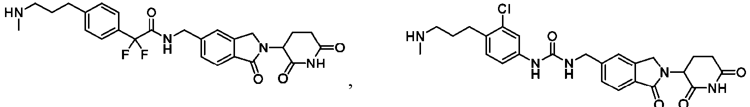 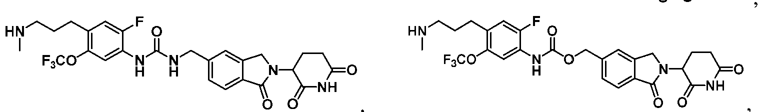 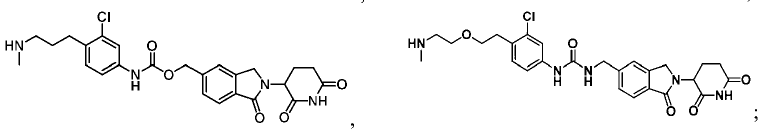
preferably, the FAK inhibitor is Ifebemtinib (CAS 1227948-82-4), Defactinib (CAS 1073154-85-4), PF-562271 (CAS 717907-75-0), or analogs thereof, selected from the following structures: 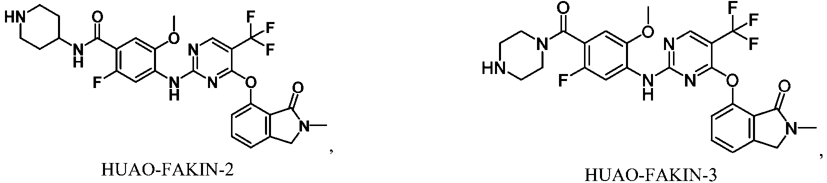 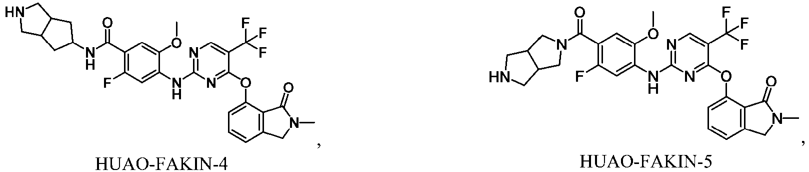 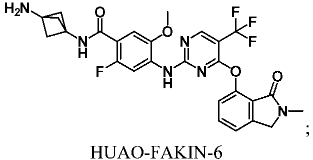
preferably, the PLK1 inhibitor is BI2536 (CAS 755038-02-9), Volasertib (CAS 755038-65-4), GSK461364 (CAS 929095-18-1), Onvansertib (CAS 1034616-18-6), or analogs thereof;
preferably, the N-myristoyltransferase (NMT) inhibitor is MYX1715 (CAS 2445448-66-6) or analogs thereof;
more preferably, D₁, D₂, and D₃ are selected from the combinations in the following table:
| Drug Combination | D1 | D2 | D3 |
|---|---|---|---|
| Combination 1 | TOPO1 inhibitor | PARP inhibitor | / |
| Combination 2 | TOPO1 inhibitor | ATR inhibitor | / |
| Combination 3 | TOPO1 inhibitor | CHK1 inhibitor | / |
| Combination 4 | TOPO1 inhibitor | PARP inhibitor | ATR inhibitor |
| Combination 5 | TOPO1 inhibitor | PARP inhibitor | CHK1 inhibitor |
| Combination 6 | TOPO1 inhibitor | PARP inhibitor | WEE1 inhibitor |
| Combination 7 | TOPO1 inhibitor | ATR inhibitor | WEE1 inhibitor |
| Combination 8 | TOPO1 inhibitor | CHK1 inhibitor | WEE1 inhibitor |
| Combination 9 | TOPO1 inhibitor | DNA-PK inhibitor | / |
| Combination 10 | TOPO1 inhibitor | POLQ inhibitor | / |
| Combination 11 | TOPO1 inhibitor | TOPO2 inhibitor | / |
| Combination 12 | PARP inhibitor | ATR/CHK1/WEE1 inhibitor | / |
| Combination 13 | PARP inhibitor | ATR inhibitor | CHK1 inhibitor |
| Combination 14 | TOPO1 inhibitor | Immune activator (PD-L1 inhibitor, CBL-B inhibitor, TLR7/8 agonist, PTPN2/1 inhibitor, or STING agonist) | / |
| Combination 15 | TOPO1 inhibitor | BCL-2 family inhibitor | / |
| Combination 16 | TOPO1 inhibitor | BCL-2 family inhibitor | MCL-1 inhibitor |
| Combination 17 | TOPO1 inhibitor | DNA synthesis inhibitor | / |
| Combination 18 | TOPO1 inhibitor | PI3K/mTOR inhibitor | / |
| Combination 19 | TOPO1 inhibitor | EGFR inhibitor | / |
| Combination 20 | TOPO1 inhibitor | Autophagy inhibitor | / |
| Combination 21 | TOPO1 inhibitor | FAK inhibitor | |
| Combination 22 | TOPO1 inhibitor | PARP inhibitor | FAK inhibitor |
| Combination 23 | TOPO1 inhibitor | PARP inhibitor | Androgen receptor antagonist/degrader |
| Combination 24 | TOPO1 inhibitor | GSPT1 degrader | / |
| Combination 25 | TOPO1 inhibitor | PARP inhibitor | GSPT1 degrader |
| Combination 26 | TOPO1 inhibitor | Tubulin inhibitor | / |
| Combination 27 | TOPO1 inhibitor | Epigenetic regulator | / |
| Combination 28 | Tubulin inhibitor | Immune activator (PD-L1 inhibitor, CBL-B inhibitor, TLR7/8 agonist, PTPN2/1 inhibitor, or STING agonist) | / |
| Combination 29 | Tubulin inhibitor | BCL-2/BCL-XL inhibitor | / |
| Combination 30 | Tubulin inhibitor | BCL-2/BCL-XL inhibitor | MCL-1 inhibitor |
| Combination 31 | Tubulin inhibitor | PI3K/AKT/mTOR inhibitor | / |
| Combination 32 | Tubulin inhibitor | FAK inhibitor | |
| Combination 33 | CDK4/6 inhibitor | MEK inhibitor | / |
| Combination 34 | CDK4/6 inhibitor | MEK inhibitor | EZH2 inhibitor |
| Combination 35 | CDK4/6 inhibitor | Autophagy inhibitor | / |
| Combination 36 | MEK inhibitor | Autophagy inhibitor | / |
| Combination 37 | ERK inhibitor | Autophagy inhibitor | / |
| Combination 38 | CDK4/6 inhibitor | MEK inhibitor | Autophagy inhibitor |
| Combination 39 | CDK4/6 inhibitor | ERK inhibitor | Autophagy inhibitor |
| Combination 40 | CDK4/6 inhibitor | ERK inhibitor | (MEK inhibitor) |
| Combination 41 | CDK4/6 inhibitor | MEK inhibitor | KRAS inhibitor |
| Combination 42 | CDK4/6 inhibitor | MEK inhibitor | SOS1 inhibitor |
| Combination 43 | CDK4/6 inhibitor | MEK inhibitor | FAK inhibitor |
| Combination 44 | CDK4/6 inhibitor | PRMT5 inhibitor | / |
| Combination 45 | CDK4/6 inhibitor | Estrogen receptor antagonist/degrader | / |
| Combination 46 | CDK4/6 inhibitor | Estrogen receptor antagonist/degrader | PI3K/mTOR inhibitor |
| Combination 47 | CDK4/6 inhibitor | MEK inhibitor | GSPT1 degrader |
| Combination 48 | CDK4/6 inhibitor | ERK inhibitor | GSPT1 degrader |
| Combination 49 | CDK4/6 inhibitor | Autophagy inhibitor | GSPT1 degrader |
| Combination 50 | BRD4 inhibitor | EZH2 inhibitor | / |
| Combination 51 | PRMT5 inhibitor | MAT2A inhibitor | / |
| Combination 52 | PRMT5 inhibitor | Gemcitabine | / |
| Combination 53 | PRMT5 inhibitor | CDK4/6 inhibitor | / |
| Combination 54 | PRMT5 inhibitor | PRMT1 inhibitor | / |
| Combination 55 | PRMT5 inhibitor | PRMT1 inhibitor | CDK4/6 inhibitor |
| Combination 56 | PRMT5 inhibitor | PRMT1 inhibitor | MEK inhibitor |
| Combination 57 | RAF inhibitor | MEK inhibitor | / |
| Combination 58 | EGFR inhibitor | MEK inhibitor | / |
| Combination 59 | RAF inhibitor | MEK inhibitor | SHP2 inhibitor |
| Combination 60 | CDK4/6 inhibitor | HIF-2alpha inhibitor | / |
| Combination 61 | TOPO1 inhibitor | WEE1 inhibitor | / |
| Combination 62 | WEE1 inhibitor | CHK1 inhibitor | / |
| Combination 63 | TOPO1 inhibitor | TOPO1 inhibitor | / |
| Combination 64 | CDK inhibitor | MEK inhibitor | |
| Combination 65 | FAK inhibitor | Smo inhibitor | / |
| Combination 66 | PLK1 inhibitor | CDK inhibitor | / |
| Combination 67 | PLK1 inhibitor | CDK inhibitor | PARP inhibitor/ |
| Combination 68 | TOPO1 inhibitor | PLK1 inhibitor | / |
| Combination 69 | TOPO1 inhibitor | PLK1 inhibitor | PARP inhibitor/ |
| Combination 70 | TOPO1 inhibitor | PLK1 inhibitor | ATR inhibitor/ |
| Combination 71 | TOPO1 inhibitor | NMT inhibitor | / |
| Combination 72 | Tubulin inhibitor | NMT inhibitor | / |
more preferably,
(i) at least one of D₁, D₂, and D₃ is a cytotoxic drug, the cytotoxic drug is selected from drugs targeting topoisomerase, for example a TOPO1 inhibitor, at least another one is a tumor-targeted therapeutic drug, the tumor-targeted therapeutic drug is a drug targeting DNA damage response (DDR) or "synthetic lethality" related pathways, selected from PARP inhibitors, ATR inhibitors, CHK1 inhibitors, ATM inhibitors, DNA-PK inhibitors, WEE1 inhibitors, POLQ inhibitors, CDK12 inhibitors, USP1 inhibitors, PKMYT1 inhibitors, Rad51 inhibitors;
(ii) at least two of D₁, D₂, and D₃ are cytotoxic drugs and are independently selected from drug units targeting topoisomerase and drug units targeting tubulin, for example TOPO1 inhibitors, TOPO2 inhibitors, and tubulin inhibitors;
(iii) at least two or all three of D₁, D₂, and D₃ are tumor-targeted therapeutic drugs, and are independently selected from drug units targeting DNA damage response (DDR) or "synthetic lethality" related pathways, drug units targeting epigenetics, drug units targeting apoptosis-related pathways, drug units targeting cell cycle pathways, drug units targeting transcription factors, and drug units targeting immune activation pathway-related targets, comprising: inhibitors of EFGR pathway-related targets; Ras-Raf-MAPK pathway inhibitors; PI3K/AKT/mTOR pathway inhibitors; cell cycle pathway inhibitors; cGAS-STING signaling pathway agonists; estrogen receptor antagonists; androgen receptor antagonists; glucocorticoid receptor modulators; autophagy inhibitors; FAK inhibitors; Smo inhibitors; BTK inhibitors; PDE4 inhibitors; Lck inhibitors; PLK1 inhibitors; TLR7/8 modulators; N-myristoyltransferase (NMT) inhibitors;
(iv) one of D₁, D₂, and D₃ is a cytotoxic drug, the cytotoxic drug is selected from drug units targeting topoisomerase and drug units targeting tubulin, for example a TOPO1 inhibitor or a tubulin inhibitor, and the other one or two are tumor-targeted therapeutic drugs, the tumor-targeted therapeutic drugs are selected from drug units targeting DNA damage response (DDR) or "synthetic lethality" related pathways, drug units targeting epigenetics, drug units targeting apoptosis-related pathways, drug units targeting cell cycle pathways, drug units targeting transcription factors, and drug units targeting immune activation pathway-related targets, comprising: inhibitors of EFGR pathway-related targets; Ras-Raf-MAPK pathway inhibitors; PI3K/AKT/mTOR pathway inhibitors; cell cycle pathway inhibitors; cGAS-STING signaling pathway agonists; estrogen receptor antagonists; androgen receptor antagonists; glucocorticoid receptor modulators; autophagy inhibitors; FAK inhibitors; Smo inhibitors; BTK inhibitors; PDE4 inhibitors; Lck inhibitors; PLK1 inhibitors; TLR7/8 modulators; N-myristoyltransferase (NMT) inhibitors;
more preferably, D₁, D₂, and D₃ are selected from the combinations shown in Table A.

26. The compound, or a tautomer, a mesomer, a racemate, an enantiomer, or a diastereomer thereof, or a deuterated compound form thereof, or a mixture form thereof, or a pharmaceutically acceptable salt, prodrug, or solvate thereof, according to any one of claims 14 to 25, wherein the compound is selected from formulas **B₁ to B190:**

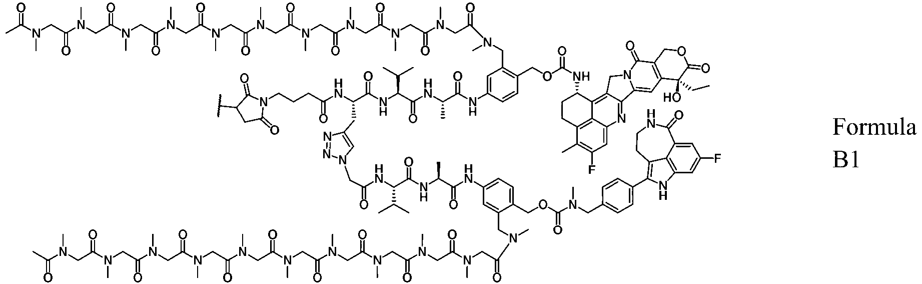

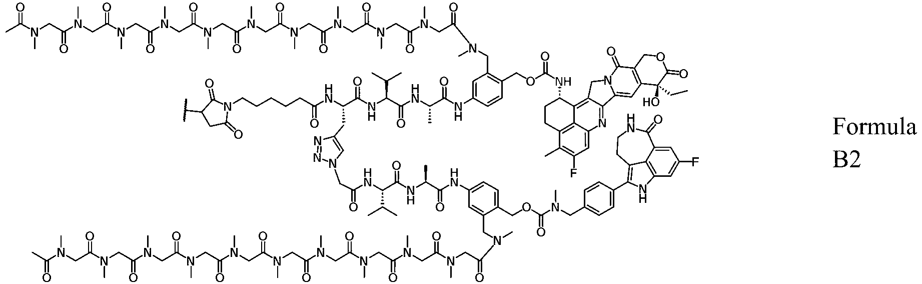

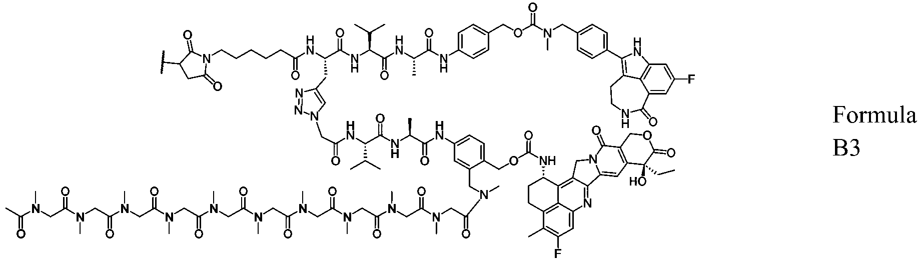

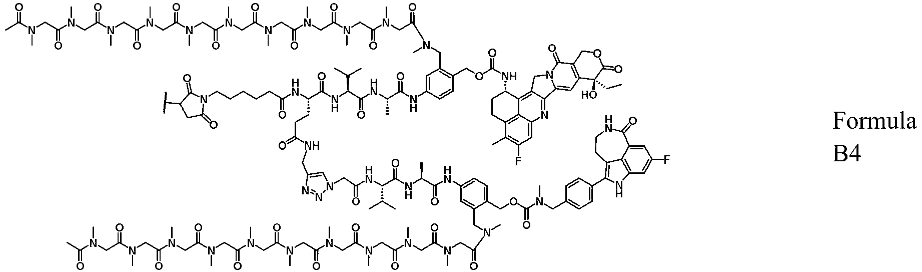

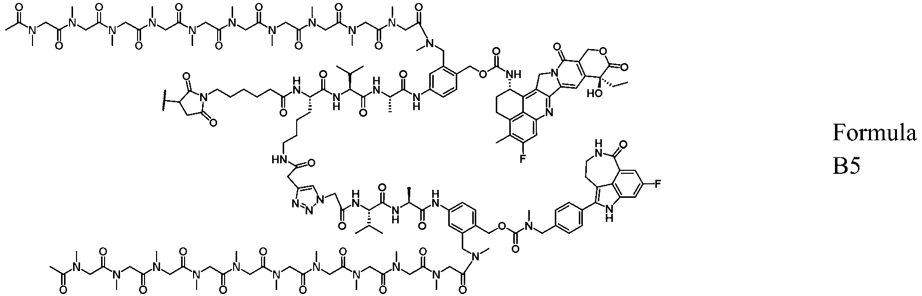

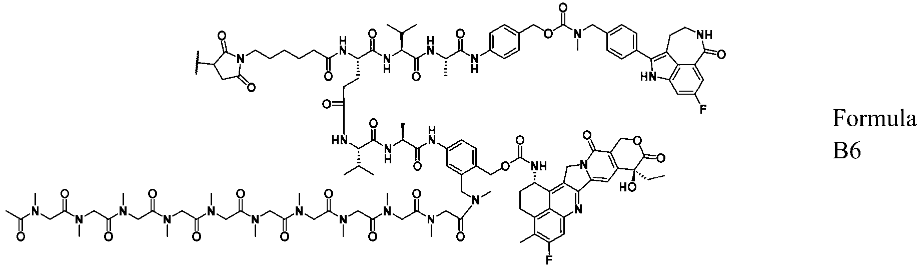

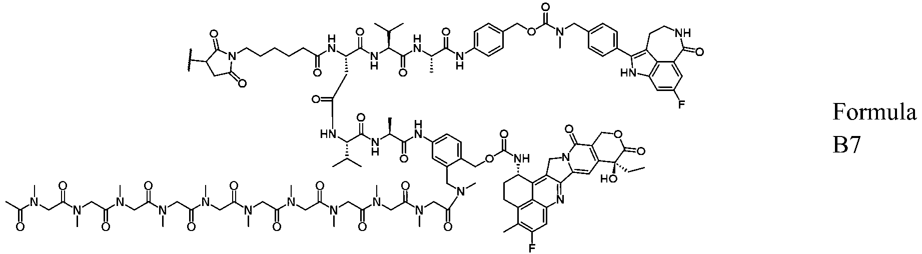

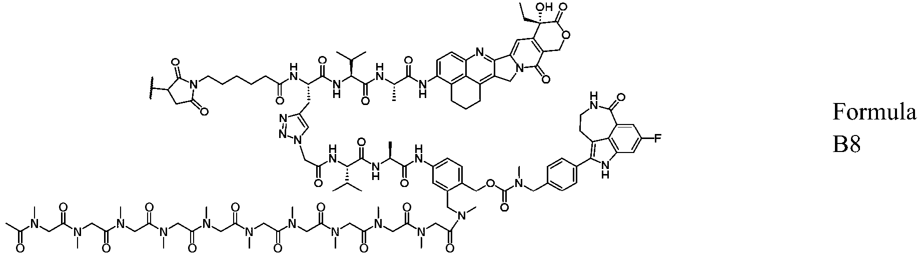

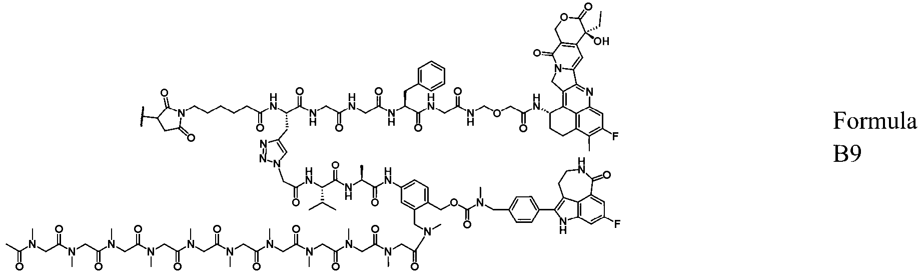

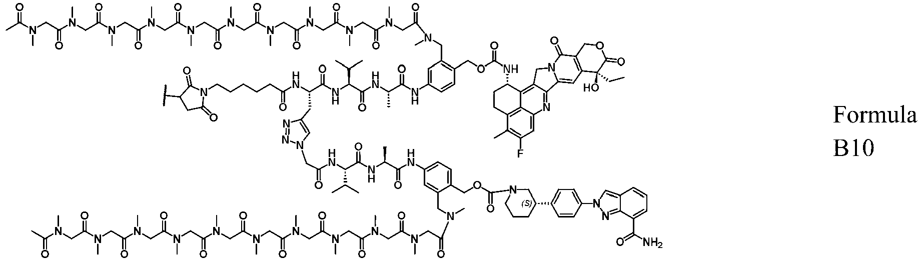

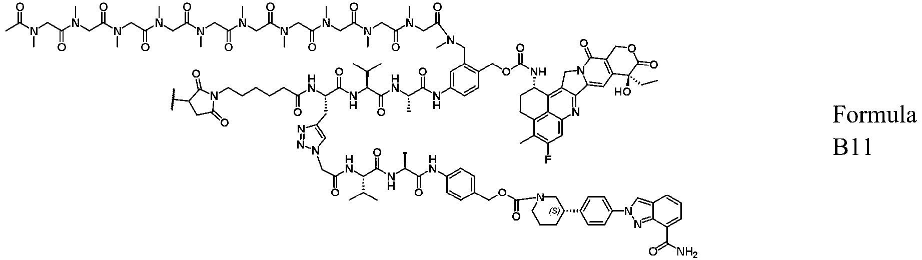

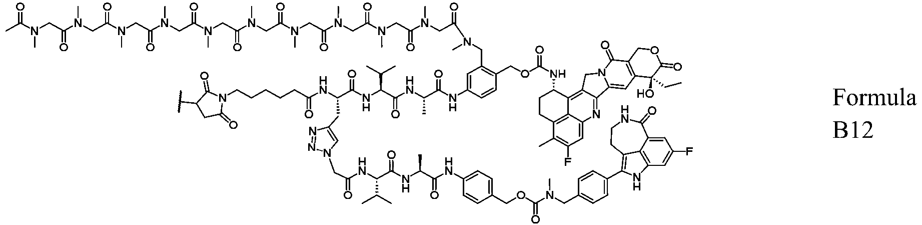

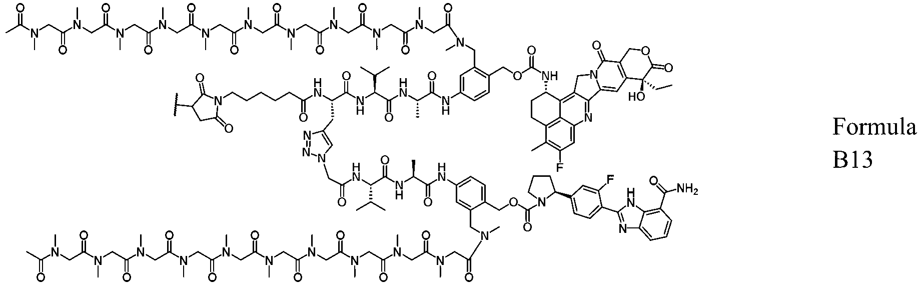

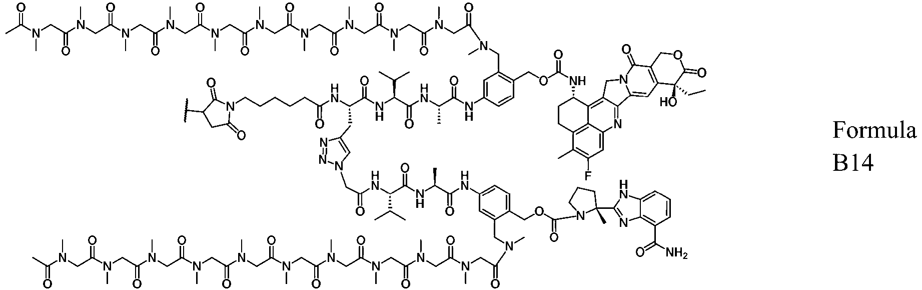

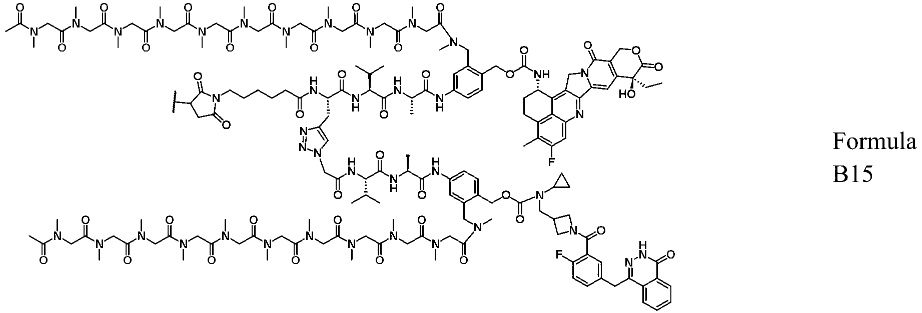

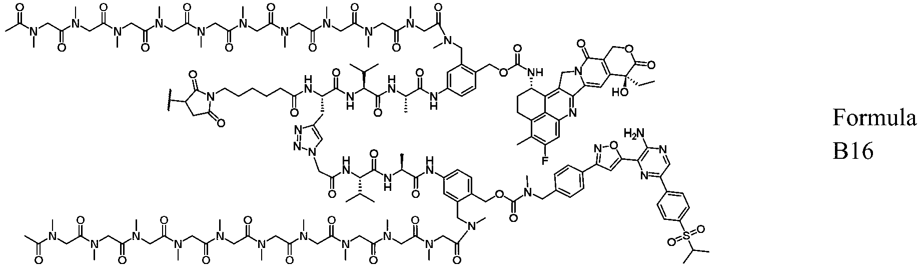

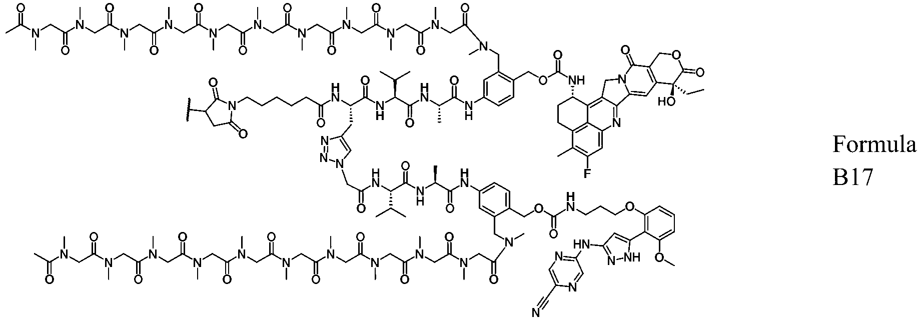

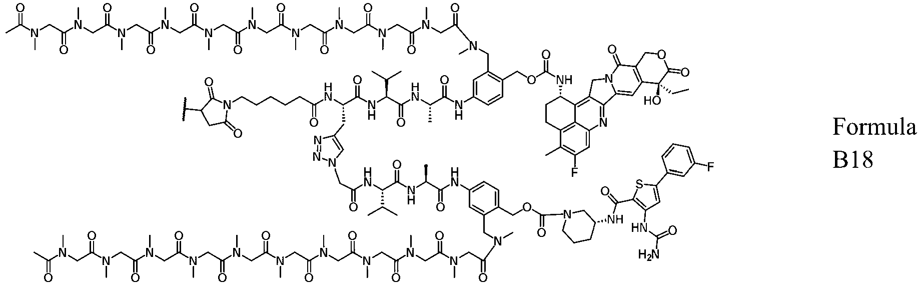

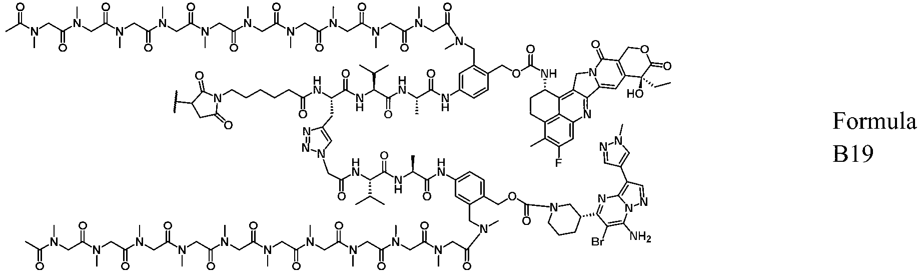

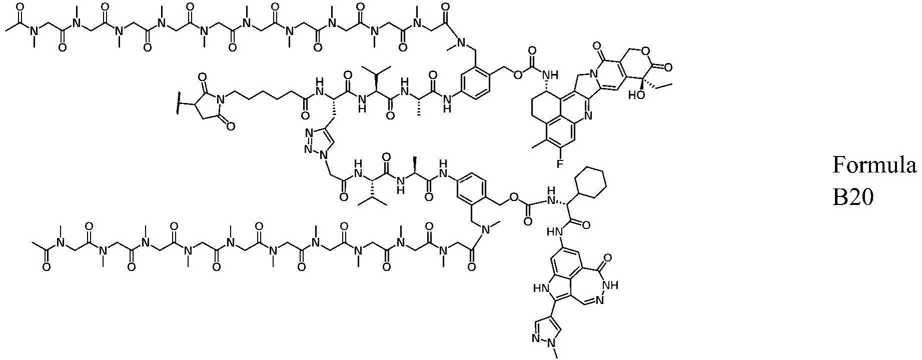

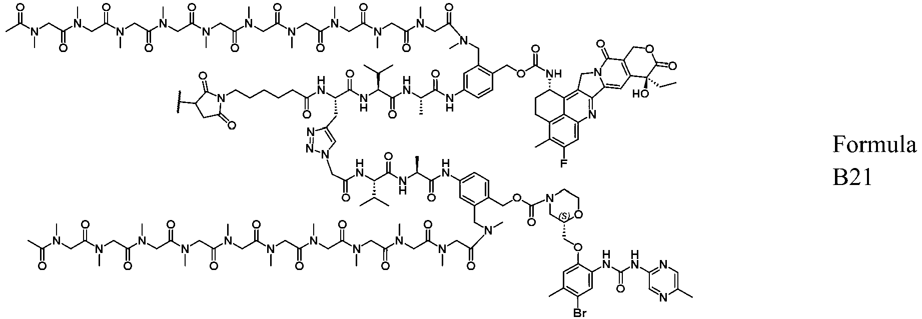

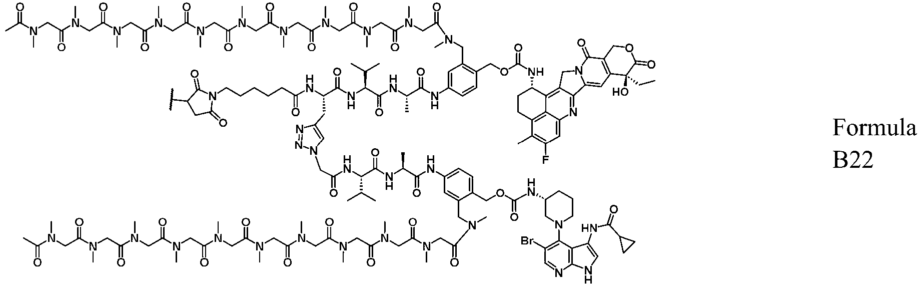

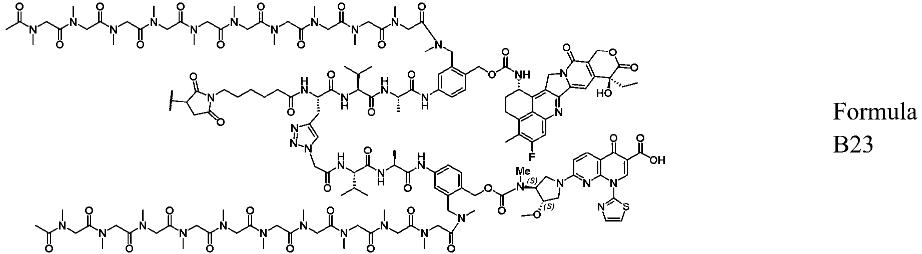

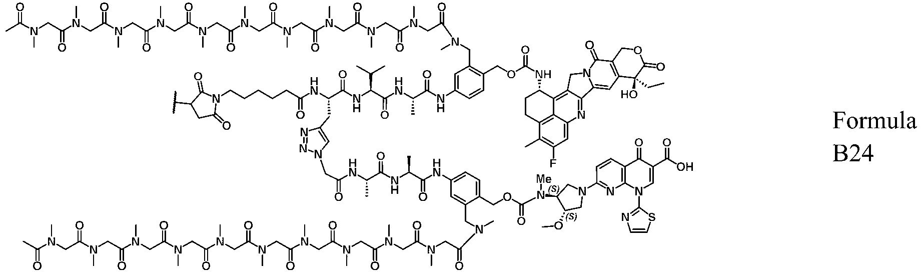

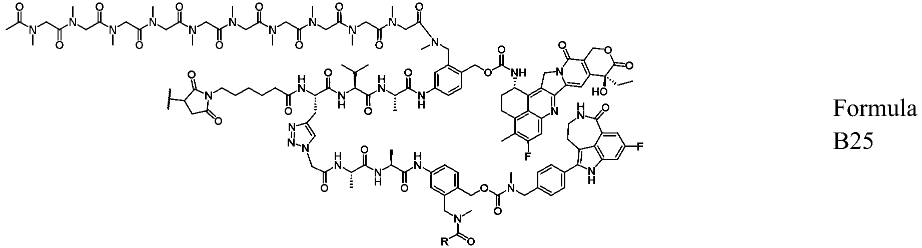

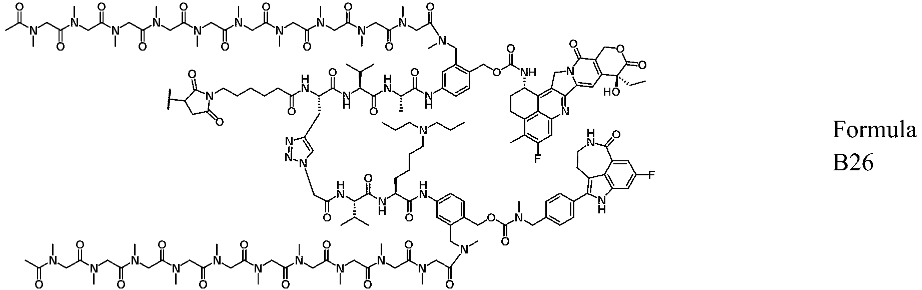

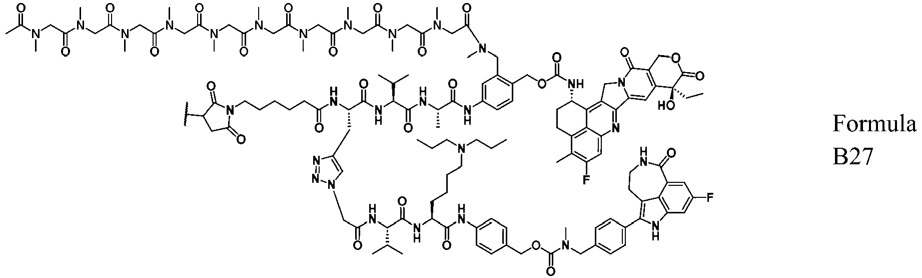

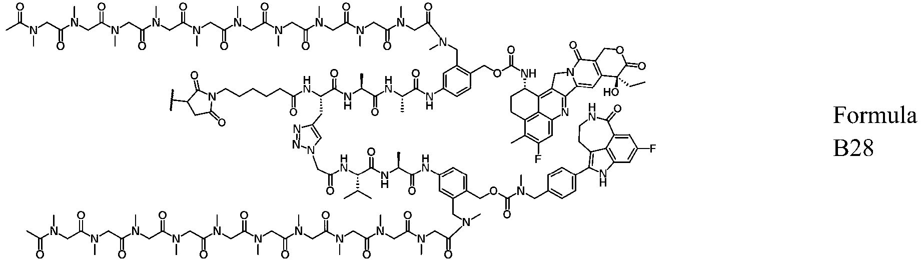

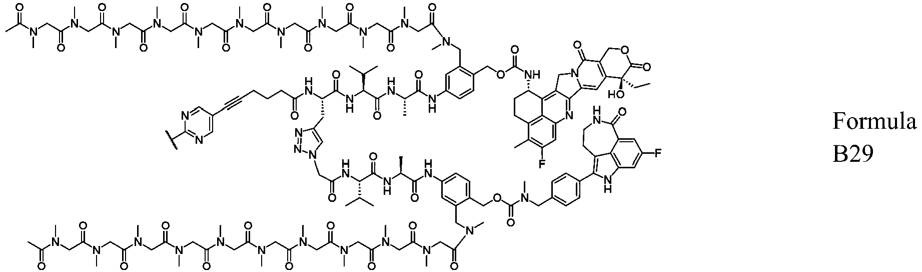

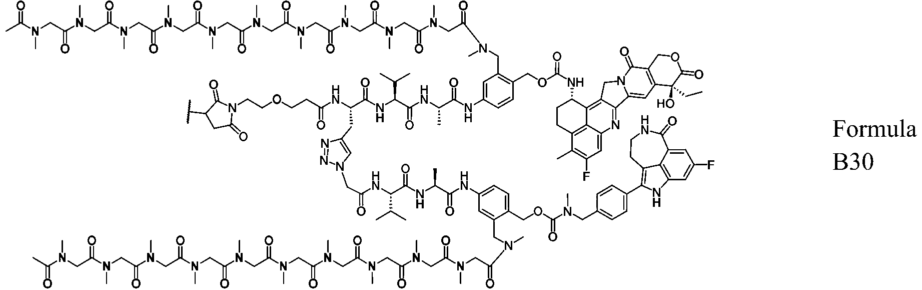

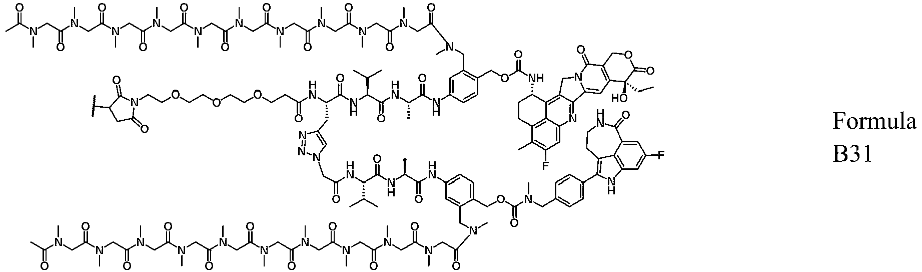

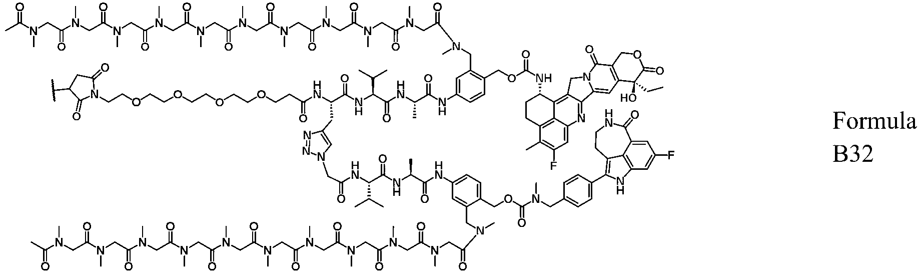

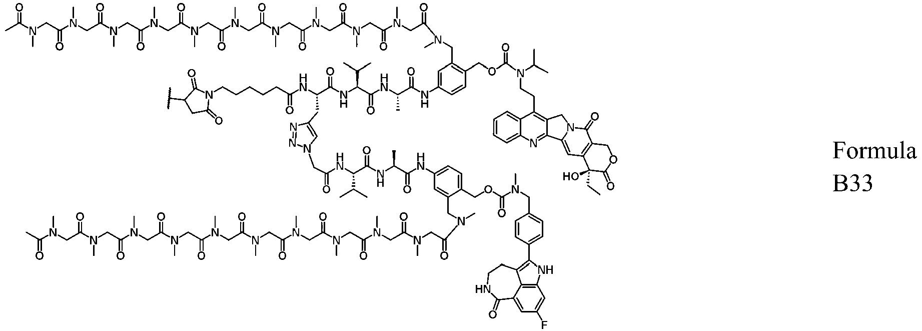

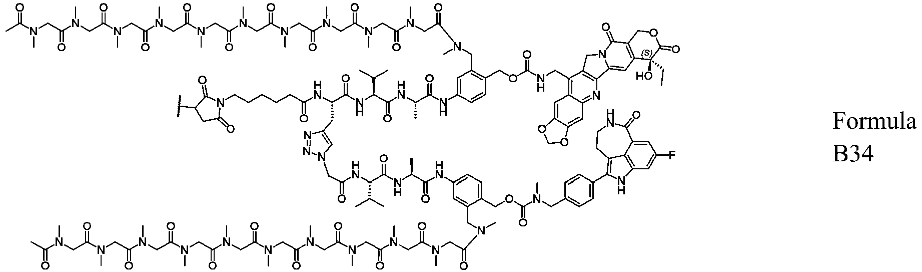

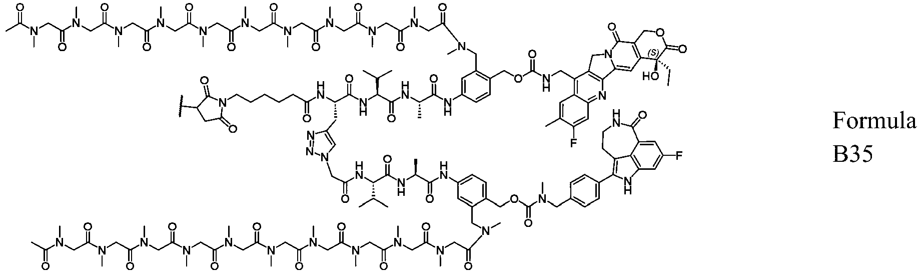

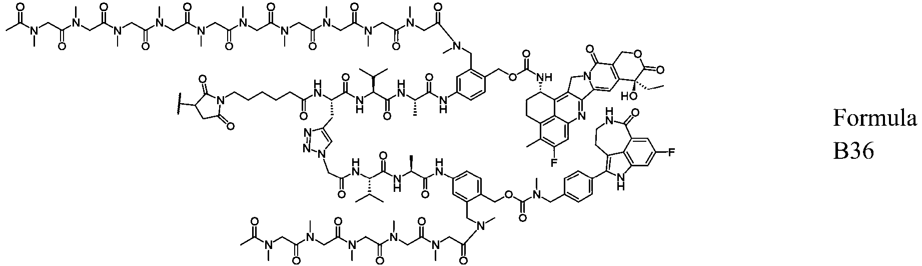

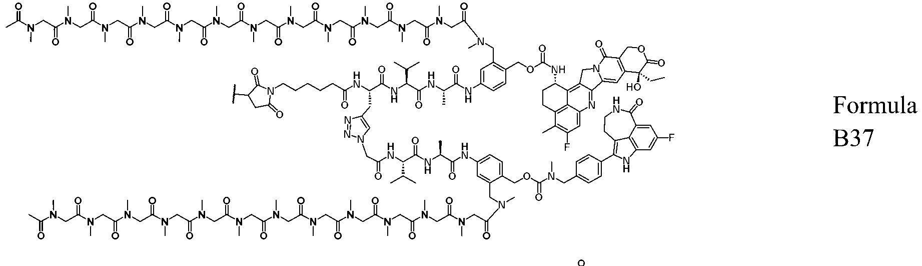

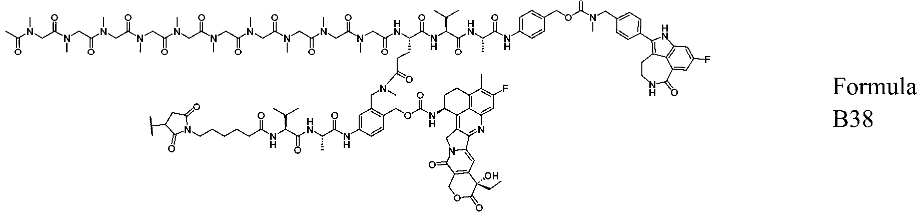

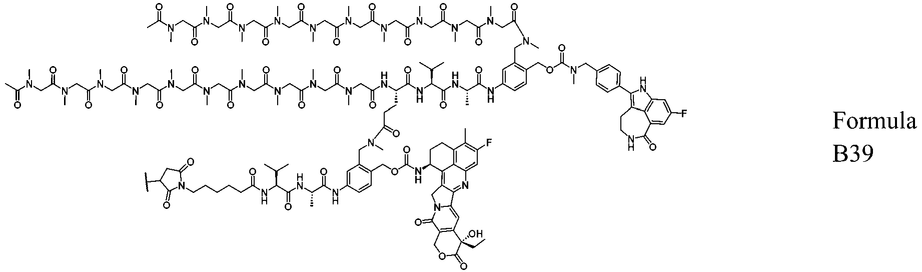

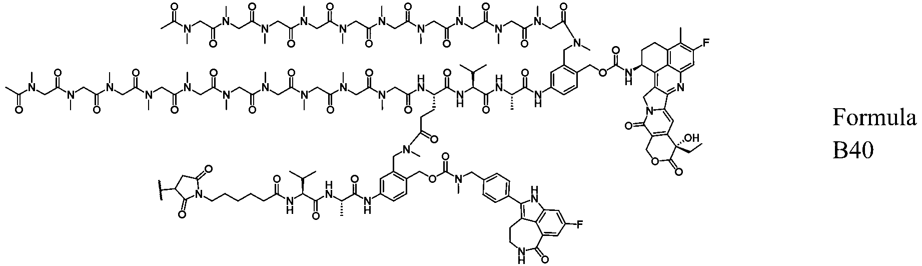

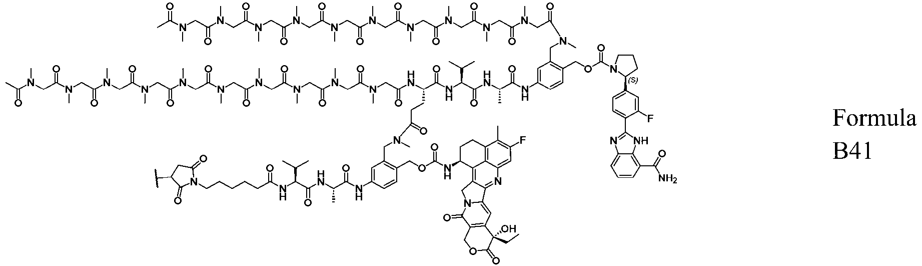

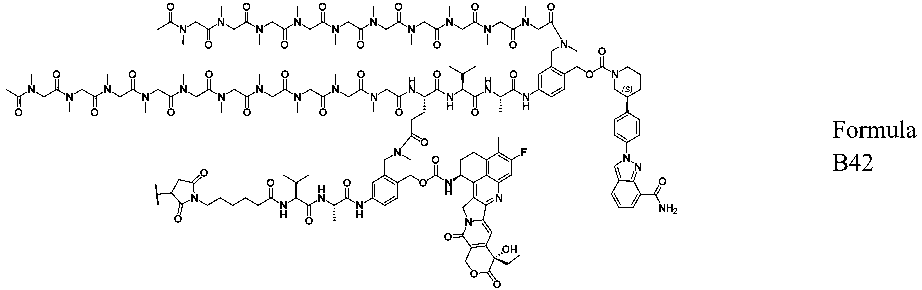

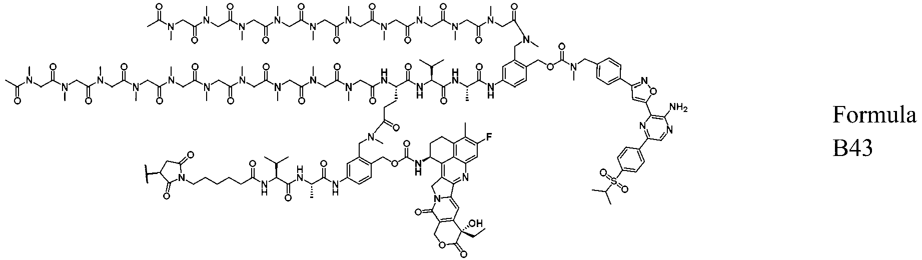

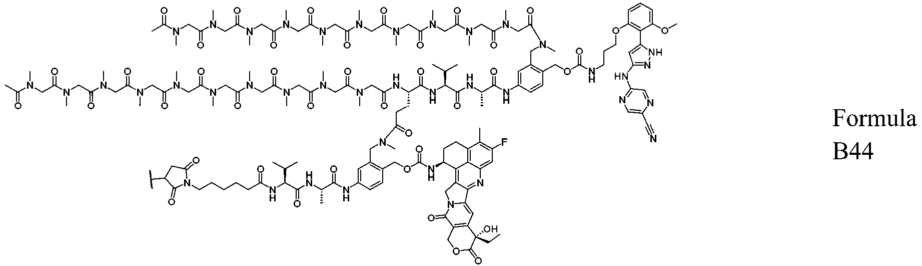

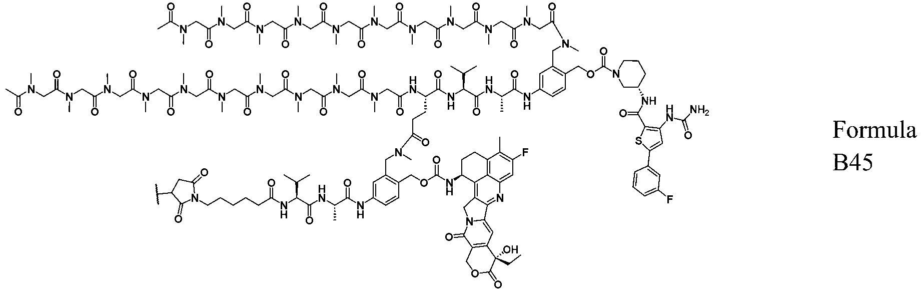

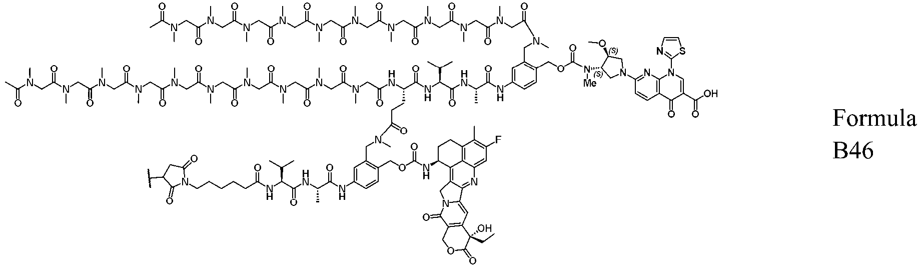

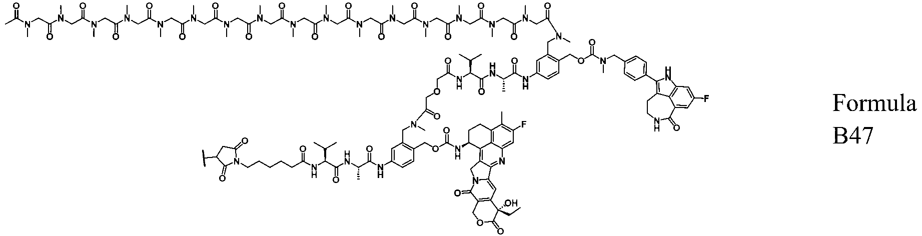

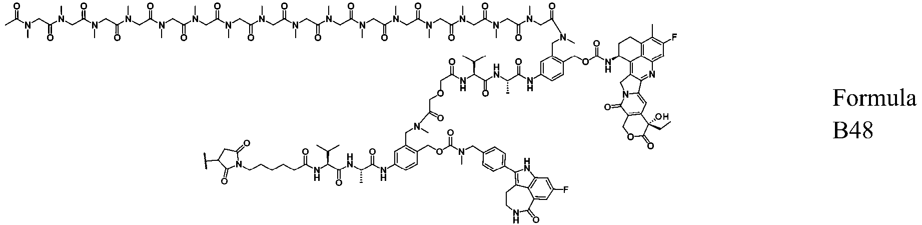

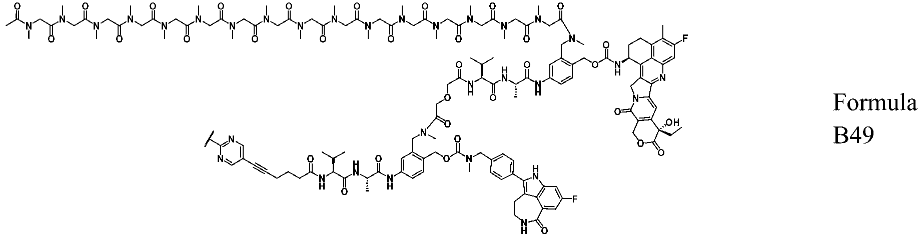

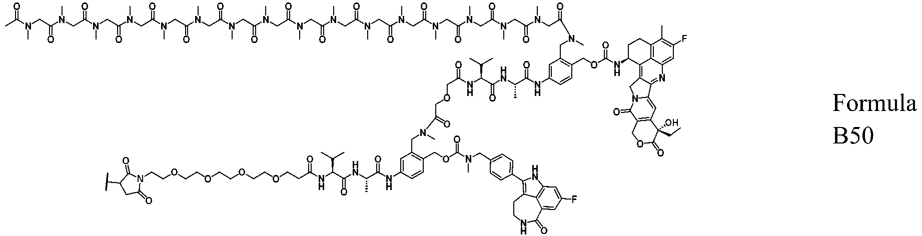

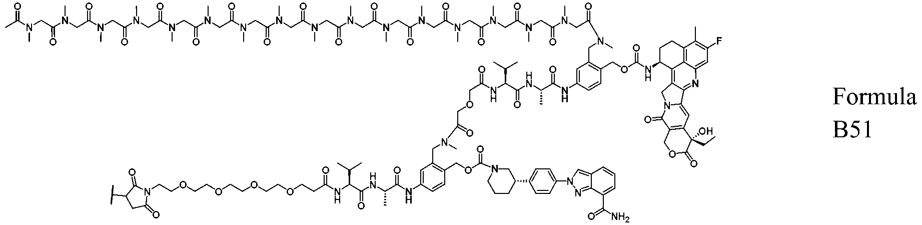

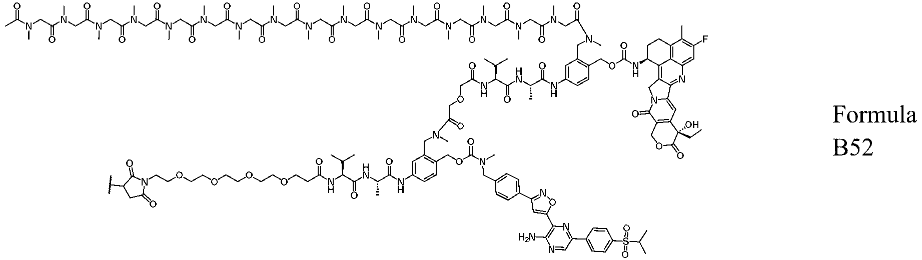

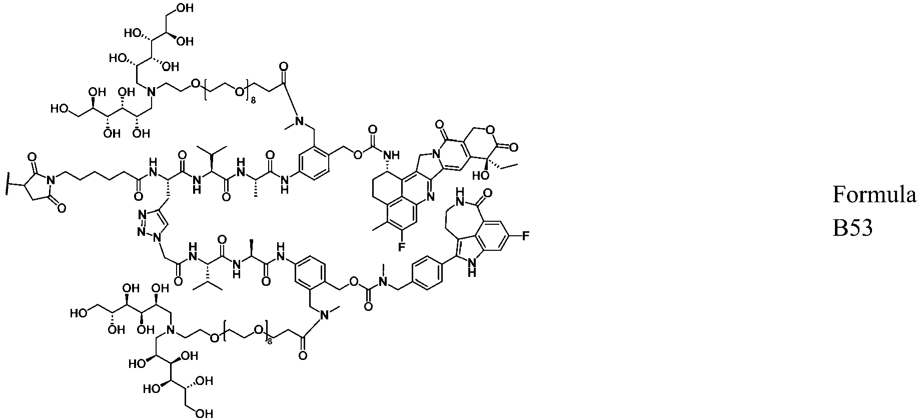

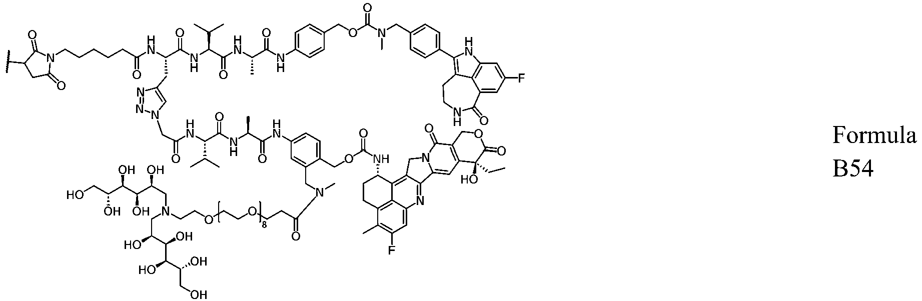

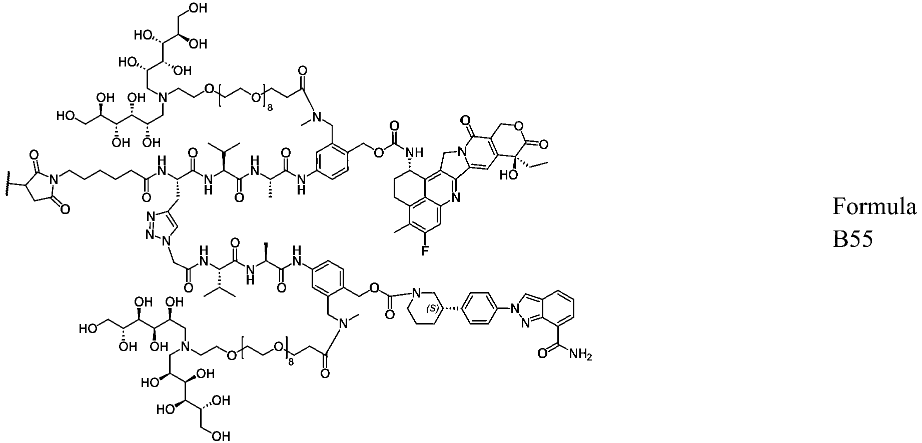

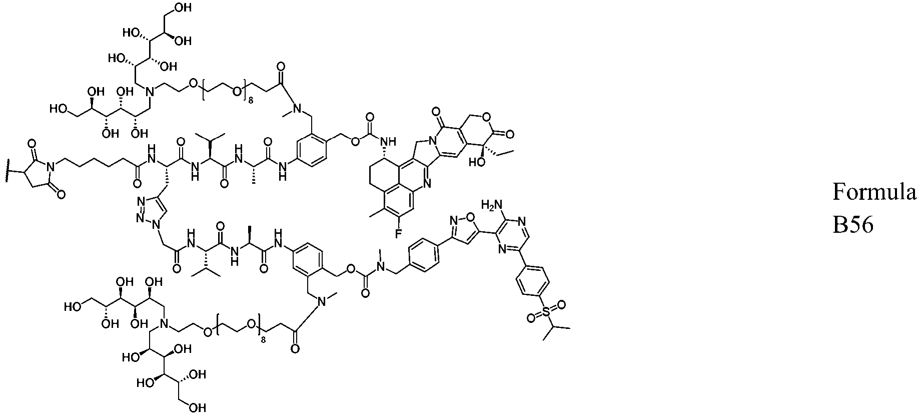

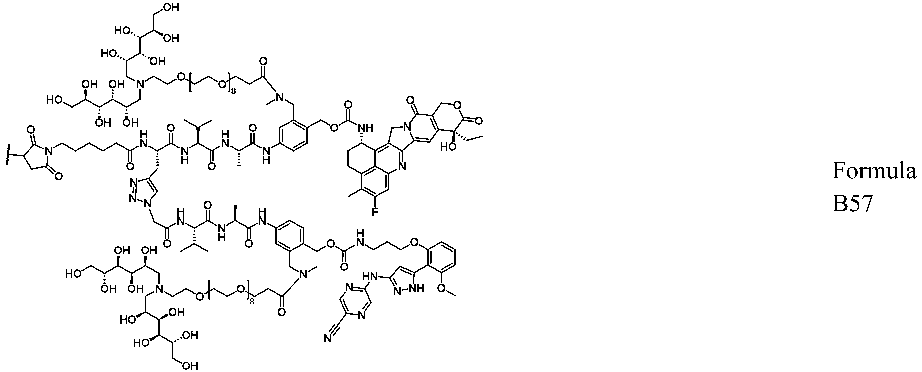

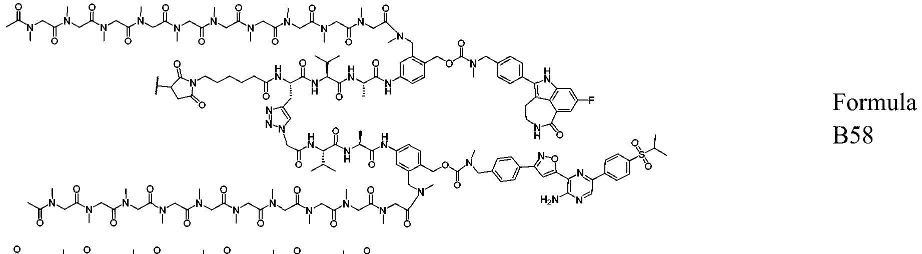

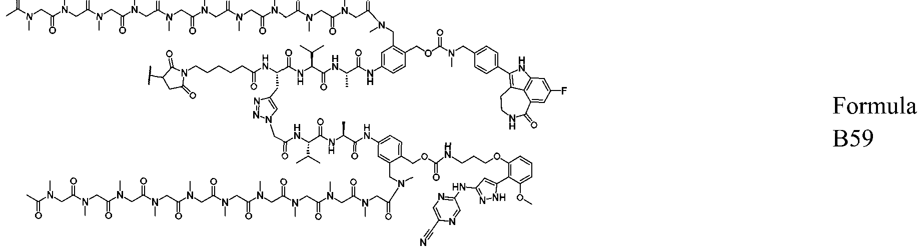

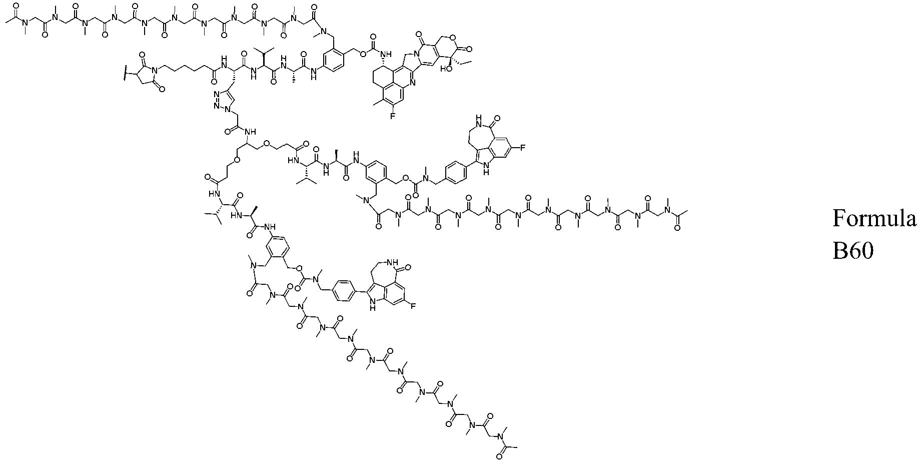

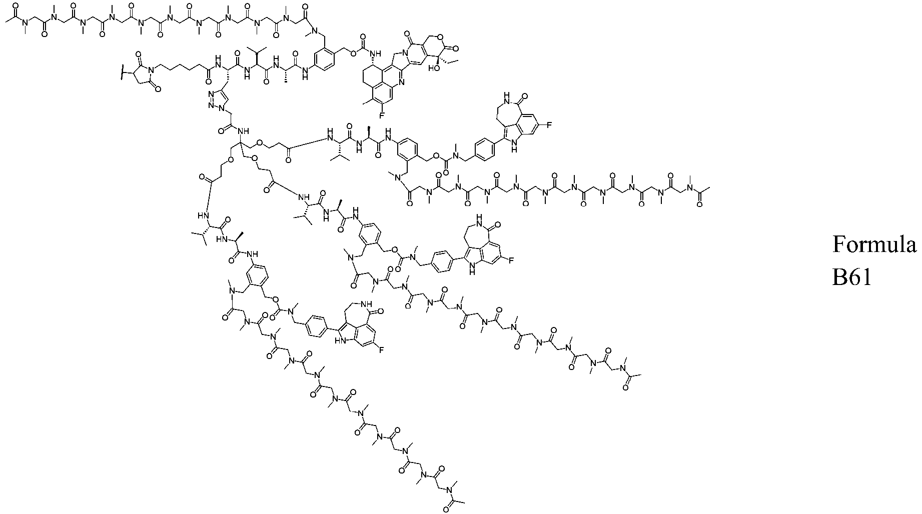

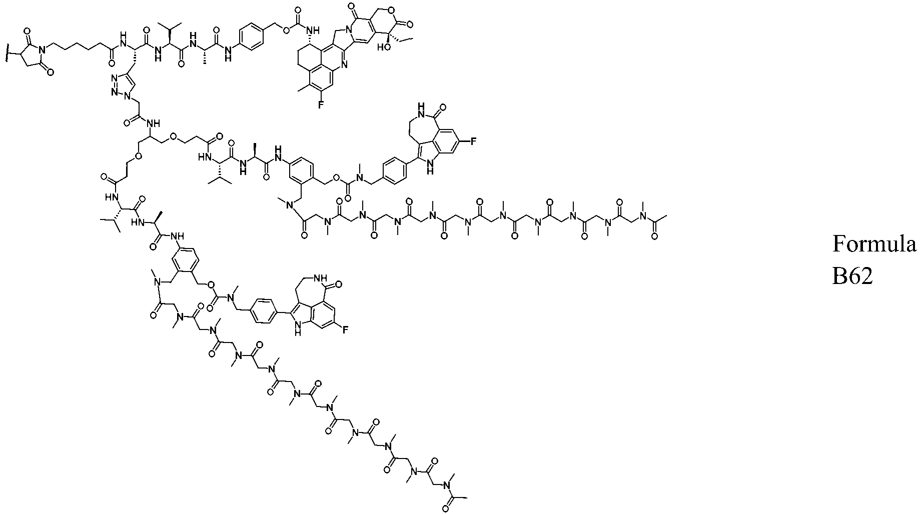

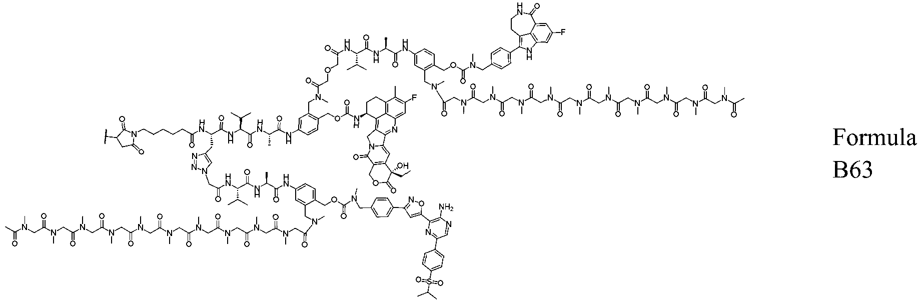

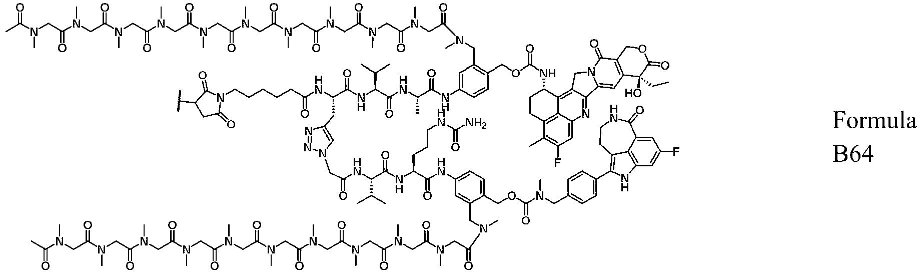

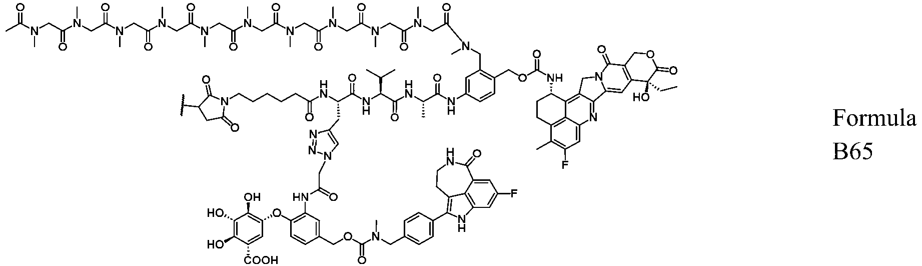

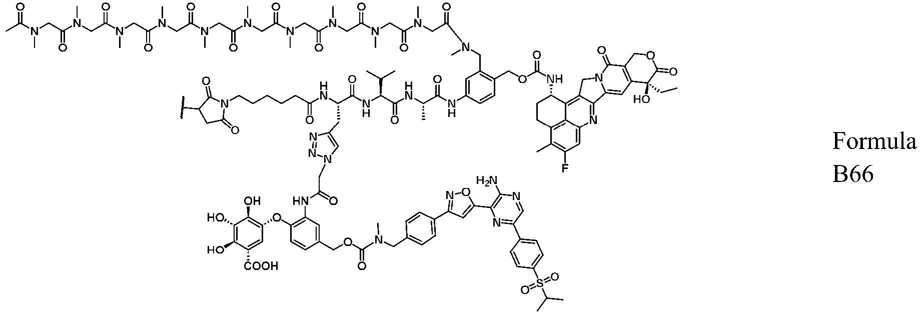

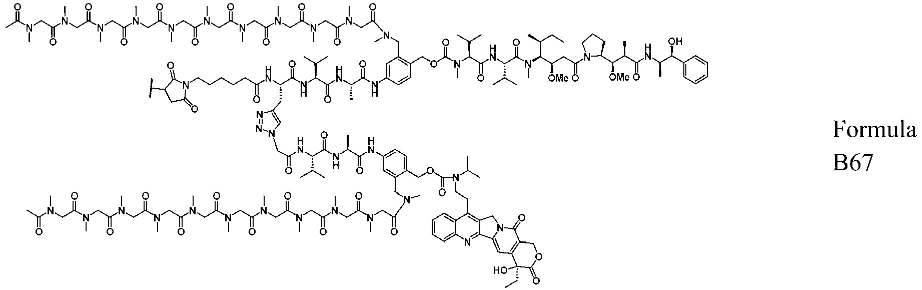

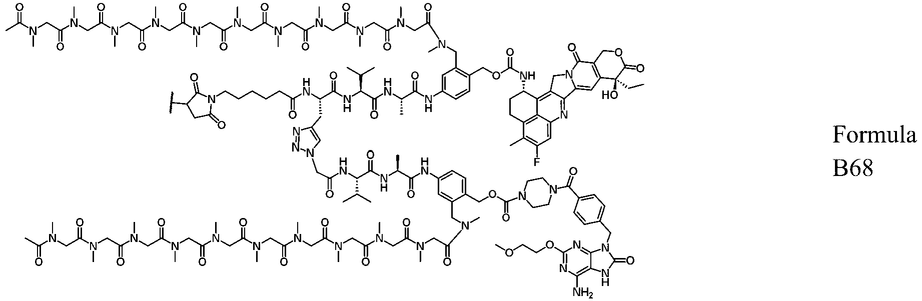

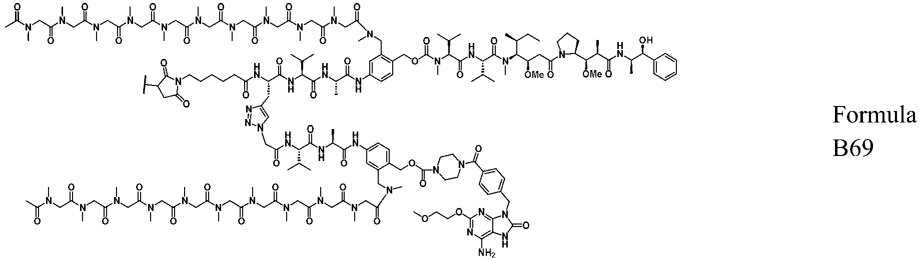

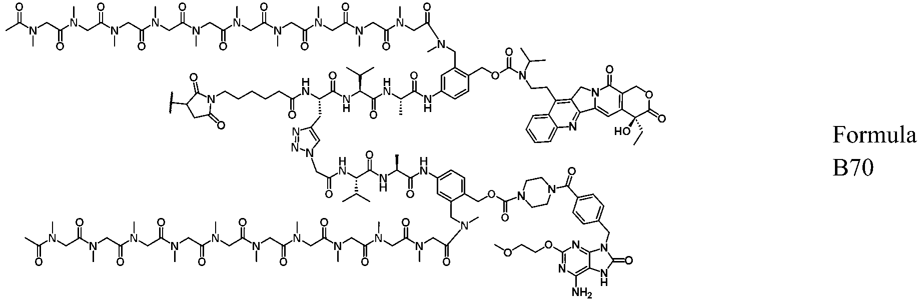

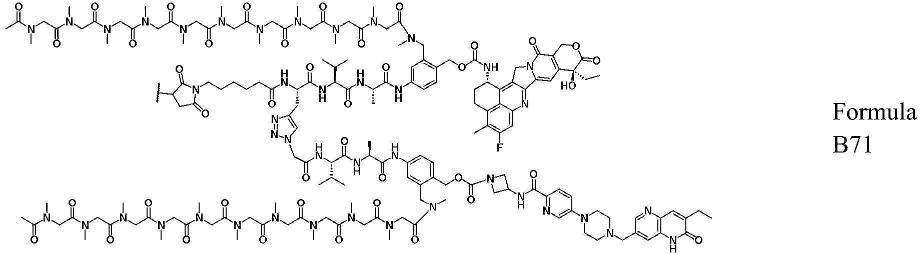

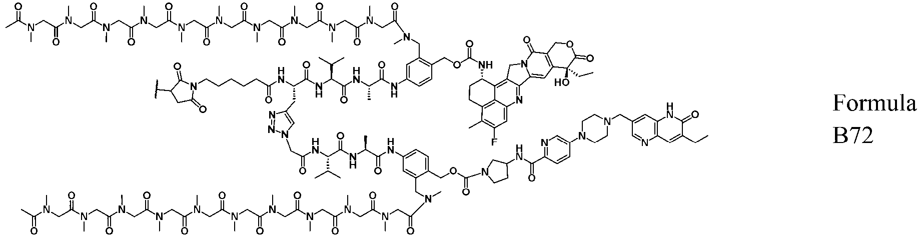

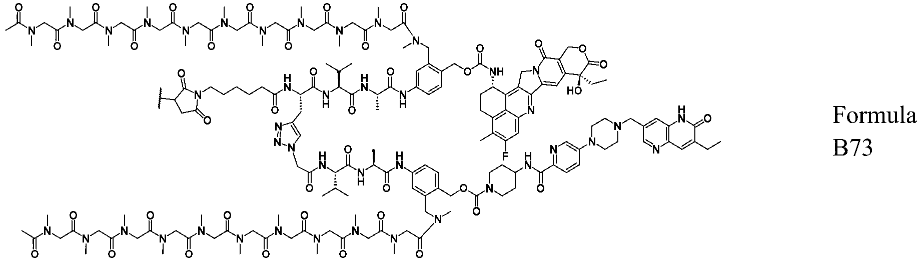

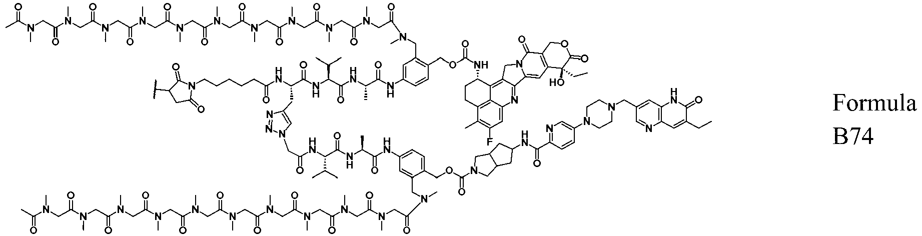

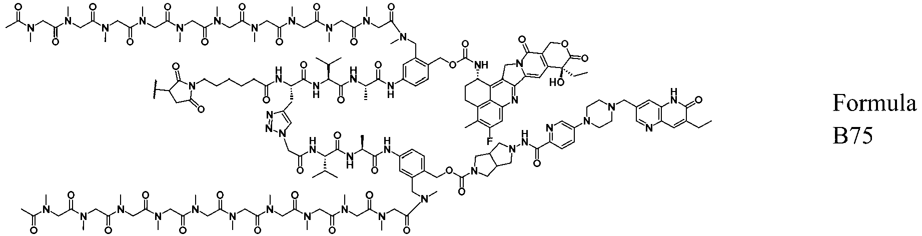

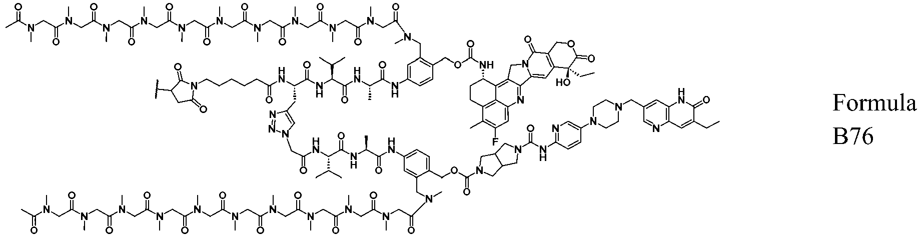

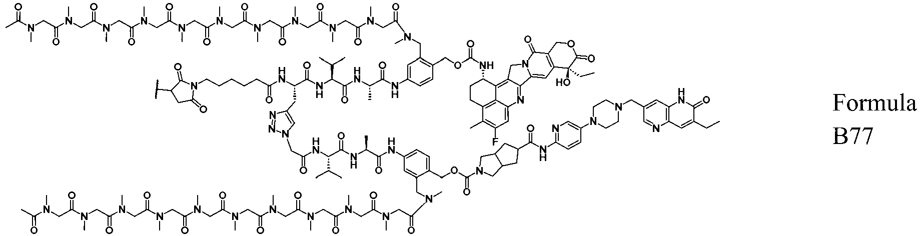

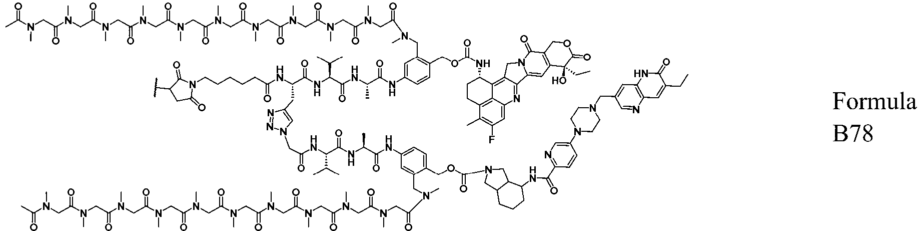

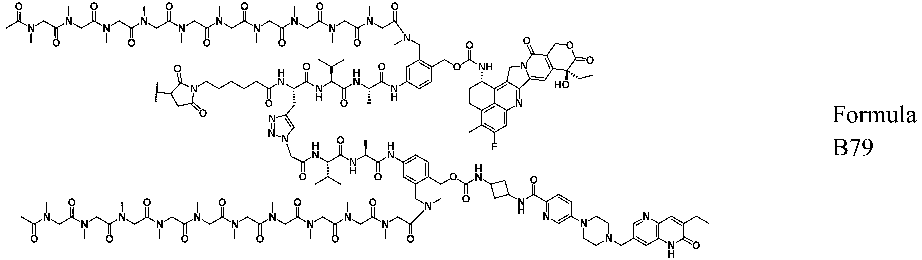

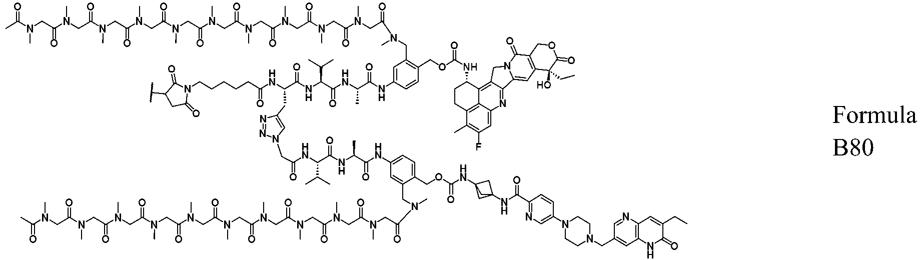

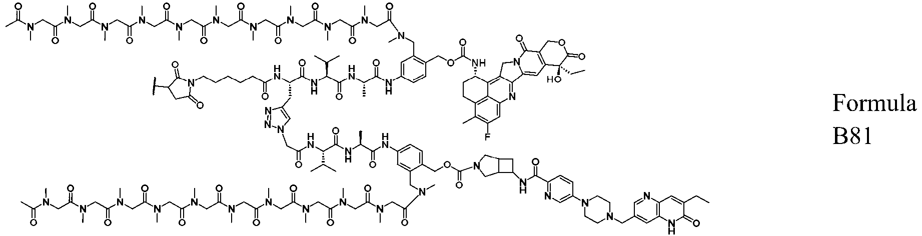

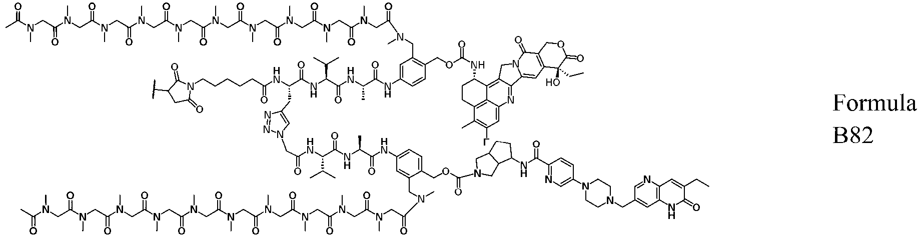

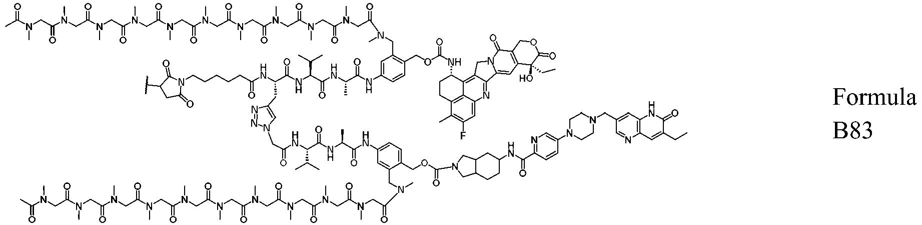

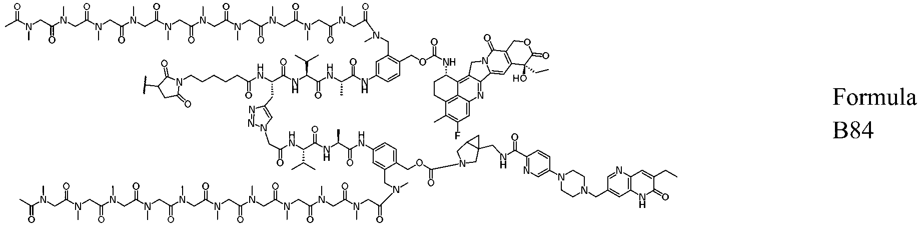

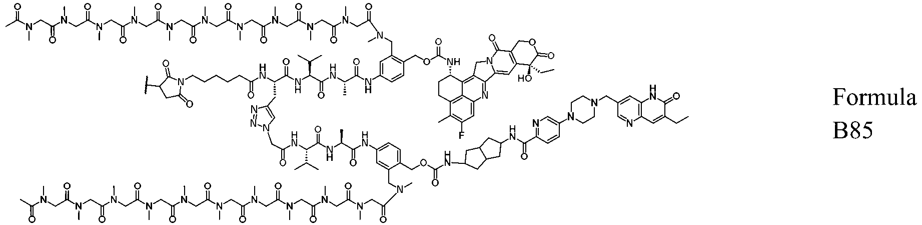

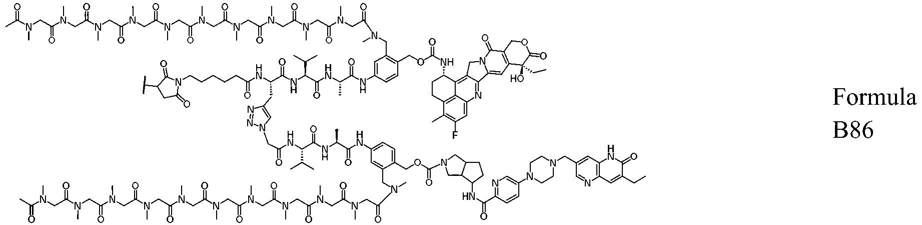

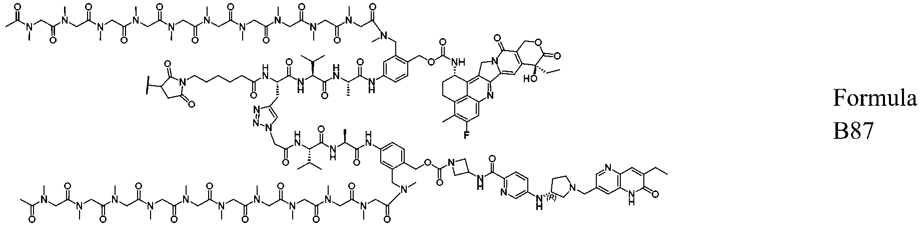

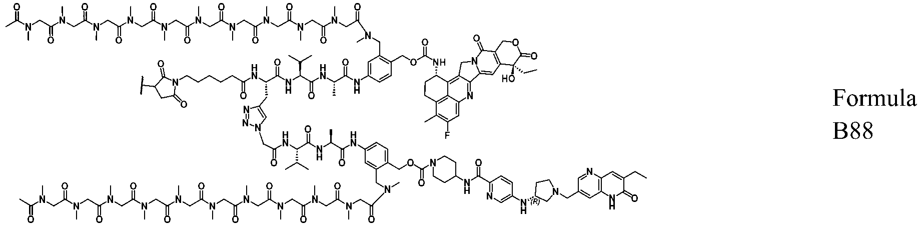

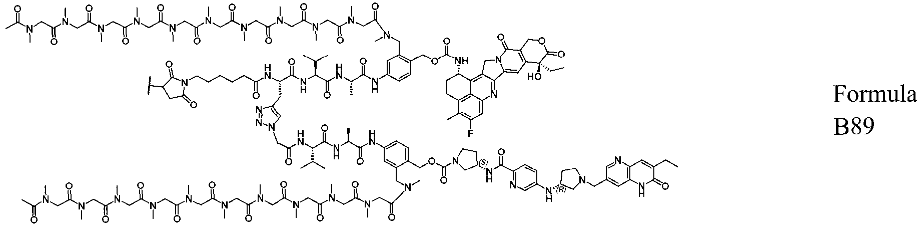

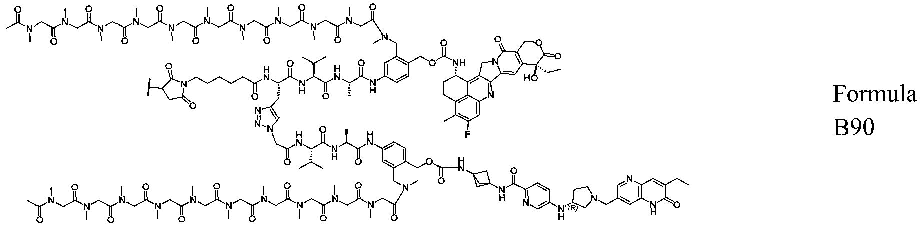

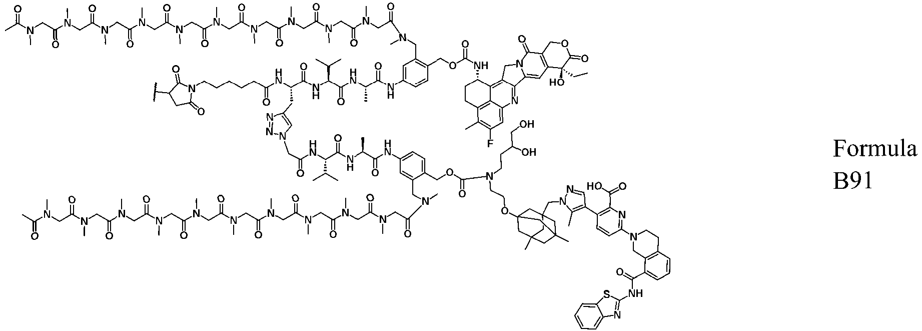

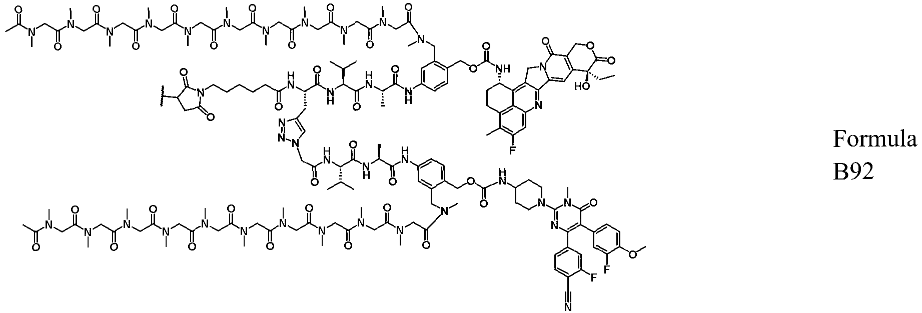

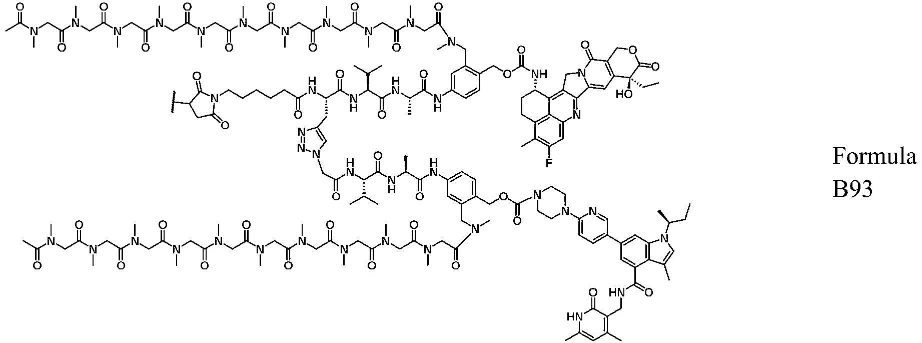

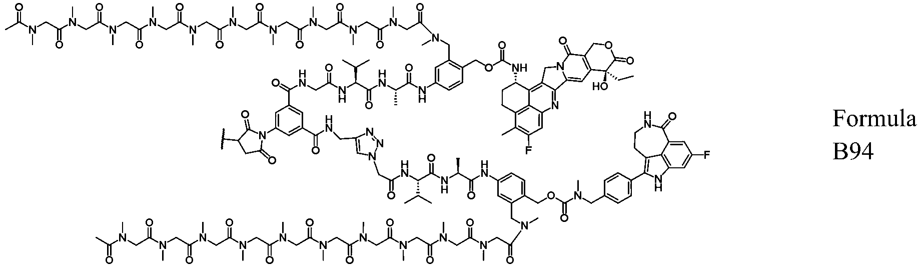

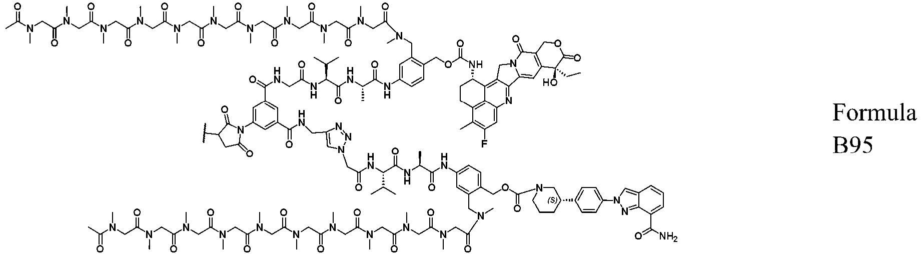

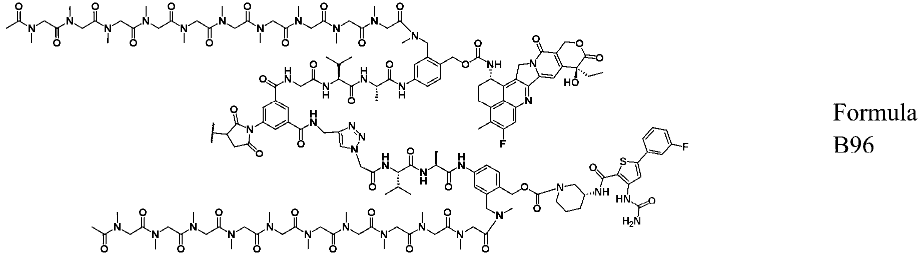

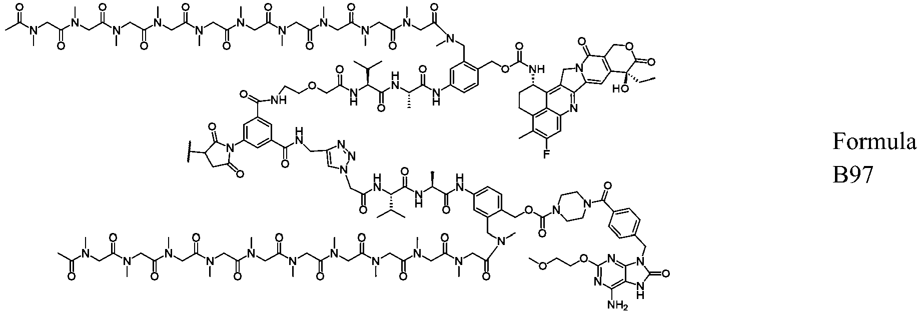

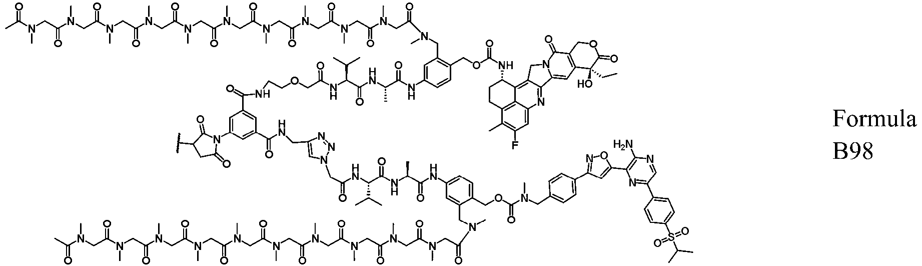

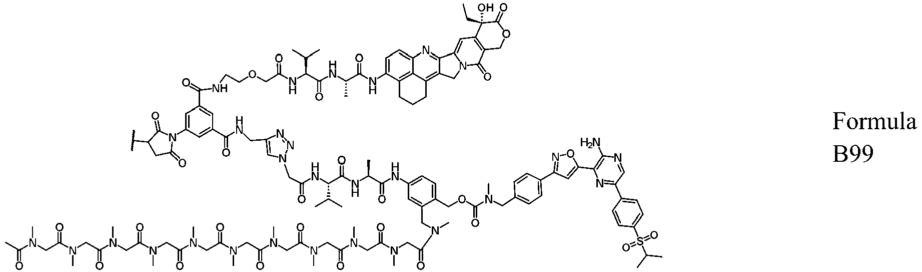

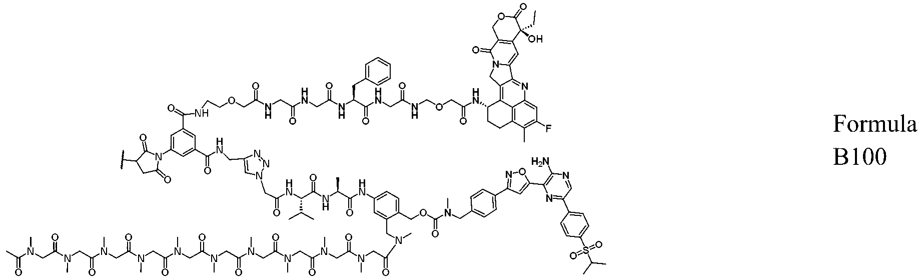

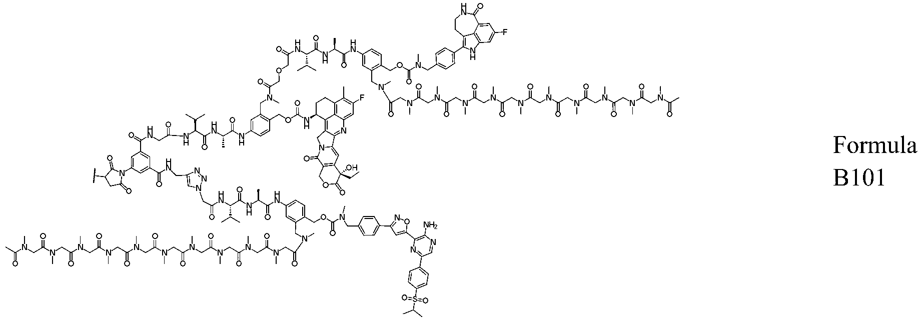

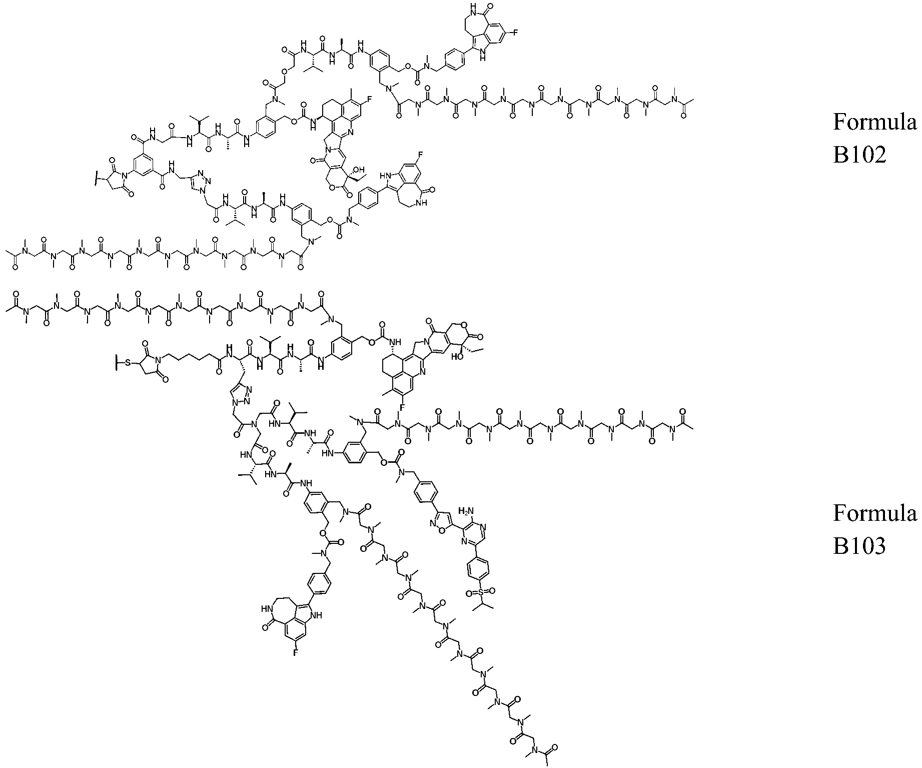

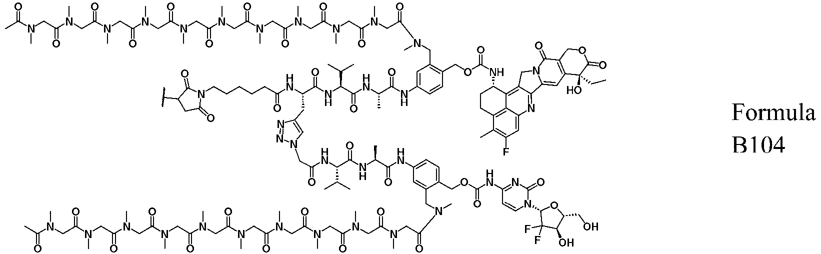

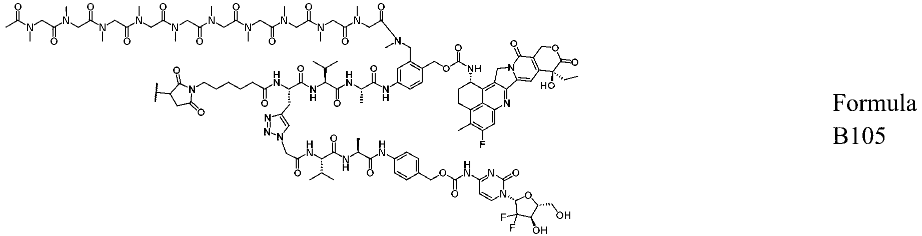

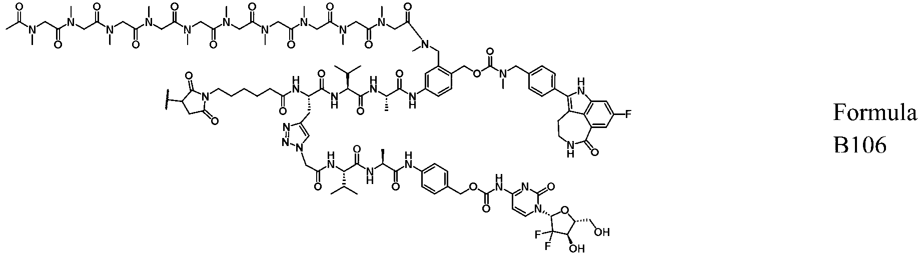

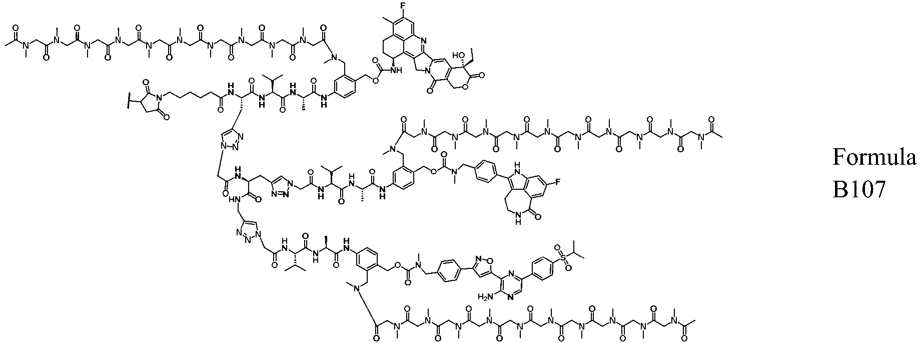

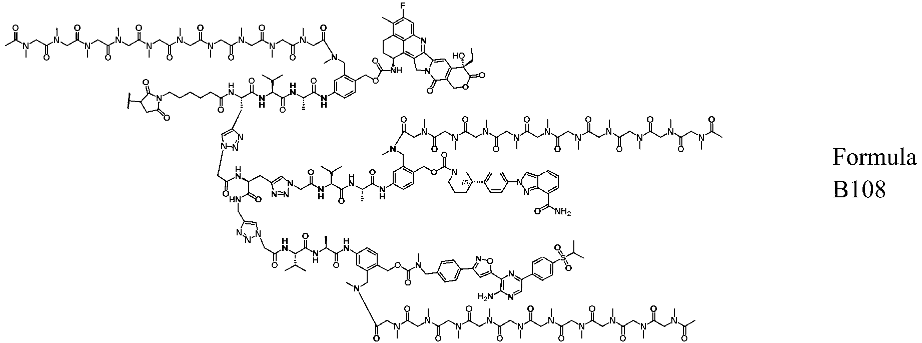

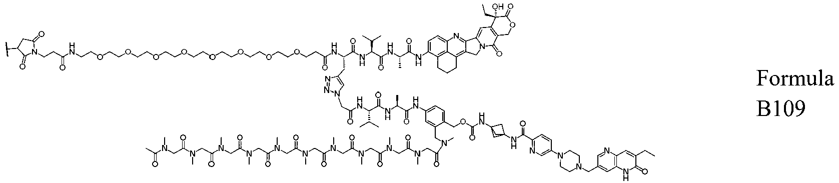

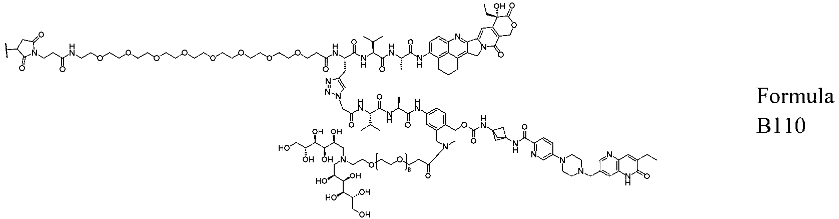

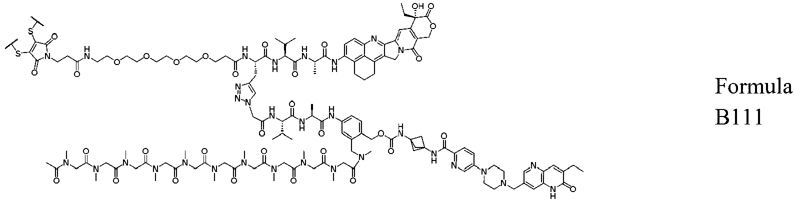

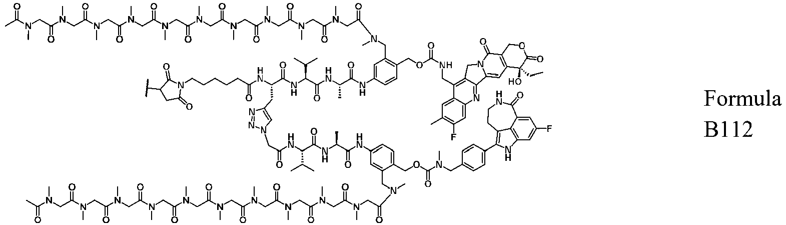

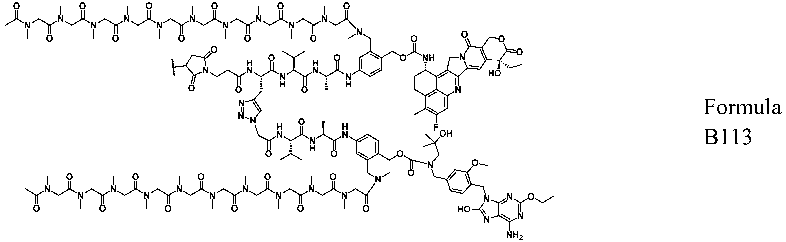

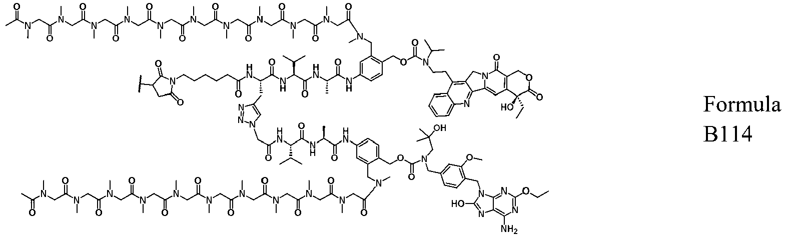

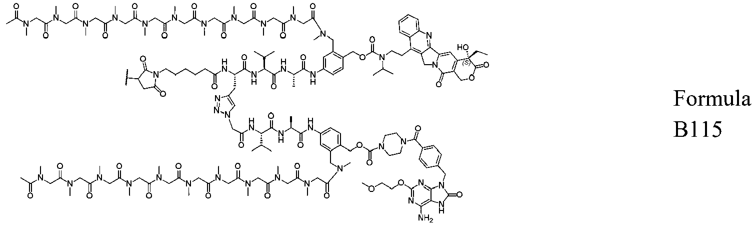

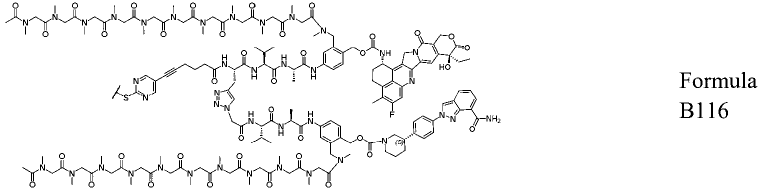

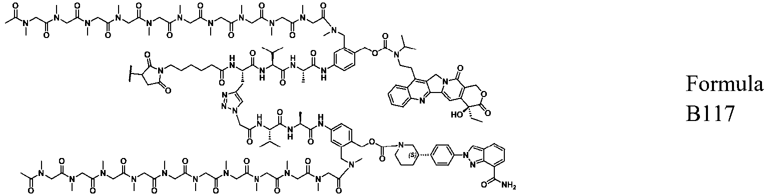

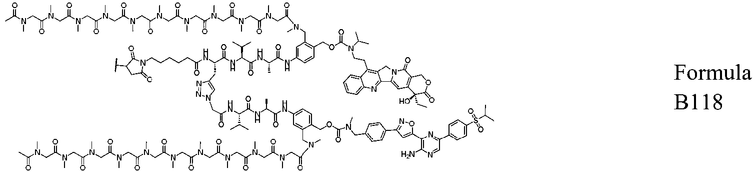

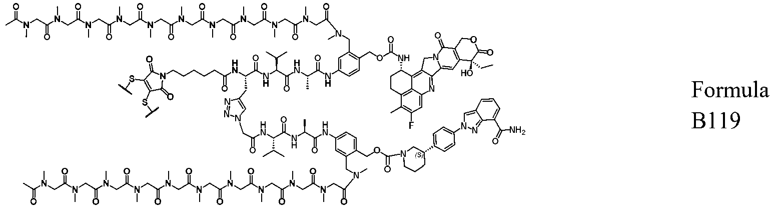

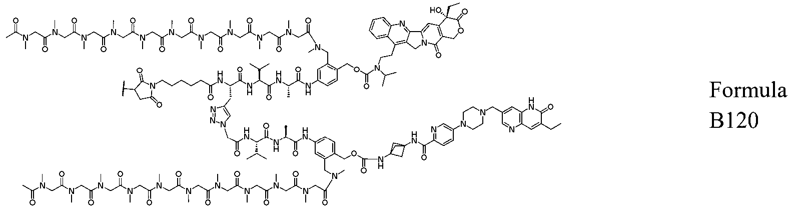

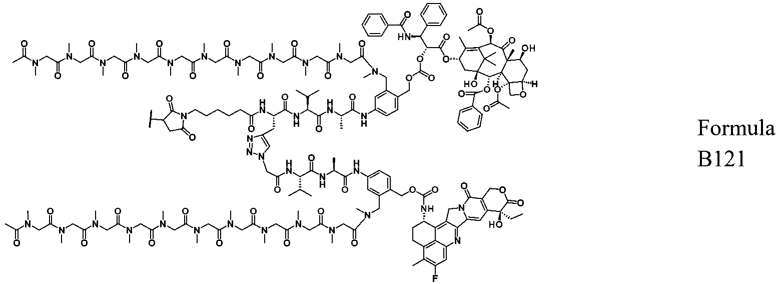

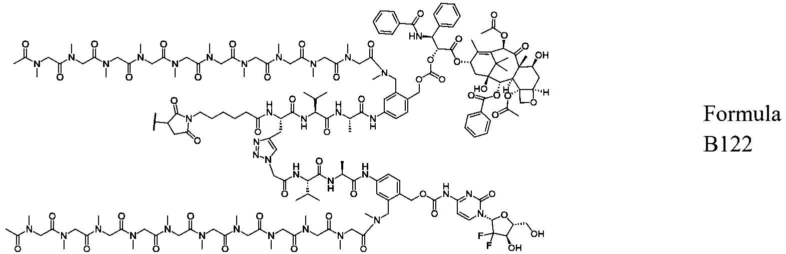

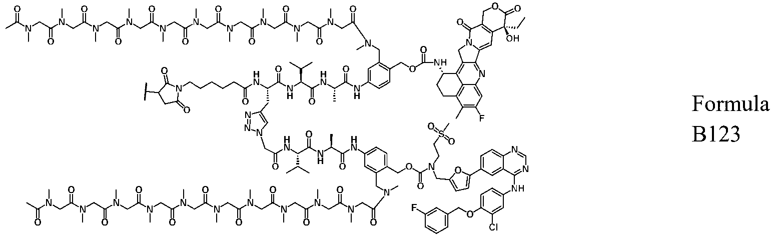

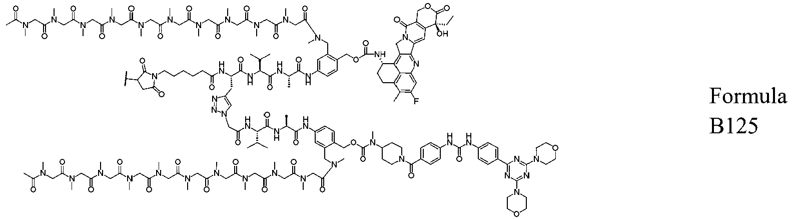

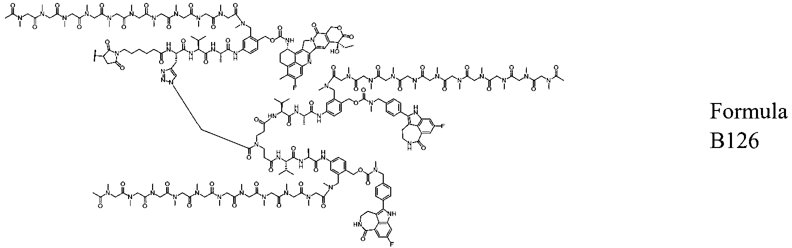

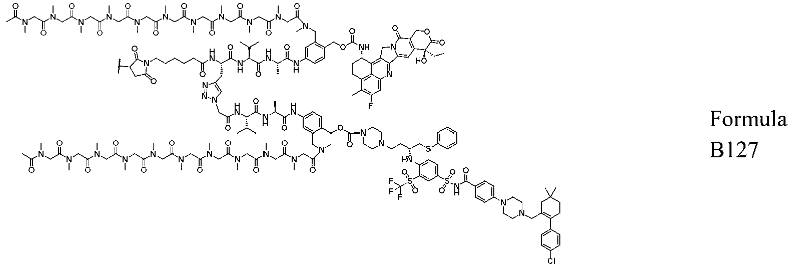

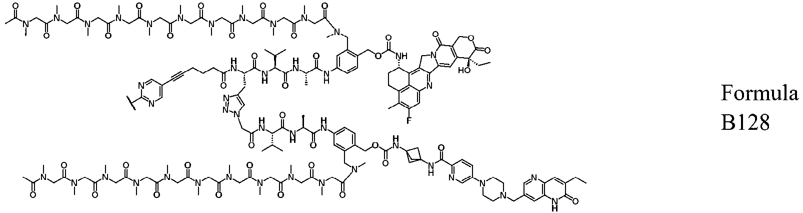

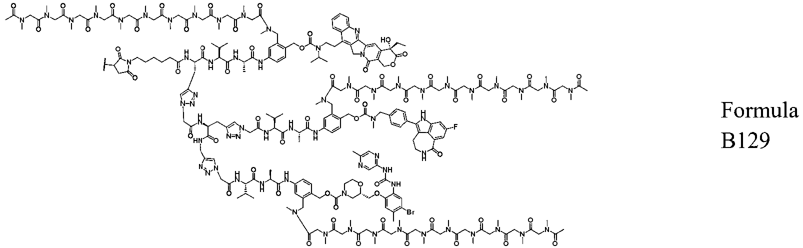

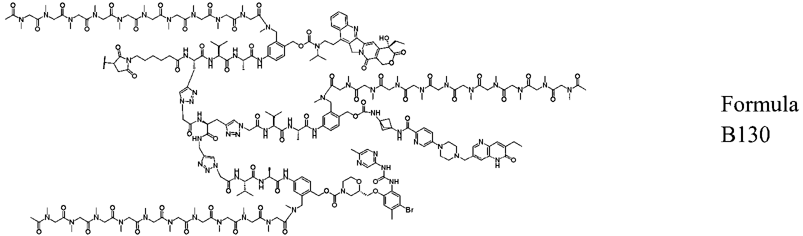

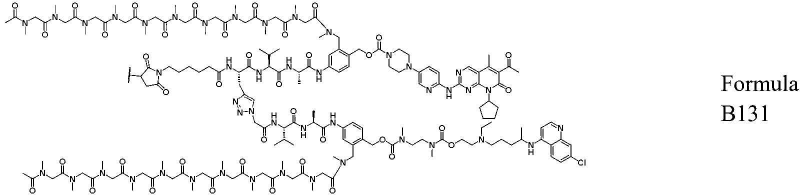

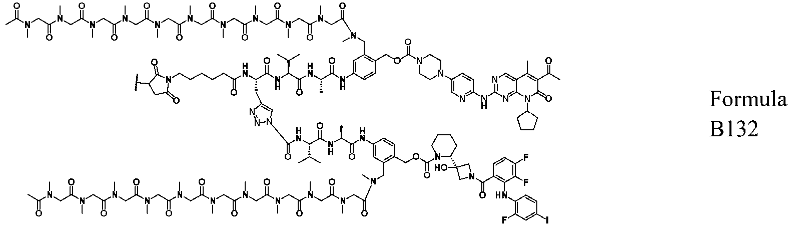

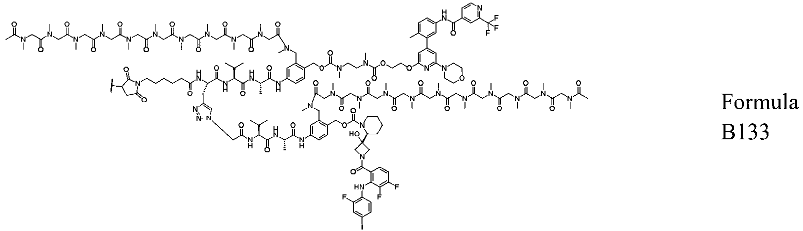

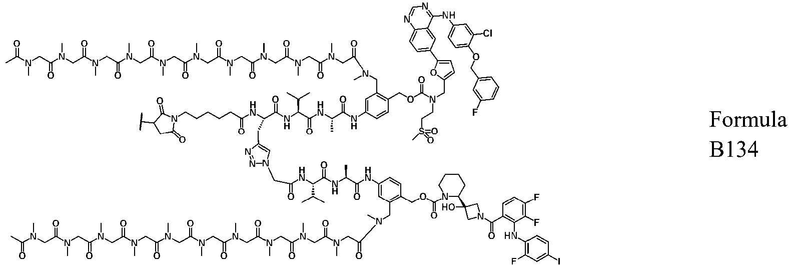

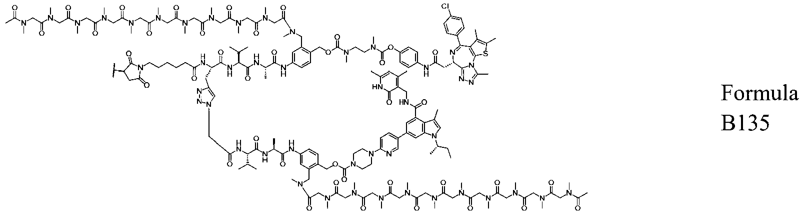

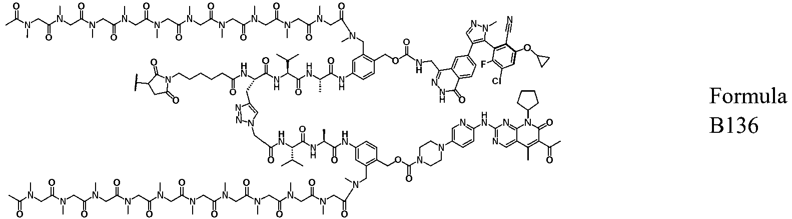

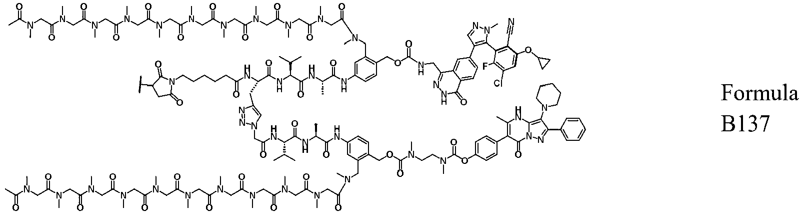

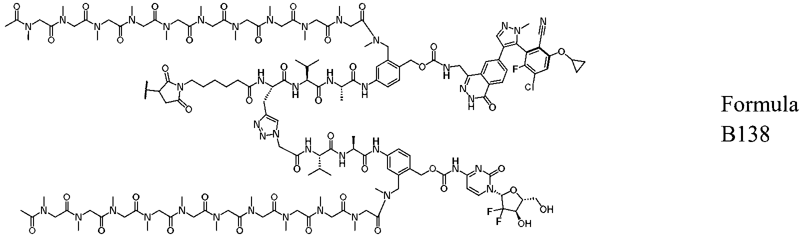

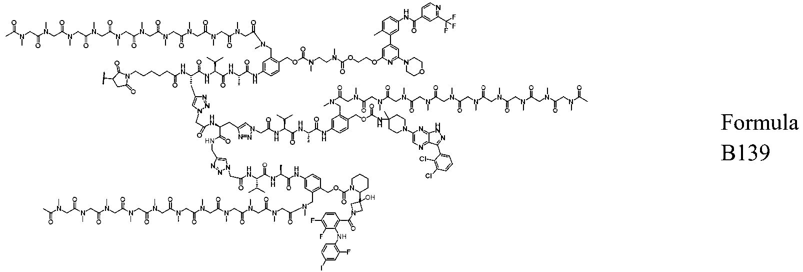

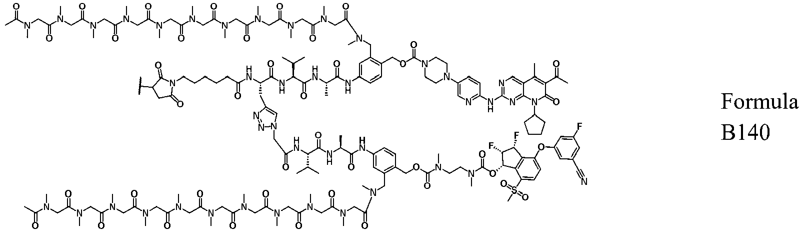

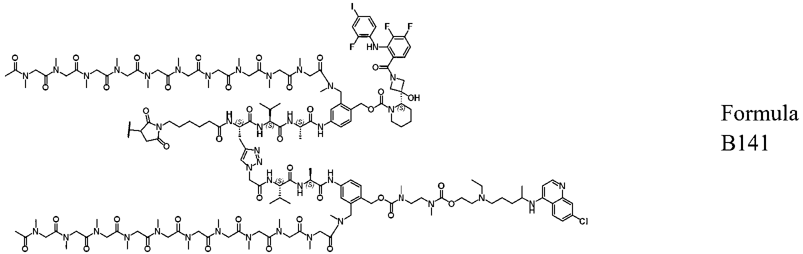

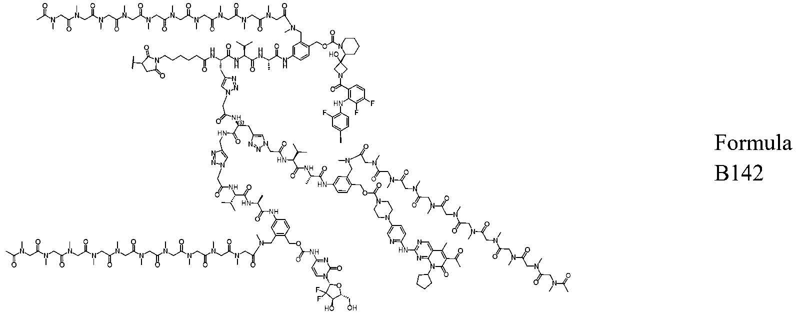

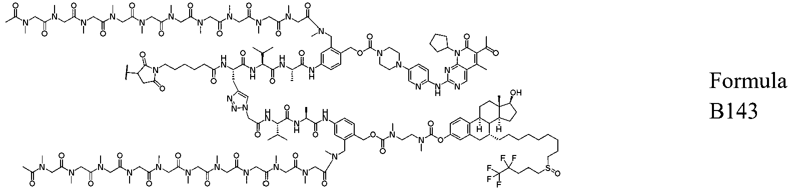

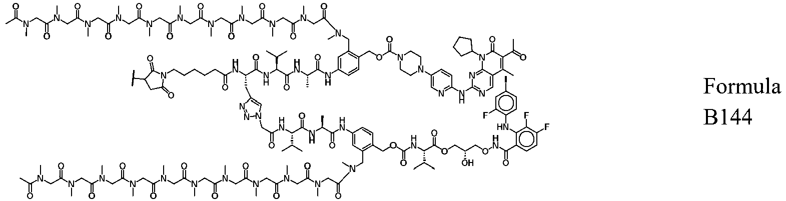

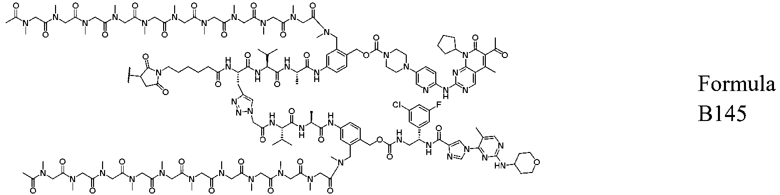

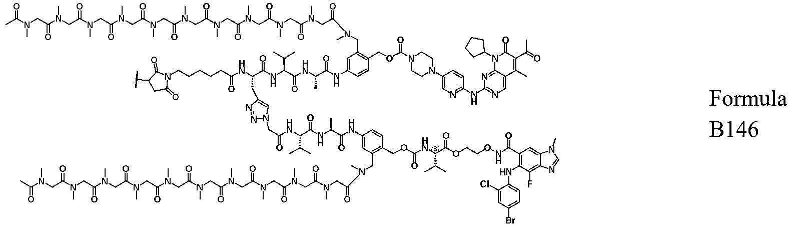

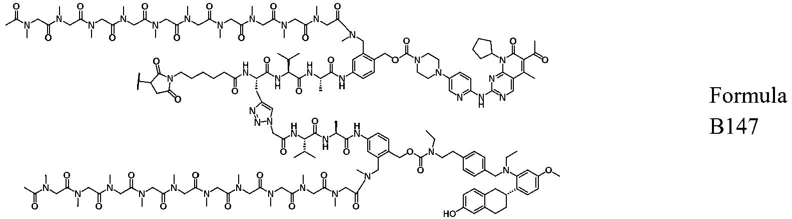

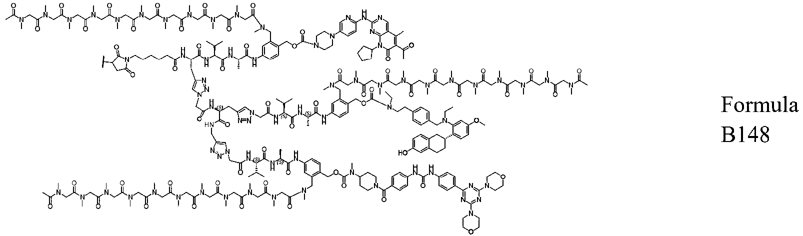

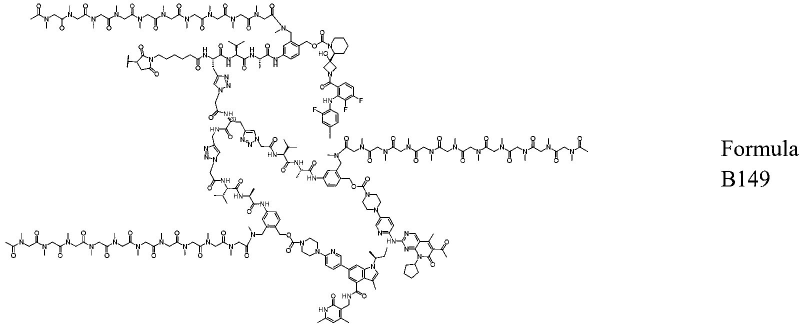

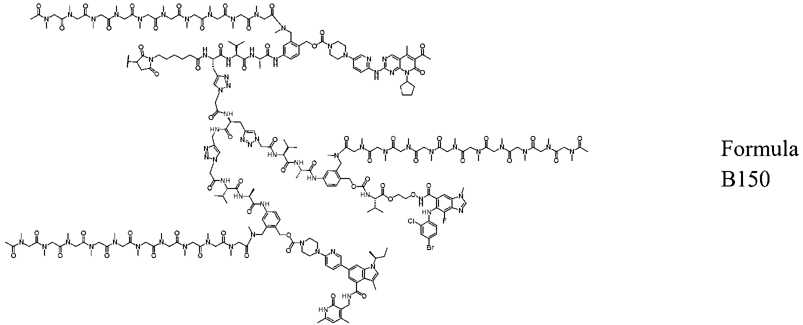

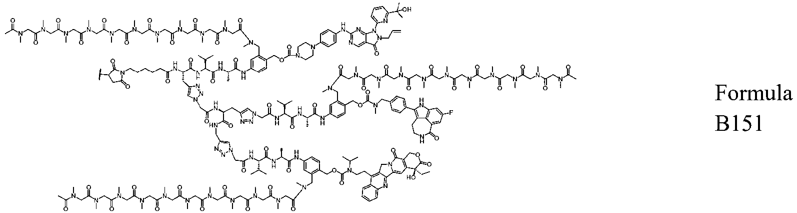

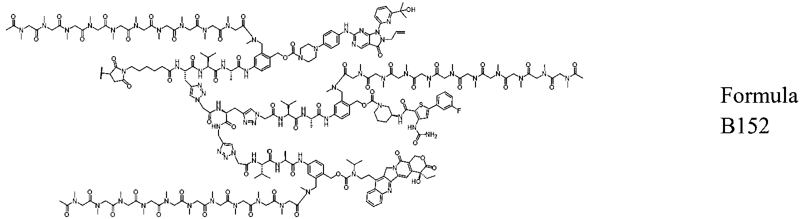

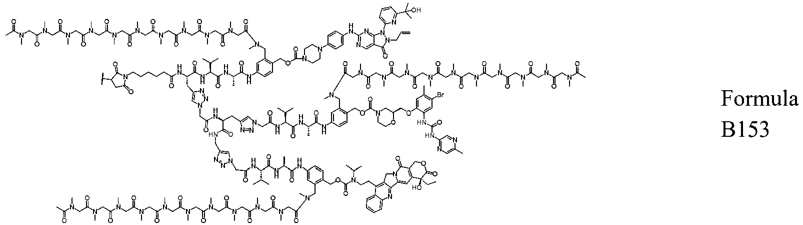

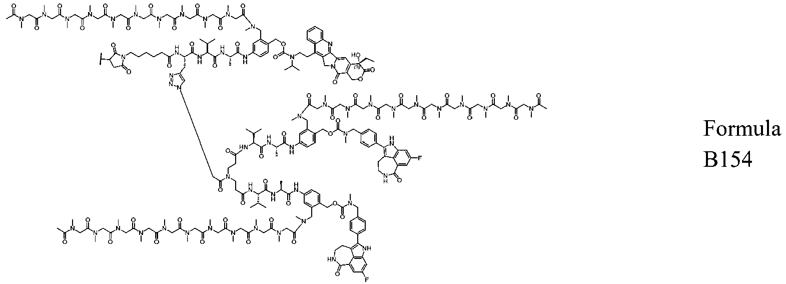

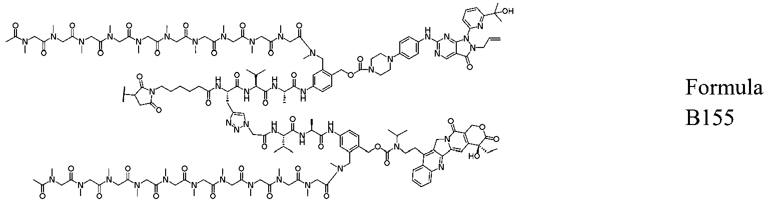

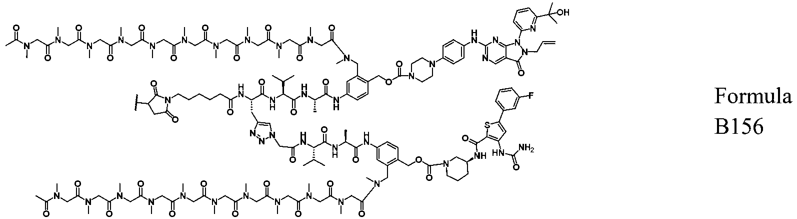

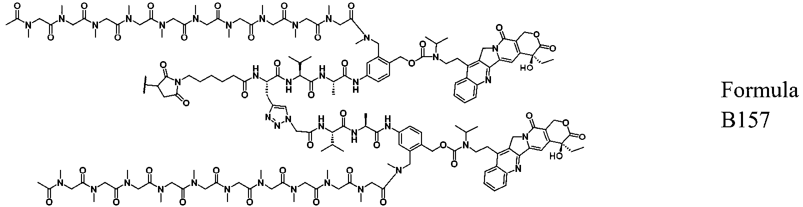

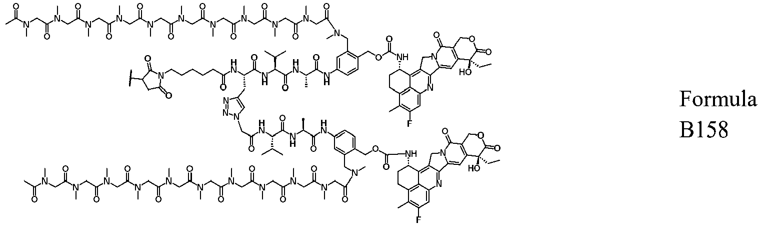

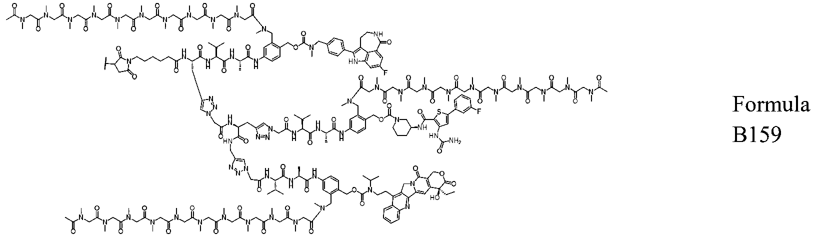

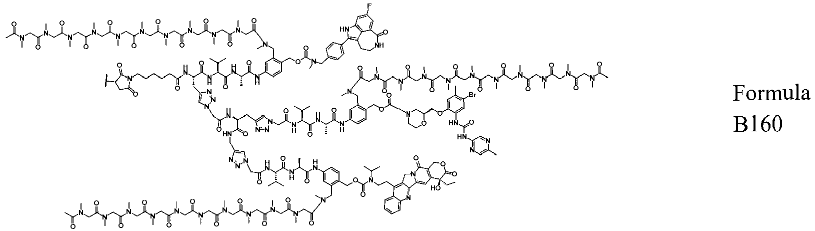

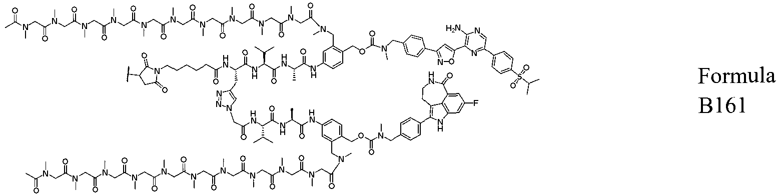

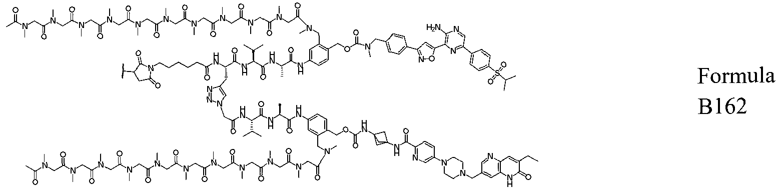

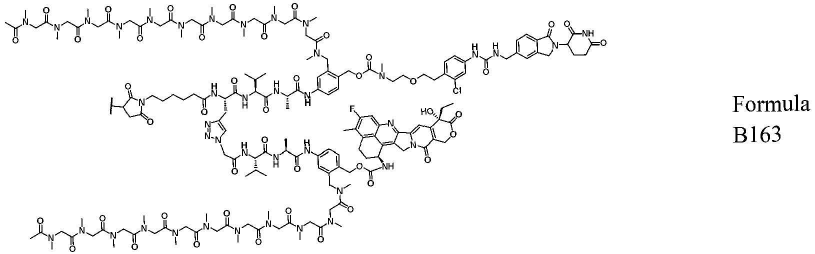

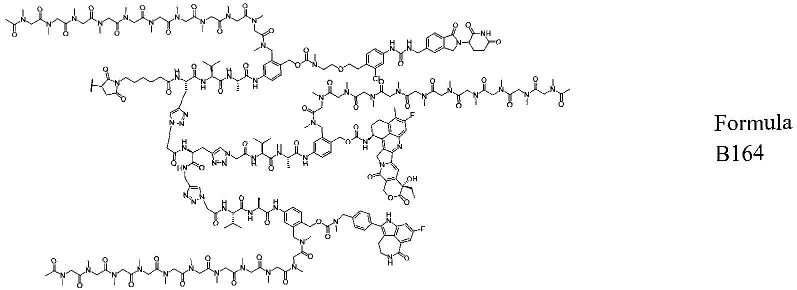

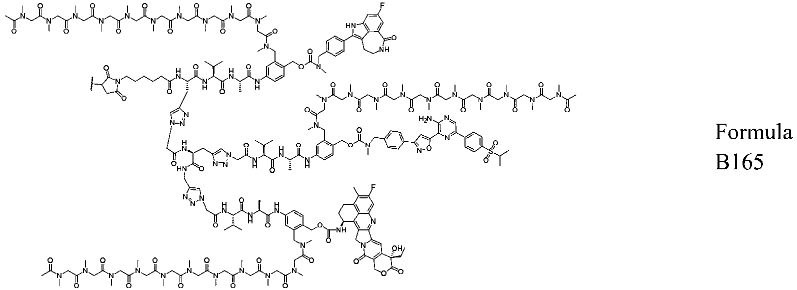

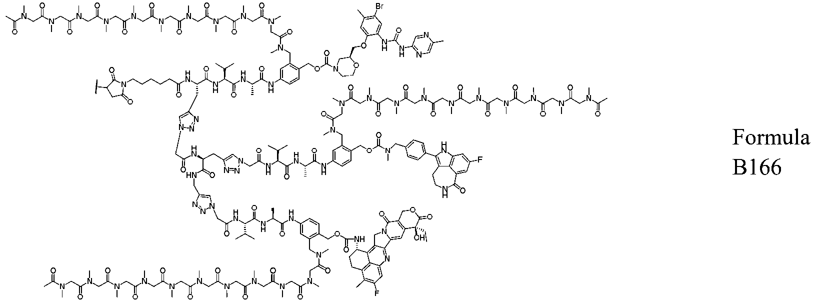

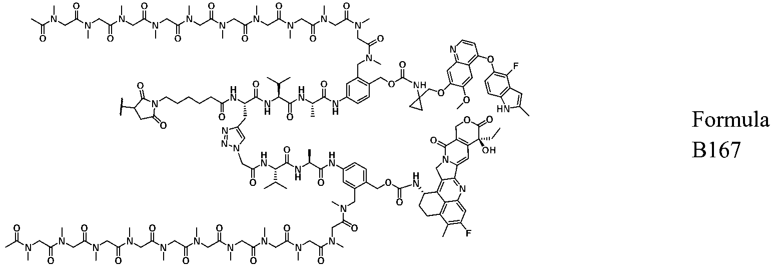

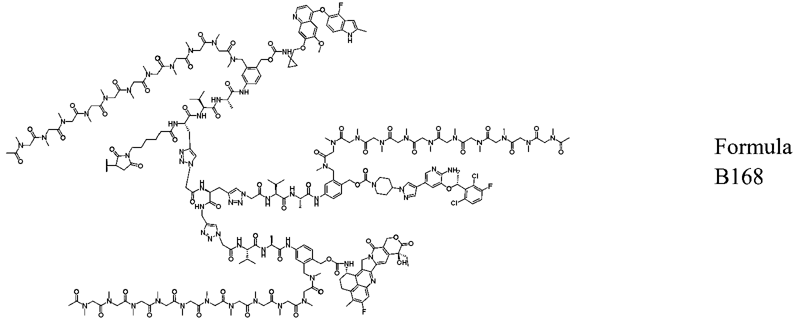

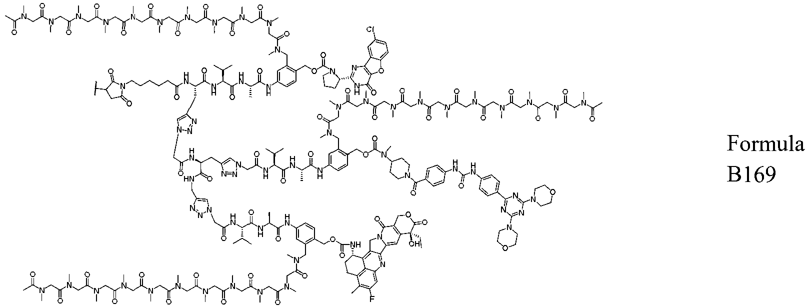

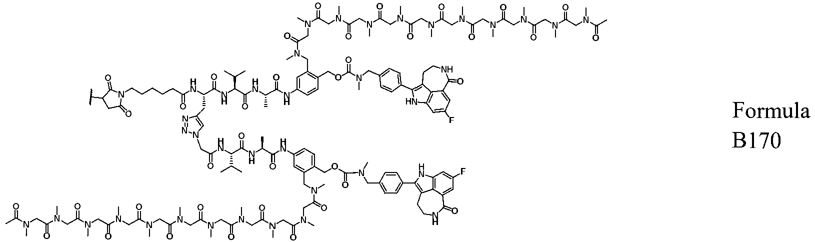

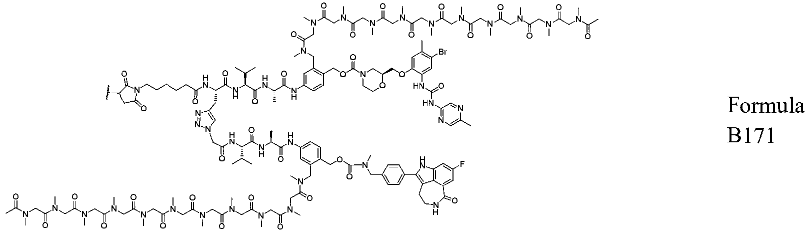

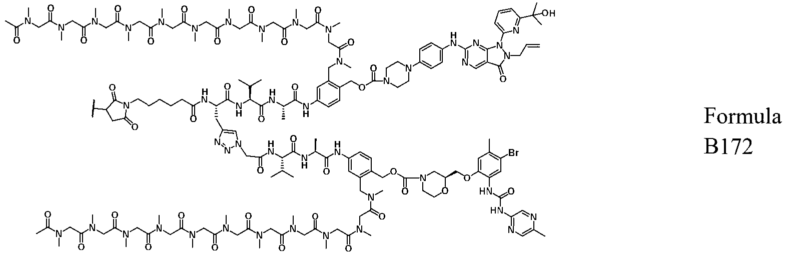

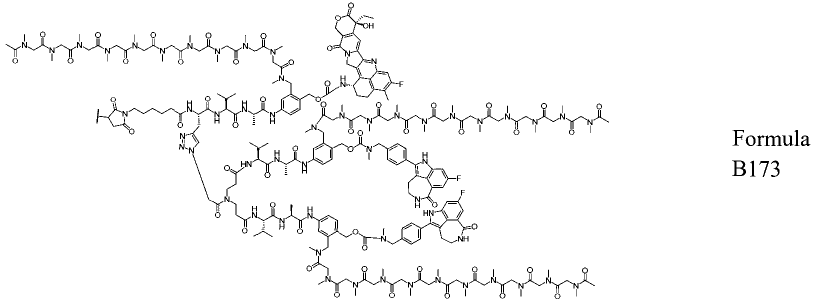

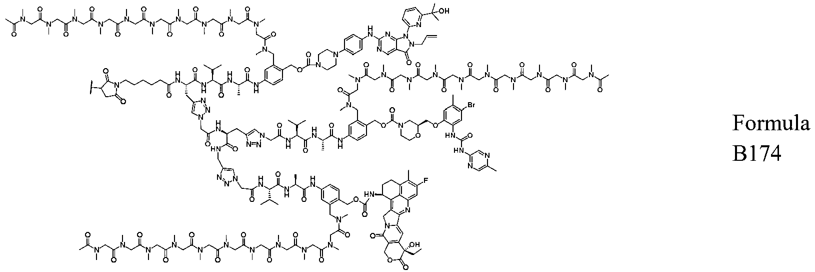

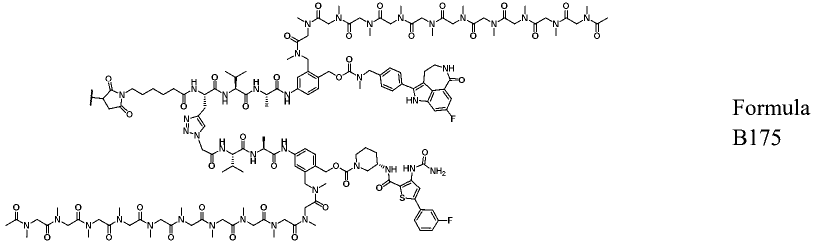

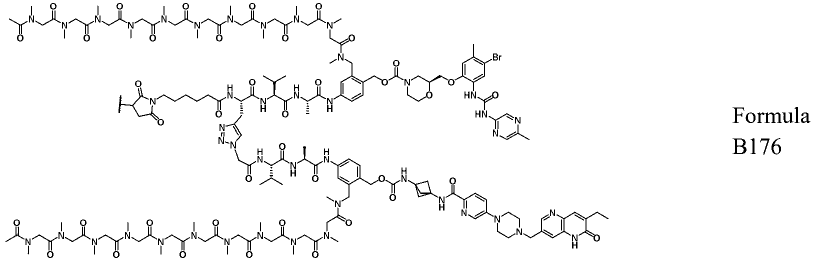

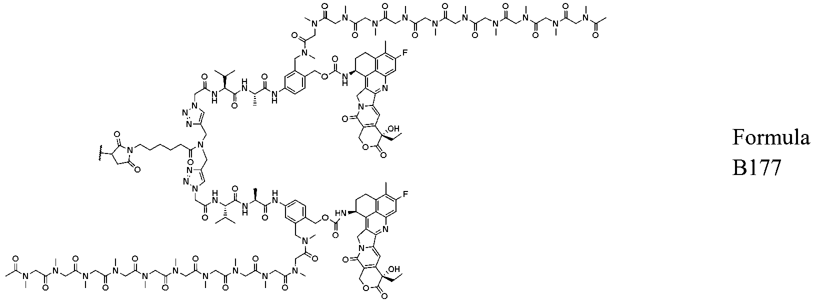

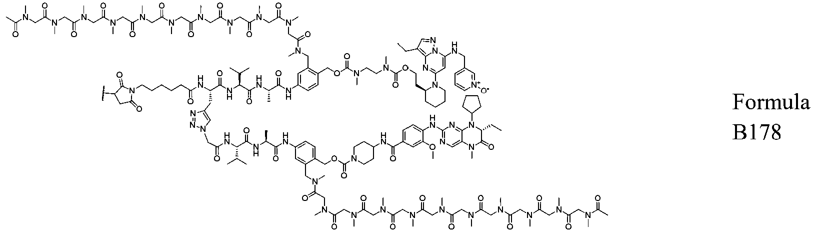

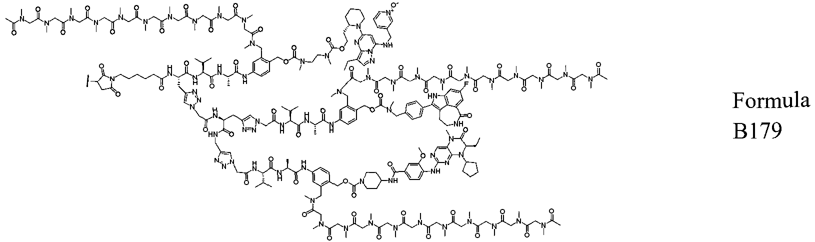

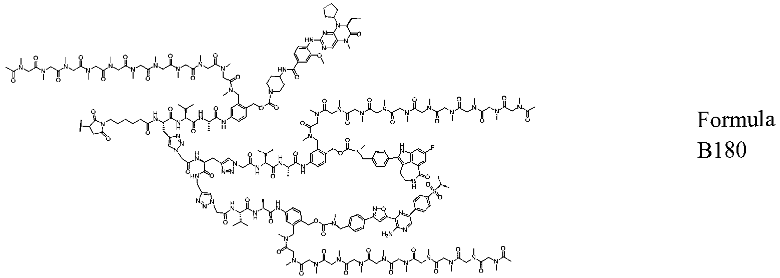

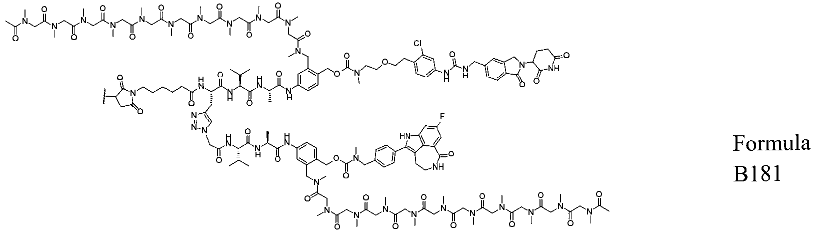

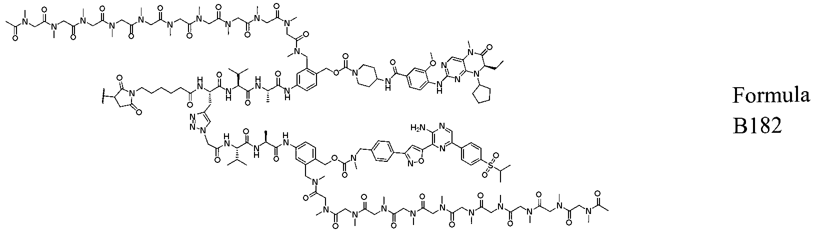

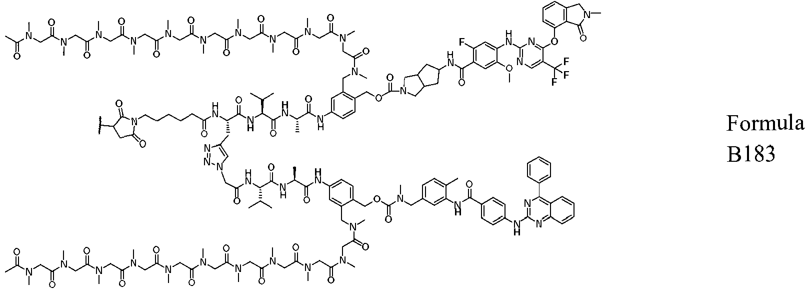

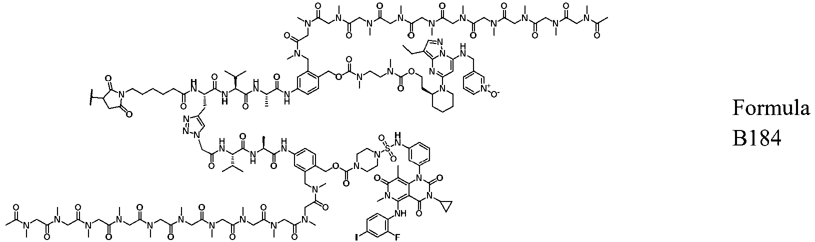

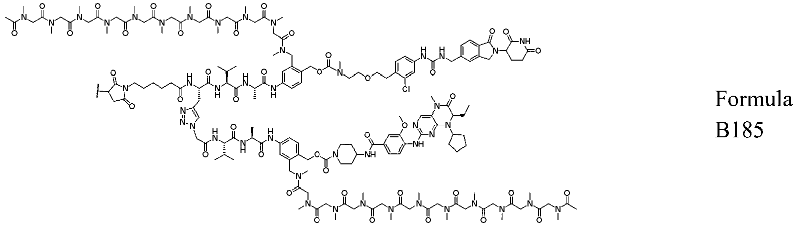

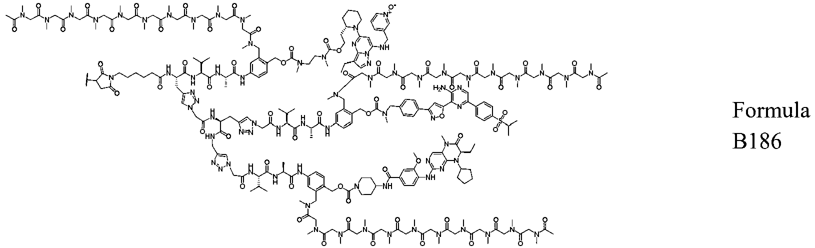

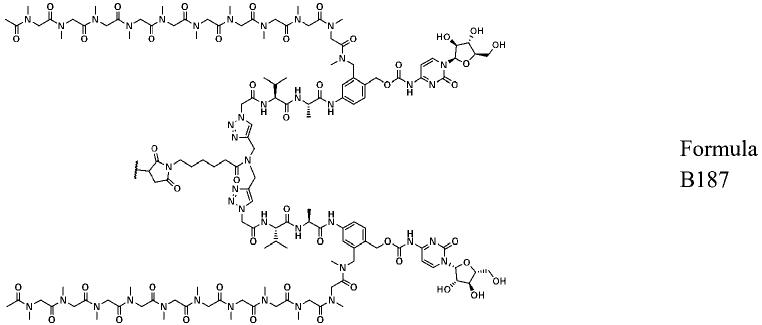

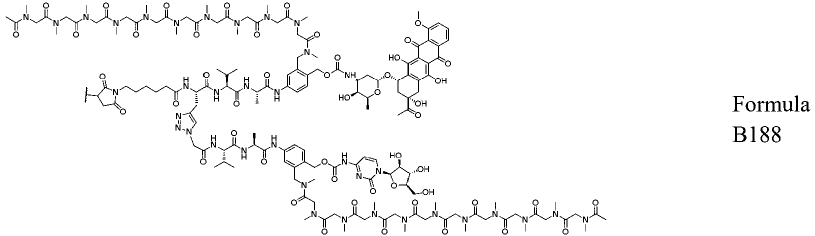

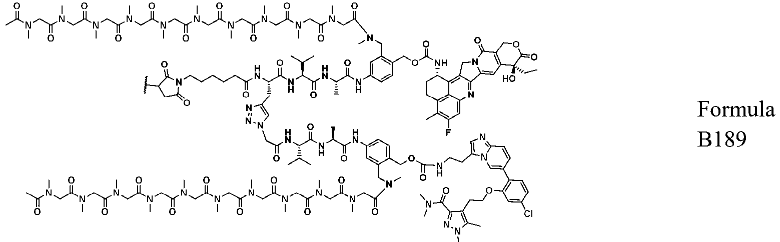

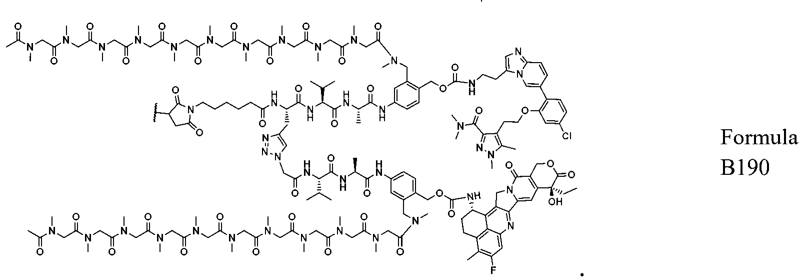

27. A compound, or a tautomer, a mesomer, a racemate, an enantiomer, or a diastereomer thereof, or a deuterated compound form thereof, or a mixture form thereof, or a pharmaceutically acceptable salt, prodrug, or solvate thereof, wherein the compound comprises a structure represented by formula III: 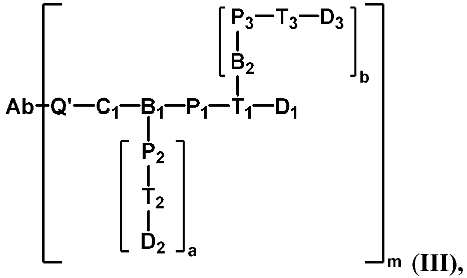
wherein, Ab is an antibody molecule, m is an integer or decimal from 1 to 8;
Q' is a functional group capable of conjugating to cysteine, lysine, a non-natural amino acid, or a glycosyl of an antibody molecule;
C₁ is selected from the group consisting of: a direct bond, optionally substituted alkylene, optionally substituted polyethylene glycol group, optionally substituted alkenylene, optionally substituted alkynylene, optionally substituted alicyclylene, optionally substituted aliphatic heterocyclylene, optionally substituted arylene, and optionally substituted heteroarylene; when substituted, the substituent(s) are selected from halogen, hydroxyl, amino, carboxyl, sulfonic acid group, sulfone group, phosphonic acid group, and alkoxy;
B₁ and B₂ are independently a direct bond or a branching group, provided that when C₁ is a direct bond, B₁ is not a direct bond;
P₁, P₂, and P₃ are each independently an optionally substituted polypeptide residue or a glucose fragment;
T₁, T₂, and T₃ are each independently a direct bond or an optionally substituted spacer group, provided that at most two of T₁, T₂, and T₃ are direct bonds and at least one of B₂, T₁, T₂, and T₃ is substituted with a hydrophilic group;
D₁, D₂, and D₃ are a first drug unit, a second drug unit, and a third drug unit, respectively, and are the same or different;
a and b are independently 0, 1, 2, or 3, provided that a and b are not both 0; and
when a=0, B₁ is a direct bond and T₁ is not a direct bond;
when b=0, B₁ is not a direct bond.

28. The compound, or a tautomer, a mesomer, a racemate, an enantiomer, or a diastereomer thereof, or a deuterated compound form thereof, or a mixture form thereof, or a pharmaceutically acceptable salt, prodrug, or solvate thereof, according to claim 27, wherein the compound comprises a structure represented by formula IIIa: 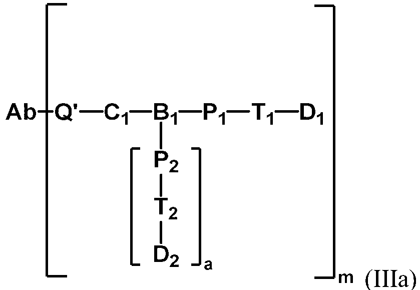 wherein:
Q' is a functional group capable of conjugating to cysteine, lysine, a non-natural amino acid, or a glycosyl of an antibody molecule;
C₁ is selected from the group consisting of: a direct bond, optionally substituted alkylene, optionally substituted polyethylene glycol group, optionally substituted alkenylene, optionally substituted alkynylene, optionally substituted alicyclylene, optionally substituted aliphatic heterocyclylene, optionally substituted arylene, and optionally substituted heteroarylene; when substituted, the substituent(s) are selected from halogen, hydroxyl, amino, carboxyl, sulfonic acid group, sulfone group, phosphonic acid group, and alkoxy;
B₁ is a branching group;
P₁ and P₂ are each independently an optionally substituted polypeptide residue or a glucose fragment;
T₁ and T₂ are each independently a direct bond or an optionally substituted spacer group, provided that at most one of T₁ and T₂ is a direct bond, and at least one of T₁ and T₂ is substituted with a hydrophilic group;
D₁ and D₂ are a first drug unit and a second drug unit, respectively, and are the same or different;
a is 1, 2, or 3.

29. The compound, or a tautomer, a mesomer, a racemate, an enantiomer, or a diastereomer thereof, or a deuterated compound form thereof, or a mixture form thereof, or a pharmaceutically acceptable salt, prodrug, or solvate thereof, according to claim 27, wherein the compound comprises a structure represented by formula IIIb: 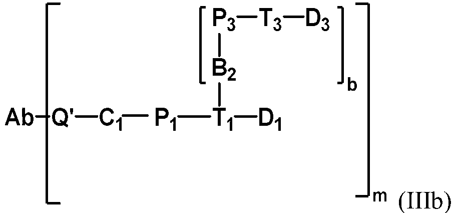
Q' is a functional group capable of conjugating to cysteine, lysine, a non-natural amino acid, or a glycosyl of an antibody molecule;
C₁ is selected from the group consisting of: optionally substituted alkylene, optionally substituted polyethylene glycol group, optionally substituted alkenylene, optionally substituted alkynylene, optionally substituted alicyclylene, optionally substituted aliphatic heterocyclylene, optionally substituted arylene, and optionally substituted heteroarylene; when substituted, the substituent(s) are selected from halogen, hydroxyl, amino, carboxyl, sulfonic acid group, sulfone group, phosphonic acid group, and alkoxy;
B₂ is a branching group;
P₁ and P₃ are each independently a direct bond or an optionally substituted polypeptide residue or a glucose fragment;
T₁ is an optionally substituted spacer group, T₃ is a direct bond or an optionally substituted spacer group, and at least one of B₂ and T₃ is substituted with a hydrophilic group;
D₁ and D₃ are a first drug unit and a third drug unit, respectively, and are the same or different;
b is 1, 2, or 3.

30. The compound, or a tautomer, a mesomer, a racemate, an enantiomer, or a diastereomer thereof, or a deuterated compound form thereof, or a mixture form thereof, or a pharmaceutically acceptable salt, prodrug, or solvate thereof, according to claim 27, wherein:
B₁ and B₂ are independently a branching group;
P₁, P₂, and P₃ are each independently an optionally substituted polypeptide residue or a glucose fragment;
T₁ is an optionally substituted spacer group, T₂ and T₃ are each independently a direct bond or an optionally substituted spacer group, and at least one of B₂, T₂, and T₃ is substituted with a hydrophilic group;
D₁, D₂, and D₃ are a first drug unit, a second drug unit, and a third drug unit, respectively, and are the same or different; and
a and b are independently 1, 2, or 3.

31. The compound, or a tautomer, a mesomer, a racemate, an enantiomer, or a diastereomer thereof, or a deuterated compound form thereof, or a mixture form thereof, or a pharmaceutically acceptable salt, prodrug, or solvate thereof, according to any one of claims 27 to 30, wherein:
Q' is selected from the group consisting of: optionally substituted 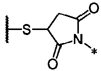 (Q1'), optionally substituted 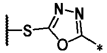 (Q2'), optionally substituted 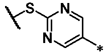 (Q3'), optionally substituted 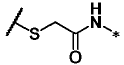 (Q4'), optionally substituted 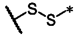 (Q5'), optionally substituted 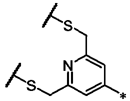 (Q6'), optionally substituted 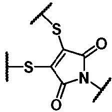 (Q7'), and optionally substituted 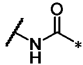 (Q8'), wherein * represents a site connected to C₁, and the wave line represents a site connected to Ab.

32. The compound, or a tautomer, a mesomer, a racemate, an enantiomer, or a diastereomer thereof, or a deuterated compound form thereof, or a mixture form thereof, or a pharmaceutically acceptable salt, prodrug, or solvate thereof, according to any one of claims 27 to 31, wherein:
C₁ is selected from: a direct bond, -(CH₂)ₘ₁-, -(CH₂)ₘ₁-(O-CH₂-CH₂)ₘ₂-, -(CH₂)ₘ₁(O-CH₂-CH₂)ₘ₂-(CH₂)ₘ₃-, -(CH₂)ₘ₁-C(O)NH-(CH₂)ₘ₂-, -(CH₂)ₘ₁-NHC(O)-(CH₂)ₘ₂-, and -(C≡ C-(CH₂)ₘ₁-, wherein m1, m2, and m3 are independently integers from 1 to 6, and the indicated substituent is connected to Q' on the left side and to B₁ or P₁ on the right side.

33. The compound, or a tautomer, a mesomer, a racemate, an enantiomer, or a diastereomer thereof, or a deuterated compound form thereof, or a mixture form thereof, or a pharmaceutically acceptable salt, prodrug, or solvate thereof, according to any one of claims 27 to 32, wherein:
B₁ is selected from the group consisting of: a direct bond, 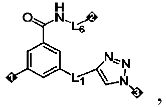 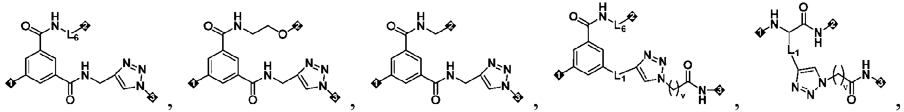 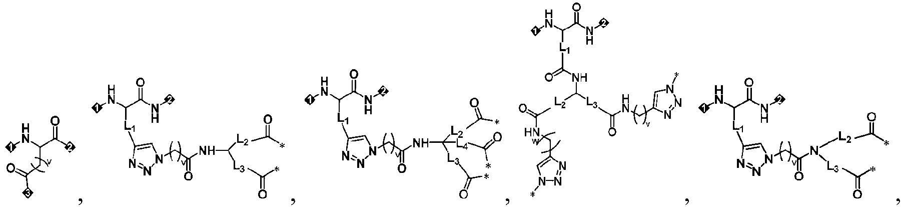 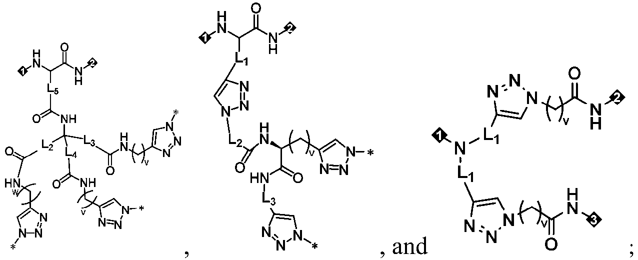
preferably, 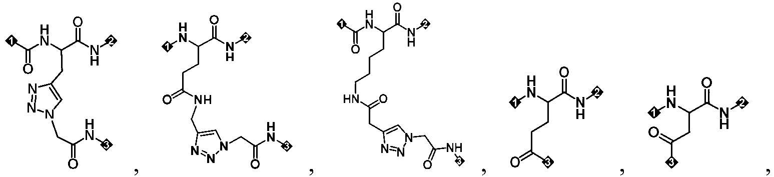 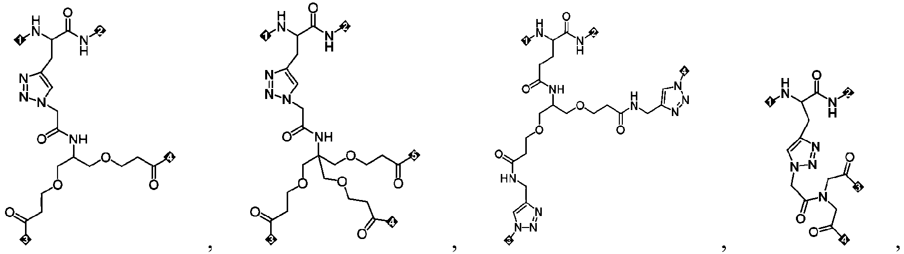 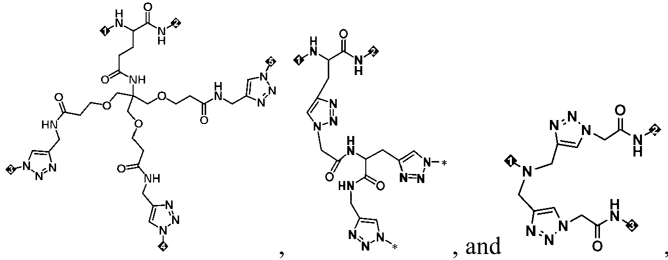 wherein attachment point 1 is connected to C₁ or Q' (when C₁ is a direct bond), attachment point 2 is connected to P₁, attachment points *, 3, 4, and 5 are connected to P₂, L₁ is selected from -(CH₂)ₘ₁-, -(CH₂)ₘ₁-C(O)NH-(CH₂)ₘ₂-, -(CH₂)ₘ₁-NHC(O)-(CH₂)ₘ₂-, L₂, L₃, and L₄ are independently -(CH₂)ₘ₁-(O-CH₂-CH₂)ₘ₂- or -(CH₂)ₘ₁-(O-CH₂-CH₂)ₘ₂-(CH₂)ₘ₃-, L₅ is -(CH₂)ₘ₁-, L₆ is -(CH₂)ₘ₁- or - (CH₂-CH₂-O)m₂, wherein m1, m2, m3, w, and v are each independently 1, 2, 3, 4, 5, or 6;
preferably, when C₁ is a direct bond, B₁ is

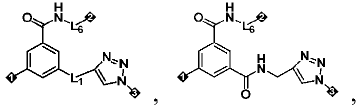

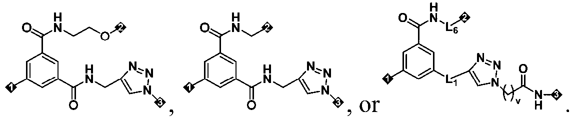

34. The compound, or a tautomer, a mesomer, a racemate, an enantiomer, or a diastereomer thereof, or a deuterated compound form thereof, or a mixture form thereof, or a pharmaceutically acceptable salt, prodrug, or solvate thereof, according to any one of claims 27 to 33, wherein:
B₂ is selected from the group consisting of: 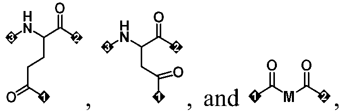 wherein attachment point 1 is connected to T₁, attachment point 2 is connected to P₃, attachment point 3 is connected to the hydrophilic group, M is selected from the group consisting of: optionally substituted C₁-C₆ alkylene, optionally substituted C₁-C₆ alkoxy, and optionally substituted C₁-C₅ alkenylene.

35. The compound, or a tautomer, a mesomer, a racemate, an enantiomer, or a diastereomer thereof, or a deuterated compound form thereof, or a mixture form thereof, or a pharmaceutically acceptable salt, prodrug, or solvate thereof, according to any one of claims 27 to 34, wherein:
T₁, T₂, T₃ are independently selected from the group consisting of: a direct bond, 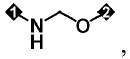 and optionally substituted 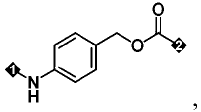 wherein attachment point 1 is connected to P₁, P₂, or P₃, and attachment point 2 is connected to D₁, D₂, or D₃.

36. The compound, or a tautomer, a mesomer, a racemate, an enantiomer, or a diastereomer thereof, or a deuterated compound form thereof, or a mixture form thereof, or a pharmaceutically acceptable salt, prodrug, or solvate thereof, according to any one of claims 27 to 35, wherein:
P₁, P₂, P₃ comprises an optionally substituted polypeptide residue composed of amino acids selected from the group consisting of: phenylalanine, isoleucine, leucine, tryptophan, valine, methionine, tyrosine, alanine, threonine, histidine, serine, glutamine, arginine, lysine, asparagine, glutamic acid, proline, citrulline, aspartic acid, and glycine; preferably, P₁, P₂, P₃ comprises an optionally substituted polypeptide residue composed of amino acids selected from the group consisting of: glycine, phenylalanine, valine, alanine, arginine, citrulline, aspartic acid, asparagine, and lysine;
more preferably, P₁, P₂, P₃ comprises an optionally substituted polypeptide residue selected from the group consisting of: phenylalanine-lysine (Phe-Lys), valine-alanine (Val-Ala), valine-citrulline (Val-Cit), glutamic acid-valine-alanine (Glu-Val-Ala), glutamic acid-valine-citrulline (Glu-Val-Cit), valine-lysine (Val-Lys), alanine-alanine (Ala-Ala), alanine-alanine-alanine (Ala-Ala-Ala), alanine-alanine-asparagine (Ala-Ala-Asn), alanine-leucine (Ala-Leu), leucine-leucine (Leu-Leu), phenylalanine-arginine (Phe-Arg), phenylalanine-lysine (Phe-Lys), 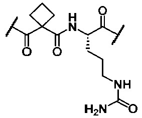 (cBu-Cit), 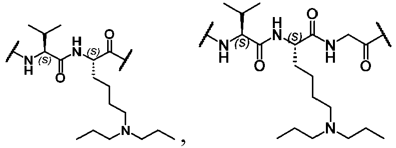 glycine-glycine-phenylalanine-glycine (Gly-Gly-Phe-Gly), glycine-proline (Gly-Pro);
or, wherein one of the combination P₂-T₂ and the combination P₃-T₃ is 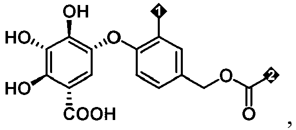 wherein attachment point 1 is connected to B₁ or B₂, and attachment point 2 is connected to D₂ or D₃.

37. The compound, or a tautomer, a mesomer, a racemate, an enantiomer, or a diastereomer thereof, or a deuterated compound form thereof, or a mixture form thereof, or a pharmaceutically acceptable salt, prodrug, or solvate thereof, according to any one of claims 27 to 36, wherein:
the hydrophilic group is selected from: substituted polysarcosine residue, polyglycerol, polyol, glycosyl, cyclodextrin, substituted ethylene glycol fragment, substituted glycosylated polyethylene glycol, substituted glycosylated polyglycerol, substituted cyclodextrin polyethylene glycol, or a combination thereof;
preferably, the substituted polysarcosine residue is 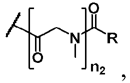 wherein n₂ is an integer between 4 and 20, for example between 4 and 16, R is selected from: C₁-C₆ alkyl, C₁-C₆ cycloalkyl, and C₁-C₆ alkoxy;
preferably, the substituted glycosylated polyethylene glycol fragment is 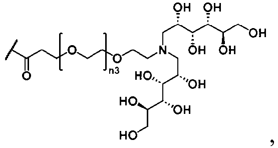 wherein n₃ is an integer between 4 and 18, for example between 4 and 12;
preferably, the substituted glycosylated polyglycerol fragment is 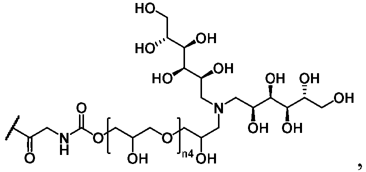 wherein n₄ is an integer between 4 and 12, for example between 4 and 10;
preferably, the substituted polyethylene glycol fragment is 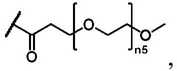 wherein n₅ is an integer between 4 and 24, for example between 8 and 18;
preferably, at least one of T₁, T₂, T₃ is 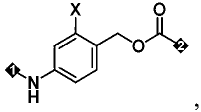 wherein X is optionally substituted 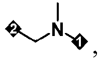 the hydrophilic group is connected to T₁, T₂, or T₃ via X, wherein attachment point 1 is connected to the hydrophilic group, and attachment point 2 is connected to one of T₁, T₂, T₃.

38. The compound, or a tautomer, a mesomer, a racemate, an enantiomer, or a diastereomer thereof, or a deuterated compound form thereof, or a mixture form thereof, or a pharmaceutically acceptable salt, prodrug, or solvate thereof, according to any one of claims 27 to 37, wherein:
D₁, D₂, D₃ are a first anti-cancer agent, a second anti-cancer agent, and a third anti-cancer agent; preferably, at least two of the first, second, and third anti-cancer agents have a synergistic anti-cancer effect;
preferably, D₁, D₂, D₃ are independently a cytotoxic drug or a tumor-targeted therapeutic drug; preferably, at least one of D₁, D₂, and D₃ is a cytotoxic drug, and at least another one is a tumor-targeted therapeutic drug; also preferably, at least two of D₁, D₂, and D₃ are tumor-targeted therapeutic drugs, for example, tumor-targeted therapeutic drugs that can produce a synergistic or additive effect;
preferably, the cytotoxic drug is selected from drug units targeting topoisomerase, drug units targeting tubulin, nucleoside antimetabolite anti-cancer drug units, and drug units targeting DNA;
preferably, the tumor-targeted therapeutic drug is selected from drug units targeting DNA damage response (DDR) or "synthetic lethality" related pathways, drug units targeting epigenetics, drug units targeting apoptosis-related pathways, drug units targeting transcription factors, and drug units targeting immune activation pathway-related targets, comprising: inhibitors of EFGR pathway-related targets; Ras-Raf-MAPK pathway inhibitors; PI3K/AKT/mTOR pathway inhibitors; cell cycle pathway inhibitors; cGAS-STING signaling pathway agonists; estrogen receptor antagonists; androgen receptor antagonists; glucocorticoid receptor modulators; autophagy inhibitors; FAK inhibitors; Smo inhibitors; BTK inhibitors; PDE4 inhibitors; Lck inhibitors; PLK1 inhibitors; TLR7/8 modulators; N-myristoyltransferase (NMT) inhibitors;
preferably, the drug unit targeting topoisomerase is a topoisomerase I (TOPO1) inhibitor or topoisomerase II (TOPO2) inhibitor, comprising Exatecan (CAS 171335-80-1), DXd (CAS 1599440-33-1), 7-ethyl-10-hydroxycamptothecin (SN38, CAS 86639-52-3), Belotecan (CAS 213819-48-8), (4-NH₂)-Exatecan (AZD'0132, CAS 2495742-21-5), 7-MAD-MDCPT (CAS 765871-81-6), 7-aminomethyl-10-methyl-11-fluorocamptothecin (CAS 2378616-23-8), Voreloxin (CAS 175414-77-4), or derivatives and analogs thereof;
preferably, the drug unit targeting tubulin is Eribulin, Vinblastine, Paclitaxel, MMAE, MMAF, Maytansine, or derivatives thereof;
preferably, the nucleoside antimetabolite anti-cancer drug is Gemcitabine, Decitabine;
preferably, the drug unit targeting DNA is a DNA minor groove binder (PBD), a DNA alkylator (Duocarmycin), Trabectedin, Lurbinectedin, or derivatives thereof;
preferably, the drug unit targeting DNA damage response (DDR) or "synthetic lethality" related pathways is a PARP inhibitor, ATR inhibitor, CHK1 inhibitor, ATM inhibitor, DNA-PK inhibitor, WEE1 inhibitor, POLQ inhibitor, CDK12 inhibitor, USP1 inhibitor, PKMYT1 inhibitor, or Rad51 inhibitor;
the PARP inhibitor is preferably Rucaparib, Niraparib, Veliparib, A-966492, Talazoparib, AZD5305, Venadaparib, Mefuparib, or analogs thereof; the ATR inhibitor is preferably Berzosertib, Ceralasertib, or analogs thereof; and/or the CHK1 inhibitor is preferably Prexasertib (LY2606368), AZD7762, Rabusertib (LY2603618), MK-8776 (SCH 900776), CHIR-124, PF-477736, CCT245737 (SRA737, PNT-737), GDC-0575 (ARRY-575), or analogs thereof; and/or the WEE1 inhibitor is preferably ZN-C3, Adavosertib (AZD1775), or analogs thereof;
preferably, an analog of the AZD5305 comprises the following structure: 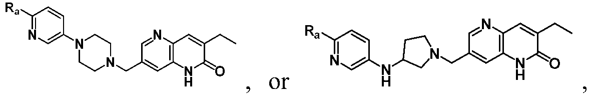 wherein Rₐ is selected from - C(O)NH-R_{b} or -NHC(O)-R_{b}, R_{b} is selected from: C₂₋₇ alkyl, C₃₋₇ monocyclic cycloalkyl, C₄₋₁₀ bicyclic cycloalkyl, C₅₋₉ spirocycloalkyl, and C₅₋₉ bridged cycloalkyl, each substituted with at least one primary or secondary amino group, or R_{b} is a 4-6 membered monocyclic heterocycloalkyl, C₄₋₁₀ bicyclic heterocycloalkyl, C₅₋₉ spiroheterocycloalkyl, C₅₋₉ bridged heterocycloalkyl containing 1-5 nitrogen, oxygen, sulfur atoms, wherein the R_{b} group comprises at least one primary or secondary amino group;
preferably, the AZD5305 analog is selected from the following structures: 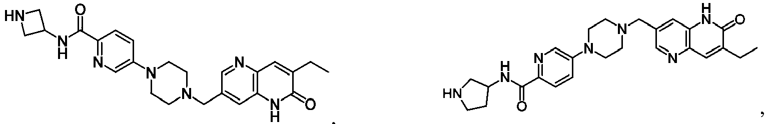 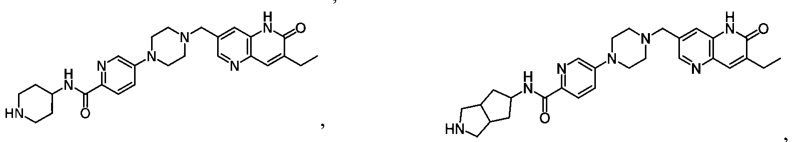 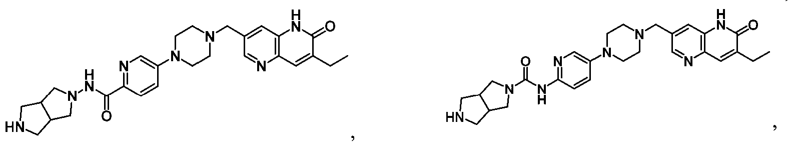 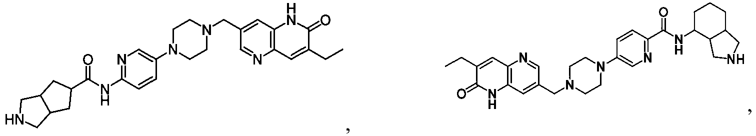 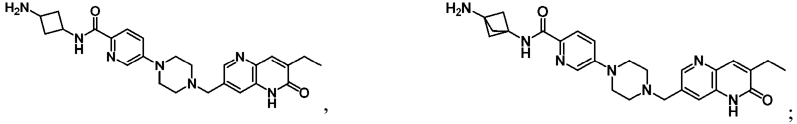
preferably, the PKMYT1 inhibitor is preferably RP-6306 and GSK-1520489A or analogs thereof;
preferably, analogs of the WEE1 inhibitors Adavosertib (AZD1775, CAS 955365-80-7), Debio 0123 (CAS 2243882-74-6) are selected from the following structures: 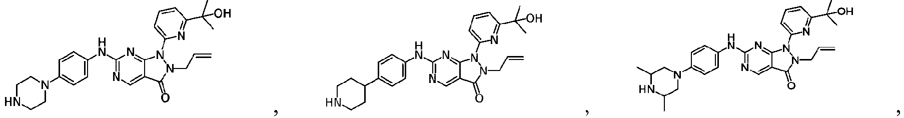 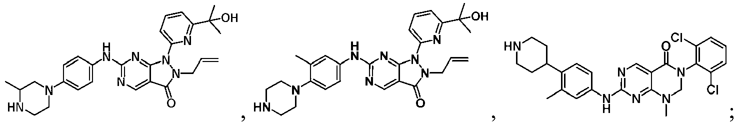
preferably, the drug unit targeting apoptosis-related pathways is a Bcl-2 family protein inhibitor; the Bcl-2 family protein inhibitor comprises BCL-2 inhibitors, BCL-XL inhibitors, and MCL-1 inhibitors, preferably Venetoclax (CAS 1257044-40-8), Navitoclax (CAS 923564-51-6), Navitoclax analog (CAS 2143096-93-7), ABT-737 (CAS 852808-04-9), A-1331852 (CAS 1430844-80-6), S64315 (CAS 1799631-75-6), or analogs thereof;
preferably, the A-1331852, S64315 analogs are selected from the following structures: 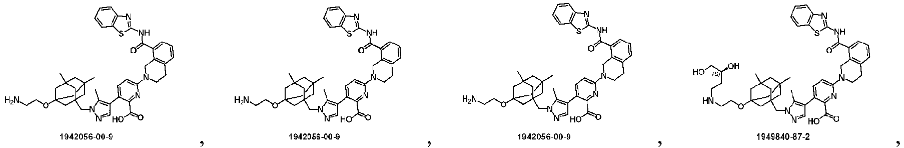 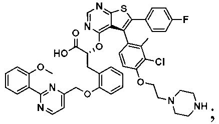
preferably, the drug unit targeting epigenetics is an LSD1 inhibitor, EZH2 inhibitor, BRD4 inhibitor, PRMT5 inhibitor, or PRMT1 inhibitor;
the LSD1 inhibitor is preferably Tranylcypromine, ORY-1001 (Iadademstat, CAS 1431303-72-8), CC-90011 (Pulrodemstat, CAS 1821307-10-1), ORY-2001, GSK-2879552, IMG-7289, INCB059872, or TAK-418 or analogs thereof; the EZH2 inhibitor is preferably Tazemetostat (CAS 1403254-99-8), GSK2816126, CPI-1205, PF-06821497, SHR2554, XNW5004, HH2853, or analogs thereof; the BRD4 inhibitor is a BI-2536 analog, Birabresib; the PRMT5 inhibitor is preferably GSK3326595, AMG 193, MRTX1719, SKL27969, TNG908, SCR-6920, SH3765, SYHX2001, or analogs thereof; the PRMT1 inhibitor is preferably GSK3368715 (CAS 1629013-22-4), MS023 (CAS 1831110-54-3), or analogs thereof;
preferably, the drug unit targeting immune activation pathway-related targets is a PD-L1 inhibitor, CBL-B inhibitor, TLR7/8 agonist, PTPN2/1 inhibitor, or STING agonist;
preferably, the Ras-Raf-MAPK pathway inhibitors comprise: SOS1 inhibitors BAY-293 (CAS 2244904-70-7), BI-3406 (CAS 2230836-55-0), or analogs thereof; KRAS inhibitors BI-2493 (CAS 2937344-16-4), MRTX1133 (CAS 2621928-55-8), or analogs thereof; MEK inhibitors Cobimetinib (CAS 934660-93-2), Selumetinib (CAS 606143-52-6), Mirdametinib (CAS 391210-10-9), Binimetinib (CAS 606143-89-9), TAK-733 (CAS 1035555-63-5), GDC-0623 (CAS 1168091-68-6), AZD8330 (CAS 869357-68-6), Trametinib (CAS 871700-17-3), Trametiglue (CAS 2666940-97-0), or analogs thereof; ERK inhibitors ASN007 (CAS 2055597-12-9) or analogs thereof;
preferably, the cell cycle pathway inhibitors comprise: CDK4/6 inhibitors Palbociclib (CAS 571190-30-2), Abemaciclib (CAS 1231929-97-7), Ribociclib (CAS 1211441-98-3), Dalpiciclib (CAS 1637781-04-4), or analogs thereof; pan-CDK inhibitors Dinaciclib (CAS 779353-01-4) or analogs thereof;
preferably, the PI3K/AKT/mTOR pathway inhibitors comprise: PKI-587 (CAS 1197160-78-3), desmethyl PKI-587 (CAS 1950569-63-7), AZD8055 (CAS 1009298-09-2), Afuresertib (CAS 1047644-62-1), or analogs thereof;
preferably, the estrogen receptor antagonists/degraders comprise Fulvestrant (CAS 129453-61-8), Elacestrant (CAS 1349723-93-8); the androgen receptor antagonists comprise abiraterone (CAS 154229-19-3), JNJ-63576253 (CAS 2110428-64-1), or analogs thereof;
preferably, the autophagy inhibitor is Hydroxychloroquine (HCQ, CAS 118-42-3) and Chloroquine (CQ, CAS 54-05-7) or analogs thereof;
preferably, the drug targeting transcription factors is a GSPT1 degrader (molecular glue), and the GSPT1 degrader is selected from the following structures: 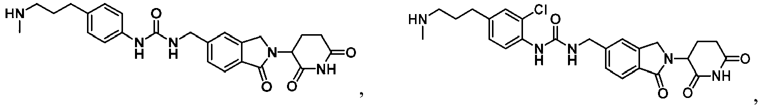 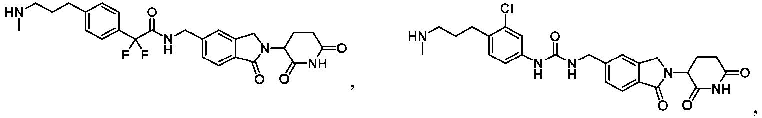 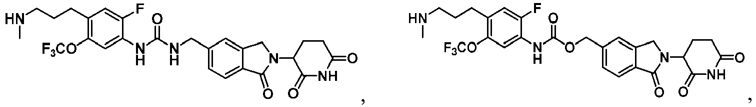 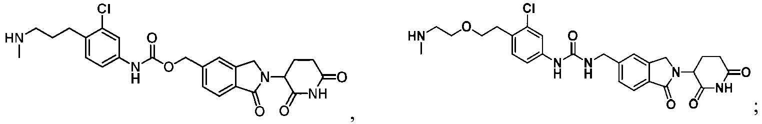
preferably, the FAK inhibitor is Ifebemtinib (CAS 1227948-82-4), Defactinib (CAS 1073154-85-4), PF-562271 (CAS 717907-75-0), or analogs thereof, selected from the following structures: 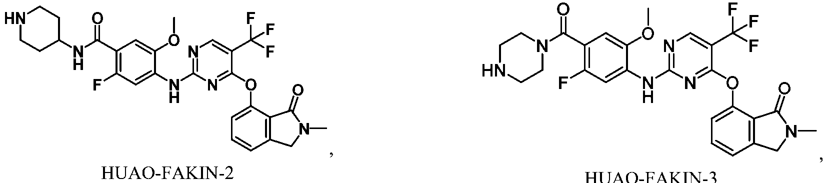 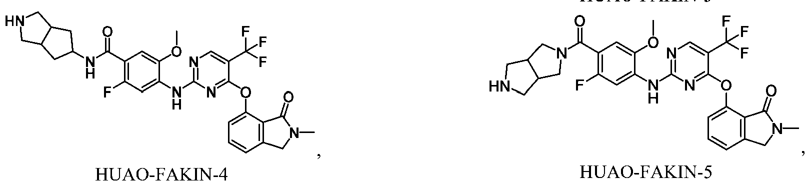 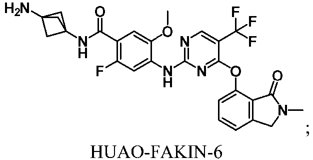
preferably, the PLK1 inhibitor is BI2536 (CAS 755038-02-9), Volasertib (CAS 755038-65-4), GSK461364 (CAS 929095-18-1), Onvansertib (CAS 1034616-18-6), or analogs thereof;
preferably, the N-myristoyltransferase (NMT) inhibitor is MYX1715 (CAS 2445448-66-6) or analogs thereof;
more preferably, D₁, D₂, and D₃ are selected from the combinations in the following table:
| | | | |
|---|---|---|---|
| Drug Combination | D1 | D2 | D3 |
| Combination 1 | TOPO1 inhibitor | PARP inhibitor | / |
| Combination 2 | TOPO1 inhibitor | ATR inhibitor | / |
| Combination 3 | TOPO1 inhibitor | CHK1 inhibitor | / |
| Combination 4 | TOPO1 inhibitor | PARP inhibitor | ATR inhibitor |
| Combination 5 | TOPO1 inhibitor | PARP inhibitor | CHK1 inhibitor |
| Combination 6 | TOPO1 inhibitor | PARP inhibitor | WEE1 inhibitor |
| Combination 7 | TOPO1 inhibitor | ATR inhibitor | WEE1 inhibitor |
| Combination 8 | TOPO1 inhibitor | CHK1 inhibitor | WEE1 inhibitor |
| Combination 9 | TOPO1 inhibitor | DNA-PK inhibitor | / |
| Combination 10 | TOPO1 inhibitor | POLQ inhibitor | / |
| Combination 11 | TOPO1 inhibitor | TOPO2 inhibitor | / |
| Combination 12 | PARP inhibitor | ATR/CHK1/WEE1 inhibitor | / |
| Combination 13 | PARP inhibitor | ATR inhibitor | CHK1 inhibitor |
| Combination 14 | TOPO1 inhibitor | Immune activator (PD-L1 inhibitor, CBL-B inhibitor, TLR7/8 agonist, PTPN2/1 inhibitor, or STING agonist) | / |
| Combination 15 | TOPO1 inhibitor | BCL-2 family inhibitor | / |
| Combination 16 | TOPO1 inhibitor | BCL-2 family inhibitor | MCL-1 inhibitor |
| Combination 17 | TOPO1 inhibitor | DNA synthesis inhibitor | / |
| Combination 18 | TOPO1 inhibitor | PI3K/mTOR inhibitor | / |
| Combination 19 | TOPO1 inhibitor | EGFR inhibitor | / |
| Combination 20 | TOPO1 inhibitor | Autophagy inhibitor | / |
| Combination 21 | TOPO1 inhibitor | FAK inhibitor | |
| Combination 22 | TOPO1 inhibitor | PARP inhibitor | FAK inhibitor |
| Combination 23 | TOPO1 inhibitor | PARP inhibitor | Androgen receptor antagonist/degrader |
| Combination 24 | TOPO1 inhibitor | GSPT1 degrader | / |
| Combination 25 | TOPO1 inhibitor | PARP inhibitor | GSPT1 degrader |
| Combination 26 | TOPO1 inhibitor | Tubulin inhibitor | / |
| Combination 27 | TOPO1 inhibitor | Epigenetic regulator | / |
| Combination 28 | Tubulin inhibitor | Immune activator (PD-L1 inhibitor, CBL-B inhibitor, TLR7/8 agonist, PTPN2/1 inhibitor, or STING agonist) | / |
| Combination 29 | Tubulin inhibitor | BCL-2/BCL-XL inhibitor | / |
| Combination 30 | Tubulin inhibitor | BCL-2/BCL-XL inhibitor | MCL-1 inhibitor |
| Combination 31 | Tubulin inhibitor | PI3K/AKT/mTOR inhibitor | / |
| Combination 32 | Tubulin inhibitor | FAK inhibitor | |
| Combination 33 | CDK4/6 inhibitor | MEK inhibitor | / |
| Combination 34 | CDK4/6 inhibitor | MEK inhibitor | EZH2 inhibitor |
| Combination 35 | CDK4/6 inhibitor | Autophagy inhibitor | / |
| Combination 36 | MEK inhibitor | Autophagy inhibitor | / |
| Combination 37 | ERK inhibitor | Autophagy inhibitor | / |
| Combination 38 | CDK4/6 inhibitor | MEK inhibitor | Autophagy inhibitor |
| Combination 39 | CDK4/6 inhibitor | ERK inhibitor | Autophagy inhibitor |
| Combination 40 | CDK4/6 inhibitor | ERK inhibitor | (MEK inhibitor) |
| Combination 41 | CDK4/6 inhibitor | MEK inhibitor | KRAS inhibitor |
| Combination 42 | CDK4/6 inhibitor | MEK inhibitor | SOS1 inhibitor |
| Combination 43 | CDK4/6 inhibitor | MEK inhibitor | FAK inhibitor |
| Combination 44 | CDK4/6 inhibitor | PRMT5 inhibitor | / |
| Combination 45 | CDK4/6 inhibitor | Estrogen receptor antagonist/degrader | / |
| Combination 46 | CDK4/6 inhibitor | Estrogen receptor antagonist/degrader | PI3K/mTOR inhibitor |
| Combination 47 | CDK4/6 inhibitor | MEK inhibitor | GSPT1 degrader |
| Combination 48 | CDK4/6 inhibitor | ERK inhibitor | GSPT1 degrader |
| Combination 49 | CDK4/6 inhibitor | Autophagy inhibitor | GSPT1 degrader |
| Combination 50 | BRD4 inhibitor | EZH2 inhibitor | / |
| Combination 51 | PRMT5 inhibitor | MAT2A inhibitor | / |
| Combination 52 | PRMT5 inhibitor | Gemcitabine | / |
| Combination 53 | PRMT5 inhibitor | CDK4/6 inhibitor | / |
| Combination 54 | PRMT5 inhibitor | PRMT1 inhibitor | / |
| Combination 55 | PRMT5 inhibitor | PRMT1 inhibitor | CDK4/6 inhibitor |
| Combination 56 | PRMT5 inhibitor | PRMT1 inhibitor | MEK inhibitor |
| Combination 57 | RAF inhibitor | MEK inhibitor | / |
| Combination 58 | EGFR inhibitor | MEK inhibitor | / |
| Combination 59 | RAF inhibitor | MEK inhibitor | SHP2 inhibitor |
| Combination 60 | CDK4/6 inhibitor | HIF-2alpha inhibitor | / |
| Combination 61 | TOPO1 inhibitor | WEE1 inhibitor | / |
| Combination 62 | WEE1 inhibitor | CHK1 inhibitor | / |
| Combination 63 | TOPO1 inhibitor | TOPO1 inhibitor | / |
| Combination 64 | CDK inhibitor | MEK inhibitor | |
| Combination 65 | FAK inhibitor | Smo inhibitor | / |
| Combination 66 | PLK1 inhibitor | CDK inhibitor | / |
| Combination 67 | PLK1 inhibitor | CDK inhibitor | PARP inhibitor/ |
| Combination 68 | TOPO1 inhibitor | PLK1 inhibitor | / |
| Combination 69 | TOPO1 inhibitor | PLK1 inhibitor | PARP inhibitor/ |
| Combination 70 | TOPO1 inhibitor | PLK1 inhibitor | ATR inhibitor/ |
| Combination 71 | TOPO1 inhibitor | NMT inhibitor | / |
| Combination 72 | Tubulin inhibitor | NMT inhibitor | / |
more preferably,
(i) at least one of D₁, D₂, and D₃ is a cytotoxic drug, the cytotoxic drug is selected from drugs targeting topoisomerase, for example a TOPO1 inhibitor, at least another one is a tumor-targeted therapeutic drug, the tumor-targeted therapeutic drug is a drug targeting DNA damage response (DDR) or "synthetic lethality" related pathways, selected from PARP inhibitors, ATR inhibitors, CHK1 inhibitors, ATM inhibitors, DNA-PK inhibitors, WEE1 inhibitors, POLQ inhibitors, CDK12 inhibitors, USP1 inhibitors, PKMYT1 inhibitors, Rad51 inhibitors;
(ii) at least two of D₁, D₂, and D₃ are cytotoxic drugs and are independently selected from drug units targeting topoisomerase and drug units targeting tubulin, for example TOPO1 inhibitors, TOPO2 inhibitors, and tubulin inhibitors;
(iii) at least two or all three of D₁, D₂, and D₃ are tumor-targeted therapeutic drugs, and are independently selected from drug units targeting DNA damage response (DDR) or "synthetic lethality" related pathways, drug units targeting epigenetics, drug units targeting apoptosis-related pathways, drug units targeting cell cycle pathways, drug units targeting transcription factors, and drug units targeting immune activation pathway-related targets, comprising: inhibitors of EFGR pathway-related targets; Ras-Raf-MAPK pathway inhibitors; PI3K/AKT/mTOR pathway inhibitors; cell cycle pathway inhibitors; cGAS-STING signaling pathway agonists; estrogen receptor antagonists; androgen receptor antagonists; glucocorticoid receptor modulators; autophagy inhibitors; FAK inhibitors; Smo inhibitors; BTK inhibitors; PDE4 inhibitors; Lck inhibitors; PLK1 inhibitors; TLR7/8 modulators; N-myristoyltransferase (NMT) inhibitors;
(iv) one of D₁, D₂, and D₃ is a cytotoxic drug, the cytotoxic drug is selected from drug units targeting topoisomerase and drug units targeting tubulin, for example a TOPO1 inhibitor or a tubulin inhibitor, and the other one or two are tumor-targeted therapeutic drugs, the tumor-targeted therapeutic drugs are selected from drug units targeting DNA damage response (DDR) or "synthetic lethality" related pathways, drug units targeting epigenetics, drug units targeting apoptosis-related pathways, drug units targeting cell cycle pathways, drug units targeting transcription factors, and drug units targeting immune activation pathway-related targets, comprising: inhibitors of EFGR pathway-related targets; Ras-Raf-MAPK pathway inhibitors; PI3K/AKT/mTOR pathway inhibitors; cell cycle pathway inhibitors; cGAS-STING signaling pathway agonists; estrogen receptor antagonists; androgen receptor antagonists; glucocorticoid receptor modulators; autophagy inhibitors; FAK inhibitors; Smo inhibitors; BTK inhibitors; PDE4 inhibitors; Lck inhibitors; PLK1 inhibitors; TLR7/8 modulators; N-myristoyltransferase (NMT) inhibitors;
more preferably, D₁, D₂, and D₃ are selected from the combinations shown in Table A.

39. The compound, or a tautomer, a mesomer, a racemate, an enantiomer, or a diastereomer thereof, or a deuterated compound form thereof, or a mixture form thereof, or a pharmaceutically acceptable salt, prodrug, or solvate thereof, according to any one of claims 27 to 38, wherein the compound is selected from formulas **C₁ to C190:** 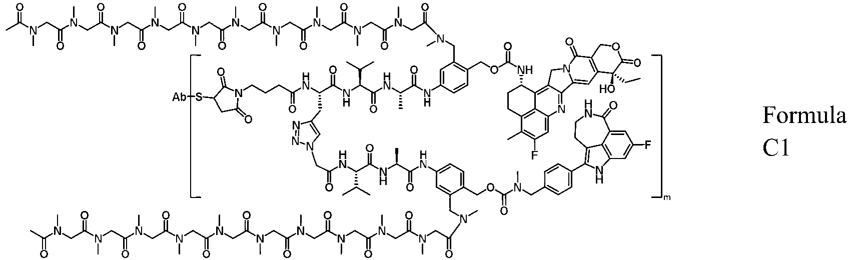 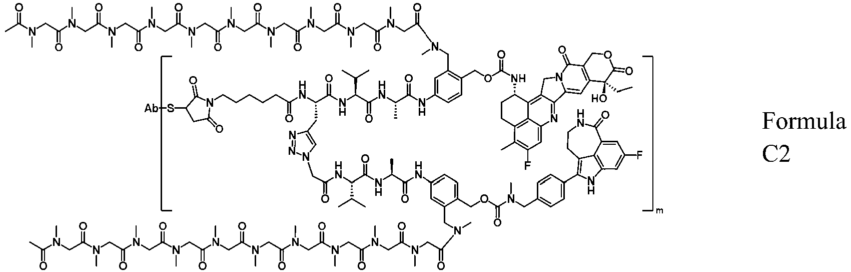 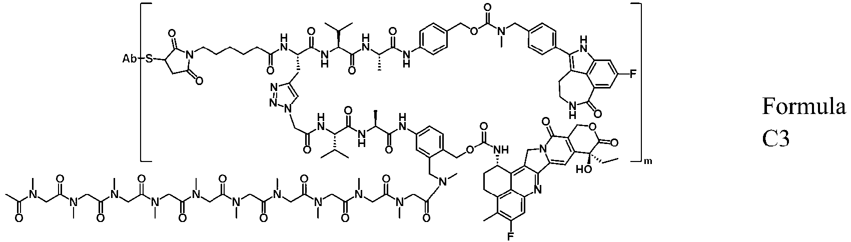 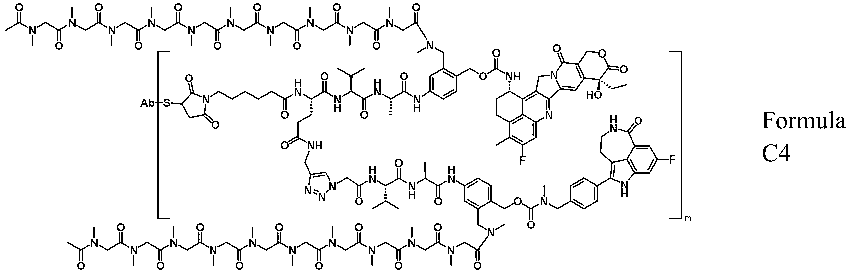 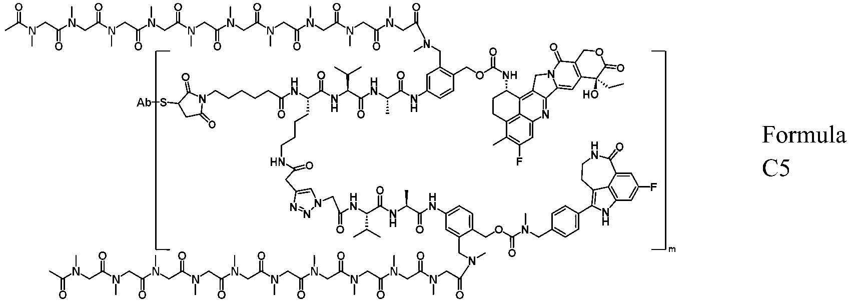 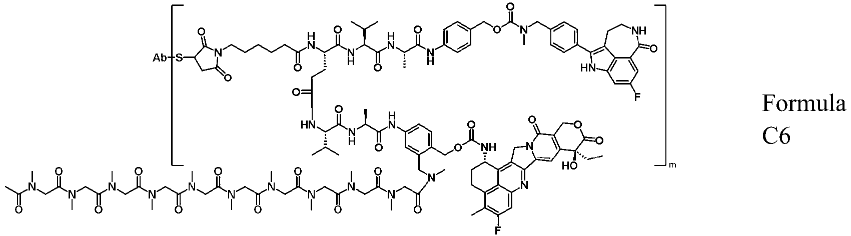 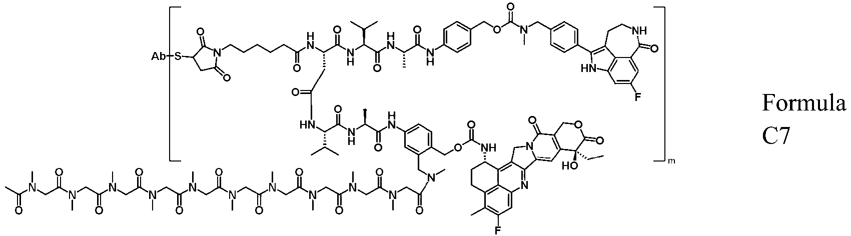 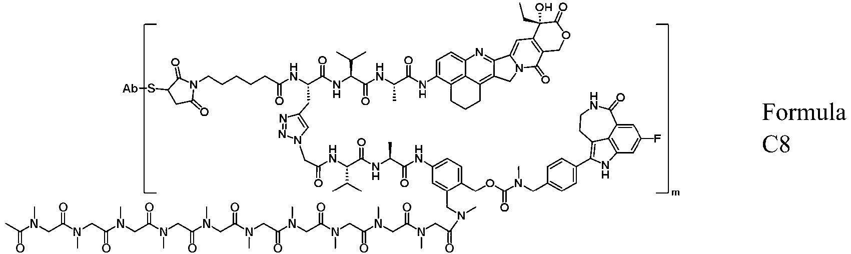 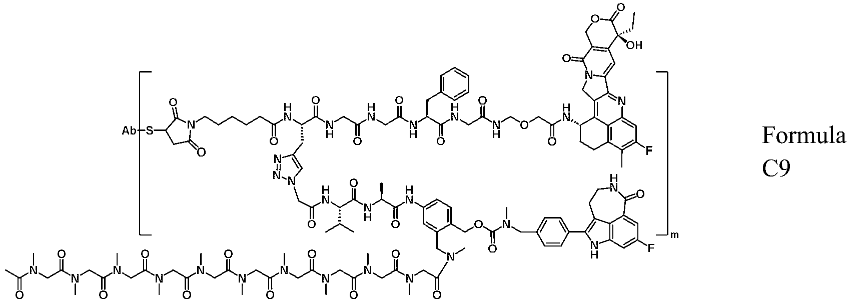 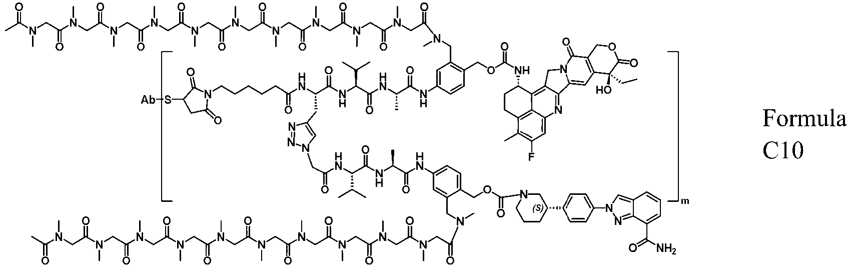 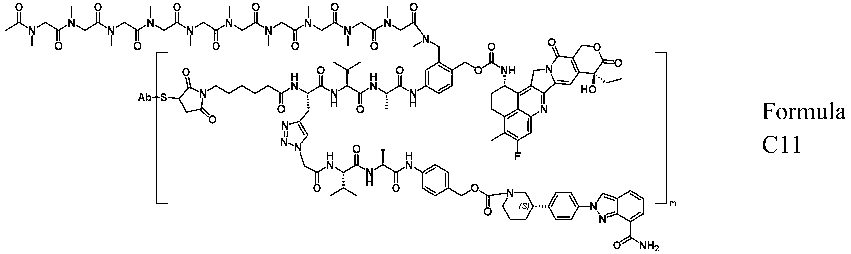 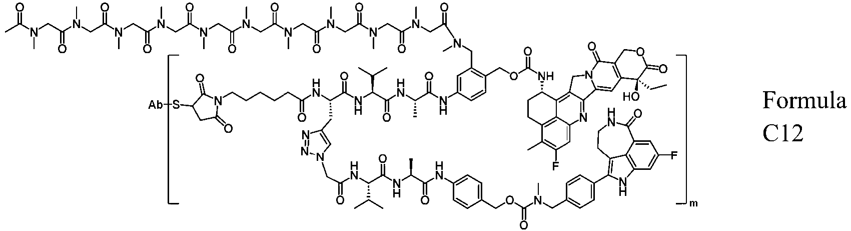 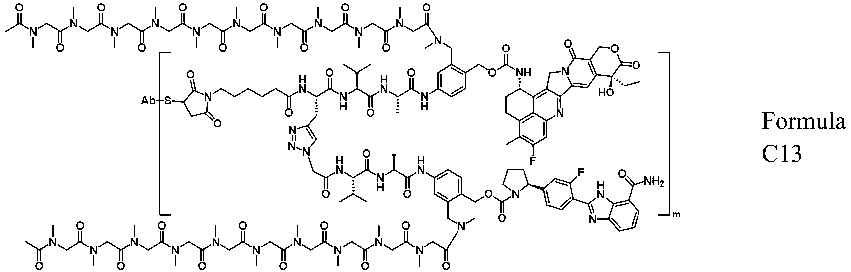 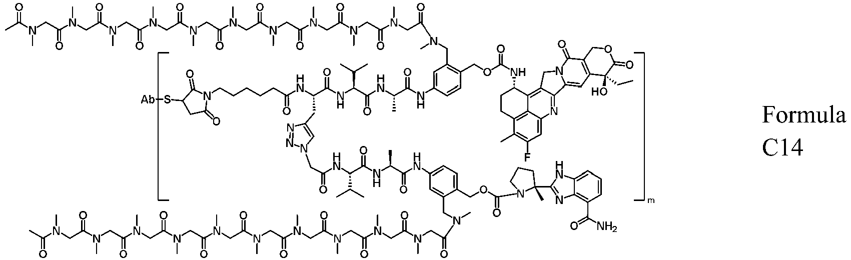 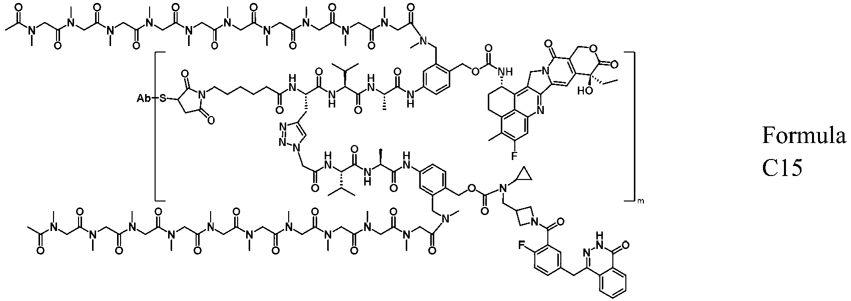 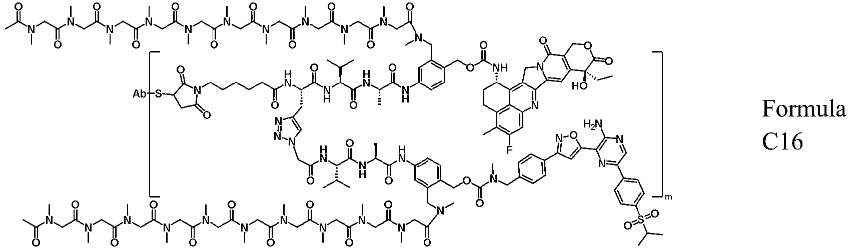 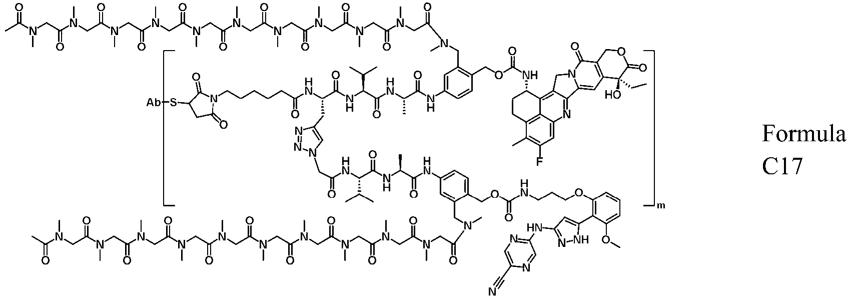 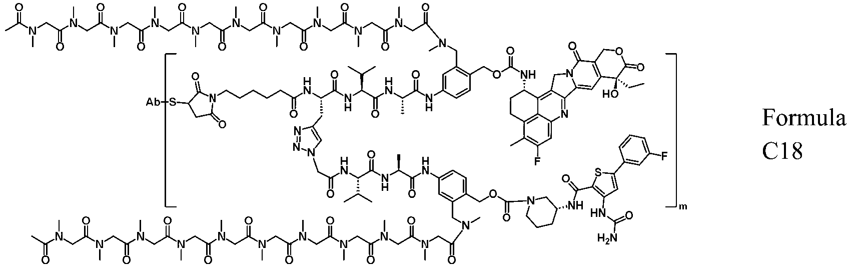 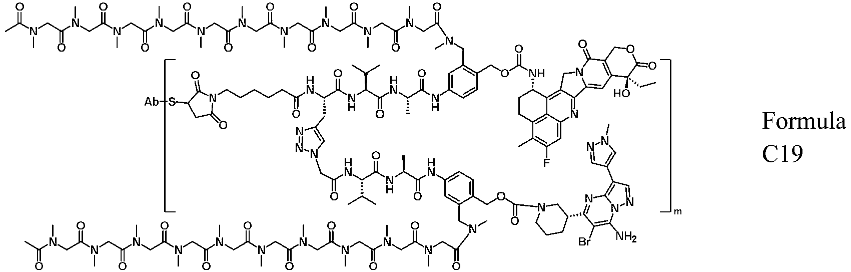 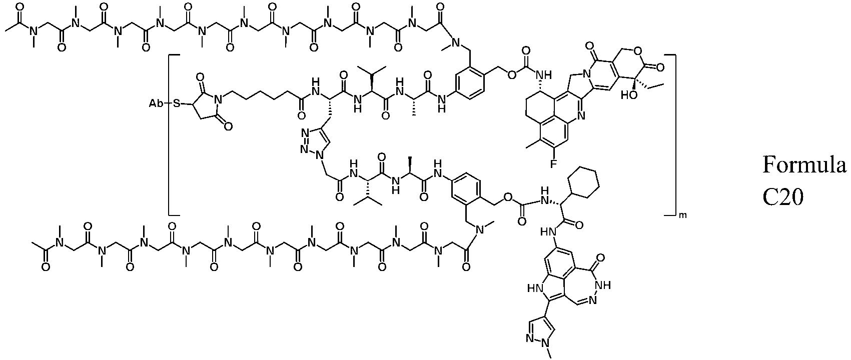 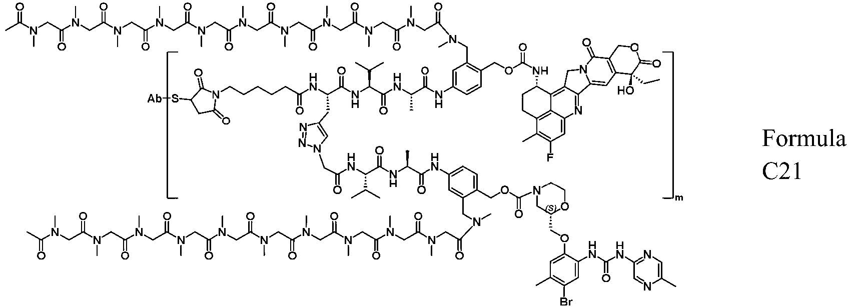 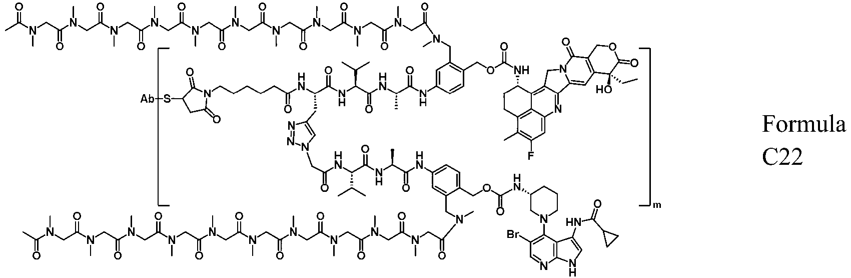 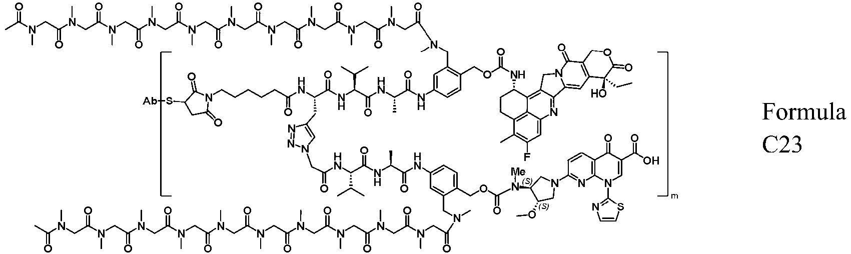 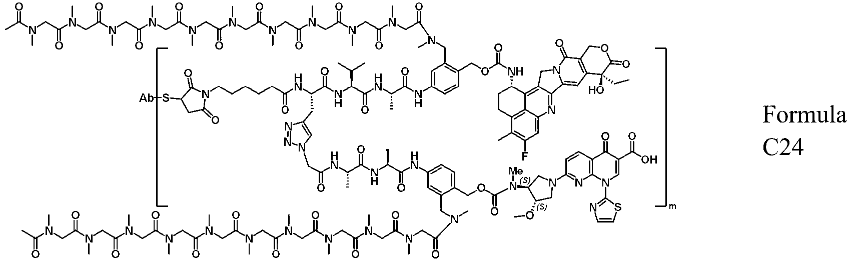 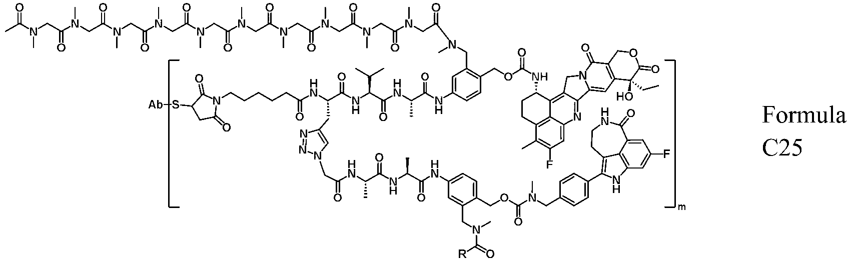 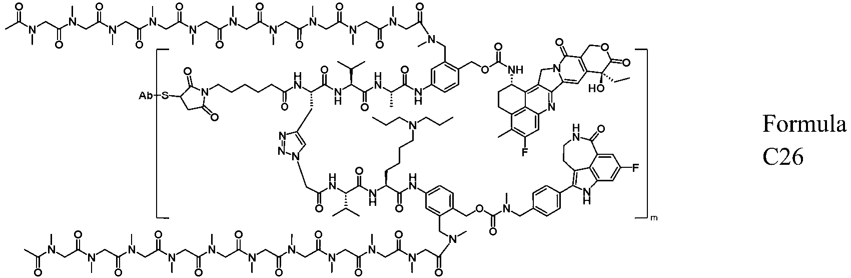 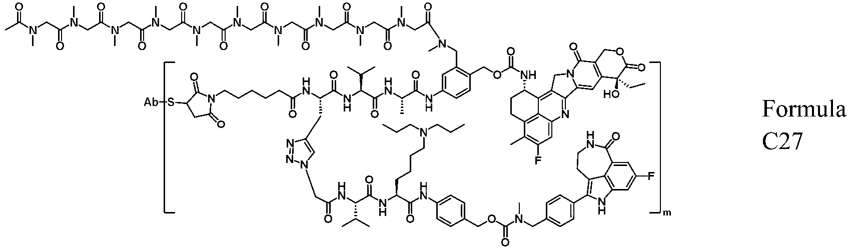 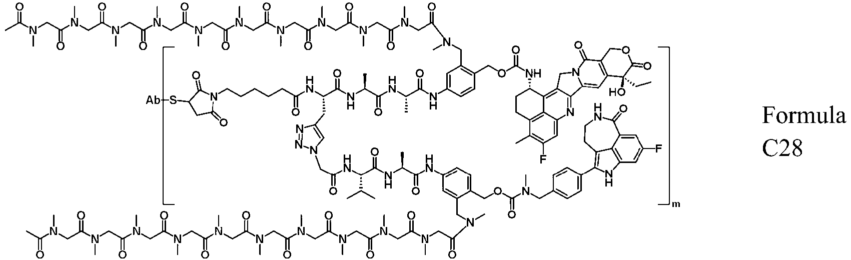 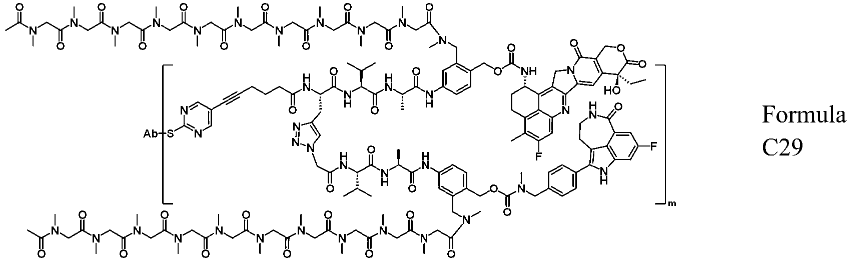 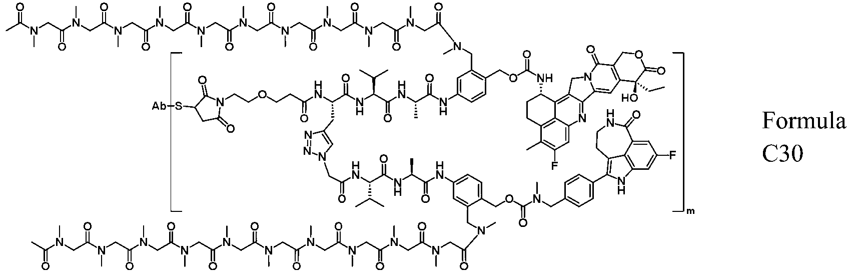 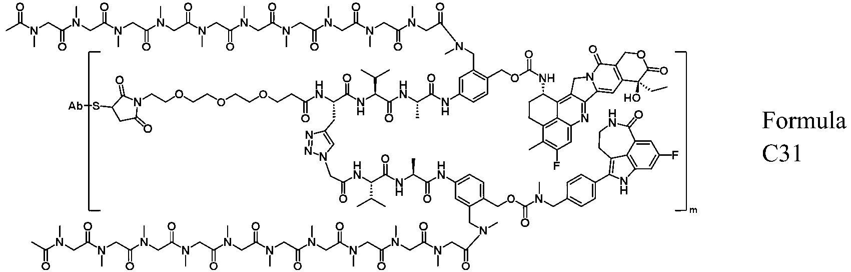 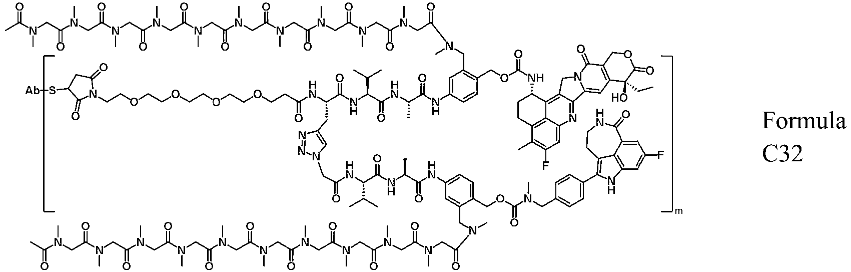 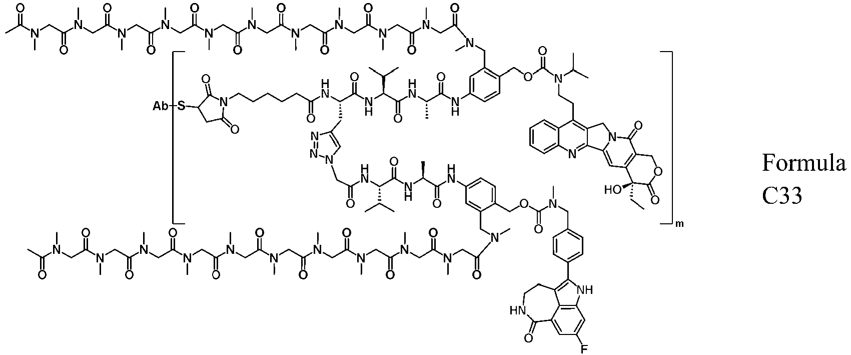 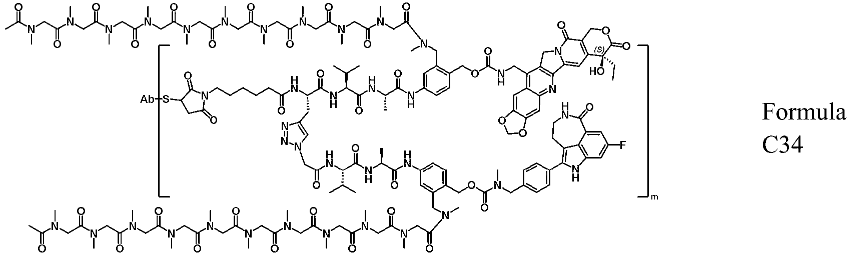 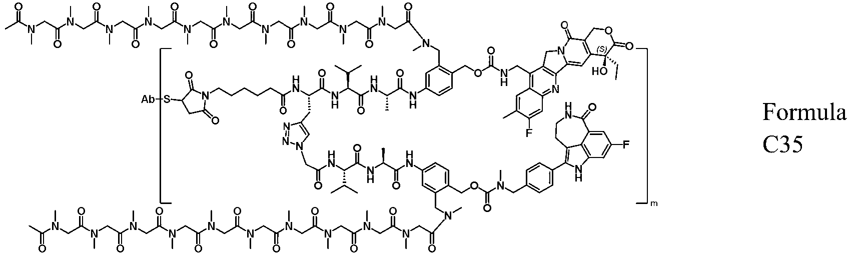 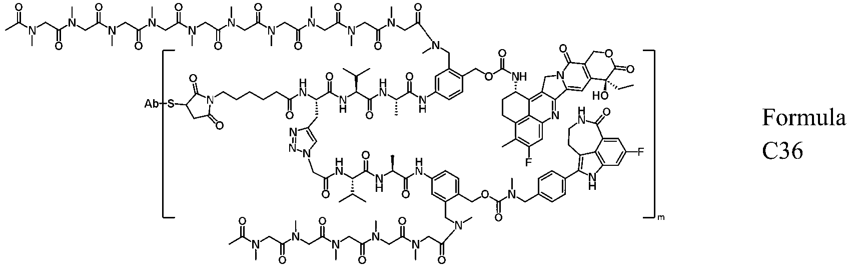 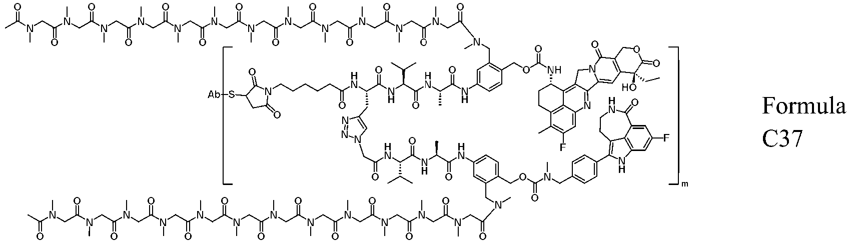 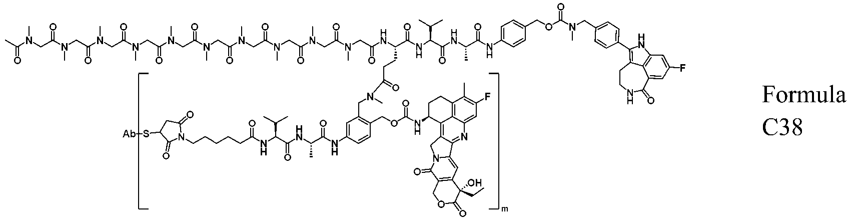 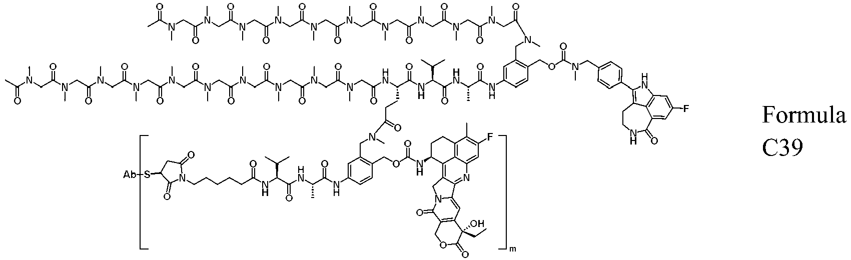 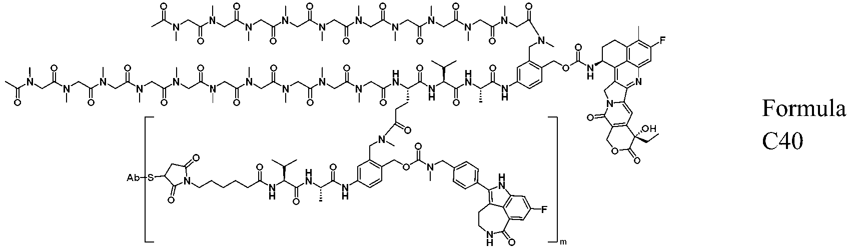 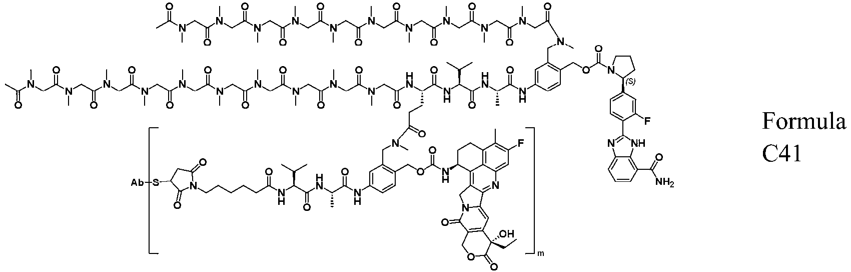 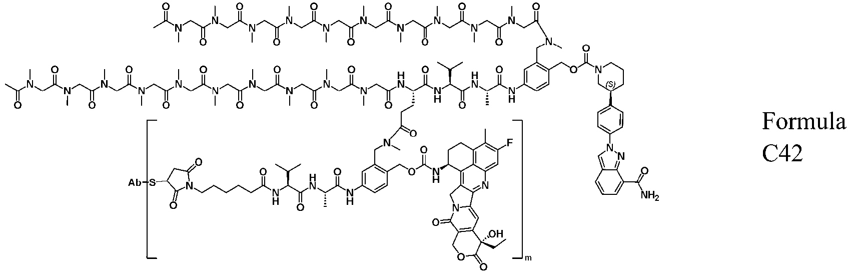 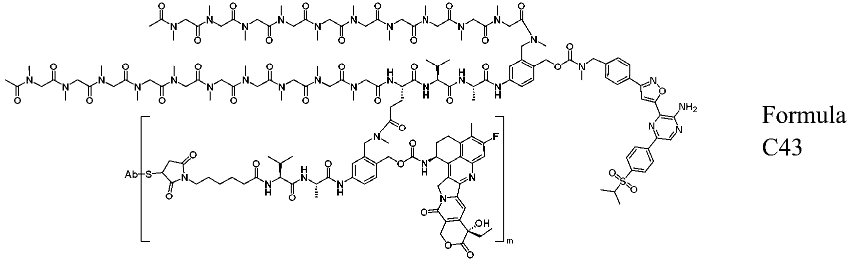 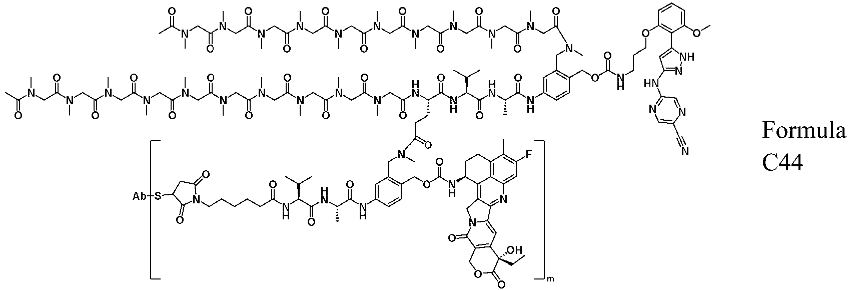 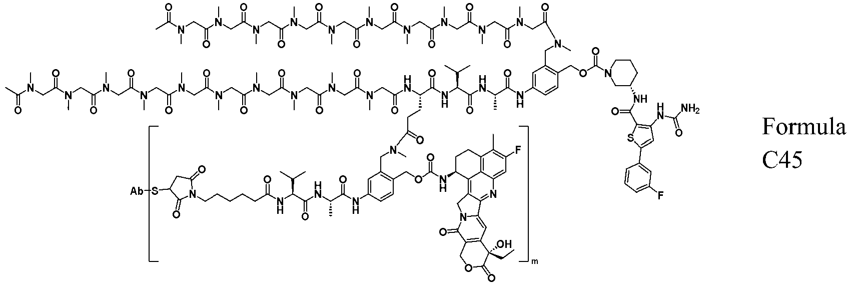 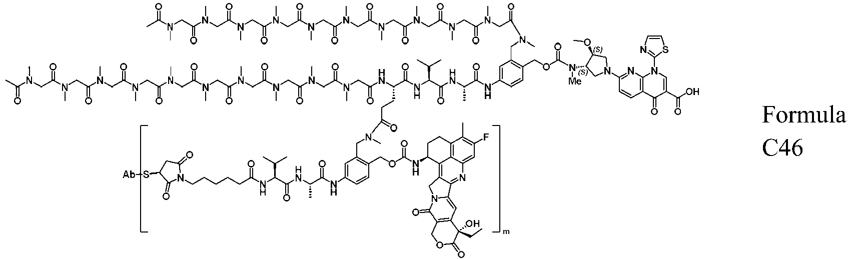 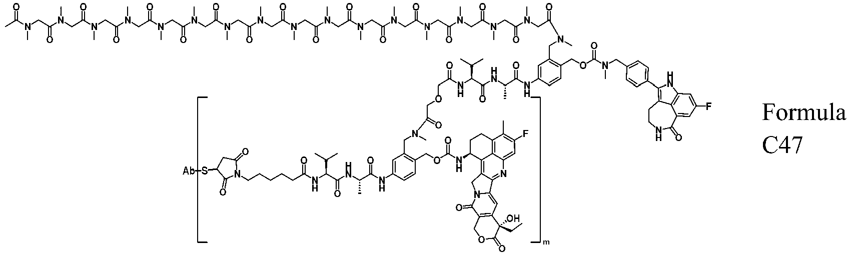 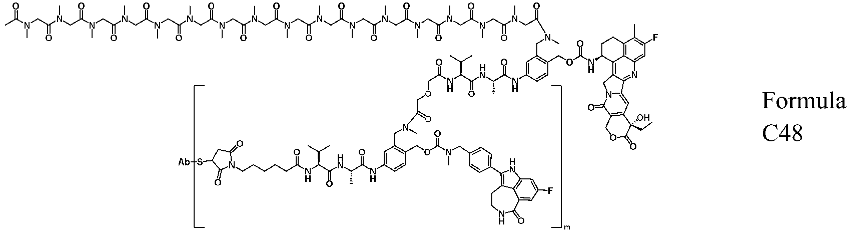 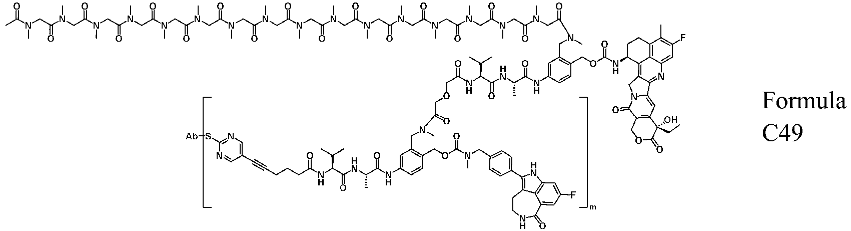 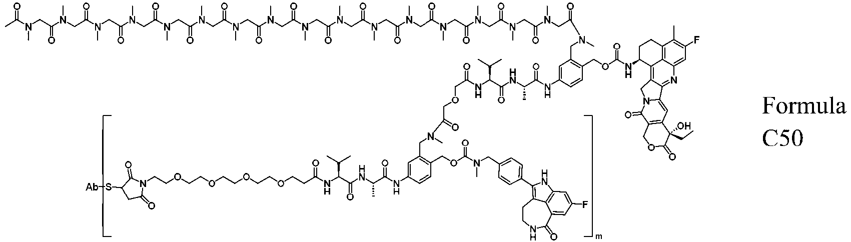 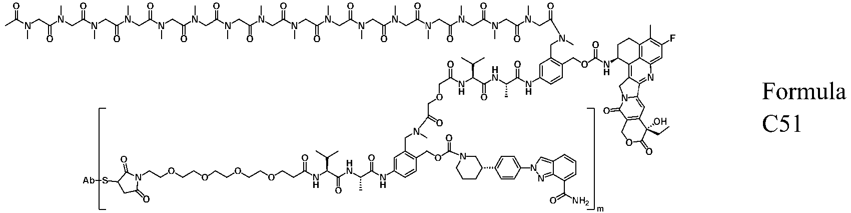 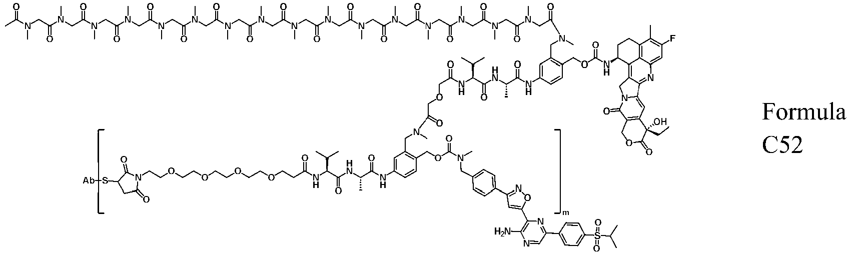 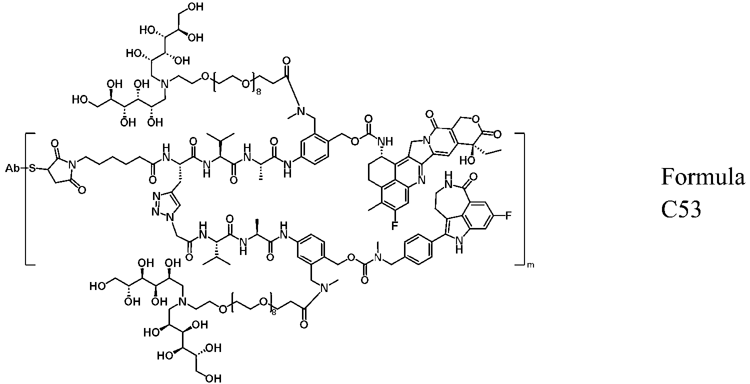 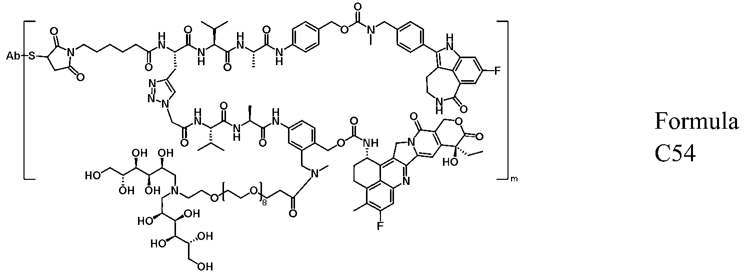 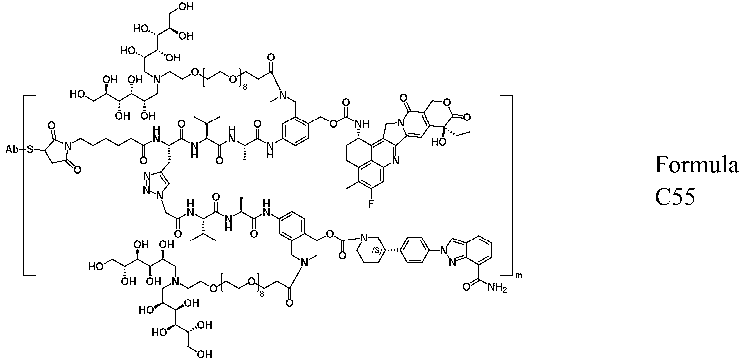 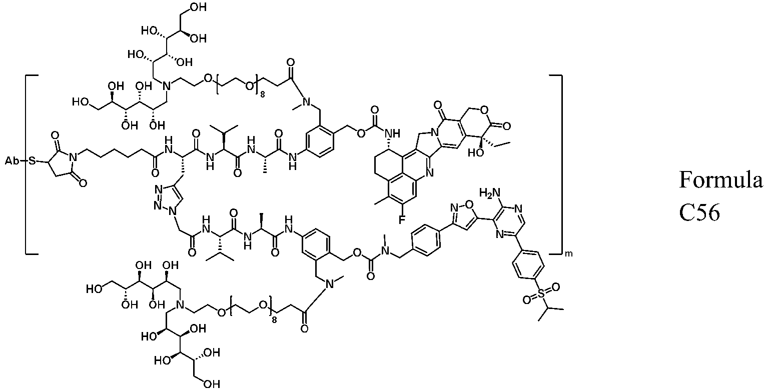 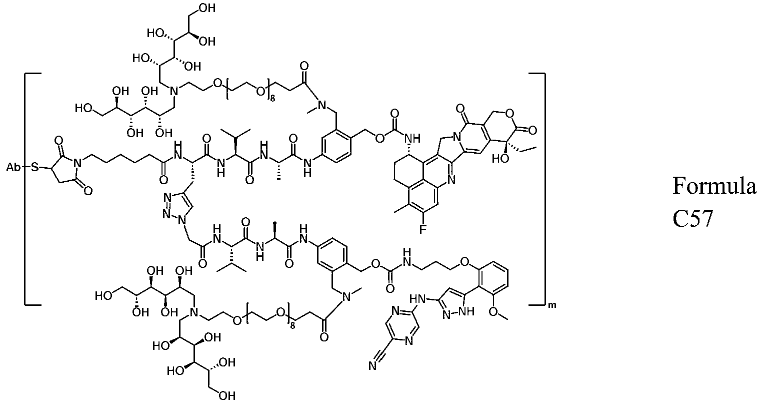 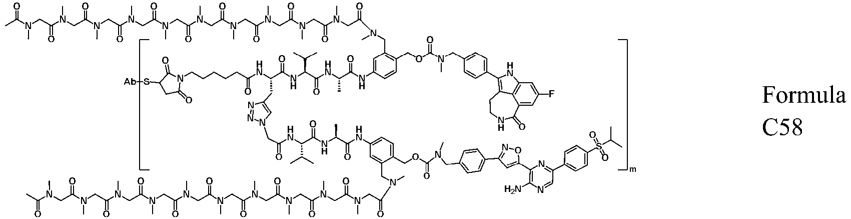 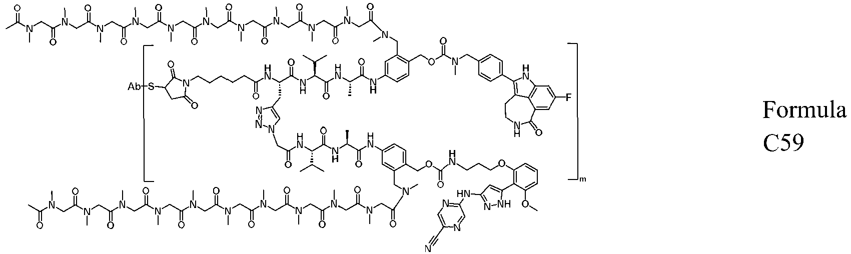 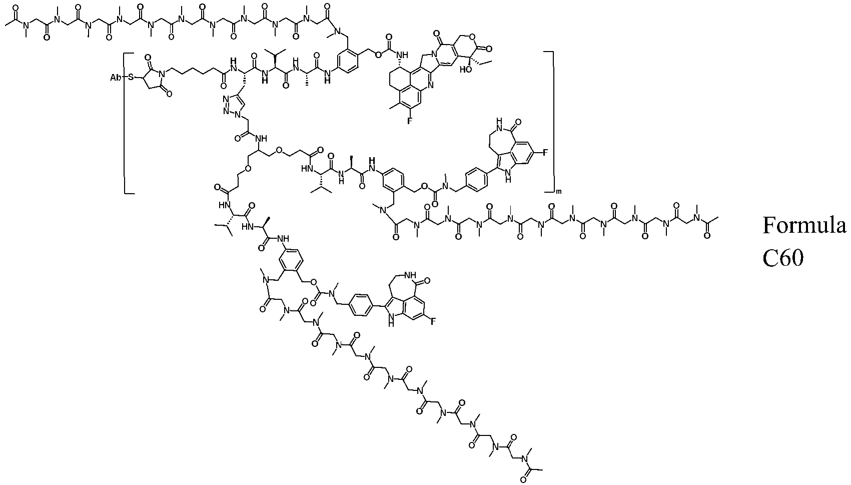 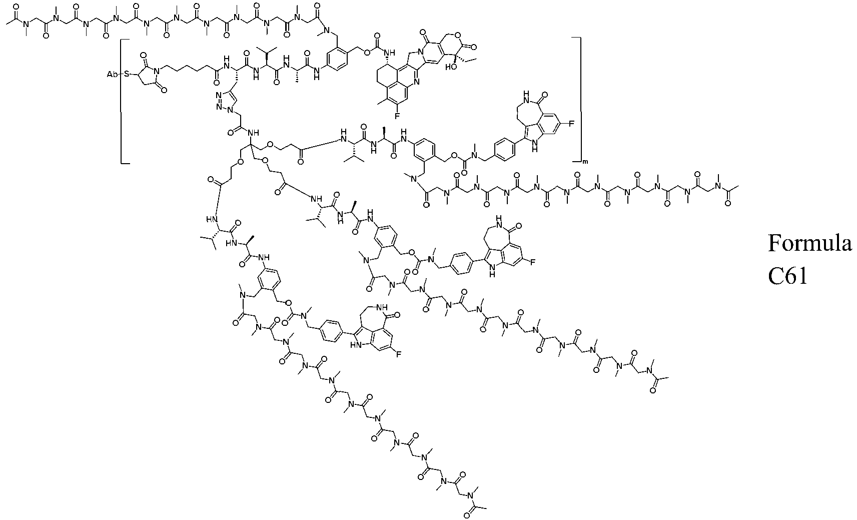 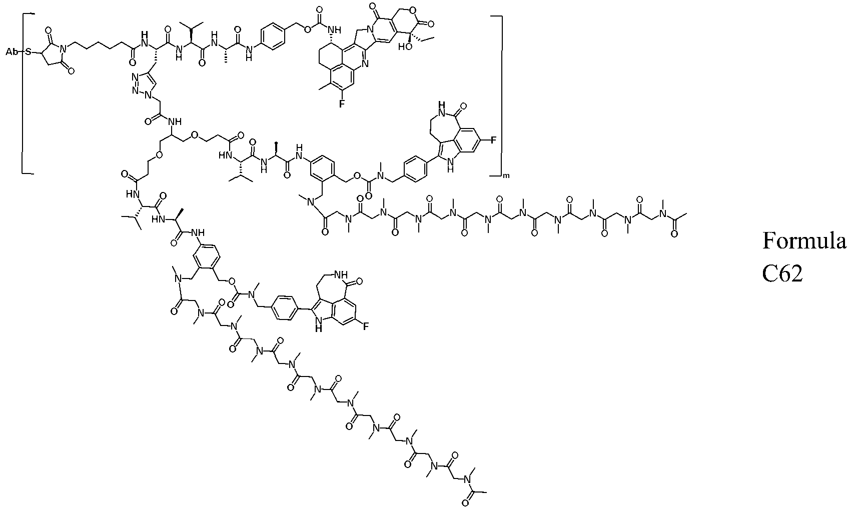 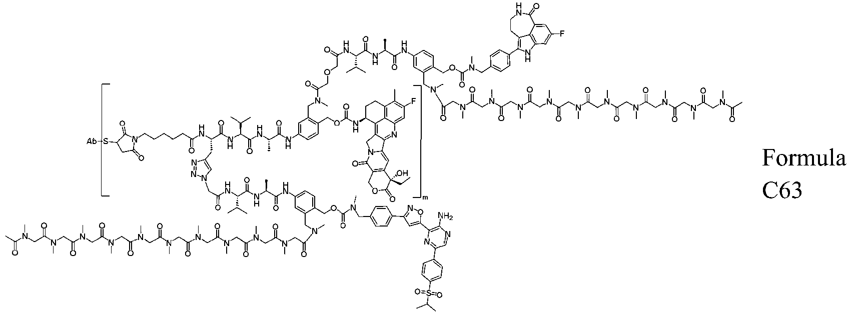 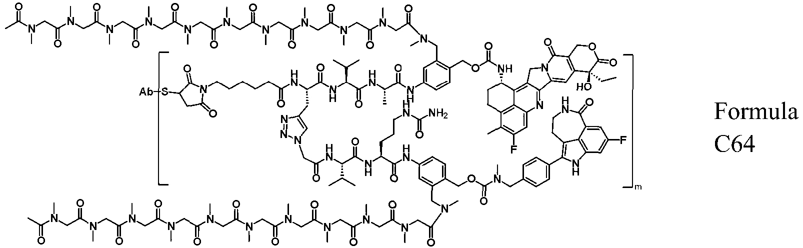 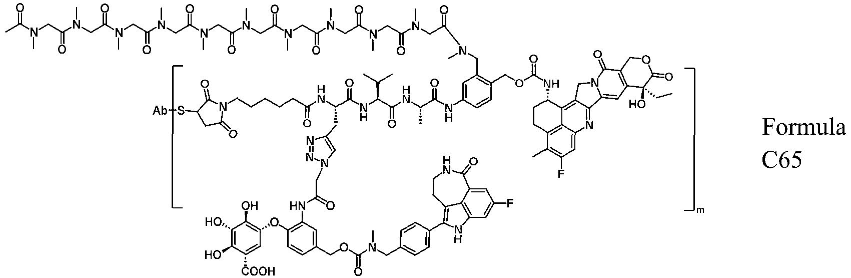 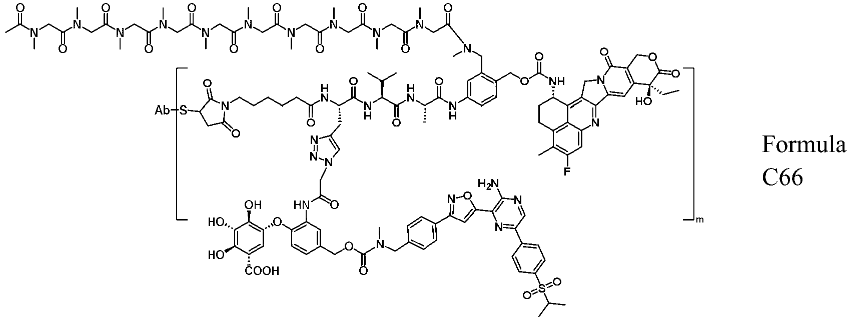 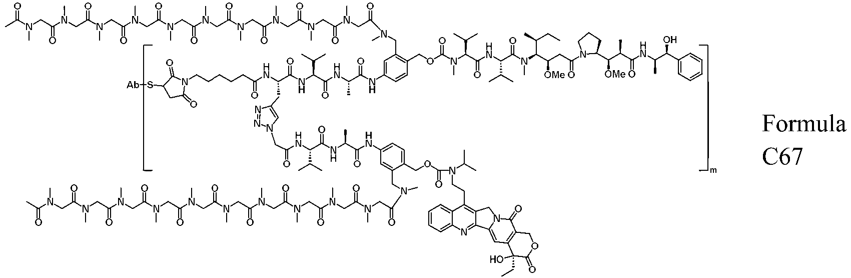 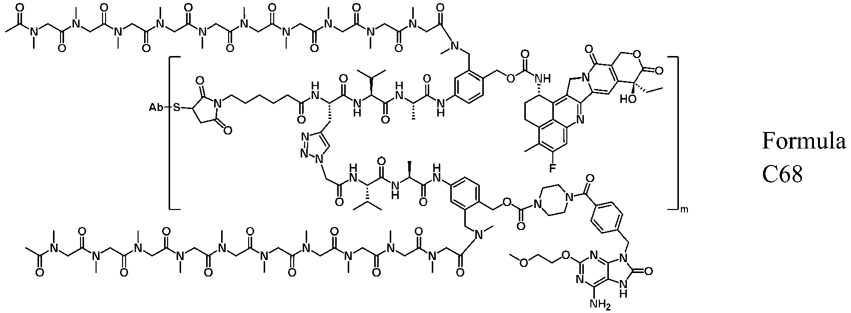 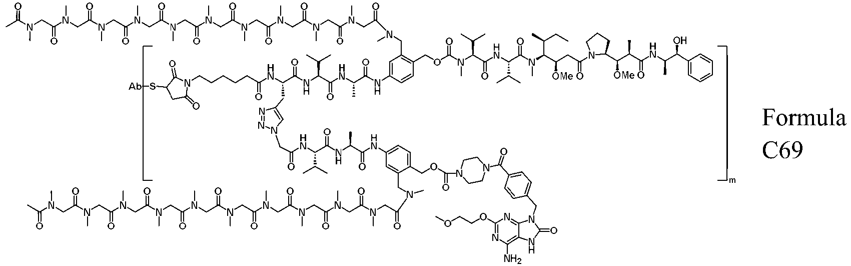 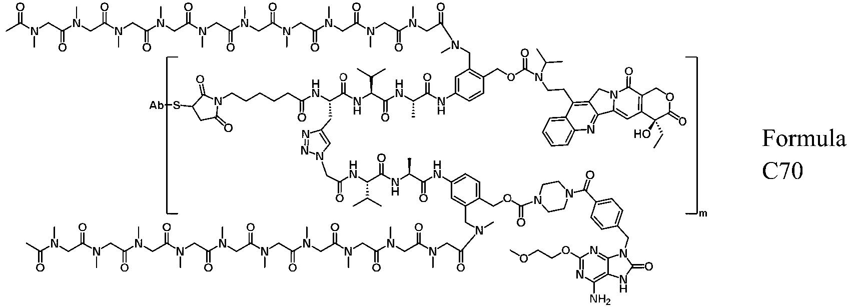 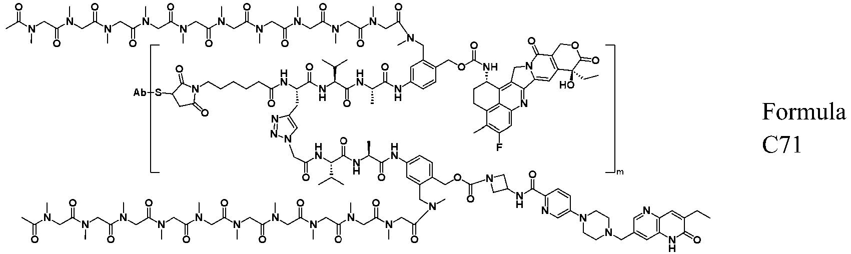 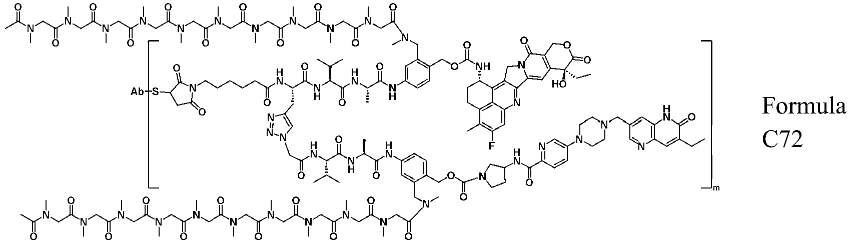 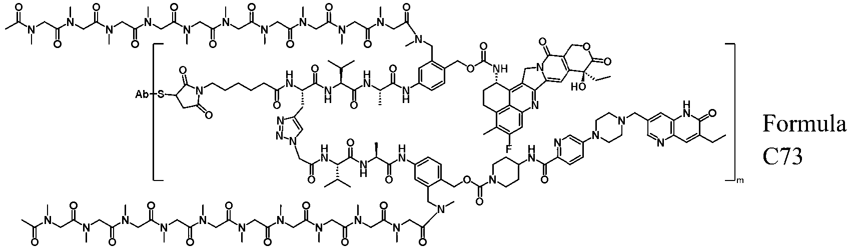 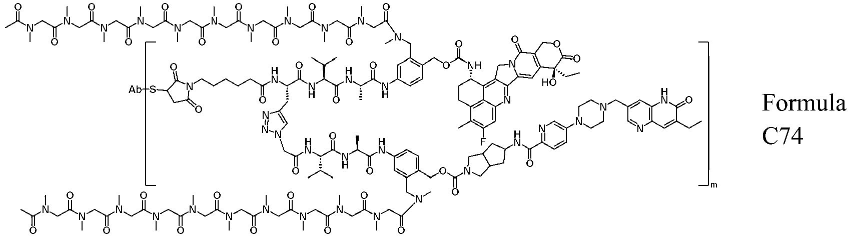 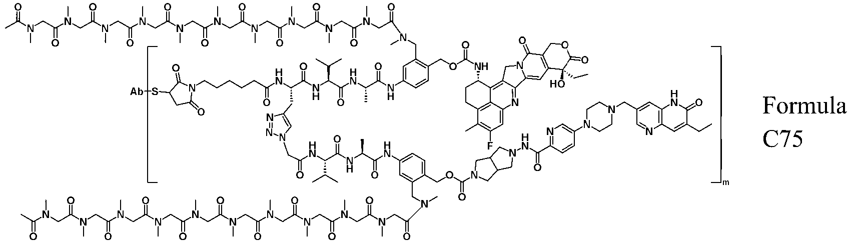 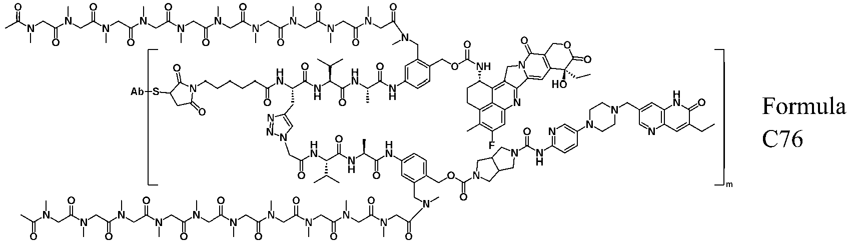 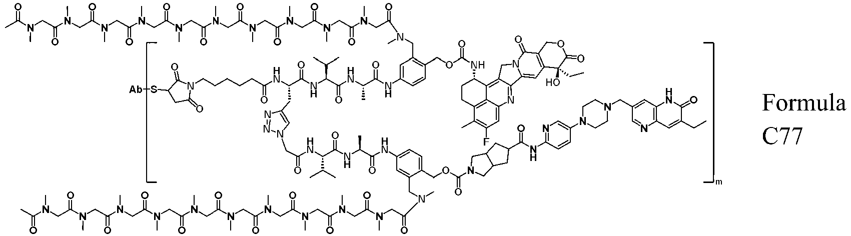 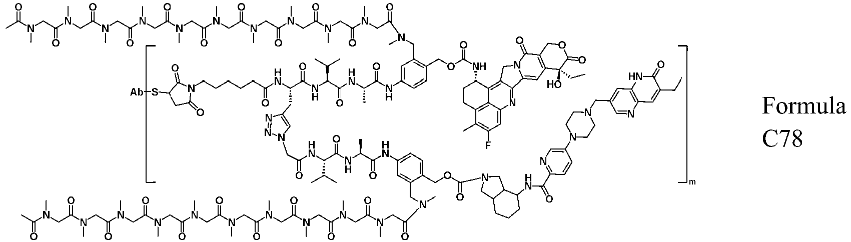 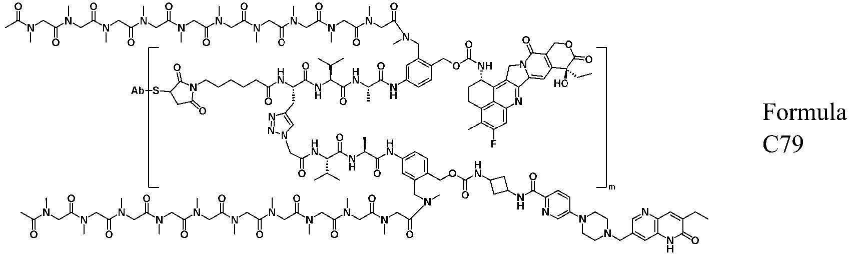 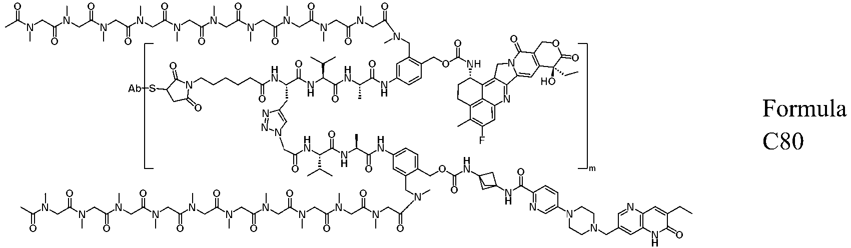 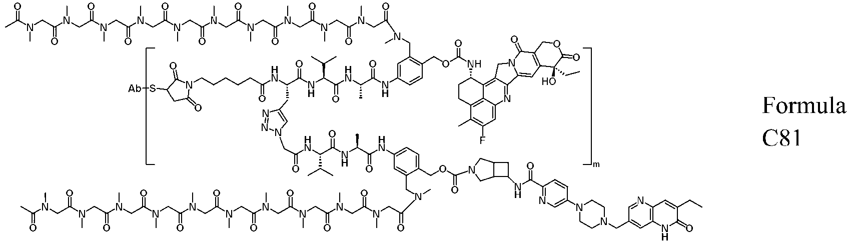 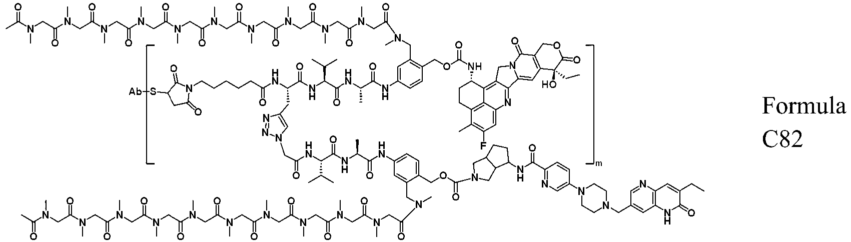 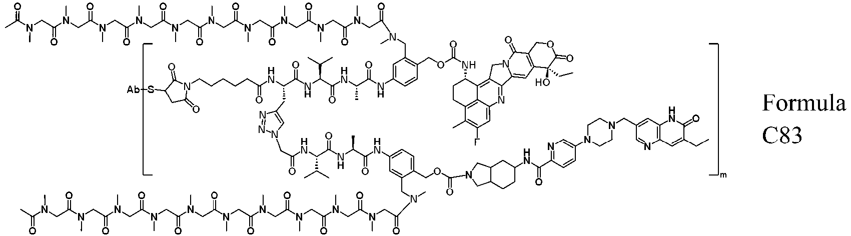 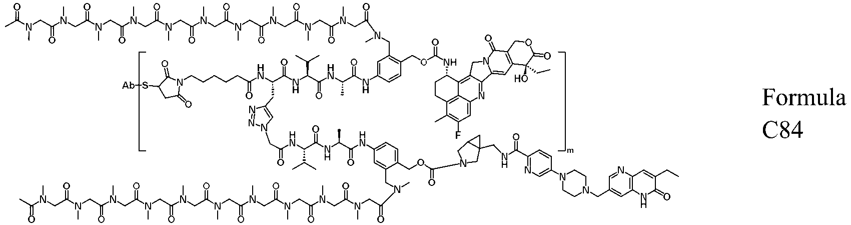 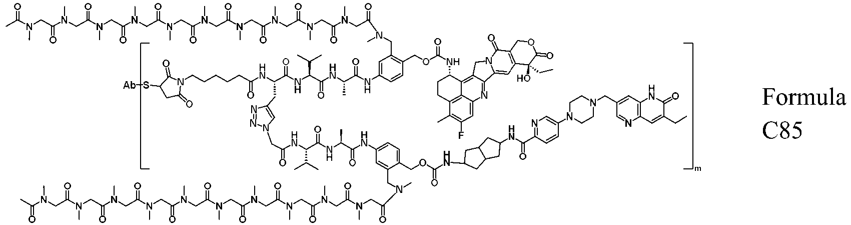 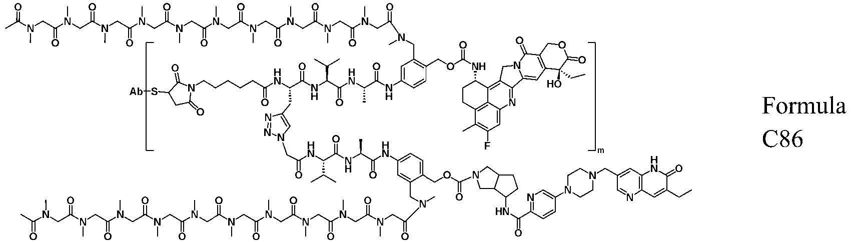 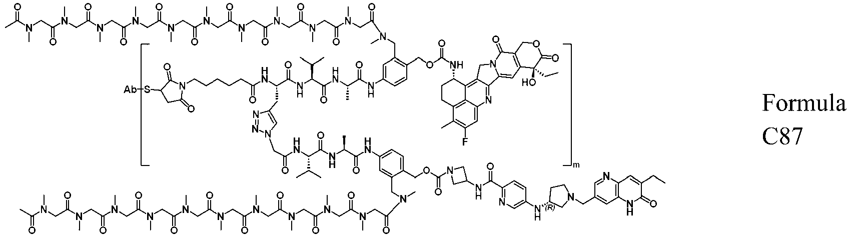 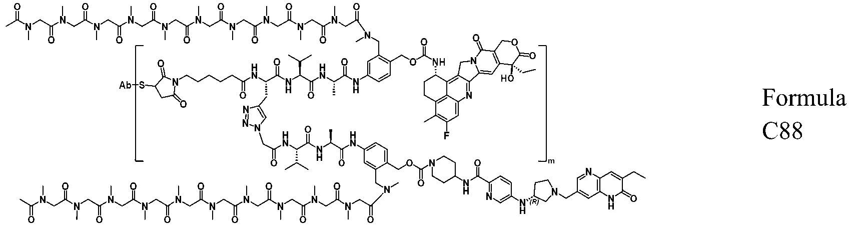 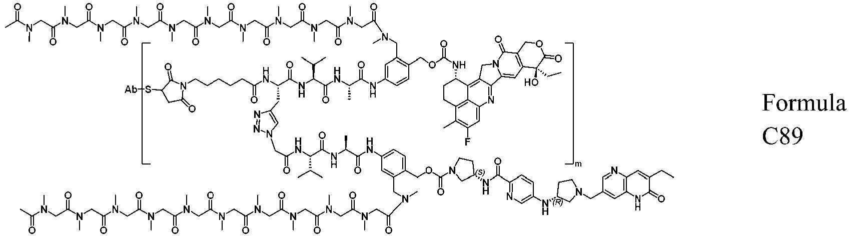 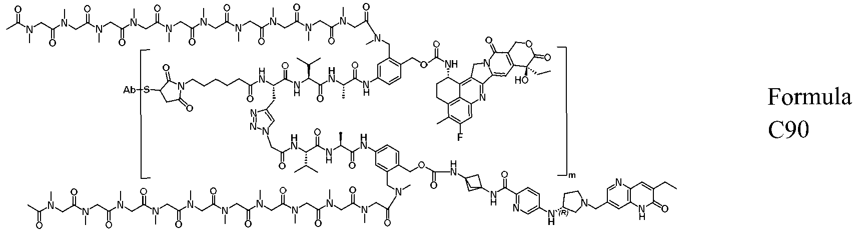 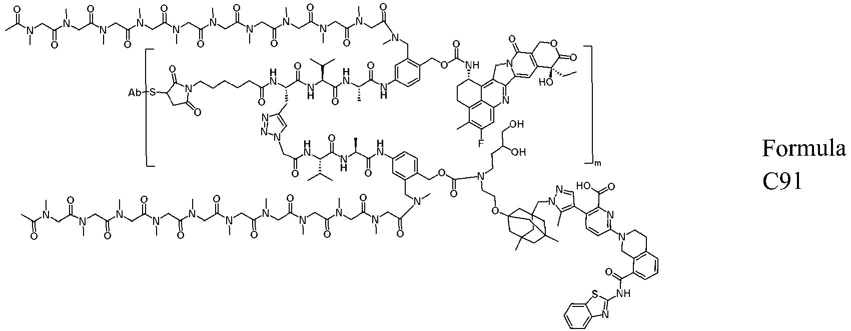 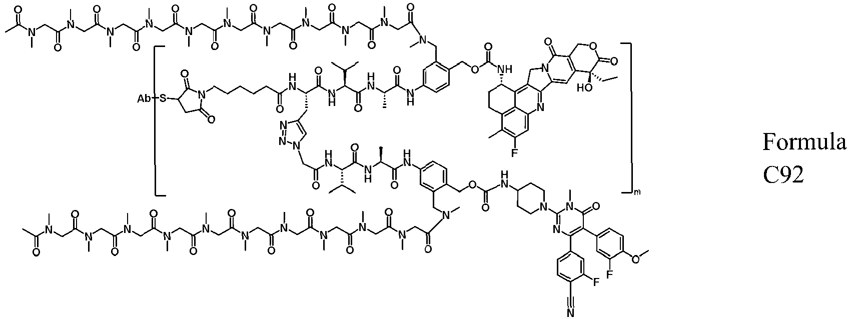 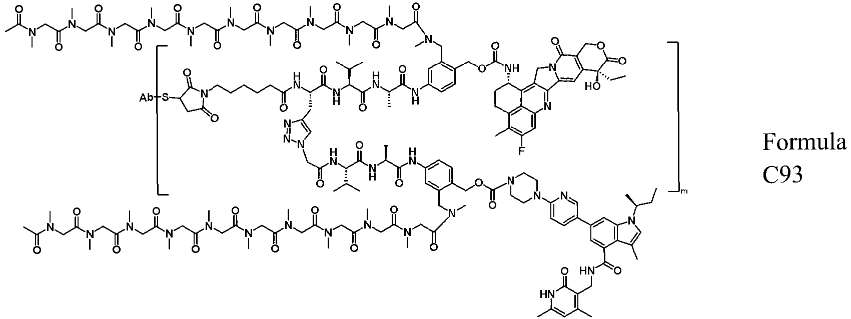 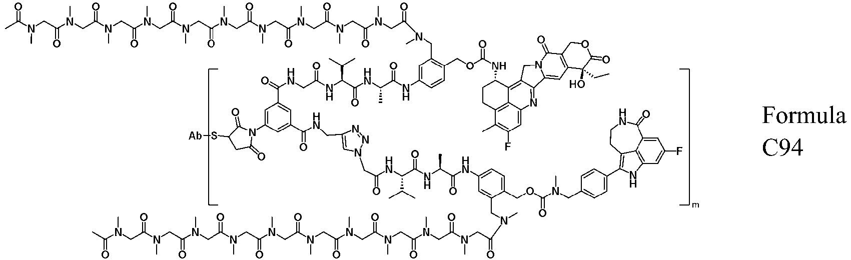 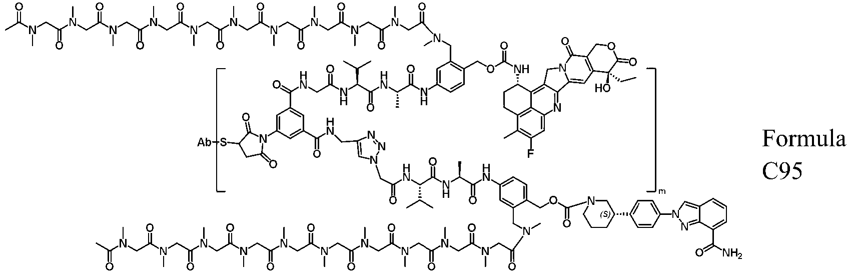 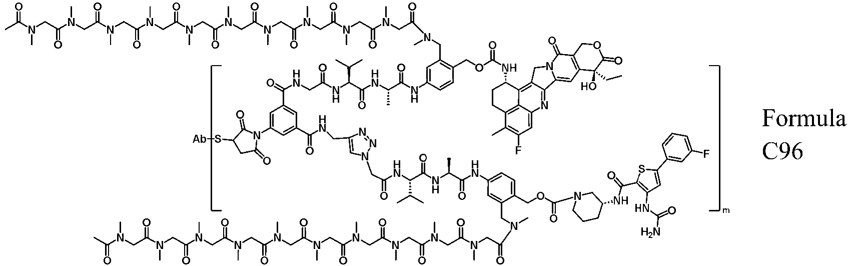 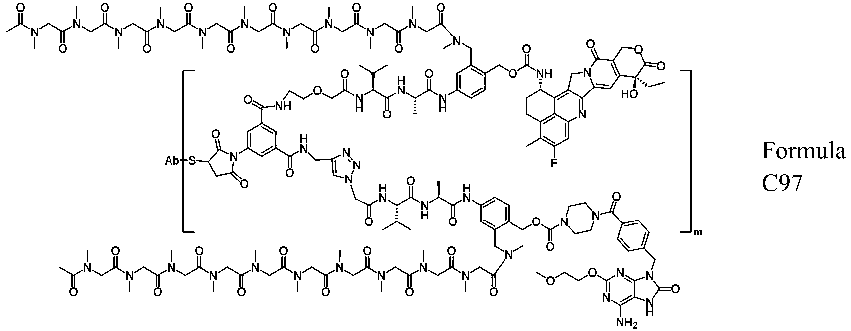 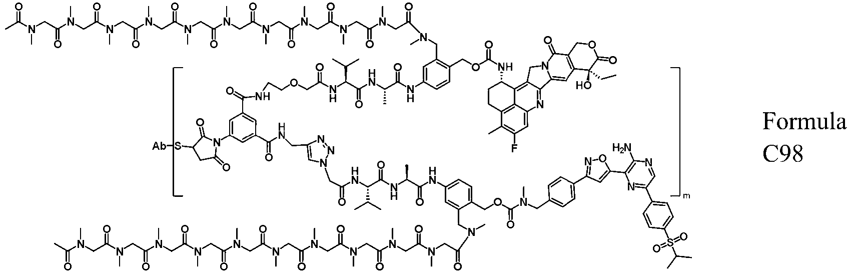 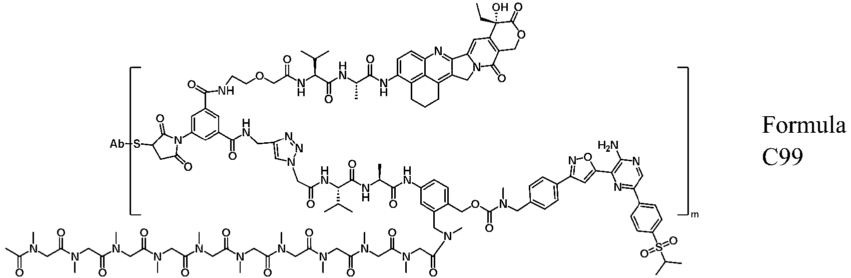 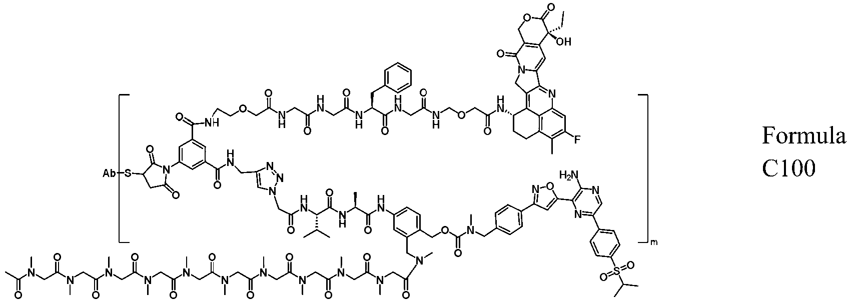 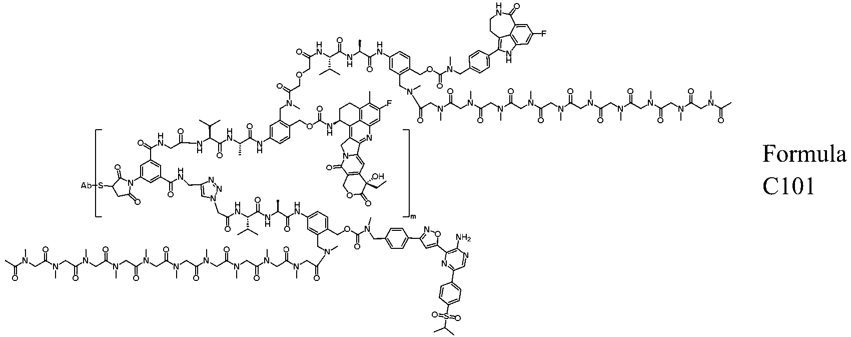 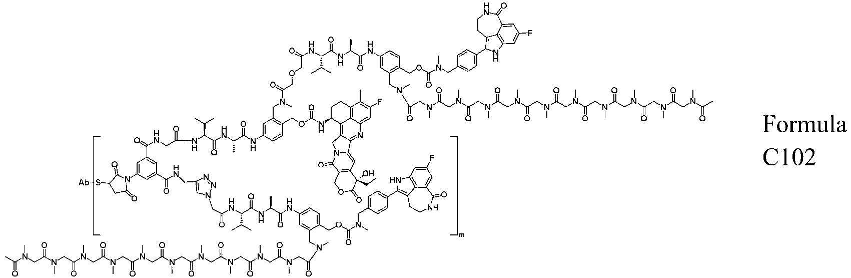 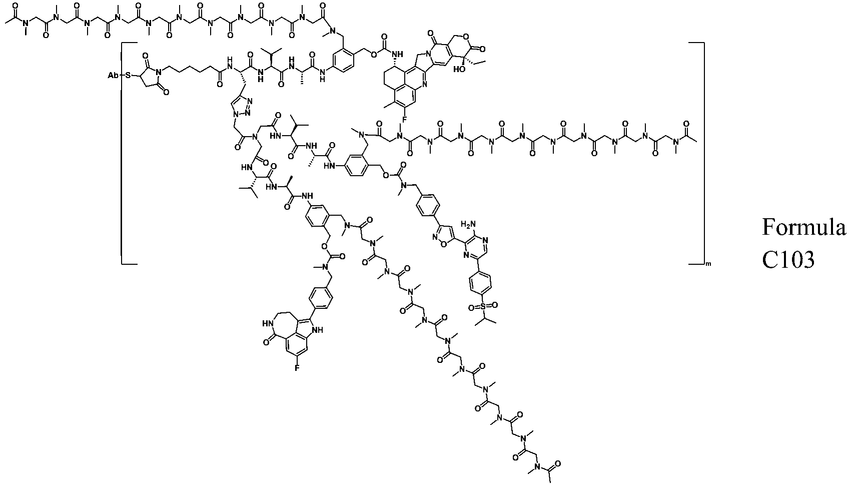 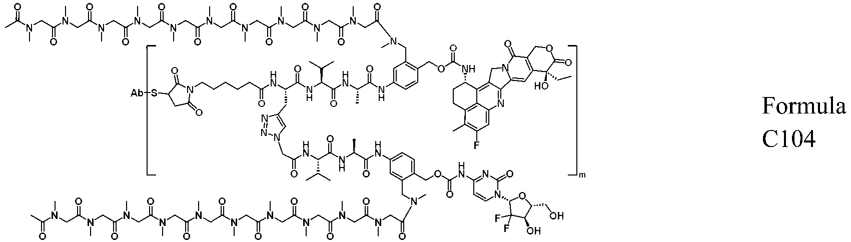 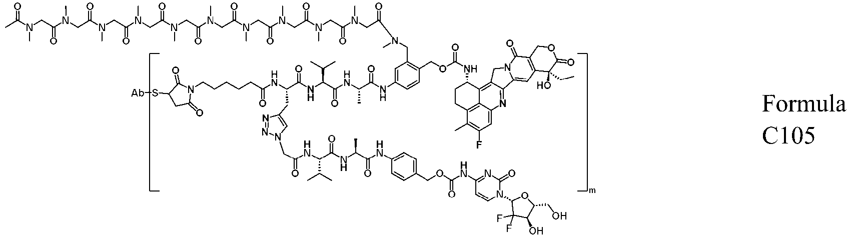 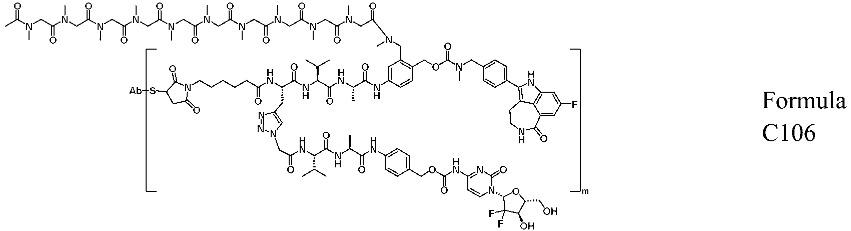 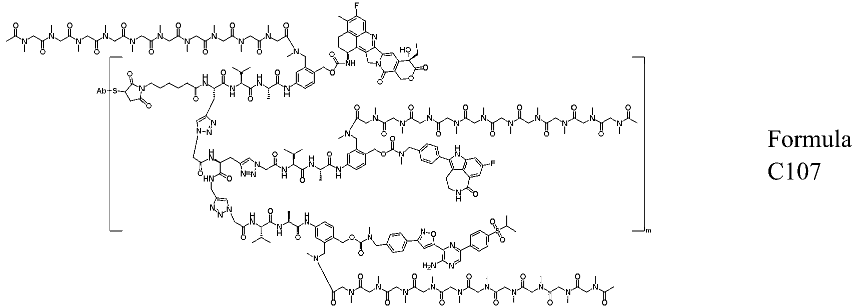 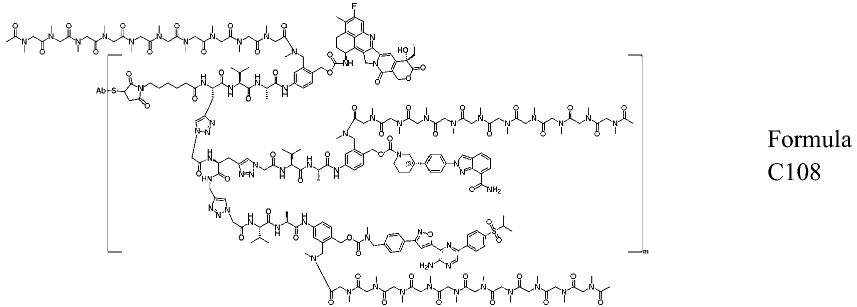 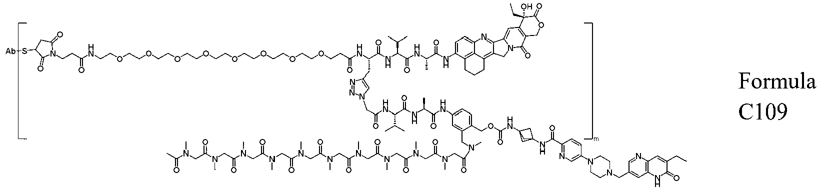 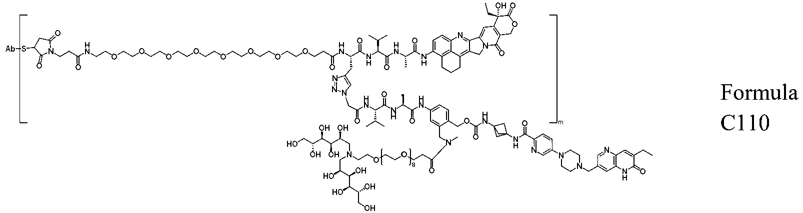 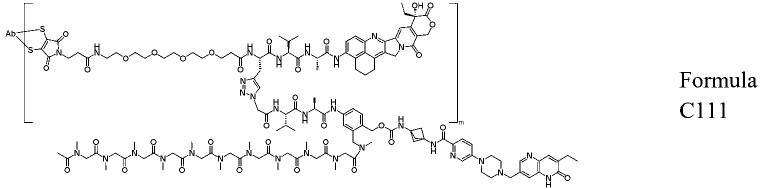 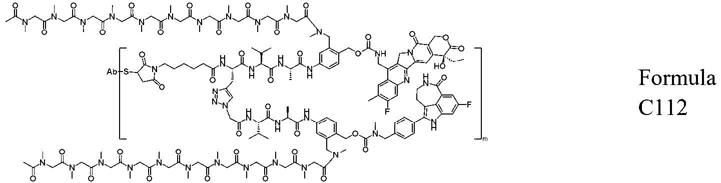 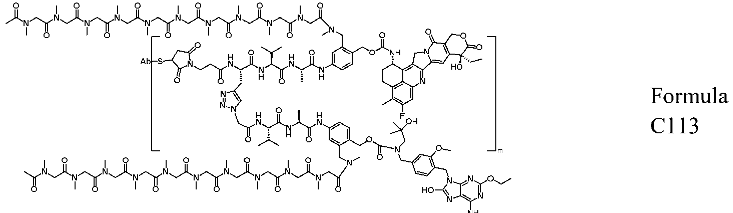 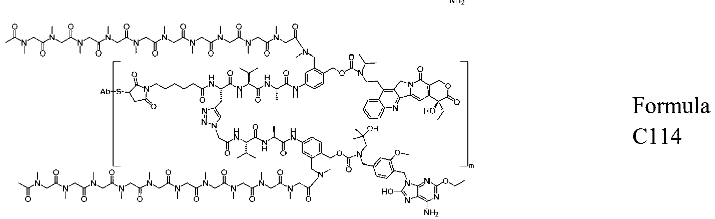 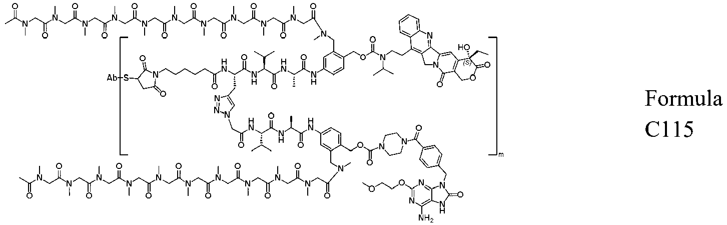 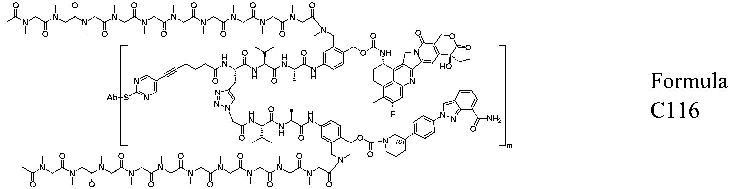 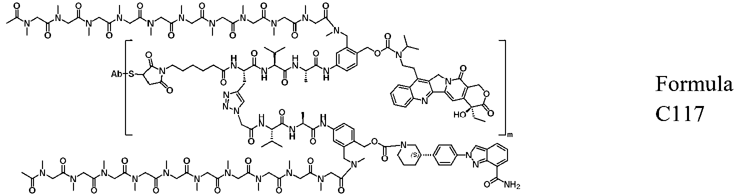 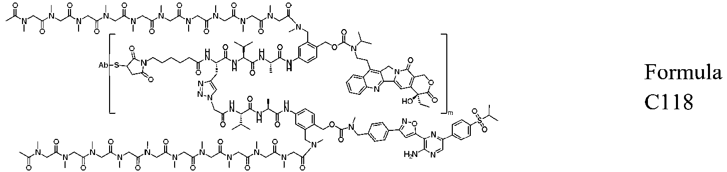 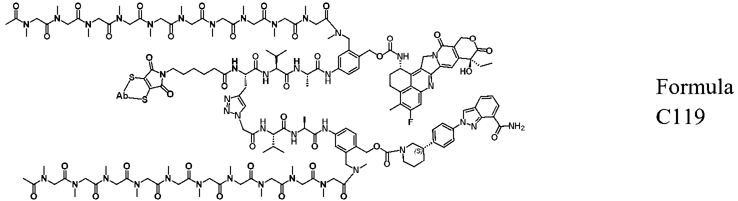 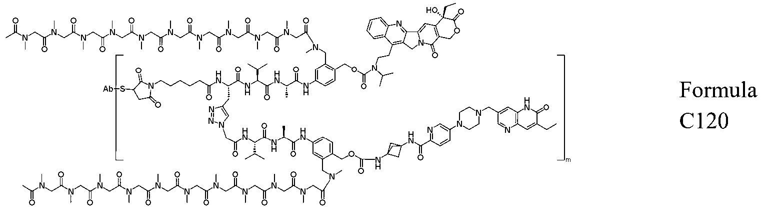 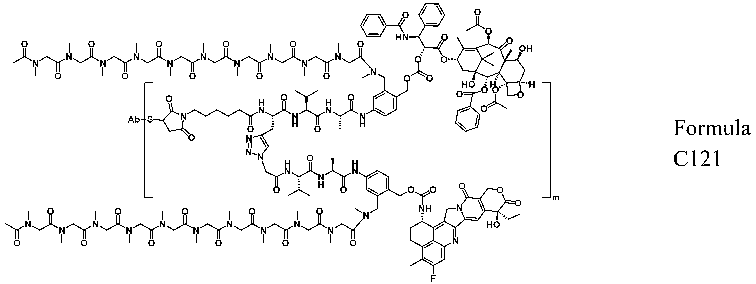 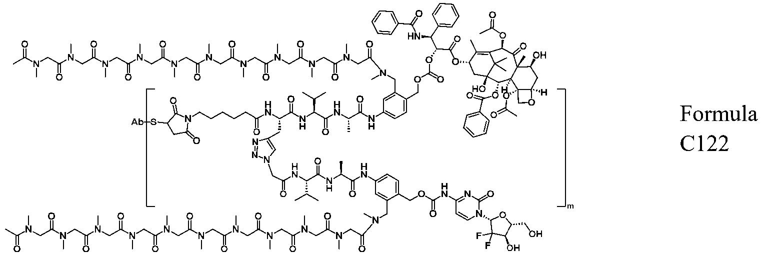 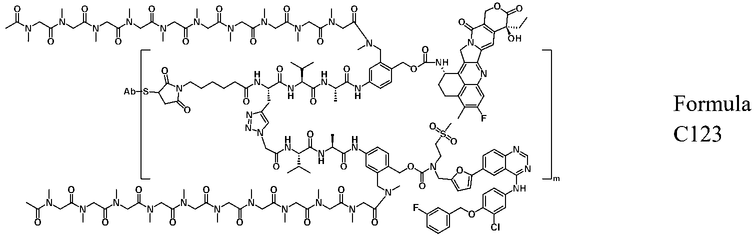 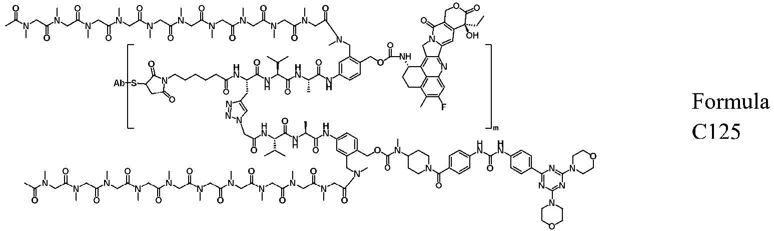 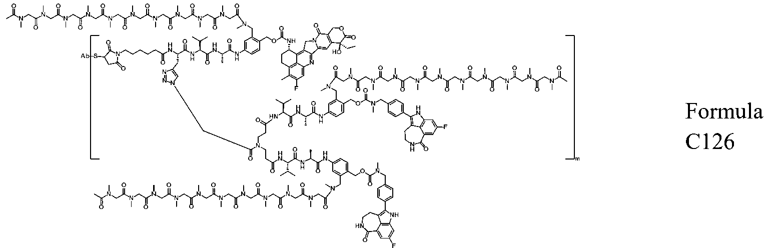 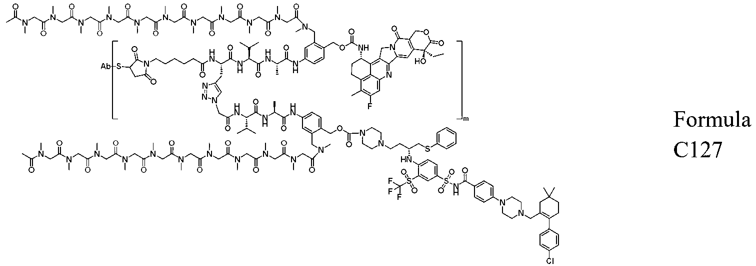 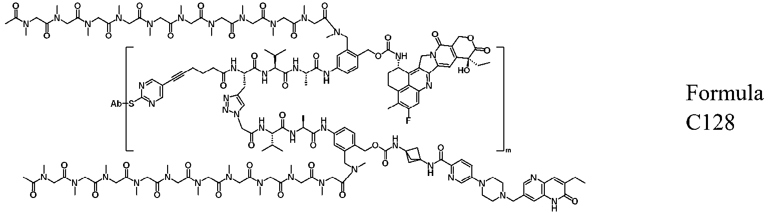 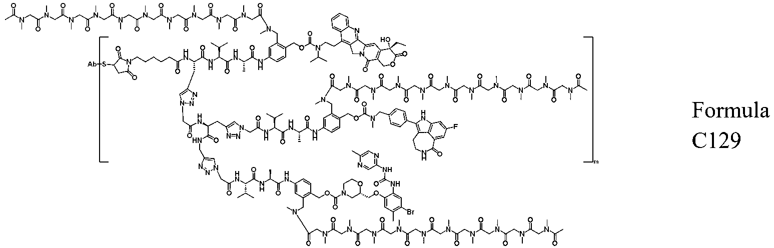 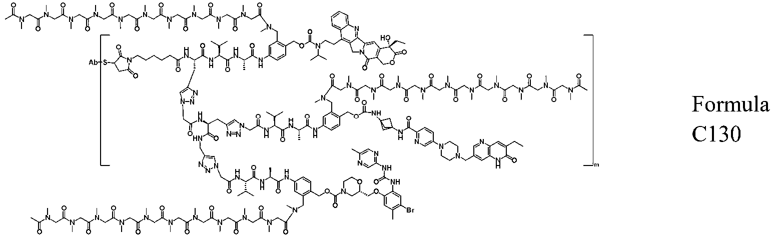 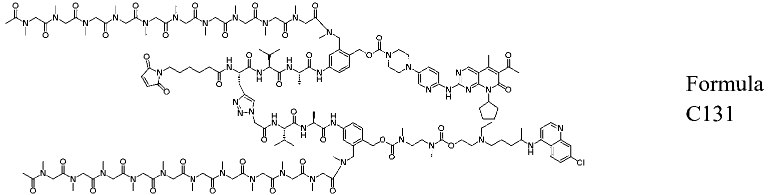 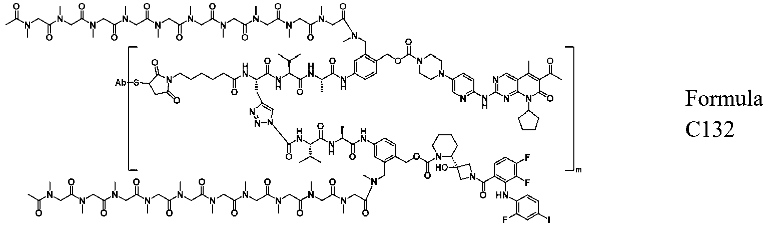 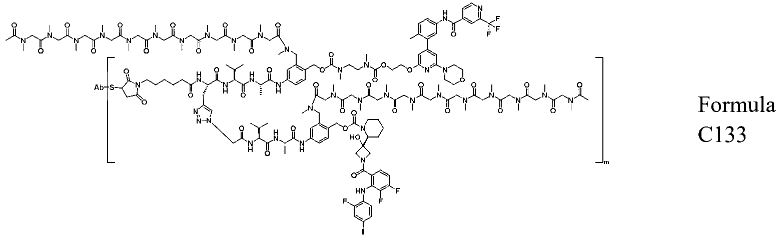 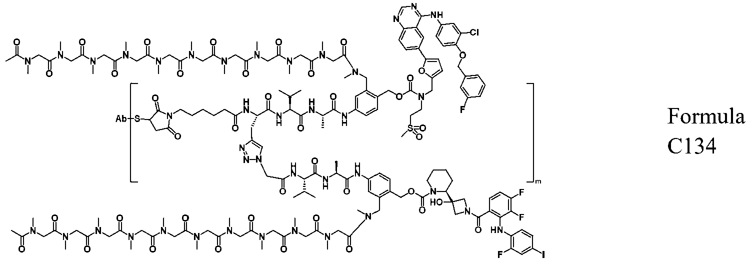 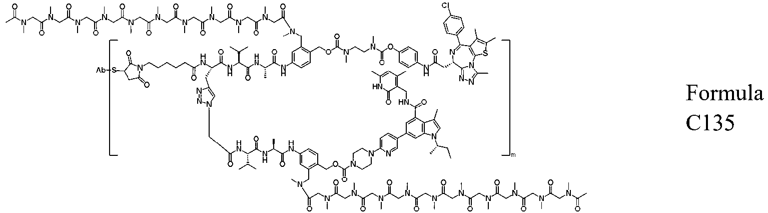 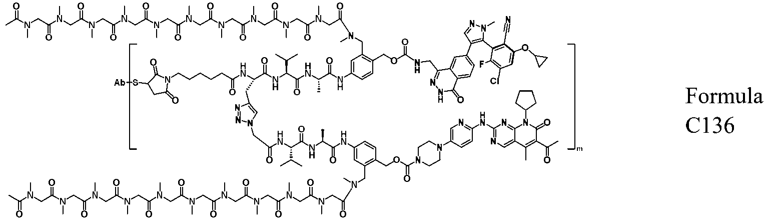 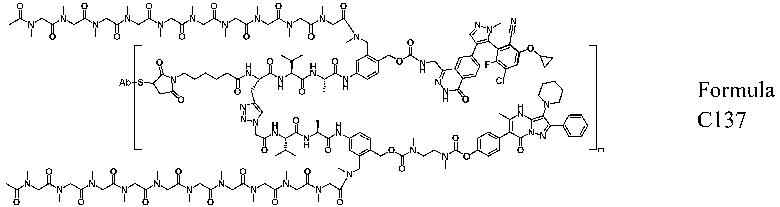 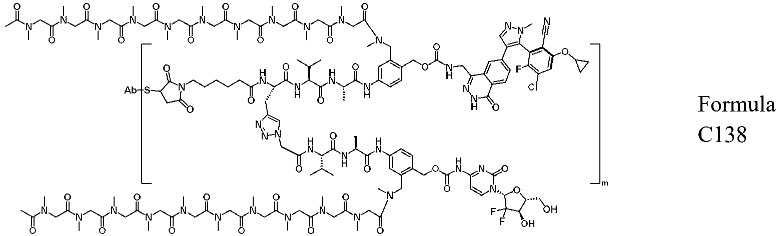 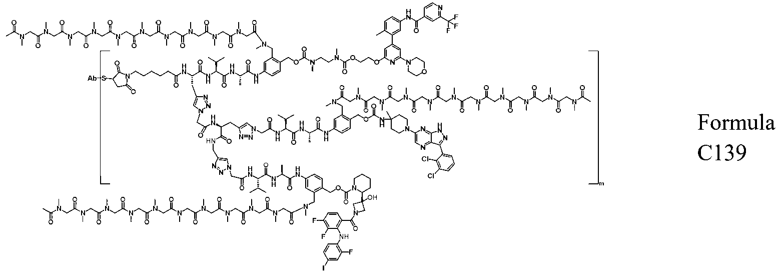 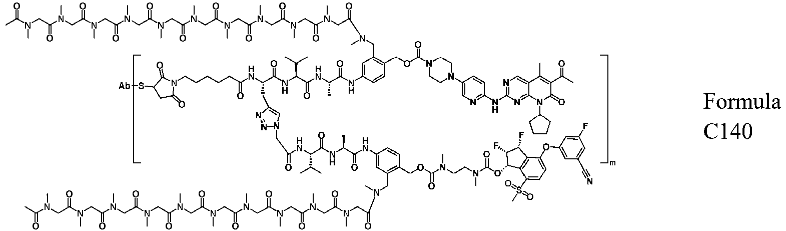 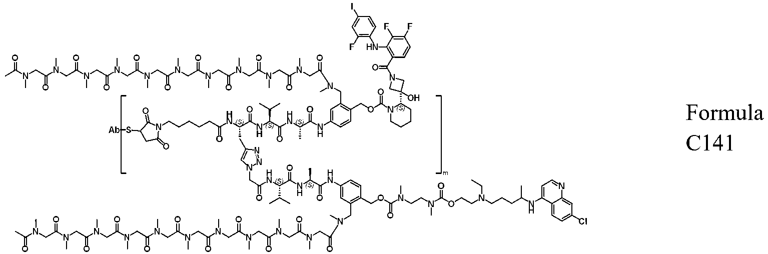 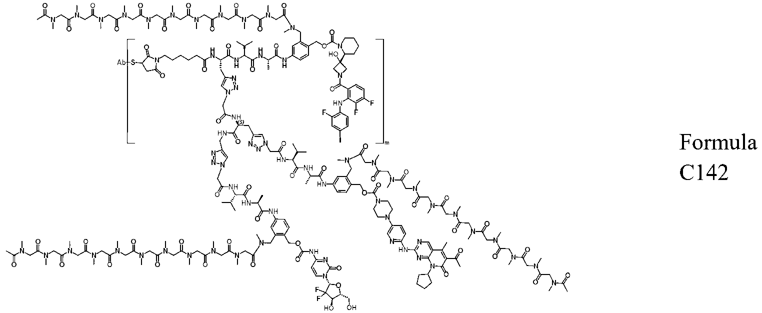 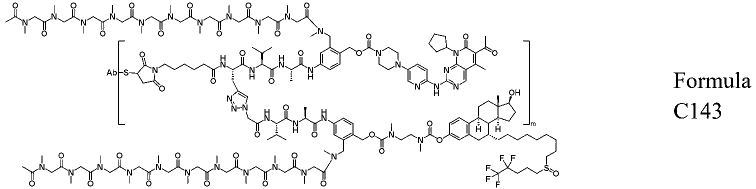 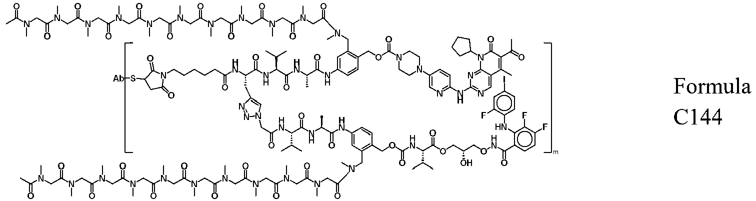 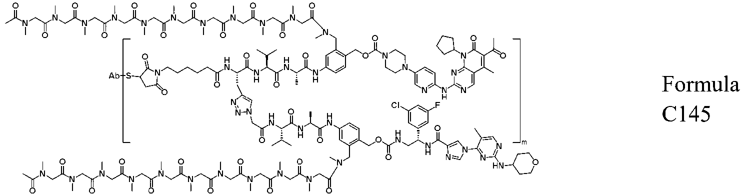 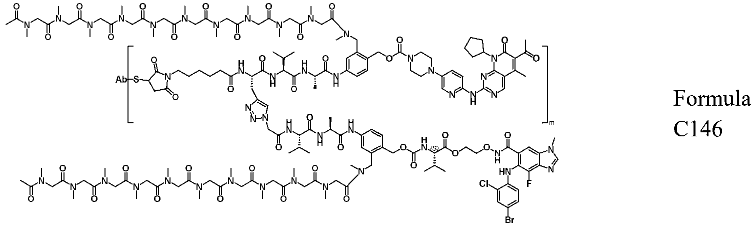 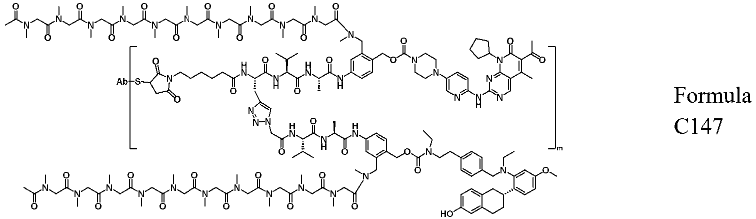 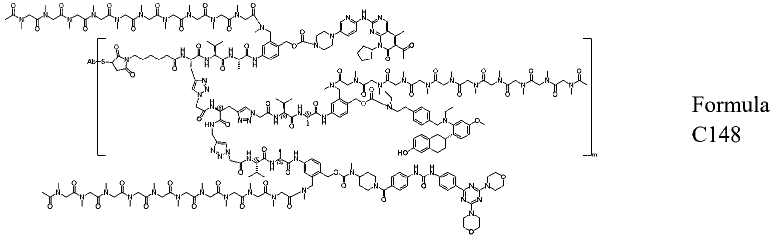 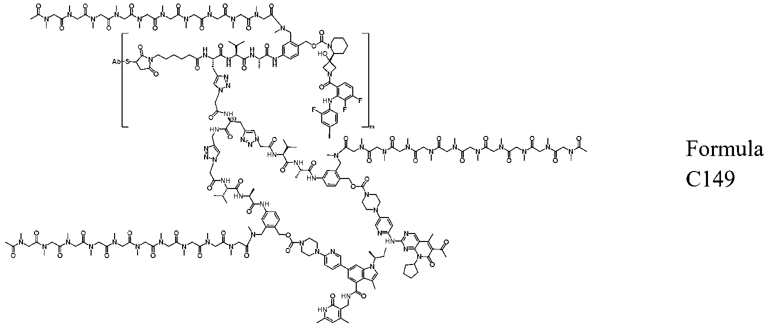 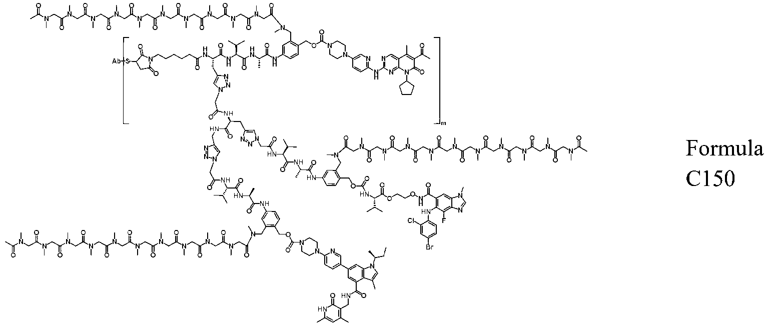 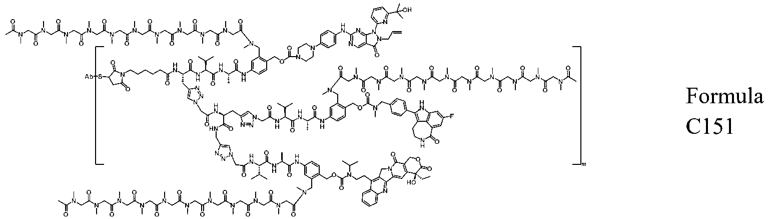 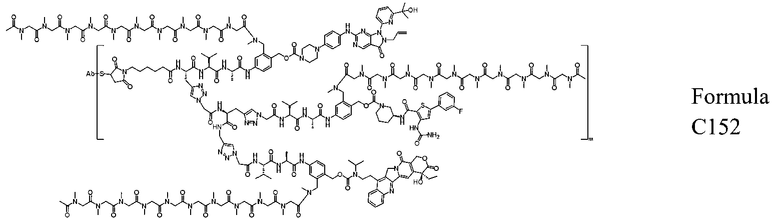 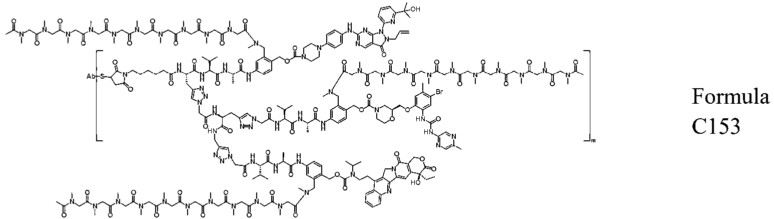 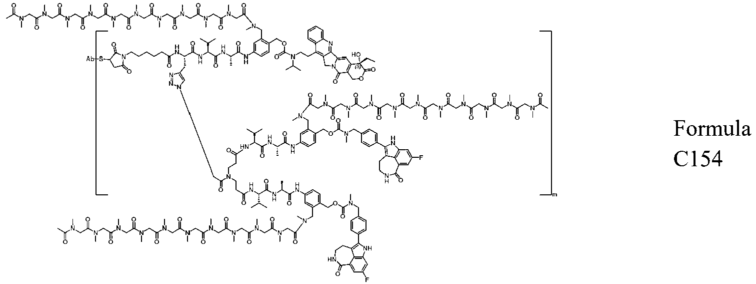 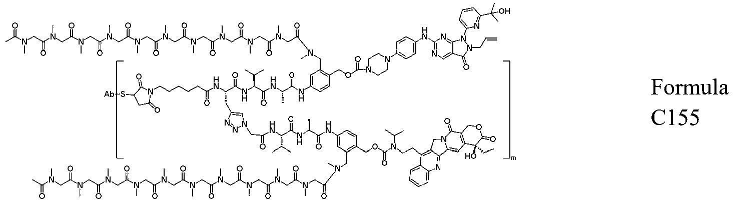 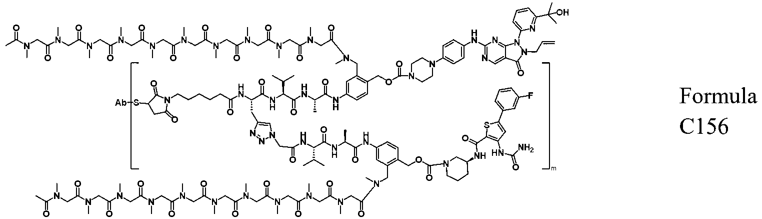 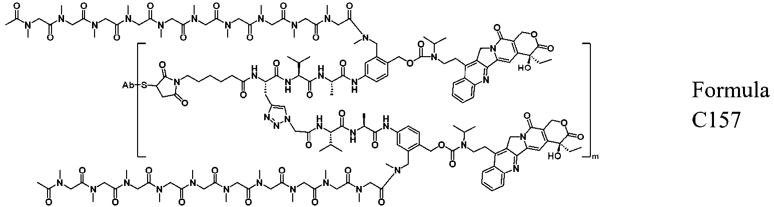 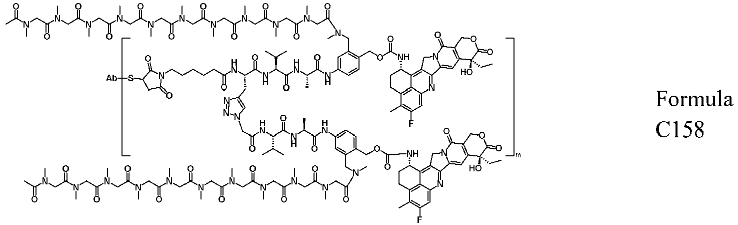 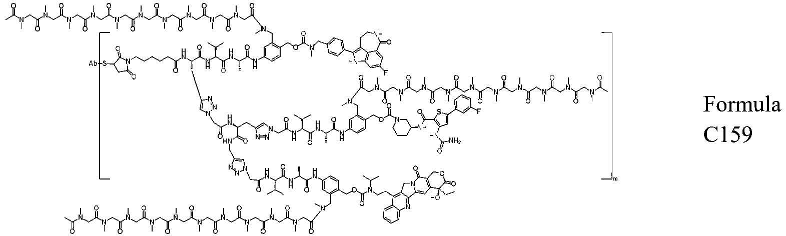 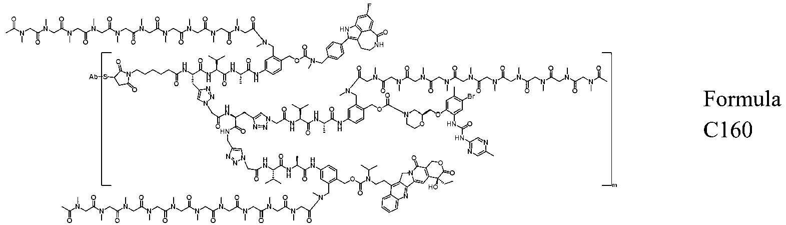 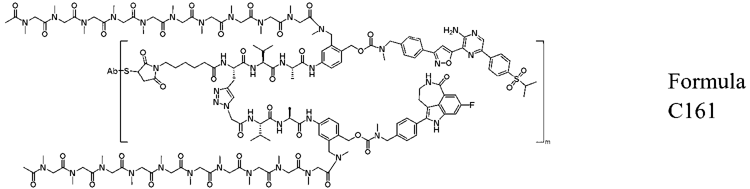 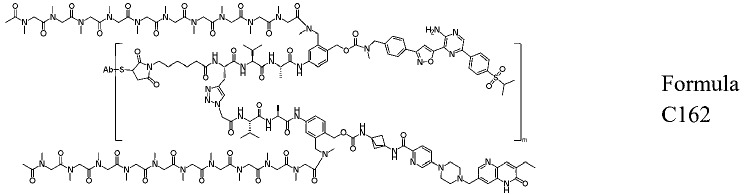 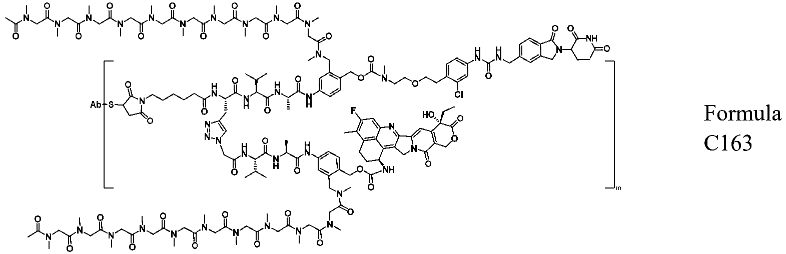 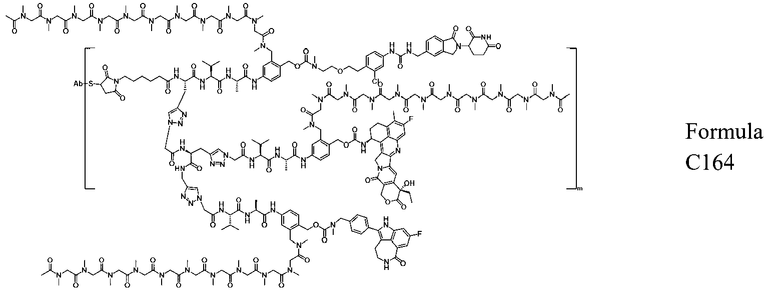 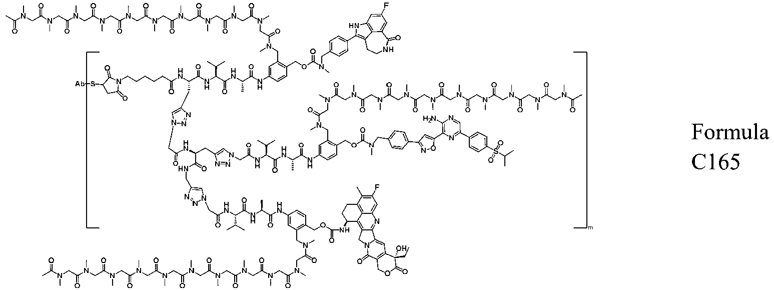 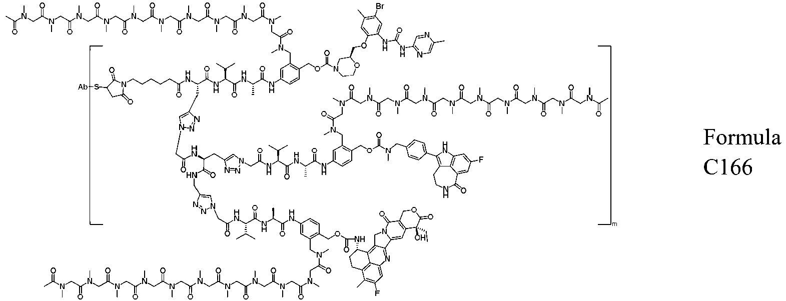 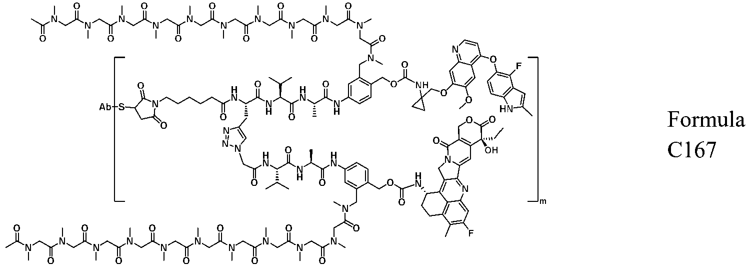 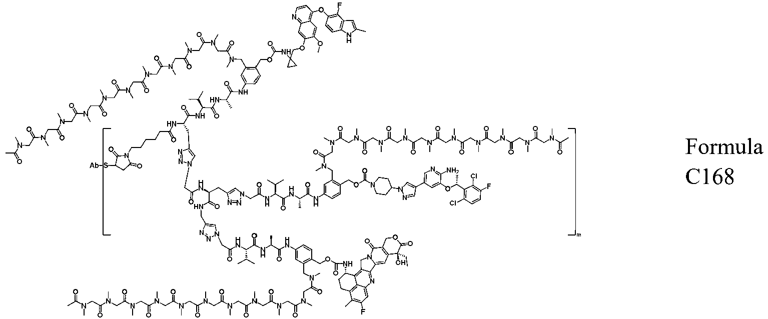 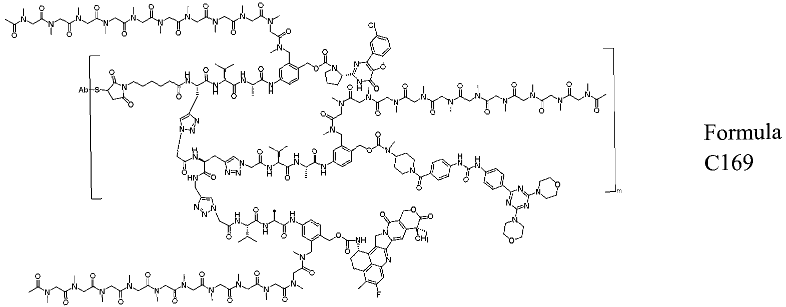 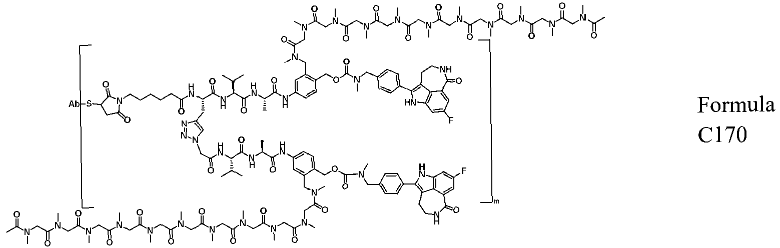 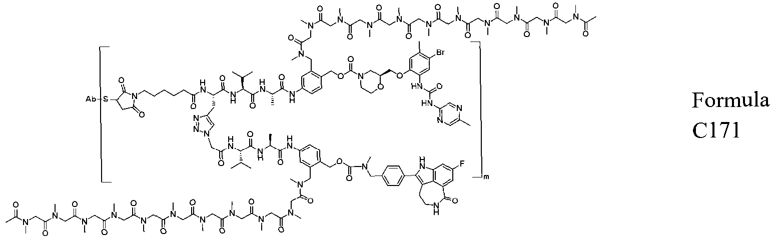 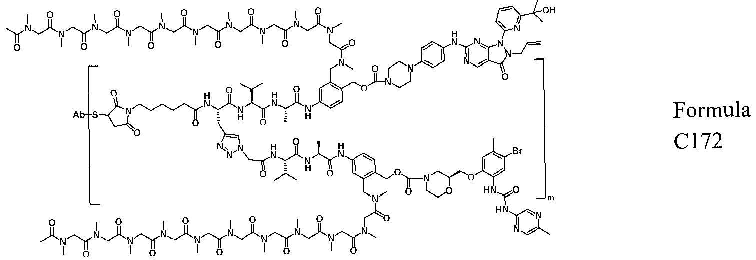 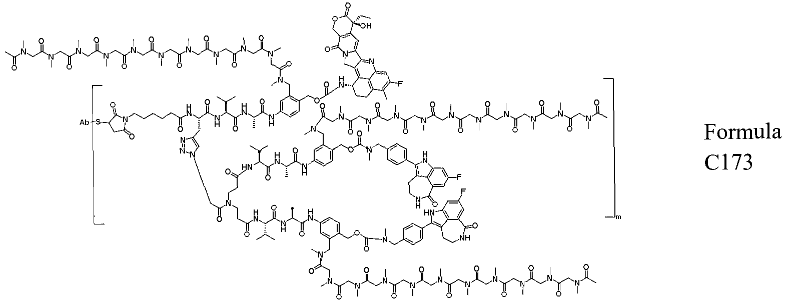 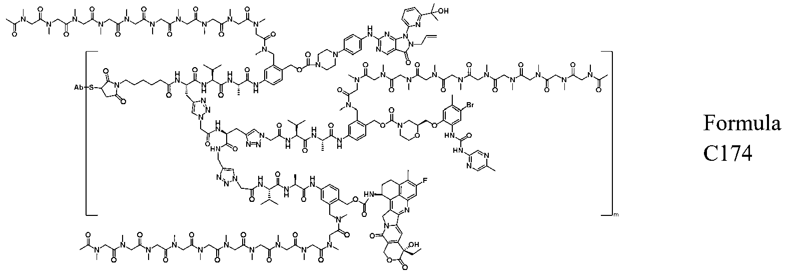 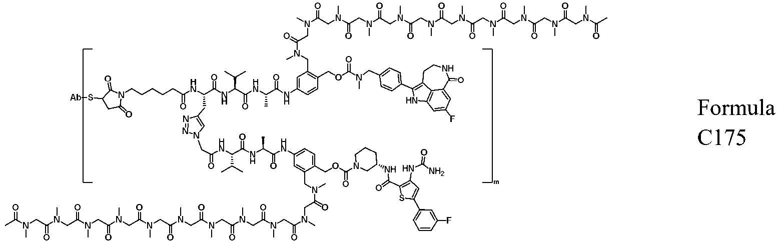 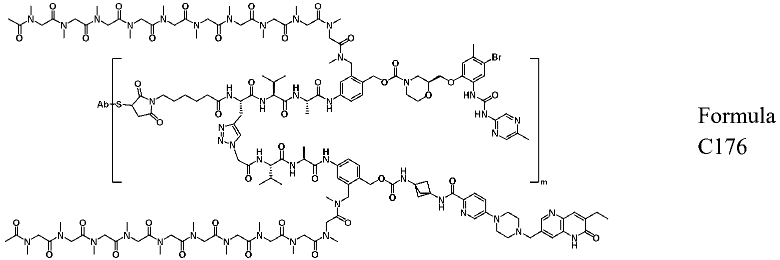 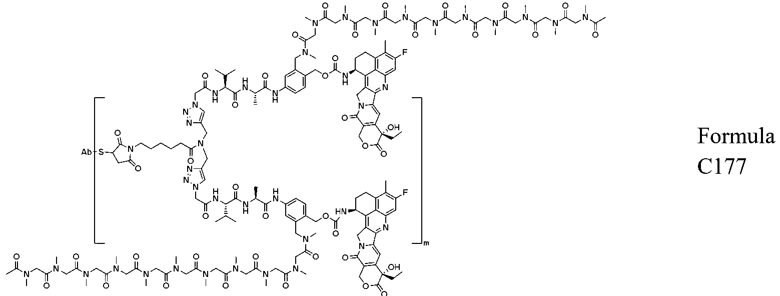 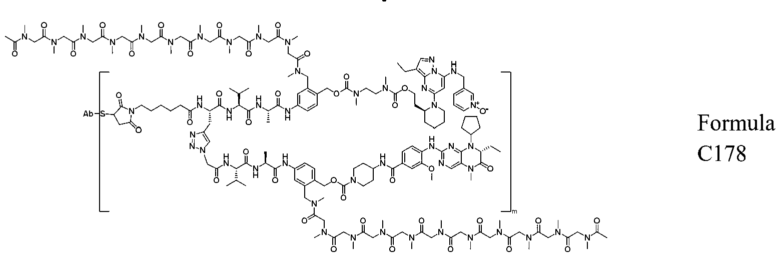 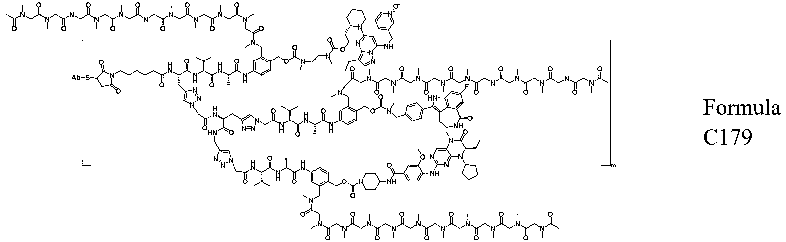 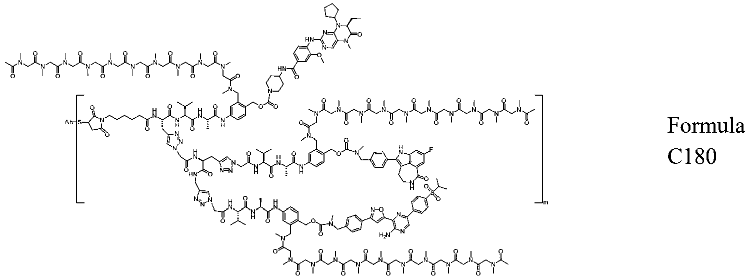 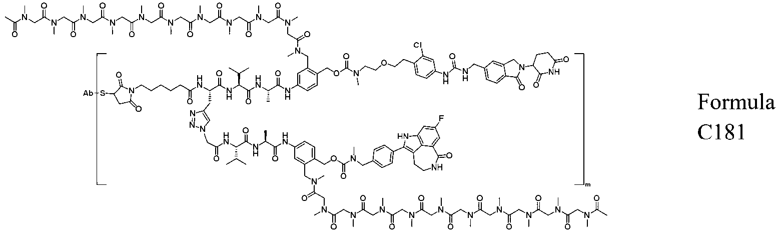 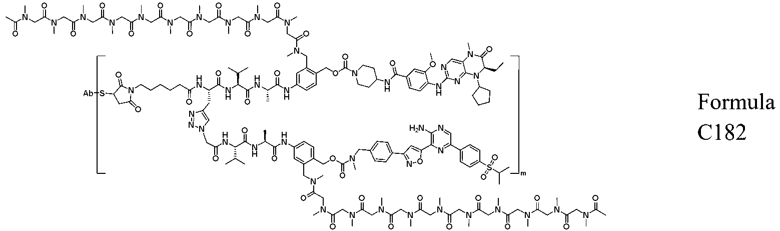 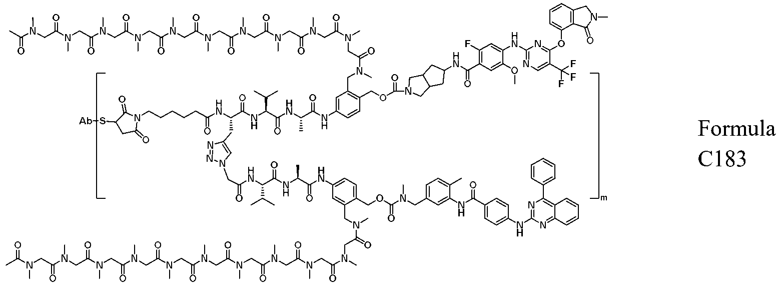 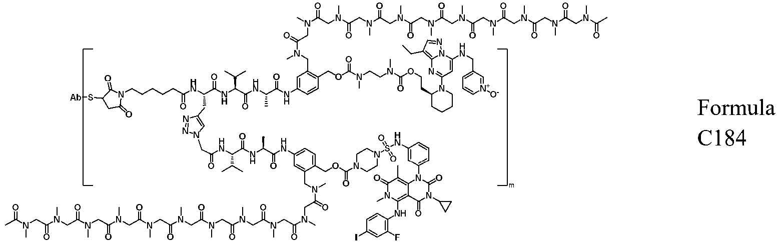 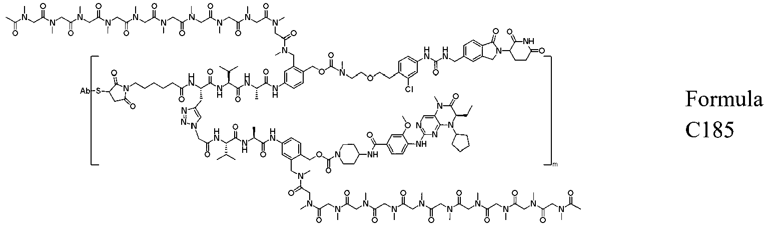 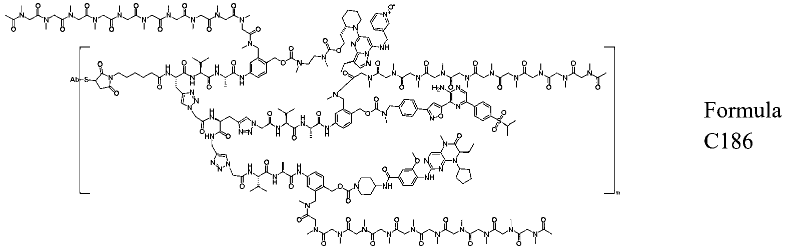 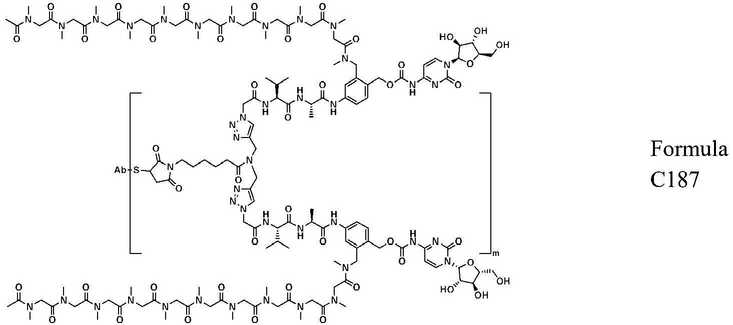 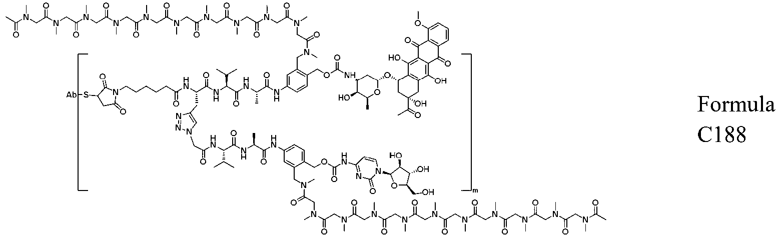 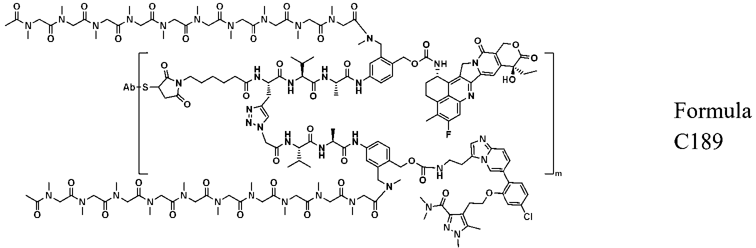 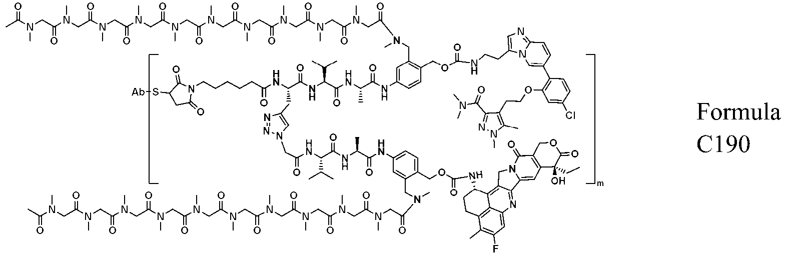 wherein Ab is an antibody or antigen-binding fragment thereof, and m is an integer or decimal from 1 to 12.

40. The compound, or a tautomer, a mesomer, a racemate, an enantiomer, or a diastereomer thereof, or a deuterated compound form thereof, or a mixture form thereof, or a pharmaceutically acceptable salt, prodrug, or solvate thereof, according to any one of claims 27 to 39, wherein the Ab is a monoclonal antibody or an antigen-binding fragment thereof; preferably, the monoclonal antibody is an antibody against an antigen selected from: HER2, HER3, TROP2, B7-H3, B7-H4, GPC20, GPRC5D, CDH6, EGFR, EGFRvIII, AXL, Nectin-4, Tissue factor, TIM-1, PSMA, PTK7, EpCAM, MUC1, STEAP1, GPNMB, FGF2, FOLR1, c-MET, GFR, AGS-16, Guanylyl cyclase C, Mesothelin, SLC44A4, EphA2, AGS-5, GPC-3, c-KIT, ROR1, ROR2, PD-L1, CD27L, 5T4, MerTK, Mucin 16, NaPi2b, STEAP, SLITRK6, ETBR, BCMA, CEACAM5, ICAM1, SC-16, SLC39A6, Delta-like protein3, Claudin 18.2, Claudin 3, Claudin 6, Claudin 9, CD19, CD20, CD22, CD30, CD33, CD37, CD45, CD56, CD66e, CD70, CD73, CD74, CD79b, CD123, CD138, CD147, CD166, CD223, MUC16, MSLN, ENPP3, SLTRK6, FGFR, LIV-1, Lewis Y, av-integrin, ITGB6 (integrin beta-6), ASCT2, C4.4a, CA-IX, CD324, CD352, CD44v6, CD48a, CLL-1, Cripto, CS1, DPEP3, Ephrin-A2, Ephrin-A4, ETBR, FGFR2, FGFR3, FLT3, GD3, Globo H, GPC3, LAMP-1, LRRC15, Ly6E, TM4SF1, MFI2, NOTCH3, p-cadherin, PRLR, CLDN1, CD228, CUB domain-containing protein 1 (CDCP1), CXCR4, LGR4, FZD7, CTR, GPR56, CCR7, DDR1, and RNF43.

41. A linker, or a tautomer, a mesomer, a racemate, an enantiomer, or a diastereomer thereof, or a deuterated compound form thereof, or a mixture form thereof, or a pharmaceutically acceptable salt, prodrug, or solvate thereof, wherein the linker has a structure represented by formula IV: 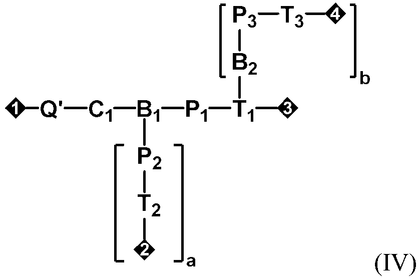 wherein,
Q' is a functional group capable of conjugating to cysteine, lysine, a non-natural amino acid, or a glycosyl of an antibody molecule, and attachment point 1 is connected to the antibody;
C₁ is selected from the group consisting of: a direct bond, optionally substituted alkylene, optionally substituted polyethylene glycol group, optionally substituted alkenylene, optionally substituted alkynylene, optionally substituted alicyclylene, optionally substituted aliphatic heterocyclylene, optionally substituted arylene, and optionally substituted heteroarylene; when substituted, the substituent(s) are selected from halogen, hydroxyl, amino, carboxyl, sulfonic acid group, sulfone group, phosphonic acid group, and alkoxy;
B₁ and B₂ are independently a direct bond or a branching group, provided that when C₁ is a direct bond, B₁ is not a direct bond;
P₁, P₂, and P₃ are each independently an optionally substituted polypeptide residue or a glucose fragment;
T₁, T₂, and T₃ are each independently a direct bond or an optionally substituted spacer group, provided that at most two of T₁, T₂, and T₃ are direct bonds and at least one of B₂, T₁, T₂, and T₃ is substituted with a hydrophilic group;
attachment points 2, 3, 4 are connected to drug units;
a and b are independently 0, 1, 2, or 3, provided that a and b are not both 0; and
when a=0, B₁ is a direct bond and T₁ is not a direct bond;
when b=0, B₁ is not a direct bond.

42. The linker, or a tautomer, a mesomer, a racemate, an enantiomer, or a diastereomer thereof, or a deuterated compound form thereof, or a mixture form thereof, or a pharmaceutically acceptable salt, prodrug, or solvate thereof, according to claim 41, wherein the compound comprises a structure represented by formula IVa: 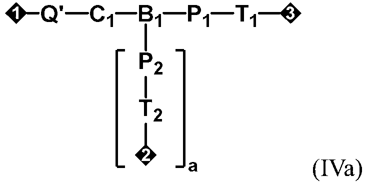 wherein:
Q' is a functional group capable of conjugating to cysteine, lysine, a non-natural amino acid, or a glycosyl of an antibody molecule, and attachment point 1 is connected to the antibody;
C₁ is selected from the group consisting of: a direct bond, optionally substituted alkylene, optionally substituted polyethylene glycol group, optionally substituted alkenylene, optionally substituted alkynylene, optionally substituted alicyclylene, optionally substituted aliphatic heterocyclylene, optionally substituted arylene, and optionally substituted heteroarylene; when substituted, the substituent(s) are selected from halogen, hydroxyl, amino, carboxyl, sulfonic acid group, sulfone group, phosphonic acid group, and alkoxy;
B₁ is a branching group;
P₁ and P₂ are each independently an optionally substituted polypeptide residue or a glucose fragment;
T₁ and T₂ are each independently a direct bond or an optionally substituted spacer group, provided that at most one of T₁ and T₂ is a direct bond, and at least one of T₁ and T₂ is substituted with a hydrophilic group;
attachment points 2 and 3 are connected to drug units;
a is 1, 2, or 3.

43. The linker, or a tautomer, a mesomer, a racemate, an enantiomer, or a diastereomer thereof, or a deuterated compound form thereof, or a mixture form thereof, or a pharmaceutically acceptable salt, prodrug, or solvate thereof, according to claim 41, wherein the compound comprises a structure represented by formula IVb: 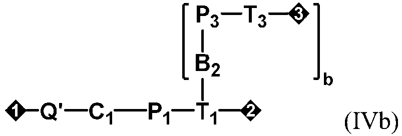
Q' is a functional group capable of conjugating to cysteine, lysine, a non-natural amino acid, or a glycosyl of an antibody molecule, and attachment point 1 is connected to the antibody;
C₁ is selected from the group consisting of: optionally substituted alkylene, optionally substituted polyethylene glycol group, optionally substituted alkenylene, optionally substituted alkynylene, optionally substituted alicyclylene, optionally substituted aliphatic heterocyclylene, optionally substituted arylene, and optionally substituted heteroarylene; when substituted, the substituent(s) are selected from halogen, hydroxyl, amino, carboxyl, sulfonic acid group, sulfone group, phosphonic acid group, and alkoxy;
B₂ is a branching group;
P₁ and P₃ are each independently a direct bond or an optionally substituted polypeptide residue or a glucose fragment;
T₁ is an optionally substituted spacer group, T₃ is a direct bond or an optionally substituted spacer group, and at least one of B₂ and T₃ is substituted with a hydrophilic group;
attachment points 2 and 3 are connected to drug units;
b is 1, 2, or 3.

44. The linker, or a tautomer, a mesomer, a racemate, an enantiomer, or a diastereomer thereof, or a deuterated compound form thereof, or a mixture form thereof, or a pharmaceutically acceptable salt, prodrug, or solvate thereof, according to claim 41, wherein:
B₁ and B₂ are independently a branching group;
P₁, P₂, and P₃ are each independently an optionally substituted polypeptide residue or a glucose fragment;
T₁ is an optionally substituted spacer group, T₂ and T₃ are each independently a direct bond or an optionally substituted spacer group, and at least one of B₂, T₂, and T₃ is substituted with a hydrophilic group; and
a and b are independently 1, 2, or 3.

45. The linker, or a tautomer, a mesomer, a racemate, an enantiomer, or a diastereomer thereof, or a deuterated compound form thereof, or a mixture form thereof, or a pharmaceutically acceptable salt, prodrug, or solvate thereof, according to any one of claims 41 to 44, wherein:
Q' is selected from the group consisting of: optionally substituted 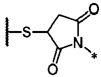 (Q1'), optionally substituted 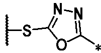 (Q2'), optionally substituted 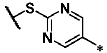 (Q3'), optionally substituted 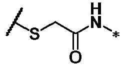 (Q4'), optionally substituted 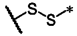 (Q5'), optionally substituted 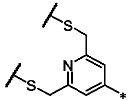 (Q6'), optionally substituted 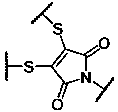 (Q7'), and optionally substituted 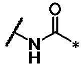 (Q8'), wherein * represents a site connected to C₁, and the wave line represents a site connected to the antibody molecule.

46. The linker, or a tautomer, a mesomer, a racemate, an enantiomer, or a diastereomer thereof, or a deuterated compound form thereof, or a mixture form thereof, or a pharmaceutically acceptable salt, prodrug, or solvate thereof, according to any one of claims 41 to 45, wherein:
C₁ is selected from: a direct bond, -(CH₂)ₘ₁-, -(CH₂)ₘ₁-(O-CH₂-CH₂)ₘ₂-, -(CH₂)ₘ₁-(O-CH₂-CH₂)ₘ₂-(CH₂)ₘ₃-, -(CH₂)ₘ₁-C(O)NH-(CH₂)ₘ₂-, -(CH₂)ₘ₁-NHC(O)-(CH₂)ₘ₂-, and -(C≡ C-(CH₂)ₘ₁-, wherein m1, m2, and m3 are independently integers from 1 to 6, and the indicated substituent is connected to Q' on the left side and B₁ or P₁ on the right side.

47. The linker, or a tautomer, a mesomer, a racemate, an enantiomer, or a diastereomer thereof, or a deuterated compound form thereof, or a mixture form thereof, or a pharmaceutically acceptable salt, prodrug, or solvate thereof, according to any one of claims 41 to 46, wherein:
B₁ is selected from the group consisting of: a direct bond, 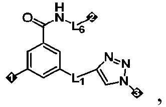 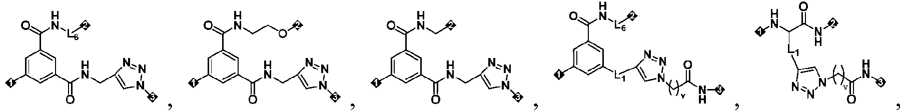 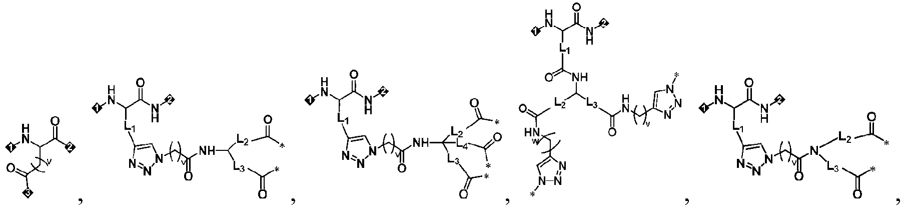 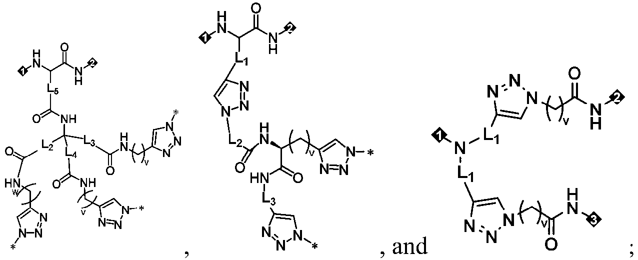
preferably, 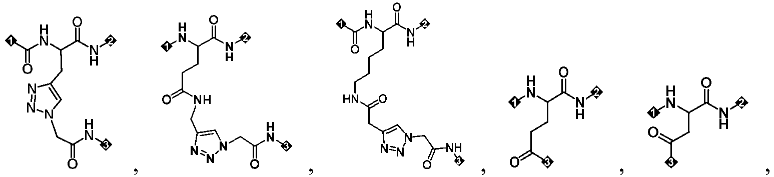 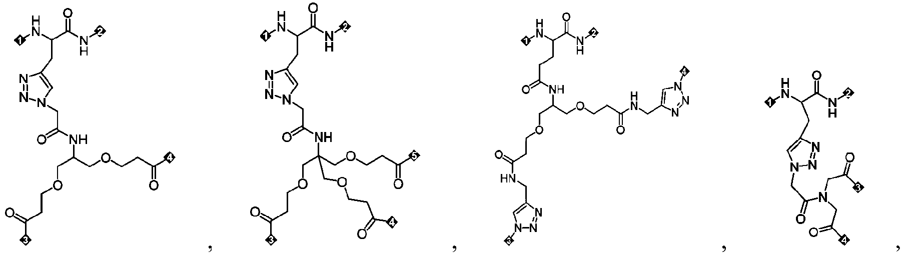 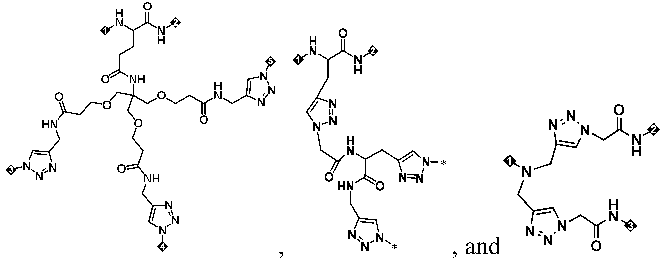 wherein attachment point 1 is connected to C₁ or Q' (when C₁ is a direct bond), attachment point 2 is connected to P₁, attachment points *, 3, 4, and 5 are connected to P₂, L₁ is selected from -(CH₂)ₘ₁-, -(CH₂)ₘ₁-C(O)NH-(CH₂)ₘ₂-, -(CH₂)ₘ₁-NHC(O)-(CH₂)ₘ₂-, L₂, L₃, and L₄ are independently -(CH₂)ₘ₁-(O-CH₂-CH₂)ₘ₂- or -(CH₂)ₘ₁-(O-CH₂-CH₂)ₘ₂-(CH₂)ₘ₃-, L₅ is -(CH₂)ₘ₁-, L₆ is -(CH₂)ₘ₁- or - (CH₂-CH₂-O)m₂, wherein m1, m2, m3, w, and v are each independently 1, 2, 3, 4, 5, or 6;
preferably, when C₁ is a direct bond, B₁ is

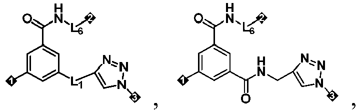

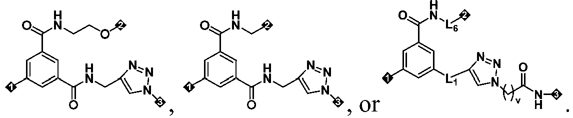

48. The linker, or a tautomer, a mesomer, a racemate, an enantiomer, or a diastereomer thereof, or a deuterated compound form thereof, or a mixture form thereof, or a pharmaceutically acceptable salt, prodrug, or solvate thereof, according to any one of claims 41 to 47, wherein:
B₂ is selected from the group consisting of: 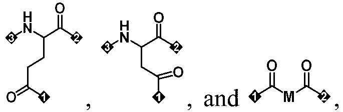 wherein attachment point 1 is connected to T₁, attachment point 2 is connected to P₃, attachment point 3 is connected to the hydrophilic group, M is selected from the group consisting of: optionally substituted C₁-C₆ alkylene, optionally substituted C₁-C₆ alkoxy, and optionally substituted C₁-C₅ alkenylene.

49. The linker, or a tautomer, a mesomer, a racemate, an enantiomer, or a diastereomer thereof, or a deuterated compound form thereof, or a mixture form thereof, or a pharmaceutically acceptable salt, prodrug, or solvate thereof, according to any one of claims 41 to 48, wherein:
T₁, T₂, T₃ are independently selected from the group consisting of: a direct bond, 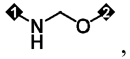 and optionally substituted 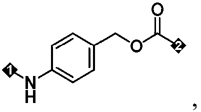 wherein attachment point 1 is connected to P₁, P₂, or P₃, and attachment point 2 is connected to D₁, D₂, or D₃.

50. The linker, or a tautomer, a mesomer, a racemate, an enantiomer, or a diastereomer thereof, or a deuterated compound form thereof, or a mixture form thereof, or a pharmaceutically acceptable salt, prodrug, or solvate thereof, according to any one of claims 41 to 49, wherein:
P₁, P₂, P₃ comprises an optionally substituted polypeptide residue composed of amino acids selected from the group consisting of: phenylalanine, isoleucine, leucine, tryptophan, valine, methionine, tyrosine, alanine, threonine, histidine, serine, glutamine, arginine, lysine, asparagine, glutamic acid, proline, citrulline, aspartic acid, and glycine; preferably, P₁, P₂, P₃ comprises an optionally substituted polypeptide residue composed of amino acids selected from the group consisting of: glycine, phenylalanine, valine, alanine, arginine, citrulline, aspartic acid, asparagine, and lysine;
more preferably, P₁, P₂, P₃ comprises an optionally substituted polypeptide residue selected from the group consisting of: phenylalanine-lysine (Phe-Lys), valine-alanine (Val-Ala), valine-citrulline (Val-Cit), glutamic acid-valine-alanine (Glu-Val-Ala), glutamic acid-valine-citrulline (Glu-Val-Cit), valine-lysine (Val-Lys), alanine-alanine (Ala-Ala), alanine-alanine-alanine (Ala-Ala-Ala), alanine-alanine-asparagine (Ala-Ala-Asn), alanine-leucine (Ala-Leu), leucine-leucine (Leu-Leu), phenylalanine-arginine (Phe-Arg), phenylalanine-lysine (Phe-Lys), 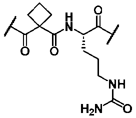 (cBu-Cit), 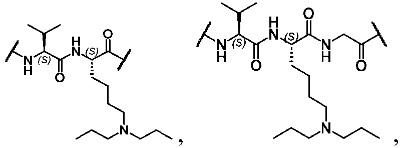 glycine-glycine-phenylalanine-glycine (Gly-Gly-Phe-Gly), glycine-proline (Gly-Pro);
or, wherein one of the combination P₂-T₂ and the combination P₃-T₃ is 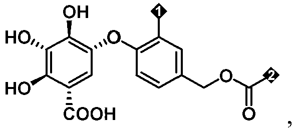 wherein attachment point 1 is connected to B₁ or B₂, and attachment point 2 is connected to D₂ or D₃.

51. The linker, or a tautomer, a mesomer, a racemate, an enantiomer, or a diastereomer thereof, or a deuterated compound form thereof, or a mixture form thereof, or a pharmaceutically acceptable salt, prodrug, or solvate thereof, according to any one of claims 41 to 50, wherein:
the hydrophilic group is selected from: substituted polysarcosine residue, polyglycerol, polyol, glycosyl, cyclodextrin, substituted ethylene glycol fragment, substituted glycosylated polyethylene glycol, substituted glycosylated polyglycerol, substituted cyclodextrin polyethylene glycol, or a combination thereof;
preferably, the substituted polysarcosine residue is 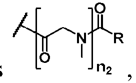 wherein n₂ is an integer between 4 and 20, for example between 4 and 16, R is selected from: C₁-C₆ alkyl, C₁-C₆ cycloalkyl, and C₁-C₆ alkoxy;
preferably, the substituted glycosylated polyethylene glycol fragment is 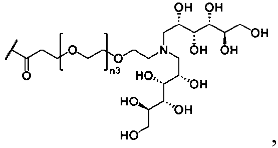 wherein n₃ is an integer between 4 and 18, for example between 4 and 12;
preferably, the substituted glycosylated polyglycerol fragment is 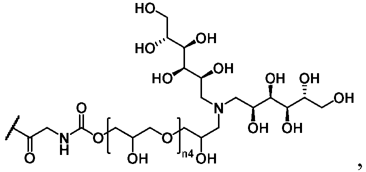 wherein n₄ is an integer between 4 and 12, for example between 4 and 10;
preferably, the substituted polyethylene glycol fragment is 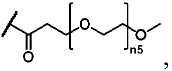 wherein n₅ is an integer between 4 and 24, for example between 8 and 18;
preferably, at least one of T₁, T₂, T₃ is 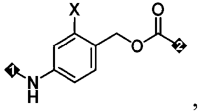 wherein X is optionally substituted 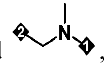 the hydrophilic group is connected to T₁, T₂, or T₃ via X, wherein attachment point 1 is connected to the hydrophilic group, and attachment point 2 is connected to one of T₁, T₂, T₃.

52. The linker, or a tautomer, a mesomer, a racemate, an enantiomer, or a diastereomer thereof, or a deuterated compound form thereof, or a mixture form thereof, or a pharmaceutically acceptable salt, prodrug, or solvate thereof, according to any one of claims 41 to 51, wherein the linker has a structure represented by formulas D1 to D38:

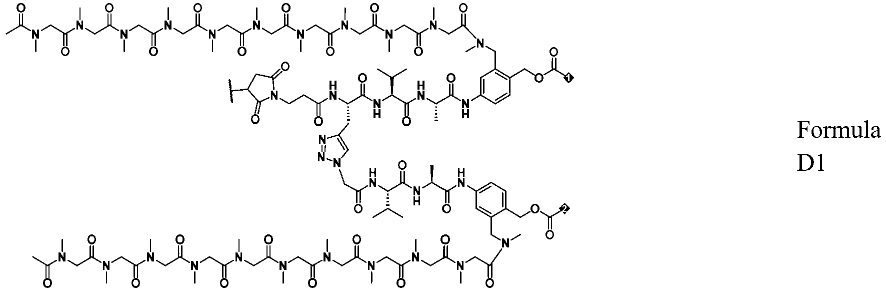

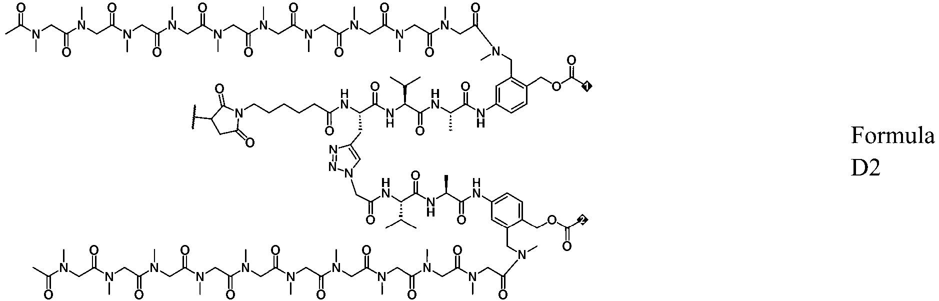

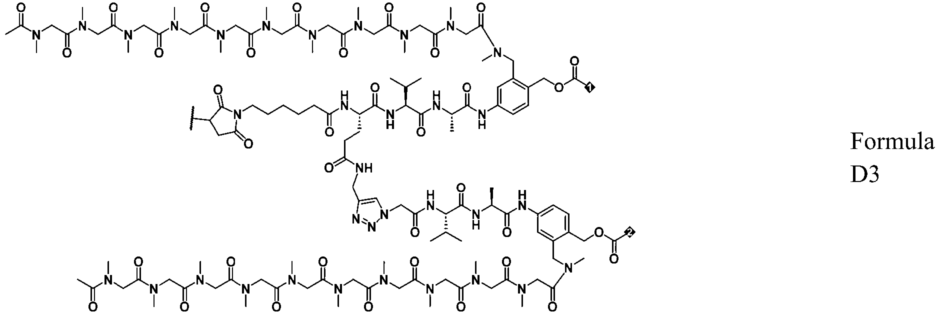

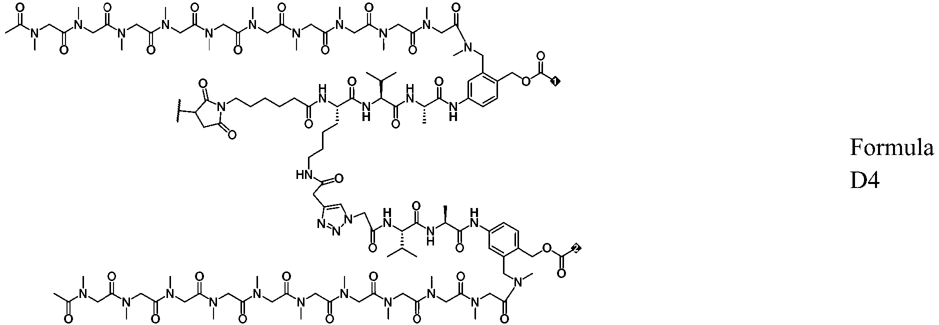

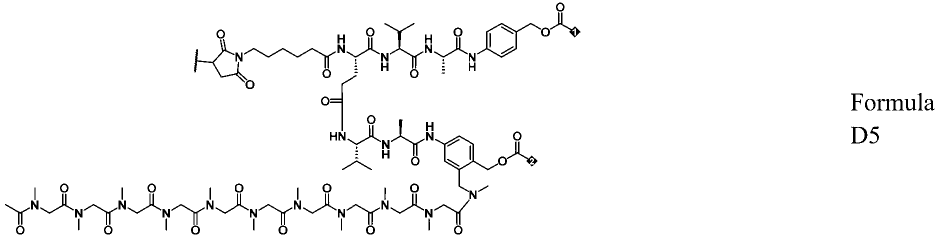

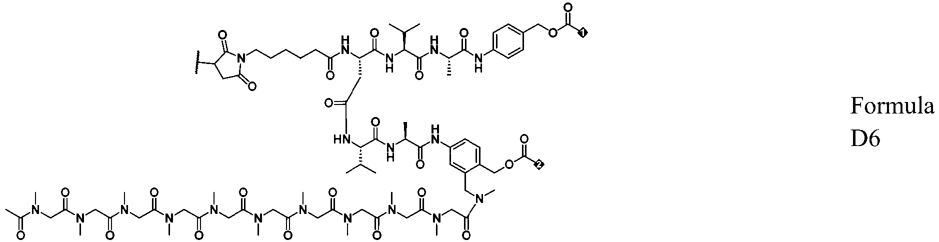

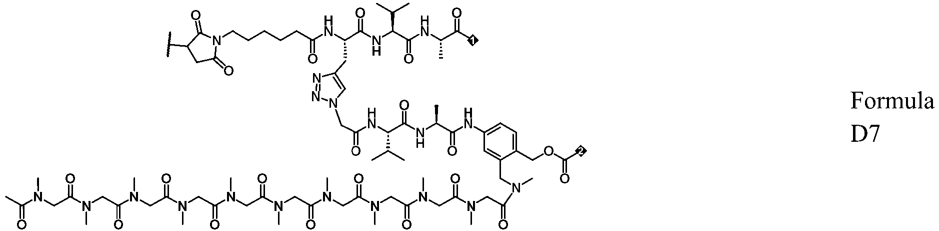

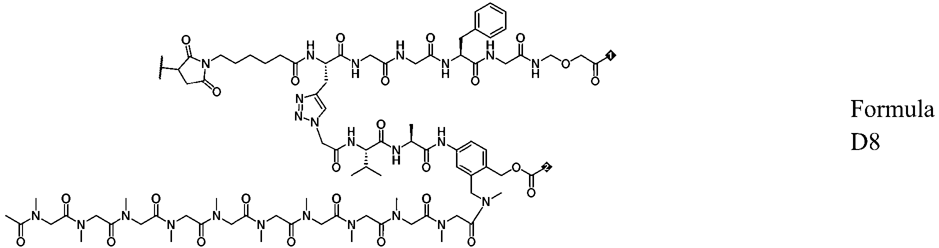

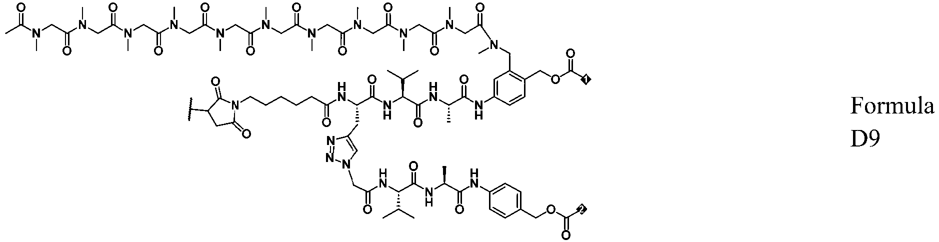

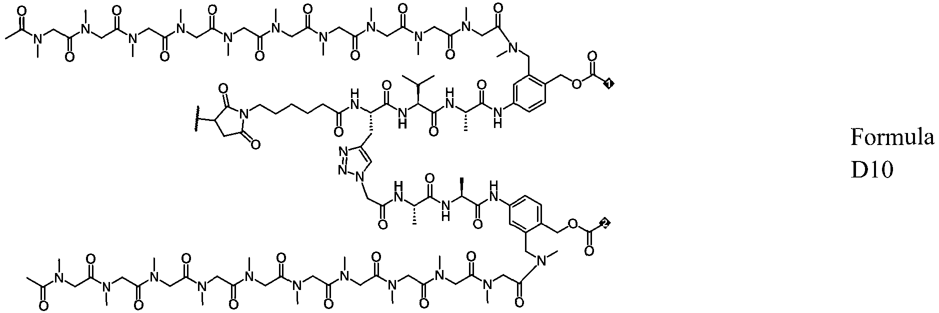

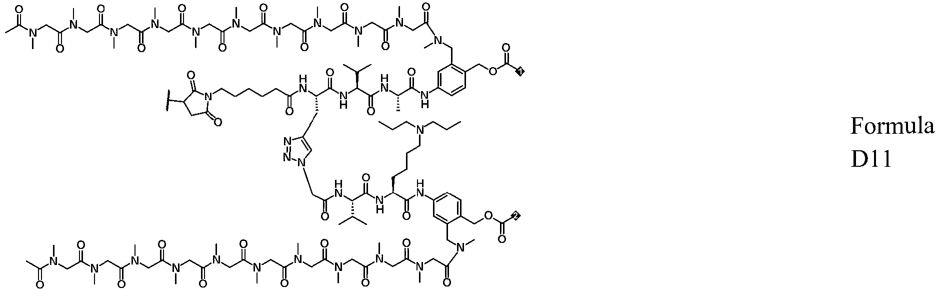

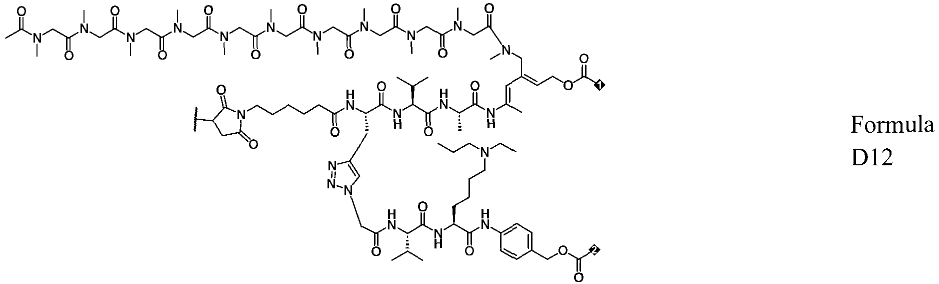

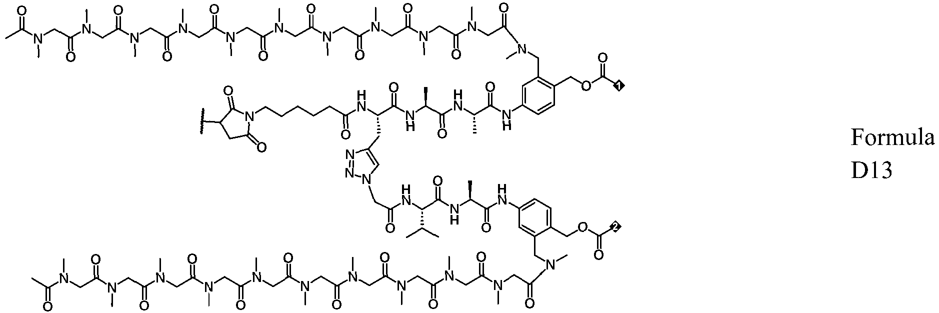

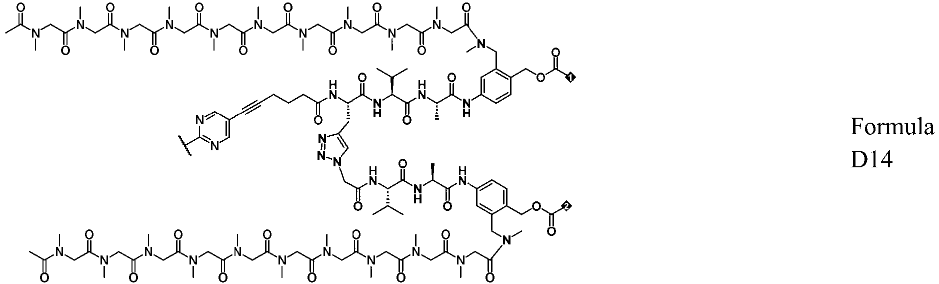

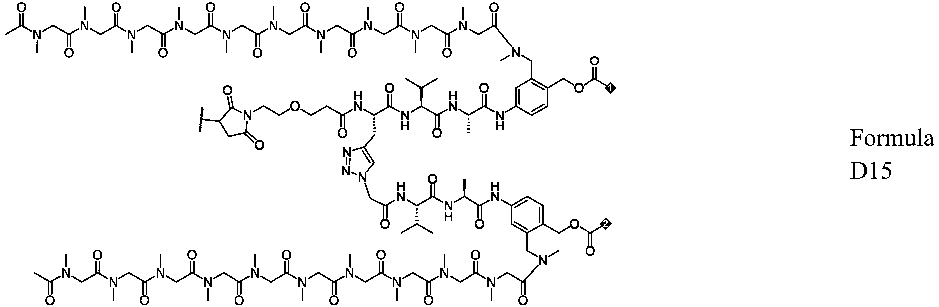

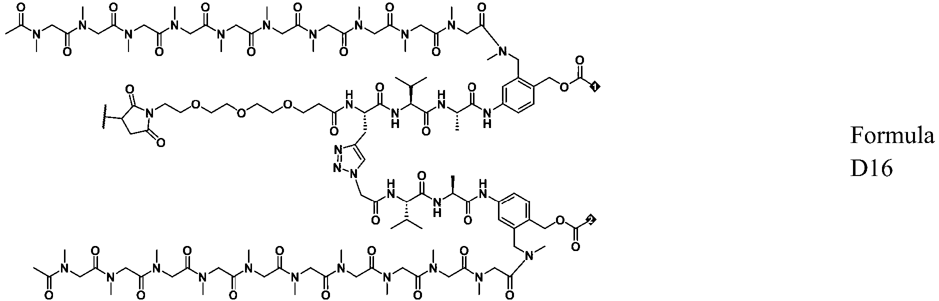

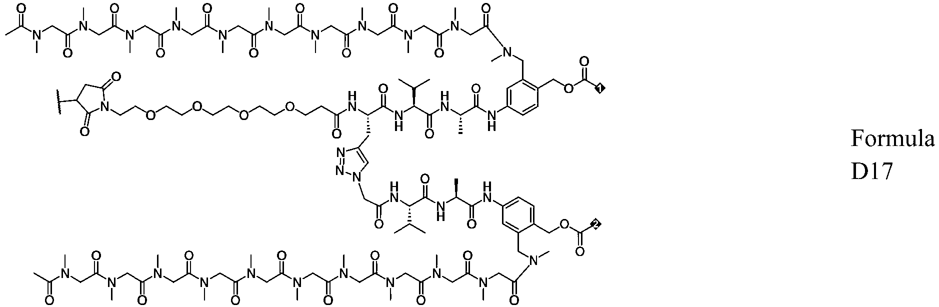

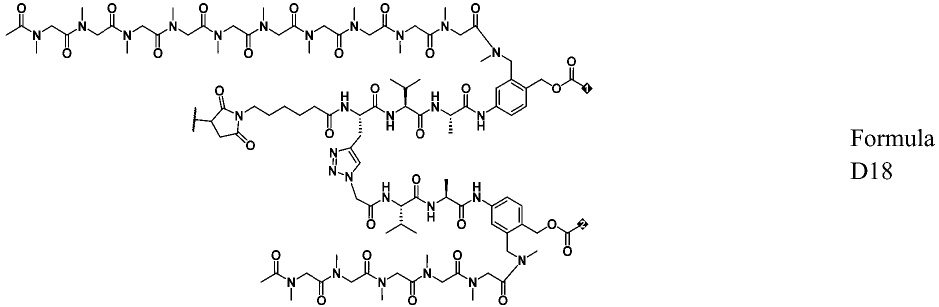

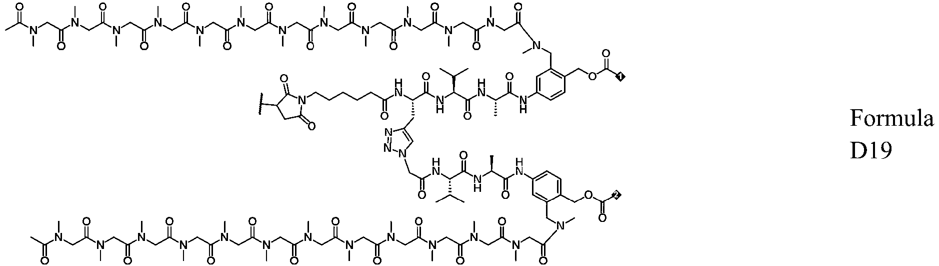

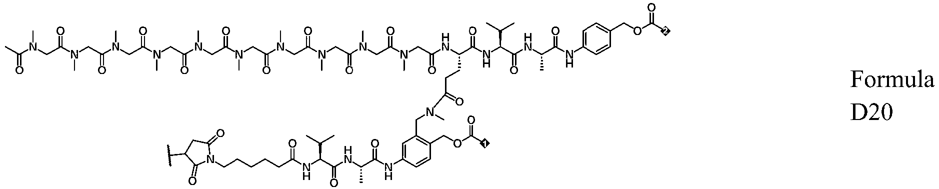

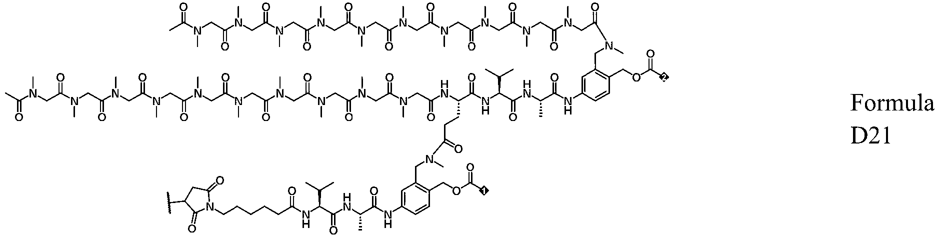

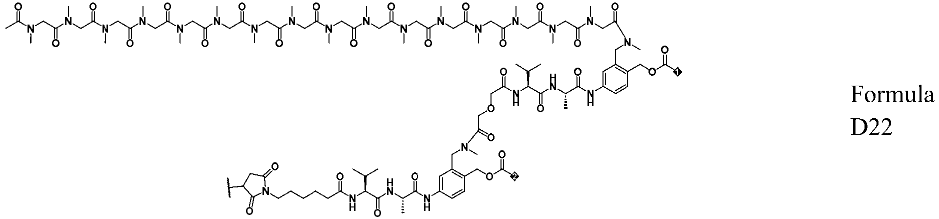

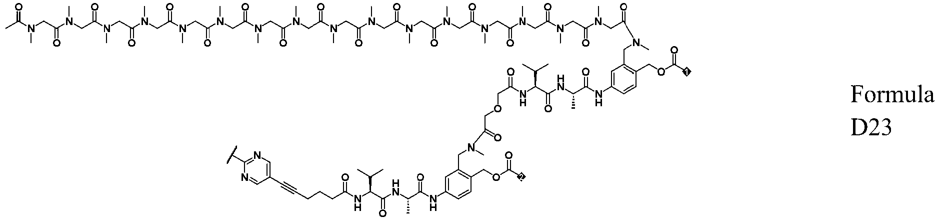

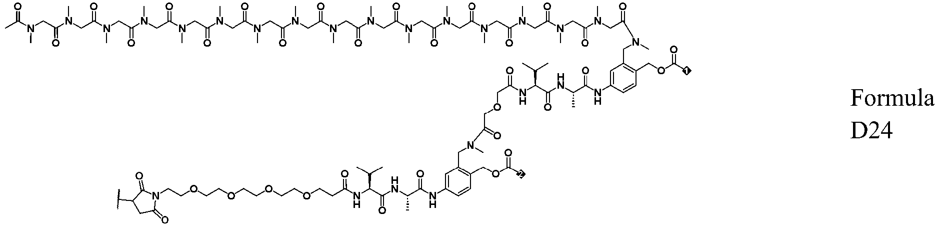

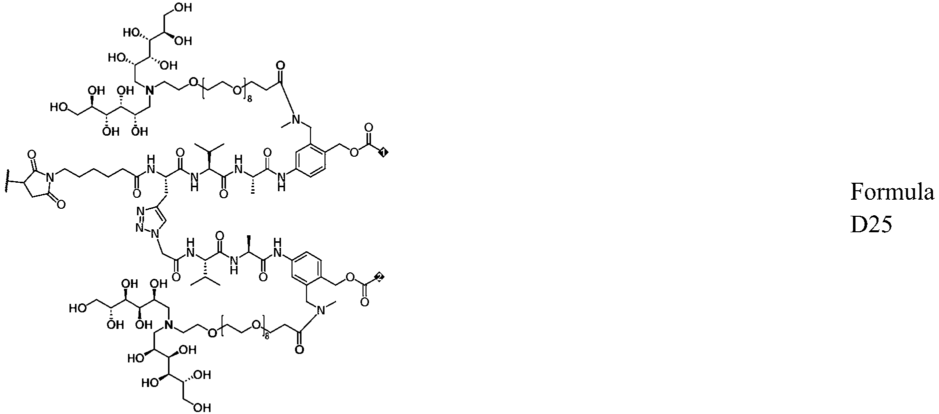

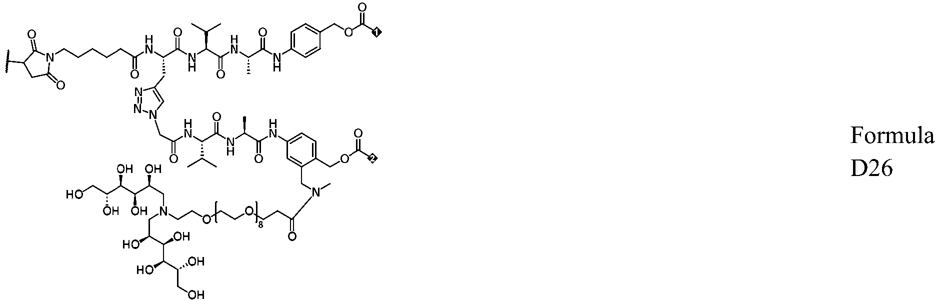

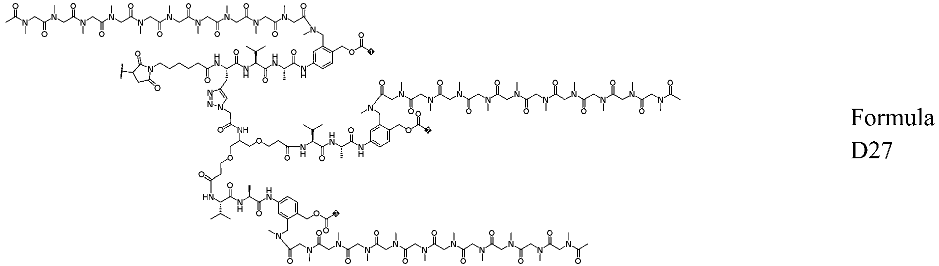

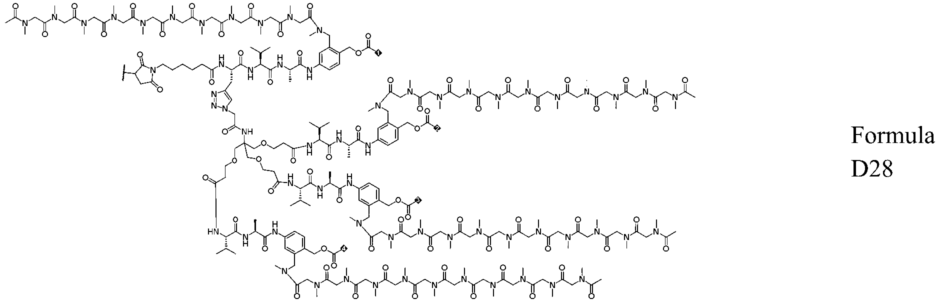

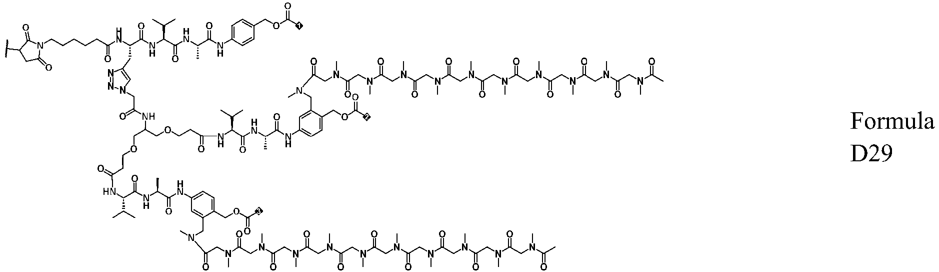

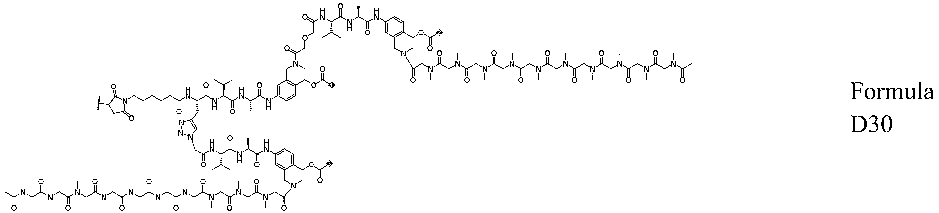

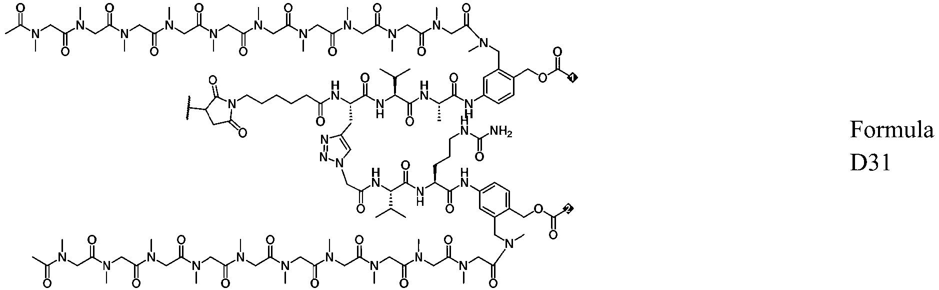

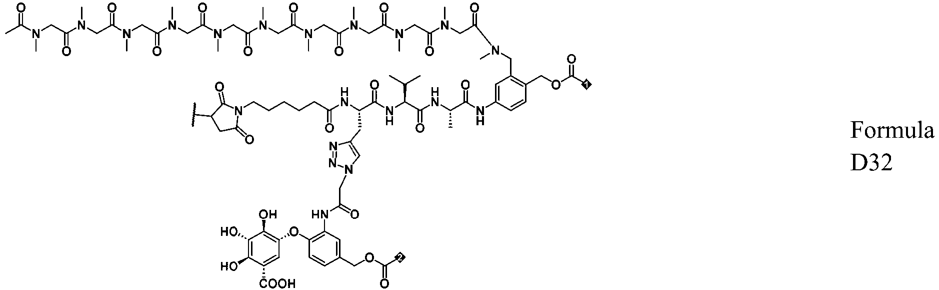

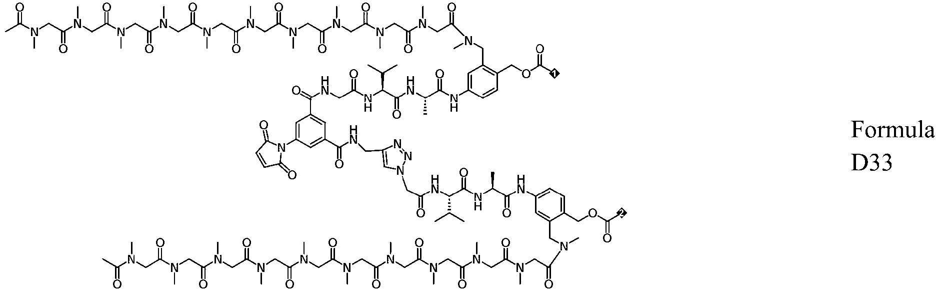

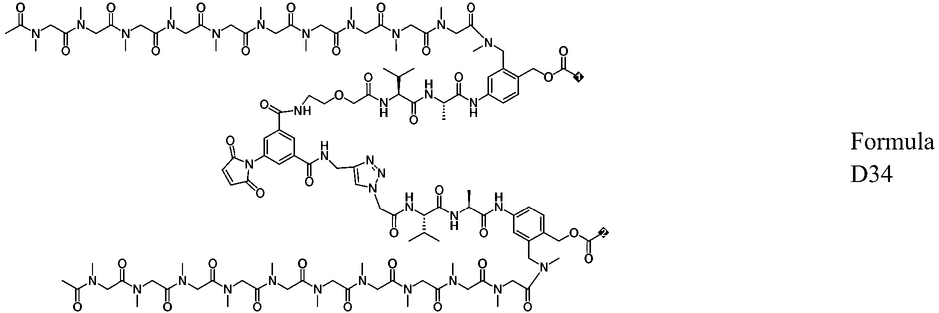

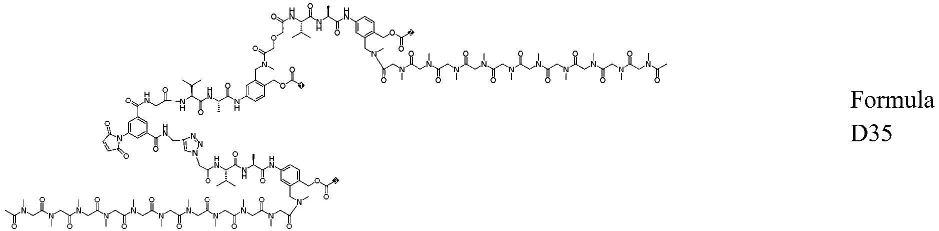

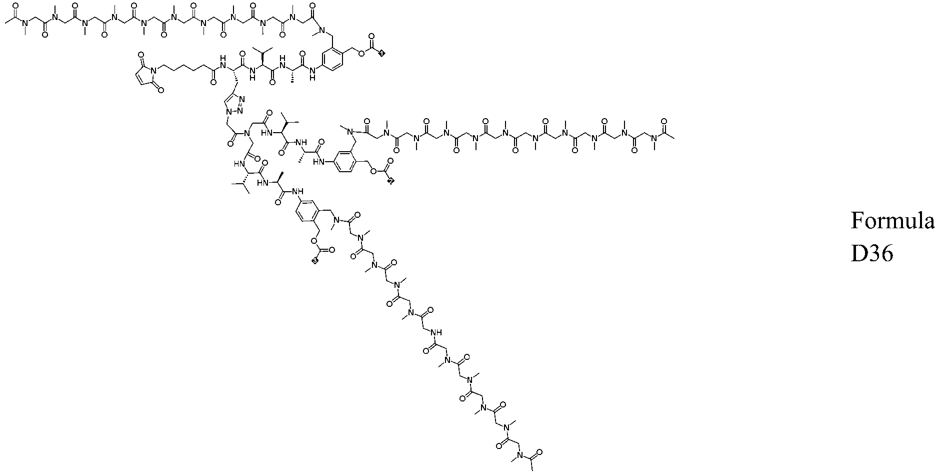

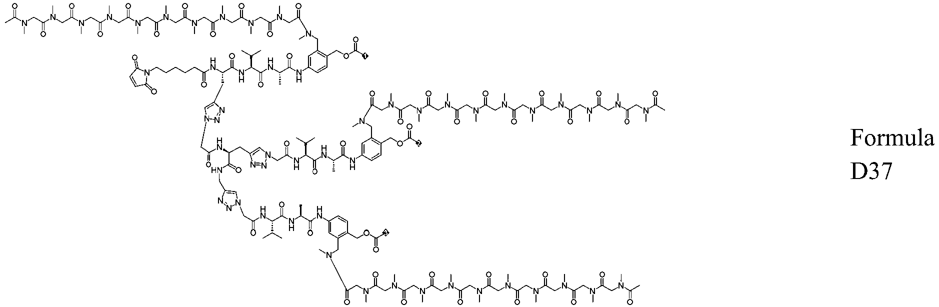

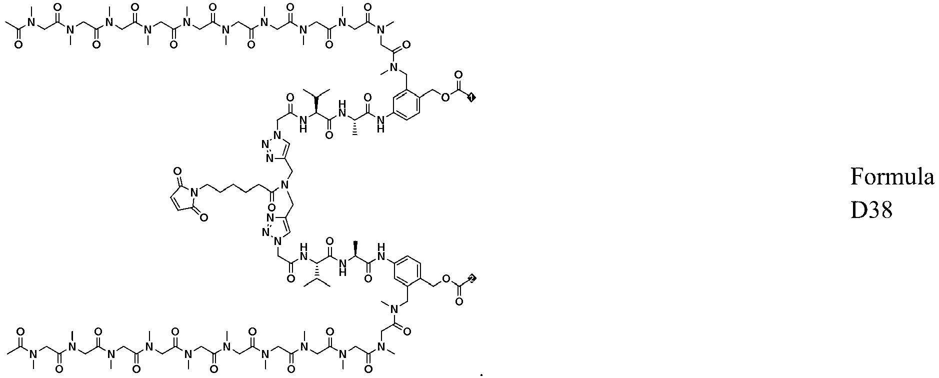

53. A pharmaceutical composition, comprising the compound, or a tautomer, a mesomer, a racemate, an enantiomer, or a diastereomer thereof, or a deuterated compound form thereof, or a mixture form thereof, or a pharmaceutically acceptable salt, prodrug, or solvate thereof, according to any one of claims 1 to 13 or 27 to 40; and a pharmaceutically acceptable carrier.

54. Use of the compound, or a tautomer, a mesomer, a racemate, an enantiomer, or a diastereomer thereof, or a deuterated compound form thereof, or a mixture form thereof, or a pharmaceutically acceptable salt, prodrug, or solvate thereof, according to any one of claims 1 to 40, or the linker according to any one of claims 41 to 52, or the pharmaceutical composition according to claim 53, in the manufacture of a medicament for treating and/or preventing a tumor.

55. The use according to claim 54, wherein the tumor is a solid tumor or a hematological malignancy; preferably, the tumor is a homologous recombination repair deficiency (HRD) related tumor; more preferably, the tumor is a tumor positive for or having high expression of a target selected from: HER2, HER3, TROP2, B7-H3, B7-H4, GPC20, GPRC5D, CDH6, EGFR, EGFRvIII, AXL, Nectin-4, Tissue factor, TIM-1, PSMA, PTK7, EpCAM, MUC1, STEAP1, GPNMB, FGF2, FOLR1, c-MET, GFR, AGS-16, Guanylyl cyclase C, Mesothelin, SLC44A4, EphA2, AGS-5, GPC-3, c-KIT, ROR1, ROR2, PD-L1, CD27L, 5T4, MerTK, Mucin 16, NaPi2b, STEAP, SLITRK6, ETBR, BCMA, CEACAM5, ICAM1, SC-16, SLC39A6, Delta-like protein3, Claudin 18.2, Claudin 3, Claudin 6, Claudin 9, CD19, CD20, CD22, CD30, CD33, CD37, CD45, CD56, CD66e, CD70, CD73, CD74, CD79b, CD123, CD138, CD147, CD166, CD223, MUC16, MSLN, ENPP3, SLTRK6, FGFR, LIV-1, Lewis Y, av-integrin, ITGB6 (integrin beta-6), ASCT2, C4.4a, CA-IX, CD324, CD352, CD44v6, CD48a, CLL-1, Cripto, CS1, DPEP3, Ephrin-A2, Ephrin-A4, ETBR, FGFR2, FGFR3, FLT3, GD3, Globo H, GPC3, LAMP-1, LRRC15, Ly6E, TM4SF1, MFI2, NOTCH3, p-cadherin, PRLR, CLDN1, CD228, CUB domain-containing protein 1 (CDCP1), CXCR4, LGR4, FZD7, CTR, GPR56, CCR7, DDR1 and RNF43; more preferably, the tumor is selected from breast cancer, ovarian cancer, non-Hodgkin's lymphoma, Hodgkin's lymphoma, acute lymphocytic leukemia, anaplastic large cell lymphoma, multiple myeloma, prostate cancer, non-small cell lung cancer, small cell lung cancer, malignant melanoma, squamous cell carcinoma, glioblastoma, renal cell carcinoma, gastrointestinal tumors, pancreatic cancer, prostate cancer, colorectal, gastric cancer, bladder cancer, gastrointestinal stromal tumor, cervical cancer, esophageal cancer, peritoneal cancer, liver cancer, colon cancer, glioma, mesothelioma, rectal cancer, colorectal cancer, uterine cancer, salivary gland cancer, kidney cancer, vulvar cancer, thyroid cancer, penile cancer, malignant lymphoma, plasmacytoma, myeloma, and sarcoma.

56. A method of combination administration, the combination administration comprising delivering at least two drugs selected from a first drug (D₁), a second drug (D₂), and a third drug (D₃) to tumor tissue in a same molecule, the method comprising forming at least two of D₁, D₂, and D₃ into a compound according to any one of claims 27 to 40; preferably, D₁, D₂, and D₃ are selected from the combinations shown in Table A.

57. A method for increasing the drug-to-antibody ratio (DAR value) of an ADC compound, the method comprising forming a drug into the compound according to any one of claims 27 to 40; preferably, the DAR value is an integer or decimal from 16 to 56.

58. A compound, or a tautomer, a mesomer, a racemate, an enantiomer, or a diastereomer thereof, or a deuterated compound form thereof, or a mixture form thereof, or a pharmaceutically acceptable salt, prodrug, or solvate thereof, wherein the compound is an antibody-drug conjugate, comprising an antibody molecule and at least two compounds conjugated to the antibody molecule, the at least two compounds conjugated to the antibody molecule are independently selected from the compound, or a tautomer, a mesomer, a racemate, an enantiomer, or a diastereomer thereof, or a deuterated compound form thereof, or a mixture form thereof, or a pharmaceutically acceptable salt, prodrug, or solvate thereof, according to any one of claims 14 to 26; preferably, the ratio of different drugs is any ratio from 1:1 to 1:24; preferably, the at least two compounds conjugated to the antibody molecule are conjugated to different conjugation sites on the antibody molecule; preferably, the different conjugation sites are selected from natural or modified sulfhydryl groups, amino groups, and non-natural amino acids of the antibody.
